(19) Europäisches Patentamt
European Patent Office
Office européen des brevets

(11) **EP 2 503 000 A1**

(12) # EUROPEAN PATENT APPLICATION

(43) Date of publication:
**26.09.2012 Bulletin 2012/39**

(21) Application number: **12170712.9**

(22) Date of filing: **31.07.2008**

(51) Int Cl.:
***C12N 15/82*** (2006.01) ***A01H 5/00*** (2006.01)
***A01H 5/10*** (2006.01) ***C12N 9/00*** (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.09.2007 US 970071 P**
**05.09.2007 US 970066 P**
**28.09.2007 US 975876 P**
**31.07.2007 EP 07113570**
**31.07.2007 EP 07113571**
**18.09.2007 EP 07116620**

(62) Document number(s) of the earlier application(s) in accordance with Art. 76 EPC:
**08786685.1 / 2 183 372**

(27) Previously filed application:
**31.07.2008 PCT/EP2008/060060**

(71) Applicants:
- **BASF Plant Science GmbH**
  **67056 Ludwigshafen (DE)**
- **Crop Functional Genomics Center**
  **151-921 Seoul (KR)**

(72) Inventors:
- **KIM, Woo Taek**
  **151-921 Gwanak-Gu (KR)**
- **CHOI, Seok Keun**
  **151-921 Gwanak-Gu (KR)**
- **HWANG, In Gyu**
  **151-921 Ganak-Gu (KR)**
- **MOON, Jae Sun**
  **151-921 Gwanak-Gu (KR)**
- **JWA, Nam-Soo**
  **151-921 Gwanak-Gu (KR)**
- **CHOI, Okhee**
  **151-921 Gwanak-Gu (KR)**
- **CHOI, Yang Do**
  **151-921 Gwanak-Gu (KR)**
- **PARK, Youn-Il**
  **151-921 Gwanak-Gu (KR)**
- **CHUNG, Suck Won**
  **151-921 Gwanak-Gu (KR)**

(74) Representative: **Fitzner, Uwe**
**Hauser Ring 10**
**40878 Ratingen (DE)**

Remarks:
This application was filed on 04-06-2012 as a divisional application to the application mentioned under INID code 62.

(54) **Plants having enhanced yield-related traits and a method for making the same**

(57) The present invention relates generally to the field of molecular biology and concerns a method for enhancing yield-related traits in plants by modulating expression in a plant of a nucleic acid encoding a yield increasing polypeptide selected from the group consisting of:
phosphoenolpyruvate carboxylase (PEPC),
Class III U-Box protein and
PQQC protein. The present invention also concerns plants having modulated expression of a nucleic acid encoding yield increasing polypeptide selected from the group consisting of:
phosphoenolpyruvate carboxylase (PEPC),
Class III U-Box protein and
PQQC protein, which plants have enhanced yield-related traits relative to corresponding wild type plants or other control plants. The invention also provides constructs useful in the methods of the invention.

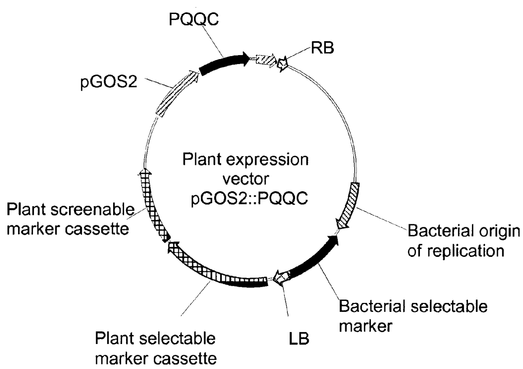


FIGURE 9B

EP 2 503 000 A1

## Description

The present invention relates generally to the field of molecular biology and concerns a method for enhancing yield-related traits in plants by modulating expression in a plant of a nucleic acid encoding a yield increasing polypeptide selected from the group consisting of:

phosphoenolpyruvate carboxylase (PEPC),

Class III U-Box protein and
PQQC protein. The present invention also concerns plants having modulated expression of a nucleic acid encoding a yield increasing polypeptide, which plants have enhanced yield-related traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example, for applications such as forage or wood production, or for bio-fuel production, an increase in the vegetative parts of a plant may be desirable, and for applications such as flour, starch or oil production, an increase or improvement of seed parameters may be particularly desirable. Even amongst the seed parameters, some may be favoured over others, depending on the end application. Various mechanisms may contribute to increasing seed yield, whether that is in the form of increased seed size or increased seed number or any other seed-related trait.

One approach to increasing yield (seed yield and/or biomass) in plants may be through modification of the inherent growth mechanisms of a plant, such as the cell cycle or various signalling pathways involved in plant growth or in defence mechanisms.

It has now been found that yield-related traits may be enhanced in plants by modulating expression in a plant of a nucleic acid encoding a yield increasing polypeptide selected from the group consisting of:

phosphoenolpyruvate carboxylase (PEPC),

Class III U-Box protein and
PQQC protein.

Background

Phosphoenolpyruvate carboxylase (also known as PEP carboxylase, PEPCase, or PEPC; EC 4.1.1.31) is an enzyme in the family of carboxy-lyases that catalyzes the addition of $CO_2$ to phosphoenolpyruvate (PEP) to form the four-carbon compound oxaloacetate:

$$PEP + CO_2 \rightarrow oxaloacetate + Pi$$

This reaction is used for carbon fixation in so-called "CAM" and "C4" plants where it plays a key role in photosynthesis. Besides plants, the enzyme is also found in some bacteria, but not in animals or fungi.

Carbon fixation via PEP carboxylase assimilates the available $CO_2$ into a four-carbon compound (oxaloacetate, which is further converted to malate) that can be stored or shuttled between plant cells. This allows for a separation of initial $CO_2$ fixation by contact with air and secondary $CO_2$ fixation into sugars by RuBisCO during the light-independent reactions of photosynthesis.

In succulent CAM plants adapted for growth in very dry conditions, PEP carboxylase fixes $CO_2$ during the night when the plant opens its stomata to allow for gas exchange. During the day time, the plant closes the stomata to preserve water and releases $CO_2$ inside the leaf from the storage compounds produced during the night. This allows the plants to thrive in dry climates by conducting photosynthesis without losing water through open stomata during the day.

In C4 plants, for example maize, PEP carboxylase fixes $CO_2$ in the mesophyll cells of the leaf and the resulting four-carbon compound shuttles into the bundle sheath cells where it releases $CO_2$ for fixation by RuBisCO. Thus, the two processes are separated spatially, allowing for RuBisCO to operate in a low-oxygen environment to circumvent photorespiration. Photorespiration occurs due to the inherent oxygenase activity of RuBisCO in which the enzyme uses oxygen instead of carbon dioxide without incorporating carbon into sugars or generating ATP. As such, it is a wasteful reaction for the plant. By comparison, C4 carbon fixation via PEP carboxylase is more efficient.

Radchuk et al., 2007 (The Plant Journal 51, 819-839) reported the overexpression of Corynebacterium PEPC in Vicia narbonensis seeds to alter seed metabolism and channel carbon into organic acids, resulting in greater seed storage capacity and increased protein content.

The U-box is a domain of ~70 amino acids that is present from yeast to humans. The prototype U-box protein, yeast Ufd2, was identified as a ubiquitin chain assembly factor (E4) that cooperates with a ubiquitin-activating enzyme (E1), a ubiquitin-conjugating enzyme (E2), and an E3 to catalyze the formation of a ubiquitin chain on artificial substrates, Hatakeyama et al., 2003 (Biochemical and Biophysical Research Communications 302, pp635-645). Azevedo et al. 2001 (Trends in Plant Science Vol. 6 No. 8). report that a database search revealed 37 predicted proteins containing a U-box in Arabidopsis, although UFD2 is reportedly the only U-box protein in yeast, and only a few have been identified in the human genome and in the genomes of Caenorhabditis elegans and Drosophila melanogaster. Based on domain analysis, the plant U-box (PUB) proteins were found to form five distinct subclasses. Class III was found to consist of 12 members with a domain containing ~14% leucine residues. The U-box domain in Class III members was found at the N-terminal region, in contrast to its position in other Class members.

The U-box proteins are predicted to be either E3 or E4 factors in the ubiquitination machinery

Cho et al., 2006 (Plant Physiology, December 2006, Vol. 142, pp.1664-1682) isolated a putative U-box protein 1 (CaPUB1) from water-stressed hot pepper (Capsicum annuum L. cv Pukang), encoding a protein containing a single U-box motif in its N-terminal region. In vitro ubiquitination and site-directed mutagenesis assays revealed that CaPUB1 possessed E3 ubiquitin ligase activity and that the U-box motif was essential for its enzyme activity. They overexpressed in Arabidopsis CaPUB1 under the control of the Cauliflower mosaic virus 35S promoter. Transgenic Arabidopsis plants were reported to have markedly longer hypocotyls and roots and grew more rapidly than the wild type, leading to an early bolting phenotype. The morphology of cotyledons was found to be similar in the dark. The roots of 35S::CaPUB1 were shown to have increased number of small sized cells resulting in disordered, highly populated cell layers in the cortex, endodermis and stele. Transgenic plants also displayed increased sensitivity to water stress and mild salinity.

PqqC facilitates cyclization and oxidation of 3a-(2-amino-2-carboxyethyl)-4,5-dioxo-4,5,6,7,8,9-hexahydroquinoline-7,9-dicarboxylic acid to PQQ in a reaction that occurs in the absence of any apparent cofactor. Pyrroloquinoline quinone [4,5-dihydro-4,5-dioxo-1H-pyrrolo- [2,3-f]quinoline-2,7,9-tricarboxylic acid; PQQ] is an aromatic, tricyclic ortho-quinone that serves as the redox cofactor for several bacterial dehydrogenases. Among the best known examples are methanol dehydrogenase and glucose dehydrogenase. PQQ belongs to the family of quinone cofactors that has been recognized as the third class of redox cofactors following pyridine nucleotide- and flavin-dependent cofactors. Although plants and animals do not produce PQQ themselves, PQQ has invoked considerable interest because of its presence in human milk and its antioxidant properties.

Genes involved in PQQ synthesis have been cloned from Acinetobacter calcoaceticus, Klebsiella, Pseudomonas Fluorescens, Methylobacterium organophilum and Methylobacterium extorquens.

PqqCcatalyses the last step in PQQ biosynthesis. PqqC is a 251-residue protein (molecular mass 28.91 kDa) that forms a homodimer in solution, as evident from gel filtration experiments. PqqC folds into a compact seven-helix bundle, with six circular aligned helices, partly embracing a seventh hydrophobic helix. Analysis of the PqqC structure shows that the seven helices provide the scaffold for an active site cavity. The cavity is lined with 42 mostly hydrophilic and aromatic residues that are highly conserved within PqqC proteins from different bacteria. The cavity shows a distinct overall positive charge.

Summary

Surprisingly, it has now been found that modulating expression of a nucleic acid encoding a yield increasing polypeptide selected from the group consisting of: phosphoenolpyruvate carboxylase (PEPC),

Class III U-Box protein and
PQQC protein gives plants having enhanced yield-related traits, in particular increased yield relative to control plants.

It was particularly surprising to find that the increase in yield was not manifested in the form of bigger seeds, as might have been expected from the teachings of Radchuck et al., 2007, but was manifested in the form of an array of altered yield-related traits including, but not limited to, increased aboveground area, increased emergence vigour, increased total number of seeds, increased seed weight, increased fill rate, increased number of flowers per panicle, increased number of seeds, increased harvest index, increased height and an increase in the root thickness, each relative to control plants.

According one embodiment, there is provided a method for enhancing yield related traits in plants relative to control plants, comprising modulating expression of a nucleic acid encoding a PEPC protein in a plant.

According one embodiment, there is provided a method for enhancing yield related traits, in particular increased yield in plants relative to control plants, comprising modulating expression of a nucleic acid encoding a Class III U-Box protein in a plant.

According one embodiment, there is provided a method for enhancing yield related traits, in particular increased yield in plants relative to control plants, comprising modulating expression of a nucleic acid encoding a PQQC protein in a plant.

Definitions

<u>Polypeptide(s)/Protein(s)</u>

The terms "polypeptide" and "protein" are used interchangeably herein and refer to amino acids in a polymeric form of any length, linked together by peptide bonds.

<u>Polynucleotide(s)/Nucleic acid(s)/Nucleic acid sequence(s)/nucleotide sequence(s)</u>

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

<u>Control plant(s)</u>

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

<u>Homologue(s)</u>

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures).

Amino acid substitutions are typically of single residues, but may be clustered depending upon functional constraints placed upon the polypeptide; insertions will usually be of the order of about 1 to 10 amino acid residues. For large proteins, the number of insertions may even be in the order of 50, 100, 150, 200 or more. The amino acid substitutions are preferably conservative amino acid substitutions. Conservative substitution tables are well known in the art (see for example Creighton (1984) Proteins. W.H. Freeman and Company (Eds) and Table 1 below).

Table 1: Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

<u>Derivatives</u>

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or-10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

Table 2a: Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| ocs | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

Table 2b: Examples of root-specific promoters

| Gene Source | Reference |
|---|---|
| RCc3 | Plant Mol Biol. 1995 Jan;27(2):237-48 |
| Arabidopsis phosphate transporter PHT1 | Kovama et al., 2005 |
| Medicago phosphate transporter | Xiao et al., 2006 |
| Arabidopsis Pyk10 | Nitz et al. (2001) Plant Sci 161(2): 337-346 |

A seed-specific promoter is transcriptionally active predominantly in seed tissue, but not necessarily exclusively in seed tissue (in case of leaky expression). The seed-specific promoter may be active during seed development and/or during germination. The seed specific promoter may be endosperm/aleurone/embryo specific. Examples of seed-specific promoters are shown in Tables 2c, d, e and f below. Further examples of seed-specific promoters are given in Qing Qu and Takaiwa (Plant Biotechnol. J. 2, 113-125, 2004), which disclosure is incorporated by reference herein as if fully set forth.

Table 2c: Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophos-phorylase | Trans Res 6:157-68, 1997 |

(continued)

| Gene source | Reference |
|---|---|
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

Table 2d: examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |

(continued)

| Gene source | Reference |
|---|---|
| sorghum kafirin | DeRose et al. (1996) Plant Mol Biol 32:1029-35 |

Table 2e: Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

Table 2f: Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

Table 2g: Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, preferably the expression level is increased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an $\alpha$-anomeric nucleic acid sequence. An $\alpha$-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116). Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, a polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

<u>Transgenic/Transgene/Recombinant</u>

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, such as plants used as a model, like Arabidopsis (Arabidopsis thaliana is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss Physcomitrella. Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per acre for a crop and year, which is determined by dividing total production (includes both harvested and appraised production) by planted acres. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per hectare or acre; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Triticosecale rimpaui, Triticum spp. (e.g. Triticum aestivum, Triticum durum, Triticum turgidum, Triticum hybernum, Triticum macha, Triticum sativum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

Detailed description of the invention

Surprisingly, it has now been found that modulating expression in a plant of a nucleic acid encoding a yield increasing polypeptide selected from the group consisting of: phosphoenolpyruvate carboxylase (PEPC),
Class III U-Box protein and
PQQC protein
gives plants having enhanced yield-related traits relative to control plants. According to a first embodiment, the present invention provides a method for enhancing yield-related traits in plants relative to control plants, comprising modulating expression in a plant of a nucleic acid encoding a PEPC protein.

In one embodiment the method for modulating (preferably, increasing) expression of a nucleic acid encoding a PEPC protein is by introducing and expressing in a plant a nucleic acid encoding a PEPC protein.

In one embodiment the method for modulating (preferably, increasing) expression of a nucleic acid encoding a Class III U-Box protein is by introducing and expressing in a plant a nucleic acid encoding a Class III U-Box protein.

In one embodiment the method for modulating (preferably, increasing) expression of a nucleic acid encoding a PQQC protein is by introducing and expressing in a plant a nucleic acid encoding a PQQC protein.

In one embodiment any reference hereinafter to a "protein useful in the methods of the invention" is taken to mean a PEPC protein as defined herein. Any reference hereinafter to a "nucleic acid useful in the methods of the invention" is taken to mean a nucleic acid capable of encoding such a PEPC protein. The nucleic acid to be introduced into a plant (and therefore useful in performing the methods of the invention) is any nucleic acid encoding the type of protein which will now be described, hereafter also named "PEPC nucleic acid" or "PEPC gene".

In one embodiment any reference hereinafter to a "protein useful in the methods of the invention" is taken to mean a Class III U-Box protein as defined herein. Any reference hereinafter to a "nucleic acid useful in the methods of the invention" is taken to mean a nucleic acid capable of encoding such a Class III U-Box protein. The nucleic acid to be introduced into a plant (and therefore useful in performing the methods of the invention) is any nucleic acid encoding the type of protein which will now be described, hereinafter also named "Class III U-Box nucleic acid" or "Class III U-Box gene".

In one embodiment any reference hereinafter to a "protein useful in the methods of the invention" is taken to mean a PQQC protein as defined herein. Any reference hereinafter to a "nucleic acid useful in the methods of the invention" is taken to mean a nucleic acid capable of encoding such a PQQC protein. The nucleic acid to be introduced into a plant (and therefore useful in performing the methods of the invention) is any nucleic acid encoding the type of protein which will now be described, hereafter also named "PQQC nucleic acid" or "PQQC gene".

A "PEPC polypeptide" as defined herein refers to the PEPC polypeptide represented by SEQ ID NO: 2 and to orthologues and paralogues thereof. Orthologues and paralogues of SEQ ID NO: 2 are identifiable by performing a so-called reciprocal blast search. Typically, this involves a first BLAST involving BLASTing a query sequence (for example using any of the sequences listed in Table A of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against Synechocystis sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Examples of nucleic acids encoding Class III U-Box polypeptides are given in Table A of Example 1 herein. Such nucleic acids are useful in performing the methods of the invention. The amino acid sequences given in Table A of Example 1 are example sequences of orthologues and paralogues of the Class III U-Box polypeptide represented by SEQ ID NO: 75, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 74 or SEQ ID NO: 75, the second BLAST would therefore be against Capsicum sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

A "PQQC polypeptide" as defined herein refers to the PQQC polypeptide represented by SEQ ID NO: 105 and to orthologues and paralogues thereof. Orthologues and paralogues of SEQ ID NO: 105 are identifiable by performing a so-called reciprocal blast search. Typically, this involves a first BLAST involving BLASTing a query sequence (for example using any of the sequences listed in Table A of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 104 or SEQ ID NO: 105, the second BLAST would therefore be against Pseudomonas sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Preferably, PEPC polypeptides have, in increasing order of preference, at least 30%, 35% 40%, 45%, 50%, 55%, 60%, 65%, 70%, 75%, 80%, 85%, 90%, 95%, 96%, 97%, 98% or 99% or more sequence identity to the polypeptide represented by SEQ ID NO: 2.

Preferably, PEPC polypeptides have a conserved C-terminal region having, in increasing order of preference, at least 70%, 75%, 80%, 85%, 90%, 95%, 96%, 97%, 98% or 99% or more sequence identity to the C-terminal region of the polypeptide represented by SEQ ID NO: 2. A person skilled in the art will be well aware of the extent of a C-terminal region of a protein. The C-terminal region may be regarded as about the last third of a protein.

PEPC polypeptides comprise several motifs which are well-conserved across PEPC-containing species. For example, Motifs I to IV below are typically found in both plant and micoorganism PEPCs.

Motif I: QEIMVGYSDSNKD

Motif II: FHGRGGSVGRGGGPAYKAIL

Motif III: LRAIPWVF

Motif IV: GIAAGMRNTG

PEPCs useful in the methods of the invention therefore preferably comprise any one or more of Motifs I to IV.

PEPCs useful in the methods of the invention may also comprise a motif having, in increasing order of preference, at least 65% 70%, 75%, 80%, 85%, 90%, 95%, 96%, 97%, 98% or 99% identity to any one or more of Motifs I to IV.

Motif I is preferably: QE V/I M I/L/V GYSDS G/N/S K D/F.

Motif II is preferably: FHGRGG T/S V/I GRGGGP T/S/A H/Y L/I/E/D/K Al L/Q/V.

Motif III is preferably: LRAIPW I/L/V F.

Motif IV is preferably: G I/V A/S AG L/M Q/R/K N T/S G.

PEPC polypeptides from micoorganisms also comprise several motifs that are well-conserved across micoorganisms, for example, Motif V below.

Motif V: SGFLSSNWE

Motif V is preferably: S/G G F/I L/T/V S/T S N/Q/G WE

Motif V or a motif having, in increasing order of preference, at least 65% 70%, 75%, 80%, 85%, 90%, 95%, 96%, 97%, 98% or 99% to Motif V may be useful in performing the methods of the invention.

PEPC polypeptides from plants also comprise several motifs that are well-conserved across plants, for example, Motif VI below.

Motif VI: SYKEWPEDKR

Motif VI is preferably: S/T Y S/L/K/A/R/Q E/K/D W S/P/T/D E E/D R/G/K/Q R/K

Motif VI or a motif having, in increasing order of preference, at least 65% 70%, 75%, 80%, 85%, 90%, 95%, 96%, 97%, 98% or 99% to Motif VI may be useful in performing the methods of the invention.

A "Class III U-Box polypeptide" as defined herein refers to any polypeptide comprising: A U-Box domain in the N-terminal region of a protein.

Preferably, the U-box domain has, in increasing order of preference, at least 50%, 55%, 60%, 65%, 70%, 75%, 80%, 85%, 90%, 95%, 96%, 97%, 98%, 99% or more sequence identity to the U-box domain of SEQ ID NO: 76; and/or comprises Motif VII: GI T/S YDR E/S/D/A N/S/G/A I E/A K/R/M W L/M/V/I F/L/A and/or Motif II: Q S/A/G W C/G/A I/R/A/T S/V/L/R/A H/N/R/N/V A/C/S.

Most preferably, Motif VII is: GITYDRENIERWIF (SEQ ID NO: 77), or a motif having in increasing order of preference, at least 60%, 65%, 70%, 75%, 80%, 85%, 90%, 95%, 96%, 97%, 98%, 99% or more sequence identity to Motif VII; and

Motif VIII is: QSWCTLNA (SEQ ID NO: 78) or a motif having in increasing order of preference, at least 60%, 65%, 70%, 75%, 80%, 85%, 90%, 95%, 96%, 97%, 98%, 99% or more sequence identity to Motif VIII.

Additionally, the Class III U-Box polypeptide may comprise in its C-terminal region one or more of the following motifs:

Motif IX: GR S/A/L A/E L/F L/V S/K/N/A H

Motif X: G/A I/UV A A/I/V V S/C/A/G/T K/C/R R/K/A A/I/V/T M/L/T R V/L/I S/P

Motif XI: K/R L C/L F/V/M/L V/L/A V/L/A/I Q/R V/A/S D/S/G/E

Motif XII: S/A/L YP

Most preferably, Motif IX is: GRAELLNH (SEQ ID NO: 79) or a motif having in increasing order of preference, at least 60%, 65%, 70%, 75%, 80%, 85%, 90%, 95%, 96%, 97%, 98%, 99% or more sequence identity to Motif IX;

Motif X is: GLAIVSKKILRVS (SEQ ID NO: 80) or a motif having in increasing order of preference, at least 60%, 65%, 70%, 75%, 80%, 85%, 90%, 95%, 96%, 97%, 98%, 99% or more sequence identity to Motif X;

Motif XI is: KLCLVLQVD (SEQ ID NO: 81) or a motif having in increasing order of preference, at least 60%, 65%, 70%, 75%, 80%, 85%, 90%, 95%, 96%, 97%, 98%, 99% or more sequence identity to Motif XI; and

Motif XII is: SYP (SEQ ID NO: 82) or a motif having in increasing order of preference, at least 60%, 65%, 70%, 75%, 80%, 85%, 90%, 95%, 96%, 97%, 98%, 99% or more sequence identity to Motif XII.

Class III U-Box polypeptides typically cluster with other Class III U-Box polypeptides rather than with any other class of U-box protein in a phylogenetic tree comprising full length U-box proteins.

Class III U-Box polypeptides typically have, in increasing order of preference, at least 50%, 55%, 60%, 65%, 70%, 75%, 80%, 85%, 90%, 95%, 96%, 97%, 98%, 99% or more sequence identity to the Class III U-box polypeptide represented by SEQ ID NO: 75.

Preferably, PQQC polypeptides have, in increasing order of preference, at least 45%, 50%, 55%, 60%, 65%, 70%, 75%, 80%, 85%, 90%, 95%, 96%, 97%, 98% or 99% or more sequence identity to the polypeptide represented by SEQ ID NO: 105.

Examples of nucleic acids encoding PQQC polypeptides are given in Table A of Example 1 herein. Such nucleic acids are useful in performing the methods of the invention. The amino acid sequences given in Table A of Example 1 are examples of orthologues and paralogues of the PQQC polypeptide represented by SEQ ID NO: 105, the terms "orthologues" and "paralogues" being as defined herein.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for in silico analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters.

Examples of nucleic acids encoding PEPC polypeptides are given in Table A of Example 1 herein. Such nucleic acids are useful in performing the methods of the invention. The amino acid sequences given in Table A of Example 1 are examples of orthologues and paralogues of the PEPC polypeptide represented by SEQ ID NO: 2, the terms "orthologues" and "paralogues" being as defined herein.

Another preferred feature of PEPC is that it catalyzes the addition of $CO_2$ to phosphoenolpyruvate (PEP) to form the four-carbon compound oxaloacetate:

$$PEP + CO_2 \rightarrow oxaloacetate + Pi$$

For a description of the measurement of PEPC activity, see Chen et al., 2002 (Plant Cell Physiol 43(2): 159-169); and Chen et al., 2004 (Planta 219 pp440-449).

Another feature of the PEPC is that it falls under Enzyme Classification code EC 4.1.1.31

In one embodiment the present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any PEPC-encoding nucleic acid or PEPC polypeptide as defined herein.

Furthermore, Class III U-Box polypeptides (at least in their native form) typically have E3 ubiquitin ligase activity (see Cho et al., 2006).

In one embodiment the present invention is illustrated by transforming plants with the nucleic acid sequence represented by SEQ ID NO: 74, encoding the polypeptide sequence of SEQ ID NO: 75. However, performance of the invention is not restricted to these sequences; the methods of the invention may advantageously be performed using any Class III U-Box-encoding nucleic acid or Class III U-Box polypeptide as defined herein.

Another feature of PQQC is that it catalyses the last step in PQQ biosynthesis.

In one embodiment the present invention is illustrated by transforming plants with the nucleic acid sequence represented by SEQ ID NO: 104, encoding the polypeptide sequence of SEQ ID NO: 105. However, performance of the invention is not restricted to these sequences; the methods of the invention may advantageously be performed using any PQQC-encoding nucleic acid or PQQC polypeptide as defined herein.

Nucleic acid variants may also be useful in practising the methods of the invention. Examples of such variants include nucleic acids encoding homologues and derivatives of any one of the amino acid sequences given in Table A of Example 1, the terms "homologue" and "derivative" being as defined herein. Also useful in the methods of the invention are nucleic acids encoding homologues and derivatives of orthologues or paralogues of any one of the amino acid sequences given in Table A of Example 1. Homologues and derivatives useful in the methods of the present invention have substantially the same biological and functional activity as the unmodified protein from which they are derived.

Further nucleic acid variants useful in practising the methods of the invention include portions of nucleic acids encoding yield increasing polypeptide selected from the group consisting of:

PEPC, Class III U-Box and PQQC polypeptides, nucleic acids hybridising to nucleic acids encoding yield increasing polypeptide selected from the group consisting of:
PEPC, Class III U-Box and PQQC polypeptides, splice variants of nucleic acids encoding yield increasing polypeptide selected from the group consisting of: PEPC,
Class III U-Box and PQQC polypeptides, allelic variants of nucleic acids encoding yield increasing polypeptide selected from the group consisting of: PEPC, Class III U-Box and PQQC polypeptides and variants of nucleic acids encoding yield increasing polypeptide selected from the group consisting of: PEPC, Class III U-Box and PQQC polypeptides obtained by gene shuffling. The terms hybridising sequence, splice variant, allelic variant and gene shuffling are as described herein.

Nucleic acids encoding yield increasing polypeptide selected from the group consisting of: PEPC, Class III U-Box and PQQC polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A of Example 1, or a portion of a nucleic acid encoding an orthologue, paralogue or homologue of any of the amino acid sequences given in Table A of Example 1.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

In one embodiment the portions useful in the methods of the invention, encode a PEPC polypeptide as defined herein, and have substantially the same biological activity as the amino acid sequences given in Table A of Example 1. Preferably, the portion is a portion of any one of the nucleic acids given in Table A of Example 1, or is a portion of a nucleic acid encoding an orthologue or paralogue of any one of the amino acid sequences given in Table A of Example 1. Preferably the portion is at least 2000, 2250, 2500, 2750, 3000, 3100 consecutive nucleotides in length, the consecutive nucleotides being of any one of the nucleic acid sequences given in Table A of Example 1, or of a nucleic acid encoding an orthologue or paralogue of any one of the amino acid sequences given in Table A of Example 1. Most preferably the portion is a portion of the nucleic acid of SEQ ID NO: 1.

In one embodiment the portions useful in the methods of the invention, encode a Class III U-Box polypeptide as defined herein, and have substantially the same biological activity as the amino acid sequences given in Table A of Example 1. Preferably, the portion is a portion of any one of the nucleic acids given in Table A of Example 1, or is a portion of a nucleic acid encoding an orthologue or paralogue of any one of the amino acid sequences given in Table A of Example 1. Preferably the portion is at least 500, 550, 600, 650, 700, 750, 800, 850, 900, 950, 1000, 1050, 1100, 1150, 1200, 1250, 1300, 1350 consecutive nucleotides in length, the consecutive nucleotides being of any one of the nucleic acid sequences given in Table A of Example 1, or of a nucleic acid encoding an orthologue or paralogue of any one of the amino acid sequences given in Table A of Example 1. Most preferably the portion is a portion of the nucleic acid of SEQ ID NO: 74.

In one embodiment the portions useful in the methods of the invention, encode a PQQC polypeptide as defined herein, and have substantially the same biological activity as the amino acid sequences given in Table A of Example 1. Preferably, the portion is a portion of any one of the nucleic acids given in Table A of Example 1, or is a portion of a nucleic acid encoding an orthologue or paralogue of any one of the amino acid sequences given in Table A of Example 1. Preferably the portion is at least 500, 550, 600, 650, 700, 750 consecutive nucleotides in length, the consecutive nucleotides being of any one of the nucleic acid sequences given in Table A of Example 1, or of a nucleic acid encoding an orthologue or paralogue of any one of the amino acid sequences given in Table A of Example 1. Most preferably the portion is a portion of the nucleic acid of SEQ ID NO: 104.

Another nucleic acid variant useful in the methods of the invention is a nucleic acid capable of hybridising, under reduced stringency conditions, preferably under stringent conditions, with a nucleic acid encoding a yield increasing polypeptide selected from the group consisting of: PEPC, Class III U-Box and PQQC polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A of Example 1, or comprising introducing and expressing in a plant a nucleic acid capable of hybridising to a nucleic acid encoding an orthologue, paralogue or homologue of any of the nucleic acid sequences given in Table A of Example 1.

Hybridising sequences useful in the methods of the invention encode a PEPC polypeptide as defined herein, having substantially the same biological activity as the amino acid sequences given in Table A of Example 1. Preferably, the hybridising sequence is capable of hybridising to any one of the nucleic acids given in Table A of Example 1, or to a portion of any of these sequences, a portion being as defined above, or the hybridising sequence is capable of hybridising to a nucleic acid encoding an orthologue or paralogue of any one of the amino acid sequences given in Table A of Example 1. Most preferably, the hybridising sequence is capable of hybridising to a nucleic acid as represented by a sequence selected from the group consisting of SEQ ID NO: 1, SEQ ID NO: 74 and SEQ ID NO: 104 or to a portion thereof, a portion being as defined above.

Another nucleic acid variant useful in the methods of the invention is a splice variant encoding a yield increasing polypeptide selected from the group consisting of: PEPC, Class III U-Box and PQQC polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A of Example 1, or a splice variant of a nucleic acid encoding an orthologue, paralogue or homologue of any of the amino acid sequences given in Table A of Example 1.

In one embodiment preferred splice variants are splice variants of a nucleic acid represented by SEQ ID NO: 1, or a splice variant of a nucleic acid encoding an orthologue or paralogue of SEQ ID NO: 2.

In one embodiment preferred splice variants are splice variants of a nucleic acid represented by SEQ ID NO: 74, or a splice variant of a nucleic acid encoding an orthologue or paralogue of SEQ ID NO: 75.

In one embodiment preferred splice variants are splice variants of a nucleic acid represented by SEQ ID NO: 104, or a splice variant of a nucleic acid encoding an orthologue or paralogue of SEQ ID NO: 105.

Another nucleic acid variant useful in performing the methods of the invention is an allelic variant of a nucleic acid encoding a yield increasing polypeptide selected from the group consisting of: phosphoenolpyruvate carboxylase (PEPC), Class III U-Box protein and PQQC polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A of Example 1, or comprising introducing and expressing in a plant an allelic variant of a nucleic acid encoding an orthologue, paralogue or homologue of any of the amino acid sequences given in Table A of Example 1.

In one embodiment the allelic variants useful in the methods of the present invention have substantially the same biological activity as the PEPC polypeptide of SEQ ID NO: 2 and any of the amino acids depicted in Table A of Example 1. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2.

In one embodiment the allelic variants useful in the methods of the present invention have substantially the same biological activity as the Class III U-Box polypeptide of SEQ ID NO: 75 and any of the amino acids depicted in Table A of Example 1. Allelic variants exist in nature, and encompassed within the methods of the present invention is the use of these natural alleles. Preferably, the allelic variant is an allelic variant of SEQ ID NO: 74 or an allelic variant of a nucleic acid encoding an orthologue or paralogue of SEQ ID NO: 75.

In one embodiment the allelic variants useful in the methods of the present invention have substantially the same biological activity as the PQQC polypeptide of SEQ ID NO: 105 and any of the amino acids depicted in Table A of Example 1. Allelic variants exist in nature, and encompassed within the methods of the present invention is the use of these natural alleles. Preferably, the allelic variant is an allelic variant of SEQ ID NO: 104 or an allelic variant of a nucleic acid encoding an orthologue or paralogue of SEQ ID NO: 105.

Gene shuffling or directed evolution may also be used to generate variants of nucleic acids encoding yield increasing polypeptide selected from the group consisting of: PEPC, Class III U-Box and PQQC polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A of Example 1, or comprising introducing and expressing in a plant a variant of a nucleic acid encoding an orthologue, paralogue or homologue of any of the amino acid sequences given in Table A of Example 1, which variant nucleic acid is obtained by gene shuffling.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

In one embodiment nucleic acids encoding PEPC polypeptides may be derived from any natural or artificial source. The nucleic acid may be modified from its native form in composition and/or genomic environment through deliberate human manipulation. Preferably the PEPC polypeptide-encoding nucleic acid is bacterial, further preferably from any one of Synechococcus, Anacystis, Anabaena, Nostoc, Prochlorococcus, Arthrobacter, Steptomyces, Kineococcus, Nitobacter, Ralsstonia, Nitrosomonas, Rhodococcus, Rhodopseudomonas, Mycobacterium, Neiseria, Lactobacillus, Corynebacterium, most preferably from Synechocystis.

In one embodiment nucleic acids encoding Class III U-Box polypeptides may be derived from any natural or artificial source. The nucleic acid may be modified from its native form in composition and/or genomic environment through deliberate human manipulation. Preferably the Class III U-Box polypeptide-encoding nucleic acid is from a plant, further preferably from a dicotyledonous plant, more preferably from the family Solanaceae, most preferably the nucleic acid is from Capsicum annuum.

In one embodiment nucleic acids encoding PQQC polypeptides may be derived from any natural or artificial source. The nucleic acid may be modified from its native form in composition and/or genomic environment through deliberate human manipulation. Preferably the PQQC polypeptide-encoding nucleic acid is bacterial, further preferably from Pseudomonas.

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

It was particularly surprising to find that the increase in yield was not manifested in the form of bigger seeds, as might have been expected from the teachings of Radchuck et al., 2007, but was manifested in the form of an array of altered yield-related traits including, but not limited to, increased aboveground area, increased emergence vigour, increased total number of seeds, increased seed weight, increased fill rate, increased number of flowers per panicle, increased number of seeds, increased harvest index, increased height and an increase in the root thickness, each relative to control plants.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression, preferably increasing expression, in a plant of a nucleic acid encoding a yield increasing polypeptide selected from the group consisting of: PEPC, Class III U-Box and PQQC polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a yield increasing polypeptide selected from the group consisting of: PEPC, Class III U-Box and PQQC polypeptide as defined herein.

In one embodiment an increase in yield occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

In one embodiment an increase in yield occurs whether the plant is under non-stress conditions or under conditions of mild drought. Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a yield increasing polypeptide selected from the group consisting of: PEPC, Class III U-Box and PQQC polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a yield increasing polypeptide selected from the group consisting of: PEPC, Class III U-Box and PQQC polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a yield increasing polypeptide selected from the group consisting of: PEPC, Class III U-Box and PQQC polypeptide as defined above.

The invention also provides genetic constructs and vectors to facilitate introduction and/or expression in plants of nucleic acids encoding yield increasing polypeptide selected from the group consisting of: PEPC, Class III U-Box and PQQC polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a yield increasing polypeptide selected from the group consisting of: PEPC, Class III U-Box and PQQC polypeptide as defined above;

(b) one or more control sequences capable of driving expression of the nucleic acid sequence of (a); and optionally

(c) a transcription termination sequence.

Preferably, the nucleic acid encoding a PEPC polypeptide is as defined above.

Preferably, the nucleic acid encoding a Class III U-Box polypeptide is as defined above.

Preferably, the nucleic acid encoding a PQQC polypeptide is as defined above.

The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. See the "Definitions" section herein for definitions of the various promoter types.

A young green tissue-specific promoter is particularly useful in the methods.

In one embodiment the PEPC-encoding nucleic acid is operably linked to a young green tissue-specific promoter as defined herein. The young green tissue-specific promoter is preferably a protochlorophyllid reductase (PcR) promoter, more preferably the protochlorophyllid reductase promoter represented by a nucleic acid sequence substantially similar to SEQ ID NO: 71, most preferably the promoter is as represented by SEQ ID NO: 71.

In one embodiment the Class III U-box-encoding nucleic acid is operably linked to a young green tissue-specific promoter as defined herein. The young green tissue-specific promoter is preferably a protochlorophyllid reductase (PcR) promoter, more preferably the protochlorophyllid reductase promoter represented by a nucleic acid sequence substantially similar to SEQ ID NO: 101, most preferably the promoter is as represented by SEQ ID NO: 101.

In one embodiment the PQQC-encoding nucleic acid is operably linked to a young green tissue-specific promoter as defined herein. The young green tissue-specific promoter is preferably a protochlorophyllide reductase (PcR) promoter, more preferably the protochlorophyllide reductase promoter represented by a nucleic acid sequence substantially similar to SEQ ID NO: 131, most preferably the promoter is as represented by SEQ ID NO: 131.

Furthermore, a constitutive promoter is particularly useful in the methods. The constitutive promoter is preferably a GOS2 promoter, preferably a GOS2 promoter from rice. Further preferably the constitutive promoter is represented by a nucleic acid sequence substantially similar to SEQ ID NO: 227, most preferably the constitutive promoter is as represented by SEQ ID NO: 227. See Table 2 in the "Definitions" section herein for further examples of constitutive promoters.

It should be clear that the applicability of the present invention is not restricted to the PEPC -encoding nucleic acid represented by SEQ ID NO: 1, nor is the applicability of the invention restricted to expression of such a PEPC-encoding nucleic acid when driven by a protochlorophyllid reductase promoter. It should be clear that the applicability of the present invention is not restricted to the U-box-encoding nucleic acid represented by SEQ ID NO: 74, nor is the applicability of the invention restricted to expression of such a U-box-encoding nucleic acid when driven by a protochlorophyllid reductase promoter. It should be clear that the applicability of the present invention is not restricted to the PQQC -encoding nucleic acid represented by SEQ ID NO: 104, nor is the applicability of the invention restricted to expression of such a PQQC-encoding nucleic acid when driven by a protochlorophyllide reductase promoter or when driven by a GOS2 promoter.

Examples of other (young) green tissue-specific promoters which may also be used to perform the methods of the invention are shown in Table 2g herein and examples of other constitutive promoters are also given, see Table 2a.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding a yield increasing polypeptide selected from the group consisting of: PEPC, Class III U-Box and PQQC polypeptide as defined hereinabove.

More specifically, the present invention provides a method for the production of transgenic plants having increased enhanced yield-related traits, particularly increased (seed) yield, which method comprises:

(i) introducing and expressing in a plant or plant cell a - nucleic acid encoding a yield increasing polypeptide selected from the group consisting of: PEPC, Class III U-Box and PQQC ; and

(ii) cultivating the plant cell under conditions promoting plant growth and development.

In one embodiment thenucleic acid of (i) may be any of the nucleic acids capable of encoding a PEPC polypeptide as defined herein.

In one embodiment thenucleic acid of (i) may be any of the nucleic acids capable of encoding a Class III U-Box polypeptide as defined herein.

In one embodiment thenucleic acid of (i) may be any of the nucleic acids capable of encoding a PQQC polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a yield increasing polypeptide selected from the group consisting of: PEPC, Class III U-Box and PQQC polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane.

More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating (preferably, increasing) expression of a nucleic acid encoding a yield increasing polypeptide selected from the group consisting of: PEPC, Class III U-Box and PQQC polypeptide is by introducing and expressing in a plant a nucleic acid encoding a yield increasing polypeptide selected from the group consisting of: PEPC, Class III U-Box and PQQC polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

In one embodiment thepresent invention also encompasses use of nucleic acids encoding PEPC polypeptides as described herein and use of these PEPC polypeptides in enhancing any of the aforementioned yield-related traits in plants.

In one embodiment thepresent invention also encompasses use of nucleic acids encoding Class III U-Box polypeptides as described herein and use of these Class III U-Box polypeptides in enhancing any of the aforementioned yield-related traits in plants.

In one embodiment thepresent invention also encompasses use of nucleic acids encoding PQQC polypeptides as described herein and use of these PQQC polypeptides in enhancing any of the aforementioned yield-related traits in plants.

Nucleic acids encoding a yield increasing polypeptide selected from the group consisting of: PEPC, Class III U-Box and PQQC polypeptide described herein, or the yield increasing polypeptide selected from the group consisting of: PEPC, Class III U-Box and PQQC polypeptides themselves, may find use in breeding programmes in which a DNA marker is identified which may be genetically linked to a yield increasing polypeptide selected from the group consisting of: PEPC, Class III U-Box and PQQC polypeptide-encoding gene. The nucleic acids/genes, or the yield increasing polypeptide selected from the group consisting of: PEPC, Class III U-Box and PQQC polypeptides themselves may be used to define a molecular marker. This DNA or protein marker may then be used in breeding programmes to select plants having enhanced yield-related traits as defined hereinabove in the methods of the invention.

Allelic variants of a yield increasing polypeptide selected from the group consisting of: PEPC, Class III U-Box and PQQC polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding a yield increasing polypeptide selected from the group consisting of: PEPC, Class III U-Box and PQQC polypeptides may also be used as probes for genetically and physically mapping the genes that they are a part of, and as markers for traits linked to those genes. Such information may be useful in plant breeding in order to develop lines with desired phenotypes. Such use of a yield increasing polypeptide selected from the group consisting of: PEPC, Class III U-Box and PQQC polypeptide-encoding nucleic acids requires only a nucleic acid sequence of at least 15 nucleotides in length. The yield increasing polypeptide selected from the group consisting of: PEPC, Class III U-Box and PQQC polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the yield increasing polypeptide selected from the group consisting of: PEPC, Class III U-Box and PQQC -encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the yield increasing polypeptide selected from the group consisting of: PEPC, Class III U-Box and PQQC polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

The term "table A" used in this specification is to be taken to specify the content of table A1, A2 and/or A3. The term "table A1" used in this specification is to be taken to specify the content of table A1. The term "table A2" used in this specification is to be taken to specify the content of table A2. The term "table A3" used in this specification is to be taken to specify the content of table A3.

In one preferred embodiment, the term "table A" means table A1. In one preferred embodiment, the term "table A" means table A2. In one preferred embodiment, the term "table A" means table A3.

In one embodiment the invention relates to subject mater summarized as follows:

Item 1. A method for enhancing yield-related traits in plants relative to control plants, comprising modulating expression in a plant of a nucleic acid encoding a phosphoenolpyruvate carboxylase (PEPC) protein.

Item 2. Method according to item 1, wherein said PEPC protein is SEQ ID NO: 2 and orthologues and paralogues thereof.

Item 3. Method according to item 1 or 2, wherein said PEPC protein has a C-terminal region having, in increasing order of preference, at least 70%, 75%, 80%, 85%, 90%, 95%, 96%, 97%, 98% or 99% or more sequence identity to the C-terminal region of the polypeptide represented by SEQ ID NO: 2.

Item 4. Method according to any preceding item, wherein said PEPC protein comprises any one or more of the following motifs:

Motif I: QEIMVGYSDSNKD, or a motif having, in increasing order of preference, at least 65% 70%, 75%, 80%, 85%, 90%, 95%, 96%, 97%, 98% or 99% to Motif I;
Motif II: FHGRGGSVGRGGGPAYKAIL, or a motif having, in increasing order of preference, at least 65% 70%, 75%, 80%, 85%, 90%, 95%, 96%, 97%, 98% or 99% to Motif II;
Motif III: LRAIPWVF, or a motif having, in increasing order of preference, at least 65% 70%, 75%, 80%, 85%, 90%, 95%, 96%, 97%, 98% or 99% to Motif III;
Motif IV: GIAAGMRNTG, or a motif having, in increasing order of preference, at least 65% 70%, 75%, 80%, 85%, 90%, 95%, 96%, 97%, 98% or 99% to Motif IV;
Motif V: SGFLSSNWE, or a motif having, in increasing order of preference, at least 65% 70%, 75%, 80%, 85%, 90%, 95%, 96%, 97%, 98% or 99% to Motif V;
Motif VI: SYKEWPEDKR, or a motif having, in increasing order of preference, at least 65% 70%, 75%, 80%, 85%, 90%, 95%, 96%, 97%, 98% or 99% to Motif VI.

Item 5. Method according to item 4, wherein
Motif I is preferably: QE V/I M I/L/V GYSDS G/N/S K D/F;
Motif II is preferably: FHGRGG T/S V/I GRGGGP T/S/A H/Y L/I/E/D/K AI L/Q/V;
Motif III is preferably: LRAIPW I/L/V F;
Motif IV is preferably: G I/V A/S AG L/M Q/R/K N T/S G;
Motif V is preferably: S/G G F/I L/T/V S/T S N/Q/G WE;
Motif VI is preferably: S/T Y S/L/K/A/R/Q E/K/D W S/P/T/D E E/D R/G/K/Q R/K.

Item 6. Method according to any preceding item, wherein said modulated expression is effected by introducing and expressing in a plant a nucleic acid encoding a PEPC polypeptide.

Item 7. Method according to any preceding item, wherein said nucleic acid encoding a PEPC polypeptide encodes any one of the proteins listed in Table A or is a portion of such a nucleic acid, or a nucleic acid capable of hybridising with such a nucleic acid.

Item 8. Method according to any preceding item, wherein said enhanced yield-related traits comprise one or more of the following, increased aboveground area, increased emergence vigour, increased total number of seeds, increased seed weight, increased fill rate, increased number of flowers per panicle, increased number of seeds, increased harvest index, increased height and an increase in the root thickness, each relative to control plants.

Item 9. Method according to any preceding item, wherein said enhanced yield-related traits are obtained under non-stress conditions and under mild drought conditions. Item 9. Method according to any preceding item, wherein said enhanced yield-related traits are obtained under conditions of nitrogen deficiency.

Item 10. Method according to any preceding item, wherein said nucleic acid is operably linked to a young green tissue-specific promoter, preferably to a protochlorophyllid reductase promoter.

Item 11. Method according to any preceding item, wherein said nucleic acid encoding a PEPC polypeptide is of bacterial origin, preferably from Synechocystis.

Item 12. Plant or part thereof, including seeds, obtainable by a method according to any preceding item, wherein said plant or part thereof comprises a recombinant nucleic acid encoding a PEPC polypeptide.

Item 13. Construct comprising:

(i) nucleic acid encoding a PEPC polypeptide as defined in any one of items 1 to 5;
(ii) one or more control sequences capable of driving expression of the nucleic acid sequence of (a); and optionally
(iii) a transcription termination sequence.

Item 14. Construct according to item 13, wherein one of said control sequences is a young green tissue specific promoter, preferably a protochlorophyllid reductase promoter.

Item 15. Use of a construct according to item 13 or 14 in a method for making plants having one or more of the following: increased aboveground area, increased emergence vigour, increased total number of seeds, increased seed weight, increased fill rate, increased number of flowers per panicle, increased number of seeds, increased harvest index, increased height and an increase in the root thickness, each relative to control plants.

Item 16. Plant, plant part or plant cell transformed with a construct according to item 13 or 14.

Item 17. Method for the production of a transgenic plant having increased yield, particularly increased biomass and/or increased seed yield relative to control plants, comprising:

(i) introducing and expressing in a plant a nucleic acid encoding a PEPC polypeptide as defined in any one of items 1 to 5; and
(ii) cultivating the plant cell under conditions promoting plant growth and development.

Item 18. Transgenic plant having one or more of the following: increased aboveground area, increased emergence vigour, increased total number of seeds, increased seed weight, increased fill rate, increased number of flowers per panicle, increased number of seeds, increased harvest index, increased height and an increase in the root thickness, each relative to control plants, resulting from increased expression of a nucleic acid encoding a PEPC polypeptide as defined in any one of items 1 to 5, or a transgenic plant cell derived from said transgenic plant.
Item 19. Transgenic plant according to item 12, 16 or 18, or a transgenic plant cell derived thereof, wherein said plant is a crop plant or a monocot or a cereal, such as rice, maize, wheat, barley, millet, rye, triticale, sorghum and oats.
Item 20. Harvestable parts of a plant according to item 19, wherein said harvestable parts are preferably shoot biomass and/or seeds.
Item 21. Products derived from a plant according to item 19 and/or from harvestable parts of a plant according to item 20.
Item 22. Use of a nucleic acid encoding a PEPC polypeptide in increasing yield, particularly in increasing one or more of the following, increased aboveground area, increased emergence vigour, increased total number of seeds, increased seed weight, increased fill rate, increased number of flowers per panicle, increased number of seeds, increased harvest index, increased height and an increase in the root thickness, each relative to control plants.

In one embodiment the invention relates to subject mater summarized as follows:

Item 23. A method for enhancing yield-related traits in plants relative to control plants, comprising modulating expression in a plant of a nucleic acid encoding a class III U-box protein comprising a U-box domain in its N-terminal region.

Item24. Method according to item 23, wherein said U-box domain has, in increasing order of preference, at least 50%, 55%, 60%, 65%, 70%, 75%, 80%, 85%, 90%, 95%, 96%, 97%, 98%, 99% or more sequence identity to the U-box domain of SEQ ID NO: 76 and/or comprises Motif VII: GI T/S YDR E/S/D/A N/S/G/A I E/A K/R/M W L/M/V/I F/L/A and/or Motif VIII: Q S/A/G W C/G/A I/R/A/T S/V/L/R/A H/N/R/N/V A/C/S.

Item 25. Method according to item 24, wherein said Motif VII is: GITYDRENIERWIF (SEQ ID NO: 77), or a motif having in increasing order of preference, at least 60%, 65%, 70%, 75%, 80%, 85%, 90%, 95%, 96%, 97%, 98%, 99% or more sequence identity to Motif VII; and

Motif VIII is: QSWCTLNA (SEQ ID NO: 78) or a motif having in increasing order of preference, at least 60%, 65%, 70%, 75%, 80%, 85%, 90%, 95%, 96%, 97%, 98%, 99% or more sequence identity to Motif VIII.

Item 26. Method according to any preceding item 23 to 25, wherein the Class III U-Box polypeptide comprises in its C-terminal region one or more of the following motifs:

Motif IX: GR S/A/L A/E L/F L/V S/K/N/A H

Motif X: G/A I/L/V A A/I/V V S/C/A/G/T K/C/R R/K/A A/I/V/T M/L/T R V/L/I S/P

Motif XI: K/R L C/L F/V/M/L V/L/A V/L/A/I Q/R V/A/S D/S/G/E

Motif XII: S/A/L YP

Item 27. Method according to item 26, wherein Motif IX is: GRAELLNH (SEQ ID NO: 79) or a motif having in increasing order of preference, at least 60%, 65%, 70%, 75%, 80%, 85%, 90%, 95%, 96%, 97%, 98%, 99% or more sequence identity to Motif IX;
Motif X is: GLAIVSKKILRVS (SEQ ID NO: 80) or a motif having in increasing order of preference, at least 60%, 65%, 70%, 75%, 80%, 85%, 90%, 95%, 96%, 97%, 98%, 99% or more sequence identity to Motif X;
Motif XI is: KLCLVLQVD (SEQ ID NO: 81) or a motif having in increasing order of preference, at least 60%, 65%, 70%, 75%, 80%, 85%, 90%, 95%, 96%, 97%, 98%, 99% or more sequence identity to Motif XI; and
Motif XII is: SYP (SEQ ID NO: 82) or a motif having in increasing order of preference, at least 60%, 65%, 70%, 75%, 80%, 85%, 90%, 95%, 96%, 97%, 98%, 99% or more sequence identity to Motif XII.

Item 28. Method according to any preceding item 23 to 27, wherein said modulated expression is effected by introducing and expressing in a plant a nucleic acid encoding a Class III U-box polypeptide.

Item 29. Method according to any preceding item 23 to 28, wherein said nucleic acid encoding a Class III U-box polypeptide encodes any one of the proteins listed in Table A2 or is a portion of such a nucleic acid, or a nucleic acid capable of hybridising with such a nucleic acid.

Item 30. Method according to any preceding item 23 to 29, wherein said nucleic acid sequence encodes an orthologue or paralogue of any of the proteins given in Table A2.

Item 31. Method according to any preceding item 23 to 30, wherein said enhanced yield-related traits comprise increased yield, preferably increased biomass and/or increased seed yield relative to control plants.

Item 32. Method according to any preceding item 23 to 31, wherein said enhanced yield-related traits are obtained under non-stress conditions and under mild drought conditions.

Item 33. Method according to any preceding item 23 to 32, wherein said enhanced yield-related traits are obtained under conditions of nitrogen deficiency.

Item 34. Method according to any preceding item 23 to 33, wherein said nucleic acid is operably linked to a young green tissue-specific promoter, preferably to a protochlorophyllid reductase promoter.

Item 35. Method according to any preceding item 23 to 34, wherein said nucleic acid encoding a Class III polypeptide is of plant origin, preferably from a dicotyledonous plant, further preferably from the family Solanaceae, more preferably from the genus Capsicum, most preferably from Capsicum annuus.

Item 36. Plant or part thereof, including seeds, obtainable by a method according to any preceding item 23 to 35, wherein said plant or part thereof comprises a recombinant nucleic acid encoding a Class III U-box polypeptide.

Item 37. Construct comprising:

(iii) nucleic acid encoding a Class III U-box polypeptide as defined in item 23 to 27;

(iv) one or more control sequences capable of driving expression of the nucleic acid sequence of (a); and optionally

(v) a transcription termination sequence.

Item 38. Construct according to item 37, wherein one of said control sequences is a yound green tissue specific promoter, preferably a protochlorophyllid reductase promoter.

Item 39. Use of a construct according to item 37 or 38 in a method for making plants having increased yield, particularly increased biomass and/or increased seed yield relative to control plants.

Item 40. Plant, plant part or plant cell transformed with a construct according to item 37 or 38.

Item 41. Method for the production of a transgenic plant having increased yield, particularly increased biomass and/or increased seed yield relative to control plants, comprising:

(d) introducing and expressing in a plant a nucleic acid encoding a Class III U-box polypeptide as defined in any one of item 23 to 27; and

(e) cultivating the plant cell under conditions promoting plant growth and development.

Item 42. Transgenic plant having increased yield, particularly increased biomass and/or increased seed yield, relative to control plants, resulting from increased expression of a nucleic acid encoding a Class III U-box polypeptide as defined in any one of item 23 to 27, or a transgenic plant cell derived from said transgenic plant.

Item 43. Transgenic plant according to item 36, 40 or42, or a transgenic plant cell derived thereof, wherein said plant is a crop plant or a monocot or a cereal, such as rice, maize, wheat, barley, millet, rye, triticale, sorghum and oats.

Item 44. Harvestable parts of a plant according to item 43, wherein said harvestable parts are preferably shoot biomass and/or seeds.

Item 45. Products derived from a plant according to item 43 and/or from harvestable parts of a plant according to item 44.

Item 46. Use of a nucleic acid encoding a Class III U-box polypeptide in increasing yield, particularly in increasing seed yield and/or shoot biomass in plants, relative to control plants.

In one embodiment the invention relates to subject mater summarized as follows:

Item 47 A method for enhancing yield-related traits in plants relative to control plants, comprising modulating expression in a plant of a nucleic acid encoding a PQQC protein.

Item 48. Method according to item 47, wherein said PQQC protein has, in increasing order of preference, at least 45%, 50%, 55%, 60%, 65%, 70%, 75%, 80%, 85%, 90%, 95%, 96%, 97%, 98%, 99% or more sequence identity to the sequence represented by SEQ ID NO: 105.

Item 49. Method according to any preceding item 47 to 48, wherein said modulated expression is effected by introducing and expressing in a plant a nucleic acid encoding a PQQC polypeptide.

Item 50. Method according to any preceding item 47 to 49, wherein said nucleic acid encoding a PQQC polypeptide encodes any one of the proteins listed in Table A3 or is a portion of such a nucleic acid, or a nucleic acid capable of hybridising with such a nucleic acid.

Item 51. Method according to any preceding item 47 to 50, wherein said enhanced yield-related traits comprise increased yield, preferably increased biomass and/or increased seed yield relative to control plants.

Item 52. Method according to any preceding item 47 to 51, wherein said enhanced yield-related traits are obtained under non-stress conditions and under mild drought conditions.

Item 53. Method according to any preceding item 47 to 52, wherein said enhanced yield-related traits are obtained under conditions of nitrogen deficiency.

Item 54. Method according to any preceding item 47 to 53, wherein said nucleic acid is operably linked to a young green tissue-specific promoter, preferably to a protochlorophyllid reductase promoter.

Item 55. Method according to any preceding item 47 to 54, wherein said nucleic acid is operably linked to a constitutive promoter, preferably to a GOS2 promoter, most preferably to a GOS2 promoter from rice.

Item 56. Method according to any preceding item 47 to 55, wherein said nucleic acid encoding a PQQC polypeptide is of bacterial origin, preferably from pseudomonas. Item 57. Plant or part thereof, including seeds, obtainable by a method according to any preceding claim, wherein said plant or part thereof comprises a recombinant nucleic acid encoding a PQQC polypeptide.

Item 58. Construct comprising:

- nucleic acid encoding a PQQC polypeptide as defined in item 47 to 48;
- one or more control sequences capable of driving expression of the nucleic acid sequence of (a); and optionally
- a transcription termination sequence.

Item 59. Construct according to item 58, wherein one of said control sequences is a young green tissue specific promoter, preferably a protochlorophyllid reductase promoter.

Item 60. Construct according to item 58, wherein one of said control sequences is a constitutive promoter, preferably a GOS2 promoter, most a GOS2 promoter from rice.

Item 61. Use of a construct according to any one of item 58 to 60in a method for making plants having increased yield, particularly increased biomass and/or increased seed yield relative to control plants.

Item 62. Plant, plant part or plant cell transformed with a construct according to any one of item 58 to 61.

Item 63. Method for the production of a transgenic plant having increased yield, particularly increased biomass and/or increased seed yield relative to control plants, comprising:

(i) introducing and expressing in a plant a nucleic acid encoding a PQQC polypeptide as defined in item 47 to 48; and

(ii) cultivating the plant cell under conditions promoting plant growth and development.

Item 64. Transgenic plant having increased yield, particularly increased biomass and/or increased seed yield, relative to control plants, resulting from increased expression of a nucleic acid encoding a PQQC polypeptide as defined in item 47 to 48, or a transgenic plant cell derived from said transgenic plant.

Item 65. Transgenic plant according to item 57, 62 or64, or a transgenic plant cell derived thereof, wherein said plant is a crop plant or a monocot or a cereal, such as rice, maize, wheat, barley, millet, rye, triticale, sorghum and oats.

Item 66. Harvestable parts of a plant according to item 65, wherein said harvestable parts are preferably shoot biomass and/or seeds.

Item 67. Products derived from a plant according to item 65 and/or from harvestable parts of a plant according to item 66.

Item 68. Use of a nucleic acid encoding a PQQC polypeptide in increasing yield, particularly in increasing seed yield and/or shoot biomass in plants, relative to control plants.

Description of figures

The present invention will now be described with reference to the following figures in which:

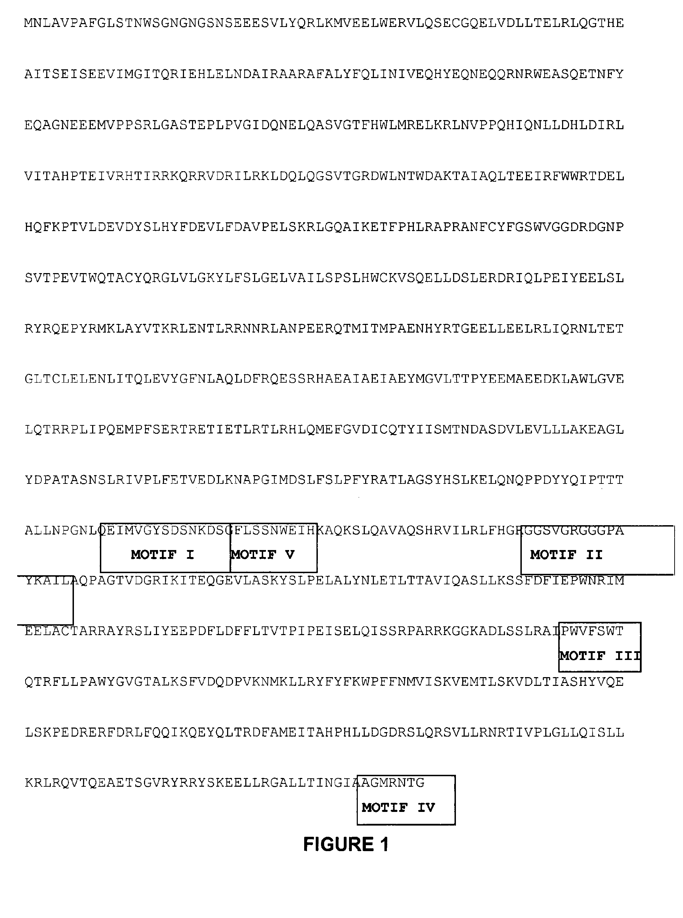

Fig. 1 shows Synechocystis PEPC protein with Motifs I to V highlighted.

Fig. 2 shows a multiple alignment of a selection of PEPC proteins.

Fig. 3 represents the expression vector for increased expression in Oryza sativa of a PEPC-encoding nucleic acid under the control of a protochlorophyllid reductase promoter.

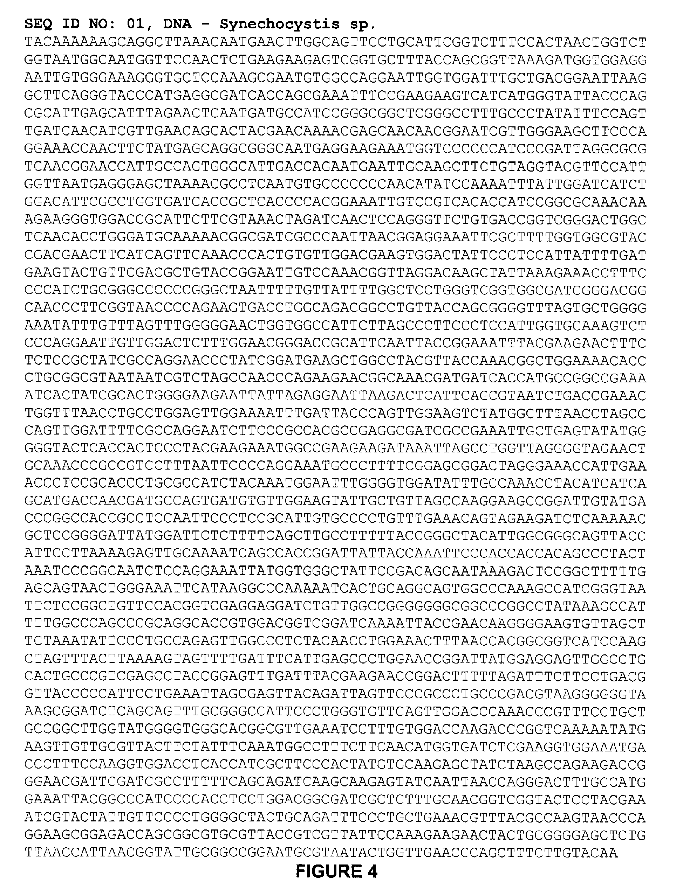

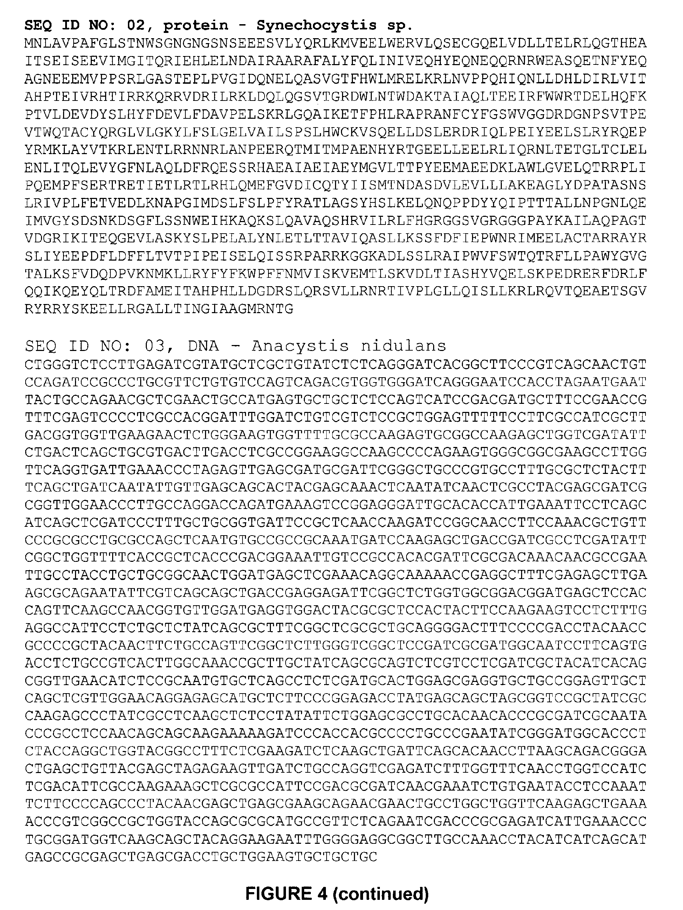

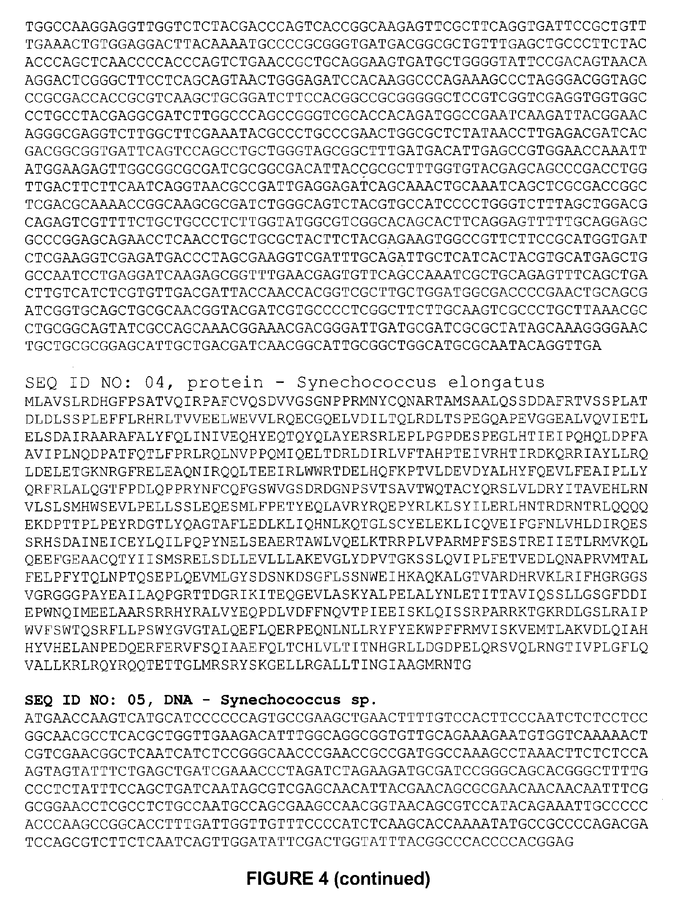

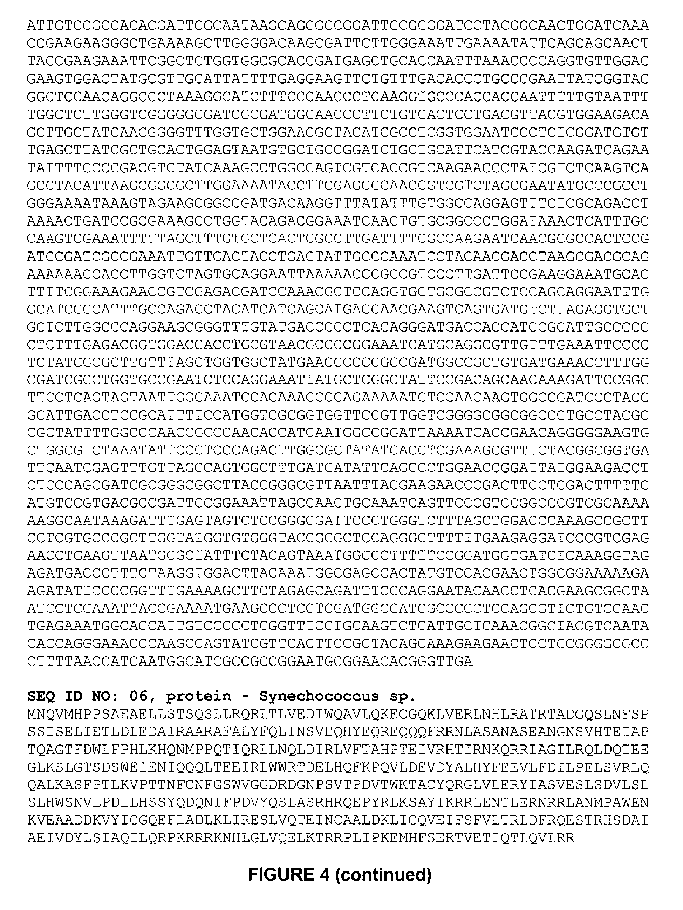

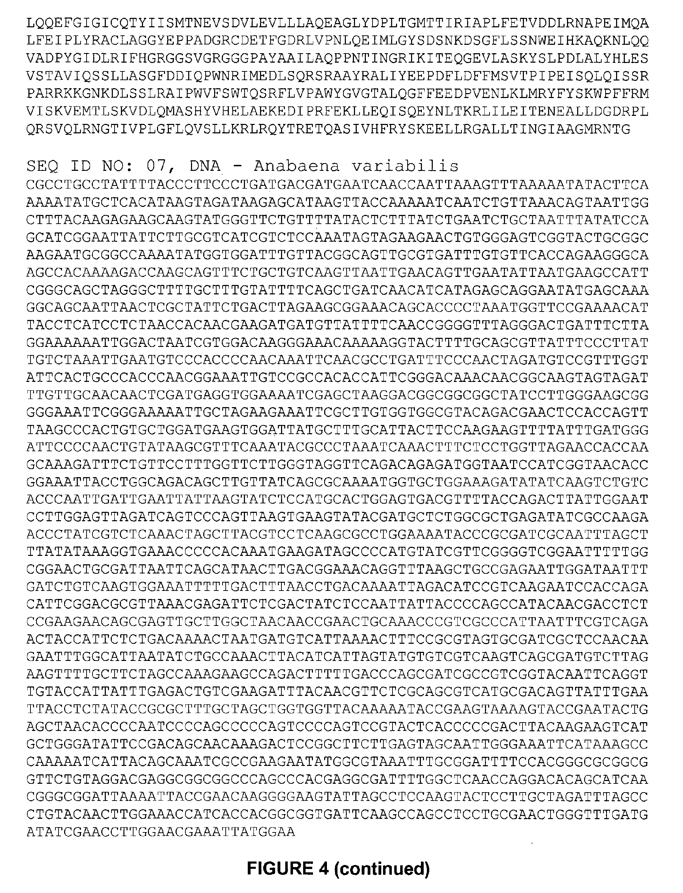

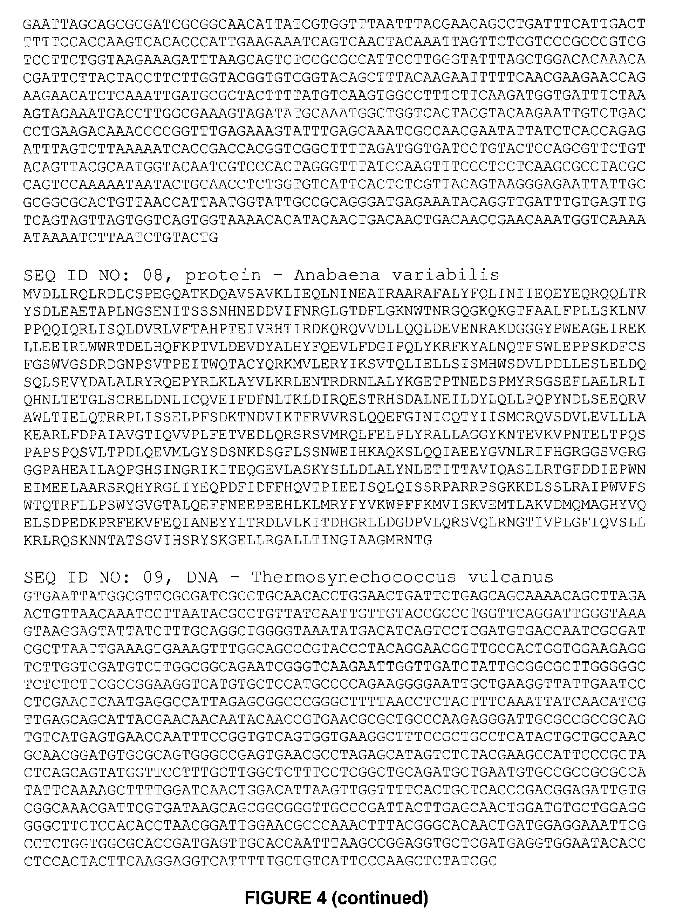

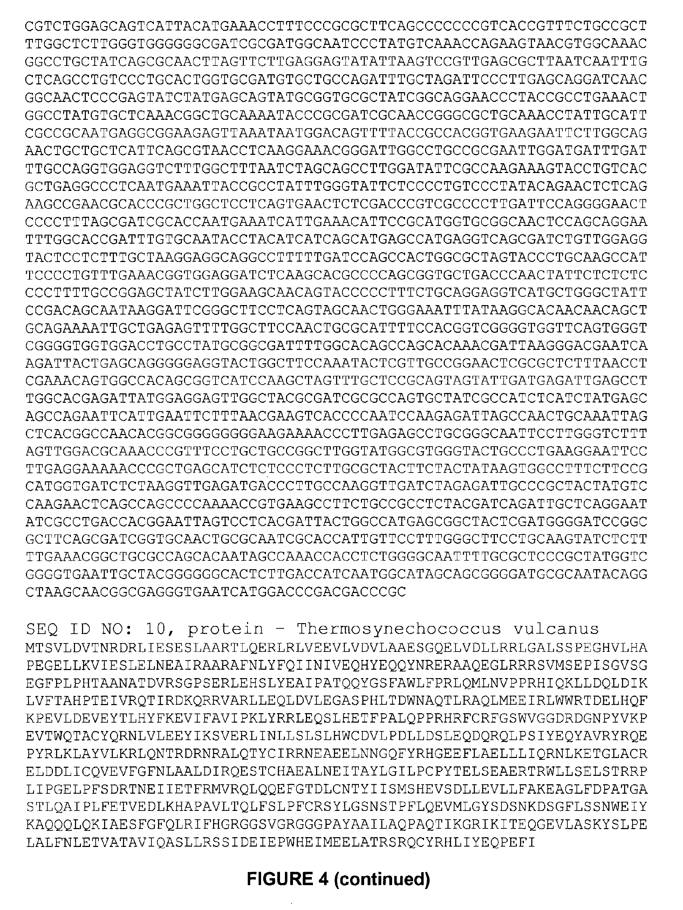

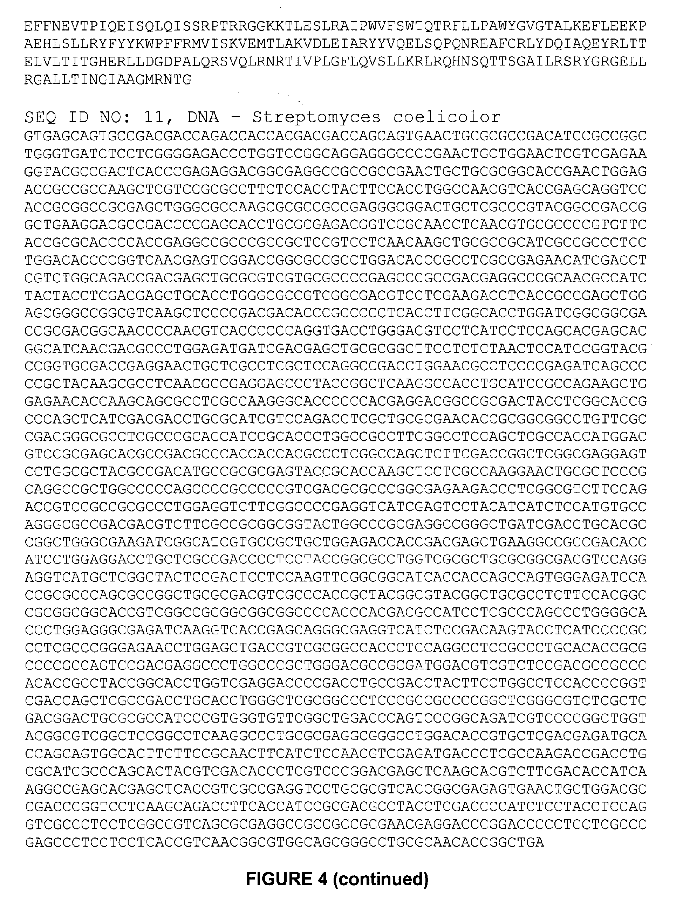

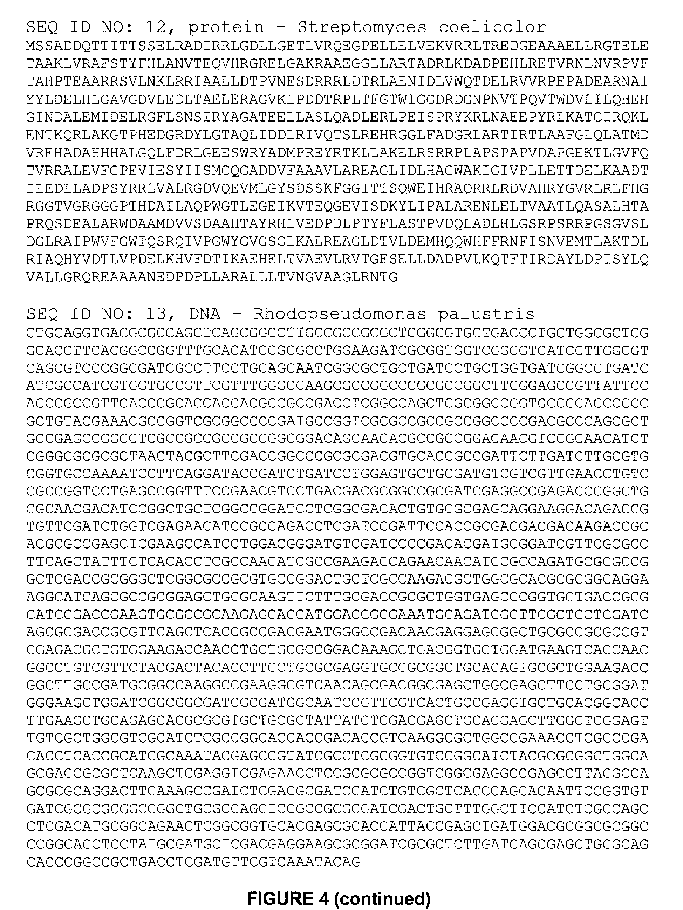

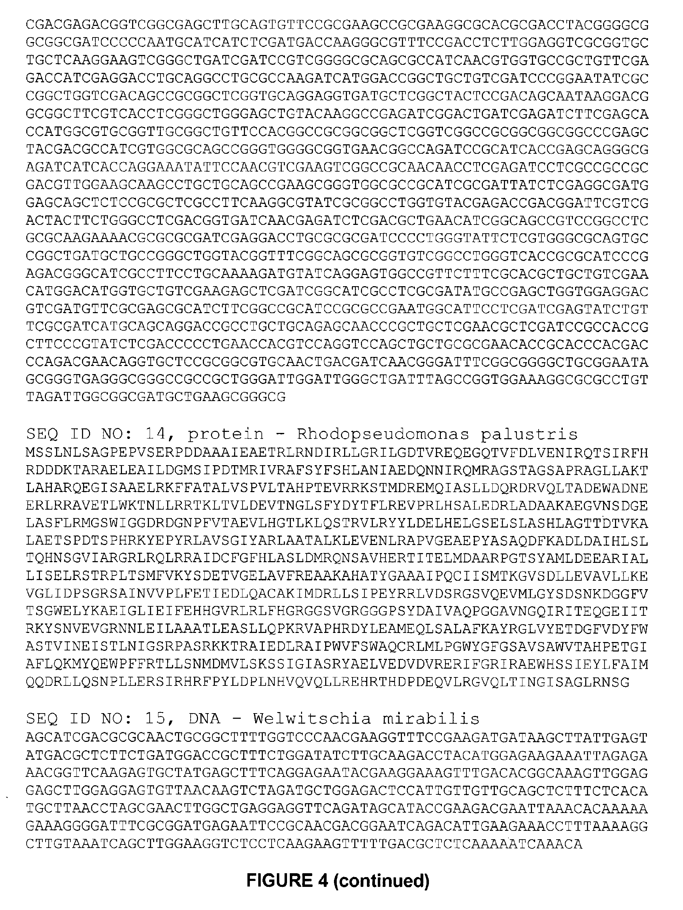

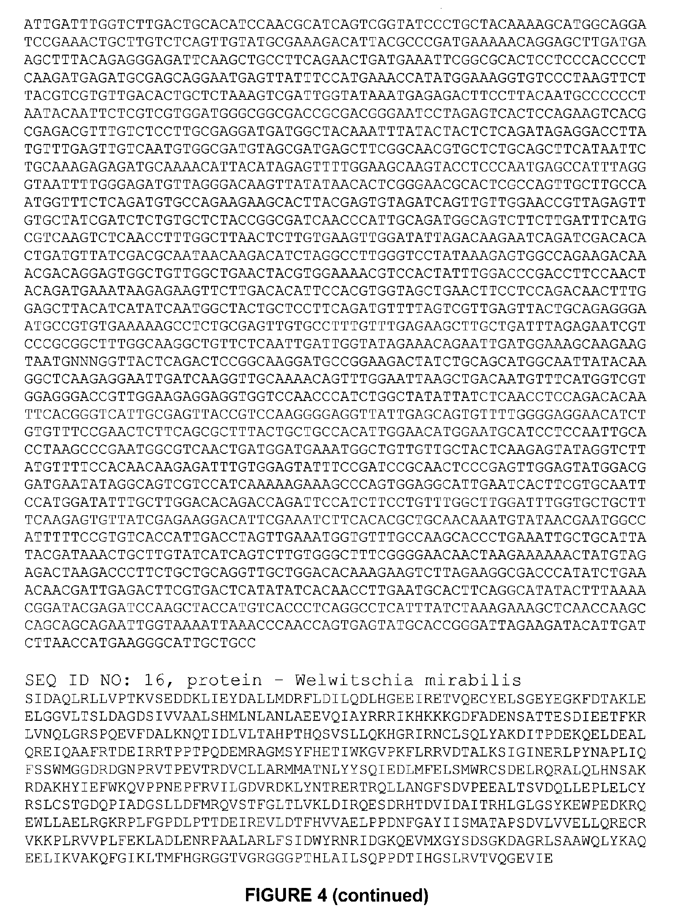

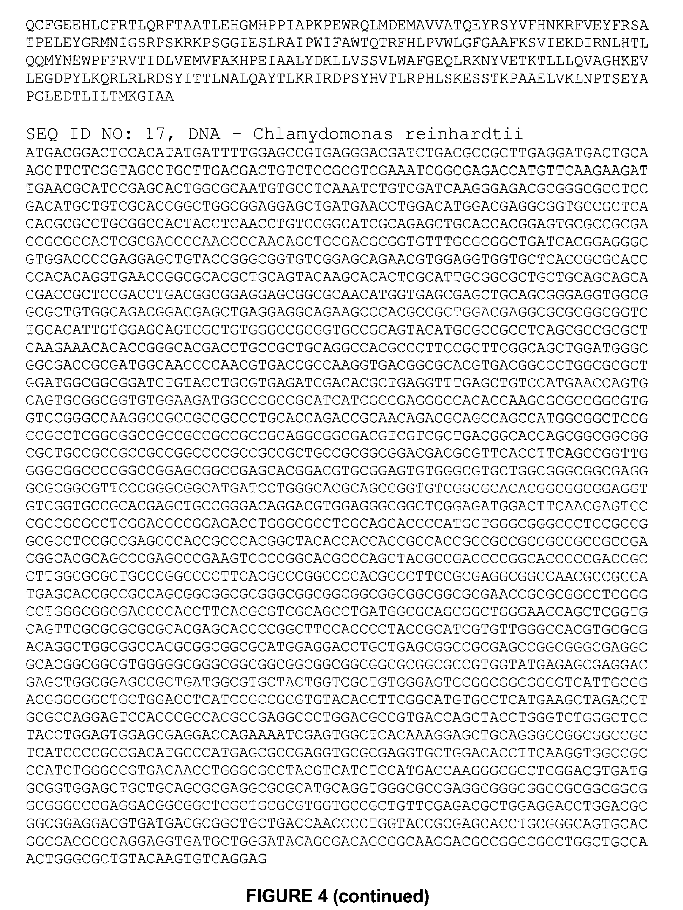

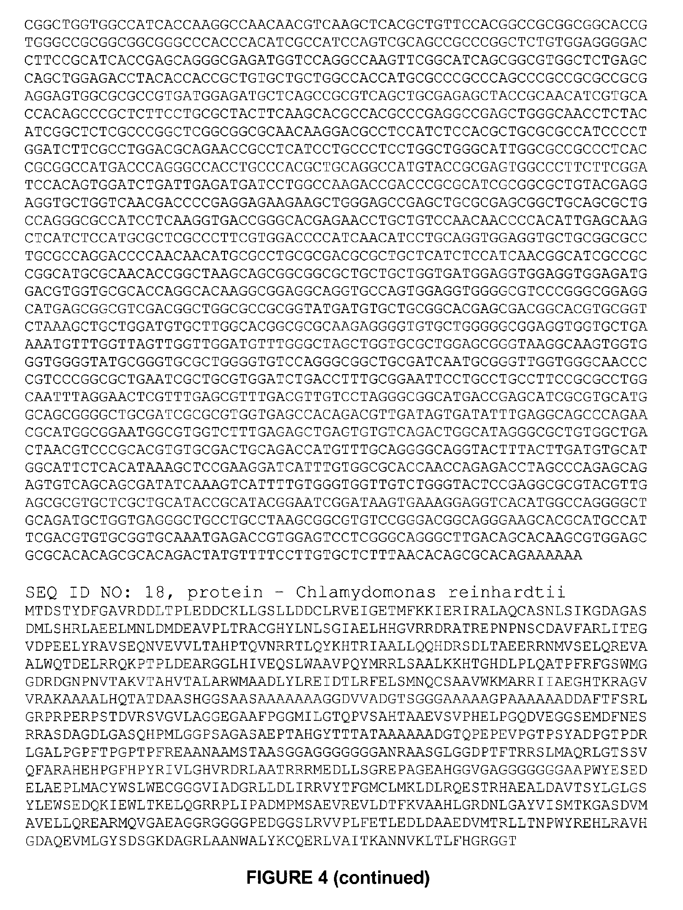

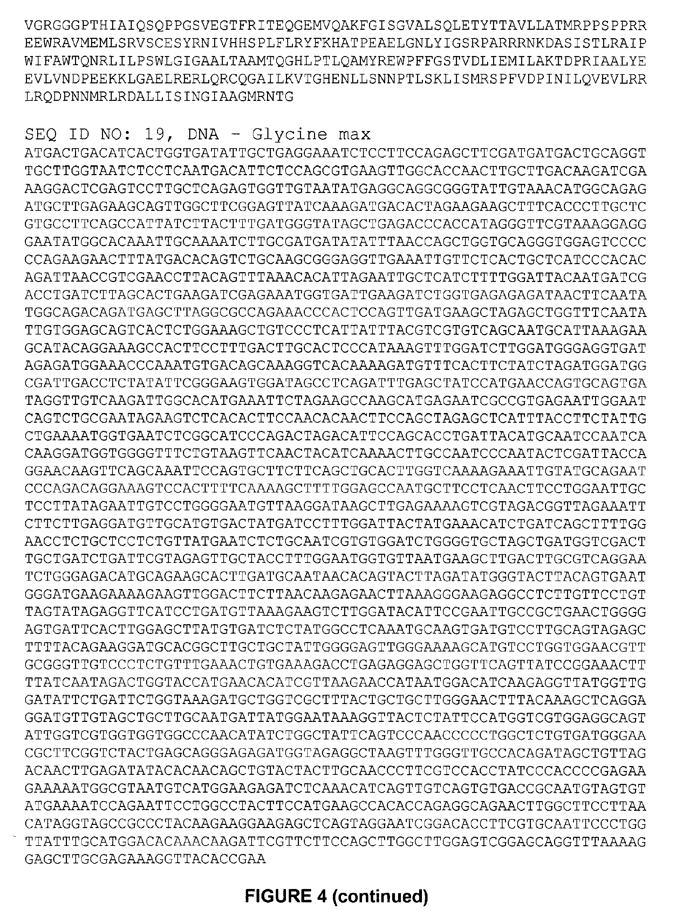

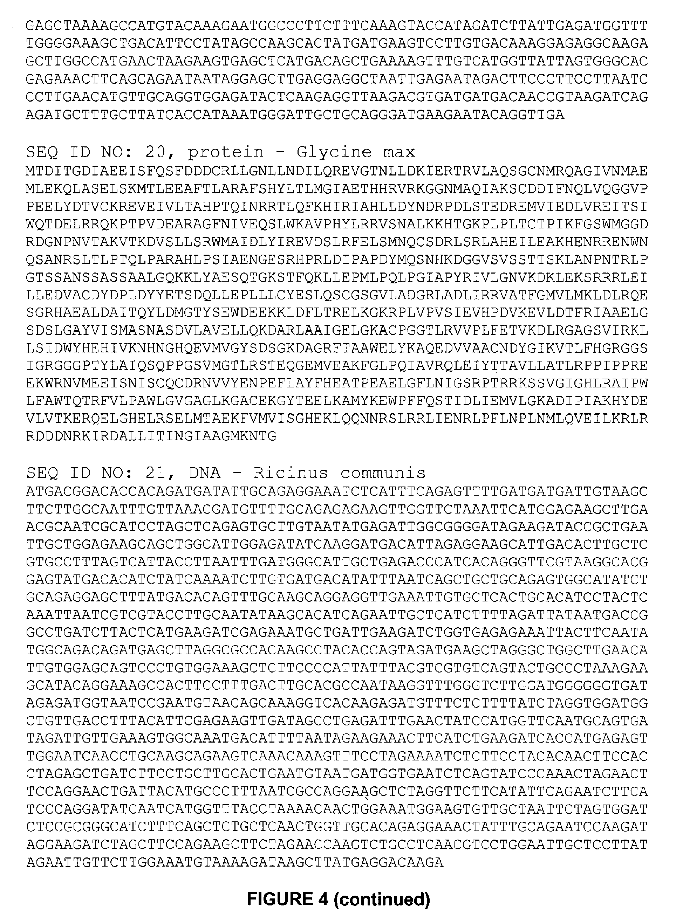

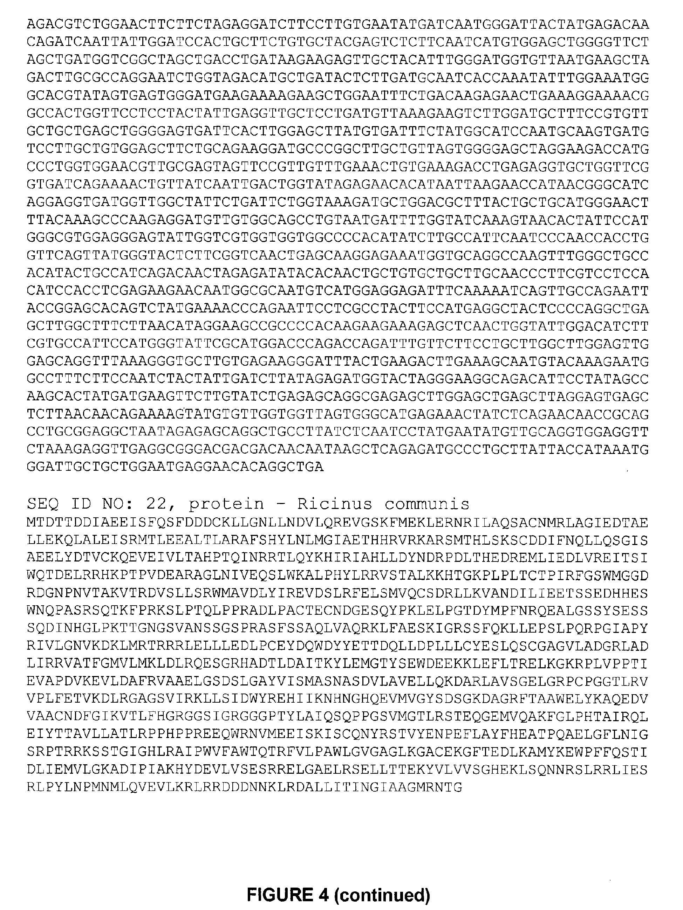

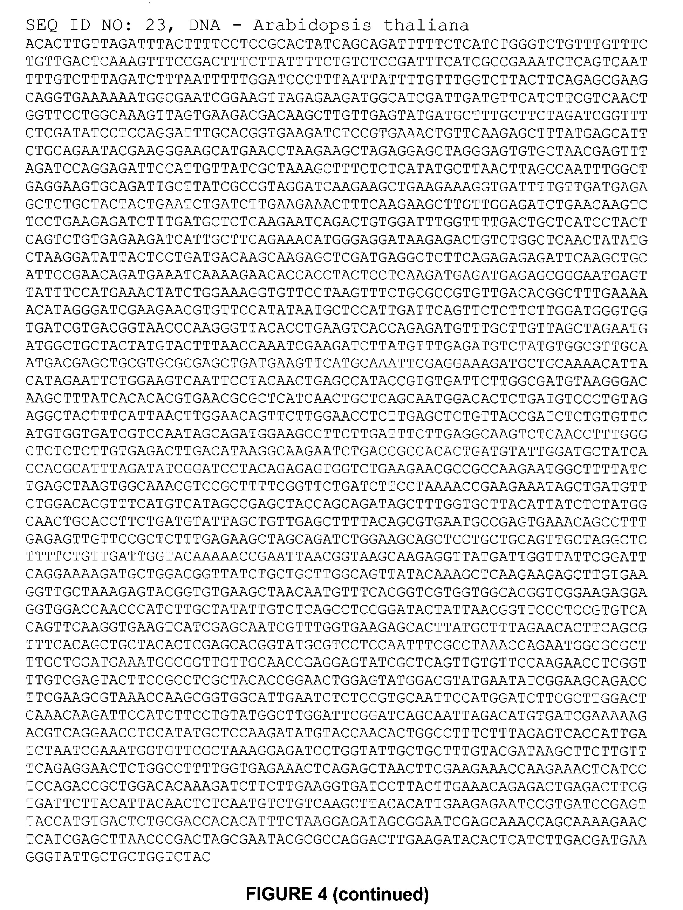

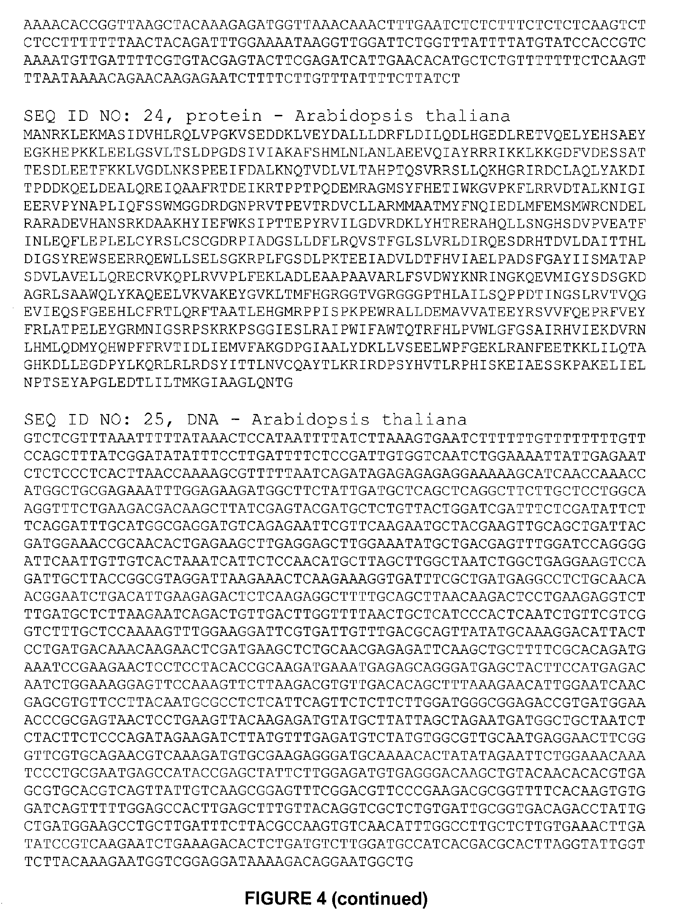

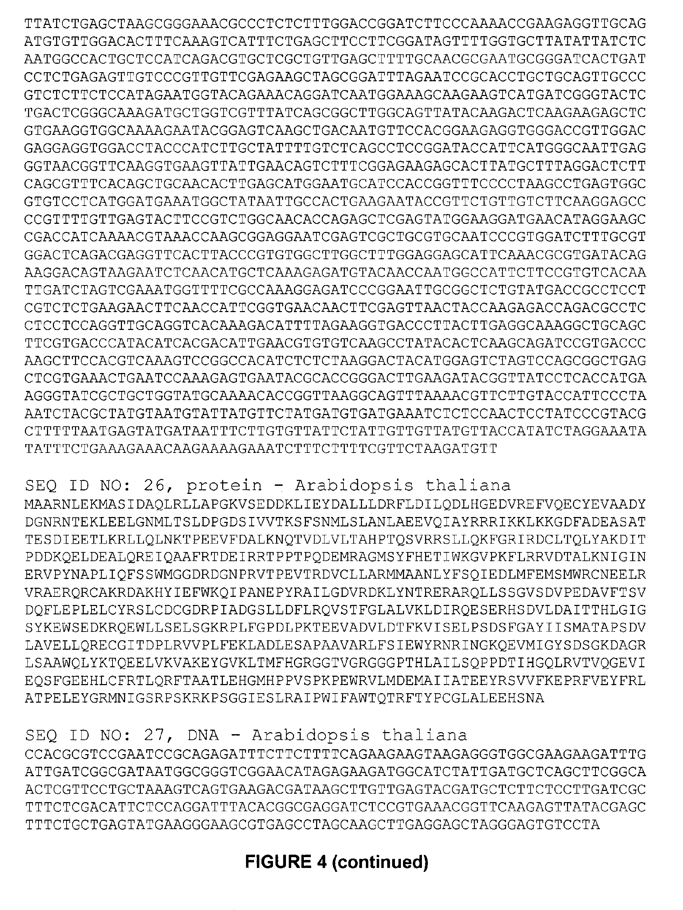

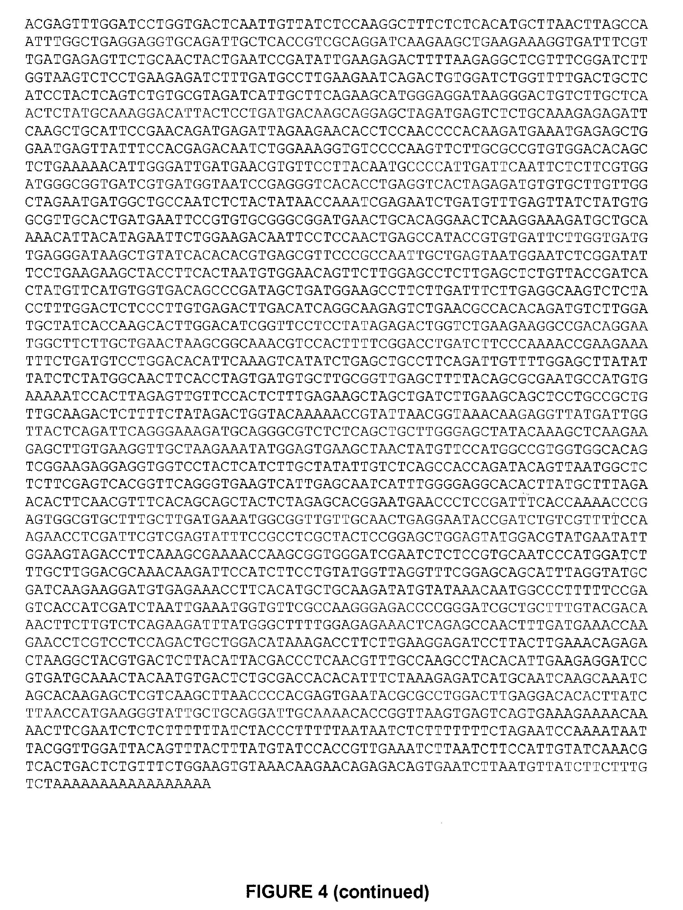

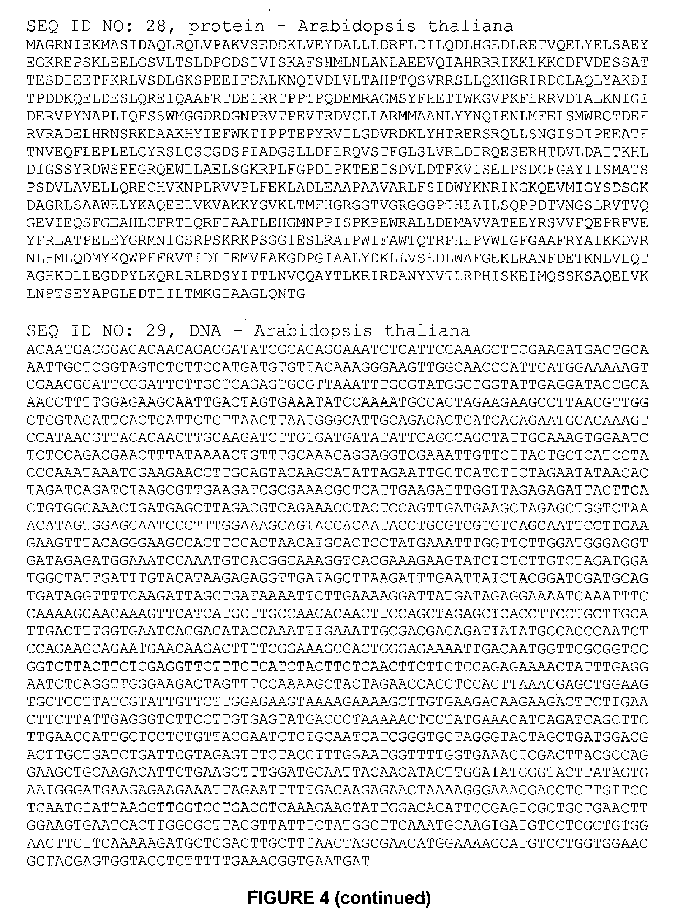

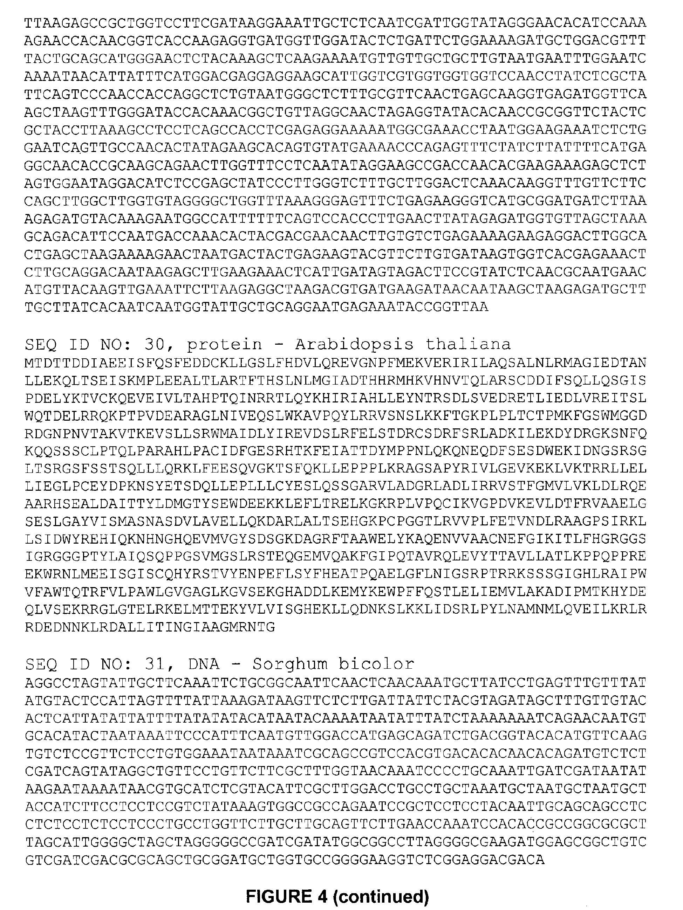

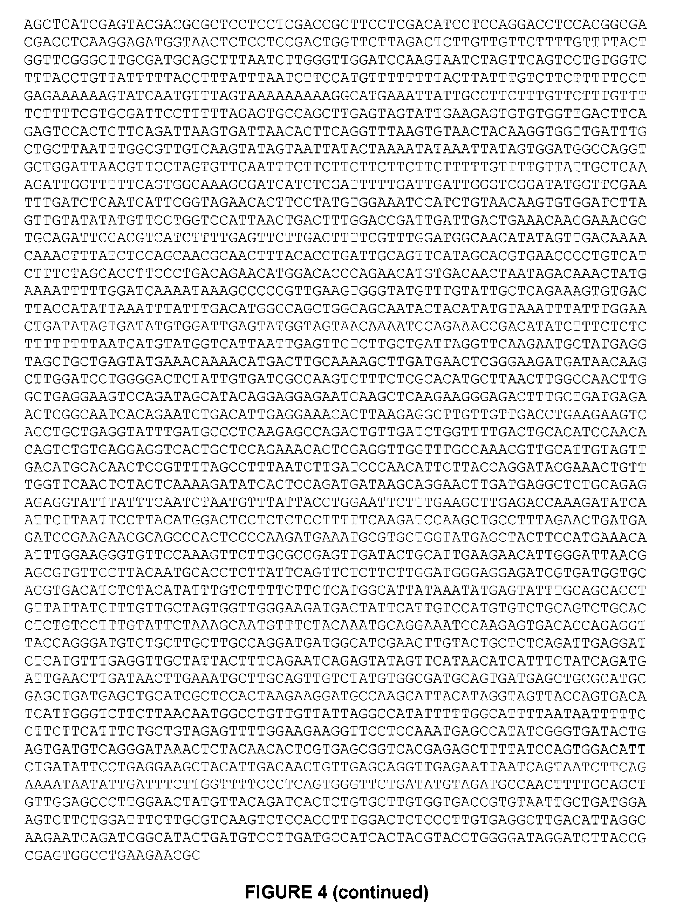

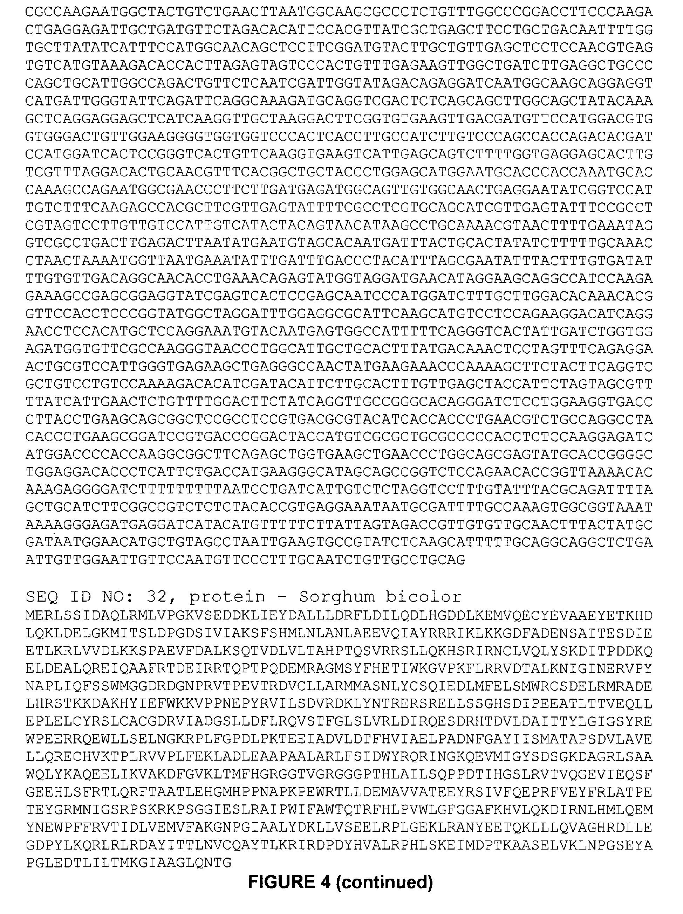

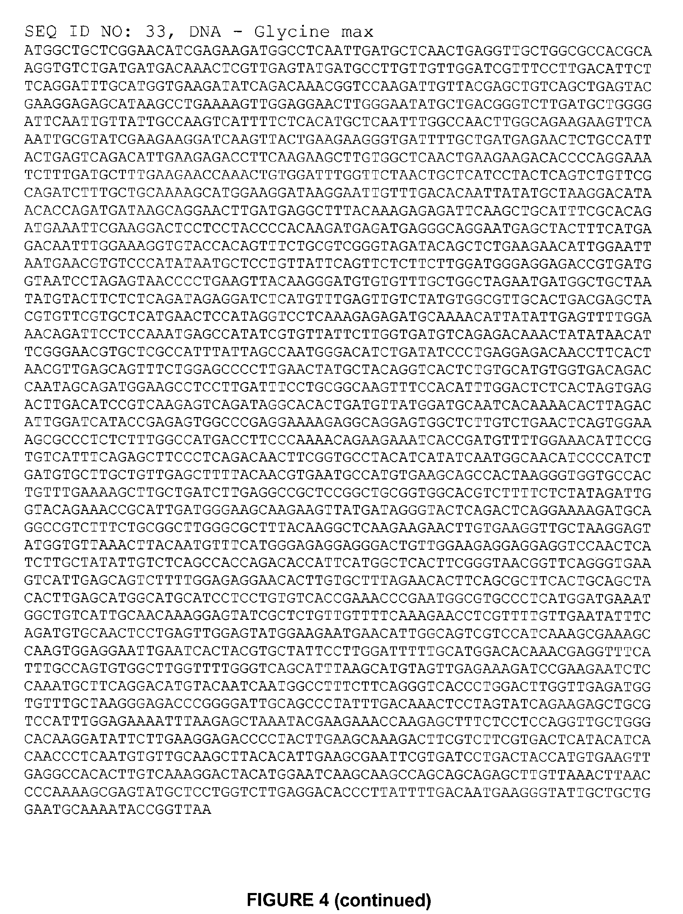

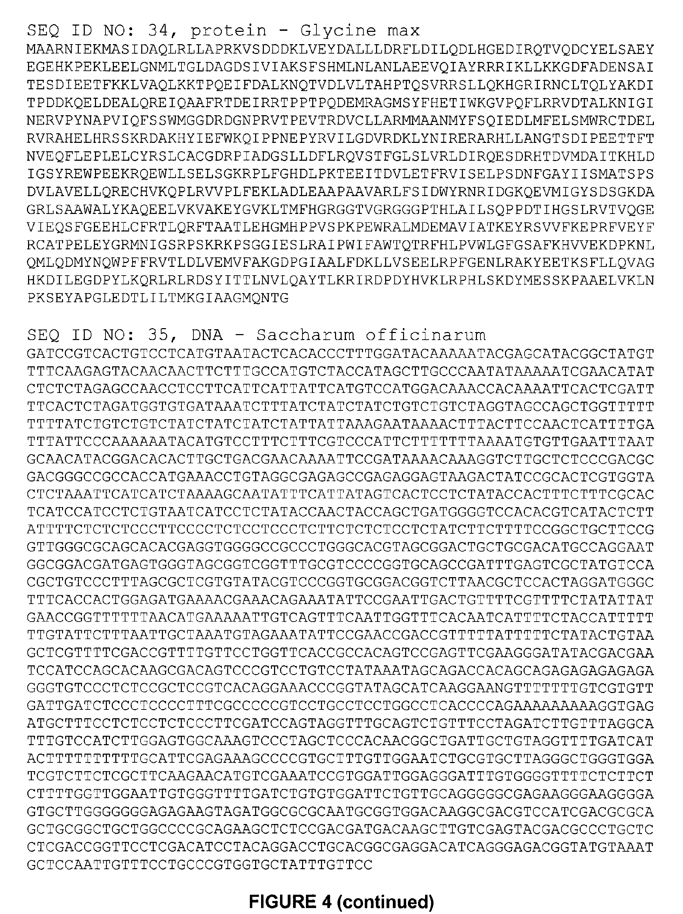

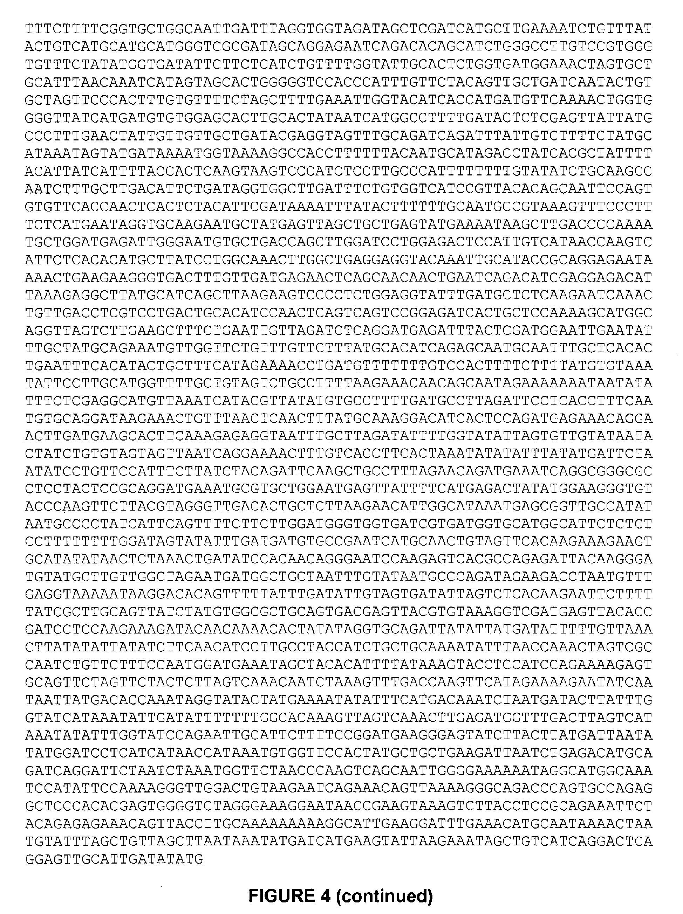

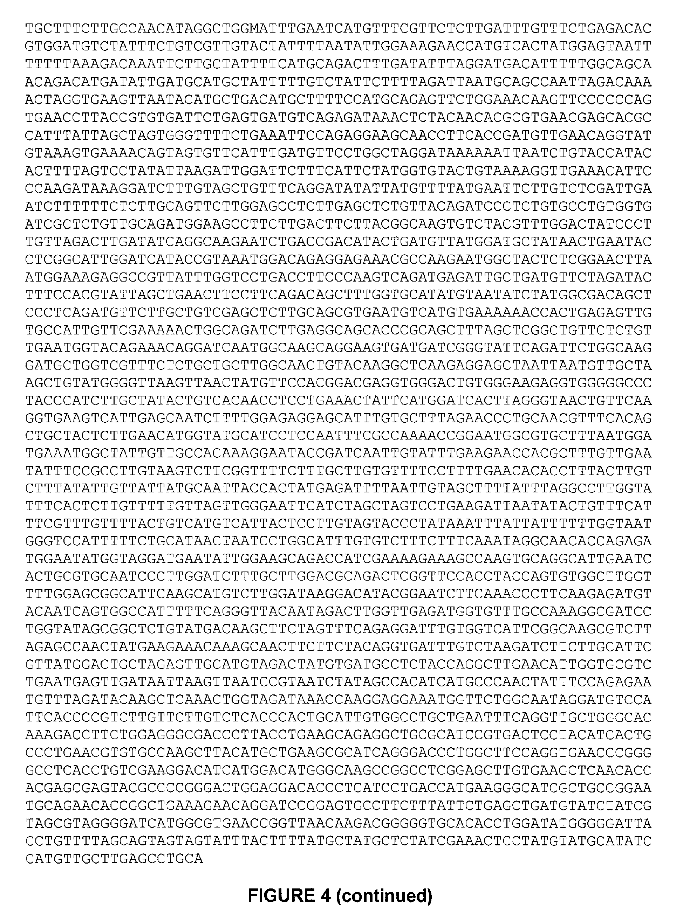

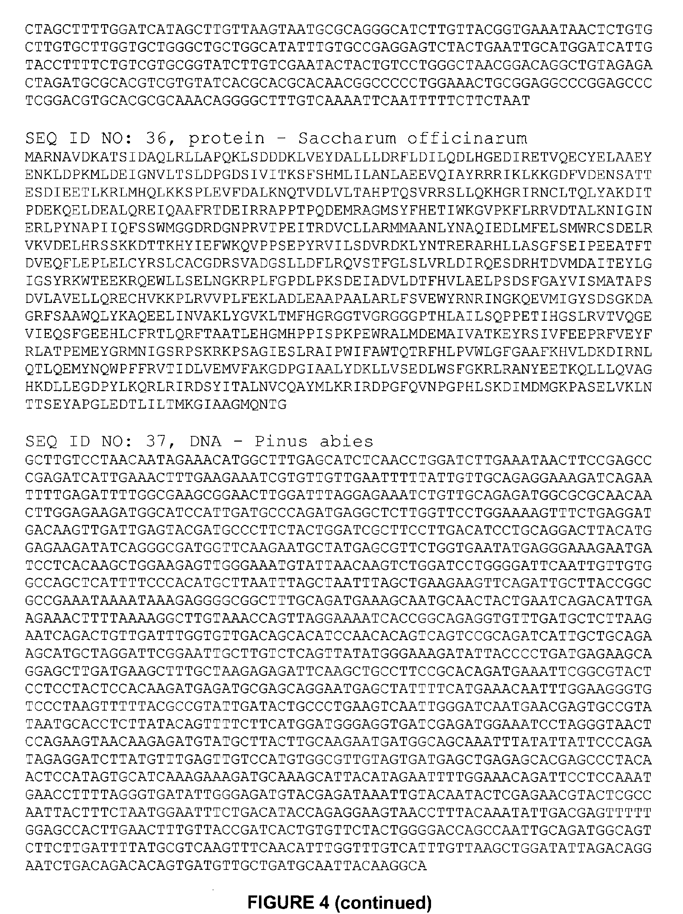

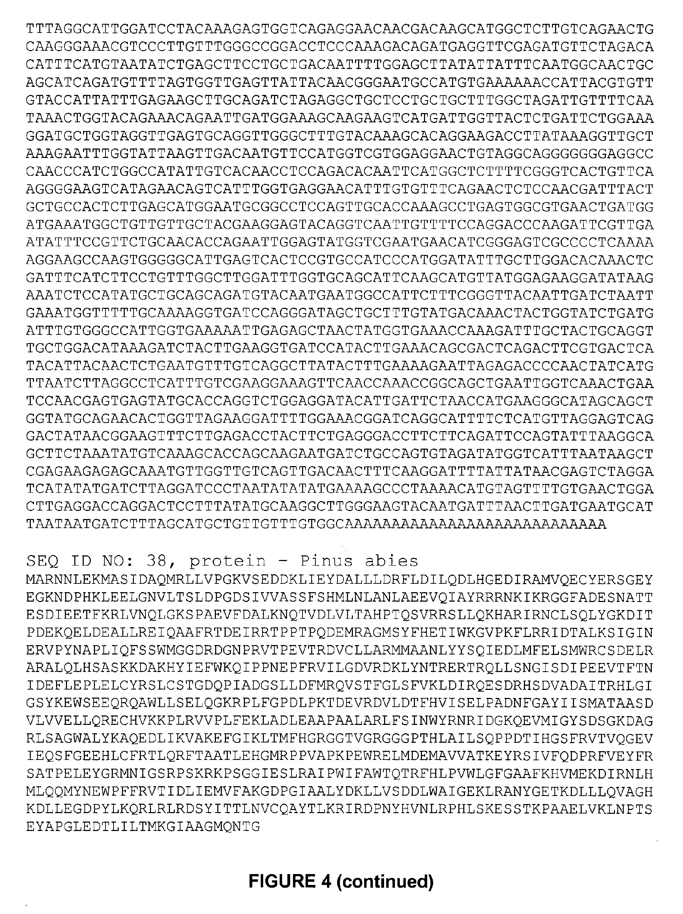

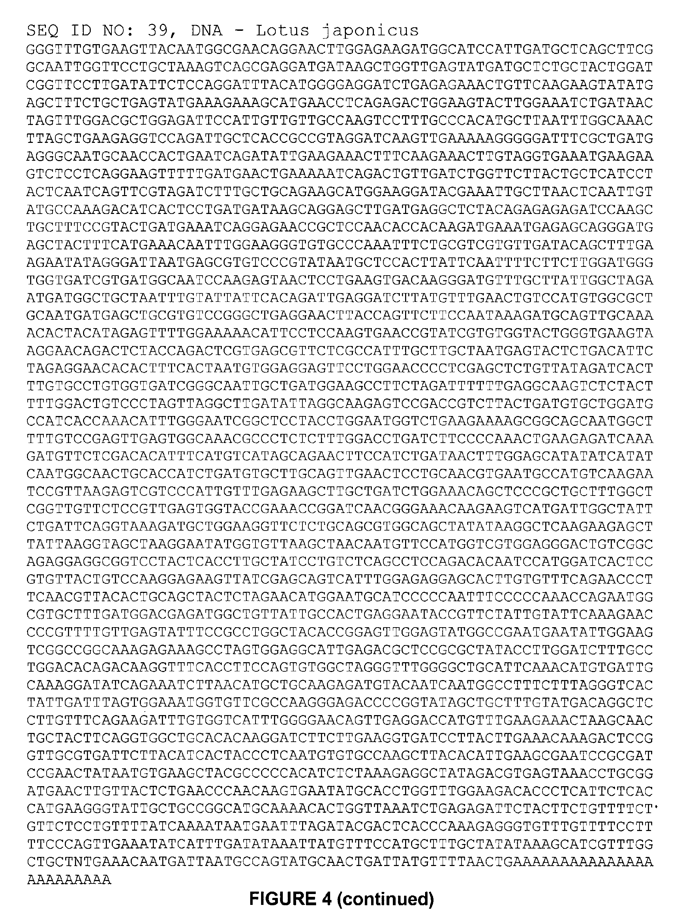

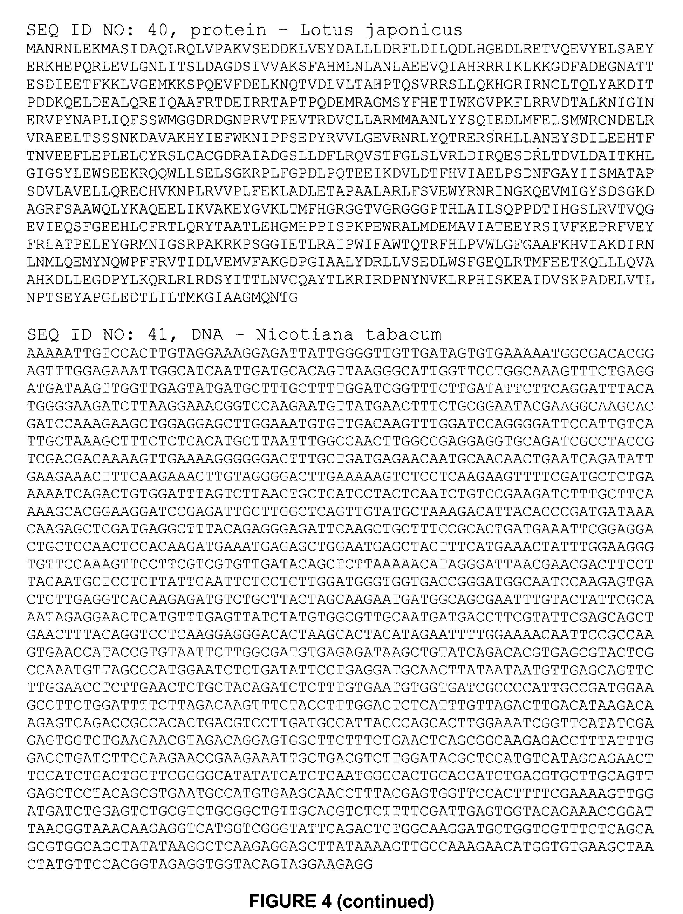

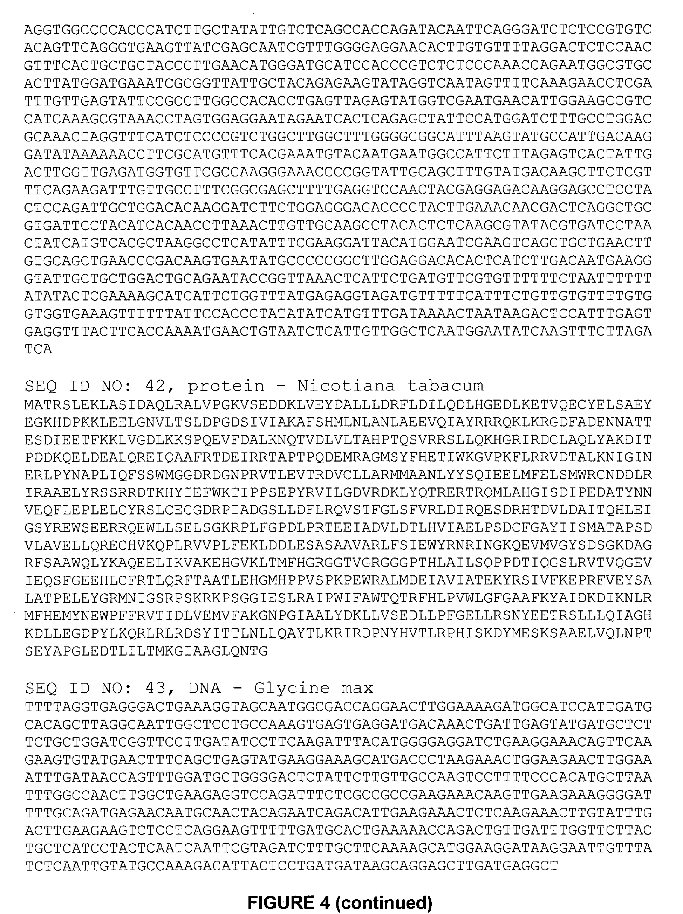

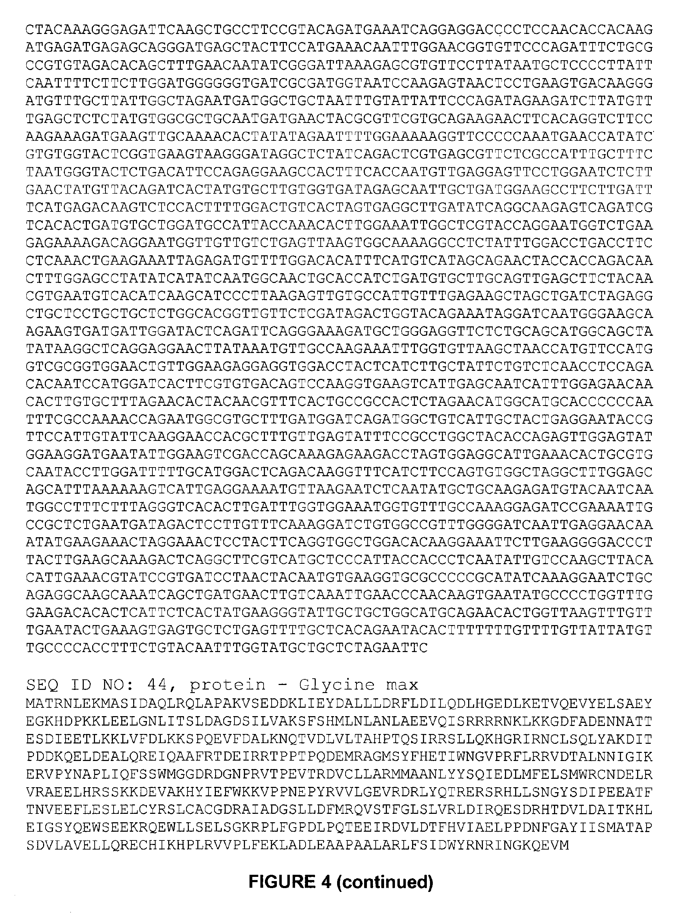

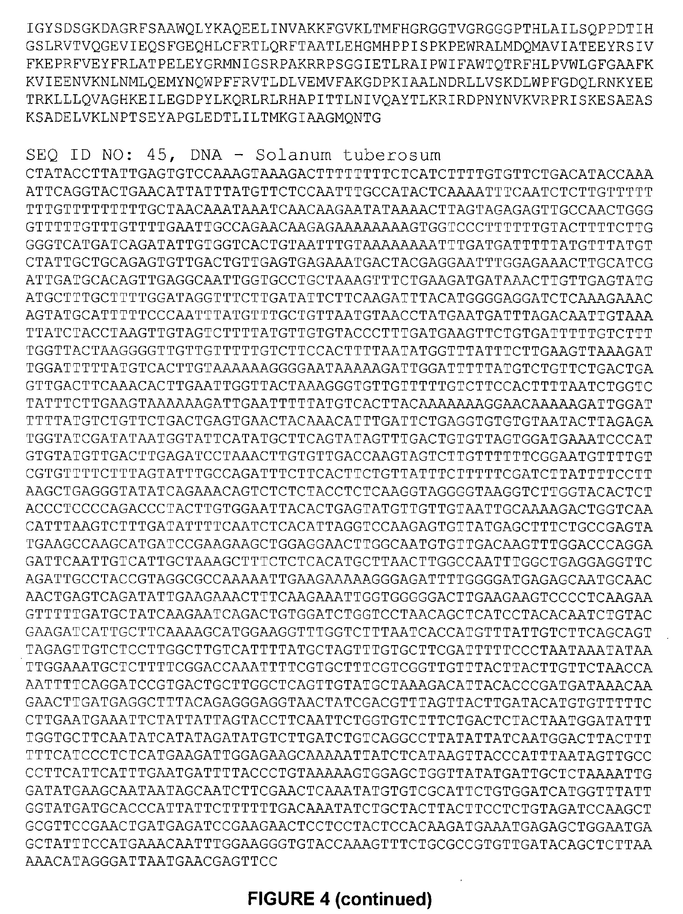

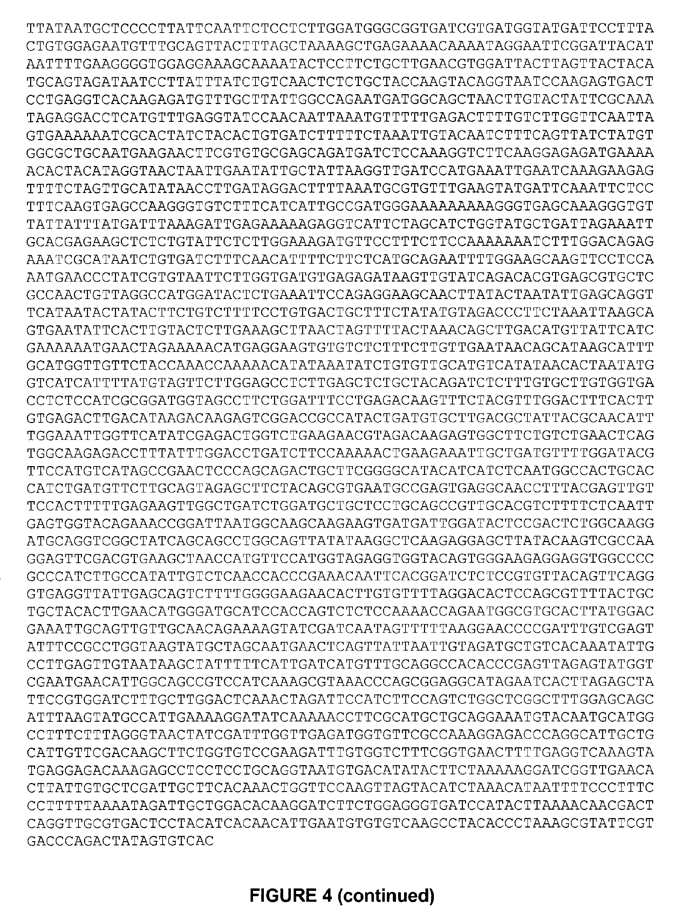

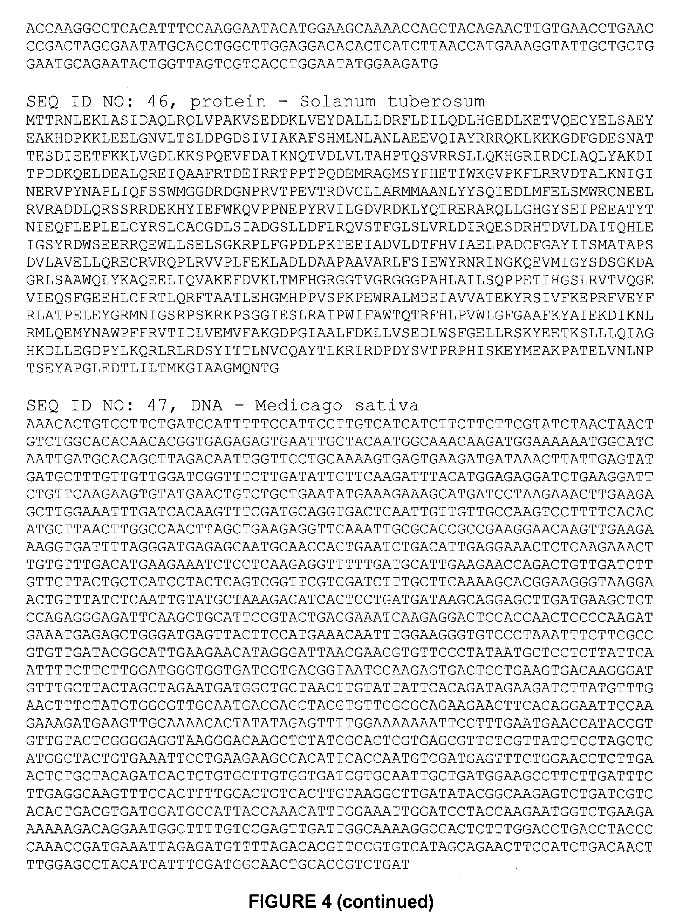

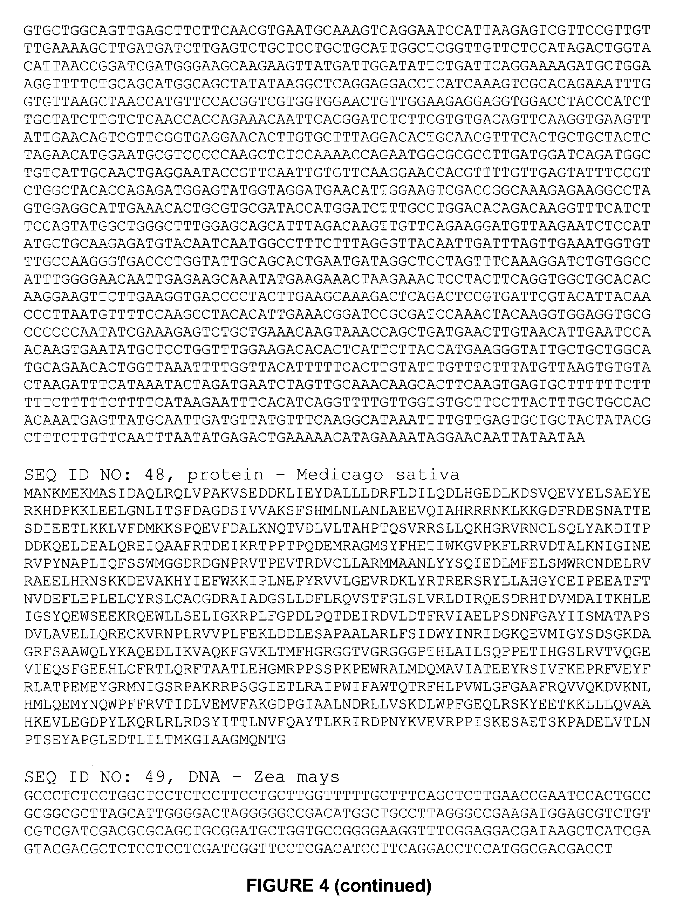

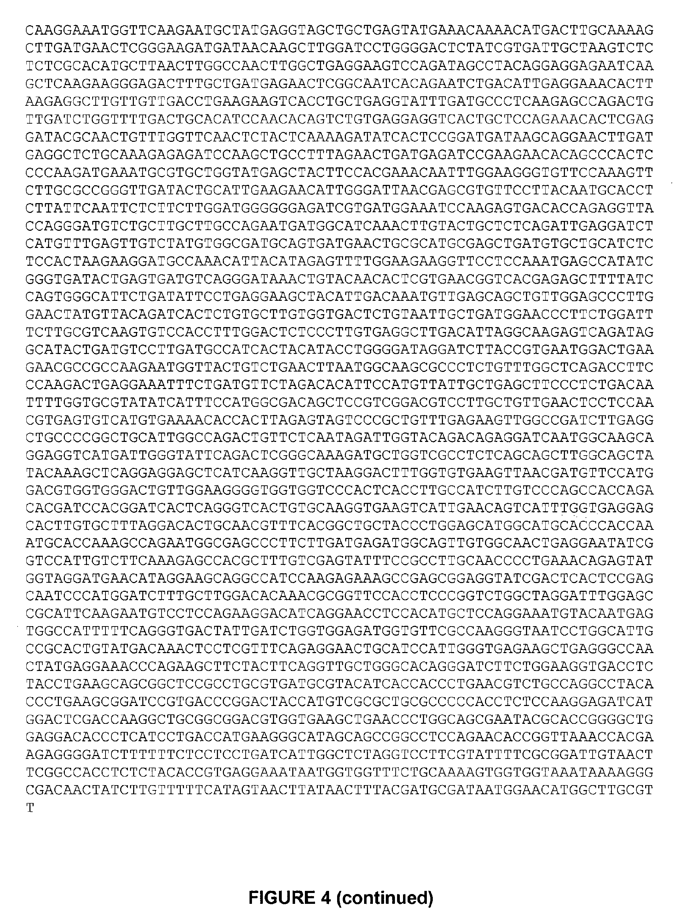

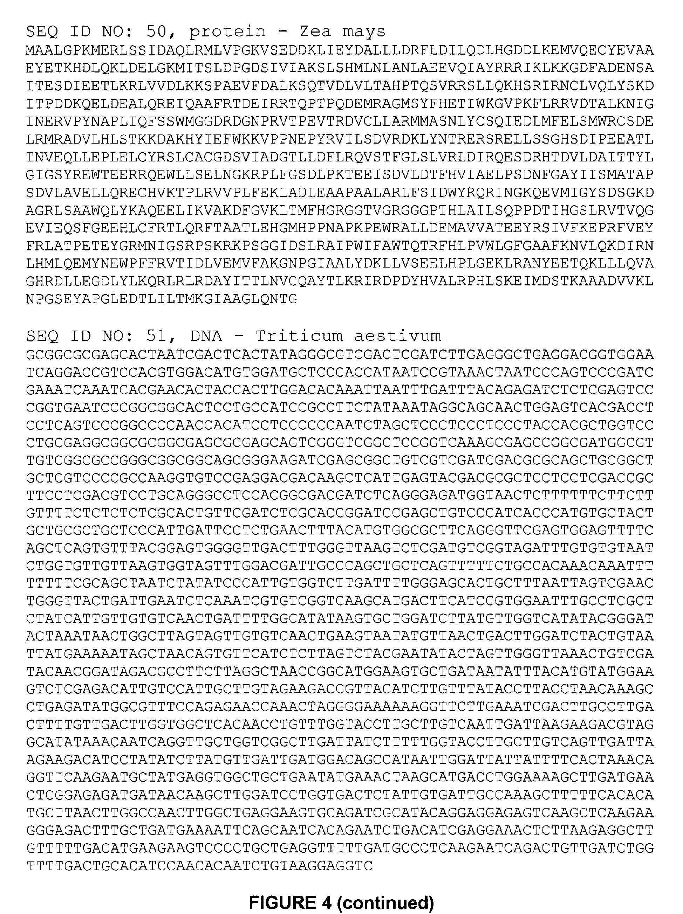

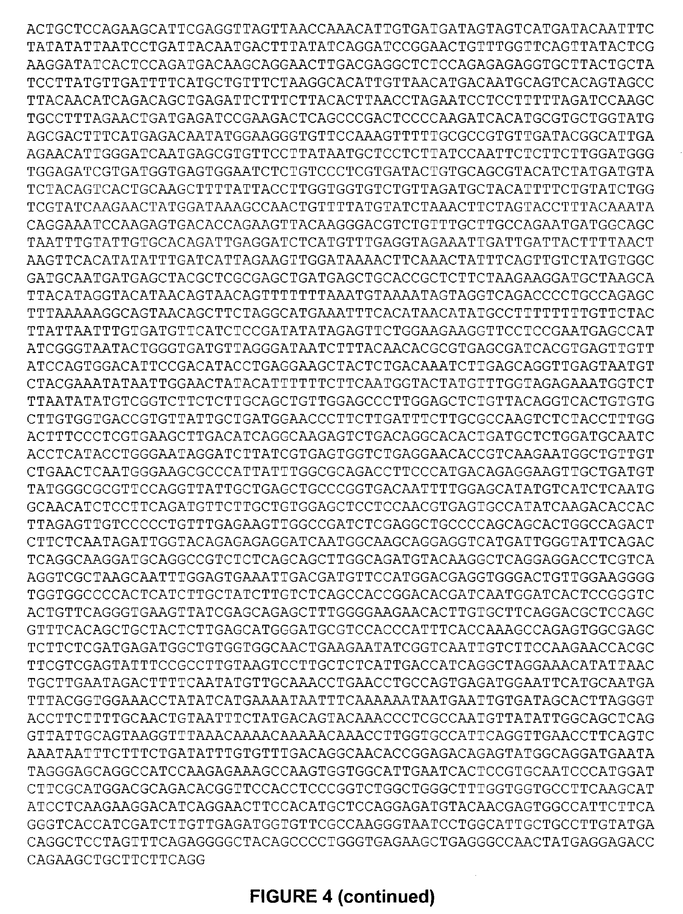

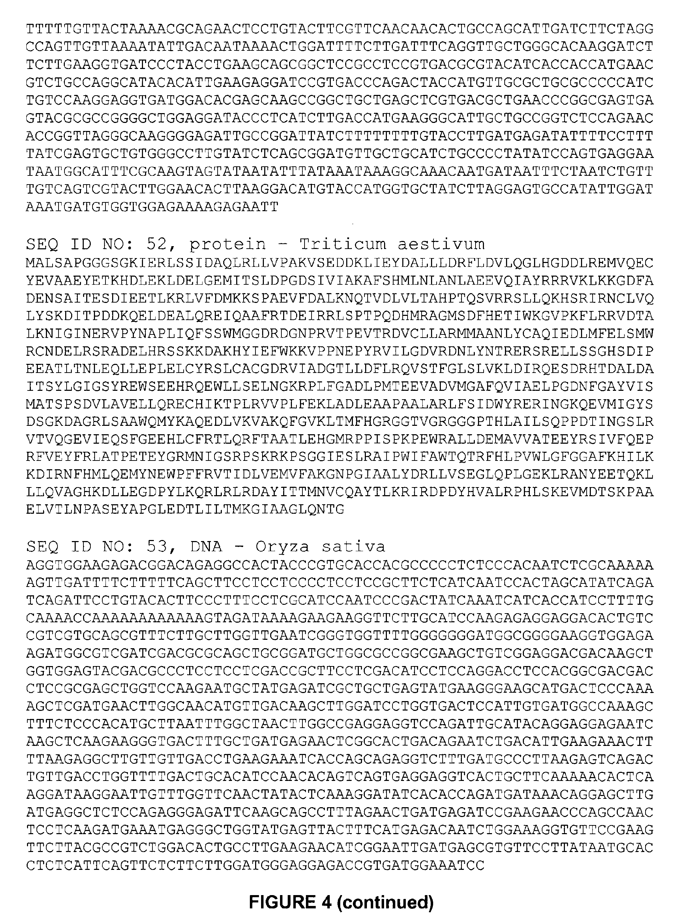

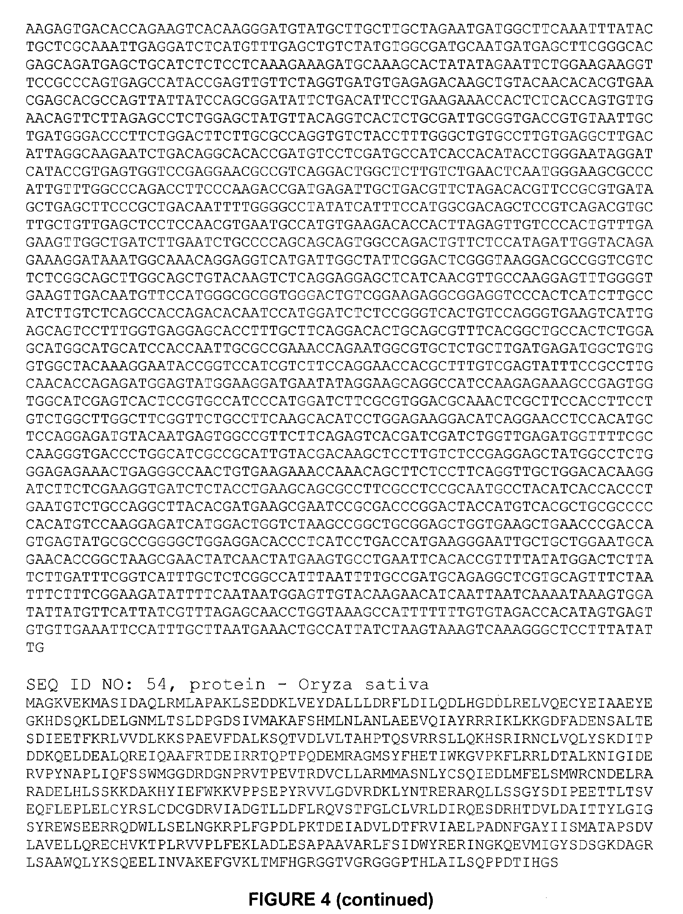

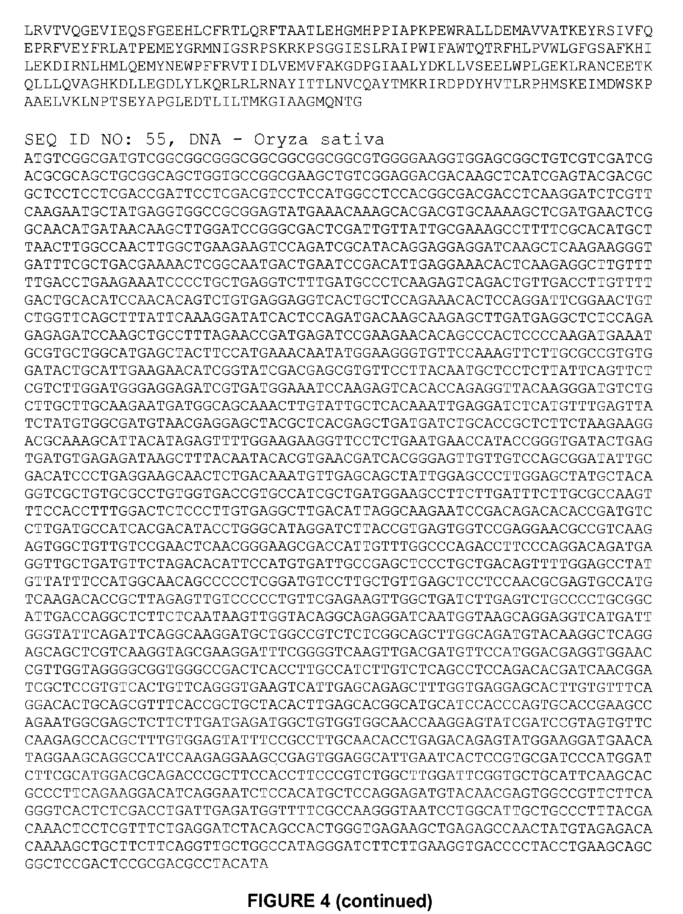

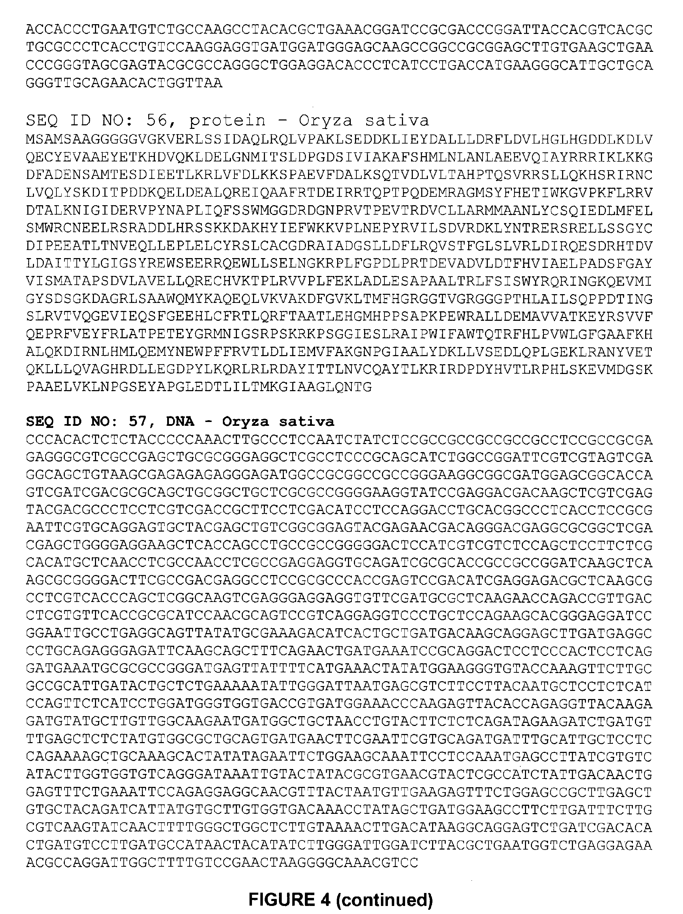

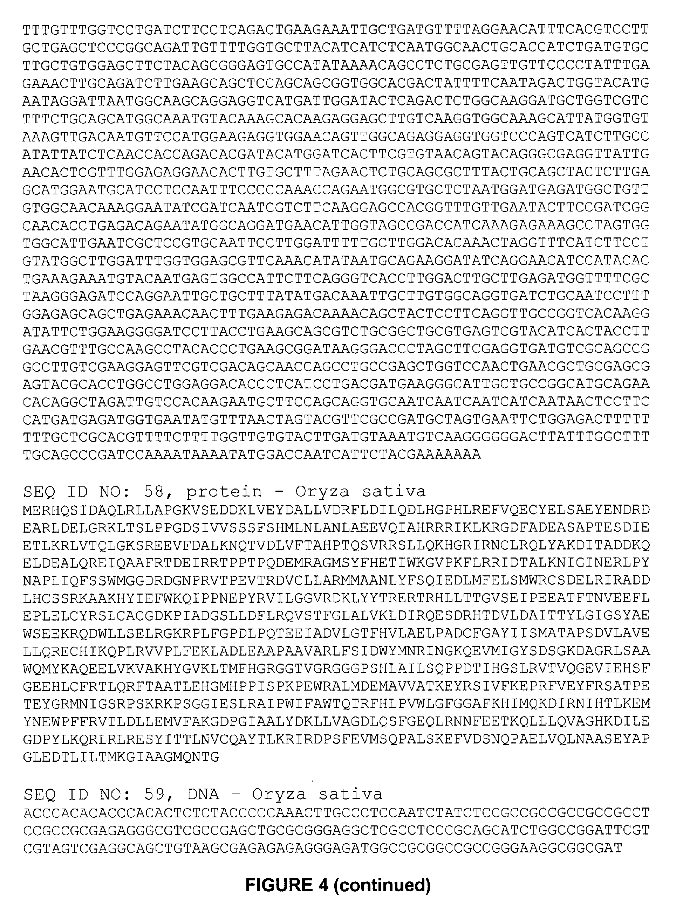

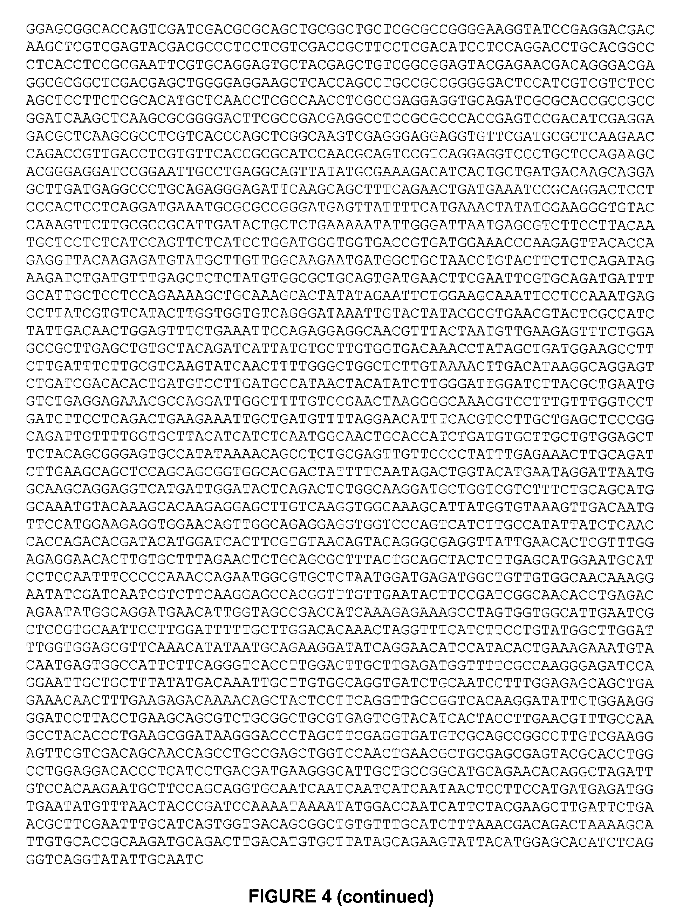

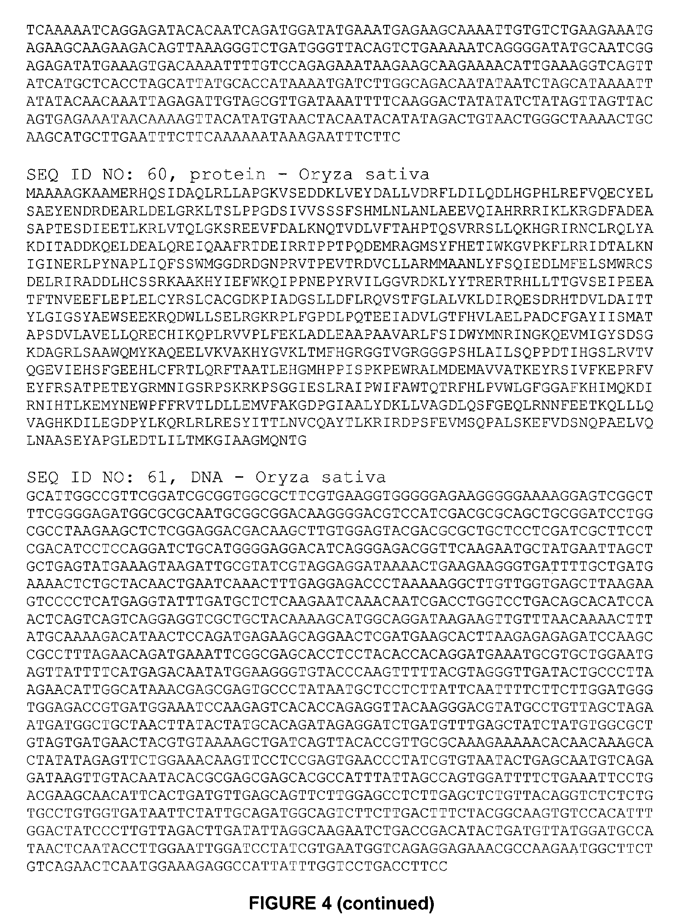

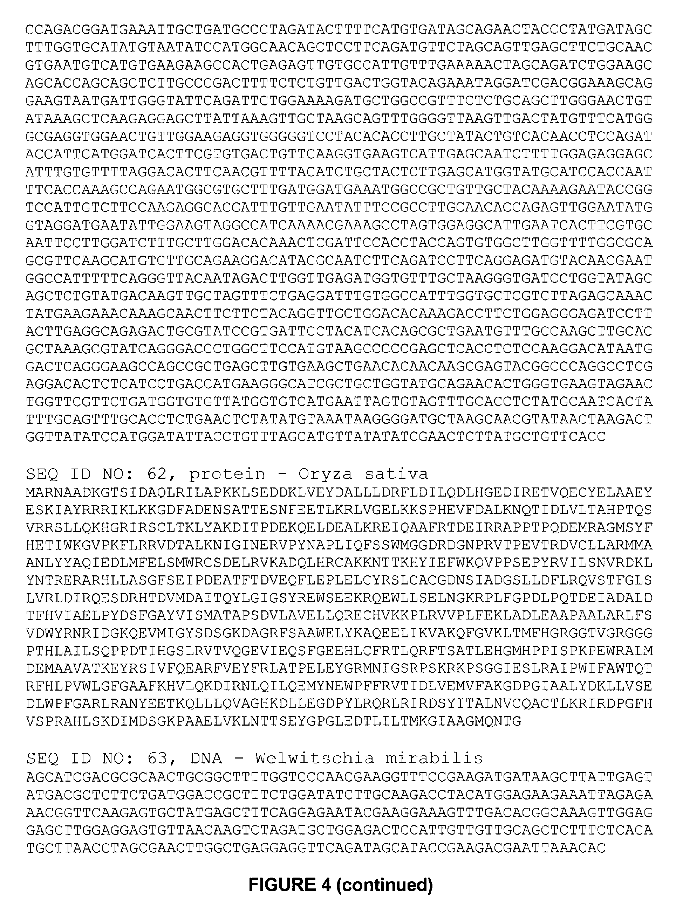

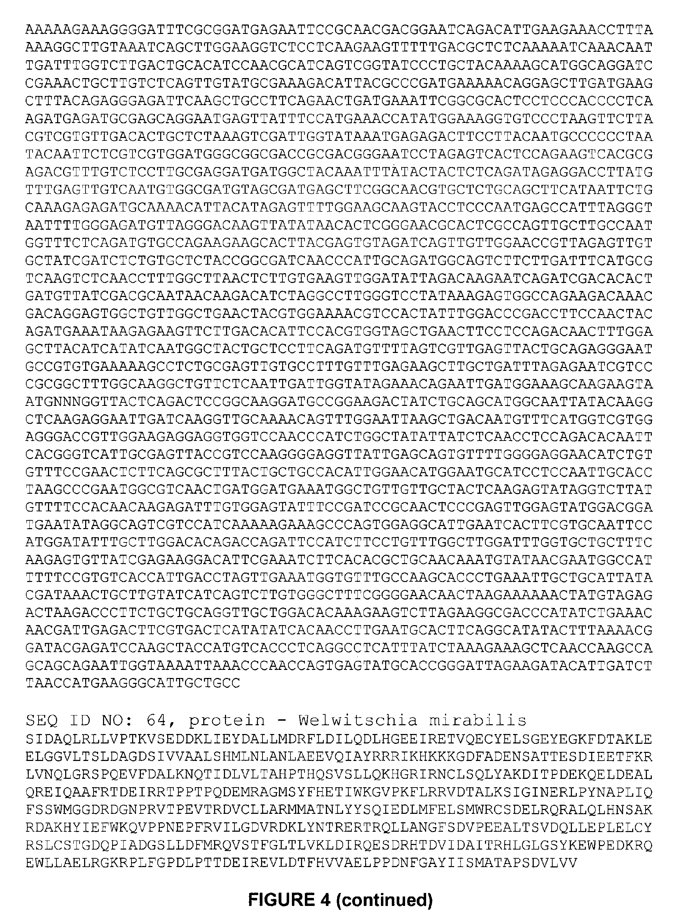

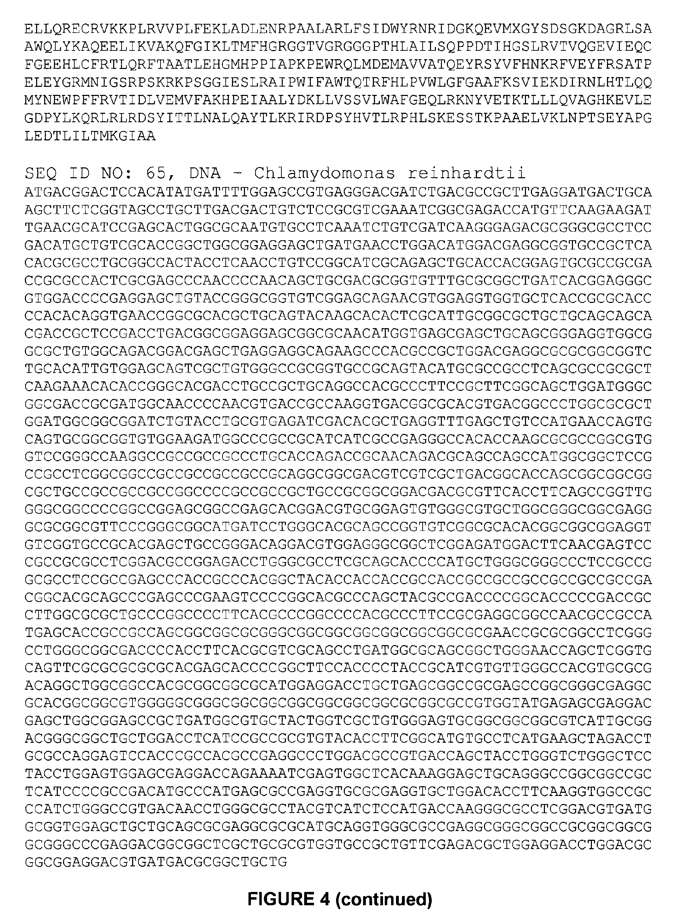

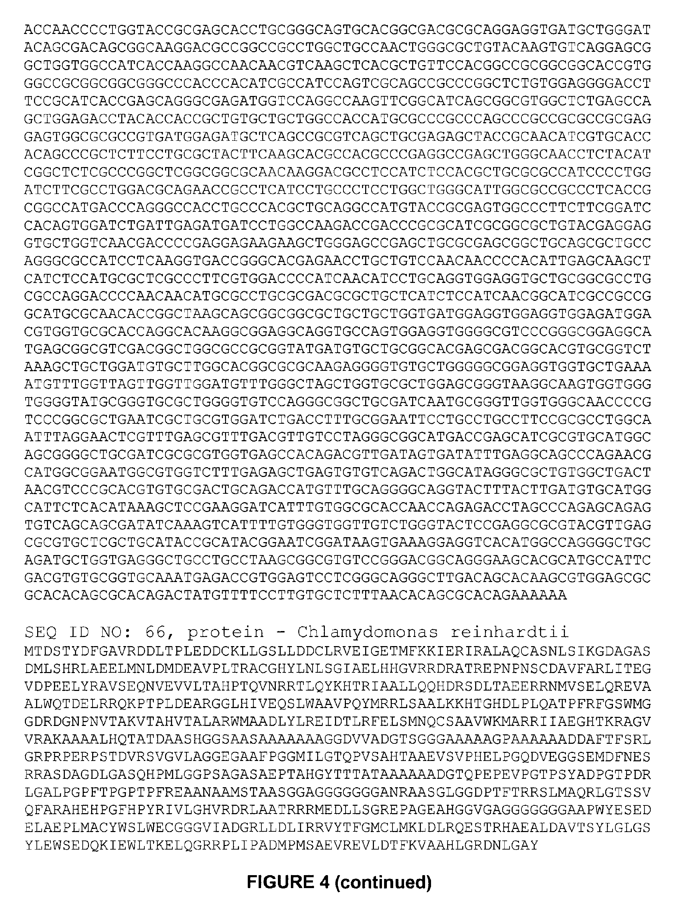

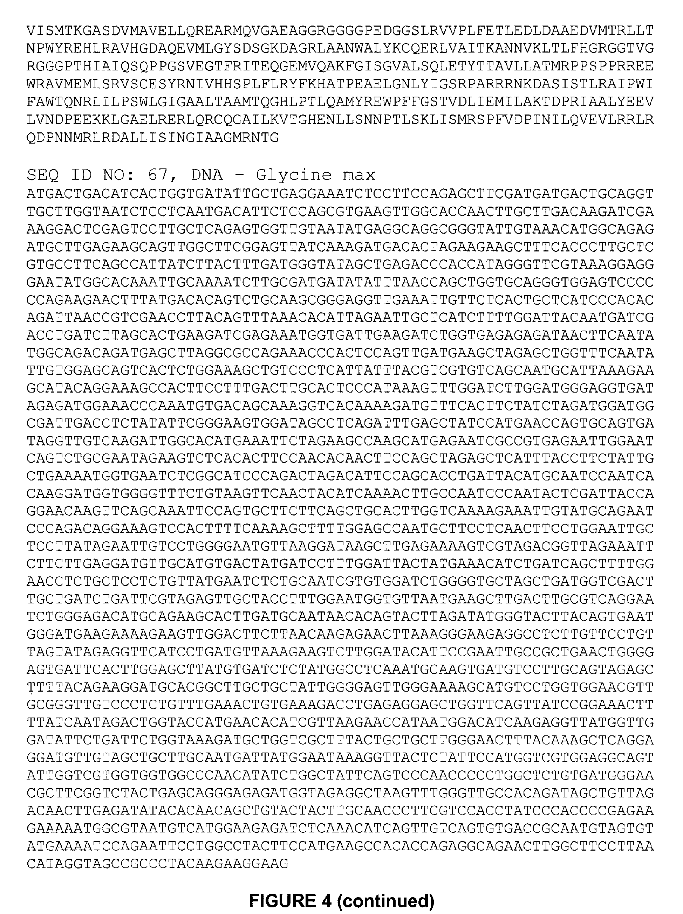

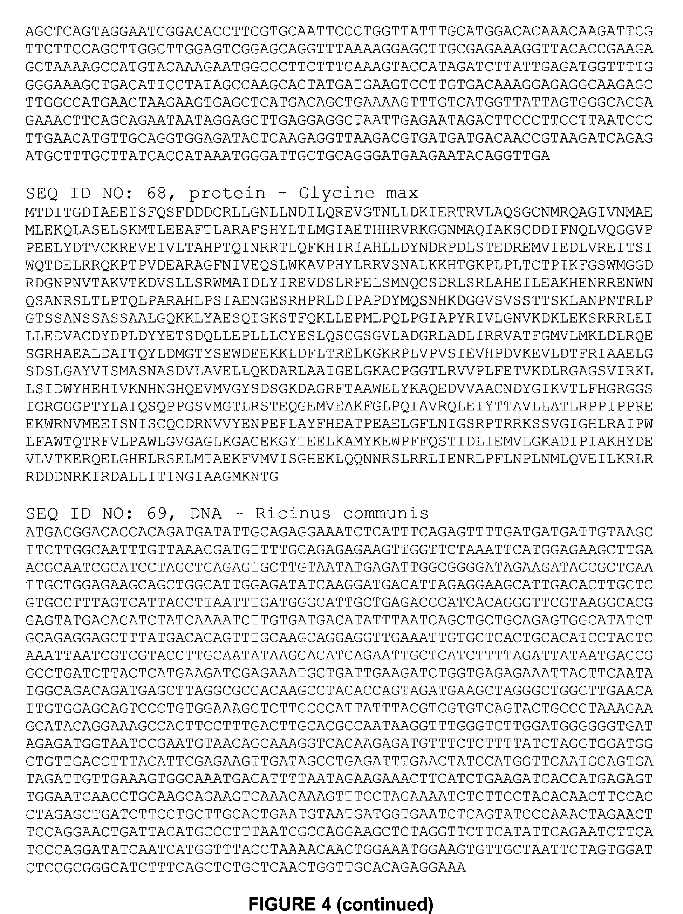

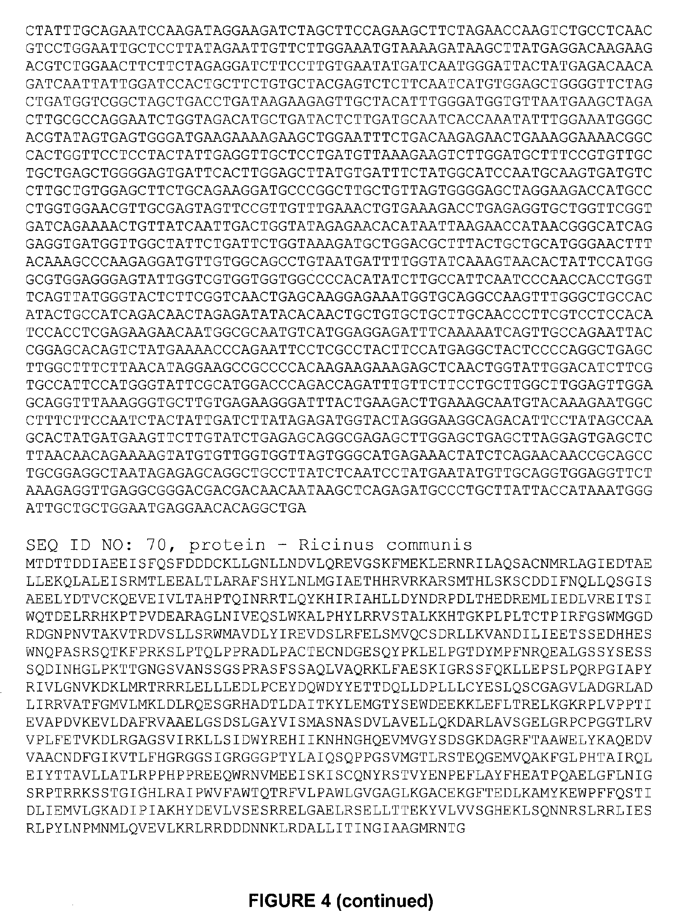

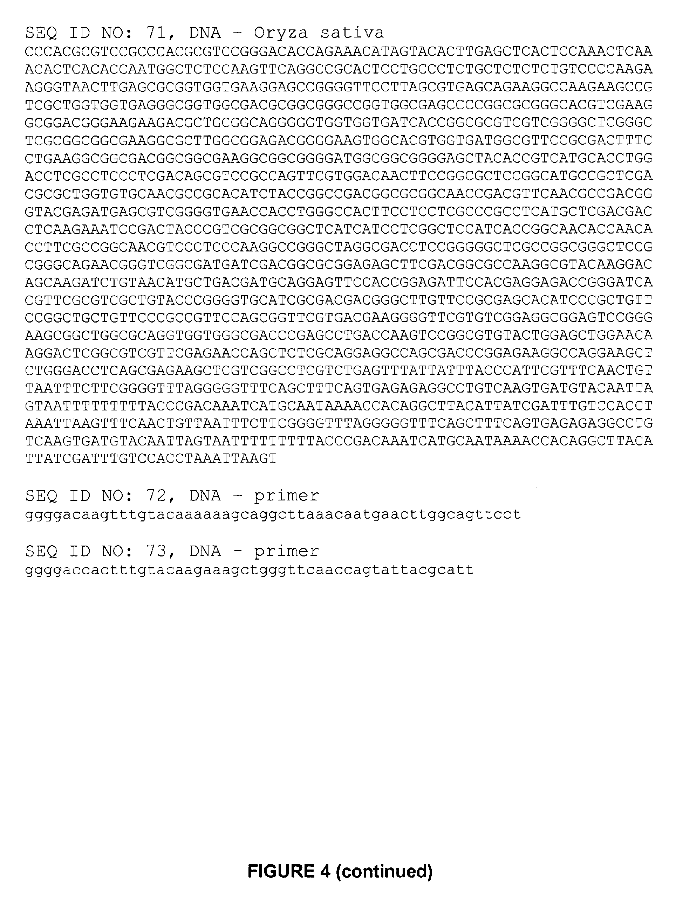

Fig. 4 details examples of sequences useful in performing the methods according to the present invention.

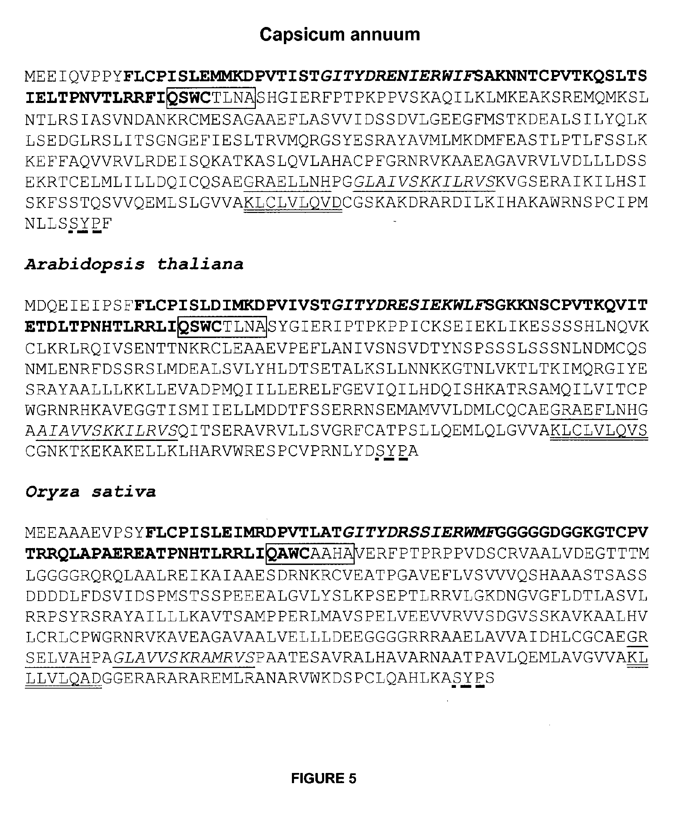

Fig. 5 shows the amino acid sequence for a Class III U-box protein from Capsicum, Arabidopsis and rice in which the U-box domain has been indicated as well as conserved Motifs I to VI. The U-box domain is shown in bold; Motif I in bold italic, Motif II is boxed, Motif III is underlined, Moif IV is italic and underlined, Motif V is double-undrlined and Motif VI is broken-underlined.

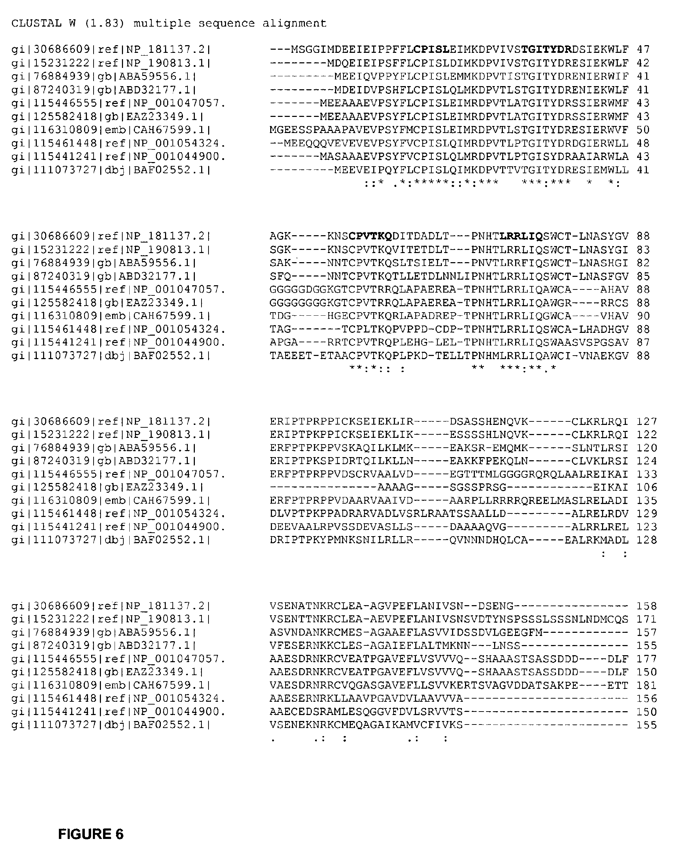

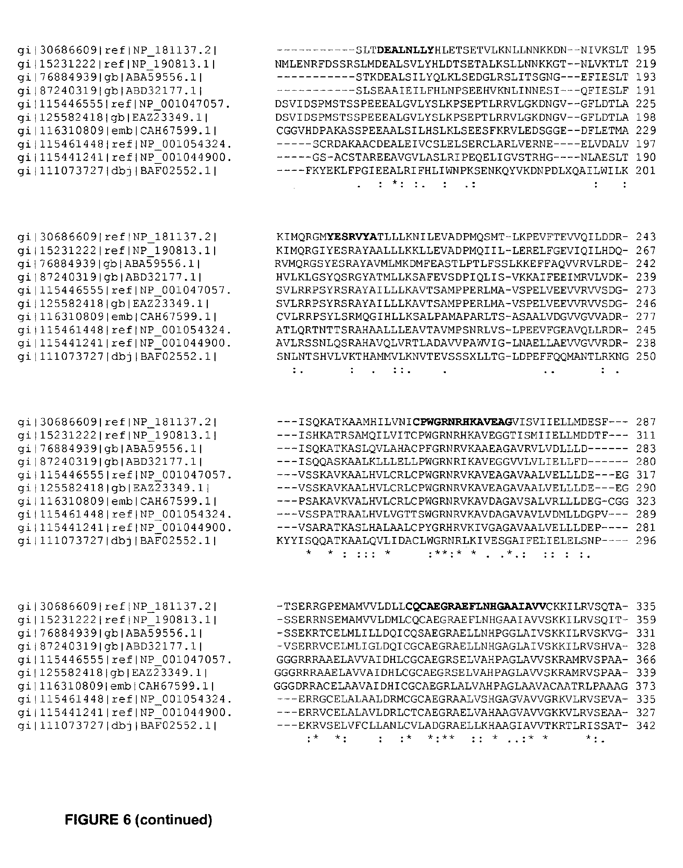

Fig. 6 shows a multiple alignment of a selection of Class III U-box proteins.

Fig. 7 represents the binary vector for increased expression in Oryza sativa of a Class III U-box-encoding nucleic acid under the control of a protochlorophyllid reductase promoter.

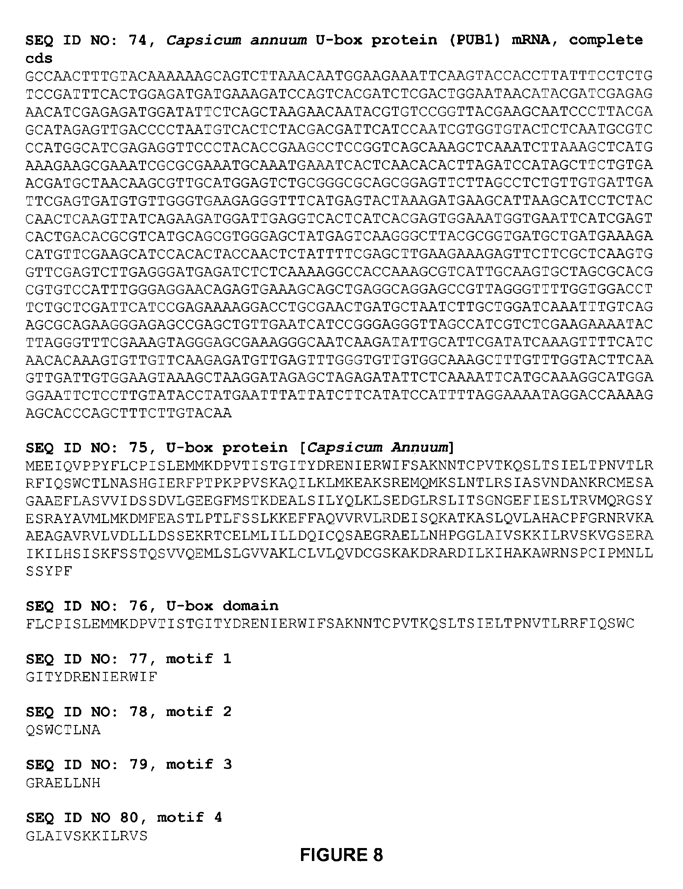

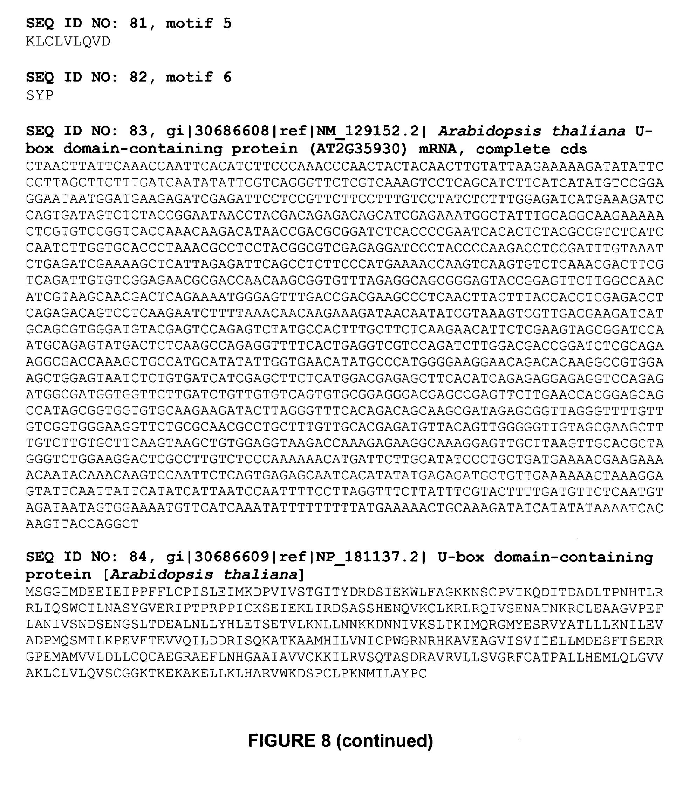

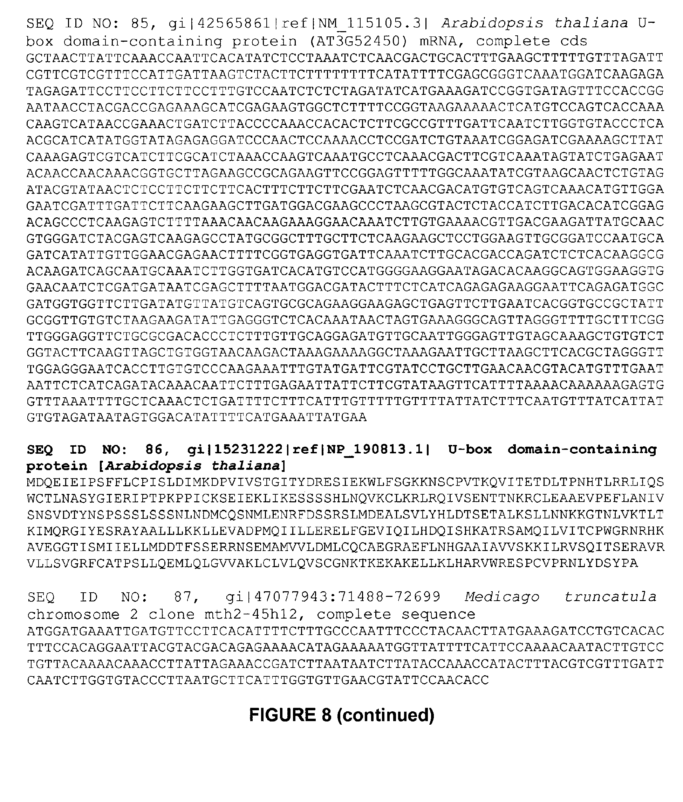

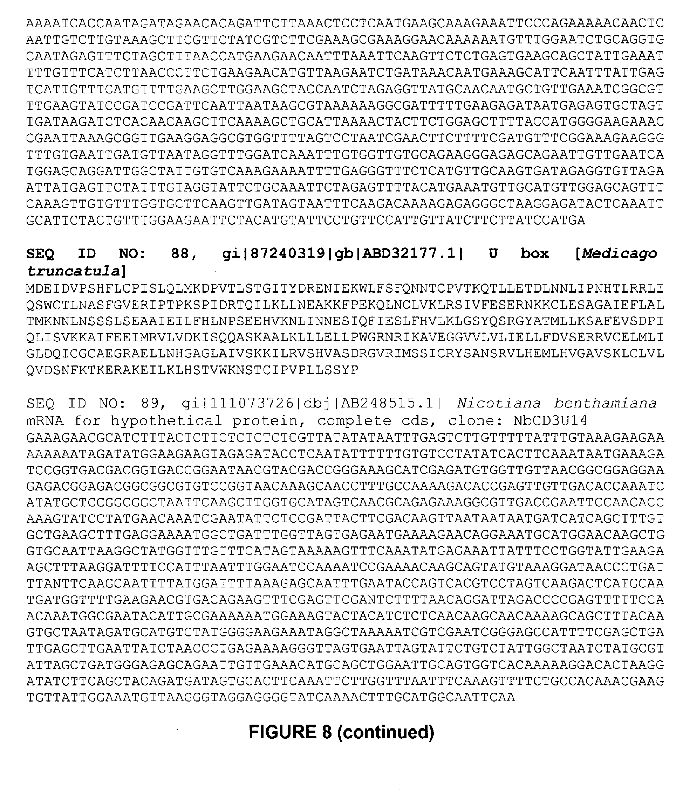

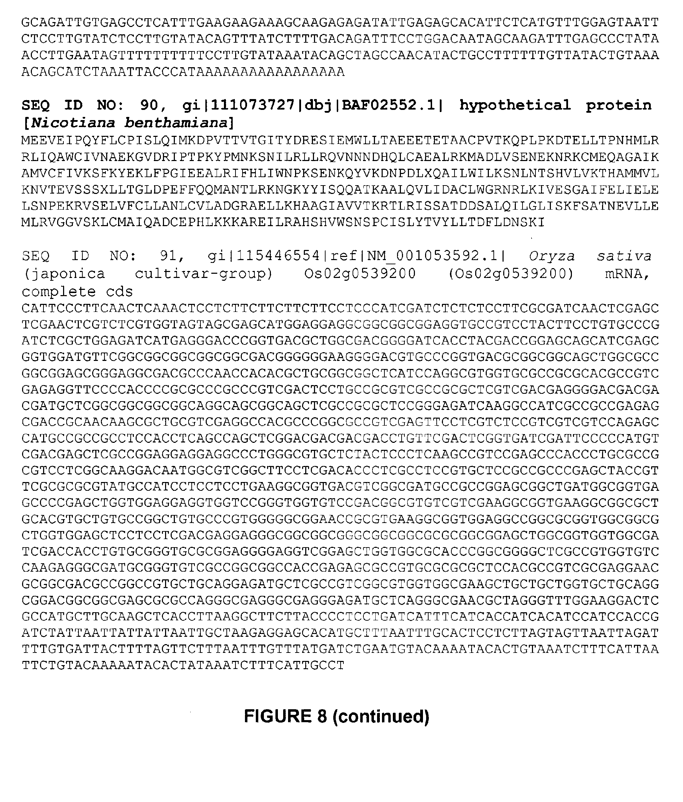

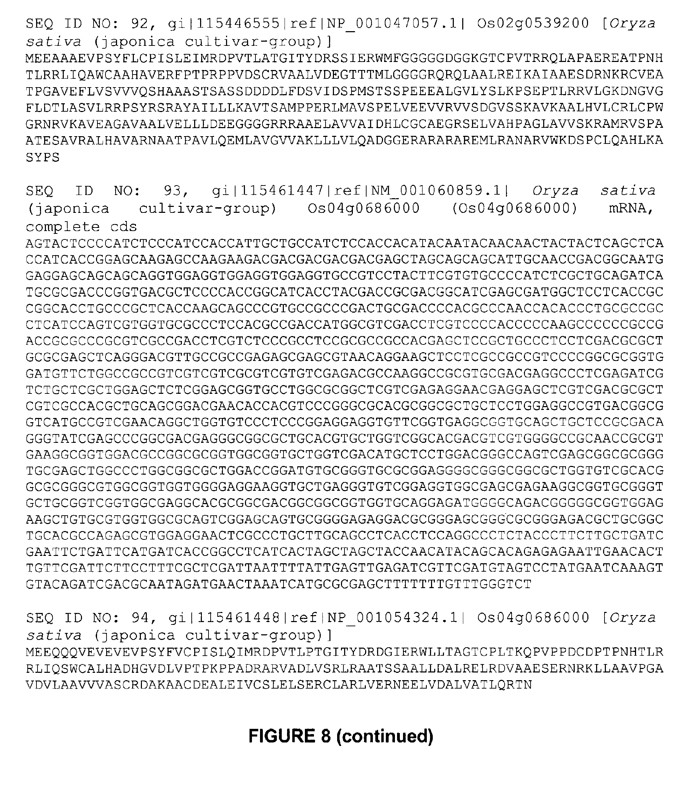

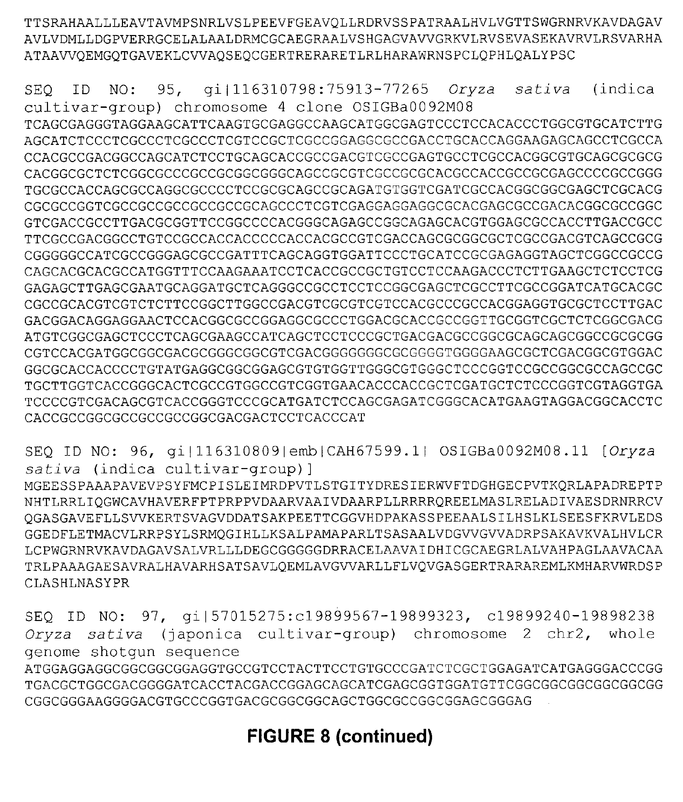

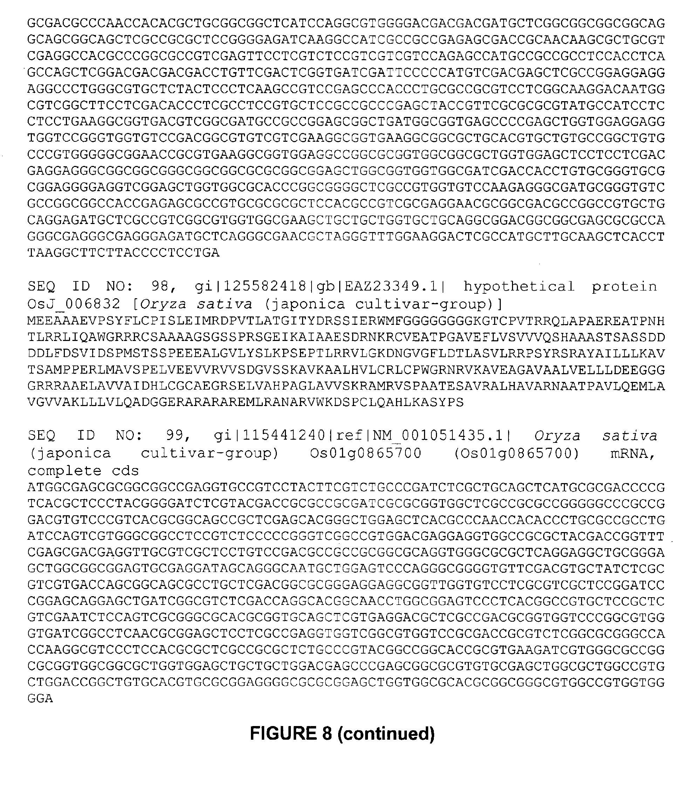

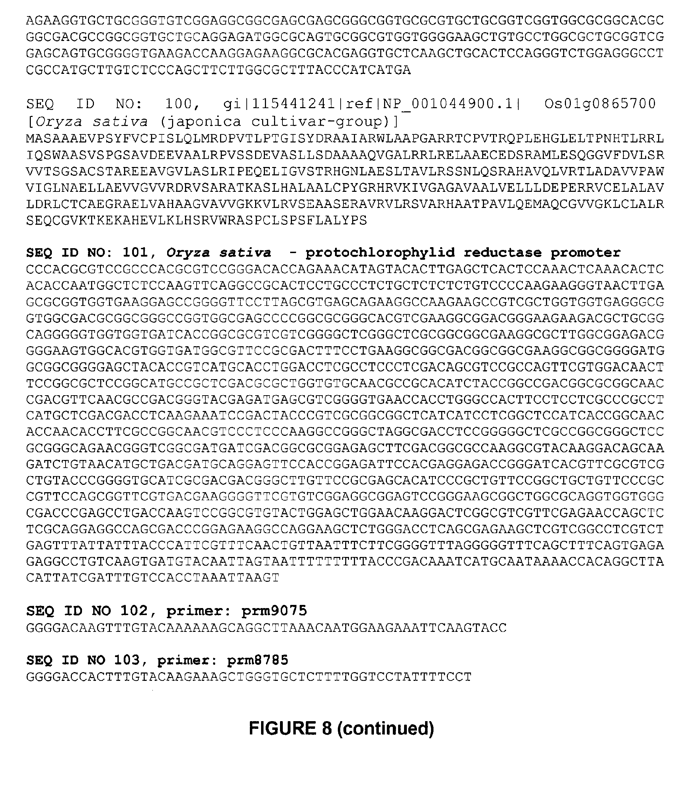

Fig. 8 details examples of sequences useful in performing the methods according to the present invention.

Fig. 9 shows a multiple alignment of a selection of PQQC proteins.

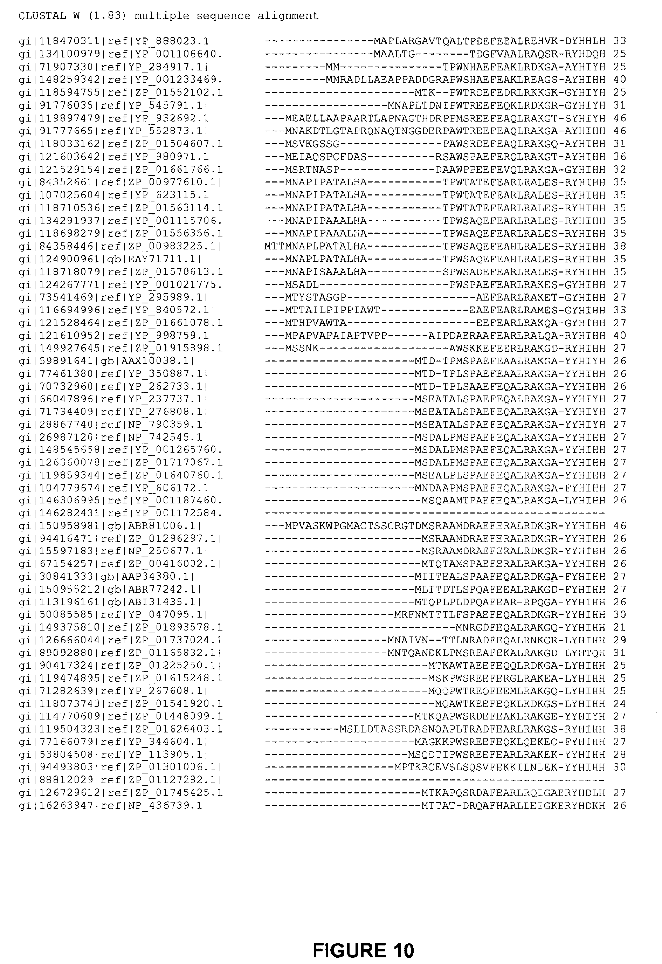

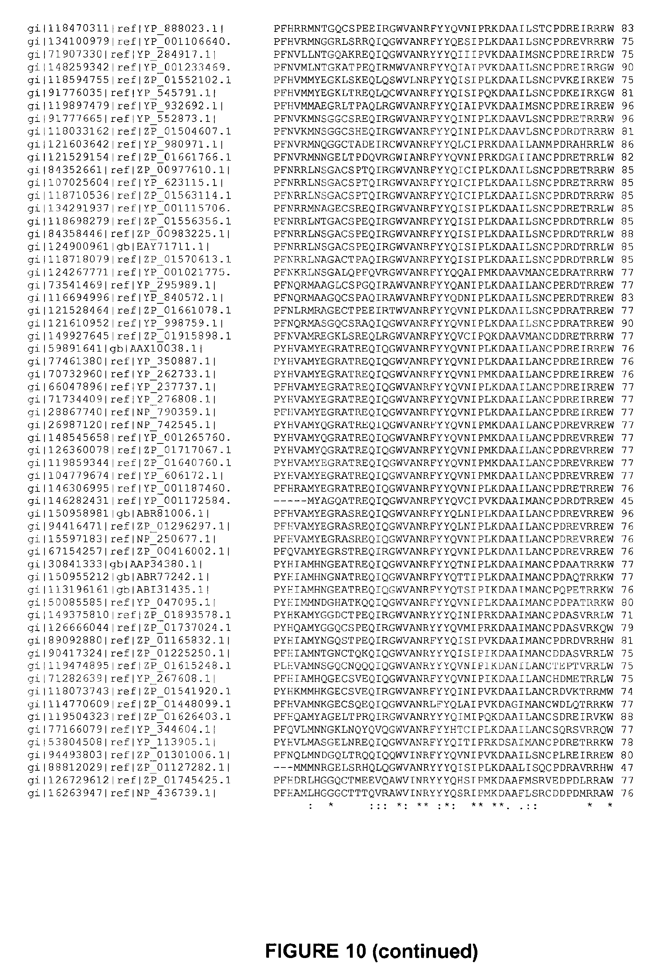

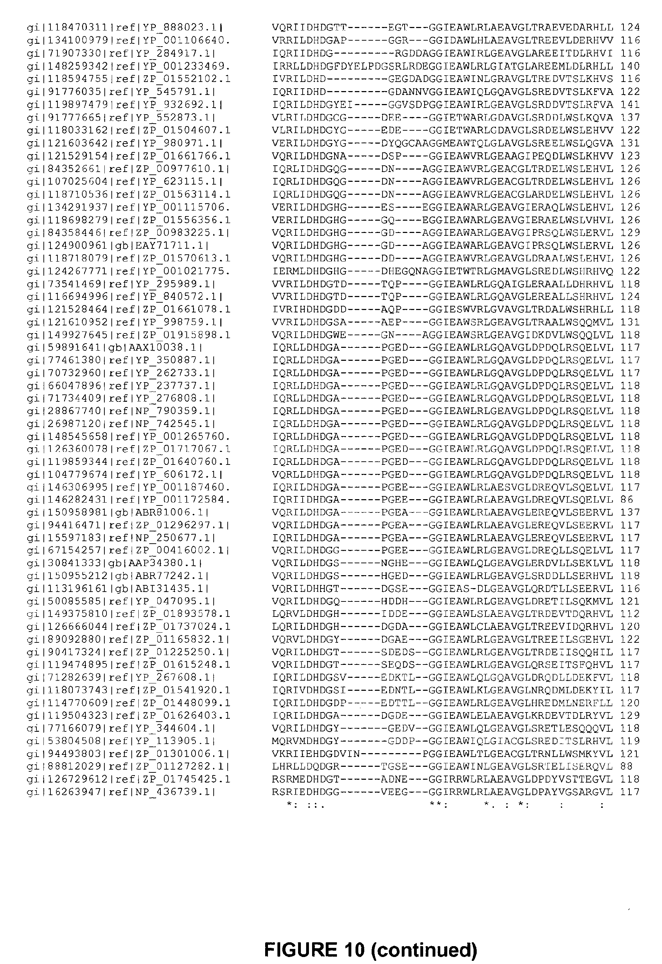

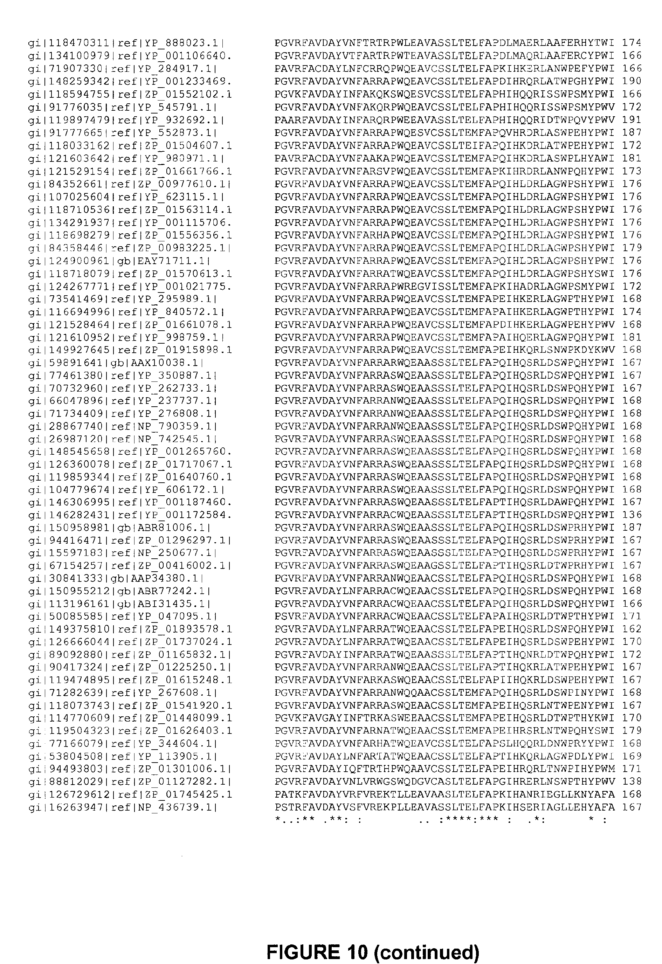

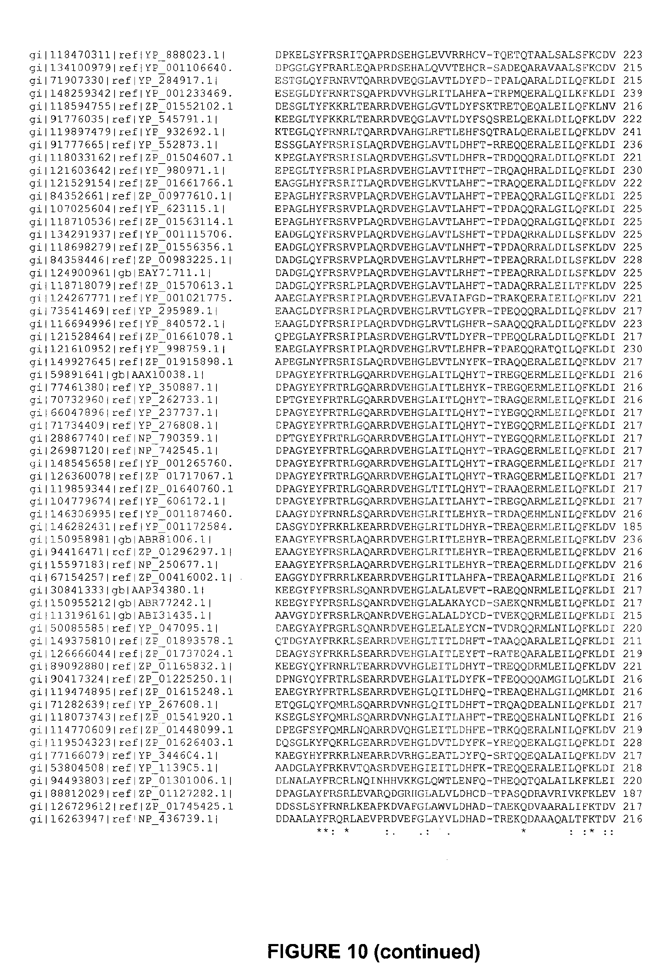

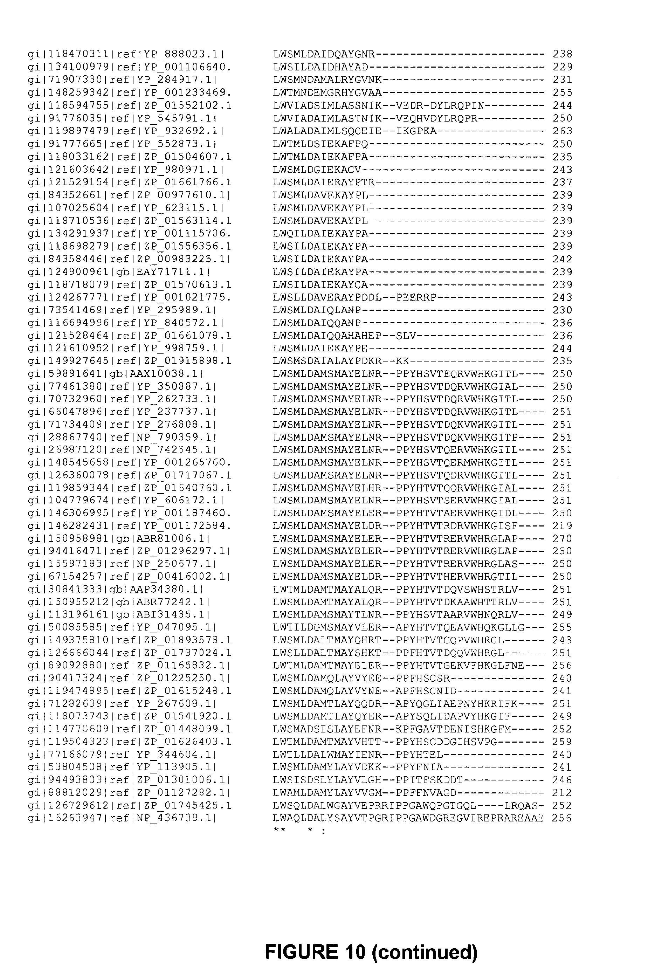

Fig. 10 represents the binary vector for increased expression in Oryza sativa of a PQQC-encoding nucleic acid under the control of a protochlorophyllid reductase promoter.

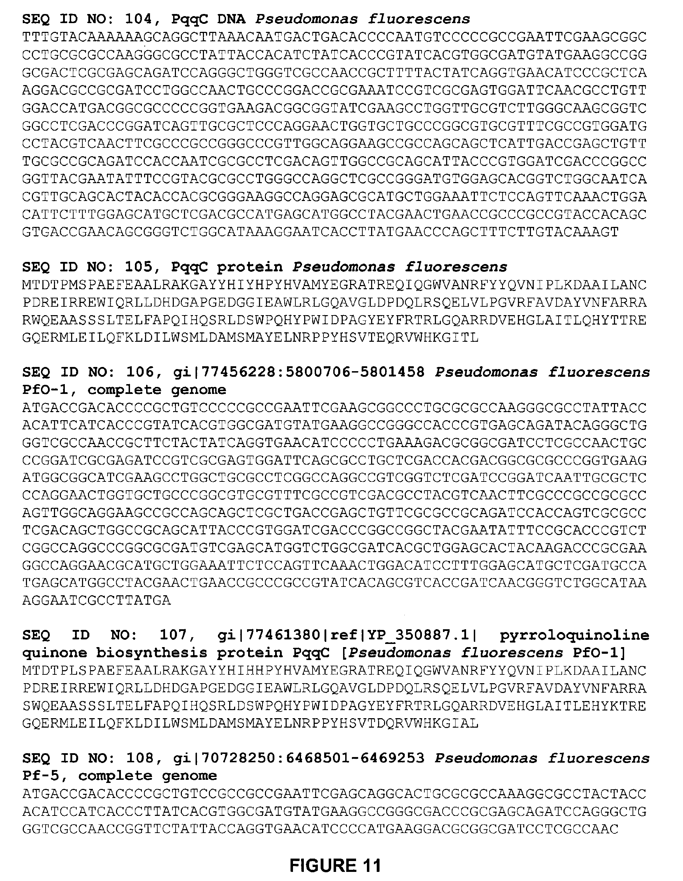

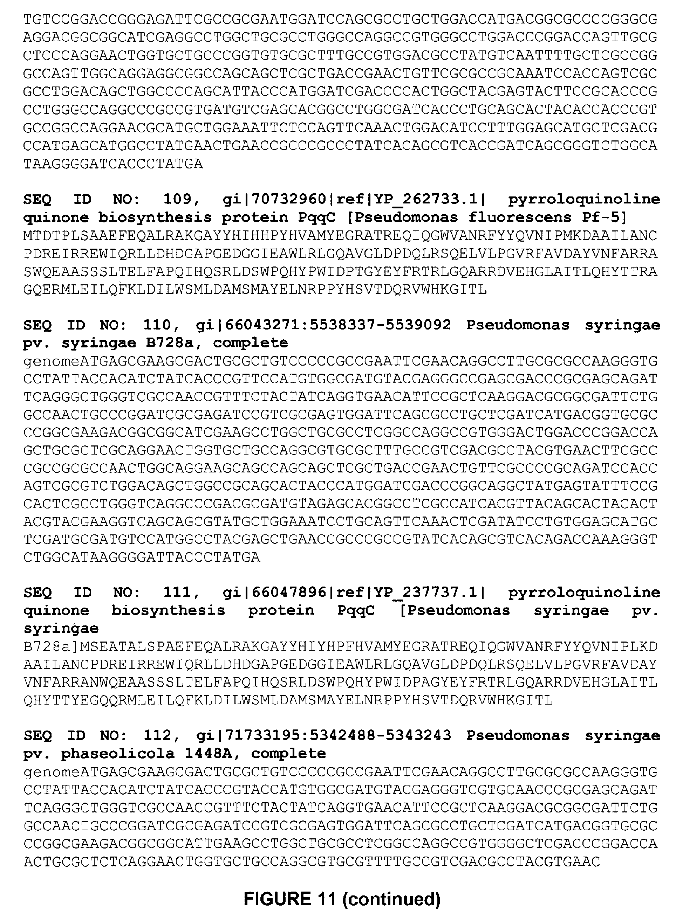

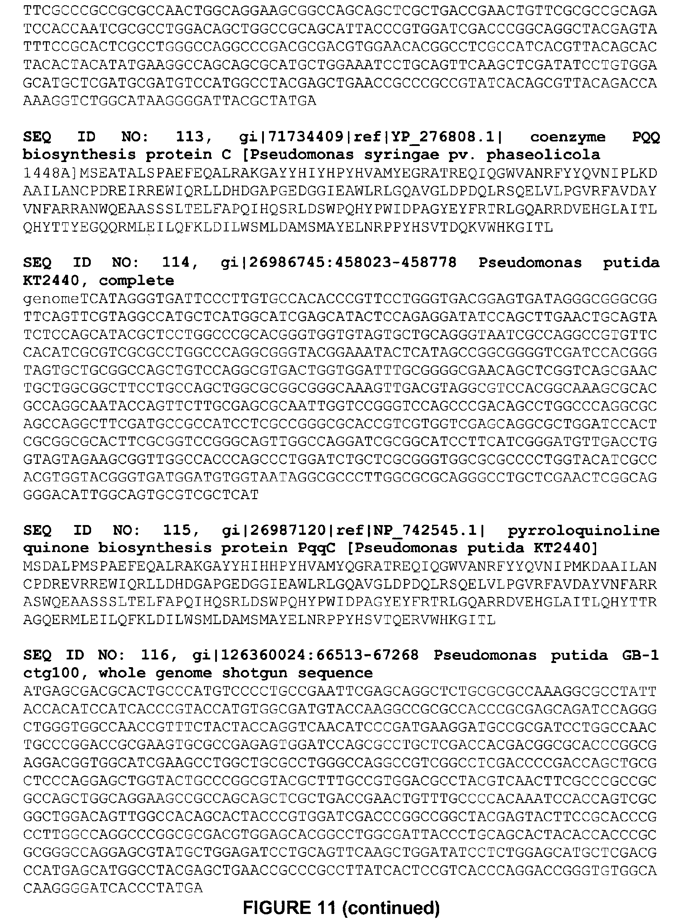

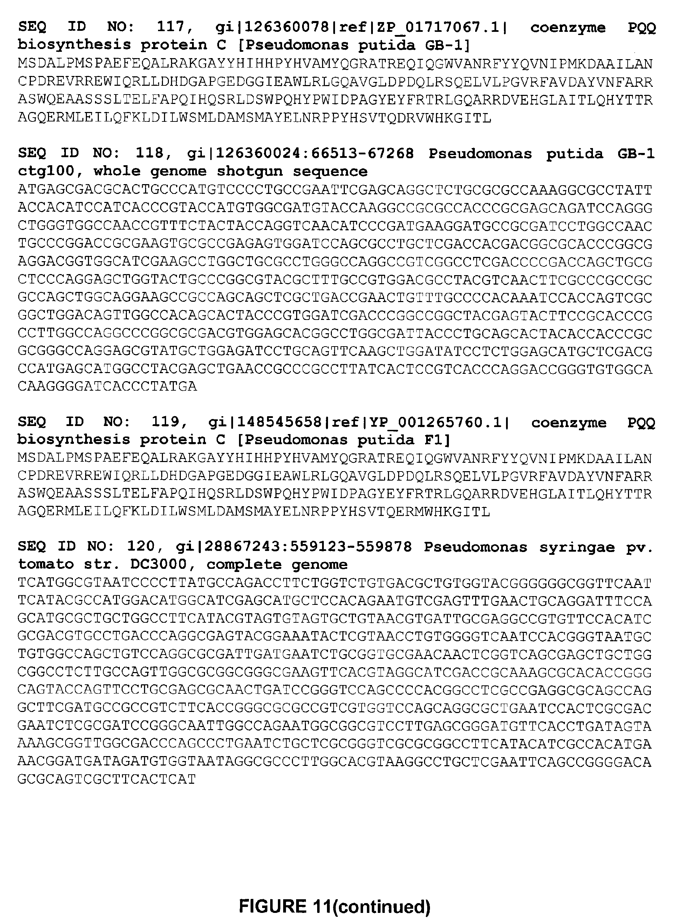

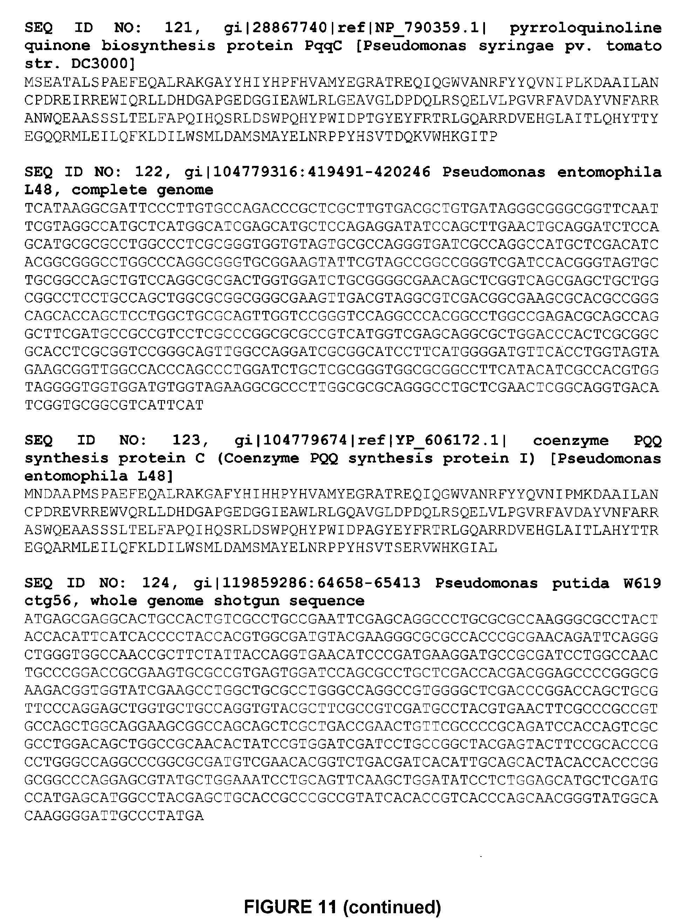

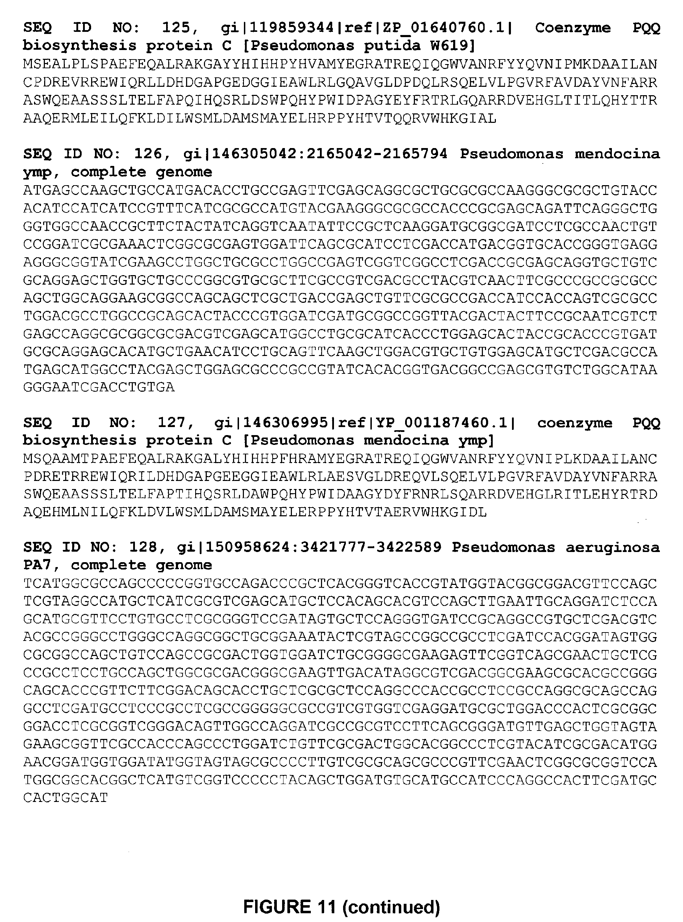

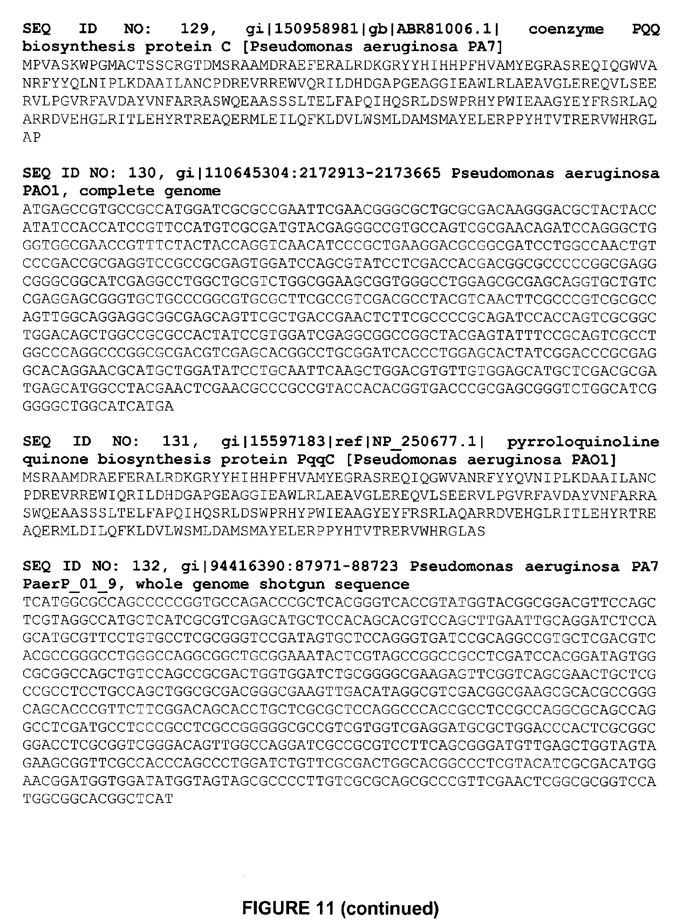

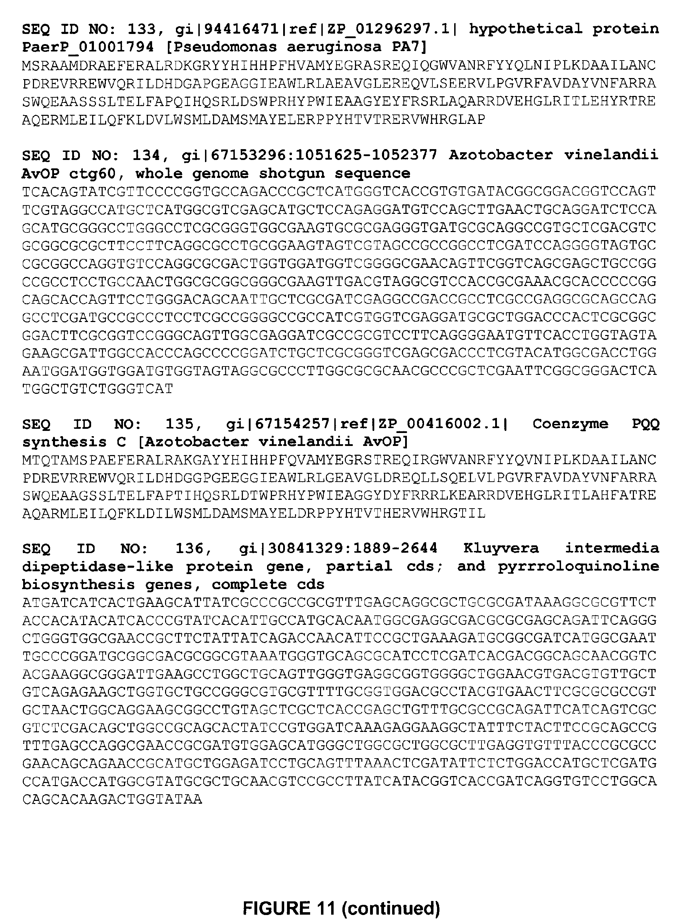

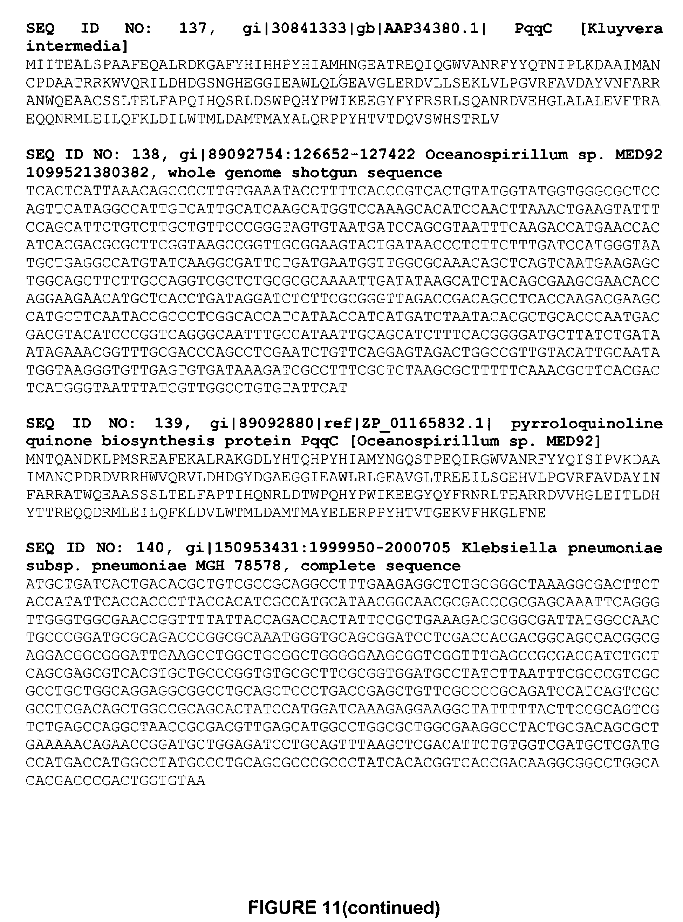

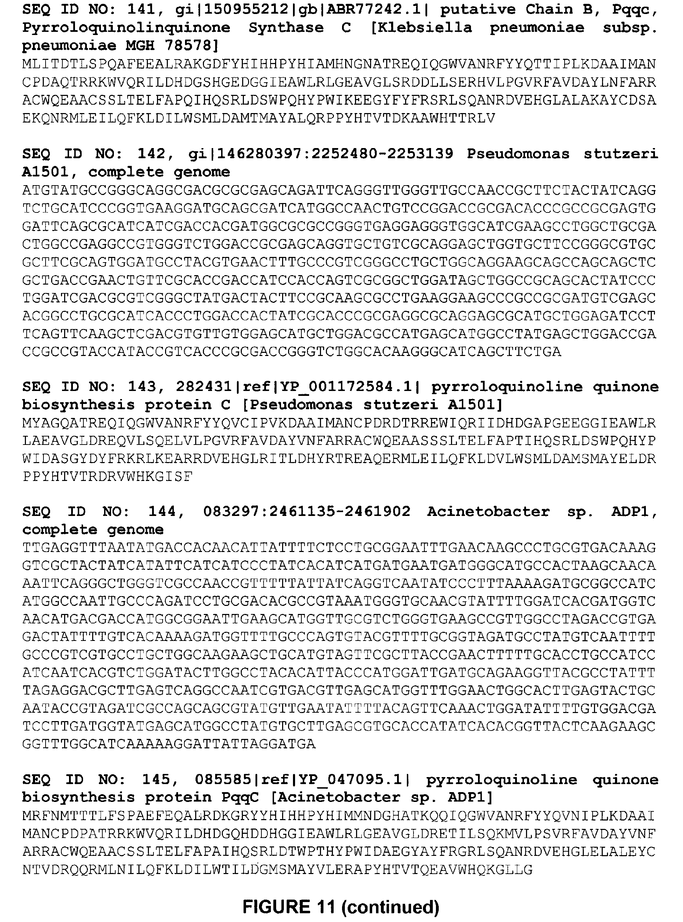

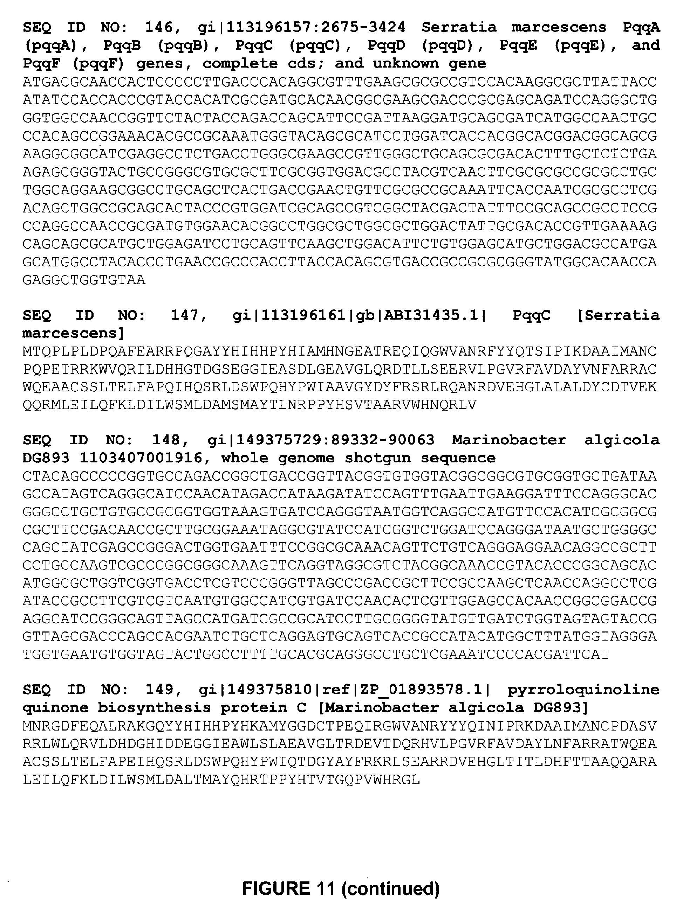

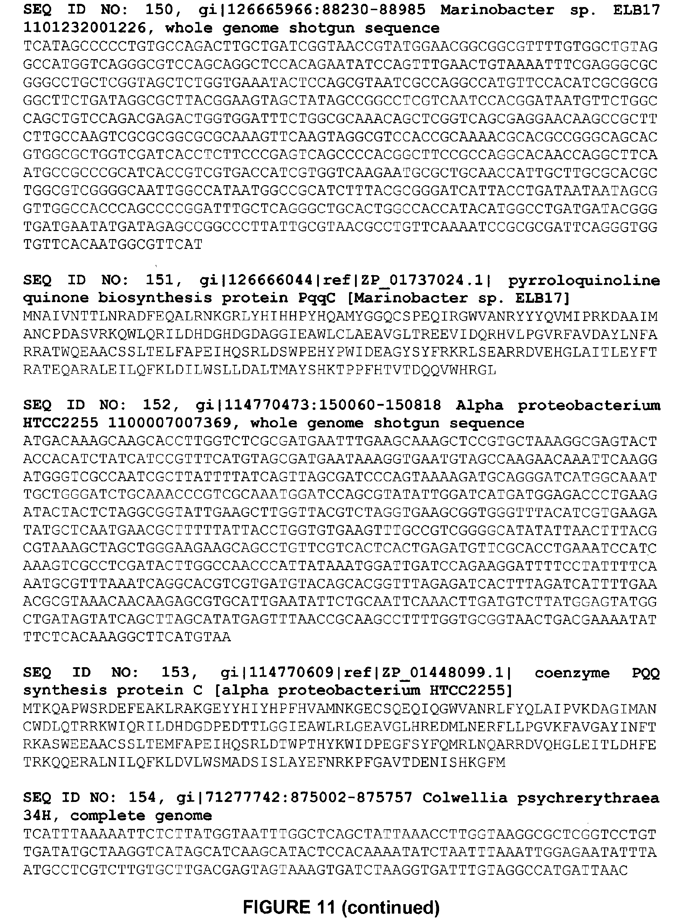

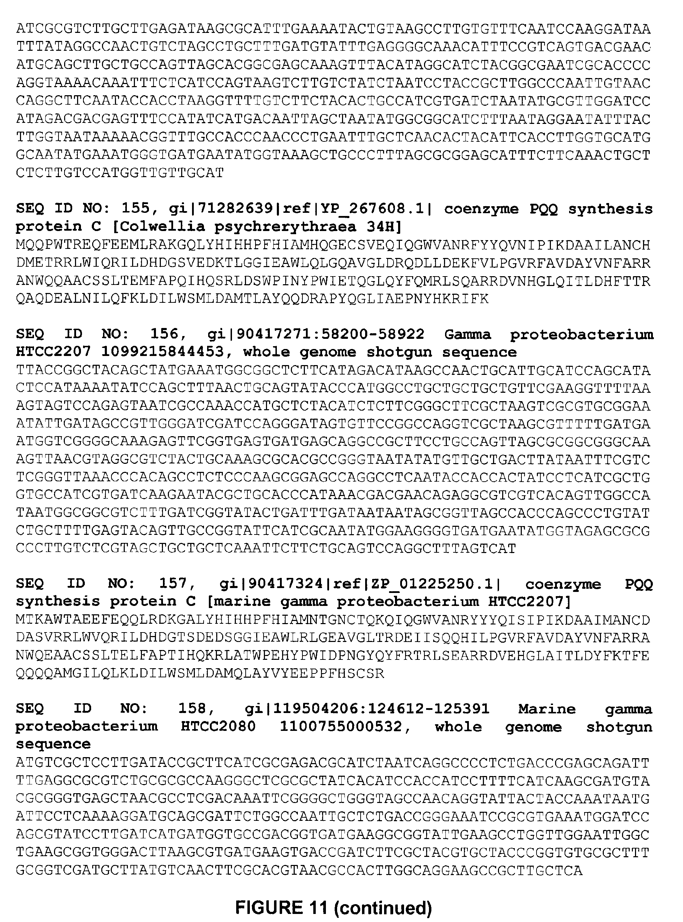

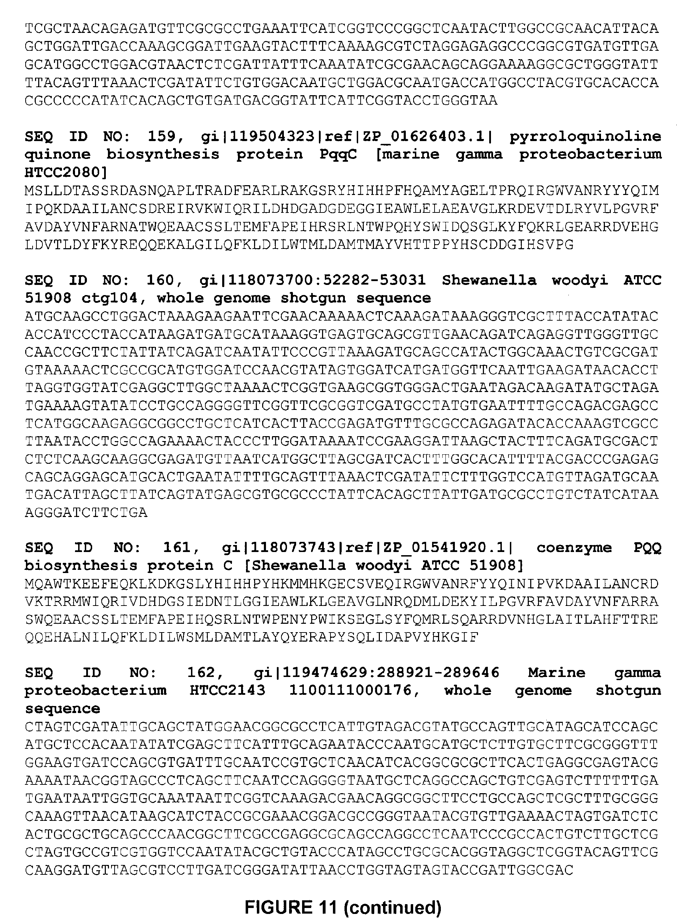

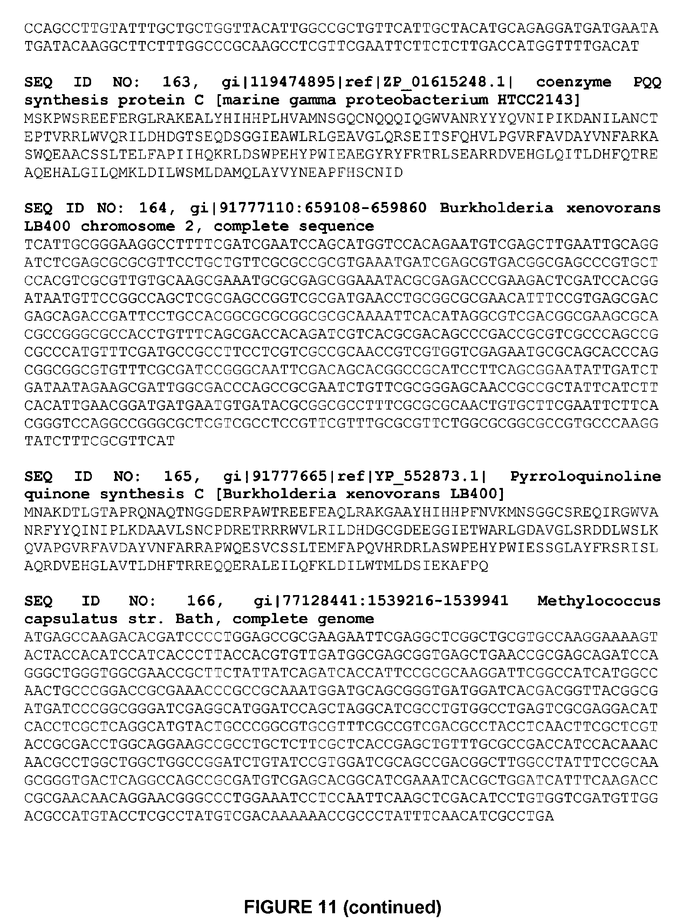

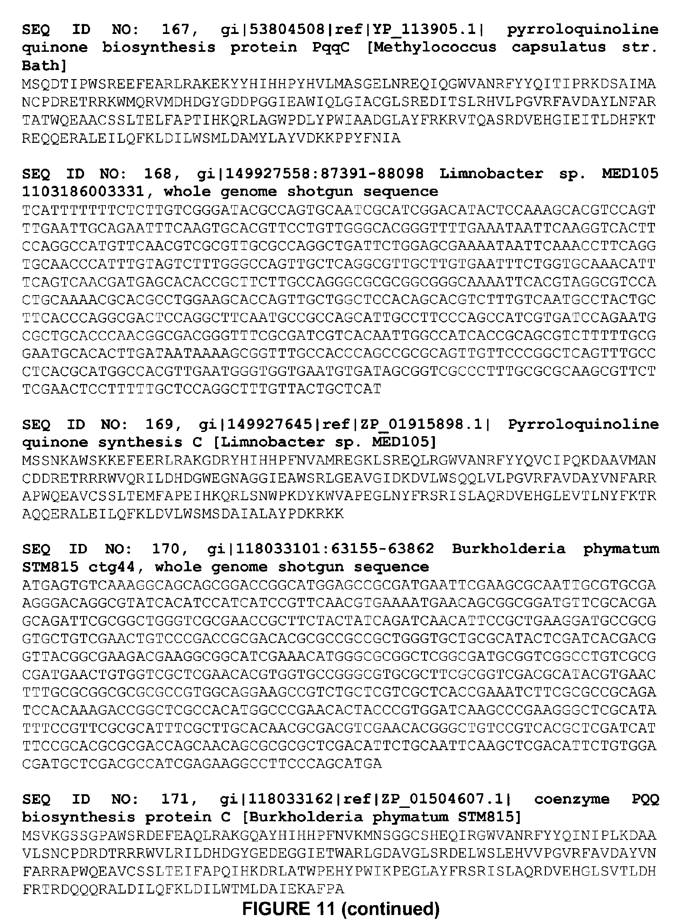

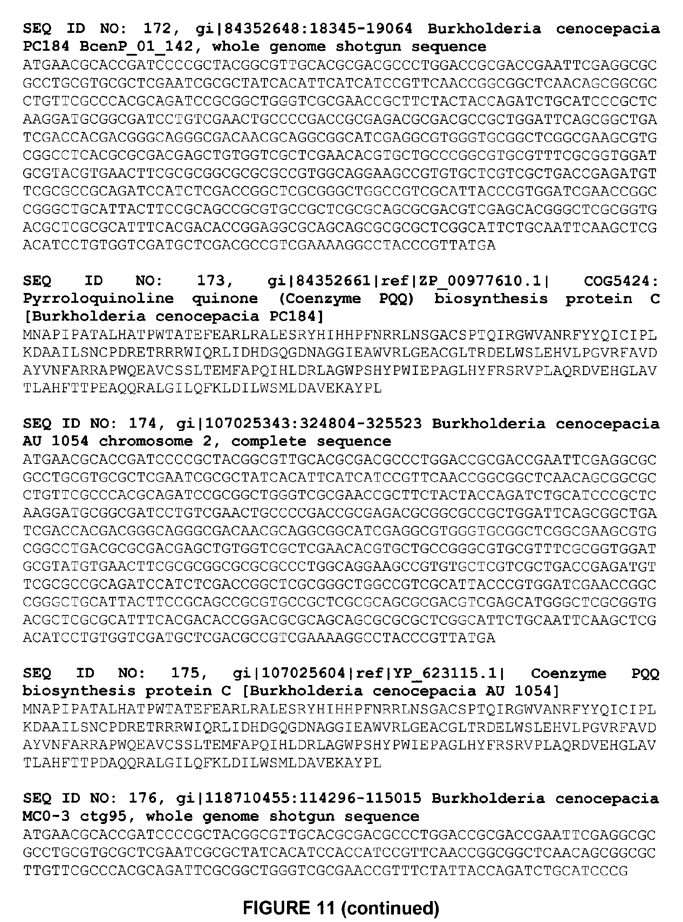

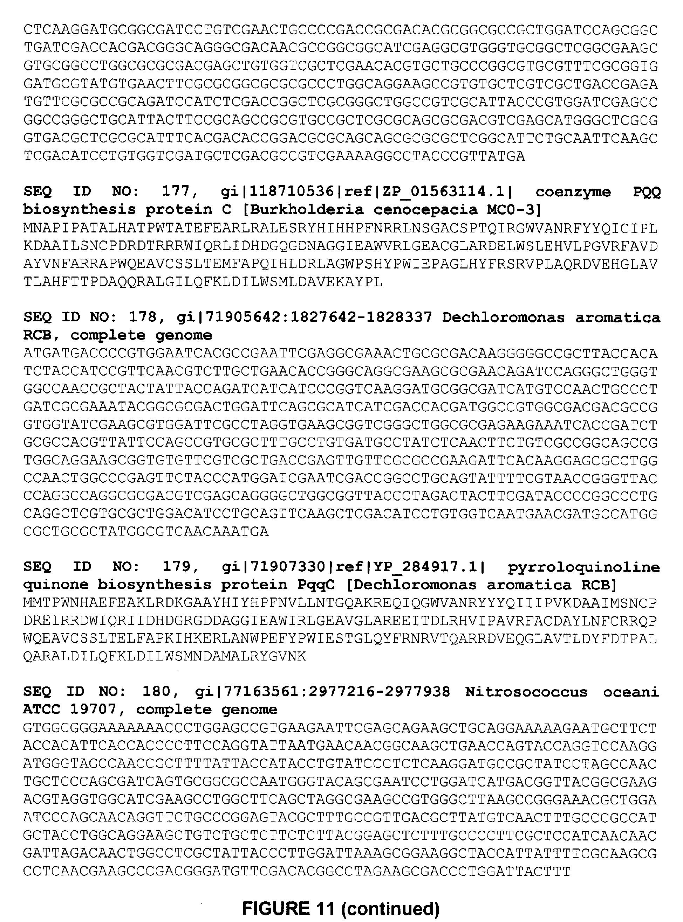

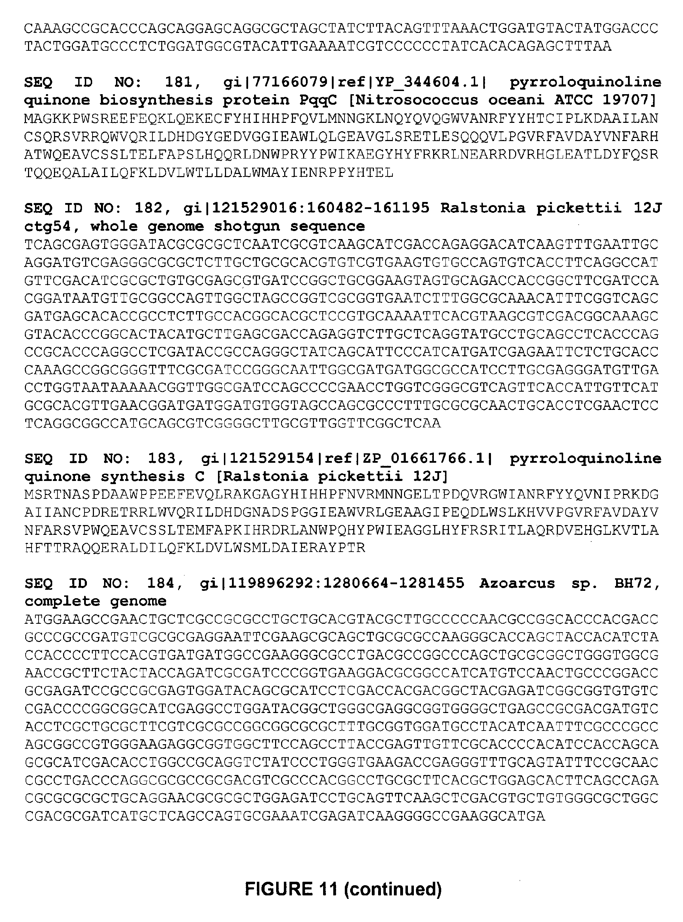

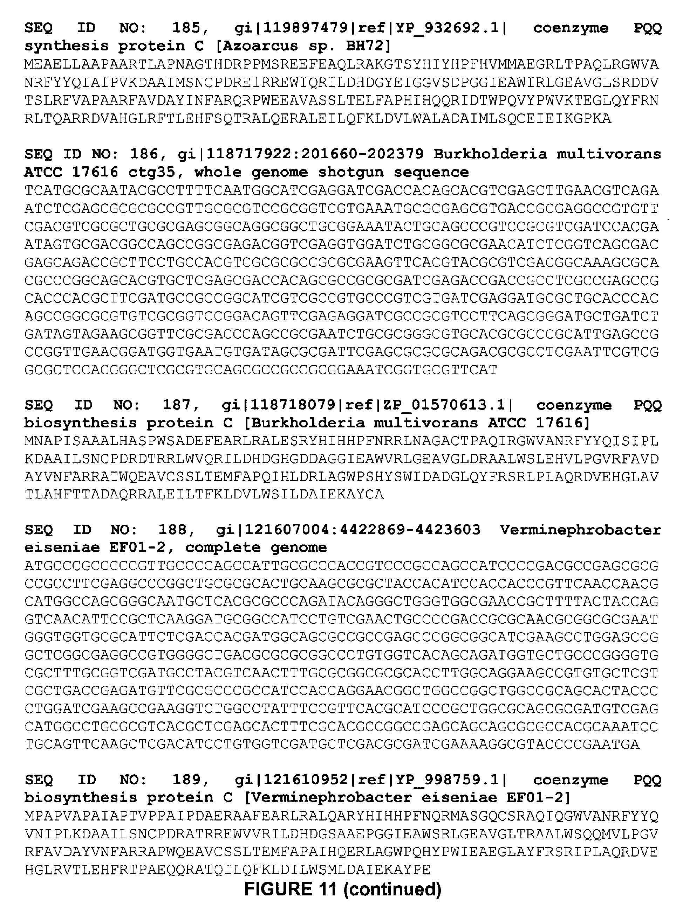

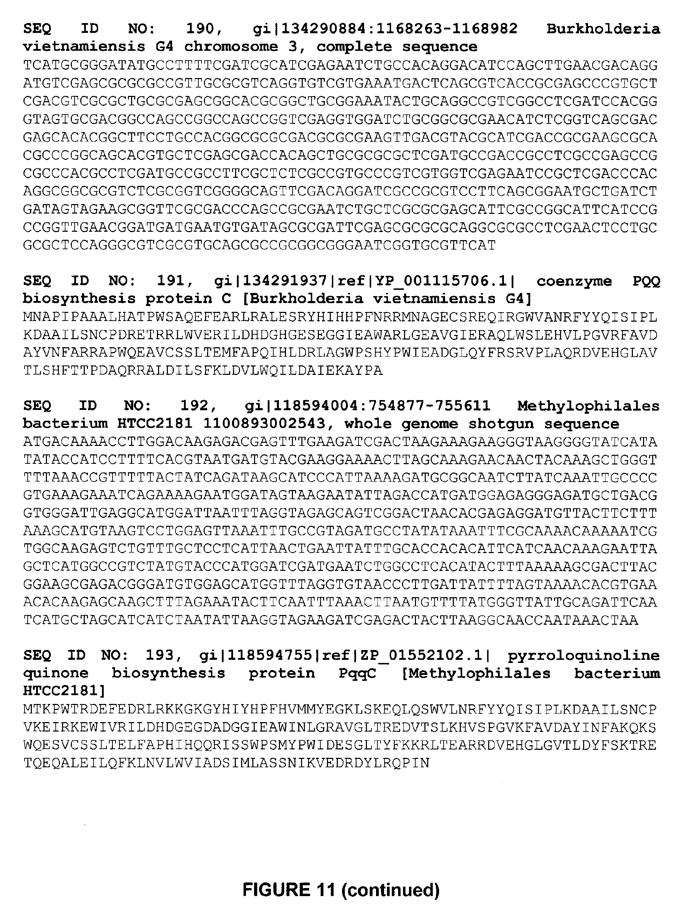

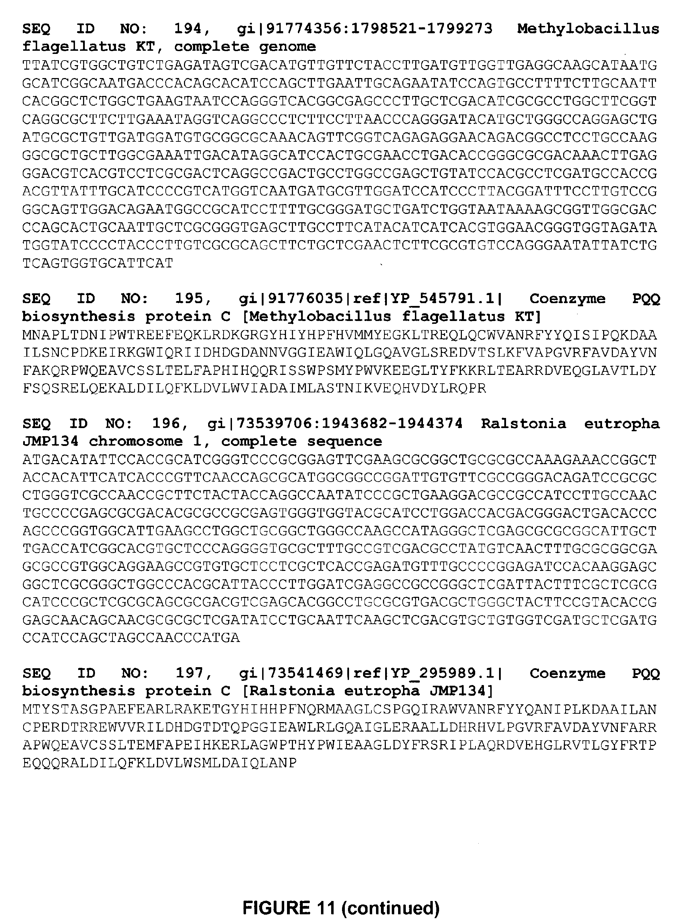

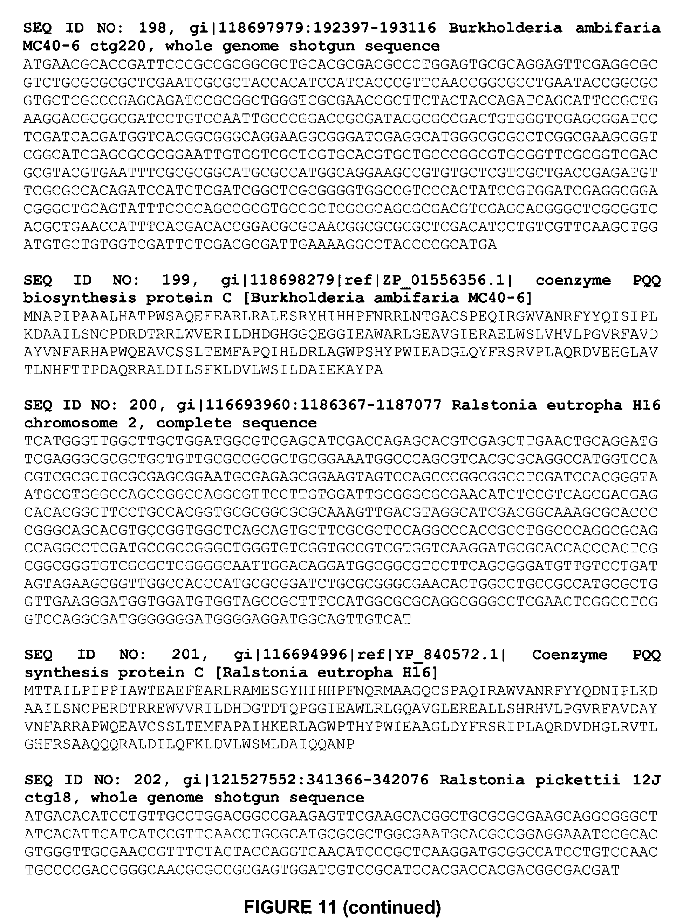

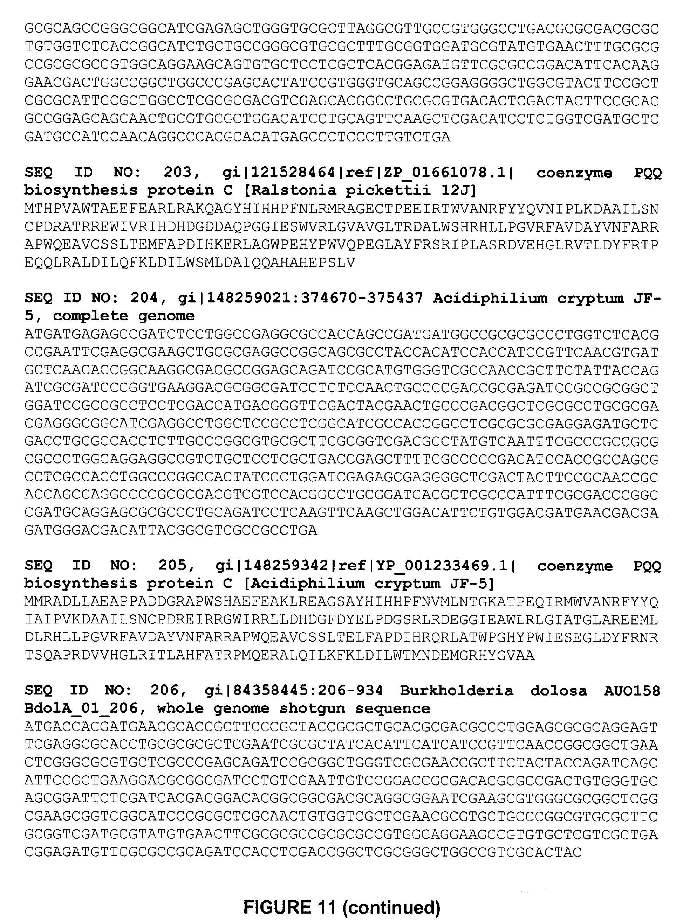

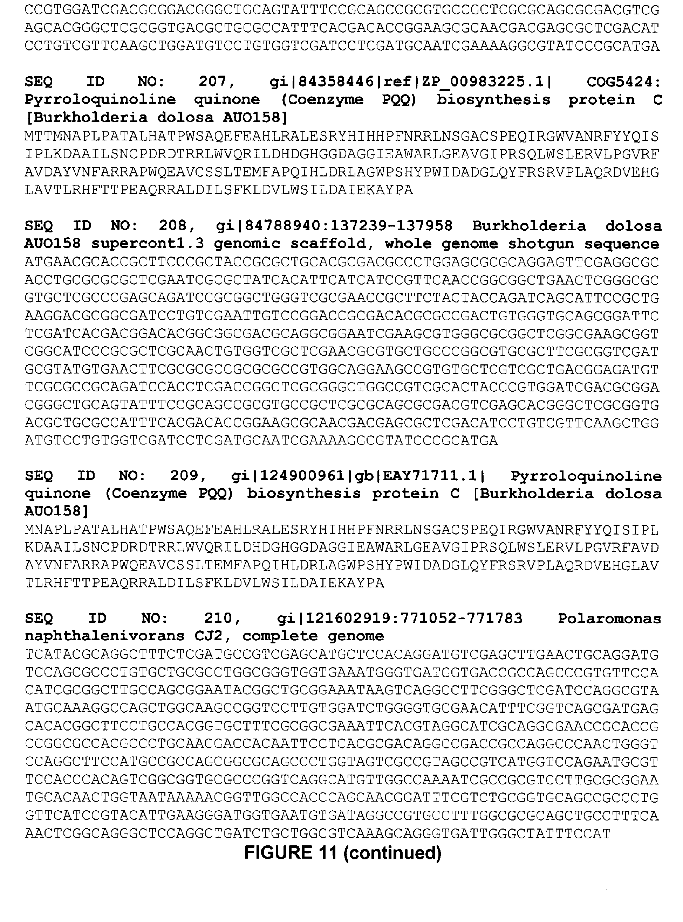

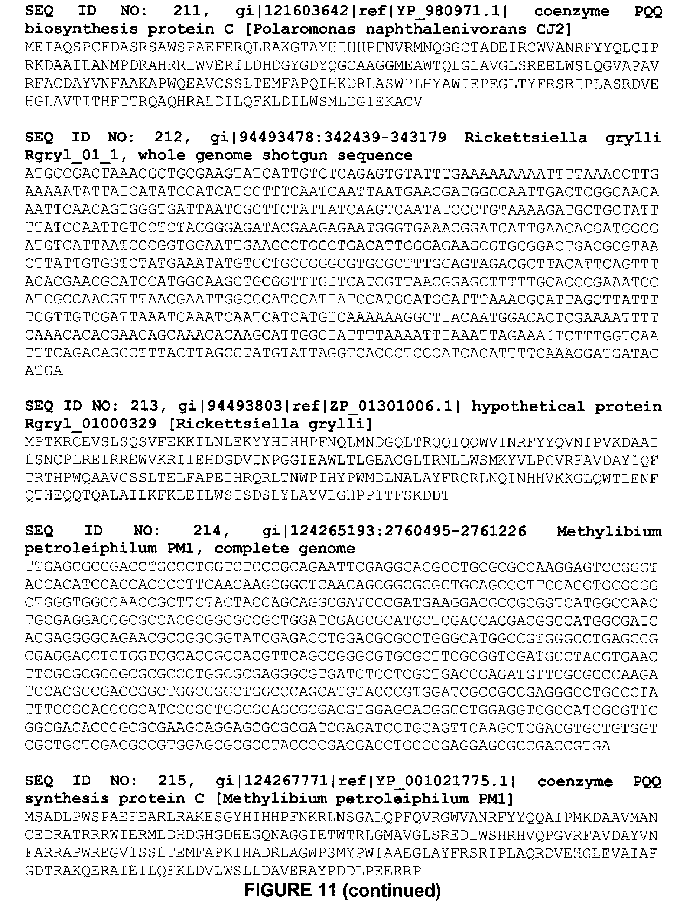

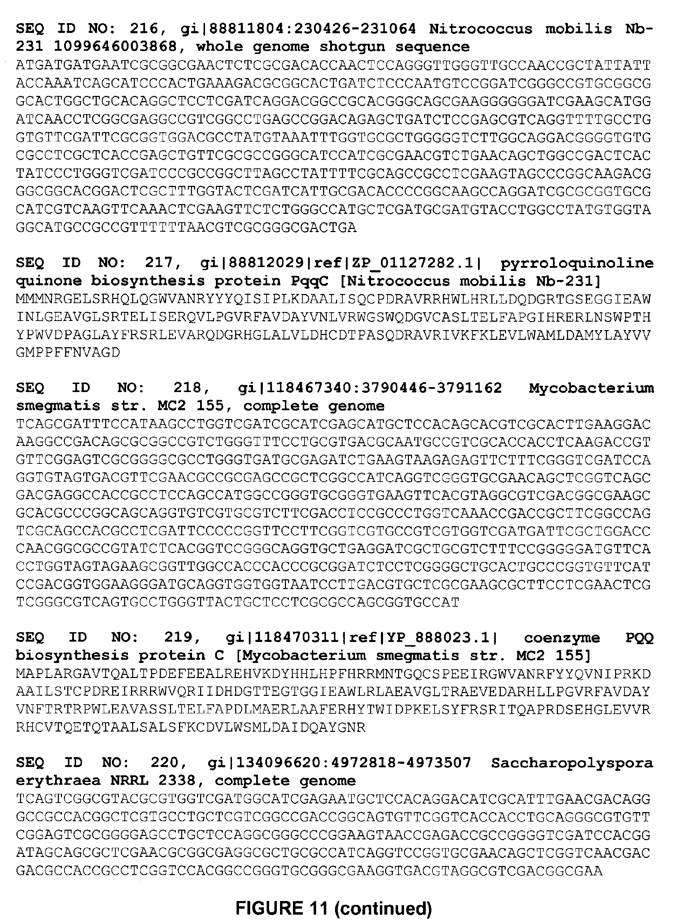

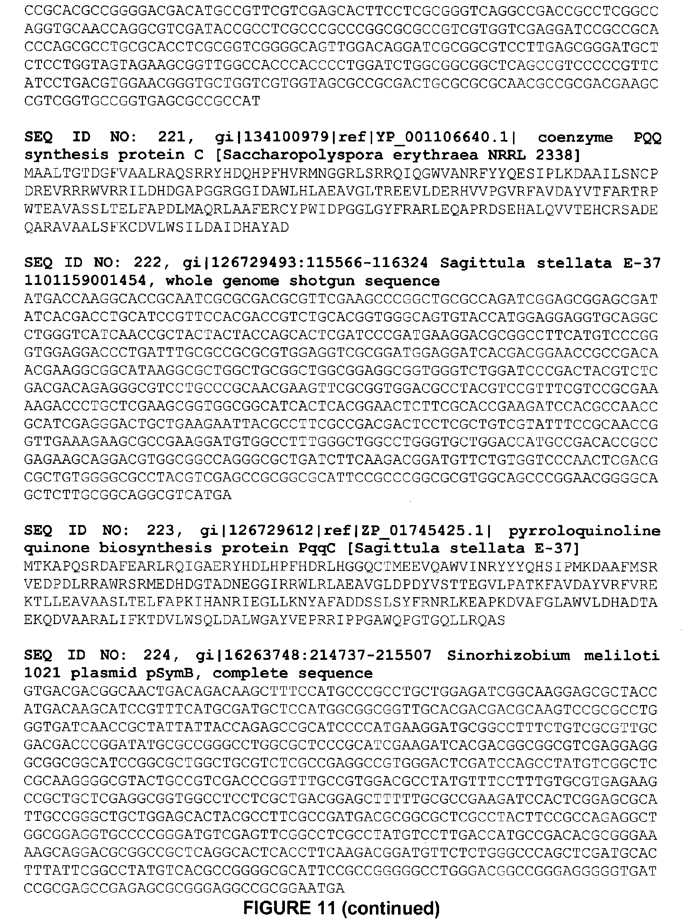

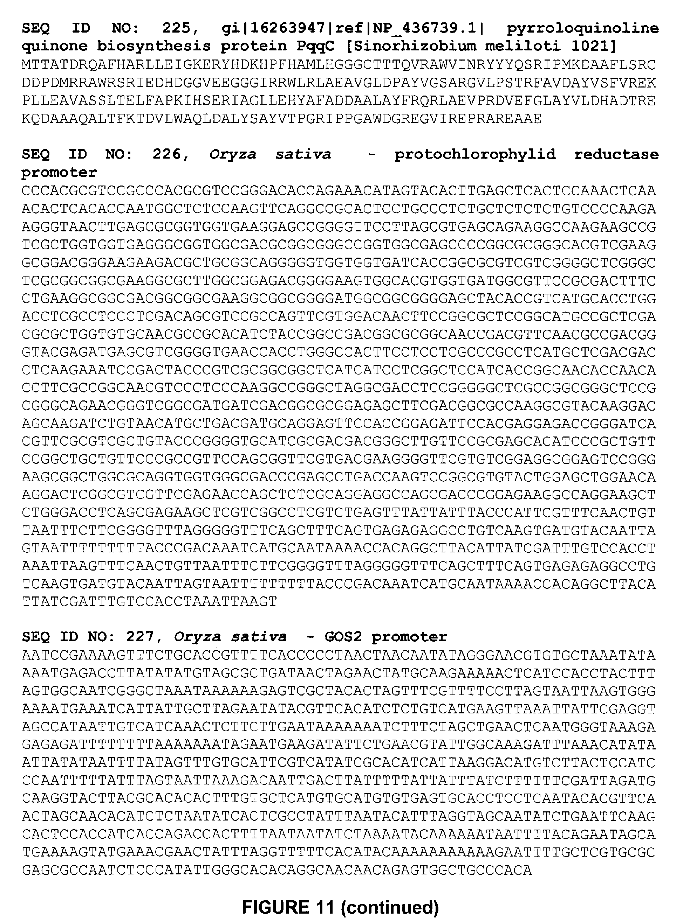

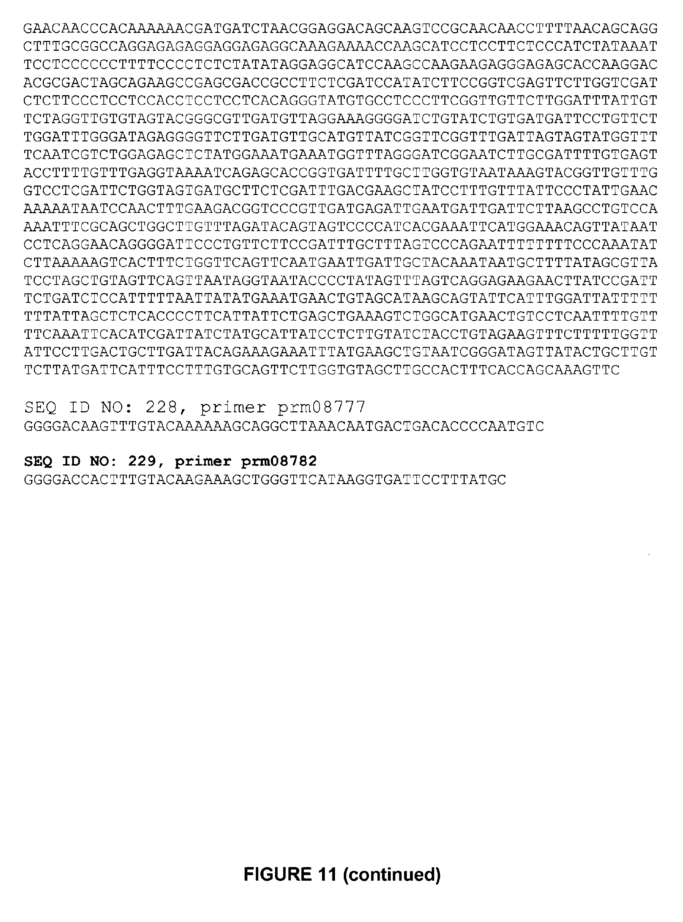

Fig. 11 details examples of sequences useful in performing the methods according to the present invention.

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### Example 1: Identification of sequences related to the nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to the nucleic acid sequence used in the methods of the present invention.

Table A1: Examples of PEPC polypeptides:

| Source | Nucleic acid SEQ ID NO: | Protein SEQ ID NO: |
|---|---|---|
| see Figure 4 | see Figure 4 | see Figure 4 |

Table A2: Examples of Class III U-box polypeptides:

| Plant Source | Nucleic acid SEQ ID NO: | Protein SEQ ID NO: |
|---|---|---|
| Capsicum annuum | 74 | 75 |
| Arabidopsis thaliana | 83 | 84 |
| Arabidopsis thaliana | 85 | 86 |
| Medicago truncatula | 87 | 88 |
| Nicotiana benthamiana | 89 | 90 |
| Oryza sativa | 91 | 92 |
| Oryza sativa | 93 | 94 |
| Oryza sativa | 95 | 96 |
| Oryza sativa | 97 | 98 |

(continued)

| Plant Source | Nucleic acid SEQ ID NO: | Protein SEQ ID NO: |
|---|---|---|
| Oryza sativa | 99 | 100 |

Table A3: Examples of PQQC polypeptides:

| Source | Nucleic acid SEQ ID NO: | Protein SEQ ID NO: |
|---|---|---|
| see Figure 11 | see Figure 11 | see Figure 11 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

Given below is the output of a BLASTP showing further PEPC sequences related to SEQ ID NO: 2 and therefore useful in the methods of the invention.

1: NP_441713
phosphoenolpyruvate carboxylase [Synechocystis sp. PCC 6803] gi|16330985|ref|NP_441713.1|[16330985]

2:ZP_01729388
phosphoenolpyruvate carboxylase [Cyanothece sp. CCY0110] gil|26658238|ref|ZP_01729388.1|[126658238]

3:ZP_00517310
Phosphoenolpyruvate carboxylase [Crocosphaera watsonii WH 8501] gi|67923850|ref|ZP_00517310.1|[67923850]

4:ZP_00108354
COG2352: Phosphoenolpyruvate carboxylase [Nostoc punctiforme PCC 73102] gi|23126460|ref|ZP_00108354.1|[23126460]

5:ZP_01631340
phosphoenolpyruvate carboxylase [Nodularia spumigena CCY9414] gi|119512252|ref|ZP-01631340.1|[119512252]

6: YP_322649
phosphoenolpyruvate carboxylase [Anabaena variabilis ATCC 29413] gi|75908353|ref|YP_322649.1|[75908353]

7: AAV90837
phosphoenolpyruvate carboxylase [Synechococcus sp. PCC 7002] gi|56417897|gb|AAV90837.1|[56417897]

8:ZP_01620914
Phosphoenolpyruvate carboxylase [Lyngbya sp. PCC 8106] gi|119487042|ref|ZP_01620914.1|[119487042]

9:P28594
Phosphoenolpyruvate carboxylase (PEPCase) (PEPC) gi|25014052|sp|P28594|CAPP_ANASP[25014052]

10:YP_172556
phosphoenolpyruvate carboxylase [Synechococcus elongatus PCC 6301] gi|56751855|ref|YP_172556.1|[56751855]

11:YP_401269
Phosphoenolpyruvate carboxylase [Synechococcus elongatus PCC 7942] gi|81301061|ref|YP_401269.1|[813010611]

12:YP_720722
Phosphoenolpyruvate carboxylase [Trichodesmium erythraeum IMS101] gil|13474661|ref|YP_720722.1|[113474661]

13: NP_682702
phosphoenolpyruvate carboxylase [Thermosynechococcus elongatus BP-1] gi|22299455|ref|NP_682702.1|[22299455]

14: NP_488901
phosphoenolpyruvate carboxylase [Nostoc sp. PCC 7120] gi|17232353|ref|NP_488901.1|[17232353]

15:YP_478060
phosphoenolpyruvate carboxylase [Synechococcus sp. JA-2-3B'a(2-13)] gi|86609298|ref|YP_478060.1|[86609298]

16:ZP_01083776
phosphoenolpyruvate carboxylase [Synechococcus sp. WH 5701] gi|87300934|ref|ZP_01083776.1|[87300934]

17:ZP_01080788
Phosphoenolpyruvate carboxylase [Synechococcus sp. RS9917] gi|87124941|ref|ZP_01080788.1|[87124941]

18:YP_474305
phosphoenolpyruvate carboxylase [Synechococcus sp. JA-3-3Ab] gi|86605542|ref|YP_474305.1|[86605542]

19:ZP_01469854
phosphoenolpyruvate carboxylase [Synechococcus sp. BL107] gi|116072588|ref|ZP_01469854.1|[116072588]

20: NP_876121
phosphoenolpyruvate carboxylase [Prochlorococcus marinus subsp. marinus str. CCMP1375] gi|33241179|ref|NP_876121.1|[33241179]

21:ZP_01473048
phosphoenolpyruvate carboxylase [Synechococcus sp. RS9916] gi|116075789|ref|ZP_01473048.1|[116075789]

22:ZP_01006468
phosphoenolpyruvate carboxylase [Prochlorococcus marinus str. MIT 9211] gi|84519119|ref|ZP_01006468.1|[84519119]

23:ABE11140
phosphoenolpyruvate carboxylase [uncultured Prochlorococcus marinus clone HF10-11H11] gi|91070220|gb|ABE11140.1|[91070220]

24:YP_001091990
Phosphoenolpyruvate carboxylase [Prochlorococcus marinus str. MIT 9301] gi|126697104|ref|YP_001091990.1|[126697104]

25: YP_377934
phosphoenolpyruvate carboxylase [Synechococcus sp. CC9902] gi|78185499|ref|YP_377934.1|[78185499]

26:YP_398164
Phosphoenolpyruvate carboxylase [Prochlorococcus marinus str. MIT 9312] gi|78780052|ref|YP_398164.1|[78780052]

27:YP_292340
phosphoenolpyruvate carboxylase [Prochlorococcus marinus str. NATL2A] gi|72382985|ref|YP_292340.1|[72382985]

28:YP_001010172
Phosphoenolpyruvate carboxylase [Prochlorococcus marinus str. AS9601] gi|123969314|ref|YP_001010172.1|[123969314]

29: NP_895540
phosphoenolpyruvate carboxylase [Prochlorococcus marinus str. MIT 9313] gi|33863980|ref|NP_895540.1|[33863980]

30:YP_001015841
Phosphoenolpyruvate carboxylase [Prochlorococcus marinus str. NATL1A] gi|124026726|ref|YP_001015841.1|[124026726]

31:YP_001018274
Phosphoenolpyruvate carboxylase [Prochlorococcus marinus str. MIT 9303] gi|124023967|ref|YP_001018274.1|[124023967]

32:ZP_01123374
phosphoenolpyruvate carboxylase [Synechococcus sp. WH 7805] gi|88807863|ref|ZP_01123374.1|[88807863]

33:YP_001224177
Phosphoenolpyruvate carboxylase [Synechococcus sp. WH 7803] gi|148238790|ref|YP_001224177.1|[148238790]

34: NP_898138
phosphoenolpyruvate carboxylase [Synechococcus sp. WH 8102] gi|33866579|ref|NP_898138.1|[33866579]

35: YP_380727
phosphoenolpyruvate carboxylase [Synechococcus sp. CC9605] gi|78211948|ref|YP_380727.1|[78211948]

36: NP_893692
phosphoenolpyruvate carboxylase [Prochlorococcus marinus subsp. pastoris str. CCMP1986] gi|33862131|ref|NP_893692.1|[33862131]

37:YP_001012076
Phosphoenolpyruvate carboxylase [Prochlorococcus marinus str. MIT 9515] gi|123966995|ref|YP_001012076.1|[123966995]

38: YP_001228346
Phosphoenolpyruvate carboxylase [Synechococcus sp. RCC307] gi|148243189|ref|YP_001228346.1|[148243189]

39: YP_729688
phosphoenolpyruvate carboxylase [Synechococcus sp. CC9311] gi|113954224|ref|YP_729688.1|[113954224]

40: NP_923360
phosphoenolpyruvate carboxylase [Gloeobacter violaceus PCC 7421] gi|37519983|ref|NP_923360.1|[37519983]

41:ZP_01424999
Phosphoenolpyruvate carboxylase [Herpetosiphon aurantiacus ATCC 23779] gi|113939140|ref|ZP_01424999.1|[113939140]

42:YP_830113
Phosphoenolpyruvate carboxylase [Arthrobacter sp. FB24] gi|116669180|ref|YP_830113.1|[116669180]

43:YP_946565
phosphoenolpyruvate carboxylase [Arthrobacter aurescens TC1] gi|119962288|ref|YP_946565.1|[119962288]

44:ZP_01696337
phosphoenolpyruvate carboxylase [Bacillus coagulans 36D1] gi|124521401|ref|ZP_01696337.1|[124521401]

45:YP_342813
Phosphoenolpyruvate carboxylase [Nitrosococcus oceani ATCC 19707] gi|77164288|ref|YP_342813.1|[77164288]

46: NP_627344
phosphoenolpyruvate carboxylase [Streptomyces coelicolor A3(2)] gi|21221565|ref|NP_627344.1|[21221565]

47: AAY89988
predicted PEP carboxylase [uncultured bacterium BAC13K9BAC] gi|68304977|gb|AAY89988.1|[68304977]

48: NP_824743
phosphoenolpyruvate carboxylase [Streptomyces avermitilis MA-4680] gi|29830109|ref|NP_824743.1|[29830109]

49:ZP_01649489
Phosphoenolpyruvate carboxylase [Salinispora arenicola CNS205] gi|119883229|ref|ZP-01649489.1|[119883229]

50:ZP_00568542
Phosphoenolpyruvate carboxylase [Frankia sp. EAN1 pec] gi|68229346|ref|ZP_00568542.1|[68229346]

51:YP_001363100
Phosphoenolpyruvate carboxylase [Kineococcus radiotolerans SRS30216] gi|152967316|ref|YP_001363100.1|[152967316]

52:YP_001157738
Phosphoenolpyruvate carboxylase [Salinispora tropica CNB-440] gi|145593441|ref|YP_001157738.1|[145593441]

53:YP_318884
phosphoenolpyruvate carboxylase [Nitrobacter winogradskyi Nb-255] gi|75676463|ref|YP_318884.1|[75676463]

54: YP_294925
phosphoenolpyruvate carboxylase [Ralstonia eutropha JMP134] gi|73540405|ref|YP_294925.1|[73540405]

55: YP_577930
Phosphoenolpyruvate carboxylase [Nitrobacter hamburgensis X14] gi|92118201|ref|YP_577930.1|[92118201]

56:ZP_02010108
Phosphoenolpyruvate carboxylase [Ralstonia pickettii 12D] gi|153888973|ref|ZP_02010108.1|[153888973]

57: NP_840673
Phosphoenolpyruvate carboxylase [Nitrosomonas europaea ATCC 19718] gi|30248603|ref|NP_840673.1|[30248603]

58: NP_947117
phosphoenolpyruvate carboxylase [Rhodopseudomonas palustris CGA009] gi|39934841|ref|NP_947117.1|[39934841]

59:YP_391787
Phosphoenolpyruvate carboxylase [Thiomicrospira crunogena XCL-2] gi|78485862|ref|YP_391787.1|[78485862]

60:ZP_01661147
Phosphoenolpyruvate carboxylase [Ralstonia pickettii 12J] gi|121528533|ref|ZP_01661147.1|[121528533]

61: YP_001204637
phosphoenolpyruvate carboxylase [Bradyrhizobium sp. ORS278] gi|146339589|ref|YP_001204637.1|[146339589]

62: YP_932496
probable phosphoenolpyruvate carboxylase [Azoarcus sp. BH72] gi|119897283|ref|YP_932496.1|[119897283]

63: YP_970358
Phosphoenolpyruvate carboxylase [Acidovorax avenae subsp. citrulli AAC00-1] gi|120610680|ref|YP_970358.1|[120610680]

64:ZP_01047635
phosphoenolpyruvate carboxylase [Nitrobacter sp. Nb-311A] gi|85716666|ref|ZP_01047635.1|[85716666]

65: YP_369991
phosphoenolpyruvate carboxylase [Burkholderia sp. 383] gi|78067222|ref|YP_369991.1|[78067222]

66: YP_314388
phosphoenolpyruvate carboxylase [Thiobacillus denitrificans ATCC 25259] gi|74316648|ref|YP_314388.1|[74316648]

67: NP_520479
phosphoenolpyruvate carboxylase [Ralstonia solanacearum GMI1000] gi|17547077|ref|NP_520479.1|[17547077]

68: YP_727365
phosphoenolpyruvate carboxylase [Ralstonia eutropha H16] gi|113868876|ref|YP_727365.1|[113868876]

69:YP_001238961
phosphoenolpyruvate carboxylase [Bradyrhizobium sp. BTAi1] gi|148254376|ref|YP_001238961.1|[148254376]

70:YP_001415233
Phosphoenolpyruvate carboxylase [Xanthobacter autotrophicus Py2] gi|154244275|ref|YP_001415233.1|[154244275]

71:YP_487198
Phosphoenolpyruvate carboxylase [Rhodopseudomonas palustris HaA2] gi|86750702|ref|YP_487198.1|[86750702]

72: NP_769595
phosphoenolpyruvate carboxylase [Bradyrhizobium japonicum USDA 110] gi|27378066|ref|NP_769595.1|[27378066]

73:YP_569012
Phosphoenolpyruvate carboxylase [Rhodopseudomonas palustris BisB5] gi|91976353|ref|YP_569012.1|[91976353]

74:ZP_00946208
Phosphoenolpyruvate carboxylase [Ralstonia solanacearum UW551] gi|83749206|ref|ZP_00946208.1|[83749206]

75:YP_780719
Phosphoenolpyruvate carboxylase [Rhodopseudomonas palustris BisA53] gi|115523808|ref|YP_780719.1|[115523808]

76: BAA21835
phosphoenolpyruvate carboxylase [Rhodopseudomonas palustris] gi|2339975|dbj|BAA21835.1|[2339975]

77: Q59757
Phosphoenolpyruvate carboxylase (PEPCase) (PEPC) gi|2499464|sp|Q59757|CAPP_RHOMR[2499464]

78:YP_747257
Phosphoenolpyruvate carboxylase [Nitrosomonas eutropha C91] gi|114331035|ref|YP_747257.1|[114331035]

79:ZP_01554849
Phosphoenolpyruvate carboxylase [Burkholderia ambifaria MC40-6] gi|118696768|ref|ZP_01554849.1|[118696768]

80: YP_774359
Phosphoenolpyruvate carboxylase [Burkholderia cepacia AMMD] gi|115352520|ref|YP_774359.1|[115352520]

81: EAY68193
Phosphoenolpyruvate carboxylase [Burkholderia dolosa AUO158] gi|124894313|gb|EAY68193.1|[124894313]

82:YP_621690
Phosphoenolpyruvate carboxylase [Burkholderia cenocepacia AU 1054] gi|107023363|ref|YP_621690.1|[107023363]

83: YP_836069
Phosphoenolpyruvate carboxylase [Burkholderia cenocepacia H12424] gi|116690446|ref|YP_836069.1|[116690446]

84:ZP_00987174
COG2352: Phosphoenolpyruvate carboxylase [Burkholderia dolosa AUO158] gi|84362555|ref|ZP_00987174.1|[84362555]

85:ZP_01559873
Phosphoenolpyruvate carboxylase [Burkholderia cenocepacia MC0-3] gi|118707287|ref|ZP_01559873.1|[118707287]

86: YP_584892
phosphoenolpyruvate carboxylase [Ralstonia metallidurans CH34] gi|94311682|ref|YP_584892.1|[94311682]

87:YP_987106
Phosphoenolpyruvate carboxylase [Acidovorax sp. JS42] gi|121595210|ref|YP_987106.1|[121595210]

88: ZP_00980295
COG2352: Phosphoenolpyruvate carboxylase [Burkholderia cenocepacia PC184] gi|84355411|ref|ZP_00980295.1|[84355411]

89: EAY64002
Phosphoenolpyruvate carboxylase [Burkholderia cenocepacia PC184] gi|124872286|gb|EAY64002.1|[124872286]

90:YP_531562
Phosphoenolpyruvate carboxylase [Rhodopseudomonas palustris BisB18] gi|90423192|ref|YP_531562.1|[90423192]

91:YP_710875
Phosphoenolpyruvate carboxylase (PEPCase) (PEPC) [Frankia alni ACN14a] gi|111220081|ref|YP_710875.1|[111220081]

92:ZP_01325009
hypothetical protein BpseP_03001239 [Burkholderia pseudomallei Pasteur] gi|100063478|ref|ZP_01325009.1|[100063478]

93: YP_995835
Phosphoenolpyruvate carboxylase [Verminephrobacter eiseniae EF01-2] gi|121608028|ref|YP_995835.1|[121608028]

94: CAM76812
Phosphoenolpyruvate carboxylase [Magnetospirillum gryphiswaldense MSR-1] gi|144899948|emb|CAM76812.1|[144899948]

95:ZP_01317342
hypothetical protein Bpse1_03003262 [Burkholderia pseudomallei 1655] gi|99910315|ref|ZP_01317342.1|[99910315]

96:ZP_01213561
hypothetical protein Bpse17_02001265 [Burkholderia pseudomallei 1710a] gi|90293964|ref|ZP_01213561.1|[90293964]

97:YP_001065355

phosphoenolpyruvate carboxylase [Burkholderia pseudomallei 1106a] gi|126451534|ref|YP_001065355.1|[126451534]

98: YP_107641
phosphoenolpyruvate carboxylase [Burkholderia pseudomallei K96243] gi|53718655|ref|YP_107641.1|[53718655]

99:YP_707103
phosphoenolpyruvate carboxylase [Rhodococcus sp. RHA1]
gi|111024131|ref|YP_707103.1|[111024131]

100:YP_001058119
phosphoenolpyruvate carboxylase [Burkholderia pseudomallei 668] gi|126440608|ref|YP_001058119.1|[126440608]

Example 2: Alignment of PEPC polypeptide sequences

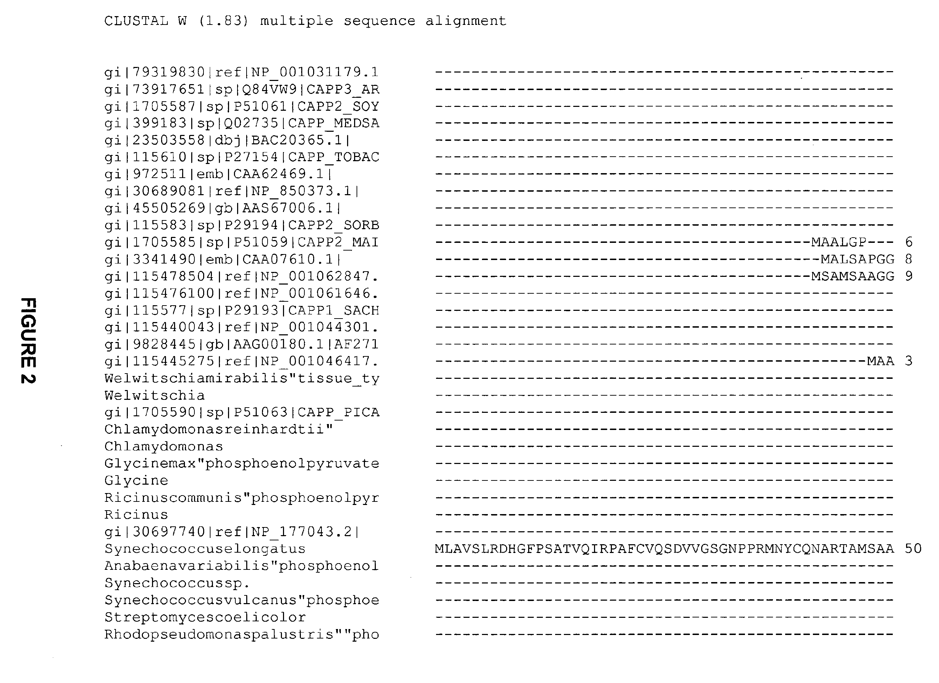

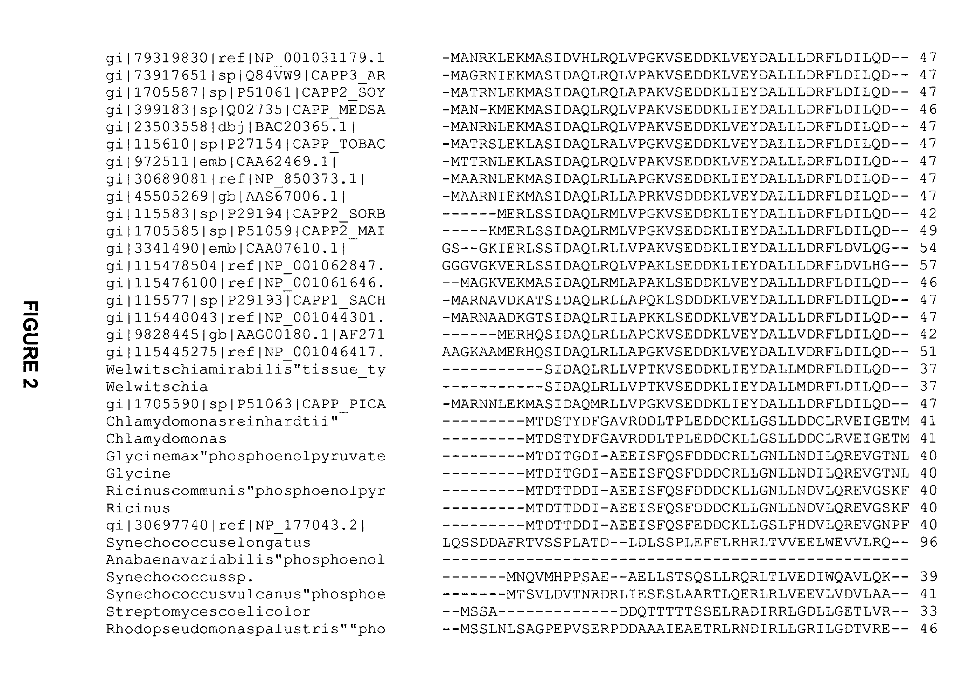

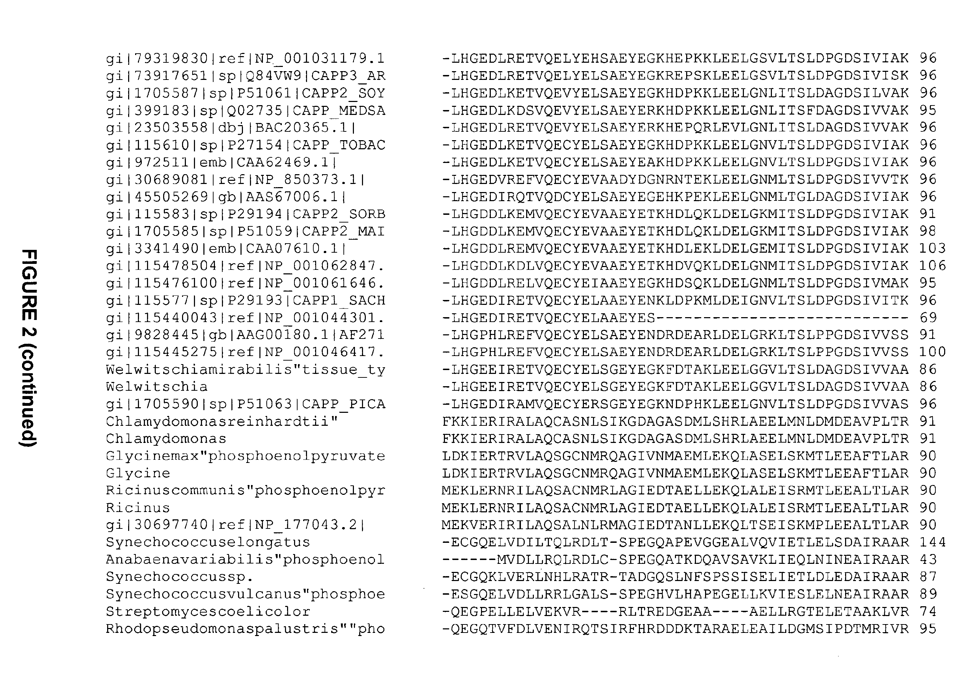

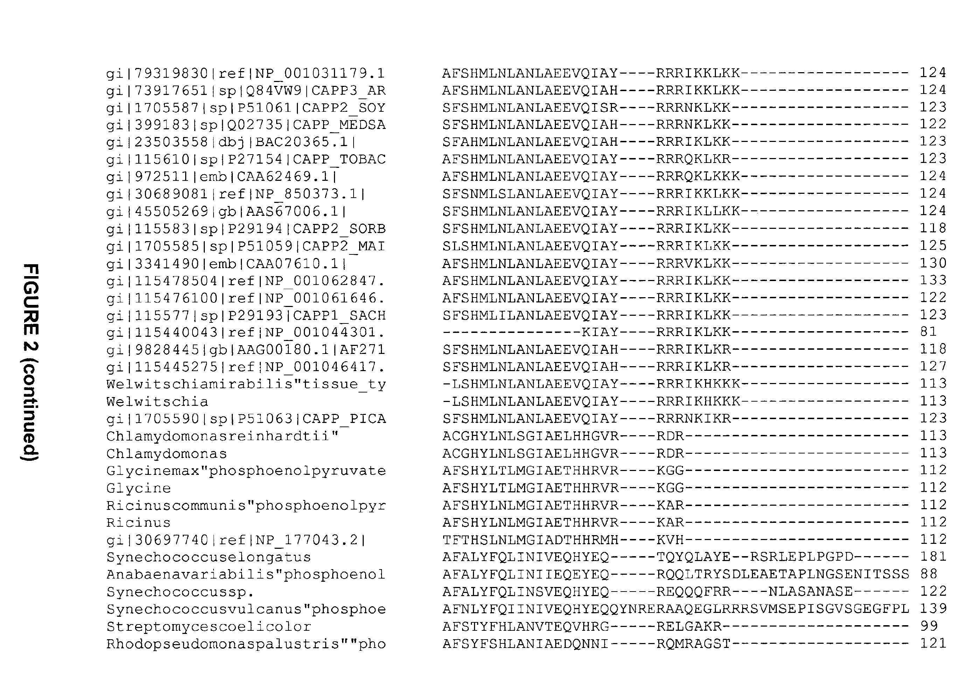

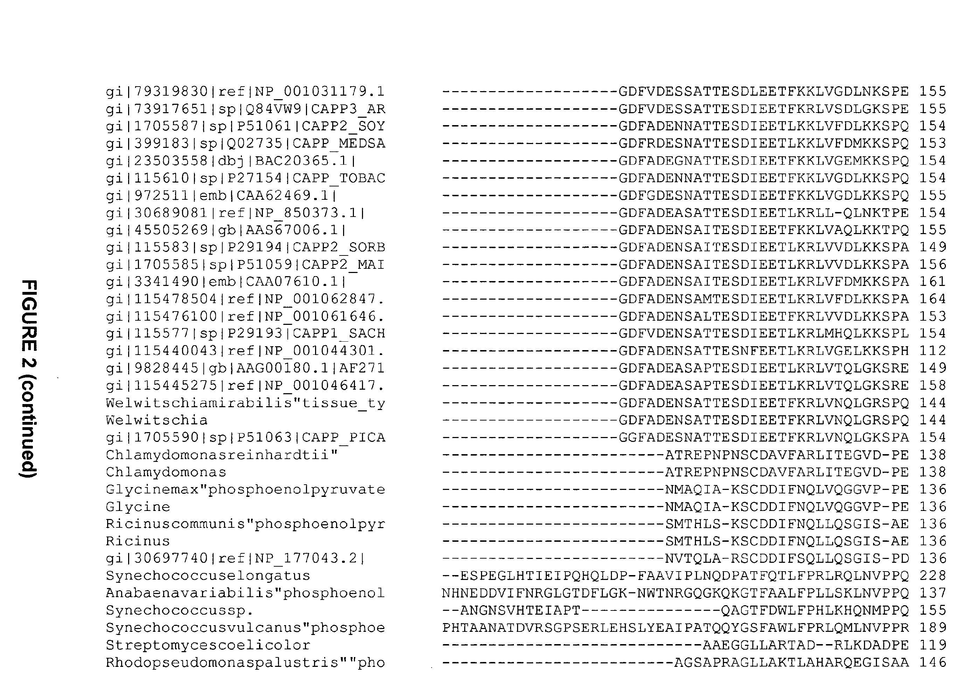

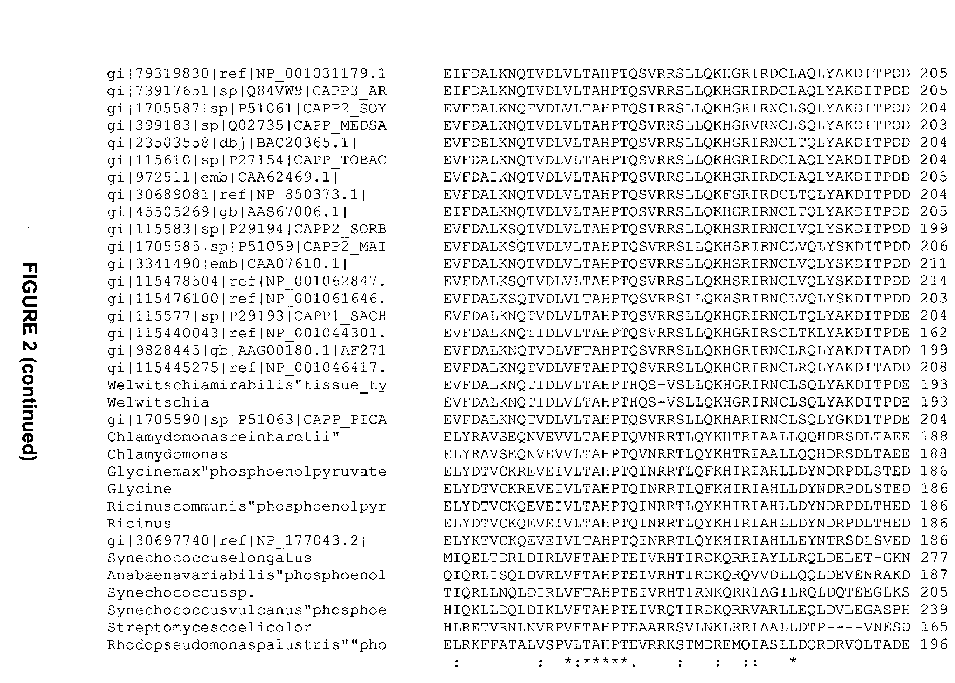

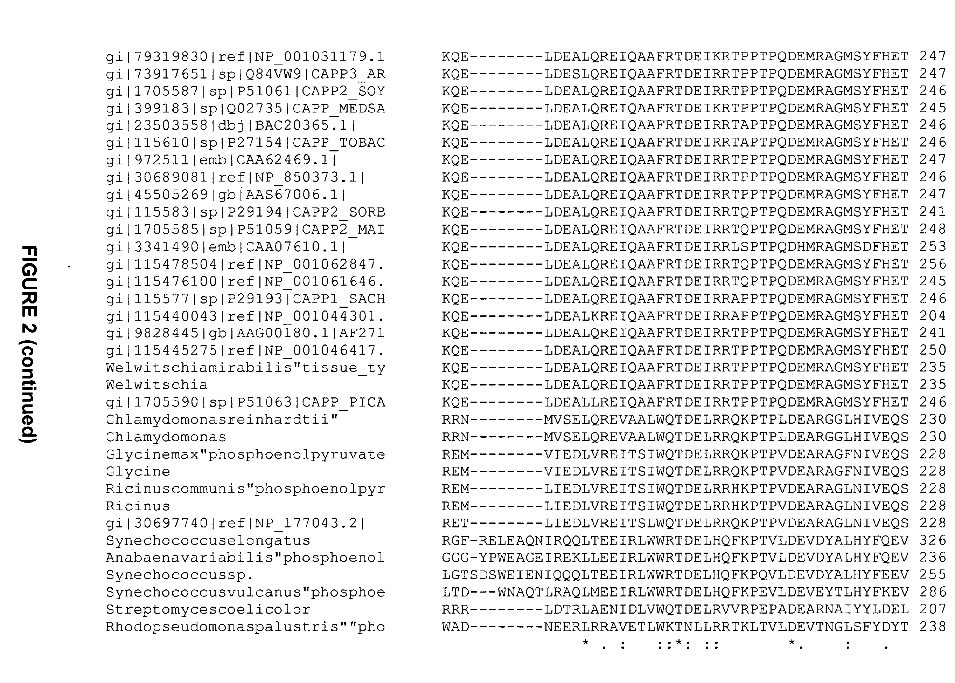

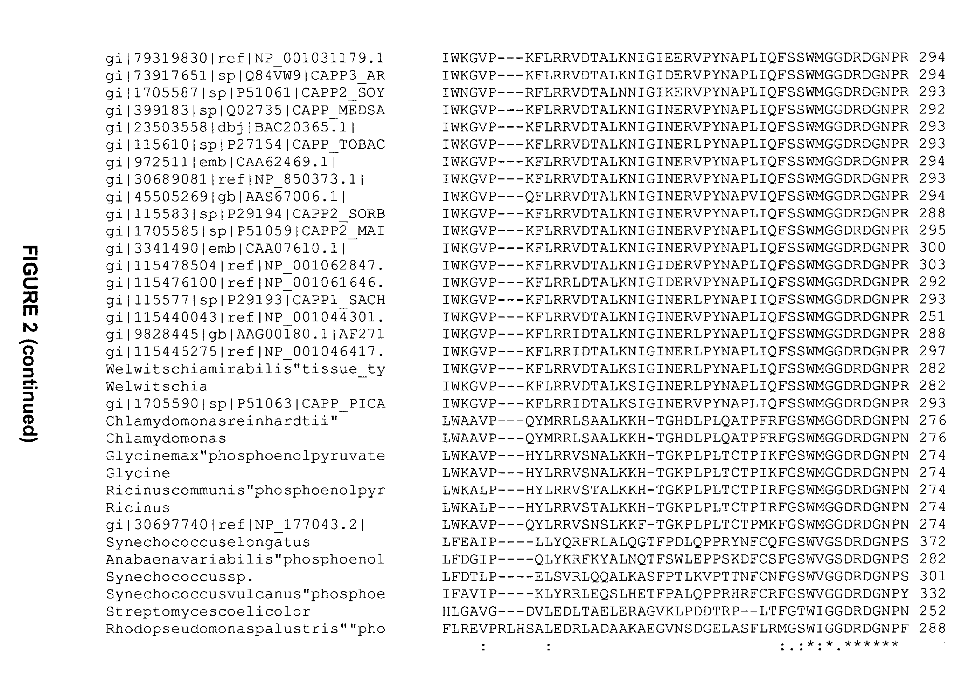

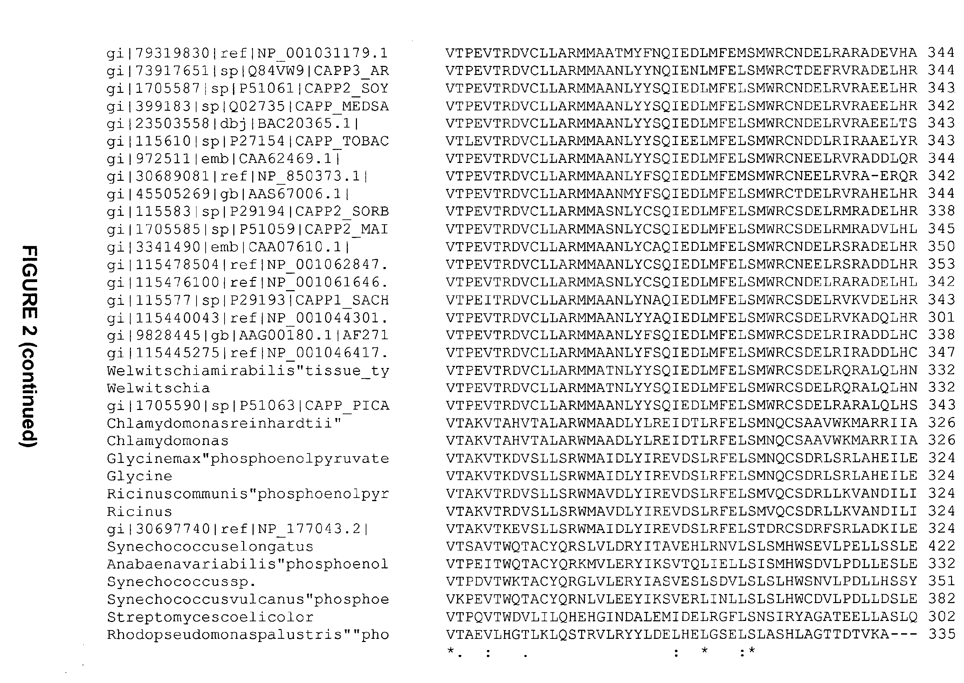

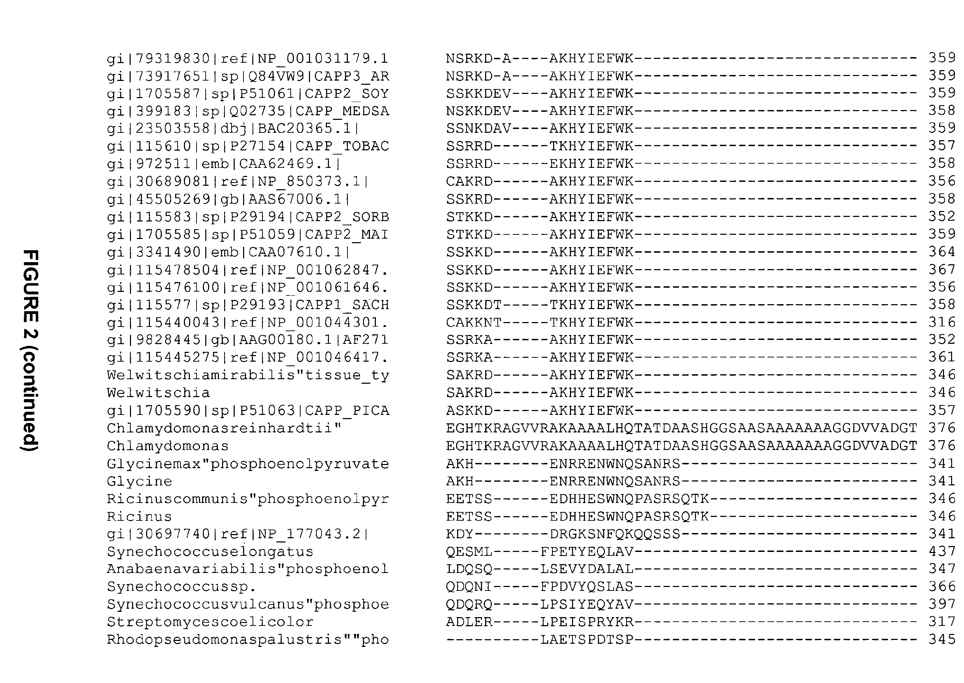

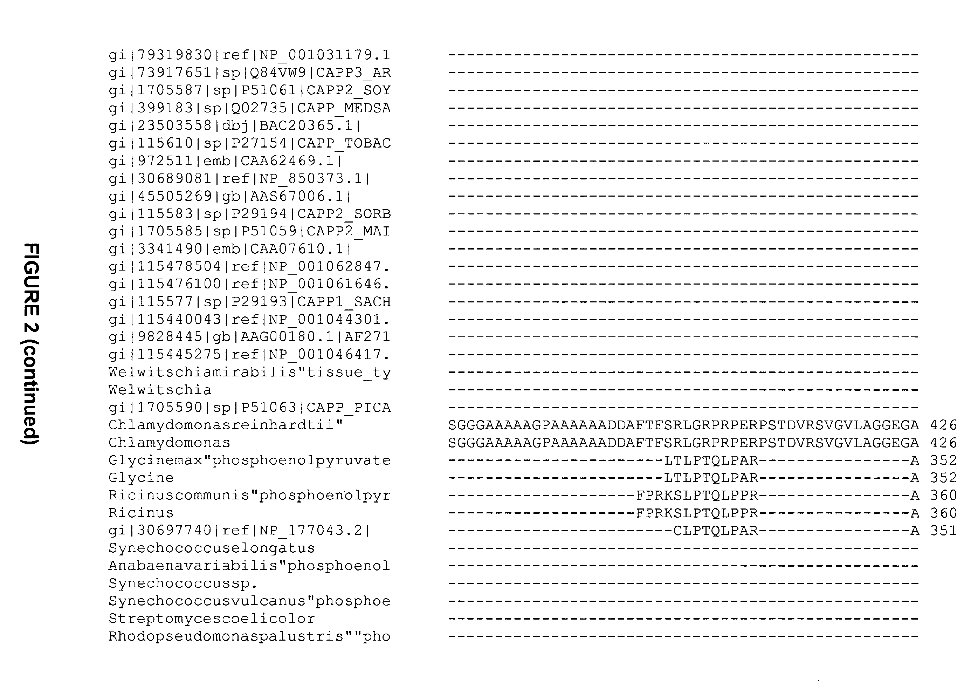

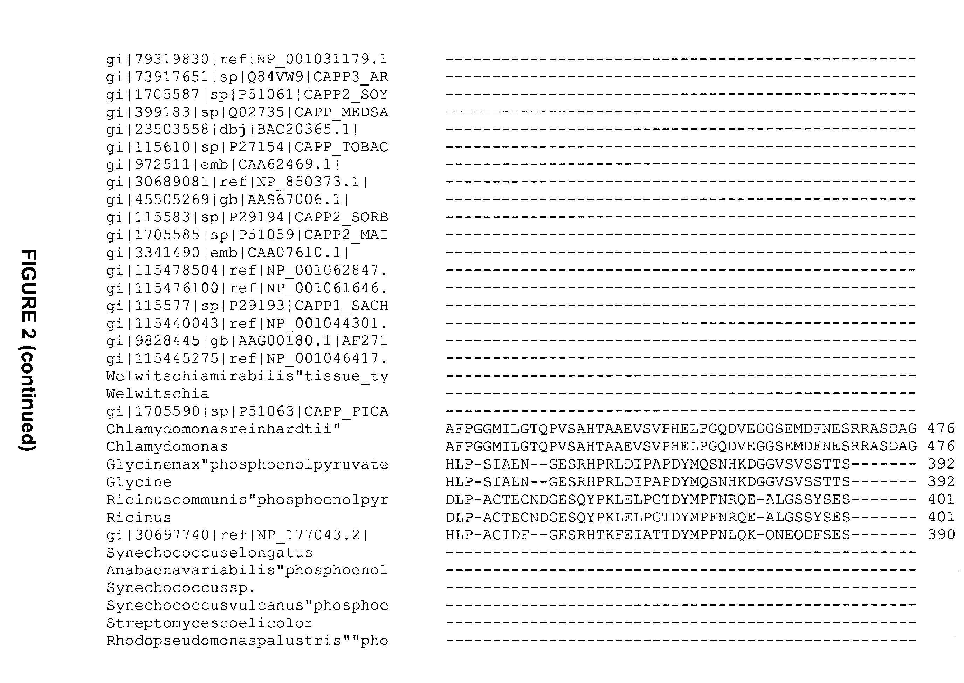

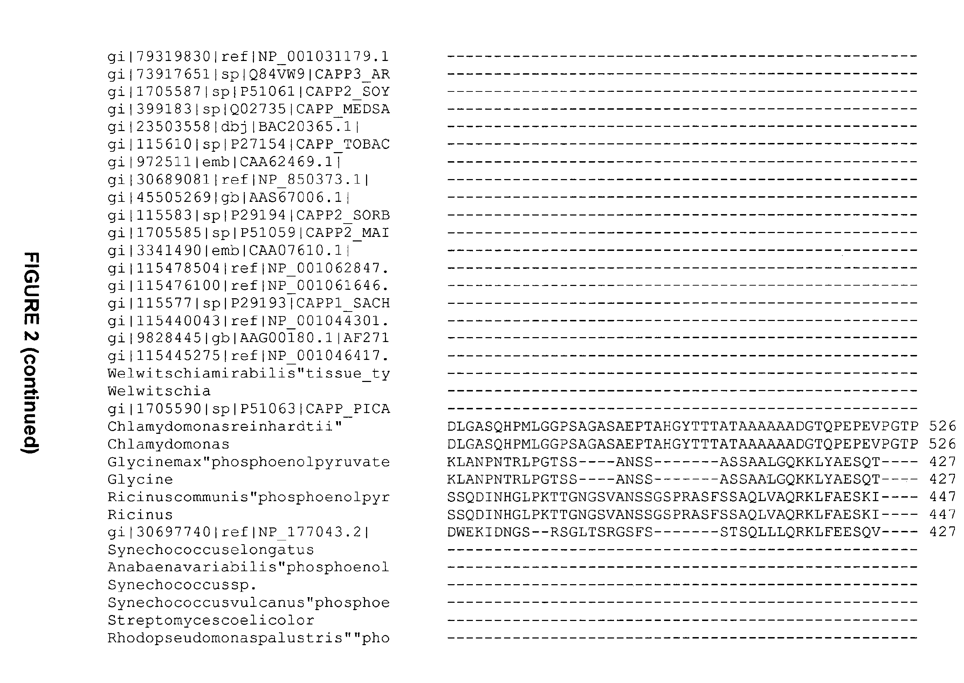

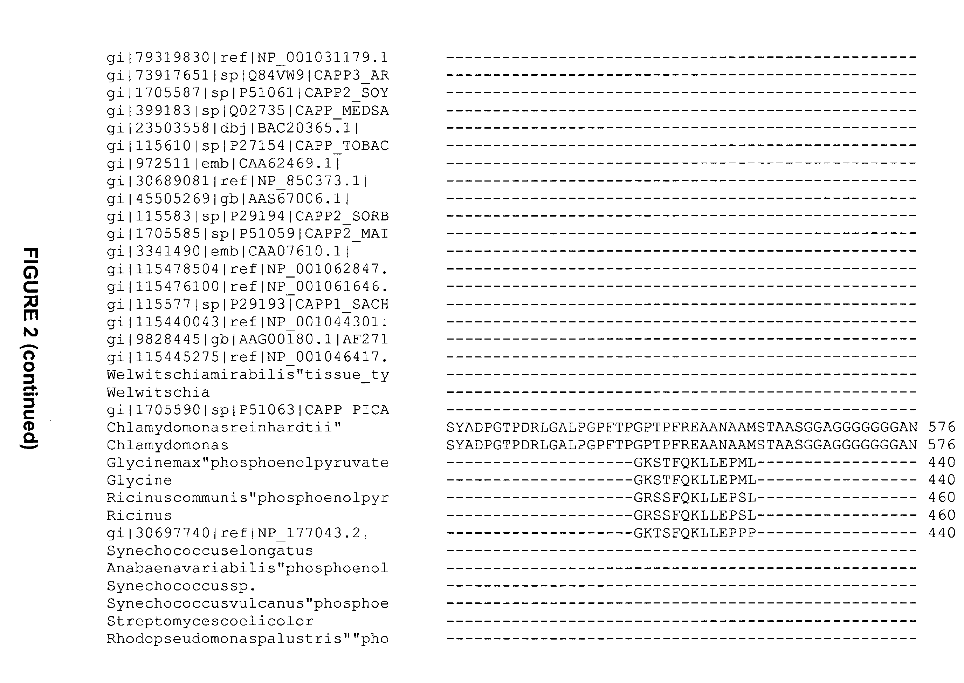

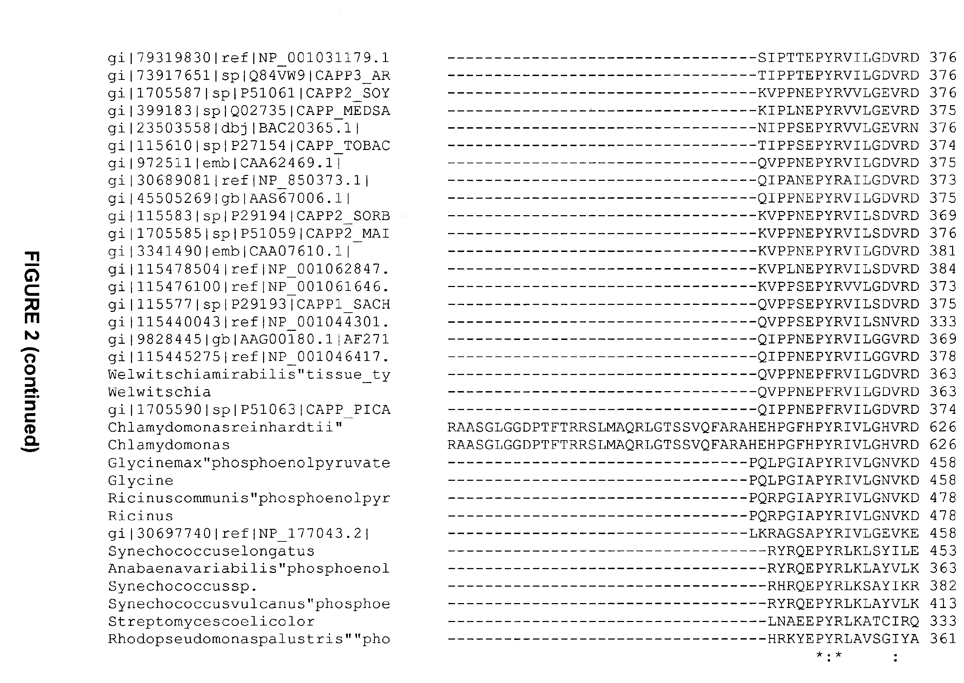

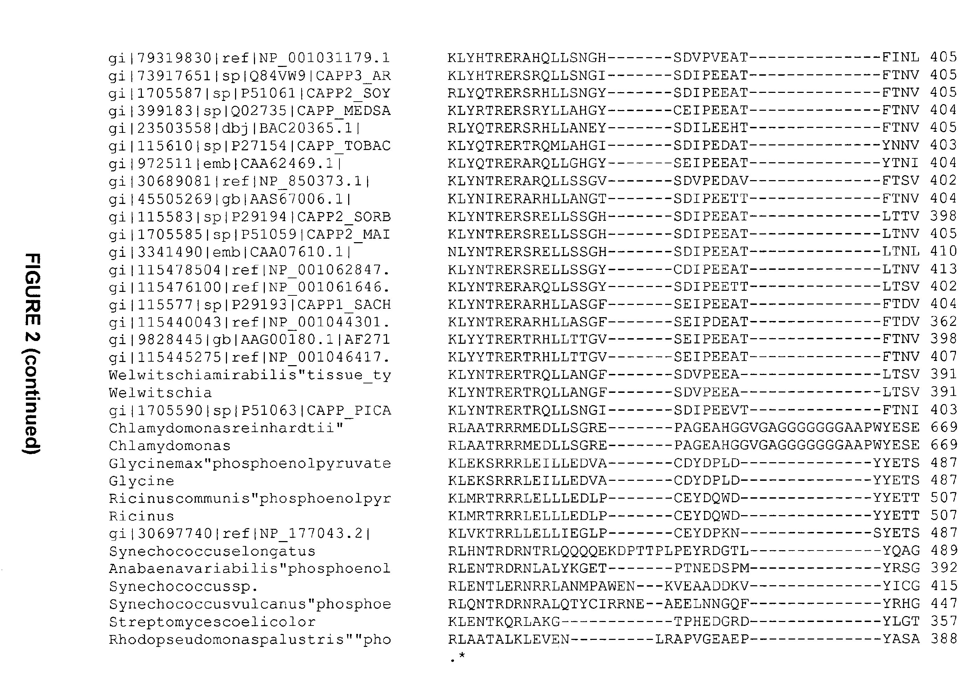

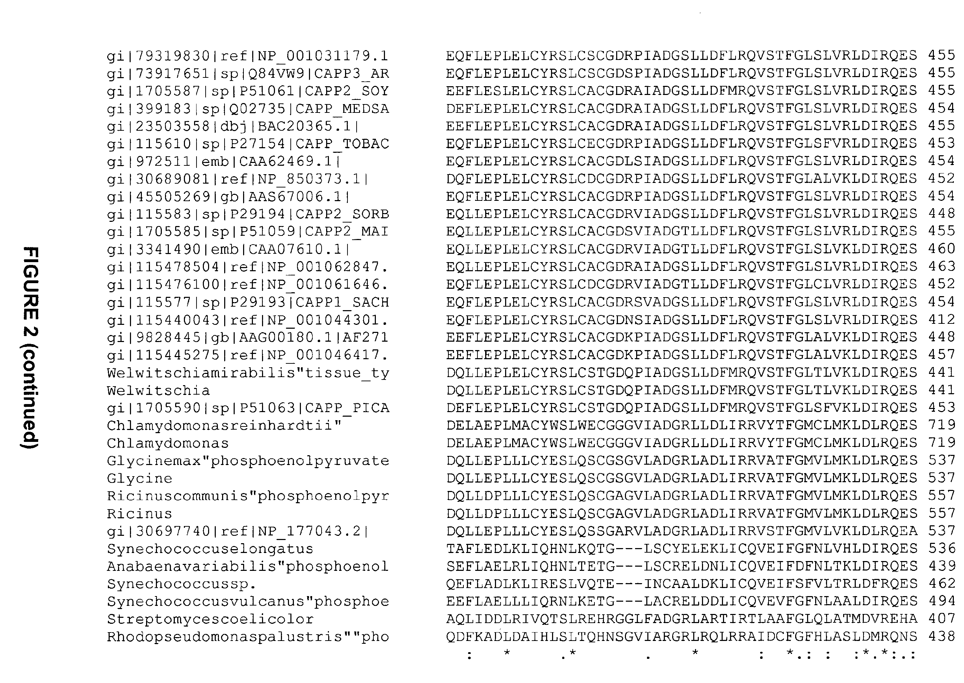

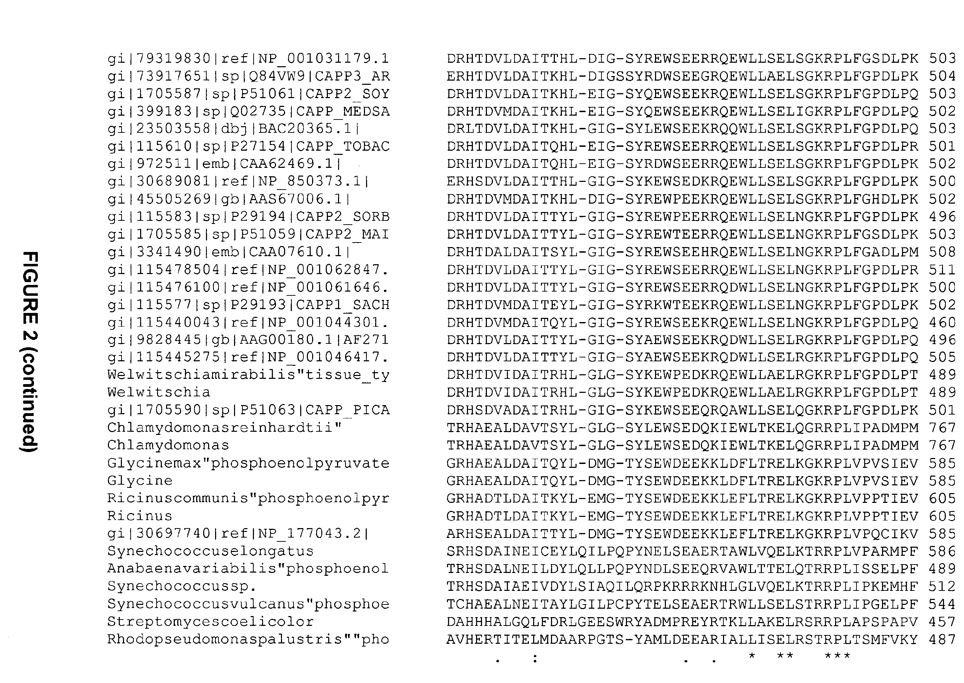

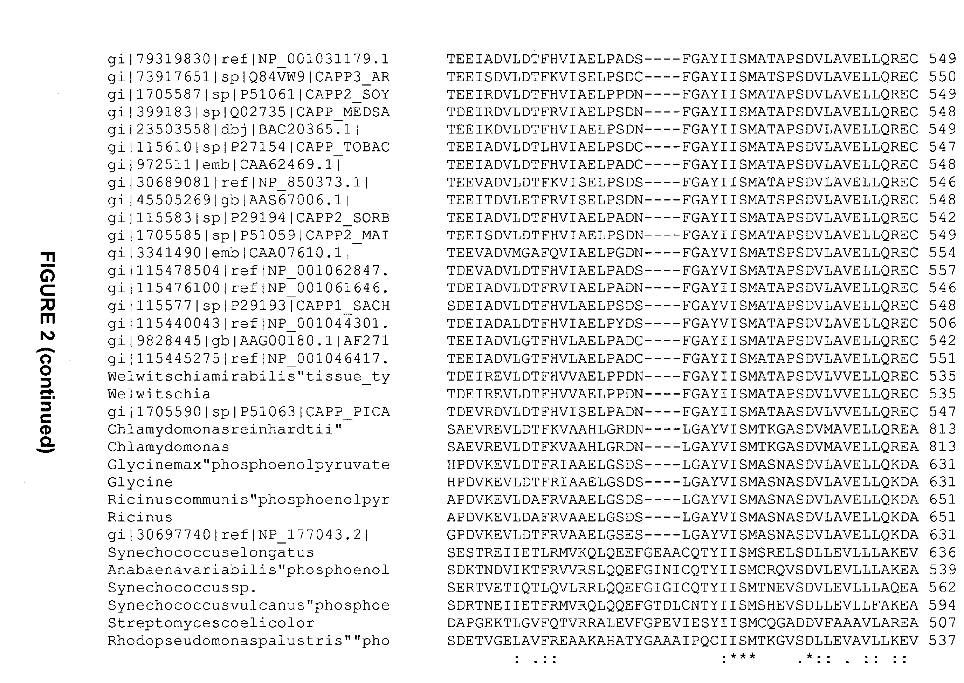

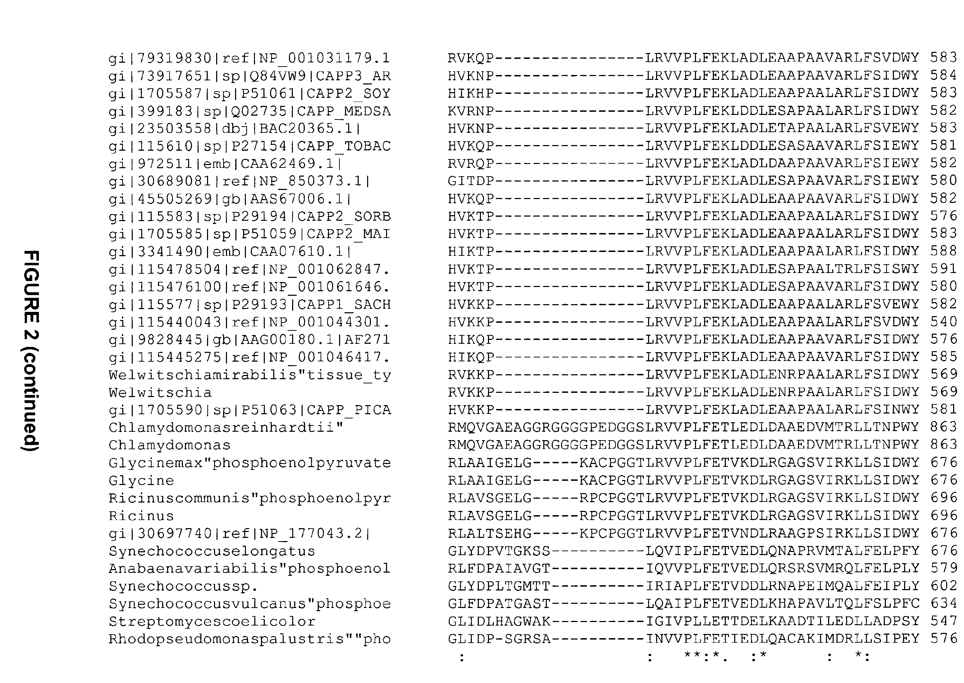

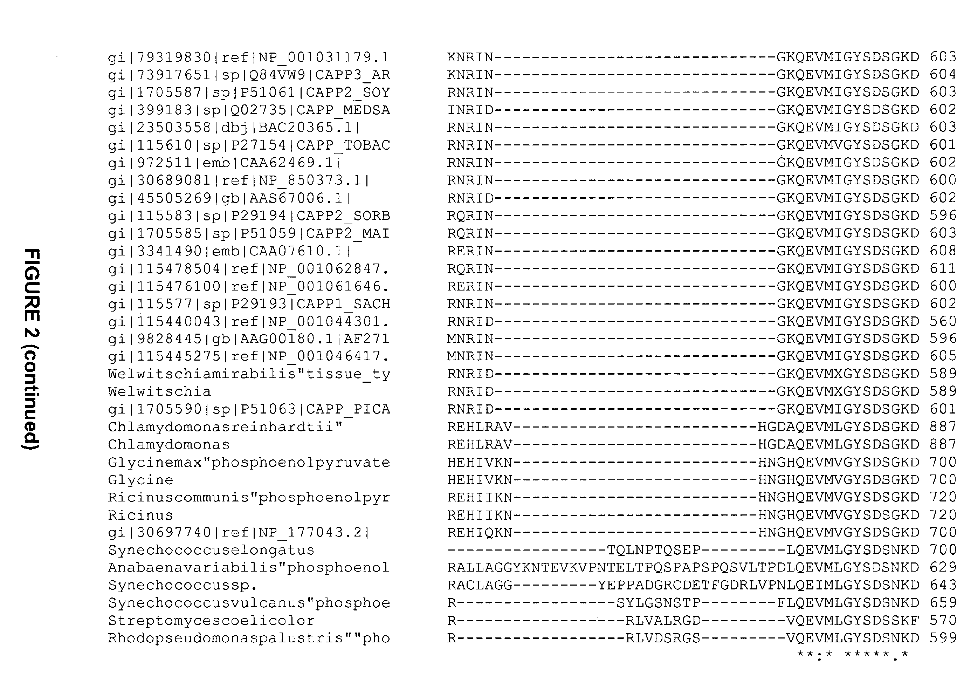

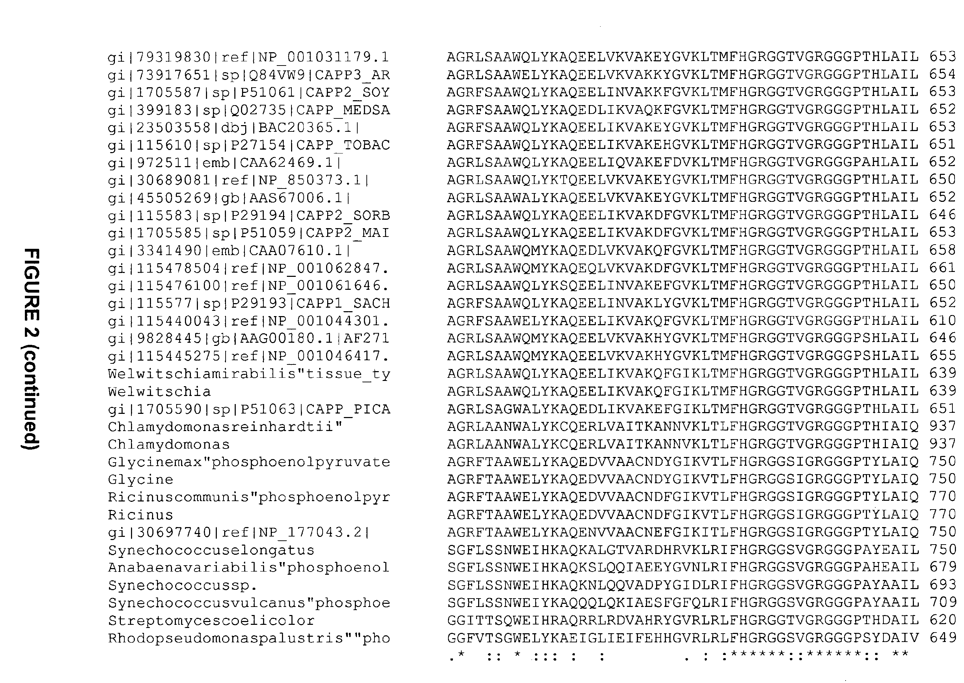

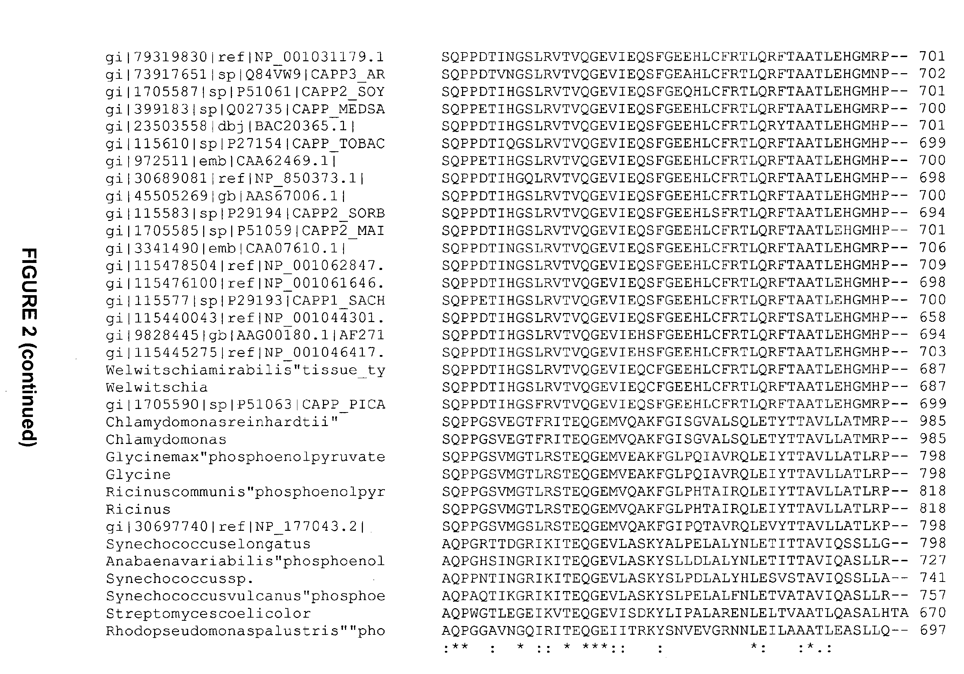

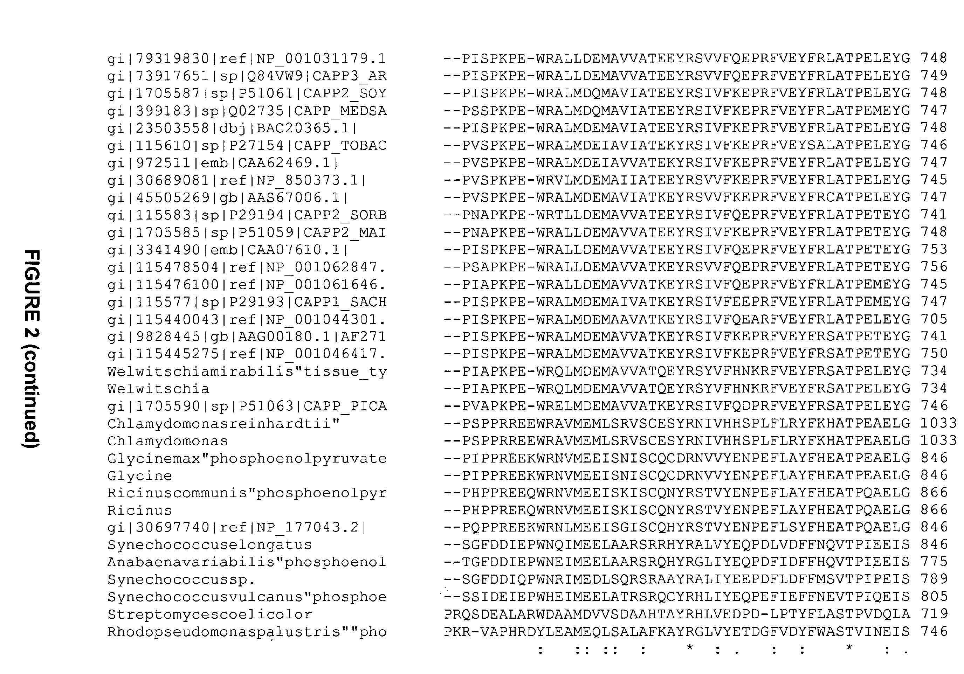

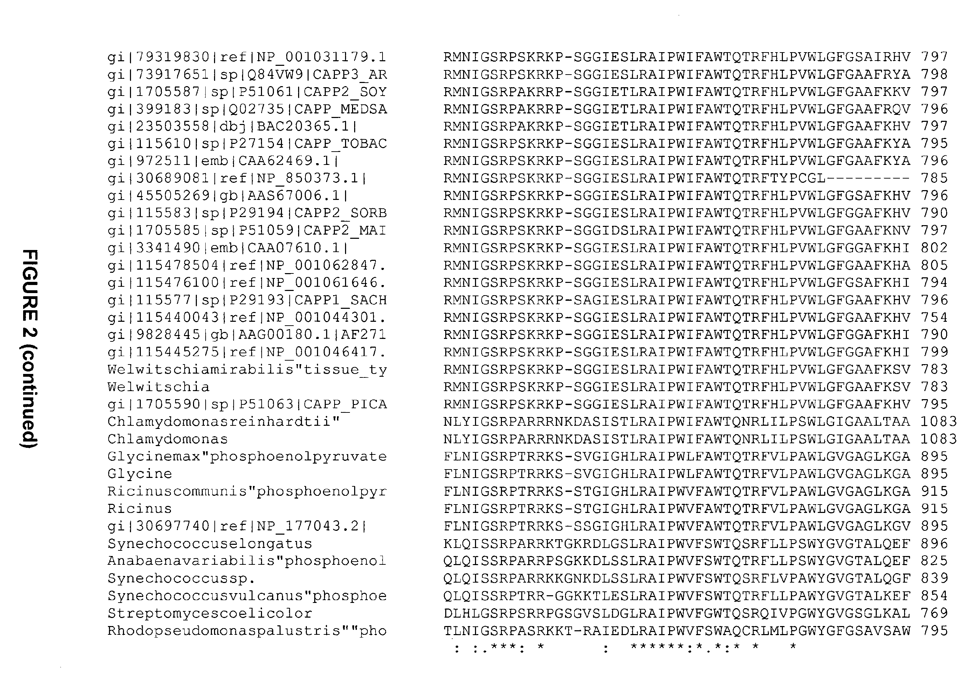

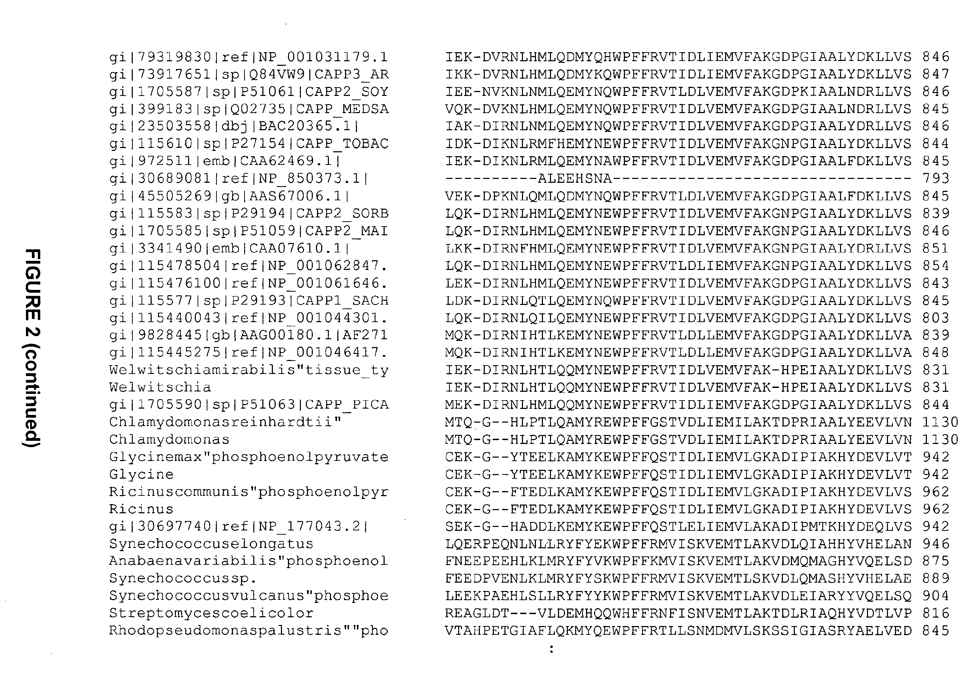

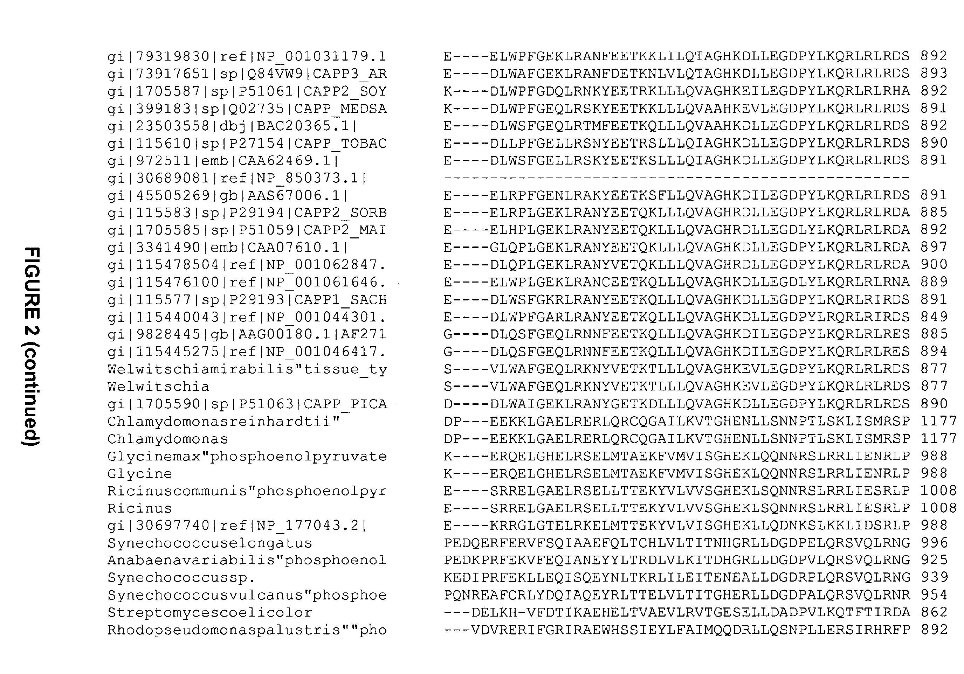

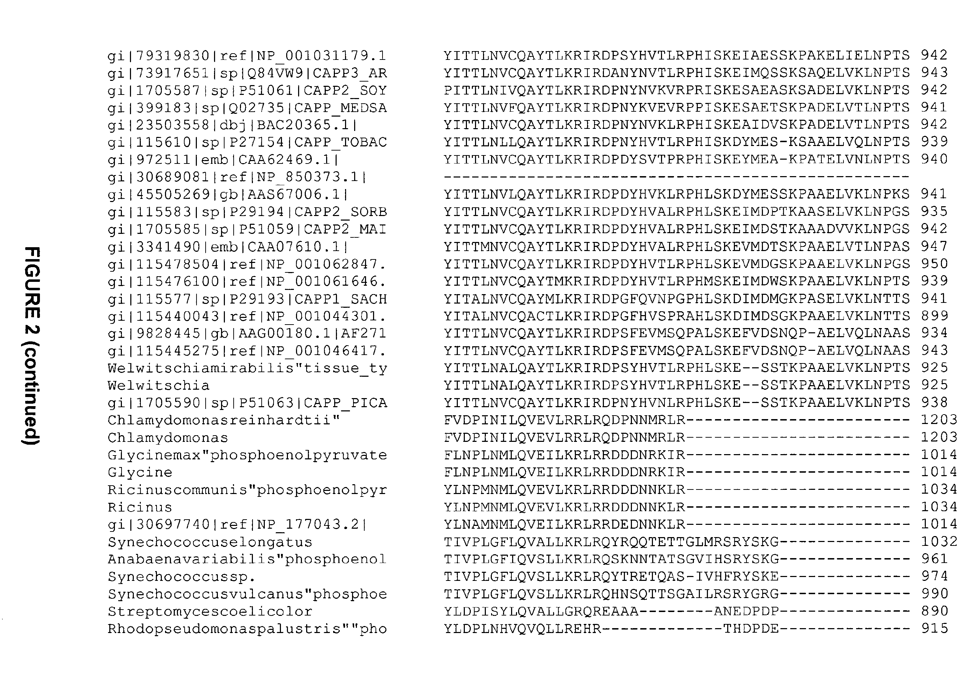

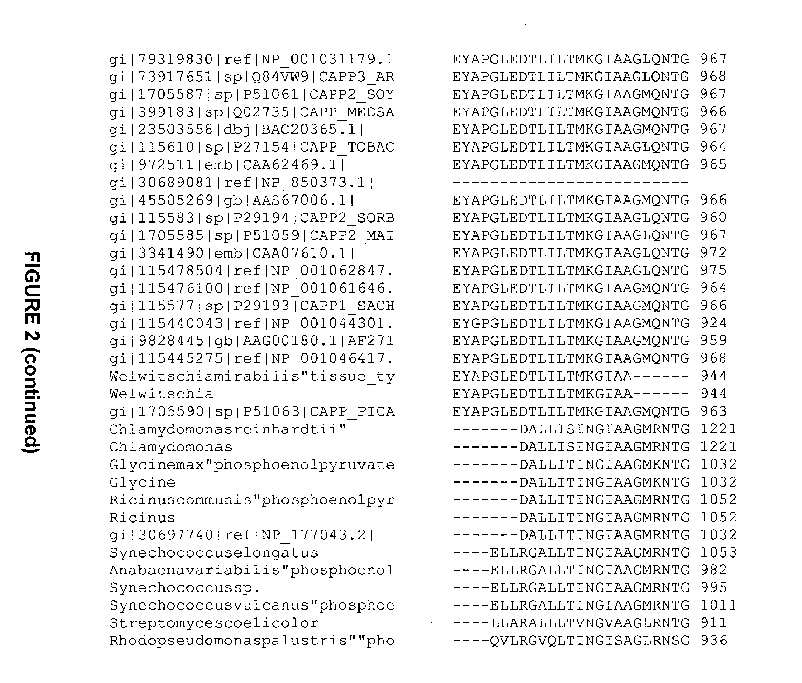

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Minor manual editing was done to further optimise the alignment. See Figure 2 for the alignment.

Example 3: Calculation of global percentage identity between polypeptide sequences useful in performing the methods of the invention

Global percentages of similarity and identity between full length polypeptide sequences useful in performing the methods of the invention is determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.
Parameters used in the comparison are:

| | |
|---|---|
| Scoring matrix: | Blosum62 |
| First Gap: | 12 |
| Extending gap: | 2 |

Example 4: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.
The results for a PEPC polypeptide gave an INTERPRO domain: IPR001449 (phosphoenolpyruvate carboxylase) and PFAM: 00311.
The results of the InterPro scan of the polypeptide sequence as represented by SEQ ID NO: 75 are given below.
SMART: 00504
InterPro: IPR003613 and IPR008938

Example 5: Topology prediction of the polypeptide sequences useful in performing the methods of the invention

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters are selected: the "plant" organism group is selected, no cutoffs defined, and the predicted length of the transit peptide requested.

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

Example 6: Functional assay for the polypeptide of the invention

PEPC is that it catalyzes the addition of $CO_2$ to phosphoenolpyruvate (PEP) to form the four-carbon compound oxaloacetate:

$$PEP + CO_2 \rightarrow oxaloacetate + Pi$$

For a description of the measurement of PEPC activity, see Chen et al., 2002 (Plant Cell Physiol 43(2): 159-169); and Chen et al., 2004 (Planta 219 pp440-449).

The Class III U-box polypeptide exhibits E3 ubiquitin ligase activity.

PQQC catalyses the last step in PQQ biosynthesis

Example 7: Cloning of the nucleic acid sequence used in the methods of the invention The nucleic acid sequence encoding a PEPC polypeptide and used in the methods of the invention was amplified by PCR Hifi Taq DNA polymerase in standard conditions, using 200 ng of template in a 50 μl PCR mix. The primers used were prm08777 (SEQ ID NO: 72; sense, start codon in bold):

5-ggggacaagtttgtacaaaaaagcaggcttaaacaatgaacttggcagttcct-3’

and prm08782 (SEQ ID NO: 73; reverse, complementary):

5’-ggggaccactttgtacaagaaagctgggttcaaccagtattacgcatt-3’,

which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pPEPC. Plasmid pDONR201 was purchased from Invitrogen, as part of the Gateway® technology.

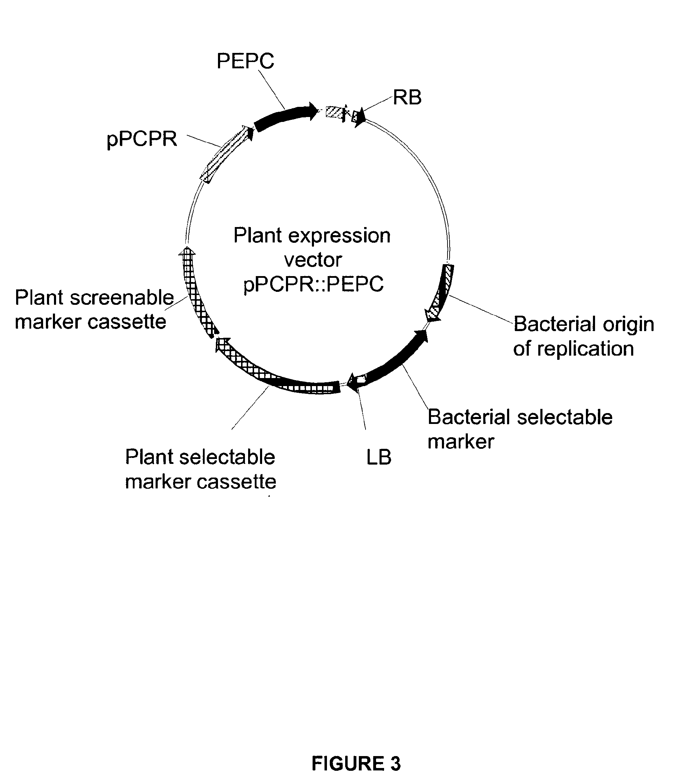

The entry clone comprising SEQ ID NO: 1 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A protochlorophyllid reductase promoter (SEQ ID NO: 71) for young green tissue specific expression was located upstream of this Gateway cassette - see Figure 3.

After the LR recombination step, the resulting expression vector pPCPR::PEPC (Figure 3) was transformed into Agrobacterium strain LBA4044 according to methods well known in the art.

The nucleic acid sequence encoding a Class III U-Box protein and used in the methods of the invention was amplified by PCR Hifi Taq DNA polymerase in standard conditions, using 200 ng of template in a 50 μl PCR mix. The primers used were prm9075 (SEQ ID NO: 102; sense, start codon in bold):

5'-ggggacaagtttgtacaaaaaagcaggcttaaacaatggaagaaattcaagtacc-3'

and prm8785 (SEQ ID NO: 103; reverse, complementary):

5'-ggggaccactttgtacaagaaagctgggtgctcttttggtcctattttcct-3',

which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pU-box. Plasmid pDONR201 was purchased from Invitrogen, as part of the Gateway® technology.

The entry clone comprising SEQ ID NO: 74 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A protochlorophyllid reductase promoter (SEQ ID NO: 101) for young green tissue specific expression was located upstream of this Gateway cassette.

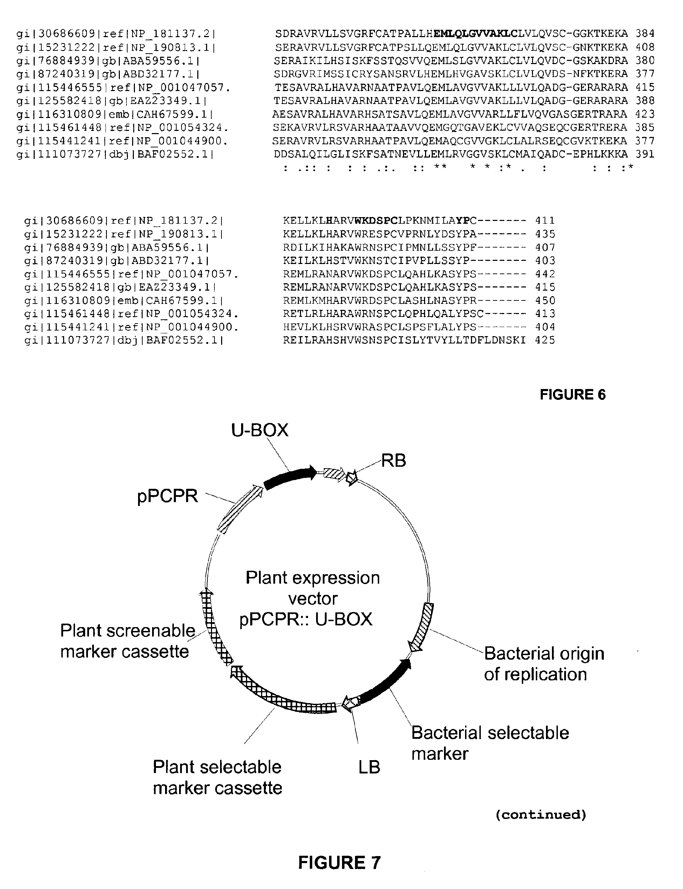

After the LR recombination step, the resulting expression vector pPCPR::U-BOX (Figure 7) was transformed into Agrobacterium strain LBA4044 according to methods well known in the art.

The nucleic acid sequence encoding a PQQC protein and used in the methods of the invention was amplified by PCR Hifi Taq DNA polymerase in standard conditions, using 200 ng of template in a 50 μl PCR mix. The primers used were prm08777 (SEQ ID NO: 228; sense, start codon in bold):

5-ggggacaagtttgtacaaaaaagcaggcttaaacaatgactgacaccccaatgtc-3'

and prm08782 (SEQ ID NO: 229; reverse, complementary):

5'-ggggaccactttgtacaagaaagctgggttcataaggtgattcctttatgc-3',

which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pPQQC. Plasmid pDONR201 was purchased from Invitrogen, as part of the Gateway® technology.

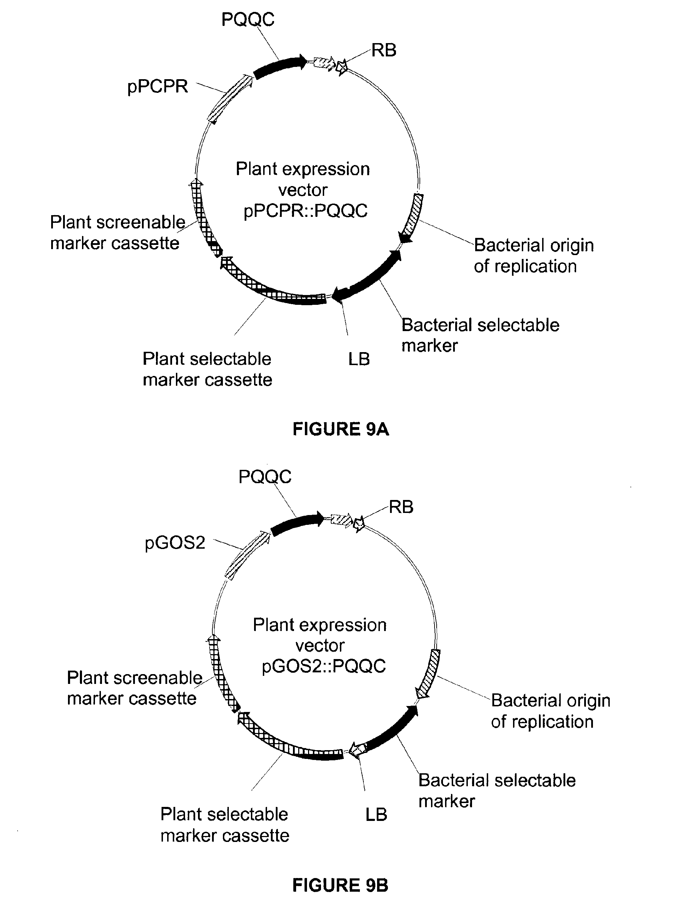

The entry clone comprising SEQ ID NO: 104 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A protochlorophyllid reductase promoter (SEQ ID NO: 226) for young green tissue specific expression was located upstream of this Gateway cassette or a GOS2 promoter (SEQ ID NO 227) - see Figure 9.

After the LR recombination step, the resulting expression vector pGOS2::PQQC (Figure 9B) was transformed into Agrobacterium strain LBA4044 according to methods well known in the art.

After the LR recombination step, the resulting expression vector pPCPR::PQQC (Figure 9A) was transformed into Agrobacterium strain LBA4044 according to methods well known in the art.

Example 8: Plant transformation

Rice transformation

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

Agrobacterium strain LBA4404 containing the expression vector was used for co-cultivation. Agrobacterium was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C.

Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Corn transformation

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5-10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

Alfalfa transformation

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

Example 9: Phenotypic evaluation procedure

9.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

Drought screen

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters are recorded as detailed for growth under normal conditions.

Nitrogen use efficiency screen

Rice plants from T2 seeds are grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters are recorded as detailed for growth under normal conditions.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

9.3 Parameters measured

Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles. The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

Example 10: Results of the phenotypic evaluation of the transgenic plants

Transgenic rice plants expressing a nucleic acid encoding the PEPC polypeptide (SEQ ID NO: 1) under the control of a protochlorophyllid reductase promoter gave the following results in either the T1 or T2 generation or both.

| Parameter | Normal conditions | Drought |
| --- | --- | --- |
| Total seed weight | Increase in 4 out of 4 events. 12% overall increase | Increase in 5 out of 5 events. 28% overall increase |
| Number filled seeds | Increase in 4 out of 4 events. 10% overall increase | Increase in 5 out of 5 events. 27% overall increase |
| Fill rate | Increase in 4 out of 4 events. 8% overall increase | Increase in 5 out of 5 events. 18% overall increase |
| Number flowers per panicle or Number of seeds | Increase in 4 out of 4 events. 8% overall increase | Increase in 5 out of 5 events. 19% overall increase |
| Harvest index | Increase in 4 out of 4 events. 13% overall increase | Increase in 5 out of 5 events. 22% overall increase |

In addition to the results shown above, the plants overexpressing PEPC under drought conditions also showed an increase in plant height (with 2 out of 5 lines giving an average 11.5% increase); an increase in root thickness (with 4 out of 5 lines giving an average of 23.5% increase); an increase in aboveground area (with 2 out of 5 lines giving an average increase of 14%); and an increase in emergence vigour (with 2 out of 5 lines giving a 47.5% increase).

Transgenic plants expressing a nucleic acid encoding the Class III U-box polypeptide (SEQ ID NO: 74) under the control of a protochlorophyllid reductase promoter gave the following results.

Results under non-stressed conditions:

Transgenic rice plants showed an increase in height, aboveground area, number of panicles, number of flowers, TKW (thousand kernel weight), total seed weight, total number seeds and in the total number of filled seeds relative to control plants.

Results under (mild) drought conditions:

Transgenic rice plants showed an increase in height, aboveground area, total weight seeds and in the number of filled seeds relative to control plants.

Transgenic rice plants expressing a nucleic acid encoding the PQQC polypeptide (SEQ ID NO: 104) under the control of a protochlorophyllid reductase promoter gave the following results.

Results under non-stressed conditions:

Transgenic rice plants showed an increase in height, aboveground area, number of panicles, number of flowers, total seed weight, total number seeds, number of filled seeds relative to control plants.

B. Transgenic rice plants expressing a nucleic acid encoding the PQQC polypeptide (SEQ ID NO: 104) under the control of a protochlorophyllid reductase promoter gave the following results. Similar results were obtained for transgenic rice plants expressing a nucleic acid encoding the PQQC polypeptide (SEQ ID NO: 104) under the control of a GOS2 promoter.

Results under (mild) drought conditions:

Transgenic rice plants showed an increase in height, fill rate, TKW (thousand kernel weight), number of filled seeds and in the total weight of seeds relative to control plants.

## Claims

1. A method for enhancing yield-related traits in plants relative to control plants, comprising introducing and expressing in a plant a nucleic acid encoding a yield increasing polypeptide consisting of a PQQC polypeptide.

2. Method according to claim 1, wherein said nucleic acid encoding a yield increasing polypeptide consisting of a PQQC polypeptide encodes any one of the proteins listed in Table A3 or is a portion of such a nucleic acid, or a nucleic acid capable of hybridising with such a nucleic acid.

3. Method according to claim 1 or 2, wherein said yield increasing polypeptide is represented by SEQ ID NO: 105, 135, 139 and orthologues and paralogues thereof.

4. Method according to any one of claims 1 to 3, wherein PQQC polypeptides have, in increasing order of preference, at least 45%, preferably 70%, more preferably 80%, more preferably 95%, or more sequence identity to the polypeptide represented by SEQ ID NO: 105.

5. Method according to any one of claims 1 to 4, wherein said enhanced yield-related traits comprise one or more of the following, increased aboveground area, increased total number of seeds, increased seed weight, increased fill rate, increased number of flowers per panicle, increased height, TKW, number of filled seeds, each relative to control plants.

6. Method according to any one of claims 1 to 5, wherein said nucleic acid is operably linked to a young green tissue-specific promoter or to a GOS2 promoter.

7. Plant or part thereof, including seeds, obtainable by a method any one of claims 1 to 6, wherein said plant or part thereof comprises a recombinant nucleic acid encoding a yield increasing polypeptide consisting of a PQQC polypeptide.

8. Construct comprising:

   (i) nucleic acid encoding a yield increasing polypeptide consisting of a PQQC polypeptide as defined in any one of claims 1 to 4;
   (ii) one or more control sequences capable of driving expression of the nucleic acid sequence of (a), preferably one of said control sequences is a young green tissue specific promoter or GOS2 promoter; and optionally
   (iii) a transcription termination sequence.

9. Use of a construct according to claim 8 in a method for making plants having one or more of the following: increased aboveground area, increased total number of seeds, increased seed weight, increased fill rate, increased number of flowers per panicle, increased height, TKW, number of filled seeds, each relative to control plants.

10. Plant, plant part or plant cell transformed with a construct according to claim 8.

11. Method for the production of a transgenic plant having increased yield, particularly increased aboveground area, increased total number of seeds, increased seed weight, increased fill rate, increased number of flowers per panicle, increased height, TKW, number of filled seeds relative to control plants, comprising:

   (i) introducing and expressing in a plant or plant cell a nucleic acid encoding a yield increasing polypeptide consisting of the PQQC polypeptide as defined in any one of claims 1 to 4; and
   (ii) cultivating the plant cell under conditions promoting plant growth and development.

12. Transgenic plant having one or more of the following: increased aboveground area, increased total number of seeds, increased seed weight, increased fill rate, increased number of flowers per panicle, increased height, TKW, number of filled seeds, each relative to control plants, resulting from increased expression of a nucleic acid encoding a yield increasing polypeptide consisting of the PQQC polypeptide as defined in any one of claims 1 to 4, or a transgenic plant cell derived from said transgenic plant.

13. Transgenic plant according to claim 7, 10 or 12, or a transgenic plant cell derived thereof, wherein said plant is a crop plant or a monocot or a cereal, such as rice, maize, wheat, barley, millet, rye, triticale, sorghum and oats.

**14.** Harvestable parts of a plant according to claim 13, wherein said harvestable parts are preferably shoot biomass and/or seeds.

**15.** Products derived from a plant according to claim 13 and/or from harvestable parts of a plant according to claim 14.

**16.** Use of a nucleic acid encoding a yield increasing polypeptide consisting of a PQQC polypeptide in increasing yield, particularly in increasing one or more of the following, increased aboveground area, increased total number of seeds, increased seed weight, increased fill rate, increased number of flowers per panicle, increased height, TKW, number of filled seeds, each relative to control plants.

**17.** Use of a nucleic acid sequence encoding a PQQC polypeptide, or use of a PQQC polypeptide as a molecular marker.

MNLAVPAFGLSTNWSGNGNGSNSEEESVLYQRLKMVEELWERVLQSECGQELVDLLTELRLQGTHE

AITSEISEEVIMGITQRIEHLELNDAIRAARAFALYFQLINIVEQHYEQNEQQRNRWEASQETNFY

EQAGNEEEMVPPSRLGASTEPLPVGIDQNELQASVGTFHWLMRELKRLNVPPQHIQNLLDHLDIRL

VITAHPTEIVRHTIRRKQRRVDRILRKLDQLQGSVTGRDWLNTWDAKTAIAQLTEEIRFWWRTDEL

HQFKPTVLDEVDYSLHYFDEVLFDAVPELSKRLGQAIKETFPHLRAPRANFCYFGSWVGGDRDGNP

SVTPEVTWQTACYQRGLVLGKYLFSLGELVAILSPSLHWCKVSQELLDSLERDRIQLPEIYEELSL

RYRQEPYRMKLAYVTKRLENTLRRNNRLANPEERQTMITMPAENHYRTGEELLEELRLIQRNLTET

GLTCLELENLITQLEVYGFNLAQLDFRQESSRHAEAIAEIAEYMGVLTTPYEEMAEEDKLAWLGVE

LQTRRPLIPQEMPFSERTRETIETLRTLRHLQMEFGVDICQTYIISMTNDASDVLEVLLLAKEAGL

YDPATASNSLRIVPLFETVEDLKNAPGIMDSLFSLPFYRATLAGSYHSLKELQNQPPDYYQIPTTT

ALLNPGNLQEIMVGYSDSNKDSGFLSSNWEIHKAQKSLQAVAQSHRVILRLFHGRGGSVGRGGGPA

**MOTIF I** **MOTIF V** **MOTIF II**

YKAILAQPAGTVDGRIKITEQGEVLASKYSLPELALYNLETLTTAVIQASLLKSSFDFIEPWNRIM

EELACTARRAYRSLIYEEPDFLDFFLTVTPIPEISELQISSRPARRKGGKADLSSLRAIPWVFSWT

**MOTIF III**

QTRFLLPAWYGVGTALKSFVDQDPVKNMKLLRYFYFKWPFFNMVISKVEMTLSKVDLTIASHYVQE

LSKPEDRERFDRLFQQIKQEYQLTRDFAMEITAHPHLLDGDRSLQRSVLLRNRTIVPLGLLQISLL

KRLRQVTQEAETSGVRYRRYSKEELLRGALLTINGIAAGMRNTG

**MOTIF IV**

**FIGURE 1**

```
CLUSTAL W (1.83) multiple sequence alignment

gi|79319830|ref|NP_001031179.1      --------------------------------------------------
gi|73917651|sp|Q84VW9|CAPP3_AR      --------------------------------------------------
gi|1705587|sp|P51061|CAPP2_SOY      --------------------------------------------------
gi|399183|sp|Q02735|CAPP_MEDSA      --------------------------------------------------
gi|23503558|dbj|BAC20365.1|         --------------------------------------------------
gi|115610|sp|P27154|CAPP_TOBAC      --------------------------------------------------
gi|972511|emb|CAA62469.1|           --------------------------------------------------
gi|30689081|ref|NP_850373.1|        --------------------------------------------------
gi|45505269|gb|AAS67006.1|          --------------------------------------------------
gi|115583|sp|P29194|CAPP2_SORB      --------------------------------------------------
gi|1705585|sp|P51059|CAPP2_MAI      -----------------------------------------MAALGP--- 6
gi|3341490|emb|CAA07610.1|          ------------------------------------------MALSAPGG 8
gi|115478504|ref|NP_001062847.      -----------------------------------------MSAMSAAGG 9
gi|115476100|ref|NP_001061646.      --------------------------------------------------
gi|115577|sp|P29193|CAPP1_SACH      --------------------------------------------------
gi|115440043|ref|NP_001044301.      --------------------------------------------------
gi|9828445|gb|AAG00180.1|AF271      --------------------------------------------------
gi|115445275|ref|NP_001046417.      -----------------------------------------------MAA 3
Welwitschiamirabilis"tissue_ty      --------------------------------------------------
Welwitschia                         --------------------------------------------------
gi|1705590|sp|P51063|CAPP_PICA      --------------------------------------------------
Chlamydomonasreinhardtii"           --------------------------------------------------
Chlamydomonas                       --------------------------------------------------
Glycinemax"phosphoenolpyruvate      --------------------------------------------------
Glycine                             --------------------------------------------------
Ricinuscommunis"phosphoenolpyr      --------------------------------------------------
Ricinus                             --------------------------------------------------
gi|30697740|ref|NP_177043.2|        --------------------------------------------------
Synechococcuselongatus              MLAVSLRDHGFPSATVQIRPAFCVQSDVVGSGNPPRMNYCQNARTAMSAA 50
Anabaenavariabilis"phosphoenol      --------------------------------------------------
Synechococcussp.                    --------------------------------------------------
Synechococcusvulcanus"phosphoe      --------------------------------------------------
Streptomycescoelicolor              --------------------------------------------------
Rhodopseudomonaspalustris""pho      --------------------------------------------------
```

FIGURE 2

```
gi|79319830|ref|NP_001031179.1      -MANRKLEKMASIDVHLRQLVPGKVSEDDKLVEYDALLLDRFLDILQD-- 47
gi|73917651|sp|Q84VW9|CAPP3_AR      -MAGRNIEKMASIDAQLRQLVPAKVSEDDKLVEYDALLLDRFLDILQD-- 47
gi|1705587|sp|P51061|CAPP2_SOY      -MATRNLEKMASIDAQLRQLAPAKVSEDDKLIEYDALLLDRFLDILQD-- 47
gi|399183|sp|Q02735|CAPP_MEDSA      -MAN-KMEKMASIDAQLRQLVPAKVSEDDKLIEYDALLLDRFLDILQD-- 46
gi|23503558|dbj|BAC20365.1|         -MANRNLEKMASIDAQLRQLVPAKVSEDDKLVEYDALLLDRFLDILQD-- 47
gi|115610|sp|P27154|CAPP_TOBAC      -MATRSLEKLASIDAQLRALVPGKVSEDDKLVEYDALLLDRFLDILQD-- 47
gi|972511|emb|CAA62469.1|           -MTTRNLEKLASIDAQLRQLVPAKVSEDDKLVEYDALLLDRFLDILQD-- 47
gi|30689081|ref|NP_850373.1|        -MAARNLEKMASIDAQLRLLAPGKVSEDDKLIEYDALLLDRFLDILQD-- 47
gi|45505269|gb|AAS67006.1|          -MAARNIEKMASIDAQLRLLAPRKVSDDDKLVEYDALLLDRFLDILQD-- 47
gi|115583|sp|P29194|CAPP2_SORB      ------MERLSSIDAQLRMLVPGKVSEDDKLIEYDALLLDRFLDILQD-- 42
gi|1705585|sp|P51059|CAPP2_MAI      -----KMERLSSIDAQLRMLVPGKVSEDDKLIEYDALLLDRFLDILQD-- 49
gi|3341490|emb|CAA07610.1|          GS--GKIERLSSIDAQLRLLVPAKVSEDDKLIEYDALLLDRFLDVLQG-- 54
gi|115478504|ref|NP_001062847.      GGGVGKVERLSSIDAQLRQLVPAKLSEDDKLIEYDALLLDRFLDVLHG-- 57
gi|115476100|ref|NP_001061646.      --MAGKVEKMASIDAQLRMLAPAKLSEDDKLVEYDALLLDRFLDILQD-- 46
gi|115577|sp|P29193|CAPP1_SACH      -MARNAVDKATSIDAQLRLLAPQKLSDDDKLVEYDALLLDRFLDILQD-- 47
gi|115440043|ref|NP_001044301.      -MARNAADKGTSIDAQLRILAPKKLSEDDKLVEYDALLLDRFLDILQD-- 47
gi|9828445|gb|AAG00180.1|AF271      ------MERHQSIDAQLRLLAPGKVSEDDKLVEYDALLVDRFLDILQD-- 42
gi|115445275|ref|NP_001046417.      AAGKAAMERHQSIDAQLRLLAPGKVSEDDKLVEYDALLVDRFLDILQD-- 51
Welwitschiamirabilis"tissue_ty      -----------SIDAQLRLLVPTKVSEDDKLIEYDALLMDRFLDILQD-- 37
Welwitschia                         -----------SIDAQLRLLVPTKVSEDDKLIEYDALLMDRFLDILQD-- 37
gi|1705590|sp|P51063|CAPP_PICA      -MARNNLEKMASIDAQMRLLVPGKVSEDDKLIEYDALLLDRFLDILQD-- 47
Chlamydomonasreinhardtii"           ---------MTDSTYDFGAVRDDLTPLEDDCKLLGSLLDDCLRVEIGETM 41
Chlamydomonas                       ---------MTDSTYDFGAVRDDLTPLEDDCKLLGSLLDDCLRVEIGETM 41
Glycinemax"phosphoenolpyruvate      ---------MTDITGDI-AEEISFQSFDDDCRLLGNLLNDILQREVGTNL 40
Glycine                             ---------MTDITGDI-AEEISFQSFDDDCRLLGNLLNDILQREVGTNL 40
Ricinuscommunis"phosphoenolpyr      ---------MTDTTDDI-AEEISFQSFDDDCKLLGNLLNDVLQREVGSKF 40
Ricinus                             ---------MTDTTDDI-AEEISFQSFDDDCKLLGNLLNDVLQREVGSKF 40
gi|30697740|ref|NP_177043.2|        ---------MTDTTDDI-AEEISFQSFEDDCKLLGSLFHDVLQREVGNPF 40
Synechococcuselongatus              LQSSDDAFRTVSSPLATD--LDLSSPLEFFLRHRLTVVEELWEVVLRQ-- 96
Anabaenavariabilis"phosphoenol      --------------------------------------------------
Synechococcussp.                    -------MNQVMHPPSAE--AELLSTSQSLLRQRLTLVEDIWQAVLQK-- 39
Synechococcusvulcanus"phosphoe      -------MTSVLDVTNRDRLIESESLAARTLQERLRLVEEVLVDVLAA-- 41
Streptomycescoelicolor              --MSSA-------------DDQTTTTTSSELRADIRRLGDLLGETLVR-- 33
Rhodopseudomonaspalustris""pho      --MSSLNLSAGPEPVSERPDDAAAIEAETRLRNDIRLLGRILGDTVRE-- 46
```

FIGURE 2

```
gi|79319830|ref|NP_001031179.1       -LHGEDLRETVQELYEHSAEYEGKHEPKKLEELGSVLTSLDPGDSIVIAK 96
gi|73917651|sp|Q84VW9|CAPP3_AR       -LHGEDLRETVQELYELSAEYEGKREPSKLEELGSVLTSLDPGDSIVISK 96
gi|1705587|sp|P51061|CAPP2_SOY       -LHGEDLKETVQEVYELSAEYEGKHDPKKLEELGNLITSLDAGDSILVAK 96
gi|399183|sp|Q02735|CAPP_MEDSA       -LHGEDLKDSVQEVYELSAEYERKHDPKKLEELGNLITSFDAGDSIVVAK 95
gi|23503558|dbj|BAC20365.1|          -LHGEDLRETVQEVYELSAEYERKHEPQRLEVLGNLITSLDAGDSIVVAK 96
gi|115610|sp|P27154|CAPP_TOBAC       -LHGEDLKETVQECYELSAEYEGKHDPKKLEELGNVLTSLDPGDSIVIAK 96
gi|972511|emb|CAA62469.1|            -LHGEDLKETVQECYELSAEYEAKHDPKKLEELGNVLTSLDPGDSIVIAK 96
gi|30689081|ref|NP_850373.1|         -LHGEDVREFVQECYEVAADYDGNRNTEKLEELGNMLTSLDPGDSIVVTK 96
gi|45505269|gb|AAS67006.1|           -LHGEDIRQTVQDCYELSAEYEGEHKPEKLEELGNMLTGLDAGDSIVIAK 96
gi|115583|sp|P29194|CAPP2_SORB       -LHGDDLKEMVQECYEVAAEYETKHDLQKLDELGKMITSLDPGDSIVIAK 91
gi|1705585|sp|P51059|CAPP2_MAI       -LHGDDLKEMVQECYEVAAEYETKHDLQKLDELGKMITSLDPGDSIVIAK 98
gi|3341490|emb|CAA07610.1|           -LHGDDLREMVQECYEVAAEYETKHDLEKLDELGEMITSLDPGDSIVIAK 103
gi|115478504|ref|NP_001062847.       -LHGDDLKDLVQECYEVAAEYETKHDVQKLDELGNMITSLDPGDSIVIAK 106
gi|115476100|ref|NP_001061646.       -LHGDDLRELVQECYEIAAEYEGKHDSQKLDELGNMLTSLDPGDSIVMAK 95
gi|115577|sp|P29193|CAPP1_SACH       -LHGEDIRETVQECYELAAEYENKLDPKMLDEIGNVLTSLDPGDSIVITK 96
gi|115440043|ref|NP_001044301.       -LHGEDIRETVQECYELAAEYES-------------------------- 69
gi|9828445|gb|AAG00180.1|AF271       -LHGPHLREFVQECYELSAEYENDRDEARLDELGRKLTSLPPGDSIVVSS 91
gi|115445275|ref|NP_001046417.       -LHGPHLREFVQECYELSAEYENDRDEARLDELGRKLTSLPPGDSIVVSS 100
Welwitschiamirabilis"tissue_ty       -LHGEEIRETVQECYELSGEYEGKFDTAKLEELGGVLTSLDAGDSIVVAA 86
Welwitschia                          -LHGEEIRETVQECYELSGEYEGKFDTAKLEELGGVLTSLDAGDSIVVAA 86
gi|1705590|sp|P51063|CAPP_PICA       -LHGEDIRAMVQECYERSGEYEGKNDPHKLEELGNVLTSLDPGDSIVVAS 96
Chlamydomonasreinhardtii"            FKKIERIRALAQCASNLSIKGDAGASDMLSHRLAEELMNLDMDEAVPLTR 91
Chlamydomonas                        FKKIERIRALAQCASNLSIKGDAGASDMLSHRLAEELMNLDMDEAVPLTR 91
Glycinemax"phosphoenolpyruvate       LDKIERTRVLAQSGCNMRQAGIVNMAEMLEKQLASELSKMTLEEAFTLAR 90
Glycine                              LDKIERTRVLAQSGCNMRQAGIVNMAEMLEKQLASELSKMTLEEAFTLAR 90
Ricinuscommunis"phosphoenolpyr       MEKLERNRILAQSACNMRLAGIEDTAELLEKQLALEISRMTLEEALTLAR 90
Ricinus                              MEKLERNRILAQSACNMRLAGIEDTAELLEKQLALEISRMTLEEALTLAR 90
gi|30697740|ref|NP_177043.2|         MEKVERIRILAQSALNLRMAGIEDTANLLEKQLTSEISKMPLEEALTLAR 90
Synechococcuselongatus               -ECGQELVDILTQLRDLT-SPEGQAPEVGGEALVQVIETLELSDAIRAAR 144
Anabaenavariabilis"phosphoenol       ------MVDLLRQLRDLC-SPEGQATKDQAVSAVKLIEQLNINEAIRAAR 43
Synechococcussp.                     -ECGQKLVERLNHLRATR-TADGQSLNFSPSSISELIETLDLEDAIRAAR 87
Synechococcusvulcanus"phosphoe       -ESGQELVDLLRRLGALS-SPEGHVLHAPEGELLKVIESLELNEAIRAAR 89
Streptomycescoelicolor               -QEGPELLELVEKVR----RLTREDGEAA----AELLRGTELETAAKLVR 74
Rhodopseudomonaspalustris""pho       -QEGQTVFDLVENIRQTSIRFHRDDDKTARAELEAILDGMSIPDTMRIVR 95
```

FIGURE 2 (continued)

```
gi|79319830|ref|NP_001031179.1      AFSHMLNLANLAEEVQIAY----RRRIKKLKK------------------ 124
gi|73917651|sp|Q84VW9|CAPP3_AR      AFSHMLNLANLAEEVQIAH----RRRIKKLKK------------------ 124
gi|1705587|sp|P51061|CAPP2_SOY      SFSHMLNLANLAEEVQISR----RRRNKLKK------------------- 123
gi|399183|sp|Q02735|CAPP_MEDSA      SFSHMLNLANLAEEVQIAH----RRRNKLKK------------------- 122
gi|23503558|dbj|BAC20365.1|         SFAHMLNLANLAEEVQIAH----RRRIKLKK------------------- 123
gi|115610|sp|P27154|CAPP_TOBAC      AFSHMLNLANLAEEVQIAY----RRRQKLKR------------------- 123
gi|972511|emb|CAA62469.1|           AFSHMLNLANLAEEVQIAY----RRRQKLKKK------------------ 124
gi|30689081|ref|NP_850373.1|        SFSNMLSLANLAEEVQIAY----RRRIKKLKK------------------ 124
gi|45505269|gb|AAS67006.1|          SFSHMLNLANLAEEVQIAY----RRRIKLLKK------------------ 124
gi|115583|sp|P29194|CAPP2_SORB      SFSHMLNLANLAEEVQIAY----RRRIKLKK------------------- 118
gi|1705585|sp|P51059|CAPP2_MAI      SLSHMLNLANLAEEVQIAY----RRRIKLKK------------------- 125
gi|3341490|emb|CAA07610.1|          AFSHMLNLANLAEEVQIAY----RRRVKLKK------------------- 130
gi|115478504|ref|NP_001062847.      AFSHMLNLANLAEEVQIAY----RRRIKLKK------------------- 133
gi|115476100|ref|NP_001061646.      AFSHMLNLANLAEEVQIAY----RRRIKLKK------------------- 122
gi|115577|sp|P29193|CAPP1_SACH      SFSHMLILANLAEEVQIAY----RRRIKLKK------------------- 123
gi|115440043|ref|NP_001044301.      ---------------KIAY----RRRIKLKK------------------- 81
gi|9828445|gb|AAG00180.1|AF271      SFSHMLNLANLAEEVQIAH----RRRIKLKR------------------- 118
gi|115445275|ref|NP_001046417.      SFSHMLNLANLAEEVQIAH----RRRIKLKR------------------- 127
Welwitschiamirabilis"tissue_ty      -LSHMLNLANLAEEVQIAY----RRRIKHKKK------------------ 113
Welwitschia                         -LSHMLNLANLAEEVQIAY----RRRIKHKKK------------------ 113
gi|1705590|sp|P51063|CAPP_PICA      SFSHMLNLANLAEEVQIAY----RRRNKIKR------------------- 123
Chlamydomonasreinhardtii"           ACGHYLNLSGIAELHHGVR----RDR------------------------ 113
Chlamydomonas                       ACGHYLNLSGIAELHHGVR----RDR------------------------ 113
Glycinemax"phosphoenolpyruvate      AFSHYLTLMGIAETHHRVR----KGG------------------------ 112
Glycine                             AFSHYLTLMGIAETHHRVR----KGG------------------------ 112
Ricinuscommunis"phosphoenolpyr      AFSHYLNLMGIAETHHRVR----KAR------------------------ 112
Ricinus                             AFSHYLNLMGIAETHHRVR----KAR------------------------ 112
gi|30697740|ref|NP_177043.2|        TFTHSLNLMGIADTHHRMH----KVH------------------------ 112
Synechococcuselongatus              AFALYFQLINIVEQHYEQ-----TQYQLAYE--RSRLEPLPGPD------ 181
Anabaenavariabilis"phosphoenol      AFALYFQLINIIEQEYEQ-----RQQLTRYSDLEAETAPLNGSENITSSS 88
Synechococcussp.                    AFALYFQLINSVEQHYEQ-----REQQFRR----NLASANASE------- 122
Synechococcusvulcanus"phosphoe      AFNLYFQIINIVEQHYEQQYNRERAAQEGLRRRSVMSEPISGVSGEGFPL 139
Streptomycescoelicolor              AFSTYFHLANVTEQVHRG-----RELGAKR-------------------- 99
Rhodopseudomonaspalustris""pho      AFSYFSHLANIAEDQNNI-----RQMRAGST------------------- 121
```

FIGURE 2 (continued)

```
gi|79319830|ref|NP_001031179.1        -------------------GDFVDESSATTESDLEETFKKLVGDLNKSPE 155
gi|73917651|sp|Q84VW9|CAPP3_AR        -------------------GDFVDESSATTESDIEETFKRLVSDLGKSPE 155
gi|1705587|sp|P51061|CAPP2_SOY        -------------------GDFADENNATTESDIEETLKKLVFDLKKSPQ 154
gi|399183|sp|Q02735|CAPP_MEDSA        -------------------GDFRDESNATTESDIEETLKKLVFDMKKSPQ 153
gi|23503558|dbj|BAC20365.1|           -------------------GDFADEGNATTESDIEETFKKLVGEMKKSPQ 154
gi|115610|sp|P27154|CAPP_TOBAC        -------------------GDFADENNATTESDIEETFKKLVGDLKKSPQ 154
gi|972511|emb|CAA62469.1|             -------------------GDFGDESNATTESDIEETFKKLVGDLKKSPQ 155
gi|30689081|ref|NP_850373.1|          -------------------GDFADEASATTESDIEETLKRLL-QLNKTPE 154
gi|45505269|gb|AAS67006.1|            -------------------GDFADENSAITESDIEETFKKLVAQLKKTPQ 155
gi|115583|sp|P29194|CAPP2_SORB        -------------------GDFADENSAITESDIEETLKRLVVDLKKSPA 149
gi|1705585|sp|P51059|CAPP2_MAI        -------------------GDFADENSAITESDIEETLKRLVVDLKKSPA 156
gi|3341490|emb|CAA07610.1|            -------------------GDFADENSAITESDIEETLKRLVFDMKKSPA 161
gi|115478504|ref|NP_001062847.        -------------------GDFADENSAMTESDIEETLKRLVFDLKKSPA 164
gi|115476100|ref|NP_001061646.        -------------------GDFADENSALTESDIEETFKRLVVDLKKSPA 153
gi|115577|sp|P29193|CAPP1_SACH        -------------------GDFVDENSATTESDIEETLKRLMHQLKKSPL 154
gi|115440043|ref|NP_001044301.        -------------------GDFADENSATTESNFEETLKRLVGELKKSPH 112
gi|9828445|gb|AAG00180.1|AF271        -------------------GDFADEASAPTESDIEETLKRLVTQLGKSRE 149
gi|115445275|ref|NP_001046417.        -------------------GDFADEASAPTESDIEETLKRLVTQLGKSRE 158
Welwitschiamirabilis"tissue_ty        -------------------GDFADENSATTESDIEETFKRLVNQLGRSPQ 144
Welwitschia                           -------------------GDFADENSATTESDIEETFKRLVNQLGRSPQ 144
gi|1705590|sp|P51063|CAPP_PICA        -------------------GGFADESNATTESDIEETFKRLVNQLGKSPA 154
Chlamydomonasreinhardtii"             ------------------------ATREPNPNSCDAVFARLITEGVD-PE 138
Chlamydomonas                         ------------------------ATREPNPNSCDAVFARLITEGVD-PE 138
Glycinemax"phosphoenolpyruvate        ------------------------NMAQIA-KSCDDIFNQLVQGGVP-PE 136
Glycine                               ------------------------NMAQIA-KSCDDIFNQLVQGGVP-PE 136
Ricinuscommunis"phosphoenolpyr        ------------------------SMTHLS-KSCDDIFNQLLQSGIS-AE 136
Ricinus                               ------------------------SMTHLS-KSCDDIFNQLLQSGIS-AE 136
gi|30697740|ref|NP_177043.2|          ------------------------NVTQLA-RSCDDIFSQLLQSGIS-PD 136
Synechococcuselongatus                --ESPEGLHTIEIPQHQLDP-FAAVIPLNQDPATFQTLFPRLRQLNVPPQ 228
Anabaenavariabilis"phosphoenol        NHNEDDVIFNRGLGTDFLGK-NWTNRGQGKQKGTFAALFPLLSKLNVPPQ 137
Synechococcussp.                      --ANGNSVHTEIAPT---------------QAGTFDWLFPHLKHQNMPPQ 155
Synechococcusvulcanus"phosphoe        PHTAANATDVRSGPSERLEHSLYEAIPATQQYGSFAWLFPRLQMLNVPPR 189
Streptomycescoelicolor                ----------------------------AAEGGLLARTAD--RLKDADPE 119
Rhodopseudomonaspalustris""pho        -------------------------AGSAPRAGLLAKTLAHARQEGISAA 146
```

FIGURE 2 (continued)

```
gi|79319830|ref|NP_001031179.1      EIFDALKNQTVDLVLTAHPTQSVRRSLLQKHGRIRDCLAQLYAKDITPDD 205
gi|73917651|sp|Q84VW9|CAPP3_AR      EIFDALKNQTVDLVLTAHPTQSVRRSLLQKHGRIRDCLAQLYAKDITPDD 205
gi|1705587|sp|P51061|CAPP2_SOY      EVFDALKNQTVDLVLTAHPTQSIRRSLLQKHGRIRNCLSQLYAKDITPDD 204
gi|399183|sp|Q02735|CAPP_MEDSA      EVFDALKNQTVDLVLTAHPTQSVRRSLLQKHGRVRNCLSQLYAKDITPDD 203
gi|23503558|dbj|BAC20365.1|         EVFDELKNQTVDLVLTAHPTQSVRRSLLQKHGRIRNCLTQLYAKDITPDD 204
gi|115610|sp|P27154|CAPP_TOBAC      EVFDALKNQTVDLVLTAHPTQSVRRSLLQKHGRIRDCLAQLYAKDITPDD 204
gi|972511|emb|CAA62469.1|           EVFDAIKNQTVDLVLTAHPTQSVRRSLLQKHGRIRDCLAQLYAKDITPDD 205
gi|30689081|ref|NP_850373.1|        EVFDALKNQTVDLVLTAHPTQSVRRSLLQKFGRIRDCLTQLYAKDITPDD 204
gi|45505269|gb|AAS67006.1|          EIFDALKNQTVDLVLTAHPTQSVRRSLLQKHGRIRNCLTQLYAKDITPDD 205
gi|115583|sp|P29194|CAPP2_SORB      EVFDALKSQTVDLVLTAHPTQSVRRSLLQKHSRIRNCLVQLYSKDITPDD 199
gi|1705585|sp|P51059|CAPP2_MAI      EVFDALKSQTVDLVLTAHPTQSVRRSLLQKHSRIRNCLVQLYSKDITPDD 206
gi|3341490|emb|CAA07610.1|          EVFDALKNQTVDLVLTAHPTQSVRRSLLQKHSRIRNCLVQLYSKDITPDD 211
gi|115478504|ref|NP_001062847.      EVFDALKSQTVDLVLTAHPTQSVRRSLLQKHSRIRNCLVQLYSKDITPDD 214
gi|115476100|ref|NP_001061646.      EVFDALKSQTVDLVLTAHPTQSVRRSLLQKHSRIRNCLVQLYSKDITPDD 203
gi|115577|sp|P29193|CAPP1_SACH      EVFDALKNQTVDLVLTAHPTQSVRRSLLQKHGRIRNCLTQLYAKDITPDE 204
gi|115440043|ref|NP_001044301.      EVFDALKNQTIDLVLTAHPTQSVRRSLLQKHGRIRSCLTKLYAKDITPDE 162
gi|9828445|gb|AAG00180.1|AF271      EVFDALKNQTVDLVFTAHPTQSVRRSLLQKHGRIRNCLRQLYAKDITADD 199
gi|115445275|ref|NP_001046417.      EVFDALKNQTVDLVFTAHPTQSVRRSLLQKHGRIRNCLRQLYAKDITADD 208
Welwitschiamirabilis"tissue_ty      EVFDALKNQTIDLVLTAHPTHQS-VSLLQKHGRIRNCLSQLYAKDITPDE 193
Welwitschia                         EVFDALKNQTIDLVLTAHPTHQS-VSLLQKHGRIRNCLSQLYAKDITPDE 193
gi|1705590|sp|P51063|CAPP_PICA      EVFDALKNQTVDLVLTAHPTQSVRRSLLQKHARIRNCLSQLYGKDITPDE 204
Chlamydomonasreinhardtii"           ELYRAVSEQNVEVVLTAHPTQVNRRTLQYKHTRIAALLQQHDRSDLTAEE 188
Chlamydomonas                       ELYRAVSEQNVEVVLTAHPTQVNRRTLQYKHTRIAALLQQHDRSDLTAEE 188
Glycinemax"phosphoenolpyruvate      ELYDTVCKREVEIVLTAHPTQINRRTLQFKHIRIAHLLDYNDRPDLSTED 186
Glycine                             ELYDTVCKREVEIVLTAHPTQINRRTLQFKHIRIAHLLDYNDRPDLSTED 186
Ricinuscommunis"phosphoenolpyr      ELYDTVCKQEVEIVLTAHPTQINRRTLQYKHIRIAHLLDYNDRPDLTHED 186
Ricinus                             ELYDTVCKQEVEIVLTAHPTQINRRTLQYKHIRIAHLLDYNDRPDLTHED 186
gi|30697740|ref|NP_177043.2|        ELYKTVCKQEVEIVLTAHPTQINRRTLQYKHIRIAHLLEYNTRSDLSVED 186
Synechococcuselongatus              MIQELTDRLDIRLVFTAHPTEIVRHTIRDKQRRIAYLLRQLDELET-GKN 277
Anabaenavariabilis"phosphoenol      QIQRLISQLDVRLVFTAHPTEIVRHTIRDKQRQVVDLLQQLDEVENRAKD 187
Synechococcussp.                    TIQRLLNQLDIRLVFTAHPTEIVRHTIRNKQRRIAGILRQLDQTEEGLKS 205
Synechococcusvulcanus"phosphoe      HIQKLLDQLDIKLVFTAHPTEIVRQTIRDKQRRVARLLEQLDVLEGASPH 239
Streptomycescoelicolor              HLRETVRNLNVRPVFTAHPTEAARRSVLNKLRRIAALLDTP----VNESD 165
Rhodopseudomonaspalustris""pho      ELRKFFATALVSPVLTAHPTEVRRKSTMDREMQIASLLDQRDRVQLTADE 196
                                     :         :  *:*****.    :   :  ::    *
```

FIGURE 2 (continued)

```
gi|79319830|ref|NP_001031179.1      KQE--------LDEALQREIQAAFRTDEIKRTPPTPQDEMRAGMSYFHET 247
gi|73917651|sp|Q84VW9|CAPP3_AR      KQE--------LDESLQREIQAAFRTDEIRRTPPTPQDEMRAGMSYFHET 247
gi|1705587|sp|P51061|CAPP2_SOY      KQE--------LDEALQREIQAAFRTDEIRRTPPTPQDEMRAGMSYFHET 246
gi|399183|sp|Q02735|CAPP_MEDSA      KQE--------LDEALQREIQAAFRTDEIKRTPPTPQDEMRAGMSYFHET 245
gi|23503558|dbj|BAC20365.1|         KQE--------LDEALQREIQAAFRTDEIRRTAPTPQDEMRAGMSYFHET 246
gi|115610|sp|P27154|CAPP_TOBAC      KQE--------LDEALQREIQAAFRTDEIRRTAPTPQDEMRAGMSYFHET 246
gi|972511|emb|CAA62469.1|           KQE--------LDEALQREIQAAFRTDEIRRTPPTPQDEMRAGMSYFHET 247
gi|30689081|ref|NP_850373.1|        KQE--------LDEALQREIQAAFRTDEIRRTPPTPQDEMRAGMSYFHET 246
gi|45505269|gb|AAS67006.1|          KQE--------LDEALQREIQAAFRTDEIRRTPPTPQDEMRAGMSYFHET 247
gi|115583|sp|P29194|CAPP2_SORB      KQE--------LDEALQREIQAAFRTDEIRRTQPTPQDEMRAGMSYFHET 241
gi|1705585|sp|P51059|CAPP2_MAI      KQE--------LDEALQREIQAAFRTDEIRRTQPTPQDEMRAGMSYFHET 248
gi|3341490|emb|CAA07610.1|          KQE--------LDEALQREIQAAFRTDEIRRLSPTPQDHMRAGMSDFHET 253
gi|115478504|ref|NP_001062847.      KQE--------LDEALQREIQAAFRTDEIRRTQPTPQDEMRAGMSYFHET 256
gi|115476100|ref|NP_001061646.      KQE--------LDEALQREIQAAFRTDEIRRTQPTPQDEMRAGMSYFHET 245
gi|115577|sp|P29193|CAPP1_SACH      KQE--------LDEALQREIQAAFRTDEIRRAPPTPQDEMRAGMSYFHET 246
gi|115440043|ref|NP_001044301.      KQE--------LDEALKREIQAAFRTDEIRRAPPTPQDEMRAGMSYFHET 204
gi|9828445|gb|AAG00180.1|AF271      KQE--------LDEALQREIQAAFRTDEIRRTPPTPQDEMRAGMSYFHET 241
gi|115445275|ref|NP_001046417.      KQE--------LDEALQREIQAAFRTDEIRRTPPTPQDEMRAGMSYFHET 250
Welwitschiamirabilis"tissue_ty      KQE--------LDEALQREIQAAFRTDEIRRTPPTPQDEMRAGMSYFHET 235
Welwitschia                         KQE--------LDEALQREIQAAFRTDEIRRTPPTPQDEMRAGMSYFHET 235
gi|1705590|sp|P51063|CAPP_PICA      KQE--------LDEALLREIQAAFRTDEIRRTPPTPQDEMRAGMSYFHET 246
Chlamydomonasreinhardtii"           RRN--------MVSELQREVAALWQTDELRRQKPTPLDEARGGLHIVEQS 230
Chlamydomonas                       RRN--------MVSELQREVAALWQTDELRRQKPTPLDEARGGLHIVEQS 230
Glycinemax"phosphoenolpyruvate      REM--------VIEDLVREITSIWQTDELRRQKPTPVDEARAGFNIVEQS 228
Glycine                             REM--------VIEDLVREITSIWQTDELRRQKPTPVDEARAGFNIVEQS 228
Ricinuscommunis"phosphoenolpyr      REM--------LIEDLVREITSIWQTDELRRHKPTPVDEARAGLNIVEQS 228
Ricinus                             REM--------LIEDLVREITSIWQTDELRRHKPTPVDEARAGLNIVEQS 228
gi|30697740|ref|NP_177043.2|        RET--------LIEDLVREITSLWQTDELRRQKPTPVDEARAGLNIVEQS 228
Synechococcuselongatus              RGF-RELEAQNIRQQLTEEIRLWWRTDELHQFKPTVLDEVDYALHYFQEV 326
Anabaenavariabilis"phosphoenol      GGG-YPWEAGEIREKLLEEIRLWWRTDELHQFKPTVLDEVDYALHYFQEV 236
Synechococcussp.                    LGTSDSWEIENIQQQLTEEIRLWWRTDELHQFKPQVLDEVDYALHYFEEV 255
Synechococcusvulcanus"phosphoe      LTD---WNAQTLRAQLMEEIRLWWRTDELHQFKPEVLDEVEYTLHYFKEV 286
Streptomycescoelicolor              RRR--------LDTRLAENIDLVWQTDELRVVRPEPADEARNAIYYLDEL 207
Rhodopseudomonaspalustris""pho      WAD--------NEERLRRAVETLWKTNLLRRTKLTVLDEVTNGLSFYDYT 238
                                                   * . :   ::*: ::       *.    :   .
```

FIGURE 2 (continued)

```
gi|79319830|ref|NP_001031179.1      IWKGVP---KFLRRVDTALKNIGIEERVPYNAPLIQFSSWMGGDRDGNPR 294
gi|73917651|sp|Q84VW9|CAPP3_AR      IWKGVP---KFLRRVDTALKNIGIDERVPYNAPLIQFSSWMGGDRDGNPR 294
gi|1705587|sp|P51061|CAPP2_SOY      IWNGVP---RFLRRVDTALNNIGIKERVPYNAPLIQFSSWMGGDRDGNPR 293
gi|399183|sp|Q02735|CAPP_MEDSA      IWKGVP---KFLRRVDTALKNIGINERVPYNAPLIQFSSWMGGDRDGNPR 292
gi|23503558|dbj|BAC20365.1|         IWKGVP---KFLRRVDTALKNIGINERVPYNAPLIQFSSWMGGDRDGNPR 293
gi|115610|sp|P27154|CAPP_TOBAC      IWKGVP---KFLRRVDTALKNIGINERLPYNAPLIQFSSWMGGDRDGNPR 293
gi|972511|emb|CAA62469.1|           IWKGVP---KFLRRVDTALKNIGINERVPYNAPLIQFSSWMGGDRDGNPR 294
gi|30689081|ref|NP_850373.1|        IWKGVP---KFLRRVDTALKNIGINERVPYNAPLIQFSSWMGGDRDGNPR 293
gi|45505269|gb|AAS67006.1|          IWKGVP---QFLRRVDTALKNIGINERVPYNAPVIQFSSWMGGDRDGNPR 294
gi|115583|sp|P29194|CAPP2_SORB      IWKGVP---KFLRRVDTALKNIGINERVPYNAPLIQFSSWMGGDRDGNPR 288
gi|1705585|sp|P51059|CAPP2_MAI      IWKGVP---KFLRRVDTALKNIGINERVPYNAPLIQFSSWMGGDRDGNPR 295
gi|3341490|emb|CAA07610.1|          IWKGVP---KFLRRVDTALKNIGINERVPYNAPLIQFSSWMGGDRDGNPR 300
gi|115478504|ref|NP_001062847.      IWKGVP---KFLRRVDTALKNIGIDERVPYNAPLIQFSSWMGGDRDGNPR 303
gi|115476100|ref|NP_001061646.      IWKGVP---KFLRRLDTALKNIGIDERVPYNAPLIQFSSWMGGDRDGNPR 292
gi|115577|sp|P29193|CAPP1_SACH      IWKGVP---KFLRRVDTALKNIGINERLPYNAPIIQFSSWMGGDRDGNPR 293
gi|115440043|ref|NP_001044301.      IWKGVP---KFLRRVDTALKNIGINERVPYNAPLIQFSSWMGGDRDGNPR 251
gi|9828445|gb|AAG00180.1|AF271      IWKGVP---KFLRRIDTALKNIGINERLPYNAPLIQFSSWMGGDRDGNPR 288
gi|115445275|ref|NP_001046417.      IWKGVP---KFLRRIDTALKNIGINERLPYNAPLIQFSSWMGGDRDGNPR 297
Welwitschiamirabilis"tissue_ty      IWKGVP---KFLRRVDTALKSIGINERLPYNAPLIQFSSWMGGDRDGNPR 282
Welwitschia                         IWKGVP---KFLRRVDTALKSIGINERLPYNAPLIQFSSWMGGDRDGNPR 282
gi|1705590|sp|P51063|CAPP_PICA      IWKGVP---KFLRRIDTALKSIGINERVPYNAPLIQFSSWMGGDRDGNPR 293
Chlamydomonasreinhardtii"           LWAAVP---QYMRRLSAALKKH-TGHDLPLQATPFRFGSWMGGDRDGNPN 276
Chlamydomonas                       LWAAVP---QYMRRLSAALKKH-TGHDLPLQATPFRFGSWMGGDRDGNPN 276
Glycinemax"phosphoenolpyruvate      LWKAVP---HYLRRVSNALKKH-TGKPLPLTCTPIKFGSWMGGDRDGNPN 274
Glycine                             LWKAVP---HYLRRVSNALKKH-TGKPLPLTCTPIKFGSWMGGDRDGNPN 274
Ricinuscommunis"phosphoenolpyr      LWKALP---HYLRRVSTALKKH-TGKPLPLTCTPIRFGSWMGGDRDGNPN 274
Ricinus                             LWKALP---HYLRRVSTALKKH-TGKPLPLTCTPIRFGSWMGGDRDGNPN 274
gi|30697740|ref|NP_177043.2|        LWKAVP---QYLRRVSNSLKKF-TGKPLPLTCTPMKFGSWMGGDRDGNPN 274
Synechococcuselongatus              LFEAIP----LLYQRFRLALQGTFPDLQPPRYNFCQFGSWVGSDRDGNPS 372
Anabaenavariabilis"phosphoenol      LFDGIP----QLYKRFKYALNQTFSWLEPPSKDFCSFGSWVGSDRDGNPS 282
Synechococcussp.                    LFDTLP----ELSVRLQQALKASFPTLKVPTTNFCNFGSWVGGDRDGNPS 301
Synechococcusvulcanus"phosphoe      IFAVIP----KLYRRLEQSLHETFPALQPPRHRFCRFGSWVGGDRDGNPY 332
Streptomycescoelicolor              HLGAVG---DVLEDLTAELERAGVKLPDDTRP--LTFGTWIGGDRDGNPN 252
Rhodopseudomonaspalustris""pho      FLREVPRLHSALEDRLADAAKAEGVNSDGELASFLRMGSWIGGDRDGNPF 288
                                        :      :                        :.:*:*.******
```

FIGURE 2 (continued)

```
gi|79319830|ref|NP_001031179.1      VTPEVTRDVCLLARMMAATMYFNQIEDLMFEMSMWRCNDELRARADEVHA 344
gi|73917651|sp|Q84VW9|CAPP3_AR      VTPEVTRDVCLLARMMAANLYYNQIENLMFELSMWRCTDEFRVRADELHR 344
gi|1705587|sp|P51061|CAPP2_SOY      VTPEVTRDVCLLARMMAANLYYSQIEDLMFELSMWRCNDELRVRAEELHR 343
gi|399183|sp|Q02735|CAPP_MEDSA      VTPEVTRDVCLLARMMAANLYYSQIEDLMFELSMWRCNDELRVRAEELHR 342
gi|23503558|dbj|BAC20365.1|         VTPEVTRDVCLLARMMAANLYYSQIEDLMFELSMWRCNDELRVRAEELTS 343
gi|115610|sp|P27154|CAPP_TOBAC      VTLEVTRDVCLLARMMAANLYYSQIEELMFELSMWRCNDDLRIRAAELYR 343
gi|972511|emb|CAA62469.1|           VTPEVTRDVCLLARMMAANLYYSQIEDLMFELSMWRCNEELRVRADDLQR 344
gi|30689081|ref|NP_850373.1|        VTPEVTRDVCLLARMMAANLYFSQIEDLMFEMSMWRCNEELRVRA-ERQR 342
gi|45505269|gb|AAS67006.1|          VTPEVTRDVCLLARMMAANMYFSQIEDLMFELSMWRCTDELRVRAHELHR 344
gi|115583|sp|P29194|CAPP2_SORB      VTPEVTRDVCLLARMMASNLYCSQIEDLMFELSMWRCSDELRMRADELHR 338
gi|1705585|sp|P51059|CAPP2_MAI      VTPEVTRDVCLLARMMASNLYCSQIEDLMFELSMWRCSDELRMRADVLHL 345
gi|3341490|emb|CAA07610.1|          VTPEVTRDVCLLARMMAANLYCAQIEDLMFELSMWRCNDELRSRADELHR 350
gi|115478504|ref|NP_001062847.      VTPEVTRDVCLLARMMAANLYCSQIEDLMFELSMWRCNEELRSRADDLHR 353
gi|115476100|ref|NP_001061646.      VTPEVTRDVCLLARMMASNLYCSQIEDLMFELSMWRCNDELRARADELHL 342
gi|115577|sp|P29193|CAPP1_SACH      VTPEITRDVCLLARMMAANLYNAQIEDLMFELSMWRCSDELRVKVDELHR 343
gi|115440043|ref|NP_001044301.      VTPEVTRDVCLLARMMAANLYYAQIEDLMFELSMWRCSDELRVKADQLHR 301
gi|9828445|gb|AAG00180.1|AF271      VTPEVTRDVCLLARMMAANLYFSQIEDLMFELSMWRCSDELRIRADDLHC 338
gi|115445275|ref|NP_001046417.      VTPEVTRDVCLLARMMAANLYFSQIEDLMFELSMWRCSDELRIRADDLHC 347
Welwitschiamirabilis"tissue_ty      VTPEVTRDVCLLARMMATNLYYSQIEDLMFELSMWRCSDELRQRALQLHN 332
Welwitschia                         VTPEVTRDVCLLARMMATNLYYSQIEDLMFELSMWRCSDELRQRALQLHN 332
gi|1705590|sp|P51063|CAPP_PICA      VTPEVTRDVCLLARMMAANLYYSQIEDLMFELSMWRCSDELRARALQLHS 343
Chlamydomonasreinhardtii"           VTAKVTAHVTALARWMAADLYLREIDTLRFELSMNQCSAAVWKMARRIIA 326
Chlamydomonas                       VTAKVTAHVTALARWMAADLYLREIDTLRFELSMNQCSAAVWKMARRIIA 326
Glycinemax"phosphoenolpyruvate      VTAKVTKDVSLLSRWMAIDLYIREVDSLRFELSMNQCSDRLSRLAHEILE 324
Glycine                             VTAKVTKDVSLLSRWMAIDLYIREVDSLRFELSMNQCSDRLSRLAHEILE 324
Ricinuscommunis"phosphoenolpyr      VTAKVTRDVSLLSRWMAVDLYIREVDSLRFELSMVQCSDRLLKVANDILI 324
Ricinus                             VTAKVTRDVSLLSRWMAVDLYIREVDSLRFELSMVQCSDRLLKVANDILI 324
gi|30697740|ref|NP_177043.2|        VTAKVTKEVSLLSRWMAIDLYIREVDSLRFELSTDRCSDRFSRLADKILE 324
Synechococcuselongatus              VTSAVTWQTACYQRSLVLDRYITAVEHLRNVLSLSMHWSEVLPELLSSLE 422
Anabaenavariabilis"phosphoenol      VTPEITWQTACYQRKMVLERYIKSVTQLIELLSISMHWSDVLPDLLESLE 332
Synechococcussp.                    VTPDVTWKTACYQRGLVLERYIASVESLSDVLSLSLHWSNVLPDLLHSSY 351
Synechococcusvulcanus"phosphoe      VKPEVTWQTACYQRNLVLEEYIKSVERLINLLSLSLHWCDVLPDLLDSLE 382
Streptomycescoelicolor              VTPQVTWDVLILQHEHGINDALEMIDELRGFLSNSIRYAGATEELLASLQ 302
Rhodopseudomonaspalustris""pho      VTAEVLHGTLKLQSTRVLRYYLDELHELGSELSLASHLAGTTDTVKA--- 335
                                    *.  :   .                  :  *   :*
```

FIGURE 2 (continued)

```
gi|79319830|ref|NP_001031179.1      NSRKD-A----AKHYIEFWK------------------------------ 359
gi|73917651|sp|Q84VW9|CAPP3_AR      NSRKD-A----AKHYIEFWK------------------------------ 359
gi|1705587|sp|P51061|CAPP2_SOY      SSKKDEV----AKHYIEFWK------------------------------ 359
gi|399183|sp|Q02735|CAPP_MEDSA      NSKKDEV----AKHYIEFWK------------------------------ 358
gi|23503558|dbj|BAC20365.1|         SSNKDAV----AKHYIEFWK------------------------------ 359
gi|115610|sp|P27154|CAPP_TOBAC      SSRRD------TKHYIEFWK------------------------------ 357
gi|972511|emb|CAA62469.1|           SSRRD------EKHYIEFWK------------------------------ 358
gi|30689081|ref|NP_850373.1|        CAKRD------AKHYIEFWK------------------------------ 356
gi|45505269|gb|AAS67006.1|          SSKRD------AKHYIEFWK------------------------------ 358
gi|115583|sp|P29194|CAPP2_SORB      STKKD------AKHYIEFWK------------------------------ 352
gi|1705585|sp|P51059|CAPP2_MAI      STKKD------AKHYIEFWK------------------------------ 359
gi|3341490|emb|CAA07610.1|          SSKKD------AKHYIEFWK------------------------------ 364
gi|115478504|ref|NP_001062847.      SSKKD------AKHYIEFWK------------------------------ 367
gi|115476100|ref|NP_001061646.      SSKKD------AKHYIEFWK------------------------------ 356
gi|115577|sp|P29193|CAPP1_SACH      SSKKDT-----TKHYIEFWK------------------------------ 358
gi|115440043|ref|NP_001044301.      CAKKNT-----TKHYIEFWK------------------------------ 316
gi|9828445|gb|AAG00180.1|AF271      SSRKA------AKHYIEFWK------------------------------ 352
gi|115445275|ref|NP_001046417.      SSRKA------AKHYIEFWK------------------------------ 361
Welwitschiamirabilis"tissue_ty      SAKRD------AKHYIEFWK------------------------------ 346
Welwitschia                         SAKRD------AKHYIEFWK------------------------------ 346
gi|1705590|sp|P51063|CAPP_PICA      ASKKD------AKHYIEFWK------------------------------ 357
Chlamydomonasreinhardtii"           EGHTKRAGVVRAKAAAALHQTATDAASHGGSAASAAAAAAAGGDVVADGT 376
Chlamydomonas                       EGHTKRAGVVRAKAAAALHQTATDAASHGGSAASAAAAAAAGGDVVADGT 376
Glycinemax"phosphoenolpyruvate      AKH--------ENRRENWNQSANRS------------------------- 341
Glycine                             AKH--------ENRRENWNQSANRS------------------------- 341
Ricinuscommunis"phosphoenolpyr      EETSS------EDHHESWNQPASRSQTK---------------------- 346
Ricinus                             EETSS------EDHHESWNQPASRSQTK---------------------- 346
gi|30697740|ref|NP_177043.2|        KDY--------DRGKSNFQKQQSSS------------------------- 341
Synechococcuselongatus              QESML-----FPETYEQLAV------------------------------ 437
Anabaenavariabilis"phosphoenol      LDQSQ-----LSEVYDALAL------------------------------ 347
Synechococcussp.                    QDQNI-----FPDVYQSLAS------------------------------ 366
Synechococcusvulcanus"phosphoe      QDQRQ-----LPSIYEQYAV------------------------------ 397
Streptomycescoelicolor              ADLER-----LPEISPRYKR------------------------------ 317
Rhodopseudomonaspalustris""pho      ----------LAETSPDTSP------------------------------ 345
```

**FIGURE 2 (continued)**

```
gi|79319830|ref|NP_001031179.1     --------------------------------------------------
gi|73917651|sp|Q84VW9|CAPP3_AR     --------------------------------------------------
gi|1705587|sp|P51061|CAPP2_SOY     --------------------------------------------------
gi|399183|sp|Q02735|CAPP_MEDSA     --------------------------------------------------
gi|23503558|dbj|BAC20365.1|        --------------------------------------------------
gi|115610|sp|P27154|CAPP_TOBAC     --------------------------------------------------
gi|972511|emb|CAA62469.1|          --------------------------------------------------
gi|30689081|ref|NP_850373.1|       --------------------------------------------------
gi|45505269|gb|AAS67006.1|         --------------------------------------------------
gi|115583|sp|P29194|CAPP2_SORB     --------------------------------------------------
gi|1705585|sp|P51059|CAPP2_MAI     --------------------------------------------------
gi|3341490|emb|CAA07610.1|         --------------------------------------------------
gi|115478504|ref|NP_001062847.     --------------------------------------------------
gi|115476100|ref|NP_001061646.     --------------------------------------------------
gi|115577|sp|P29193|CAPP1_SACH     --------------------------------------------------
gi|115440043|ref|NP_001044301.     --------------------------------------------------
gi|9828445|gb|AAG00180.1|AF271     --------------------------------------------------
gi|115445275|ref|NP_001046417.     --------------------------------------------------
Welwitschiamirabilis"tissue_ty     --------------------------------------------------
Welwitschia                        --------------------------------------------------
gi|1705590|sp|P51063|CAPP_PICA     --------------------------------------------------
Chlamydomonasreinhardtii"          SGGGAAAAAGPAAAAAADDAFTFSRLGRPRPERPSTDVRSVGVLAGGEGA 426
Chlamydomonas                      SGGGAAAAAGPAAAAAADDAFTFSRLGRPRPERPSTDVRSVGVLAGGEGA 426
Glycinemax"phosphoenolpyruvate     -----------------------LTLPTQLPAR----------------A 352
Glycine                            -----------------------LTLPTQLPAR----------------A 352
Ricinuscommunis"phosphoenolpyr     --------------------FPRKSLPTQLPPR----------------A 360
Ricinus                            --------------------FPRKSLPTQLPPR----------------A 360
gi|30697740|ref|NP_177043.2|       ------------------------CLPTQLPAR----------------A 351
Synechococcuselongatus             --------------------------------------------------
Anabaenavariabilis"phosphoenol     --------------------------------------------------
Synechococcussp.                   --------------------------------------------------
Synechococcusvulcanus"phosphoe     --------------------------------------------------
Streptomycescoelicolor             --------------------------------------------------
Rhodopseudomonaspalustris""pho     --------------------------------------------------
```

FIGURE 2 (continued)

```
gi|79319830|ref|NP_001031179.1      --------------------------------------------------
gi|73917651|sp|Q84VW9|CAPP3_AR      --------------------------------------------------
gi|1705587|sp|P51061|CAPP2_SOY      --------------------------------------------------
gi|399183|sp|Q02735|CAPP_MEDSA      --------------------------------------------------
gi|23503558|dbj|BAC20365.1|         --------------------------------------------------
gi|115610|sp|P27154|CAPP_TOBAC      --------------------------------------------------
gi|972511|emb|CAA62469.1|           --------------------------------------------------
gi|30689081|ref|NP_850373.1|        --------------------------------------------------
gi|45505269|gb|AAS67006.1|          --------------------------------------------------
gi|115583|sp|P29194|CAPP2_SORB      --------------------------------------------------
gi|1705585|sp|P51059|CAPP2_MAI      --------------------------------------------------
gi|3341490|emb|CAA07610.1|          --------------------------------------------------
gi|115478504|ref|NP_001062847.      --------------------------------------------------
gi|115476100|ref|NP_001061646.      --------------------------------------------------
gi|115577|sp|P29193|CAPP1_SACH      --------------------------------------------------
gi|115440043|ref|NP_001044301.      --------------------------------------------------
gi|9828445|gb|AAG00180.1|AF271      --------------------------------------------------
gi|115445275|ref|NP_001046417.      --------------------------------------------------
Welwitschiamirabilis"tissue_ty      --------------------------------------------------
Welwitschia                         --------------------------------------------------
gi|1705590|sp|P51063|CAPP_PICA      --------------------------------------------------
Chlamydomonasreinhardtii"           AFPGGMILGTQPVSAHTAAEVSVPHELPGQDVEGGSEMDFNESRRASDAG 476
Chlamydomonas                       AFPGGMILGTQPVSAHTAAEVSVPHELPGQDVEGGSEMDFNESRRASDAG 476
Glycinemax"phosphoenolpyruvate      HLP-SIAEN--GESRHPRLDIPAPDYMQSNHKDGGVSVSSTTS------- 392
Glycine                             HLP-SIAEN--GESRHPRLDIPAPDYMQSNHKDGGVSVSSTTS------- 392
Ricinuscommunis"phosphoenolpyr      DLP-ACTECNDGESQYPKLELPGTDYMPFNRQE-ALGSSYSES------- 401
Ricinus                             DLP-ACTECNDGESQYPKLELPGTDYMPFNRQE-ALGSSYSES------- 401
gi|30697740|ref|NP_177043.2|        HLP-ACIDF--GESRHTKFEIATTDYMPPNLQK-QNEQDFSES------- 390
Synechococcuselongatus              --------------------------------------------------
Anabaenavariabilis"phosphoenol      --------------------------------------------------
Synechococcussp.                    --------------------------------------------------
Synechococcusvulcanus"phosphoe      --------------------------------------------------
Streptomycescoelicolor              --------------------------------------------------
Rhodopseudomonaspalustris""pho      --------------------------------------------------
```

FIGURE 2 (continued)

```
gi|79319830|ref|NP_001031179.1      --------------------------------------------------
gi|73917651|sp|Q84VW9|CAPP3_AR      --------------------------------------------------
gi|1705587|sp|P51061|CAPP2_SOY      --------------------------------------------------
gi|399183|sp|Q02735|CAPP_MEDSA      --------------------------------------------------
gi|23503558|dbj|BAC20365.1|         --------------------------------------------------
gi|115610|sp|P27154|CAPP_TOBAC      --------------------------------------------------
gi|972511|emb|CAA62469.1|           --------------------------------------------------
gi|30689081|ref|NP_850373.1|        --------------------------------------------------
gi|45505269|gb|AAS67006.1|          --------------------------------------------------
gi|115583|sp|P29194|CAPP2_SORB      --------------------------------------------------
gi|1705585|sp|P51059|CAPP2_MAI      --------------------------------------------------
gi|3341490|emb|CAA07610.1|          --------------------------------------------------
gi|115478504|ref|NP_001062847.      --------------------------------------------------
gi|115476100|ref|NP_001061646.      --------------------------------------------------
gi|115577|sp|P29193|CAPP1_SACH      --------------------------------------------------
gi|115440043|ref|NP_001044301.      --------------------------------------------------
gi|9828445|gb|AAG00180.1|AF271      --------------------------------------------------
gi|115445275|ref|NP_001046417.      --------------------------------------------------
Welwitschiamirabilis"tissue_ty      --------------------------------------------------
Welwitschia                         --------------------------------------------------
gi|1705590|sp|P51063|CAPP_PICA      --------------------------------------------------
Chlamydomonasreinhardtii"           DLGASQHPMLGGPSAGASAEPTAHGYTTTATAAAAAADGTQPEPEVPGTP 526
Chlamydomonas                       DLGASQHPMLGGPSAGASAEPTAHGYTTTATAAAAAADGTQPEPEVPGTP 526
Glycinemax"phosphoenolpyruvate      KLANPNTRLPGTSS----ANSS-------ASSAALGQKKLYAESQT---- 427
Glycine                             KLANPNTRLPGTSS----ANSS-------ASSAALGQKKLYAESQT---- 427
Ricinuscommunis"phosphoenolpyr      SSQDINHGLPKTTGNGSVANSSGSPRASFSSAQLVAQRKLFAESKI---- 447
Ricinus                             SSQDINHGLPKTTGNGSVANSSGSPRASFSSAQLVAQRKLFAESKI---- 447
gi|30697740|ref|NP_177043.2|        DWEKIDNGS--RSGLTSRGSFS-------STSQLLLQRKLFEESQV---- 427
Synechococcuselongatus              --------------------------------------------------
Anabaenavariabilis"phosphoenol      --------------------------------------------------
Synechococcussp.                    --------------------------------------------------
Synechococcusvulcanus"phosphoe      --------------------------------------------------
Streptomycescoelicolor              --------------------------------------------------
Rhodopseudomonaspalustris""pho      --------------------------------------------------
```

FIGURE 2 (continued)

```
gi|79319830|ref|NP_001031179.1      --------------------------------------------------
gi|73917651|sp|Q84VW9|CAPP3_AR      --------------------------------------------------
gi|1705587|sp|P51061|CAPP2_SOY      --------------------------------------------------
gi|399183|sp|Q02735|CAPP_MEDSA      --------------------------------------------------
gi|23503558|dbj|BAC20365.1|         --------------------------------------------------
gi|115610|sp|P27154|CAPP_TOBAC      --------------------------------------------------
gi|972511|emb|CAA62469.1|           --------------------------------------------------
gi|30689081|ref|NP_850373.1|        --------------------------------------------------
gi|45505269|gb|AAS67006.1|          --------------------------------------------------
gi|115583|sp|P29194|CAPP2_SORB      --------------------------------------------------
gi|1705585|sp|P51059|CAPP2_MAI      --------------------------------------------------
gi|3341490|emb|CAA07610.1|          --------------------------------------------------
gi|115478504|ref|NP_001062847.      --------------------------------------------------
gi|115476100|ref|NP_001061646.      --------------------------------------------------
gi|115577|sp|P29193|CAPP1_SACH      --------------------------------------------------
gi|115440043|ref|NP_001044301.      --------------------------------------------------
gi|9828445|gb|AAG00180.1|AF271      --------------------------------------------------
gi|115445275|ref|NP_001046417.      --------------------------------------------------
Welwitschiamirabilis"tissue_ty      --------------------------------------------------
Welwitschia                         --------------------------------------------------
gi|1705590|sp|P51063|CAPP_PICA      --------------------------------------------------
Chlamydomonasreinhardtii"           SYADPGTPDRLGALPGPFTPGPTPFREAANAAMSTAASGGAGGGGGGGAN 576
Chlamydomonas                       SYADPGTPDRLGALPGPFTPGPTPFREAANAAMSTAASGGAGGGGGGGAN 576
Glycinemax"phosphoenolpyruvate      --------------------GKSTFQKLLEPML----------------- 440
Glycine                             --------------------GKSTFQKLLEPML----------------- 440
Ricinuscommunis"phosphoenolpyr      --------------------GRSSFQKLLEPSL----------------- 460
Ricinus                             --------------------GRSSFQKLLEPSL----------------- 460
gi|30697740|ref|NP_177043.2|        --------------------GKTSFQKLLEPPP----------------- 440
Synechococcuselongatus              --------------------------------------------------
Anabaenavariabilis"phosphoenol      --------------------------------------------------
Synechococcussp.                    --------------------------------------------------
Synechococcusvulcanus"phosphoe      --------------------------------------------------
Streptomycescoelicolor              --------------------------------------------------
Rhodopseudomonaspalustris""pho      --------------------------------------------------
```

FIGURE 2 (continued)

```
gi|79319830|ref|NP_001031179.1       ---------------------------------SIPTTEPYRVILGDVRD 376
gi|73917651|sp|Q84VW9|CAPP3_AR       ---------------------------------TIPPTEPYRVILGDVRD 376
gi|1705587|sp|P51061|CAPP2_SOY       ---------------------------------KVPPNEPYRVVLGEVRD 376
gi|399183|sp|Q02735|CAPP_MEDSA       ---------------------------------KIPLNEPYRVVLGEVRD 375
gi|23503558|dbj|BAC20365.1|          ---------------------------------NIPPSEPYRVVLGEVRN 376
gi|115610|sp|P27154|CAPP_TOBAC       ---------------------------------TIPPSEPYRVILGDVRD 374
gi|972511|emb|CAA62469.1|            ---------------------------------QVPPNEPYRVILGDVRD 375
gi|30689081|ref|NP_850373.1|         ---------------------------------QIPANEPYRAILGDVRD 373
gi|45505269|gb|AAS67006.1|           ---------------------------------QIPPNEPYRVILGDVRD 375
gi|115583|sp|P29194|CAPP2_SORB       ---------------------------------KVPPNEPYRVILSDVRD 369
gi|1705585|sp|P51059|CAPP2_MAI       ---------------------------------KVPPNEPYRVILSDVRD 376
gi|3341490|emb|CAA07610.1|           ---------------------------------KVPPNEPYRVILGDVRD 381
gi|115478504|ref|NP_001062847.       ---------------------------------KVPLNEPYRVILSDVRD 384
gi|115476100|ref|NP_001061646.       ---------------------------------KVPPSEPYRVVLGDVRD 373
gi|115577|sp|P29193|CAPP1_SACH       ---------------------------------QVPPSEPYRVILSDVRD 375
gi|115440043|ref|NP_001044301.       ---------------------------------QVPPSEPYRVILSNVRD 333
gi|9828445|gb|AAG00180.1|AF271       ---------------------------------QIPPNEPYRVILGGVRD 369
gi|115445275|ref|NP_001046417.       ---------------------------------QIPPNEPYRVILGGVRD 378
Welwitschiamirabilis"tissue_ty       ---------------------------------QVPPNEPFRVILGDVRD 363
Welwitschia                          ---------------------------------QVPPNEPFRVILGDVRD 363
gi|1705590|sp|P51063|CAPP_PICA       ---------------------------------QIPPNEPFRVILGDVRD 374
Chlamydomonasreinhardtii"            RAASGLGGDPTFTRRSLMAQRLGTSSVQFARAHEHPGFHPYRIVLGHVRD 626
Chlamydomonas                        RAASGLGGDPTFTRRSLMAQRLGTSSVQFARAHEHPGFHPYRIVLGHVRD 626
Glycinemax"phosphoenolpyruvate       --------------------------------PQLPGIAPYRIVLGNVKD 458
Glycine                              --------------------------------PQLPGIAPYRIVLGNVKD 458
Ricinuscommunis"phosphoenolpyr       --------------------------------PQRPGIAPYRIVLGNVKD 478
Ricinus                              --------------------------------PQRPGIAPYRIVLGNVKD 478
gi|30697740|ref|NP_177043.2|         --------------------------------LKRAGSAPYRIVLGEVKE 458
Synechococcuselongatus               ----------------------------------RYRQEPYRLKLSYILE 453
Anabaenavariabilis"phosphoenol       ----------------------------------RYRQEPYRLKLAYVLK 363
Synechococcussp.                     ----------------------------------RHRQEPYRLKSAYIKR 382
Synechococcusvulcanus"phosphoe       ----------------------------------RYRQEPYRLKLAYVLK 413
Streptomycescoelicolor               ----------------------------------LNAEEPYRLKATCIRQ 333
Rhodopseudomonaspalustris""pho       ----------------------------------HRKYEPYRLAVSGIYA 361
                                                                             *:*      :
```

FIGURE 2 (continued)

```
gi|79319830|ref|NP_001031179.1      KLYHTRERAHQLLSNGH-------SDVPVEAT--------------FINL 405
gi|73917651|sp|Q84VW9|CAPP3_AR      KLYHTRERSRQLLSNGI-------SDIPEEAT--------------FTNV 405
gi|1705587|sp|P51061|CAPP2_SOY      RLYQTRERSRHLLSNGY-------SDIPEEAT--------------FTNV 405
gi|399183|sp|Q02735|CAPP_MEDSA      KLYRTRERSRYLLAHGY-------CEIPEEAT--------------FTNV 404
gi|23503558|dbj|BAC20365.1|         RLYQTRERSRHLLANEY-------SDILEEHT--------------FTNV 405
gi|115610|sp|P27154|CAPP_TOBAC      KLYQTRERTRQMLAHGI-------SDIPEDAT--------------YNNV 403
gi|972511|emb|CAA62469.1|           KLYQTRERARQLLGHGY-------SEIPEEAT--------------YTNI 404
gi|30689081|ref|NP_850373.1|        KLYNTRERARQLLSSGV-------SDVPEDAV--------------FTSV 402
gi|45505269|gb|AAS67006.1|          KLYNIRERARHLLANGT-------SDIPEETT--------------FTNV 404
gi|115583|sp|P29194|CAPP2_SORB      KLYNTRERSRELLSSGH-------SDIPEEAT--------------LTTV 398
gi|1705585|sp|P51059|CAPP2_MAI      KLYNTRERSRELLSSGH-------SDIPEEAT--------------LTNV 405
gi|3341490|emb|CAA07610.1|          NLYNTRERSRELLSSGH-------SDIPEEAT--------------LTNL 410
gi|115478504|ref|NP_001062847.      KLYNTRERSRELLSSGY-------CDIPEEAT--------------LTNV 413
gi|115476100|ref|NP_001061646.      KLYNTRERARQLLSSGY-------SDIPEETT--------------LTSV 402
gi|115577|sp|P29193|CAPP1_SACH      KLYNTRERARHLLASGF-------SEIPEEAT--------------FTDV 404
gi|115440043|ref|NP_001044301.      KLYNTRERARHLLASGF-------SEIPDEAT--------------FTDV 362
gi|9828445|gb|AAG00180.1|AF271      KLYYTRERTRHLLTTGV-------SEIPEEAT--------------FTNV 398
gi|115445275|ref|NP_001046417.      KLYYTRERTRHLLTTGV-------SEIPEEAT--------------FTNV 407
Welwitschiamirabilis"tissue_ty      KLYNTRERTRQLLANGF-------SDVPEEA---------------LTSV 391
Welwitschia                         KLYNTRERTRQLLANGF-------SDVPEEA---------------LTSV 391
gi|1705590|sp|P51063|CAPP_PICA      KLYNTRERTRQLLSNGI-------SDIPEEVT--------------FTNI 403
Chlamydomonasreinhardtii"           RLAATRRRMEDLLSGRE-------PAGEAHGGVGAGGGGGGGAAPWYESE 669
Chlamydomonas                       RLAATRRRMEDLLSGRE-------PAGEAHGGVGAGGGGGGGAAPWYESE 669
Glycinemax"phosphoenolpyruvate      KLEKSRRRLEILLEDVA-------CDYDPLD--------------YYETS 487
Glycine                             KLEKSRRRLEILLEDVA-------CDYDPLD--------------YYETS 487
Ricinuscommunis"phosphoenolpyr      KLMRTRRRLELLLEDLP-------CEYDQWD--------------YYETT 507
Ricinus                             KLMRTRRRLELLLEDLP-------CEYDQWD--------------YYETT 507
gi|30697740|ref|NP_177043.2|        KLVKTRRLLELLIEGLP-------CEYDPKN--------------SYETS 487
Synechococcuselongatus              RLHNTRDRNTRLQQQEKDPTTPLPEYRDGTL--------------YQAG 489
Anabaenavariabilis"phosphoenol      RLENTRDRNLALYKGET-------PTNEDSPM--------------YRSG 392
Synechococcussp.                    RLENTLERNRRLANMPAWEN---KVEAADDKV--------------YICG 415
Synechococcusvulcanus"phosphoe      RLQNTRDRNRALQTYCIRRNE--AEELNNGQF--------------YRHG 447
Streptomycescoelicolor              KLENTKQRLAKG------------TPHEDGRD--------------YLGT 357
Rhodopseudomonaspalustris""pho      RLAATALKLEVEN---------LRAPVGEAEP--------------YASA 388
                                    .*
```

FIGURE 2 (continued)

```
gi|79319830|ref|NP_001031179.1      EQFLEPLELCYRSLCSCGDRPIADGSLLDFLRQVSTFGLSLVRLDIRQES 455
gi|73917651|sp|Q84VW9|CAPP3_AR      EQFLEPLELCYRSLCSCGDSPIADGSLLDFLRQVSTFGLSLVRLDIRQES 455
gi|1705587|sp|P51061|CAPP2_SOY      EEFLESLELCYRSLCACGDRAIADGSLLDFMRQVSTFGLSLVRLDIRQES 455
gi|399183|sp|Q02735|CAPP_MEDSA      DEFLEPLELCYRSLCACGDRAIADGSLLDFLRQVSTFGLSLVRLDIRQES 454
gi|23503558|dbj|BAC20365.1|         EEFLEPLELCYRSLCACGDRAIADGSLLDFLRQVSTFGLSLVRLDIRQES 455
gi|115610|sp|P27154|CAPP_TOBAC      EQFLEPLELCYRSLCECGDRPIADGSLLDFLRQVSTFGLSFVRLDIRQES 453
gi|972511|emb|CAA62469.1|           EQFLEPLELCYRSLCACGDLSIADGSLLDFLRQVSTFGLSLVRLDIRQES 454
gi|30689081|ref|NP_850373.1|        DQFLEPLELCYRSLCDCGDRPIADGSLLDFLRQVSTFGLALVKLDIRQES 452
gi|45505269|gb|AAS67006.1|          EQFLEPLELCYRSLCACGDRPIADGSLLDFLRQVSTFGLSLVRLDIRQES 454
gi|115583|sp|P29194|CAPP2_SORB      EQLLEPLELCYRSLCACGDRVIADGSLLDFLRQVSTFGLSLVRLDIRQES 448
gi|1705585|sp|P51059|CAPP2_MAI      EQLLEPLELCYRSLCACGDSVIADGTLLDFLRQVSTFGLSLVRLDIRQES 455
gi|3341490|emb|CAA07610.1|          EQLLEPLELCYRSLCACGDRVIADGTLLDFLRQVSTFGLSLVKLDIRQES 460
gi|115478504|ref|NP_001062847.      EQLLEPLELCYRSLCACGDRAIADGSLLDFLRQVSTFGLSLVRLDIRQES 463
gi|115476100|ref|NP_001061646.      EQFLEPLELCYRSLCDCGDRVIADGTLLDFLRQVSTFGLCLVRLDIRQES 452
gi|115577|sp|P29193|CAPP1_SACH      EQFLEPLELCYRSLCACGDRSVADGSLLDFLRQVSTFGLSLVRLDIRQES 454
gi|115440043|ref|NP_001044301.      EQFLEPLELCYRSLCACGDNSIADGSLLDFLRQVSTFGLSLVRLDIRQES 412
gi|9828445|gb|AAG00180.1|AF271      EEFLEPLELCYRSLCACGDKPIADGSLLDFLRQVSTFGLALVKLDIRQES 448
gi|115445275|ref|NP_001046417.      EEFLEPLELCYRSLCACGDKPIADGSLLDFLRQVSTFGLALVKLDIRQES 457
Welwitschiamirabilis"tissue_ty      DQLLEPLELCYRSLCSTGDQPIADGSLLDFMRQVSTFGLTLVKLDIRQES 441
Welwitschia                         DQLLEPLELCYRSLCSTGDQPIADGSLLDFMRQVSTFGLTLVKLDIRQES 441
gi|1705590|sp|P51063|CAPP_PICA      DEFLEPLELCYRSLCSTGDQPIADGSLLDFMRQVSTFGLSFVKLDIRQES 453
Chlamydomonasreinhardtii"           DELAEPLMACYWSLWECGGGVIADGRLLDLIRRVYTFGMCLMKLDLRQES 719
Chlamydomonas                       DELAEPLMACYWSLWECGGGVIADGRLLDLIRRVYTFGMCLMKLDLRQES 719
Glycinemax"phosphoenolpyruvate      DQLLEPLLLCYESLQSCGSGVLADGRLADLIRRVATFGMVLMKLDLRQES 537
Glycine                             DQLLEPLLLCYESLQSCGSGVLADGRLADLIRRVATFGMVLMKLDLRQES 537
Ricinuscommunis"phosphoenolpyr      DQLLDPLLLCYESLQSCGAGVLADGRLADLIRRVATFGMVLMKLDLRQES 557
Ricinus                             DQLLDPLLLCYESLQSCGAGVLADGRLADLIRRVATFGMVLMKLDLRQES 557
gi|30697740|ref|NP_177043.2|        DQLLEPLLLCYESLQSSGARVLADGRLADLIRRVSTFGMVLVKLDLRQEA 537
Synechococcuselongatus              TAFLEDLKLIQHNLKQTG---LSCYELEKLICQVEIFGFNLVHLDIRQES 536
Anabaenavariabilis"phosphoenol      SEFLAELRLIQHNLTETG---LSCRELDNLICQVEIFDFNLTKLDIRQES 439
Synechococcussp.                    QEFLADLKLIRESLVQTE---INCAALDKLICQVEIFSFVLTRLDFRQES 462
Synechococcusvulcanus"phosphoe      EEFLAELLLIQRNLKETG---LACRELDDLICQVEVFGFNLAALDIRQES 494
Streptomycescoelicolor              AQLIDDLRIVQTSLREHRGGLFADGRLARTIRTLAAFGLQLATMDVREHA 407
Rhodopseudomonaspalustris""pho      QDFKADLDAIHLSLTQHNSGVIARGRLRQLRRAIDCFGFHLASLDMRQNS 438
                                      :   *     .*       .    *      :  *.: :  :*.*:.:
```

FIGURE 2 (continued)

```
gi|79319830|ref|NP_001031179.1     DRHTDVLDAITTHL-DIG-SYREWSEERRQEWLLSELSGKRPLFGSDLPK 503
gi|73917651|sp|Q84VW9|CAPP3_AR     ERHTDVLDAITKHL-DIGSSYRDWSEEGRQEWLLAELSGKRPLFGPDLPK 504
gi|1705587|sp|P51061|CAPP2_SOY     DRHTDVLDAITKHL-EIG-SYQEWSEEKRQEWLLSELSGKRPLFGPDLPQ 503
gi|399183|sp|Q02735|CAPP_MEDSA     DRHTDVMDAITKHL-EIG-SYQEWSEEKRQEWLLSELIGKRPLFGPDLPQ 502
gi|23503558|dbj|BAC20365.1|        DRLTDVLDAITKHL-GIG-SYLEWSEEKRQQWLLSELSGKRPLFGPDLPQ 503
gi|115610|sp|P27154|CAPP_TOBAC     DRHTDVLDAITQHL-EIG-SYREWSEERRQEWLLSELSGKRPLFGPDLPR 501
gi|972511|emb|CAA62469.1|          DRHTDVLDAITQHL-EIG-SYRDWSEERRQEWLLSELSGKRPLFGPDLPK 502
gi|30689081|ref|NP_850373.1|       ERHSDVLDAITTHL-GIG-SYKEWSEDKRQEWLLSELSGKRPLFGPDLPK 500
gi|45505269|gb|AAS67006.1|         DRHTDVMDAITKHL-DIG-SYREWPEEKRQEWLLSELSGKRPLFGHDLPK 502
gi|115583|sp|P29194|CAPP2_SORB     DRHTDVLDAITTYL-GIG-SYREWPEERRQEWLLSELNGKRPLFGPDLPK 496
gi|1705585|sp|P51059|CAPP2_MAI     DRHTDVLDAITTYL-GIG-SYREWTEERRQEWLLSELNGKRPLFGSDLPK 503
gi|3341490|emb|CAA07610.1|         DRHTDALDAITSYL-GIG-SYREWSEEHRQEWLLSELNGKRPLFGADLPM 508
gi|115478504|ref|NP_001062847.     DRHTDVLDAITTYL-GIG-SYREWSEERRQEWLLSELNGKRPLFGPDLPR 511
gi|115476100|ref|NP_001061646.     DRHTDVLDAITTYL-GIG-SYREWSEERRQDWLLSELNGKRPLFGPDLPK 500
gi|115577|sp|P29193|CAPP1_SACH     DRHTDVMDAITEYL-GIG-SYRKWTEEKRQEWLLSELNGKRPLFGPDLPK 502
gi|115440043|ref|NP_001044301.     DRHTDVMDAITQYL-GIG-SYREWSEEKRQEWLLSELNGKRPLFGPDLPQ 460
gi|9828445|gb|AAG00180.1|AF271     DRHTDVLDAITTYL-GIG-SYAEWSEEKRQDWLLSELRGKRPLFGPDLPQ 496
gi|115445275|ref|NP_001046417.     DRHTDVLDAITTYL-GIG-SYAEWSEEKRQDWLLSELRGKRPLFGPDLPQ 505
Welwitschiamirabilis"tissue_ty     DRHTDVIDAITRHL-GLG-SYKEWPEDKRQEWLLAELRGKRPLFGPDLPT 489
Welwitschia                        DRHTDVIDAITRHL-GLG-SYKEWPEDKRQEWLLAELRGKRPLFGPDLPT 489
gi|1705590|sp|P51063|CAPP_PICA     DRHSDVADAITRHL-GIG-SYKEWSEEQRQAWLLSELQGKRPLFGPDLPK 501
Chlamydomonasreinhardtii"          TRHAEALDAVTSYL-GLG-SYLEWSEDQKIEWLTKELQGRRPLIPADMPM 767
Chlamydomonas                      TRHAEALDAVTSYL-GLG-SYLEWSEDQKIEWLTKELQGRRPLIPADMPM 767
Glycinemax"phosphoenolpyruvate     GRHAEALDAITQYL-DMG-TYSEWDEEKKLDFLTRELKGKRPLVPVSIEV 585
Glycine                            GRHAEALDAITQYL-DMG-TYSEWDEEKKLDFLTRELKGKRPLVPVSIEV 585
Ricinuscommunis"phosphoenolpyr     GRHADTLDAITKYL-EMG-TYSEWDEEKKLEFLTRELKGKRPLVPPTIEV 605
Ricinus                            GRHADTLDAITKYL-EMG-TYSEWDEEKKLEFLTRELKGKRPLVPPTIEV 605
gi|30697740|ref|NP_177043.2|       ARHSEALDAITTYL-DMG-TYSEWDEEKKLEFLTRELKGKRPLVPQCIKV 585
Synechococcuselongatus             SRHSDAINEICEYLQILPQPYNELSEAERTAWLVQELKTRRPLVPARMPF 586
Anabaenavariabilis"phosphoenol     TRHSDALNEILDYLQLLPQPYNDLSEEQRVAWLTTELQTRRPLISSELPF 489
Synechococcussp.                   TRHSDAIAEIVDYLSIAQILQRPKRRRKNHLGLVQELKTRRPLIPKEMHF 512
Synechococcusvulcanus"phosphoe     TCHAEALNEITAYLGILPCPYTELSEAERTRWLLSELSTRRPLIPGELPF 544
Streptomycescoelicolor             DAHHHALGQLFDRLGEESWRYADMPREYRTKLLAKELRSRRPLAPSPAPV 457
Rhodopseudomonaspalustris""pho     AVHERTITELMDAARPGTS-YAMLDEEARIALLISELRSTRPLTSMFVKY 487
                                        .   :               .  .   *  **   ***
```

FIGURE 2 (continued)

```
gi|79319830|ref|NP_001031179.1      TEEIADVLDTFHVIAELPADS----FGAYIISMATAPSDVLAVELLQREC 549
gi|73917651|sp|Q84VW9|CAPP3_AR      TEEISDVLDTFKVISELPSDC----FGAYIISMATSPSDVLAVELLQREC 550
gi|1705587|sp|P51061|CAPP2_SOY      TEEIRDVLDTFHVIAELPPDN----FGAYIISMATAPSDVLAVELLQREC 549
gi|399183|sp|Q02735|CAPP_MEDSA      TDEIRDVLDTFRVIAELPSDN----FGAYIISMATAPSDVLAVELLQREC 548
gi|23503558|dbj|BAC20365.1|         TEEIKDVLDTFHVIAELPSDN----FGAYIISMATAPSDVLAVELLQREC 549
gi|115610|sp|P27154|CAPP_TOBAC      TEEIADVLDTLHVIAELPSDC----FGAYIISMATAPSDVLAVELLQREC 547
gi|972511|emb|CAA62469.1|           TEEIADVLDTFHVIAELPADC----FGAYIISMATAPSDVLAVELLQREC 548
gi|30689081|ref|NP_850373.1|        TEEVADVLDTFKVISELPSDS----FGAYIISMATAPSDVLAVELLQREC 546
gi|45505269|gb|AAS67006.1|          TEEITDVLETFRVISELPSDN----FGAYIISMATSPSDVLAVELLQREC 548
gi|115583|sp|P29194|CAPP2_SORB      TEEIADVLDTFHVIAELPADN----FGAYIISMATAPSDVLAVELLQREC 542
gi|1705585|sp|P51059|CAPP2_MAI      TEEISDVLDTFHVIAELPSDN----FGAYIISMATAPSDVLAVELLQREC 549
gi|3341490|emb|CAA07610.1|          TEEVADVMGAFQVIAELPGDN----FGAYVISMATSPSDVLAVELLQREC 554
gi|115478504|ref|NP_001062847.      TDEVADVLDTFHVIAELPADS----FGAYVISMATAPSDVLAVELLQREC 557
gi|115476100|ref|NP_001061646.      TDEIADVLDTFRVIAELPADN----FGAYIISMATAPSDVLAVELLQREC 546
gi|115577|sp|P29193|CAPP1_SACH      SDEIADVLDTFHVLAELPSDS----FGAYVISMATAPSDVLAVELLQREC 548
gi|115440043|ref|NP_001044301.      TDEIADALDTFHVIAELPYDS----FGAYVISMATAPSDVLAVELLQREC 506
gi|9828445|gb|AAG00180.1|AF271      TEEIADVLGTFHVLAELPADC----FGAYIISMATAPSDVLAVELLQREC 542
gi|115445275|ref|NP_001046417.      TEEIADVLGTFHVLAELPADC----FGAYIISMATAPSDVLAVELLQREC 551
Welwitschiamirabilis"tissue_ty      TDEIREVLDTFHVVAELPPDN----FGAYIISMATAPSDVLVVELLQREC 535
Welwitschia                         TDEIREVLDTFHVVAELPPDN----FGAYIISMATAPSDVLVVELLQREC 535
gi|1705590|sp|P51063|CAPP_PICA      TDEVRDVLDTFHVISELPADN----FGAYIISMATAASDVLVVELLQREC 547
Chlamydomonasreinhardtii"           SAEVREVLDTFKVAAHLGRDN----LGAYVISMTKGASDVMAVELLQREA 813
Chlamydomonas                       SAEVREVLDTFKVAAHLGRDN----LGAYVISMTKGASDVMAVELLQREA 813
Glycinemax"phosphoenolpyruvate      HPDVKEVLDTFRIAAELGSDS----LGAYVISMASNASDVLAVELLQKDA 631
Glycine                             HPDVKEVLDTFRIAAELGSDS----LGAYVISMASNASDVLAVELLQKDA 631
Ricinuscommunis"phosphoenolpyr      APDVKEVLDAFRVAAELGSDS----LGAYVISMASNASDVLAVELLQKDA 651
Ricinus                             APDVKEVLDAFRVAAELGSDS----LGAYVISMASNASDVLAVELLQKDA 651
gi|30697740|ref|NP_177043.2|        GPDVKEVLDTFRVAAELGSES----LGAYVISMASNASDVLAVELLQKDA 631
Synechococcuselongatus              SESTREIIETLRMVKQLQEEFGEAACQTYIISMSRELSDLLEVLLLAKEV 636
Anabaenavariabilis"phosphoenol      SDKTNDVIKTFRVVRSLQQEFGINICQTYIISMCRQVSDVLEVLLLAKEA 539
Synechococcussp.                    SERTVETIQTLQVLRRLQQEFGIGICQTYIISMTNEVSDVLEVLLLAQEA 562
Synechococcusvulcanus"phosphoe      SDRTNEIIETFRMVRQLQQEFGTDLCNTYIISMSHEVSDLLEVLLFAKEA 594
Streptomycescoelicolor              DAPGEKTLGVFQTVRRALEVFGPEVIESYIISMCQGADDVFAAAVLAREA 507
Rhodopseudomonaspalustris""pho      SDETVGELAVFREAAKAHATYGAAAIPQCIISMTKGVSDLLEVAVLLKEV 537
                                              :  .::                :***     .*::  .  ::  ::
```

FIGURE 2 (continued)

```
gi|79319830|ref|NP_001031179.1   RVKQP----------------LRVVPLFEKLADLEAAPAAVARLFSVDWY 583
gi|73917651|sp|Q84VW9|CAPP3_AR   HVKNP----------------LRVVPLFEKLADLEAAPAAVARLFSIDWY 584
gi|1705587|sp|P51061|CAPP2_SOY   HIKHP----------------LRVVPLFEKLADLEAAPAALARLFSIDWY 583
gi|399183|sp|Q02735|CAPP_MEDSA   KVRNP----------------LRVVPLFEKLDDLESAPAALARLFSIDWY 582
gi|23503558|dbj|BAC20365.1|      HVKNP----------------LRVVPLFEKLADLETAPAALARLFSVEWY 583
gi|115610|sp|P27154|CAPP_TOBAC   HVKQP----------------LRVVPLFEKLDDLESASAAVARLFSIEWY 581
gi|972511|emb|CAA62469.1|        RVRQP----------------LRVVPLFEKLADLDAAPAAVARLFSIEWY 582
gi|30689081|ref|NP_850373.1|     GITDP----------------LRVVPLFEKLADLESAPAAVARLFSIEWY 580
gi|45505269|gb|AAS67006.1|       HVKQP----------------LRVVPLFEKLADLEAAPAAVARLFSIDWY 582
gi|115583|sp|P29194|CAPP2_SORB   HVKTP----------------LRVVPLFEKLADLEAAPAALARLFSIDWY 576
gi|1705585|sp|P51059|CAPP2_MAI   HVKTP----------------LRVVPLFEKLADLEAAPAALARLFSIDWY 583
gi|3341490|emb|CAA07610.1|       HIKTP----------------LRVVPLFEKLADLEAAPAALARLFSIDWY 588
gi|115478504|ref|NP_001062847.   HVKTP----------------LRVVPLFEKLADLESAPAALTRLFSISWY 591
gi|115476100|ref|NP_001061646.   HVKTP----------------LRVVPLFEKLADLESAPAAVARLFSIDWY 580
gi|115577|sp|P29193|CAPP1_SACH   HVKKP----------------LRVVPLFEKLADLEAAPAALARLFSVEWY 582
gi|115440043|ref|NP_001044301.   HVKKP----------------LRVVPLFEKLADLEAAPAALARLFSVDWY 540
gi|9828445|gb|AAG00180.1|AF271   HIKQP----------------LRVVPLFEKLADLEAAPAAVARLFSIDWY 576
gi|115445275|ref|NP_001046417.   HIKQP----------------LRVVPLFEKLADLEAAPAAVARLFSIDWY 585
Welwitschiamirabilis"tissue_ty   RVKKP----------------LRVVPLFEKLADLENRPAALARLFSIDWY 569
Welwitschia                      RVKKP----------------LRVVPLFEKLADLENRPAALARLFSIDWY 569
gi|1705590|sp|P51063|CAPP_PICA   HVKKP----------------LRVVPLFEKLADLEAAPAALARLFSINWY 581
Chlamydomonasreinhardtii"        RMQVGAEAGGRGGGGPEDGGSLRVVPLFETLEDLDAAEDVMTRLLTNPWY 863
Chlamydomonas                    RMQVGAEAGGRGGGGPEDGGSLRVVPLFETLEDLDAAEDVMTRLLTNPWY 863
Glycinemax"phosphoenolpyruvate   RLAAIGELG-----KACPGGTLRVVPLFETVKDLRGAGSVIRKLLSIDWY 676
Glycine                          RLAAIGELG-----KACPGGTLRVVPLFETVKDLRGAGSVIRKLLSIDWY 676
Ricinuscommunis"phosphoenolpyr   RLAVSGELG-----RPCPGGTLRVVPLFETVKDLRGAGSVIRKLLSIDWY 696
Ricinus                          RLAVSGELG-----RPCPGGTLRVVPLFETVKDLRGAGSVIRKLLSIDWY 696
gi|30697740|ref|NP_177043.2|     RLALTSEHG-----KPCPGGTLRVVPLFETVNDLRAAGPSIRKLLSIDWY 676
Synechococcuselongatus           GLYDPVTGKSS----------LQVIPLFETVEDLQNAPRVMTALFELPFY 676
Anabaenavariabilis"phosphoenol   RLFDPAIAVGT----------IQVVPLFETVEDLQRSRSVMRQLFELPLY 579
Synechococcussp.                 GLYDPLTGMTT----------IRIAPLFETVDDLRNAPEIMQALFEIPLY 602
Synechococcusvulcanus"phosphoe   GLFDPATGAST----------LQAIPLFETVEDLKHAPAVLTQLFSLPFC 634
Streptomycescoelicolor           GLIDLHAGWAK----------IGIVPLLETTDELKAADTILEDLLADPSY 547
Rhodopseudomonaspalustris""pho   GLIDP-SGRSA----------INVVPLFETIEDLQACAKIMDRLLSIPEY 576
                                  :                   :   **:*.  :*      :  *:
```

FIGURE 2 (continued)

```
gi|79319830|ref|NP_001031179.1     KNRIN------------------------------GKQEVMIGYSDSGKD 603
gi|73917651|sp|Q84VW9|CAPP3_AR     KNRIN------------------------------GKQEVMIGYSDSGKD 604
gi|1705587|sp|P51061|CAPP2_SOY     RNRIN------------------------------GKQEVMIGYSDSGKD 603
gi|399183|sp|Q02735|CAPP_MEDSA     INRID------------------------------GKQEVMIGYSDSGKD 602
gi|23503558|dbj|BAC20365.1|        RNRIN------------------------------GKQEVMIGYSDSGKD 603
gi|115610|sp|P27154|CAPP_TOBAC     RNRIN------------------------------GKQEVMVGYSDSGKD 601
gi|972511|emb|CAA62469.1|          RNRIN------------------------------GKQEVMIGYSDSGKD 602
gi|30689081|ref|NP_850373.1|       RNRIN------------------------------GKQEVMIGYSDSGKD 600
gi|45505269|gb|AAS67006.1|         RNRID------------------------------GKQEVMIGYSDSGKD 602
gi|115583|sp|P29194|CAPP2_SORB     RQRIN------------------------------GKQEVMIGYSDSGKD 596
gi|1705585|sp|P51059|CAPP2_MAI     RQRIN------------------------------GKQEVMIGYSDSGKD 603
gi|3341490|emb|CAA07610.1|         RERIN------------------------------GKQEVMIGYSDSGKD 608
gi|115478504|ref|NP_001062847.     RQRIN------------------------------GKQEVMIGYSDSGKD 611
gi|115476100|ref|NP_001061646.     RERIN------------------------------GKQEVMIGYSDSGKD 600
gi|115577|sp|P29193|CAPP1_SACH     RNRIN------------------------------GKQEVMIGYSDSGKD 602
gi|115440043|ref|NP_001044301.     RNRID------------------------------GKQEVMIGYSDSGKD 560
gi|9828445|gb|AAG00180.1|AF271     MNRIN------------------------------GKQEVMIGYSDSGKD 596
gi|115445275|ref|NP_001046417.     MNRIN------------------------------GKQEVMIGYSDSGKD 605
Welwitschiamirabilis"tissue_ty     RNRID------------------------------GKQEVMXGYSDSGKD 589
Welwitschia                        RNRID------------------------------GKQEVMXGYSDSGKD 589
gi|1705590|sp|P51063|CAPP_PICA     RNRID------------------------------GKQEVMIGYSDSGKD 601
Chlamydomonasreinhardtii"          REHLRAV--------------------------HGDAQEVMLGYSDSGKD 887
Chlamydomonas                      REHLRAV--------------------------HGDAQEVMLGYSDSGKD 887
Glycinemax"phosphoenolpyruvate     HEHIVKN--------------------------HNGHQEVMVGYSDSGKD 700
Glycine                            HEHIVKN--------------------------HNGHQEVMVGYSDSGKD 700
Ricinuscommunis"phosphoenolpyr     REHIIKN--------------------------HNGHQEVMVGYSDSGKD 720
Ricinus                            REHIIKN--------------------------HNGHQEVMVGYSDSGKD 720
gi|30697740|ref|NP_177043.2|       REHIQKN--------------------------HNGHQEVMVGYSDSGKD 700
Synechococcuselongatus             -----------------TQLNPTQSEP---------LQEVMLGYSDSNKD 700
Anabaenavariabilis"phosphoenol     RALLAGGYKNTEVKVPNTELTPQSPAPSPQSVLTPDLQEVMLGYSDSNKD 629
Synechococcussp.                   RACLAGG---------YEPPADGRCDETFGDRLVPNLQEIMLGYSDSNKD 643
Synechococcusvulcanus"phosphoe     R-----------------SYLGSNSTP--------FLQEVMLGYSDSNKD 659
Streptomycescoelicolor             R------------------RLVALRGD---------VQEVMLGYSDSSKF 570
Rhodopseudomonaspalustris""pho     R------------------RLVDSRGS---------VQEVMLGYSDSNKD 599
                                                                        **:* *****.*
```

FIGURE 2 (continued)

```
gi|79319830|ref|NP_001031179.1      AGRLSAAWQLYKAQEELVKVAKEYGVKLTMFHGRGGTVGRGGGPTHLAIL 653
gi|73917651|sp|Q84VW9|CAPP3_AR      AGRLSAAWELYKAQEELVKVAKKYGVKLTMFHGRGGTVGRGGGPTHLAIL 654
gi|1705587|sp|P51061|CAPP2_SOY      AGRFSAAWQLYKAQEELINVAKKFGVKLTMFHGRGGTVGRGGGPTHLAIL 653
gi|399183|sp|Q02735|CAPP_MEDSA      AGRFSAAWQLYKAQEDLIKVAQKFGVKLTMFHGRGGTVGRGGGPTHLAIL 652
gi|23503558|dbj|BAC20365.1|         AGRFSAAWQLYKAQEELIKVAKEYGVKLTMFHGRGGTVGRGGGPTHLAIL 653
gi|115610|sp|P27154|CAPP_TOBAC      AGRFSAAWQLYKAQEELIKVAKEHGVKLTMFHGRGGTVGRGGGPTHLAIL 651
gi|972511|emb|CAA62469.1|           AGRLSAAWQLYKAQEELIQVAKEFDVKLTMFHGRGGTVGRGGGPAHLAIL 652
gi|30689081|ref|NP_850373.1|        AGRLSAAWQLYKTQEELVKVAKEYGVKLTMFHGRGGTVGRGGGPTHLAIL 650
gi|45505269|gb|AAS67006.1|          AGRLSAAWALYKAQEELVKVAKEYGVKLTMFHGRGGTVGRGGGPTHLAIL 652
gi|115583|sp|P29194|CAPP2_SORB      AGRLSAAWQLYKAQEELIKVAKDFGVKLTMFHGRGGTVGRGGGPTHLAIL 646
gi|1705585|sp|P51059|CAPP2_MAI      AGRLSAAWQLYKAQEELIKVAKDFGVKLTMFHGRGGTVGRGGGPTHLAIL 653
gi|3341490|emb|CAA07610.1|          AGRLSAAWQMYKAQEDLVKVAKQFGVKLTMFHGRGGTVGRGGGPTHLAIL 658
gi|115478504|ref|NP_001062847.     AGRLSAAWQMYKAQEQLVKVAKDFGVKLTMFHGRGGTVGRGGGPTHLAIL 661
gi|115476100|ref|NP_001061646.     AGRLSAAWQLYKSQEELINVAKEFGVKLTMFHGRGGTVGRGGGPTHLAIL 650
gi|115577|sp|P29193|CAPP1_SACH      AGRFSAAWQLYKAQEELINVAKLYGVKLTMFHGRGGTVGRGGGPTHLAIL 652
gi|115440043|ref|NP_001044301.     AGRFSAAWELYKAQEELIKVAKQFGVKLTMFHGRGGTVGRGGGPTHLAIL 610
gi|9828445|gb|AAG00180.1|AF271      AGRLSAAWQMYKAQEELVKVAKHYGVKLTMFHGRGGTVGRGGGPSHLAIL 646
gi|115445275|ref|NP_001046417.     AGRLSAAWQMYKAQEELVKVAKHYGVKLTMFHGRGGTVGRGGGPSHLAIL 655
Welwitschiamirabilis"tissue_ty      AGRLSAAWQLYKAQEELIKVAKQFGIKLTMFHGRGGTVGRGGGPTHLAIL 639
Welwitschia                         AGRLSAAWQLYKAQEELIKVAKQFGIKLTMFHGRGGTVGRGGGPTHLAIL 639
gi|1705590|sp|P51063|CAPP_PICA      AGRLSAGWALYKAQEDLIKVAKEFGIKLTMFHGRGGTVGRGGGPTHLAIL 651
Chlamydomonasreinhardtii"           AGRLAANWALYKCQERLVAITKANNVKLTLFHGRGGTVGRGGGPTHIAIQ 937
Chlamydomonas                       AGRLAANWALYKCQERLVAITKANNVKLTLFHGRGGTVGRGGGPTHIAIQ 937
Glycinemax"phosphoenolpyruvate      AGRFTAAWELYKAQEDVVAACNDYGIKVTLFHGRGGSIGRGGGPTYLAIQ 750
Glycine                             AGRFTAAWELYKAQEDVVAACNDYGIKVTLFHGRGGSIGRGGGPTYLAIQ 750
Ricinuscommunis"phosphoenolpyr      AGRFTAAWELYKAQEDVVAACNDFGIKVTLFHGRGGSIGRGGGPTYLAIQ 770
Ricinus                             AGRFTAAWELYKAQEDVVAACNDFGIKVTLFHGRGGSIGRGGGPTYLAIQ 770
gi|30697740|ref|NP_177043.2|        AGRFTAAWELYKAQENVVAACNEFGIKITLFHGRGGSIGRGGGPTYLAIQ 750
Synechococcuselongatus              SGFLSSNWEIHKAQKALGTVARDHRVKLRIFHGRGGSVGRGGGPAYEAIL 750
Anabaenavariabilis"phosphoenol      SGFLSSNWEIHKAQKSLQQIAEEYGVNLRIFHGRGGSVGRGGGPAHEAIL 679
Synechococcussp.                    SGFLSSNWEIHKAQKNLQQVADPYGIDLRIFHGRGGSVGRGGGPAYAAIL 693
Synechococcusvulcanus"phosphoe      SGFLSSNWEIYKAQQQLQKIAESFGFQLRIFHGRGGSVGRGGGPAYAAIL 709
Streptomycescoelicolor              GGITTSQWEIHRAQRRLRDVAHRYGVRLRLFHGRGGTVGRGGGPTHDAIL 620
Rhodopseudomonaspalustris""pho      GGFVTSGWELYKAEIGLIEIFEHHGVRLRLFHGRGGSVGRGGGPSYDAIV 649
                                    .*   :: * .::: :   :          . : :******::******:: **
```

FIGURE 2 (continued)

```
gi|79319830|ref|NP_001031179.1      SQPPDTINGSLRVTVQGEVIEQSFGEEHLCFRTLQRFTAATLEHGMRP-- 701
gi|73917651|sp|Q84VW9|CAPP3_AR      SQPPDTVNGSLRVTVQGEVIEQSFGEAHLCFRTLQRFTAATLEHGMNP-- 702
gi|1705587|sp|P51061|CAPP2_SOY      SQPPDTIHGSLRVTVQGEVIEQSFGEQHLCFRTLQRFTAATLEHGMHP-- 701
gi|399183|sp|Q02735|CAPP_MEDSA      SQPPETIHGSLRVTVQGEVIEQSFGEEHLCFRTLQRFTAATLEHGMRP-- 700
gi|23503558|dbj|BAC20365.1|         SQPPDTIHGSLRVTVQGEVIEQSFGEEHLCFRTLQRYTAATLEHGMHP-- 701
gi|115610|sp|P27154|CAPP_TOBAC      SQPPDTIQGSLRVTVQGEVIEQSFGEEHLCFRTLQRFTAATLEHGMHP-- 699
gi|972511|emb|CAA62469.1|           SQPPETIHGSLRVTVQGEVIEQSFGEEHLCFRTLQRFTAATLEHGMHP-- 700
gi|30689081|ref|NP_850373.1|        SQPPDTIHGQLRVTVQGEVIEQSFGEEHLCFRTLQRFTAATLEHGMHP-- 698
gi|45505269|gb|AAS67006.1|          SQPPDTIHGSLRVTVQGEVIEQSFGEEHLCFRTLQRFTAATLEHGMHP-- 700
gi|115583|sp|P29194|CAPP2_SORB      SQPPDTIHGSLRVTVQGEVIEQSFGEEHLSFRTLQRFTAATLEHGMHP-- 694
gi|1705585|sp|P51059|CAPP2_MAI      SQPPDTIHGSLRVTVQGEVIEQSFGEEHLCFRTLQRFTAATLEHGMHP-- 701
gi|3341490|emb|CAA07610.1|          SQPPDTINGSLRVTVQGEVIEQSFGEEHLCFRTLQRFTAATLEHGMRP-- 706
gi|115478504|ref|NP_001062847.      SQPPDTINGSLRVTVQGEVIEQSFGEEHLCFRTLQRFTAATLEHGMHP-- 709
gi|115476100|ref|NP_001061646.      SQPPDTIHGSLRVTVQGEVIEQSFGEEHLCFRTLQRFTAATLEHGMHP-- 698
gi|115577|sp|P29193|CAPP1_SACH      SQPPETIHGSLRVTVQGEVIEQSFGEEHLCFRTLQRFTAATLEHGMHP-- 700
gi|115440043|ref|NP_001044301.      SQPPDTIHGSLRVTVQGEVIEQSFGEEHLCFRTLQRFTSATLEHGMHP-- 658
gi|9828445|gb|AAG00180.1|AF271      SQPPDTIHGSLRVTVQGEVIEHSFGEEHLCFRTLQRFTAATLEHGMHP-- 694
gi|115445275|ref|NP_001046417.      SQPPDTIHGSLRVTVQGEVIEHSFGEEHLCFRTLQRFTAATLEHGMHP-- 703
Welwitschiamirabilis"tissue_ty      SQPPDTIHGSLRVTVQGEVIEQCFGEEHLCFRTLQRFTAATLEHGMHP-- 687
Welwitschia                         SQPPDTIHGSLRVTVQGEVIEQCFGEEHLCFRTLQRFTAATLEHGMHP-- 687
gi|1705590|sp|P51063|CAPP_PICA      SQPPDTIHGSFRVTVQGEVIEQSFGEEHLCFRTLQRFTAATLEHGMRP-- 699
Chlamydomonasreinhardtii"           SQPPGSVEGTFRITEQGEMVQAKFGISGVALSQLETYTTAVLLATMRP-- 985
Chlamydomonas                       SQPPGSVEGTFRITEQGEMVQAKFGISGVALSQLETYTTAVLLATMRP-- 985
Glycinemax"phosphoenolpyruvate      SQPPGSVMGTLRSTEQGEMVEAKFGLPQIAVRQLEIYTTAVLLATLRP-- 798
Glycine                             SQPPGSVMGTLRSTEQGEMVEAKFGLPQIAVRQLEIYTTAVLLATLRP-- 798
Ricinuscommunis"phosphoenolpyr      SQPPGSVMGTLRSTEQGEMVQAKFGLPHTAIRQLEIYTTAVLLATLRP-- 818
Ricinus                             SQPPGSVMGTLRSTEQGEMVQAKFGLPHTAIRQLEIYTTAVLLATLRP-- 818
gi|30697740|ref|NP_177043.2|        SQPPGSVMGSLRSTEQGEMVQAKFGIPQTAVRQLEVYTTAVLLATLKP-- 798
Synechococcuselongatus              AQPGRTTDGRIKITEQGEVLASKYALPELALYNLETITTAVIQSSLLG-- 798
Anabaenavariabilis"phosphoenol      AQPGHSINGRIKITEQGEVLASKYSLLDLALYNLETITTAVIQASLLR-- 727
Synechococcussp.                    AQPPNTINGRIKITEQGEVLASKYSLPDLALYHLESVSTAVIQSSLLA-- 741
Synechococcusvulcanus"phosphoe      AQPAQTIKGRIKITEQGEVLASKYSLPELALFNLETVATAVIQASLLR-- 757
Streptomycescoelicolor              AQPWGTLEGEIKVTEQGEVISDKYLIPALARENLELTVAATLQASALHTA 670
Rhodopseudomonaspalustris""pho      AQPGGAVNGQIRITEQGEIITRKYSNVEVGRNNLEILAAATLEASLLQ-- 697
                                    :**  :  * :: * ***::   :         *:   :*.:
```

FIGURE 2 (continued)

```
gi|79319830|ref|NP_001031179.1      --PISPKPE-WRALLDEMAVVATEEYRSVVFQEPRFVEYFRLATPELEYG 748
gi|73917651|sp|Q84VW9|CAPP3_AR      --PISPKPE-WRALLDEMAVVATEEYRSVVFQEPRFVEYFRLATPELEYG 749
gi|1705587|sp|P51061|CAPP2_SOY      --PISPKPE-WRALMDQMAVIATEEYRSIVFKEPRFVEYFRLATPELEYG 748
gi|399183|sp|Q02735|CAPP_MEDSA      --PSSPKPE-WRALMDQMAVIATEEYRSIVFKEPRFVEYFRLATPEMEYG 747
gi|23503558|dbj|BAC20365.1|         --PISPKPE-WRALMDEMAVIATEEYRSIVFKEPRFVEYFRLATPELEYG 748
gi|115610|sp|P27154|CAPP_TOBAC      --PVSPKPE-WRALMDEIAVIATEKYRSIVFKEPRFVEYSALATPELEYG 746
gi|972511|emb|CAA62469.1|           --PVSPKPE-WRALMDEIAVVATEKYRSIVFKEPRFVEYFRLATPELEYG 747
gi|30689081|ref|NP_850373.1|        --PVSPKPE-WRVLMDEMAIIATEEYRSVVFKEPRFVEYFRLATPELEYG 745
gi|45505269|gb|AAS67006.1|          --PVSPKPE-WRALMDEMAVIATKEYRSVVFKEPRFVEYFRCATPELEYG 747
gi|115583|sp|P29194|CAPP2_SORB      --PNAPKPE-WRTLLDEMAVVATEEYRSIVFQEPRFVEYFRLATPETEYG 741
gi|1705585|sp|P51059|CAPP2_MAI      --PNAPKPE-WRALLDEMAVVATEEYRSIVFKEPRFVEYFRLATPETEYG 748
gi|3341490|emb|CAA07610.1|          --PISPKPE-WRALLDEMAVVATEEYRSIVFQEPRFVEYFRLATPETEYG 753
gi|115478504|ref|NP_001062847.      --PSAPKPE-WRALLDEMAVVATKEYRSVVFQEPRFVEYFRLATPETEYG 756
gi|115476100|ref|NP_001061646.      --PIAPKPE-WRALLDEMAVVATKEYRSIVFQEPRFVEYFRLATPEMEYG 745
gi|115577|sp|P29193|CAPP1_SACH      --PISPKPE-WRALMDEMAIVATKEYRSIVFEEPRFVEYFRLATPEMEYG 747
gi|115440043|ref|NP_001044301.      --PISPKPE-WRALMDEMAAVATKEYRSIVFQEARFVEYFRLATPELEYG 705
gi|9828445|gb|AAG00180.1|AF271      --PISPKPE-WRALMDEMAVVATKEYRSIVFKEPRFVEYFRSATPETEYG 741
gi|115445275|ref|NP_001046417.      --PISPKPE-WRALMDEMAVVATKEYRSIVFKEPRFVEYFRSATPETEYG 750
Welwitschiamirabilis"tissue_ty      --PIAPKPE-WRQLMDEMAVVATQEYRSYVFHNKRFVEYFRSATPELEYG 734
Welwitschia                         --PIAPKPE-WRQLMDEMAVVATQEYRSYVFHNKRFVEYFRSATPELEYG 734
gi|1705590|sp|P51063|CAPP_PICA      --PVAPKPE-WRELMDEMAVVATKEYRSIVFQDPRFVEYFRSATPELEYG 746
Chlamydomonasreinhardtii"           --PSPPRREEWRAVMEMLSRVSCESYRNIVHHSPLFLRYFKHATPEAELG 1033
Chlamydomonas                       --PSPPRREEWRAVMEMLSRVSCESYRNIVHHSPLFLRYFKHATPEAELG 1033
Glycinemax"phosphoenolpyruvate      --PIPPREEKWRNVMEEISNISCQCDRNVVYENPEFLAYFHEATPEAELG 846
Glycine                             --PIPPREEKWRNVMEEISNISCQCDRNVVYENPEFLAYFHEATPEAELG 846
Ricinuscommunis"phosphoenolpyr      --PHPPREEQWRNVMEEISKISCQNYRSTVYENPEFLAYFHEATPQAELG 866
Ricinus                             --PHPPREEQWRNVMEEISKISCQNYRSTVYENPEFLAYFHEATPQAELG 866
gi|30697740|ref|NP_177043.2|        --PQPPREEKWRNLMEEISGISCQHYRSTVYENPEFLSYFHEATPQAELG 846
Synechococcuselongatus              --SGFDDIEPWNQIMEELAARSRRHYRALVYEQPDLVDFFNQVTPIEEIS 846
Anabaenavariabilis"phosphoenol      --TGFDDIEPWNEIMEELAARSRQHYRGLIYEQPDFIDFFHQVTPIEEIS 775
Synechococcussp.                    --SGFDDIQPWNRIMEDLSQRSRAAYRALIYEEPDFLDFFMSVTPIPEIS 789
Synechococcusvulcanus"phosphoe      --SSIDEIEPWHEIMEELATRSRQCYRHLIYEQPEFIEFFNEVTPIQEIS 805
Streptomycescoelicolor              PRQSDEALARWDAAMDVVSDAAHTAYRHLVEDPD-LPTYFLASTPVDQLA 719
Rhodopseudomonaspalustris""pho      PKR-VAPHRDYLEAMEQLSALAFKAYRGLVYETDGFVDYFWASTVINEIS 746
                                          :   :: ::  :    *  : .   :  :    *   : .
```

FIGURE 2 (continued)

```
gi|79319830|ref|NP_001031179.1        RMNIGSRPSKRKP-SGGIESLRAIPWIFAWTQTRFHLPVWLGFGSAIRHV 797
gi|73917651|sp|Q84VW9|CAPP3_AR        RMNIGSRPSKRKP-SGGIESLRAIPWIFAWTQTRFHLPVWLGFGAAFRYA 798
gi|1705587|sp|P51061|CAPP2_SOY        RMNIGSRPAKRRP-SGGIETLRAIPWIFAWTQTRFHLPVWLGFGAAFKKV 797
gi|399183|sp|Q02735|CAPP_MEDSA        RMNIGSRPAKRRP-SGGIETLRAIPWIFAWTQTRFHLPVWLGFGAAFRQV 796
gi|23503558|dbj|BAC20365.1|           RMNIGSRPAKRKP-SGGIETLRAIPWIFAWTQTRFHLPVWLGFGAAFKHV 797
gi|115610|sp|P27154|CAPP_TOBAC        RMNIGSRPSKRKP-SGGIESLRAIPWIFAWTQTRFHLPVWLGFGAAFKYA 795
gi|972511|emb|CAA62469.1|             RMNIGSRPSKRKP-SGGIESLRAIPWIFAWTQTRFHLPVWLGFGAAFKYA 796
gi|30689081|ref|NP_850373.1|          RMNIGSRPSKRKP-SGGIESLRAIPWIFAWTQTRFTYPCGL--------- 785
gi|45505269|gb|AAS67006.1|            RMNIGSRPSKRKP-SGGIESLRAIPWIFAWTQTRFHLPVWLGFGSAFKHV 796
gi|115583|sp|P29194|CAPP2_SORB        RMNIGSRPSKRKP-SGGIESLRAIPWIFAWTQTRFHLPVWLGFGGAFKHV 790
gi|1705585|sp|P51059|CAPP2_MAI        RMNIGSRPSKRKP-SGGIDSLRAIPWIFAWTQTRFHLPVWLGFGAAFKNV 797
gi|3341490|emb|CAA07610.1|            RMNIGSRPSKRKP-SGGIESLRAIPWIFAWTQTRFHLPVWLGFGGAFKHI 802
gi|115478504|ref|NP_001062847.        RMNIGSRPSKRKP-SGGIESLRAIPWIFAWTQTRFHLPVWLGFGAAFKHA 805
gi|115476100|ref|NP_001061646.        RMNIGSRPSKRKP-SGGIESLRAIPWIFAWTQTRFHLPVWLGFGSAFKHI 794
gi|115577|sp|P29193|CAPP1_SACH        RMNIGSRPSKRKP-SAGIESLRAIPWIFAWTQTRFHLPVWLGFGAAFKHV 796
gi|115440043|ref|NP_001044301.        RMNIGSRPSKRKP-SGGIESLRAIPWIFAWTQTRFHLPVWLGFGAAFKHV 754
gi|9828445|gb|AAG00180.1|AF271        RMNIGSRPSKRKP-SGGIESLRAIPWIFAWTQTRFHLPVWLGFGGAFKHI 790
gi|115445275|ref|NP_001046417.        RMNIGSRPSKRKP-SGGIESLRAIPWIFAWTQTRFHLPVWLGFGGAFKHI 799
Welwitschiamirabilis"tissue_ty        RMNIGSRPSKRKP-SGGIESLRAIPWIFAWTQTRFHLPVWLGFGAAFKSV 783
Welwitschia                           RMNIGSRPSKRKP-SGGIESLRAIPWIFAWTQTRFHLPVWLGFGAAFKSV 783
gi|1705590|sp|P51063|CAPP_PICA        RMNIGSRPSKRKP-SGGIESLRAIPWIFAWTQTRFHLPVWLGFGAAFKHV 795
Chlamydomonasreinhardtii"             NLYIGSRPARRRNKDASISTLRAIPWIFAWTQNRLILPSWLGIGAALTAA 1083
Chlamydomonas                         NLYIGSRPARRRNKDASISTLRAIPWIFAWTQNRLILPSWLGIGAALTAA 1083
Glycinemax"phosphoenolpyruvate        FLNIGSRPTRRKS-SVGIGHLRAIPWLFAWTQTRFVLPAWLGVGAGLKGA 895
Glycine                               FLNIGSRPTRRKS-SVGIGHLRAIPWLFAWTQTRFVLPAWLGVGAGLKGA 895
Ricinuscommunis"phosphoenolpyr        FLNIGSRPTRRKS-STGIGHLRAIPWVFAWTQTRFVLPAWLGVGAGLKGA 915
Ricinus                               FLNIGSRPTRRKS-STGIGHLRAIPWVFAWTQTRFVLPAWLGVGAGLKGA 915
gi|30697740|ref|NP_177043.2|          FLNIGSRPTRRKS-SSGIGHLRAIPWVFAWTQTRFVLPAWLGVGAGLKGV 895
Synechococcuselongatus                KLQISSRPARRKTGKRDLGSLRAIPWVFSWTQSRFLLPSWYGVGTALQEF 896
Anabaenavariabilis"phosphoenol        QLQISSRPARRPSGKKDLSSLRAIPWVFSWTQTRFLLPSWYGVGTALQEF 825
Synechococcussp.                      QLQISSRPARRKKGNKDLSSLRAIPWVFSWTQSRFLVPAWYGVGTALQGF 839
Synechococcusvulcanus"phosphoe        QLQISSRPTRR-GGKKTLESLRAIPWVFSWTQTRFLLPAWYGVGTALKEF 854
Streptomycescoelicolor                DLHLGSRPSRRPGSGVSLDGLRAIPWVFGWTQSRQIVPGWYGVGSGLKAL 769
Rhodopseudomonaspalustris""pho        TLNIGSRPASRKKT-RAIEDLRAIPWVFSWAQCRLMLPGWYGFGSAVSAW 795
                                       : :.***: *       :  ******:*.*:*  *    *
```

FIGURE 2 (continued)

```
gi|79319830|ref|NP_001031179.1      IEK-DVRNLHMLQDMYQHWPFFRVTIDLIEMVFAKGDPGIAALYDKLLVS 846
gi|73917651|sp|Q84VW9|CAPP3_AR      IKK-DVRNLHMLQDMYKQWPFFRVTIDLIEMVFAKGDPGIAALYDKLLVS 847
gi|1705587|sp|P51061|CAPP2_SOY      IEE-NVKNLNMLQEMYNQWPFFRVTLDLVEMVFAKGDPKIAALNDRLLVS 846
gi|399183|sp|Q02735|CAPP_MEDSA      VQK-DVKNLHMLQEMYNQWPFFRVTIDLVEMVFAKGDPGIAALNDRLLVS 845
gi|23503558|dbj|BAC20365.1|         IAK-DIRNLNMLQEMYNQWPFFRVTIDLVEMVFAKGDPGIAALYDRLLVS 846
gi|115610|sp|P27154|CAPP_TOBAC      IDK-DIKNLRMFHEMYNEWPFFRVTIDLVEMVFAKGNPGIAALYDKLLVS 844
gi|972511|emb|CAA62469.1|           IEK-DIKNLRMLQEMYNAWPFFRVTIDLVEMVFAKGDPGIAALFDKLLVS 845
gi|30689081|ref|NP_850373.1|        ----------ALEEHSNA-------------------------------- 793
gi|45505269|gb|AAS67006.1|          VEK-DPKNLQMLQDMYNQWPFFRVTLDLVEMVFAKGDPGIAALFDKLLVS 845
gi|115583|sp|P29194|CAPP2_SORB      LQK-DIRNLHMLQEMYNEWPFFRVTIDLVEMVFAKGNPGIAALYDKLLVS 839
gi|1705585|sp|P51059|CAPP2_MAI      LQK-DIRNLHMLQEMYNEWPFFRVTIDLVEMVFAKGNPGIAALYDKLLVS 846
gi|3341490|emb|CAA07610.1|          LKK-DIRNFHMLQEMYNEWPFFRVTIDLVEMVFAKGNPGIAALYDRLLVS 851
gi|115478504|ref|NP_001062847.      LQK-DIRNLHMLQEMYNEWPFFRVTLDLIEMVFAKGNPGIAALYDKLLVS 854
gi|115476100|ref|NP_001061646.      LEK-DIRNLHMLQEMYNEWPFFRVTIDLVEMVFAKGDPGIAALYDKLLVS 843
gi|115577|sp|P29193|CAPP1_SACH      LDK-DIRNLQTLQEMYNQWPFFRVTIDLVEMVFAKGDPGIAALYDKLLVS 845
gi|115440043|ref|NP_001044301.      LQK-DIRNLQILQEMYNEWPFFRVTIDLVEMVFAKGDPGIAALYDKLLVS 803
gi|9828445|gb|AAG00180.1|AF271      MQK-DIRNIHTLKEMYNEWPFFRVTLDLLEMVFAKGDPGIAALYDKLLVA 839
gi|115445275|ref|NP_001046417.      MQK-DIRNIHTLKEMYNEWPFFRVTLDLLEMVFAKGDPGIAALYDKLLVA 848
Welwitschiamirabilis"tissue_ty      IEK-DIRNLHTLQQMYNEWPFFRVTIDLVEMVFAK-HPEIAALYDKLLVS 831
Welwitschia                         IEK-DIRNLHTLQQMYNEWPFFRVTIDLVEMVFAK-HPEIAALYDKLLVS 831
gi|1705590|sp|P51063|CAPP_PICA      MEK-DIRNLHMLQQMYNEWPFFRVTIDLIEMVFAKGDPGIAALYDKLLVS 844
Chlamydomonasreinhardtii"           MTQ-G--HLPTLQAMYREWPFFGSTVDLIEMILAKTDPRIAALYEEVLVN 1130
Chlamydomonas                       MTQ-G--HLPTLQAMYREWPFFGSTVDLIEMILAKTDPRIAALYEEVLVN 1130
Glycinemax"phosphoenolpyruvate      CEK-G--YTEELKAMYKEWPFFQSTIDLIEMVLGKADIPIAKHYDEVLVT 942
Glycine                             CEK-G--YTEELKAMYKEWPFFQSTIDLIEMVLGKADIPIAKHYDEVLVT 942
Ricinuscommunis"phosphoenolpyr      CEK-G--FTEDLKAMYKEWPFFQSTIDLIEMVLGKADIPIAKHYDEVLVS 962
Ricinus                             CEK-G--FTEDLKAMYKEWPFFQSTIDLIEMVLGKADIPIAKHYDEVLVS 962
gi|30697740|ref|NP_177043.2|        SEK-G--HADDLKEMYKEWPFFQSTLELIEMVLAKADIPMTKHYDEQLVS 942
Synechococcuselongatus              LQERPEQNLNLLRYFYEKWPFFRMVISKVEMTLAKVDLQIAHHYVHELAN 946
Anabaenavariabilis"phosphoenol      FNEEPEEHLKLMRYFYVKWPFFKMVISKVEMTLAKVDMQMAGHYVQELSD 875
Synechococcussp.                    FEEDPVENLKLMRYFYSKWPFFRMVISKVEMTLSKVDLQMASHYVHELAE 889
Synechococcusvulcanus"phosphoe      LEEKPAEHLSLLRYFYYKWPFFRMVISKVEMTLAKVDLEIARYYVQELSQ 904
Streptomycescoelicolor              REAGLDT---VLDEMHQQWHFFRNFISNVEMTLAKTDLRIAQHYVDTLVP 816
Rhodopseudomonaspalustris""pho      VTAHPETGIAFLQKMYQEWPFFRTLLSNMDMVLSKSSIGIASRYAELVED 845
                                                       :
```

FIGURE 2 (continued)

```
gi|79319830|ref|NP_001031179.1      E----ELWPFGEKLRANFEETKKLILQTAGHKDLLEGDPYLKQRLRLRDS 892
gi|73917651|sp|Q84VW9|CAPP3_AR      E----DLWAFGEKLRANFDETKNLVLQTAGHKDLLEGDPYLKQRLRLRDS 893
gi|1705587|sp|P51061|CAPP2_SOY      K----DLWPFGDQLRNKYEETRKLLLQVAGHKEILEGDPYLKQRLRLRHA 892
gi|399183|sp|Q02735|CAPP_MEDSA      K----DLWPFGEQLRSKYEETKKLLLQVAAHKEVLEGDPYLKQRLRLRDS 891
gi|23503558|dbj|BAC20365.1|         E----DLWSFGEQLRTMFEETKQLLLQVAAHKDLLEGDPYLKQRLRLRDS 892
gi|115610|sp|P27154|CAPP_TOBAC      E----DLLPFGELLRSNYEETRSLLLQIAGHKDLLEGDPYLKQRLRLRDS 890
gi|972511|emb|CAA62469.1|           E----DLWSFGELLRSKYEETKSLLLQIAGHKDLLEGDPYLKQRLRLRDS 891
gi|30689081|ref|NP_850373.1|        --------------------------------------------------
gi|45505269|gb|AAS67006.1|          E----ELRPFGENLRAKYEETKSFLLQVAGHKDILEGDPYLKQRLRLRDS 891
gi|115583|sp|P29194|CAPP2_SORB      E----ELRPLGEKLRANYEETQKLLLQVAGHRDLLEGDPYLKQRLRLRDA 885
gi|1705585|sp|P51059|CAPP2_MAI      E----ELHPLGEKLRANYEETQKLLLQVAGHRDLLEGDLYLKQRLRLRDA 892
gi|3341490|emb|CAA07610.1|          E----GLQPLGEKLRANYEETQKLLLQVAGHKDLLEGDPYLKQRLRLRDA 897
gi|115478504|ref|NP_001062847.      E----DLQPLGEKLRANYVETQKLLLQVAGHRDLLEGDPYLKQRLRLRDA 900
gi|115476100|ref|NP_001061646.      E----ELWPLGEKLRANCEETKQLLLQVAGHKDLLEGDLYLKQRLRLRNA 889
gi|115577|sp|P29193|CAPP1_SACH      E----DLWSFGKRLRANYEETKQLLLQVAGHKDLLEGDPYLKQRLRIRDS 891
gi|115440043|ref|NP_001044301.      E----DLWPFGARLRANYEETKQLLLQVAGHKDLLEGDPYLRQRLRIRDS 849
gi|9828445|gb|AAG00180.1|AF271      G----DLQSFGEQLRNNFEETKQLLLQVAGHKDILEGDPYLKQRLRLRES 885
gi|115445275|ref|NP_001046417.      G----DLQSFGEQLRNNFEETKQLLLQVAGHKDILEGDPYLKQRLRLRES 894
Welwitschiamirabilis"tissue_ty      S----VLWAFGEQLRKNYVETKTLLLQVAGHKEVLEGDPYLKQRLRLRDS 877
Welwitschia                         S----VLWAFGEQLRKNYVETKTLLLQVAGHKEVLEGDPYLKQRLRLRDS 877
gi|1705590|sp|P51063|CAPP_PICA      D----DLWAIGEKLRANYGETKDLLLQVAGHKDLLEGDPYLKQRLRLRDS 890
Chlamydomonasreinhardtii"           DP---EEKKLGAELRERLQRCQGAILKVTGHENLLSNNPTLSKLISMRSP 1177
Chlamydomonas                       DP---EEKKLGAELRERLQRCQGAILKVTGHENLLSNNPTLSKLISMRSP 1177
Glycinemax"phosphoenolpyruvate      K----ERQELGHELRSELMTAEKFVMVISGHEKLQQNNRSLRRLIENRLP 988
Glycine                             K----ERQELGHELRSELMTAEKFVMVISGHEKLQQNNRSLRRLIENRLP 988
Ricinuscommunis"phosphoenolpyr      E----SRRELGAELRSELLTTEKYVLVVSGHEKLSQNNRSLRRLIESRLP 1008
Ricinus                             E----SRRELGAELRSELLTTEKYVLVVSGHEKLSQNNRSLRRLIESRLP 1008
gi|30697740|ref|NP_177043.2|        E----KRRGLGTELRKELMTTEKYVLVISGHEKLLQDNKSLKKLIDSRLP 988
Synechococcuselongatus              PEDQERFERVFSQIAAEFQLTCHLVLTITNHGRLLDGDPELQRSVQLRNG 996
Anabaenavariabilis"phosphoenol      PEDKPRFEKVFEQIANEYYLTRDLVLKITDHGRLLDGDPVLQRSVQLRNG 925
Synechococcussp.                    KEDIPRFEKLLEQISQEYNLTKRLILEITENEALLDGDRPLQRSVQLRNG 939
Synechococcusvulcanus"phosphoe      PQNREAFCRLYDQIAQEYRLTTELVLTITGHERLLDGDPALQRSVQLRNR 954
Streptomycescoelicolor              ---DELKH-VFDTIKAEHELTVAEVLRVTGESELLDADPVLKQTFTIRDA 862
Rhodopseudomonaspalustris""pho      ---VDVRERIFGRIRAEWHSSIEYLFAIMQQDRLLQSNPLLERSIRHRFP 892
```

**FIGURE 2 (continued)**

```
gi|79319830|ref|NP_001031179.1      YITTLNVCQAYTLKRIRDPSYHVTLRPHISKEIAESSKPAKELIELNPTS 942
gi|73917651|sp|Q84VW9|CAPP3_AR      YITTLNVCQAYTLKRIRDANYNVTLRPHISKEIMQSSKSAQELVKLNPTS 943
gi|1705587|sp|P51061|CAPP2_SOY      PITTLNIVQAYTLKRIRDPNYNVKVRPRISKESAEASKSADELVKLNPTS 942
gi|399183|sp|Q02735|CAPP_MEDSA      YITTLNVFQAYTLKRIRDPNYKVEVRPPISKESAETSKPADELVTLNPTS 941
gi|23503558|dbj|BAC20365.1|         YITTLNVCQAYTLKRIRDPNYNVKLRPHISKEAIDVSKPADELVTLNPTS 942
gi|115610|sp|P27154|CAPP_TOBAC      YITTLNLLQAYTLKRIRDPNYHVTLRPHISKDYMES-KSAAELVQLNPTS 939
gi|972511|emb|CAA62469.1|           YITTLNVCQAYTLKRIRDPDYSVTPRPHISKEYMEA-KPATELVNLNPTS 940
gi|30689081|ref|NP_850373.1|        --------------------------------------------------
gi|45505269|gb|AAS67006.1|          YITTLNVLQAYTLKRIRDPDYHVKLRPHLSKDYMESSKPAAELVKLNPKS 941
gi|115583|sp|P29194|CAPP2_SORB      YITTLNVCQAYTLKRIRDPDYHVALRPHLSKEIMDPTKAASELVKLNPGS 935
gi|1705585|sp|P51059|CAPP2_MAI      YITTLNVCQAYTLKRIRDPDYHVALRPHLSKEIMDSTKAAADVVKLNPGS 942
gi|3341490|emb|CAA07610.1|          YITTMNVCQAYTLKRIRDPDYHVALRPHLSKEVMDTSKPAAELVTLNPAS 947
gi|115478504|ref|NP_001062847.      YITTLNVCQAYTLKRIRDPDYHVTLRPHLSKEVMDGSKPAAELVKLNPGS 950
gi|115476100|ref|NP_001061646.      YITTLNVCQAYTMKRIRDPDYHVTLRPHMSKEIMDWSKPAAELVKLNPTS 939
gi|115577|sp|P29193|CAPP1_SACH      YITALNVCQAYMLKRIRDPGFQVNPGPHLSKDIMDMGKPASELVKLNTTS 941
gi|115440043|ref|NP_001044301.      YITALNVCQACTLKRIRDPGFHVSPRAHLSKDIMDSGKPAAELVKLNTTS 899
gi|9828445|gb|AAG00180.1|AF271      YITTLNVCQAYTLKRIRDPSFEVMSQPALSKEFVDSNQP-AELVQLNAAS 934
gi|115445275|ref|NP_001046417.      YITTLNVCQAYTLKRIRDPSFEVMSQPALSKEFVDSNQP-AELVQLNAAS 943
Welwitschiamirabilis"tissue_ty      YITTLNALQAYTLKRIRDPSYHVTLRPHLSKE--SSTKPAAELVKLNPTS 925
Welwitschia                         YITTLNALQAYTLKRIRDPSYHVTLRPHLSKE--SSTKPAAELVKLNPTS 925
gi|1705590|sp|P51063|CAPP_PICA      YITTLNVCQAYTLKRIRDPNYHVNLRPHLSKE--SSTKPAAELVKLNPTS 938
Chlamydomonasreinhardtii"           FVDPINILQVEVLRRLRQDPNNMRLR------------------------ 1203
Chlamydomonas                       FVDPINILQVEVLRRLRQDPNNMRLR------------------------ 1203
Glycinemax"phosphoenolpyruvate      FLNPLNMLQVEILKRLRRDDDNRKIR------------------------ 1014
Glycine                             FLNPLNMLQVEILKRLRRDDDNRKIR------------------------ 1014
Ricinuscommunis"phosphoenolpyr      YLNPMNMLQVEVLKRLRRDDDNNKLR------------------------ 1034
Ricinus                             YLNPMNMLQVEVLKRLRRDDDNNKLR------------------------ 1034
gi|30697740|ref|NP_177043.2|        YLNAMNMLQVEILKRLRRDEDNNKLR------------------------ 1014
Synechococcuselongatus              TIVPLGFLQVALLKRLRQYRQQTETTGLMRSRYSKG-------------- 1032
Anabaenavariabilis"phosphoenol      TIVPLGFIQVSLLKRLRQSKNNTATSGVIHSRYSKG-------------- 961
Synechococcussp.                    TIVPLGFLQVSLLKRLRQYTRETQAS-IVHFRYSKE-------------- 974
Synechococcusvulcanus"phosphoe      TIVPLGFLQVSLLKRLRQHNSQTTSGAILRSRYGRG-------------- 990
Streptomycescoelicolor              YLDPISYLQVALLGRQREAAA--------ANEDPDP-------------- 890
Rhodopseudomonaspalustris""pho      YLDPLNHVQVQLLREHR-------------THDPDE-------------- 915
```

FIGURE 2 (continued)

```
gi|79319830|ref|NP_001031179.1      EYAPGLEDTLILTMKGIAAGLQNTG 967
gi|73917651|sp|Q84VW9|CAPP3_AR      EYAPGLEDTLILTMKGIAAGLQNTG 968
gi|1705587|sp|P51061|CAPP2_SOY      EYAPGLEDTLILTMKGIAAGMQNTG 967
gi|399183|sp|Q02735|CAPP_MEDSA      EYAPGLEDTLILTMKGIAAGMQNTG 966
gi|23503558|dbj|BAC20365.1|         EYAPGLEDTLILTMKGIAAGMQNTG 967
gi|115610|sp|P27154|CAPP_TOBAC      EYAPGLEDTLILTMKGIAAGLQNTG 964
gi|972511|emb|CAA62469.1|           EYAPGLEDTLILTMKGIAAGMQNTG 965
gi|30689081|ref|NP_850373.1|        -------------------------
gi|45505269|gb|AAS67006.1|          EYAPGLEDTLILTMKGIAAGMQNTG 966
gi|115583|sp|P29194|CAPP2_SORB      EYAPGLEDTLILTMKGIAAGLQNTG 960
gi|1705585|sp|P51059|CAPP2_MAI      EYAPGLEDTLILTMKGIAAGLQNTG 967
gi|3341490|emb|CAA07610.1|          EYAPGLEDTLILTMKGIAAGLQNTG 972
gi|115478504|ref|NP_001062847.      EYAPGLEDTLILTMKGIAAGLQNTG 975
gi|115476100|ref|NP_001061646.      EYAPGLEDTLILTMKGIAAGMQNTG 964
gi|115577|sp|P29193|CAPP1_SACH      EYAPGLEDTLILTMKGIAAGMQNTG 966
gi|115440043|ref|NP_001044301.      EYGPGLEDTLILTMKGIAAGMQNTG 924
gi|9828445|gb|AAG00180.1|AF271      EYAPGLEDTLILTMKGIAAGMQNTG 959
gi|115445275|ref|NP_001046417.      EYAPGLEDTLILTMKGIAAGMQNTG 968
Welwitschiamirabilis"tissue_ty      EYAPGLEDTLILTMKGIAA------ 944
Welwitschia                         EYAPGLEDTLILTMKGIAA------ 944
gi|1705590|sp|P51063|CAPP_PICA      EYAPGLEDTLILTMKGIAAGMQNTG 963
Chlamydomonasreinhardtii"           -------DALLISINGIAAGMRNTG 1221
Chlamydomonas                       -------DALLISINGIAAGMRNTG 1221
Glycinemax"phosphoenolpyruvate      -------DALLITINGIAAGMKNTG 1032
Glycine                             -------DALLITINGIAAGMKNTG 1032
Ricinuscommunis"phosphoenolpyr      -------DALLITINGIAAGMRNTG 1052
Ricinus                             -------DALLITINGIAAGMRNTG 1052
gi|30697740|ref|NP_177043.2|        -------DALLITINGIAAGMRNTG 1032
Synechococcuselongatus              ----ELLRGALLTINGIAAGMRNTG 1053
Anabaenavariabilis"phosphoenol      ----ELLRGALLTINGIAAGMRNTG 982
Synechococcussp.                    ----ELLRGALLTINGIAAGMRNTG 995
Synechococcusvulcanus"phosphoe      ----ELLRGALLTINGIAAGMRNTG 1011
Streptomycescoelicolor              ----LLARALLLTVNGVAAGLRNTG 911
Rhodopseudomonaspalustris""pho      ----QVLRGVQLTINGISAGLRNSG 936
```

FIGURE 2 (continued)

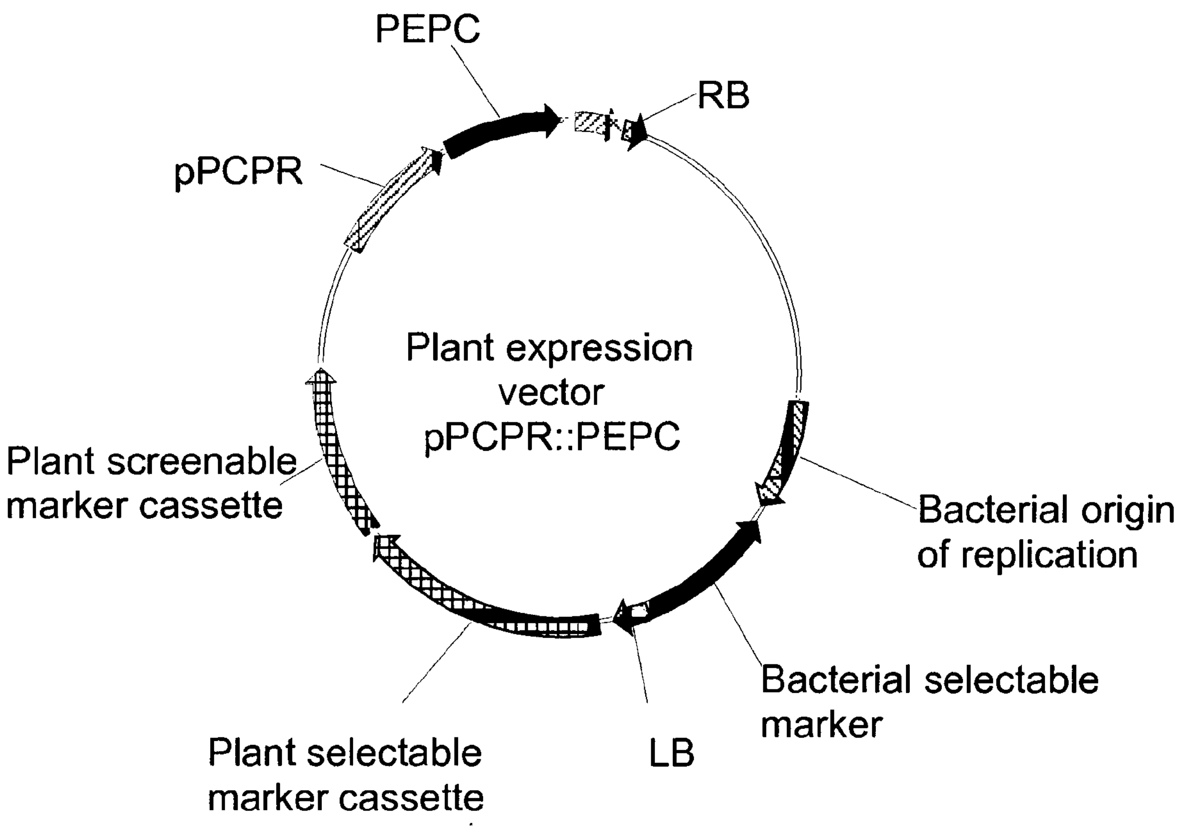
PEPC
RB
pPCPR
Plant expression vector pPCPR::PEPC
Plant screenable marker cassette
Bacterial origin of replication
Bacterial selectable marker
LB
Plant selectable marker cassette

**FIGURE 3**

**SEQ ID NO: 01, DNA - Synechocystis sp.**

TACAAAAAAGCAGGCTTAAACAATGAACTTGGCAGTTCCTGCATTCGGTCTTTCCACTAACTGGTCT
GGTAATGGCAATGGTTCCAACTCTGAAGAAGAGTCGGTGCTTTACCAGCGGTTAAAGATGGTGGAGG
AATTGTGGGAAAGGGTGCTCCAAAGCGAATGTGGCCAGGAATTGGTGGATTTGCTGACGGAATTAAG
GCTTCAGGGTACCCATGAGGCGATCACCAGCGAAATTTCCGAAGAAGTCATCATGGGTATTACCCAG
CGCATTGAGCATTTAGAACTCAATGATGCCATCCGGGCGGCTCGGGCCTTTGCCCTATATTTCCAGT
TGATCAACATCGTTGAACAGCACTACGAACAAAACGAGCAACAACGGAATCGTTGGGAAGCTTCCCA
GGAAACCAACTTCTATGAGCAGGCGGGCAATGAGGAAGAAATGGTCCCCCCATCCCGATTAGGCGCG
TCAACGGAACCATTGCCAGTGGGCATTGACCAGAATGAATTGCAAGCTTCTGTAGGTACGTTCCATT
GGTTAATGAGGGAGCTAAAACGCCTCAATGTGCCCCCCCAACATATCCAAAATTTATTGGATCATCT
GGACATTCGCCTGGTGATCACCGCTCACCCCACGGAAATTGTCCGTCACACCATCCGGCGCAAACAA
AGAAGGGTGGACCGCATTCTTCGTAAACTAGATCAACTCCAGGGTTCTGTGACCGGTCGGGACTGGC
TCAACACCTGGGATGCAAAAACGGCGATCGCCCAATTAACGGAGGAAATTCGCTTTTGGTGGCGTAC
CGACGAACTTCATCAGTTCAAACCCACTGTGTTGGACGAAGTGGACTATTCCCTCCATTATTTTGAT
GAAGTACTGTTCGACGCTGTACCGGAATTGTCCAAACGGTTAGGACAAGCTATTAAAGAAACCTTTC
CCCATCTGCGGGCCCCCCGGGCTAATTTTTGTTATTTTGGCTCCTGGGTCGGTGGCGATCGGGACGG
CAACCCTTCGGTAACCCCAGAAGTGACCTGGCAGACGGCCTGTTACCAGCGGGGTTTAGTGCTGGGG
AAATATTTGTTTAGTTTGGGGGAACTGGTGGCCATTCTTAGCCCTTCCCTCCATTGGTGCAAAGTCT
CCCAGGAATTGTTGGACTCTTTGGAACGGGACCGCATTCAATTACCGGAAATTTACGAAGAACTTTC
TCTCCGCTATCGCCAGGAACCCTATCGGATGAAGCTGGCCTACGTTACCAAACGGCTGGAAAACACC
CTGCGGCGTAATAATCGTCTAGCCAACCCAGAAGAACGGCAAACGATGATCACCATGCCGGCCGAAA
ATCACTATCGCACTGGGGAAGAATTATTAGAGGAATTAAGACTCATTCAGCGTAATCTGACCGAAAC
TGGTTTAACCTGCCTGGAGTTGGAAAATTTGATTACCCAGTTGGAAGTCTATGGCTTTAACCTAGCC
CAGTTGGATTTTCGCCAGGAATCTTCCCGCCACGCCGAGGCGATCGCCGAAATTGCTGAGTATATGG
GGGTACTCACCACTCCCTACGAAGAAATGGCCGAAGAAGATAAATTAGCCTGGTTAGGGGTAGAACT
GCAAACCCGCCGTCCTTTAATTCCCCAGGAAATGCCCTTTTCGGAGCGGACTAGGGAAACCATTGAA
ACCCTCCGCACCCTGCGCCATCTACAAATGGAATTTGGGGTGGATATTTGCCAAACCTACATCATCA
GCATGACCAACGATGCCAGTGATGTGTTGGAAGTATTGCTGTTAGCCAAGGAAGCCGGATTGTATGA
CCCGGCCACCGCCTCCAATTCCCTCCGCATTGTGCCCCTGTTTGAAACAGTAGAAGATCTCAAAAAC
GCTCCGGGGATTATGGATTCTCTTTTCAGCTTGCCTTTTTACCGGGCTACATTGGCGGGCAGTTACC
ATTCCTTAAAAGAGTTGCAAAATCAGCCACCGGATTATTACCAAATTCCCACCACCACAGCCCTACT
AAATCCCGGCAATCTCCAGGAAATTATGGTGGGCTATTCCGACAGCAATAAAGACTCCGGCTTTTTG
AGCAGTAACTGGGAAATTCATAAGGCCCAAAAATCACTGCAGGCAGTGGCCCAAAGCCATCGGGTAA
TTCTCCGGCTGTTCCACGGTCGAGGAGGATCTGTTGGCCGGGGGGGCGGCCCGGCCTATAAAGCCAT
TTTGGCCCAGCCCGCAGGCACCGTGGACGGTCGGATCAAAATTACCGAACAAGGGGAAGTGTTAGCT
TCTAAATATTCCCTGCCAGAGTTGGCCCTCTACAACCTGGAAACTTTAACCACGGCGGTCATCCAAG
CTAGTTTACTTAAAAGTAGTTTTGATTTCATTGAGCCCTGGAACCGGATTATGGAGGAGTTGGCCTG
CACTGCCCGTCGAGCCTACCGGAGTTTGATTTACGAAGAACCGGACTTTTTAGATTTCTTCCTGACG
GTTACCCCCATTCCTGAAATTAGCGAGTTACAGATTAGTTCCCGCCCTGCCCGACGTAAGGGGGGTA
AAGCGGATCTCAGCAGTTTGCGGGCCATTCCCTGGGTGTTCAGTTGGACCCAAACCCGTTTCCTGCT
GCCGGCTTGGTATGGGGTGGGCACGGCGTTGAAATCCTTTGTGGACCAAGACCCGGTCAAAAATATG
AAGTTGTTGCGTTACTTCTATTTCAAATGGCCTTTCTTCAACATGGTGATCTCGAAGGTGGAAATGA
CCCTTTCCAAGGTGGACCTCACCATCGCTTCCCACTATGTGCAAGAGCTATCTAAGCCAGAAGACCG
GGAACGATTCGATCGCCTTTTTCAGCAGATCAAGCAAGAGTATCAATTAACCAGGGACTTTGCCATG
GAAATTACGGCCCATCCCCACCTCCTGGACGGCGATCGCTCTTTGCAACGGTCGGTACTCCTACGAA
ATCGTACTATTGTTCCCCTGGGGCTACTGCAGATTTCCCTGCTGAAACGTTTACGCCAAGTAACCCA
GGAAGCGGAGACCAGCGGCGTGCGTTACCGTCGTTATTCCAAAGAAGAACTACTGCGGGGAGCTCTG
TTAACCATTAACGGTATTGCGGCCGGAATGCGTAATACTGGTTGAACCCAGCTTTCTTGTACAA

**FIGURE 4**

**SEQ ID NO: 02, protein - Synechocystis sp.**
MNLAVPAFGLSTNWSGNGNGSNSEEESVLYQRLKMVEELWERVLQSECGQELVDLLTELRLQGTHEA
ITSEISEEVIMGITQRIEHLELNDAIRAARAFALYFQLINIVEQHYEQNEQQRNRWEASQETNFYEQ
AGNEEEMVPPSRLGASTEPLPVGIDQNELQASVGTFHWLMRELKRLNVPPQHIQNLLDHLDIRLVIT
AHPTEIVRHTIRRKQRRVDRILRKLDQLQGSVTGRDWLNTWDAKTAIAQLTEEIRFWWRTDELHQFK
PTVLDEVDYSLHYFDEVLFDAVPELSKRLGQAIKETFPHLRAPRANFCYFGSWVGGDRDGNPSVTPE
VTWQTACYQRGLVLGKYLFSLGELVAILSPSLHWCKVSQELLDSLERDRIQLPEIYEELSLRYRQEP
YRMKLAYVTKRLENTLRRNNRLANPEERQTMITMPAENHYRTGEELLEELRLIQRNLTETGLTCLEL
ENLITQLEVYGFNLAQLDFRQESSRHAEAIAEIAEYMGVLTTPYEEMAEEDKLAWLGVELQTRRPLI
PQEMPFSERTRETIETLRTLRHLQMEFGVDICQTYIISMTNDASDVLEVLLLAKEAGLYDPATASNS
LRIVPLFETVEDLKNAPGIMDSLFSLPFYRATLAGSYHSLKELQNQPPDYYQIPTTTALLNPGNLQE
IMVGYSDSNKDSGFLSSNWEIHKAQKSLQAVAQSHRVILRLFHGRGGSVGRGGGPAYKAILAQPAGT
VDGRIKITEQGEVLASKYSLPELALYNLETLTTAVIQASLLKSSFDFIEPWNRIMEELACTARRAYR
SLIYEEPDFLDFFLTVTPIPEISELQISSRPARRKGGKADLSSLRAIPWVFSWTQTRFLLPAWYGVG
TALKSFVDQDPVKNMKLLRYFYFKWPFFNMVISKVEMTLSKVDLTIASHYVQELSKPEDRERFDRLF
QQIKQEYQLTRDFAMEITAHPHLLDGDRSLQRSVLLRNRTIVPLGLLQISLLKRLRQVTQEAETSGV
RYRRYSKEELLRGALLTINGIAAGMRNTG

SEQ ID NO: 03, DNA - Anacystis nidulans
CTGGGTCTCCTTGAGATCGTATGCTCGCTGTATCTCTCAGGGATCACGGCTTCCCGTCAGCAACTGT
CCAGATCCGCCCTGCGTTCTGTGTCCAGTCAGACGTGGTGGGATCAGGGAATCCACCTAGAATGAAT
TACTGCCAGAACGCTCGAACTGCCATGAGTGCTGCTCTCCAGTCATCCGACGATGCTTTCCGAACCG
TTTCGAGTCCCCTCGCCACGGATTTGGATCTGTCGTCTCCGCTGGAGTTTTTCCTTCGCCATCGCTT
GACGGTGGTTGAAGAACTCTGGGAAGTGGTTTTGCGCCAAGAGTGCGGCCAAGAGCTGGTCGATATT
CTGACTCAGCTGCGTGACTTGACCTCGCCGGAAGGCCAAGCCCCAGAAGTGGGCGGCGAAGCCTTGG
TTCAGGTGATTGAAACCCTAGAGTTGAGCGATGCGATTCGGGCTGCCCGTGCCTTTGCGCTCTACTT
TCAGCTGATCAATATTGTTGAGCAGCACTACGAGCAAACTCAATATCAACTCGCCTACGAGCGATCG
CGGTTGGAACCCTTGCCAGGACCAGATGAAAGTCCGGAGGGATTGCACACCATTGAAATTCCTCAGC
ATCAGCTCGATCCCTTTGCTGCGGTGATTCCGCTCAACCAAGATCCGGCAACCTTCCAAACGCTGTT
CCCGCGCCTGCGCCAGCTCAATGTGCCGCCGCAAATGATCCAAGAGCTGACCGATCGCCTCGATATT
CGGCTGGTTTTCACCGCTCACCCGACGGAAATTGTCCGCCACACGATTCGCGACAAACAACGCCGAA
TTGCCTACCTGCTGCGGCAACTGGATGAGCTCGAAACAGGCAAAAACCGAGGCTTTCGAGAGCTTGA
AGCGCAGAATATTCGTCAGCAGCTGACCGAGGAGATTCGGCTCTGGTGGCGGACGGATGAGCTCCAC
CAGTTCAAGCCAACGGTGTTGGATGAGGTGGACTACGCGCTCCACTACTTCCAAGAAGTCCTCTTTG
AGGCCATTCCTCTGCTCTATCAGCGCTTTCGGCTCGCGCTGCAGGGGACTTTCCCCGACCTACAACC
GCCCCGCTACAACTTCTGCCAGTTCGGCTCTTGGGTCGGCTCCGATCGCGATGGCAATCCTTCAGTG
ACCTCTGCCGTCACTTGGCAAACCGCTTGCTATCAGCGCAGTCTCGTCCTCGATCGCTACATCACAG
CGGTTGAACATCTCCGCAATGTGCTCAGCCTCTCGATGCACTGGAGCGAGGTGCTGCCGGAGTTGCT
CAGCTCGTTGGAACAGGAGAGCATGCTCTTCCCGGAGACCTATGAGCAGCTAGCGGTCCGCTATCGC
CAAGAGCCCTATCGCCTCAAGCTCTCCTATATTCTGGAGCGCCTGCACAACACCCGCGATCGCAATA
CCCGCCTCCAACAGCAGCAAGAAAAAGATCCCACCACGCCCCTGCCCGAATATCGGGATGGCACCCT
CTACCAGGCTGGTACGGCCTTTCTCGAAGATCTCAAGCTGATTCAGCACAACCTTAAGCAGACGGGA
CTGAGCTGTTACGAGCTAGAGAAGTTGATCTGCCAGGTCGAGATCTTTGGTTTCAACCTGGTCCATC
TCGACATTCGCCAAGAAAGCTCGCGCCATTCCGACGCGATCAACGAAATCTGTGAATACCTCCAAAT
TCTTCCCCAGCCCTACAACGAGCTGAGCGAAGCAGAACGAACTGCCTGGCTGGTTCAAGAGCTGAAA
ACCCGTCGGCCGCTGGTACCAGCGCGCATGCCGTTCTCAGAATCGACCCGCGAGATCATTGAAACCC
TGCGGATGGTCAAGCAGCTACAGGAAGAATTTGGGGAGGCGGCTTGCCAAACCTACATCATCAGCAT
GAGCCGCGAGCTGAGCGACCTGCTGGAAGTGCTGCTGC

**FIGURE 4 (continued)**

TGGCCAAGGAGGTTGGTCTCTACGACCCAGTCACCGGCAAGAGTTCGCTTCAGGTGATTCCGCTGTT
TGAAACTGTGGAGGACTTACAAAATGCCCCGCGGGTGATGACGGCGCTGTTTGAGCTGCCCTTCTAC
ACCCAGCTCAACCCCACCCAGTCTGAACCGCTGCAGGAAGTGATGCTGGGGTATTCCGACAGTAACA
AGGACTCGGGCTTCCTCAGCAGTAACTGGGAGATCCACAAGGCCCAGAAAGCCCTAGGGACGGTAGC
CCGCGACCACCGCGTCAAGCTGCGGATCTTCCACGGCCGCGGGGGCTCCGTCGGTCGAGGTGGTGGC
CCTGCCTACGAGGCGATCTTGGCCCAGCCGGGTCGCACCACAGATGGCCGAATCAAGATTACGGAAC
AGGGCGAGGTCTTGGCTTCGAAATACGCCCTGCCCGAACTGGCGCTCTATAACCTTGAGACGATCAC
GACGGCGGTGATTCAGTCCAGCCTGCTGGGTAGCGGCTTTGATGACATTGAGCCGTGGAACCAAATT
ATGGAAGAGTTGGCGGCGCGATCGCGGCGACATTACCGCGCTTTGGTGTACGAGCAGCCCGACCTGG
TTGACTTCTTCAATCAGGTAACGCCGATTGAGGAGATCAGCAAACTGCAAATCAGCTCGCGACCGGC
TCGACGCAAAACCGGCAAGCGCGATCTGGGCAGTCTACGTGCCATCCCCTGGGTCTTTAGCTGGACG
CAGAGTCGTTTTCTGCTGCCCTCTTGGTATGGCGTCGGCACAGCACTTCAGGAGTTTTTGCAGGAGC
GCCCGGAGCAGAACCTCAACCTGCTGCGCTACTTCTACGAGAAGTGGCCGTTCTTCCGCATGGTGAT
CTCGAAGGTCGAGATGACCCTAGCGAAGGTCGATTTGCAGATTGCTCATCACTACGTGCATGAGCTG
GCCAATCCTGAGGATCAAGAGCGGTTTGAACGAGTGTTCAGCCAAATCGCTGCAGAGTTTCAGCTGA
CTTGTCATCTCGTGTTGACGATTACCAACCACGGTCGCTTGCTGGATGGCGACCCCGAACTGCAGCG
ATCGGTGCAGCTGCGCAACGGTACGATCGTGCCCCTCGGCTTCTTGCAAGTCGCCCTGCTTAAACGC
CTGCGGCAGTATCGCCAGCAAACGGAAACGACGGGATTGATGCGATCGCGCTATAGCAAAGGGGAAC
TGCTGCGCGGAGCATTGCTGACGATCAACGGCATTGCGGCTGGCATGCGCAATACAGGTTGA

SEQ ID NO: 04, protein - Synechococcus elongatus
MLAVSLRDHGFPSATVQIRPAFCVQSDVVGSGNPPRMNYCQNARTAMSAALQSSDDAFRTVSSPLAT
DLDLSSPLEFFLRHRLTVVEELWEVVLRQECGQELVDILTQLRDLTSPEGQAPEVGGEALVQVIETL
ELSDAIRAARAFALYFQLINIVEQHYEQTQYQLAYERSRLEPLPGPDESPEGLHTIEIPQHQLDPFA
AVIPLNQDPATFQTLFPRLRQLNVPPQMIQELTDRLDIRLVFTAHPTEIVRHTIRDKQRRIAYLLRQ
LDELETGKNRGFRELEAQNIRQQLTEEIRLWWRTDELHQFKPTVLDEVDYALHYFQEVLFEAIPLLY
QRFRLALQGTFPDLQPPRYNFCQFGSWVGSDRDGNPSVTSAVTWQTACYQRSLVLDRYITAVEHLRN
VLSLSMHWSEVLPELLSSLEQESMLFPETYEQLAVRYRQEPYRLKLSYILERLHNTRDRNTRLQQQQ
EKDPTTPLPEYRDGTLYQAGTAFLEDLKLIQHNLKQTGLSCYELEKLICQVEIFGFNLVHLDIRQES
SRHSDAINEICEYLQILPQPYNELSEAERTAWLVQELKTRRPLVPARMPFSESTREIIETLRMVKQL
QEEFGEAACQTYIISMSRELSDLLEVLLLAKEVGLYDPVTGKSSLQVIPLFETVEDLQNAPRVMTAL
FELPFYTQLNPTQSEPLQEVMLGYSDSNKDSGFLSSNWEIHKAQKALGTVARDHRVKLRIFHGRGGS
VGRGGGPAYEAILAQPGRTTDGRIKITEQGEVLASKYALPELALYNLETITTAVIQSSLLGSGFDDI
EPWNQIMEELAARSRRHYRALVYEQPDLVDFFNQVTPIEEISKLQISSRPARRKTGKRDLGSLRAIP
WVFSWTQSRFLLPSWYGVGTALQEFLQERPEQNLNLLRYFYEKWPFFRMVISKVEMTLAKVDLQIAH
HYVHELANPEDQERFERVFSQIAAEFQLTCHLVLTITNHGRLLDGDPELQRSVQLRNGTIVPLGFLQ
VALLKRLRQYRQQTETTGLMRSRYSKGELLRGALLTINGIAAGMRNTG

**SEQ ID NO: 05, DNA - Synechococcus sp.**
ATGAACCAAGTCATGCATCCCCCCAGTGCCGAAGCTGAACTTTTGTCCACTTCCCAATCTCTCCTCC
GGCAACGCCTCACGCTGGTTGAAGACATTTGGCAGGCGGTGTTGCAGAAAGAATGTGGTCAAAAACT
CGTCGAACGGCTCAATCATCTCCGGGCAACCCGAACCGCCGATGGCCAAAGCCTAAACTTCTCTCCA
AGTAGTATTTCTGAGCTGATCGAAACCCTAGATCTAGAAGATGCGATCCGGGCAGCACGGGCTTTTG
CCCTCTATTTCCAGCTGATCAATAGCGTCGAGCAACATTACGAACAGCGCGAACAACAACAATTTCG
GCGGAACCTCGCCTCTGCCAATGCCAGCGAAGCCAACGGTAACAGCGTCCATACAGAAATTGCCCCC
ACCCAAGCCGGCACCTTTGATTGGTTGTTTCCCCATCTCAAGCACCAAAATATGCCGCCCCAGACGA
TCCAGCGTCTTCTCAATCAGTTGGATATTCGACTGGTATTTACGGCCCACCCCACGGAG

## FIGURE 4 (continued)

ATTGTCCGCCACACGATTCGCAATAAGCAGCGGCGGATTGCGGGGATCCTACGGCAACTGGATCAAA
CCGAAGAAGGGCTGAAAAGCTTGGGGACAAGCGATTCTTGGGAAATTGAAAATATTCAGCAGCAACT
TACCGAAGAAATTCGGCTCTGGTGGCGCACCGATGAGCTGCACCAATTTAAACCCCAGGTGTTGGAC
GAAGTGGACTATGCGTTGCATTATTTTGAGGAAGTTCTGTTTGACACCCTGCCCGAATTATCGGTAC
GGCTCCAACAGGCCCTAAAGGCATCTTTCCCAACCCTCAAGGTGCCCACCACCAATTTTTGTAATTT
TGGCTCTTGGGTCGGGGGCGATCGCGATGGCAACCCTTCTGTCACTCCTGACGTTACGTGGAAGACA
GCTTGCTATCAACGGGGTTTGGTGCTGGAACGCTACATCGCCTCGGTGGAATCCCTCTCGGATGTGT
TGAGCTTATCGCTGCACTGGAGTAATGTGCTGCCGGATCTGCTGCATTCATCGTACCAAGATCAGAA
TATTTTCCCCGACGTCTATCAAAGCCTGGCCAGTCGTCACCGTCAAGAACCCTATCGTCTCAAGTCA
GCCTACATTAAGCGGCGCTTGGAAAATACCTTGGAGCGCAACCGTCGTCTAGCGAATATGCCCGCCT
GGGAAAATAAAGTAGAAGCGGCCGATGACAAGGTTTATATTTGTGGCCAGGAGTTTCTCGCAGACCT
AAAACTGATCCGCGAAAGCCTGGTACAGACGGAAATCAACTGTGCGGCCCTGGATAAACTCATTTGC
CAAGTCGAAATTTTTAGCTTTGTGCTCACTCGCCTTGATTTTCGCCAAGAATCAACGCGCCACTCCG
ATGCGATCGCCGAAATTGTTGACTACCTGAGTATTGCCCAAATCCTACAACGACCTAAGCGACGCAG
AAAAAACCACCTTGGTCTAGTGCAGGAATTAAAAACCCGCCGTCCCTTGATTCCGAAGGAAATGCAC
TTTTCGGAAAGAACCGTCGAGACGATCCAAACGCTCCAGGTGCTGCGCCGTCTCCAGCAGGAATTTG
GCATCGGCATTTGCCAGACCTACATCATCAGCATGACCAACGAAGTCAGTGATGTCTTAGAGGTGCT
GCTCTTGGCCCAGGAAGCGGGTTTGTATGACCCCCTCACAGGGATGACCACCATCCGCATTGCCCCC
CTCTTTGAGACGGTGGACGACCTGCGTAACGCCCCGGAAATCATGCAGGCGTTGTTTGAAATTCCCC
TCTATCGCGCTTGTTTAGCTGGTGGCTATGAACCCCCCGCCGATGGCCGCTGTGATGAAACCTTTGG
CGATCGCCTGGTGCCGAATCTCCAGGAAATTATGCTCGGCTATTCCGACAGCAACAAAGATTCCGGC
TTCCTCAGTAGTAATTGGGAAATCCACAAAGCCCAGAAAAATCTCCAACAAGTGGCCGATCCCTACG
GCATTGACCTCCGCATTTTCCATGGTCGCGGTGGTTCCGTTGGTCGGGGCGGCGGCCCTGCCTACGC
CGCTATTTTGGCCCAACCGCCCAACACCATCAATGGCCGGATTAAAATCACCGAACAGGGGGAAGTG
CTGGCGTCTAAATATTCCCTCCCAGACTTGGCGCTATATCACCTCGAAAGCGTTTCTACGGCGGTGA
TTCAATCGAGTTTGTTAGCCAGTGGCTTTGATGATATTCAGCCCTGGAACCGGATTATGGAAGACCT
CTCCCAGCGATCGCGGGCGGCTTACCGGGCGTTAATTTACGAAGAACCCGACTTCCTCGACTTTTTC
ATGTCCGTGACGCCGATTCCGGAAATTAGCCAACTGCAAATCAGTTCCCGTCCGGCCCGTCGCAAAA
AAGGCAATAAAGATTTGAGTAGTCTCCGGGCGATTCCCTGGGTCTTTAGCTGGACCCAAAGCCGCTT
CCTCGTGCCCGCTTGGTATGGTGTGGGTACCGCGCTCCAGGGCTTTTTTGAAGAGGATCCCGTCGAG
AACCTGAAGTTAATGCGCTATTTCTACAGTAAATGGCCCTTTTTCCGGATGGTGATCTCAAAGGTAG
AGATGACCCTTTCTAAGGTGGACTTACAAATGGCGAGCCACTATGTCCACGAACTGGCGGAAAAAGA
AGATATTCCCCGGTTTGAAAAGCTTCTAGAGCAGATTTCCCAGGAATACAACCTCACGAAGCGGCTA
ATCCTCGAAATTACCGAAAATGAAGCCCTCCTCGATGGCGATCGCCCCCTCCAGCGTTCTGTCCAAC
TGAGAAATGGCACCATTGTCCCCCTCGGTTTCCTGCAAGTCTCATTGCTCAAACGGCTACGTCAATA
CACCAGGGAAACCCAAGCCAGTATCGTTCACTTCCGCTACAGCAAAGAAGAACTCCTGCGGGGCGCC
CTTTTAACCATCAATGGCATCGCCGCCGGAATGCGGAACACGGGTTGA

**SEQ ID NO: 06, protein - Synechococcus sp.**
MNQVMHPPSAEAELLSTSQSLLRQRLTLVEDIWQAVLQKECGQKLVERLNHLRATRTADGQSLNFSP
SSISELIETLDLEDAIRAARAFALYFQLINSVEQHYEQREQQQFRRNLASANASEANGNSVHTEIAP
TQAGTFDWLFPHLKHQNMPPQTIQRLLNQLDIRLVFTAHPTEIVRHTIRNKQRRIAGILRQLDQTEE
GLKSLGTSDSWEIENIQQQLTEEIRLWWRTDELHQFKPQVLDEVDYALHYFEEVLFDTLPELSVRLQ
QALKASFPTLKVPTTNFCNFGSWVGGDRDGNPSVTPDVTWKTACYQRGLVLERYIASVESLSDVLSL
SLHWSNVLPDLLHSSYQDQNIFPDVYQSLASRHRQEPYRLKSAYIKRRLENTLERNRRLANMPAWEN
KVEAADDKVYICGQEFLADLKLIRESLVQTEINCAALDKLICQVEIFSFVLTRLDFRQESTRHSDAI
AEIVDYLSIAQILQRPKRRRKNHLGLVQELKTRRPLIPKEMHFSERTVETIQTLQVLRR

## FIGURE 4 (continued)

LQQEFGIGICQTYIISMTNEVSDVLEVLLLAQEAGLYDPLTGMTTIRIAPLFETVDDLRNAPEIMQA
LFEIPLYRACLAGGYEPPADGRCDETFGDRLVPNLQEIMLGYSDSNKDSGFLSSNWEIHKAQKNLQQ
VADPYGIDLRIFHGRGGSVGRGGGPAYAAILAQPPNTINGRIKITEQGEVLASKYSLPDLALYHLES
VSTAVIQSSLLASGFDDIQPWNRIMEDLSQRSRAAYRALIYEEPDFLDFFMSVTPIPEISQLQISSR
PARRKKGNKDLSSLRAIPWVFSWTQSRFLVPAWYGVGTALQGFFEEDPVENLKLMRYFYSKWPFFRM
VISKVEMTLSKVDLQMASHYVHELAEKEDIPRFEKLLEQISQEYNLTKRLILEITENEALLDGDRPL
QRSVQLRNGTIVPLGFLQVSLLKRLRQYTRETQASIVHFRYSKEELLRGALLTINGIAAGMRNTG

SEQ ID NO: 07, DNA - Anabaena variabilis
CGCCTGCCTATTTTACCCTTCCCTGATGACGATGAATCAACCAATTAAAGTTTAAAAATATACTTCA
AAAATATGCTCACATAAGTAGATAAGAGCATAAGTTACCAAAAATCAATCTGTTAAACAGTAATTGG
CTTTACAAGAGAAGCAAGTATGGGTTCTGTTTTATACTCTTTATCTGAATCTGCTAATTTATATCCA
GCATCGGAATTATTCTTGCGTCATCGTCTCCAAATAGTAGAAGAACTGTGGGAGTCGGTACTGCGGC
AAGAATGCGGCCAAAATATGGTGGATTTGTTACGGCAGTTGCGTGATTTGTGTTCACCAGAAGGGCA
AGCCACAAAAGACCAAGCAGTTTCTGCTGTCAAGTTAATTGAACAGTTGAATATTAATGAAGCCATT
CGGGCAGCTAGGGCTTTTGCTTTGTATTTTCAGCTGATCAACATCATAGAGCAGGAATATGAGCAAA
GGCAGCAATTAACTCGCTATTCTGACTTAGAAGCGGAAACAGCACCCCTAAATGGTTCCGAAAACAT
TACCTCATCCTCTAACCACAACGAAGATGATGTTATTTTCAACCGGGGTTTAGGGACTGATTTCTTA
GGAAAAAATTGGACTAATCGTGGACAAGGGAAACAAAAAGGTACTTTTGCAGCGTTATTTCCCTTAT
TGTCTAAATTGAATGTCCCACCCCAACAAATTCAACGCCTGATTTCCCAACTAGATGTCCGTTTGGT
ATTCACTGCCCACCCAACGGAAATTGTCCGCCACACCATTCGGGACAAACAACGGCAAGTAGTAGAT
TTGTTGCAACAACTCGATGAGGTGGAAAATCGAGCTAAGGACGGCGGCGGCTATCCTTGGGAAGCGG
GGGAAATTCGGGAAAAATTGCTAGAAGAAATTCGCTTGTGGTGGCGTACAGACGAACTCCACCAGTT
TAAGCCCACTGTGCTGGATGAAGTGGATTATGCTTTGCATTACTTCCAAGAAGTTTTATTTGATGGG
ATTCCCCAACTGTATAAGCGTTTCAAATACGCCCTAAATCAAACTTTCTCCTGGTTAGAACCACCAA
GCAAAGATTTCTGTTCCTTTGGTTCTTGGGTAGGTTCAGACAGAGATGGTAATCCATCGGTAACACC
GGAAATTACCTGGCAGACAGCTTGTTATCAGCGCAAAATGGTGCTGGAAAGATATATCAAGTCTGTC
ACCCAATTGATTGAATTATTAAGTATCTCCATGCACTGGAGTGACGTTTTACCAGACTTATTGGAAT
CCTTGGAGTTAGATCAGTCCCAGTTAAGTGAAGTATACGATGCTCTGGCGCTGAGATATCGCCAAGA
ACCCTATCGTCTCAAACTAGCTTACGTCCTCAAGCGCCTGGAAAATACCCGCGATCGCAATTTAGCT
TTATATAAAGGTGAAACCCCCACAAATGAAGATAGCCCCATGTATCGTTCGGGGTCGGAATTTTTGG
CGGAACTGCGATTAATTCAGCATAACTTGACGGAAACAGGTTTAAGCTGCCGAGAATTGGATAATTT
GATCTGTCAAGTGGAAATTTTTGACTTTAACCTGACAAAATTAGACATCCGTCAAGAATCCACCAGA
CATTCGGACGCGTTAAACGAGATTCTCGACTATCTCCAATTATTACCCCAGCCATACAACGACCTCT
CCGAAGAACAGCGAGTTGCTTGGCTAACAACCGAACTGCAAACCCGTCGCCCATTAATTTCGTCAGA
ACTACCATTCTCTGACAAAACTAATGATGTCATTAAAACTTTCCGCGTAGTGCGATCGCTCCAACAA
GAATTTGGCATTAATATCTGCCAAACTTACATCATTAGTATGTGTCGTCAAGTCAGCGATGTCTTAG
AAGTTTTGCTTCTAGCCAAAGAAGCCAGACTTTTTGACCCAGCGATCGCCGTCGGTACAATTCAGGT
TGTACCATTATTTGAGACTGTCGAAGATTTACAACGTTCTCGCAGCGTCATGCGACAGTTATTTGAA
TTACCTCTATACCGCGCTTTGCTAGCTGGTGGTTACAAAAATACCGAAGTAAAAGTACCGAATACTG
AGCTAACACCCCAATCCCCAGCCCCCAGTCCCCAGTCCGTACTCACCCCCGACTTACAAGAAGTCAT
GCTGGGATATTCCGACAGCAACAAAGACTCCGGCTTCTTGAGTAGCAATTGGGAAATTCATAAAGCC
CAAAAATCATTACAGCAAATCGCCGAAGAATATGGCGTAAATTTGCGGATTTTCCACGGGCGCGGCG
GTTCTGTAGGACGAGGCGGCGGCCCAGCCCACGAGGCGATTTTGGCTCAACCAGGACACAGCATCAA
CGGGCGGATTAAAATTACCGAACAAGGGGAAGTATTAGCCTCCAAGTACTCCTTGCTAGATTTAGCC
CTGTACAACTTGGAAACCATCACCACGGCGGTGATTCAAGCCAGCCTCCTGCGAACTGGGTTTGATG
ATATCGAACCTTGGAACGAAATTATGGAA

## FIGURE 4 (continued)

GAATTAGCAGCGCGATCGCGGCAACATTATCGTGGTTTAATTTACGAACAGCCTGATTTCATTGACT
TTTTCCACCAAGTCACACCCATTGAAGAAATCAGTCAACTACAAATTAGTTCTCGTCCCGCCCGTCG
TCCTTCTGGTAAGAAAGATTTAAGCAGTCTCCGCGCCATTCCTTGGGTATTTAGCTGGACACAAACA
CGATTCTTACTACCTTCTTGGTACGGTGTCGGTACAGCTTTACAAGAATTTTTCAACGAAGAACCAG
AAGAACATCTCAAATTGATGCGCTACTTTTATGTCAAGTGGCCTTTCTTCAAGATGGTGATTTCTAA
AGTAGAAATGACCTTGGCGAAAGTAGATATGCAAATGGCTGGTCACTACGTACAAGAATTGTCTGAC
CCTGAAGACAAACCCCGGTTTGAGAAAGTATTTGAGCAAATCGCCAACGAATATTATCTCACCAGAG
ATTTAGTCTTAAAAATCACCGACCACGGTCGGCTTTTAGATGGTGATCCTGTACTCCAGCGTTCTGT
ACAGTTACGCAATGGTACAATCGTCCCACTAGGGTTTATCCAAGTTTCCCTCCTCAAGCGCCTACGC
CAGTCCAAAAATAATACTGCAACCTCTGGTGTCATTCACTCTCGTTACAGTAAGGGAGAATTATTGC
GCGGCGCACTGTTAACCATTAATGGTATTGCCGCAGGGATGAGAAATACAGGTTGATTTGTGAGTTG
TCAGTAGTTAGTGGTCAGTGGTAAAACACATACAACTGACAACTGACAACCGAACAAATGGTCAAAA
ATAAAATCTTAATCTGTACTG

SEQ ID NO: 08, protein - Anabaena variabilis
MVDLLRQLRDLCSPEGQATKDQAVSAVKLIEQLNINEAIRAARAFALYFQLINIIEQEYEQRQQLTR
YSDLEAETAPLNGSENITSSSNHNEDDVIFNRGLGTDFLGKNWTNRGQGKQKGTFAALFPLLSKLNV
PPQQIQRLISQLDVRLVFTAHPTEIVRHTIRDKQRQVVDLLQQLDEVENRAKDGGGYPWEAGEIREK
LLEEIRLWWRTDELHQFKPTVLDEVDYALHYFQEVLFDGIPQLYKRFKYALNQTFSWLEPPSKDFCS
FGSWVGSDRDGNPSVTPEITWQTACYQRKMVLERYIKSVTQLIELLSISMHWSDVLPDLLESLELDQ
SQLSEVYDALALRYRQEPYRLKLAYVLKRLENTRDRNLALYKGETPTNEDSPMYRSGSEFLAELRLI
QHNLTETGLSCRELDNLICQVEIFDFNLTKLDIRQESTRHSDALNEILDYLQLLPQPYNDLSEEQRV
AWLTTELQTRRPLISSELPFSDKTNDVIKTFRVVRSLQQEFGINICQTYIISMCRQVSDVLEVLLLA
KEARLFDPAIAVGTIQVVPLFETVEDLQRSRSVMRQLFELPLYRALLAGGYKNTEVKVPNTELTPQS
PAPSPQSVLTPDLQEVMLGYSDSNKDSGFLSSNWEIHKAQKSLQQIAEEYGVNLRIFHGRGGSVGRG
GGPAHEAILAQPGHSINGRIKITEQGEVLASKYSLLDLALYNLETITTAVIQASLLRTGFDDIEPWN
EIMEELAARSRQHYRGLIYEQPDFIDFFHQVTPIEEISQLQISSRPARRPSGKKDLSSLRAIPWVFS
WTQTRFLLPSWYGVGTALQEFFNEEPEEHLKLMRYFYVKWPFFKMVISKVEMTLAKVDMQMAGHYVQ
ELSDPEDKPRFEKVFEQIANEYYLTRDLVLKITDHGRLLDGDPVLQRSVQLRNGTIVPLGFIQVSLL
KRLRQSKNNTATSGVIHSRYSKGELLRGALLTINGIAAGMRNTG

SEQ ID NO: 09, DNA - Thermosynechococcus vulcanus
GTGAATTATGGCGTTCGCGATCGCCTGCAACACCTGGAACTGATTCTGAGCAGCAAAACAGCTTAGA
ACTGTTAACAAATCCTTAATACGCCTGTTATCAATTGTTGTACCGCCCTGGTTCAGGATTGGGTAAA
GTAAGGAGTATTATCTTTGCAGGCTGGGGTAAATATGACATCAGTCCTCGATGTGACCAATCGCGAT
CGCTTAATTGAAAGTGAAAGTTTGGCAGCCCGTACCCTACAGGAACGGTTGCGACTGGTGGAAGAGG
TCTTGGTCGATGTCTTGGCGGCAGAATCGGGTCAAGAATTGGTTGATCTATTGCGGCGCTTGGGGGC
TCTCTCTTCGCCGGAAGGTCATGTGCTCCATGCCCCAGAAGGGGAATTGCTGAAGGTTATTGAATCC
CTCGAACTCAATGAGGCCATTAGAGCGGCCCGGGCTTTTAACCTCTACTTTCAAATTATCAACATCG
TTGAGCAGCATTACGAACAACAATACAACCGTGAACGCGCTGCCCAAGAGGGATTGCGCCGCCGCAG
TGTCATGAGTGAACCAATTTCCGGTGTCAGTGGTGAAGGCTTTCCGCTGCCTCATACTGCTGCCAAC
GCAACGGATGTGCGCAGTGGGCCGAGTGAACGCCTAGAGCATAGTCTCTACGAAGCCATTCCCGCTA
CTCAGCAGTATGGTTCCTTTGCTTGGCTCTTTCCTCGGCTGCAGATGCTGAATGTGCCGCCGCGCCA
TATTCAAAAGCTTTTGGATCAACTGGACATTAAGTTGGTTTTCACTGCTCACCCGACGGAGATTGTG
CGGCAAACGATTCGTGATAAGCAGCGGCGGGTTGCCCGATTACTTGAGCAACTGGATGTGCTGGAGG
GGGCTTCTCCACACCTAACGGATTGGAACGCCCAAACTTTACGGGCACAACTGATGGAGGAAATTCG
CCTCTGGTGGCGCACCGATGAGTTGCACCAATTTAAGCCGGAGGTGCTCGATGAGGTGGAATACACC
CTCCACTACTTCAAGGAGGTCATTTTTGCTGTCATTCCCAAGCTCTATCGC

**FIGURE 4 (continued)**

CGTCTGGAGCAGTCATTACATGAAACCTTTCCCGCGCTTCAGCCCCCCCGTCACCGTTTCTGCCGCT
TTGGCTCTTGGGTGGGGGGCGATCGCGATGGCAATCCCTATGTCAAACCAGAAGTAACGTGGCAAAC
GGCCTGCTATCAGCGCAACTTAGTTCTTGAGGAGTATATTAAGTCCGTTGAGCGCTTAATCAATTTG
CTCAGCCTGTCCCTGCACTGGTGCGATGTGCTGCCAGATTTGCTAGATTCCCTTGAGCAGGATCAAC
GGCAACTCCCGAGTATCTATGAGCAGTATGCGGTGCGCTATCGGCAGGAACCCTACCGCCTGAAACT
GGCCTATGTGCTCAAACGGCTGCAAAATACCCGCGATCGCAACCGGGCGCTGCAAACCTATTGCATT
CGCCGCAATGAGGCGGAAGAGTTAAATAATGGACAGTTTTACCGCCACGGTGAAGAATTCTTGGCAG
AACTGCTGCTCATTCAGCGTAACCTCAAGGAAACGGGATTGGCCTGCCGCGAATTGGATGATTTGAT
TTGCCAGGTGGAGGTCTTTGGCTTTAATCTAGCAGCCTTGGATATTCGCCAAGAAAGTACCTGTCAC
GCTGAGGCCCTCAATGAAATTACCGCCTATTTGGGTATTCTCCCCTGTCCCTATACAGAACTCTCAG
AAGCCGAACGCACCCGCTGGCTCCTCAGTGAACTCTCGACCCGTCGCCCCTTGATTCCAGGGGAACT
CCCCTTTAGCGATCGCACCAATGAAATCATTGAAACATTCCGCATGGTGCGGCAACTCCAGCAGGAA
TTTGGCACCGATTTGTGCAATACCTACATCATCAGCATGAGCCATGAGGTCAGCGATCTGTTGGAGG
TACTCCTCTTTGCTAAGGAGGCAGGCCTTTTTGATCCAGCCACTGGCGCTAGTACCCTGCAAGCCAT
TCCCCTGTTTGAAACGGTGGAGGATCTCAAGCACGCCCCAGCGGTGCTGACCCAACTATTCTCTCTC
CCCTTTTGCCGGAGCTATCTTGGAAGCAACAGTACCCCCTTTCTGCAGGAGGTCATGCTGGGCTATT
CCGACAGCAATAAGGATTCGGGCTTCCTCAGTAGCAACTGGGAAATTTATAAGGCACAACAACAGCT
GCAGAAAATTGCTGAGAGTTTTGGCTTCCAACTGCGCATTTTCCACGGTCGGGGTGGTTCAGTGGGT
CGGGGTGGTGGACCTGCCTATGCGGCGATTTTGGCACAGCCAGCACAAACGATTAAGGGACGAATCA
AGATTACTGAGCAGGGGGAGGTACTGGCTTCCAAATACTCGTTGCCGGAACTCGCGCTCTTTAACCT
CGAAACAGTGGCCACAGCGGTCATCCAAGCTAGTTTGCTCCGCAGTAGTATTGATGAGATTGAGCCT
TGGCACGAGATTATGGAGGAGTTGGCTACGCGATCGCGCCAGTGCTATCGCCATCTCATCTATGAGC
AGCCAGAATTCATTGAATTCTTTAACGAAGTCACCCCAATCCAAGAGATTAGCCAACTGCAAATTAG
CTCACGGCCAACACGGCGGGGGGGGAAGAAAACCCTTGAGAGCCTGCGGGCAATTCCTTGGGTCTTT
AGTTGGACGCAAACCCGTTTCCTGCTGCCGGCTTGGTATGGCGTGGGTACTGCCCTGAAGGAATTCC
TTGAGGAAAAACCCGCTGAGCATCTCTCCCTCTTGCGCTACTTCTACTATAAGTGGCCTTTCTTCCG
CATGGTGATCTCTAAGGTTGAGATGACCCTTGCCAAGGTTGATCTAGAGATTGCCCGCTACTATGTC
CAAGAACTCAGCCAGCCCCAAAACCGTGAAGCCTTCTGCCGCCTCTACGATCAGATTGCTCAGGAAT
ATCGCCTGACCACGGAATTAGTCCTCACGATTACTGGCCATGAGCGGCTACTCGATGGGGATCCGGC
GCTTCAGCGATCGGTGCAACTGCGCAATCGCACCATTGTTCCTTTGGGCTTCCTGCAAGTATCTCTT
TTGAAACGGCTGCGCCAGCACAATAGCCAAACCACCTCTGGGGCAATTTTGCGCTCCCGCTATGGTC
GGGGTGAATTGCTACGGGGGGCACTCTTGACCATCAATGGCATAGCAGCGGGGATGCGCAATACAGG
CTAAGCAACGGCGAGGGTGAATCATGGACCCGACGACCCGC

SEQ ID NO: 10, protein - Thermosynechococcus vulcanus
MTSVLDVTNRDRLIESESLAARTLQERLRLVEEVLVDVLAAESGQELVDLLRRLGALSSPEGHVLHA
PEGELLKVIESLELNEAIRAARAFNLYFQIINIVEQHYEQQYNRERAAQEGLRRRSVMSEPISGVSG
EGFPLPHTAANATDVRSGPSERLEHSLYEAIPATQQYGSFAWLFPRLQMLNVPPRHIQKLLDQLDIK
LVFTAHPTEIVRQTIRDKQRRVARLLEQLDVLEGASPHLTDWNAQTLRAQLMEEIRLWWRTDELHQF
KPEVLDEVEYTLHYFKEVIFAVIPKLYRRLEQSLHETFPALQPPRHRFCRFGSWVGGDRDGNPYVKP
EVTWQTACYQRNLVLEEYIKSVERLINLLSLSLHWCDVLPDLLDSLEQDQRQLPSIYEQYAVRYRQE
PYRLKLAYVLKRLQNTRDRNRALQTYCIRRNEAEELNNGQFYRHGEEFLAELLLIQRNLKETGLACR
ELDDLICQVEVFGFNLAALDIRQESTCHAEALNEITAYLGILPCPYTELSEAERTRWLLSELSTRRP
LIPGELPFSDRTNEIIETFRMVRQLQQEFGTDLCNTYIISMSHEVSDLLEVLLFAKEAGLFDPATGA
STLQAIPLFETVEDLKHAPAVLTQLFSLPFCRSYLGSNSTPFLQEVMLGYSDSNKDSGFLSSNWEIY
KAQQQLQKIAESFGFQLRIFHGRGGSVGRGGGPAYAAILAQPAQTIKGRIKITEQGEVLASKYSLPE
LALFNLETVATAVIQASLLRSSIDEIEPWHEIMEELATRSRQCYRHLIYEQPEFI

**FIGURE 4 (continued)**

EFFNEVTPIQEISQLQISSRPTRRGGKKTLESLRAIPWVFSWTQTRFLLPAWYGVGTALKEFLEEKP
AEHLSLLRYFYYKWPFFRMVISKVEMTLAKVDLEIARYYVQELSQPQNREAFCRLYDQIAQEYRLTT
ELVLTITGHERLLDGDPALQRSVQLRNRTIVPLGFLQVSLLKRLRQHNSQTTSGAILRSRYGRGELL
RGALLTINGIAAGMRNTG

SEQ ID NO: 11, DNA - Streptomyces coelicolor
GTGAGCAGTGCCGACGACCAGACCACCACGACGACCAGCAGTGAACTGCGCGCCGACATCCGCCGGC
TGGGTGATCTCCTCGGGGAGACCCTGGTCCGGCAGGAGGGCCCCGAACTGCTGGAACTCGTCGAGAA
GGTACGCCGACTCACCCGAGAGGACGGCGAGGCCGCCGCCGAACTGCTGCGCGGCACCGAACTGGAG
ACCGCCGCCAAGCTCGTCCGCGCCTTCTCCACCTACTTCCACCTGGCCAACGTCACCGAGCAGGTCC
ACCGCGGCCGCGAGCTGGGCGCCAAGCGCGCCGCCGAGGGCGGACTGCTCGCCCGTACGGCCGACCG
GCTGAAGGACGCCGACCCCGAGCACCTGCGCGAGACGGTCCGCAACCTCAACGTGCGCCCCGTGTTC
ACCGCGCACCCCACCGAGGCCGCCCGCCGCTCCGTCCTCAACAAGCTGCGCCGCATCGCCGCCCTCC
TGGACACCCCGGTCAACGAGTCGGACCGGCGCCGCCTGGACACCCGCCTCGCCGAGAACATCGACCT
CGTCTGGCAGACCGACGAGCTGCGCGTCGTGCGCCCCGAGCCCGCCGACGAGGCCCGCAACGCCATC
TACTACCTCGACGAGCTGCACCTGGGCGCCGTCGGCGACGTCCTCGAAGACCTCACCGCCGAGCTGG
AGCGGGCCGGCGTCAAGCTCCCCGACGACACCCGCCCCCTCACCTTCGGCACCTGGATCGGCGGCGA
CCGCGACGGCAACCCCAACGTCACCCCCCAGGTGACCTGGGACGTCCTCATCCTCCAGCACGAGCAC
GGCATCAACGACGCCCTGGAGATGATCGACGAGCTGCGCGGCTTCCTCTCTAACTCCATCCGGTACG
CCGGTGCGACCGAGGAACTGCTCGCCTCGCTCCAGGCCGACCTGGAACGCCTCCCCGAGATCAGCCC
CCGCTACAAGCGCCTCAACGCCGAGGAGCCCTACCGGCTCAAGGCCACCTGCATCCGCCAGAAGCTG
GAGAACACCAAGCAGCGCCTCGCCAAGGGCACCCCCCACGAGGACGGCCGCGACTACCTCGGCACCG
CCCAGCTCATCGACGACCTGCGCATCGTCCAGACCTCGCTGCGCGAACACCGCGGCGGCCTGTTCGC
CGACGGGCGCCTCGCCCGCACCATCCGCACCCTGGCCGCCTTCGGCCTCCAGCTCGCCACCATGGAC
GTCCGCGAGCACGCCGACGCCCACCACCACGCCCTCGGCCAGCTCTTCGACCGGCTCGGCGAGGAGT
CCTGGCGCTACGCCGACATGCCGCGCGAGTACCGCACCAAGCTCCTCGCCAAGGAACTGCGCTCCCG
CAGGCCGCTGGCCCCCAGCCCCGCCCCCGTCGACGCGCCCGGCGAGAAGACCCTCGGCGTCTTCCAG
ACCGTCCGCCGCGCCCTGGAGGTCTTCGGCCCCGAGGTCATCGAGTCCTACATCATCTCCATGTGCC
AGGGCGCCGACGACGTCTTCGCCGCGGCGGTACTGGCCCGCGAGGCCGGGCTGATCGACCTGCACGC
CGGCTGGGCGAAGATCGGCATCGTGCCGCTGCTGGAGACCACCGACGAGCTGAAGGCCGCCGACACC
ATCCTGGAGGACCTGCTCGCCGACCCCTCCTACCGGCGCCTGGTCGCGCTGCGCGGCGACGTCCAGG
AGGTCATGCTCGGCTACTCCGACTCCTCCAAGTTCGGCGGCATCACCACCAGCCAGTGGGAGATCCA
CCGCGCCCAGCGCCGGCTGCGCGACGTCGCCCACCGCTACGGCGTACGGCTGCGCCTCTTCCACGGC
CGCGGCGGCACCGTCGGCCGCGGCGGCGGCCCCACCCACGACGCCATCCTCGCCCAGCCCTGGGGCA
CCCTGGAGGGCGAGATCAAGGTCACCGAGCAGGGCGAGGTCATCTCCGACAAGTACCTCATCCCCGC
CCTCGCCCGGGAGAACCTGGAGCTGACCGTCGCGGCCACCCTCCAGGCCTCCGCCCTGCACACCGCG
CCCCGCCAGTCCGACGAGGCCCTGGCCCGCTGGGACGCCGCGATGGACGTCGTCTCCGACGCCGCCC
ACACCGCCTACCGGCACCTGGTCGAGGACCCCGACCTGCCGACCTACTTCCTGGCCTCCACCCCGGT
CGACCAGCTCGCCGACCTGCACCTGGGCTCGCGGCCCTCCCGCCGCCCCGGCTCGGGCGTCTCGCTC
GACGGACTGCGCGCCATCCCGTGGGTGTTCGGCTGGACCCAGTCCCGGCAGATCGTCCCCGGCTGGT
ACGGCGTCGGCTCCGGCCTCAAGGCCCTGCGCGAGGCGGGCCTGGACACCGTGCTCGACGAGATGCA
CCAGCAGTGGCACTTCTTCCGCAACTTCATCTCCAACGTCGAGATGACCCTCGCCAAGACCGACCTG
CGCATCGCCCAGCACTACGTCGACACCCTCGTCCCGGACGAGCTCAAGCACGTCTTCGACACCATCA
AGGCCGAGCACGAGCTCACCGTCGCCGAGGTCCTGCGCGTCACCGGCGAGAGTGAACTGCTGGACGC
CGACCCGGTCCTCAAGCAGACCTTCACCATCCGCGACGCCTACCTCGACCCCATCTCCTACCTCCAG
GTCGCCCTCCTCGGCCGTCAGCGCGAGGCCGCCGCCGCGAACGAGGACCCGGACCCCCTCCTCGCCC
GAGCCCTCCTCCTCACCGTCAACGGCGTGGCAGCGGGCCTGCGCAACACCGGCTGA

**FIGURE 4 (continued)**

SEQ ID NO: 12, protein - Streptomyces coelicolor

MSSADDQTTTTTSSELRADIRRLGDLLGETLVRQEGPELLELVEKVRRLTREDGEAAAELLRGTELE
TAAKLVRAFSTYFHLANVTEQVHRGRELGAKRAAEGGLLARTADRLKDADPEHLRETVRNLNVRPVF
TAHPTEAARRSVLNKLRRIAALLDTPVNESDRRRLDTRLAENIDLVWQTDELRVVRPEPADEARNAI
YYLDELHLGAVGDVLEDLTAELERAGVKLPDDTRPLTFGTWIGGDRDGNPNVTPQVTWDVLILQHEH
GINDALEMIDELRGFLSNSIRYAGATEELLASLQADLERLPEISPRYKRLNAEEPYRLKATCIRQKL
ENTKQRLAKGTPHEDGRDYLGTAQLIDDLRIVQTSLREHRGGLFADGRLARTIRTLAAFGLQLATMD
VREHADAHHHALGQLFDRLGEESWRYADMPREYRTKLLAKELRSRRPLAPSPAPVDAPGEKTLGVFQ
TVRRALEVFGPEVIESYIISMCQGADDVFAAVLAREAGLIDLHAGWAKIGIVPLLETTDELKAADT
ILEDLLADPSYRRLVALRGDVQEVMLGYSDSSKFGGITTSQWEIHRAQRRLRDVAHRYGVRLRLFHG
RGGTVGRGGGPTHDAILAQPWGTLEGEIKVTEQGEVISDKYLIPALARENLELTVAATLQASALHTA
PRQSDEALARWDAAMDVVSDAAHTAYRHLVEDPDLPTYFLASTPVDQLADLHLGSRPSRRPGSGVSL
DGLRAIPWVFGWTQSRQIVPGWYGVGSGLKALREAGLDTVLDEMHQQWHFFRNFISNVEMTLAKTDL
RIAQHYVDTLVPDELKHVFDTIKAEHELTVAEVLRVTGESELLDADPVLKQTFTIRDAYLDPISYLQ
VALLGRQREAAAANEDPDPLLARALLLTVNGVAAGLRNTG

SEQ ID NO: 13, DNA - Rhodopseudomonas palustris

CTGCAGGTGACGCGCCAGCTCAGCGGCCTTGCCGCCGCGCTCGGCGTGCTGACCCTGCTGGCGCTCG
GCACCTTCACGGCCGGTTTGCACATCCGCGCCTGGAAGATCGCGGTGGTCGGCGTCATCCTTGGCGT
CAGCGTCCCGGCGATCGCCTTCCTGCAGCAATCGGCGCTGCTGATCCTGCTGGTGATCGGCCTGATC
ATCGCCATCGTGGTGCCGTTCGTTTGGGCCAAGCGCCGGCCCGCGCCGGCTTCGGAGCCGTTATTCC
AGCCGCCGTTCACCCGCACCACCACGCCGCCGACCTCGGCCAGCTCGCGGCCGGTGCCGCAGCCGCC
GCTGTACGAAACGCCGGTCGCGGCCCCGATGCCGGTCGCGCCGCCGCCGGCCCCGACGCCCAGCGCT
GCCGAGCCGGCCTCGCCGCCGCCGCCGGCGGACAGCAACACGCCGCCGGACAACGTCCGCAACATCT
CGGGCGCGCGCTAACTACGCTTCGACCGGCCCGCGCGACGTGCACCGCCGATTCTTGATCTTGCGTG
CGGTGCCAAAATCCTTCAGGATACCGATCTGATCCTGGAGTGCTGCGATGTCGTCGTTGAACCTGTC
CGCCGGTCCTGAGCCGGTTTCCGAACGTCCTGACGACGCGGCCGCGATCGAGGCCGAGACCCGGCTG
CGCAACGACATCCGGCTGCTCGGCCGGATCCTCGGCGACACTGTGCGCGAGCAGGAAGGACAGACCG
TGTTCGATCTGGTCGAGAACATCCGCCAGACCTCGATCCGATTCCACCGCGACGACGACAAGACCGC
ACGCGCCGAGCTCGAAGCCATCCTGGACGGGATGTCGATCCCCGACACGATGCGGATCGTTCGCGCC
TTCAGCTATTTCTCACACCTCGCCAACATCGCCGAAGACCAGAACAACATCCGCCAGATGCGCGCCG
GCTCGACCGCGGGCTCGGCGCCGCGTGCCGGACTGCTCGCCAAGACGCTGGCGCACGCGCGGCAGGA
AGGCATCAGCGCCGCGGAGCTGCGCAAGTTCTTTGCGACCGCGCTGGTGAGCCCGGTGCTGACCGCG
CATCCGACCGAAGTGCGCCGCAAGAGCACGATGGACCGCGAAATGCAGATCGCTTCGCTGCTCGATC
AGCGCGACCGCGTTCAGCTCACCGCCGACGAATGGGCCGACAACGAGGAGCGGCTGCGCCGCGCCGT
CGAGACGCTGTGGAAGACCAACCTGCTGCGCCGGACAAAGCTGACGGTGCTGGATGAAGTCACCAAC
GGCCTGTCGTTCTACGACTACACCTTCCTGCGCGAGGTGCCGCGGCTGCACAGTGCGCTGGAAGACC
GGCTTGCCGATGCGGCCAAGGCCGAAGGCGTCAACAGCGACGGCGAGCTGGCGAGCTTCCTGCGGAT
GGGAAGCTGGATCGGCGGCGATCGCGATGGCAATCCGTTCGTCACTGCCGAGGTGCTGCACGGCACC
TTGAAGCTGCAGAGCACGCGCGTGCTGCGCTATTATCTCGACGAGCTGCACGAGCTTGGCTCGGAGT
TGTCGCTGGCGTCGCATCTCGCCGGCACCACCGACACCGTCAAGGCGCTGGCCGAAACCTCGCCCGA
CACCTCACCGCATCGCAAATACGAGCCGTATCGCCTCGCGGTGTCCGGCATCTACGCGCGGCTGGCA
GCGACCGCGCTCAAGCTCGAGGTCGAGAACCTCCGCGCGCCGGTCGGCGAGGCCGAGCCTTACGCCA
GCGCGCAGGACTTCAAAGCCGATCTCGACGCGATCCATCTGTCGCTCACCCAGCACAATTCCGGTGT
GATCGCGCGCGGCCGGCTGCGCCAGCTCCGCCGCGCGATCGACTGCTTTGGCTTCCATCTCGCCAGC
CTCGACATGCGGCAGAACTCGGCGGTGCACGAGCGCACCATTACCGAGCTGATGGACGCGGCGCGGC
CCGGCACCTCCTATGCGATGCTCGACGAGGAAGCGCGGATCGCGCTCTTGATCAGCGAGCTGCGCAG
CACCCGGCCGCTGACCTCGATGTTCGTCAAATACAG

**FIGURE 4 (continued)**

CGACGAGACGGTCGGCGAGCTTGCAGTGTTCCGCGAAGCCGCGAAGGCGCACGCGACCTACGGGGCG
GCGGCGATCCCCCAATGCATCATCTCGATGACCAAGGGCGTTTCCGACCTCTTGGAGGTCGCGGTGC
TGCTCAAGGAAGTCGGGCTGATCGATCCGTCGGGGCGCAGCGCCATCAACGTGGTGCCGCTGTTCGA
GACCATCGAGGACCTGCAGGCCTGCGCCAAGATCATGGACCGGCTGCTGTCGATCCCGGAATATCGC
CGGCTGGTCGACAGCCGCGGCTCGGTGCAGGAGGTGATGCTCGGCTACTCCGACAGCAATAAGGACG
GCGGCTTCGTCACCTCGGGCTGGGAGCTGTACAAGGCCGAGATCGGACTGATCGAGATCTTCGAGCA
CCATGGCGTGCGGTTGCGGCTGTTCCACGGCCGCGGCGGCTCGGTCGGCCGCGGCGGCGGCCCGAGC
TACGACGCCATCGTGGCGCAGCCGGGTGGGGCGGTGAACGGCCAGATCCGCATCACCGAGCAGGGCG
AGATCATCACCAGGAAATATTCCAACGTCGAAGTCGGCCGCAACAACCTCGAGATCCTCGCCGCCGC
GACGTTGGAAGCAAGCCTGCTGCAGCCGAAGCGGGTGGCGCCGCATCGCGATTATCTCGAGGCGATG
GAGCAGCTCTCCGCGCTCGCCTTCAAGGCGTATCGCGGCCTGGTGTACGAGACCGACGGATTCGTCG
ACTACTTCTGGGCCTCGACGGTGATCAACGAGATCTCGACGCTGAACATCGGCAGCCGTCCGGCCTC
GCGCAAGAAAACGCGCGCGATCGAGGACCTGCGCGCGATCCCCTGGGTATTCTCGTGGGCGCAGTGC
CGGCTGATGCTGCCGGGCTGGTACGGTTTCGGCAGCGCGGTGTCGGCCTGGGTCACCGCGCATCCCG
AGACGGGCATCGCCTTCCTGCAAAAGATGTATCAGGAGTGGCCGTTCTTTCGCACGCTGCTGTCGAA
CATGGACATGGTGCTGTCGAAGAGCTCGATCGGCATCGCCTCGCGATATGCCGAGCTGGTGGAGGAC
GTCGATGTTCGCGAGCGCATCTTCGGCCGCATCCGCGCCGAATGGCATTCCTCGATCGAGTATCTGT
TCGCGATCATGCAGCAGGACCGCCTGCTGCAGAGCAACCCGCTGCTCGAACGCTCGATCCGCCACCG
CTTCCCGTATCTCGACCCCCTGAACCACGTCCAGGTCCAGCTGCTGCGCGAACACCGCACCCACGAC
CCAGACGAACAGGTGCTCCGCGGCGTGCAACTGACGATCAACGGGATTTCGGCGGGGCTGCGGAATA
GCGGGTGAGGGCGGGCCGCCGCTGGGATTGGATTGGGCTGATTTAGCCGGTGGAAAGGCGCGCCTGT
TAGATTGGCGGCGATGCTGAAGCGGGCG

SEQ ID NO: 14, protein - Rhodopseudomonas palustris
MSSLNLSAGPEPVSERPDDAAAIEAETRLRNDIRLLGRILGDTVREQEGQTVFDLVENIRQTSIRFH
RDDDKTARAELEAILDGMSIPDTMRIVRAFSYFSHLANIAEDQNNIRQMRAGSTAGSAPRAGLLAKT
LAHARQEGISAAELRKFFATALVSPVLTAHPTEVRRKSTMDREMQIASLLDQRDRVQLTADEWADNE
ERLRRAVETLWKTNLLRRTKLTVLDEVTNGLSFYDYTFLREVPRLHSALEDRLADAAKAEGVNSDGE
LASFLRMGSWIGGDRDGNPFVTAEVLHGTLKLQSTRVLRYYLDELHELGSELSLASHLAGTTDTVKA
LAETSPDTSPHRKYEPYRLAVSGIYARLAATALKLEVENLRAPVGEAEPYASAQDFKADLDAIHLSL
TQHNSGVIARGRLRQLRRAIDCFGFHLASLDMRQNSAVHERTITELMDAARPGTSYAMLDEEARIAL
LISELRSTRPLTSMFVKYSDETVGELAVFREAAKAHATYGAAAIPQCIISMTKGVSDLLEVAVLLKE
VGLIDPSGRSAINVVPLFETIEDLQACAKIMDRLLSIPEYRRLVDSRGSVQEVMLGYSDSNKDGGFV
TSGWELYKAEIGLIEIFEHHGVRLRLFHGRGGSVGRGGGPSYDAIVAQPGGAVNGQIRITEQGEIIT
RKYSNVEVGRNNLEILAAATLEASLLQPKRVAPHRDYLEAMEQLSALAFKAYRGLVYETDGFVDYFW
ASTVINEISTLNIGSRPASRKKTRAIEDLRAIPWVFSWAQCRLMLPGWYGFGSAVSAWVTAHPETGI
AFLQKMYQEWPFFRTLLSNMDMVLSKSSIGIASRYAELVEDVDVRERIFGRIRAEWHSSIEYLFAIM
QQDRLLQSNPLLERSIRHRFPYLDPLNHVQVQLLREHRTHDPDEQVLRGVQLTINGISAGLRNSG

SEQ ID NO: 15, DNA - Welwitschia mirabilis
AGCATCGACGCGCAACTGCGGCTTTTGGTCCCAACGAAGGTTTCCGAAGATGATAAGCTTATTGAGT
ATGACGCTCTTCTGATGGACCGCTTTCTGGATATCTTGCAAGACCTACATGGAGAAGAAATTAGAGA
AACGGTTCAAGAGTGCTATGAGCTTTCAGGAGAATACGAAGGAAAGTTTGACACGGCAAAGTTGGAG
GAGCTTGGAGGAGTGTTAACAAGTCTAGATGCTGGAGACTCCATTGTTGTTGCAGCTCTTTCTCACA
TGCTTAACCTAGCGAACTTGGCTGAGGAGGTTCAGATAGCATACCGAAGACGAATTAAACACAAAAA
GAAAGGGGATTTCGCGGATGAGAATTCCGCAACGACGGAATCAGACATTGAAGAAACCTTTAAAAGG
CTTGTAAATCAGCTTGGAAGGTCTCCTCAAGAAGTTTTTGACGCTCTCAAAAATCAAACA

**FIGURE 4 (continued)**

ATTGATTTGGTCTTGACTGCACATCCAACGCATCAGTCGGTATCCCTGCTACAAAAGCATGGCAGGA
TCCGAAACTGCTTGTCTCAGTTGTATGCGAAAGACATTACGCCCGATGAAAAACAGGAGCTTGATGA
AGCTTTACAGAGGGAGATTCAAGCTGCCTTCAGAACTGATGAAATTCGGCGCACTCCTCCCACCCCT
CAAGATGAGATGCGAGCAGGAATGAGTTATTTCCATGAAACCATATGGAAAGGTGTCCCTAAGTTCT
TACGTCGTGTTGACACTGCTCTAAAGTCGATTGGTATAAATGAGAGACTTCCTTACAATGCCCCCCT
AATACAATTCTCGTCGTGGATGGGCGGCGACCGCGACGGGAATCCTAGAGTCACTCCAGAAGTCACG
CGAGACGTTTGTCTCCTTGCGAGGATGATGGCTACAAATTTATACTACTCTCAGATAGAGGACCTTA
TGTTTGAGTTGTCAATGTGGCGATGTAGCGATGAGCTTCGGCAACGTGCTCTGCAGCTTCATAATTC
TGCAAAGAGAGATGCAAAACATTACATAGAGTTTTGGAAGCAAGTACCTCCCAATGAGCCATTTAGG
GTAATTTTGGGAGATGTTAGGGACAAGTTATATAACACTCGGGAACGCACTCGCCAGTTGCTTGCCA
ATGGTTTCTCAGATGTGCCAGAAGAAGCACTTACGAGTGTAGATCAGTTGTTGGAACCGTTAGAGTT
GTGCTATCGATCTCTGTGCTCTACCGGCGATCAACCCATTGCAGATGGCAGTCTTCTTGATTTCATG
CGTCAAGTCTCAACCTTTGGCTTAACTCTTGTGAAGTTGGATATTAGACAAGAATCAGATCGACACA
CTGATGTTATCGACGCAATAACAAGACATCTAGGCCTTGGGTCCTATAAAGAGTGGCCAGAAGACAA
ACGACAGGAGTGGCTGTTGGCTGAACTACGTGGAAAACGTCCACTATTTGGACCCGACCTTCCAACT
ACAGATGAAATAAGAGAAGTTCTTGACACATTCCACGTGGTAGCTGAACTTCCTCCAGACAACTTTG
GAGCTTACATCATATCAATGGCTACTGCTCCTTCAGATGTTTTAGTCGTTGAGTTACTGCAGAGGGA
ATGCCGTGTGAAAAAGCCTCTGCGAGTTGTGCCTTTGTTTGAGAAGCTTGCTGATTTAGAGAATCGT
CCCGCGGCTTTGGCAAGGCTGTTCTCAATTGATTGGTATAGAAACAGAATTGATGGAAAGCAAGAAG
TAATGNNNGGTTACTCAGACTCCGGCAAGGATGCCGGAAGACTATCTGCAGCATGGCAATTATACAA
GGCTCAAGAGGAATTGATCAAGGTTGCAAAACAGTTTGGAATTAAGCTGACAATGTTTCATGGTCGT
GGAGGGACCGTTGGAAGAGGAGGTGGTCCAACCCATCTGGCTATATTATCTCAACCTCCAGACACAA
TTCACGGGTCATTGCGAGTTACCGTCCAAGGGGAGGTTATTGAGCAGTGTTTTGGGGAGGAACATCT
GTGTTTCCGAACTCTTCAGCGCTTTACTGCTGCCACATTGGAACATGGAATGCATCCTCCAATTGCA
CCTAAGCCCGAATGGCGTCAACTGATGGATGAAATGGCTGTTGTTGCTACTCAAGAGTATAGGTCTT
ATGTTTTCCACAACAAGAGATTTGTGGAGTATTTCCGATCCGCAACTCCCGAGTTGGAGTATGGACG
GATGAATATAGGCAGTCGTCCATCAAAAAGAAAGCCCAGTGGAGGCATTGAATCACTTCGTGCAATT
CCATGGATATTTGCTTGGACACAGACCAGATTCCATCTTCCTGTTTGGCTTGGATTTGGTGCTGCTT
TCAAGAGTGTTATCGAGAAGGACATTCGAAATCTTCACACGCTGCAACAAATGTATAACGAATGGCC
ATTTTTCCGTGTCACCATTGACCTAGTTGAAATGGTGTTTGCCAAGCACCCTGAAATTGCTGCATTA
TACGATAAACTGCTTGTATCATCAGTCTTGTGGGCTTTCGGGGAACAACTAAGAAAAAACTATGTAG
AGACTAAGACCCTTCTGCTGCAGGTTGCTGGACACAAAGAAGTCTTAGAAGGCGACCCATATCTGAA
ACAACGATTGAGACTTCGTGACTCATATATCACAACCTTGAATGCACTTCAGGCATATACTTTAAAA
CGGATACGAGATCCAAGCTACCATGTCACCCTCAGGCCTCATTTATCTAAAGAAAGCTCAACCAAGC
CAGCAGCAGAATTGGTAAAATTAAACCCAACCAGTGAGTATGCACCGGGATTAGAAGATACATTGAT
CTTAACCATGAAGGGCATTGCTGCC

SEQ ID NO: 16, protein - Welwitschia mirabilis
SIDAQLRLLVPTKVSEDDKLIEYDALLMDRFLDILQDLHGEEIRETVQECYELSGEYEGKFDTAKLE
ELGGVLTSLDAGDSIVVAALSHMLNLANLAEEVQIAYRRRIKHKKKGDFADENSATTESDIEETFKR
LVNQLGRSPQEVFDALKNQTIDLVLTAHPTHQSVSLLQKHGRIRNCLSQLYAKDITPDEKQELDEAL
QREIQAAFRTDEIRRTPPTPQDEMRAGMSYFHETIWKGVPKFLRRVDTALKSIGINERLPYNAPLIQ
FSSWMGGDRDGNPRVTPEVTRDVCLLARMMATNLYYSQIEDLMFELSMWRCSDELRQRALQLHNSAK
RDAKHYIEFWKQVPPNEPFRVILGDVRDKLYNTRERTRQLLANGFSDVPEEALTSVDQLLEPLELCY
RSLCSTGDQPIADGSLLDFMRQVSTFGLTLVKLDIRQESDRHTDVIDAITRHLGLGSYKEWPEDKRQ
EWLLAELRGKRPLFGPDLPTTDEIREVLDTFHVVAELPPDNFGAYIISMATAPSDVLVVELLQRECR
VKKPLRVVPLFEKLADLENRPAALARLFSIDWYRNRIDGKQEVMXGYSDSGKDAGRLSAAWQLYKAQ
EELIKVAKQFGIKLTMFHGRGGTVGRGGGPTHLAILSQPPDTIHGSLRVTVQGEVIE

## FIGURE 4 (continued)

QCFGEEHLCFRTLQRFTAATLEHGMHPPIAPKPEWRQLMDEMAVVATQEYRSYVFHNKRFVEYFRSA
TPELEYGRMNIGSRPSKRKPSGGIESLRAIPWIFAWTQTRFHLPVWLGFGAAFKSVIEKDIRNLHTL
QQMYNEWPFFRVTIDLVEMVFAKHPEIAALYDKLLVSSVLWAFGEQLRKNYVETKTLLLQVAGHKEV
LEGDPYLKQRLRLRDSYITTLNALQAYTLKRIRDPSYHVTLRPHLSKESSTKPAAELVKLNPTSEYA
PGLEDTLILTMKGIAA

SEQ ID NO: 17, DNA - Chlamydomonas reinhardtii
ATGACGGACTCCACATATGATTTTGGAGCCGTGAGGGACGATCTGACGCCGCTTGAGGATGACTGCA
AGCTTCTCGGTAGCCTGCTTGACGACTGTCTCCGCGTCGAAATCGGCGAGACCATGTTCAAGAAGAT
TGAACGCATCCGAGCACTGGCGCAATGTGCCTCAAATCTGTCGATCAAGGGAGACGCGGGCGCCTCC
GACATGCTGTCGCACCGGCTGGCGGAGGAGCTGATGAACCTGGACATGGACGAGGCGGTGCCGCTCA
CACGCGCCTGCGGCCACTACCTCAACCTGTCCGGCATCGCAGAGCTGCACCACGGAGTGCGCCGCGA
CCGCGCCACTCGCGAGCCCAACCCCAACAGCTGCGACGCGGTGTTTGCGCGGCTGATCACGGAGGGC
GTGGACCCCGAGGAGCTGTACCGGGCGGTGTCGGAGCAGAACGTGGAGGTGGTGCTCACCGCGCACC
CCACACAGGTGAACCGGCGCACGCTGCAGTACAAGCACACTCGCATTGCGGCGCTGCTGCAGCAGCA
CGACCGCTCCGACCTGACGGCGGAGGAGCGGCGCAACATGGTGAGCGAGCTGCAGCGGGAGGTGGCG
GCGCTGTGGCAGACGGACGAGCTGAGGAGGCAGAAGCCCACGCCGCTGGACGAGGCGCGCGGCGGTC
TGCACATTGTGGAGCAGTCGCTGTGGGCCGCGGTGCCGCAGTACATGCGCCGCCTCAGCGCCGCGCT
CAAGAAACACACCGGGCACGACCTGCCGCTGCAGGCCACGCCCTTCCGCTTCGGCAGCTGGATGGGC
GGCGACCGCGATGGCAACCCCAACGTGACCGCCAAGGTGACGGCGCACGTGACGGCCCTGGCGCGCT
GGATGGCGGCGGATCTGTACCTGCGTGAGATCGACACGCTGAGGTTTGAGCTGTCCATGAACCAGTG
CAGTGCGGCGGTGTGGAAGATGGCCCGCCGCATCATCGCCGAGGGCCACACCAAGCGCGCCGGCGTG
GTCCGGGCCAAGGCCGCCGCCGCCCTGCACCAGACCGCAACAGACGCAGCCAGCCATGGCGGCTCCG
CCGCCTCGGCGGCCGCCGCCGCCGCCGCAGGCGGCGACGTCGTCGCTGACGGCACCAGCGGCGGCGG
CGCTGCCGCCGCCGCCGGCCCCGCCGCCGCTGCCGCGGCGGACGACGCGTTCACCTTCAGCCGGTTG
GGGCGGCCCCGGCCGGAGCGGCCGAGCACGGACGTGCGGAGTGTGGGCGTGCTGGCGGGCGGCGAGG
GCGCGGCGTTCCCGGGCGGCATGATCCTGGGCACGCAGCCGGTGTCGGCGCACACGGCGGCGGAGGT
GTCGGTGCCGCACGAGCTGCCGGGACAGGACGTGGAGGGCGGCTCGGAGATGGACTTCAACGAGTCC
CGCCGCGCCTCGGACGCCGGAGACCTGGGCGCCTCGCAGCACCCCATGCTGGGCGGGCCCTCCGCCG
GCGCCTCCGCCGAGCCCACCGCCCACGGCTACACCACCACCGCCACCGCCGCCGCCGCCGCCGCCGA
CGGCACGCAGCCCGAGCCCGAAGTCCCCGGCACGCCCAGCTACGCCGACCCCGGCACCCCCGACCGC
CTTGGCGCGCTGCCCGGCCCCTTCACGCCCGGCCCCACGCCCTTCCGCGAGGCGGCCAACGCCGCCA
TGAGCACCGCCGCCAGCGGCGGCGCGGGCGGCGGCGGCGGCGGCGCGAACCGCGCGGCCTCGGG
CCTGGGCGGCGACCCCACCTTCACGCGTCGCAGCCTGATGGCGCAGCGGCTGGGAACCAGCTCGGTG
CAGTTCGCGCGCGCGCACGAGCACCCCGGCTTCCACCCCTACCGCATCGTGTTGGGCCACGTGCGCG
ACAGGCTGGCGGCCACGCGGCGGCGCATGGAGGACCTGCTGAGCGGCCGCGAGCCGGCGGGCGAGGC
GCACGGCGGCGTGGGGGCGGGCGGCGGCGGCGGCGGCGGCGCGGCGCCGTGGTATGAGAGCGAGGAC
GAGCTGGCGGAGCCGCTGATGGCGTGCTACTGGTCGCTGTGGGAGTGCGGCGGCGGCGTCATTGCGG
ACGGGCGGCTGCTGGACCTCATCCGCCGCGTGTACACCTTCGGCATGTGCCTCATGAAGCTAGACCT
GCGCCAGGAGTCCACCCGCCACGCCGAGGCCCTGGACGCCGTGACCAGCTACCTGGGTCTGGGCTCC
TACCTGGAGTGGAGCGAGGACCAGAAAATCGAGTGGCTCACAAAGGAGCTGCAGGGCCGGCGGCCGC
TCATCCCCGCCGACATGCCCATGAGCGCCGAGGTGCGCGAGGTGCTGGACACCTTCAAGGTGGCCGC
CCATCTGGGCCGTGACAACCTGGGCGCCTACGTCATCTCCATGACCAAGGGCGCCTCGGACGTGATG
GCGGTGGAGCTGCTGCAGCGCGAGGCGCGCATGCAGGTGGGCGCCGAGGCGGGCGGCCGCGGCGGCG
GCGGGCCCGAGGACGGCGGCTCGCTGCGCGTGGTGCCGCTGTTCGAGACGCTGGAGGACCTGGACGC
GGCGGAGGACGTGATGACGCGGCTGCTGACCAACCCCTGGTACCGCGAGCACCTGCGGGCAGTGCAC
GGCGACGCGCAGGAGGTGATGCTGGGATACAGCGACAGCGGCAAGGACGCCGGCCGCCTGGCTGCCA
ACTGGGCGCTGTACAAGTGTCAGGAG

## FIGURE 4 (continued)

CGGCTGGTGGCCATCACCAAGGCCAACAACGTCAAGCTCACGCTGTTCCACGGCCGCGGCGGCACCG
TGGGCCGCGGCGGCGGGCCCACCCACATCGCCATCCAGTCGCAGCCGCCCGGCTCTGTGGAGGGGAC
CTTCCGCATCACCGAGCAGGGCGAGATGGTCCAGGCCAAGTTCGGCATCAGCGGCGTGGCTCTGAGC
CAGCTGGAGACCTACACCACCGCTGTGCTGCTGGCCACCATGCGCCCGCCCAGCCCGCCGCGCCGCG
AGGAGTGGCGCGCCGTGATGGAGATGCTCAGCCGCGTCAGCTGCGAGAGCTACCGCAACATCGTGCA
CCACAGCCCGCTCTTCCTGCGCTACTTCAAGCACGCCACGCCCGAGGCCGAGCTGGGCAACCTCTAC
ATCGGCTCTCGCCCGGCTCGGCGGCGCAACAAGGACGCCTCCATCTCCACGCTGCGCGCCATCCCCT
GGATCTTCGCCTGGACGCAGAACCGCCTCATCCTGCCCTCCTGGCTGGGCATTGGCGCCGCCCTCAC
CGCGGCCATGACCCAGGGCCACCTGCCCACGCTGCAGGCCATGTACCGCGAGTGGCCCTTCTTCGGA
TCCACAGTGGATCTGATTGAGATGATCCTGGCCAAGACCGACCCGCGCATCGCGGCGCTGTACGAGG
AGGTGCTGGTCAACGACCCCGAGGAGAAGAAGCTGGGAGCCGAGCTGCGCGAGCGGCTGCAGCGCTG
CCAGGGCGCCATCCTCAAGGTGACCGGGCACGAGAACCTGCTGTCCAACAACCCCACATTGAGCAAG
CTCATCTCCATGCGCTCGCCCTTCGTGGACCCCATCAACATCCTGCAGGTGGAGGTGCTGCGGCGCC
TGCGCCAGGACCCCAACAACATGCGCCTGCGCGACGCGCTGCTCATCTCCATCAACGGCATCGCCGC
CGGCATGCGCAACACCGGCTAAGCAGCGGCGGCGCTGCTGCTGGTGATGGAGGTGGAGGTGGAGATG
GACGTGGTGCGCACCAGGCACAAGGCGGAGGCAGGTGCCAGTGGAGGTGGGGCGTCCCGGGCGGAGG
CATGAGCGGCGTCGACGGCTGGCGCCGCGGTATGATGTGCTGCGGCACGAGCGACGGCACGTGCGGT
CTAAAGCTGCTGGATGTGCTTGGCACGGCGCGCAAGAGGGGTGTGCTGGGGGCGGAGGTGGTGCTGA
AAATGTTTGGTTAGTTGGTTGGATGTTTGGGCTAGCTGGTGCGCTGGAGCGGGTAAGGCAAGTGGTG
GGTGGGGTATGCGGGTGCGCTGGGGTGTCCAGGGCGGCTGCGATCAATGCGGGTTGGTGGGCAACCC
CGTCCCGGCGCTGAATCGCTGCGTGGATCTGACCTTTGCGGAATTCCTGCCTGCCTTCCGCGCCTGG
CAATTTAGGAACTCGTTTGAGCGTTTGACGTTGTCCTAGGGCGGCATGACCGAGCATCGCGTGCATG
GCAGCGGGGCTGCGATCGCGCGTGGTGAGCCACAGACGTTGATAGTGATATTTGAGGCAGCCCAGAA
CGCATGGCGGAATGGCGTGGTCTTTGAGAGCTGAGTGTGTCAGACTGGCATAGGGCGCTGTGGCTGA
CTAACGTCCCGCACGTGTGCGACTGCAGACCATGTTTGCAGGGGCAGGTACTTTACTTGATGTGCAT
GGCATTCTCACATAAAGCTCCGAAGGATCATTTGTGGCGCACCAACCAGAGACCTAGCCCAGAGCAG
AGTGTCAGCAGCGATATCAAAGTCATTTTGTGGGTGGTTGTCTGGGTACTCCGAGGCGCGTACGTTG
AGCGCGTGCTCGCTGCATACCGCATACGGAATCGGATAAGTGAAAGGAGGTCACATGGCCAGGGGCT
GCAGATGCTGGTGAGGGCTGCCTGCCTAAGCGGCGTGTCCGGGACGGCAGGGAAGCACGCATGCCAT
TCGACGTGTGCGGTGCAAATGAGACCGTGGAGTCCTCGGGCAGGGCTTGACAGCACAAGCGTGGAGC
GCGCACACAGCGCACAGACTATGTTTTCCTTGTGCTCTTTAACACAGCGCACAGAAAAAA

SEQ ID NO: 18, protein - Chlamydomonas reinhardtii
MTDSTYDFGAVRDDLTPLEDDCKLLGSLLDDCLRVEIGETMFKKIERIRALAQCASNLSIKGDAGAS
DMLSHRLAEELMNLDMDEAVPLTRACGHYLNLSGIAELHHGVRRDRATREPNPNSCDAVFARLITEG
VDPEELYRAVSEQNVEVVLTAHPTQVNRRTLQYKHTRIAALLQQHDRSDLTAEERRNMVSELQREVA
ALWQTDELRRQKPTPLDEARGGLHIVEQSLWAAVPQYMRRLSAALKKHTGHDLPLQATPFRFGSWMG
GDRDGNPNVTAKVTAHVTALARWMAADLYLREIDTLRFELSMNQCSAAVWKMARRIIAEGHTKRAGV
VRAKAAAALHQTATDAASHGGSAASAAAAAAAGGDVVADGTSGGGAAAAAGPAAAAAADDAFTFSRL
GRPRPERPSTDVRSVGVLAGGEGAAFPGGMILGTQPVSAHTAAEVSVPHELPGQDVEGGSEMDFNES
RRASDAGDLGASQHPMLGGPSAGASAEPTAHGYTTTATAAAAAADGTQPEPEVPGTPSYADPGTPDR
LGALPGPFTPGPTPFREAANAAMSTAASGGAGGGGGGGANRAASGLGGDPTFTRRSLMAQRLGTSSV
QFARAHEHPGFHPYRIVLGHVRDRLAATRRRMEDLLSGREPAGEAHGGVGAGGGGGGGAAPWYESED
ELAEPLMACYWSLWECGGGVIADGRLLDLIRRVYTFGMCLMKLDLRQESTRHAEALDAVTSYLGLGS
YLEWSEDQKIEWLTKELQGRRPLIPADMPMSAEVREVLDTFKVAAHLGRDNLGAYVISMTKGASDVM
AVELLQREARMQVGAEAGGRGGGGPEDGGSLRVVPLFETLEDLDAAEDVMTRLLTNPWYREHLRAVH
GDAQEVMLGYSDSGKDAGRLAANWALYKCQERLVAITKANNVKLTLFHGRGGT

**FIGURE 4 (continued)**

VGRGGGPTHIAIQSQPPGSVEGTFRITEQGEMVQAKFGISGVALSQLETYTTAVLLATMRPPSPPRR
EEWRAVMEMLSRVSCESYRNIVHHSPLFLRYFKHATPEAELGNLYIGSRPARRRNKDASISTLRAIP
WIFAWTQNRLILPSWLGIGAALTAAMTQGHLPTLQAMYREWPFFGSTVDLIEMILAKTDPRIAALYE
EVLVNDPEEKKLGAELRERLQRCQGAILKVTGHENLLSNNPTLSKLISMRSPFVDPINILQVEVLRR
LRQDPNNMRLRDALLISINGIAAGMRNTG

SEQ ID NO: 19, DNA - Glycine max
ATGACTGACATCACTGGTGATATTGCTGAGGAAATCTCCTTCCAGAGCTTCGATGATGACTGCAGGT
TGCTTGGTAATCTCCTCAATGACATTCTCCAGCGTGAAGTTGGCACCAACTTGCTTGACAAGATCGA
AAGGACTCGAGTCCTTGCTCAGAGTGGTTGTAATATGAGGCAGGCGGGTATTGTAAACATGGCAGAG
ATGCTTGAGAAGCAGTTGGCTTCGGAGTTATCAAAGATGACACTAGAAGAAGCTTTCACCCTTGCTC
GTGCCTTCAGCCATTATCTTACTTTGATGGGTATAGCTGAGACCCACCATAGGGTTCGTAAAGGAGG
GAATATGGCACAAATTGCAAAATCTTGCGATGATATATTTAACCAGCTGGTGCAGGGTGGAGTCCCC
CCAGAAGAACTTTATGACACAGTCTGCAAGCGGGAGGTTGAAATTGTTCTCACTGCTCATCCCACAC
AGATTAACCGTCGAACCTTACAGTTTAAACACATTAGAATTGCTCATCTTTTGGATTACAATGATCG
ACCTGATCTTAGCACTGAAGATCGAGAAATGGTGATTGAAGATCTGGTGAGAGAGATAACTTCAATA
TGGCAGACAGATGAGCTTAGGCGCCAGAAACCCACTCCAGTTGATGAAGCTAGAGCTGGTTTCAATA
TTGTGGAGCAGTCACTCTGGAAAGCTGTCCCTCATTATTTACGTCGTGTCAGCAATGCATTAAAGAA
GCATACAGGAAAGCCACTTCCTTTGACTTGCACTCCCATAAAGTTTGGATCTTGGATGGGAGGTGAT
AGAGATGGAAACCCAAATGTGACAGCAAAGGTCACAAAAGATGTTTCACTTCTATCTAGATGGATGG
CGATTGACCTCTATATTCGGGAAGTGGATAGCCTCAGATTTGAGCTATCCATGAACCAGTGCAGTGA
TAGGTTGTCAAGATTGGCACATGAAATTCTAGAAGCCAAGCATGAGAATCGCCGTGAGAATTGGAAT
CAGTCTGCGAATAGAAGTCTCACACTTCCAACACAACTTCCAGCTAGAGCTCATTTACCTTCTATTG
CTGAAAATGGTGAATCTCGGCATCCCAGACTAGACATTCCAGCACCTGATTACATGCAATCCAATCA
CAAGGATGGTGGGGTTTCTGTAAGTTCAACTACATCAAAACTTGCCAATCCCAATACTCGATTACCA
GGAACAAGTTCAGCAAATTCCAGTGCTTCTTCAGCTGCACTTGGTCAAAAGAAATTGTATGCAGAAT
CCCAGACAGGAAAGTCCACTTTTCAAAAGCTTTTGGAGCCAATGCTTCCTCAACTTCCTGGAATTGC
TCCTTATAGAATTGTCCTGGGGAATGTTAAGGATAAGCTTGAGAAAAGTCGTAGACGGTTAGAAATT
CTTCTTGAGGATGTTGCATGTGACTATGATCCTTTGGATTACTATGAAACATCTGATCAGCTTTTGG
AACCTCTGCTCCTCTGTTATGAATCTCTGCAATCGTGTGGATCTGGGGTGCTAGCTGATGGTCGACT
TGCTGATCTGATTCGTAGAGTTGCTACCTTTGGAATGGTGTTAATGAAGCTTGACTTGCGTCAGGAA
TCTGGGAGACATGCAGAAGCACTTGATGCAATAACACAGTACTTAGATATGGGTACTTACAGTGAAT
GGGATGAAGAAAAGAAGTTGGACTTCTTAACAAGAGAACTTAAAGGGAAGAGGCCTCTTGTTCCTGT
TAGTATAGAGGTTCATCCTGATGTTAAAGAAGTCTTGGATACATTCCGAATTGCCGCTGAACTGGGG
AGTGATTCACTTGGAGCTTATGTGATCTCTATGGCCTCAAATGCAAGTGATGTCCTTGCAGTAGAGC
TTTTACAGAAGGATGCACGGCTTGCTGCTATTGGGGAGTTGGGAAAAGCATGTCCTGGTGGAACGTT
GCGGGTTGTCCCTCTGTTTGAAACTGTGAAAGACCTGAGAGGAGCTGGTTCAGTTATCCGGAAACTT
TTATCAATAGACTGGTACCATGAACACATCGTTAAGAACCATAATGGACATCAAGAGGTTATGGTTG
GATATTCTGATTCTGGTAAAGATGCTGGTCGCTTTACTGCTGCTTGGGAACTTTACAAAGCTCAGGA
GGATGTTGTAGCTGCTTGCAATGATTATGGAATAAAGGTTACTCTATTCCATGGTCGTGGAGGCAGT
ATTGGTCGTGGTGGTGGCCCAACATATCTGGCTATTCAGTCCCAACCCCCTGGCTCTGTGATGGGAA
CGCTTCGGTCTACTGAGCAGGGAGAGATGGTAGAGGCTAAGTTTGGGTTGCCACAGATAGCTGTTAG
ACAACTTGAGATATACACAACAGCTGTACTACTTGCAACCCTTCGTCCACCTATCCCACCCCGAGAA
GAAAAATGGCGTAATGTCATGGAAGAGATCTCAAACATCAGTTGTCAGTGTGACCGCAATGTAGTGT
ATGAAAATCCAGAATTCCTGGCCTACTTCCATGAAGCCACACCAGAGGCAGAACTTGGCTTCCTTAA
CATAGGTAGCCGCCCTACAAGAAGGAAGAGCTCAGTAGGAATCGGACACCTTCGTGCAATTCCCTGG
TTATTTGCATGGACACAAACAAGATTCGTTCTTCCAGCTTGGCTTGGAGTCGGAGCAGGTTTAAAAG
GAGCTTGCGAGAAAGGTTACACCGAA

**FIGURE 4 (continued)**

GAGCTAAAAGCCATGTACAAAGAATGGCCCTTCTTTCAAAGTACCATAGATCTTATTGAGATGGTTT
TGGGGAAAGCTGACATTCCTATAGCCAAGCACTATGATGAAGTCCTTGTGACAAAGGAGAGGCAAGA
GCTTGGCCATGAACTAAGAAGTGAGCTCATGACAGCTGAAAAGTTTGTCATGGTTATTAGTGGGCAC
GAGAAACTTCAGCAGAATAATAGGAGCTTGAGGAGGCTAATTGAGAATAGACTTCCCTTCCTTAATC
CCTTGAACATGTTGCAGGTGGAGATACTCAAGAGGTTAAGACGTGATGATGACAACCGTAAGATCAG
AGATGCTTTGCTTATCACCATAAATGGGATTGCTGCAGGGATGAAGAATACAGGTTGA

SEQ ID NO: 20, protein - Glycine max
MTDITGDIAEEISFQSFDDDCRLLGNLLNDILQREVGTNLLDKIERTRVLAQSGCNMRQAGIVNMAE
MLEKQLASELSKMTLEEAFTLARAFSHYLTLMGIAETHHRVRKGGNMAQIAKSCDDIFNQLVQGGVP
PEELYDTVCKREVEIVLTAHPTQINRRTLQFKHIRIAHLLDYNDRPDLSTEDREMVIEDLVREITSI
WQTDELRRQKPTPVDEARAGFNIVEQSLWKAVPHYLRRVSNALKKHTGKPLPLTCTPIKFGSWMGGD
RDGNPNVTAKVTKDVSLLSRWMAIDLYIREVDSLRFELSMNQCSDRLSRLAHEILEAKHENRRENWN
QSANRSLTLPTQLPARAHLPSIAENGESRHPRLDIPAPDYMQSNHKDGGVSVSSTTSKLANPNTRLP
GTSSANSSASSAALGQKKLYAESQTGKSTFQKLLEPMLPQLPGIAPYRIVLGNVKDKLEKSRRRLEI
LLEDVACDYDPLDYYETSDQLLEPLLLCYESLQSCGSGVLADGRLADLIRRVATFGMVLMKLDLRQE
SGRHAEALDAITQYLDMGTYSEWDEEKKLDFLTRELKGKRPLVPVSIEVHPDVKEVLDTFRIAAELG
SDSLGAYVISMASNASDVLAVELLQKDARLAAIGELGKACPGGTLRVVPLFETVKDLRGAGSVIRKL
LSIDWYHEHIVKNHNGHQEVMVGYSDSGKDAGRFTAAWELYKAQEDVVAACNDYGIKVTLFHGRGGS
IGRGGGPTYLAIQSQPPGSVMGTLRSTEQGEMVEAKFGLPQIAVRQLEIYTTAVLLATLRPPIPPRE
EKWRNVMEEISNISCQCDRNVVYENPEFLAYFHEATPEAELGFLNIGSRPTRRKSSVGIGHLRAIPW
LFAWTQTRFVLPAWLGVGAGLKGACEKGYTEELKAMYKEWPFFQSTIDLIEMVLGKADIPIAKHYDE
VLVTKERQELGHELRSELMTAEKFVMVISGHEKLQQNNRSLRRLIENRLPFLNPLNMLQVEILKRLR
RDDDNRKIRDALLITINGIAAGMKNTG

SEQ ID NO: 21, DNA - Ricinus communis
ATGACGGACACCACAGATGATATTGCAGAGGAAATCTCATTTCAGAGTTTTGATGATGATTGTAAGC
TTCTTGGCAATTTGTTAAACGATGTTTTGCAGAGAGAAGTTGGTTCTAAATTCATGGAGAAGCTTGA
ACGCAATCGCATCCTAGCTCAGAGTGCTTGTAATATGAGATTGGCGGGGATAGAAGATACCGCTGAA
TTGCTGGAGAAGCAGCTGGCATTGGAGATATCAAGGATGACATTAGAGGAAGCATTGACACTTGCTC
GTGCCTTTAGTCATTACCTTAATTTGATGGGCATTGCTGAGACCCATCACAGGGTTCGTAAGGCACG
GAGTATGACACATCTATCAAAATCTTGTGATGACATATTTAATCAGCTGCTGCAGAGTGGCATATCT
GCAGAGGAGCTTTATGACACAGTTTGCAAGCAGGAGGTTGAAATTGTGCTCACTGCACATCCTACTC
AAATTAATCGTCGTACCTTGCAATATAAGCACATCAGAATTGCTCATCTTTTAGATTATAATGACCG
GCCTGATCTTACTCATGAAGATCGAGAAATGCTGATTGAAGATCTGGTGAGAGAAATTACTTCAATA
TGGCAGACAGATGAGCTTAGGCGCCACAAGCCTACACCAGTAGATGAAGCTAGGGCTGGCTTGAACA
TTGTGGAGCAGTCCCTGTGGAAAGCTCTTCCCCATTATTTACGTCGTGTCAGTACTGCCCTAAAGAA
GCATACAGGAAAGCCACTTCCTTTGACTTGCACGCCAATAAGGTTTGGGTCTTGGATGGGGGGTGAT
AGAGATGGTAATCCGAATGTAACAGCAAAGGTCACAAGAGATGTTTCTCTTTTATCTAGGTGGATGG
CTGTTGACCTTTACATTCGAGAAGTTGATAGCCTGAGATTTGAACTATCCATGGTTCAATGCAGTGA
TAGATTGTTGAAAGTGGCAAATGACATTTTAATAGAAGAAACTTCATCTGAAGATCACCATGAGAGT
TGGAATCAACCTGCAAGCAGAAGTCAAACAAAGTTTCCTAGAAAATCTCTTCCTACACAACTTCCAC
CTAGAGCTGATCTTCCTGCTTGCACTGAATGTAATGATGGTGAATCTCAGTATCCCAAACTAGAACT
TCCAGGAACTGATTACATGCCCTTTAATCGCCAGGAAGCTCTAGGTTCTTCATATTCAGAATCTTCA
TCCCAGGATATCAATCATGGTTTACCTAAAACAACTGGAAATGGAAGTGTTGCTAATTCTAGTGGAT
CTCCGCGGGCATCTTTCAGCTCTGCTCAACTGGTTGCACAGAGGAAACTATTTGCAGAATCCAAGAT
AGGAAGATCTAGCTTCCAGAAGCTTCTAGAACCAAGTCTGCCTCAACGTCCTGGAATTGCTCCTTAT
AGAATTGTTCTTGGAAATGTAAAAGATAAGCTTATGAGGACAAGA

## FIGURE 4 (continued)

AGACGTCTGGAACTTCTTCTAGAGGATCTTCCTTGTGAATATGATCAATGGGATTACTATGAGACAA
CAGATCAATTATTGGATCCACTGCTTCTGTGCTACGAGTCTCTTCAATCATGTGGAGCTGGGGTTCT
AGCTGATGGTCGGCTAGCTGACCTGATAAGAAGAGTTGCTACATTTGGGATGGTGTTAATGAAGCTA
GACTTGCGCCAGGAATCTGGTAGACATGCTGATACTCTTGATGCAATCACCAAATATTTGGAAATGG
GCACGTATAGTGAGTGGGATGAAGAAAAGAAGCTGGAATTTCTGACAAGAGAACTGAAAGGAAAACG
GCCACTGGTTCCTCCTACTATTGAGGTTGCTCCTGATGTTAAAGAAGTCTTGGATGCTTTCCGTGTT
GCTGCTGAGCTGGGGAGTGATTCACTTGGAGCTTATGTGATTTCTATGGCATCCAATGCAAGTGATG
TCCTTGCTGTGGAGCTTCTGCAGAAGGATGCCCGGCTTGCTGTTAGTGGGGAGCTAGGAAGACCATG
CCCTGGTGGAACGTTGCGAGTAGTTCCGTTGTTTGAAACTGTGAAAGACCTGAGAGGTGCTGGTTCG
GTGATCAGAAAACTGTTATCAATTGACTGGTATAGAGAACACATAATTAAGAACCATAACGGGCATC
AGGAGGTGATGGTTGGCTATTCTGATTCTGGTAAAGATGCTGGACGCTTTACTGCTGCATGGGAACT
TTACAAAGCCCAAGAGGATGTTGTGGCAGCCTGTAATGATTTTGGTATCAAAGTAACACTATTCCAT
GGGCGTGGAGGGAGTATTGGTCGTGGTGGTGGCCCCACATATCTTGCCATTCAATCCCAACCACCTG
GTTCAGTTATGGGTACTCTTCGGTCAACTGAGCAAGGAGAAATGGTGCAGGCCAAGTTTGGGCTGCC
ACATACTGCCATCAGACAACTAGAGATATACACAACTGCTGTGCTGCTTGCAACCCTTCGTCCTCCA
CATCCACCTCGAGAAGAACAATGGCGCAATGTCATGGAGGAGATTTCAAAAATCAGTTGCCAGAATT
ACCGGAGCACAGTCTATGAAAACCCAGAATTCCTCGCCTACTTCCATGAGGCTACTCCCCAGGCTGA
GCTTGGCTTTCTTAACATAGGAAGCCGCCCCACAAGAAGAAAGAGCTCAACTGGTATTGGACATCTT
CGTGCCATTCCATGGGTATTCGCATGGACCCAGACCAGATTTGTTCTTCCTGCTTGGCTTGGAGTTG
GAGCAGGTTTAAAGGGTGCTTGTGAGAAGGGATTTACTGAAGACTTGAAAGCAATGTACAAAGAATG
GCCTTTCTTCCAATCTACTATTGATCTTATAGAGATGGTACTAGGGAAGGCAGACATTCCTATAGCC
AAGCACTATGATGAAGTTCTTGTATCTGAGAGCAGGCGAGAGCTTGGAGCTGAGCTTAGGAGTGAGC
TCTTAACAACAGAAAAGTATGTGTTGGTGGTTAGTGGGCATGAGAAACTATCTCAGAACAACCGCAG
CCTGCGGAGGCTAATAGAGAGCAGGCTGCCTTATCTCAATCCTATGAATATGTTGCAGGTGGAGGTT
CTAAAGAGGTTGAGGCGGGACGACGACAACAATAAGCTCAGAGATGCCCTGCTTATTACCATAAATG
GGATTGCTGCTGGAATGAGGAACACAGGCTGA

SEQ ID NO: 22, protein - Ricinus communis
MTDTTDDIAEEISFQSFDDDCKLLGNLLNDVLQREVGSKFMEKLERNRILAQSACNMRLAGIEDTAE
LLEKQLALEISRMTLEEALTLARAFSHYLNLMGIAETHHRVRKARSMTHLSKSCDDIFNQLLQSGIS
AEELYDTVCKQEVEIVLTAHPTQINRRTLQYKHIRIAHLLDYNDRPDLTHEDREMLIEDLVREITSI
WQTDELRRHKPTPVDEARAGLNIVEQSLWKALPHYLRRVSTALKKHTGKPLPLTCTPIRFGSWMGGD
RDGNPNVTAKVTRDVSLLSRWMAVDLYIREVDSLRFELSMVQCSDRLLKVANDILIEETSSEDHHES
WNQPASRSQTKFPRKSLPTQLPPRADLPACTECNDGESQYPKLELPGTDYMPFNRQEALGSSYSESS
SQDINHGLPKTTGNGSVANSSGSPRASFSSAQLVAQRKLFAESKIGRSSFQKLLEPSLPQRPGIAPY
RIVLGNVKDKLMRTRRRLELLLEDLPCEYDQWDYYETTDQLLDPLLLCYESLQSCGAGVLADGRLAD
LIRRVATFGMVLMKLDLRQESGRHADTLDAITKYLEMGTYSEWDEEKKLEFLTRELKGKRPLVPPTI
EVAPDVKEVLDAFRVAAELGSDSLGAYVISMASNASDVLAVELLQKDARLAVSGELGRPCPGGTLRV
VPLFETVKDLRGAGSVIRKLLSIDWYREHIIKNHNGHQEVMVGYSDSGKDAGRFTAAWELYKAQEDV
VAACNDFGIKVTLFHGRGGSIGRGGGPTYLAIQSQPPGSVMGTLRSTEQGEMVQAKFGLPHTAIRQL
EIYTTAVLLATLRPPHPPREEQWRNVMEEISKISCQNYRSTVYENPEFLAYFHEATPQAELGFLNIG
SRPTRRKSSTGIGHLRAIPWVFAWTQTRFVLPAWLGVGAGLKGACEKGFTEDLKAMYKEWPFFQSTI
DLIEMVLGKADIPIAKHYDEVLVSESRRELGAELRSELLTTEKYVLVVSGHEKLSQNNRSLRRLIES
RLPYLNPMNMLQVEVLKRLRRDDDNNKLRDALLITINGIAAGMRNTG

FIGURE 4 (continued)

SEQ ID NO: 23, DNA - Arabidopsis thaliana
ACACTTGTTAGATTTACTTTTCCTCCGCACTATCAGCAGATTTTTCTCATCTGGGTCTGTTTGTTTC
TGTTGACTCAAAGTTTCCGACTTTCTTATTTTCTGTCTCCGATTTCATCGCCGAAATCTCAGTCAAT
TTTGTCTTTAGATCTTTAATTTTTGGATCCCTTTAATTATTTTGTTTGGTCTTACTTCAGAGCGAAG
CAGGTGAAAAAATGGCGAATCGGAAGTTAGAGAAGATGGCATCGATTGATGTTCATCTTCGTCAACT
GGTTCCTGGCAAAGTTAGTGAAGACGACAAGCTTGTTGAGTATGATGCTTTGCTTCTAGATCGGTTT
CTCGATATCCTCCAGGATTTGCACGGTGAAGATCTCCGTGAAACTGTTCAAGAGCTTTATGAGCATT
CTGCAGAATACGAAGGGAAGCATGAACCTAAGAAGCTAGAGGAGCTAGGGAGTGTGCTAACGAGTTT
AGATCCAGGAGATTCCATTGTTATCGCTAAAGCTTTCTCTCATATGCTTAACTTAGCCAATTTGGCT
GAGGAAGTGCAGATTGCTTATCGCCGTAGGATCAAGAAGCTGAAGAAAGGTGATTTTGTTGATGAGA
GCTCTGCTACTACTGAATCTGATCTTGAAGAAACTTTCAAGAAGCTTGTTGGAGATCTGAACAAGTC
TCCTGAAGAGATCTTTGATGCTCTCAAGAATCAGACTGTGGATTTGGTTTTGACTGCTCATCCTACT
CAGTCTGTGAGAAGATCATTGCTTCAGAAACATGGGAGGATAAGAGACTGTCTGGCTCAACTATATG
CTAAGGATATTACTCCTGATGACAAGCAAGAGCTCGATGAGGCTCTTCAGAGAGAGATTCAAGCTGC
ATTCCGAACAGATGAAATCAAAAGAACACCACCTACTCCTCAAGATGAGATGAGAGCGGGAATGAGT
TATTTCCATGAAACTATCTGGAAAGGTGTTCCTAAGTTTCTGCGCCGTGTTGACACGGCTTTGAAAA
ACATAGGGATCGAAGAACGTGTTCCATATAATGCTCCATTGATTCAGTTCTCTTCTTGGATGGGTGG
TGATCGTGACGGTAACCCAAGGGTTACACCTGAAGTCACCAGAGATGTTTGCTTGTTAGCTAGAATG
ATGGCTGCTACTATGTACTTTAACCAAATCGAAGATCTTATGTTTGAGATGTCTATGTGGCGTTGCA
ATGACGAGCTGCGTGCGCGAGCTGATGAAGTTCATGCAAATTCGAGGAAAGATGCTGCAAAACATTA
CATAGAATTCTGGAAGTCAATTCCTACAACTGAGCCATACCGTGTGATTCTTGGCGATGTAAGGGAC
AAGCTTTATCACACACGTGAACGCGCTCATCAACTGCTCAGCAATGGACACTCTGATGTCCCTGTAG
AGGCTACTTTCATTAACTTGGAACAGTTCTTGGAACCTCTTGAGCTCTGTTACCGATCTCTGTGTTC
ATGTGGTGATCGTCCAATAGCAGATGGAAGCCTTCTTGATTTCTTGAGGCAAGTCTCAACCTTTGGG
CTCTCTCTTGTGAGACTTGACATAAGGCAAGAATCTGACCGCCACACTGATGTATTGGATGCTATCA
CCACGCATTTAGATATCGGATCCTACAGAGAGTGGTCTGAAGAACGCCGCCAAGAATGGCTTTTATC
TGAGCTAAGTGGCAAACGTCCGCTTTTCGGTTCTGATCTTCCTAAAACCGAAGAAATAGCTGATGTT
CTGGACACGTTTCATGTCATAGCCGAGCTACCAGCAGATAGCTTTGGTGCTTACATTATCTCTATGG
CAACTGCACCTTCTGATGTATTAGCTGTTGAGCTTTTACAGCGTGAATGCCGAGTGAAACAGCCTTT
GAGAGTTGTTCCGCTCTTTGAGAAGCTAGCAGATCTGGAAGCAGCTCCTGCTGCAGTTGCTAGGCTC
TTTTCTGTTGATTGGTACAAAAACCGAATTAACGGTAAGCAAGAGGTTATGATTGGTTATTCGGATT
CAGGAAAAGATGCTGGACGGTTATCTGCTGCTTGGCAGTTATACAAAGCTCAAGAAGAGCTTGTGAA
GGTTGCTAAAGAGTACGGTGTGAAGCTAACAATGTTTCACGGTCGTGGTGGCACGGTCGGAAGAGGA
GGTGGACCAACCCATCTTGCTATATTGTCTCAGCCTCCGGATACTATTAACGGTTCCCTCCGTGTCA
CAGTTCAAGGTGAAGTCATCGAGCAATCGTTTGGTGAAGAGCACTTATGCTTTAGAACACTTCAGCG
TTTCACAGCTGCTACACTCGAGCACGGTATGCGTCCTCCAATTTCGCCTAAACCAGAATGGCGCGCT
TTGCTGGATGAAATGGCGGTTGTTGCAACCGAGGAGTATCGCTCAGTTGTGTTCCAAGAACCTCGGT
TTGTCGAGTACTTCCGCCTCGCTACACCGGAACTGGAGTATGGACGTATGAATATCGGAAGCAGACC
TTCGAAGCGTAAACCAAGCGGTGGCATTGAATCTCTCCGTGCAATTCCATGGATCTTCGCTTGGACT
CAAACAAGATTCCATCTTCCTGTATGGCTTGGATTCGGATCAGCAATTAGACATGTGATCGAAAAAG
ACGTCAGGAACCTCCATATGCTCCAAGATATGTACCAACACTGGCCTTTCTTTAGAGTCACCATTGA
TCTAATCGAAATGGTGTTCGCTAAAGGAGATCCTGGTATTGCTGCTTTGTACGATAAGCTTCTTGTT
TCAGAGGAACTCTGGCCTTTTGGTGAGAAACTCAGAGCTAACTTCGAAGAAACCAAGAAACTCATCC
TCCAGACCGCTGGACACAAAGATCTTCTTGAAGGTGATCCTTACTTGAAACAGAGACTGAGACTTCG
TGATTCTTACATTACAACTCTCAATGTCTGTCAAGCTTACACATTGAAGAGAATCCGTGATCCGAGT
TACCATGTGACTCTGCGACCACACATTTCTAAGGAGATAGCGGAATCGAGCAAACCAGCAAAAGAAC
TCATCGAGCTTAACCCGACTAGCGAATACGCGCCAGGACTTGAAGATACACTCATCTTGACGATGAA
GGGTATTGCTGCTGGTCTAC

**FIGURE 4 (continued)**

AAAACACCGGTTAAGCTACAAAGAGATGGTTAAACAAACTTTGAATCTCTCTTTCTCTCTCAAGTCT
CTCCTTTTTTTAACTACAGATTTGGAAAATAAGGTTGGATTCTGGTTTATTTTATGTATCCACCGTC
AAAATGTTGATTTTCGTGTACGAGTACTTCGAGATCATTGAACACATGCTCTGTTTTTTTCTCAAGT
TTAATAAAACAGAACAAGAGAATCTTTTCTTGTTTATTTTCTTATCT

SEQ ID NO: 24, protein - Arabidopsis thaliana
MANRKLEKMASIDVHLRQLVPGKVSEDDKLVEYDALLLDRFLDILQDLHGEDLRETVQELYEHSAEY
EGKHEPKKLEELGSVLTSLDPGDSIVIAKAFSHMLNLANLAEEVQIAYRRRIKKLKKGDFVDESSAT
TESDLEETFKKLVGDLNKSPEEIFDALKNQTVDLVLTAHPTQSVRRSLLQKHGRIRDCLAQLYAKDI
TPDDKQELDEALQREIQAAFRTDEIKRTPPTPQDEMRAGMSYFHETIWKGVPKFLRRVDTALKNIGI
EERVPYNAPLIQFSSWMGGDRDGNPRVTPEVTRDVCLLARMMAATMYFNQIEDLMFEMSMWRCNDEL
RARADEVHANSRKDAAKHYIEFWKSIPTTEPYRVILGDVRDKLYHTRERAHQLLSNGHSDVPVEATF
INLEQFLEPLELCYRSLCSCGDRPIADGSLLDFLRQVSTFGLSLVRLDIRQESDRHTDVLDAITTHL
DIGSYREWSEERRQEWLLSELSGKRPLFGSDLPKTEEIADVLDTFHVIAELPADSFGAYIISMATAP
SDVLAVELLQRECRVKQPLRVVPLFEKLADLEAAPAAVARLFSVDWYKNRINGKQEVMIGYSDSGKD
AGRLSAAWQLYKAQEELVKVAKEYGVKLTMFHGRGGTVGRGGGPTHLAILSQPPDTINGSLRVTVQG
EVIEQSFGEEHLCFRTLQRFTAATLEHGMRPPISPKPEWRALLDEMAVVATEEYRSVVFQEPRFVEY
FRLATPELEYGRMNIGSRPSKRKPSGGIESLRAIPWIFAWTQTRFHLPVWLGFGSAIRHVIEKDVRN
LHMLQDMYQHWPFFRVTIDLIEMVFAKGDPGIAALYDKLLVSEELWPFGEKLRANFEETKKLILQTA
GHKDLLEGDPYLKQRLRLRDSYITTLNVCQAYTLKRIRDPSYHVTLRPHISKEIAESSKPAKELIEL
NPTSEYAPGLEDTLILTMKGIAAGLQNTG

SEQ ID NO: 25, DNA - Arabidopsis thaliana
GTCTCGTTTAAATTTTTATAAACTCCATAATTTTATCTTAAAGTGAATCTTTTTTGTTTTTTTTGTT
CCAGCTTTATCGGATATATTTCCTTGATTTTCTCCGATTGTGGTCAATCTGGAAAATTATTGAGAAT
CTCTCCCTCACTTAACCAAAAGCGTTTTTAATCAGATAGAGAGAGAGGAAAAAGCATCAACCAAACC
ATGGCTGCGAGAAATTTGGAGAAGATGGCTTCTATTGATGCTCAGCTCAGGCTTCTTGCTCCTGGCA
AGGTTTCTGAAGACGACAAGCTTATCGAGTACGATGCTCTGTTACTGGATCGATTTCTCGATATTCT
TCAGGATTTGCATGGCGAGGATGTCAGAGAATTCGTTCAAGAATGCTACGAAGTTGCAGCTGATTAC
GATGGAAACCGCAACACTGAGAAGCTTGAGGAGCTTGGAAATATGCTGACGAGTTTGGATCCAGGGG
ATTCAATTGTTGTCACTAAATCATTCTCCAACATGCTTAGCTTGGCTAATCTGGCTGAGGAAGTCCA
GATTGCTTACCGGCGTAGGATTAAGAAACTCAAGAAAGGTGATTTCGCTGATGAGGCCTCTGCAACA
ACGGAATCTGACATTGAAGAGACTCTCAAGAGGCTTTTGCAGCTTAACAAGACTCCTGAAGAGGTCT
TTGATGCTCTTAAGAATCAGACTGTTGACTTGGTTTTAACTGCTCATCCCACTCAATCTGTTCGTCG
GTCTTTGCTCCAAAAGTTTGGAAGGATTCGTGATTGTTTGACGCAGTTATATGCAAAGGACATTACT
CCTGATGACAAACAAGAACTCGATGAAGCTCTGCAACGAGAGATTCAAGCTGCTTTTCGCACAGATG
AAATCCGAAGAACTCCTCCTACACCGCAAGATGAAATGAGAGCAGGGATGAGCTACTTCCATGAGAC
AATCTGGAAAGGAGTTCCAAAGTTCTTAAGACGTGTTGACACAGCTTTAAAGAACATTGGAATCAAC
GAGCGTGTTCCTTACAATGCGCCTCTCATTCAGTTCTCTTCTTGGATGGGCGGAGACCGTGATGGAA
ACCCGCGAGTAACTCCTGAAGTTACAAGAGATGTATGCTTATTAGCTAGAATGATGGCTGCTAATCT
CTACTTCTCCCAGATAGAAGATCTTATGTTTGAGATGTCTATGTGGCGTTGCAATGAGGAACTTCGG
GTTCGTGCAGAACGTCAAAGATGTGCGAAGAGGGATGCAAAACACTATATAGAATTCTGGAAACAAA
TCCCTGCGAATGAGCCATACCGAGCTATTCTTGGAGATGTGAGGGACAAGCTGTACAACACACGTGA
GCGTGCACGTCAGTTATTGTCAAGCGGAGTTTCGGACGTTCCCGAAGACGCGGTTTTCACAAGTGTG
GATCAGTTTTTGGAGCCACTTGAGCTTTGTTACAGGTCGCTCTGTGATTGCGGTGACAGACCTATTG
CTGATGGAAGCCTGCTTGATTTCTTACGCCAAGTGTCAACATTTGGCCTTGCTCTTGTGAAACTTGA
TATCCGTCAAGAATCTGAAAGACACTCTGATGTCTTGGATGCCATCACGACGCACTTAGGTATTGGT
TCTTACAAAGAATGGTCGGAGGATAAAAGACAGGAATGGCTG

**FIGURE 4 (continued)**

TTATCTGAGCTAAGCGGGAAACGCCCTCTCTTTGGACCGGATCTTCCCAAAACCGAAGAGGTTGCAG
ATGTGTTGGACACTTTCAAAGTCATTTCTGAGCTTCCTTCGGATAGTTTTGGTGCTTATATTATCTC
AATGGCCACTGCTCCATCAGACGTGCTCGCTGTTGAGCTTTTGCAACGCGAATGCGGGATCACTGAT
CCTCTGAGAGTTGTCCCGTTGTTCGAGAAGCTAGCGGATTTAGAATCCGCACCTGCTGCAGTTGCCC
GTCTCTTCTCCATAGAATGGTACAGAAACAGGATCAATGGAAAGCAAGAAGTCATGATCGGGTACTC
TGACTCGGGCAAAGATGCTGGTCGTTTATCAGCGGCTTGGCAGTTATACAAGACTCAAGAAGAGCTC
GTGAAGGTGGCAAAAGAATACGGAGTCAAGCTGACAATGTTCCACGGAAGAGGTGGGACCGTTGGAC
GAGGAGGTGGACCTACCCATCTTGCTATTTTGTCTCAGCCTCCGGATACCATTCATGGGCAATTGAG
GGTAACGGTTCAAGGTGAAGTTATTGAACAGTCTTTCGGAGAAGAGCACTTATGCTTTAGGACTCTT
CAGCGTTTCACAGCTGCAACACTTGAGCATGGAATGCATCCACCGGTTTCCCCTAAGCCTGAGTGGC
GTGTCCTCATGGATGAAATGGCTATAATTGCCACTGAAGAATACCGTTCTGTTGTCTTCAAGGAGCC
CCGTTTTGTTGAGTACTTCCGTCTGGCAACACCAGAGCTCGAGTATGGAAGGATGAACATAGGAAGC
CGACCATCAAAACGTAAACCAAGCGGAGGAATCGAGTCGCTGCGTGCAATCCCGTGGATCTTTGCGT
GGACTCAGACGAGGTTCACTTACCCGTGTGGCTTGGCTTTGGAGGAGCATTCAAACGCGTGATACAG
AAGGACAGTAAGAATCTCAACATGCTCAAAGAGATGTACAACCAATGGCCATTCTTCCGTGTCACAA
TTGATCTAGTCGAAATGGTTTTCGCCAAAGGAGATCCCGGAATTGCGGCTCTGTATGACCGCCTCCT
CGTCTCTGAAGAACTTCAACCATTCGGTGAACAACTTCGAGTTAACTACCAAGAGACCAGACGCCTC
CTCCTCCAGGTTGCAGGTCACAAAGACATTTTAGAAGGTGACCCTTACTTGAGGCAAAGGCTGCAGC
TTCGTGACCCATACATCACGACATTGAACGTGTGTCAAGCCTATACACTCAAGCAGATCCGTGACCC
AAGCTTCCACGTCAAAGTCCGGCCACATCTCTCTAAGGACTACATGGAGTCTAGTCCAGCGGCTGAG
CTCGTGAAACTGAATCCAAAGAGTGAATACGCACCGGGACTTGAAGATACGGTTATCCTCACCATGA
AGGGTATCGCTGCTGGTATGCAAAACACCGGTTAAGGCAGTTTAAAACGTTCTTGTACCATTCCCTA
AATCTACGCTATGTAATGTATTATGTTCTATGATGTGATGAAATCTCTCCAACTCCTATCCCGTACG
CTTTTTAATGAGTATGATAATTTCTTGTGTTATTCTATTGTTGTTATGTTACCATATCTAGGAAATA
TATTTCTGAAAGAAACAAGAAAAGAAATCTTTCTTTTCGTTCTAAGATGTT

SEQ ID NO: 26, protein - Arabidopsis thaliana
MAARNLEKMASIDAQLRLLAPGKVSEDDKLIEYDALLLDRFLDILQDLHGEDVREFVQECYEVAADY
DGNRNTEKLEELGNMLTSLDPGDSIVVTKSFSNMLSLANLAEEVQIAYRRRIKKLKKGDFADEASAT
TESDIEETLKRLLQLNKTPEEVFDALKNQTVDLVLTAHPTQSVRRSLLQKFGRIRDCLTQLYAKDIT
PDDKQELDEALQREIQAAFRTDEIRRTPPTPQDEMRAGMSYFHETIWKGVPKFLRRVDTALKNIGIN
ERVPYNAPLIQFSSWMGGDRDGNPRVTPEVTRDVCLLARMMAANLYFSQIEDLMFEMSMWRCNEELR
VRAERQRCAKRDAKHYIEFWKQIPANEPYRAILGDVRDKLYNTRERARQLLSSGVSDVPEDAVFTSV
DQFLEPLELCYRSLCDCGDRPIADGSLLDFLRQVSTFGLALVKLDIRQESERHSDVLDAITTHLGIG
SYKEWSEDKRQEWLLSELSGKRPLFGPDLPKTEEVADVLDTFKVISELPSDSFGAYIISMATAPSDV
LAVELLQRECGITDPLRVVPLFEKLADLESAPAAVARLFSIEWYRNRINGKQEVMIGYSDSGKDAGR
LSAAWQLYKTQEELVKVAKEYGVKLTMFHGRGGTVGRGGGPTHLAILSQPPDTIHGQLRVTVQGEVI
EQSFGEEHLCFRTLQRFTAATLEHGMHPPVSPKPEWRVLMDEMAIIATEEYRSVVFKEPRFVEYFRL
ATPELEYGRMNIGSRPSKRKPSGGIESLRAIPWIFAWTQTRFTYPCGLALEEHSNA

SEQ ID NO: 27, DNA - Arabidopsis thaliana
CCACGCGTCCGAATCCGCAGAGATTTCTTCTTTTCAGAAGAAGTAAGAGGGTGGCGAAGAAGATTTG
ATTGATCGGCGATAATGGCGGGTCGGAACATAGAGAAGATGGCATCTATTGATGCTCAGCTTCGGCA
ACTCGTTCCTGCTAAAGTCAGTGAAGACGATAAGCTTGTTGAGTACGATGCTCTTCTCCTTGATCGC
TTTCTCGACATTCTCCAGGATTTACACGGCGAGGATCTCCGTGAAACGGTTCAAGAGTTATACGAGC
TTTCTGCTGAGTATGAAGGGAAGCGTGAGCCTAGCAAGCTTGAGGAGCTAGGGAGTGTCCTA

**FIGURE 4 (continued)**

ACGAGTTTGGATCCTGGTGACTCAATTGTTATCTCCAAGGCTTTCTCTCACATGCTTAACTTAGCCA
ATTTGGCTGAGGAGGTGCAGATTGCTCACCGTCGCAGGATCAAGAAGCTGAAGAAAGGTGATTTCGT
TGATGAGAGTTCTGCAACTACTGAATCCGATATTGAAGAGACTTTTAAGAGGCTCGTTTCGGATCTT
GGTAAGTCTCCTGAAGAGATCTTTGATGCCTTGAAGAATCAGACTGTGGATCTGGTTTTGACTGCTC
ATCCTACTCAGTCTGTGCGTAGATCATTGCTTCAGAAGCATGGGAGGATAAGGGACTGTCTTGCTCA
ACTCTATGCAAAGGACATTACTCCTGATGACAAGCAGGAGCTAGATGAGTCTCTGCAAAGAGAGATT
CAAGCTGCATTCCGAACAGATGAGATTAGAAGAACACCTCCAACCCCACAAGATGAAATGAGAGCTG
GAATGAGTTATTTCCACGAGACAATCTGGAAAGGTGTCCCCAAGTTCTTGCGCCGTGTGGACACAGC
TCTGAAAAACATTGGGATTGATGAACGTGTTCCTTACAATGCCCCATTGATTCAATTCTCTTCGTGG
ATGGGCGGTGATCGTGATGGTAATCCGAGGGTCACACCTGAGGTCACTAGAGATGTGTGCTTGTTGG
CTAGAATGATGGCTGCCAATCTCTACTATAACCAAATCGAGAATCTGATGTTTGAGTTATCTATGTG
GCGTTGCACTGATGAATTCCGTGTGCGGGCGGATGAACTGCACAGGAACTCAAGGAAAGATGCTGCA
AAACATTACATAGAATTCTGGAAGACAATTCCTCCAACTGAGCCATACCGTGTGATTCTTGGTGATG
TGAGGGATAAGCTGTATCACACACGTGAGCGTTCCCGCCAATTGCTGAGTAATGGAATCTCGGATAT
TCCTGAAGAAGCTACCTTCACTAATGTGGAACAGTTCTTGGAGCCTCTTGAGCTCTGTTACCGATCA
CTATGTTCATGTGGTGACAGCCCGATAGCTGATGGAAGCCTTCTTGATTTCTTGAGGCAAGTCTCTA
CCTTTGGACTCTCCCTTGTGAGACTTGACATCAGGCAAGAGTCTGAACGCCACACAGATGTCTTGGA
TGCTATCACCAAGCACTTGGACATCGGTTCCTCCTATAGAGACTGGTCTGAAGAAGGCCGACAGGAA
TGGCTTCTTGCTGAACTAAGCGGCAAACGTCCACTTTTCGGACCTGATCTTCCCAAAACCGAAGAAA
TTTCTGATGTCCTGGACACATTCAAAGTCATATCTGAGCTGCCTTCAGATTGTTTTGGAGCTTATAT
TATCTCTATGGCAACTTCACCTAGTGATGTGCTTGCGGTTGAGCTTTTACAGCGCGAATGCCATGTG
AAAAATCCACTTAGAGTTGTTCCACTCTTTGAGAAGCTAGCTGATCTTGAAGCAGCTCCTGCCGCTG
TTGCAAGACTCTTTTCTATAGACTGGTACAAAAACCGTATTAACGGTAAACAAGAGGTTATGATTGG
TTACTCAGATTCAGGGAAAGATGCAGGGCGTCTCTCAGCTGCTTGGGAGCTATACAAAGCTCAAGAA
GAGCTTGTGAAGGTTGCTAAGAAATATGGAGTGAAGCTAACTATGTTCCATGGCCGTGGTGGCACAG
TCGGAAGAGGAGGTGGTCCTACTCATCTTGCTATATTGTCTCAGCCACCAGATACAGTTAATGGCTC
TCTTCGAGTCACGGTTCAGGGTGAAGTCATTGAGCAATCATTTGGGGAGGCACACTTATGCTTTAGA
ACACTTCAACGTTTCACAGCAGCTACTCTAGAGCACGGAATGAACCCTCCGATTTCACCAAAACCCG
AGTGGCGTGCTTTGCTTGATGAAATGGCGGTTGTTGCAACTGAGGAATACCGATCTGTCGTTTTCCA
AGAACCTCGATTCGTCGAGTATTTCCGCCTCGCTACTCCGGAGCTGGAGTATGGACGTATGAATATT
GGAAGTAGACCTTCAAAGCGAAAACCAAGCGGTGGGATCGAATCTCTCCGTGCAATCCCATGGATCT
TTGCTTGGACGCAAACAAGATTCCATCTTCCTGTATGGTTAGGTTTCGGAGCAGCATTTAGGTATGC
GATCAAGAAGGATGTGAGAAACCTTCACATGCTGCAAGATATGTATAAACAATGGCCCTTTTTCCGA
GTCACCATCGATCTAATTGAAATGGTGTTCGCCAAGGGAGACCCCGGGATCGCTGCTTTGTACGACA
AACTTCTTGTCTCAGAAGATTTATGGGCTTTTGGAGAGAAACTCAGAGCCAACTTTGATGAAACCAA
GAACCTCGTCCTCCAGACTGCTGGACATAAAGACCTTCTTGAAGGAGATCCTTACTTGAAACAGAGA
CTAAGGCTACGTGACTCTTACATTACGACCCTCAACGTTTGCCAAGCCTACACATTGAAGAGGATCC
GTGATGCAAACTACAATGTGACTCTGCGACCACACATTTCTAAAGAGATCATGCAATCAAGCAAATC
AGCACAAGAGCTCGTCAAGCTTAACCCCACGAGTGAATACGCGCCTGGACTTGAGGACACACTTATC
TTAACCATGAAGGGTATTGCTGCAGGATTGCAAAACACCGGTTAAGTGAGTCAGTGAAAGAAAACAA
AACTTCGAATCTCTCTTTTTTATCTACCCTTTTAATAATCTCTTTTTTTCTAGAATCCAAAATAAT
TACGGTTGGATTACAGTTTACTTTATGTATCCACCGTTGAAATCTTAATCTTCCATTGTATCAAACG
TCACTGACTCTGTTTCTGGAAGTGTAAACAAGAACAGAGACAGTGAATCTTAATGTTATCTTCTTTG
TCTAAAAAAAAAAAAAAAAA

**FIGURE 4 (continued)**

SEQ ID NO: 28, protein - Arabidopsis thaliana
MAGRNIEKMASIDAQLRQLVPAKVSEDDKLVEYDALLLDRFLDILQDLHGEDLRETVQELYELSAEY
EGKREPSKLEELGSVLTSLDPGDSIVISKAFSHMLNLANLAEEVQIAHRRRIKKLKKGDFVDESSAT
TESDIEETFKRLVSDLGKSPEEIFDALKNQTVDLVLTAHPTQSVRRSLLQKHGRIRDCLAQLYAKDI
TPDDKQELDESLQREIQAAFRTDEIRRTPPTPQDEMRAGMSYFHETIWKGVPKFLRRVDTALKNIGI
DERVPYNAPLIQFSSWMGGDRDGNPRVTPEVTRDVCLLARMMAANLYYNQIENLMFELSMWRCTDEF
RVRADELHRNSRKDAAKHYIEFWKTIPPTEPYRVILGDVRDKLYHTRERSRQLLSNGISDIPEEATF
TNVEQFLEPLELCYRSLCSCGDSPIADGSLLDFLRQVSTFGLSLVRLDIRQESERHTDVLDAITKHL
DIGSSYRDWSEEGRQEWLLAELSGKRPLFGPDLPKTEEISDVLDTFKVISELPSDCFGAYIISMATS
PSDVLAVELLQRECHVKNPLRVVPLFEKLADLEAAPAAVARLFSIDWYKNRINGKQEVMIGYSDSGK
DAGRLSAAWELYKAQEELVKVAKKYGVKLTMFHGRGGTVGRGGGPTHLAILSQPPDTVNGSLRVTVQ
GEVIEQSFGEAHLCFRTLQRFTAATLEHGMNPPISPKPEWRALLDEMAVVATEEYRSVVFQEPRFVE
YFRLATPELEYGRMNIGSRPSKRKPSGGIESLRAIPWIFAWTQTRFHLPVWLGFGAAFRYAIKKDVR
NLHMLQDMYKQWPFFRVTIDLIEMVFAKGDPGIAALYDKLLVSEDLWAFGEKLRANFDETKNLVLQT
AGHKDLLEGDPYLKQRLRLRDSYITTLNVCQAYTLKRIRDANYNVTLRPHISKEIMQSSKSAQELVK
LNPTSEYAPGLEDTLILTMKGIAAGLQNTG

SEQ ID NO: 29, DNA - Arabidopsis thaliana
ACAATGACGGACACAACAGACGATATCGCAGAGGAAATCTCATTCCAAAGCTTCGAAGATGACTGCA
AATTGCTCGGTAGTCTCTTCCATGATGTGTTACAAAGGGAAGTTGGCAACCCATTCATGGAAAAAGT
CGAACGCATTCGGATTCTTGCTCAGAGTGCGTTAAATTTGCGTATGGCTGGTATTGAGGATACCGCA
AACCTTTTGGAGAAGCAATTGACTAGTGAAATATCCAAAATGCCACTAGAAGAAGCCTTAACGTTGG
CTCGTACATTCACTCATTCTCTTAACTTAATGGGCATTGCAGACACTCATCACAGAATGCACAAAGT
CCATAACGTTACACAACTTGCAAGATCTTGTGATGATATATTCAGCCAGCTATTGCAAAGTGGAATC
TCTCCAGACGAACTTTATAAAACTGTTTGCAAACAGGAGGTCGAAATTGTTCTTACTGCTCATCCTA
CCCAAATAAATCGAAGAACCTTGCAGTACAAGCATATTAGAATTGCTCATCTTCTAGAATATAACAC
TAGATCAGATCTAAGCGTTGAAGATCGCGAAACGCTCATTGAAGATTTGGTTAGAGAGATTACTTCA
CTGTGGCAAACTGATGAGCTTAGACGTCAGAAACCTACTCCAGTTGATGAAGCTAGAGCTGGTCTAA
ACATAGTGGAGCAATCCCTTTGGAAAGCAGTACCACAATACCTGCGTCGTGTCAGCAATTCCTTGAA
GAAGTTTACAGGGAAGCCACTTCCACTAACATGCACTCCTATGAAATTTGGTTCTTGGATGGGAGGT
GATAGAGATGGAAATCCAAATGTCACGGCAAAGGTCACGAAAGAAGTATCTCTCTTGTCTAGATGGA
TGGCTATTGATTTGTACATAAGAGAGGTTGATAGCTTAAGATTTGAATTATCTACGGATCGATGCAG
TGATAGGTTTTCAAGATTAGCTGATAAAATTCTTGAAAAGGATTATGATAGAGGAAAATCAAATTTC
CAAAAGCAACAAAGTTCATCATGCTTGCCAACACAACTTCCAGCTAGAGCTCACCTTCCTGCTTGCA
TTGACTTTGGTGAATCACGACATACCAAATTTGAAATTGCGACGACAGATTATATGCCACCCAATCT
CCAGAAGCAGAATGAACAAGACTTTTCGGAAAGCGACTGGGAGAAAATTGACAATGGTTCGCGGTCC
GGTCTTACTTCTCGAGGTTCTTTCTCATCTACTTCTCAACTTCTTCTCCAGAGAAAACTATTTGAGG
AATCTCAGGTTGGGAAGACTAGTTTCCAAAAGCTACTAGAACCACCTCCACTTAAACGAGCTGGAAG
TGCTCCTTATCGTATTGTTCTTGGAGAAGTAAAAGAAAAGCTTGTGAAGACAAGAAGACTTCTTGAA
CTTCTTATTGAGGGTCTTCCTTGTGAGTATGACCCTAAAAACTCCTATGAAACATCAGATCAGCTTC
TTGAACCATTGCTCCTCTGTTACGAATCTCTGCAATCATCGGGTGCTAGGGTACTAGCTGATGGACG
ACTTGCTGATCTGATTCGTAGAGTTTCTACCTTTGGAATGGTTTTGGTGAAACTCGACTTACGCCAG
GAAGCTGCAAGACATTCTGAAGCTTTGGATGCAATTACAACATACTTGGATATGGGTACTTATAGTG
AATGGGATGAAGAGAAGAAATTAGAATTTTTGACAAGAGAACTAAAAGGGAAACGACCTCTTGTTCC
TCAATGTATTAAGGTTGGTCCTGACGTCAAAGAAGTATTGGACACATTCCGAGTCGCTGCTGAACTT
GGAAGTGAATCACTTGGCGCTTACGTTATTTCTATGGCTTCAAATGCAAGTGATGTCCTCGCTGTGG
AACTTCTTCAAAAAGATGCTCGACTTGCTTTAACTAGCGAACATGGAAAACCATGTCCTGGTGGAAC
GCTACGAGTGGTACCTCTTTTTGAAACGGTGAATGAT

**FIGURE 4 (continued)**

TTAAGAGCCGCTGGTCCTTCGATAAGGAAATTGCTCTCAATCGATTGGTATAGGGAACACATCCAAA
AGAACCACAACGGTCACCAAGAGGTGATGGTTGGATACTCTGATTCTGGAAAAGATGCTGGACGTTT
TACTGCAGCATGGGAACTCTACAAAGCTCAAGAAAATGTTGTTGCTGCTTGTAATGAATTTGGAATC
AAAATAACATTATTTCATGGACGAGGAGGAAGCATTGGTCGTGGTGGTGGTCCAACCTATCTCGCTA
TTCAGTCCCAACCACCAGGCTCTGTAATGGGCTCTTTGCGTTCAACTGAGCAAGGTGAGATGGTTCA
AGCTAAGTTTGGGATACCACAAACGGCTGTTAGGCAACTAGAGGTATACACAACCGCGGTTCTACTC
GCTACCTTAAAGCCTCCTCAGCCACCTCGAGAGGAAAAATGGCGAAACCTAATGGAAGAAATCTCTG
GAATCAGTTGCCAACACTATAGAAGCACAGTGTATGAAAACCCAGAGTTTCTATCTTATTTTCATGA
GGCAACACCGCAAGCAGAACTTGGTTTCCTCAATATAGGAAGCCGACCAACACGAAGAAAGAGCTCT
AGTGGAATAGGACATCTCCGAGCTATCCCTTGGGTCTTTGCTTGGACTCAAACAAGGTTTGTTCTTC
CAGCTTGGCTTGGTGTAGGGGCTGGTTTAAAGGGAGTTTCTGAGAAGGGTCATGCGGATGATCTTAA
AGAGATGTACAAAGAATGGCCATTTTTTCAGTCCACCCTTGAACTTATAGAGATGGTGTTAGCTAAA
GCAGACATTCCAATGACCAAACACTACGACGAACAACTTGTGTCTGAGAAAAGAAGAGGACTTGGCA
CTGAGCTAAGAAAAGAACTAATGACTACTGAGAAGTACGTTCTTGTGATAAGTGGTCACGAGAAACT
CTTGCAGGACAATAAGAGCTTGAAGAAACTCATTGATAGTAGACTTCCGTATCTCAACGCAATGAAC
ATGTTACAAGTTGAAATTCTTAAGAGGCTAAGACGTGATGAAGATAACAATAAGCTAAGAGATGCTT
TGCTTATCACAATCAATGGTATTGCTGCAGGAATGAGAAATACCGGTTAA

SEQ ID NO: 30, protein - Arabidopsis thaliana
MTDTTDDIAEEISFQSFEDDCKLLGSLFHDVLQREVGNPFMEKVERIRILAQSALNLRMAGIEDTAN
LLEKQLTSEISKMPLEEALTLARTFTHSLNLMGIADTHHRMHKVHNVTQLARSCDDIFSQLLQSGIS
PDELYKTVCKQEVEIVLTAHPTQINRRTLQYKHIRIAHLLEYNTRSDLSVEDRETLIEDLVREITSL
WQTDELRRQKPTPVDEARAGLNIVEQSLWKAVPQYLRRVSNSLKKFTGKPLPLTCTPMKFGSWMGGD
RDGNPNVTAKVTKEVSLLSRWMAIDLYIREVDSLRFELSTDRCSDRFSRLADKILEKDYDRGKSNFQ
KQQSSSCLPTQLPARAHLPACIDFGESRHTKFEIATTDYMPPNLQKQNEQDFSESDWEKIDNGSRSG
LTSRGSFSSTSQLLLQRKLFEESQVGKTSFQKLLEPPPLKRAGSAPYRIVLGEVKEKLVKTRRLLEL
LIEGLPCEYDPKNSYETSDQLLEPLLLCYESLQSSGARVLADGRLADLIRRVSTFGMVLVKLDLRQE
AARHSEALDAITTYLDMGTYSEWDEEKKLEFLTRELKGKRPLVPQCIKVGPDVKEVLDTFRVAAELG
SESLGAYVISMASNASDVLAVELLQKDARLALTSEHGKPCPGGTLRVVPLFETVNDLRAAGPSIRKL
LSIDWYREHIQKNHNGHQEVMVGYSDSGKDAGRFTAAWELYKAQENVVAACNEFGIKITLFHGRGGS
IGRGGGPTYLAIQSQPPGSVMGSLRSTEQGEMVQAKFGIPQTAVRQLEVYTTAVLLATLKPPQPPRE
EKWRNLMEEISGISCQHYRSTVYENPEFLSYFHEATPQAELGFLNIGSRPTRRKSSSGIGHLRAIPW
VFAWTQTRFVLPAWLGVGAGLKGVSEKGHADDLKEMYKEWPFFQSTLELIEMVLAKADIPMTKHYDE
QLVSEKRRGLGTELRKELMTTEKYVLVISGHEKLLQDNKSLKKLIDSRLPYLNAMNMLQVEILKRLR
RDEDNNKLRDALLITINGIAAGMRNTG

SEQ ID NO: 31, DNA - Sorghum bicolor
AGGCCTAGTATTGCTTCAAATTCTGCGGCAATTCAACTCAACAAATGCTTATCCTGAGTTTGTTTAT
ATGTACTCCATTAGTTTTATTAAAGATAAGTTCTCTTGATTATTCTACGTAGATAGCTTTGTTGTAC
ACTCATTATATTATTTTATATATACATAATACAAAATAATATTTATCTAAAAAAATCAGAACAATGT
GCACATACTAATAAATTCCCATTTCAATGTTGGACCATGAGCAGATCTGACGGTACACATGTTCAAG
TGTCTCCGTTCTCCTGTGGAAATAATAAATCGCAGCCGTCCACGTGACACACAACACAGATGTCTCT
CGATCAGTATAGGCTGTTCCTGTTCTTCGCTTTGGTAACAAATCCCCTGCAAATTGATCGATAATAT
AAGAATAAAATAACGTGCATCTCGTACATTCGCTTGGACCTGCCTGCTAAATGCTAATGCTAATGCT
ACCATCTTCCTCCTCCGTCTATAAAGTGGCCGCCAGAATCCGCTCCTCCTACAATTGCAGCAGCCTC
CTCTCCTCTCCTCCCTGCCTGGTTCTTGCTTGCAGTTCTTGAACCAAATCCACACCGCCGGCGCGCT
TAGCATTGGGGCTAGCTAGGGGGCCGATCGATATGGCGGCCTTAGGGGCGAAGATGGAGCGGCTGTC
GTCGATCGACGCGCAGCTGCGGATGCTGGTGCCGGGGAAGGTCTCGGAGGACGACA

**FIGURE 4 (continued)**

AGCTCATCGAGTACGACGCGCTCCTCCTCGACCGCTTCCTCGACATCCTCCAGGACCTCCACGGCGA
CGACCTCAAGGAGATGGTAACTCTCCTCCGACTGGTTCTTAGACTCTTGTTGTTCTTTTGTTTTACT
GGTTCGGGCTTGCGATGCAGCTTTAATCTTGGGTTGGATCCAAGTAATCTAGTTCAGTCCTGTGGTC
TTTACCTGTTATTTTTACCTTTATTTAATCTTCCATGTTTTTTTTACTTATTTGTCTTCTTTTTCCT
GAGAAAAAAGTATCAATGTTTAGTAAAAAAAAAAGGCATGAAATTATTGCCTTCTTTGTTCTTTGTTT
TCTTTTCGTGCGATTCCTTTTTAGAGTGCCAGCTTGAGTAGTATTGAAGAGTGTGTGGTTGACTTCA
GAGTCCACTCTTCAGATTAAGTGATTAACACTTCAGGTTTAAGTGTAACTACAAGGTGGTTGATTTG
CTGCTTAATTTGGCGTTGTCAAGTATAGTAATTATACTAAAATATAAATTATAGTGGATGGCCAGGT
GCTGGATTAACGTTCCTAGTGTTCAATTTCTTCTTCTTCTTCTTTTTGTTTTGTTATTGCTCAA
AGATTGGTTTTTCAGTGGCAAAGCGATCATCTCGATTTTTGATTGATTGGGTCGGATATGGTTCGAA
TTTGATCTCAATCATTCGGTAGAACACTTCCTATGTGGAAATCCATCTGTAACAAGTGTGGATCTTA
GTTGTATATATGTTCCTGGTCCATTAACTGACTTTGGACCGATTGATTGACTGAAACAACGAAACGC
TGCAGATTCCACGTCATCTTTTGAGTTCTTGACTTTTCGTTTGGATGGCAACATATAGTTGACAAAA
CAAACTTTATCTCCAGCAACGCAACTTTACACCTGATTGCAGTTCATAGCACGTGAACCCCTGTCAT
CTTTCTAGCACCTTCCCTGACAGAACATGGACACCCAGAACATGTGACAACTAATAGACAAACTATG
AAAATTTTTGGATCAAAATAAAGCCCCCGTTGAAGTGGGTATGTTTGTATTGCTCAGAAAGTGTGAC
TTACCATATTAAATTTATTTGACATGGCCAGCTGGCAGCAATACTACATATGTAAATTTATTTGGAA
CTGATATAGTGATATGTGGATTGAGTATGGTAGTAACAAAATCCAGAAACCGACATATCTTTCTCTC
TTTTTTTTAATCATGTATGGTCATTAATTGAGTTCTCTTGCTGATTAGGTTCAAGAATGCTATGAGG
TAGCTGCTGAGTATGAAACAAAACATGACTTGCAAAAGCTTGATGAACTCGGGAAGATGATAACAAG
CTTGGATCCTGGGGACTCTATTGTGATCGCCAAGTCTTTCTCGCACATGCTTAACTTGGCCAACTTG
GCTGAGGAAGTCCAGATAGCATACAGGAGGAGAATCAAGCTCAAGAAGGGAGACTTTGCTGATGAGA
ACTCGGCAATCACAGAATCTGACATTGAGGAAACACTTAAGAGGCTTGTTGTTGACCTGAAGAAGTC
ACCTGCTGAGGTATTTGATGCCCTCAAGAGCCAGACTGTTGATCTGGTTTTGACTGCACATCCAACA
CAGTCTGTGAGGAGGTCACTGCTCCAGAAACACTCGAGGTTGGTTTGCCAAACGTTGCATTGTAGTT
GACATGCACAACTCCGTTTTAGCCTTTAATCTTGATCCCAACATTCTTACCAGGATACGAAACTGTT
TGGTTCAACTCTACTCAAAAGATATCACTCCAGATGATAAGCAGGAACTTGATGAGGCTCTGCAGAG
AGAGGTATTTATTTCAATCTAATGTTTATTACCTGGAATTCTTTGAAGCTTGAGACCAAAGATATCA
ATTCTTAATTCCTTACATGGACTCCTCTCTCCTTTTTCAAGATCCAAGCTGCCTTTAGAACTGATGA
GATCCGAAGAACGCAGCCCACTCCCCAAGATGAAATGCGTGCTGGTATGAGCTACTTCCATGAAACA
ATTTGGAAGGGTGTTCCAAAGTTCTTGCGCCGAGTTGATACTGCATTGAAGAACATTGGGATTAACG
AGCGTGTTCCTTACAATGCACCTCTTATTCAGTTCTCTTCTTGGATGGGAGGAGATCGTGATGGTGC
ACGTGACATCTCTACATATTTGTCTTTTCTTCTCATGGCATTATAAATATGAGTATTTGCAGCACCT
GTTATTATCTTTGTTGCTAGTGGTTGGGAAGATGACTATTCATTGTCCATGTGTCTGCAGTCTGCAC
CTCTGTCCTTTGTATTCTAAAGCAATGTTTCTACAAATGCAGGAAATCCAAGAGTGACACCAGAGGT
TACCAGGGATGTCTGCTTGCTTGCCAGGATGATGGCATCGAACTTGTACTGCTCTCAGATTGAGGAT
CTCATGTTTGAGGTTGCTATTACTTTCAGAATCAGAGTATAGTTCATAACATCATTTCTATCAGATG
ATTGAACTTGATAACTTGAAATGCTTGCAGTTGTCTATGTGGCGATGCAGTGATGAGCTGCGCATGC
GAGCTGATGAGCTGCATCGCTCCACTAAGAAGGATGCCAAGCATTACATAGGTAGTTACCAGTGACA
TCATTGGGTCTTCTTAACAATGGCCTGTTGTTATTAGGCCATATTTTTGGCATTTTAATAATTTTTC
CTTCTTCATTTCTGCTGTAGAGTTTTGGAAGAAGGTTCCTCCAAATGAGCCATATCGGGTGATACTG
AGTGATGTCAGGGATAAACTCTACAACACTCGTGAGCGGTCACGAGAGCTTTTATCCAGTGGACATT
CTGATATTCCTGAGGAAGCTACATTGACAACTGTTGAGCAGGTTGAGAATTAATCAGTAATCTTCAG
AAAATAATATTGATTTCTTGGTTTTCCCTCAGTGGGTTCTGATATGTAGATGCCAACTTTTGCAGCT
GTTGGAGCCCTTGGAACTATGTTACAGATCACTCTGTGCTTGTGGTGACCGTGTAATTGCTGATGGA
AGTCTTCTGGATTTCTTGCGTCAAGTCTCCACCTTTGGACTCTCCCTTGTGAGGCTTGACATTAGGC
AAGAATCAGATCGGCATACTGATGTCCTTGATGCCATCACTACGTACCTGGGGATAGGATCTTACCG
CGAGTGGCCTGAAGAACGC

**FIGURE 4 (continued)**

CGCCAAGAATGGCTACTGTCTGAACTTAATGGCAAGCGCCCTCTGTTTGGCCCGGACCTTCCCAAGA
CTGAGGAGATTGCTGATGTTCTAGACACATTCCACGTTATCGCTGAGCTTCCTGCTGACAATTTTGG
TGCTTATATCATTTCCATGGCAACAGCTCCTTCGGATGTACTTGCTGTTGAGCTCCTCCAACGTGAG
TGTCATGTAAAGACACCACTTAGAGTAGTCCCACTGTTTGAGAAGTTGGCTGATCTTGAGGCTGCCC
CAGCTGCATTGGCCAGACTGTTCTCAATCGATTGGTATAGACAGAGGATCAATGGCAAGCAGGAGGT
CATGATTGGGTATTCAGATTCAGGCAAAGATGCAGGTCGACTCTCAGCAGCTTGGCAGCTATACAAA
GCTCAGGAGGAGCTCATCAAGGTTGCTAAGGACTTCGGTGTGAAGTTGACGATGTTCCATGGACGTG
GTGGGACTGTTGGAAGGGGTGGTGGTCCCACTCACCTTGCCATCTTGTCCCAGCCACCAGACACGAT
CCATGGATCACTCCGGGTCACTGTTCAAGGTGAAGTCATTGAGCAGTCTTTTGGTGAGGAGCACTTG
TCGTTTAGGACACTGCAACGTTTCACGGCTGCTACCCTGGAGCATGGAATGCACCCACCAAATGCAC
CAAAGCCAGAATGGCGAACCCTTCTTGATGAGATGGCAGTTGTGGCAACTGAGGAATATCGGTCCAT
TGTCTTTCAAGAGCCACGCTTCGTTGAGTATTTTCGCCTCGTGCAGCATCGTTGAGTATTTCCGCCT
CGTAGTCCTTGTTGTCCATTGTCATACTACAGTAACATAAGCCTGCAAAACGTAACTTTTGAAATAG
GTCGCCTGACTTGAGACTTAATATGAATGTAGCACAATGATTTACTGCACTATATCTTTTTGCAAAC
CTAACTAAAATGGTTAATGAAATATTTGATTTGACCCTACATTTAGCGAATATTTACTTTGTGATAT
TTGTGTTGACAGGCAACACCTGAAACAGAGTATGGTAGGATGAACATAGGAAGCAGGCCATCCAAGA
GAAAGCCGAGCGGAGGTATCGAGTCACTCCGAGCAATCCCATGGATCTTTGCTTGGACACAAACACG
GTTCCACCTCCCGGTATGGCTAGGATTTGGAGGCGCATTCAAGCATGTCCTCCAGAAGGACATCAGG
AACCTCCACATGCTCCAGGAAATGTACAATGAGTGGCCATTTTTCAGGGTCACTATTGATCTGGTGG
AGATGGTGTTCGCCAAGGGTAACCCTGGCATTGCTGCACTTTATGACAAACTCCTAGTTTCAGAGGA
ACTGCGTCCATTGGGTGAGAAGCTGAGGGCCAACTATGAAGAAACCCAAAAGCTTCTACTTCAGGTC
GCTGTCCTGTCCAAAAGACACATCGATACATTCTTGCACTTTGTTGAGCTACCATTCTAGTAGCGTT
TTATCATTGAACTCTGTTTTGGACTTCTATCAGGTTGCCGGGCACAGGGATCTCCTGGAAGGTGACC
CTTACCTGAAGCAGCGGCTCCGCCTCCGTGACGCGTACATCACCACCCTGAACGTCTGCCAGGCCTA
CACCCTGAAGCGGATCCGTGACCCGGACTACCATGTCGCGCTGCGCCCCCACCTCTCCAAGGAGATC
ATGGACCCCACCAAGGCGGCTTCAGAGCTGGTGAAGCTGAACCCTGGCAGCGAGTATGCACCGGGGC
TGGAGGACACCCTCATTCTGACCATGAAGGGCATAGCAGCCGGTCTCCAGAACACCGGTTAAAACAC
AAAGAGGGGATCTTTTTTTTTAATCCTGATCATTGTCTCTAGGTCCTTTGTATTTACGCAGATTTTA
GCTGCATCTTCGGCCGTCTCTCTACACCGTGAGGAAATAATGCGATTTTGCCAAAGTGGCGGTAAAT
AAAAGGGAGATGAGGATCATACATGTTTTTCTTATTAGTAGACCGTTGTGTTGCAACTTTACTATGC
GATAATGGAACATGCTGTAGCCTAATTGAAGTGCCGTATCTCAAGCATTTTTGCAGGCAGGCTCTGA
ATTGTTGGAATTGTTCCAATGTTCCCTTTGCAATCTGTTGCCTGCAG

SEQ ID NO: 32, protein - Sorghum bicolor

MERLSSIDAQLRMLVPGKVSEDDKLIEYDALLLDRFLDILQDLHGDDLKEMVQECYEVAAEYETKHD
LQKLDELGKMITSLDPGDSIVIAKSFSHMLNLANLAEEVQIAYRRRIKLKKGDFADENSAITESDIE
ETLKRLVVDLKKSPAEVFDALKSQTVDLVLTAHPTQSVRRSLLQKHSRIRNCLVQLYSKDITPDDKQ
ELDEALQREIQAAFRTDEIRRTQPTPQDEMRAGMSYFHETIWKGVPKFLRRVDTALKNIGINERVPY
NAPLIQFSSWMGGDRDGNPRVTPEVTRDVCLLARMMASNLYCSQIEDLMFELSMWRCSDELRMRADE
LHRSTKKDAKHYIEFWKKVPPNEPYRVILSDVRDKLYNTRERSRELLSSGHSDIPEEATLTTVEQLL
EPLELCYRSLCACGDRVIADGSLLDFLRQVSTFGLSLVRLDIRQESDRHTDVLDAITTYLGIGSYRE
WPEERRQEWLLSELNGKRPLFGPDLPKTEEIADVLDTFHVIAELPADNFGAYIISMATAPSDVLAVE
LLQRECHVKTPLRVVPLFEKLADLEAAPAALARLFSIDWYRQRINGKQEVMIGYSDSGKDAGRLSAA
WQLYKAQEELIKVAKDFGVKLTMFHGRGGTVGRGGGPTHLAILSQPPDTIHGSLRVTVQGEVIEQSF
GEEHLSFRTLQRFTAATLEHGMHPPNAPKPEWRTLLDEMAVVATEEYRSIVFQEPRFVEYFRLATPE
TEYGRMNIGSRPSKRKPSGGIESLRAIPWIFAWTQTRFHLPVWLGFGGAFKHVLQKDIRNLHMLQEM
YNEWPFFRVTIDLVEMVFAKGNPGIAALYDKLLVSEELRPLGEKLRANYEETQKLLLQVAGHRDLLE
GDPYLKQRLRLRDAYITTLNVCQAYTLKRIRDPDYHVALRPHLSKEIMDPTKAASELVKLNPGSEYA
PGLEDTLILTMKGIAAGLQNTG

**FIGURE 4 (continued)**

SEQ ID NO: 33, DNA - Glycine max
ATGGCTGCTCGGAACATCGAGAAGATGGCCTCAATTGATGCTCAACTGAGGTTGCTGGCGCCACGCA
AGGTGTCTGATGATGACAAACTCGTTGAGTATGATGCCTTGTTGTTGGATCGTTTCCTTGACATTCT
TCAGGATTTGCATGGTGAAGATATCAGACAAACGGTCCAAGATTGTTACGAGCTGTCAGCTGAGTAC
GAAGGAGAGCATAAGCCTGAAAAGTTGGAGGAACTTGGGAATATGCTGACGGGTCTTGATGCTGGGG
ATTCAATTGTTATTGCCAAGTCATTTTCTCACATGCTCAATTTGGCCAACTTGGCAGAAGAAGTTCA
AATTGCGTATCGAAGAAGGATCAAGTTACTGAAGAAGGGTGATTTTGCTGATGAGAACTCTGCCATT
ACTGAGTCAGACATTGAAGAGACCTTCAAGAAGCTTGTGGCTCAACTGAAGAAGACACCCCAGGAAA
TCTTTGATGCTTTGAAGAACCAAACTGTGGATTTGGTTCTAACTGCTCATCCTACTCAGTCTGTTCG
CAGATCTTTGCTGCAAAAGCATGGAAGGATAAGGAATTGTTTGACACAATTATATGCTAAGGACATA
ACACCAGATGATAAGCAGGAACTTGATGAGGCTTTACAAAGAGAGATTCAAGCTGCATTTCGCACAG
ATGAAATTCGAAGGACTCCTCCTACCCCACAAGATGAGATGAGGGCAGGAATGAGCTACTTTCATGA
GACAATTTGGAAAGGTGTACCACAGTTTCTGCGTCGGGTAGATACAGCTCTGAAGAACATTGGAATT
AATGAACGTGTCCCATATAATGCTCCTGTTATTCAGTTCTCTTCTTGGATGGGAGGAGACCGTGATG
GTAATCCTAGAGTAACCCCTGAAGTTACAAGGGATGTGTGTTTGCTGGCTAGAATGATGGCTGCTAA
TATGTACTTCTCTCAGATAGAGGATCTCATGTTTGAGTTGTCTATGTGGCGTTGCACTGACGAGCTA
CGTGTTCGTGCTCATGAACTCCATAGGTCCTCAAAGAGAGATGCAAAACATTATATTGAGTTTTGGA
AACAGATTCCTCCAAATGAGCCATATCGTGTTATTCTTGGTGATGTCAGAGACAAACTATATAACAT
TCGGGAACGTGCTCGCCATTTATTAGCCAATGGGACATCTGATATCCCTGAGGAGACAACCTTCACT
AACGTTGAGCAGTTTCTGGAGCCCCTTGAACTATGCTACAGGTCACTCTGTGCATGTGGTGACAGAC
CAATAGCAGATGGAAGCCTCCTTGATTTCCTGCGGCAAGTTTCCACATTTGGACTCTCACTAGTGAG
ACTTGACATCCGTCAAGAGTCAGATAGGCACACTGATGTTATGGATGCAATCACAAAACACTTAGAC
ATTGGATCATACCGAGAGTGGCCCGAGGAAAAGAGGCAGGAGTGGCTCTTGTCTGAACTCAGTGGAA
AGCGCCCTCTCTTTGGCCATGACCTTCCCAAAACAGAAGAAATCACCGATGTTTTGGAAACATTCCG
TGTCATTTCAGAGCTTCCCTCAGACAACTTCGGTGCCTACATCATATCAATGGCAACATCCCCATCT
GATGTGCTTGCTGTTGAGCTTTTACAACGTGAATGCCATGTGAAGCAGCCACTAAGGGTGGTGCCAC
TGTTTGAAAAGCTTGCTGATCTTGAGGCCGCTCCGGCTGCGGTGGCACGTCTTTTCTCTATAGATTG
GTACAGAAACCGCATTGATGGGAAGCAAGAAGTTATGATAGGGTACTCAGACTCAGGAAAAGATGCA
GGCCGTCTTTCTGCGGCTTGGGCGCTTTACAAGGCTCAAGAAGAACTTGTGAAGGTTGCTAAGGAGT
ATGGTGTTAAACTTACAATGTTTCATGGGAGAGGAGGGACTGTTGGAAGAGGAGGAGGTCCAACTCA
TCTTGCTATATTGTCTCAGCCACCAGACACCATTCATGGCTCACTTCGGGTAACGGTTCAGGGTGAA
GTCATTGAGCAGTCTTTTGGAGAGGAACACTTGTGCTTTAGAACACTTCAGCGCTTCACTGCAGCTA
CACTTGAGCATGGCATGCATCCTCCTGTGTCACCGAAACCCGAATGGCGTGCCCTCATGGATGAAAT
GGCTGTCATTGCAACAAAGGAGTATCGCTCTGTTGTTTTCAAAGAACCTCGTTTTGTTGAATATTTC
AGATGTGCAACTCCTGAGTTGGAGTATGGAAGAATGAACATTGGCAGTCGTCCATCAAAGCGAAAGC
CAAGTGGAGGAATTGAATCACTACGTGCTATTCCTTGGATTTTTGCATGGACACAAACGAGGTTTCA
TTTGCCAGTGTGGCTTGGTTTTGGGTCAGCATTTAAGCATGTAGTTGAGAAAGATCCGAAGAATCTC
CAAATGCTTCAGGACATGTACAATCAATGGCCTTTCTTCAGGGTCACCCTGGACTTGGTTGAGATGG
TGTTTGCTAAGGGAGACCCGGGGATTGCAGCCCTATTTGACAAACTCCTAGTATCAGAAGAGCTGCG
TCCATTTGGAGAAAATTTAAGAGCTAAATACGAAGAAACCAAGAGCTTTCTCCTCCAGGTTGCTGGG
CACAAGGATATTCTTGAAGGAGACCCCTACTTGAAGCAAAGACTTCGTCTTCGTGACTCATACATCA
CAACCCTCAATGTGTTGCAAGCTTACACATTGAAGCGAATTCGTGATCCTGACTACCATGTGAAGTT
GAGGCCACACTTGTCAAAGGACTACATGGAATCAAGCAAGCCAGCAGCAGAGCTTGTTAAACTTAAC
CCCAAAAGCGAGTATGCTCCTGGTCTTGAGGACACCCTTATTTGACAATGAAGGGTATTGCTGCTG
GAATGCAAAATACCGGTTAA

**FIGURE 4 (continued)**

SEQ ID NO: 34, protein - Glycine max
MAARNIEKMASIDAQLRLLAPRKVSDDDKLVEYDALLLDRFLDILQDLHGEDIRQTVQDCYELSAEY
EGEHKPEKLEELGNMLTGLDAGDSIVIAKSFSHMLNLANLAEEVQIAYRRRIKLLKKGDFADENSAI
TESDIEETFKKLVAQLKKTPQEIFDALKNQTVDLVLTAHPTQSVRRSLLQKHGRIRNCLTQLYAKDI
TPDDKQELDEALQREIQAAFRTDEIRRTPPTPQDEMRAGMSYFHETIWKGVPQFLRRVDTALKNIGI
NERVPYNAPVIQFSSWMGGDRDGNPRVTPEVTRDVCLLARMMAANMYFSQIEDLMFELSMWRCTDEL
RVRAHELHRSSKRDAKHYIEFWKQIPPNEPYRVILGDVRDKLYNIRERARHLLANGTSDIPEETTFT
NVEQFLEPLELCYRSLCACGDRPIADGSLLDFLRQVSTFGLSLVRLDIRQESDRHTDVMDAITKHLD
IGSYREWPEEKRQEWLLSELSGKRPLFGHDLPKTEEITDVLETFRVISELPSDNFGAYIISMATSPS
DVLAVELLQRECHVKQPLRVVPLFEKLADLEAAPAAVARLFSIDWYRNRIDGKQEVMIGYSDSGKDA
GRLSAAWALYKAQEELVKVAKEYGVKLTMFHGRGGTVGRGGGPTHLAILSQPPDTIHGSLRVTVQGE
VIEQSFGEEHLCFRTLQRFTAATLEHGMHPPVSPKPEWRALMDEMAVIATKEYRSVVFKEPRFVEYF
RCATPELEYGRMNIGSRPSKRKPSGGIESLRAIPWIFAWTQTRFHLPVWLGFGSAFKHVVEKDPKNL
QMLQDMYNQWPFFRVTLDLVEMVFAKGDPGIAALFDKLLVSEELRPFGENLRAKYEETKSFLLQVAG
HKDILEGDPYLKQRLRLRDSYITTLNVLQAYTLKRIRDPDYHVKLRPHLSKDYMESSKPAAELVKLN
PKSEYAPGLEDTLILTMKGIAAGMQNTG

SEQ ID NO: 35, DNA - Saccharum officinarum
GATCCGTCACTGTCCTCATGTAATACTCACACCCTTTGGATACAAAAATACGAGCATACGGCTATGT
TTTCAAGAGTACAACAACTTCTTTGCCATGTCTACCATAGCTTGCCCAATATAAAAATCGAACATAT
CTCTCTAGAGCCAACCTCCTTCATTCATTATTCATGTCCATGGACAAACCACAAAATTCACTCGATT
TTCACTCTAGATGGTGTGATAAATCTTTATCTATCTATCTGTCTGTCTAGGTAGCCAGCTGGTTTTT
TTTTATCTGTCTGTCTATCTATCTATCTATTATTAAAGAATAAAACTTTACTTCCAACTCATTTTGA
TTTATTCCCAAAAAATACATGTCCTTTCTTTCGTCCCATTCTTTTTTTAAAATGTGTTGAATTTAAT
GCAACATACGGACACACTTGCTGACGAACAAAATTCCGATAAAACAAAGGTCTTGCTCTCCCGACGC
GACGGGCCGCCACCATGAAACCTGTAGGCGAGAGCCGAGAGGAGTAAGACTATCCGCACTCGTGGTA
CTCTAAATTCATCATCTAAAAGCAATATTTCATTATAGTCACTCCTCTATACCACTTTCTTTCGCAC
TCATCCATCCTCTGTAATCATCCTCTATACCAACTACCAGCTGATGGGGTCCACACGTCATACTCTT
ATTTTCTCTCTCCCTTCCCCTCTCCTCCCTCTTCTCTCTCCTCTATCTTCTTTTCCGGCTGCTTCCG
GTTGGGCGCAGCACACGAGGTGGGGCCGCCCTGGGCACGTAGCGGACTGCTGCGACATGCCAGGAAT
GGCGGACGATGAGTGGGTAGCGGTCGGTTTGCGTCCCCGGTGCAGCCGATTTGAGTCGCTATGTCCA
CGCTGTCCCTTTAGCGCTCGTGTATACGTCCCGGTGCGGACGGTCTTAACGCTCCACTAGGATGGGC
TTTCACCACTGGAGATGAAAACGAAACAGAAATATTCCGAATTGACTGTTTTCGTTTTCTATATTAT
GAACCGGTTTTTTAACATGAAAAATTGTCAGTTTCAATTGGTTTCACAATCATTTTCTACCATTTTT
TTGTATTCTTTAATTGCTAAATGTAGAAATATTCCGAACCGACCGTTTTTATTTTTCTATACTGTAA
GCTCGTTTTCGACCGTTTTGTTCCTGGTTCACCGCCACAGTCCGAGTTCGAAGGGATATACGACGAA
TCCATCCAGCACAAGCGACAGTCCCGTCCTGTCCTATAAATAGCAGACCACAGCAGAGAGAGAGAGA
GGGTGTCCCTCTCCGCTCCGTCACAGGAAACCCGGTATAGCATCAAGGAANGTTTTTTTGTCGTGTT
GATTGATCTCCCTCCCCTTTCGCCCCCGTCCTGCCTCCTGGCCTCACCCCAGAAAAAAAAAGGTGAG
ATGCTTTCCTCTCCTCTCCCTTCGATCCAGTAGGTTTGCAGTCTGTTTCCTAGATCTTGTTTAGGCA
TTTGTCCATCTTGGAGTGGCAAAGTCCCTAGCTCCCACAACGGCTGATTGCTGTAGGTTTTGATCAT
ACTTTTTTTTTTGCATTCGAGAAAGCCCCGTGCTTTGTTGGAATCTGCGTGCTTAGGGCTGGGTGGA
TCGTCTTCTCGCTTCAAGAACATGTCGAAATCCGTGGATTGGAGGGATTTGTGGGGTTTCTCTTCT
CTTTTGGTTGGAATTGTGGGTTTTGATCTGTGTGGATTCTGTTGCAGGGGGCGAGAAGGGAAGGGGA
GTGCTTGGGGGGGAGAGAAGTAGATGGCGCGCAATGCGGTGGACAAGGCGACGTCCATCGACGCGCA
GCTGCGGCTGCTGGCCCCGCAGAAGCTCTCCGACGATGACAAGCTTGTCGAGTACGACGCCCTGCTC
CTCGACCGGTTCCTCGACATCCTACAGGACCTGCACGGCGAGGACATCAGGGAGACGGTATGTAAAT
GCTCCAATTGTTTCCTGCCCGTGGTGCTATTTGTTCC

**FIGURE 4 (continued)**

TTTCTTTTCGGTGCTGGCAATTGATTTAGGTGGTAGATAGCTCGATCATGCTTGAAAATCTGTTTAT
ACTGTCATGCATGCATGGGTCGCGATAGCAGGAGAATCAGACACAGCATCTGGGCCTTGTCCGTGGG
TGTTTCTATATGGTGATATTCTTCTCATCTGTTTTGGTATTGCACTCTGGTGATGGAAACTAGTGCT
GCATTTAACAAATCATAGTAGCACTGGGGGTCCACCCATTTGTTCTACAGTTGCTGATCAATACTGT
GCTAGTTCCCACTTTGTGTTTTCTAGCTTTTGAAATTGGTACATCACCATGATGTTCAAAACTGGTG
GGGTTATCATGATGTGTGGAGCACTTGCACTATAATCATGGCCTTTTGATACTCTCGAGTTATTATG
CCCTTTGAACTATTGTTGTTGCTGATACGAGGTAGTTTGCAGATCAGATTTATTGTCTTTTCTATGC
ATAAATAGTATGATAAAATGGTAAAAGGCCACCTTTTTTACAATGCATAGACCTATCACGCTATTTT
ACATTATCATTTTACCACTCAAGTAAGTCCCATCTCCTTGCCCATTTTTTTTGTATATCTGCAAGCC
AATCTTTGCTTGACATTCTGATAGGTGGCTTGATTTCTGTGGTCATCCGTTACACAGCAATTCCAGT
GTGTTCACCAACTCACTCTACATTCGATAAAATTTATACTTTTTTGCAATGCCGTAAAGTTTCCCTT
TCTCATGAATAGGTGCAAGAATGCTATGAGTTAGCTGCTGAGTATGAAAATAAGCTTGACCCCAAAA
TGCTGGATGAGATTGGGAATGTGCTGACCAGCTTGGATCCTGGAGACTCCATTGTCATAACCAAGTC
ATTCTCACACATGCTTATCCTGGCAAACTTGGCTGAGGAGGTACAAATTGCATACCGCAGGAGAATA
AAACTGAAGAAGGGTGACTTTGTTGATGAGAACTCAGCAACAACTGAATCAGACATCGAGGAGACAT
TAAAGAGGCTTATGCATCAGCTTAAGAAGTCCCCTCTGGAGGTATTTGATGCTCTCAAGAATCAAAC
TGTTGACCTCGTCCTGACTGCACATCCAACTCAGTCAGTCCGGAGATCACTGCTCCAAAAGCATGGC
AGGTTAGTCTTGAAGCTTTCTGAATTGTTAGATCTCAGGATGAGATTTACTCGATGGAATTGAATAT
TTGCTATGCAGAAATGTTGGTTCTGTTTGTTCTTTATGCACATCAGAGCAATGCAATTTGCTCACAC
TGAATTTCACATACTGCTTTCATAGAAAACCTGATGTTTTTTTGTCCACTTTTCTTTTATGTGTAAA
TATTCCTTGCATGGTTTTGCTGTAGTCTGCCTTTTAAGAAACAACAGCAATAGAAAAAAAATAATATA
TTTCTCGAGGCATGTTAAATCATACGTTATATGTGCCTTTTGATGCCTTAGATTCCTCACCTTTCAA
TGTGCAGGATAAGAAACTGTTTAACTCAACTTTATGCAAAGGACATCACTCCAGATGAGAAACAGGA
ACTTGATGAAGCACTTCAAAGAGAGGTAATTTGCTTAGATATTTTGGTATATTAGTGTTGTATAATA
CTATCTGTGTAGTAGTTAATCAGGAAAACTTTGTCACCTTCACTAAATATATATTTATATGATTCTA
ATATCCTGTTCCATTTCTTATCTACAGATTCAAGCTGCCTTTAGAACAGATGAAATCAGGCGGGCGC
CTCCTACTCCGCAGGATGAAATGCGTGCTGGAATGAGTTATTTTCATGAGACTATATGGAAGGGTGT
ACCCAAGTTCTTACGTAGGGTTGACACTGCTCTTAAGAACATTGGCATAAATGAGCGGTTGCCATAT
AATGCCCCTATCATTCAGTTTTCTTCTTGGATGGGTGGTGATCGTGATGGTGCATGGCATTCTCTCT
CCTTTTTTTTGGATAGTATATTTGATGATGTGCCGAATCATGCAACTGTAGTTCACAAGAAAGAAGT
GCATATATAACTCTAAACTGATATCCACAACAGGGAATCCAAGAGTCACGCCAGAGATTACAAGGGA
TGTATGCTTGTTGGCTAGAATGATGGCTGCTAATTTGTATAATGCCCAGATAGAAGACCTAATGTTT
GAGGTAAAAATAAGGACACAGTTTTTATTTGATATTGTAGTGATATTAGTCTCACAAGAATTCTTTT
TATCGCTTGCAGTTATCTATGTGGCGCTGCAGTGACGAGTTACGTGTAAAGGTCGATGAGTTACACC
GATCCTCCAAGAAAGATACAACAAAACACTATATAGGTGCAGATTATATTATGATATTTTTGTTAAA
CTTATATATTATATCTTCAACATCCTTGCCTACCATCTGCTGCAAAATATTTAACCAAACTAGTCGC
CAATCTGTTCTTTCCAATGGATGAAATAGCTACACATTTTATAAAGTACCTCCATCCAGAAAAGAGT
GCAGTTCTAGTTCTACTCTTAGTCAAACAATCTAAAGTTTGACCAAGTTCATAGAAAAGAATATCAA
TAATTATGACACCAAATAGGTATACTATGAAAATATATTTCATGACAAATCTAATGATACTTATTTG
GTATCATAAATATTGATATTTTTTTGGCACAAAGTTAGTCAAACTTGAGATGGTTTGACTTAGTCAT
AAATATATTTGGTATCCAGAATTGCATTCTTTTCCGGATGAAGGGAGTATCTTACTTATGATTAATA
TATGGATCCTCATCATAACCATAAATGTGGTTCCACTATGCTGCTGAAGATTAATCTGAGACATGCA
GATCAGGATTCTAATCTAAATGGTTCTAACCCAAGTCAGCAATTGGGGAAAAAATAGGCATGGCAAA
TCCATATTCCAAAAGGGTTGGACTGTAAGAATCAGAAACAGTTAAAAGGGCAGACCCAGTGCCAGAG
GCTCCCACACGAGTGGGGTCTAGGGAAAGGAATAACCGAAGTAAAGTCTTACCTCCGCAGAAATTCT
ACAGAGAGAAACAGTTACCTTGCAAAAAAAAAGGCATTGAAGGATTTGAAACATGCAATAAAACTAA
TGTATTTAGCTGTTAGCTTAATAAATATGATCATGAAGTATTAAGAAATAGCTGTCATCAGGACTCA
GGAGTTGCATTGATATATG

**FIGURE 4 (continued)**

TGCTTTCTTGCCAACATAGGCTGGMATTTGAATCATGTTTCGTTCTCTTGATTTGTTTCTGAGACAC
GTGGATGTCTATTTCTGTCGTTGTACTATTTTAATATTGGAAAGAACCATGTCACTATGGAGTAATT
TTTTTAAAGACAAATTCTTGCTATTTTCATGCAGACTTTGATATTTAGGATGACATTTTTGGCAGCA
ACAGACATGATATTGATGCATGCTATTTTTGTCTATTCTTTTAGATTAATGCAGCCAATTAGACAAA
ACTAGGTGAAGTTAATACATGCTGACATGCTTTTCCATGCAGAGTTCTGGAAACAAGTTCCCCCCAG
TGAACCTTACCGTGTGATTCTGAGTGATGTCAGAGATAAACTCTACAACACGCGTGAACGAGCACGC
CATTTATTAGCTAGTGGGTTTTCTGAAATTCCAGAGGAAGCAACCTTCACCGATGTTGAACAGGTAT
GTAAAGTGAAAACAGTAGTGTTCATTTGATGTTCCTGGCTAGGATAAAAAATTAATCTGTACCATAC
ACTTTTAGTCCTATATTAAGATTGGATTCTTTCATTCTATGGTGTACTGTAAAAGGTTGAAACATTC
CCAAGATAAAGGATCTTTGTAGCTGTTTCAGGATATATTATGTTTTATGAATTCTTGTCTCGATTGA
ATCTTTTTTCTCTTGCAGTTCTTGGAGCCTCTTGAGCTCTGTTACAGATCCCTCTGTGCCTGTGGTG
ATCGCTCTGTTGCAGATGGAAGCCTTCTTGACTTCTTACGGCAAGTGTCTACGTTTGGACTATCCCT
TGTTAGACTTGATATCAGGCAAGAATCTGACCGACATACTGATGTTATGGATGCTATAACTGAATAC
CTCGGCATTGGATCATACCGTAAATGGACAGAGGAGAAACGCCAAGAATGGCTACTCTCGGAACTTA
ATGGAAAGAGGCCGTTATTTGGTCCTGACCTTCCCAAGTCAGATGAGATTGCTGATGTTCTAGATAC
TTTCCACGTATTAGCTGAACTTCCTTCAGACAGCTTTGGTGCATATGTAATATCTATGGCGACAGCT
CCCTCAGATGTTCTTGCTGTCGAGCTCTTGCAGCGTGAATGTCATGTGAAAAAACCACTGAGAGTTG
TGCCATTGTTCGAAAAACTGGCAGATCTTGAGGCAGCACCCGCAGCTTTAGCTCGGCTGTTCTCTGT
TGAATGGTACAGAAACAGGATCAATGGCAAGCAGGAAGTGATGATCGGGTATTCAGATTCTGGCAAG
GATGCTGGTCGTTTCTCTGCTGCTTGGCAACTGTACAAGGCTCAAGAGGAGCTAATTAATGTTGCTA
AGCTGTATGGGGTTAAGTTAACTATGTTCCACGGACGAGGTGGGACTGTGGGAAGAGGTGGGGGCCC
TACCCATCTTGCTATACTGTCACAACCTCCTGAAACTATTCATGGATCACTTAGGGTAACTGTTCAA
GGTGAAGTCATTGAGCAATCTTTTGGAGAGGAGCATTTGTGCTTTAGAACCCTGCAACGTTTCACAG
CTGCTACTCTTGAACATGGTATGCATCCTCCAATTTCGCCAAAACCGGAATGGCGTGCTTTAATGGA
TGAAATGGCTATTGTTGCCACAAAGGAATACCGATCAATTGTATTTGAAGAACCACGCTTTGTTGAA
TATTTCCGCCTTGTAAGTCTTCGGTTTTCTTTGCTTGTGTTTTCCTTTTGAACACACCTTTACTTGT
CTTTATATTGTTATTATGCAATTACCACTATGAGATTTTAATTGTAGCTTTTATTTAGGCCTTGGTA
TTTCACTCTTGTTTTTGTTAGTTGGGAATTCATCTAGCTAGTCCTGAAGATTAATATACTGTTTCAT
TTCGTTTGTTTTACTGTCATGTCATTACTCCTTGTAGTACCCTATAAATTTATTATTTTTTGGTAAT
GGGTCCATTTTTCTGCATAACTAATCCTGGCATTTGTGTCTTTCTTTCAAATAGGCAACACCAGAGA
TGGAATATGGTAGGATGAATATTGGAAGCAGACCATCGAAAAGAAAGCCAAGTGCAGGCATTGAATC
ACTGCGTGCAATCCCTTGGATCTTTGCTTGGACGCAGACTCGGTTCCACCTACCAGTGTGGCTTGGT
TTTGGAGCGGCATTCAAGCATGTCTTGGATAAGGACATACGGAATCTTCAAACCCTTCAAGAGATGT
ACAATCAGTGGCCATTTTTCAGGGTTACAATAGACTTGGTTGAGATGGTGTTTGCCAAAGGCGATCC
TGGTATAGCGGCTCTGTATGACAAGCTTCTAGTTTCAGAGGATTTGTGGTCATTCGGCAAGCGTCTT
AGAGCCAACTATGAAGAAACAAAGCAACTTCTTCTACAGGTGATTTGTCTAAGATCTTCTTGCATTC
GTTATGGACTGCTAGAGTTGCATGTAGACTATGTGATGCCTCTACCAGGCTTGAACATTGGTGCGTC
TGAATGAGTTGATAATTAAGTTAATCCGTAATCTATAGCCACATCATGCCCAACTATTTCCAGAGAA
TGTTTAGATACAAGCTCAAACTGGTAGATAAACCAAGGAGGAAATGGTTCTGGCAATAGGATGTCCA
TTCACCCCGTCTTGTTCTTGTCTCACCCACTGCATTGTGGCCTGCTGAATTTCAGGTTGCTGGGCAC
AAAGACCTTCTGGAGGGCGACCCTTACCTGAAGCAGAGGCTGCGCATCCGTGACTCCTACATCACTG
CCCTGAACGTGTGCCAAGCTTACATGCTGAAGCGCATCAGGGACCCTGGCTTCCAGGTGAACCCGGG
GCCTCACCTGTCGAAGGACATCATGGACATGGGCAAGCCGGCCTCGGAGCTTGTGAAGCTCAACACC
ACGAGCGAGTACGCCCCGGGACTGGAGGACACCCTCATCCTGACCATGAAGGGCATCGCTGCCGGAA
TGCAGAACACCGGCTGAAAGAACAGGATCCGGAGTGCCTTCTTTATTCTGAGCTGATGTATCTATCG
TAGCGTAGGGGATCATGGCGTGAACCGGTTAACAAGACGGGGGTGCACACCTGGATATGGGGGATTA
CCTGTTTTAGCAGTAGTAGTATTTACTTTTATGCTATGCTCTATCGAAACTCCTATGTATGCATATC
CATGTTGCTTGAGCCTGCA

**FIGURE 4 (continued)**

CTAGCTTTTGGATCATAGCTTGTTAAGTAATGCGCAGGGCATCTTGTTACGGTGAAATAACTCTGTG
CTTGTGCTTGGTGCTGGGCTGCTGGCATATTTGTGCCGAGGAGTCTACTGAATTGCATGGATCATTG
TACCTTTTCTGTCGTGCGGTATCTTGTCGAATACTACTGTCCTGGGCTAACGGACAGGCTGTAGAGA
CTAGATGCGCACGTCGTGTATCACGCACGCACAACGGCCCCCTGGAAACTGCGGAGGCCCGGAGCCC
TCGGACGTGCACGCGCAAACAGGGGCTTTGTCAAAATTCAATTTTTCTTCTAAT

SEQ ID NO: 36, protein - Saccharum officinarum
MARNAVDKATSIDAQLRLLAPQKLSDDDKLVEYDALLLDRFLDILQDLHGEDIRETVQECYELAAEY
ENKLDPKMLDEIGNVLTSLDPGDSIVITKSFSHMLILANLAEEVQIAYRRRIKLKKGDFVDENSATT
ESDIEETLKRLMHQLKKSPLEVFDALKNQTVDLVLTAHPTQSVRRSLLQKHGRIRNCLTQLYAKDIT
PDEKQELDEALQREIQAAFRTDEIRRAPPTPQDEMRAGMSYFHETIWKGVPKFLRRVDTALKNIGIN
ERLPYNAPIIQFSSWMGGDRDGNPRVTPEITRDVCLLARMMAANLYNAQIEDLMFELSMWRCSDELR
VKVDELHRSSKKDTTKHYIEFWKQVPPSEPYRVILSDVRDKLYNTRERARHLLASGFSEIPEEATFT
DVEQFLEPLELCYRSLCACGDRSVADGSLLDFLRQVSTFGLSLVRLDIRQESDRHTDVMDAITEYLG
IGSYRKWTEEKRQEWLLSELNGKRPLFGPDLPKSDEIADVLDTFHVLAELPSDSFGAYVISMATAPS
DVLAVELLQRECHVKKPLRVVPLFEKLADLEAAPAALARLFSVEWYRNRINGKQEVMIGYSDSGKDA
GRFSAAWQLYKAQEELINVAKLYGVKLTMFHGRGGTVGRGGGPTHLAILSQPPETIHGSLRVTVQGE
VIEQSFGEEHLCFRTLQRFTAATLEHGMHPPISPKPEWRALMDEMAIVATKEYRSIVFEEPRFVEYF
RLATPEMEYGRMNIGSRPSKRKPSAGIESLRAIPWIFAWTQTRFHLPVWLGFGAAFKHVLDKDIRNL
QTLQEMYNQWPFFRVTIDLVEMVFAKGDPGIAALYDKLLVSEDLWSFGKRLRANYEETKQLLLQVAG
HKDLLEGDPYLKQRLRIRDSYITALNVCQAYMLKRIRDPGFQVNPGPHLSKDIMDMGKPASELVKLN
TTSEYAPGLEDTLILTMKGIAAGMQNTG

SEQ ID NO: 37, DNA - Pinus abies
GCTTGTCCTAACAATAGAAACATGGCTTTGAGCATCTCAACCTGGATCTTGAAATAACTTCCGAGCC
CGAGATCATTGAAACTTTGAAGAAATCGTGTTGTTGAATTTTTATTGTTGCAGAGGAAAGATCAGAA
TTTTGAGATTTTGGCGAAGCGGAACTTGGATTTAGGAGAAATCTGTTGCAGAGATGGCGCGCAACAA
CTTGGAGAAGATGGCATCCATTGATGCCCAGATGAGGCTCTTGGTTCCTGGAAAAGTTTCTGAGGAT
GACAAGTTGATTGAGTACGATGCCCTTCTACTGGATCGCTTCCTTGACATCCTGCAGGACTTACATG
GAGAAGATATCAGGGCGATGGTTCAAGAATGCTATGAGCGTTCTGGTGAATATGAGGGAAAGAATGA
TCCTCACAAGCTGGAAGAGTTGGGAAATGTATTAACAAGTCTGGATCCTGGGGATTCAATTGTTGTG
GCCAGCTCATTTTCCCACATGCTTAATTTAGCTAATTTAGCTGAAGAAGTTCAGATTGCTTACCGGC
GCCGAAATAAAATAAAGAGGGGCGGCTTTGCAGATGAAAGCAATGCAACTACTGAATCAGACATTGA
AGAAACTTTTAAAAGGCTTGTAAACCAGTTAGGAAAATCACCGGCAGAGGTGTTTGATGCTCTTAAG
AATCAGACTGTTGATTTGGTGTTGACAGCACATCCAACACAGTCAGTCCGCAGATCATTGCTGCAGA
AGCATGCTAGGATTCGGAATTGCTTGTCTCAGTTATATGGGAAAGATATTACCCCTGATGAGAAGCA
GGAGCTTGATGAAGCTTTGCTAAGAGAGATTCAAGCTGCCTTCCGCACAGATGAAATTCGGCGTACT
CCTCCTACTCCACAAGATGAGATGCGAGCAGGAATGAGCTATTTTCATGAAACAATTTGGAAGGGTG
TCCCTAAGTTTTTACGCCGTATTGATACTGCCCTGAAGTCAATTGGGATCAATGAACGAGTGCCGTA
TAATGCACCTCTTATACAGTTTTCTTCATGGATGGGAGGTGATCGAGATGGAAATCCTAGGGTAACT
CCAGAAGTAACAAGAGATGTATGCTTACTTGCAAGAATGATGGCAGCAAATTTATATTATTCCCAGA
TAGAGGATCTTATGTTTGAGTTGTCCATGTGGCGTTGTAGTGATGAGCTGAGAGCACGAGCCCTACA
ACTCCATAGTGCATCAAAGAAAGATGCAAAGCATTACATAGAATTTTGGAAACAGATTCCTCCAAAT
GAACCTTTTAGGGTGATATTGGGAGATGTACGAGATAAATTGTACAATACTCGAGAACGTACTCGCC
AATTACTTTCTAATGGAATTTCTGACATACCAGAGGAAGTAACCTTTACAAATATTGACGAGTTTTT
GGAGCCACTTGAACTTTGTTACCGATCACTGTGTTCTACTGGGGACCAGCCAATTGCAGATGGCAGT
CTTCTTGATTTTATGCGTCAAGTTTCAACATTTGGTTTGTCATTTGTTAAGCTGGATATTAGACAGG
AATCTGACAGACACAGTGATGTTGCTGATGCAATTACAAGGCA

**FIGURE 4 (continued)**

TTTAGGCATTGGATCCTACAAAGAGTGGTCAGAGGAACAACGACAAGCATGGCTCTTGTCAGAACTG
CAAGGGAAACGTCCCTTGTTTGGGCCGGACCTCCCAAAGACAGATGAGGTTCGAGATGTTCTAGACA
CATTTCATGTAATATCTGAGCTTCCTGCTGACAATTTTGGAGCTTATATTATTTCAATGGCAACTGC
AGCATCAGATGTTTTAGTGGTTGAGTTATTACAACGGGAATGCCATGTGAAAAAACCATTACGTGTT
GTACCATTATTTGAGAAGCTTGCAGATCTAGAGGCTGCTCCTGCTGCTTTGGCTAGATTGTTTTCAA
TAAACTGGTACAGAAACAGAATTGATGGAAAGCAAGAAGTCATGATTGGTTACTCTGATTCTGGAAA
GGATGCTGGTAGGTTGAGTGCAGGTTGGGCTTTGTACAAAGCACAGGAAGACCTTATAAAGGTTGCT
AAAGAATTTGGTATTAAGTTGACAATGTTCCATGGTCGTGGAGGAACTGTAGGCAGGGGGGGAGGCC
CAACCCATCTGGCCATATTGTCACAACCTCCAGACACAATTCATGGCTCTTTTCGGGTCACTGTTCA
AGGGGAAGTCATAGAACAGTCATTTGGTGAGGAACATTTGTGTTTCAGAACTCTCCAACGATTTACT
GCTGCCACTCTTGAGCATGGAATGCGGCCTCCAGTTGCACCAAAGCCTGAGTGGCGTGAACTGATGG
ATGAAATGGCTGTTGTTGCTACGAAGGAGTACAGGTCAATTGTTTTCCAGGACCCAAGATTCGTTGA
ATATTTCCGTTCTGCAACACCAGAATTGGAGTATGGTCGAATGAACATCGGGAGTCGCCCCTCAAAA
AGGAAGCCAAGTGGGGGCATTGAGTCACTCCGTGCCATCCCATGGATATTTGCTTGGACACAAACTC
GATTTCATCTTCCTGTTTGGCTTGGATTTGGTGCAGCATTCAAGCATGTTATGGAGAAGGATATAAG
AAATCTCCATATGCTGCAGCAGATGTACAATGAATGGCCATTCTTTCGGGTTACAATTGATCTAATT
GAAATGGTTTTTGCAAAAGGTGATCCAGGGATAGCTGCTTTGTATGACAAACTACTGGTATCTGATG
ATTTGTGGGCCATTGGTGAAAAATTGAGAGCTAACTATGGTGAAACCAAAGATTTGCTACTGCAGGT
TGCTGGACATAAAGATCTACTTGAAGGTGATCCATACTTGAAACAGCGACTCAGACTTCGTGACTCA
TACATTACAACTCTGAATGTTTGTCAGGCTTATACTTTGAAAAGAATTAGAGACCCCAACTATCATG
TTAATCTTAGGCCTCATTTGTCGAAGGAAAGTTCAACCAAACCGGCAGCTGAATTGGTCAAACTGAA
TCCAACGAGTGAGTATGCACCAGGTCTGGAGGATACATTGATTCTAACCATGAAGGGCATAGCAGCT
GGTATGCAGAACACTGGTTAGAAGGATTTTGGAAACGGATCAGGCATTTTCTCATGTTAGGAGTCAG
GACTATAACGGAAGTTTCTTGAGACCTACTTCTGAGGGACCTTCTTCAGATTCCAGTATTTAAGGCA
GCTTCTAAATATGTCAAAGCACCAGCAAGAATGATCTGCCAGTGTAGATATGGTCATTTAATAAGCT
CGAGAAGAGAGCAAATGTTGGTTGTCAGTTGACAACTTTCAAGGATTTTATTATAACGAGTCTAGGA
TCATATATGATCTTAGGATCCCTAATATATATGAAAAGCCCTAAAACATGTAGTTTTGTGAACTGGA
CTTGAGGACCAGGACTCCTTTATATGCAAGGCTTGGGAAGTACAATGATTTAACTTGATGAATGCAT
TAATAATGATCTTTAGCATGCTGTTGTTTGTGGCAAAAAAAAAAAAAAAAAAAAAAAAAAAA

SEQ ID NO: 38, protein - Pinus abies
MARNNLEKMASIDAQMRLLVPGKVSEDDKLIEYDALLLDRFLDILQDLHGEDIRAMVQECYERSGEY
EGKNDPHKLEELGNVLTSLDPGDSIVVASSFSHMLNLANLAEEVQIAYRRRNKIKRGGFADESNATT
ESDIEETFKRLVNQLGKSPAEVFDALKNQTVDLVLTAHPTQSVRRSLLQKHARIRNCLSQLYGKDIT
PDEKQELDEALLREIQAAFRTDEIRRTPPTPQDEMRAGMSYFHETIWKGVPKFLRRIDTALKSIGIN
ERVPYNAPLIQFSSWMGGDRDGNPRVTPEVTRDVCLLARMMAANLYYSQIEDLMFELSMWRCSDELR
ARALQLHSASKKDAKHYIEFWKQIPPNEPFRVILGDVRDKLYNTRERTRQLLSNGISDIPEEVTFTN
IDEFLEPLELCYRSLCSTGDQPIADGSLLDFMRQVSTFGLSFVKLDIRQESDRHSDVADAITRHLGI
GSYKEWSEEQRQAWLLSELQGKRPLFGPDLPKTDEVRDVLDTFHVISELPADNFGAYIISMATAASD
VLVVELLQRECHVKKPLRVVPLFEKLADLEAAPAALARLFSINWYRNRIDGKQEVMIGYSDSGKDAG
RLSAGWALYKAQEDLIKVAKEFGIKLTMFHGRGGTVGRGGGPTHLAILSQPPDTIHGSFRVTVQGEV
IEQSFGEEHLCFRTLQRFTAATLEHGMRPPVAPKPEWRELMDEMAVVATKEYRSIVFQDPRFVEYFR
SATPELEYGRMNIGSRPSKRKPSGGIESLRAIPWIFAWTQTRFHLPVWLGFGAAFKHVMEKDIRNLH
MLQQMYNEWPFFRVTIDLIEMVFAKGDPGIAALYDKLLVSDDLWAIGEKLRANYGETKDLLLQVAGH
KDLLEGDPYLKQRLRLRDSYITTLNVCQAYTLKRIRDPNYHVNLRPHLSKESSTKPAAELVKLNPTS
EYAPGLEDTLILTMKGIAAGMQNTG

**FIGURE 4 (continued)**

SEQ ID NO: 39, DNA - Lotus japonicus
GGGTTTGTGAAGTTACAATGGCGAACAGGAACTTGGAGAAGATGGCATCCATTGATGCTCAGCTTCG
GCAATTGGTTCCTGCTAAAGTCAGCGAGGATGATAAGCTGGTTGAGTATGATGCTCTGCTACTGGAT
CGGTTCCTTGATATTCTCCAGGATTTACATGGGGAGGATCTGAGAGAAACTGTTCAAGAAGTATATG
AGCTTTCTGCTGAGTATGAAAGAAAGCATGAACCTCAGAGACTGGAAGTACTTGGAAATCTGATAAC
TAGTTTGGACGCTGGAGATTCCATTGTTGTTGCCAAGTCCTTTGCCCACATGCTTAATTTGGCAAAC
TTAGCTGAAGAGGTCCAGATTGCTCACCGCCGTAGGATCAAGTTGAAAAAGGGGGATTTCGCTGATG
AGGGCAATGCAACCACTGAATCAGATATTGAAGAAACTTTCAAGAAACTTGTAGGTGAAATGAAGAA
GTCTCCTCAGGAAGTTTTTGATGAACTGAAAAATCAGACTGTTGATCTGGTTCTTACTGCTCATCCT
ACTCAATCAGTTCGTAGATCTTTGCTGCAGAAGCATGGAAGGATACGAAATTGCTTAACTCAATTGT
ATGCCAAAGACATCACTCCTGATGATAAGCAGGAGCTTGATGAGGCTCTACAGAGAGAGATCCAAGC
TGCTTTCCGTACTGATGAAATCAGGAGAACCGCTCCAACACCACAAGATGAAATGAGAGCAGGGATG
AGCTACTTTCATGAAACAATTTGGAAGGGTGTGCCCAAATTTCTGCGTCGTGTTGATACAGCTTTGA
AGAATATAGGGATTAATGAGCGTGTCCCGTATAATGCTCCACTTATTCAATTTTCTTCTTGGATGGG
TGGTGATCGTGATGGCAATCCAAGAGTAACTCCTGAAGTGACAAGGGATGTTTGCTTATTGGCTAGA
ATGATGGCTGCTAATTTGTATTATTCACAGATTGAGGATCTTATGTTTGAACTGTCCATGTGGCGCT
GCAATGATGAGCTGCGTGTCCGGGCTGAGGAACTTACCAGTTCTTCCAATAAAGATGCAGTTGCAAA
ACACTACATAGAGTTTTGGAAAAACATTCCTCCAAGTGAACCGTATCGTGTGGTACTGGGTGAAGTA
AGGAACAGACTCTACCAGACTCGTGAGCGTTCTCGCCATTTGCTTGCTAATGAGTACTCTGACATTC
TAGAGGAACACACTTTCACTAATGTGGAGGAGTTCCTGGAACCCCTCGAGCTCTGTTATAGATCACT
TTGTGCCTGTGGTGATCGGGCAATTGCTGATGGAAGCCTTCTAGATTTTTTGAGGCAAGTCTCTACT
TTTGGACTGTCCCTAGTTAGGCTTGATATTAGGCAAGAGTCCGACCGTCTTACTGATGTGCTGGATG
CCATCACCAAACATTTGGGAATCGGCTCCTACCTGGAATGGTCTGAAGAAAAGCGGCAGCAATGGCT
TTTGTCCGAGTTGAGTGGCAAACGCCCTCTCTTTGGACCTGATCTTCCCCAAACTGAAGAGATCAAA
GATGTTCTCGACACATTTCATGTCATAGCAGAACTTCCATCTGATAACTTTGGAGCATATATCATAT
CAATGGCAACTGCACCATCTGATGTGCTTGCAGTTGAACTCCTGCAACGTGAATGCCATGTCAAGAA
TCCGTTAAGAGTCGTCCCATTGTTTGAGAAGCTTGCTGATCTGGAAACAGCTCCCGCTGCTTTGGCT
CGGTTGTTCTCCGTTGAGTGGTACCGAAACCGGATCAACGGGAAACAAGAAGTCATGATTGGCTATT
CTGATTCAGGTAAAGATGCTGGAAGGTTCTCTGCAGCGTGGCAGCTATATAAGGCTCAAGAAGAGCT
TATTAAGGTAGCTAAGGAATATGGTGTTAAGCTAACAATGTTCCATGGTCGTGGAGGGACTGTCGGC
AGAGGAGGCGGTCCTACTCACCTTGCTATCCTGTCTCAGCCTCCAGACACAATCCATGGATCACTCC
GTGTTACTGTCCAAGGAGAAGTTATCGAGCAGTCATTTGGAGAGGAGCACTTGTGTTTCAGAACCCT
TCAACGTTACACTGCAGCTACTCTAGAACATGGAATGCATCCCCCAATTTCCCCCAAACCAGAATGG
CGTGCTTTGATGGACGAGATGGCTGTTATTGCCACTGAGGAATACCGTTCTATTGTATTCAAAGAAC
CCCGTTTTGTTGAGTATTTCCGCCTGGCTACACCGGAGTTGGAGTATGGCCGAATGAATATTGGAAG
TCGGCCGGCAAAGAGAAAGCCTAGTGGAGGCATTGAGACGCTCCGCGCTATACCTTGGATCTTTGCC
TGGACACAGACAAGGTTTCACCTTCCAGTGTGGCTAGGGTTTGGGGCTGCATTCAAACATGTGATTG
CAAAGGATATCAGAAATCTTAACATGCTGCAAGAGATGTACAATCAATGGCCTTTCTTTAGGGTCAC
TATTGATTTAGTGGAAATGGTGTTCGCCAAGGGAGACCCCGGTATAGCTGCTTTGTATGACAGGCTC
CTTGTTTCAGAAGATTTGTGGTCATTTGGGGAACAGTTGAGGACCATGTTTGAAGAAACTAAGCAAC
TGCTACTTCAGGTGGCTGCACACAAGGATCTTCTTGAAGGTGATCCTTACTTGAAACAAAGACTCCG
GTTGCGTGATTCTTACATCACTACCCTCAATGTGTGCCAAGCTTACACATTGAAGCGAATCCGCGAT
CCGAACTATAATGTGAAGCTACGCCCCCACATCTCTAAAGAGGCTATAGACGTGAGTAAACCTGCGG
ATGAACTTGTTACTCTGAACCCAACAAGTGAATATGCACCTGGTTTGGAAGACACCCTCATTCTCAC
CATGAAGGGTATTGCTGCCGGCATGCAAAACACTGGTTAAATCTGAGAGATTCTACTTCTGTTTTCT
GTTCTCCTGTTTTATCAAAATAATGAATTTAGATACGACTCACCCAAAGAGGGTGTTTGTTTTCCTT
TTCCCAGTTGAAATATCATTTGATATAAATTATGTTTCCATGCTTTGCTATATAAAGCATCGTTTGG
CTGCTNTGAAACAATGATTAATGCCAGTATGCAACTGATTATGTTTTAACTGAAAAAAAAAAAAAAA
AAAAAAAAA

**FIGURE 4 (continued)**

SEQ ID NO: 40, protein - Lotus japonicus
MANRNLEKMASIDAQLRQLVPAKVSEDDKLVEYDALLLDRFLDILQDLHGEDLRETVQEVYELSAEY
ERKHEPQRLEVLGNLITSLDAGDSIVVAKSFAHMLNLANLAEEVQIAHRRRIKLKKGDFADEGNATT
ESDIEETFKKLVGEMKKSPQEVFDELKNQTVDLVLTAHPTQSVRRSLLQKHGRIRNCLTQLYAKDIT
PDDKQELDEALQREIQAAFRTDEIRRTAPTPQDEMRAGMSYFHETIWKGVPKFLRRVDTALKNIGIN
ERVPYNAPLIQFSSWMGGDRDGNPRVTPEVTRDVCLLARMMAANLYYSQIEDLMFELSMWRCNDELR
VRAEELTSSSNKDAVAKHYIEFWKNIPPSEPYRVVLGEVRNRLYQTRERSRHLLANEYSDILEEHTF
TNVEEFLEPLELCYRSLCACGDRAIADGSLLDFLRQVSTFGLSLVRLDIRQESDRLTDVLDAITKHL
GIGSYLEWSEEKRQQWLLSELSGKRPLFGPDLPQTEEIKDVLDTFHVIAELPSDNFGAYIISMATAP
SDVLAVELLQRECHVKNPLRVVPLFEKLADLETAPAALARLFSVEWYRNRINGKQEVMIGYSDSGKD
AGRFSAAWQLYKAQEELIKVAKEYGVKLTMFHGRGGTVGRGGGPTHLAILSQPPDTIHGSLRVTVQG
EVIEQSFGEEHLCFRTLQRYTAATLEHGMHPPISPKPEWRALMDEMAVIATEEYRSIVFKEPRFVEY
FRLATPELEYGRMNIGSRPAKRKPSGGIETLRAIPWIFAWTQTRFHLPVWLGFGAAFKHVIAKDIRN
LNMLQEMYNQWPFFRVTIDLVEMVFAKGDPGIAALYDRLLVSEDLWSFGEQLRTMFEETKQLLLQVA
AHKDLLEGDPYLKQRLRLRDSYITTLNVCQAYTLKRIRDPNYNVKLRPHISKEAIDVSKPADELVTL
NPTSEYAPGLEDTLILTMKGIAAGMQNTG

SEQ ID NO: 41, DNA - Nicotiana tabacum
AAAAATTGTCCACTTGTAGGAAAGGAGATTATTGGGGTTGTTGATAGTGTGAAAAATGGCGACACGG
AGTTTGGAGAAATTGGCATCAATTGATGCACAGTTAAGGGCATTGGTTCCTGGCAAAGTTTCTGAGG
ATGATAAGTTGGTTGAGTATGATGCTTTGCTTTTGGATCGGTTTCTTGATATTCTTCAGGATTTACA
TGGGGAAGATCTTAAGGAAACGGTCCAAGAATGTTATGAACTTTCTGCGGAATACGAAGGCAAGCAC
GATCCAAAGAAGCTGGAGGAGCTTGGAAATGTGTTGACAAGTTTGGATCCAGGGGATTCCATTGTCA
TTGCTAAAGCTTTCTCTCACATGCTTAATTTGGCCAACTTGGCCGAGGAGGTGCAGATCGCCTACCG
TCGACGACAAAAGTTGAAAAGGGGGGACTTTGCTGATGAGAACAATGCAACAACTGAATCAGATATT
GAAGAAACTTTCAAGAAACTTGTAGGGGACTTGAAAAAGTCTCCTCAAGAAGTTTTCGATGCTCTGA
AAAATCAGACTGTGGATTTAGTCTTAACTGCTCATCCTACTCAATCTGTCCGAAGATCTTTGCTTCA
AAAGCACGGAAGGATCCGAGATTGCTTGGCTCAGTTGTATGCTAAAGACATTACACCCGATGATAAA
CAAGAGCTCGATGAGGCTTTACAGAGGGAGATTCAAGCTGCTTTCCGCACTGATGAAATTCGGAGGA
CTGCTCCAACTCCACAAGATGAAATGAGAGCTGGAATGAGCTACTTTCATGAAACTATTTGGAAGGG
TGTTCCAAAGTTCCTTCGTCGTGTTGATACAGCTCTTAAAAACATAGGGATTAACGAACGACTTCCT
TACAATGCTCCTCTTATTCAATTCTCCTCTTGGATGGGTGGTGACCGGGATGGCAATCCAAGAGTGA
CTCTTGAGGTCACAAGAGATGTCTGCTTACTAGCAAGAATGATGGCAGCGAATTTGTACTATTCGCA
AATAGAGGAACTCATGTTTGAGTTATCTATGTGGCGTTGCAATGATGACCTTCGTATTCGAGCAGCT
GAACTTTACAGGTCCTCAAGGAGGGACACTAAGCACTACATAGAATTTTGGAAAACAATTCCGCCAA
GTGAACCATACCGTGTAATTCTTGGCGATGTGAGAGATAAGCTGTATCAGACACGTGAGCGTACTCG
CCAAATGTTAGCCCATGGAATCTCTGATATTCCTGAGGATGCAACTTATAATAATGTTGAGCAGTTC
TTGGAACCTCTTGAACTCTGCTACAGATCTCTTTGTGAATGTGGTGATCGCCCCATTGCCGATGGAA
GCCTTCTGGATTTTCTTAGACAAGTTTCTACCTTTGGACTCTCATTTGTTAGACTTGACATAAGACA
AGAGTCAGACCGCCACACTGACGTCCTTGATGCCATTACCCAGCACTTGGAAATCGGTTCATATCGA
GAGTGGTCTGAAGAACGTAGACAGGAGTGGCTTCTTTCTGAACTCAGCGGCAAGAGACCTTTATTTG
GACCTGATCTTCCAAGAACCGAAGAAATTGCTGACGTCTTGGATACGCTCCATGTCATAGCAGAACT
TCCATCTGACTGCTTCGGGGCATATATCATCTCAATGGCCACTGCACCATCTGACGTGCTTGCAGTT
GAGCTCCTACAGCGTGAATGCCATGTGAAGCAACCTTTACGAGTGGTTCCACTTTTCGAAAAGTTGG
ATGATCTGGAGTCTGCGTCTGCGGCTGTTGCACGTCTCTTTTCGATTGAGTGGTACAGAAACCGGAT
TAACGGTAAACAAGAGGTCATGGTCGGGTATTCAGACTCTGGCAAGGATGCTGGTCGTTTCTCAGCA
GCGTGGCAGCTATATAAGGCTCAAGAGGAGCTTATAAAAGTTGCCAAAGAACATGGTGTGAAGCTAA
CTATGTTCCACGGTAGAGGTGGTACAGTAGGAAGAGG

**FIGURE 4 (continued)**

AGGTGGCCCCACCCATCTTGCTATATTGTCTCAGCCACCAGATACAATTCAGGGATCTCTCCGTGTC
ACAGTTCAGGGTGAAGTTATCGAGCAATCGTTTGGGGAGGAACACTTGTGTTTTAGGACTCTCCAAC
GTTTCACTGCTGCTACCCTTGAACATGGGATGCATCCACCCGTCTCTCCCAAACCAGAATGGCGTGC
ACTTATGGATGAAATCGCGGTTATTGCTACAGAGAAGTATAGGTCAATAGTTTTCAAAGAACCTCGA
TTTGTTGAGTATTCCGCCTTGGCCACACCTGAGTTAGAGTATGGTCGAATGAACATTGGAAGCCGTC
CATCAAAGCGTAAACCTAGTGGAGGAATAGAATCACTCAGAGCTATTCCATGGATCTTTGCCTGGAC
GCAAACTAGGTTTCATCTCCCCGTCTGGCTTGGCTTTGGGGCGGCATTTAAGTATGCCATTGACAAG
GATATAAAAAACCTTCGCATGTTTCACGAAATGTACAATGAATGGCCATTCTTTAGAGTCACTATTG
ACTTGGTTGAGATGGTGTTCGCCAAGGGAAACCCCGGTATTGCAGCTTTGTATGACAAGCTTCTCGT
TTCAGAAGATTTGTTGCCTTTCGGCGAGCTTTTGAGGTCCAACTACGAGGAGACAAGGAGCCTCCTA
CTCCAGATTGCTGGACACAAGGATCTTCTGGAGGGAGACCCCTACTTGAAACAACGACTCAGGCTGC
GTGATTCCTACATCACAACCTTAAACTTGTTGCAAGCCTACACTCTCAAGCGTATACGTGATCCTAA
CTATCATGTCACGCTAAGGCCTCATATTTCGAAGGATTACATGGAATCGAAGTCAGCTGCTGAACTT
GTGCAGCTGAACCCGACAAGTGAATATGCCCCCGGCTTGGAGGACACACTCATCTTGACAATGAAGG
GTATTGCTGCTGGACTGCAGAATACCGGTTAAACTCATTCTGATGTTCGTGTTTTTTCTAATTTTTT
ATATACTCGAAAAGCATCATTCTGGTTTATGAGAGGTAGATGTTTTTCATTTCTGTTGTGTTTTGTG
GTGGTGAAAGTTTTTTATTCCACCCTATATATCATGTTTGATAAAACTAATAAGACTCCATTTGAGT
GAGGTTTACTTCACCAAAATGAACTGTAATCTCATTGTTGGCTCAATGGAATATCAAGTTTCTTAGA
TCA

SEQ ID NO: 42, protein - Nicotiana tabacum
MATRSLEKLASIDAQLRALVPGKVSEDDKLVEYDALLLDRFLDILQDLHGEDLKETVQECYELSAEY
EGKHDPKKLEELGNVLTSLDPGDSIVIAKAFSHMLNLANLAEEVQIAYRRRQKLKRGDFADENNATT
ESDIEETFKKLVGDLKKSPQEVFDALKNQTVDLVLTAHPTQSVRRSLLQKHGRIRDCLAQLYAKDIT
PDDKQELDEALQREIQAAFRTDEIRRTAPTPQDEMRAGMSYFHETIWKGVPKFLRRVDTALKNIGIN
ERLPYNAPLIQFSSWMGGDRDGNPRVTLEVTRDVCLLARMMAANLYYSQIEELMFELSMWRCNDDLR
IRAAELYRSSRRDTKHYIEFWKTIPPSEPYRVILGDVRDKLYQTRERTRQMLAHGISDIPEDATYNN
VEQFLEPLELCYRSLCECGDRPIADGSLLDFLRQVSTFGLSFVRLDIRQESDRHTDVLDAITQHLEI
GSYREWSEERRQEWLLSELSGKRPLFGPDLPRTEEIADVLDTLHVIAELPSDCFGAYIISMATAPSD
VLAVELLQRECHVKQPLRVVPLFEKLDDLESASAAVARLFSIEWYRNRINGKQEVMVGYSDSGKDAG
RFSAAWQLYKAQEELIKVAKEHGVKLTMFHGRGGTVGRGGGPTHLAILSQPPDTIQGSLRVTVQGEV
IEQSFGEEHLCFRTLQRFTAATLEHGMHPPVSPKPEWRALMDEIAVIATEKYRSIVFKEPRFVEYSA
LATPELEYGRMNIGSRPSKRKPSGGIESLRAIPWIFAWTQTRFHLPVWLGFGAAFKYAIDKDIKNLR
MFHEMYNEWPFFRVTIDLVEMVFAKGNPGIAALYDKLLVSEDLLPFGELLRSNYEETRSLLLQIAGH
KDLLEGDPYLKQRLRLRDSYITTLNLLQAYTLKRIRDPNYHVTLRPHISKDYMESKSAAELVQLNPT
SEYAPGLEDTLILTMKGIAAGLQNTG

SEQ ID NO: 43, DNA - Glycine max
TTTTAGGTGAGGGACTGAAAGGTAGCAATGGCGACCAGGAACTTGGAAAAGATGGCATCCATTGATG
CACAGCTTAGGCAATTGGCTCCTGCCAAAGTGAGTGAGGATGACAAACTGATTGAGTATGATGCTCT
TCTGCTGGATCGGTTCCTTGATATCCTTCAAGATTTACATGGGGAGGATCTGAAGGAAACAGTTCAA
GAAGTGTATGAACTTTCAGCTGAGTATGAAGGAAAGCATGACCCTAAGAAACTGGAAGAACTTGGAA
ATTTGATAACCAGTTTGGATGCTGGGGACTCTATTCTTGTTGCCAAGTCCTTTTCCCACATGCTTAA
TTTGGCCAACTTGGCTGAAGAGGTCCAGATTTCTCGCCGCCGAAGAAACAAGTTGAAGAAAGGGGAT
TTTGCAGATGAGAACAATGCAACTACAGAATCAGACATTGAAGAAACTCTCAAGAAACTTGTATTTG
ACTTGAAGAAGTCTCCTCAGGAAGTTTTTGATGCACTGAAAAACCAGACTGTTGATTTGGTTCTTAC
TGCTCATCCTACTCAATCAATTCGTAGATCTTTGCTTCAAAAGCATGGAAGGATAAGGAATTGTTTA
TCTCAATTGTATGCCAAAGACATTACTCCTGATGATAAGCAGGAGCTTGATGAGGCT

**FIGURE 4 (continued)**

CTACAAAGGGAGATTCAAGCTGCCTTCCGTACAGATGAAATCAGGAGGACCCCTCCAACACCACAAG
ATGAGATGAGAGCAGGGATGAGCTACTTCCATGAAACAATTTGGAACGGTGTTCCCAGATTTCTGCG
CCGTGTAGACACAGCTTTGAACAATATCGGGATTAAAGAGCGTGTTCCTTATAATGCTCCCCTTATT
CAATTTTCTTCTTGGATGGGGGGTGATCGCGATGGTAATCCAAGAGTAACTCCTGAAGTGACAAGGG
ATGTTTGCTTATTGGCTAGAATGATGGCTGCTAATTTGTATTATTCCCAGATAGAAGATCTTATGTT
TGAGCTCTCTATGTGGCGCTGCAATGATGAACTACGCGTTCGTGCAGAAGAACTTCACAGGTCTTCC
AAGAAAGATGAAGTTGCAAAACACTATATAGAATTTTGGAAAAAGGTTCCCCCAAATGAACCATATC
GTGTGGTACTCGGTGAAGTAAGGGATAGGCTCTATCAGACTCGTGAGCGTTCTCGCCATTTGCTTTC
TAATGGGTACTCTGACATTCCAGAGGAAGCCACTTTCACCAATGTTGAGGAGTTCCTGGAATCTCTT
GAACTATGTTACAGATCACTATGTGCTTGTGGTGATAGAGCAATTGCTGATGGAAGCCTTCTTGATT
TCATGAGACAAGTCTCCACTTTTGGACTGTCACTAGTGAGGCTTGATATCAGGCAAGAGTCAGATCG
TCACACTGATGTGCTGGATGCCATTACCAAACACTTGGAAATTGGCTCGTACCAGGAATGGTCTGAA
GAGAAAAGACAGGAATGGTTGTTGTCTGAGTTAAGTGGCAAAAGGCCTCTATTTGGACCTGACCTTC
CTCAAACTGAAGAAATTAGAGATGTTTTGGACACATTTCATGTCATAGCAGAACTACCACCAGACAA
CTTTGGAGCCTATATCATATCAATGGCAACTGCACCATCTGATGTGCTTGCAGTTGAGCTTCTACAA
CGTGAATGTCACATCAAGCATCCCTTAAGAGTTGTGCCATTGTTTGAGAAGCTAGCTGATCTAGAGG
CTGCTCCTGCTGCTCTGGCACGGTTGTTCTCGATAGACTGGTACAGAAATAGGATCAATGGGAAGCA
AGAAGTGATGATTGGATACTCAGATTCAGGGAAAGATGCTGGGAGGTTCTCTGCAGCATGGCAGCTA
TATAAGGCTCAGGAGGAACTTATAAATGTTGCCAAGAAATTTGGTGTTAAGCTAACCATGTTCCATG
GTCGCGGTGGAACTGTTGGAAGAGGAGGTGGACCTACTCATCTTGCTATTCTGTCTCAACCTCCAGA
CACAATCCATGGATCACTTCGTGTGACAGTCCAAGGTGAAGTCATTGAGCAATCATTTGGAGAACAA
CACTTGTGCTTTAGAACACTACAACGTTTCACTGCCGCCACTCTAGAACATGGCATGCACCCCCCAA
TTTCGCCAAAACCAGAATGGCGTGCTTTGATGGATCAGATGGCTGTCATTGCTACTGAGGAATACCG
TTCCATTGTATTCAAGGAACCACGCTTTGTTGAGTATTTCCGCCTGGCTACACCAGAGTTGGAGTAT
GGAAGGATGAATATTGGAAGTCGACCAGCAAAGAGAAGACCTAGTGGAGGCATTGAAACACTGCGTG
CAATACCTTGGATTTTTGCATGGACTCAGACAAGGTTTCATCTTCCAGTGTGGCTAGGCTTTGGAGC
AGCATTTAAAAAAGTCATTGAGGAAAATGTTAAGAATCTCAATATGCTGCAAGAGATGTACAATCAA
TGGCCTTTCTTTAGGGTCACACTTGATTTGGTGGAAATGGTGTTTGCCAAAGGAGATCCGAAAATTG
CCGCTCTGAATGATAGACTCCTTGTTTCAAAGGATCTGTGGCCGTTTGGGGATCAATTGAGGAACAA
ATATGAAGAAACTAGGAAACTCCTACTTCAGGTGGCTGGACACAAGGAAATTCTTGAAGGGGACCCT
TACTTGAAGCAAAGACTCAGGCTTCGTCATGCTCCCATTACCACCCTCAATATTGTCCAAGCTTACA
CATTGAAACGTATCCGTGATCCTAACTACAATGTGAAGGTGCGCCCCCGCATATCAAAGGAATCTGC
AGAGGCAAGCAAATCAGCTGATGAACTTGTCAAATTGAACCCAACAAGTGAATATGCCCCTGGTTTG
GAAGACACACTCATTCTCACTATGAAGGGTATTGCTGCTGGCATGCAGAACACTGGTTAAGTTTGTT
TGAATACTGAAAGTGAGTGCTCTGAGTTTTGCTCACAGAATACACTTTTTTTGTTTTGTTATTATGT
TGCCCCACCTTTCTGTACAATTTGGTATGCTGCTCTAGAATTC

SEQ ID NO: 44, protein - Glycine max
MATRNLEKMASIDAQLRQLAPAKVSEDDKLIEYDALLLDRFLDILQDLHGEDLKETVQEVYELSAEY
EGKHDPKKLEELGNLITSLDAGDSILVAKSFSHMLNLANLAEEVQISRRRRNKLKKGDFADENNATT
ESDIEETLKKLVFDLKKSPQEVFDALKNQTVDLVLTAHPTQSIRRSLLQKHGRIRNCLSQLYAKDIT
PDDKQELDEALQREIQAAFRTDEIRRTPPTPQDEMRAGMSYFHETIWNGVPRFLRRVDTALNNIGIK
ERVPYNAPLIQFSSWMGGDRDGNPRVTPEVTRDVCLLARMMAANLYYSQIEDLMFELSMWRCNDELR
VRAEELHRSSKKDEVAKHYIEFWKKVPPNEPYRVVLGEVRDRLYQTRERSRHLLSNGYSDIPEEATF
TNVEEFLESLELCYRSLCACGDRAIADGSLLDFMRQVSTFGLSLVRLDIRQESDRHTDVLDAITKHL
EIGSYQEWSEEKRQEWLLSELSGKRPLFGPDLPQTEEIRDVLDTFHVIAELPPDNFGAYIISMATAP
SDVLAVELLQRECHIKHPLRVVPLFEKLADLEAAPAALARLFSIDWYRNRINGKQEVM

## FIGURE 4 (continued)

IGYSDSGKDAGRFSAAWQLYKAQEELINVAKKFGVKLTMFHGRGGTVGRGGGPTHLAILSQPPDTIH
GSLRVTVQGEVIEQSFGEQHLCFRTLQRFTAATLEHGMHPPISPKPEWRALMDQMAVIATEEYRSIV
FKEPRFVEYFRLATPELEYGRMNIGSRPAKRRPSGGIETLRAIPWIFAWTQTRFHLPVWLGFGAAFK
KVIEENVKNLNMLQEMYNQWPFFRVTLDLVEMVFAKGDPKIAALNDRLLVSKDLWPFGDQLRNKYEE
TRKLLLQVAGHKEILEGDPYLKQRLRLRHAPITTLNIVQAYTLKRIRDPNYNVKVRPRISKESAEAS
KSADELVKLNPTSEYAPGLEDTLILTMKGIAAGMQNTG

SEQ ID NO: 45, DNA - Solanum tuberosum
CTATACCTTATTGAGTGTCCAAAGTAAAGACTTTTTTTTCTCATCTTTTGTGTTCTGACATACCAAA
ATTCAGGTACTGAACATTATTTATGTTCTCCAATTTGCCATACTCAAAATTTCAATCTCTTGTTTTT
TTTGTTTTTTTTTGCTAACAAATAAATCAACAAGAATATAAAACTTAGTAGAGAGTTGCCAACTGGG
GTTTTTGTTTGTTTTGAATTGCCAGAACAAGAGAAAAAAAAGTGGTCCCTTTTTTGTACTTTTCTTG
GGGTCATGATCAGATATTGTGGTCACTGTAATTTGTAAAAAAAATTTGATGATTTTTATGTTTATGT
CTATTGCTGCAGAGTGTTGACTGTTGAGTGAGAAATGACTACGAGGAATTTGGAGAAACTTGCATCG
ATTGATGCACAGTTGAGGCAATTGGTGCCTGCTAAAGTTTCTGAAGATGATAAACTTGTTGAGTATG
ATGCTTTGCTTTTGGATAGGTTTCTTGATATTCTTCAAGATTTACATGGGGAGGATCTCAAAGAAAC
AGTATGCATTTTTCCCAATTTATGTTTGCTGTTAATGTAACCTATGAATGATTTAGACAATTGTAAA
TTATCTACCTAAGTTGTAGTCTTTTATGTTGTGTACCCTTTGATGAAGTTCTGTGATTTTTGTCTTT
TGGTTACTAAGGGGTTGTTGTTTTTGTCTTCCACTTTTAATATGGTTTATTTCTTGAAGTTAAAGAT
TGGATTTTTATGTCACTTGTAAAAAAGGGGAATAAAAAGATTGGATTTTTATGTCTGTTCTGACTGA
GTTGACTTCAAACACTTGAATTGGTTACTAAAGGGTGTTGTTTTTGTCTTCCACTTTTAATCTGGTC
TATTTCTTGAAGTAAAAAAGATTGAATTTTTATGTCACTTACAAAAAAAGGAACAAAAAGATTGGAT
TTTTATGTCTGTTCTGACTGAGTGAACTACAAACATTTGATTCTGAGGTGTGTGTAATACTTAGAGA
TGGTATCGATATAATGGTATTCATATGCTTCAGTATAGTTTGACTGTGTTAGTGGATGAAATCCCAT
GTGTATGTTGACTTGAGATCCTAAACTTGTGTTGACCAAGTAGTCTTGTTTTTTCGGAATGTTTTGT
CGTGTTTTCTTTAGTATTTGCCAGATTTCTTCACTTCTGTTATTTCTTTTTCGATCTTATTTTCCTT
AAGCTGAGGGTATATCAGAAACAGTCTCTCTACCTCTCAAGGTAGGGGTAAGGTCTTGGTACACTCT
ACCCTCCCCAGACCCTACTTGTGGAATTACACTGAGTATGTTGTTGTAATTGCAAAAGACTGGTCAA
CATTTAAGTCTTTGATATTTTCAATCTCACATTAGGTCCAAGAGTGTTATGAGCTTTCTGCCGAGTA
TGAAGCCAAGCATGATCCGAAGAAGCTGGAGGAACTTGGCAATGTGTTGACAAGTTTGGACCCAGGA
GATTCAATTGTCATTGCTAAAGCTTTCTCTCACATGCTTAACTTGGCCAATTTGGCTGAGGAGGTTC
AGATTGCCTACCGTAGGCGCCAAAAATTGAAGAAAAAGGGAGATTTTGGGGATGAGAGCAATGCAAC
AACTGAGTCAGATATTGAAGAAACTTTCAAGAAATTGGTGGGGGACTTGAAGAAGTCCCCTCAAGAA
GTTTTTGATGCTATCAAGAATCAGACTGTGGATCTGGTCCTAACAGCTCATCCTACACAATCTGTAC
GAAGATCATTGCTTCAAAAGCATGGAAGGTTTGGTCTTTAATCACCATGTTTATTGTCTTCAGCAGT
TAGAGTTGTCTCCTTGGCTTGTCATTTTATGCTAGTTTGTGCTTCGATTTTTCCCTAATAAATATAA
TTGGAAATGCTCTTTTCGGACCAAATTTTCGTGCTTTCGTCGGTTGTTTACTTACTTGTTCTAACCA
AATTTTCAGGATCCGTGACTGCTTGGCTCAGTTGTATGCTAAAGACATTACACCCGATGATAAACAA
GAACTTGATGAGGCTTTACAGAGGGAGGTAACTATCGACGTTTAGTTACTTGATACATGTGTTTTTC
CTTGAATGAAATTCTATTATTAGTACCTTCAATTCTGGTGTCTTTCTGACTCTACTAATGGATATTT
TGGTGCTTCAATATCATATAGATATGTCTTGATCTGTCAGGCCTTATATTATCAATGGACTTACTTT
TTTCATCCCTCTCATGAAGATTGGAGAAGCAAAAATTATCTCATAAGTTACCCATTTAATAGTTGCC
CCTTCATTCATTTGAATGATTTTACCCTGTAAAAAGTGGAGCTGGTTATATGATTGCTCTAAAATTG
GATATGAAGCAATAATAGCAATCTTCGAACTCAAATATGTGTCGCATTCTGTGGATCATGGTTTATT
GGTATGATGCACCCATTATTCTTTTTTGACAAATATCTGCTACTTACTTCCTCTGTAGATCCAAGCT
GCGTTCCGAACTGATGAGATCCGAAGAACTCCTCCTACTCCACAAGATGAAATGAGAGCTGGAATGA
GCTATTTCCATGAAACAATTTGGAAGGGTGTACCAAAGTTTCTGCGCCGTGTTGATACAGCTCTTAA
AAACATAGGGATTAATGAACGAGTTCC

**FIGURE 4 (continued)**

TTATAATGCTCCCCTTATTCAATTCTCCTCTTGGATGGGCGGTGATCGTGATGGTATGATTCCTTTA
CTGTGGAGAATGTTTGCAGTTACTTTAGCTAAAAGCTGAGAAAACAAAATAGGAATTCGGATTACAT
AATTTTGAAGGGGTGGAGGAAAGCAAAATACTCCTTCTGCTTGAACGTGGATTACTTAGTTACTACA
TGCAGTAGATAATCCTTATTTATCTGTCAACTCTCTGCTACCAAGTACAGGTAATCCAAGAGTGACT
CCTGAGGTCACAAGAGATGTTTGCTTATTGGCCAGAATGATGGCAGCTAACTTGTACTATTCGCAAA
TAGAGGACCTCATGTTTGAGGTATCCAACAATTAAATGTTTTTGAGACTTTTGTCTTGGTTCAATTA
GTGAAAAAATCGCACTATCTACACTGTGATCTTTTTCTAAATTGTACAATCTTTCAGTTATCTATGT
GGCGCTGCAATGAAGAACTTCGTGTGCGAGCAGATGATCTCCAAAGGTCTTCAAGGAGAGATGAAAA
ACACTACATAGGTAACTAATTGAATATTGCTATTAAGGTTGATCCATGAAATTGAATCAAAGAAGAG
TTTTCTAGTTGCATATAACCTTGATAGGACTTTTAAATGCGTGTTTGAAGTATGATTCAAATTCTCC
TTTCAAGTGAGCCAAGGGTGTCTTTCATCATTGCCGATGGGAAAAAAAAAGGGTGAGCAAAGGGTGT
TATTATTTATGATTTAAAGATTGAGAAAAAGAGGTCATTCTAGCATCTGGTATGCTGATTAGAAATT
GCACGAGAAGCTCTCTGTATTCTCTTGGAAAGATGTTCCTTTCTTCCAAAAAAATCTTTGGACAGAG
AAATCGCATAATCTGTGATCTTTCAACATTTTCTTCTCATGCAGAATTTTGGAAGCAAGTTCCTCCA
AATGAACCCTATCGTGTAATTCTTGGTGATGTGAGAGATAAGTTGTATCAGACACGTGAGCGTGCTC
GCCAACTGTTAGGCCATGGATACTCTGAAATTCCAGAGGAAGCAACTTATACTAATATTGAGCAGGT
TCATAATACTATACTTCTGTCTTTTCCTGTGACTGCTTTCTATATGTAGACCCTTCTAAATTAAGCA
GTGAATATTCACTTGTACTCTTGAAAGCTTAACTAGTTTTACTAAACAGCTTGACATGTTATTCATC
GAAAAAATGAACTAGAAAAACATGAGGAAGTGTGTCTCTTTCTTGTTGAATAACAGCATAAGCATTT
GCATGGTTGTTCTACCAAACCAAAAACATATAAATATCTGTGTTGCATGTCATATAACACTAATATG
GTCATCATTTTATGTAGTTCTTGGAGCCTCTTGAGCTCTGCTACAGATCTCTTTGTGCTTGTGGTGA
CCTCTCCATCGCGGATGGTAGCCTTCTGGATTTCCTGAGACAAGTTTCTACGTTTGGACTTTCACTT
GTGAGACTTGACATAAGACAAGAGTCGGACCGCCATACTGATGTGCTTGACGCTATTACGCAACATT
TGGAAATTGGTTCATATCGAGACTGGTCTGAAGAACGTAGACAAGAGTGGCTTCTGTCTGAACTCAG
TGGCAAGAGACCTTTATTTGGACCTGATCTTCCAAAAACTGAAGAAATTGCTGATGTTTTGGATACG
TTCCATGTCATAGCCGAACTCCCAGCAGACTGCTTCGGGGCATACATCATCTCAATGGCCACTGCAC
CATCTGATGTTCTTGCAGTAGAGCTTCTACAGCGTGAATGCCGAGTGAGGCAACCTTTACGAGTTGT
TCCACTTTTTGAGAAGTTGGCTGATCTGGATGCTGCTCCTGCAGCCGTTGCACGTCTTTTCTCAATT
GAGTGGTACAGAAACCGGATTAATGGCAAGCAAGAAGTGATGATTGGATACTCCGACTCTGGCAAGG
ATGCAGGTCGGCTATCAGCAGCCTGGCAGTTATATAAGGCTCAAGAGGAGCTTATACAAGTCGCCAA
GGAGTTCGACGTGAAGCTAACCATGTTCCATGGTAGAGGTGGTACAGTGGGAAGAGGAGGTGGCCCC
GCCCATCTTGCCATATTGTCTCAACCACCCGAAACAATTCACGGATCTCTCCGTGTTACAGTTCAGG
GTGAGGTTATTGAGCAGTCTTTTGGGGAAGAACACTTGTGTTTTAGGACACTCCAGCGTTTTACTGC
TGCTACACTTGAACATGGGATGCATCCACCAGTCTCTCCAAAACCAGAATGGCGTGCACTTATGGAC
GAAATTGCAGTTGTTGCAACAGAAAAGTATCGATCAATAGTTTTTAAGGAACCCCGATTTGTCGAGT
ATTTCCGCCTGGTAAGTATGCTAGCAATGAACTCAGTTATTAATTGTAGATGCTGTCACAAATATTG
CCTTGAGTTGTAATAAGCTATTTTTCATTGATCATGTTTGCAGGCCACACCCGAGTTAGAGTATGGT
CGAATGAACATTGGCAGCCGTCCATCAAAGCGTAAACCCAGCGGAGGCATAGAATCACTTAGAGCTA
TTCCGTGGATCTTTGCTTGGACTCAAACTAGATTCCATCTTCCAGTCTGGCTCGGCTTTGGAGCAGC
ATTTAAGTATGCCATTGAAAAGGATATCAAAAACCTTCGCATGCTGCAGGAAATGTACAATGCATGG
CCTTTCTTTAGGGTAACTATCGATTTGGTTGAGATGGTGTTCGCCAAAGGAGACCCAGGCATTGCTG
CATTGTTCGACAAGCTTCTGGTGTCCGAAGATTTGTGGTCTTTCGGTGAACTTTTGAGGTCAAAGTA
TGAGGAGACAAAGAGCCTCCTCCTGCAGGTAATGTGACATATACTTCTAAAAAGGATCGGTTGAACA
CTTATTGTGCTCGATTGCTTCACAAACTGGTTCCAAGTTAGTACATCTAAACATAATTTTCCCTTTC
CCTTTTTAAAATAGATTGCTGGACACAAGGATCTTCTGGAGGGTGATCCATACTTAAAACAACGACT
CAGGTTGCGTGACTCCTACATCACAACATTGAATGTGTGTCAAGCCTACACCCTAAAGCGTATTCGT
GACCCAGACTATAGTGTCAC

# FIGURE 4 (continued)

ACCAAGGCCTCACATTTCCAAGGAATACATGGAAGCAAAACCAGCTACAGAACTTGTGAACCTGAAC
CCGACTAGCGAATATGCACCTGGCTTGGAGGACACACTCATCTTAACCATGAAAGGTATTGCTGCTG
GAATGCAGAATACTGGTTAGTCGTCACCTGGAATATGGAAGATG

SEQ ID NO: 46, protein - Solanum tuberosum
MTTRNLEKLASIDAQLRQLVPAKVSEDDKLVEYDALLLDRFLDILQDLHGEDLKETVQECYELSAEY
EAKHDPKKLEELGNVLTSLDPGDSIVIAKAFSHMLNLANLAEEVQIAYRRRQKLKKKGDFGDESNAT
TESDIEETFKKLVGDLKKSPQEVFDAIKNQTVDLVLTAHPTQSVRRSLLQKHGRIRDCLAQLYAKDI
TPDDKQELDEALQREIQAAFRTDEIRRTPPTPQDEMRAGMSYFHETIWKGVPKFLRRVDTALKNIGI
NERVPYNAPLIQFSSWMGGDRDGNPRVTPEVTRDVCLLARMMAANLYYSQIEDLMFELSMWRCNEEL
RVRADDLQRSSRRDEKHYIEFWKQVPPNEPYRVILGDVRDKLYQTRERARQLLGHGYSEIPEEATYT
NIEQFLEPLELCYRSLCACGDLSIADGSLLDFLRQVSTFGLSLVRLDIRQESDRHTDVLDAITQHLE
IGSYRDWSEERRQEWLLSELSGKRPLFGPDLPKTEEIADVLDTFHVIAELPADCFGAYIISMATAPS
DVLAVELLQRECRVRQPLRVVPLFEKLADLDAAPAAVARLFSIEWYRNRINGKQEVMIGYSDSGKDA
GRLSAAWQLYKAQEELIQVAKEFDVKLTMFHGRGGTVGRGGGPAHLAILSQPPETIHGSLRVTVQGE
VIEQSFGEEHLCFRTLQRFTAATLEHGMHPPVSPKPEWRALMDEIAVVATEKYRSIVFKEPRFVEYF
RLATPELEYGRMNIGSRPSKRKPSGGIESLRAIPWIFAWTQTRFHLPVWLGFGAAFKYAIEKDIKNL
RMLQEMYNAWPFFRVTIDLVEMVFAKGDPGIAALFDKLLVSEDLWSFGELLRSKYEETKSLLLQIAG
HKDLLEGDPYLKQRLRLRDSYITTLNVCQAYTLKRIRDPDYSVTPRPHISKEYMEAKPATELVNLNP
TSEYAPGLEDTLILTMKGIAAGMQNTG

SEQ ID NO: 47, DNA - Medicago sativa
AAACACTGTCCTTCTGATCCATTTTTCCATTCCTTGTCATCATCTTCTTCTTCGTATCTAACTAACT
GTCTGGCACACAACACGGTGAGAGAGTGAATTGCTACAATGGCAAACAAGATGGAAAAAATGGCATC
AATTGATGCACAGCTTAGACAATTGGTTCCTGCAAAAGTGAGTGAAGATGATAAACTTATTGAGTAT
GATGCTTTGTTGTTGGATCGGTTTCTTGATATTCTTCAAGATTTACATGGAGAGGATCTGAAGGATT
CTGTTCAAGAAGTGTATGAACTGTCTGCTGAATATGAAAGAAAGCATGATCCTAAGAAACTTGAAGA
GCTTGGAAATTTGATCACAAGTTTCGATGCAGGTGACTCAATTGTTGTTGCCAAGTCCTTTTCACAC
ATGCTTAACTTGGCCAACTTAGCTGAAGAGGTTCAAATTGCGCACCGCCGAAGGAACAAGTTGAAGA
AAGGTGATTTTAGGGATGAGAGCAATGCAACCACTGAATCTGACATTGAGGAAACTCTCAAGAAACT
TGTGTTTGACATGAAGAAATCTCCTCAAGAGGTTTTTGATGCATTGAAGAACCAGACTGTTGATCTT
GTTCTTACTGCTCATCCTACTCAGTCGGTTCGTCGATCTTTGCTTCAAAAGCACGGAAGGGTAAGGA
ACTGTTTATCTCAATTGTATGCTAAAGACATCACTCCTGATGATAAGCAGGAGCTTGATGAAGCTCT
CCAGAGGGAGATTCAAGCTGCATTCCGTACTGACGAAATCAAGAGGACTCCACCAACTCCCCAAGAT
GAAATGAGAGCTGGGATGAGTTACTTCCATGAAACAATTTGGAAGGGTGTCCCTAAATTTCTTCGCC
GTGTTGATACGGCATTGAAGAACATAGGGATTAACGAACGTGTTCCCTATAATGCTCCTCTTATTCA
ATTTTCTTCTTGGATGGGTGGTGATCGTGACGGTAATCCAAGAGTGACTCCTGAAGTGACAAGGGAT
GTTTGCTTACTAGCTAGAATGATGGCTGCTAACTTGTATTATTCACAGATAGAAGATCTTATGTTTG
AACTTTCTATGTGGCGTTGCAATGACGAGCTACGTGTTCGCGCAGAAGAACTTCACAGGAATTCCAA
GAAAGATGAAGTTGCAAAACACTATATAGAGTTTTGGAAAAAAATTCCTTTGAATGAACCATACCGT
GTTGTACTCGGGGAGGTAAGGGACAAGCTCTATCGCACTCGTGAGCGTTCTCGTTATCTCCTAGCTC
ATGGCTACTGTGAAATTCCTGAAGAAGCCACATTCACCAATGTCGATGAGTTTCTGGAACCTCTTGA
ACTCTGCTACAGATCACTCTGTGCTTGTGGTGATCGTGCAATTGCTGATGGAAGCCTTCTTGATTTC
TTGAGGCAAGTTTCCACTTTTGGACTGTCACTTGTAAGGCTTGATATACGGCAAGAGTCTGATCGTC
ACACTGACGTGATGGATGCCATTACCAAACATTTGGAAATTGGATCCTACCAAGAATGGTCTGAAGA
AAAAAGACAGGAATGGCTTTTGTCCGAGTTGATTGGCAAAAGGCCACTCTTTGGACCTGACCTACCC
CAAACCGATGAAATTAGAGATGTTTTAGACACGTTCCGTGTCATAGCAGAACTTCCATCTGACAACT
TTGGAGCCTACATCATTTCGATGGCAACTGCACCGTCTGAT

**FIGURE 4 (continued)**

GTGCTGGCAGTTGAGCTTCTTCAACGTGAATGCAAAGTCAGGAATCCATTAAGAGTCGTTCCGTTGT
TTGAAAAGCTTGATGATCTTGAGTCTGCTCCTGCTGCATTGGCTCGGTTGTTCTCCATAGACTGGTA
CATTAACCGGATCGATGGGAAGCAAGAAGTTATGATTGGATATTCTGATTCAGGAAAAGATGCTGGA
AGGTTTTCTGCAGCATGGCAGCTATATAAGGCTCAGGAGGACCTCATCAAAGTCGCACAGAAATTTG
GTGTTAAGCTAACCATGTTCCACGGTCGTGGTGGAACTGTTGGAAGAGGAGGTGGACCTACCCATCT
TGCTATCTTGTCTCAACCACCAGAAACAATTCACGGATCTCTTCGTGTGACAGTTCAAGGTGAAGTT
ATTGAACAGTCGTTCGGTGAGGAACACTTGTGCTTTAGGACACTGCAACGTTTCACTGCTGCTACTC
TAGAACATGGAATGCGTCCCCCAAGCTCTCCAAAACCAGAATGGCGCGCCTTGATGGATCAGATGGC
TGTCATTGCAACTGAGGAATACCGTTCAATTGTGTTCAAGGAACCACGTTTTGTTGAGTATTTCCGT
CTGGCTACACCAGAGATGGAGTATGGTAGGATGAACATTGGAAGTCGACCGGCAAAGAGAAGGCCTA
GTGGAGGCATTGAAACACTGCGTGCGATACCATGGATCTTTGCCTGGACACAGACAAGGTTTCATCT
TCCAGTATGGCTGGGCTTTGGAGCAGCATTTAGACAAGTTGTTCAGAAGGATGTTAAGAATCTCCAT
ATGCTGCAAGAGATGTACAATCAATGGCCTTTCTTTAGGGTTACAATTGATTTAGTTGAAATGGTGT
TTGCCAAGGGTGACCCTGGTATTGCAGCACTGAATGATAGGCTCCTAGTTTCAAAGGATCTGTGGCC
ATTTGGGGAACAATTGAGAAGCAAATATGAAGAAACTAAGAAACTCCTACTTCAGGTGGCTGCACAC
AAGGAAGTTCTTGAAGGTGACCCCTACTTGAAGCAAAGACTCAGACTCCGTGATTCGTACATTACAA
CCCTTAATGTTTTCCAAGCCTACACATTGAAACGGATCCGCGATCCAAACTACAAGGTGGAGGTGCG
CCCCCCAATATCGAAAGAGTCTGCTGAAACAAGTAAACCAGCTGATGAACTTGTAACATTGAATCCA
ACAAGTGAATATGCTCCTGGTTTGGAAGACACACTCATTCTTACCATGAAGGGTATTGCTGCTGGCA
TGCAGAACACTGGTTAAATTTTGGTTACATTTTTCACTTGTATTTGTTTCTTTATGTTAAGTGTGTA
CTAAGATTTCATAAATACTAGATGAATCTAGTTGCAAACAAGCACTTCAAGTGAGTGCTTTTTTCTT
TTTCTTTTTCTTTTCATAAGAATTTCACATCAGGTTTTGTTGGTGTGCTTCCTTACTTTGCTGCCAC
ACAAATGAGTTATGCAATTGATGTTATGTTTCAAGGCATAAATTTTGTTGAGTGCTGCTACTATACG
CTTTCTTGTTCAATTTAATATGAGACTGAAAAACATAGAAAATAGGAACAATTATAATAA

SEQ ID NO: 48, protein - Medicago sativa
MANKMEKMASIDAQLRQLVPAKVSEDDKLIEYDALLLDRFLDILQDLHGEDLKDSVQEVYELSAEYE
RKHDPKKLEELGNLITSFDAGDSIVVAKSFSHMLNLANLAEEVQIAHRRRNKLKKGDFRDESNATTE
SDIEETLKKLVFDMKKSPQEVFDALKNQTVDLVLTAHPTQSVRRSLLQKHGRVRNCLSQLYAKDITP
DDKQELDEALQREIQAAFRTDEIKRTPPTPQDEMRAGMSYFHETIWKGVPKFLRRVDTALKNIGINE
RVPYNAPLIQFSSWMGGDRDGNPRVTPEVTRDVCLLARMMAANLYYSQIEDLMFELSMWRCNDELRV
RAEELHRNSKKDEVAKHYIEFWKKIPLNEPYRVVLGEVRDKLYRTRERSRYLLAHGYCEIPEEATFT
NVDEFLEPLELCYRSLCACGDRAIADGSLLDFLRQVSTFGLSLVRLDIRQESDRHTDVMDAITKHLE
IGSYQEWSEEKRQEWLLSELIGKRPLFGPDLPQTDEIRDVLDTFRVIAELPSDNFGAYIISMATAPS
DVLAVELLQRECKVRNPLRVVPLFEKLDDLESAPAALARLFSIDWYINRIDGKQEVMIGYSDSGKDA
GRFSAAWQLYKAQEDLIKVAQKFGVKLTMFHGRGGTVGRGGGPTHLAILSQPPETIHGSLRVTVQGE
VIEQSFGEEHLCFRTLQRFTAATLEHGMRPPSSPKPEWRALMDQMAVIATEEYRSIVFKEPRFVEYF
RLATPEMEYGRMNIGSRPAKRRPSGGIETLRAIPWIFAWTQTRFHLPVWLGFGAAFRQVVQKDVKNL
HMLQEMYNQWPFFRVTIDLVEMVFAKGDPGIAALNDRLLVSKDLWPFGEQLRSKYEETKKLLLQVAA
HKEVLEGDPYLKQRLRLRDSYITTLNVFQAYTLKRIRDPNYKVEVRPPISKESAETSKPADELVTLN
PTSEYAPGLEDTLILTMKGIAAGMQNTG

SEQ ID NO: 49, DNA - Zea mays
GCCCTCTCCTGGCTCCTCTCCTTCCTGCTTGGTTTTTGCTTTCAGCTCTTGAACCGAATCCACTGCC
GCGGCGCTTAGCATTGGGGACTAGGGGGCCGACATGGCTGCCTTAGGGCCGAAGATGGAGCGTCTGT
CGTCGATCGACGCGCAGCTGCGGATGCTGGTGCCGGGGAAGGTTTCGGAGGACGATAAGCTCATCGA
GTACGACGCTCTCCTCCTCGATCGGTTCCTCGACATCCTTCAGGACCTCCATGGCGACGACCT

**FIGURE 4 (continued)**

CAAGGAAATGGTTCAAGAATGCTATGAGGTAGCTGCTGAGTATGAAACAAAACATGACTTGCAAAAG
CTTGATGAACTCGGGAAGATGATAACAAGCTTGGATCCTGGGGACTCTATCGTGATTGCTAAGTCTC
TCTCGCACATGCTTAACTTGGCCAACTTGGCTGAGGAAGTCCAGATAGCCTACAGGAGGAGAATCAA
GCTCAAGAAGGGAGACTTTGCTGATGAGAACTCGGCAATCACAGAATCTGACATTGAGGAAACACTT
AAGAGGCTTGTTGTTGACCTGAAGAAGTCACCTGCTGAGGTATTTGATGCCCTCAAGAGCCAGACTG
TTGATCTGGTTTTGACTGCACATCCAACACAGTCTGTGAGGAGGTCACTGCTCCAGAAACACTCGAG
GATACGCAACTGTTTGGTTCAACTCTACTCAAAAGATATCACTCCGGATGATAAGCAGGAACTTGAT
GAGGCTCTGCAAAGAGAGATCCAAGCTGCCTTTAGAACTGATGAGATCCGAAGAACACAGCCCACTC
CCCAAGATGAAATGCGTGCTGGTATGAGCTACTTCCACGAAACAATTTGGAAGGGTGTTCCAAAGTT
CTTGCGCCGGGTTGATACTGCATTGAAGAACATTGGGATTAACGAGCGTGTTCCTTACAATGCACCT
CTTATTCAATTCTCTTCTTGGATGGGGGGAGATCGTGATGGAAATCCAAGAGTGACACCAGAGGTTA
CCAGGGATGTCTGCTTGCTTGCCAGAATGATGGCATCAAACTTGTACTGCTCTCAGATTGAGGATCT
CATGTTTGAGTTGTCTATGTGGCGATGCAGTGATGAACTGCGCATGCGAGCTGATGTGCTGCATCTC
TCCACTAAGAAGGATGCCAAACATTACATAGAGTTTTGGAAGAAGGTTCCTCCAAATGAGCCATATC
GGGTGATACTGAGTGATGTCAGGGATAAACTGTACAACACTCGTGAACGGTCACGAGAGCTTTTATC
CAGTGGGCATTCTGATATTCCTGAGGAAGCTACATTGACAAATGTTGAGCAGCTGTTGGAGCCCTTG
GAACTATGTTACAGATCACTCTGTGCTTGTGGTGACTCTGTAATTGCTGATGGAACCCTTCTGGATT
TCTTGCGTCAAGTGTCCACCTTTGGACTCTCCCTTGTGAGGCTTGACATTAGGCAAGAGTCAGATAG
GCATACTGATGTCCTTGATGCCATCACTACATACCTGGGGATAGGATCTTACCGTGAATGGACTGAA
GAACGCCGCCAAGAATGGTTACTGTCTGAACTTAATGGCAAGCGCCCTCTGTTTGGCTCAGACCTTC
CCAAGACTGAGGAAATTTCTGATGTTCTAGACACATTCCATGTTATTGCTGAGCTTCCCTCTGACAA
TTTTGGTGCGTATATCATTTCCATGGCGACAGCTCCGTCGGACGTCCTTGCTGTTGAACTCCTCCAA
CGTGAGTGTCATGTGAAAACACCACTTAGAGTAGTCCCGCTGTTTGAGAAGTTGGCCGATCTTGAGG
CTGCCCCGGCTGCATTGGCCAGACTGTTCTCAATAGATTGGTACAGACAGAGGATCAATGGCAAGCA
GGAGGTCATGATTGGGTATTCAGACTCGGGCAAAGATGCTGGTCGCCTCTCAGCAGCTTGGCAGCTA
TACAAAGCTCAGGAGGAGCTCATCAAGGTTGCTAAGGACTTTGGTGTGAAGTTAACGATGTTCCATG
GACGTGGTGGGACTGTTGGAAGGGGTGGTGGTCCCACTCACCTTGCCATCTTGTCCCAGCCACCAGA
CACGATCCACGGATCACTCAGGGTCACTGTGCAAGGTGAAGTCATTGAACAGTCATTTGGTGAGGAG
CACTTGTGCTTTAGGACACTGCAACGTTTCACGGCTGCTACCCTGGAGCATGGCATGCACCCACCAA
ATGCACCAAAGCCAGAATGGCGAGCCCTTCTTGATGAGATGGCAGTTGTGGCAACTGAGGAATATCG
GTCCATTGTCTTCAAAGAGCCACGCTTTGTCGAGTATTTCCGCCTTGCAACCCCTGAAACAGAGTAT
GGTAGGATGAACATAGGAAGCAGGCCATCCAAGAGAAAGCCGAGCGGAGGTATCGACTCACTCCGAG
CAATCCCATGGATCTTTGCTTGGACACAAACGCGGTTCCACCTCCCGGTCTGGCTAGGATTTGGAGC
CGCATTCAAGAATGTCCTCCAGAAGGACATCAGGAACCTCCACATGCTCCAGGAAATGTACAATGAG
TGGCCATTTTTCAGGGTGACTATTGATCTGGTGGAGATGGTGTTCGCCAAGGGTAATCCTGGCATTG
CCGCACTGTATGACAAACTCCTCGTTTCAGAGGAACTGCATCCATTGGGTGAGAAGCTGAGGGCCAA
CTATGAGGAAACCCAGAAGCTTCTACTTCAGGTTGCTGGGCACAGGGATCTTCTGGAAGGTGACCTC
TACCTGAAGCAGCGGCTCCGCCTGCGTGATGCGTACATCACCACCCTGAACGTCTGCCAGGCCTACA
CCCTGAAGCGGATCCGTGACCCGGACTACCATGTCGCGCTGCGCCCCCACCTCTCCAAGGAGATCAT
GGACTCGACCAAGGCTGCGGCGGACGTGGTGAAGCTGAACCCTGGCAGCGAATACGCACCGGGGCTG
GAGGACACCCTCATCCTGACCATGAAGGGCATAGCAGCCGGCCTCCAGAACACCGGTTAAACCACGA
AGAGGGGATCTTTTTTCTCCTCCTGATCATTGGCTCTAGGTCCTTCGTATTTTCGCGGATTGTAACT
TCGGCCACCTCTCTACACCGTGAGGAAATAATGGTGGTTTCTGCAAAAGTGGTGGTAAATAAAAGGG
CGACAACTATCTTGTTTTTCATAGTAACTTATAACTTTACGATGCGATAATGGAACATGGCTTGCGT
T

## FIGURE 4 (continued)

SEQ ID NO: 50, protein - Zea mays
MAALGPKMERLSSIDAQLRMLVPGKVSEDDKLIEYDALLLDRFLDILQDLHGDDLKEMVQECYEVAA
EYETKHDLQKLDELGKMITSLDPGDSIVIAKSLSHMLNLANLAEEVQIAYRRRIKLKKGDFADENSA
ITESDIEETLKRLVVDLKKSPAEVFDALKSQTVDLVLTAHPTQSVRRSLLQKHSRIRNCLVQLYSKD
ITPDDKQELDEALQREIQAAFRTDEIRRTQPTPQDEMRAGMSYFHETIWKGVPKFLRRVDTALKNIG
INERVPYNAPLIQFSSWMGGDRDGNPRVTPEVTRDVCLLARMMASNLYCSQIEDLMFELSMWRCSDE
LRMRADVLHLSTKKDAKHYIEFWKKVPPNEPYRVILSDVRDKLYNTRERSRELLSSGHSDIPEEATL
TNVEQLLEPLELCYRSLCACGDSVIADGTLLDFLRQVSTFGLSLVRLDIRQESDRHTDVLDAITTYL
GIGSYREWTEERRQEWLLSELNGKRPLFGSDLPKTEEISDVLDTFHVIAELPSDNFGAYIISMATAP
SDVLAVELLQRECHVKTPLRVVPLFEKLADLEAAPAALARLFSIDWYRQRINGKQEVMIGYSDSGKD
AGRLSAAWQLYKAQEELIKVAKDFGVKLTMFHGRGGTVGRGGGPTHLAILSQPPDTIHGSLRVTVQG
EVIEQSFGEEHLCFRTLQRFTAATLEHGMHPPNAPKPEWRALLDEMAVVATEEYRSIVFKEPRFVEY
FRLATPETEYGRMNIGSRPSKRKPSGGIDSLRAIPWIFAWTQTRFHLPVWLGFGAAFKNVLQKDIRN
LHMLQEMYNEWPFFRVTIDLVEMVFAKGNPGIAALYDKLLVSEELHPLGEKLRANYEETQKLLLQVA
GHRDLLEGDLYLKQRLRLRDAYITTLNVCQAYTLKRIRDPDYHVALRPHLSKEIMDSTKAADVVKL
NPGSEYAPGLEDTLILTMKGIAAGLQNTG

SEQ ID NO: 51, DNA - Triticum aestivum
GCGGCGCGAGCACTAATCGACTCACTATAGGGCGTCGACTCGATCTTGAGGGCTGAGGACGGTGGAA
TCAGGACCGTCCACGTGGACATGTGGATGCTCCCACCATAATCCGTAAACTAATCCCAGTCCCGATC
GAAATCAAATCACGAACACTACCACTTGGACACAAATTAATTTGATTTACAGAGATCTCTCGAGTCC
CGGTGAATCCCGGCGGCACTCCTGCCATCCGCCTTCTATAAATAGGCAGCAACTGGAGTCACGACCT
CCTCAGTCCCGGCCCCAACCACATCCTCCCCCCAATCTAGCTCCCTCCCTCCCTACCACGCTGGTCC
CTGCGAGGCGGCGCGGCGAGCGCGAGCAGTCGGGTCGGCTCCGGTCAAAGCGAGCCGGCGATGGCGT
TGTCGGCGCCGGGCGGCGGCAGCGGGAAGATCGAGCGGCTGTCGTCGATCGACGCGCAGCTGCGGCT
GCTCGTCCCCGCCAAGGTGTCCGAGGACGACAAGCTCATTGAGTACGACGCGCTCCTCCTCGACCGC
TTCCTCGACGTCCTGCAGGGCCTCCACGGCGACGATCTCAGGGAGATGGTAACTCTTTTTTCTTCTT
GTTTTCTCTCTCTCGCACTGTTCGATCTCGCACCGGATCCGAGCTGTCCCATCACCCATGTGCTACT
GCTGCGCTGCTCCCATTGATTCCTCTGAACTTTACATGTGGCGCTTCAGGGTTCGAGTGGAGTTTTC
AGCTCAGTGTTTACGGAGTGGGGTTGACTTTGGGTTAAGTCTCGATGTCGGTAGATTTGTGTGTAAT
CTGGTGTTGTTAAGTGGTAGTTTGGACGATTGCCCAGCTGCTCAGTTTTTCTGCCACAAACAAATTT
TTTTTCGCAGCTAATCTATATCCCATTGTGGTCTTGATTTTGGGAGCACTGCTTTAATTAGTCGAAC
TGGGTTACTGATTGAATCTCAAATCGTGTCGGTCAAGCATGACTTCATCCGTGGAATTTGCCTCGCT
CTATCATTGTTGTGTCAACTGATTTTGGCATATAAGTGCTGGATCTTATGTTGGTCATATACGGGAT
ACTAAATAACTGGCTTAGTAGTTGTGTCAACTGAAGTAATATGTTAACTGACTTGGATCTACTGTAA
TTATGAAAAATAGCTAACAGTGTTCATCTCTTAGTCTACGAATATACTAGTTGGGTTAAACTGTCGA
TACAACGGATAGACGCCTTCTTAGGCTAACCGGCATGGAAGTGCTGATAATATTTACATGTATGGAA
GTCTCGAGACATTGTCCATTGCTTGTAGAAGACCGTTACATCTTGTTTATACCTTACCTAACAAAGC
CTGAGATATGGCGTTTCCAGAGAACCAAACTAGGGGAAAAAAGGTTCTTGAAATCGACTTGCCTTGA
CTTTTGTTGACTTGGTGGCTCACAACCTGTTTGGTACCTTGCTTGTCAATTGATTAAGAAGACGTAG
GCATATAAACAATCAGGTTGCTGGTCGGCTTGATTATCTTTTTGGTACCTTGCTTGTCAGTTGATTA
AGAAGACATCCTATATCTTATGTTGATTGATGGACAGCCATAATTGGATTATTATTTTCACTAAACA
GGTTCAAGAATGCTATGAGGTGGCTGCTGAATATGAAACTAAGCATGACCTGGAAAAGCTTGATGAA
CTCGGAGAGATGATAACAAGCTTGGATCCTGGTGACTCTATTGTGATTGCCAAAGCTTTTTCACACA
TGCTTAACTTGGCCAACTTGGCTGAGGAAGTGCAGATCGCATACAGGAGGAGAGTCAAGCTCAAGAA
GGGAGACTTTGCTGATGAAAATTCAGCAATCACAGAATCTGACATCGAGGAAACTCTTAAGAGGCTT
GTTTTTGACATGAAGAAGTCCCCTGCTGAGGTTTTTGATGCCCTCAAGAATCAGACTGTTGATCTGG
TTTTGACTGCACATCCAACACAATCTGTAAGGAGGTC

**FIGURE 4 (continued)**

ACTGCTCCAGAAGCATTCGAGGTTAGTTAACCAAACATTGTGATGATAGTAGTCATGATACAATTTC
TATATATTAATCCTGATTACAATGACTTTATATCAGGATCCGGAACTGTTTGGTTCAGTTATACTCG
AAGGATATCACTCCAGATGACAAGCAGGAACTTGACGAGGCTCTCCAGAGAGAGGTGCTTACTGCTA
TCCTTATGTTGATTTTCATGCTGTTTCTAAGGCACATTGTTAACATGACAATGCAGTCACAGTAGCC
TTACAACATCAGACAGCTGAGATTCTTTCTTACACTTAACCTAGAATCCTCCTTTTTAGATCCAAGC
TGCCTTTAGAACTGATGAGATCCGAAGACTCAGCCCGACTCCCCAAGATCACATGCGTGCTGGTATG
AGCGACTTTCATGAGACAATATGGAAGGGTGTTCCAAAGTTTTTGCGCCGTGTTGATACGGCATTGA
AGAACATTGGGATCAATGAGCGTGTTCCTTATAATGCTCCTCTTATCCAATTCTCTTCTTGGATGGG
TGGAGATCGTGATGGTGAGTGGAATCTCTGTCCCTCGTGATACTGTGCAGCGTACATCTATGATGTA
TCTACAGTCACTGCAAGCTTTTATTACCTTGGTGGTGTCTGTTAGATGCTACATTTTCTGTATCTGG
TCGTATCAAGAACTATGGATAAAGCCAACTGTTTTATGTATCTAAACTTCTAGTACCTTTACAAATA
CAGGAAATCCAAGAGTGACACCAGAAGTTACAAGGGACGTCTGTTTGCTTGCCAGAATGATGGCAGC
TAATTTGTATTGTGCACAGATTGAGGATCTCATGTTTGAGGTAGAAATTGATTGATTACTTTTAACT
AAGTTCACATATATTTGATCATTAGAAGTTGGATAAAACTTCAAACTATTTCAGTTGTCTATGTGGC
GATGCAATGATGAGCTACGCTCGCGAGCTGATGAGCTGCACCGCTCTTCTAAGAAGGATGCTAAGCA
TTACATAGGTACATAACAGTAACAGTTTTTTAAATGTAAAATAGTAGGTCAGACCCCTGCCAGAGC
TTTAAAAAGGCAGTAACAGCTTCTAGGCATGAAATTTCACATAACATATGCCTTTTTTTGTTCTAC
TTATTAATTTGTGATGTTCATCTCCGATATATAGAGTTCTGGAAGAAGGTTCCTCCGAATGAGCCAT
ATCGGGTAATACTGGGTGATGTTAGGGATAATCTTTACAACACGCGTGAGCGATCACGTGAGTTGTT
ATCCAGTGGACATTCCGACATACCTGAGGAAGCTACTCTGACAAATCTTGAGCAGGTTGAGTAATGT
CTACGAAATATAATTGGAACTATACATTTTTTCTTCAATGGTACTATGTTTGGTAGAGAAATGGTCT
TTAATATATGTCGGTCTTCTCTTGCAGCTGTTGGAGCCCTTGGAGCTCTGTTACAGGTCACTGTGTG
CTTGTGGTGACCGTGTTATTGCTGATGGAACCCTTCTTGATTTCTTGCGCCAAGTCTCTACCTTTGG
ACTTTCCCTCGTGAAGCTTGACATCAGGCAAGAGTCTGACAGGCACACTGATGCTCTGGATGCAATC
ACCTCATACCTGGGAATAGGATCTTATCGTGAGTGGTCTGAGGAACACCGTCAAGAATGGCTGTTGT
CTGAACTCAATGGGAAGCGCCCATTATTTGGCGCAGACCTTCCCATGACAGAGGAAGTTGCTGATGT
TATGGGCGCGTTCCAGGTTATTGCTGAGCTGCCCGGTGACAATTTTGGAGCATATGTCATCTCAATG
GCAACATCTCCTTCAGATGTTCTTGCTGTGGAGCTCCTCCAACGTGAGTGCCATATCAAGACACCAC
TTAGAGTTGTCCCCCTGTTTGAGAAGTTGGCCGATCTCGAGGCTGCCCCAGCAGCACTGGCCAGACT
CTTCTCAATAGATTGGTACAGAGAGAGGATCAATGGCAAGCAGGAGGTCATGATTGGGTATTCAGAC
TCAGGCAAGGATGCAGGCCGTCTCTCAGCAGCTTGGCAGATGTACAAGGCTCAGGAGGACCTCGTCA
AGGTCGCTAAGCAATTTGGAGTGAAATTGACGATGTTCCATGGACGAGGTGGGACTGTTGGAAGGGG
TGGTGGCCCCACTCATCTTGCTATCTTGTCTCAGCCACCGGACACGATCAATGGATCACTCCGGGTC
ACTGTTCAGGGTGAAGTTATCGAGCAGAGCTTTGGGGAAGAACACTTGTGCTTCAGGACGCTCCAGC
GTTTCACAGCTGCTACTCTTGAGCATGGGATGCGTCCACCCATTTCACCAAAGCCAGAGTGGCGAGC
TCTTCTCGATGAGATGGCTGTGGTGGCAACTGAAGAATATCGGTCAATTGTCTTCCAAGAACCACGC
TTCGTCGAGTATTTCCGCCTTGTAAGTCCTTGCTCTCATTGACCATCAGGCTAGGAAACATATTAAC
TGCTTGAATAGACTTTTCAATATGTTGCAAACCTGAACCTGCCAGTGAGATGGAATTCATGCAATGA
TTTACGGTGGAAACCTATATCATGAAAATAATTTCAAAAAATAATGAATTGTGATAGCACTTAGGGT
ACCTTCTTTTGCAACTGTAATTTCTATGACAGTACAAACCCTCGCCAATGTTATATTGGCAGCTCAG
GTTATTGCAGTAAGGTTTAAACAAAACAAAAACAAACCTTGGTGCCATTCAGGTTGAACCTTCAGTC
AAATAATTTCTTTCTGATATTTGTGTTTGACAGGCAACACCGGAGACAGAGTATGGCAGGATGAATA
TAGGGAGCAGGCCATCCAAGAGAAAGCCAAGTGGTGGCATTGAATCACTCCGTGCAATCCCATGGAT
CTTCGCATGGACGCAGACACGGTTCCACCTCCCGGTCTGGCTGGGCTTTGGTGGTGCCTTCAAGCAT
ATCCTCAAGAAGGACATCAGGAACTTCCACATGCTCCAGGAGATGTACAACGAGTGGCCATTCTTCA
GGGTCACCATCGATCTTGTTGAGATGGTGTTCGCCAAGGGTAATCCTGGCATTGCTGCCTTGTATGA
CAGGCTCCTAGTTTCAGAGGGGCTACAGCCCCTGGGTGAGAAGCTGAGGGCCAACTATGAGGAGACC
CAGAAGCTGCTTCTTCAGG

**FIGURE 4 (continued)**

TTTTTGTTACTAAAACGCAGAACTCCTGTACTTCGTTCAACAACACTGCCAGCATTGATCTTCTAGG
CCAGTTGTTAAAATATTGACAATAAAACTGGATTTTCTTGATTTCAGGTTGCTGGGCACAAGGATCT
TCTTGAAGGTGATCCCTACCTGAAGCAGCGGCTCCGCCTCCGTGACGCGTACATCACCACCATGAAC
GTCTGCCAGGCATACACATTGAAGAGGATCCGTGACCCAGACTACCATGTTGCGCTGCGCCCCCATC
TGTCCAAGGAGGTGATGGACACGAGCAAGCCGGCTGCTGAGCTCGTGACGCTGAACCCGGCGAGTGA
GTACGCGCCGGGGCTGGAGGATACCCTCATCTTGACCATGAAGGGCATTGCTGCCGGTCTCCAGAAC
ACCGGTTAGGGCAAGGGGAGATTGCCGGATTATCTTTTTTTGTACCTTGATGAGATATTTTCCTTT
TATCGAGTGCTGTGGGCCTTGTATCTCAGCGGATGTTGCTGCATCTGCCCCTATATCCAGTGAGGAA
TAATGGCATTTCGCAAGTAGTATAATATTTATAAATAAAGGCAAACAATGATAATTTCTAATCTGTT
TGTCAGTCGTACTTGGAACACTTAAGGACATGTACCATGGTGCTATCTTAGGAGTGCCATATTGGAT
AAATGATGTGGTGGAGAAAAGAGAATT

SEQ ID NO: 52, protein - Triticum aestivum
MALSAPGGGSGKIERLSSIDAQLRLLVPAKVSEDDKLIEYDALLLDRFLDVLQGLHGDDLREMVQEC
YEVAAEYETKHDLEKLDELGEMITSLDPGDSIVIAKAFSHMLNLANLAEEVQIAYRRRVKLKKGDFA
DENSAITESDIEETLKRLVFDMKKSPAEVFDALKNQTVDLVLTAHPTQSVRRSLLQKHSRIRNCLVQ
LYSKDITPDDKQELDEALQREIQAAFRTDEIRRLSPTPQDHMRAGMSDFHETIWKGVPKFLRRVDTA
LKNIGINERVPYNAPLIQFSSWMGGDRDGNPRVTPEVTRDVCLLARMMAANLYCAQIEDLMFELSMW
RCNDELRSRADELHRSSKKDAKHYIEFWKKVPPNEPYRVILGDVRDNLYNTRERSRELLSSGHSDIP
EEATLTNLEQLLEPLELCYRSLCACGDRVIADGTLLDFLRQVSTFGLSLVKLDIRQESDRHTDALDA
ITSYLGIGSYREWSEEHRQEWLLSELNGKRPLFGADLPMTEEVADVMGAFQVIAELPGDNFGAYVIS
MATSPSDVLAVELLQRECHIKTPLRVVPLFEKLADLEAAPAALARLFSIDWYRERINGKQEVMIGYS
DSGKDAGRLSAAWQMYKAQEDLVKVAKQFGVKLTMFHGRGGTVGRGGGPTHLAILSQPPDTINGSLR
VTVQGEVIEQSFGEEHLCFRTLQRFTAATLEHGMRPPISPKPEWRALLDEMAVVATEEYRSIVFQEP
RFVEYFRLATPETEYGRMNIGSRPSKRKPSGGIESLRAIPWIFAWTQTRFHLPVWLGFGGAFKHILK
KDIRNFHMLQEMYNEWPFFRVTIDLVEMVFAKGNPGIAALYDRLLVSEGLQPLGEKLRANYEETQKL
LLQVAGHKDLLEGDPYLKQRLRLRDAYITTMNVCQAYTLKRIRDPDYHVALRPHLSKEVMDTSKPAA
ELVTLNPASEYAPGLEDTLILTMKGIAAGLQNTG

SEQ ID NO: 53, DNA - Oryza sativa
AGGTGGAAGAGACGGACAGAGGCCACTACCCGTGCACCACGCCCCCTCTCCCACAATCTCGCAAAAA
AGTTGATTTTCTTTTTCAGCTTCCTCCTCCCCTCCTCCGCTTCTCATCAATCCACTAGCATATCAGA
TCAGATTCCTGTACACTTCCCTTTCCTCGCATCCAATCCCGACTATCAAATCATCACCATCCTTTTG
CAAAACCAAAAAAAAAAAAAGTAGATAAAAGAAGAAGGTTCTTGCATCCAAGAGAGGAGGACACTGTC
CGTCGTGCAGCGTTTCTTGCTTGGTTGAATCGGGTGGTTTTGGGGGGGATGGCGGGGAAGGTGGAGA
AGATGGCGTCGATCGACGCGCAGCTGCGGATGCTGGCGCCGGCGAAGCTGTCGGAGGACGACAAGCT
GGTGGAGTACGACGCCCTCCTCCTCGACCGCTTCCTCGACATCCTCCAGGACCTCCACGGCGACGAC
CTCCGCGAGCTGGTCCAAGAATGCTATGAGATCGCTGCTGAGTATGAAGGGAAGCATGACTCCCAAA
AGCTCGATGAACTTGGCAACATGTTGACAAGCTTGGATCCTGGTGACTCCATTGTGATGGCCAAAGC
TTTCTCCCACATGCTTAATTTGGCTAACTTGGCCGAGGAGGTCCAGATTGCATACAGGAGGAGAATC
AAGCTCAAGAAGGGTGACTTTGCTGATGAGAACTCGGCACTGACAGAATCTGACATTGAAGAAACTT
TTAAGAGGCTTGTTGTTGACCTGAAGAAATCACCAGCAGAGGTCTTTGATGCCCTTAAGAGTCAGAC
TGTTGACCTGGTTTTGACTGCACATCCAACACAGTCAGTGAGGAGGTCACTGCTTCAAAAACACTCA
AGGATAAGGAATTGTTTGGTTCAACTATACTCAAAGGATATCACACCAGATGATAAACAGGAGCTTG
ATGAGGCTCTCCAGAGGGAGATTCAAGCAGCCTTTAGAACTGATGAGATCCGAAGAACCCAGCCAAC
TCCTCAAGATGAAATGAGGGCTGGTATGAGTTACTTTCATGAGACAATCTGGAAAGGTGTTCCGAAG
TTCTTACGCCGTCTGGACACTGCCTTGAAGAACATCGGAATTGATGAGCGTGTTCCTTATAATGCAC
CTCTCATTCAGTTCTCTTCTTGGATGGGAGGAGACCGTGATGGAAATCC

**FIGURE 4 (continued)**

AAGAGTGACACCAGAAGTCACAAGGGATGTATGCTTGCTTGCTAGAATGATGGCTTCAAATTTATAC
TGCTCGCAAATTGAGGATCTCATGTTTGAGCTGTCTATGTGGCGATGCAATGATGAGCTTCGGGCAC
GAGCAGATGAGCTGCATCTCTCCTCAAAGAAAGATGCAAAGCACTATATAGAATTCTGGAAGAAGGT
TCCGCCCAGTGAGCCATACCGAGTTGTTCTAGGTGATGTGAGAGACAAGCTGTACAACACACGTGAA
CGAGCACGCCAGTTATTATCCAGCGGATATTCTGACATTCCTGAAGAAACCACTCTCACCAGTGTTG
AACAGTTCTTAGAGCCTCTGGAGCTATGTTACAGGTCACTCTGCGATTGCGGTGACCGTGTAATTGC
TGATGGGACCCTTCTGGACTTCTTGCGCCAGGTGTCTACCTTTGGGCTGTGCCTTGTGAGGCTTGAC
ATTAGGCAAGAATCTGACAGGCACACCGATGTCCTCGATGCCATCACCACATACCTGGGAATAGGAT
CATACCGTGAGTGGTCCGAGGAACGCCGTCAGGACTGGCTCTTGTCTGAACTCAATGGGAAGCGCCC
ATTGTTTGGCCCAGACCTTCCCAAGACCGATGAGATTGCTGACGTTCTAGACACGTTCCGCGTGATA
GCTGAGCTTCCCGCTGACAATTTTGGGGCCTATATCATTTCCATGGCGACAGCTCCGTCAGACGTGC
TTGCTGTTGAGCTCCTCCAACGTGAATGCCATGTGAAGACACCACTTAGAGTTGTCCCACTGTTTGA
GAAGTTGGCTGATCTTGAATCTGCCCCAGCAGCAGTGGCCAGACTGTTCTCCATAGATTGGTACAGA
GAAAGGATAAATGGCAAACAGGAGGTCATGATTGGCTATTCGGACTCGGGTAAGGACGCCGGTCGTC
TCTCGGCAGCTTGGCAGCTGTACAAGTCTCAGGAGGAGCTCATCAACGTTGCCAAGGAGTTTGGGGT
GAAGTTGACAATGTTCCATGGGCGCGGTGGGACTGTCGGAAGAGGCGGAGGTCCCACTCATCTTGCC
ATCTTGTCTCAGCCACCAGACACAATCCATGGATCTCTCCGGGTCACTGTCCAGGGTGAAGTCATTG
AGCAGTCCTTTGGTGAGGAGCACCTTTGCTTCAGGACACTGCAGCGTTTCACGGCTGCCACTCTGGA
GCATGGCATGCATCCACCAATTGCGCCGAAACCAGAATGGCGTGCTCTGCTTGATGAGATGGCTGTG
GTGGCTACAAAGGAATACCGGTCCATCGTCTTCCAGGAACCACGCTTTGTCGAGTATTTCCGCCTTG
CAACACCAGAGATGGAGTATGGAAGGATGAATATAGGAAGCAGGCCATCCAAGAGAAAGCCGAGTGG
TGGCATCGAGTCACTCCGTGCCATCCCATGGATCTTCGCGTGGACGCAAACTCGCTTCCACCTTCCT
GTCTGGCTTGGCTTCGGTTCTGCCTTCAAGCACATCCTGGAGAAGGACATCAGGAACCTCCACATGC
TCCAGGAGATGTACAATGAGTGGCCGTTCTTCAGAGTCACGATCGATCTGGTTGAGATGGTTTTCGC
CAAGGGTGACCCTGGCATCGCCGCATTGTACGACAAGCTCCTTGTCTCCGAGGAGCTATGGCCTCTG
GGAGAGAAACTGAGGGCCAACTGTGAAGAAACCAAACAGCTTCTCCTTCAGGTTGCTGGACACAAGG
ATCTTCTCGAAGGTGATCTCTACCTGAAGCAGCGCCTTCGCCTCCGCAATGCCTACATCACCACCCT
GAATGTCTGCCAGGCTTACACGATGAAGCGAATCCGCGACCCGGACTACCATGTCACGCTGCGCCCC
CACATGTCCAAGGAGATCATGGACTGGTCTAAGCCGGCTGCGGAGCTGGTGAAGCTGAACCCGACCA
GTGAGTATGCGCCGGGGCTGGAGGACACCCTCATCCTGACCATGAAGGGAATTGCTGCTGGAATGCA
GAACACCGGCTAAGCGAACTATCAACTATGAAGTGCCTGAATTCACACCGTTTTATATGGACTCTTA
TCTTGATTTCGGTCATTTGCTCTCGGCCATTTAATTTTGCCGATGCAGAGGCTCGTGCAGTTTCTAA
TTTCTTTCGGAAGATATTTTCAATAATGGAGTTGTACAAGAACATCAATTAATCAAAATAAAGTGGA
TATTATGTTCATTATCGTTTAGAGCAACCTGGTAAAGCCATTTTTTTGTGTAGACCACATAGTGAGT
GTGTTGAAATTCCATTTGCTTAATGAAACTGCCATTATCTAAGTAAAGTCAAAGGGCTCCTTTATAT
TG

SEQ ID NO: 54, protein - Oryza sativa
MAGKVEKMASIDAQLRMLAPAKLSEDDKLVEYDALLLDRFLDILQDLHGDDLRELVQECYEIAAEYE
GKHDSQKLDELGNMLTSLDPGDSIVMAKAFSHMLNLANLAEEVQIAYRRRIKLKKGDFADENSALTE
SDIEETFKRLVVDLKKSPAEVFDALKSQTVDLVLTAHPTQSVRRSLLQKHSRIRNCLVQLYSKDITP
DDKQELDEALQREIQAAFRTDEIRRTQPTPQDEMRAGMSYFHETIWKGVPKFLRRLDTALKNIGIDE
RVPYNAPLIQFSSWMGGDRDGNPRVTPEVTRDVCLLARMMASNLYCSQIEDLMFELSMWRCNDELRA
RADELHLSSKKDAKHYIEFWKKVPPSEPYRVVLGDVRDKLYNTRERARQLLSSGYSDIPEETTLTSV
EQFLEPLELCYRSLCDCGDRVIADGTLLDFLRQVSTFGLCLVRLDIRQESDRHTDVLDAITTYLGIG
SYREWSEERRQDWLLSELNGKRPLFGPDLPKTDEIADVLDTFRVIAELPADNFGAYIISMATAPSDV
LAVELLQRECHVKTPLRVVPLFEKLADLESAPAAVARLFSIDWYRERINGKQEVMIGYSDSGKDAGR
LSAAWQLYKSQEELINVAKEFGVKLTMFHGRGGTVGRGGGPTHLAILSQPPDTIHGS

## FIGURE 4 (continued)

LRVTVQGEVIEQSFGEEHLCFRTLQRFTAATLEHGMHPPIAPKPEWRALLDEMAVVATKEYRSIVFQ
EPRFVEYFRLATPEMEYGRMNIGSRPSKRKPSGGIESLRAIPWIFAWTQTRFHLPVWLGFGSAFKHI
LEKDIRNLHMLQEMYNEWPFFRVTIDLVEMVFAKGDPGIAALYDKLLVSEELWPLGEKLRANCEETK
QLLLQVAGHKDLLEGDLYLKQRLRLRNAYITTLNVCQAYTMKRIRDPDYHVTLRPHMSKEIMDWSKP
AAELVKLNPTSEYAPGLEDTLILTMKGIAAGMQNTG

SEQ ID NO: 55, DNA - Oryza sativa
ATGTCGGCGATGTCGGCGGCGGGCGGCGGCGGCGGCGTGGGGAAGGTGGAGCGGCTGTCGTCGATCG
ACGCGCAGCTGCGGCAGCTGGTGCCGGCGAAGCTGTCGGAGGACGACAAGCTCATCGAGTACGACGC
GCTCCTCCTCGACCGATTCCTCGACGTCCTCCATGGCCTCCACGGCGACGACCTCAAGGATCTCGTT
CAAGAATGCTATGAGGTGGCCGCGGAGTATGAAACAAAGCACGACGTGCAAAAGCTCGATGAACTCG
GCAACATGATAACAAGCTTGGATCCGGGCGACTCGATTGTTATTGCGAAAGCCTTTTCGCACATGCT
TAACTTGGCCAACTTGGCTGAAGAAGTCCAGATCGCATACAGGAGGAGGATCAAGCTCAAGAAGGGT
GATTTCGCTGACGAAAACTCGGCAATGACTGAATCCGACATTGAGGAAACACTCAAGAGGCTTGTTT
TTGACCTGAAGAAATCCCCTGCTGAGGTCTTTGATGCCCTCAAGAGTCAGACTGTTGACCTTGTTTT
GACTGCACATCCAACACAGTCTGTGAGGAGGTCACTGCTCCAGAAACACTCCAGGATTCGGAACTGT
CTGGTTCAGCTTTATTCAAAGGATATCACTCCAGATGACAAGCAAGAGCTTGATGAGGCTCTCCAGA
GAGAGATCCAAGCTGCCTTTAGAACCGATGAGATCCGAAGAACACAGCCCACTCCCCAAGATGAAAT
GCGTGCTGGCATGAGCTACTTCCATGAAACAATATGGAAGGGTGTTCCAAAGTTCTTGCGCCGTGTG
GATACTGCATTGAAGAACATCGGTATCGACGAGCGTGTTCCTTACAATGCTCCTCTTATTCAGTTCT
CGTCTTGGATGGGAGGAGATCGTGATGGAAATCCAAGAGTCACACCAGAGGTTACAAGGGATGTCTG
CTTGCTTGCAAGAATGATGGCAGCAAACTTGTATTGCTCACAAATTGAGGATCTCATGTTTGAGTTA
TCTATGTGGCGATGTAACGAGGAGCTACGCTCACGAGCTGATGATCTGCACCGCTCTTCTAAGAAGG
ACGCAAAGCATTACATAGAGTTTTGGAAGAAGGTTCCTCTGAATGAACCATACCGGGTGATACTGAG
TGATGTGAGAGATAAGCTTTACAATACACGTGAACGATCACGGGAGTTGTTGTCCAGCGGATATTGC
GACATCCCTGAGGAAGCAACTCTGACAAATGTTGAGCAGCTATTGGAGCCCTTGGAGCTATGCTACA
GGTCGCTGTGCGCCTGTGGTGACCGTGCCATCGCTGATGGAAGCCTTCTTGATTTCTTGCGCCAAGT
TTCCACCTTTGGACTCTCCCTTGTGAGGCTTGACATTAGGCAAGAATCCGACAGACACACCGATGTC
CTTGATGCCATCACGACATACCTGGGCATAGGATCTTACCGTGAGTGGTCCGAGGAACGCCGTCAAG
AGTGGCTGTTGTCCGAACTCAACGGGAAGCGACCATTGTTTGGCCCAGACCTTCCCAGGACAGATGA
GGTTGCTGATGTTCTAGACACATTCCATGTGATTGCCGAGCTCCCTGCTGACAGTTTTGGAGCCTAT
GTTATTTCCATGGCAACAGCCCCCTCGGATGTCCTTGCTGTTGAGCTCCTCCAACGCGAGTGCCATG
TCAAGACACCGCTTAGAGTTGTCCCCCTGTTCGAGAAGTTGGCTGATCTTGAGTCTGCCCCTGCGGC
ATTGACCAGGCTCTTCTCAATAAGTTGGTACAGGCAGAGGATCAATGGTAAGCAGGAGGTCATGATT
GGGTATTCAGATTCAGGCAAGGATGCTGGCCGTCTCTCGGCAGCTTGGCAGATGTACAAGGCTCAGG
AGCAGCTCGTCAAGGTAGCGAAGGATTTCGGGGTCAAGTTGACGATGTTCCATGGACGAGGTGGAAC
CGTTGGTAGGGGCGGTGGGCCGACTCACCTTGCCATCTTGTCTCAGCCTCCAGACACGATCAACGGA
TCGCTCCGTGTCACTGTTCAGGGTGAAGTCATTGAGCAGAGCTTTGGTGAGGAGCACTTGTGTTTCA
GGACACTGCAGCGTTTCACCGCTGCTACACTTGAGCACGGCATGCATCCACCCAGTGCACCGAAGCC
AGAATGGCGAGCTCTTCTTGATGAGATGGCTGTGGTGGCAACCAAGGAGTATCGATCCGTAGTGTTC
CAAGAGCCACGCTTTGTGGAGTATTTCCGCCTTGCAACACCTGAGACAGAGTATGGAAGGATGAACA
TAGGAAGCAGGCCATCCAAGAGGAAGCCGAGTGGAGGCATTGAATCACTCCGTGCGATCCCATGGAT
CTTCGCATGGACGCAGACCCGCTTCCACCTTCCCGTCTGGCTTGGATTCGGTGCTGCATTCAAGCAC
GCCCTTCAGAAGGACATCAGGAATCTCCACATGCTCCAGGAGATGTACAACGAGTGGCCGTTCTTCA
GGGTCACTCTCGACCTGATTGAGATGGTTTTCGCCAAGGGTAATCCTGGCATTGCTGCCCTTTACGA
CAAACTCCTCGTTTCTGAGGATCTACAGCCACTGGGTGAGAAGCTGAGAGCCAACTATGTAGAGACA
CAAAAGCTGCTTCTTCAGGTTGCTGGCCATAGGGATCTTCTTGAAGGTGACCCCTACCTGAAGCAGC
GGCTCCGACTCCGCGACGCCTACATA

## FIGURE 4 (continued)

ACCACCCTGAATGTCTGCCAAGCCTACACGCTGAAACGGATCCGCGACCCGGATTACCACGTCACGC
TGCGCCCTCACCTGTCCAAGGAGGTGATGGATGGGAGCAAGCCGGCCGCGGAGCTTGTGAAGCTGAA
CCCGGGTAGCGAGTACGCGCCAGGGCTGGAGGACACCCTCATCCTGACCATGAAGGGCATTGCTGCA
GGGTTGCAGAACACTGGTTAA

SEQ ID NO: 56, protein - Oryza sativa
MSAMSAAGGGGGVGKVERLSSIDAQLRQLVPAKLSEDDKLIEYDALLLDRFLDVLHGLHGDDLKDLV
QECYEVAAEYETKHDVQKLDELGNMITSLDPGDSIVIAKAFSHMLNLANLAEEVQIAYRRRIKLKKG
DFADENSAMTESDIEETLKRLVFDLKKSPAEVFDALKSQTVDLVLTAHPTQSVRRSLLQKHSRIRNC
LVQLYSKDITPDDKQELDEALQREIQAAFRTDEIRRTQPTPQDEMRAGMSYFHETIWKGVPKFLRRV
DTALKNIGIDERVPYNAPLIQFSSWMGGDRDGNPRVTPEVTRDVCLLARMMAANLYCSQIEDLMFEL
SMWRCNEELRSRADDLHRSSKKDAKHYIEFWKKVPLNEPYRVILSDVRDKLYNTRERSRELLSSGYC
DIPEEATLTNVEQLLEPLELCYRSLCACGDRAIADGSLLDFLRQVSTFGLSLVRLDIRQESDRHTDV
LDAITTYLGIGSYREWSEERRQEWLLSELNGKRPLFGPDLPRTDEVADVLDTFHVIAELPADSFGAY
VISMATAPSDVLAVELLQRECHVKTPLRVVPLFEKLADLESAPAALTRLFSISWYRQRINGKQEVMI
GYSDSGKDAGRLSAAWQMYKAQEQLVKVAKDFGVKLTMFHGRGGTVGRGGGPTHLAILSQPPDTING
SLRVTVQGEVIEQSFGEEHLCFRTLQRFTAATLEHGMHPPSAPKPEWRALLDEMAVVATKEYRSVVF
QEPRFVEYFRLATPETEYGRMNIGSRPSKRKPSGGIESLRAIPWIFAWTQTRFHLPVWLGFGAAFKH
ALQKDIRNLHMLQEMYNEWPFFRVTLDLIEMVFAKGNPGIAALYDKLLVSEDLQPLGEKLRANYVET
QKLLLQVAGHRDLLEGDPYLKQRLRLRDAYITTLNVCQAYTLKRIRDPDYHVTLRPHLSKEVMDGSK
PAAELVKLNPGSEYAPGLEDTLILTMKGIAAGLQNTG

**SEQ ID NO: 57, DNA - Oryza sativa**
CCCACACTCTCTACCCCCAAACTTGCCCTCCAATCTATCTCCGCCGCCGCCGCCGCCTCCGCCGCGA
GAGGGCGTCGCCGAGCTGCGCGGGAGGCTCGCCTCCCGCAGCATCTGGCCGGATTCGTCGTAGTCGA
GGCAGCTGTAAGCGAGAGAGAGGGAGATGGCCGCGGCCGCCGGGAAGGCGGCGATGGAGCGGCACCA
GTCGATCGACGCGCAGCTGCGGCTGCTCGCGCCGGGGAAGGTATCCGAGGACGACAAGCTCGTCGAG
TACGACGCCCTCCTCGTCGACCGCTTCCTCGACATCCTCCAGGACCTGCACGGCCCTCACCTCCGCG
AATTCGTGCAGGAGTGCTACGAGCTGTCGGCGGAGTACGAGAACGACAGGGACGAGGCGCGGCTCGA
CGAGCTGGGGAGGAAGCTCACCAGCCTGCCGCCGGGGGACTCCATCGTCGTCTCCAGCTCCTTCTCG
CACATGCTCAACCTCGCCAACCTCGCCGAGGAGGTGCAGATCGCGCACCGCCGCCGGATCAAGCTCA
AGCGCGGGGACTTCGCCGACGAGGCCTCCGCGCCCACCGAGTCCGACATCGAGGAGACGCTCAAGCG
CCTCGTCACCCAGCTCGGCAAGTCGAGGGAGGAGGTGTTCGATGCGCTCAAGAACCAGACCGTTGAC
CTCGTGTTCACCGCGCATCCAACGCAGTCCGTCAGGAGGTCCCTGCTCCAGAAGCACGGGAGGATCC
GGAATTGCCTGAGGCAGTTATATGCGAAAGACATCACTGCTGATGACAAGCAGGAGCTTGATGAGGC
CCTGCAGAGGGAGATTCAAGCAGCTTTCAGAACTGATGAAATCCGCAGGACTCCTCCCACTCCTCAG
GATGAAATGCGCGCCGGGATGAGTTATTTTCATGAAACTATATGGAAGGGTGTACCAAAGTTCTTGC
GCCGCATTGATACTGCTCTGAAAAATATTGGGATTAATGAGCGTCTTCCTTACAATGCTCCTCTCAT
CCAGTTCTCATCCTGGATGGGTGGTGACCGTGATGGAAACCCAAGAGTTACACCAGAGGTTACAAGA
GATGTATGCTTGTTGGCAAGAATGATGGCTGCTAACCTGTACTTCTCTCAGATAGAAGATCTGATGT
TTGAGCTCTCTATGTGGCGCTGCAGTGATGAACTTCGAATTCGTGCAGATGATTTGCATTGCTCCTC
CAGAAAAGCTGCAAAGCACTATATAGAATTCTGGAAGCAAATTCCTCCAAATGAGCCTTATCGTGTC
ATACTTGGTGGTGTCAGGGATAAATTGTACTATACGCGTGAACGTACTCGCCATCTATTGACAACTG
GAGTTTCTGAAATTCCAGAGGAGGCAACGTTTACTAATGTTGAAGAGTTTCTGGAGCCGCTTGAGCT
GTGCTACAGATCATTATGTGCTTGTGGTGACAAACCTATAGCTGATGGAAGCCTTCTTGATTTCTTG
CGTCAAGTATCAACTTTTGGGCTGGCTCTTGTAAAACTTGACATAAGGCAGGAGTCTGATCGACACA
CTGATGTCCTTGATGCCATAACTACATATCTTGGGATTGGATCTTACGCTGAATGGTCTGAGGAGAA
ACGCCAGGATTGGCTTTTGTCCGAACTAAGGGGCAAACGTCC

## FIGURE 4 (continued)

TTTGTTTGGTCCTGATCTTCCTCAGACTGAAGAAATTGCTGATGTTTTAGGAACATTTCACGTCCTT
GCTGAGCTCCCGGCAGATTGTTTTGGTGCTTACATCATCTCAATGGCAACTGCACCATCTGATGTGC
TTGCTGTGGAGCTTCTACAGCGGGAGTGCCATATAAAACAGCCTCTGCGAGTTGTTCCCCTATTTGA
GAAACTTGCAGATCTTGAAGCAGCTCCAGCAGCGGTGGCACGACTATTTTCAATAGACTGGTACATG
AATAGGATTAATGGCAAGCAGGAGGTCATGATTGGATACTCAGACTCTGGCAAGGATGCTGGTCGTC
TTTCTGCAGCATGGCAAATGTACAAAGCACAAGAGGAGCTTGTCAAGGTGGCAAAGCATTATGGTGT
AAAGTTGACAATGTTCCATGGAAGAGGTGGAACAGTTGGCAGAGGAGGTGGTCCCAGTCATCTTGCC
ATATTATCTCAACCACCAGACACGATACATGGATCACTTCGTGTAACAGTACAGGGCGAGGTTATTG
AACACTCGTTTGGAGAGGAACACTTGTGCTTTAGAACTCTGCAGCGCTTTACTGCAGCTACTCTTGA
GCATGGAATGCATCCTCCAATTTCCCCCAAACCAGAATGGCGTGCTCTAATGGATGAGATGGCTGTT
GTGGCAACAAAGGAATATCGATCAATCGTCTTCAAGGAGCCACGGTTTGTTGAATACTTCCGATCGG
CAACACCTGAGACAGAATATGGCAGGATGAACATTGGTAGCCGACCATCAAAGAGAAAGCCTAGTGG
TGGCATTGAATCGCTCCGTGCAATTCCTTGGATTTTTGCTTGGACACAAACTAGGTTTCATCTTCCT
GTATGGCTTGGATTTGGTGGAGCGTTCAAACATATAATGCAGAAGGATATCAGGAACATCCATACAC
TGAAAGAAATGTACAATGAGTGGCCATTCTTCAGGGTCACCTTGGACTTGCTTGAGATGGTTTTCGC
TAAGGGAGATCCAGGAATTGCTGCTTTATATGACAAATTGCTTGTGGCAGGTGATCTGCAATCCTTT
GGAGAGCAGCTGAGAAACAACTTTGAAGAGACAAAACAGCTACTCCTTCAGGTTGCCGGTCACAAGG
ATATTCTGGAAGGGGATCCTTACCTGAAGCAGCGTCTGCGGCTGCGTGAGTCGTACATCACTACCTT
GAACGTTTGCCAAGCCTACACCCTGAAGCGGATAAGGGACCCTAGCTTCGAGGTGATGTCGCAGCCG
GCCTTGTCGAAGGAGTTCGTCGACAGCAACCAGCCTGCCGAGCTGGTCCAACTGAACGCTGCGAGCG
AGTACGCACCTGGCCTGGAGGACACCCTCATCCTGACGATGAAGGGCATTGCTGCCGGCATGCAGAA
CACAGGCTAGATTGTCCACAAGAATGCTTCCAGCAGGTGCAATCAATCAATCATCAATAACTCCTTC
CATGATGAGATGGTGAATATGTTTAACTAGTACGTTCGCCGATGCTAGTGAATTCTGGAGACTTTTT
TTTGCTCGCACGTTTTCTTTTGGTTGTGTACTTGATGTAAATGTCAAGGGGGGACTTATTTGGCTTT
TGCAGCCCGATCCAAAATAAAATATGGACCAATCATTCTACGAAAAAAA

SEQ ID NO: 58, protein - Oryza sativa
MERHQSIDAQLRLLAPGKVSEDDKLVEYDALLVDRFLDILQDLHGPHLREFVQECYELSAEYENDRD
EARLDELGRKLTSLPPGDSIVVSSSFSHMLNLANLAEEVQIAHRRRIKLKRGDFADEASAPTESDIE
ETLKRLVTQLGKSREEVFDALKNQTVDLVFTAHPTQSVRRSLLQKHGRIRNCLRQLYAKDITADDKQ
ELDEALQREIQAAFRTDEIRRTPPTPQDEMRAGMSYFHETIWKGVPKFLRRIDTALKNIGINERLPY
NAPLIQFSSWMGGDRDGNPRVTPEVTRDVCLLARMMAANLYFSQIEDLMFELSMWRCSDELRIRADD
LHCSSRKAAKHYIEFWKQIPPNEPYRVILGGVRDKLYYTRERTRHLLTTGVSEIPEEATFTNVEEFL
EPLELCYRSLCACGDKPIADGSLLDFLRQVSTFGLALVKLDIRQESDRHTDVLDAITTYLGIGSYAE
WSEEKRQDWLLSELRGKRPLFGPDLPQTEEIADVLGTFHVLAELPADCFGAYIISMATAPSDVLAVE
LLQRECHIKQPLRVVPLFEKLADLEAAPAAVARLFSIDWYMNRINGKQEVMIGYSDSGKDAGRLSAA
WQMYKAQEELVKVAKHYGVKLTMFHGRGGTVGRGGGPSHLAILSQPPDTIHGSLRVTVQGEVIEHSF
GEEHLCFRTLQRFTAATLEHGMHPPISPKPEWRALMDEMAVVATKEYRSIVFKEPRFVEYFRSATPE
TEYGRMNIGSRPSKRKPSGGIESLRAIPWIFAWTQTRFHLPVWLGFGGAFKHIMQKDIRNIHTLKEM
YNEWPFFRVTLDLLEMVFAKGDPGIAALYDKLLVAGDLQSFGEQLRNNFEETKQLLLQVAGHKDILE
GDPYLKQRLRLRESYITTLNVCQAYTLKRIRDPSFEVMSQPALSKEFVDSNQPAELVQLNAASEYAP
GLEDTLILTMKGIAAGMQNTG

SEQ ID NO: 59, DNA - Oryza sativa
ACCCACACACCCACACTCTCTACCCCCAAACTTGCCCTCCAATCTATCTCCGCCGCCGCCGCCGCCT
CCGCCGCGAGAGGGCGTCGCCGAGCTGCGCGGGAGGCTCGCCTCCCGCAGCATCTGGCCGGATTCGT
CGTAGTCGAGGCAGCTGTAAGCGAGAGAGAGGGAGATGGCCGCGGCCGCCGGGAAGGCGGCGAT

**FIGURE 4 (continued)**

GGAGCGGCACCAGTCGATCGACGCGCAGCTGCGGCTGCTCGCGCCGGGGAAGGTATCCGAGGACGAC
AAGCTCGTCGAGTACGACGCCCTCCTCGTCGACCGCTTCCTCGACATCCTCCAGGACCTGCACGGCC
CTCACCTCCGCGAATTCGTGCAGGAGTGCTACGAGCTGTCGGCGGAGTACGAGAACGACAGGGACGA
GGCGCGGCTCGACGAGCTGGGGAGGAAGCTCACCAGCCTGCCGCCGGGGGACTCCATCGTCGTCTCC
AGCTCCTTCTCGCACATGCTCAACCTCGCCAACCTCGCCGAGGAGGTGCAGATCGCGCACCGCCGCC
GGATCAAGCTCAAGCGCGGGGACTTCGCCGACGAGGCCTCCGCGCCCACCGAGTCCGACATCGAGGA
GACGCTCAAGCGCCTCGTCACCCAGCTCGGCAAGTCGAGGGAGGAGGTGTTCGATGCGCTCAAGAAC
CAGACCGTTGACCTCGTGTTCACCGCGCATCCAACGCAGTCCGTCAGGAGGTCCCTGCTCCAGAAGC
ACGGGAGGATCCGGAATTGCCTGAGGCAGTTATATGCGAAAGACATCACTGCTGATGACAAGCAGGA
GCTTGATGAGGCCCTGCAGAGGGAGATTCAAGCAGCTTTCAGAACTGATGAAATCCGCAGGACTCCT
CCCACTCCTCAGGATGAAATGCGCGCCGGGATGAGTTATTTCATGAAACTATATGGAAGGGTGTAC
CAAAGTTCTTGCGCCGCATTGATACTGCTCTGAAAAATATTGGGATTAATGAGCGTCTTCCTTACAA
TGCTCCTCTCATCCAGTTCTCATCCTGGATGGGTGGTGACCGTGATGGAAACCCAAGAGTTACACCA
GAGGTTACAAGAGATGTATGCTTGTTGGCAAGAATGATGGCTGCTAACCTGTACTTCTCTCAGATAG
AAGATCTGATGTTTGAGCTCTCTATGTGGCGCTGCAGTGATGAACTTCGAATTCGTGCAGATGATTT
GCATTGCTCCTCCAGAAAAGCTGCAAAGCACTATATAGAATTCTGGAAGCAAATTCCTCCAAATGAG
CCTTATCGTGTCATACTTGGTGGTGTCAGGGATAAATTGTACTATACGCGTGAACGTACTCGCCATC
TATTGACAACTGGAGTTTCTGAAATTCCAGAGGAGGCAACGTTTACTAATGTTGAAGAGTTTCTGGA
GCCGCTTGAGCTGTGCTACAGATCATTATGTGCTTGTGGTGACAAACCTATAGCTGATGGAAGCCTT
CTTGATTTCTTGCGTCAAGTATCAACTTTTGGGCTGGCTCTTGTAAAACTTGACATAAGGCAGGAGT
CTGATCGACACACTGATGTCCTTGATGCCATAACTACATATCTTGGGATTGGATCTTACGCTGAATG
GTCTGAGGAGAAACGCCAGGATTGGCTTTTGTCCGAACTAAGGGGCAAACGTCCTTTGTTTGGTCCT
GATCTTCCTCAGACTGAAGAAATTGCTGATGTTTTAGGAACATTTCACGTCCTTGCTGAGCTCCCGG
CAGATTGTTTTGGTGCTTACATCATCTCAATGGCAACTGCACCATCTGATGTGCTTGCTGTGGAGCT
TCTACAGCGGGAGTGCCATATAAAACAGCCTCTGCGAGTTGTTCCCCTATTTGAGAAACTTGCAGAT
CTTGAAGCAGCTCCAGCAGCGGTGGCACGACTATTTTCAATAGACTGGTACATGAATAGGATTAATG
GCAAGCAGGAGGTCATGATTGGATACTCAGACTCTGGCAAGGATGCTGGTCGTCTTTCTGCAGCATG
GCAAATGTACAAAGCACAAGAGGAGCTTGTCAAGGTGGCAAAGCATTATGGTGTAAAGTTGACAATG
TTCCATGGAAGAGGTGGAACAGTTGGCAGAGGAGGTGGTCCCAGTCATCTTGCCATATTATCTCAAC
CACCAGACACGATACATGGATCACTTCGTGTAACAGTACAGGGCGAGGTTATTGAACACTCGTTTGG
AGAGGAACACTTGTGCTTTAGAACTCTGCAGCGCTTTACTGCAGCTACTCTTGAGCATGGAATGCAT
CCTCCAATTTCCCCCAAACCAGAATGGCGTGCTCTAATGGATGAGATGGCTGTTGTGGCAACAAAGG
AATATCGATCAATCGTCTTCAAGGAGCCACGGTTTGTTGAATACTTCCGATCGGCAACACCTGAGAC
AGAATATGGCAGGATGAACATTGGTAGCCGACCATCAAAGAGAAAGCCTAGTGGTGGCATTGAATCG
CTCCGTGCAATTCCTTGGATTTTTGCTTGGACACAAACTAGGTTTCATCTTCCTGTATGGCTTGGAT
TTGGTGGAGCGTTCAAACATATAATGCAGAAGGATATCAGGAACATCCATACACTGAAAGAAATGTA
CAATGAGTGGCCATTCTTCAGGGTCACCTTGGACTTGCTTGAGATGGTTTTCGCCAAGGGAGATCCA
GGAATTGCTGCTTTATATGACAAATTGCTTGTGGCAGGTGATCTGCAATCCTTTGGAGAGCAGCTGA
GAAACAACTTTGAAGAGACAAAACAGCTACTCCTTCAGGTTGCCGGTCACAAGGATATTCTGGAAGG
GGATCCTTACCTGAAGCAGCGTCTGCGGCTGCGTGAGTCGTACATCACTACCTTGAACGTTTGCCAA
GCCTACACCCTGAAGCGGATAAGGGACCCTAGCTTCGAGGTGATGTCGCAGCCGGCCTTGTCGAAGG
AGTTCGTCGACAGCAACCAGCCTGCCGAGCTGGTCCAACTGAACGCTGCGAGCGAGTACGCACCTGG
CCTGGAGGACACCCTCATCCTGACGATGAAGGGCATTGCTGCCGGCATGCAGAACACAGGCTAGATT
GTCCACAAGAATGCTTCCAGCAGGTGCAATCAATCAATCATCAATAACTCCTTCCATGATGAGATGG
TGAATATGTTTAACTACCCGATCCAAAATAAAATATGGACCAATCATTCTACGAAGCTTGATTCTGA
ACGCTTCGAATTTGCATCAGTGGTGACAGCGGCTGTGTTTGCATCTTTAAACGACAGACTAAAAGCA
TTGTGCACCGCAAGATGCAGACTTGACATGTGCTTATAGCAGAAGTATTACATGGAGCACATCTCAG
GGTCAGGTATATTGCAATC

**FIGURE 4 (continued)**

TCAAAAAATCAGGAGATACACAATCAGATGGATATGAAATGAGAAGCAAAATTGTGTCTGAAGAAATG
AGAAGCAAGAAGACAGTTAAAGGGTCTGATGGGTTACAGTCTGAAAAATCAGGGGATATGCAATCGG
AGAGATATGAAAGTGACAAAATTTTGTCCAGAGAAATAAGAAGCAAGAAAACATTGAAAGGTCAGTT
ATCATGCTCACCTAGCATTATGCACCATAAAATGATCTTGGCAGACAATATAATCTAGCATAAAATT
ATATACAACAAATTAGAGATTGTAGCGTTGATAAATTTTCAAGGACTATATATCTATAGTTAGTTAC
AGTGAGAAATAACAAAAGTTACATATGTAACTACAATACATATAGACTGTAACTGGGCTAAAACTGC
AAGCATGCTTGAATTTCTTCAAAAAATAAAGAATTTCTTC

SEQ ID NO: 60, protein - Oryza sativa
MAAAAGKAAMERHQSIDAQLRLLAPGKVSEDDKLVEYDALLVDRFLDILQDLHGPHLREFVQECYEL
SAEYENDRDEARLDELGRKLTSLPPGDSIVVSSSFSHMLNLANLAEEVQIAHRRRIKLKRGDFADEA
SAPTESDIEETLKRLVTQLGKSREEVFDALKNQTVDLVFTAHPTQSVRRSLLQKHGRIRNCLRQLYA
KDITADDKQELDEALQREIQAAFRTDEIRRTPPTPQDEMRAGMSYFHETIWKGVPKFLRRIDTALKN
IGINERLPYNAPLIQFSSWMGGDRDGNPRVTPEVTRDVCLLARMMAANLYFSQIEDLMFELSMWRCS
DELRIRADDLHCSSRKAAKHYIEFWKQIPPNEPYRVILGGVRDKLYYTRERTRHLLTTGVSEIPEEA
TFTNVEEFLEPLELCYRSLCACGDKPIADGSLLDFLRQVSTFGLALVKLDIRQESDRHTDVLDAITT
YLGIGSYAEWSEEKRQDWLLSELRGKRPLFGPDLPQTEEIADVLGTFHVLAELPADCFGAYIISMAT
APSDVLAVELLQRECHIKQPLRVVPLFEKLADLEAAPAAVARLFSIDWYMNRINGKQEVMIGYSDSG
KDAGRLSAAWQMYKAQEELVKVAKHYGVKLTMFHGRGGTVGRGGGPSHLAILSQPPDTIHGSLRVTV
QGEVIEHSFGEEHLCFRTLQRFTAATLEHGMHPPISPKPEWRALMDEMAVVATKEYRSIVFKEPRFV
EYFRSATPETEYGRMNIGSRPSKRKPSGGIESLRAIPWIFAWTQTRFHLPVWLGFGGAFKHIMQKDI
RNIHTLKEMYNEWPFFRVTLDLLEMVFAKGDPGIAALYDKLLVAGDLQSFGEQLRNNFEETKQLLLQ
VAGHKDILEGDPYLKQRLRLRESYITTLNVCQAYTLKRIRDPSFEVMSQPALSKEFVDSNQPAELVQ
LNAASEYAPGLEDTLILTMKGIAAGMQNTG

SEQ ID NO: 61, DNA - Oryza sativa
GCATTGGCCGTTCGGATCGCGGTGGCGCTTCGTGAAGGTGGGGGAGAAGGGGGAAAAGGAGTCGGCT
TTCGGGGAGATGGCGCGCAATGCGGCGGACAAGGGGACGTCCATCGACGCGCAGCTGCGGATCCTGG
CGCCTAAGAAGCTCTCGGAGGACGACAAGCTTGTGGAGTACGACGCGCTGCTCCTCGATCGCTTCCT
CGACATCCTCCAGGATCTGCATGGGGAGGACATCAGGGAGACGGTTCAAGAATGCTATGAATTAGCT
GCTGAGTATGAAAGTAAGATTGCGTATCGTAGGAGGATAAAACTGAAGAAGGGTGATTTTGCTGATG
AAAACTCTGCTACAACTGAATCAAACTTTGAGGAGACCCTAAAAAGGCTTGTTGGTGAGCTTAAGAA
GTCCCCTCATGAGGTATTTGATGCTCTCAAGAATCAAACAATCGACCTGGTCCTGACAGCACATCCA
ACTCAGTCAGTCAGGAGGTCGCTGCTACAAAAGCATGGCAGGATAAGAAGTTGTTTAACAAAACTTT
ATGCAAAAGACATAACTCCAGATGAGAAGCAGGAACTCGATGAAGCACTTAAGAGAGAGATCCAAGC
CGCCTTTAGAACAGATGAAATTCGGCGAGCACCTCCTACACCACAGGATGAAATGCGTGCTGGAATG
AGTTATTTTCATGAGACAATATGGAAGGGTGTACCCAAGTTTTTACGTAGGGTTGATACTGCCCTTA
AGAACATTGGCATAAACGAGCGAGTGCCCTATAATGCTCCTCTTATTCAATTTTCTTCTTGGATGGG
TGGAGACCGTGATGGAAATCCAAGAGTCACACCAGAGGTTACAAGGGACGTATGCCTGTTAGCTAGA
ATGATGGCTGCTAACTTATACTATGCACAGATAGAGGATCTGATGTTTGAGCTATCTATGTGGCGCT
GTAGTGATGAACTACGTGTAAAAGCTGATCAGTTACACCGTTGCGCAAAGAAAAACACAACAAAGCA
CTATATAGAGTTCTGGAAACAAGTTCCTCCGAGTGAACCCTATCGTGTAATACTGAGCAATGTCAGA
GATAAGTTGTACAATACACGCGAGCGAGCACGCCATTTATTAGCCAGTGGATTTTCTGAAATTCCTG
ACGAAGCAACATTCACTGATGTTGAGCAGTTCTTGGAGCCTCTTGAGCTCTGTTACAGGTCTCTCTG
TGCCTGTGGTGATAATTCTATTGCAGATGGCAGTCTTCTTGACTTTCTACGGCAAGTGTCCACATTT
GGACTATCCCTTGTTAGACTTGATATTAGGCAAGAATCTGACCGACATACTGATGTTATGGATGCCA
TAACTCAATACCTTGGAATTGGATCCTATCGTGAATGGTCAGAGGAGAAACGCCAAGAATGGCTTCT
GTCAGAACTCAATGGAAAGAGGCCATTATTTGGTCCTGACCTTCC

**FIGURE 4 (continued)**

CCAGACGGATGAAATTGCTGATGCCCTAGATACTTTTCATGTGATAGCAGAACTACCCTATGATAGC
TTTGGTGCATATGTAATATCCATGGCAACAGCTCCTTCAGATGTTCTAGCAGTTGAGCTTCTGCAAC
GTGAATGTCATGTGAAGAAGCCACTGAGAGTTGTGCCATTGTTTGAAAAACTAGCAGATCTGGAAGC
AGCACCAGCAGCTCTTGCCCGACTTTTCTCTGTTGACTGGTACAGAAATAGGATCGACGGAAAGCAG
GAAGTAATGATTGGGTATTCAGATTCTGGAAAAGATGCTGGCCGTTTCTCTGCAGCTTGGGAACTGT
ATAAAGCTCAAGAGGAGCTTATTAAAGTTGCTAAGCAGTTTGGGGTTAAGTTGACTATGTTTCATGG
GCGAGGTGGAACTGTTGGAAGAGGTGGGGGTCCTACACACCTTGCTATACTGTCACAACCTCCAGAT
ACCATTCATGGATCACTTCGTGTGACTGTTCAAGGTGAAGTCATTGAGCAATCTTTTGGAGAGGAGC
ATTTGTGTTTTAGGACACTTCAACGTTTTACATCTGCTACTCTTGAGCATGGTATGCATCCACCAAT
TTCACCAAAGCCAGAATGGCGTGCTTTGATGGATGAAATGGCCGCTGTTGCTACAAAAGAATACCGG
TCCATTGTCTTCCAAGAGGCACGATTTGTTGAATATTTCCGCCTTGCAACACCAGAGTTGGAATATG
GTAGGATGAATATTGGAAGTAGGCCATCAAAACGAAAGCCTAGTGGAGGCATTGAATCACTTCGTGC
AATTCCTTGGATCTTTGCTTGGACACAAACTCGATTCCACCTACCAGTGTGGCTTGGTTTTGGCGCA
GCGTTCAAGCATGTCTTGCAGAAGGACATACGCAATCTTCAGATCCTTCAGGAGATGTACAACGAAT
GGCCATTTTTCAGGGTTACAATAGACTTGGTTGAGATGGTGTTTGCTAAGGGTGATCCTGGTATAGC
AGCTCTGTATGACAAGTTGCTAGTTTCTGAGGATTTGTGGCCATTTGGTGCTCGTCTTAGAGCAAAC
TATGAAGAAACAAAGCAACTTCTTCTACAGGTTGCTGGACACAAAGACCTTCTGGAGGGAGATCCTT
ACTTGAGGCAGAGACTGCGTATCCGTGATTCCTACATCACAGCGCTGAATGTTTGCCAAGCTTGCAC
GCTAAAGCGTATCAGGGACCCTGGCTTCCATGTAAGCCCCCGAGCTCACCTCTCCAAGGACATAATG
GACTCAGGGAAGCCAGCCGCTGAGCTTGTGAAGCTGAACACAACAAGCGAGTACGGCCCAGGCCTCG
AGGACACTCTCATCCTGACCATGAAGGGCATCGCTGCTGGTATGCAGAACACTGGGTGAAGTAGAAC
TGGTTCGTTCTGATGGTGTGTTATGGTGTCATGAATTAGTGTAGTTTGCACCTCTATGCAATCACTA
TTTGCAGTTTGCACCTCTGAACTCTATATGTAAATAAGGGGATGCTAAGCAACGTATAACTAAGACT
GGTTATATCCATGGATATTACCTGTTTAGCATGTTATATATCGAACTCTTATGCTGTTCACC

SEQ ID NO: 62, protein - Oryza sativa
MARNAADKGTSIDAQLRILAPKKLSEDDKLVEYDALLLDRFLDILQDLHGEDIRETVQECYELAAEY
ESKIAYRRRIKLKKGDFADENSATTESNFEETLKRLVGELKKSPHEVFDALKNQTIDLVLTAHPTQS
VRRSLLQKHGRIRSCLTKLYAKDITPDEKQELDEALKREIQAAFRTDEIRRAPPTPQDEMRAGMSYF
HETIWKGVPKFLRRVDTALKNIGINERVPYNAPLIQFSSWMGGDRDGNPRVTPEVTRDVCLLARMMA
ANLYYAQIEDLMFELSMWRCSDELRVKADQLHRCAKKNTTKHYIEFWKQVPPSEPYRVILSNVRDKL
YNTRERARHLLASGFSEIPDEATFTDVEQFLEPLELCYRSLCACGDNSIADGSLLDFLRQVSTFGLS
LVRLDIRQESDRHTDVMDAITQYLGIGSYREWSEEKRQEWLLSELNGKRPLFGPDLPQTDEIADALD
TFHVIAELPYDSFGAYVISMATAPSDVLAVELLQRECHVKKPLRVVPLFEKLADLEAAPAALARLFS
VDWYRNRIDGKQEVMIGYSDSGKDAGRFSAAWELYKAQEELIKVAKQFGVKLTMFHGRGGTVGRGGG
PTHLAILSQPPDTIHGSLRVTVQGEVIEQSFGEEHLCFRTLQRFTSATLEHGMHPPISPKPEWRALM
DEMAAVATKEYRSIVFQEARFVEYFRLATPELEYGRMNIGSRPSKRKPSGGIESLRAIPWIFAWTQT
RFHLPVWLGFGAAFKHVLQKDIRNLQILQEMYNEWPFFRVTIDLVEMVFAKGDPGIAALYDKLLVSE
DLWPFGARLRANYEETKQLLLQVAGHKDLLEGDPYLRQRLRIRDSYITALNVCQACTLKRIRDPGFH
VSPRAHLSKDIMDSGKPAAELVKLNTTSEYGPGLEDTLILTMKGIAAGMQNTG

SEQ ID NO: 63, DNA - Welwitschia mirabilis
AGCATCGACGCGCAACTGCGGCTTTTGGTCCCAACGAAGGTTTCCGAAGATGATAAGCTTATTGAGT
ATGACGCTCTTCTGATGGACCGCTTTCTGGATATCTTGCAAGACCTACATGGAGAAGAAATTAGAGA
AACGGTTCAAGAGTGCTATGAGCTTTCAGGAGAATACGAAGGAAAGTTTGACACGGCAAAGTTGGAG
GAGCTTGGAGGAGTGTTAACAAGTCTAGATGCTGGAGACTCCATTGTTGTTGCAGCTCTTTCTCACA
TGCTTAACCTAGCGAACTTGGCTGAGGAGGTTCAGATAGCATACCGAAGACGAATTAAACAC

**FIGURE 4 (continued)**

AAAAAGAAAGGGGATTTCGCGGATGAGAATTCCGCAACGACGGAATCAGACATTGAAGAAACCTTTA
AAAGGCTTGTAAATCAGCTTGGAAGGTCTCCTCAAGAAGTTTTTGACGCTCTCAAAAATCAAACAAT
TGATTTGGTCTTGACTGCACATCCAACGCATCAGTCGGTATCCCTGCTACAAAAGCATGGCAGGATC
CGAAACTGCTTGTCTCAGTTGTATGCGAAAGACATTACGCCCGATGAAAAACAGGAGCTTGATGAAG
CTTTACAGAGGGAGATTCAAGCTGCCTTCAGAACTGATGAAATTCGGCGCACTCCTCCCACCCCTCA
AGATGAGATGCGAGCAGGAATGAGTTATTTCCATGAAACCATATGGAAAGGTGTCCCTAAGTTCTTA
CGTCGTGTTGACACTGCTCTAAAGTCGATTGGTATAAATGAGAGACTTCCTTACAATGCCCCCCTAA
TACAATTCTCGTCGTGGATGGGCGGCGACCGCGACGGGAATCCTAGAGTCACTCCAGAAGTCACGCG
AGACGTTTGTCTCCTTGCGAGGATGATGGCTACAAATTTATACTACTCTCAGATAGAGGACCTTATG
TTTGAGTTGTCAATGTGGCGATGTAGCGATGAGCTTCGGCAACGTGCTCTGCAGCTTCATAATTCTG
CAAAGAGAGATGCAAAACATTACATAGAGTTTTGGAAGCAAGTACCTCCCAATGAGCCATTTAGGGT
AATTTTGGGAGATGTTAGGGACAAGTTATATAACACTCGGGAACGCACTCGCCAGTTGCTTGCCAAT
GGTTTCTCAGATGTGCCAGAAGAAGCACTTACGAGTGTAGATCAGTTGTTGGAACCGTTAGAGTTGT
GCTATCGATCTCTGTGCTCTACCGGCGATCAACCCATTGCAGATGGCAGTCTTCTTGATTTCATGCG
TCAAGTCTCAACCTTTGGCTTAACTCTTGTGAAGTTGGATATTAGACAAGAATCAGATCGACACACT
GATGTTATCGACGCAATAACAAGACATCTAGGCCTTGGGTCCTATAAAGAGTGGCCAGAAGACAAAC
GACAGGAGTGGCTGTTGGCTGAACTACGTGGAAAACGTCCACTATTTGGACCCGACCTTCCAACTAC
AGATGAAATAAGAGAAGTTCTTGACACATTCCACGTGGTAGCTGAACTTCCTCCAGACAACTTTGGA
GCTTACATCATATCAATGGCTACTGCTCCTTCAGATGTTTTAGTCGTTGAGTTACTGCAGAGGGAAT
GCCGTGTGAAAAAGCCTCTGCGAGTTGTGCCTTTGTTTGAGAAGCTTGCTGATTTAGAGAATCGTCC
CGCGGCTTTGGCAAGGCTGTTCTCAATTGATTGGTATAGAAACAGAATTGATGGAAAGCAAGAAGTA
ATGNNNGGTTACTCAGACTCCGGCAAGGATGCCGGAAGACTATCTGCAGCATGGCAATTATACAAGG
CTCAAGAGGAATTGATCAAGGTTGCAAAACAGTTTGGAATTAAGCTGACAATGTTTCATGGTCGTGG
AGGGACCGTTGGAAGAGGAGGTGGTCCAACCCATCTGGCTATATTATCTCAACCTCCAGACACAATT
CACGGGTCATTGCGAGTTACCGTCCAAGGGGAGGTTATTGAGCAGTGTTTTGGGGAGGAACATCTGT
GTTTCCGAACTCTTCAGCGCTTTACTGCTGCCACATTGGAACATGGAATGCATCCTCCAATTGCACC
TAAGCCCGAATGGCGTCAACTGATGGATGAAATGGCTGTTGTTGCTACTCAAGAGTATAGGTCTTAT
GTTTTCCACAACAAGAGATTTGTGGAGTATTTCCGATCCGCAACTCCCGAGTTGGAGTATGGACGGA
TGAATATAGGCAGTCGTCCATCAAAAAGAAAGCCCAGTGGAGGCATTGAATCACTTCGTGCAATTCC
ATGGATATTTGCTTGGACACAGACCAGATTCCATCTTCCTGTTTGGCTTGGATTTGGTGCTGCTTTC
AAGAGTGTTATCGAGAAGGACATTCGAAATCTTCACACGCTGCAACAAATGTATAACGAATGGCCAT
TTTTCCGTGTCACCATTGACCTAGTTGAAATGGTGTTTGCCAAGCACCCTGAAATTGCTGCATTATA
CGATAAACTGCTTGTATCATCAGTCTTGTGGGCTTTCGGGGAACAACTAAGAAAAAACTATGTAGAG
ACTAAGACCCTTCTGCTGCAGGTTGCTGGACACAAAGAAGTCTTAGAAGGCGACCCATATCTGAAAC
AACGATTGAGACTTCGTGACTCATATATCACAACCTTGAATGCACTTCAGGCATATACTTTAAAACG
GATACGAGATCCAAGCTACCATGTCACCCTCAGGCCTCATTTATCTAAAGAAAGCTCAACCAAGCCA
GCAGCAGAATTGGTAAAATTAAACCCAACCAGTGAGTATGCACCGGGATTAGAAGATACATTGATCT
TAACCATGAAGGGCATTGCTGCC

SEQ ID NO: 64, protein - Welwitschia mirabilis
SIDAQLRLLVPTKVSEDDKLIEYDALLMDRFLDILQDLHGEEIRETVQECYELSGEYEGKFDTAKLE
ELGGVLTSLDAGDSIVVAALSHMLNLANLAEEVQIAYRRRIKHKKKGDFADENSATTESDIEETFKR
LVNQLGRSPQEVFDALKNQTIDLVLTAHPTHQSVSLLQKHGRIRNCLSQLYAKDITPDEKQELDEAL
QREIQAAFRTDEIRRTPPTPQDEMRAGMSYFHETIWKGVPKFLRRVDTALKSIGINERLPYNAPLIQ
FSSWMGGDRDGNPRVTPEVTRDVCLLARMMATNLYYSQIEDLMFELSMWRCSDELRQRALQLHNSAK
RDAKHYIEFWKQVPPNEPFRVILGDVRDKLYNTRERTRQLLANGFSDVPEEALTSVDQLLEPLELCY
RSLCSTGDQPIADGSLLDFMRQVSTFGLTLVKLDIRQESDRHTDVIDAITRHLGLGSYKEWPEDKRQ
EWLLAELRGKRPLFGPDLPTTDEIREVLDTFHVVAELPPDNFGAYIISMATAPSDVLVV

## FIGURE 4 (continued)

ELLQRECRVKKPLRVVPLFEKLADLENRPAALARLFSIDWYRNRIDGKQEVMXGYSDSGKDAGRLSA
AWQLYKAQEELIKVAKQFGIKLTMFHGRGGTVGRGGGPTHLAILSQPPDTIHGSLRVTVQGEVIEQC
FGEEHLCFRTLQRFTAATLEHGMHPPIAPKPEWRQLMDEMAVVATQEYRSYVFHNKRFVEYFRSATP
ELEYGRMNIGSRPSKRKPSGGIESLRAIPWIFAWTQTRFHLPVWLGFGAAFKSVIEKDIRNLHTLQQ
MYNEWPFFRVTIDLVEMVFAKHPEIAALYDKLLVSSVLWAFGEQLRKNYVETKTLLLQVAGHKEVLE
GDPYLKQRLRLRDSYITTLNALQAYTLKRIRDPSYHVTLRPHLSKESSTKPAAELVKLNPTSEYAPG
LEDTLILTMKGIAA

SEQ ID NO: 65, DNA - Chlamydomonas reinhardtii
ATGACGGACTCCACATATGATTTTGGAGCCGTGAGGGACGATCTGACGCCGCTTGAGGATGACTGCA
AGCTTCTCGGTAGCCTGCTTGACGACTGTCTCCGCGTCGAAATCGGCGAGACCATGTTCAAGAAGAT
TGAACGCATCCGAGCACTGGCGCAATGTGCCTCAAATCTGTCGATCAAGGGAGACGCGGGCGCCTCC
GACATGCTGTCGCACCGGCTGGCGGAGGAGCTGATGAACCTGGACATGGACGAGGCGGTGCCGCTCA
CACGCGCCTGCGGCCACTACCTCAACCTGTCCGGCATCGCAGAGCTGCACCACGGAGTGCGCCGCGA
CCGCGCCACTCGCGAGCCCAACCCCAACAGCTGCGACGCGGTGTTTGCGCGGCTGATCACGGAGGGC
GTGGACCCCGAGGAGCTGTACCGGGCGGTGTCGGAGCAGAACGTGGAGGTGGTGCTCACCGCGCACC
CCACACAGGTGAACCGGCGCACGCTGCAGTACAAGCACACTCGCATTGCGGCGCTGCTGCAGCAGCA
CGACCGCTCCGACCTGACGGCGGAGGAGCGGCGCAACATGGTGAGCGAGCTGCAGCGGGAGGTGGCG
GCGCTGTGGCAGACGGACGAGCTGAGGAGGCAGAAGCCCACGCCGCTGGACGAGGCGCGCGGCGGTC
TGCACATTGTGGAGCAGTCGCTGTGGGCCGCGGTGCCGCAGTACATGCGCCGCCTCAGCGCCGCGCT
CAAGAAACACACCGGGCACGACCTGCCGCTGCAGGCCACGCCCTTCCGCTTCGGCAGCTGGATGGGC
GGCGACCGCGATGGCAACCCCAACGTGACCGCCAAGGTGACGGCGCACGTGACGGCCCTGGCGCGCT
GGATGGCGGCGGATCTGTACCTGCGTGAGATCGACACGCTGAGGTTTGAGCTGTCCATGAACCAGTG
CAGTGCGGCGGTGTGGAAGATGGCCCGCCGCATCATCGCCGAGGGCCACACCAAGCGCGCCGGCGTG
GTCCGGGCCAAGGCCGCCGCCGCCCTGCACCAGACCGCAACAGACGCAGCCAGCCATGGCGGCTCCG
CCGCCTCGGCGGCCGCCGCCGCCGCCGCAGGCGGCGACGTCGTCGCTGACGGCACCAGCGGCGGCGG
CGCTGCCGCCGCCGCCGGCCCCGCCGCCGCTGCCGCGGCGGACGACGCGTTCACCTTCAGCCGGTTG
GGGCGGCCCCGGCCGGAGCGGCCGAGCACGGACGTGCGGAGTGTGGGCGTGCTGGCGGGCGGCGAGG
GCGCGGCGTTCCCGGGCGGCATGATCCTGGGCACGCAGCCGGTGTCGGCGCACACGGCGGCGGAGGT
GTCGGTGCCGCACGAGCTGCCGGGACAGGACGTGGAGGGCGGCTCGGAGATGGACTTCAACGAGTCC
CGCCGCGCCTCGGACGCCGGAGACCTGGGCGCCTCGCAGCACCCCATGCTGGGCGGGCCCTCCGCCG
GCGCCTCCGCCGAGCCCACCGCCCACGGCTACACCACCACCGCCACCGCCGCCGCCGCCGCCGCCGA
CGGCACGCAGCCCGAGCCCGAAGTCCCCGGCACGCCCAGCTACGCCGACCCCGGCACCCCCGACCGC
CTTGGCGCGCTGCCCGGCCCCTTCACGCCCGGCCCCACGCCCTTCCGCGAGGCGGCCAACGCCGCCA
TGAGCACCGCCGCCAGCGGCGGCGCGGGCGGCGGCGGCGGCGGCGGCGCGAACCGCGCGGCCTCGGG
CCTGGGCGGCGACCCCACCTTCACGCGTCGCAGCCTGATGGCGCAGCGGCTGGGAACCAGCTCGGTG
CAGTTCGCGCGCGCGCACGAGCACCCCGGCTTCCACCCCTACCGCATCGTGTTGGGCCACGTGCGCG
ACAGGCTGGCGGCCACGCGGCGGCGCATGGAGGACCTGCTGAGCGGCCGCGAGCCGGCGGGCGAGGC
GCACGGCGGCGTGGGGGCGGGCGGCGGCGGCGGCGGCGGCGCGGCGCCGTGGTATGAGAGCGAGGAC
GAGCTGGCGGAGCCGCTGATGGCGTGCTACTGGTCGCTGTGGGAGTGCGGCGGCGGCGTCATTGCGG
ACGGGCGGCTGCTGGACCTCATCCGCCGCGTGTACACCTTCGGCATGTGCCTCATGAAGCTAGACCT
GCGCCAGGAGTCCACCCGCCACGCCGAGGCCCTGGACGCCGTGACCAGCTACCTGGGTCTGGGCTCC
TACCTGGAGTGGAGCGAGGACCAGAAAATCGAGTGGCTCACAAAGGAGCTGCAGGGCCGGCGGCCGC
TCATCCCCGCCGACATGCCCATGAGCGCCGAGGTGCGCGAGGTGCTGGACACCTTCAAGGTGGCCGC
CCATCTGGGCCGTGACAACCTGGGCGCCTACGTCATCTCCATGACCAAGGGCGCCTCGGACGTGATG
GCGGTGGAGCTGCTGCAGCGCGAGGCGCGCATGCAGGTGGGCGCCGAGGCGGGCGGCCGCGGCGGCG
GCGGGCCCGAGGACGGCGGCTCGCTGCGCGTGGTGCCGCTGTTCGAGACGCTGGAGGACCTGGACGC
GGCGGAGGACGTGATGACGCGGCTGCTG

**FIGURE 4 (continued)**

ACCAACCCCTGGTACCGCGAGCACCTGCGGGCAGTGCACGGCGACGCGCAGGAGGTGATGCTGGGAT
ACAGCGACAGCGGCAAGGACGCCGGCCGCCTGGCTGCCAACTGGGCGCTGTACAAGTGTCAGGAGCG
GCTGGTGGCCATCACCAAGGCCAACAACGTCAAGCTCACGCTGTTCCACGGCCGCGGCGGCACCGTG
GGCCGCGGCGGCGGGCCCACCCACATCGCCATCCAGTCGCAGCCGCCCGGCTCTGTGGAGGGGACCT
TCCGCATCACCGAGCAGGGCGAGATGGTCCAGGCCAAGTTCGGCATCAGCGGCGTGGCTCTGAGCCA
GCTGGAGACCTACACCACCGCTGTGCTGCTGGCCACCATGCGCCCGCCCAGCCCGCCGCGCCGCGAG
GAGTGGCGCGCCGTGATGGAGATGCTCAGCCGCGTCAGCTGCGAGAGCTACCGCAACATCGTGCACC
ACAGCCCGCTCTTCCTGCGCTACTTCAAGCACGCCACGCCCGAGGCCGAGCTGGGCAACCTCTACAT
CGGCTCTCGCCCGGCTCGGCGGCGCAACAAGGACGCCTCCATCTCCACGCTGCGCGCCATCCCCTGG
ATCTTCGCCTGGACGCAGAACCGCCTCATCCTGCCCTCCTGGCTGGGCATTGGCGCCGCCCTCACCG
CGGCCATGACCCAGGGCCACCTGCCCACGCTGCAGGCCATGTACCGCGAGTGGCCCTTCTTCGGATC
CACAGTGGATCTGATTGAGATGATCCTGGCCAAGACCGACCCGCGCATCGCGGCGCTGTACGAGGAG
GTGCTGGTCAACGACCCCGAGGAGAAGAAGCTGGGAGCCGAGCTGCGCGAGCGGCTGCAGCGCTGCC
AGGGCGCCATCCTCAAGGTGACCGGGCACGAGAACCTGCTGTCCAACAACCCCACATTGAGCAAGCT
CATCTCCATGCGCTCGCCCTTCGTGGACCCCATCAACATCCTGCAGGTGGAGGTGCTGCGGCGCCTG
CGCCAGGACCCCAACAACATGCGCCTGCGCGACGCGCTGCTCATCTCCATCAACGGCATCGCCGCCG
GCATGCGCAACACCGGCTAAGCAGCGGCGGCGCTGCTGCTGGTGATGGAGGTGGAGGTGGAGATGGA
CGTGGTGCGCACCAGGCACAAGGCGGAGGCAGGTGCCAGTGGAGGTGGGGCGTCCCGGGCGGAGGCA
TGAGCGGCGTCGACGGCTGGCGCCGCGGTATGATGTGCTGCGGCACGAGCGACGGCACGTGCGGTCT
AAAGCTGCTGGATGTGCTTGGCACGGCGCGCAAGAGGGGTGTGCTGGGGGCGGAGGTGGTGCTGAAA
ATGTTTGGTTAGTTGGTTGGATGTTTGGGCTAGCTGGTGCGCTGGAGCGGGTAAGGCAAGTGGTGGG
TGGGGTATGCGGGTGCGCTGGGGTGTCCAGGGCGGCTGCGATCAATGCGGGTTGGTGGGCAACCCCG
TCCCGGCGCTGAATCGCTGCGTGGATCTGACCTTTGCGGAATTCCTGCCTGCCTTCCGCGCCTGGCA
ATTTAGGAACTCGTTTGAGCGTTTGACGTTGTCCTAGGGCGGCATGACCGAGCATCGCGTGCATGGC
AGCGGGGCTGCGATCGCGCGTGGTGAGCCACAGACGTTGATAGTGATATTTGAGGCAGCCCAGAACG
CATGGCGGAATGGCGTGGTCTTTGAGAGCTGAGTGTGTCAGACTGGCATAGGGCGCTGTGGCTGACT
AACGTCCCGCACGTGTGCGACTGCAGACCATGTTTGCAGGGGCAGGTACTTTACTTGATGTGCATGG
CATTCTCACATAAAGCTCCGAAGGATCATTTGTGGCGCACCAACCAGAGACCTAGCCCAGAGCAGAG
TGTCAGCAGCGATATCAAAGTCATTTTGTGGGTGGTTGTCTGGGTACTCCGAGGCGCGTACGTTGAG
CGCGTGCTCGCTGCATACCGCATACGGAATCGGATAAGTGAAAGGAGGTCACATGGCCAGGGGCTGC
AGATGCTGGTGAGGGCTGCCTGCCTAAGCGGCGTGTCCGGGACGGCAGGGAAGCACGCATGCCATTC
GACGTGTGCGGTGCAAATGAGACCGTGGAGTCCTCGGGCAGGGCTTGACAGCACAAGCGTGGAGCGC
GCACACAGCGCACAGACTATGTTTTCCTTGTGCTCTTTAACACAGCGCACAGAAAAAA

SEQ ID NO: 66, protein - Chlamydomonas reinhardtii
MTDSTYDFGAVRDDLTPLEDDCKLLGSLLDDCLRVEIGETMFKKIERIRALAQCASNLSIKGDAGAS
DMLSHRLAEELMNLDMDEAVPLTRACGHYLNLSGIAELHHGVRRDRATREPNPNSCDAVFARLITEG
VDPEELYRAVSEQNVEVVLTAHPTQVNRRTLQYKHTRIAALLQQHDRSDLTAEERRNMVSELQREVA
ALWQTDELRRQKPTPLDEARGGLHIVEQSLWAAVPQYMRRLSAALKKHTGHDLPLQATPFRFGSWMG
GDRDGNPNVTAKVTAHVTALARWMAADLYLREIDTLRFELSMNQCSAAVWKMARRIIAEGHTKRAGV
VRAKAAAALHQTATDAASHGGSAASAAAAAAAGGDVVADGTSGGGAAAAAGPAAAAAADDAFTFSRL
GRPRPERPSTDVRSVGVLAGGEGAAFPGGMILGTQPVSAHTAAEVSVPHELPGQDVEGGSEMDFNES
RRASDAGDLGASQHPMLGGPSAGASAEPTAHGYTTTATAAAAAADGTQPEPEVPGTPSYADPGTPDR
LGALPGPFTPGPTPFREAANAAMSTAASGGAGGGGGGGANRAASGLGGDPTFTRRSLMAQRLGTSSV
QFARAHEHPGFHPYRIVLGHVRDRLAATRRRMEDLLSGREPAGEAHGGVGAGGGGGGGAAPWYESED
ELAEPLMACYWSLWECGGGVIADGRLLDLIRRVYTFGMCLMKLDLRQESTRHAEALDAVTSYLGLGS
YLEWSEDQKIEWLTKELQGRRPLIPADMPMSAEVREVLDTFKVAAHLGRDNLGAY

## FIGURE 4 (continued)

VISMTKGASDVMAVELLQREARMQVGAEAGGRGGGGPEDGGSLRVVPLFETLEDLDAAEDVMTRLLT
NPWYREHLRAVHGDAQEVMLGYSDSGKDAGRLAANWALYKCQERLVAITKANNVKLTLFHGRGGTVG
RGGGPTHIAIQSQPPGSVEGTFRITEQGEMVQAKFGISGVALSQLETYTTAVLLATMRPPSPPRREE
WRAVMEMLSRVSCESYRNIVHHSPLFLRYFKHATPEAELGNLYIGSRPARRRNKDASISTLRAIPWI
FAWTQNRLILPSWLGIGAALTAAMTQGHLPTLQAMYREWPFFGSTVDLIEMILAKTDPRIAALYEEV
LVNDPEEKKLGAELRERLQRCQGAILKVTGHENLLSNNPTLSKLISMRSPFVDPINILQVEVLRRLR
QDPNNMRLRDALLISINGIAAGMRNTG

SEQ ID NO: 67, DNA - Glycine max
ATGACTGACATCACTGGTGATATTGCTGAGGAAATCTCCTTCCAGAGCTTCGATGATGACTGCAGGT
TGCTTGGTAATCTCCTCAATGACATTCTCCAGCGTGAAGTTGGCACCAACTTGCTTGACAAGATCGA
AAGGACTCGAGTCCTTGCTCAGAGTGGTTGTAATATGAGGCAGGCGGGTATTGTAAACATGGCAGAG
ATGCTTGAGAAGCAGTTGGCTTCGGAGTTATCAAAGATGACACTAGAAGAAGCTTTCACCCTTGCTC
GTGCCTTCAGCCATTATCTTACTTTGATGGGTATAGCTGAGACCCACCATAGGGTTCGTAAAGGAGG
GAATATGGCACAAATTGCAAAATCTTGCGATGATATATTTAACCAGCTGGTGCAGGGTGGAGTCCCC
CCAGAAGAACTTTATGACACAGTCTGCAAGCGGGAGGTTGAAATTGTTCTCACTGCTCATCCCACAC
AGATTAACCGTCGAACCTTACAGTTTAAACACATTAGAATTGCTCATCTTTTGGATTACAATGATCG
ACCTGATCTTAGCACTGAAGATCGAGAAATGGTGATTGAAGATCTGGTGAGAGAGATAACTTCAATA
TGGCAGACAGATGAGCTTAGGCGCCAGAAACCCACTCCAGTTGATGAAGCTAGAGCTGGTTTCAATA
TTGTGGAGCAGTCACTCTGGAAAGCTGTCCCTCATTATTTACGTCGTGTCAGCAATGCATTAAAGAA
GCATACAGGAAAGCCACTTCCTTTGACTTGCACTCCCATAAAGTTTGGATCTTGGATGGGAGGTGAT
AGAGATGGAAACCCAAATGTGACAGCAAAGGTCACAAAAGATGTTTCACTTCTATCTAGATGGATGG
CGATTGACCTCTATATTCGGGAAGTGGATAGCCTCAGATTTGAGCTATCCATGAACCAGTGCAGTGA
TAGGTTGTCAAGATTGGCACATGAAATTCTAGAAGCCAAGCATGAGAATCGCCGTGAGAATTGGAAT
CAGTCTGCGAATAGAAGTCTCACACTTCCAACACAACTTCCAGCTAGAGCTCATTTACCTTCTATTG
CTGAAAATGGTGAATCTCGGCATCCCAGACTAGACATTCCAGCACCTGATTACATGCAATCCAATCA
CAAGGATGGTGGGGTTTCTGTAAGTTCAACTACATCAAAACTTGCCAATCCCAATACTCGATTACCA
GGAACAAGTTCAGCAAATTCCAGTGCTTCTTCAGCTGCACTTGGTCAAAAGAAATTGTATGCAGAAT
CCCAGACAGGAAAGTCCACTTTTCAAAAGCTTTTGGAGCCAATGCTTCCTCAACTTCCTGGAATTGC
TCCTTATAGAATTGTCCTGGGGAATGTTAAGGATAAGCTTGAGAAAAGTCGTAGACGGTTAGAAATT
CTTCTTGAGGATGTTGCATGTGACTATGATCCTTTGGATTACTATGAAACATCTGATCAGCTTTTGG
AACCTCTGCTCCTCTGTTATGAATCTCTGCAATCGTGTGGATCTGGGGTGCTAGCTGATGGTCGACT
TGCTGATCTGATTCGTAGAGTTGCTACCTTTGGAATGGTGTTAATGAAGCTTGACTTGCGTCAGGAA
TCTGGGAGACATGCAGAAGCACTTGATGCAATAACACAGTACTTAGATATGGGTACTTACAGTGAAT
GGGATGAAGAAAAGAAGTTGGACTTCTTAACAAGAGAACTTAAAGGGAAGAGGCCTCTTGTTCCTGT
TAGTATAGAGGTTCATCCTGATGTTAAAGAAGTCTTGGATACATTCCGAATTGCCGCTGAACTGGGG
AGTGATTCACTTGGAGCTTATGTGATCTCTATGGCCTCAAATGCAAGTGATGTCCTTGCAGTAGAGC
TTTTACAGAAGGATGCACGGCTTGCTGCTATTGGGGAGTTGGGAAAAGCATGTCCTGGTGGAACGTT
GCGGGTTGTCCCTCTGTTTGAAACTGTGAAAGACCTGAGAGGAGCTGGTTCAGTTATCCGGAAACTT
TTATCAATAGACTGGTACCATGAACACATCGTTAAGAACCATAATGGACATCAAGAGGTTATGGTTG
GATATTCTGATTCTGGTAAAGATGCTGGTCGCTTTACTGCTGCTTGGGAACTTTACAAAGCTCAGGA
GGATGTTGTAGCTGCTTGCAATGATTATGGAATAAAGGTTACTCTATTCCATGGTCGTGGAGGCAGT
ATTGGTCGTGGTGGTGGCCCAACATATCTGGCTATTCAGTCCCAACCCCCTGGCTCTGTGATGGGAA
CGCTTCGGTCTACTGAGCAGGGAGAGATGGTAGAGGCTAAGTTTGGGTTGCCACAGATAGCTGTTAG
ACAACTTGAGATATACACAACAGCTGTACTACTTGCAACCCTTCGTCCACCTATCCCACCCCGAGAA
GAAAAATGGCGTAATGTCATGGAAGAGATCTCAAACATCAGTTGTCAGTGTGACCGCAATGTAGTGT
ATGAAAATCCAGAATTCCTGGCCTACTTCCATGAAGCCACACCAGAGGCAGAACTTGGCTTCCTTAA
CATAGGTAGCCGCCCTACAAGAAGGAAG

## FIGURE 4 (continued)

AGCTCAGTAGGAATCGGACACCTTCGTGCAATTCCCTGGTTATTTGCATGGACACAAACAAGATTCG
TTCTTCCAGCTTGGCTTGGAGTCGGAGCAGGTTTAAAAGGAGCTTGCGAGAAAGGTTACACCGAAGA
GCTAAAAGCCATGTACAAAGAATGGCCCTTCTTTCAAAGTACCATAGATCTTATTGAGATGGTTTTG
GGGAAAGCTGACATTCCTATAGCCAAGCACTATGATGAAGTCCTTGTGACAAAGGAGAGGCAAGAGC
TTGGCCATGAACTAAGAAGTGAGCTCATGACAGCTGAAAAGTTTGTCATGGTTATTAGTGGGCACGA
GAAACTTCAGCAGAATAATAGGAGCTTGAGGAGGCTAATTGAGAATAGACTTCCCTTCCTTAATCCC
TTGAACATGTTGCAGGTGGAGATACTCAAGAGGTTAAGACGTGATGATGACAACCGTAAGATCAGAG
ATGCTTTGCTTATCACCATAAATGGGATTGCTGCAGGGATGAAGAATACAGGTTGA

SEQ ID NO: 68, protein - Glycine max
MTDITGDIAEEISFQSFDDDCRLLGNLLNDILQREVGTNLLDKIERTRVLAQSGCNMRQAGIVNMAE
MLEKQLASELSKMTLEEAFTLARAFSHYLTLMGIAETHHRVRKGGNMAQIAKSCDDIFNQLVQGGVP
PEELYDTVCKREVEIVLTAHPTQINRRTLQFKHIRIAHLLDYNDRPDLSTEDREMVIEDLVREITSI
WQTDELRRQKPTPVDEARAGFNIVEQSLWKAVPHYLRRVSNALKKHTGKPLPLTCTPIKFGSWMGGD
RDGNPNVTAKVTKDVSLLSRWMAIDLYIREVDSLRFELSMNQCSDRLSRLAHEILEAKHENRRENWN
QSANRSLTLPTQLPARAHLPSIAENGESRHPRLDIPAPDYMQSNHKDGGVSVSSTTSKLANPNTRLP
GTSSANSSASSAALGQKKLYAESQTGKSTFQKLLEPMLPQLPGIAPYRIVLGNVKDKLEKSRRRLEI
LLEDVACDYDPLDYYETSDQLLEPLLLCYESLQSCGSGVLADGRLADLIRRVATFGMVLMKLDLRQE
SGRHAEALDAITQYLDMGTYSEWDEEKKLDFLTRELKGKRPLVPVSIEVHPDVKEVLDTFRIAAELG
SDSLGAYVISMASNASDVLAVELLQKDARLAAIGELGKACPGGTLRVVPLFETVKDLRGAGSVIRKL
LSIDWYHEHIVKNHNGHQEVMVGYSDSGKDAGRFTAAWELYKAQEDVVAACNDYGIKVTLFHGRGGS
IGRGGGPTYLAIQSQPPGSVMGTLRSTEQGEMVEAKFGLPQIAVRQLEIYTTAVLLATLRPPIPPRE
EKWRNVMEEISNISCQCDRNVVYENPEFLAYFHEATPEAELGFLNIGSRPTRRKSSVGIGHLRAIPW
LFAWTQTRFVLPAWLGVGAGLKGACEKGYTEELKAMYKEWPFFQSTIDLIEMVLGKADIPIAKHYDE
VLVTKERQELGHELRSELMTAEKFVMVISGHEKLQQNNRSLRRLIENRLPFLNPLNMLQVEILKRLR
RDDDNRKIRDALLITINGIAAGMKNTG

SEQ ID NO: 69, DNA - Ricinus communis
ATGACGGACACCACAGATGATATTGCAGAGGAAATCTCATTTCAGAGTTTTGATGATGATTGTAAGC
TTCTTGGCAATTTGTTAAACGATGTTTTGCAGAGAGAAGTTGGTTCTAAATTCATGGAGAAGCTTGA
ACGCAATCGCATCCTAGCTCAGAGTGCTTGTAATATGAGATTGGCGGGGATAGAAGATACCGCTGAA
TTGCTGGAGAAGCAGCTGGCATTGGAGATATCAAGGATGACATTAGAGGAAGCATTGACACTTGCTC
GTGCCTTTAGTCATTACCTTAATTTGATGGGCATTGCTGAGACCCATCACAGGGTTCGTAAGGCACG
GAGTATGACACATCTATCAAAATCTTGTGATGACATATTTAATCAGCTGCTGCAGAGTGGCATATCT
GCAGAGGAGCTTTATGACACAGTTTGCAAGCAGGAGGTTGAAATTGTGCTCACTGCACATCCTACTC
AAATTAATCGTCGTACCTTGCAATATAAGCACATCAGAATTGCTCATCTTTTAGATTATAATGACCG
GCCTGATCTTACTCATGAAGATCGAGAAATGCTGATTGAAGATCTGGTGAGAGAAATTACTTCAATA
TGGCAGACAGATGAGCTTAGGCGCCACAAGCCTACACCAGTAGATGAAGCTAGGGCTGGCTTGAACA
TTGTGGAGCAGTCCCTGTGGAAAGCTCTTCCCCATTATTTACGTCGTGTCAGTACTGCCCTAAAGAA
GCATACAGGAAAGCCACTTCCTTTGACTTGCACGCCAATAAGGTTTGGGTCTTGGATGGGGGGTGAT
AGAGATGGTAATCCGAATGTAACAGCAAAGGTCACAAGAGATGTTTCTCTTTTATCTAGGTGGATGG
CTGTTGACCTTTACATTCGAGAAGTTGATAGCCTGAGATTTGAACTATCCATGGTTCAATGCAGTGA
TAGATTGTTGAAAGTGGCAAATGACATTTTAATAGAAGAAACTTCATCTGAAGATCACCATGAGAGT
TGGAATCAACCTGCAAGCAGAAGTCAAACAAAGTTTCCTAGAAAATCTCTTCCTACACAACTTCCAC
CTAGAGCTGATCTTCCTGCTTGCACTGAATGTAATGATGGTGAATCTCAGTATCCCAAACTAGAACT
TCCAGGAACTGATTACATGCCCTTTAATCGCCAGGAAGCTCTAGGTTCTTCATATTCAGAATCTTCA
TCCCAGGATATCAATCATGGTTTACCTAAAACAACTGGAAATGGAAGTGTTGCTAATTCTAGTGGAT
CTCCGCGGGCATCTTTCAGCTCTGCTCAACTGGTTGCACAGAGGAAA

**FIGURE 4 (continued)**

CTATTTGCAGAATCCAAGATAGGAAGATCTAGCTTCCAGAAGCTTCTAGAACCAAGTCTGCCTCAAC
GTCCTGGAATTGCTCCTTATAGAATTGTTCTTGGAAATGTAAAAGATAAGCTTATGAGGACAAGAAG
ACGTCTGGAACTTCTTCTAGAGGATCTTCCTTGTGAATATGATCAATGGGATTACTATGAGACAACA
GATCAATTATTGGATCCACTGCTTCTGTGCTACGAGTCTCTTCAATCATGTGGAGCTGGGGTTCTAG
CTGATGGTCGGCTAGCTGACCTGATAAGAAGAGTTGCTACATTTGGGATGGTGTTAATGAAGCTAGA
CTTGCGCCAGGAATCTGGTAGACATGCTGATACTCTTGATGCAATCACCAAATATTTGGAAATGGGC
ACGTATAGTGAGTGGGATGAAGAAAAGAAGCTGGAATTTCTGACAAGAGAACTGAAAGGAAAACGGC
CACTGGTTCCTCCTACTATTGAGGTTGCTCCTGATGTTAAAGAAGTCTTGGATGCTTTCCGTGTTGC
TGCTGAGCTGGGGAGTGATTCACTTGGAGCTTATGTGATTTCTATGGCATCCAATGCAAGTGATGTC
CTTGCTGTGGAGCTTCTGCAGAAGGATGCCCGGCTTGCTGTTAGTGGGGAGCTAGGAAGACCATGCC
CTGGTGGAACGTTGCGAGTAGTTCCGTTGTTTGAAACTGTGAAAGACCTGAGAGGTGCTGGTTCGGT
GATCAGAAAACTGTTATCAATTGACTGGTATAGAGAACACATAATTAAGAACCATAACGGGCATCAG
GAGGTGATGGTTGGCTATTCTGATTCTGGTAAAGATGCTGGACGCTTTACTGCTGCATGGGAACTTT
ACAAAGCCCAAGAGGATGTTGTGGCAGCCTGTAATGATTTTGGTATCAAAGTAACACTATTCCATGG
GCGTGGAGGGAGTATTGGTCGTGGTGGTGGCCCCACATATCTTGCCATTCAATCCCAACCACCTGGT
TCAGTTATGGGTACTCTTCGGTCAACTGAGCAAGGAGAAATGGTGCAGGCCAAGTTTGGGCTGCCAC
ATACTGCCATCAGACAACTAGAGATATACACAACTGCTGTGCTGCTTGCAACCCTTCGTCCTCCACA
TCCACCTCGAGAAGAACAATGGCGCAATGTCATGGAGGAGATTTCAAAAATCAGTTGCCAGAATTAC
CGGAGCACAGTCTATGAAAACCCAGAATTCCTCGCCTACTTCCATGAGGCTACTCCCCAGGCTGAGC
TTGGCTTTCTTAACATAGGAAGCCGCCCCACAAGAAGAAAGAGCTCAACTGGTATTGGACATCTTCG
TGCCATTCCATGGGTATTCGCATGGACCCAGACCAGATTTGTTCTTCCTGCTTGGCTTGGAGTTGGA
GCAGGTTTAAAGGGTGCTTGTGAGAAGGGATTTACTGAAGACTTGAAAGCAATGTACAAAGAATGGC
CTTTCTTCCAATCTACTATTGATCTTATAGAGATGGTACTAGGGAAGGCAGACATTCCTATAGCCAA
GCACTATGATGAAGTTCTTGTATCTGAGAGCAGGCGAGAGCTTGGAGCTGAGCTTAGGAGTGAGCTC
TTAACAACAGAAAAGTATGTGTTGGTGGTTAGTGGGCATGAGAAACTATCTCAGAACAACCGCAGCC
TGCGGAGGCTAATAGAGAGCAGGCTGCCTTATCTCAATCCTATGAATATGTTGCAGGTGGAGGTTCT
AAAGAGGTTGAGGCGGGACGACGACAACAATAAGCTCAGAGATGCCCTGCTTATTACCATAAATGGG
ATTGCTGCTGGAATGAGGAACACAGGCTGA

SEQ ID NO: 70, protein - Ricinus communis
MTDTTDDIAEEISFQSFDDDCKLLGNLLNDVLQREVGSKFMEKLERNRILAQSACNMRLAGIEDTAE
LLEKQLALEISRMTLEEALTLARAFSHYLNLMGIAETHHRVRKARSMTHLSKSCDDIFNQLLQSGIS
AEELYDTVCKQEVEIVLTAHPTQINRRTLQYKHIRIAHLLDYNDRPDLTHEDREMLIEDLVREITSI
WQTDELRRHKPTPVDEARAGLNIVEQSLWKALPHYLRRVSTALKKHTGKPLPLTCTPIRFGSWMGGD
RDGNPNVTAKVTRDVSLLSRWMAVDLYIREVDSLRFELSMVQCSDRLLKVANDILIEETSSEDHHES
WNQPASRSQTKFPRKSLPTQLPPRADLPACTECNDGESQYPKLELPGTDYMPFNRQEALGSSYSESS
SQDINHGLPKTTGNGSVANSSGSPRASFSSAQLVAQRKLFAESKIGRSSFQKLLEPSLPQRPGIAPY
RIVLGNVKDKLMRTRRRLELLLEDLPCEYDQWDYYETTDQLLDPLLLCYESLQSCGAGVLADGRLAD
LIRRVATFGMVLMKLDLRQESGRHADTLDAITKYLEMGTYSEWDEEKKLEFLTRELKGKRPLVPPTI
EVAPDVKEVLDAFRVAAELGSDSLGAYVISMASNASDVLAVELLQKDARLAVSGELGRPCPGGTLRV
VPLFETVKDLRGAGSVIRKLLSIDWYREHIIKNHNGHQEVMVGYSDSGKDAGRFTAAWELYKAQEDV
VAACNDFGIKVTLFHGRGGSIGRGGGPTYLAIQSQPPGSVMGTLRSTEQGEMVQAKFGLPHTAIRQL
EIYTTAVLLATLRPPHPPREEQWRNVMEEISKISCQNYRSTVYENPEFLAYFHEATPQAELGFLNIG
SRPTRRKSSTGIGHLRAIPWVFAWTQTRFVLPAWLGVGAGLKGACEKGFTEDLKAMYKEWPFFQSTI
DLIEMVLGKADIPIAKHYDEVLVSESRRELGAELRSELLTTEKYVLVVSGHEKLSQNNRSLRRLIES
RLPYLNPMNMLQVEVLKRLRRDDDNNKLRDALLITINGIAAGMRNTG

## FIGURE 4 (continued)

SEQ ID NO: 71, DNA - Oryza sativa
CCCACGCGTCCGCCCACGCGTCCGGGACACCAGAAACATAGTACACTTGAGCTCACTCCAAACTCAA
ACACTCACACCAATGGCTCTCCAAGTTCAGGCCGCACTCCTGCCCTCTGCTCTCTCTGTCCCCAAGA
AGGGTAACTTGAGCGCGGTGGTGAAGGAGCCGGGGTTCCTTAGCGTGAGCAGAAGGCCAAGAAGCCG
TCGCTGGTGGTGAGGGCGGTGGCGACGCGGCGGGCCGGTGGCGAGCCCCGGCGCGGGCACGTCGAAG
GCGGACGGGAAGAAGACGCTGCGGCAGGGGGTGGTGGTGATCACCGGCGCGTCGTCGGGGCTCGGGC
TCGCGGCGGCGAAGGCGCTTGGCGGAGACGGGGAAGTGGCACGTGGTGATGGCGTTCCGCGACTTTC
CTGAAGGCGGCGACGGCGGCGAAGGCGGCGGGGATGGCGGCGGGGAGCTACACCGTCATGCACCTGG
ACCTCGCCTCCCTCGACAGCGTCCGCCAGTTCGTGGACAACTTCCGGCGCTCCGGCATGCCGCTCGA
CGCGCTGGTGTGCAACGCCGCACATCTACCGGCCGACGGCGCGGCAACCGACGTTCAACGCCGACGG
GTACGAGATGAGCGTCGGGGTGAACCACCTGGGCCACTTCCTCCTCGCCCGCCTCATGCTCGACGAC
CTCAAGAAATCCGACTACCCGTCGCGGCGGCTCATCATCCTCGGCTCCATCACCGGCAACACCAACA
CCTTCGCCGGCAACGTCCCTCCCAAGGCCGGGCTAGGCGACCTCCGGGGGCTCGCCGGCGGGCTCCG
CGGGCAGAACGGGTCGGCGATGATCGACGGCGCGGAGAGCTTCGACGGCGCCAAGGCGTACAAGGAC
AGCAAGATCTGTAACATGCTGACGATGCAGGAGTTCCACCGGAGATTCCACGAGGAGACCGGGATCA
CGTTCGCGTCGCTGTACCCGGGGTGCATCGCGACGACGGGCTTGTTCCGCGAGCACATCCCGCTGTT
CCGGCTGCTGTTCCCGCCGTTCCAGCGGTTCGTGACGAAGGGGTTCGTGTCGGAGGCGGAGTCCGGG
AAGCGGCTGGCGCAGGTGGTGGGCGACCCGAGCCTGACCAAGTCCGGCGTGTACTGGAGCTGGAACA
AGGACTCGGCGTCGTTCGAGAACCAGCTCTCGCAGGAGGCCAGCGACCCGGAGAAGGCCAGGAAGCT
CTGGGACCTCAGCGAGAAGCTCGTCGGCCTCGTCTGAGTTTATTATTTACCCATTCGTTTCAACTGT
TAATTTCTTCGGGGTTTAGGGGGTTTCAGCTTTCAGTGAGAGAGGCCTGTCAAGTGATGTACAATTA
GTAATTTTTTTTTACCCGACAAATCATGCAATAAAACCACAGGCTTACATTATCGATTTGTCCACCT
AAATTAAGTTTCAACTGTTAATTTCTTCGGGGTTTAGGGGGTTTCAGCTTTCAGTGAGAGAGGCCTG
TCAAGTGATGTACAATTAGTAATTTTTTTTTACCCGACAAATCATGCAATAAAACCACAGGCTTACA
TTATCGATTTGTCCACCTAAATTAAGT

SEQ ID NO: 72, DNA - primer
ggggacaagtttgtacaaaaaagcaggcttaaacaatgaacttggcagttcct

SEQ ID NO: 73, DNA - primer
ggggaccactttgtacaagaaagctgggttcaaccagtattacgcatt

FIGURE 4 (continued)

## Capsicum annuum

MEEIQVPPY**FLCPISLEMMKDPVTIST*GITYDRENIERWIF*SAKNNTCPVTKQSLTS**
**IELTPNVTLRRFIQSWC**TLNASHGIERFPTPKPPVSKAQILKLMKEAKSREMQMKSL
NTLRSIASVNDANKRCMESAGAAEFLASVVIDSSDVLGEEGFMSTKDEALSILYQLK
LSEDGLRSLITSGNGEFIESLTRVMQRGSYESRAYAVMLMKDMFEASTLPTLFSSLK
KEFFAQVVRVLRDEISQKATKASLQVLAHACPFGRNRVKAAEAGAVRVLVDLLLDSS
EKRTCELMLILLDQICQSAEGRAELLNHPG*GLAIVSKKILRVS*KVGSERAIKILHSI
SKFSSTQSVVQEMLSLGVVAKLCLVLQVDCGSKAKDRARDILKIHAKAWRNSPCIPM
NLLSSYPF

***Arabidopsis thaliana***

MDQEIEIPSF**FLCPISLDIMKDPVIVST*GITYDRESIEKWLF*SGKKNSCPVTKQVIT**
**ETDLTPNHTLRRLIQSWC**TLNASYGIERIPTPKPPICKSEIEKLIKESSSSHLNQVK
CLKRLRQIVSENTTNKRCLEAAEVPEFLANIVSNSVDTYNSPSSSLSSSNLNDMCQS
NMLENRFDSSRSLMDEALSVLYHLDTSETALKSLLNNKKGTNLVKTLTKIMQRGIYE
SRAYAALLLKKLLEVADPMQIILLERELFGEVIQILHDQISHKATRSAMQILVITCP
WGRNRHKAVEGGTISMIIELLMDDTFSSERRNSEMAMVVLDMLCQCAEGRAEFLNHG
A*AIAVVSKKILRVS*QITSERAVRVLLSVGRFCATPSLLQEMLQLGVVAKLCLVLQVS
CGNKTKEKAKELLKLHARVWRESPCVPRNLYDSYPA

***Oryza sativa***

MEEAAAEVPSY**FLCPISLEIMRDPVTLAT*GITYDRSSIERWMF*GGGGGDGGKGTCPV**
**TRRQLAPAEREATPNHTLRRLIQAWC**AAHAVERFPTPRPPVDSCRVAALVDEGTTTM
LGGGGRQRQLAALREIKAIAAESDRNKRCVEATPGAVEFLVSVVVQSHAAASTSASS
DDDDLFDSVIDSPMSTSSPEEEALGVLYSLKPSEPTLRRVLGKDNGVGFLDTLASVL
RRPSYRSRAYAILLLKAVTSAMPPERLMAVSPELVEEVVRVVSDGVSSKAVKAALHV
LCRLCPWGRNRVKAVEAGAVAALVELLLDEEGGGGRRRAAELAVVAIDHLCGCAEGR
SELVAHPA*GLAVVSKRAMRVS*PAATESAVRALHAVARNAATPAVLQEMLAVGVVAKL
LLVLQADGGERARARAREMLRANARVWKDSPCLQAHLKASYPS

FIGURE 5

```
CLUSTAL W (1.83) multiple sequence alignment

gi|30686609|ref|NP_181137.2|      ---MSGGIMDEEIEIPPFFLCPISLEIMKDPVIVSTGITYDRDSIEKWLF 47
gi|15231222|ref|NP_190813.1|      --------MDQEIEIPSFFLCPISLDIMKDPVIVSTGITYDRESIEKWLF 42
gi|76884939|gb|ABA59556.1|        ---------MEEIQVPPYFLCPISLEMMKDPVTISTGITYDRENIERWIF 41
gi|87240319|gb|ABD32177.1|        ---------MDEIDVPSHFLCPISLQLMKDPVTLSTGITYDRENIEKWLF 41
gi|115446555|ref|NP_001047057.    -------MEEAAAEVPSYFLCPISLEIMRDPVTLATGITYDRSSIERWMF 43
gi|125582418|gb|EAZ23349.1|       -------MEEAAAEVPSYFLCPISLEIMRDPVTLATGITYDRSSIERWMF 43
gi|116310809|emb|CAH67599.1|      MGEESSPAAAPAVEVPSYFMCPISLEIMRDPVTLSTGITYDRESIERWVF 50
gi|115461448|ref|NP_001054324.    --MEEQQQVEVEVEVPSYFVCPISLQIMRDPVTLPTGITYDRDGIERWLL 48
gi|115441241|ref|NP_001044900.    -------MASAAAEVPSYFVCPISLQLMRDPVTLPTGISYDRAAIARWLA 43
gi|111073727|dbj|BAF02552.1|      ---------MEEVEIPQYFLCPISLQIMKDPVTTVTGITYDRESIEMWLL 41
                                                ::* .*:*****::*:***    ***:***  *  *:

gi|30686609|ref|NP_181137.2|      AGK-----KNSCPVTKQDITDADLT---PNHTLRRLIQSWCT-LNASYGV 88
gi|15231222|ref|NP_190813.1|      SGK-----KNSCPVTKQVITETDLT---PNHTLRRLIQSWCT-LNASYGI 83
gi|76884939|gb|ABA59556.1|        SAK-----NNTCPVTKQSLTSIELT---PNVTLRRFIQSWCT-LNASHGI 82
gi|87240319|gb|ABD32177.1|        SFQ-----NNTCPVTKQTLLETDLNNLIPNHTLRRLIQSWCT-LNASFGV 85
gi|115446555|ref|NP_001047057.    GGGGGDGGKGTCPVTRRQLAPAEREA-TPNHTLRRLIQAWCA----AHAV 88
gi|125582418|gb|EAZ23349.1|       GGGGGGGGKGTCPVTRRQLAPAEREA-TPNHTLRRLIQAWGR----RRCS 88
gi|116310809|emb|CAH67599.1|      TDG-----HGECPVTKQRLAPADREP-TPNHTLRRLIQGWCA----VHAV 90
gi|115461448|ref|NP_001054324.    TAG-------TCPLTKQPVPPD-CDP-TPNHTLRRLIQSWCA-LHADHGV 88
gi|115441241|ref|NP_001044900.    APGA----RRTCPVTRQPLEHG-LEL-TPNHTLRRLIQSWAASVSPGSAV 87
gi|111073727|dbj|BAF02552.1|      TAEEET-ETAACPVTKQPLPKD-TELLTPNHMLRRLIQAWCI-VNAEKGV 88
                                              **:*:: :            **  ***:**.*

gi|30686609|ref|NP_181137.2|      ERIPTPRPPICKSEIEKLIR-----DSASSHENQVK------CLKRLRQI 127
gi|15231222|ref|NP_190813.1|      ERIPTPKPPICKSEIEKLIK-----ESSSSHLNQVK------CLKRLRQI 122
gi|76884939|gb|ABA59556.1|        ERFPTPKPPVSKAQILKLMK-----EAKSR-EMQMK------SLNTLRSI 120
gi|87240319|gb|ABD32177.1|        ERIPTPKSPIDRTQILKLLN-----EAKKFPEKQLN------CLVKLRSI 124
gi|115446555|ref|NP_001047057.    ERFPTPRPPVDSCRVAALVD-----EGTTTMLGGGGRQRQLAALREIKAI 133
gi|125582418|gb|EAZ23349.1|       ---------------AAAAG-----SGSSPRSG------------EIKAI 106
gi|116310809|emb|CAH67599.1|      ERFPTPRPPVDAARVAAIVD-----AARPLLRRRRQREELMASLRELADI 135
gi|115461448|ref|NP_001054324.    DLVPTPKPPADRARVADLVSRLRAATSSAALLD---------ALRELRDV 129
gi|115441241|ref|NP_001044900.    DEEVAALRPVSSDEVASLLS-----DAAAAQVG---------ALRRLREL 123
gi|111073727|dbj|BAF02552.1|      DRIPTPKYPMNKSNILRLLR-----QVNNNDHQLCA-----EALRKMADL 128
                                                                                :  :

gi|30686609|ref|NP_181137.2|      VSENATNKRCLEA-AGVPEFLANIVSN--DSENG---------------- 158
gi|15231222|ref|NP_190813.1|      VSENTTNKRCLEA-AEVPEFLANIVSNSVDTYNSPSSSLSSSNLNDMCQS 171
gi|76884939|gb|ABA59556.1|        ASVNDANKRCMES-AGAAEFLASVVIDSSDVLGEEGFM------------ 157
gi|87240319|gb|ABD32177.1|        VFESERNKKCLES-AGAIEFLALTMKNN---LNSS--------------- 155
gi|115446555|ref|NP_001047057.    AAESDRNKRCVEATPGAVEFLVSVVVQ--SHAAASTSASSDDD----DLF 177
gi|125582418|gb|EAZ23349.1|       AAESDRNKRCVEATPGAVEFLVSVVVQ--SHAAASTSASSDDD----DLF 150
gi|116310809|emb|CAH67599.1|      VAESDRNRRCVQGASGAVEFLLSVVKERTSVAGVDDATSAKPE----ETT 181
gi|115461448|ref|NP_001054324.    AAESERNRKLLAAVPGAVDVLAAVVVA----------------------- 156
gi|115441241|ref|NP_001044900.    AAECEDSRAMLESQGGVFDVLSRVVTS----------------------- 150
gi|111073727|dbj|BAF02552.1|      VSENEKNRKCMEQAGAIKAMVCFIVKS----------------------- 155
                                  .      .:  :           .:    :
```

FIGURE 6

```
gi|30686609|ref|NP_181137.2|        -----------SLTDEALNLLYHLETSETVLKNLLNNKKDN--NIVKSLT 195
gi|15231222|ref|NP_190813.1|        NMLENRFDSSRSLMDEALSVLYHLDTSETALKSLLNNKKGT--NLVKTLT 219
gi|76884939|gb|ABA59556.1|          -----------STKDEALSILYQLKLSEDGLRSLITSGNG---EFIESLT 193
gi|87240319|gb|ABD32177.1|          -----------SLSEAAIEILFHLNPSEEHVKNLINNESI---QFIESLF 191
gi|115446555|ref|NP_001047057.      DSVIDSPMSTSSPEEEALGVLYSLKPSEPTLRRVLGKDNGV--GFLDTLA 225
gi|125582418|gb|EAZ23349.1|         DSVIDSPMSTSSPEEEALGVLYSLKPSEPTLRRVLGKDNGV--GFLDTLA 198
gi|116310809|emb|CAH67599.1|        CGGVHDPAKASSPEEAALSILHSLKLSEESFKRVLEDSGGE--DFLETMA 229
gi|115461448|ref|NP_001054324.      -----SCRDAKAACDEALEIVCSLELSERCLARLVERNE----ELVDALV 197
gi|115441241|ref|NP_001044900.      -----GS-ACSTAREEAVGVLASLRIPEQELIGVSTRHG----NLAESLT 190
gi|111073727|dbj|BAF02552.1|        ----FKYEKLFPGIEEALRIFHLIWNPKSENKQYVKDNPDLXQAILWILK 201
                                               .  : *: :.  :  .:              :   :

gi|30686609|ref|NP_181137.2|        KIMQRGMYESRVYATLLLKNILEVADPMQSMT-LKPEVFTEVVQILDDR- 243
gi|15231222|ref|NP_190813.1|        KIMQRGIYESRAYAALLLKKLLEVADPMQIIL-LERELFGEVIQILHDQ- 267
gi|76884939|gb|ABA59556.1|          RVMQRGSYESRAYAVMLMKDMFEASTLPTLFSSLKKEFFAQVVRVLRDE- 242
gi|87240319|gb|ABD32177.1|          HVLKLGSYQSRGYATMLLKSAFEVSDPIQLIS-VKKAIFEEIMRVLVDK- 239
gi|115446555|ref|NP_001047057.      SVLRRPSYRSRAYAILLLKAVTSAMPPERLMA-VSPELVEEVVRVVSDG- 273
gi|125582418|gb|EAZ23349.1|         SVLRRPSYRSRAYAILLLKAVTSAMPPERLMA-VSPELVEEVVRVVSDG- 246
gi|116310809|emb|CAH67599.1|        CVLRRPSYLSRMQGIHLLKSALPAMAPARLTS-ASAALVDGVVGVVADR- 277
gi|115461448|ref|NP_001054324.      ATLQRTNTTSRAHAALLLEAVTAVMPSNRLVS-LPEEVFGEAVQLLRDR- 245
gi|115441241|ref|NP_001044900.      AVLRSSNLQSRAHAVQLVRTLADAVVPAWVIG-LNAELLAEVVGVVRDR- 238
gi|111073727|dbj|BAF02552.1|        SNLNTSHVLVKTHAMMVLKNVTEVSSSXLLTG-LDPEFFQQMANTLRKNG 250
                                      :.        :  .  ::.     .               ..      : .

gi|30686609|ref|NP_181137.2|        ---ISQKATKAAMHILVNICPWGRNRHKAVEAGVISVIIELLMDESF--- 287
gi|15231222|ref|NP_190813.1|        ---ISHKATRSAMQILVITCPWGRNRHKAVEGGTISMIIELLMDDTF--- 311
gi|76884939|gb|ABA59556.1|          ---ISQKATKASLQVLAHACPFGRNRVKAAEAGAVRVLVDLLLD------ 283
gi|87240319|gb|ABD32177.1|          ---ISQQASKAALKLLLELLPWGRNRIKAVEGGVVLVLIELLFD------ 280
gi|115446555|ref|NP_001047057.      ---VSSKAVKAALHVLCRLCPWGRNRVKAVEAGAVAALVELLLDE---EG 317
gi|125582418|gb|EAZ23349.1|         ---VSSKAVKAALHVLCRLCPWGRNRVKAVEAGAVAALVELLLDE---EG 290
gi|116310809|emb|CAH67599.1|        ---PSAKAVKVALHVLCRLCPWGRNRVKAVDAGAVSALVRLLLDEG-CGG 323
gi|115461448|ref|NP_001054324.      ---VSSPATRAALHVLVGTTSWGRNRVKAVDAGAVAVLVDMLLDGPV--- 289
gi|115441241|ref|NP_001044900.      ---VSARATKASLHALAALCPYGRHRVKIVGAGAVAALVELLLDEP---- 281
gi|111073727|dbj|BAF02552.1|        KYYISQQATKAALQVLIDACLWGRNRLKIVESGAIFELIELELSNP---- 296
                                         *   *  : ::: *      :**:* *  . .*.:  :: : :.

gi|30686609|ref|NP_181137.2|        -TSERRGPEMAMVVLDLLCQCAEGRAEFLNHGAAIAVVCKKILRVSQTA- 335
gi|15231222|ref|NP_190813.1|        -SSERRNSEMAMVVLDMLCQCAEGRAEFLNHGAAIAVVSKKILRVSQIT- 359
gi|76884939|gb|ABA59556.1|          -SSEKRTCELMLILLDQICQSAEGRAELLNHPGGLAIVSKKILRVSKVG- 331
gi|87240319|gb|ABD32177.1|          -VSERRVCELMLIGLDQICGCAEGRAELLNHGAGLAIVSKKILRVSHVA- 328
gi|115446555|ref|NP_001047057.      GGGRRRAAELAVVAIDHLCGCAEGRSELVAHPAGLAVVSKRAMRVSPAA- 366
gi|125582418|gb|EAZ23349.1|         GGGRRRAAELAVVAIDHLCGCAEGRSELVAHPAGLAVVSKRAMRVSPAA- 339
gi|116310809|emb|CAH67599.1|        GGGDRRACELAAVAIDHICGCAEGRLALVAHPAGLAAVACAATRLPAAAG 373
gi|115461448|ref|NP_001054324.      ---ERRGCELALAALDRMCGCAEGRAALVSHGAGVAVVGRKVLRVSEVA- 335
gi|115441241|ref|NP_001044900.      ---ERRVCELALAVLDRLCTCAEGRAELVAHAAGVAVVGKKVLRVSEAA- 327
gi|111073727|dbj|BAF02552.1|        ---EKRVSELVFCLLANLCVLADGRAELLKHAAGIAVVTKRTLRISSAT- 342
                                        :*  *:    :  :*  *:**  :: * ..:* *     *:.
```

**FIGURE 6 (continued)**

```
gi|30686609|ref|NP_181137.2|       SDRAVRVLLSVGRFCATPALLHEMLQLGVVAKLCLVLQVSC-GGKTKEKA 384
gi|15231222|ref|NP_190813.1|       SERAVRVLLSVGRFCATPSLLQEMLQLGVVAKLCLVLQVSC-GNKTKEKA 408
gi|76884939|gb|ABA59556.1|         SERAIKILHSISKFSSTQSVVQEMLSLGVVAKLCLVLQVDC-GSKAKDRA 380
gi|87240319|gb|ABD32177.1|         SDRGVRIMSSICRYSANSRVLHEMLHVGAVSKLCLVLQVDS-NFKTKERA 377
gi|115446555|ref|NP_001047057.     TESAVRALHAVARNAATPAVLQEMLAVGVVAKLLLVLQADG-GERARARA 415
gi|125582418|gb|EAZ23349.1|        TESAVRALHAVARNAATPAVLQEMLAVGVVAKLLLVLQADG-GERARARA 388
gi|116310809|emb|CAH67599.1|       AESAVRALHAVARHSATSAVLQEMLAVGVVARLLFLVQVGASGERTRARA 423
gi|115461448|ref|NP_001054324.     SEKAVRVLRSVARHAATAAVVQEMGQTGAVEKLCVVAQSEQCGERTRERA 385
gi|115441241|ref|NP_001044900.     SERAVRVLRSVARHAATPAVLQEMAQCGVVGKLCLALRSEQCGVKTKEKA 377
gi|111073727|dbj|BAF02552.1|       DDSALQILGLISKFSATNEVLLEMLRVGGVSKLCMAIQADC-EPHLKKKA 391
                                    : .:: :  : : .:.  :: **   * * :* .  :      : : :*

gi|30686609|ref|NP_181137.2|       KELLKLHARVWKDSPCLPKNMILAYPC------- 411
gi|15231222|ref|NP_190813.1|       KELLKLHARVWRESPCVPRNLYDSYPA------- 435
gi|76884939|gb|ABA59556.1|         RDILKIHAKAWRNSPCIPMNLLSSYPF------- 407
gi|87240319|gb|ABD32177.1|         KEILKLHSTVWKNSTCIPVPLLSSYP-------- 403
gi|115446555|ref|NP_001047057.     REMLRANARVWKDSPCLQAHLKASYPS------- 442
gi|125582418|gb|EAZ23349.1|        REMLRANARVWKDSPCLQAHLKASYPS------- 415
gi|116310809|emb|CAH67599.1|       REMLKMHARVWRDSPCLASHLNASYPR------- 450
gi|115461448|ref|NP_001054324.     RETLRLHARAWRNSPCLQPHLQALYPSC------ 413
gi|115441241|ref|NP_001044900.     HEVLKLHSRVWRASPCLSPSFLALYPS------- 404
gi|111073727|dbj|BAF02552.1|       REILRAHSHVWSNSPCISLYTVYLLTDFLDNSKI 425
```

FIGURE 6

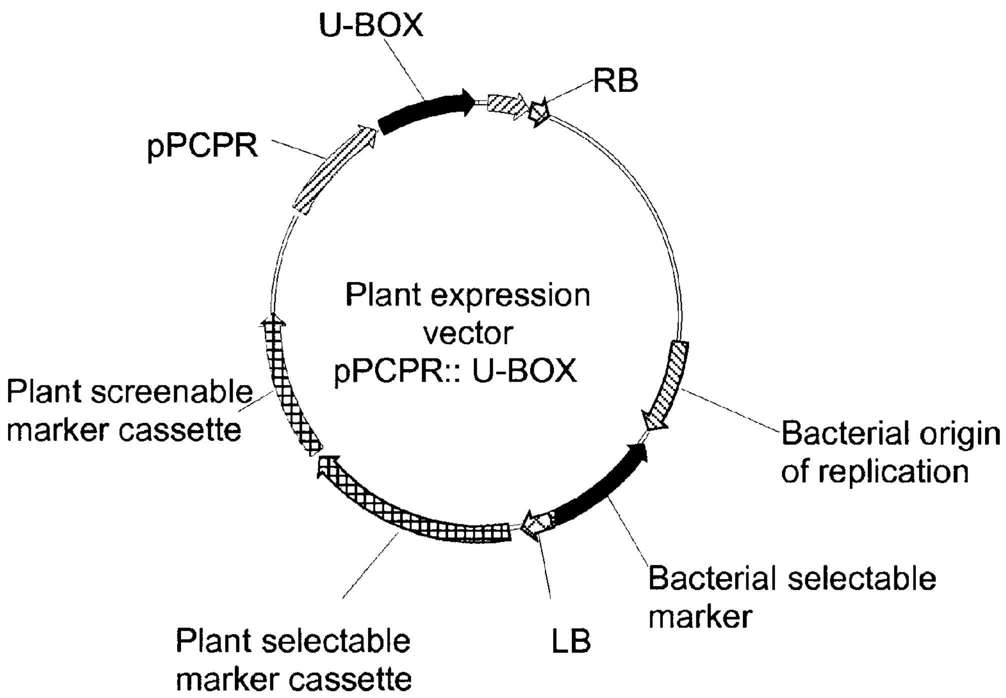


(continued)

FIGURE 7

**SEQ ID NO: 74, *Capsicum annuum* U-box protein (PUB1) mRNA, complete cds**

GCCAACTTTGTACAAAAAAGCAGTCTTAAACAATGGAAGAAATTCAAGTACCACCTTATTTCCTCTG
TCCGATTTCACTGGAGATGATGAAAGATCCAGTCACGATCTCGACTGGAATAACATACGATCGAGAG
AACATCGAGAGATGGATATTCTCAGCTAAGAACAATACGTGTCCGGTTACGAAGCAATCCCTTACGA
GCATAGAGTTGACCCCTAATGTCACTCTACGACGATTCATCCAATCGTGGTGTACTCTCAATGCGTC
CCATGGCATCGAGAGGTTCCCTACACCGAAGCCTCCGGTCAGCAAAGCTCAAATCTTAAAGCTCATG
AAAGAAGCGAAATCGCGCGAAATGCAAATGAAATCACTCAACACACTTAGATCCATAGCTTCTGTGA
ACGATGCTAACAAGCGTTGCATGGAGTCTGCGGGCGCAGCGGAGTTCTTAGCCTCTGTTGTGATTGA
TTCGAGTGATGTGTTGGGTGAAGAGGGTTTCATGAGTACTAAAGATGAAGCATTAAGCATCCTCTAC
CAACTCAAGTTATCAGAAGATGGATTGAGGTCACTCATCACGAGTGGAAATGGTGAATTCATCGAGT
CACTGACACGCGTCATGCAGCGTGGGAGCTATGAGTCAAGGGCTTACGCGGTGATGCTGATGAAAGA
CATGTTCGAAGCATCCACACTACCAACTCTATTTTCGAGCTTGAAGAAAGAGTTCTTCGCTCAAGTG
GTTCGAGTCTTGAGGGATGAGATCTCTCAAAAGGCCACCAAAGCGTCATTGCAAGTGCTAGCGCACG
CGTGTCCATTTGGGAGGAACAGAGTGAAAGCAGCTGAGGCAGGAGCCGTTAGGGTTTTGGTGGACCT
TCTGCTCGATTCATCCGAGAAAAGGACCTGCGAACTGATGCTAATCTTGCTGGATCAAATTTGTCAG
AGCGCAGAAGGGAGAGCCGAGCTGTTGAATCATCCGGGAGGGTTAGCCATCGTCTCGAAGAAAATAC
TTAGGGTTTCGAAAGTAGGGAGCGAAAGGGCAATCAAGATATTGCATTCGATATCAAAGTTTTCATC
AACACAAAGTGTTGTTCAAGAGATGTTGAGTTTGGGTGTTGTGGCAAAGCTTTGTTTGGTACTTCAA
GTTGATTGTGGAAGTAAAGCTAAGGATAGAGCTAGAGATATTCTCAAAATTCATGCAAAGGCATGGA
GGAATTCTCCTTGTATACCTATGAATTTATTATCTTCATATCCATTTTAGGAAAATAGGACCAAAAG
AGCACCCAGCTTTCTTGTACAA

**SEQ ID NO: 75, U-box protein [*Capsicum Annuum*]**

MEEIQVPPYFLCPISLEMMKDPVTISTGITYDRENIERWIFSAKNNTCPVTKQSLTSIELTPNVTLR
RFIQSWCTLNASHGIERFPTPKPPVSKAQILKLMKEAKSREMQMKSLNTLRSIASVNDANKRCMESA
GAAEFLASVVIDSSDVLGEEGFMSTKDEALSILYQLKLSEDGLRSLITSGNGEFIESLTRVMQRGSY
ESRAYAVMLMKDMFEASTLPTLFSSLKKEFFAQVVRVLRDEISQKATKASLQVLAHACPFGRNRVKA
AEAGAVRVLVDLLLDSSEKRTCELMLILLDQICQSAEGRAELLNHPGGLAIVSKKILRVSKVGSERA
IKILHSISKFSSTQSVVQEMLSLGVVAKLCLVLQVDCGSKAKDRARDILKIHAKAWRNSPCIPMNLL
SSYPF

**SEQ ID NO: 76, U-box domain**

FLCPISLEMMKDPVTISTGITYDRENIERWIFSAKNNTCPVTKQSLTSIELTPNVTLRRFIQSWC

**SEQ ID NO: 77, motif 1**

GITYDRENIERWIF

**SEQ ID NO: 78, motif 2**

QSWCTLNA

**SEQ ID NO: 79, motif 3**

GRAELLNH

**SEQ ID NO 80, motif 4**

GLAIVSKKILRVS

FIGURE 8

**SEQ ID NO: 81, motif 5**
KLCLVLQVD

**SEQ ID NO: 82, motif 6**
SYP

**SEQ ID NO: 83, gi|30686608|ref|NM_129152.2| *Arabidopsis thaliana* U-box domain-containing protein (AT2G35930) mRNA, complete cds**
CTAACTTATTCAAACCAATTCACATCTTCCCAAACCCAACTACTACAACTTGTATTAAGAAAAAGATATATTC
CCTTAGCTTCTTTGATCAATATATTCGTCAGGGTTCTCGTCAAAGTCCTCAGCATCTTCATCATATGTCCGGA
GGAATAATGGATGAAGAGATCGAGATTCCTCCGTTCTTCCTTTGTCCTATCTCTTTGGAGATCATGAAAGATC
CAGTGATAGTCTCTACCGGAATAACCTACGACAGAGACAGCATCGAGAAATGGCTATTTGCAGGCAAGAAAAA
CTCGTGTCCGGTCACCAAACAAGACATAACCGACGCGGATCTCACCCCGAATCACACTCTACGCCGTCTCATC
CAATCTTGGTGCACCCTAAACGCCTCCTACGGCGTCGAGAGGATCCCTACCCCAAGACCTCCGATTTGTAAAT
CTGAGATCGAAAAGCTCATTAGAGATTCAGCCTCTTCCCATGAAAACCAAGTCAAGTGTCTCAAACGACTTCG
TCAGATTGTGTCGGAGAACGCGACCAACAAGCGGTGTTTAGAGGCAGCGGGAGTACCGGAGTTCTTGGCCAAC
ATCGTAAGCAACGACTCAGAAAATGGGAGTTTGACCGACGAAGCCCTCAACTTACTTTACCACCTCGAGACCT
CAGAGACAGTCCTCAAGAATCTTTTAAACAACAAGAAAGATAACAATATCGTAAAGTCGTTGACGAAGATCAT
GCAGCGTGGGATGTACGAGTCCAGAGTCTATGCCACTTTGCTTCTCAAGAACATTCTCGAAGTAGCGGATCCA
ATGCAGAGTATGACTCTCAAGCCAGAGGTTTTCACTGAGGTCGTCCAGATCTTGGACGACCGGATCTCGCAGA
AGGCGACCAAAGCTGCCATGCATATATTGGTGAACATATGCCCATGGGGAAGGAACAGACACAAGGCCGTGGA
AGCTGGAGTAATCTCTGTGATCATCGAGCTTCTCATGGACGAGAGCTTCACATCAGAGAGGAGAGGTCCAGAG
ATGGCGATGGTGGTTCTTGATCTGTTGTGTCAGTGTGCGGAGGGACGAGCCGAGTTCTTGAACCACGGAGCAG
CCATAGCGGTGGTGTGCAAGAAGATACTTAGGGTTTCACAGACAGCAAGCGATAGAGCGGTTAGGGTTTTGTT
GTCGGTGGGAAGGTTCTGCGCAACGCCTGCTTTGTTGCACGAGATGTTACAGTTGGGGGTTGTAGCGAAGCTT
TGTCTTGTGCTTCAAGTAAGCTGTGGAGGTAAGACCAAAGAGAAGGCAAAGGAGTTGCTTAAGTTGCACGCTA
GGGTCTGGAAGGACTCGCCTTGTCTCCCAAAAAACATGATTCTTGCATATCCCTGCTGATGAAAACGAAGAAA
ACAATACAAACAAGTCCAATTCTCAGTGAGAGCAATCACATATATGAGAGATGCTGTTGAAAAAACTAAAGGA
GTATTCAATTATTCATATCATTAATCCAATTTTCCTTAGGTTTCTTATTTCGTACTTTTGATGTTCTCAATGT
AGATAATAGTGGAAAATGTTCATCAAATATTTTTTTTATGAAAAACTGCAAAGATATCATATATAAAATCAC
AAGTTACCAGGCT

**SEQ ID NO: 84, gi|30686609|ref|NP_181137.2| U-box domain-containing protein [*Arabidopsis thaliana*]**
MSGGIMDEEIEIPPFFLCPISLEIMKDPVIVSTGITYDRDSIEKWLFAGKKNSCPVTKQDITDADLTPNHTLR
RLIQSWCTLNASYGVERIPTPRPPICKSEIEKLIRDSASSHENQVKCLKRLRQIVSENATNKRCLEAAGVPEF
LANIVSNDSENGSLTDEALNLLYHLETSETVLKNLLNNKKDNNIVKSLTKIMQRGMYESRVYATLLLKNILEV
ADPMQSMTLKPEVFTEVVQILDDRISQKATKAAMHILVNICPWGRNRHKAVEAGVISVIIELLMDESFTSERR
GPEMAMVVLDLLCQCAEGRAEFLNHGAAIAVVCKKILRVSQTASDRAVRVLLSVGRFCATPALLHEMLQLGVV
AKLCLVLQVSCGGKTEKAKELLKLHARVWKDSPCLPKNMILAYPC

## FIGURE 8 (continued)

SEQ ID NO: 85, gi|42565861|ref|NM_115105.3| *Arabidopsis thaliana* U-box domain-containing protein (AT3G52450) mRNA, complete cds
GCTAACTTATTCAAACCAATTCACATATCTCCTAAATCTCAACGACTGCACTTTGAAGCTTTTTGTTTAGATT
CGTTCGTCGTTTCCATTGATTAAGTCTACTTCTTTTTTTTCATATTTTCGAGCGGGTCAAATGGATCAAGAGA
TAGAGATTCCTTCCTTCTTCCTTTGTCCAATCTCTCTAGATATCATGAAAGATCCGGTGATAGTTTCCACCGG
AATAACCTACGACCGAGAAAGCATCGAGAAGTGGCTCTTTTCCGGTAAGAAAAACTCATGTCCAGTCACCAAA
CAAGTCATAACCGAAACTGATCTTACCCCAAACCACACTCTTCGCCGTTTGATTCAATCTTGGTGTACCCTCA
ACGCATCATATGGTATAGAGAGGATCCCAACTCCAAAACCTCCGATCTGTAAATCGGAGATCGAAAAGCTTAT
CAAAGAGTCGTCATCTTCGCATCTAAACCAAGTCAAATGCCTCAAACGACTTCGTCAAATAGTATCTGAGAAT
ACAACCAACAAACGGTGCTTAGAAGCCGCAGAAGTTCCGGAGTTTTTGGCAAATATCGTAAGCAACTCTGTAG
ATACGTATAACTCTCCTTCTTCTTCACTTTCTTCTTCGAATCTCAACGACATGTGTCAGTCAAACATGTTGGA
GAATCGATTTGATTCTTCAAGAAGCTTGATGGACGAAGCCCTAAGCGTACTCTACCATCTTGACACATCGGAG
ACAGCCCTCAAGAGTCTTTTAAACAACAAGAAAGGAACAAATCTTGTGAAAACGTTGACGAAGATTATGCAAC
GTGGGATCTACGAGTCAAGAGCCTATGCGGCTTTGCTTCTCAAGAAGCTCCTGGAAGTTGCGGATCCAATGCA
GATCATATTGTTGGAACGAGAACTTTTCGGTGAGGTGATTCAAATCTTGCACGACCAGATCTCTCACAAGGCG
ACAAGATCAGCAATGCAAATCTTGGTGATCACATGTCCATGGGGAAGGAATAGACACAAGGCAGTGGAAGGTG
GAACAATCTCGATGATAATCGAGCTTTTAATGGACGATACTTTCTCATCAGAGAGAAGGAATTCAGAGATGGC
GATGGTGGTTCTTGATATGTTATGTCAGTGCGCAGAAGGAAGAGCTGAGTTCTTGAATCACGGTGCCGCTATT
GCGGTTGTGTCTAAGAAGATATTGAGGGTCTCACAAATAACTAGTGAAAGGGCAGTTAGGGTTTTGCTTTCGG
TTGGGAGGTTCTGCGCGACACCCTCTTTGTTGCAGGAGATGTTGCAATTGGGAGTTGTAGCAAAGCTGTGTCT
GGTACTTCAAGTTAGCTGTGGTAACAAGACTAAAGAAAAGGCTAAAGAATTGCTTAAGCTTCACGCTAGGGTT
TGGAGGGAATCACCTTGTGTCCCAAGAAATTTGTATGATTCGTATCCTGCTTGAACAACGTACATGTTTGAAT
AATTCTCATCAGATACAAACAATTCTTTGAGAATTATTCTTCGTATAAGTTCATTTTAAAACAAAAAAGAGTG
GTTTAAATTTTGCTCAAACTCTGATTTTCTTTCATTTGTTTTTGTTTTATTATCTTTCAATGTTTATCATTAT
GTGTAGATAATAGTGGACATATTTTCATGAAATTATGAA

**SEQ ID NO: 86, gi|15231222|ref|NP_190813.1| U-box domain-containing protein [*Arabidopsis thaliana*]**
MDQEIEIPSFFLCPISLDIMKDPVIVSTGITYDRESIEKWLFSGKKNSCPVTKQVITETDLTPNHTLRRLIQS
WCTLNASYGIERIPTPKPPICKSEIEKLIKESSSSHLNQVKCLKRLRQIVSENTTNKRCLEAAEVPEFLANIV
SNSVDTYNSPSSSLSSSNLNDMCQSNMLENRFDSSRSLMDEALSVLYHLDTSETALKSLLNNKKGTNLVKTLT
KIMQRGIYESRAYAALLLKKLLEVADPMQIILLERELFGEVIQILHDQISHKATRSAMQILVITCPWGRNRHK
AVEGGTISMIIELLMDDTFSSERRNSEMAMVVLDMLCQCAEGRAEFLNHGAAIAVVSKKILRVSQITSERAVR
VLLSVGRFCATPSLLQEMLQLGVVAKLCLVLQVSCGNKTKEKAKELLKLHARVWRESPCVPRNLYDSYPA

SEQ ID NO: 87, gi|47077943:71488-72699 *Medicago truncatula* chromosome 2 clone mth2-45h12, complete sequence
ATGGATGAAATTGATGTTCCTTCACATTTTCTTTGCCCAATTTCCCTACAACTTATGAAAGATCCTGTCACAC
TTTCCACAGGAATTACGTACGACAGAGAAAACATAGAAAAATGGTTATTTTCATTCCAAAACAATACTTGTCC
TGTTACAAAACAAACCTTATTAGAAACCGATCTTAATAATCTTATACCAAACCATACTTTACGTCGTTTGATT
CAATCTTGGTGTACCCTTAATGCTTCATTTGGTGTTGAACGTATTCCAACACC

## FIGURE 8 (continued)

AAAATCACCAATAGATAGAACACAGATTCTTAAACTCCTCAATGAAGCAAAGAAATTCCCAGAAAAACAACTC
AATTGTCTTGTAAAGCTTCGTTCTATCGTCTTCGAAAGCGAAAGGAACAAAAAATGTTTGGAATCTGCAGGTG
CAATAGAGTTTCTAGCTTTAACCATGAAGAACAATTTAAATTCAAGTTCTCTGAGTGAAGCAGCTATTGAAAT
TTTGTTTCATCTTAACCCTTCTGAAGAACATGTTAAGAATCTGATAAACAATGAAAGCATTCAATTTATTGAG
TCATTGTTTCATGTTTTGAAGCTTGGAAGCTACCAATCTAGAGGTTATGCAACAATGCTGTTGAAATCGGCGT
TTGAAGTATCCGATCCGATTCAATTAATAAGCGTAAAAAAGGCGATTTTTGAAGAGATAATGAGAGTGCTAGT
TGATAAGATCTCACAACAAGCTTCAAAAGCTGCATTAAAACTACTTCTGGAGCTTTTACCATGGGGAAGAAAC
CGAATTAAAGCGGTTGAAGGAGGCGTGGTTTTAGTCCTAATCGAACTTCTTTTCGATGTTTCGGAAAGAAGGG
TTTGTGAATTGATGTTAATAGGTTTGGATCAAATTTGTGGTTGTGCAGAAGGGAGAGCAGAATTGTTGAATCA
TGGAGCAGGATTGGCTATTGTGTCAAAGAAAATTTTGAGGGTTTCTCATGTTGCAAGTGATAGAGGTGTTAGA
ATTATGAGTTCTATTTGTAGGTATTCTGCAAATTCTAGAGTTTTACATGAAATGTTGCATGTTGGAGCAGTTT
CAAAGTTGTGTTTGGTGCTTCAAGTTGATAGTAATTTCAAGACAAAAGAGAGGGCTAAGGAGATACTCAAATT
GCATTCTACTGTTTGGAAGAATTCTACATGTATTCCTGTTCCATTGTTATCTTCTTATCCATGA

**SEQ ID NO: 88, gi|87240319|gb|ABD32177.1| U box [*Medicago truncatula*]**
MDEIDVPSHFLCPISLQLMKDPVTLSTGITYDRENIEKWLFSFQNNTCPVTKQTLLETDLNNLIPNHTLRRLI
QSWCTLNASFGVERIPTPKSPIDRTQILKLLNEAKKFPEKQLNCLVKLRSIVFESERNKKCLESAGAIEFLAL
TMKNNLNSSSLSEAAIEILFHLNPSEEHVKNLINNESIQFIESLFHVLKLGSYQSRGYATMLLKSAFEVSDPI
QLISVKKAIFEEIMRVLVDKISQQASKAALKLLLELLPWGRNRIKAVEGGVVLVLIELLFDVSERRVCELMLI
GLDQICGCAEGRAELLNHGAGLAIVSKKILRVSHVASDRGVRIMSSICRYSANSRVLHEMLHVGAVSKLCLVL
QVDSNFKTKERAKEILKLHSTVWKNSTCIPVPLLSSYP

SEQ ID NO: 89, gi|111073726|dbj|AB248515.1| *Nicotiana benthamiana* mRNA for hypothetical protein, complete cds, clone: NbCD3U14
GAAAGAACGCATCTTTACTCTTCTCTCTCGTTATATATAATTTGAGTCTTGTTTTTATTTGTAAAGAAGAA
AAAAAATAGATATGGAAGAAGTAGAGATACCTCAATATTTTTTGTGTCCTATATCACTTCAAATAATGAAAGA
TCCGGTGACGACGGTGACCGGAATAACGTACGACCGGGAAAGCATCGAGATGTGGTTGTTAACGGCGGAGGAA
GAGACGGAGACGGCGGCGTGTCCGGTAACAAAGCAACCTTTGCCAAAAGACACCGAGTTGTTGACACCAAATC
ATATGCTCCGGCGGCTAATTCAAGCTTGGTGCATAGTCAACGCAGAGAAAGGCGTTGACCGAATTCCAACACC
AAAGTATCCTATGAACAAATCGAATATTCTCCGATTACTTCGACAAGTTAATAATAATGATCATCAGCTTTGT
GCTGAAGCTTTGAGGAAAATGGCTGATTTGGTTAGTGAGAATGAAAAGAACAGGAAATGCATGGAACAAGCTG
GTGCAATTAAGGCTATGGTTTGTTTCATAGTAAAAAGTTTCAAATATGAGAAATTATTTCCTGGTATTGAAGA
AGCTTTAAGGATTTTCCATTTAATTTGGAATCCAAAATCCGAAAACAAGCAGTATGTAAAGGATAACCCTGAT
TTANTTCAAGCAATTTTATGGATTTTAAAGAGCAATTTGAATACCAGTCACGTCCTAGTCAAGACTCATGCAA
TGATGGTTTTGAAGAACGTGACAGAAGTTTCGAGTTCGANTCTTTTAACAGGATTAGACCCCGAGTTTTTCCA
ACAAATGGCGAATACATTGCGAAAAAATGGAAAGTACTACATCTCTCAACAAGCAACAAAAGCAGCTTTACAA
GTGCTAATAGATGCATGTCTATGGGGAAGAAATAGGCTAAAAATCGTCGAATCGGGAGCCATTTTCGAGCTGA
TTGAGCTTGAATTATCTAACCCTGAGAAAAGGGTTAGTGAATTAGTATTCTGTCTATTGGCTAATCTATGCGT
ATTAGCTGATGGGAGAGCAGAATTGTTGAAACATGCAGCTGGAATTGCAGTGGTCACAAAAAGGACACTAAGG
ATATCTTCAGCTACAGATGATAGTGCACTTCAAATTCTTGGTTTAATTTCAAAGTTTTCTGCCACAAACGAAG
TGTTATTGGAAATGTTAAGGGTAGGAGGGGTATCAAAACTTTGCATGGCAATTCAA

**FIGURE 8 (continued)**

GCAGATTGTGAGCCTCATTTGAAGAAGAAAGCAAGAGAGATATTGAGAGCACATTCTCATGTTTGGAGTAATT
CTCCTTGTATCTCCTTGTATACAGTTTATCTTTTGACAGATTTCCTGGACAATAGCAAGATTTGAGCCCTATA
ACCTTGAATAGTTTTTTTTTTCCTTGTATAAATACAGCTAGCCAACATACTGCCTTTTTTGTTATACTGTAAA
ACAGCATCTAAATTACCCATAAAAAAAAAAAAAAAAA

**SEQ ID NO: 90, gi|111073727|dbj|BAF02552.1| hypothetical protein [*Nicotiana benthamiana*]**
MEEVEIPQYFLCPISLQIMKDPVTTVTGITYDRESIEMWLLTAEEETETAACPVTKQPLPKDTELLTPNHMLR
RLIQAWCIVNAEKGVDRIPTPKYPMNKSNILRLLRQVNNNDHQLCAEALRKMADLVSENEKNRKCMEQAGAIK
AMVCFIVKSFKYEKLFPGIEEALRIFHLIWNPKSENKQYVKDNPDLXQAILWILKSNLNTSHVLVKTHAMMVL
KNVTEVSSSXLLTGLDPEFFQQMANTLRKNGKYYISQQATKAALQVLIDACLWGRNRLKIVESGAIFELIELE
LSNPEKRVSELVFCLLANLCVLADGRAELLKHAAGIAVVTKRTLRISSATDDSALQILGLISKFSATNEVLLE
MLRVGGVSKLCMAIQADCEPHLKKKAREILRAHSHVWSNSPCISLYTVYLLTDFLDNSKI

SEQ ID NO: 91, gi|115446554|ref|NM_001053592.1| *Oryza sativa* (japonica cultivar-group) Os02g0539200 (Os02g0539200) mRNA, complete cds
CATTCCCTTCAACTCAAACTCCTCTTCTTCTTCTTCCTCCCATCGATCTCTCTCCTTCGCGATCAACTCGAGC
TCGAACTCGTCTCGTGGTAGTAGCGAGCATGGAGGAGGCGGCGGCGGAGGTGCCGTCCTACTTCCTGTGCCCG
ATCTCGCTGGAGATCATGAGGGACCCGGTGACGCTGGCGACGGGGATCACCTACGACCGGAGCAGCATCGAGC
GGTGGATGTTCGGCGGCGGCGGCGGCGACGGGGGGAAGGGGACGTGCCCGGTGACGCGGCGGCAGCTGGCGCC
GGCGGAGCGGGAGGCGACGCCCAACCACACGCTGCGGCGGCTCATCCAGGCGTGGTGCGCCGCGCACGCCGTC
GAGAGGTTCCCCACCCCGCGCCCGCCCGTCGACTCCTGCCGCGTCGCCGCGCTCGTCGACGAGGGGACGACGA
CGATGCTCGGCGGCGGCGGCAGGCAGCGGCAGCTCGCCGCGCTCCGGGAGATCAAGGCCATCGCCGCCGAGAG
CGACCGCAACAAGCGCTGCGTCGAGGCCACGCCCGGCGCCGTCGAGTTCCTCGTCTCCGTCGTCGTCCAGAGC
CATGCCGCCGCCTCCACCTCAGCCAGCTCGGACGACGACGACCTGTTCGACTCGGTGATCGATTCCCCCATGT
CGACGAGCTCGCCGGAGGAGGAGGCCCTGGGCGTGCTCTACTCCCTCAAGCCGTCCGAGCCCACCCTGCGCCG
CGTCCTCGGCAAGGACAATGGCGTCGGCTTCCTCGACACCCTCGCCTCCGTGCTCCGCCGCCCGAGCTACCGT
TCGCGCGCGTATGCCATCCTCCTCCTGAAGGCGGTGACGTCGGCGATGCCGCCGGAGCGGCTGATGGCGGTGA
GCCCCGAGCTGGTGGAGGAGGTGGTCCGGGTGGTGTCCGACGGCGTGTCGTCGAAGGCGGTGAAGGCGGCGCT
GCACGTGCTGTGCCGGCTGTGCCCGTGGGGGCGGAACCGCGTGAAGGCGGTGGAGGCCGGCGCGGTGGCGGCG
CTGGTGGAGCTCCTCCTCGACGAGGAGGGCGGCGGCGGGCGGCGGCGCGCGGCGGAGCTGGCGGTGGTGGCGA
TCGACCACCTGTGCGGGTGCGCGGAGGGGAGGTCGGAGCTGGTGGCGCACCCGGCGGGGCTCGCCGTGGTGTC
CAAGAGGGCGATGCGGGTGTCGCCGGCGGCCACCGAGAGCGCCGTGCGCGCGCTCCACGCCGTCGCGAGGAAC
GCGGCGACGCCGGCCGTGCTGCAGGAGATGCTCGCCGTCGGCGTGGTGGCGAAGCTGCTGCTGGTGCTGCAGG
CGGACGGCGGCGAGCGCGCCAGGGCGAGGGCGAGGGAGATGCTCAGGGCGAACGCTAGGGTTTGGAAGGACTC
GCCATGCTTGCAAGCTCACCTTAAGGCTTCTTACCCCTCCTGATCATTTCATCACCATCACATCCATCCACCG
ATCTATTAATTATTATTAATTGCTAAGAGGAGCACATGCTTTAATTTGCACTCCTCTTAGTAGTTAATTAGAT
TTTGTGATTACTTTTAGTTCTTTAATTTGTTTATGATCTGAATGTACAAAATACACTGTAAATCTTTCATTAA
TTCTGTACAAAAATACACTATAAATCTTTCATTGCCT

**FIGURE 8 (continued)**

SEQ ID NO: 92, gi|115446555|ref|NP_001047057.1| Os02g0539200 [*Oryza sativa* (japonica cultivar-group)]
MEEAAAEVPSYFLCPISLEIMRDPVTLATGITYDRSSIERWMFGGGGDGGKGTCPVTRRQLAPAEREATPNH
TLRRLIQAWCAAHAVERFPTPRPPVDSCRVAALVDEGTTTMLGGGGRQRQLAALREIKAIAAESDRNKRCVEA
TPGAVEFLVSVVVQSHAAASTSASSDDDDLFDSVIDSPMSTSSPEEEALGVLYSLKPSEPTLRRVLGKDNGVG
FLDTLASVLRRPSYRSRAYAILLLKAVTSAMPPERLMAVSPELVEEVVRVVSDGVSSKAVKAALHVLCRLCPW
GRNRVKAVEAGAVAALVELLLDEEGGGGRRRAAELAVVAIDHLCGCAEGRSELVAHPAGLAVVSKRAMRVSPA
ATESAVRALHAVARNAATPAVLQEMLAVGVVAKLLLVLQADGGERARARAREMLRANARVWKDSPCLQAHLKA
SYPS

SEQ ID NO: 93, gi|115461447|ref|NM_001060859.1| *Oryza sativa* (japonica cultivar-group) Os04g0686000 (Os04g0686000) mRNA, complete cds
AGTACTCCCCATCTCCCATCCACCATTGCTGCCATCTCCACCACATACAATACAACAACTACTACTCAGCTCA
CCATCACCGGAGCAAGAGCCAAGAAGACGACGACGACGACGAGCTAGCAGCAGCATTGCAACCGACGGCAATG
GAGGAGCAGCAGCAGGTGGAGGTGGAGGTGGAGGTGCCGTCCTACTTCGTGTGCCCCATCTCGCTGCAGATCA
TGCGCGACCCGGTGACGCTCCCCACCGGCATCACCTACGACCGCGACGGCATCGAGCGATGGCTCCTCACCGC
CGGCACCTGCCCGCTCACCAAGCAGCCCGTGCCGCCCGACTGCGACCCCACGCCCAACCACACCCTGCGCCGC
CTCATCCAGTCGTGGTGCGCCCTCCACGCCGACCATGGCGTCGACCTCGTCCCCACCCCCAAGCCCCCCGCCG
ACCGCGCCCGCGTCGCCGACCTCGTCTCCCGCCTCCGCGCCGCCACGAGCTCCGCTGCCCTCCTCGACGCGCT
GCGCGAGCTCAGGGACGTTGCCGCCGAGAGCGAGCGTAACAGGAAGCTCCTCGCCGCCGTCCCCGGCGCGGTG
GATGTTCTGGCCGCCGTCGTCGTCGCGTCGTGTCGAGACGCCAAGGCCGCGTGCGACGAGGCCCTCGAGATCG
TCTGCTCGCTGGAGCTCTCGGAGCGGTGCCTGGCGCGGCTCGTCGAGAGGAACGAGGAGCTCGTCGACGCGCT
CGTCGCCACGCTGCAGCGGACGAACACCACGTCCCGGGCGCACGCGGCGCTGCTCCTGGAGGCCGTGACGGCG
GTCATGCCGTCGAACAGGCTGGTGTCCCTCCCGGAGGAGGTGTTCGGTGAGGCGGTGCAGCTGCTCCGCGACA
GGGTATCGAGCCCGGCGACGAGGGCGGCGCTGCACGTGCTGGTCGGCACGACGTCGTGGGGCCGCAACCGCGT
GAAGGCGGTGGACGCCGGCGCGGTGGCGGTGCTGGTCGACATGCTCCTGGACGGGCCAGTCGAGCGGCGCGGG
TGCGAGCTGGCCCTGGCGGCGCTGGACCGGATGTGCGGGTGCGCGGAGGGGCGGGCGGCGCTGGTGTCGCACG
GCGCGGGCGTGGCGGTGGTGGGGAGGAAGGTGCTGAGGGTGTCGGAGGTGGCGAGCGAGAAGGCGGTGCGGGT
GCTGCGGTCGGTGGCGAGGCACGCGGCGACGGCGGCGGTGGTGCAGGAGATGGGGCAGACGGGGGCGGTGGAG
AAGCTGTGCGTGGTGGCGCAGTCGGAGCAGTGCGGGGAGAGGACGCGGGAGCGGGCGCGGGAGACGCTGCGGC
TGCACGCCAGAGCGTGGAGGAACTCGCCCTGCTTGCAGCCTCACCTCCAGGCCCTCTACCCTTCTTGCTGATC
GAATTCTGATTCATGATCACCGGCCTCATCACTAGCTAGCTACCAACATACAGCACAGAGAGAATTGAACACT
TGTTCGATTCTTCCTTTCGCTCGATTAATTTTATTGAGTTGAGATCGTTCGATGTAGTCCTATGAATCAAAGT
GTACAGATCGACGCAATAGATGAACTAAATCATGCGCGAGCTTTTTTTGTTTGGGTCT

SEQ ID NO: 94, gi|115461448|ref|NP_001054324.1| Os04g0686000 [*Oryza sativa* (japonica cultivar-group)]
MEEQQQVEVEVEVPSYFVCPISLQIMRDPVTLPTGITYDRDGIERWLLTAGTCPLTKQPVPPDCDPTPNHTLR
RLIQSWCALHADHGVDLVPTPKPPADRARVADLVSRLRAATSSAALLDALRELRDVAAESERNRKLLAAVPGA
VDVLAAVVVASCRDAKAACDEALEIVCSLELSERCLARLVERNEELVDALVATLQRTN

## FIGURE 8 (continued)

TTSRAHAALLLEAVTAVMPSNRLVSLPEEVFGEAVQLLRDRVSSPATRAALHVLVGTTSWGRNRVKAVDAGAV
AVLVDMLLDGPVERRGCELALAALDRMCGCAEGRAALVSHGAGVAVVGRKVLRVSEVASEKAVRVLRSVARHA
ATAAVVQEMGQTGAVEKLCVVAQSEQCGERTRERARETLRLHARAWRNSPCLQPHLQALYPSC

SEQ ID NO: 95, gi|116310798:75913-77265 *Oryza sativa* (indica cultivar-group) chromosome 4 clone OSIGBa0092M08
TCAGCGAGGGTAGGAAGCATTCAAGTGCGAGGCCAAGCATGGCGAGTCCCTCCACACCCTGGCGTGCATCTTG
AGCATCTCCCTCGCCCTCGCCCTCGTCCGCTCGCCGGAGGCGCCGACCTGCACCAGGAAGAGCAGCCTCGCCA
CCACGCCGACGGCCAGCATCTCCTGCAGCACCGCCGACGTCGCCGAGTGCCTCGCCACGGCGTGCAGCGCGCG
CACGGCGCTCTCGGCGCCCGCCGCGGCGGGCAGCCGCGTCGCCGCGCACGCCACCGCCGCGAGCCCCGCCGGG
TGCGCCACCAGCGCCAGGCGCCCCTCCGCGCAGCCGCAGATGTGGTCGATCGCCACGGCGGCGAGCTCGCACG
CGCGCCGGTCGCCGCCGCCGCCGCCGCAGCCCTCGTCGAGGAGGAGGCGCACGAGCGCCGACACGGCGCCGGC
GTCGACCGCCTTGACGCGGTTCCGGCCCCACGGGCAGAGCCGGCAGAGCACGTGGAGCGCCACCTTGACCGCC
TTCGCCGACGGCCTGTCCGCCACCACCCCCACCACGCCGTCGACCAGCGCGGCGCTCGCCGACGTCAGCCGCG
CGGGGGCCATCGCCGGGAGCGCCGATTTCAGCAGGTGGATTCCCTGCATCCGCGAGAGGTAGCTCGGCCGCCG
CAGCACGCACGCCATGGTTTCCAAGAAATCCTCACCGCCGCTGTCCTCCAAGACCCTCTTGAAGCTCTCCTCG
GAGAGCTTGAGCGAATGCAGGATGCTCAGGGCCGCCTCCTCCGGCGAGCTCGCCTTCGCCGGATCATGCACGC
CGCCGCACGTCGTCTCTTCCGGCTTGGCCGACGTCGCGTCGTCCACGCCCGCCACGGAGGTGCGCTCCTTGAC
GACGGACAGGAGGAACTCCACGGCGCCGGAGGCGCCCTGGACGCACCGCCGGTTGCGGTCGCTCTCGGCGACG
ATGTCGGCGAGCTCCCTCAGCGAAGCCATCAGCTCCTCCCGCTGACGACGCCGGCGCAGCAGCGGCCGCGCGG
CGTCCACGATGGCGGCGACGCGGGCGGCGTCGACGGGGGGGCGCGGGGTGGGGAAGCGCTCGACGGCGTGGAC
GGCGCACCACCCCTGTATGAGGCGGCGGAGCGTGTGGTTGGGCGTGGGCTCCCGGTCCGCCGGCGCCAGCCGC
TGCTTGGTCACCGGGCACTCGCCGTGGCCGTCGGTGAACACCCACCGCTCGATGCTCTCCCGGTCGTAGGTGA
TCCCCGTCGACAGCGTCACCGGGTCCCGCATGATCTCCAGCGAGATCGGGCACATGAAGTAGGACGGCACCTC
CACCGCCGGCGCCGCCGCCGGCGACGACTCCTCACCCAT

SEQ ID NO: 96, gi|116310809|emb|CAH67599.1| OSIGBa0092M08.11 [*Oryza sativa* (indica cultivar-group)]
MGEESSPAAAPAVEVPSYFMCPISLEIMRDPVTLSTGITYDRESIERWVFTDGHGECPVTKQRLAPADREPTP
NHTLRRLIQGWCAVHAVERFPTPRPPVDAARVAAIVDAARPLLRRRRQREELMASLRELADIVAESDRNRRCV
QGASGAVEFLLSVVKERTSVAGVDDATSAKPEETTCGGVHDPAKASSPEEAALSILHSLKLSEESFKRVLEDS
GGEDFLETMACVLRRPSYLSRMQGIHLLKSALPAMAPARLTSASAALVDGVVGVVADRPSAKAVKVALHVLCR
LCPWGRNRVKAVDAGAVSALVRLLLDEGCGGGGDRRACELAAVAIDHICGCAEGRLALVAHPAGLAAVACAA
TRLPAAAGAESAVRALHAVARHSATSAVLQEMLAVGVVARLLFLVQVGASGERTRARAREMLKMHARVWRDSP
CLASHLNASYPR

SEQ ID NO: 97, gi|57015275:c19899567-19899323, c19899240-19898238 *Oryza sativa* (japonica cultivar-group) chromosome 2 chr2, whole genome shotgun sequence
ATGGAGGAGGCGGCGGCGGAGGTGCCGTCCTACTTCCTGTGCCCGATCTCGCTGGAGATCATGAGGGACCCGG
TGACGCTGGCGACGGGGATCACCTACGACCGGAGCAGCATCGAGCGGTGGATGTTCGGCGGCGGCGGCGGCGG
CGGCGGGAAGGGGACGTGCCCGGTGACGCGGCGGCAGCTGGCGCCGGCGGAGCGGGAG

**FIGURE 8 (continued)**

GCGACGCCCAACCACACGCTGCGGCGGCTCATCCAGGCGTGGGGACGACGACGATGCTCGGCGGCGGCGGCAG
GCAGCGGCAGCTCGCCGCGCTCCGGGGAGATCAAGGCCATCGCCGCCGAGAGCGACCGCAACAAGCGCTGCGT
CGAGGCCACGCCCGGCGCCGTCGAGTTCCTCGTCTCCGTCGTCGTCCAGAGCCATGCCGCCGCCTCCACCTCA
GCCAGCTCGGACGACGACGACCTGTTCGACTCGGTGATCGATTCCCCCATGTCGACGAGCTCGCCGGAGGAGG
AGGCCCTGGGCGTGCTCTACTCCCTCAAGCCGTCCGAGCCCACCCTGCGCCGCGTCCTCGGCAAGGACAATGG
CGTCGGCTTCCTCGACACCCTCGCCTCCGTGCTCCGCCGCCCGAGCTACCGTTCGCGCGCGTATGCCATCCTC
CTCCTGAAGGCGGTGACGTCGGCGATGCCGCCGGAGCGGCTGATGGCGGTGAGCCCCGAGCTGGTGGAGGAGG
TGGTCCGGGTGGTGTCCGACGGCGTGTCGTCGAAGGCGGTGAAGGCGGCGCTGCACGTGCTGTGCCGGCTGTG
CCCGTGGGGGCGGAACCGCGTGAAGGCGGTGGAGGCCGGCGCGGTGGCGGCGCTGGTGGAGCTCCTCCTCGAC
GAGGAGGGCGGCGGCGGGCGGCGGCGCGCGGCGGAGCTGGCGGTGGTGGCGATCGACCACCTGTGCGGGTGCG
CGGAGGGGAGGTCGGAGCTGGTGGCGCACCCGGCGGGGCTCGCCGTGGTGTCCAAGAGGGCGATGCGGGTGTC
GCCGGCGGCCACCGAGAGCGCCGTGCGCGCGCTCCACGCCGTCGCGAGGAACGCGGCGACGCCGGCCGTGCTG
CAGGAGATGCTCGCCGTCGGCGTGGTGGCGAAGCTGCTGCTGGTGCTGCAGGCGGACGGCGGCGAGCGCGCCA
GGGCGAGGGCGAGGGAGATGCTCAGGGCGAACGCTAGGGTTTGGAAGGACTCGCCATGCTTGCAAGCTCACCT
TAAGGCTTCTTACCCCTCCTGA

SEQ ID NO: 98, gi|125582418|gb|EAZ23349.1| hypothetical protein OsJ_006832 [*Oryza sativa* (japonica cultivar-group)]
MEEAAAEVPSYFLCPISLEIMRDPVTLATGITYDRSSIERWMFGGGGGGGGKGTCPVTRRQLAPAEREATPNH
TLRRLIQAWGRRRCSAAAAGSGSSPRSGEIKAIAAESDRNKRCVEATPGAVEFLVSVVVQSHAAASTSASSDD
DDLFDSVIDSPMSTSSPEEEALGVLYSLKPSEPTLRRVLGKDNGVGFLDTLASVLRRPSYRSRAYAILLLKAV
TSAMPPERLMAVSPELVEEVVRVVSDGVSSKAVKAALHVLCRLCPWGRNRVKAVEAGAVAALVELLLDEEGGG
GRRRAAELAVVAIDHLCGCAEGRSELVAHPAGLAVVSKRAMRVSPAATESAVRALHAVARNAATPAVLQEMLA
VGVVAKLLLVLQADGGERARARAREMLRANARVWKDSPCLQAHLKASYPS

SEQ ID NO: 99, gi|115441240|ref|NM_001051435.1| *Oryza sativa* (japonica cultivar-group) Os01g0865700 (Os01g0865700) mRNA, complete cds
ATGGCGAGCGCGGCGGCCGAGGTGCCGTCCTACTTCGTCTGCCCGATCTCGCTGCAGCTCATGCGCGACCCCG
TCACGCTCCCTACGGGGATCTCGTACGACCGCGCCGCGATCGCGCGGTGGCTCGCCGCGCCGGGGGCCCGCCG
GACGTGTCCCGTCACGCGGCAGCCGCTCGAGCACGGGCTGGAGCTCACGCCCAACCACACCCTGCGCCGCCTG
ATCCAGTCGTGGGCGGCCTCCGTCTCCCCCGGGTCGGCCGTGGACGAGGAGGTGGCCGCGCTACGACCGGTTT
CGAGCGACGAGGTTGCGTCGCTCCTGTCCGACGCCGCCGCGGCGCAGGTGGGCGCGCTCAGGAGGCTGCGGGA
GCTGGCGGCGGAGTGCGAGGATAGCAGGGCAATGCTGGAGTCCCAGGGCGGGGTGTTCGACGTGCTATCTCGC
GTCGTGACCAGCGGCAGCGCCTGCTCGACGGCGCGGGAGGAGGCGGTTGGTGTCCTCGCGTCGCTCCGGATCC
CGGAGCAGGAGCTGATCGGCGTCTCGACCAGGCACGGCAACCTGGCGGAGTCCCTCACGGCCGTGCTCCGCTC
GTCGAATCTCCAGTCGCGGGCGCACGCGGTGCAGCTCGTGAGGACGCTCGCCGACGCGGTGGTCCCGGCGTGG
GTGATCGGCCTCAACGCGGAGCTCCTCGCCGAGGTGGTCGGCGTGGTCCGCGACCGCGTCTCGGCGCGGGCCA
CCAAGGCGTCCCTCCACGCGCTCGCCGCGCTCTGCCCGTACGGCCGGCACCGCGTGAAGATCGTGGGCGCCGG
CGCGGTGGCGGCGCTGGTGGAGCTGCTGCTGGACGAGCCCGAGCGGCGCGTGTGCGAGCTGGCGCTGGCCGTG
CTGGACCGGCTGTGCACGTGCGCGGAGGGGCGCGCGGAGCTGGTGGCGCACGCGGCGGGCGTGGCCGTGGTGG
GGA

## FIGURE 8 (continued)

AGAAGGTGCTGCGGGTGTCGGAGGCGGCGAGCGAGCGGGCGGTGCGCGTGCTGCGGTCGGTGGCGCGGCACGC
GGCGACGCCGGCGGTGCTGCAGGAGATGGCGCAGTGCGGCGTGGTGGGGAAGCTGTGCCTGGCGCTGCGGTCG
GAGCAGTGCGGGGTGAAGACCAAGGAGAAGGCGCACGAGGTGCTCAAGCTGCACTCCAGGGTCTGGAGGGCCT
CGCCATGCTTGTCTCCCAGCTTCTTGGCGCTTTACCCATCATGA

SEQ ID NO: 100, gi|115441241|ref|NP_001044900.1| Os01g0865700 [*Oryza sativa* (japonica cultivar-group)]
MASAAAEVPSYFVCPISLQLMRDPVTLPTGISYDRAAIARWLAAPGARRTCPVTRQPLEHGLELTPNHTLRRL
IQSWAASVSPGSAVDEEVAALRPVSSDEVASLLSDAAAAQVGALRRLRELAAECEDSRAMLESQGGVFDVLSR
VVTSGSACSTAREEAVGVLASLRIPEQELIGVSTRHGNLAESLTAVLRSSNLQSRAHAVQLVRTLADAVVPAW
VIGLNAELLAEVVGVVRDRVSARATKASLHALAALCPYGRHRVKIVGAGAVAALVELLLDEPERRVCELALAV
LDRLCTCAEGRAELVAHAAGVAVVGKKVLRVSEAASERAVRVLRSVARHAATPAVLQEMAQCGVVGKLCLALR
SEQCGVKTKEKAHEVLKLHSRVWRASPCLSPSFLALYPS

**SEQ ID NO: 101, *Oryza sativa* - protochlorophylid reductase promoter**
CCCACGCGTCCGCCCACGCGTCCGGGACACCAGAAACATAGTACACTTGAGCTCACTCCAAACTCAAACACTC
ACACCAATGGCTCTCCAAGTTCAGGCCGCACTCCTGCCCTCTGCTCTCTCTGTCCCCAAGAAGGGTAACTTGA
GCGCGGTGGTGAAGGAGCCGGGGTTCCTTAGCGTGAGCAGAAGGCCAAGAAGCCGTCGCTGGTGGTGAGGGCG
GTGGCGACGCGGCGGGCCGGTGGCGAGCCCCGGCGCGGGCACGTCGAAGGCGGACGGGAAGAAGACGCTGCGG
CAGGGGGTGGTGGTGATCACCGGCGCGTCGTCGGGGCTCGGGCTCGCGGCGGCGAAGGCGCTTGGCGGAGACG
GGGAAGTGGCACGTGGTGATGGCGTTCCGCGACTTTCCTGAAGGCGGCGACGGCGGCGAAGGCGGCGGGGATG
GCGGCGGGGAGCTACACCGTCATGCACCTGGACCTCGCCTCCCTCGACAGCGTCCGCCAGTTCGTGGACAACT
TCCGGCGCTCCGGCATGCCGCTCGACGCGCTGGTGTGCAACGCCGCACATCTACCGGCCGACGGCGCGGCAAC
CGACGTTCAACGCCGACGGGTACGAGATGAGCGTCGGGGTGAACCACCTGGGCCACTTCCTCCTCGCCCGCCT
CATGCTCGACGACCTCAAGAAATCCGACTACCCGTCGCGGCGGCTCATCATCCTCGGCTCCATCACCGGCAAC
ACCAACACCTTCGCCGGCAACGTCCCTCCCAAGGCCGGGCTAGGCGACCTCCGGGGGCTCGCCGGCGGGCTCC
GCGGGCAGAACGGGTCGGCGATGATCGACGGCGCGGAGAGCTTCGACGGCGCCAAGGCGTACAAGGACAGCAA
GATCTGTAACATGCTGACGATGCAGGAGTTCCACCGGAGATTCCACGAGGAGACCGGGATCACGTTCGCGTCG
CTGTACCCGGGGTGCATCGCGACGACGGGCTTGTTCCGCGAGCACATCCCGCTGTTCCGGCTGCTGTTCCCGC
CGTTCCAGCGGTTCGTGACGAAGGGGTTCGTGTCGGAGGCGGAGTCCGGGAAGCGGCTGGCGCAGGTGGTGGG
CGACCCGAGCCTGACCAAGTCCGGCGTGTACTGGAGCTGGAACAAGGACTCGGCGTCGTTCGAGAACCAGCTC
TCGCAGGAGGCCAGCGACCCGGAGAAGGCCAGGAAGCTCTGGGACCTCAGCGAGAAGCTCGTCGGCCTCGTCT
GAGTTTATTATTTACCCATTCGTTTCAACTGTTAATTTCTTCGGGGTTTAGGGGGTTTCAGCTTTCAGTGAGA
GAGGCCTGTCAAGTGATGTACAATTAGTAATTTTTTTTACCCGACAAATCATGCAATAAAACCACAGGCTTA
CATTATCGATTTGTCCACCTAAATTAAGT

**SEQ ID NO 102, primer: prm9075**
GGGGACAAGTTTGTACAAAAAAGCAGGCTTAAACAATGGAAGAAATTCAAGTACC

**SEQ ID NO 103, primer: prm8785**
GGGGACCACTTTGTACAAGAAAGCTGGGTGCTCTTTTGGTCCTATTTTCCT

## FIGURE 8 (continued)

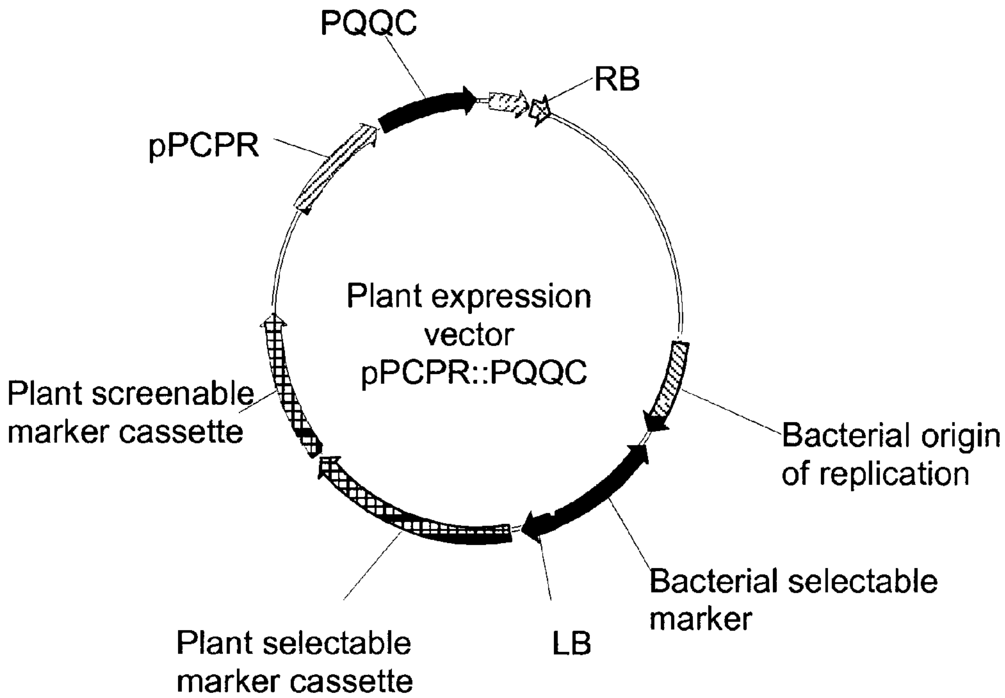


**FIGURE 9A**

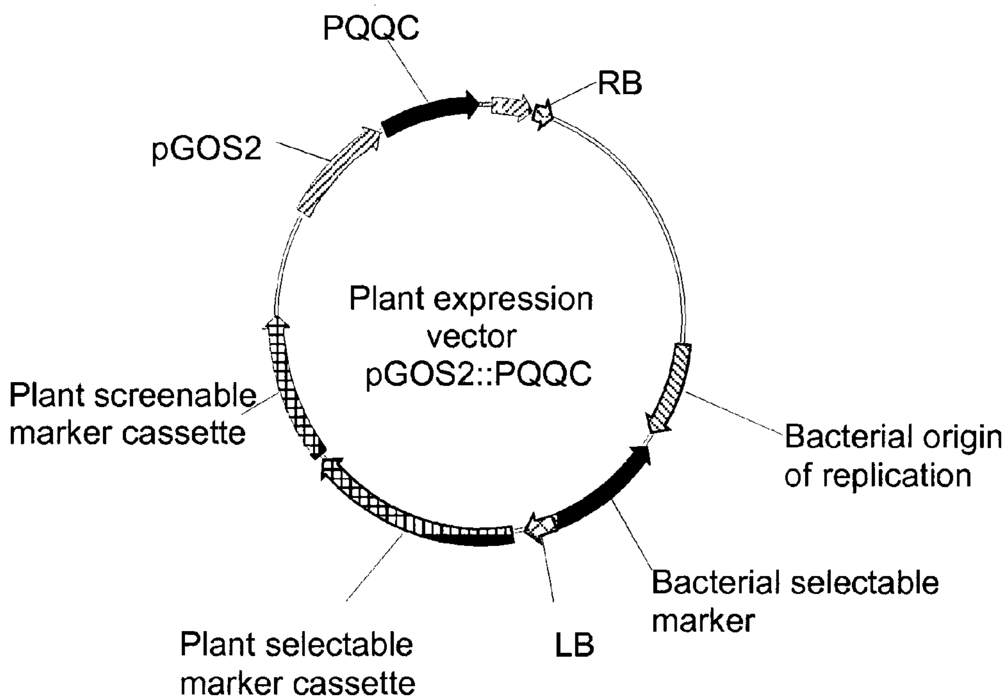


**FIGURE 9B**

```
CLUSTAL W (1.83) multiple sequence alignment

gi|118470311|ref|YP_888023.1|      ----------------MAPLARGAVTQALTPDEFEEALREHVK-DYHHLH 33
gi|134100979|ref|YP_001106640.     ----------------MAALTG--------TDGFVAALRAQSR-RYHDQH 25
gi|71907330|ref|YP_284917.1|       ---------MM---------------TPWNHAEFEAKLRDKGA-AYHIYH 25
gi|148259342|ref|YP_001233469.     ---------MMRADLLAEAPPADDGRAPWSHAEFEAKLREAGS-AYHIHH 40
gi|118594755|ref|ZP_01552102.1     ----------------------MTK--PWTRDEFEDRLRKKGK-GYHIYH 25
gi|91776035|ref|YP_545791.1|       ------------------MNAPLTDNIPWTREEFEQKLRDKGR-GYHIYH 31
gi|119897479|ref|YP_932692.1|      ---MEAELLAAPAARTLAPNAGTHDRPPMSREEFEAQLRAKGT-SYHIYH 46
gi|91777665|ref|YP_552873.1|       ---MNAKDTLGTAPRQNAQTNGGDERPAWTREEFEAQLRAKGA-AYHIHH 46
gi|118033162|ref|ZP_01504607.1     ---MSVKGSSG---------------PAWSRDEFEAQLRAKGQ-AYHIHH 31
gi|121603642|ref|YP_980971.1|      ---MEIAQSPCFDAS-----------RSAWSPAEFERQLRAKGT-AYHIHH 36
gi|121529154|ref|ZP_01661766.1     ---MSRTNASP--------------DAAWPPEEFEVQLRAKGA-GYHIHH 32
gi|84352661|ref|ZP_00977610.1|     ---MNAPIPATALHA-----------TPWTATEFEARLRALES-RYHIHH 35
gi|107025604|ref|YP_623115.1|      ---MNAPIPATALHA-----------TPWTATEFEARLRALES-RYHIHH 35
gi|118710536|ref|ZP_01563114.1     ---MNAPIPATALHA-----------TPWTATEFEARLRALES-RYHIHH 35
gi|134291937|ref|YP_001115706.     ---MNAPIPAAALHA-----------TPWSAQEFEARLRALES-RYHIHH 35
gi|118698279|ref|ZP_01556356.1     ---MNAPIPAAALHA-----------TPWSAQEFEARLRALES-RYHIHH 35
gi|84358446|ref|ZP_00983225.1|     MTTMNAPLPATALHA-----------TPWSAQEFEAHLRALES-RYHIHH 38
gi|124900961|gb|EAY71711.1|        ---MNAPLPATALHA-----------TPWSAQEFEAHLRALES-RYHIHH 35
gi|118718079|ref|ZP_01570613.1     ---MNAPISAAALHA-----------SPWSADEFEARLRALES-RYHIHH 35
gi|124267771|ref|YP_001021775.     ---MSADL-------------------PWSPAEFEARLRAKES-GYHIHH 27
gi|73541469|ref|YP_295989.1|       ---MTYSTASGP-------------------AEFEARLRAKET-GYHIHH 27
gi|116694996|ref|YP_840572.1|      ---MTTAILPIPPIAWT-------------EAEFEARLRAMES-GYHIHH 33
gi|121528464|ref|ZP_01661078.1     ---MTHPVAWTA-------------------EEFEARLRAKQA-GYHIHH 27
gi|121610952|ref|YP_998759.1|      ---MPAPVAPAIAPTVPP------AIPDAERAAFEARLRALQA-RYHIHH 40
gi|149927645|ref|ZP_01915898.1     ---MSSNK-------------------AWSKKEFEERLRAKGD-RYHIHH 27
gi|59891641|gb|AAX10038.1|         ----------------------MTD-TPMSPAEFEAALRAKGA-YYHIYH 26
gi|77461380|ref|YP_350887.1|       ----------------------MTD-TPLSPAEFEAALRAKGA-YYHIHH 26
gi|70732960|ref|YP_262733.1|       ----------------------MTD-TPLSAAEFEQALRAKGA-YYHIHH 26
gi|66047896|ref|YP_237737.1|       ----------------------MSEATALSPAEFEQALRAKGA-YYHIYH 27
gi|71734409|ref|YP_276808.1|       ----------------------MSEATALSPAEFEQALRAKGA-YYHIYH 27
gi|28867740|ref|NP_790359.1|       ----------------------MSEATALSPAEFEQALRAKGA-YYHIYH 27
gi|26987120|ref|NP_742545.1|       ----------------------MSDALPMSPAEFEQALRAKGA-YYHIHH 27
gi|148545658|ref|YP_001265760.     ----------------------MSDALPMSPAEFEQALRAKGA-YYHIHH 27
gi|126360078|ref|ZP_01717067.1     ----------------------MSDALPMSPAEFEQALRAKGA-YYHIHH 27
gi|119859344|ref|ZP_01640760.1     ----------------------MSEALPLSPAEFEQALRAKGA-YYHIHH 27
gi|104779674|ref|YP_606172.1|      ----------------------MNDAAPMSPAEFEQALRAKGA-FYHIHH 27
gi|146306995|ref|YP_001187460.     -----------------------MSQAAMTPAEFEQALRAKGA-LYHIHH 26
gi|146282431|ref|YP_001172584.     --------------------------------------------------
gi|150958981|gb|ABR81006.1|        ---MPVASKWPGMACTSSCRGTDMSRAAMDRAEFERALRDKGR-YYHIHH 46
gi|94416471|ref|ZP_01296297.1|     -----------------------MSRAAMDRAEFERALRDKGR-YYHIHH 26
gi|15597183|ref|NP_250677.1|       -----------------------MSRAAMDRAEFERALRDKGR-YYHIHH 26
gi|67154257|ref|ZP_00416002.1|     -----------------------MTQTAMSPAEFERALRAKGA-YYHIHH 26
gi|30841333|gb|AAP34380.1|         ----------------------MIITEALSPAAFEQALRDKGA-FYHIHH 27
gi|150955212|gb|ABR77242.1|        ----------------------MLITDTLSPQAFEEALRAKGD-FYHIHH 27
gi|113196161|gb|ABI31435.1|        ----------------------MTQPLPLDPQAFEAR-RPQGA-YYHIHH 26
gi|50085585|ref|YP_047095.1|       -------------------MRFNMTTTLFSPAEFEQALRDKGR-YYHIHH 30
gi|149375810|ref|ZP_01893578.1     ----------------------------MNRGDFEQALRAKGQ-YYHIHH 21
gi|126666044|ref|ZP_01737024.1     ------------------MNAIVN--TTLNRADFEQALRNKGR-LYHIHH 29
gi|89092880|ref|ZP_01165832.1|     ------------------MNTQANDKLPMSREAFEKALRAKGD-LYHTQH 31
gi|90417324|ref|ZP_01225250.1|     ------------------------MTKAWTAEEFEQQLRDKGA-LYHIHH 25
gi|119474895|ref|ZP_01615248.1     ------------------------MSKPWSREEFERGLRAKEA-LYHIHH 25
gi|71282639|ref|YP_267608.1|       ------------------------MQQPWTREQFEEMLRAKGQ-LYHIHH 25
gi|118073743|ref|ZP_01541920.1     -------------------------MQAWTKEEFEQKLKDKGS-LYHIHH 24
gi|114770609|ref|ZP_01448099.1     ----------------------MTKQAPWSRDEFEAKLRAKGE-YYHIYH 27
gi|119504323|ref|ZP_01626403.1     -----------MSLLDTASSRDASNQAPLTRADFEARLRAKGS-RYHIHH 38
gi|77166079|ref|YP_344604.1|       ----------------------MAGKKPWSREEFEQKLQEKEC-FYHIHH 27
gi|53804508|ref|YP_113905.1|       ---------------------MSQDTIPWSREEFEARLRAKEK-YYHIHH 28
gi|94493803|ref|ZP_01301006.1|     -------------------MPTKRCEVSLSQSVFEKKILNLEK-YYHIHH 30
gi|88812029|ref|ZP_01127282.1|     --------------------------------------------------
gi|126729612|ref|ZP_01745425.1     -----------------------MTKAPQSRDAFEARLRQIGAERYHDLH 27
gi|16263947|ref|NP_436739.1|       -----------------------MTTAT-DRQAFHARLLEIGKERYHDKH 26
```

FIGURE 10

```
gi|118470311|ref|YP_888023.1|       PFHRRMNTGQCSPEEIRGWVANRFYYQVNIPRKDAAILSTCPDREIRRRW 83
gi|134100979|ref|YP_001106640.      PFHVRMNGGRLSRRQIQGWVANRFYYQESIPLKDAAILSNCPDREVRRRW 75
gi|71907330|ref|YP_284917.1|        PFNVLLNTGQAKREQIQGWVANRYYYQIIIPVKDAAIMSNCPDREIRRDW 75
gi|148259342|ref|YP_001233469.      PFNVMLNTGKATPEQIRMWVANRFYYQIAIPVKDAAILSNCPDREIRRGW 90
gi|118594755|ref|ZP_01552102.1      PFHVMMYEGKLSKEQLQSWVLNRFYYQISIPLKDAAILSNCPVKEIRKEW 75
gi|91776035|ref|YP_545791.1|        PFHVMMYEGKLTREQLQCWVANRFYYQISIPQKDAAILSNCPDKEIRKGW 81
gi|119897479|ref|YP_932692.1|       PFHVMMAEGRLTPAQLRGWVANRFYYQIAIPVKDAAIMSNCPDREIRREW 96
gi|91777665|ref|YP_552873.1|        PFNVKMNSGGCSREQIRGWVANRFYYQINIPLKDAAVLSNCPDRETRRRW 96
gi|118033162|ref|ZP_01504607.1      PFNVKMNSGGCSHEQIRGWVANRFYYQINIPLKDAAVLSNCPDRDTRRRW 81
gi|121603642|ref|YP_980971.1|       PFNVRMNQGGCTADEIRCWVANRFYYQLCIPRKDAAILANMPDRAHRRLW 86
gi|121529154|ref|ZP_01661766.1      PFNVRMNNGELTPDQVRGWIANRFYYQVNIPRKDGAIIANCPDRETRRLW 82
gi|84352661|ref|ZP_00977610.1|      PFNRRLNSGACSPTQIRGWVANRFYYQICIPLKDAAILSNCPDRETRRRW 85
gi|107025604|ref|YP_623115.1|       PFNRRLNSGACSPTQIRGWVANRFYYQICIPLKDAAILSNCPDRETRRRW 85
gi|118710536|ref|ZP_01563114.1      PFNRRLNSGACSPTQIRGWVANRFYYQICIPLKDAAILSNCPDRDTRRRW 85
gi|134291937|ref|YP_001115706.      PFNRRMNAGECSREQIRGWVANRFYYQISIPLKDAAILSNCPDRETRRLW 85
gi|118698279|ref|ZP_01556356.1      PFNRRLNTGACSPEQIRGWVANRFYYQISIPLKDAAILSNCPDRDTRRLW 85
gi|84358446|ref|ZP_00983225.1|      PFNRRLNSGACSPEQIRGWVANRFYYQISIPLKDAAILSNCPDRDTRRLW 88
gi|124900961|gb|EAY71711.1|         PFNRRLNSGACSPEQIRGWVANRFYYQISIPLKDAAILSNCPDRDTRRLW 85
gi|118718079|ref|ZP_01570613.1      PFNRRLNAGACTPAQIRGWVANRFYYQISIPLKDAAILSNCPDRDTRRLW 85
gi|124267771|ref|YP_001021775.      PFNKRLNSGALQPFQVRGWVANRFYYQQAIPMKDAAVMANCEDRATRRRW 77
gi|73541469|ref|YP_295989.1|        PFNQRMAAGLCSPGQIRAWVANRFYYQANIPLKDAAILANCPERDTRREW 77
gi|116694996|ref|YP_840572.1|       PFNQRMAAGQCSPAQIRAWVANRFYYQDNIPLKDAAILSNCPERDTRREW 83
gi|121528464|ref|ZP_01661078.1      PFNLRMRAGECTPEEIRTWVANRFYYQVNIPLKDAAILSNCPDRATRREW 77
gi|121610952|ref|YP_998759.1|       PFNQRMASGQCSRAQIQGWVANRFYYQVNIPLKDAAILSNCPDRATRREW 90
gi|149927645|ref|ZP_01915898.1      PFNVAMREGKLSREQLRGWVANRFYYQVCIPQKDAAVMANCDDRETRRRW 77
gi|59891641|gb|AAX10038.1|          PYHVAMYEGRATREQIQGWVANRFYYQVNIPLKDAAILANCPDREIRREW 76
gi|77461380|ref|YP_350887.1|        PYHVAMYEGRATREQIQGWVANRFYYQVNIPLKDAAILANCPDREIRREW 76
gi|70732960|ref|YP_262733.1|        PYHVAMYEGRATREQIQGWVANRFYYQVNIPMKDAAILANCPDREIRREW 76
gi|66047896|ref|YP_237737.1|        PFHVAMYEGRATREQIQGWVANRFYYQVNIPLKDAAILANCPDREIRREW 77
gi|71734409|ref|YP_276808.1|        PYHVAMYEGRATREQIQGWVANRFYYQVNIPLKDAAILANCPDREIRREW 77
gi|28867740|ref|NP_790359.1|        PFHVAMYEGRATREQIQGWVANRFYYQVNIPLKDAAILANCPDREIRREW 77
gi|26987120|ref|NP_742545.1|        PYHVAMYQGRATREQIQGWVANRFYYQVNIPMKDAAILANCPDREVRREW 77
gi|148545658|ref|YP_001265760.      PYHVAMYQGRATREQIQGWVANRFYYQVNIPMKDAAILANCPDREVRREW 77
gi|126360078|ref|ZP_01717067.1      PYHVAMYQGRATREQIQGWVANRFYYQVNIPMKDAAILANCPDREVRREW 77
gi|119859344|ref|ZP_01640760.1      PYHVAMYEGRATREQIQGWVANRFYYQVNIPMKDAAILANCPDREVRREW 77
gi|104779674|ref|YP_606172.1|       PYHVAMYEGRATREQIQGWVANRFYYQVNIPMKDAAILANCPDREVRREW 77
gi|146306995|ref|YP_001187460.      PFHRAMYEGRATREQIQGWVANRFYYQVNIPLKDAAILANCPDRETRREW 76
gi|146282431|ref|YP_001172584.      -----MYAGQATREQIQGWVANRFYYQVCIPVKDAAIMANCPDRDTRREW 45
gi|150958981|gb|ABR81006.1|         PFHVAMYEGRASREQIQGWVANRFYYQLNIPLKDAAILANCPDREVRREW 96
gi|94416471|ref|ZP_01296297.1|      PFHVAMYEGRASREQIQGWVANRFYYQLNIPLKDAAILANCPDREVRREW 76
gi|15597183|ref|NP_250677.1|        PFHVAMYEGRASREQIQGWVANRFYYQVNIPLKDAAILANCPDREVRREW 76
gi|67154257|ref|ZP_00416002.1|      PFQVAMYEGRSTREQIRGWVANRFYYQVNIPLKDAAILANCPDREVRREW 76
gi|30841333|gb|AAP34380.1|          PYHIAMHNGEATREQIQGWVANRFYYQTNIPLKDAAIMANCPDAATRRKW 77
gi|150955212|gb|ABR77242.1|         PYHIAMHNGNATREQIQGWVANRFYYQTTIPLKDAAIMANCPDAQTRRKW 77
gi|113196161|gb|ABI31435.1|         PYHIAMHNGEATREQIQGWVANRFYYQTSIPIKDAAIMANCPQPETRRKW 76
gi|50085585|ref|YP_047095.1|        PYHIMMNDGHATKQQIQGWVANRFYYQVNIPLKDAAIMANCPDPATRRKW 80
gi|149375810|ref|ZP_01893578.1      PYHKAMYGGDCTPEQIRGWVANRYYYQINIPRKDAAIMANCPDASVRRLW 71
gi|126666044|ref|ZP_01737024.1      PYHQAMYGGQCSPEQIRGWVANRYYYQVMIPRKDAAIMANCPDASVRKQW 79
gi|89092880|ref|ZP_01165832.1|      PYHIAMYNGQSTPEQIRGWVANRFYYQISIPVKDAAIMANCPDRDVRRHW 81
gi|90417324|ref|ZP_01225250.1|      PFHIAMNTGNCTQKQIQGWVANRYYYQISIPIKDAAIMANCDDASVRRLW 75
gi|119474895|ref|ZP_01615248.1      PLHVAMNSGQCNQQQIQGWVANRYYYQVNIPIKDANILANCTEPTVRRLW 75
gi|71282639|ref|YP_267608.1|        PFHIAMHQGECSVEQIQGWVANRFYYQVNIPIKDAAILANCHDMETRRLW 75
gi|118073743|ref|ZP_01541920.1      PYHKMMHKGECSVEQIRGWVANRFYYQINIPVKDAAILANCRDVKTRRMW 74
gi|114770609|ref|ZP_01448099.1      PFHVAMNKGECSQEQIQGWVANRLFYQLAIPVKDAGIMANCWDLQTRRKW 77
gi|119504323|ref|ZP_01626403.1      PFHQAMYAGELTPRQIRGWVANRYYYQIMIPQKDAAILANCSDREIRVKW 88
gi|77166079|ref|YP_344604.1|        PFQVLMNNGKLNQYQVQGWVANRFYYHTCIPLKDAAILANCSQRSVRRQW 77
gi|53804508|ref|YP_113905.1|        PYHVLMASGELNREQIQGWVANRFYYQITIPRKDSAIMANCPDRETRRKW 78
gi|94493803|ref|ZP_01301006.1|      PFNQLMNDGQLTRQQIQQWVINRFYYQVNIPVKDAAILSNCPLREIRREW 80
gi|88812029|ref|ZP_01127282.1|      ---MMMNRGELSRHQLQGWVANRYYYQISIPLKDAALISQCPDRAVRRHW 47
gi|126729612|ref|ZP_01745425.1      PFHDRLHGGQCTMEEVQAWVINRYYYQHSIPMKDAAFMSRVEDPDLRRAW 77
gi|16263947|ref|NP_436739.1|        PFHAMLHGGGCTTTQVRAWVINRYYYQSRIPMKDAAFLSRCDDPDMRRAW 76
                                          :  *      ::: *: ** :*:   ** **. .::        *  *
```

# FIGURE 10 (continued)

```
gi|118470311|ref|YP_888023.1|      VQRIIDHDGTT------EGT---GGIEAWLRLAEAVGLTRAEVEDARHLL 124
gi|134100979|ref|YP_001106640.     VRRILDHDGAP------GGR---GGIDAWLHLAEAVGLTREEVLDERHVV 116
gi|71907330|ref|YP_284917.1|       IQRIIDHDG---------RGDDAGGIEAWIRLGEAVGLAREEITDLRHVI 116
gi|148259342|ref|YP_001233469.     IRRLLDHDGFDYELPDGSRLRDEGGIEAWLRLGIATGLAREEMLDLRHLL 140
gi|118594755|ref|ZP_01552102.1     IVRILDHD---------GEGDADGGIEAWINLGRAVGLTREDVTSLKHVS 116
gi|91776035|ref|YP_545791.1|       IQRIIDHD---------GDANNVGGIEAWIQLGQAVGLSREDVTSLKFVA 122
gi|119897479|ref|YP_932692.1|      IQRILDHDGYEI-----GGVSDPGGIEAWIRLGEAVGLSRDDVTSLRFVA 141
gi|91777665|ref|YP_552873.1|       VLRILDHDGCG-----DEE----GGIETWARLGDAVGLSRDDLWSLKQVA 137
gi|118033162|ref|ZP_01504607.1     VLRILDHDGYG-----EDE----GGIETWARLGDAVGLSRDELWSLEHVV 122
gi|121603642|ref|YP_980971.1|      VERILDHDGYG-----DYQGCAAGGMEAWTQLGLAVGLSREELWSLQGVA 131
gi|121529154|ref|ZP_01661766.1     VQRILDHDGNA-----DSP----GGIEAWVRLGEAAGIPEQDLWSLKHVV 123
gi|84352661|ref|ZP_00977610.1|     IQRLIDHDGQG-----DN----AGGIEAWVRLGEACGLTRDELWSLEHVL 126
gi|107025604|ref|YP_623115.1|      IQRLIDHDGQG-----DN----AGGIEAWVRLGEACGLTRDELWSLEHVL 126
gi|118710536|ref|ZP_01563114.1     IQRLIDHDGQG-----DN----AGGIEAWVRLGEACGLARDELWSLEHVL 126
gi|134291937|ref|YP_001115706.     VERILDHDGHG-----ES----EGGIEAWARLGEAVGIERAQLWSLEHVL 126
gi|118698279|ref|ZP_01556356.1     VERILDHDGHG-----GQ----EGGIEAWARLGEAVGIERAELWSLVHVL 126
gi|84358446|ref|ZP_00983225.1|     VQRILDHDGHG-----GD----AGGIEAWARLGEAVGIPRSQLWSLERVL 129
gi|124900961|gb|EAY71711.1|        VQRILDHDGHG-----GD----AGGIEAWARLGEAVGIPRSQLWSLERVL 126
gi|118718079|ref|ZP_01570613.1     VQRILDHDGHG-----DD----AGGIEAWVRLGEAVGLDRAALWSLEHVL 126
gi|124267771|ref|YP_001021775.     IERMLDHDGHG-----DHEGQNAGGIETWTRLGMAVGLSREDLWSHRHVQ 122
gi|73541469|ref|YP_295989.1|       VVRILDHDGTD-----TQP----GGIEAWLRLGQAIGLERAALLDHRHVL 118
gi|116694996|ref|YP_840572.1|      VVRILDHDGTD-----TQP----GGIEAWLRLGQAVGLEREALLSHRHVL 124
gi|121528464|ref|ZP_01661078.1     IVRIHDHDGDD-----AQP----GGIESWVRLGVAVGLTRDALWSHRHLL 118
gi|121610952|ref|YP_998759.1|      VVRILDHDGSA-----AEP----GGIEAWSRLGEAVGLTRAALWSQQMVL 131
gi|149927645|ref|ZP_01915898.1     VQRILDHDGWE-----GN----AGGIEAWSRLGEAVGIDKDVLWSQQLVL 118
gi|59891641|gb|AAX10038.1|         IQRLLDHDGA------PGED---GGIEAWLRLGQAVGLDPDQLRSQELVL 117
gi|77461380|ref|YP_350887.1|       IQRLLDHDGA------PGED---GGIEAWLRLGQAVGLDPDQLRSQELVL 117
gi|70732960|ref|YP_262733.1|       IQRLLDHDGA------PGED---GGIEAWLRLGQAVGLDPDQLRSQELVL 117
gi|66047896|ref|YP_237737.1|       IQRLLDHDGA------PGED---GGIEAWLRLGQAVGLDPDQLRSQELVL 118
gi|71734409|ref|YP_276808.1|       IQRLLDHDGA------PGED---GGIEAWLRLGQAVGLDPDQLRSQELVL 118
gi|28867740|ref|NP_790359.1|       IQRLLDHDGA------PGED---GGIEAWLRLGEAVGLDPDQLRSQELVL 118
gi|26987120|ref|NP_742545.1|       IQRLLDHDGA------PGED---GGIEAWLRLGQAVGLDPDQLRSQELVL 118
gi|148545658|ref|YP_001265760.     IQRLLDHDGA------PGED---GGIEAWLRLGQAVGLDPDQLRSQELVL 118
gi|126360078|ref|ZP_01717067.1     IQRLLDHDGA------PGED---GGIEAWLRLGQAVGLDPDQLRSQELVL 118
gi|119859344|ref|ZP_01640760.1     IQRLLDHDGA------PGED---GGIEAWLRLGQAVGLDPDQLRSQELVL 118
gi|104779674|ref|YP_606172.1|      VQRLLDHDGA------PGED---GGIEAWLRLGQAVGLDPDQLRSQELVL 118
gi|146306995|ref|YP_001187460.     IQRILDHDGA------PGEE---GGIEAWLRLAESVGLDREQVLSQELVL 117
gi|146282431|ref|YP_001172584.     IQRIIDHDGA------PGEE---GGIEAWLRLAEAVGLDREQVLSQELVL 86
gi|150958981|gb|ABR81006.1|        VQRILDHDGA------PGEA---GGIEAWLRLAEAVGLEREQVLSEERVL 137
gi|94416471|ref|ZP_01296297.1|     VQRILDHDGA------PGEA---GGIEAWLRLAEAVGLEREQVLSEERVL 117
gi|15597183|ref|NP_250677.1|       IQRILDHDGA------PGEA---GGIEAWLRLAEAVGLEREQVLSEERVL 117
gi|67154257|ref|ZP_00416002.1|     VQRILDHDGG------PGEE---GGIEAWLRLGEAVGLDREQLLSQELVL 117
gi|30841333|gb|AAP34380.1|         VQRILDHDGS------NGHE---GGIEAWLQLGEAVGLERDVLLSEKLVL 118
gi|150955212|gb|ABR77242.1|        VQRILDHDGS------HGED---GGIEAWLRLGEAVGLSRDDLLSERHVL 118
gi|113196161|gb|ABI31435.1|        VQRILDHHGT------DGSE---GGIEAS-DLGEAVGLQRDTLLSEERVL 116
gi|50085585|ref|YP_047095.1|       VQRILDHDGQ------HDDH---GGIEAWLRLGEAVGLDRETILSQKMVL 121
gi|149375810|ref|ZP_01893578.1     LQRVLDHDGH------IDDE---GGIEAWLSLAEAVGLTRDEVTDQRHVL 112
gi|126666044|ref|ZP_01737024.1     LQRILDHDGH------DGDA---GGIEAWLCLAEAVGLTREEVIDQRHVL 120
gi|89092880|ref|ZP_01165832.1|     VQRVLDHDGY------DGAE---GGIEAWLRLGEAVGLTREEILSGEHVL 122
gi|90417324|ref|ZP_01225250.1|     VQRILDHDGT------SDEDS--GGIEAWLRLGEAVGLTRDEIISQQHIL 117
gi|119474895|ref|ZP_01615248.1     VQRILDHDGT------SEQDS--GGIEAWLRLGEAVGLQRSEITSFQHVL 117
gi|71282639|ref|YP_267608.1|       IQRILDHDGSV-----EDKTL--GGIEAWLQLGQAVGLDRQDLLDEKFVL 118
gi|118073743|ref|ZP_01541920.1     IQRIVDHDGSI-----EDNTL--GGIEAWLKLGEAVGLNRQDMLDEKYIL 117
gi|114770609|ref|ZP_01448099.1     IQRILDHDGDP-----EDTTL--GGIEAWLRLGEAVGLHREDMLNERFLL 120
gi|119504323|ref|ZP_01626403.1     IQRILDHDGA------DGDE---GGIEAWLELAEAVGLKRDEVTDLRYVL 129
gi|77166079|ref|YP_344604.1|       VQRILDHDGY-------GEDV--GGIEAWLQLGEAVGLSRETLESQQQVL 118
gi|53804508|ref|YP_113905.1|       MQRVMDHDGY-------GDDP--GGIEAWIQLGIACGLSREDITSLRHVL 119
gi|94493803|ref|ZP_01301006.1|     VKRIIEHDGDVIN---------PGGIEAWLTLGEACGLTRNLLWSMKYVL 121
gi|88812029|ref|ZP_01127282.1|     LHRLLDQDGR------TGSE---GGIEAWINLGEAVGLSRIELISERQVL 88
gi|126729612|ref|ZP_01745425.1     RSRMEDHDGT------ADNE---GGIRRWLRLAEAVGLDPDYVSTTEGVL 118
gi|16263947|ref|NP_436739.1|       RSRIEDHDGG------VEEG---GGIRRWLRLAEAVGLDPAYVGSARGVL 117
                                     *: ::.             **:      *. : *:     :      :
```

FIGURE 10 (continued)

```
gi|118470311|ref|YP_888023.1|     PGVRFAVDAYVNFTRTRPWLEAVASSLTELFAPDLMAERLAAFERHYTWI 174
gi|134100979|ref|YP_001106640.    PGVRFAVDAYVTFARTRPWTEAVASSLTELFAPDLMAQRLAAFERCYPWI 166
gi|71907330|ref|YP_284917.1|      PAVRFACDAYLNFCRRQPWQEAVCSSLTELFAPKIHKERLANWPEFYPWI 166
gi|148259342|ref|YP_001233469.    PGVRFAVDAYVNFARRAPWQEAVCSSLTELFAPDIHRQRLATWPGHYPWI 190
gi|118594755|ref|ZP_01552102.1    PGVKFAVDAYINFAKQKSWQESVCSSLTELFAPHIHQQRISSWPSMYPWI 166
gi|91776035|ref|YP_545791.1|      PGVRFAVDAYVNFAKQRPWQEAVCSSLTELFAPHIHQQRISSWPSMYPWV 172
gi|119897479|ref|YP_932692.1|     PAARFAVDAYINFARQRPWEEAVASSLTELFAPHIHQQRIDTWPQVYPWV 191
gi|91777665|ref|YP_552873.1|      PGVRFAVDAYVNFARRAPWQESVCSSLTEMFAPQVHRDRLASWPEHYPWI 187
gi|118033162|ref|ZP_01504607.1    PGVRFAVDAYVNFARRAPWQEAVCSSLTEIFAPQIHKDRLATWPEHYPWI 172
gi|121603642|ref|YP_980971.1|     PAVRFACDAYVNFAAKAPWQEAVCSSLTEMFAPQIHKDRLASWPLHYAWI 181
gi|121529154|ref|ZP_01661766.1    PGVRFAVDAYVNFARSVPWQEAVCSSLTEMFAPKIHRDRLANWPQHYPWI 173
gi|84352661|ref|ZP_00977610.1|    PGVRFAVDAYVNFARRAPWQEAVCSSLTEMFAPQIHLDRLAGWPSHYPWI 176
gi|107025604|ref|YP_623115.1|     PGVRFAVDAYVNFARRAPWQEAVCSSLTEMFAPQIHLDRLAGWPSHYPWI 176
gi|118710536|ref|ZP_01563114.1    PGVRFAVDAYVNFARRAPWQEAVCSSLTEMFAPQIHLDRLAGWPSHYPWI 176
gi|134291937|ref|YP_001115706.    PGVRFAVDAYVNFARRAPWQEAVCSSLTEMFAPQIHLDRLAGWPSHYPWI 176
gi|118698279|ref|ZP_01556356.1    PGVRFAVDAYVNFARHAPWQEAVCSSLTEMFAPQIHLDRLAGWPSHYPWI 176
gi|84358446|ref|ZP_00983225.1|    PGVRFAVDAYVNFARRAPWQEAVCSSLTEMFAPQIHLDRLAGWPSHYPWI 179
gi|124900961|gb|EAY71711.1|       PGVRFAVDAYVNFARRAPWQEAVCSSLTEMFAPQIHLDRLAGWPSHYPWI 176
gi|118718079|ref|ZP_01570613.1    PGVRFAVDAYVNFARRATWQEAVCSSLTEMFAPQIHLDRLAGWPSHYSWI 176
gi|124267771|ref|YP_001021775.    PGVRFAVDAYVNFARRAPWREGVISSLTEMFAPKIHADRLAGWPSMYPWI 172
gi|73541469|ref|YP_295989.1|      PGVRFAVDAYVNFARRAPWQEAVCSSLTEMFAPEIHKERLAGWPTHYPWI 168
gi|116694996|ref|YP_840572.1|     PGVRFAVDAYVNFARRAPWQEAVCSSLTEMFAPAIHKERLAGWPTHYPWI 174
gi|121528464|ref|ZP_01661078.1    PGVRFAVDAYVNFARRAPWQEAVCSSLTEMFAPDIHKERLAGWPEHYPWV 168
gi|121610952|ref|YP_998759.1|     PGVRFAVDAYVNFARRAPWQEAVCSSLTEMFAPAIHQERLAGWPQHYPWI 181
gi|149927645|ref|ZP_01915898.1    PGVRFAVDAYVNFARRAPWQEAVCSSLTEMFAPEIHKQRLSNWPKDYKWV 168
gi|59891641|gb|AAX10038.1|        PGVRFAVDAYVNFARRARWQEAASSSLTELFAPQIHQSRLDSWPQHYPWI 167
gi|77461380|ref|YP_350887.1|      PGVRFAVDAYVNFARRASWQEAASSSLTELFAPQIHQSRLDSWPQHYPWI 167
gi|70732960|ref|YP_262733.1|      PGVRFAVDAYVNFARRASWQEAASSSLTELFAPQIHQSRLDSWPQHYPWI 167
gi|66047896|ref|YP_237737.1|      PGVRFAVDAYVNFARRANWQEAASSSLTELFAPQIHQSRLDSWPQHYPWI 168
gi|71734409|ref|YP_276808.1|      PGVRFAVDAYVNFARRANWQEAASSSLTELFAPQIHQSRLDSWPQHYPWI 168
gi|28867740|ref|NP_790359.1|      PGVRFAVDAYVNFARRANWQEAASSSLTELFAPQIHQSRLDSWPQHYPWI 168
gi|26987120|ref|NP_742545.1|      PGVRFAVDAYVNFARRASWQEAASSSLTELFAPQIHQSRLDSWPQHYPWI 168
gi|148545658|ref|YP_001265760.    PGVRFAVDAYVNFARRASWQEAASSSLTELFAPQIHQSRLDSWPQHYPWI 168
gi|126360078|ref|ZP_01717067.1    PGVRFAVDAYVNFARRASWQEAASSSLTELFAPQIHQSRLDSWPQHYPWI 168
gi|119859344|ref|ZP_01640760.1    PGVRFAVDAYVNFARRASWQEAASSSLTELFAPQIHQSRLDSWPQHYPWI 168
gi|104779674|ref|YP_606172.1|     PGVRFAVDAYVNFARRASWQEAASSSLTELFAPQIHQSRLDSWPQHYPWI 168
gi|146306995|ref|YP_001187460.    PGVRFAVDAYVNFARRASWQEAASSSLTELFAPTIHQSRLDAWPQHYPWI 167
gi|146282431|ref|YP_001172584.    PGVRFAVDAYVNFARRACWQEAASSSLTELFAPTIHQSRLDSWPQHYPWI 136
gi|150958981|gb|ABR81006.1|       PGVRFAVDAYVNFARRASWQEAASSSLTELFAPQIHQSRLDSWPRHYPWI 187
gi|94416471|ref|ZP_01296297.1|    PGVRFAVDAYVNFARRASWQEAASSSLTELFAPQIHQSRLDSWPRHYPWI 167
gi|15597183|ref|NP_250677.1|      PGVRFAVDAYVNFARRASWQEAASSSLTELFAPQIHQSRLDSWPRHYPWI 167
gi|67154257|ref|ZP_00416002.1|    PGVRFAVDAYVNFARRASWQEAAGSSLTELFAPTIHQSRLDTWPRHYPWI 167
gi|30841333|gb|AAP34380.1|        PGVRFAVDAYVNFARRANWQEAACSSLTELFAPQIHQSRLDSWPQHYPWI 168
gi|150955212|gb|ABR77242.1|       PGVRFAVDAYLNFARRACWQEAACSSLTELFAPQIHQSRLDSWPQHYPWI 168
gi|113196161|gb|ABI31435.1|       PGVRFAVDAYVNFARRACWQEAACSSLTELFAPQIHQSRLDSWPQHYPWI 166
gi|50085585|ref|YP_047095.1|      PSVRFAVDAYVNFARRACWQEAACSSLTELFAPAIHQSRLDTWPTHYPWI 171
gi|149375810|ref|ZP_01893578.1    PGVRFAVDAYLNFARRATWQEAACSSLTELFAPEIHQSRLDSWPQHYPWI 162
gi|126666044|ref|ZP_01737024.1    PGVRFAVDAYLNFARRATWQEAACSSLTELFAPEIHQSRLDSWPEHYPWI 170
gi|89092880|ref|ZP_01165832.1|    PGVRFAVDAYINFARRATWQEAASSSLTELFAPTIHQNRLDTWPQHYPWI 172
gi|90417324|ref|ZP_01225250.1|    PGVRFAVDAYVNFARRANWQEAACSSLTELFAPTIHQKRLATWPEHYPWI 167
gi|119474895|ref|ZP_01615248.1    PGVRFAVDAYVNFARKASWQEAACSSLTELFAPIIHQKRLDSWPEHYPWI 167
gi|71282639|ref|YP_267608.1|      PGVRFAVDAYVNFARRANWQQAACSSLTEMFAPQIHQSRLDSWPINYPWI 168
gi|118073743|ref|ZP_01541920.1    PGVRFAVDAYVNFARRASWQEAACSSLTEMFAPEIHQSRLNTWPENYPWI 167
gi|114770609|ref|ZP_01448099.1    PGVKFAVGAYINFTRKASWEEAACSSLTEMFAPEIHQSRLDTWPTHYKWI 170
gi|119504323|ref|ZP_01626403.1    PGVRFAVDAYVNFARNATWQEAACSSLTEMFAPEIHRSRLNTWPQHYSWI 179
gi|77166079|ref|YP_344604.1|      PGVRFAVDAYVNFARHATWQEAVCSSLTELFAPSLHQQRLDNWPRYYPWI 168
gi|53804508|ref|YP_113905.1|      PGVRFAVDAYLNFARTATWQEAACSSLTELFAPTIHKQRLAGWPDLYPWI 169
gi|94493803|ref|ZP_01301006.1|    PGVRFAVDAYIQFTRTHPWQAAVCSSLTELFAPEIHRQRLTNWPIHYPWM 171
gi|88812029|ref|ZP_01127282.1|    PGVRFAVDAYVNLVRWGSWQDGVCASLTELFAPGIHRERLNSWPTHYPWV 138
gi|126729612|ref|ZP_01745425.1    PATKFAVDAYVRFVREKTLLEAVAASLTELFAPKIHANRIEGLLKNYAFA 168
gi|16263947|ref|NP_436739.1|      PSTRFAVDAYVSFVREKPLLEAVASSLTELFAPKIHSERIAGLLEHYAFA 167
                                  *..:** .**: :        .. :****:*** :  .*:      * :
```

FIGURE 10 (continued)

```
gi|118470311|ref|YP_888023.1|       DPKELSYFRSRITQAPRDSEHGLEVVRRHCV-TQETQTAALSALSFKCDV 223
gi|134100979|ref|YP_001106640.      DPGGLGYFRARLEQAPRDSEHALQVVTEHCR-SADEQARAVAALSFKCDV 215
gi|71907330|ref|YP_284917.1|        ESTGLQYFRNRVTQARRDVEQGLAVTLDYFD-TPALQARALDILQFKLDI 215
gi|148259342|ref|YP_001233469.      ESEGLDYFRNRTSQAPRDVVHGLRITLAHFA-TRPMQERALQILKFKLDI 239
gi|118594755|ref|ZP_01552102.1      DESGLTYFKKRLTEARRDVEHGLGVTLDYFSKTRETQEQALEILQFKLNV 216
gi|91776035|ref|YP_545791.1|        KEEGLTYFKKRLTEARRDVEQGLAVTLDYFSQSRELQEKALDILQFKLDV 222
gi|119897479|ref|YP_932692.1|       KTEGLQYFRNRLTQARRDVAHGLRFTLEHFSQTRALQERALEILQFKLDV 241
gi|91777665|ref|YP_552873.1|        ESSGLAYFRSRISLAQRDVEHGLAVTLDHFT-RREQQERALEILQFKLDI 236
gi|118033162|ref|ZP_01504607.1      KPEGLAYFRSRISLAQRDVEHGLSVTLDHFR-TRDQQQRALDILQFKLDI 221
gi|121603642|ref|YP_980971.1|       EPEGLTYFRSRIPLASRDVEHGLAVTITHFT-TRQAQHRALDILQFKLDI 230
gi|121529154|ref|ZP_01661766.1      EAGGLHYFRSRITLAQRDVEHGLKVTLAHFT-TRAQQERALDILQFKLDV 222
gi|84352661|ref|ZP_00977610.1|      EPAGLHYFRSRVPLAQRDVEHGLAVTLAHFT-TPEAQQRALGILQFKLDI 225
gi|107025604|ref|YP_623115.1|       EPAGLHYFRSRVPLAQRDVEHGLAVTLAHFT-TPDAQQRALGILQFKLDI 225
gi|118710536|ref|ZP_01563114.1      EPAGLHYFRSRVPLAQRDVEHGLAVTLAHFT-TPDAQQRALGILQFKLDI 225
gi|134291937|ref|YP_001115706.      EADGLQYFRSRVPLAQRDVEHGLAVTLSHFT-TPDAQRRALDILSFKLDV 225
gi|118698279|ref|ZP_01556356.1      EADGLQYFRSRVPLAQRDVEHGLAVTLNHFT-TPDAQRRALDILSFKLDV 225
gi|84358446|ref|ZP_00983225.1|      DADGLQYFRSRVPLAQRDVEHGLAVTLRHFT-TPEAQRRALDILSFKLDV 228
gi|124900961|gb|EAY71711.1|         DADGLQYFRSRVPLAQRDVEHGLAVTLRHFT-TPEAQRRALDILSFKLDV 225
gi|118718079|ref|ZP_01570613.1      DADGLQYFRSRLPLAQRDVEHGLAVTLAHFT-TADAQRRALEILTFKLDV 225
gi|124267771|ref|YP_001021775.      AAEGLAYFRSRIPLAQRDVEHGLEVAIAFGD-TRAKQERAIEILQFKLDV 221
gi|73541469|ref|YP_295989.1|        EAAGLDYFRSRIPLAQRDVEHGLRVTLGYFR-TPEQQQRALDILQFKLDV 217
gi|116694996|ref|YP_840572.1|       EAAGLDYFRSRIPLAQRDVDHGLRVTLGHFR-SAAQQQRALDILQFKLDV 223
gi|121528464|ref|ZP_01661078.1      QPEGLAYFRSRIPLASRDVEHGLRVTLDYFR-TPEQQLRALDILQFKLDI 217
gi|121610952|ref|YP_998759.1|       EAEGLAYFRSRIPLAQRDVEHGLRVTLEHFR-TPAEQQRATQILQFKLDI 230
gi|149927645|ref|ZP_01915898.1      APEGLNYFRSRISLAQRDVEHGLEVTLNYFK-TRAQQERALEILQFKLDV 217
gi|59891641|gb|AAX10038.1|          DPAGYEYFRTRLGQARRDVEHGLAITLQHYT-TREGQERMLEILQFKLDI 216
gi|77461380|ref|YP_350887.1|        DPAGYEYFRTRLGQARRDVEHGLAITLEHYK-TREGQERMLEILQFKLDI 216
gi|70732960|ref|YP_262733.1|        DPTGYEYFRTRLGQARRDVEHGLAITLQHYT-TRAGQERMLEILQFKLDI 216
gi|66047896|ref|YP_237737.1|        DPAGYEYFRTRLGQARRDVEHGLAITLQHYT-TYEGQQRMLEILQFKLDI 217
gi|71734409|ref|YP_276808.1|        DPAGYEYFRTRLGQARRDVEHGLAITLQHYT-TYEGQQRMLEILQFKLDI 217
gi|28867740|ref|NP_790359.1|        DPTGYEYFRTRLGQARRDVEHGLAITLQHYT-TYEGQQRMLEILQFKLDI 217
gi|26987120|ref|NP_742545.1|        DPAGYEYFRTRLGQARRDVEHGLAITLQHYT-TRAGQERMLEILQFKLDI 217
gi|148545658|ref|YP_001265760.      DPAGYEYFRTRLGQARRDVEHGLAITLQHYT-TRAGQERMLEILQFKLDI 217
gi|126360078|ref|ZP_01717067.1      DPAGYEYFRTRLGQARRDVEHGLAITLQHYT-TRAGQERMLEILQFKLDI 217
gi|119859344|ref|ZP_01640760.1      DPAGYEYFRTRLGQARRDVEHGLTITLQHYT-TRAAQERMLEILQFKLDI 217
gi|104779674|ref|YP_606172.1|       DPAGYEYFRTRLGQARRDVEHGLAITLAHYT-TREGQARMLEILQFKLDI 217
gi|146306995|ref|YP_001187460.      DAAGYDYFRNRLSQARRDVEHGLRITLEHYR-TRDAQEHMLNILQFKLDV 216
gi|146282431|ref|YP_001172584.      DASGYDYFRKRLKEARRDVEHGLRITLDHYR-TREAQERMLEILQFKLDV 185
gi|150958981|gb|ABR81006.1|         EAAGYEYFRSRLAQARRDVEHGLRITLEHYR-TREAQERMLEILQFKLDV 236
gi|94416471|ref|ZP_01296297.1|      EAAGYEYFRSRLAQARRDVEHGLRITLEHYR-TREAQERMLEILQFKLDV 216
gi|15597183|ref|NP_250677.1|        EAAGYEYFRSRLAQARRDVEHGLRITLEHYR-TREAQERMLDILQFKLDV 216
gi|67154257|ref|ZP_00416002.1|      EAGGYDYFRRRLKEARRDVEHGLRITLAHFA-TREAQARMLEILQFKLDI 216
gi|30841333|gb|AAP34380.1|          KEEGYFYFRSRLSQANRDVEHGLALALEVFT-RAEQQNRMLEILQFKLDI 217
gi|150955212|gb|ABR77242.1|         KEEGYFYFRSRLSQANRDVEHGLALAKAYCD-SAEKQNRMLEILQFKLDI 217
gi|113196161|gb|ABI31435.1|         AAVGYDYFRSRLRQANRDVEHGLALALDYCD-TVEKQQRMLEILQFKLDI 215
gi|50085585|ref|YP_047095.1|        DAEGYAYFRGRLSQANRDVEHGLELALEYCN-TVDRQQRMLNILQFKLDI 220
gi|149375810|ref|ZP_01893578.1      QTDGYAYFRKRLSEARRDVEHGLTITLDHFT-TAAQQARALEILQFKLDI 211
gi|126666044|ref|ZP_01737024.1      DEAGYSYFRKRLSEARRDVEHGLAITLEYFT-RATEQARALEILQFKLDI 219
gi|89092880|ref|ZP_01165832.1|      KEEGYQYFRNRLTEARRDVVHGLEITLDHYT-TREQQDRMLEILQFKLDV 221
gi|90417324|ref|ZP_01225250.1|      DPNGYQYFRTRLSEARRDVEHGLAITLDYFK-TFEQQQQAMGILQLKLDI 216
gi|119474895|ref|ZP_01615248.1      EAEGYRYFRTRLSEARRDVEHGLQITLDHFQ-TREAQEHALGILQMKLDI 216
gi|71282639|ref|YP_267608.1|        ETQGLQYFQMRLSQARRDVNHGLQITLDHFT-TRQAQDEALNILQFKLDI 217
gi|118073743|ref|ZP_01541920.1      KSEGLSYFQMRLSQARRDVNHGLAITLAHFT-TREQQEHALNILQFKLDI 216
gi|114770609|ref|ZP_01448099.1      DPEGFSYFQMRLNQARRDVQHGLEITLDHFE-TRKQQERALNILQFKLDV 219
gi|119504323|ref|ZP_01626403.1      DQSGLKYFQKRLGEARRDVEHGLDVTLDYFK-YREQQEKALGILQFKLDI 228
gi|77166079|ref|YP_344604.1|        KAEGYHYFRKRLNEARRDVRHGLEATLDYFQ-SRTQQEQALAILQFKLDV 217
gi|53804508|ref|YP_113905.1|        AADGLAYFRKRVTQASRDVEHGIEITLDHFK-TREQQERALEILQFKLDI 218
gi|94493803|ref|ZP_01301006.1|      DLNALAYFRCRLNQINHHVKKGLQWTLENFQ-THEQQTQALAILKFKLEI 220
gi|88812029|ref|ZP_01127282.1|      DPAGLAYFRSRLEVARQDGRHGLALVLDHCD-TPASQDRAVRIVKFKLEV 187
gi|126729612|ref|ZP_01745425.1      DDSSLSYFRNRLKEAPKDVAFGLAWVLDHAD-TAEKQDVAARALIFKTDV 217
gi|16263947|ref|NP_436739.1|        DDAALAYFRQRLAEVPRDVEFGLAYVLDHAD-TREKQDAAAQALTFKTDV 216
                                             **: *     :.   .:  .          *     : :* ::
```

FIGURE 10 (continued)

```
gi|118470311|ref|YP_888023.1|          LWSMLDAIDQAYGNR------------------------- 238
gi|134100979|ref|YP_001106640.         LWSILDAIDHAYAD-------------------------- 229
gi|71907330|ref|YP_284917.1|           LWSMNDAMALRYGVNK------------------------ 231
gi|148259342|ref|YP_001233469.         LWTMNDEMGRHYGVAA------------------------ 255
gi|118594755|ref|ZP_01552102.1         LWVIADSIMLASSNIK--VEDR-DYLRQPIN--------- 244
gi|91776035|ref|YP_545791.1|           LWVIADAIMLASTNIK--VEQHVDYLRQPR---------- 250
gi|119897479|ref|YP_932692.1|          LWALADAIMLSQCEIE--IKGPKA---------------- 263
gi|91777665|ref|YP_552873.1|           LWTMLDSIEKAFPQ-------------------------- 250
gi|118033162|ref|ZP_01504607.1         LWTMLDAIEKAFPA-------------------------- 235
gi|121603642|ref|YP_980971.1|          LWSMLDGIEKACV--------------------------- 243
gi|121529154|ref|ZP_01661766.1         LWSMLDAIERAYPTR------------------------- 237
gi|84352661|ref|ZP_00977610.1|         LWSMLDAVEKAYPL-------------------------- 239
gi|107025604|ref|YP_623115.1|          LWSMLDAVEKAYPL-------------------------- 239
gi|118710536|ref|ZP_01563114.1         LWSMLDAVEKAYPL-------------------------- 239
gi|134291937|ref|YP_001115706.         LWQILDAIEKAYPA-------------------------- 239
gi|118698279|ref|ZP_01556356.1         LWSILDAIEKAYPA-------------------------- 239
gi|84358446|ref|ZP_00983225.1|         LWSILDAIEKAYPA-------------------------- 242
gi|124900961|gb|EAY71711.1|            LWSILDAIEKAYPA-------------------------- 239
gi|118718079|ref|ZP_01570613.1         LWSILDAIEKAYCA-------------------------- 239
gi|124267771|ref|YP_001021775.         LWSLLDAVERAYPDDL--PEERRP---------------- 243
gi|73541469|ref|YP_295989.1|           LWSMLDAIQLANP--------------------------- 230
gi|116694996|ref|YP_840572.1|          LWSMLDAIQQANP--------------------------- 236
gi|121528464|ref|ZP_01661078.1         LWSMLDAIQQAHAHEP--SLV------------------- 236
gi|121610952|ref|YP_998759.1|          LWSMLDAIEKAYPE-------------------------- 244
gi|149927645|ref|ZP_01915898.1         LWSMSDAIALAYPDKR--KK-------------------- 235
gi|59891641|gb|AAX10038.1|             LWSMLDAMSMAYELNR--PPYHSVTEQRVWHKGITL---- 250
gi|77461380|ref|YP_350887.1|           LWSMLDAMSMAYELNR--PPYHSVTDQRVWHKGIAL---- 250
gi|70732960|ref|YP_262733.1|           LWSMLDAMSMAYELNR--PPYHSVTDQRVWHKGITL---- 250
gi|66047896|ref|YP_237737.1|           LWSMLDAMSMAYELNR--PPYHSVTDQRVWHKGITL---- 251
gi|71734409|ref|YP_276808.1|           LWSMLDAMSMAYELNR--PPYHSVTDQKVWHKGITL---- 251
gi|28867740|ref|NP_790359.1|           LWSMLDAMSMAYELNR--PPYHSVTDQKVWHKGITP---- 251
gi|26987120|ref|NP_742545.1|           LWSMLDAMSMAYELNR--PPYHSVTQERVWHKGITL---- 251
gi|148545658|ref|YP_001265760.         LWSMLDAMSMAYELNR--PPYHSVTQERMWHKGITL---- 251
gi|126360078|ref|ZP_01717067.1         LWSMLDAMSMAYELNR--PPYHSVTQDRVWHKGITL---- 251
gi|119859344|ref|ZP_01640760.1         LWSMLDAMSMAYELHR--PPYHTVTQQRVWHKGIAL---- 251
gi|104779674|ref|YP_606172.1|          LWSMLDAMSMAYELNR--PPYHSVTSERVWHKGIAL---- 251
gi|146306995|ref|YP_001187460.         LWSMLDAMSMAYELER--PPYHTVTAERVWHKGIDL---- 250
gi|146282431|ref|YP_001172584.         LWSMLDAMSMAYELDR--PPYHTVTRDRVWHKGISF---- 219
gi|150958981|gb|ABR81006.1|            LWSMLDAMSMAYELER--PPYHTVTRERVWHRGLAP---- 270
gi|94416471|ref|ZP_01296297.1|         LWSMLDAMSMAYELER--PPYHTVTRERVWHRGLAP---- 250
gi|15597183|ref|NP_250677.1|           LWSMLDAMSMAYELER--PPYHTVTRERVWHRGLAS---- 250
gi|67154257|ref|ZP_00416002.1|         LWSMLDAMSMAYELDR--PPYHTVTHERVWHRGTIL---- 250
gi|30841333|gb|AAP34380.1|             LWTMLDAMTMAYALQR--PPYHTVTDQVSWHSTRLV---- 251
gi|150955212|gb|ABR77242.1|            LWSMLDAMTMAYALQR--PPYHTVTDKAAWHTTRLV---- 251
gi|113196161|gb|ABI31435.1|            LWSMLDAMSMAYTLNR--PPYHSVTAARVWHNQRLV---- 249
gi|50085585|ref|YP_047095.1|           LWTILDGMSMAYVLER--APYHTVTQEAVWHQKGLLG--- 255
gi|149375810|ref|ZP_01893578.1         LWSMLDALTMAYQHRT--PPYHTVTGQPVWHRGL------ 243
gi|126666044|ref|ZP_01737024.1         LWSLLDALTMAYSHKT--PPFHTVTDQQVWHRGL------ 251
gi|89092880|ref|ZP_01165832.1|         LWTMLDAMTMAYELER--PPYHTVTGEKVFHKGLFNE--- 256
gi|90417324|ref|ZP_01225250.1|         LWSMLDAMQLAYVYEE--PPFHSCSR-------------- 240
gi|119474895|ref|ZP_01615248.1         LWSMLDAMQLAYVYNE--APFHSCNID------------- 241
gi|71282639|ref|YP_267608.1|           LWSMLDAMTLAYQQDR--APYQGLIAEPNYHKRIFK---- 251
gi|118073743|ref|ZP_01541920.1         LWSMLDAMTLAYQYER--APYSQLIDAPVYHKGIF----- 249
gi|114770609|ref|ZP_01448099.1         LWSMADSISLAYEFNR--KPFGAVTDENISHKGFM----- 252
gi|119504323|ref|ZP_01626403.1         LWTMLDAMTMAYVHTT--PPYHSCDDGIHSVPG------- 259
gi|77166079|ref|YP_344604.1|           LWTLLDALWMAYIENR--PPYHTEL--------------- 240
gi|53804508|ref|YP_113905.1|           LWSMLDAMYLAYVDKK--PPYFNIA--------------- 241
gi|94493803|ref|ZP_01301006.1|         LWSISDSLYLAYVLGH--PPITFSKDDT------------ 246
gi|88812029|ref|ZP_01127282.1|         LWAMLDAMYLAYVVGM--PPFFNVAGD------------- 212
gi|126729612|ref|ZP_01745425.1         LWSQLDALWGAYVEPRRIPPGAWQPGTGQL----LRQAS- 252
gi|16263947|ref|NP_436739.1|           LWAQLDALYSAYVTPGRIPPGAWDGREGVIREPRAREAAE 256
                                       **    * :
```

FIGURE 10 (continued)

**SEQ ID NO: 104, PqqC DNA *Pseudomonas fluorescens***

TTTGTACAAAAAAGCAGGCTTAAACAATGACTGACACCCCAATGTCCCCCGCCGAATTCGAAGCGGC
CCTGCGCGCCAAGGGCGCCTATTACCACATCTATCACCCGTATCACGTGGCGATGTATGAAGGCCGG
GCGACTCGCGAGCAGATCCAGGGCTGGGTCGCCAACCGCTTTTACTATCAGGTGAACATCCCGCTCA
AGGACGCCGCGATCCTGGCCAACTGCCCGGACCGCGAAATCCGTCGCGAGTGGATTCAACGCCTGTT
GGACCATGACGGCGCCCCCGGTGAAGACGGCGGTATCGAAGCCTGGTTGCGTCTTGGGCAAGCGGTC
GGCCTCGACCCGGATCAGTTGCGCTCCCAGGAACTGGTGCTGCCCGGCGTGCGTTTCGCCGTGGATG
CCTACGTCAACTTCGCCCGCCGGGCCCGTTGGCAGGAAGCCGCCAGCAGCTCATTGACCGAGCTGTT
TGCGCCGCAGATCCACCAATCGCGCCTCGACAGTTGGCCGCAGCATTACCCGTGGATCGACCCGGCC
GGTTACGAATATTTCCGTACGCGCCTGGGCCAGGCTCGCCGGGATGTGGAGCACGGTCTGGCAATCA
CGTTGCAGCACTACACCACGCGGGAAGGCCAGGAGCGCATGCTGGAAATTCTCCAGTTCAAACTGGA
CATTCTTTGGAGCATGCTCGACGCCATGAGCATGGCCTACGAACTGAACCGCCCGCCGTACCACAGC
GTGACCGAACAGCGGGTCTGGCATAAAGGAATCACCTTATGAACCCAGCTTTCTTGTACAAAGT

**SEQ ID NO: 105, PqqC protein *Pseudomonas fluorescens***

MTDTPMSPAEFEAALRAKGAYYHIYHPYHVAMYEGRATREQIQGWVANRFYYQVNIPLKDAAILANC
PDREIRREWIQRLLDHDGAPGEDGGIEAWLRLGQAVGLDPDQLRSQELVLPGVRFAVDAYVNFARRA
RWQEAASSSLTELFAPQIHQSRLDSWPQHYPWIDPAGYEYFRTRLGQARRDVEHGLAITLQHYTTRE
GQERMLEILQFKLDILWSMLDAMSMAYELNRPPYHSVTEQRVWHKGITL

**SEQ ID NO: 106, gi|77456228:5800706-5801458 *Pseudomonas fluorescens* PfO-1, complete genome**

ATGACCGACACCCCGCTGTCCCCCGCCGAATTCGAAGCGGCCCTGCGCGCCAAGGGCGCCTATTACC
ACATTCATCACCCGTATCACGTGGCGATGTATGAAGGCCGGGCCACCCGTGAGCAGATACAGGGCTG
GGTCGCCAACCGCTTCTACTATCAGGTGAACATCCCCCTGAAAGACGCGGCGATCCTCGCCAACTGC
CCGGATCGCGAGATCCGTCGCGAGTGGATTCAGCGCCTGCTCGACCACGACGGCGCGCCCGGTGAAG
ATGGCGGCATCGAAGCCTGGCTGCGCCTCGGCCAGGCCGTCGGTCTCGATCCGGATCAATTGCGCTC
CCAGGAACTGGTGCTGCCCGGCGTGCGTTTCGCCGTCGACGCCTACGTCAACTTCGCCCGCCGCGCC
AGTTGGCAGGAAGCCGCCAGCAGCTCGCTGACCGAGCTGTTCGCGCCGCAGATCCACCAGTCGCGCC
TCGACAGCTGGCCGCAGCATTACCCGTGGATCGACCCGGCCGGCTACGAATATTTCCGCACCCGTCT
CGGCCAGGCCCGGCGCGATGTCGAGCATGGTCTGGCGATCACGCTGGAGCACTACAAGACCCGCGAA
GGCCAGGAACGCATGCTGGAAATTCTCCAGTTCAAACTGGACATCCTTTGGAGCATGCTCGATGCCA
TGAGCATGGCCTACGAACTGAACCGCCCGCCGTATCACAGCGTCACCGATCAACGGGTCTGGCATAA
AGGAATCGCCTTATGA

**SEQ ID NO: 107, gi|77461380|ref|YP_350887.1| pyrroloquinoline quinone biosynthesis protein PqqC [*Pseudomonas fluorescens* PfO-1]**

MTDTPLSPAEFEAALRAKGAYYHIHHPYHVAMYEGRATREQIQGWVANRFYYQVNIPLKDAAILANC
PDREIRREWIQRLLDHDGAPGEDGGIEAWLRLGQAVGLDPDQLRSQELVLPGVRFAVDAYVNFARRA
SWQEAASSSLTELFAPQIHQSRLDSWPQHYPWIDPAGYEYFRTRLGQARRDVEHGLAITLEHYKTRE
GQERMLEILQFKLDILWSMLDAMSMAYELNRPPYHSVTDQRVWHKGIAL

**SEQ ID NO: 108, gi|70728250:6468501-6469253 *Pseudomonas fluorescens* Pf-5, complete genome**

ATGACCGACACCCCGCTGTCCGCCGCCGAATTCGAGCAGGCACTGCGCGCCAAAGGCGCCTACTACC
ACATCCATCACCCTTATCACGTGGCGATGTATGAAGGCCGGGCGACCCGCGAGCAGATCCAGGGCTG
GGTCGCCAACCGGTTCTATTACCAGGTGAACATCCCCATGAAGGACGCGGCGATCCTCGCCAAC

## FIGURE 11

TGTCCGGACCGGGAGATTCGCCGCGAATGGATCCAGCGCCTGCTGGACCATGACGGCGCCCCGGGCG
AGGACGGCGGCATCGAGGCCTGGCTGCGCCTGGGCCAGGCCGTGGGCCTGGACCCGGACCAGTTGCG
CTCCCAGGAACTGGTGCTGCCCGGTGTGCGCTTTGCCGTGGACGCCTATGTCAATTTTGCTCGCCGG
GCCAGTTGGCAGGAGGCGGCCAGCAGCTCGCTGACCGAACTGTTCGCGCCGCAAATCCACCAGTCGC
GCCTGGACAGCTGGCCCCAGCATTACCCATGGATCGACCCCACTGGCTACGAGTACTTCCGCACCCG
CCTGGGCCAGGCCCGCCGTGATGTCGAGCACGGCCTGGCGATCACCCTGCAGCACTACACCACCCGT
GCCGGCCAGGAACGCATGCTGGAAATTCTCCAGTTCAAACTGGACATCCTTTGGAGCATGCTCGACG
CCATGAGCATGGCCTATGAACTGAACCGCCCGCCCTATCACAGCGTCACCGATCAGCGGGTCTGGCA
TAAGGGGATCACCCTATGA

**SEQ ID NO: 109, gi|70732960|ref|YP_262733.1| pyrroloquinoline quinone biosynthesis protein PqqC [Pseudomonas fluorescens Pf-5]**
MTDTPLSAAEFEQALRAKGAYYHIHHPYHVAMYEGRATREQIQGWVANRFYYQVNIPMKDAAILANC
PDREIRREWIQRLLDHDGAPGEDGGIEAWLRLGQAVGLDPDQLRSQELVLPGVRFAVDAYVNFARRA
SWQEAASSSLTELFAPQIHQSRLDSWPQHYPWIDPTGYEYFRTRLGQARRDVEHGLAITLQHYTTRA
GQERMLEILQFKLDILWSMLDAMSMAYELNRPPYHSVTDQRVWHKGITL

**SEQ ID NO: 110, gi|66043271:5538337-5539092 Pseudomonas syringae pv. syringae B728a, complete**
genomeATGAGCGAAGCGACTGCGCTGTCCCCCGCCGAATTCGAACAGGCCTTGCGCGCCAAGGGTG
CCTATTACCACATCTATCACCCGTTCCATGTGGCGATGTACGAGGGCCGAGCGACCCGCGAGCAGAT
TCAGGGCTGGGTCGCCAACCGTTTCTACTATCAGGTGAACATTCCGCTCAAGGACGCGGCGATTCTG
GCCAACTGCCCGGATCGCGAGATCCGTCGCGAGTGGATTCAGCGCCTGCTCGATCATGACGGTGCGC
CCGGCGAAGACGGCGGCATCGAAGCCTGGCTGCGCCTCGGCCAGGCCGTGGGACTGGACCCGGACCA
GCTGCGCTCGCAGGAACTGGTGCTGCCAGGCGTGCGCTTTGCCGTCGACGCCTACGTGAACTTCGCC
CGCCGCGCCAACTGGCAGGAAGCAGCCAGCAGCTCGCTGACCGAACTGTTCGCCCCGCAGATCCACC
AGTCGCGTCTGGACAGCTGGCCGCAGCACTACCCATGGATCGACCCGGCAGGCTATGAGTATTTCCG
CACTCGCCTGGGTCAGGCCCGACGCGATGTAGAGCACGGCCTCGCCATCACGTTACAGCACTACACT
ACGTACGAAGGTCAGCAGCGTATGCTGGAAATCCTGCAGTTCAAACTCGATATCCTGTGGAGCATGC
TCGATGCGATGTCCATGGCCTACGAGCTGAACCGCCCGCCGTATCACAGCGTCACAGACCAAAGGGT
CTGGCATAAGGGGATTACCCTATGA

**SEQ ID NO: 111, gi|66047896|ref|YP_237737.1| pyrroloquinoline quinone biosynthesis protein PqqC [Pseudomonas syringae pv. syringae**
B728a]MSEATALSPAEFEQALRAKGAYYHIYHPFHVAMYEGRATREQIQGWVANRFYYQVNIPLKD
AAILANCPDREIRREWIQRLLDHDGAPGEDGGIEAWLRLGQAVGLDPDQLRSQELVLPGVRFAVDAY
VNFARRANWQEAASSSLTELFAPQIHQSRLDSWPQHYPWIDPAGYEYFRTRLGQARRDVEHGLAITL
QHYTTYEGQQRMLEILQFKLDILWSMLDAMSMAYELNRPPYHSVTDQRVWHKGITL

**SEQ ID NO: 112, gi|71733195:5342488-5343243 Pseudomonas syringae pv. phaseolicola 1448A, complete**
genomeATGAGCGAAGCGACTGCGCTGTCCCCCGCCGAATTCGAACAGGCCTTGCGCGCCAAGGGTG
CCTATTACCACATCTATCACCCGTACCATGTGGCGATGTACGAGGGTCGTGCAACCCGCGAGCAGAT
TCAGGGCTGGGTCGCCAACCGTTTCTACTATCAGGTGAACATTCCGCTCAAGGACGCGGCGATTCTG
GCCAACTGCCCGGATCGCGAGATCCGTCGCGAGTGGATTCAGCGCCTGCTCGATCATGACGGTGCGC
CCGGCGAAGACGGCGGCATTGAAGCCTGGCTGCGCCTCGGCCAGGCCGTGGGGCTCGACCCGGACCA
ACTGCGCTCTCAGGAACTGGTGCTGCCAGGCGTGCGTTTTGCCGTCGACGCCTACGTGAAC

**FIGURE 11 (continued)**

TTCGCCCGCCGCGCCAACTGGCAGGAAGCGGCCAGCAGCTCGCTGACCGAACTGTTCGCGCCGCAGA
TCCACCAATCGCGCCTGGACAGCTGGCCGCAGCATTACCCGTGGATCGACCCGGCAGGCTACGAGTA
TTTCCGCACTCGCCTGGGCCAGGCCCGACGCGACGTGGAACACGGCCTCGCCATCACGTTACAGCAC
TACACTACATATGAAGGCCAGCAGCGCATGCTGGAAATCCTGCAGTTCAAGCTCGATATCCTGTGGA
GCATGCTCGATGCGATGTCCATGGCCTACGAGCTGAACCGCCCGCCGTATCACAGCGTTACAGACCA
AAAGGTCTGGCATAAGGGGATTACGCTATGA

**SEQ ID NO: 113, gi|71734409|ref|YP_276808.1| coenzyme PQQ biosynthesis protein C [Pseudomonas syringae pv. phaseolicola**
1448A]MSEATALSPAEFEQALRAKGAYYHIYHPYHVAMYEGRATREQIQGWVANRFYYQVNIPLKD
AAILANCPDREIRREWIQRLLDHDGAPGEDGGIEAWLRLGQAVGLDPDQLRSQELVLPGVRFAVDAY
VNFARRANWQEAASSSLTELFAPQIHQSRLDSWPQHYPWIDPAGYEYFRTRLGQARRDVEHGLAITL
QHYTTYEGQQRMLEILQFKLDILWSMLDAMSMAYELNRPPYHSVTDQKVWHKGITL

**SEQ ID NO: 114, gi|26986745:458023-458778 Pseudomonas putida KT2440, complete**
genomeTCATAGGGTGATTCCCTTGTGCCACACCCGTTCCTGGGTGACGGAGTGATAGGGCGGGCGG
TTCAGTTCGTAGGCCATGCTCATGGCATCGAGCATACTCCAGAGGATATCCAGCTTGAACTGCAGTA
TCTCCAGCATACGCTCCTGGCCCGCACGGGTGGTGTAGTGCTGCAGGGTAATCGCCAGGCCGTGTTC
CACATCGCGTCGCGCCTGGCCCAGGCGGGTACGGAAATACTCATAGCCGGCGGGGTCGATCCACGGG
TAGTGCTGCGGCCAGCTGTCCAGGCGTGACTGGTGGATTTGCGGGGCGAACAGCTCGGTCAGCGAAC
TGCTGGCGGCTTCCTGCCAGCTGGCGCGGCGGGCAAAGTTGACGTAGGCGTCCACGGCAAAGCGCAC
GCCAGGCAATACCAGTTCTTGCGAGCGCAATTGGTCCGGGTCCAGCCCGACAGCCTGGCCCAGGCGC
AGCCAGGCTTCGATGCCGCCATCCTCGCCGGGCGCACCGTCGTGGTCGAGCAGGCGCTGGATCCACT
CGCGGCGCACTTCGCGGTCCGGGCAGTTGGCCAGGATCGCGGCATCCTTCATCGGGATGTTGACCTG
GTAGTAGAAGCGGTTGGCCACCCAGCCCTGGATCTGCTCGCGGGTGGCGCGCCCCTGGTACATCGCC
ACGTGGTACGGGTGATGGATGTGGTAATAGGCGCCCTTGGCGCGCAGGGCCTGCTCGAACTCGGCAG
GGGACATTGGCAGTGCGTCGCTCAT

**SEQ ID NO: 115, gi|26987120|ref|NP_742545.1| pyrroloquinoline quinone biosynthesis protein PqqC [Pseudomonas putida KT2440]**
MSDALPMSPAEFEQALRAKGAYYHIHHPYHVAMYQGRATREQIQGWVANRFYYQVNIPMKDAAILAN
CPDREVRREWIQRLLDHDGAPGEDGGIEAWLRLGQAVGLDPDQLRSQELVLPGVRFAVDAYVNFARR
ASWQEAASSSLTELFAPQIHQSRLDSWPQHYPWIDPAGYEYFRTRLGQARRDVEHGLAITLQHYTTR
AGQERMLEILQFKLDILWSMLDAMSMAYELNRPPYHSVTQERVWHKGITL

**SEQ ID NO: 116, gi|126360024:66513-67268 Pseudomonas putida GB-1 ctg100, whole genome shotgun sequence**
ATGAGCGACGCACTGCCCATGTCCCCTGCCGAATTCGAGCAGGCTCTGCGCGCCAAAGGCGCCTATT
ACCACATCCATCACCCGTACCATGTGGCGATGTACCAAGGCCGCGCCACCCGCGAGCAGATCCAGGG
CTGGGTGGCCAACCGTTTCTACTACCAGGTCAACATCCCGATGAAGGATGCCGCGATCCTGGCCAAC
TGCCCGGACCGCGAAGTGCGCCGAGAGTGGATCCAGCGCCTGCTCGACCACGACGGCGCACCCGGCG
AGGACGGTGGCATCGAAGCCTGGCTGCGCCTGGGCCAGGCCGTCGGCCTCGACCCCGACCAGCTGCG
CTCCCAGGAGCTGGTACTGCCCGGCGTACGCTTTGCCGTGGACGCCTACGTCAACTTCGCCCGCCGC
GCCAGCTGGCAGGAAGCCGCCAGCAGCTCGCTGACCGAACTGTTTGCCCCACAAATCCACCAGTCGC
GGCTGGACAGTTGGCCACAGCACTACCCGTGGATCGACCCGGCCGGCTACGAGTACTTCCGCACCCG
CCTTGGCCAGGCCCGGCGCGACGTGGAGCACGGCCTGGCGATTACCCTGCAGCACTACACCACCCGC
GCGGGCCAGGAGCGTATGCTGGAGATCCTGCAGTTCAAGCTGGATATCCTCTGGAGCATGCTCGACG
CCATGAGCATGGCCTACGAGCTGAACCGCCCGCCTTATCACTCCGTCACCCAGGACCGGGTGTGGCA
CAAGGGGATCACCCTATGA

**FIGURE 11 (continued)**

**SEQ ID NO: 117, gi|126360078|ref|ZP_01717067.1| coenzyme PQQ biosynthesis protein C [Pseudomonas putida GB-1]**
MSDALPMSPAEFEQALRAKGAYYHIHHPYHVAMYQGRATREQIQGWVANRFYYQVNIPMKDAAILAN
CPDREVRREWIQRLLDHDGAPGEDGGIEAWLRLGQAVGLDPDQLRSQELVLPGVRFAVDAYVNFARR
ASWQEAASSSLTELFAPQIHQSRLDSWPQHYPWIDPAGYEYFRTRLGQARRDVEHGLAITLQHYTTR
AGQERMLEILQFKLDILWSMLDAMSMAYELNRPPYHSVTQDRVWHKGITL

**SEQ ID NO: 118, gi|126360024:66513-67268 Pseudomonas putida GB-1 ctg100, whole genome shotgun sequence**
ATGAGCGACGCACTGCCCATGTCCCCTGCCGAATTCGAGCAGGCTCTGCGCGCCAAAGGCGCCTATT
ACCACATCCATCACCCGTACCATGTGGCGATGTACCAAGGCCGCGCCACCCGCGAGCAGATCCAGGG
CTGGGTGGCCAACCGTTTCTACTACCAGGTCAACATCCCGATGAAGGATGCCGCGATCCTGGCCAAC
TGCCCGGACCGCGAAGTGCGCCGAGAGTGGATCCAGCGCCTGCTCGACCACGACGGCGCACCCGGCG
AGGACGGTGGCATCGAAGCCTGGCTGCGCCTGGGCCAGGCCGTCGGCCTCGACCCCGACCAGCTGCG
CTCCCAGGAGCTGGTACTGCCCGGCGTACGCTTTGCCGTGGACGCCTACGTCAACTTCGCCCGCCGC
GCCAGCTGGCAGGAAGCCGCCAGCAGCTCGCTGACCGAACTGTTTGCCCCACAAATCCACCAGTCGC
GGCTGGACAGTTGGCCACAGCACTACCCGTGGATCGACCCGGCCGGCTACGAGTACTTCCGCACCCG
CCTTGGCCAGGCCCGGCGCGACGTGGAGCACGGCCTGGCGATTACCCTGCAGCACTACACCACCCGC
GCGGGCCAGGAGCGTATGCTGGAGATCCTGCAGTTCAAGCTGGATATCCTCTGGAGCATGCTCGACG
CCATGAGCATGGCCTACGAGCTGAACCGCCCGCCTTATCACTCCGTCACCCAGGACCGGGTGTGGCA
CAAGGGGATCACCCTATGA

**SEQ ID NO: 119, gi|148545658|ref|YP_001265760.1| coenzyme PQQ biosynthesis protein C [Pseudomonas putida F1]**
MSDALPMSPAEFEQALRAKGAYYHIHHPYHVAMYQGRATREQIQGWVANRFYYQVNIPMKDAAILAN
CPDREVRREWIQRLLDHDGAPGEDGGIEAWLRLGQAVGLDPDQLRSQELVLPGVRFAVDAYVNFARR
ASWQEAASSSLTELFAPQIHQSRLDSWPQHYPWIDPAGYEYFRTRLGQARRDVEHGLAITLQHYTTR
AGQERMLEILQFKLDILWSMLDAMSMAYELNRPPYHSVTQERMWHKGITL

**SEQ ID NO: 120, gi|28867243:559123-559878 Pseudomonas syringae pv. tomato str. DC3000, complete genome**
TCATGGCGTAATCCCCTTATGCCAGACCTTCTGGTCTGTGACGCTGTGGTACGGGGGGCGGTTCAAT
TCATACGCCATGGACATGGCATCGAGCATGCTCCACAGAATGTCGAGTTTGAACTGCAGGATTTCCA
GCATGCGCTGCTGGCCTTCATACGTAGTGTAGTGCTGTAACGTGATTGCGAGGCCGTGTTCCACATC
GCGACGTGCCTGACCCAGGCGAGTACGGAAATACTCGTAACCTGTGGGGTCAATCCACGGGTAATGC
TGTGGCCAGCTGTCCAGGCGCGATTGATGAATCTGCGGTGCGAACAACTCGGTCAGCGAGCTGCTGG
CGGCCTCTTGCCAGTTGGCGCGGCGGGCGAAGTTCACGTAGGCATCGACCGCAAAGCGCACACCGGG
CAGTACCAGTTCCTGCGAGCGCAACTGATCCGGGTCCAGCCCCACGGCCTCGCCGAGGCGCAGCCAG
GCTTCGATGCCGCCGTCTTCACCGGGCGCGCCGTCGTGGTCCAGCAGGCGCTGAATCCACTCGCGAC
GAATCTCGCGATCCGGGCAATTGGCCAGAATGGCGGCGTCCTTGAGCGGGATGTTCACCTGATAGTA
AAAGCGGTTGGCGACCCAGCCCTGAATCTGCTCGCGGGTCGCGCGGCCTTCATACATCGCCACATGA
AACGGATGATAGATGTGGTAATAGGCGCCCTTGGCACGTAAGGCCTGCTCGAATTCAGCCGGGGACA
GCGCAGTCGCTTCACTCAT

FIGURE 11(continued)

**SEQ ID NO: 121, gi|28867740|ref|NP_790359.1| pyrroloquinoline quinone biosynthesis protein PqqC [Pseudomonas syringae pv. tomato str. DC3000]**
MSEATALSPAEFEQALRAKGAYYHIYHPFHVAMYEGRATREQIQGWVANRFYYQVNIPLKDAAILAN
CPDREIRREWIQRLLDHDGAPGEDGGIEAWLRLGEAVGLDPDQLRSQELVLPGVRFAVDAYVNFARR
ANWQEAASSSLTELFAPQIHQSRLDSWPQHYPWIDPTGYEYFRTRLGQARRDVEHGLAITLQHYTTY
EGQQRMLEILQFKLDILWSMLDAMSMAYELNRPPYHSVTDQKVWHKGITP

**SEQ ID NO: 122, gi|104779316:419491-420246 Pseudomonas entomophila L48, complete genome**
TCATAAGGCGATTCCCTTGTGCCAGACCCGCTCGCTTGTGACGCTGTGATAGGGCGGGCGGTTCAAT
TCGTAGGCCATGCTCATGGCATCGAGCATGCTCCAGAGGATATCCAGCTTGAACTGCAGGATCTCCA
GCATGCGCGCCTGGCCCTCGCGGGTGGTGTAGTGCGCCAGGGTGATCGCCAGGCCATGCTCGACATC
ACGGCGGGCCTGGCCCAGGCGGGTGCGGAAGTATTCGTAGCCGGCCGGGTCGATCCACGGGTAGTGC
TGCGGCCAGCTGTCCAGGCGCGACTGGTGGATCTGCGGGGCGAACAGCTCGGTCAGCGAGCTGCTGG
CGGCCTCCTGCCAGCTGGCGCGGCGGGCGAAGTTGACGTAGGCGTCGACGGCGAAGCGCACGCCGGG
CAGCACCAGCTCCTGGCTGCGCAGTTGGTCCGGGTCCAGGCCCACGGCCTGGCCGAGACGCAGCCAG
GCTTCGATGCCGCCGTCCTCGCCCGGCGCGCCGTCATGGTCGAGCAGGCGCTGGACCCACTCGCGGC
GCACCTCGCGGTCCGGGCAGTTGGCCAGGATCGCGGCATCCTTCATGGGGATGTTCACCTGGTAGTA
GAAGCGGTTGGCCACCCAGCCCTGGATCTGCTCGCGGGTGGCGCGGCCTTCATACATCGCCACGTGG
TAGGGGTGGTGGATGTGGTAGAAGGCGCCCTTGGCGCGCAGGGCCTGCTCGAACTCGGCAGGTGACA
TCGGTGCGGCGTCATTCAT

**SEQ ID NO: 123, gi|104779674|ref|YP_606172.1| coenzyme PQQ synthesis protein C (Coenzyme PQQ synthesis protein I) [Pseudomonas entomophila L48]**
MNDAAPMSPAEFEQALRAKGAFYHIHHPYHVAMYEGRATREQIQGWVANRFYYQVNIPMKDAAILAN
CPDREVRREWVQRLLDHDGAPGEDGGIEAWLRLGQAVGLDPDQLRSQELVLPGVRFAVDAYVNFARR
ASWQEAASSSLTELFAPQIHQSRLDSWPQHYPWIDPAGYEYFRTRLGQARRDVEHGLAITLAHYTTR
EGQARMLEILQFKLDILWSMLDAMSMAYELNRPPYHSVTSERVWHKGIAL

**SEQ ID NO: 124, gi|119859286:64658-65413 Pseudomonas putida W619 ctg56, whole genome shotgun sequence**
ATGAGCGAGGCACTGCCACTGTCGCCTGCCGAATTCGAGCAGGCCCTGCGCGCCAAGGGCGCCTACT
ACCACATTCATCACCCCTACCACGTGGCGATGTACGAAGGGCGCGCCACCCGCGAACAGATTCAGGG
CTGGGTGGCCAACCGCTTCTATTACCAGGTGAACATCCCGATGAAGGATGCCGCGATCCTGGCCAAC
TGCCCGGACCGCGAAGTGCGCCGTGAGTGGATCCAGCGCCTGCTCGACCACGACGGAGCCCCGGGCG
AAGACGGTGGTATCGAAGCCTGGCTGCGCCTGGGCCAGGCCGTGGGGCTCGACCCGGACCAGCTGCG
TTCCCAGGAGCTGGTGCTGCCAGGTGTACGCTTCGCCGTCGATGCCTACGTGAACTTCGCCCGCCGT
GCCAGCTGGCAGGAAGCGGCCAGCAGCTCGCTGACCGAACTGTTCGCCCCGCAGATCCACCAGTCGC
GCCTGGACAGCTGGCCGCAACACTATCCGTGGATCGATCCTGCCGGCTACGAGTACTTCCGCACCCG
CCTGGGCCAGGCCCGGCGCGATGTCGAACACGGTCTGACGATCACATTGCAGCACTACACCACCCGG
GCGGCCCAGGAGCGTATGCTGGAAATCCTGCAGTTCAAGCTGGATATCCTCTGGAGCATGCTCGATG
CCATGAGCATGGCCTACGAGCTGCACCGCCCGCCGTATCACACCGTCACCCAGCAACGGGTATGGCA
CAAGGGGATTGCCCTATGA

**FIGURE 11 (continued)**

**SEQ ID NO: 125, gi|119859344|ref|ZP_01640760.1| Coenzyme PQQ biosynthesis protein C [Pseudomonas putida W619]**
MSEALPLSPAEFEQALRAKGAYYHIHHPYHVAMYEGRATREQIQGWVANRFYYQVNIPMKDAAILAN
CPDREVRREWIQRLLDHDGAPGEDGGIEAWLRLGQAVGLDPDQLRSQELVLPGVRFAVDAYVNFARR
ASWQEAASSSLTELFAPQIHQSRLDSWPQHYPWIDPAGYEYFRTRLGQARRDVEHGLTITLQHYTTR
AAQERMLEILQFKLDILWSMLDAMSMAYELHRPPYHTVTQQRVWHKGIAL

**SEQ ID NO: 126, gi|146305042:2165042-2165794 Pseudomonas mendocina ymp, complete genome**
ATGAGCCAAGCTGCCATGACACCTGCCGAGTTCGAGCAGGCGCTGCGCGCCAAGGGCGCGCTGTACC
ACATCCATCATCCGTTTCATCGCGCCATGTACGAAGGGCGCGCCACCCGCGAGCAGATTCAGGGCTG
GGTGGCCAACCGCTTCTACTATCAGGTCAATATTCCGCTCAAGGATGCGGCGATCCTCGCCAACTGT
CCGGATCGCGAAACTCGGCGCGAGTGGATTCAGCGCATCCTCGACCATGACGGTGCACCGGGTGAGG
AGGGCGGTATCGAAGCCTGGCTGCGCCTGGCCGAGTCGGTCGGCCTCGACCGCGAGCAGGTGCTGTC
GCAGGAGCTGGTGCTGCCCGGCGTGCGCTTCGCCGTCGACGCCTACGTCAACTTCGCCCGCCGCGCC
AGCTGGCAGGAAGCGGCCAGCAGCTCGCTGACCGAGCTGTTCGCGCCGACCATCCACCAGTCGCGCC
TGGACGCCTGGCCGCAGCACTACCCGTGGATCGATGCGGCCGGTTACGACTACTTCCGCAATCGTCT
GAGCCAGGCGCGGCGCGACGTCGAGCATGGCCTGCGCATCACCCTGGAGCACTACCGCACCCGTGAT
GCGCAGGAGCACATGCTGAACATCCTGCAGTTCAAGCTGGACGTGCTGTGGAGCATGCTCGACGCCA
TGAGCATGGCCTACGAGCTGGAGCGCCCGCCGTATCACACGGTGACGGCCGAGCGTGTCTGGCATAA
GGGAATCGACCTGTGA

**SEQ ID NO: 127, gi|146306995|ref|YP_001187460.1| coenzyme PQQ biosynthesis protein C [Pseudomonas mendocina ymp]**
MSQAAMTPAEFEQALRAKGALYHIHHPFHRAMYEGRATREQIQGWVANRFYYQVNIPLKDAAILANC
PDRETRREWIQRILDHDGAPGEEGGIEAWLRLAESVGLDREQVLSQELVLPGVRFAVDAYVNFARRA
SWQEAASSSLTELFAPTIHQSRLDAWPQHYPWIDAAGYDYFRNRLSQARRDVEHGLRITLEHYRTRD
AQEHMLNILQFKLDVLWSMLDAMSMAYELERPPYHTVTAERVWHKGIDL

**SEQ ID NO: 128, gi|150958624:3421777-3422589 Pseudomonas aeruginosa PA7, complete genome**
TCATGGCGCCAGCCCCCGGTGCCAGACCCGCTCACGGGTCACCGTATGGTACGGCGGACGTTCCAGC
TCGTAGGCCATGCTCATCGCGTCGAGCATGCTCCACAGCACGTCCAGCTTGAATTGCAGGATCTCCA
GCATGCGTTCCTGTGCCTCGCGGGTCCGATAGTGCTCCAGGGTGATCCGCAGGCCGTGCTCGACGTC
ACGCCGGGCCTGGGCCAGGCGGCTGCGGAAATACTCGTAGCCGGCCGCCTCGATCCACGGATAGTGG
CGCGGCCAGCTGTCCAGCCGCGACTGGTGGATCTGCGGGGCGAAGAGTTCGGTCAGCGAACTGCTCG
CCGCCTCCTGCCAGCTGGCGCGACGGGCGAAGTTGACATAGGCGTCGACGGCGAAGCGCACGCCGGG
CAGCACCCGTTCTTCGGACAGCACCTGCTCGCGCTCCAGGCCCACCGCCTCCGCCAGGCGCAGCCAG
GCCTCGATGCCTCCCGCCTCGCCGGGGGCGCCGTCGTGGTCGAGGATGCGCTGGACCCACTCGCGGC
GGACCTCGCGGTCGGGACAGTTGGCCAGGATCGCCGCGTCCTTCAGCGGGATGTTGAGCTGGTAGTA
GAAGCGGTTCGCCACCCAGCCCTGGATCTGTTCGCGACTGGCACGGCCCTCGTACATCGCGACATGG
AACGGATGGTGGATATGGTAGTAGCGCCCCTTGTCGCGCAGCGCCCGTTCGAACTCGGCGCGGTCCA
TGGCGGCACGGCTCATGTCGGTCCCCCTACAGCTGGATGTGCATGCCATCCCAGGCCACTTCGATGC
CACTGGCAT

**FIGURE 11 (continued)**

**SEQ ID NO: 129, gi|150958981|gb|ABR81006.1| coenzyme PQQ biosynthesis protein C [Pseudomonas aeruginosa PA7]**
MPVASKWPGMACTSSCRGTDMSRAAMDRAEFERALRDKGRYYHIHHPFHVAMYEGRASREQIQGWVA
NRFYYQLNIPLKDAAILANCPDREVRREWVQRILDHDGAPGEAGGIEAWLRLAEAVGLEREQVLSEE
RVLPGVRFAVDAYVNFARRASWQEAASSSLTELFAPQIHQSRLDSWPRHYPWIEAAGYEYFRSRLAQ
ARRDVEHGLRITLEHYRTREAQERMLEILQFKLDVLWSMLDAMSMAYELERPPYHTVTRERVWHRGL
AP

**SEQ ID NO: 130, gi|110645304:2172913-2173665 Pseudomonas aeruginosa PAO1, complete genome**
ATGAGCCGTGCCGCCATGGATCGCGCCGAATTCGAACGGGCGCTGCGCGACAAGGGACGCTACTACC
ATATCCACCATCCGTTCCATGTCGCGATGTACGAGGGCCGTGCCAGTCGCGAACAGATCCAGGGCTG
GGTGGCGAACCGTTTCTACTACCAGGTCAACATCCCGCTGAAGGACGCGGCGATCCTGGCCAACTGT
CCCGACCGCGAGGTCCGCCGCGAGTGGATCCAGCGTATCCTCGACCACGACGGCGCCCCCGGCGAGG
CGGGCGGCATCGAGGCCTGGCTGCGTCTGGCGGAAGCGGTGGGCCTGGAGCGCGAGCAGGTGCTGTC
CGAGGAGCGGGTGCTGCCCGGCGTGCGCTTCGCCGTCGACGCCTACGTCAACTTCGCCCGTCGCGCC
AGTTGGCAGGAGGCGGCGAGCAGTTCGCTGACCGAACTCTTCGCCCCGCAGATCCACCAGTCGCGGC
TGGACAGCTGGCCGCGCCACTATCCGTGGATCGAGGCGGCCGGCTACGAGTATTTCCGCAGTCGCCT
GGCCCAGGCCCGGCGCGACGTCGAGCACGGCCTGCGGATCACCCTGGAGCACTATCGGACCCGCGAG
GCACAGGAACGCATGCTGGATATCCTGCAATTCAAGCTGGACGTGTTGTGGAGCATGCTCGACGCGA
TGAGCATGGCCTACGAACTCGAACGCCCGCCGTACCACACGGTGACCCGCGAGCGGGTCTGGCATCG
GGGGCTGGCATCATGA

**SEQ ID NO: 131, gi|15597183|ref|NP_250677.1| pyrroloquinoline quinone biosynthesis protein PqqC [Pseudomonas aeruginosa PAO1]**
MSRAAMDRAEFERALRDKGRYYHIHHPFHVAMYEGRASREQIQGWVANRFYYQVNIPLKDAAILANC
PDREVRREWIQRILDHDGAPGEAGGIEAWLRLAEAVGLEREQVLSEERVLPGVRFAVDAYVNFARRA
SWQEAASSSLTELFAPQIHQSRLDSWPRHYPWIEAAGYEYFRSRLAQARRDVEHGLRITLEHYRTRE
AQERMLDILQFKLDVLWSMLDAMSMAYELERPPYHTVTRERVWHRGLAS

**SEQ ID NO: 132, gi|94416390:87971-88723 Pseudomonas aeruginosa PA7 PaerP_01_9, whole genome shotgun sequence**
TCATGGCGCCAGCCCCCGGTGCCAGACCCGCTCACGGGTCACCGTATGGTACGGCGGACGTTCCAGC
TCGTAGGCCATGCTCATCGCGTCGAGCATGCTCCACAGCACGTCCAGCTTGAATTGCAGGATCTCCA
GCATGCGTTCCTGTGCCTCGCGGGTCCGATAGTGCTCCAGGGTGATCCGCAGGCCGTGCTCGACGTC
ACGCCGGGCCTGGGCCAGGCGGCTGCGGAAATACTCGTAGCCGGCCGCCTCGATCCACGGATAGTGG
CGCGGCCAGCTGTCCAGCCGCGACTGGTGGATCTGCGGGGCGAAGAGTTCGGTCAGCGAACTGCTCG
CCGCCTCCTGCCAGCTGGCGCGACGGGCGAAGTTGACATAGGCGTCGACGGCGAAGCGCACGCCGGG
CAGCACCCGTTCTTCGGACAGCACCTGCTCGCGCTCCAGGCCCACCGCCTCCGCCAGGCGCAGCCAG
GCCTCGATGCCTCCCGCCTCGCCGGGGGCGCCGTCGTGGTCGAGGATGCGCTGGACCCACTCGCGGC
GGACCTCGCGGTCGGGACAGTTGGCCAGGATCGCCGCGTCCTTCAGCGGGATGTTGAGCTGGTAGTA
GAAGCGGTTCGCCACCCAGCCCTGGATCTGTTCGCGACTGGCACGGCCCTCGTACATCGCGACATGG
AACGGATGGTGGATATGGTAGTAGCGCCCCTTGTCGCGCAGCGCCCGTTCGAACTCGGCGCGGTCCA
TGGCGGCACGGCTCAT

**FIGURE 11 (continued)**

**SEQ ID NO: 133, gi|94416471|ref|ZP_01296297.1| hypothetical protein PaerP_01001794 [Pseudomonas aeruginosa PA7]**
MSRAAMDRAEFERALRDKGRYYHIHHPFHVAMYEGRASREQIQGWVANRFYYQLNIPLKDAAILANC
PDREVRREWVQRILDHDGAPGEAGGIEAWLRLAEAVGLEREQVLSEERVLPGVRFAVDAYVNFARRA
SWQEAASSSLTELFAPQIHQSRLDSWPRHYPWIEAAGYEYFRSRLAQARRDVEHGLRITLEHYRTRE
AQERMLEILQFKLDVLWSMLDAMSMAYELERPPYHTVTRERVWHRGLAP

**SEQ ID NO: 134, gi|67153296:1051625-1052377 Azotobacter vinelandii AvOP ctg60, whole genome shotgun sequence**
TCACAGTATCGTTCCCCGGTGCCAGACCCGCTCATGGGTCACCGTGTGATACGGCGGACGGTCCAGT
TCGTAGGCCATGCTCATGGCGTCGAGCATGCTCCAGAGGATGTCCAGCTTGAACTGCAGGATCTCCA
GCATGCGGGCCTGGGCCTCGCGGGTGGCGAAGTGCGCGAGGGTGATGCGCAGGCCGTGCTCGACGTC
GCGGCGCGCTTCCTTCAGGCGCCTGCGGAAGTAGTCGTAGCCGCCGGCCTCGATCCAGGGGTAGTGC
CGCGGCCAGGTGTCCAGGCGCGACTGGTGGATGGTCGGGGCGAACAGTTCGGTCAGCGAGCTGCCGG
CCGCCTCCTGCCAACTGGCGCGGCGGGCGAAGTTGACGTAGGCGTCCACCGCGAAACGCACCCCCGG
CAGCACCAGTTCCTGGGACAGCAATTGCTCGCGATCGAGGCCGACCGCCTCGCCGAGGCGCAGCCAG
GCCTCGATGCCGCCCTCCTCGCCGGGGCCGCCATCGTGGTCGAGGATGCGCTGGACCCACTCGCGGC
GGACTTCGCGGTCCGGGCAGTTGGCGAGGATCGCCGCGTCCTTCAGGGGAATGTTCACCTGGTAGTA
GAAGCGATTGGCCACCCAGCCCCGGATCTGCTCGCGGGTCGAGCGACCCTCGTACATGGCGACCTGG
AATGGATGGTGGATGTGGTAGTAGGCGCCCTTGGCGCGCAACGCCCGCTCGAATTCGGCGGGACTCA
TGGCTGTCTGGGTCAT

**SEQ ID NO: 135, gi|67154257|ref|ZP_00416002.1| Coenzyme PQQ synthesis C [Azotobacter vinelandii AvOP]**
MTQTAMSPAEFERALRAKGAYYHIHHPFQVAMYEGRSTREQIRGWVANRFYYQVNIPLKDAAILANC
PDREVRREWVQRILDHDGGPGEEGGIEAWLRLGEAVGLDREQLLSQELVLPGVRFAVDAYVNFARRA
SWQEAAGSSLTELFAPTIHQSRLDTWPRHYPWIEAGGYDYFRRRLKEARRDVEHGLRITLAHFATRE
AQARMLEILQFKLDILWSMLDAMSMAYELDRPPYHTVTHERVWHRGTIL

**SEQ ID NO: 136, gi|30841329:1889-2644 Kluyvera intermedia dipeptidase-like protein gene, partial cds; and pyrrroloquinoline biosynthesis genes, complete cds**
ATGATCATCACTGAAGCATTATCGCCCGCCGCGTTTGAGCAGGCGCTGCGCGATAAAGGCGCGTTCT
ACCACATACATCACCCGTATCACATTGCCATGCACAATGGCGAGGCGACGCGCGAGCAGATTCAGGG
CTGGGTGGCGAACCGCTTCTATTATCAGACCAACATTCCGCTGAAAGATGCGGCGATCATGGCGAAT
TGCCCGGATGCGGCGACGCGGCGTAAATGGGTGCAGCGCATCCTCGATCACGACGGCAGCAACGGTC
ACGAAGGCGGGATTGAAGCCTGGCTGCAGTTGGGTGAGGCGGTGGGGCTGGAACGTGACGTGTTGCT
GTCAGAGAAGCTGGTGCTGCCGGGCGTGCGTTTTGCGGTGGACGCCTACGTGAACTTCGCGCGCCGT
GCTAACTGGCAGGAAGCGGCCTGTAGCTCGCTCACCGAGCTGTTTGCGCCGCAGATTCATCAGTCGC
GTCTCGACAGCTGGCCGCAGCACTATCCGTGGATCAAAGAGGAAGGCTATTTCTACTTCCGCAGCCG
TTTGAGCCAGGCGAACCGCGATGTGGAGCATGGGCTGGCGCTGGCGCTTGAGGTGTTTACCCGCGCC
GAACAGCAGAACCGCATGCTGGAGATCCTGCAGTTTAAACTCGATATTCTCTGGACCATGCTCGATG
CCATGACCATGGCGTATGCGCTGCAACGTCCGCCTTATCATACGGTCACCGATCAGGTGTCCTGGCA
CAGCACAAGACTGGTATAA

FIGURE 11 (continued)

**SEQ ID NO: 137, gi|30841333|gb|AAP34380.1| PqqC [Kluyvera intermedia]**

MIITEALSPAAFEQALRDKGAFYHIHHPYHIAMHNGEATREQIQGWVANRFYYQTNIPLKDAAIMAN
CPDAATRRKWVQRILDHDGSNGHEGGIEAWLQLGEAVGLERDVLLSEKLVLPGVRFAVDAYVNFARR
ANWQEAACSSLTELFAPQIHQSRLDSWPQHYPWIKEEGYFYFRSRLSQANRDVEHGLALALEVFTRA
EQQNRMLEILQFKLDILWTMLDAMTMAYALQRPPYHTVTDQVSWHSTRLV

**SEQ ID NO: 138, gi|89092754:126652-127422 Oceanospirillum sp. MED92 1099521380382, whole genome shotgun sequence**

TCACTCATTAAACAGCCCCTTGTGAAATACCTTTTCACCCGTCACTGTATGGTATGGTGGGCGCTCC
AGTTCATAGGCCATTGTCATTGCATCAAGCATGGTCCAAAGCACATCCAACTTAAACTGAAGTATTT
CCAGCATTCTGTCTTGCTGTTCCCGGGTAGTGTAATGATCCAGCGTAATTTCAAGACCATGAACCAC
ATCACGACGCGCTTCGGTAAGCCGGTTGCGGAAGTACTGATAACCCTCTTCTTTGATCCATGGGTAA
TGCTGAGGCCATGTATCAAGGCGATTCTGATGAATGGTTGGCGCAAACAGCTCAGTCAATGAAGAGC
TGGCAGCTTCTTGCCAGGTCGCTCTGCGCGCAAAATTGATATAAGCATCTACAGCGAAGCGAACACC
AGGAAGAACATGCTCACCTGATAGGATCTCTTCGCGGGTTAGACCGACAGCCTCACCAAGACGAAGC
CATGCTTCAATACCGCCCTCGGCACCATCATAACCATCATGATCTAATACACGCTGCACCCAATGAC
GACGTACATCCCGGTCAGGGCAATTTGCCATAATTGCAGCATCTTTCACGGGGATGCTTATCTGATA
ATAGAAACGGTTTGCGACCCAGCCTCGAATCTGTTCAGGAGTAGACTGGCCGTTGTACATTGCAATA
TGGTAAGGGTGTTGAGTGTGATAAAGATCGCCTTTCGCTCTAAGCGCTTTTTCAAACGCTTCACGAC
TCATGGGTAATTTATCGTTGGCCTGTGTATTCAT

**SEQ ID NO: 139, gi|89092880|ref|ZP_01165832.1| pyrroloquinoline quinone biosynthesis protein PqqC [Oceanospirillum sp. MED92]**

MNTQANDKLPMSREAFEKALRAKGDLYHTQHPYHIAMYNGQSTPEQIRGWVANRFYYQISIPVKDAA
IMANCPDRDVRRHWVQRVLDHDGYDGAEGGIEAWLRLGEAVGLTREEILSGEHVLPGVRFAVDAYIN
FARRATWQEAASSSLTELFAPTIHQNRLDTWPQHYPWIKEEGYQYFRNRLTEARRDVVHGLEITLDH
YTTREQQDRMLEILQFKLDVLWTMLDAMTMAYELERPPYHTVTGEKVFHKGLFNE

**SEQ ID NO: 140, gi|150953431:1999950-2000705 Klebsiella pneumoniae subsp. pneumoniae MGH 78578, complete sequence**

ATGCTGATCACTGACACGCTGTCGCCGCAGGCCTTTGAAGAGGCTCTGCGGGCTAAAGGCGACTTCT
ACCATATTCACCACCCTTACCACATCGCCATGCATAACGGCAACGCGACCCGCGAGCAAATTCAGGG
TTGGGTGGCGAACCGGTTTTATTACCAGACCACTATTCCGCTGAAAGACGCGGCGATTATGGCCAAC
TGCCCGGATGCGCAGACCCGGCGCAAATGGGTGCAGCGGATCCTCGACCACGACGGCAGCCACGGCG
AGGACGGCGGGATTGAAGCCTGGCTGCGGCTGGGGGAAGCGGTCGGTTTGAGCCGCGACGATCTGCT
CAGCGAGCGTCACGTGCTGCCCGGTGTGCGCTTCGCGGTGGATGCCTATCTTAATTTCGCCCGTCGC
GCCTGCTGGCAGGAGGCGGCCTGCAGCTCCCTGACCGAGCTGTTCGCCCCGCAGATCCATCAGTCGC
GCCTCGACAGCTGGCCGCAGCACTATCCATGGATCAAAGAGGAAGGCTATTTTTACTTCCGCAGTCG
TCTGAGCCAGGCTAACCGCGACGTTGAGCATGGCCTGGCGCTGGCGAAGGCCTACTGCGACAGCGCT
GAAAAACAGAACCGGATGCTGGAGATCCTGCAGTTTAAGCTCGACATTCTGTGGTCGATGCTCGATG
CCATGACCATGGCCTATGCCCTGCAGCGCCCGCCCTATCACACGGTCACCGACAAGGCGGCCTGGCA
CACGACCCGACTGGTGTAA

FIGURE 11(continued)

**SEQ ID NO: 141, gi|150955212|gb|ABR77242.1| putative Chain B, Pqqc, Pyrroloquinolinquinone Synthase C [Klebsiella pneumoniae subsp. pneumoniae MGH 78578]**

MLITDTLSPQAFEEALRAKGDFYHIHHPYHIAMHNGNATREQIQGWVANRFYYQTTIPLKDAAIMAN
CPDAQTRRKWVQRILDHDGSHGEDGGIEAWLRLGEAVGLSRDDLLSERHVLPGVRFAVDAYLNFARR
ACWQEAACSSLTELFAPQIHQSRLDSWPQHYPWIKEEGYFYFRSRLSQANRDVEHGLALAKAYCDSA
EKQNRMLEILQFKLDILWSMLDAMTMAYALQRPPYHTVTDKAAWHTTRLV

**SEQ ID NO: 142, gi|146280397:2252480-2253139 Pseudomonas stutzeri A1501, complete genome**

ATGTATGCCGGGCAGGCGACGCGCGAGCAGATTCAGGGTTGGGTTGCCAACCGCTTCTACTATCAGG
TCTGCATCCCGGTGAAGGATGCAGCGATCATGGCCAACTGTCCGGACCGCGACACCCGCCGCGAGTG
GATTCAGCGCATCATCGACCACGATGGCGCGCCGGGTGAGGAGGGTGGCATCGAAGCCTGGCTGCGA
CTGGCCGAGGCCGTGGGTCTGGACCGCGAGCAGGTGCTGTCGCAGGAGCTGGTGCTTCCGGGCGTGC
GCTTCGCAGTGGATGCCTACGTGAACTTTGCCCGTCGGGCCTGCTGGCAGGAAGCAGCCAGCAGCTC
GCTGACCGAACTGTTCGCACCGACCATCCACCAGTCGCGGCTGGATAGCTGGCCGCAGCACTATCCC
TGGATCGACGCGTCGGGCTATGACTACTTCCGCAAGCGCCTGAAGGAAGCCCGCCGCGATGTCGAGC
ACGGCCTGCGCATCACCCTGGACCACTATCGCACCCGCGAGGCGCAGGAGCGCATGCTGGAGATCCT
TCAGTTCAAGCTCGACGTGTTGTGGAGCATGCTGGACGCCATGAGCATGGCCTATGAGCTGGACCGA
CCGCCGTACCATACCGTCACCCGCGACCGGGTCTGGCACAAGGGCATCAGCTTCTGA

**SEQ ID NO: 143, 282431|ref|YP_001172584.1| pyrroloquinoline quinone biosynthesis protein C [Pseudomonas stutzeri A1501]**

MYAGQATREQIQGWVANRFYYQVCIPVKDAAIMANCPDRDTRREWIQRIIDHDGAPGEEGGIEAWLR
LAEAVGLDREQVLSQELVLPGVRFAVDAYVNFARRACWQEAASSSLTELFAPTIHQSRLDSWPQHYP
WIDASGYDYFRKRLKEARRDVEHGLRITLDHYRTREAQERMLEILQFKLDVLWSMLDAMSMAYELDR
PPYHTVTRDRVWHKGISF

**SEQ ID NO: 144, 083297:2461135-2461902 Acinetobacter sp. ADP1, complete genome**

TTGAGGTTTAATATGACCACAACATTATTTCTCCTGCGGAATTTGAACAAGCCCTGCGTGACAAAG
GTCGCTACTATCATATTCATCATCCCTATCACATCATGATGAATGATGGGCATGCCACTAAGCAACA
AATTCAGGGCTGGGTCGCCAACCGTTTTTATTATCAGGTCAATATCCCTTTAAAAGATGCGGCCATC
ATGGCCAATTGCCCAGATCCTGCGACACGCCGTAAATGGGTGCAACGTATTTTGGATCACGATGGTC
AACATGACGACCATGGCGGAATTGAAGCATGGTTGCGTCTGGGTGAAGCCGTTGGCCTAGACCGTGA
GACTATTTTGTCACAAAAGATGGTTTTGCCCAGTGTACGTTTTGCGGTAGATGCCTATGTCAATTTT
GCCCGTCGTGCCTGCTGGCAAGAAGCTGCATGTAGTTCGCTTACCGAACTTTTTGCACCTGCCATCC
ATCAATCACGTCTGGATACTTGGCCTACACATTACCCATGGATTGATGCAGAAGGTTACGCCTATTT
TAGAGGACGCTTGAGTCAGGCCAATCGTGACGTTGAGCATGGTTTGGAACTGGCACTTGAGTACTGC
AATACCGTAGATCGCCAGCAGCGTATGTTGAATATTTTACAGTTCAAACTGGATATTTTGTGGACGA
TCCTTGATGGTATGAGCATGGCCTATGTGCTTGAGCGTGCACCATATCACACGGTTACTCAAGAAGC
GGTTTGGCATCAAAAAGGATTATTAGGATGA

**SEQ ID NO: 145, 085585|ref|YP_047095.1| pyrroloquinoline quinone biosynthesis protein PqqC [Acinetobacter sp. ADP1]**

MRFNMTTTLFSPAEFEQALRDKGRYYHIHHPYHIMMNDGHATKQQIQGWVANRFYYQVNIPLKDAAI
MANCPDPATRRKWVQRILDHDGQHDDHGGIEAWLRLGEAVGLDRETILSQKMVLPSVRFAVDAYVNF
ARRACWQEAACSSLTELFAPAIHQSRLDTWPTHYPWIDAEGYAYFRGRLSQANRDVEHGLELALEYC
NTVDRQQRMLNILQFKLDILWTILDGMSMAYVLERAPYHTVTQEAVWHQKGLLG

## FIGURE 11 (continued)

**SEQ ID NO: 146, gi|113196157:2675-3424 Serratia marcescens PqqA (pqqA), PqqB (pqqB), PqqC (pqqC), PqqD (pqqD), PqqE (pqqE), and PqqF (pqqF) genes, complete cds; and unknown gene**
ATGACGCAACCACTCCCCCTTGACCCACAGGCGTTTGAAGCGCGCCGTCCACAAGGCGCTTATTACC
ATATCCACCACCCGTACCACATCGCGATGCACAACGGCGAAGCGACCCGCGAGCAGATCCAGGGCTG
GGTGGCCAACCGGTTCTACTACCAGACCAGCATTCCGATTAAGGATGCAGCGATCATGGCCAACTGC
CCACAGCCGGAAACACGCCGCAAATGGGTACAGCGCATCCTGGATCACCACGGCACGGACGGCAGCG
AAGGCGGCATCGAGGCCTCTGACCTGGGCGAAGCCGTTGGGCTGCAGCGCGACACTTTGCTCTCTGA
AGAGCGGGTACTGCCGGGCGTGCGCTTCGCGGTGGACGCCTACGTCAACTTCGCGCGCCGCGCCTGC
TGGCAGGAAGCGGCCTGCAGCTCACTGACCGAACTGTTCGCGCCGCAAATTCACCAATCGCGCCTCG
ACAGCTGGCCGCAGCACTACCCGTGGATCGCAGCCGTCGGCTACGACTATTTCCGCAGCCGCCTCCG
CCAGGCCAACCGCGATGTGGAACACGGCCTGGCGCTGGCGCTGGACTATTGCGACACCGTTGAAAAG
CAGCAGCGCATGCTGGAGATCCTGCAGTTCAAGCTGGACATTCTGTGGAGCATGCTGGACGCCATGA
GCATGGCCTACACCCTGAACCGCCCACCTTACCACAGCGTGACCGCCGCGCGGGTATGGCACAACCA
GAGGCTGGTGTAA

**SEQ ID NO: 147, gi|113196161|gb|ABI31435.1| PqqC [Serratia marcescens]**
MTQPLPLDPQAFEARRPQGAYYHIHHPYHIAMHNGEATREQIQGWVANRFYYQTSIPIKDAAIMANC
PQPETRRKWVQRILDHHGTDGSEGGIEASDLGEAVGLQRDTLLSEERVLPGVRFAVDAYVNFARRAC
WQEAACSSLTELFAPQIHQSRLDSWPQHYPWIAAVGYDYFRSRLRQANRDVEHGLALALDYCDTVEK
QQRMLEILQFKLDILWSMLDAMSMAYTLNRPPYHSVTAARVWHNQRLV

**SEQ ID NO: 148, gi|149375729:89332-90063 Marinobacter algicola DG893 1103407001916, whole genome shotgun sequence**
CTACAGCCCCCGGTGCCAGACCGGCTGACCGGTTACGGTGTGGTACGGCGGCGTGCGGTGCTGATAA
GCCATAGTCAGGGCATCCAACATAGACCATAAGATATCCAGTTTGAATTGAAGGATTTCCAGGGCAC
GGGCCTGCTGTGCCGCGGTGGTAAAGTGATCCAGGGTAATGGTCAGGCCATGTTCCACATCGCGGCG
CGCTTCCGACAACCGCTTGCGGAAATAGGCGTATCCATCGGTCTGGATCCAGGGATAATGCTGGGGC
CAGCTATCGAGCCGGGACTGGTGAATTTCCGGCGCAAACAGTTCTGTCAGGGAGGAACAGGCCGCTT
CCTGCCAAGTCGCCCGGCGGGCAAAGTTCAGGTAGGCGTCTACGGCAAACCGTACACCCGGCAGCAC
ATGGCGCTGGTCGGTGACCTCGTCCCGGGTTAGCCCGACCGCTTCCGCCAAGCTCAACCAGGCCTCG
ATACCGCCTTCGTCGTCAATGTGGCCATCGTGATCCAACACTCGTTGGAGCCACAACCGGCGGACCG
AGGCATCCGGGCAGTTAGCCATGATCGCCGCATCCTTGCGGGGTATGTTGATCTGGTAGTAGTACCG
GTTAGCGACCCAGCCACGAATCTGCTCAGGAGTGCAGTCACCGCCATACATGGCTTTATGGTAGGGA
TGGTGAATGTGGTAGTACTGGCCTTTTGCACGCAGGGCCTGCTCGAAATCCCCACGATTCAT

**SEQ ID NO: 149, gi|149375810|ref|ZP_01893578.1| pyrroloquinoline quinone biosynthesis protein C [Marinobacter algicola DG893]**
MNRGDFEQALRAKGQYYHIHHPYHKAMYGGDCTPEQIRGWVANRYYYQINIPRKDAAIMANCPDASV
RRLWLQRVLDHDGHIDDEGGIEAWLSLAEAVGLTRDEVTDQRHVLPGVRFAVDAYLNFARRATWQEA
ACSSLTELFAPEIHQSRLDSWPQHYPWIQTDGYAYFRKRLSEARRDVEHGLTITLDHFTTAAQQARA
LEILQFKLDILWSMLDALTMAYQHRTPPYHTVTGQPVWHRGL

FIGURE 11 (continued)

**SEQ ID NO: 150, gi|126665966:88230-88985 Marinobacter sp. ELB17 1101232001226, whole genome shotgun sequence**

TCATAGCCCCCTGTGCCAGACTTGCTGATCGGTAACCGTATGGAACGGCGGCGTTTTGTGGCTGTAG
GCCATGGTCAGGGCGTCCAGCAGGCTCCACAGAATATCCAGTTTGAACTGTAAAATTTCGAGGGCGC
GGGCCTGCTCGGTAGCTCTGGTGAAATACTCCAGCGTAATCGCCAGGCCATGTTCCACATCGCGGCG
GGCTTCTGATAGGCGCTTACGGAAGTAGCTATAGCCGGCCTCGTCAATCCACGGATAATGTTCTGGC
CAGCTGTCCAGACGAGACTGGTGGATTTCTGGCGCAAACAGCTCGGTCAGCGAGGAACAAGCCGCTT
CTTGCCAAGTCGCGCGGCGCGCAAAGTTCAAGTAGGCGTCCACCGCAAAACGCACGCCGGGCAGCAC
GTGGCGCTGGTCGATCACCTCTTCCCGAGTCAGCCCCACGGCTTCCGCCAGGCACAACCAGGCTTCA
ATGCCGCCCGCATCACCGTCGTGACCATCGTGGTCAAGAATGCGCTGCAACCATTGCTTGCGCACGC
TGGCGTCGGGGCAATTGGCCATAATGGCCGCATCTTTACGCGGGATCATTACCTGATAATAATAGCG
GTTGGCCACCCAGCCCCGGATTTGCTCAGGGCTGCACTGGCCACCATACATGGCCTGATGATACGGG
TGATGAATATGATAGAGCCGGCCCTTATTGCGTAACGCCTGTTCAAAATCCGCGCGATTCAGGGTGG
TGTTCACAATGGCGTTCAT

**SEQ ID NO: 151, gi|126666044|ref|ZP_01737024.1| pyrroloquinoline quinone biosynthesis protein PqqC [Marinobacter sp. ELB17]**

MNAIVNTTLNRADFEQALRNKGRLYHIHHPYHQAMYGGQCSPEQIRGWVANRYYYQVMIPRKDAAIM
ANCPDASVRKQWLQRILDHDGHDGDAGGIEAWLCLAEAVGLTREEVIDQRHVLPGVRFAVDAYLNFA
RRATWQEAACSSLTELFAPEIHQSRLDSWPEHYPWIDEAGYSYFRKRLSEARRDVEHGLAITLEYFT
RATEQARALEILQFKLDILWSLLDALTMAYSHKTPPFHTVTDQQVWHRGL

**SEQ ID NO: 152, gi|114770473:150060-150818 Alpha proteobacterium HTCC2255 1100007007369, whole genome shotgun sequence**

ATGACAAAGCAAGCACCTTGGTCTCGCGATGAATTTGAAGCAAAGCTCCGTGCTAAAGGCGAGTACT
ACCACATCTATCATCCGTTTCATGTAGCGATGAATAAAGGTGAATGTAGCCAAGAACAAATTCAAGG
ATGGGTCGCCAATCGCTTATTTTATCAGTTAGCGATCCCAGTAAAAGATGCAGGGATCATGGCAAAT
TGCTGGGATCTGCAAACCCGTCGCAAATGGATCCAGCGTATATTGGATCATGATGGAGACCCTGAAG
ATACTACTCTAGGCGGTATTGAAGCTTGGTTACGTCTAGGTGAAGCGGTGGGTTTACATCGTGAAGA
TATGCTCAATGAACGCTTTTTATTACCTGGTGTGAAGTTTGCCGTCGGGGCATATATTAACTTTACG
CGTAAAGCTAGCTGGGAAGAAGCAGCCTGTTCGTCACTCACTGAGATGTTCGCACCTGAAATCCATC
AAAGTCGCCTCGATACTTGGCCAACCCATTATAAATGGATTGATCCAGAAGGATTTTCCTATTTTCA
AATGCGTTTAAATCAGGCACGTCGTGATGTACAGCACGGTTTAGAGATCACTTTAGATCATTTTGAA
ACGCGTAAACAACAAGAGCGTGCATTGAATATTCTGCAATTCAAACTTGATGTCTTATGGAGTATGG
CTGATAGTATCAGCTTAGCATATGAGTTTAACCGCAAGCCTTTTGGTGCGGTAACTGACGAAAATAT
TTCTCACAAAGGCTTCATGTAA

**SEQ ID NO: 153, gi|114770609|ref|ZP_01448099.1| coenzyme PQQ synthesis protein C [alpha proteobacterium HTCC2255]**

MTKQAPWSRDEFEAKLRAKGEYYHIYHPFHVAMNKGECSQEQIQGWVANRLFYQLAIPVKDAGIMAN
CWDLQTRRKWIQRILDHDGDPEDTTLGGIEAWLRLGEAVGLHREDMLNERFLLPGVKFAVGAYINFT
RKASWEEAACSSLTEMFAPEIHQSRLDTWPTHYKWIDPEGFSYFQMRLNQARRDVQHGLEITLDHFE
TRKQQERALNILQFKLDVLWSMADSISLAYEFNRKPFGAVTDENISHKGFM

**SEQ ID NO: 154, gi|71277742:875002-875757 Colwellia psychrerythraea 34H, complete genome**

TCATTTAAAAATTCTCTTATGGTAATTTGGCTCAGCTATTAAACCTTGGTAAGGCGCTCGGTCCTGT
TGATATGCTAAGGTCATAGCATCAAGCATACTCCACAAAATATCTAATTTAAATTGGAGAATATTTA
ATGCCTCGTCTTGTGCTTGACGAGTAGTAAAGTGATCTAAGGTGATTTGTAGGCCATGATTAAC

FIGURE 11 (continued)

ATCGCGTCTTGCTTGAGATAAGCGCATTTGAAAATACTGTAAGCCTTGTGTTTCAATCCAAGGATAA
TTTATAGGCCAACTGTCTAGCCTGCTTTGATGTATTTGAGGGGCAAACATTTCCGTCAGTGACGAAC
ATGCAGCTTGCTGCCAGTTAGCACGGCGAGCAAAGTTTACATAGGCATCTACGGCGAATCGCACCCC
AGGTAAAACAAATTTCTCATCCAGTAAGTCTTGTCTATCTAATCCTACCGCTTGGCCCAATTGTAAC
CAGGCTTCAATACCACCTAAGGTTTTGTCTTCTACACTGCCATCGTGATCTAATATGCGTTGGATCC
ATAGACGACGAGTTTCCATATCATGACAATTAGCTAATATGGCGGCATCTTTAATAGGAATATTTAC
TTGGTAATAAAAACGGTTTGCCACCCAACCCTGAATTTGCTCAACACTACATTCACCTTGGTGCATG
GCAATATGAAATGGGTGATGAATATGGTAAAGCTGCCCTTTAGCGCGGAGCATTTCTTCAAACTGCT
CTCTTGTCCATGGTTGTTGCAT

**SEQ ID NO: 155, gi|71282639|ref|YP_267608.1| coenzyme PQQ synthesis protein C [Colwellia psychrerythraea 34H]**
MQQPWTREQFEEMLRAKGQLYHIHHPFHIAMHQGECSVEQIQGWVANRFYYQVNIPIKDAAILANCH
DMETRRLWIQRILDHDGSVEDKTLGGIEAWLQLGQAVGLDRQDLLDEKFVLPGVRFAVDAYVNFARR
ANWQQAACSSLTEMFAPQIHQSRLDSWPINYPWIETQGLQYFQMRLSQARRDVNHGLQITLDHFTTR
QAQDEALNILQFKLDILWSMLDAMTLAYQQDRAPYQGLIAEPNYHKRIFK

**SEQ ID NO: 156, gi|90417271:58200-58922 Gamma proteobacterium HTCC2207 1099215844453, whole genome shotgun sequence**
TTACCGGCTACAGCTATGAAATGGCGGCTCTTCATAGACATAAGCCAACTGCATTGCATCCAGCATA
CTCCATAAAATATCCAGCTTTAACTGCAGTATACCCATGGCCTGCTGCTGCTGTTCGAAGGTTTTAA
AGTAGTCCAGAGTAATCGCCAAACCATGCTCTACATCTCTTCGGGCTTCGCTAAGTCGCGTGCGGAA
ATATTGATAGCCGTTGGGATCGATCCAGGGATAGTGTTCCGGCCAGGTCGCTAAGCGTTTTTGATGA
ATGGTCGGGGCAAAGAGTTCGGTGAGTGATGAGCAGGCCGCTTCCTGCCAGTTAGCGCGGCGGGCAA
AGTTAACGTAGGCGTCTACTGCAAAGCGCACGCCGGGTAATATATGTTGCTGACTTATAATTTCGTC
TCGGGTTAAACCCACAGCCTCTCCCAAGCGGAGCCAGGCCTCAATACCACCACTATCCTCATCGCTG
GTGCCATCGTGATCAAGAATACGCTGCACCCATAAACGACGAACAGAGGCGTCGTCACAGTTGGCCA
TAATGGCGGCGTCTTTGATCGGTATACTGATTTGATAATAATAGCGGTTAGCCACCCAGCCCTGTAT
CTGCTTTTGAGTACAGTTGCCGGTATTCATCGCAATATGGAAGGGGTGATGAATATGGTAGAGCGCG
CCCTTGTCTCGTAGCTGCTGCTCAAATTCTTCTGCAGTCCAGGCTTTAGTCAT

**SEQ ID NO: 157, gi|90417324|ref|ZP_01225250.1| coenzyme PQQ synthesis protein C [marine gamma proteobacterium HTCC2207]**
MTKAWTAEEFEQQLRDKGALYHIHHPFHIAMNTGNCTQKQIQGWVANRYYYQISIPIKDAAIMANCD
DASVRRLWVQRILDHDGTSDEDSGGIEAWLRLGEAVGLTRDEIISQQHILPGVRFAVDAYVNFARRA
NWQEAACSSLTELFAPTIHQKRLATWPEHYPWIDPNGYQYFRTRLSEARRDVEHGLAITLDYFKTFE
QQQQAMGILQLKLDILWSMLDAMQLAYVYEEPPFHSCSR

**SEQ ID NO: 158, gi|119504206:124612-125391 Marine gamma proteobacterium HTCC2080 1100755000532, whole genome shotgun sequence**
ATGTCGCTCCTTGATACCGCTTCATCGCGAGACGCATCTAATCAGGCCCCTCTGACCCGAGCAGATT
TTGAGGCGCGTCTGCGCGCCAAGGGCTCGCGCTATCACATCCACCATCCTTTTCATCAAGCGATGTA
CGCGGGTGAGCTAACGCCTCGACAAATTCGGGGCTGGGTAGCCAACAGGTATTACTACCAAATAATG
ATTCCTCAAAAGGATGCAGCGATTCTGGCCAATTGCTCTGACCGGGAAATCCGCGTGAAATGGATCC
AGCGTATCCTTGATCATGATGGTGCCGACGGTGATGAAGGCGGTATTGAAGCCTGGTTGGAATTGGC
TGAAGCGGTGGGACTTAAGCGTGATGAAGTGACCGATCTTCGCTACGTGCTACCCGGTGTGCGCTTT
GCGGTCGATGCTTATGTCAACTTCGCACGTAACGCCACTTGGCAGGAAGCCGCTTGCTCA

## FIGURE 11 (continued)

TCGCTAACAGAGATGTTCGCGCCTGAAATTCATCGGTCCCGGCTCAATACTTGGCCGCAACATTACA
GCTGGATTGACCAAAGCGGATTGAAGTACTTTCAAAAGCGTCTAGGAGAGGCCCGGCGTGATGTTGA
GCATGGCCTGGACGTAACTCTCGATTATTTCAAATATCGCGAACAGCAGGAAAAGGCGCTGGGTATT
TTACAGTTTAAACTCGATATTCTGTGGACAATGCTGGACGCAATGACCATGGCCTACGTGCACACCA
CGCCCCCATATCACAGCTGTGATGACGGTATTCATTCGGTACCTGGGTAA

**SEQ ID NO: 159, gi|119504323|ref|ZP_01626403.1| pyrroloquinoline quinone biosynthesis protein PqqC [marine gamma proteobacterium HTCC2080]**
MSLLDTASSRDASNQAPLTRADFEARLRAKGSRYHIHHPFHQAMYAGELTPRQIRGWVANRYYYQIM
IPQKDAAILANCSDREIRVKWIQRILDHDGADGDEGGIEAWLELAEAVGLKRDEVTDLRYVLPGVRF
AVDAYVNFARNATWQEAACSSLTEMFAPEIHRSRLNTWPQHYSWIDQSGLKYFQKRLGEARRDVEHG
LDVTLDYFKYREQQEKALGILQFKLDILWTMLDAMTMAYVHTTPPYHSCDDGIHSVPG

**SEQ ID NO: 160, gi|118073700:52282-53031 Shewanella woodyi ATCC 51908 ctg104, whole genome shotgun sequence**
ATGCAAGCCTGGACTAAAGAAGAATTCGAACAAAAACTCAAAGATAAAGGGTCGCTTTACCATATAC
ACCATCCCTACCATAAGATGATGCATAAAGGTGAGTGCAGCGTTGAACAGATCAGAGGTTGGGTTGC
CAACCGCTTCTATTATCAGATCAATATTCCCGTTAAAGATGCAGCCATACTGGCAAACTGTCGCGAT
GTAAAAACTCGCCGCATGTGGATCCAACGTATAGTGGATCATGATGGTTCAATTGAAGATAACACCT
TAGGTGGTATCGAGGCTTGGCTAAAACTCGGTGAAGCGGTGGGACTGAATAGACAAGATATGCTAGA
TGAAAAGTATATCCTGCCAGGGGTTCGGTTCGCGGTCGATGCCTATGTGAATTTTGCCAGACGAGCC
TCATGGCAAGAGGCGGCCTGCTCATCACTTACCGAGATGTTTGCGCCAGAGATACACCAAAGTCGCC
TTAATACCTGGCCAGAAAACTACCCTTGGATAAAATCCGAAGGATTAAGCTACTTTCAGATGCGACT
CTCTCAAGCAAGGCGAGATGTTAATCATGGCTTAGCGATCACTTTGGCACATTTTACGACCCGAGAG
CAGCAGGAGCATGCACTGAATATTTTGCAGTTTAAACTCGATATTCTTTGGTCCATGTTAGATGCAA
TGACATTAGCTTATCAGTATGAGCGTGCGCCCTATTCACAGCTTATTGATGCGCCTGTCTATCATAA
AGGGATCTTCTGA

**SEQ ID NO: 161, gi|118073743|ref|ZP_01541920.1| coenzyme PQQ biosynthesis protein C [Shewanella woodyi ATCC 51908]**
MQAWTKEEFEQKLKDKGSLYHIHHPYHKMMHKGECSVEQIRGWVANRFYYQINIPVKDAAILANCRD
VKTRRMWIQRIVDHDGSIEDNTLGGIEAWLKLGEAVGLNRQDMLDEKYILPGVRFAVDAYVNFARRA
SWQEAACSSLTEMFAPEIHQSRLNTWPENYPWIKSEGLSYFQMRLSQARRDVNHGLAITLAHFTTRE
QQEHALNILQFKLDILWSMLDAMTLAYQYERAPYSQLIDAPVYHKGIF

**SEQ ID NO: 162, gi|119474629:288921-289646 Marine gamma proteobacterium HTCC2143 1100111000176, whole genome shotgun sequence**
CTAGTCGATATTGCAGCTATGGAACGGCGCCTCATTGTAGACGTATGCCAGTTGCATAGCATCCAGC
ATGCTCCACAATATATCGAGCTTCATTTGCAGAATACCCAATGCATGCTCTTGTGCTTCGCGGGTTT
GGAAGTGATCCAGCGTGATTTGCAATCCGTGCTCAACATCACGGCGCGCTTCACTGAGGCGAGTACG
AAAATAACGGTAGCCCTCAGCTTCAATCCAGGGGTAATGCTCAGGCCAGCTGTCGAGTCTTTTTTGA
TGAATAATTGGTGCAAATAATTCGGTCAAAGACGAACAGGCGGCTTCCTGCCAGCTCGCTTTGCGGG
CAAAGTTAACATAAGCATCTACCGCGAAACGGACGCCGGGTAATACGTGTTGAAAACTAGTGATCTC
ACTGCGCTGCAGCCCAACGGCTTCGCCGAGGCGCAGCCAGGCCTCAATCCCGCCACTGTCTTGCTCG
CTAGTGCCGTCGTGGTCCAATATACGCTGTACCCATAGCCTGCGCACGGTAGGCTCGGTACAGTTCG
CAAGGATGTTAGCGTCCTTGATCGGGATATTAACCTGGTAGTAGTACCGATTGGCGAC

**FIGURE 11 (continued)**

CCAGCCTTGTATTTGCTGCTGGTTACATTGGCCGCTGTTCATTGCTACATGCAGAGGATGATGAATA
TGATACAAGGCTTCTTTGGCCCGCAAGCCTCGTTCGAATTCTTCTCTTGACCATGGTTTTGACAT

**SEQ ID NO: 163, gi|119474895|ref|ZP_01615248.1| coenzyme PQQ synthesis protein C [marine gamma proteobacterium HTCC2143]**
MSKPWSREEFERGLRAKEALYHIHHPLHVAMNSGQCNQQQIQGWVANRYYYQVNIPIKDANILANCT
EPTVRRLWVQRILDHDGTSEQDSGGIEAWLRLGEAVGLQRSEITSFQHVLPGVRFAVDAYVNFARKA
SWQEAACSSLTELFAPIIHQKRLDSWPEHYPWIEAEGYRYFRTRLSEARRDVEHGLQITLDHFQTRE
AQEHALGILQMKLDILWSMLDAMQLAYVYNEAPFHSCNID

**SEQ ID NO: 164, gi|91777110:659108-659860 Burkholderia xenovorans LB400 chromosome 2, complete sequence**
TCATTGCGGGAAGGCCTTTTCGATCGAATCCAGCATGGTCCACAGAATGTCGAGCTTGAATTGCAGG
ATCTCGAGCGCGCGTTCCTGCTGTTCGCGCCGCGTGAAATGATCGAGCGTGACGGCGAGCCCGTGCT
CCACGTCGCGTTGTGCAAGCGAAATGCGCGAGCGGAAATACGCGAGACCCGAAGACTCGATCCACGG
ATAATGTTCCGGCCAGCTCGCGAGCCGGTCGCGATGAACCTGCGGCGCGAACATTTCCGTGAGCGAC
GAGCAGACCGATTCCTGCCACGGCGCGCGGCGCGCAAAATTCACATAGGCGTCGACGGCGAAGCGCA
CGCCGGGCGCCACCTGTTTCAGCGACCACAGATCGTCACGCGACAGCCCGACCGCGTCGCCCAGCCG
CGCCCATGTTTCGATGCCGCCTTCCTCGTCGCCGCAACCGTCGTGGTCGAGAATGCGCAGCACCCAG
CGGCGGCGTGTTTCGCGATCCGGGCAATTCGACAGCACGGCCGCATCCTTCAGCGGAATATTGATCT
GATAATAGAAGCGATTGGCGACCCAGCCGCGAATCTGTTCGCGGGAGCAACCGCCGCTATTCATCTT
CACATTGAACGGATGATGAATGTGATACGCGGCGCCTTTCGCGCGCAACTGTGCTTCGAATTCTTCA
CGGGTCCAGGCCGGGCGCTCGTCGCCTCCGTTCGTTTGCGCGTTCTGGCGCGGCGCCGTGCCCAAGG
TATCTTTCGCGTTCAT

**SEQ ID NO: 165, gi|91777665|ref|YP_552873.1| Pyrroloquinoline quinone synthesis C [Burkholderia xenovorans LB400]**
MNAKDTLGTAPRQNAQTNGGDERPAWTREEFEAQLRAKGAAYHIHHPFNVKMNSGGCSREQIRGWVA
NRFYYQINIPLKDAAVLSNCPDRETRRRWVLRILDHDGCGDEEGGIETWARLGDAVGLSRDDLWSLK
QVAPGVRFAVDAYVNFARRAPWQESVCSSLTEMFAPQVHRDRLASWPEHYPWIESSGLAYFRSRISL
AQRDVEHGLAVTLDHFTRREQQERALEILQFKLDILWTMLDSIEKAFPQ

**SEQ ID NO: 166, gi|77128441:1539216-1539941 Methylococcus capsulatus str. Bath, complete genome**
ATGAGCCAAGACACGATCCCCTGGAGCCGCGAAGAATTCGAGGCTCGGCTGCGTGCCAAGGAAAAGT
ACTACCACATCCATCACCCTTACCACGTGTTGATGGCGAGCGGTGAGCTGAACCGCGAGCAGATCCA
GGGCTGGGTGGCGAACCGCTTCTATTATCAGATCACCATTCCGCGCAAGGATTCGGCCATCATGGCC
AACTGCCCGGACCGCGAAACCCGCCGCAAATGGATGCAGCGGGTGATGGATCACGACGGTTACGGCG
ATGATCCCGGCGGGATCGAGGCATGGATCCAGCTAGGCATCGCCTGTGGCCTGAGTCGCGAGGACAT
CACCTCGCTCAGGCATGTACTGCCCGGCGTGCGTTTCGCCGTCGACGCCTACCTCAACTTCGCTCGT
ACCGCGACCTGGCAGGAAGCCGCCTGCTCTTCGCTCACCGAGCTGTTTGCGCCGACCATCCACAAAC
AACGCCTGGCTGGCTGGCCGGATCTGTATCCGTGGATCGCAGCCGACGGCTTGGCCTATTTCCGCAA
GCGGGTGACTCAGGCCAGCCGCGATGTCGAGCACGGCATCGAAATCACGCTGGATCATTTCAAGACC
CGCGAACAACAGGAACGGGCCCTGGAAATCCTCCAATTCAAGCTCGACATCCTGTGGTCGATGTTGG
ACGCCATGTACCTCGCCTATGTCGACAAAAAACCGCCCTATTTCAACATCGCCTGA

**FIGURE 11 (continued)**

**SEQ ID NO: 167, gi|53804508|ref|YP_113905.1| pyrroloquinoline quinone biosynthesis protein PqqC [Methylococcus capsulatus str. Bath]**

MSQDTIPWSREEFEARLRAKEKYYHIHHPYHVLMASGELNREQIQGWVANRFYYQITIPRKDSAIMA
NCPDRETRRKWMQRVMDHDGYGDDPGGIEAWIQLGIACGLSREDITSLRHVLPGVRFAVDAYLNFAR
TATWQEAACSSLTELFAPTIHKQRLAGWPDLYPWIAADGLAYFRKRVTQASRDVEHGIEITLDHFKT
REQQERALEILQFKLDILWSMLDAMYLAYVDKKPPYFNIA

**SEQ ID NO: 168, gi|149927558:87391-88098 Limnobacter sp. MED105 1103186003331, whole genome shotgun sequence**

TCATTTTTTTCTCTTGTCGGGATACGCCAGTGCAATCGCATCGGACATACTCCAAAGCACGTCCAGT
TTGAATTGCAGAATTTCAAGTGCACGTTCCTGTTGGGCACGGGTTTTGAAATAATTCAAGGTCACTT
CCAGGCCATGTTCAACGTCGCGTTGCGCCAGGCTGATTCTGGAGCGAAAATAATTCAAACCTTCAGG
TGCAACCCATTTGTAGTCTTTGGGCCAGTTGCTCAGGCGTTGCTTGTGAATTTCTGGTGCAAACATT
TCAGTCAACGATGAGCACACCGCTTCTTGCCAGGGCGCGCGGCGGGCAAAATTCACGTAGGCGTCCA
CTGCAAAACGCACGCCTGGAAGCACCAGTTGCTGGCTCCACAGCACGTCTTTGTCAATGCCTACTGC
TTCACCCAGGCGACTCCAGGCTTCAATGCCGCCAGCATTGCCTTCCCAGCCATCGTGATCCAGAATG
CGCTGCACCCAACGGCGACGGGTTTCGCGATCGTCACAATTGGCCATCACCGCAGCGTCTTTTTGCG
GAATGCACACTTGATAATAAAAGCGGTTTGCCACCCAGCCGCGCAGTTGTTCCCGGCTCAGTTTGCC
CTCACGCATGGCCACGTTGAATGGGTGGTGAATGTGATAGCGGTCGCCCTTTGCGCGCAAGCGTTCT
TCGAACTCCTTTTTGCTCCAGGCTTTGTTACTGCTCAT

**SEQ ID NO: 169, gi|149927645|ref|ZP_01915898.1| Pyrroloquinoline quinone synthesis C [Limnobacter sp. MED105]**

MSSNKAWSKKEFEERLRAKGDRYHIHHPFNVAMREGKLSREQLRGWVANRFYYQVCIPQKDAAVMAN
CDDRETRRRWVQRILDHDGWEGNAGGIEAWSRLGEAVGIDKDVLWSQQLVLPGVRFAVDAYVNFARR
APWQEAVCSSLTEMFAPEIHKQRLSNWPKDYKWVAPEGLNYFRSRISLAQRDVEHGLEVTLNYFKTR
AQQERALEILQFKLDVLWSMSDAIALAYPDKRKK

**SEQ ID NO: 170, gi|118033101:63155-63862 Burkholderia phymatum STM815 ctg44, whole genome shotgun sequence**

ATGAGTGTCAAAGGCAGCAGCGGACCGGCATGGAGCCGCGATGAATTCGAAGCGCAATTGCGTGCGA
AGGGACAGGCGTATCACATCCATCATCCGTTCAACGTGAAAATGAACAGCGGCGGATGTTCGCACGA
GCAGATTCGCGGCTGGGTCGCGAACCGCTTCTACTATCAGATCAACATTCCGCTGAAGGATGCCGCG
GTGCTGTCGAACTGTCCCGACCGCGACACGCGCCGCCGCTGGGTGCTGCGCATACTCGATCACGACG
GTTACGGCGAAGACGAAGGCGGCATCGAAACATGGGCGCGGCTCGGCGATGCGGTCGGCCTGTCGCG
CGATGAACTGTGGTCGCTCGAACACGTGGTGCCGGGCGTGCGCTTCGCGGTCGACGCATACGTGAAC
TTTGCGCGGCGCGCGCCGTGGCAGGAAGCCGTCTGCTCGTCGCTCACCGAAATCTTCGCGCCGCAGA
TCCACAAAGACCGGCTCGCCACATGGCCCGAACACTACCCGTGGATCAAGCCCGAAGGGCTCGCATA
TTTCCGTTCGCGCATTTCGCTTGCACAACGCGACGTCGAACACGGGCTGTCCGTCACGCTCGATCAT
TTCCGCACGCGCGACCAGCAACAGCGCGCGCTCGACATTCTGCAATTCAAGCTCGACATTCTGTGGA
CGATGCTCGACGCCATCGAGAAGGCCTTCCCAGCATGA

**SEQ ID NO: 171, gi|118033162|ref|ZP_01504607.1| coenzyme PQQ biosynthesis protein C [Burkholderia phymatum STM815]**

MSVKGSSGPAWSRDEFEAQLRAKGQAYHIHHPFNVKMNSGGCSHEQIRGWVANRFYYQINIPLKDAA
VLSNCPDRDTRRRWVLRILDHDGYGEDEGGIETWARLGDAVGLSRDELWSLEHVVPGVRFAVDAYVN
FARRAPWQEAVCSSLTEIFAPQIHKDRLATWPEHYPWIKPEGLAYFRSRISLAQRDVEHGLSVTLDH
FRTRDQQQRALDILQFKLDILWTMLDAIEKAFPA

**FIGURE 11 (continued)**

**SEQ ID NO: 172, gi|84352648:18345-19064 Burkholderia cenocepacia PC184 BcenP_01_142, whole genome shotgun sequence**
ATGAACGCACCGATCCCCGCTACGGCGTTGCACGCGACGCCCTGGACCGCGACCGAATTCGAGGCGC
GCCTGCGTGCGCTCGAATCGCGCTATCACATTCATCATCCGTTCAACCGGCGGCTCAACAGCGGCGC
CTGTTCGCCCACGCAGATCCGCGGCTGGGTCGCGAACCGCTTCTACTACCAGATCTGCATCCCGCTC
AAGGATGCGGCGATCCTGTCGAACTGCCCCGACCGCGAGACGCGACGCCGCTGGATTCAGCGGCTGA
TCGACCACGACGGGCAGGGCGACAACGCAGGCGGCATCGAGGCGTGGGTGCGGCTCGGCGAAGCGTG
CGGCCTCACGCGCGACGAGCTGTGGTCGCTCGAACACGTGCTGCCCGGCGTGCGTTTCGCGGTGGAT
GCGTACGTGAACTTCGCGCGGCGCGCGCCGTGGCAGGAAGCCGTGTGCTCGTCGCTGACCGAGATGT
TCGCGCCGCAGATCCATCTCGACCGGCTCGCGGGCTGGCCGTCGCATTACCCGTGGATCGAACCGGC
CGGGCTGCATTACTTCCGCAGCCGCGTGCCGCTCGCGCAGCGCGACGTCGAGCACGGGCTCGCGGTG
ACGCTCGCGCATTTCACGACACCGGAGGCGCAGCAGCGCGCGCTCGGCATTCTGCAATTCAAGCTCG
ACATCCTGTGGTCGATGCTCGACGCCGTCGAAAAGGCCTACCCGTTATGA

**SEQ ID NO: 173, gi|84352661|ref|ZP_00977610.1| COG5424: Pyrroloquinoline quinone (Coenzyme PQQ) biosynthesis protein C [Burkholderia cenocepacia PC184]**
MNAPIPATALHATPWTATEFEARLRALESRYHIHHPFNRRLNSGACSPTQIRGWVANRFYYQICIPL
KDAAILSNCPDRETRRRWIQRLIDHDGQGDNAGGIEAWVRLGEACGLTRDELWSLEHVLPGVRFAVD
AYVNFARRAPWQEAVCSSLTEMFAPQIHLDRLAGWPSHYPWIEPAGLHYFRSRVPLAQRDVEHGLAV
TLAHFTTPEAQQRALGILQFKLDILWSMLDAVEKAYPL

**SEQ ID NO: 174, gi|107025343:324804-325523 Burkholderia cenocepacia AU 1054 chromosome 2, complete sequence**
ATGAACGCACCGATCCCCGCTACGGCGTTGCACGCGACGCCCTGGACCGCGACCGAATTCGAGGCGC
GCCTGCGTGCGCTCGAATCGCGCTATCACATTCATCATCCGTTCAACCGGCGGCTCAACAGCGGCGC
CTGTTCGCCCACGCAGATCCGCGGCTGGGTCGCGAACCGCTTCTACTACCAGATCTGCATCCCGCTC
AAGGATGCGGCGATCCTGTCGAACTGCCCCGACCGCGAGACGCGGCGCCGCTGGATTCAGCGGCTGA
TCGACCACGACGGGCAGGGCGACAACGCAGGCGGCATCGAGGCGTGGGTGCGGCTCGGCGAAGCGTG
CGGCCTGACGCGCGACGAGCTGTGGTCGCTCGAACACGTGCTGCCGGGCGTGCGTTTCGCGGTGGAT
GCGTATGTGAACTTCGCGCGGCGCGCGCCCTGGCAGGAAGCCGTGTGCTCGTCGCTGACCGAGATGT
TCGCGCCGCAGATCCATCTCGACCGGCTCGCGGGCTGGCCGTCGCATTACCCGTGGATCGAACCGGC
CGGGCTGCATTACTTCCGCAGCCGCGTGCCGCTCGCGCAGCGCGACGTCGAGCATGGGCTCGCGGTG
ACGCTCGCGCATTTCACGACACCGGACGCGCAGCAGCGCGCGCTCGGCATTCTGCAATTCAAGCTCG
ACATCCTGTGGTCGATGCTCGACGCCGTCGAAAAGGCCTACCCGTTATGA

**SEQ ID NO: 175, gi|107025604|ref|YP_623115.1| Coenzyme PQQ biosynthesis protein C [Burkholderia cenocepacia AU 1054]**
MNAPIPATALHATPWTATEFEARLRALESRYHIHHPFNRRLNSGACSPTQIRGWVANRFYYQICIPL
KDAAILSNCPDRETRRRWIQRLIDHDGQGDNAGGIEAWVRLGEACGLTRDELWSLEHVLPGVRFAVD
AYVNFARRAPWQEAVCSSLTEMFAPQIHLDRLAGWPSHYPWIEPAGLHYFRSRVPLAQRDVEHGLAV
TLAHFTTPDAQQRALGILQFKLDILWSMLDAVEKAYPL

**SEQ ID NO: 176, gi|118710455:114296-115015 Burkholderia cenocepacia MC0-3 ctg95, whole genome shotgun sequence**
ATGAACGCACCGATCCCCGCTACGGCGTTGCACGCGACGCCCTGGACCGCGACCGAATTCGAGGCGC
GCCTGCGTGCGCTCGAATCGCGCTATCACATCCACCATCCGTTCAACCGGCGGCTCAACAGCGGCGC
TTGTTCGCCCACGCAGATTCGCGGCTGGGTCGCGAACCGTTTCTATTACCAGATCTGCATCCCG

## FIGURE 11 (continued)

CTCAAGGATGCGGCGATCCTGTCGAACTGCCCCGACCGCGACACGCGGCGCCGCTGGATCCAGCGGC
TGATCGACCACGACGGGCAGGGCGACAACGCCGGCGGCATCGAGGCGTGGGTGCGGCTCGGCGAAGC
GTGCGGCCTGGCGCGCGACGAGCTGTGGTCGCTCGAACACGTGCTGCCCGGCGTGCGTTTCGCGGTG
GATGCGTATGTGAACTTCGCGCGGCGCGCGCCCTGGCAGGAAGCCGTGTGCTCGTCGCTGACCGAGA
TGTTCGCGCCGCAGATCCATCTCGACCGGCTCGCGGGCTGGCCGTCGCATTACCCGTGGATCGAGCC
GGCCGGGCTGCATTACTTCCGCAGCCGCGTGCCGCTCGCGCAGCGCGACGTCGAGCATGGGCTCGCG
GTGACGCTCGCGCATTTCACGACACCGGACGCGCAGCAGCGCGCGCTCGGCATTCTGCAATTCAAGC
TCGACATCCTGTGGTCGATGCTCGACGCCGTCGAAAAGGCCTACCCGTTATGA

**SEQ ID NO: 177, gi|118710536|ref|ZP_01563114.1| coenzyme PQQ biosynthesis protein C [Burkholderia cenocepacia MC0-3]**
MNAPIPATALHATPWTATEFEARLRALESRYHIHHPFNRRLNSGACSPTQIRGWVANRFYYQICIPL
KDAAILSNCPDRDTRRRWIQRLIDHDGQGDNAGGIEAWVRLGEACGLARDELWSLEHVLPGVRFAVD
AYVNFARRAPWQEAVCSSLTEMFAPQIHLDRLAGWPSHYPWIEPAGLHYFRSRVPLAQRDVEHGLAV
TLAHFTTPDAQQRALGILQFKLDILWSMLDAVEKAYPL

**SEQ ID NO: 178, gi|71905642:1827642-1828337 Dechloromonas aromatica RCB, complete genome**
ATGATGACCCCGTGGAATCACGCCGAATTCGAGGCGAAACTGCGCGACAAGGGGGCCGCTTACCACA
TCTACCATCCGTTCAACGTCTTGCTGAACACCGGGCAGGCGAAGCGCGAACAGATCCAGGGCTGGGT
GGCCAACCGCTACTATTACCAGATCATCATCCCGGTCAAGGATGCGGCGATCATGTCCAACTGCCCT
GATCGCGAAATACGGCGCGACTGGATTCAGCGCATCATCGACCACGATGGCCGTGGCGACGACGCCG
GTGGTATCGAAGCGTGGATTCGCCTAGGTGAAGCGGTCGGGCTGGCGCGAGAAGAAATCACCGATCT
GCGCCACGTTATTCCAGCCGTGCGCTTTGCCTGTGATGCCTATCTCAACTTCTGTCGCCGGCAGCCG
TGGCAGGAAGCGGTGTGTTCGTCGCTGACCGAGTTGTTCGCGCCGAAGATTCACAAGGAGCGCCTGG
CCAACTGGCCCGAGTTCTACCCATGGATCGAATCGACCGGCCTGCAGTATTTTCGTAACCGGGTTAC
CCAGGCCAGGCGCGACGTCGAGCAGGGGCTGGCGGTTACCCTAGACTACTTCGATACCCCGGCCCTG
CAGGCTCGTGCGCTGGACATCCTGCAGTTCAAGCTCGACATCCTGTGGTCAATGAACGATGCCATGG
CGCTGCGCTATGGCGTCAACAAATGA

**SEQ ID NO: 179, gi|71907330|ref|YP_284917.1| pyrroloquinoline quinone biosynthesis protein PqqC [Dechloromonas aromatica RCB]**
MMTPWNHAEFEAKLRDKGAAYHIYHPFNVLLNTGQAKREQIQGWVANRYYYQIIIPVKDAAIMSNCP
DREIRRDWIQRIIDHDGRGDDAGGIEAWIRLGEAVGLAREEITDLRHVIPAVRFACDAYLNFCRRQP
WQEAVCSSLTELFAPKIHKERLANWPEFYPWIESTGLQYFRNRVTQARRDVEQGLAVTLDYFDTPAL
QARALDILQFKLDILWSMNDAMALRYGVNK

**SEQ ID NO: 180, gi|77163561:2977216-2977938 Nitrosococcus oceani ATCC 19707, complete genome**
GTGGCGGGAAAAAAACCCTGGAGCCGTGAAGAATTCGAGCAGAAGCTGCAGGAAAAAGAATGCTTCT
ACCACATTCACCACCCCTTCCAGGTATTAATGAACAACGGCAAGCTGAACCAGTACCAGGTCCAAGG
ATGGGTAGCCAACCGCTTTTATTACCATACCTGTATCCCTCTCAAGGATGCCGCTATCCTAGCCAAC
TGCTCCCAGCGATCAGTGCGGCGCCAATGGGTACAGCGAATCCTGGATCATGACGGTTACGGCGAAG
ACGTAGGTGGCATCGAAGCCTGGCTTCAGCTAGGCGAAGCCGTGGGCTTAAGCCGGGAAACGCTGGA
ATCCCAGCAACAGGTTCTGCCCGGAGTACGCTTTGCCGTTGACGCTTATGTCAACTTTGCCCGCCAT
GCTACCTGGCAGGAAGCTGTCTGCTCTTCTCTTACGGAGCTCTTTGCCCCTTCGCTCCATCAACAAC
GATTAGACAACTGGCCTCGCTATTACCCTTGGATTAAAGCGGAAGGCTACCATTATTTTCGCAAGCG
CCTCAACGAAGCCCGACGGGATGTTCGACACGGCCTAGAAGCGACCCTGGATTACTTT

**FIGURE 11 (continued)**

CAAAGCCGCACCCAGCAGGAGCAGGCGCTAGCTATCTTACAGTTTAAACTGGATGTACTATGGACCC
TACTGGATGCCCTCTGGATGGCGTACATTGAAAATCGTCCCCCCTATCACACAGAGCTTTAA

**SEQ ID NO: 181, gi|77166079|ref|YP_344604.1| pyrroloquinoline quinone biosynthesis protein PqqC [Nitrosococcus oceani ATCC 19707]**
MAGKKPWSREEFEQKLQEKECFYHIHHPFQVLMNNGKLNQYQVQGWVANRFYYHTCIPLKDAAILAN
CSQRSVRRQWVQRILDHDGYGEDVGGIEAWLQLGEAVGLSRETLESQQQVLPGVRFAVDAYVNFARH
ATWQEAVCSSLTELFAPSLHQQRLDNWPRYYPWIKAEGYHYFRKRLNEARRDVRHGLEATLDYFQSR
TQQEQALAILQFKLDVLWTLLDALWMAYIENRPPYHTEL

**SEQ ID NO: 182, gi|121529016:160482-161195 Ralstonia pickettii 12J ctg54, whole genome shotgun sequence**
TCAGCGAGTGGGATACGCGCGCTCAATCGCGTCAAGCATCGACCAGAGGACATCAAGTTTGAATTGC
AGGATGTCGAGGGCGCGCTCTTGCTGCGCACGTGTCGTGAAGTGTGCCAGTGTCACCTTCAGGCCAT
GTTCGACATCGCGCTGTGCGAGCGTGATCCGGCTGCGGAAGTAGTGCAGACCACCGGCTTCGATCCA
CGGATAATGTTGCGGCCAGTTGGCTAGCCGGTCGCGGTGAATCTTTGGCGCAAACATTTCGGTCAGC
GATGAGCACACCGCCTCTTGCCACGGCACGCTCCGTGCAAAATTCACGTAAGCGTCGACGGCAAAGC
GTACACCCGGCACTACATGCTTGAGCGACCAGAGGTCTTGCTCAGGTATGCCTGCAGCCTCACCCAG
CCGCACCCAGGCCTCGATACCGCCAGGGCTATCAGCATTCCCATCATGATCGAGAATTCTCTGCACC
CAAAGCCGGCGGGTTTCGCGATCCGGGCAATTGGCGATGATGGCGCCATCCTTGCGAGGGATGTTGA
CCTGGTAATAAAAACGGTTGGCGATCCAGCCCCGAACCTGGTCGGGCGTCAGTTCACCATTGTTCAT
GCGCACGTTGAACGGATGATGGATGTGGTAGCCAGCGCCCTTTGCGCGCAACTGCACCTCGAACTCC
TCAGGCGGCCATGCAGCGTCGGGGCTTGCGTTGGTTCGGCTCAA

**SEQ ID NO: 183, gi|121529154|ref|ZP_01661766.1| pyrroloquinoline quinone synthesis C [Ralstonia pickettii 12J]**
MSRTNASPDAAWPPEEFEVQLRAKGAGYHIHHPFNVRMNNGELTPDQVRGWIANRFYYQVNIPRKDG
AIIANCPDRETRRLWVQRILDHDGNADSPGGIEAWVRLGEAAGIPEQDLWSLKHVVPGVRFAVDAYV
NFARSVPWQEAVCSSLTEMFAPKIHRDRLANWPQHYPWIEAGGLHYFRSRITLAQRDVEHGLKVTLA
HFTTRAQQERALDILQFKLDVLWSMLDAIERAYPTR

**SEQ ID NO: 184, gi|119896292:1280664-1281455 Azoarcus sp. BH72, complete genome**
ATGGAAGCCGAACTGCTCGCCGCGCCTGCTGCACGTACGCTTGCCCCCAACGCCGGCACCCACGACC
GCCCGCCGATGTCGCGCGAGGAATTCGAAGCGCAGCTGCGCGCCAAGGGCACCAGCTACCACATCTA
CCACCCCTTCCACGTGATGATGGCCGAAGGGCGCCTGACGCCGGCCCAGCTGCGCGGCTGGGTGGCG
AACCGCTTCTACTACCAGATCGCGATCCCGGTGAAGGACGCGGCCATCATGTCCAACTGCCCGGACC
GCGAGATCCGCCGCGAGTGGATACAGCGCATCCTCGACCACGACGGCTACGAGATCGGCGGTGTGTC
CGACCCCGGCGGCATCGAGGCCTGGATACGGCTGGGCGAGGCGGTGGGGCTGAGCCGCGACGATGTC
ACCTCGCTGCGCTTCGTCGCGCCGGCGGCGCGCTTTGCGGTGGATGCCTACATCAATTTCGCCCGCC
AGCGGCCGTGGGAAGAGGCGGTGGCTTCCAGCCTTACCGAGTTGTTCGCACCCCACATCCACCAGCA
GCGCATCGACACCTGGCCGCAGGTCTATCCCTGGGTGAAGACCGAGGGTTTGCAGTATTTCCGCAAC
CGCCTGACCCAGGCGCGCCGCGACGTCGCCCACGGCCTGCGCTTCACGCTGGAGCACTTCAGCCAGA
CGCGCGCGCTGCAGGAACGCGCGCTGGAGATCCTGCAGTTCAAGCTCGACGTGCTGTGGGCGCTGGC
CGACGCGATCATGCTCAGCCAGTGCGAAATCGAGATCAAGGGGCCGAAGGCATGA

**FIGURE 11 (continued)**

**SEQ ID NO: 185, gi|119897479|ref|YP_932692.1| coenzyme PQQ synthesis protein C [Azoarcus sp. BH72]**
MEAELLAAPAARTLAPNAGTHDRPPMSREEFEAQLRAKGTSYHIYHPFHVMMAEGRLTPAQLRGWVA
NRFYYQIAIPVKDAAIMSNCPDREIRREWIQRILDHDGYEIGGVSDPGGIEAWIRLGEAVGLSRDDV
TSLRFVAPAARFAVDAYINFARQRPWEEAVASSLTELFAPHIHQQRIDTWPQVYPWVKTEGLQYFRN
RLTQARRDVAHGLRFTLEHFSQTRALQERALEILQFKLDVLWALADAIMLSQCEIEIKGPKA

**SEQ ID NO: 186, gi|118717922:201660-202379 Burkholderia multivorans ATCC 17616 ctg35, whole genome shotgun sequence**
TCATGCGCAATACGCCTTTTCAATGGCATCGAGGATCGACCACAGCACGTCGAGCTTGAACGTCAGA
ATCTCGAGCGCGCGCCGTTGCGCGTCCGCGGTCGTGAAATGCGCGAGCGTGACCGCGAGGCCGTGTT
CGACGTCGCGCTGCGCGAGCGGCAGGCGGCTGCGGAAATACTGCAGCCCGTCCGCGTCGATCCACGA
ATAGTGCGACGGCCAGCCGGCGAGACGGTCGAGGTGGATCTGCGGCGCGAACATCTCGGTCAGCGAC
GAGCAGACCGCTTCCTGCCACGTCGCGCGCCGCGCGAAGTTCACGTACGCGTCGACGGCAAAGCGCA
CGCCCGGCAGCACGTGCTCGAGCGACCACAGCGCCGCGCGATCGAGACCGACCGCCTCGCCGAGCCG
CACCCACGCTTCGATGCCGCCGGCATCGTCGCCGTGCCCGTCGTGATCGAGGATGCGCTGCACCCAC
AGCCGGCGCGTGTCGCGGTCCGGACAGTTCGAGAGGATCGCCCGCGTCCTTCAGCGGGATGCTGATCT
GATAGTAGAAGCGGTTCGCGACCCAGCCGCGAATCTGCGCGGGCGTGCACGCGCCCGCATTGAGCCG
CCGGTTGAACGGATGGTGAATGTGATAGCGCGATTCGAGCGCGCGCAGACGCGCCTCGAATTCGTCG
GCGCTCCACGGGCTCGCGTGCAGCGCCGCCGCGGAAATCGGTGCGTTCAT

**SEQ ID NO: 187, gi|118718079|ref|ZP_01570613.1| coenzyme PQQ biosynthesis protein C [Burkholderia multivorans ATCC 17616]**
MNAPISAAALHASPWSADEFEARLRALESRYHIHHPFNRRLNAGACTPAQIRGWVANRFYYQISIPL
KDAAILSNCPDRDTRRLWVQRILDHDGHGDDAGGIEAWVRLGEAVGLDRAALWSLEHVLPGVRFAVD
AYVNFARRATWQEAVCSSLTEMFAPQIHLDRLAGWPSHYSWIDADGLQYFRSRLPLAQRDVEHGLAV
TLAHFTTADAQRRALEILTFKLDVLWSILDAIEKAYCA

**SEQ ID NO: 188, gi|121607004:4422869-4423603 Verminephrobacter eiseniae EF01-2, complete genome**
ATGCCCGCCCCCGTTGCCCCAGCCATTGCGCCCACCGTCCCGCCAGCCATCCCCGACGCCGAGCGCG
CCGCCTTCGAGGCCCGGCTGCGCGCACTGCAAGCGCGCTACCACATCCACCACCCGTTCAACCAACG
CATGGCCAGCGGGCAATGCTCACGCGCCCAGATACAGGGCTGGGTGGCGAACCGCTTTTACTACCAG
GTCAACATTCCGCTCAAGGATGCGGCCATCCTGTCGAACTGCCCCGACCGCGCAACGCGGCGCGAAT
GGGTGGTGCGCATTCTCGACCACGATGGCAGCGCCGCCGAGCCCGGCGGCATCGAAGCCTGGAGCCG
GCTCGGCGAGGCCGTGGGGCTGACGCGCGCGGCCCTGTGGTCACAGCAGATGGTGCTGCCCGGGGTG
CGCTTTGCGGTCGATGCCTACGTCAACTTTGCGCGGCGCGCACCTTGGCAGGAAGCCGTGTGCTCGT
CGCTGACCGAGATGTTCGCGCCCGCCATCCACCAGGAACGGCTGGCCGGCTGGCCGCAGCACTACCC
CTGGATCGAAGCCGAAGGTCTGGCCTATTTCCGTTCACGCATCCCGCTGGCGCAGCGCGATGTCGAG
CATGGCCTGCGCGTCACGCTCGAGCACTTTCGCACGCCGGCCGAGCAGCAGCGCGCCACGCAAATCC
TGCAGTTCAAGCTCGACATCCTGTGGTCGATGCTCGACGCGATCGAAAAGGCGTACCCCGAATGA

**SEQ ID NO: 189, gi|121610952|ref|YP_998759.1| coenzyme PQQ biosynthesis protein C [Verminephrobacter eiseniae EF01-2]**
MPAPVAPAIAPTVPPAIPDAERAAFEARLRALQARYHIHHPFNQRMASGQCSRAQIQGWVANRFYYQ
VNIPLKDAAILSNCPDRATRREWVVRILDHDGSAAEPGGIEAWSRLGEAVGLTRAALWSQQMVLPGV
RFAVDAYVNFARRAPWQEAVCSSLTEMFAPAIHQERLAGWPQHYPWIEAEGLAYFRSRIPLAQRDVE
HGLRVTLEHFRTPAEQQRATQILQFKLDILWSMLDAIEKAYPE

**FIGURE 11 (continued)**

**SEQ ID NO: 190, gi|134290884:1168263-1168982 Burkholderia vietnamiensis G4 chromosome 3, complete sequence**
TCATGCGGGATATGCCTTTTCGATCGCATCGAGAATCTGCCACAGGACATCCAGCTTGAACGACAGG
ATGTCGAGCGCGCGCCGTTGCGCGTCAGGTGTCGTGAAATGACTCAGCGTCACCGCGAGCCCGTGCT
CGACGTCGCGCTGCGCGAGCGGCACGCGGCTGCGGAAATACTGCAGGCCGTCGGCCTCGATCCACGG
GTAGTGCGACGGCCAGCCGGCCAGCCGGTCGAGGTGGATCTGCGGCGCGAACATCTCGGTCAGCGAC
GAGCACACGGCTTCCTGCCACGGCGCGCGACGCGCGAAGTTGACGTACGCATCGACCGCGAAGCGCA
CGCCCGGCAGCACGTGCTCGAGCGACCACAGCTGCGCGCGCTCGATGCCGACCGCCTCGCCGAGCCG
CGCCCACGCCTCGATGCCGCCTTCGCTCTCGCCGTGCCCGTCGTGGTCGAGAATCCGCTCGACCCAC
AGGCGGCGCGTCTCGCGGTCGGGGCAGTTCGACAGGATCGCCGCGTCCTTCAGCGGAATGCTGATCT
GATAGTAGAAGCGGTTCGCGACCCAGCCGCGAATCTGCTCGCGCGAGCATTCGCCGGCATTCATCCG
CCGGTTGAACGGATGATGAATGTGATAGCGCGATTCGAGCGCGCGCAGGCGCGCCTCGAACTCCTGC
GCGCTCCAGGGCGTCGCGTGCAGCGCCGCGGCGGGAATCGGTGCGTTCAT

**SEQ ID NO: 191, gi|134291937|ref|YP_001115706.1| coenzyme PQQ biosynthesis protein C [Burkholderia vietnamiensis G4]**
MNAPIPAAALHATPWSAQEFEARLRALESRYHIHHPFNRRMNAGECSREQIRGWVANRFYYQISIPL
KDAAILSNCPDRETRRLWVERILDHDGHGESEGGIEAWARLGEAVGIERAQLWSLEHVLPGVRFAVD
AYVNFARRAPWQEAVCSSLTEMFAPQIHLDRLAGWPSHYPWIEADGLQYFRSRVPLAQRDVEHGLAV
TLSHFTTPDAQRRALDILSFKLDVLWQILDAIEKAYPA

**SEQ ID NO: 192, gi|118594004:754877-755611 Methylophilales bacterium HTCC2181 1100893002543, whole genome shotgun sequence**
ATGACAAAACCTTGGACAAGAGACGAGTTTGAAGATCGACTAAGAAAGAAGGGTAAGGGGTATCATA
TATACCATCCTTTTCACGTAATGATGTACGAAGGAAAACTTAGCAAAGAACAACTACAAAGCTGGGT
TTTAAACCGTTTTTACTATCAGATAAGCATCCCATTAAAAGATGCGGCAATCTTATCAAATTGCCCC
GTGAAAGAAATCAGAAAAGAATGGATAGTAAGAATATTAGACCATGATGGAGAGGGAGATGCTGACG
GTGGGATTGAGGCATGGATTAATTTAGGTAGAGCAGTCGGACTAACACGAGAGGATGTTACTTCTTT
AAAGCATGTAAGTCCTGGAGTTAAATTTGCCGTAGATGCCTATATAAATTTCGCAAAACAAAAATCG
TGGCAAGAGTCTGTTTGCTCCTCATTAACTGAATTATTTGCACCACACATTCATCAACAAAGAATTA
GCTCATGGCCGTCTATGTACCCATGGATCGATGAATCTGGCCTCACATACTTTAAAAAGCGACTTAC
GGAAGCGAGACGGGATGTGGAGCATGGTTTAGGTGTAACCCTTGATTATTTTAGTAAAACACGTGAA
ACACAAGAGCAAGCTTTAGAAATACTTCAATTTAAACTTAATGTTTTATGGGTTATTGCAGATTCAA
TCATGCTAGCATCATCTAATATTAAGGTAGAAGATCGAGACTACTTAAGGCAACCAATAAACTAA

**SEQ ID NO: 193, gi|118594755|ref|ZP_01552102.1| pyrroloquinoline quinone biosynthesis protein PqqC [Methylophilales bacterium HTCC2181]**
MTKPWTRDEFEDRLRKKGKGYHIYHPFHVMMYEGKLSKEQLQSWVLNRFYYQISIPLKDAAILSNCP
VKEIRKEWIVRILDHDGEGDADGGIEAWINLGRAVGLTREDVTSLKHVSPGVKFAVDAYINFAKQKS
WQESVCSSLTELFAPHIHQQRISSWPSMYPWIDESGLTYFKKRLTEARRDVEHGLGVTLDYFSKTRE
TQEQALEILQFKLNVLWVIADSIMLASSNIKVEDRDYLRQPIN

## FIGURE 11 (continued)

**SEQ ID NO: 194, gi|91774356:1798521-1799273 Methylobacillus flagellatus KT, complete genome**

TTATCGTGGCTGTCTGAGATAGTCGACATGTTGTTCTACCTTGATGTTGGTTGAGGCAAGCATAATG
GCATCGGCAATGACCCACAGCACATCCAGCTTGAATTGCAGAATATCCAGTGCCTTTTCTTGCAATT
CACGGCTCTGGCTGAAGTAATCCAGGGTCACGGCGAGCCCTTGCTCGACATCGCGCCTGGCTTCGGT
CAGGCGCTTCTTGAAATAGGTCAGGCCCTCTTCCTTAACCCAGGGATACATGCTGGGCCAGGAGCTG
ATGCGCTGTTGATGGATGTGCGGCGCAAACAGTTCGGTCAGAGAGGAACAGACGGCCTCCTGCCAAG
GGCGCTGCTTGGCGAAATTGACATAGGCATCCACTGCGAACCTGACACCGGGCGCGACAAACTTGAG
GGACGTCACGTCCTCGCGACTCAGGCCGACTGCCTGGCCGAGCTGTATCCACGCCTCGATGCCACCG
ACGTTATTTGCATCCCCGTCATGGTCAATGATGCGTTGGATCCATCCCTTACGGATTTCCTTGTCCG
GGCAGTTGGACAGAATGGCCGCATCCTTTTGCGGGATGCTGATCTGGTAATAAAAGCGGTTGGCGAC
CCAGCACTGCAATTGCTCGCGGGTGAGCTTGCCTTCATACATCATCACGTGGAACGGGTGGTAGATA
TGGTATCCCCTACCCTTGTCGCGCAGCTTCTGCTCGAACTCTTCGCGTGTCCAGGGAATATTATCTG
TCAGTGGTGCATTCAT

**SEQ ID NO: 195, gi|91776035|ref|YP_545791.1| Coenzyme PQQ biosynthesis protein C [Methylobacillus flagellatus KT]**

MNAPLTDNIPWTREEFEQKLRDKGRGYHIYHPFHVMMYEGKLTREQLQCWVANRFYYQISIPQKDAA
ILSNCPDKEIRKGWIQRIIDHDGDANNVGGIEAWIQLGQAVGLSREDVTSLKFVAPGVRFAVDAYVN
FAKQRPWQEAVCSSLTELFAPHIHQQRISSWPSMYPWVKEEGLTYFKKRLTEARRDVEQGLAVTLDY
FSQSRELQEKALDILQFKLDVLWVIADAIMLASTNIKVEQHVDYLRQPR

**SEQ ID NO: 196, gi|73539706:1943682-1944374 Ralstonia eutropha JMP134 chromosome 1, complete sequence**

ATGACATATTCCACCGCATCGGGTCCCGCGGAGTTCGAAGCGCGGCTGCGCGCCAAAGAAACCGGCT
ACCACATTCATCACCCGTTCAACCAGCGCATGGCGGCCGGATTGTGTTCGCCGGGACAGATCCGCGC
CTGGGTCGCCAACCGCTTCTACTACCAGGCCAATATCCCGCTGAAGGACGCCGCCATCCTTGCCAAC
TGCCCCGAGCGCGACACGCGCCGCGAGTGGGTGGTACGCATCCTGGACCACGACGGGACTGACACCC
AGCCCGGTGGCATTGAAGCCTGGCTGCGGCTGGGCCAAGCCATAGGGCTCGAGCGCGCGGCATTGCT
TGACCATCGGCACGTGCTCCCAGGGGTGCGCTTTGCCGTCGACGCCTATGTCAACTTTGCGCGGCGA
GCGCCGTGGCAGGAAGCCGTGTGCTCCTCGCTCACCGAGATGTTTGCCCCGGAGATCCACAAGGAGC
GGCTCGCGGGCTGGCCCACGCATTACCCTTGGATCGAGGCCGCCGGGCTCGATTACTTTCGCTCGCG
CATCCCGCTCGCGCAGCGCGACGTCGAGCACGGCCTGCGCGTGACGCTGGGCTACTTCCGTACACCG
GAGCAACAGCAACGCGCGCTCGATATCCTGCAATTCAAGCTCGACGTGCTGTGGTCGATGCTCGATG
CCATCCAGCTAGCCAACCCATGA

**SEQ ID NO: 197, gi|73541469|ref|YP_295989.1| Coenzyme PQQ biosynthesis protein C [Ralstonia eutropha JMP134]**

MTYSTASGPAEFEARLRAKETGYHIHHPFNQRMAAGLCSPGQIRAWVANRFYYQANIPLKDAAILAN
CPERDTRREWVVRILDHDGTDTQPGGIEAWLRLGQAIGLERAALLDHRHVLPGVRFAVDAYVNFARR
APWQEAVCSSLTEMFAPEIHKERLAGWPTHYPWIEAAGLDYFRSRIPLAQRDVEHGLRVTLGYFRTP
EQQQRALDILQFKLDVLWSMLDAIQLANP

FIGURE 11 (continued)

**SEQ ID NO: 198, gi|118697979:192397-193116 Burkholderia ambifaria MC40-6 ctg220, whole genome shotgun sequence**
ATGAACGCACCGATTCCCGCCGCGGCGCTGCACGCGACGCCCTGGAGTGCGCAGGAGTTCGAGGCGC
GTCTGCGCGCGCTCGAATCGCGCTACCACATCCATCACCCGTTCAACCGGCGCCTGAATACCGGCGC
GTGCTCGCCCGAGCAGATCCGCGGCTGGGTCGCGAACCGCTTCTACTACCAGATCAGCATTCCGCTG
AAGGACGCGGCGATCCTGTCCAATTGCCCGGACCGCGATACGCGCCGACTGTGGGTCGAGCGGATCC
TCGATCACGATGGTCACGGCGGGCAGGAAGGCGGGATCGAGGCATGGGCGCGCCTCGGCGAAGCGGT
CGGCATCGAGCGCGCGGAATTGTGGTCGCTCGTGCACGTGCTGCCCGGCGTGCGGTTCGCGGTCGAC
GCGTACGTGAATTTCGCGCGGCATGCGCCATGGCAGGAAGCCGTGTGCTCGTCGCTGACCGAGATGT
TCGCGCCACAGATCCATCTCGATCGGCTCGCGGGGTGGCCGTCCCACTATCCGTGGATCGAGGCGGA
CGGGCTGCAGTATTTCCGCAGCCGCGTGCCGCTCGCGCAGCGCGACGTCGAGCACGGGCTCGCGGTC
ACGCTGAACCATTTCACGACACCGGACGCGCAACGGCGCGCGCTCGACATCCTGTCGTTCAAGCTGG
ATGTGCTGTGGTCGATTCTCGACGCGATTGAAAAGGCCTACCCCGCATGA

**SEQ ID NO: 199, gi|118698279|ref|ZP_01556356.1| coenzyme PQQ biosynthesis protein C [Burkholderia ambifaria MC40-6]**
MNAPIPAAALHATPWSAQEFEARLRALESRYHIHHPFNRRLNTGACSPEQIRGWVANRFYYQISIPL
KDAAILSNCPDRDTRRLWVERILDHDGHGGQEGGIEAWARLGEAVGIERAELWSLVHVLPGVRFAVD
AYVNFARHAPWQEAVCSSLTEMFAPQIHLDRLAGWPSHYPWIEADGLQYFRSRVPLAQRDVEHGLAV
TLNHFTTPDAQRRALDILSFKLDVLWSILDAIEKAYPA

**SEQ ID NO: 200, gi|116693960:1186367-1187077 Ralstonia eutropha H16 chromosome 2, complete sequence**
TCATGGGTTGGCTTGCTGGATGGCGTCGAGCATCGACCAGAGCACGTCGAGCTTGAACTGCAGGATG
TCGAGGGCGCGCTGCTGTTGCGCCGCGCTGCGGAAATGGCCCAGCGTCACGCGCAGGCCATGGTCCA
CGTCGCGCTGCGCGAGCGGAATGCGAGAGCGGAAGTAGTCCAGCCCGGCGGCCTCGATCCACGGGTA
ATGCGTGGGCCAGCCGGCCAGGCGTTCCTTGTGGATTGCGGGCGCGAACATCTCCGTCAGCGACGAG
CACACGGCTTCCTGCCACGGTGCGCGGCGCGCAAAGTTGACGTAGGCATCGACGGCAAAGCGCACCC
CGGGCAGCACGTGCCGGTGGCTCAGCAGTGCTTCGCGCTCCAGGCCCACCGCCTGGCCCAGGCGCAG
CCAGGCCTCGATGCCGCCGGGCTGGGTGTCGGTGCCGTCGTGGTCAAGGATGCGCACCACCCACTCG
CGGCGGGTGTCGCGCTCGGGGCAATTGGACAGGATGGCGGCGTCCTTCAGCGGGATGTTGTCCTGAT
AGTAGAAGCGGTTGGCCACCCATGCGCGGATCTGCGCGGGCGAACACTGGCCTGCCGCCATGCGCTG
GTTGAAGGGATGGTGGATGTGGTAGCCGCTTTCCATGGCGCGCAGGCGGGCCTCGAACTCGGCCTCG
GTCCAGGCGATGGGGGGGATGGGGAGGATGGCAGTTGTCAT

**SEQ ID NO: 201, gi|116694996|ref|YP_840572.1| Coenzyme PQQ synthesis protein C [Ralstonia eutropha H16]**
MTTAILPIPPIAWTEAEFEARLRAMESGYHIHHPFNQRMAAGQCSPAQIRAWVANRFYYQDNIPLKD
AAILSNCPERDTRREWVVRILDHDGTDTQPGGIEAWLRLGQAVGLEREALLSHRHVLPGVRFAVDAY
VNFARRAPWQEAVCSSLTEMFAPAIHKERLAGWPTHYPWIEAAGLDYFRSRIPLAQRDVDHGLRVTL
GHFRSAAQQQRALDILQFKLDVLWSMLDAIQQANP

**SEQ ID NO: 202, gi|121527552:341366-342076 Ralstonia pickettii 12J ctg18, whole genome shotgun sequence**
ATGACACATCCTGTTGCCTGGACGGCCGAAGAGTTCGAAGCACGGCTGCGCGCGAAGCAGGCGGGCT
ATCACATTCATCATCCGTTCAACCTGCGCATGCGCGCTGGCGAATGCACGCCGGAGGAAATCCGCAC
GTGGGTTGCGAACCGTTTCTACTACCAGGTCAACATCCCGCTCAAGGATGCGGCCATCCTGTCCAAC
TGCCCCGACCGGGCAACGCGCCGCGAGTGGATCGTCCGCATCCACGACCACGACGGCGACGAT

**FIGURE 11 (continued)**

GCGCAGCCGGGCGGCATCGAGAGCTGGGTGCGCTTAGGCGTTGCCGTGGGCCTGACGCGCGACGCGC
TGTGGTCTCACCGGCATCTGCTGCCGGGCGTGCGCTTTGCGGTGGATGCGTATGTGAACTTTGCGCG
CCGCGCGCCGTGGCAGGAAGCAGTGTGCTCCTCGCTCACGGAGATGTTCGCGCCGGACATTCACAAG
GAACGACTGGCCGGCTGGCCCGAGCACTATCCGTGGGTGCAGCCGGAGGGGCTGGCGTACTTCCGCT
CGCGCATTCCGCTGGCCTCGCGCGACGTCGAGCACGGCCTGCGCGTGACACTCGACTACTTCCGCAC
GCCGGAGCAGCAACTGCGTGCGCTGGACATCCTGCAGTTCAAGCTCGACATCCTCTGGTCGATGCTC
GATGCCATCCAACAGGCCCACGCACATGAGCCCTCCCTTGTCTGA

**SEQ ID NO: 203, gi|121528464|ref|ZP_01661078.1| coenzyme PQQ biosynthesis protein C [Ralstonia pickettii 12J]**
MTHPVAWTAEEFEARLRAKQAGYHIHHPFNLRMRAGECTPEEIRTWVANRFYYQVNIPLKDAAILSN
CPDRATRREWIVRIHDHDGDDAQPGGIESWVRLGVAVGLTRDALWSHRHLLPGVRFAVDAYVNFARR
APWQEAVCSSLTEMFAPDIHKERLAGWPEHYPWVQPEGLAYFRSRIPLASRDVEHGLRVTLDYFRTP
EQQLRALDILQFKLDILWSMLDAIQQAHAHEPSLV

**SEQ ID NO: 204, gi|148259021:374670-375437 Acidiphilium cryptum JF-5, complete genome**
ATGATGAGAGCCGATCTCCTGGCCGAGGCGCCACCAGCCGATGATGGCCGCGCGCCCTGGTCTCACG
CCGAATTCGAGGCGAAGCTGCGCGAGGCCGGCAGCGCCTACCACATCCACCATCCGTTCAACGTGAT
GCTCAACACCGGCAAGGCGACGCCGGAGCAGATCCGCATGTGGGTCGCCAACCGCTTCTATTACCAG
ATCGCGATCCCGGTGAAGGACGCGGCGATCCTCTCCAACTGCCCCGACCGCGAGATCCGCCGCGGCT
GGATCCGCCGCCTCCTCGACCATGACGGGTTCGACTACGAACTGCCCGACGGCTCGCGCCTGCGCGA
CGAGGGCGGCATCGAGGCCTGGCTCCGCCTCGGCATCGCCACCGGCCTCGCGCGCGAGGAGATGCTC
GACCTGCGCCACCTCTTGCCCGGCGTGCGCTTCGCGGTCGACGCCTATGTCAATTTCGCCCGCCGCG
CGCCCTGGCAGGAGGCCGTCTGCTCCTCGCTGACCGAGCTTTTCGCCCCCGACATCCACCGCCAGCG
CCTCGCCACCTGGCCCGGCCACTATCCCTGGATCGAGAGCGAGGGGCTCGACTACTTCCGCAACCGC
ACCAGCCAGGCCCCGCGCGACGTCGTCCACGGCCTGCGGATCACGCTCGCCCATTTCGCGACCCGGC
CGATGCAGGAGCGCGCCCTGCAGATCCTCAAGTTCAAGCTGGACATTCTGTGGACGATGAACGACGA
GATGGGACGACATTACGGCGTCGCCGCCTGA

**SEQ ID NO: 205, gi|148259342|ref|YP_001233469.1| coenzyme PQQ biosynthesis protein C [Acidiphilium cryptum JF-5]**
MMRADLLAEAPPADDGRAPWSHAEFEAKLREAGSAYHIHHPFNVMLNTGKATPEQIRMWVANRFYYQ
IAIPVKDAAILSNCPDREIRRGWIRRLLDHDGFDYELPDGSRLRDEGGIEAWLRLGIATGLAREEML
DLRHLLPGVRFAVDAYVNFARRAPWQEAVCSSLTELFAPDIHRQRLATWPGHYPWIESEGLDYFRNR
TSQAPRDVVHGLRITLAHFATRPMQERALQILKFKLDILWTMNDEMGRHYGVAA

**SEQ ID NO: 206, gi|84358445:206-934 Burkholderia dolosa AUO158 BdolA_01_206, whole genome shotgun sequence**
ATGACCACGATGAACGCACCGCTTCCCGCTACCGCGCTGCACGCGACGCCCTGGAGCGCGCAGGAGT
TCGAGGCGCACCTGCGCGCGCTCGAATCGCGCTATCACATTCATCATCCGTTCAACCGGCGGCTGAA
CTCGGGCGCGTGCTCGCCCGAGCAGATCCGCGGCTGGGTCGCGAACCGCTTCTACTACCAGATCAGC
ATTCCGCTGAAGGACGCGGCGATCCTGTCGAATTGTCCGGACCGCGACACGCGCCGACTGTGGGTGC
AGCGGATTCTCGATCACGACGGACACGGCGGCGACGCAGGCGGAATCGAAGCGTGGGCGCGGCTCGG
CGAAGCGGTCGGCATCCCGCGCTCGCAACTGTGGTCGCTCGAACGCGTGCTGCCCGGCGTGCGCTTC
GCGGTCGATGCGTATGTGAACTTCGCGCGCCGCGCGCCGTGGCAGGAAGCCGTGTGCTCGTCGCTGA
CGGAGATGTTCGCGCCGCAGATCCACCTCGACCGGCTCGCGGGCTGGCCGTCGCACTAC

**FIGURE 11 (continued)**

CCGTGGATCGACGCGGACGGGCTGCAGTATTTCCGCAGCCGCGTGCCGCTCGCGCAGCGCGACGTCG
AGCACGGGCTCGCGGTGACGCTGCGCCATTTCACGACACCGGAAGCGCAACGACGAGCGCTCGACAT
CCTGTCGTTCAAGCTGGATGTCCTGTGGTCGATCCTCGATGCAATCGAAAAGGCGTATCCCGCATGA

**SEQ ID NO: 207, gi|84358446|ref|ZP_00983225.1| COG5424: Pyrroloquinoline quinone (Coenzyme PQQ) biosynthesis protein C [Burkholderia dolosa AUO158]**

MTTMNAPLPATALHATPWSAQEFEAHLRALESRYHIHHPFNRRLNSGACSPEQIRGWVANRFYYQIS
IPLKDAAILSNCPDRDTRRLWVQRILDHDGHGGDAGGIEAWARLGEAVGIPRSQLWSLERVLPGVRF
AVDAYVNFARRAPWQEAVCSSLTEMFAPQIHLDRLAGWPSHYPWIDADGLQYFRSRVPLAQRDVEHG
LAVTLRHFTTPEAQRRALDILSFKLDVLWSILDAIEKAYPA

**SEQ ID NO: 208, gi|84788940:137239-137958 Burkholderia dolosa AUO158 supercont1.3 genomic scaffold, whole genome shotgun sequence**

ATGAACGCACCGCTTCCCGCTACCGCGCTGCACGCGACGCCCTGGAGCGCGCAGGAGTTCGAGGCGC
ACCTGCGCGCGCTCGAATCGCGCTATCACATTCATCATCCGTTCAACCGGCGGCTGAACTCGGGCGC
GTGCTCGCCCGAGCAGATCCGCGGCTGGGTCGCGAACCGCTTCTACTACCAGATCAGCATTCCGCTG
AAGGACGCGGCGATCCTGTCGAATTGTCCGGACCGCGACACGCGCCGACTGTGGGTGCAGCGGATTC
TCGATCACGACGGACACGGCGGCGACGCAGGCGGAATCGAAGCGTGGGCGCGGCTCGGCGAAGCGGT
CGGCATCCCGCGCTCGCAACTGTGGTCGCTCGAACGCGTGCTGCCCGGCGTGCGCTTCGCGGTCGAT
GCGTATGTGAACTTCGCGCGCCCGCGCGCCGTGGCAGGAAGCCGTGTGCTCGTCGCTGACGGAGATGT
TCGCGCCGCAGATCCACCTCGACCGGCTCGCGGGCTGGCCGTCGCACTACCCGTGGATCGACGCGGA
CGGGCTGCAGTATTTCCGCAGCCGCGTGCCGCTCGCGCAGCGCGACGTCGAGCACGGGCTCGCGGTG
ACGCTGCGCCATTTCACGACACCGGAAGCGCAACGACGAGCGCTCGACATCCTGTCGTTCAAGCTGG
ATGTCCTGTGGTCGATCCTCGATGCAATCGAAAAGGCGTATCCCGCATGA

**SEQ ID NO: 209, gi|124900961|gb|EAY71711.1| Pyrroloquinoline quinone (Coenzyme PQQ) biosynthesis protein C [Burkholderia dolosa AUO158]**

MNAPLPATALHATPWSAQEFEAHLRALESRYHIHHPFNRRLNSGACSPEQIRGWVANRFYYQISIPL
KDAAILSNCPDRDTRRLWVQRILDHDGHGGDAGGIEAWARLGEAVGIPRSQLWSLERVLPGVRFAVD
AYVNFARRAPWQEAVCSSLTEMFAPQIHLDRLAGWPSHYPWIDADGLQYFRSRVPLAQRDVEHGLAV
TLRHFTTPEAQRRALDILSFKLDVLWSILDAIEKAYPA

**SEQ ID NO: 210, gi|121602919:771052-771783 Polaromonas naphthalenivorans CJ2, complete genome**

TCATACGCAGGCTTTCTCGATGCCGTCGAGCATGCTCCACAGGATGTCGAGCTTGAACTGCAGGATG
TCCAGCGCCCTGTGCTGCGCCTGGCGGGTGGTGAAATGGGTGATGGTGACCGCCAGCCCGTGTTCCA
CATCGCGGCTTGCCAGCGGAATACGGCTGCGGAAATAAGTCAGGCCTTCGGGCTCGATCCAGGCGTA
ATGCAAAGGCCAGCTGGCAAGCCGGTCCTTGTGGATCTGGGGTGCGAACATTTCGGTCAGCGATGAG
CACACGGCTTCCTGCCACGGTGCTTTCGCGGCGAAATTCACGTAGGCATCGCAGGCGAACCGCACCG
CCGGCGCCACGCCCTGCAACGACCACAATTCCTCACGCGACAGGCCGACCGCCAGGCCCAACTGGGT
CCAGGCTTCCATGCCGCCAGCGGCGCAGCCCTGGTAGTCGCCGTAGCCGTCATGGTCCAGAATGCGT
TCCACCCACAGTCGGCGGTGCGCCCGGTCAGGCATGTTGGCCAAAATCGCCGCGTCCTTGCGCGGAA
TGCACAACTGGTAATAAAAACGGTTGGCCACCCAGCAACGGATTTCGTCTGCGGTGCAGCCGCCCTG
GTTCATCCGTACATTGAAGGGATGGTGAATGTGATAGGCCGTGCCTTTGGCGCGCAGCTGCCTTTCA
AACTCGGCAGGGCTCCAGGCTGATCTGCTGGCGTCAAAGCAGGGTGATTGGGCTATTTCCAT

**FIGURE 11 (continued)**

**SEQ ID NO: 211, gi|121603642|ref|YP_980971.1| coenzyme PQQ biosynthesis protein C [Polaromonas naphthalenivorans CJ2]**
MEIAQSPCFDASRSAWSPAEFERQLRAKGTAYHIHHPFNVRMNQGGCTADEIRCWVANRFYYQLCIP
RKDAAILANMPDRAHRRLWVERILDHDGYGDYQGCAAGGMEAWTQLGLAVGLSREELWSLQGVAPAV
RFACDAYVNFAAKAPWQEAVCSSLTEMFAPQIHKDRLASWPLHYAWIEPEGLTYFRSRIPLASRDVE
HGLAVTITHFTTRQAQHRALDILQFKLDILWSMLDGIEKACV

**SEQ ID NO: 212, gi|94493478:342439-343179 Rickettsiella grylli Rgryl_01_1, whole genome shotgun sequence**
ATGCCGACTAAACGCTGCGAAGTATCATTGTCTCAGAGTGTATTTGAAAAAAAAATTTTAAACCTTG
AAAAATATTATCATATCCATCATCCTTTCAATCAATTAATGAACGATGGCCAATTGACTCGGCAACA
AATTCAACAGTGGGTGATTAATCGCTTCTATTATCAAGTCAATATCCCTGTAAAAGATGCTGCTATT
TTATCCAATTGTCCTCTACGGGAGATACGAAGAGAATGGGTGAAACGGATCATTGAACACGATGGCG
ATGTCATTAATCCCGGTGGAATTGAAGCCTGGCTGACATTGGGAGAAGCGTGCGGACTGACGCGTAA
CTTATTGTGGTCTATGAAATATGTCCTGCCGGGCGTGCGCTTTGCAGTAGACGCTTACATTCAGTTT
ACACGAACGCATCCATGGCAAGCTGCGGTTTGTTCATCGTTAACGGAGCTTTTTGCACCCGAAATCC
ATCGCCAACGTTTAACGAATTGGCCCATCCATTATCCATGGATGGATTTAAACGCATTAGCTTATTT
TCGTTGTCGATTAAATCAAATCAATCATCATGTCAAAAAAGGCTTACAATGGACACTCGAAAATTTT
CAAACACACGAACAGCAAACACAAGCATTGGCTATTTTAAAATTTAAATTAGAAATTCTTTGGTCAA
TTTCAGACAGCCTTTACTTAGCCTATGTATTAGGTCACCCTCCCATCACATTTTCAAAGGATGATAC
ATGA

**SEQ ID NO: 213, gi|94493803|ref|ZP_01301006.1| hypothetical protein Rgryl_01000329 [Rickettsiella grylli]**
MPTKRCEVSLSQSVFEKKILNLEKYYHIHHPFNQLMNDGQLTRQQIQQWVINRFYYQVNIPVKDAAI
LSNCPLREIRREWVKRIIEHDGDVINPGGIEAWLTLGEACGLTRNLLWSMKYVLPGVRFAVDAYIQF
TRTHPWQAAVCSSLTELFAPEIHRQRLTNWPIHYPWMDLNALAYFRCRLNQINHHVKKGLQWTLENF
QTHEQQTQALAILKFKLEILWSISDSLYLAYVLGHPPITFSKDDT

**SEQ ID NO: 214, gi|124265193:2760495-2761226 Methylibium petroleiphilum PM1, complete genome**
TTGAGCGCCGACCTGCCCTGGTCTCCCGCAGAATTCGAGGCACGCCTGCGCGCCAAGGAGTCCGGGT
ACCACATCCACCACCCCTTCAACAAGCGGCTCAACAGCGGCGCGCTGCAGCCCTTCCAGGTGCGCGG
CTGGGTGGCCAACCGCTTCTACTACCAGCAGGCGATCCCGATGAAGGACGCCGCGGTCATGGCCAAC
TGCGAGGACCGCGCCACGCGGCGCCGCTGGATCGAGCGCATGCTCGACCACGACGGCCATGGCGATC
ACGAGGGGCAGAACGCCGGCGGTATCGAGACCTGGACGCGCCTGGGCATGGCCGTGGGCCTGAGCCG
CGAGGACCTCTGGTCGCACCGCCACGTTCAGCCGGGCGTGCGCTTCGCGGTCGATGCCTACGTGAAC
TTCGCGCGCCGCGCGCCCTGGCGCGAGGGCGTGATCTCCTCGCTGACCGAGATGTTCGCGCCCAAGA
TCCACGCCGACCGGCTGGCCGGCTGGCCCAGCATGTACCCGTGGATCGCCGCCGAGGGCCTGGCCTA
TTTCCGCAGCCGCATCCCGCTGGCGCAGCGCGACGTGGAGCACGGCCTGGAGGTCGCCATCGCGTTC
GGCGACACCCGCGCGAAGCAGGAGCGCGCGATCGAGATCCTGCAGTTCAAGCTCGACGTGCTGTGGT
CGCTGCTCGACGCCGTGGAGCGCGCCTACCCCGACGACCTGCCCGAGGAGCGCCGACCGTGA

**SEQ ID NO: 215, gi|124267771|ref|YP_001021775.1| coenzyme PQQ synthesis protein C [Methylibium petroleiphilum PM1]**
MSADLPWSPAEFEARLRAKESGYHIHHPFNKRLNSGALQPFQVRGWVANRFYYQQAIPMKDAAVMAN
CEDRATRRRWIERMLDHDGHGDHEGQNAGGIETWTRLGMAVGLSREDLWSHRHVQPGVRFAVDAYVN
FARRAPWREGVISSLTEMFAPKIHADRLAGWPSMYPWIAAEGLAYFRSRIPLAQRDVEHGLEVAIAF
GDTRAKQERAIEILQFKLDVLWSLLDAVERAYPDDLPEERRP

**FIGURE 11 (continued)**

**SEQ ID NO: 216, gi|88811804:230426-231064 Nitrococcus mobilis Nb-231 1099646003868, whole genome shotgun sequence**
ATGATGATGAATCGCGGCGAACTCTCGCGACACCAACTCCAGGGTTGGGTTGCCAACCGCTATTATT
ACCAAATCAGCATCCCACTGAAAGACGCGGCACTGATCTCCCAATGTCCGGATCGGGCCGTGCGGCG
GCACTGGCTGCACAGGCTCCTCGATCAGGACGGCCGCACGGGCAGCGAAGGGGGGATCGAAGCATGG
ATCAACCTCGGCGAGGCCGTCGGCCTGAGCCGGACAGAGCTGATCTCCGAGCGTCAGGTTTTGCCTG
GTGTTCGATTCGCGGTGGACGCCTATGTAAATTTGGTGCGCTGGGGGTCTTGGCAGGACGGGGTGTG
CGCCTCGCTCACCGAGCTGTTCGCGCCGGGCATCCATCGCGAACGTCTGAACAGCTGGCCGACTCAC
TATCCCTGGGTCGATCCCGCCGGCTTAGCCTATTTTCGCAGCCGCCTCGAAGTAGCCCGGCAAGACG
GGCGGCACGGACTCGCTTTGGTACTCGATCATTGCGACACCCCGGCAAGCCAGGATCGCGCGGTGCG
CATCGTCAAGTTCAAACTCGAAGTTCTCTGGGCCATGCTCGATGCGATGTACCTGGCCTATGTGGTA
GGCATGCCGCCGTTTTTTAACGTCGCGGGCGACTGA

**SEQ ID NO: 217, gi|88812029|ref|ZP_01127282.1| pyrroloquinoline quinone biosynthesis protein PqqC [Nitrococcus mobilis Nb-231]**
MMMNRGELSRHQLQGWVANRYYYQISIPLKDAALISQCPDRAVRRHWLHRLLDQDGRTGSEGGIEAW
INLGEAVGLSRTELISERQVLPGVRFAVDAYVNLVRWGSWQDGVCASLTELFAPGIHRERLNSWPTH
YPWVDPAGLAYFRSRLEVARQDGRHGLALVLDHCDTPASQDRAVRIVKFKLEVLWAMLDAMYLAYVV
GMPPFFNVAGD

**SEQ ID NO: 218, gi|118467340:3790446-3791162 Mycobacterium smegmatis str. MC2 155, complete genome**
TCAGCGATTTCCATAAGCCTGGTCGATCGCATCGAGCATGCTCCACAGCACGTCGCACTTGAAGGAC
AAGGCCGACAGCGCGGCCGTCTGGGTTTCCTGCGTGACGCAATGCCGTCGCACCACCTCAAGACCGT
GTTCGGAGTCGCGGGGCGCCTGGGTGATGCGAGATCTGAAGTAAGAGAGTTCTTTCGGGTCGATCCA
GGTGTAGTGACGTTCGAACGCCGCGAGCCGCTCGGCCATCAGGTCGGGTGCGAACAGCTCGGTCAGC
GACGAGGCCACCGCCTCCAGCCATGGCCGGGTGCGGGTGAAGTTCACGTAGGCGTCGACGGCGAAGC
GCACGCCCGGCAGCAGGTGTCGTGCGTCTTCGACCTCCGCCCTGGTCAAACCGACCGCTTCGGCCAG
TCGCAGCCACGCCTCGATTCCCCCGGTTCCTTCGGTCGTGCCGTCGTGGTCGATGATTCGCTGGACC
CAACGGCGCCGTATCTCACGGTCCGGGCAGGTGCTGAGGATCGCTGCGTCTTTCCGGGGGATGTTCA
CCTGGTAGTAGAAGCGGTTGGCCACCCACCCGCGGATCTCCTCGGGGCTGCACTGCCCGGTGTTCAT
CCGACGGTGGAAGGGATGCAGGTGGTGGTAATCCTTGACGTGCTCGCGAAGCGCTTCCTCGAACTCG
TCGGGCGTCAGTGCCTGGGTTACTGCTCCTCGCGCCAGCGGTGCCAT

**SEQ ID NO: 219, gi|118470311|ref|YP_888023.1| coenzyme PQQ biosynthesis protein C [Mycobacterium smegmatis str. MC2 155]**
MAPLARGAVTQALTPDEFEEALREHVKDYHHLHPFHRRMNTGQCSPEEIRGWVANRFYYQVNIPRKD
AAILSTCPDREIRRRWVQRIIDHDGTTEGTGGIEAWLRLAEAVGLTRAEVEDARHLLPGVRFAVDAY
VNFTRTRPWLEAVASSLTELFAPDLMAERLAAFERHYTWIDPKELSYFRSRITQAPRDSEHGLEVVR
RHCVTQETQTAALSALSFKCDVLWSMLDAIDQAYGNR

**SEQ ID NO: 220, gi|134096620:4972818-4973507 Saccharopolyspora erythraea NRRL 2338, complete genome**
TCAGTCGGCGTACGCGTGGTCGATGGCATCGAGAATGCTCCACAGGACATCGCATTTGAACGACAGG
GCCGCCACGGCTCGTGCCTGCTCGTCGGCCGACCGGCAGTGTTCGGTCACCACCTGCAGGGCGTGTT
CGGAGTCGCGGGGAGCCTGCTCCAGGCGGGCCCGGAAGTAACCGAGACCGCCGGGGTCGATCCACGG
ATAGCAGCGCTCGAACGCGGCGAGGCGCTGCGCCATCAGGTCCGGTGCGAACAGCTCGGTCAACGAC
GACGCCACCGCCTCGGTCCACGGCCGGGTGCGGGCGAAGGTGACGTAGGCGTCGACGGCGAA

## FIGURE 11 (continued)

CCGCACGCCGGGGACGACATGCCGTTCGTCGAGCACTTCCTCGCGGGTCAGGCCGACCGCCTCGGCC
AGGTGCAACCAGGCGTCGATACCGCCTCGCCCGCCCGGCGCGCCGTCGTGGTCGAGGATCCGCCGCA
CCCAGCGCCTGCGCACCTCGCGGTCGGGGCAGTTGGACAGGATCGCGGCGTCCTTGAGCGGGATGCT
CTCCTGGTAGTAGAAGCGGTTGGCCACCCACCCCTGGATCTGGCGGCGGCTCAGCCGTCCCCCGTTC
ATCCTGACGTGGAACGGGTGCTGGTCGTGGTAGCGCCGCGACTGCGCGCGCAACGCCGCGACGAAGC
CGTCGGTGCCGGTGAGCGCCGCCAT

**SEQ ID NO: 221, gi|134100979|ref|YP_001106640.1| coenzyme PQQ synthesis protein C [Saccharopolyspora erythraea NRRL 2338]**
MAALTGTDGFVAALRAQSRRYHDQHPFHVRMNGGRLSRRQIQGWVANRFYYQESIPLKDAAILSNCP
DREVRRRWVRRILDHDGAPGGRGGIDAWLHLAEAVGLTREEVLDERHVVPGVRFAVDAYVTFARTRP
WTEAVASSLTELFAPDLMAQRLAAFERCYPWIDPGGLGYFRARLEQAPRDSEHALQVVTEHCRSADE
QARAVAALSFKCDVLWSILDAIDHAYAD

**SEQ ID NO: 222, gi|126729493:115566-116324 Sagittula stellata E-37 1101159001454, whole genome shotgun sequence**
ATGACCAAGGCACCGCAATCGCGCGACGCGTTCGAAGCCCGGCTGCGCCAGATCGGAGCGGAGCGAT
ATCACGACCTGCATCCGTTCCACGACCGTCTGCACGGTGGGCAGTGTACCATGGAGGAGGTGCAGGC
CTGGGTCATCAACCGCTACTACTACCAGCACTCGATCCCGATGAAGGACGCGGCCTTCATGTCCCGG
GTGGAGGACCCTGATTTGCGCCGCGCGTGGAGGTCGCGGATGGAGGATCACGACGGAACCGCCGACA
ACGAAGGCGGCATAAGGCGCTGGCTGCGGCTGGCGGAGGCGGTGGGTCTGGATCCCGACTACGTCTC
GACGACAGAGGGCGTCCTGCCCGCAACGAAGTTCGCGGTGGACGCCTACGTCCGTTTCGTCCGCGAA
AAGACCCTGCTCGAAGCGGTGGCGGCATCACTCACGGAACTCTTCGCACCGAAGATCCACGCCAACC
GCATCGAGGGACTGCTGAAGAATTACGCCTTCGCCGACGACTCCTCGCTGTCGTATTTCCGCAACCG
GTTGAAAGAAGCGCCGAAGGATGTGGCCTTTGGGCTGGCCTGGGTGCTGGACCATGCCGACACCGCC
GAGAAGCAGGACGTGGCGGCCAGGGCGCTGATCTTCAAGACGGATGTTCTGTGGTCCCAACTCGACG
CGCTGTGGGGCGCCTACGTCGAGCCGCGGCGCATTCCGCCCGGCGCGTGGCAGCCCGGAACGGGGCA
GCTCTTGCGGCAGGCGTCATGA

**SEQ ID NO: 223, gi|126729612|ref|ZP_01745425.1| pyrroloquinoline quinone biosynthesis protein PqqC [Sagittula stellata E-37]**
MTKAPQSRDAFEARLRQIGAERYHDLHPFHDRLHGGQCTMEEVQAWVINRYYYQHSIPMKDAAFMSR
VEDPDLRRAWRSRMEDHDGTADNEGGIRRWLRLAEAVGLDPDYVSTTEGVLPATKFAVDAYVRFVRE
KTLLEAVAASLTELFAPKIHANRIEGLLKNYAFADDSSLSYFRNRLKEAPKDVAFGLAWVLDHADTA
EKQDVAARALIFKTDVLWSQLDALWGAYVEPRRIPPGAWQPGTGQLLRQAS

**SEQ ID NO: 224, gi|16263748:214737-215507 Sinorhizobium meliloti 1021 plasmid pSymB, complete sequence**
GTGACGACGGCAACTGACAGACAAGCTTTCCATGCCCGCCTGCTGGAGATCGGCAAGGAGCGCTACC
ATGACAAGCATCCGTTTCATGCGATGCTCCATGGCGGCGGTTGCACGACGACGCAAGTCCGCGCCTG
GGTGATCAACCGCTATTATTACCAGAGCCGCATCCCCATGAAGGATGCGGCCTTTCTGTCGCGTTGC
GACGACCCGGATATGCGCCGGGCCTGGCGCTCCCGCATCGAAGATCACGACGGCGGCGTCGAGGAGG
GCGGCGGCATCCGGCGCTGGCTGCGTCTCGCCGAGGCCGTGGGACTCGATCCAGCCTATGTCGGCTC
CGCAAGGGGCGTACTGCCGTCGACCCGGTTTGCCGTGGACGCCTATGTTTCCTTTGTGCGTGAGAAG
CCGCTGCTCGAGGCGGTGGCCTCCTCGCTGACGGAGCTTTTTGCGCCGAAGATCCACTCGGAGCGCA
TTGCCGGGCTGCTGGAGCACTACGCCTTCGCCGATGACGCGGCGCTCGCCTACTTCCGCCAGAGGCT
GGCGGAGGTGCCCCGGGATGTCGAGTTCGGCCTCGCCTATGTCCTTGACCATGCCGACACGCGGGAA
AAGCAGGACGCGGCCGCTCAGGCACTCACCTTCAAGACGGATGTTCTCTGGGCCCAGCTCGATGCAC
TTTATTCGGCCTATGTCACGCCGGGGCGCATTCCGCCGGGGGCCTGGGACGGCCGGGAGGGGGTGAT
CCGCGAGCCGAGAGCGCGGGAGGCCGCGGAATGA

**FIGURE 11 (continued)**

**SEQ ID NO: 225, gi|16263947|ref|NP_436739.1| pyrroloquinoline quinone biosynthesis protein PqqC [Sinorhizobium meliloti 1021]**
MTTATDRQAFHARLLEIGKERYHDKHPFHAMLHGGGCTTTQVRAWVINRYYYQSRIPMKDAAFLSRC
DDPDMRRAWRSRIEDHDGGVEEGGGIRRWLRLAEAVGLDPAYVGSARGVLPSTRFAVDAYVSFVREK
PLLEAVASSLTELFAPKIHSERIAGLLEHYAFADDAALAYFRQRLAEVPRDVEFGLAYVLDHADTRE
KQDAAAQALTFKTDVLWAQLDALYSAYVTPGRIPPGAWDGREGVIREPRAREAAE

**SEQ ID NO: 226, *Oryza sativa* - protochlorophylid reductase promoter**
CCCACGCGTCCGCCCACGCGTCCGGGACACCAGAAACATAGTACACTTGAGCTCACTCCAAACTCAA
ACACTCACACCAATGGCTCTCCAAGTTCAGGCCGCACTCCTGCCCTCTGCTCTCTCTGTCCCCAAGA
AGGGTAACTTGAGCGCGGTGGTGAAGGAGCCGGGGTTCCTTAGCGTGAGCAGAAGGCCAAGAAGCCG
TCGCTGGTGGTGAGGGCGGTGGCGACGCGGCGGGCCGGTGGCGAGCCCCGGCGCGGGCACGTCGAAG
GCGGACGGGAAGAAGACGCTGCGGCAGGGGGTGGTGGTGATCACCGGCGCGTCGTCGGGGCTCGGGC
TCGCGGCGGCGAAGGCGCTTGGCGGAGACGGGGAAGTGGCACGTGGTGATGGCGTTCCGCGACTTTC
CTGAAGGCGGCGACGGCGGCGAAGGCGGCGGGGATGGCGGCGGGGAGCTACACCGTCATGCACCTGG
ACCTCGCCTCCCTCGACAGCGTCCGCCAGTTCGTGGACAACTTCCGGCGCTCCGGCATGCCGCTCGA
CGCGCTGGTGTGCAACGCCGCACATCTACCGGCCGACGGCGCGGCAACCGACGTTCAACGCCGACGG
GTACGAGATGAGCGTCGGGGTGAACCACCTGGGCCACTTCCTCCTCGCCCGCCTCATGCTCGACGAC
CTCAAGAAATCCGACTACCCGTCGCGGCGGCTCATCATCCTCGGCTCCATCACCGGCAACACCAACA
CCTTCGCCGGCAACGTCCCTCCCAAGGCCGGGCTAGGCGACCTCCGGGGGCTCGCCGGCGGGCTCCG
CGGGCAGAACGGGTCGGCGATGATCGACGGCGCGGAGAGCTTCGACGGCGCCAAGGCGTACAAGGAC
AGCAAGATCTGTAACATGCTGACGATGCAGGAGTTCCACCGGAGATTCCACGAGGAGACCGGGATCA
CGTTCGCGTCGCTGTACCCGGGGTGCATCGCGACGACGGGCTTGTTCCGCGAGCACATCCCGCTGTT
CCGGCTGCTGTTCCCGCCGTTCCAGCGGTTCGTGACGAAGGGGTTCGTGTCGGAGGCGGAGTCCGGG
AAGCGGCTGGCGCAGGTGGTGGGCGACCCGAGCCTGACCAAGTCCGGCGTGTACTGGAGCTGGAACA
AGGACTCGGCGTCGTTCGAGAACCAGCTCTCGCAGGAGGCCAGCGACCCGGAGAAGGCCAGGAAGCT
CTGGGACCTCAGCGAGAAGCTCGTCGGCCTCGTCTGAGTTTATTATTTACCCATTCGTTTCAACTGT
TAATTTCTTCGGGGTTTAGGGGGTTTCAGCTTTCAGTGAGAGAGGCCTGTCAAGTGATGTACAATTA
GTAATTTTTTTTTACCCGACAAATCATGCAATAAAACCACAGGCTTACATTATCGATTTGTCCACCT
AAATTAAGTTTCAACTGTTAATTTCTTCGGGGTTTAGGGGGTTTCAGCTTTCAGTGAGAGAGGCCTG
TCAAGTGATGTACAATTAGTAATTTTTTTTTACCCGACAAATCATGCAATAAAACCACAGGCTTACA
TTATCGATTTGTCCACCTAAATTAAGT

**SEQ ID NO: 227, *Oryza sativa* - GOS2 promoter**
AATCCGAAAAGTTTCTGCACCGTTTTCACCCCCTAACTAACAATATAGGGAACGTGTGCTAAATATA
AAATGAGACCTTATATATGTAGCGCTGATAACTAGAACTATGCAAGAAAAACTCATCCACCTACTTT
AGTGGCAATCGGGCTAAATAAAAAAGAGTCGCTACACTAGTTTCGTTTTCCTTAGTAATTAAGTGGG
AAAATGAAATCATTATTGCTTAGAATATACGTTCACATCTCTGTCATGAAGTTAAATTATTCGAGGT
AGCCATAATTGTCATCAAACTCTTCTTGAATAAAAAAATCTTTCTAGCTGAACTCAATGGGTAAAGA
GAGAGATTTTTTTTAAAAAAAATAGAATGAAGATATTCTGAACGTATTGGCAAAGATTTAAACATATA
ATTATATAATTTTATAGTTTGTGCATTCGTCATATCGCACATCATTAAGGACATGTCTTACTCCATC
CCAATTTTTATTTAGTAATTAAAGACAATTGACTTATTTTTATTATTTATCTTTTTTCGATTAGATG
CAAGGTACTTACGCACACACTTTGTGCTCATGTGCATGTGTGAGTGCACCTCCTCAATACACGTTCA
ACTAGCAACACATCTCTAATATCACTCGCCTATTTAATACATTTAGGTAGCAATATCTGAATTCAAG
CACTCCACCATCACCAGACCACTTTTAATAATATCTAAAATACAAAAAATAATTTTACAGAATAGCA
TGAAAAGTATGAAACGAACTATTTAGGTTTTTCACATACAAAAAAAAAAAGAATTTTGCTCGTGCGC
GAGCGCCAATCTCCCATATTGGGCACACAGGCAACAACAGAGTGGCTGCCCACA

**FIGURE 11 (continued)**

GAACAACCCACAAAAAACGATGATCTAACGGAGGACAGCAAGTCCGCAACAACCTTTTAACAGCAGG
CTTTGCGGCCAGGAGAGAGGAGGAGAGGCAAAGAAAACCAAGCATCCTCCTTCTCCCATCTATAAAT
TCCTCCCCCCTTTTCCCCTCTCTATATAGGAGGCATCCAAGCCAAGAAGAGGGAGAGCACCAAGGAC
ACGCGACTAGCAGAAGCCGAGCGACCGCCTTCTCGATCCATATCTTCCGGTCGAGTTCTTGGTCGAT
CTCTTCCCTCCTCCACCTCCTCCTCACAGGGTATGTGCCTCCCTTCGGTTGTTCTTGGATTTATTGT
TCTAGGTTGTGTAGTACGGGCGTTGATGTTAGGAAAGGGGATCTGTATCTGTGATGATTCCTGTTCT
TGGATTTGGGATAGAGGGGTTCTTGATGTTGCATGTTATCGGTTCGGTTTGATTAGTAGTATGGTTT
TCAATCGTCTGGAGAGCTCTATGGAAATGAAATGGTTTAGGGATCGGAATCTTGCGATTTTGTGAGT
ACCTTTTGTTTGAGGTAAAATCAGAGCACCGGTGATTTTGCTTGGTGTAATAAAGTACGGTTGTTTG
GTCCTCGATTCTGGTAGTGATGCTTCTCGATTTGACGAAGCTATCCTTTGTTTATTCCCTATTGAAC
AAAAATAATCCAACTTTGAAGACGGTCCCGTTGATGAGATTGAATGATTGATTCTTAAGCCTGTCCA
AAATTTCGCAGCTGGCTTGTTTAGATACAGTAGTCCCCATCACGAAATTCATGGAAACAGTTATAAT
CCTCAGGAACAGGGGATTCCCTGTTCTTCCGATTTGCTTTAGTCCCAGAATTTTTTTTCCCAAATAT
CTTAAAAAGTCACTTTCTGGTTCAGTTCAATGAATTGATTGCTACAAATAATGCTTTTATAGCGTTA
TCCTAGCTGTAGTTCAGTTAATAGGTAATACCCCTATAGTTTAGTCAGGAGAAGAACTTATCCGATT
TCTGATCTCCATTTTTAATTATATGAAATGAACTGTAGCATAAGCAGTATTCATTTGGATTATTTTT
TTTATTAGCTCTCACCCCTTCATTATTCTGAGCTGAAAGTCTGGCATGAACTGTCCTCAATTTTGTT
TTCAAATTCACATCGATTATCTATGCATTATCCTCTTGTATCTACCTGTAGAAGTTTCTTTTTGGTT
ATTCCTTGACTGCTTGATTACAGAAAGAAATTTATGAAGCTGTAATCGGGATAGTTATACTGCTTGT
TCTTATGATTCATTTCCTTTGTGCAGTTCTTGGTGTAGCTTGCCACTTTCACCAGCAAAGTTC

SEQ ID NO: 228, primer prm08777
GGGGACAAGTTTGTACAAAAAAGCAGGCTTAAACAATGACTGACACCCCAATGTC

**SEQ ID NO: 229, primer prm08782**
GGGGACCACTTTGTACAAGAAAGCTGGGTTCATAAGGTGATTCCTTTATGC

FIGURE 11 (continued)

Europäisches Patentamt
European Patent Office
Office européen des brevets

# EUROPEAN SEARCH REPORT

Application Number
EP 12 17 0712

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/075807 A1 (SEOUL NAT UNIV IND FOUNDATION [KR]; HWANG INGYU [KR]; PARK CHANG-SEUK) 20 July 2006 (2006-07-20)<br>* the whole document *<br>----- | 1-17 | INV.<br>C12N15/82<br>A01H5/00<br>A01H5/10<br>C12N9/00 |
| A | O. CHOI ET AL: "Pyrroloquinoline Quinone Is a Plant Growth Promotion Factor Produced by Pseudomonas fluorescens B16", PLANT PHYSIOLOGY, vol. 146, no. 2, 1 January 2007 (2007-01-01), pages 657-668, XP55035576, ISSN: 0032-0889, DOI: 10.1104/pp.107.112748<br>* the whole document *<br>----- | 1-5,7-16 | |

TECHNICAL FIELDS SEARCHED (IPC)

C07K
C12N
A01H

The present search report has been drawn up for all claims

1

| Place of search | Date of completion of the search | Examiner |
|---|---|---|
| The Hague | 15 August 2012 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 17 0712

This annex lists the patent family members relating to the patent documents cited in the above-mentioned European search report. The members are as contained in the European Patent Office EDP file on
The European Patent Office is in no way liable for these particulars which are merely given for the purpose of information.

15-08-2012

| Patent document cited in search report | Publication date | Patent family member(s) | Publication date |
|---|---|---|---|
| WO 2006075807 A1 | 20-07-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5811238 A
- US 6395547 A
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A


### Non-patent literature cited in the description


- **WANG et al.** *Planta,* 2003, vol. 218, 1-14
- **RADCHUK et al.** *The Plant Journal,* 2007, vol. 51, 819-839
- **HATAKEYAMA et al.** *Biochemical and Biophysical Research Communications,* 2003, vol. 302, 635-645
- **AZEVEDO et al.** *Trends in Plant Science,* 2001, vol. 6 (8
- **CHO et al.** *Plant Physiology,* December 2006, vol. 142, 1664-1682
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **PATER et al.** *Plant J,* 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1986, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1996, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **NITZ et al.** *Plant Sci,* 2001, vol. 161 (2), 337-346
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245

- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **TAKAIWA et al.** *FEBS Letts,* 1987, vol. 221, 43-47
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102

- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 10 July 2003, vol. 4, 29
- **CHEN et al.** *Plant Cell Physiol,* 2002, vol. 43 (2), 159-169
- **CHEN et al.** *Planta,* 2004, vol. 219, 440-449
- Current Protocols in Molecular Biology. Wiley
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332

- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659

SEQUENCE LISTING

<110>  Crop Functional Genomics Center
       BASF Plant Science GmbH

<120>  Plants having increased yield and a method for making the same

<130>  PF59396

<160>  229

<170>  PatentIn version 3.3

<210>  1
<211>  3146
<212>  DNA
<213>  Synechocystis sp.

<400>  1
tacaaaaaag caggcttaaa caatgaactt ggcagttcct gcattcggtc tttccactaa 60
ctggtctggt aatggcaatg gttccaactc tgaagaagag tcggtgcttt accagcggtt 120
aaagatggtg gaggaattgt gggaaagggt gctccaaagc gaatgtggcc aggaattggt 180
ggatttgctg acggaattaa ggcttcaggg tacccatgag gcgatcacca gcgaaatttc 240
cgaagaagtc atcatgggta ttacccagcg cattgagcat ttagaactca atgatgccat 300
ccgggcggct cgggcctttg ccctatattt ccagttgatc aacatcgttg aacagcacta 360
cgaacaaaac gagcaacaac ggaatcgttg ggaagcttcc caggaaacca acttctatga 420
gcaggcgggc aatgaggaag aaatggtccc cccatcccga ttaggcgcgt caacggaacc 480
attgccagtg ggcattgacc agaatgaatt gcaagcttct gtaggtacgt tccattggtt 540
aatgagggag ctaaaacgcc tcaatgtgcc cccccaacat atccaaaatt tattggatca 600
tctggacatt cgcctggtga tcaccgctca ccccacggaa attgtccgtc acaccatccg 660
gcgcaaacaa agaagggtgg accgcattct tcgtaaacta gatcaactcc agggttctgt 720
gaccggtcgg gactggctca acacctggga tgcaaaaacg gcgatcgccc aattaacgga 780
ggaaattcgc ttttggtggc gtaccgacga acttcatcag ttcaaaccca ctgtgttgga 840
cgaagtggac tattccctcc attattttga tgaagtactg ttcgacgctg taccggaatt 900
gtccaaacgg ttaggacaag ctattaaaga aacctttccc catctgcggg ccccccgggc 960
taatttttgt tattttggct cctgggtcgg tggcgatcgg gacggcaacc cttcggtaac 1020
cccagaagtg acctggcaga cggcctgtta ccagcggggt ttagtgctgg ggaaatattt 1080
gtttagtttg gggaactgg tggccattct tagcccttcc ctccattggt gcaaagtctc 1140
ccaggaattg ttggactctt tggaacggga ccgcattcaa ttaccggaaa tttacgaaga 1200
actttctctc cgctatcgcc aggaacccta tcggatgaag ctggcctacg ttaccaaacg 1260
gctggaaaac accctgcggc gtaataatcg tctagccaac ccagaagaac ggcaaacgat 1320
gatcaccatg ccggccgaaa atcactatcg cactggggaa gaattattag aggaattaag 1380
actcattcag cgtaatctga ccgaaactgg tttaacctgc ctggagttgg aaaatttgat 1440
tacccagttg gaagtctatg gctttaacct agcccagttg gattttcgcc aggaatcttc 1500
ccgccacgcc gaggcgatcg ccgaaattgc tgagtatatg gggtactca ccactcccta 1560
cgaagaaatg gccgaagaag ataaattagc ctggttaggg gtagaactgc aaacccgccg 1620
tcctttaatt ccccaggaaa tgccctttc ggagcggact agggaaacca ttgaaaccct 1680
ccgcaccctg cgccatctac aaatggaatt tggggtggat atttgccaaa cctacatcat 1740
cagcatgacc aacgatgcca gtgatgtgtt ggaagtattg ctgttagcca aggaagccgg 1800
attgtatgac ccggccaccg cctccaattc cctccgcatt gtgcccctgt ttgaaacagt 1860
agaagatctc aaaaacgctc cggggattat ggattctctt ttcagcttgc ctttttaccg 1920
ggctacattg gcgggcagtt accattcctt aaaagagttg caaaatcagc caccggatta 1980
ttaccaaatt cccaccacca cagccctact aaatcccggc aatctccagg aaattatggt 2040
gggctattcc gacagcaata aagactccgg ctttttgagc agtaactggg aaattcataa 2100
ggcccaaaaa tcactgcagg cagtggccca aagccatcgg gtaattctcc ggctgttcca 2160
cggtcgagga ggatctgttg gccgggggg cggcccggcc tataaagcca ttttggccca 2220
gcccgcaggc accgtggacg gtcggatcaa aattaccgaa caaggggaag tgttagcttc 2280
taaatattcc ctgccagagt tggccctcta caacctggaa actttaacca cggcggtcat 2340
ccaagctagt ttacttaaaa gtagttttga tttcattgag ccctggaacc ggattatgga 2400
ggagttggcc tgcactgccc gtcgagccta ccggagtttg atttacgaag aaccggactt 2460
tttagatttc ttcctgacgg ttacccccat tcctgaaatt agcgagttac agattagttc 2520
ccgccctgcc cgacgtaagg ggggtaaagc ggatctcagc agtttgcggg ccattccctg 2580
ggtgttcagt tggacccaaa cccgtttcct gctgccggct tggtatgggg tgggcacggc 2640
gttgaaatcc tttgtggacc aagacccggt caaaaatatg aagttgttgc gttacttcta 2700
tttcaaatgg cctttcttca acatggtgat ctcgaaggtg gaaatgaccc tttccaaggt 2760
ggacctcacc atcgcttccc actatgtgca agagctatct aagccagaag accgggaacg 2820
attcgatcgc ctttttcagc agatcaagca agagtatcaa ttaaccaggg actttgccat 2880
ggaaattacg gcccatcccc acctcctgga cggcgatcgc tctttgcaac ggtcggtact 2940
cctacgaaat cgtactattg ttcccctggg gctactgcag atttccctgc tgaaacgttt 3000
acgccaagta acccaggaag cggagaccag cggcgtgcgt taccgtcgtt attccaaaga 3060
agaactactg cggggagctc tgttaaccat taacggtatt gcggccggaa tgcgtaatac 3120
tggttgaacc cagctttctt gtacaa 3146

<210>  2

<211> 1034
<212> PRT
<213> Synechocystis sp.

<400> 2
Met Asn Leu Ala Val Pro Ala Phe Gly Leu Ser Thr Asn Trp Ser Gly
1 5 10 15
Asn Gly Asn Gly Ser Asn Ser Glu Glu Glu Ser Val Leu Tyr Gln Arg
20 25 30
Leu Lys Met Val Glu Glu Leu Trp Glu Arg Val Leu Gln Ser Glu Cys
35 40 45
Gly Gln Glu Leu Val Asp Leu Leu Thr Glu Leu Arg Leu Gln Gly Thr
50 55 60
His Glu Ala Ile Thr Ser Glu Ile Ser Glu Glu Val Ile Met Gly Ile
65 70 75 80
Thr Gln Arg Ile Glu His Leu Glu Leu Asn Asp Ala Ile Arg Ala Ala
85 90 95
Arg Ala Phe Ala Leu Tyr Phe Gln Leu Ile Asn Ile Val Glu Gln His
100 105 110
Tyr Glu Gln Asn Glu Gln Gln Arg Asn Arg Trp Glu Ala Ser Gln Glu
115 120 125
Thr Asn Phe Tyr Glu Gln Ala Gly Asn Glu Glu Glu Met Val Pro Pro
130 135 140
Ser Arg Leu Gly Ala Ser Thr Glu Pro Leu Pro Val Gly Ile Asp Gln
145 150 155 160
Asn Glu Leu Gln Ala Ser Val Gly Thr Phe His Trp Leu Met Arg Glu
165 170 175
Leu Lys Arg Leu Asn Val Pro Pro Gln His Ile Gln Asn Leu Leu Asp
180 185 190
His Leu Asp Ile Arg Leu Val Ile Thr Ala His Pro Thr Glu Ile Val
195 200 205
Arg His Thr Ile Arg Arg Lys Gln Arg Arg Val Asp Arg Ile Leu Arg
210 215 220
Lys Leu Asp Gln Leu Gln Gly Ser Val Thr Gly Arg Asp Trp Leu Asn
225 230 235 240
Thr Trp Asp Ala Lys Thr Ala Ile Ala Gln Leu Thr Glu Glu Ile Arg
245 250 255
Phe Trp Trp Arg Thr Asp Glu Leu His Gln Phe Lys Pro Thr Val Leu
260 265 270
Asp Glu Val Asp Tyr Ser Leu His Tyr Phe Asp Glu Val Leu Phe Asp
275 280 285
Ala Val Pro Glu Leu Ser Lys Arg Leu Gly Gln Ala Ile Lys Glu Thr
290 295 300
Phe Pro His Leu Arg Ala Pro Arg Ala Asn Phe Cys Tyr Phe Gly Ser
305 310 315 320
Trp Val Gly Gly Asp Arg Asp Gly Asn Pro Ser Val Thr Pro Glu Val
325 330 335
Thr Trp Gln Thr Ala Cys Tyr Gln Arg Gly Leu Val Leu Gly Lys Tyr
340 345 350
Leu Phe Ser Leu Gly Glu Leu Val Ala Ile Leu Ser Pro Ser Leu His
355 360 365
Trp Cys Lys Val Ser Gln Glu Leu Leu Asp Ser Leu Glu Arg Asp Arg
370 375 380
Ile Gln Leu Pro Glu Ile Tyr Glu Glu Leu Ser Leu Arg Tyr Arg Gln
385 390 395 400
Glu Pro Tyr Arg Met Lys Leu Ala Tyr Val Thr Lys Arg Leu Glu Asn
405 410 415
Thr Leu Arg Arg Asn Asn Arg Leu Ala Asn Pro Glu Glu Arg Gln Thr
420 425 430
Met Ile Thr Met Pro Ala Glu Asn His Tyr Arg Thr Gly Glu Glu Leu
435 440 445
Leu Glu Glu Leu Arg Leu Ile Gln Arg Asn Leu Thr Glu Thr Gly Leu
450 455 460
Thr Cys Leu Glu Leu Glu Asn Leu Ile Thr Gln Leu Glu Val Tyr Gly
465 470 475 480
Phe Asn Leu Ala Gln Leu Asp Phe Arg Gln Glu Ser Ser Arg His Ala
485 490 495
Glu Ala Ile Ala Glu Ile Ala Glu Tyr Met Gly Val Leu Thr Thr Pro
500 505 510
Tyr Glu Glu Met Ala Glu Glu Asp Lys Leu Ala Trp Leu Gly Val Glu
515 520 525
Leu Gln Thr Arg Arg Pro Leu Ile Pro Gln Glu Met Pro Phe Ser Glu
530 535 540
Arg Thr Arg Glu Thr Ile Glu Thr Leu Arg Thr Leu Arg His Leu Gln

```
545                 550                 555                 560
Met Glu Phe Gly Val Asp Ile Cys Gln Thr Tyr Ile Ile Ser Met Thr
                565                 570                 575
Asn Asp Ala Ser Asp Val Leu Glu Val Leu Leu Leu Ala Lys Glu Ala
            580                 585                 590
Gly Leu Tyr Asp Pro Ala Thr Ala Ser Asn Ser Leu Arg Ile Val Pro
        595                 600                 605
Leu Phe Glu Thr Val Glu Asp Leu Lys Asn Ala Pro Gly Ile Met Asp
    610                 615                 620
Ser Leu Phe Ser Leu Pro Phe Tyr Arg Ala Thr Leu Ala Gly Ser Tyr
625                 630                 635                 640
His Ser Leu Lys Glu Leu Gln Asn Gln Pro Pro Asp Tyr Tyr Gln Ile
                645                 650                 655
Pro Thr Thr Thr Ala Leu Leu Asn Pro Gly Asn Leu Gln Glu Ile Met
            660                 665                 670
Val Gly Tyr Ser Asp Ser Asn Lys Asp Ser Gly Phe Leu Ser Ser Asn
        675                 680                 685
Trp Glu Ile His Lys Ala Gln Lys Ser Leu Gln Ala Val Ala Gln Ser
    690                 695                 700
His Arg Val Ile Leu Arg Leu Phe His Gly Arg Gly Gly Ser Val Gly
705                 710                 715                 720
Arg Gly Gly Gly Pro Ala Tyr Lys Ala Ile Leu Ala Gln Pro Ala Gly
                725                 730                 735
Thr Val Asp Gly Arg Ile Lys Ile Thr Glu Gln Gly Glu Val Leu Ala
            740                 745                 750
Ser Lys Tyr Ser Leu Pro Glu Leu Ala Leu Tyr Asn Leu Glu Thr Leu
        755                 760                 765
Thr Thr Ala Val Ile Gln Ala Ser Leu Leu Lys Ser Ser Phe Asp Phe
    770                 775                 780
Ile Glu Pro Trp Asn Arg Ile Met Glu Glu Leu Ala Cys Thr Ala Arg
785                 790                 795                 800
Arg Ala Tyr Arg Ser Leu Ile Tyr Glu Glu Pro Asp Phe Leu Asp Phe
                805                 810                 815
Phe Leu Thr Val Thr Pro Ile Pro Glu Ile Ser Glu Leu Gln Ile Ser
            820                 825                 830
Ser Arg Pro Ala Arg Arg Lys Gly Gly Lys Ala Asp Leu Ser Ser Leu
        835                 840                 845
Arg Ala Ile Pro Trp Val Phe Ser Trp Thr Gln Thr Arg Phe Leu Leu
    850                 855                 860
Pro Ala Trp Tyr Gly Val Gly Thr Ala Leu Lys Ser Phe Val Asp Gln
865                 870                 875                 880
Asp Pro Val Lys Asn Met Lys Leu Leu Arg Tyr Phe Tyr Phe Lys Trp
                885                 890                 895
Pro Phe Phe Asn Met Val Ile Ser Lys Val Glu Met Thr Leu Ser Lys
            900                 905                 910
Val Asp Leu Thr Ile Ala Ser His Tyr Val Gln Glu Leu Ser Lys Pro
        915                 920                 925
Glu Asp Arg Glu Arg Phe Asp Arg Leu Phe Gln Gln Ile Lys Gln Glu
    930                 935                 940
Tyr Gln Leu Thr Arg Asp Phe Ala Met Glu Ile Thr Ala His Pro His
945                 950                 955                 960
Leu Leu Asp Gly Asp Arg Ser Leu Gln Arg Ser Val Leu Leu Arg Asn
                965                 970                 975
Arg Thr Ile Val Pro Leu Gly Leu Leu Gln Ile Ser Leu Leu Lys Arg
            980                 985                 990
Leu Arg Gln Val Thr Gln Glu Ala  Glu Thr Ser Gly Val  Arg Tyr Arg
        995                 1000                1005
Arg Tyr  Ser Lys Glu Glu Leu  Leu Arg Gly Ala Leu  Leu Thr Ile
    1010                1015                1020
Asn Gly  Ile Ala Ala Gly Met  Arg Asn Thr Gly
    1025                1030
```

```
<210>  3
<211>  3182
<212>  DNA
<213>  Anacystis nidulans

<400>  3
ctgggtctcc ttgagatcgt atgctcgctg tatctctcag ggatcacggc ttcccgtcag      60
caactgtcca gatccgccct gcgttctgtg tccagtcaga cgtggtggga tcagggaatc     120
cacctagaat gaattactgc cagaacgctc gaactgccat gagtgctgct ctccagtcat     180
ccgacgatgc tttccgaacc gtttcgagtc ccctcgccac ggatttggat ctgtcgtctc     240
cgctggagtt tttccttcgc catcgcttga cggtggttga agaactctgg gaagtggttt     300
tgcgccaaga gtgcggccaa gagctggtcg atattctgac tcagctgcgt gacttgacct     360
```

```
cgccggaagg ccaagcccca gaagtgggcg gcgaagcctt ggttcaggtg attgaaaccc    420
tagagttgag cgatgcgatt cgggctgccc gtgcctttgc gctctacttt cagctgatca    480
atattgttga gcagcactac gagcaaactc aatatcaact cgcctacgag cgatcgcggt    540
tggaaccctt gccaggacca gatgaaagtc cggagggatt gcacaccatt gaaattcctc    600
agcatcagct cgatcccttt gctgcggtga ttccgctcaa ccaagatccg gcaaccttcc    660
aaacgctgtt cccgcgcctg cgccagctca atgtgccgcc gcaaatgatc caagagctga    720
ccgatcgcct cgatattcgg ctggttttca ccgctcaccc gacggaaatt gtccgccaca    780
cgattcgcga caaacaacgc cgaattgcct acctgctgcg gcaactggat gagctcgaaa    840
caggcaaaaa ccgaggcttt cgagagcttg aagcgcagaa tattcgtcag cagctgaccg    900
aggagattcg gctctggtgg cggacggatg agctccacca gttcaagcca acggtgttgg    960
atgaggtgga ctacgcgctc cactacttcc aagaagtcct ctttgaggcc attcctctgc   1020
tctatcagcg ctttcggctc gcgctgcagg ggactttccc cgacctacaa ccgcccgct    1080
acaacttctg ccagttcggc tcttgggtcg gctccgatcg cgatggcaat ccttcagtga   1140
cctctgccgt cacttggcaa accgcttgct atcagcgcag tctcgtcctc gatcgctaca   1200
tcacagcggt tgaacatctc cgcaatgtgc tcagcctctc gatgcactgg agcgaggtgc   1260
tgccggagtt gctcagctcg ttggaacagg agagcatgct cttcccggag acctatgagc   1320
agctagcggt ccgctatcgc caagagccct atcgcctcaa gctctcctat attctggagc   1380
gcctgcacaa cacccgcgat cgcaataccc gcctccaaca gcagcaagaa aaagatccca   1440
ccacgcccct gcccgaatat cgggatggca ccctctacca ggctggtacg gcctttctcg   1500
aagatctcaa gctgattcag cacaacctta agcagacggg actgagctgt tacgagctag   1560
agaagttgat ctgccaggtc gagatctttg gtttcaacct ggtccatctc gacattcgcc   1620
aagaaagctc gcgccattcc gacgcgatca acgaaatctg tgaatacctc caaattcttc   1680
cccagcccta caacgagctg agcgaagcag aacgaactgc ctggctggtt caagagctga   1740
aaacccgtcg gccgctggta ccagcgcgca tgccgttctc agaatcgacc cgcgagatca   1800
ttgaaaccct gcggatggtc aagcagctac aggaagaatt tggggaggcg gcttgccaaa   1860
cctacatcat cagcatgagc cgcgagctga gcgacctgct ggaagtgctg ctgctggcca   1920
aggaggttgg tctctacgac ccagtcaccg gcaagagttc gcttcaggtg attccgctgt   1980
ttgaaactgt ggaggactta caaaatgccc cgcgggtgat gacggcgctg tttgagctgc   2040
ccttctacac ccagctcaac cccacccagt ctgaaccgct gcaggaagtg atgctggggt   2100
attccgacag taacaaggac tcgggcttcc tcagcagtaa ctgggagatc cacaaggccc   2160
agaaagccct agggacggta gcccgcgacc accgcgtcaa gctgcggatc ttccacggcc   2220
gcgggggctc cgtcggtcga ggtggtggcc ctgcctacga ggcgatcttg gcccagccgg   2280
gtcgcaccac agatggccga atcaagatta cggaacaggg cgaggtcttg gcttcgaaat   2340
acgccctgcc cgaactggcg ctctataacc ttgagacgat cacgacggcg gtgattcagt   2400
ccagcctgct gggtagcggc tttgatgaca ttgagccgtg gaaccaaatt atggaagagt   2460
tggcggcgcg atcgcggcga cattaccgcg ctttggtgta cgagcagccc gacctggttg   2520
acttcttcaa tcaggtaacg ccgattgagg agatcagcaa actgcaaatc agctcgcgac   2580
cggctcgacg caaaaccggc aagcgcgatc tgggcagtct acgtgccatc cctggtctc    2640
ttagctggac gcagagtcgt ttctgctgc  cctcttggta tggcgtcggc acagcacttc   2700
aggagttttt gcaggagcgc ccggagcaga acctcaacct gctgcgctac ttctacgaga   2760
agtggccgtt cttccgcatg gtgatctcga aggtcgagat gaccctagcg aaggtcgatt   2820
tgcagattgc tcatcactac gtgcatgagc tggccaatcc tgaggatcaa gagcggtttg   2880
aacgagtgtt cagccaaatc gctgcagagt ttcagctgac ttgtcatctc gtgttgacga   2940
ttaccaacca cggtcgcttg ctggatggcg accccgaact gcagcgatcg gtgcagctgc   3000
gcaacggtac gatcgtgccc ctcggcttct tgcaagtcgc cctgcttaaa cgcctgcggc   3060
agtatcgcca gcaaacggaa acgacgggat tgatgcgatc gcgctatagc aaagggggaac  3120
tgctgcgcgg agcattgctg acgatcaacg gcattgcggc tggcatgcgc aatacaggtt   3180
ga                                                                  3182
```

<210> 4
<211> 1053
<212> PRT
<213> Synechococcus elongatus

<400> 4

```
Met Leu Ala Val Ser Leu Arg Asp His Gly Phe Pro Ser Ala Thr Val
1               5                   10                  15
Gln Ile Arg Pro Ala Phe Cys Val Gln Ser Asp Val Val Gly Ser Gly
            20                  25                  30
Asn Pro Pro Arg Met Asn Tyr Cys Gln Asn Ala Arg Thr Ala Met Ser
        35                  40                  45
Ala Ala Leu Gln Ser Ser Asp Asp Ala Phe Arg Thr Val Ser Ser Pro
    50                  55                  60
Leu Ala Thr Asp Leu Asp Leu Ser Ser Pro Leu Glu Phe Phe Leu Arg
65                  70                  75                  80
His Arg Leu Thr Val Val Glu Glu Leu Trp Glu Val Val Leu Arg Gln
                85                  90                  95
Glu Cys Gly Gln Glu Leu Val Asp Ile Leu Thr Gln Leu Arg Asp Leu
            100                 105                 110
Thr Ser Pro Glu Gly Gln Ala Pro Glu Val Gly Gly Glu Ala Leu Val
        115                 120                 125
Gln Val Ile Glu Thr Leu Glu Leu Ser Asp Ala Ile Arg Ala Ala Arg
    130                 135                 140
Ala Phe Ala Leu Tyr Phe Gln Leu Ile Asn Ile Val Glu Gln His Tyr
```

```
145                 150                 155                 160
Glu Gln Thr Gln Tyr Gln Leu Ala Tyr Glu Arg Ser Arg Leu Glu Pro
                165                 170                 175
Leu Pro Gly Pro Asp Glu Ser Pro Glu Gly Leu His Thr Ile Glu Ile
            180                 185                 190
Pro Gln His Gln Leu Asp Pro Phe Ala Ala Val Ile Pro Leu Asn Gln
        195                 200                 205
Asp Pro Ala Thr Phe Gln Thr Leu Phe Pro Arg Leu Arg Gln Leu Asn
    210                 215                 220
Val Pro Pro Gln Met Ile Gln Glu Leu Thr Asp Arg Leu Asp Ile Arg
225                 230                 235                 240
Leu Val Phe Thr Ala His Pro Thr Glu Ile Val Arg His Thr Ile Arg
                245                 250                 255
Asp Lys Gln Arg Arg Ile Ala Tyr Leu Leu Arg Gln Leu Asp Glu Leu
            260                 265                 270
Glu Thr Gly Lys Asn Arg Gly Phe Arg Glu Leu Glu Ala Gln Asn Ile
        275                 280                 285
Arg Gln Gln Leu Thr Glu Glu Ile Arg Leu Trp Trp Arg Thr Asp Glu
    290                 295                 300
Leu His Gln Phe Lys Pro Thr Val Leu Asp Glu Val Asp Tyr Ala Leu
305                 310                 315                 320
His Tyr Phe Gln Glu Val Leu Phe Glu Ala Ile Pro Leu Leu Tyr Gln
                325                 330                 335
Arg Phe Arg Leu Ala Leu Gln Gly Thr Phe Pro Asp Leu Gln Pro Pro
            340                 345                 350
Arg Tyr Asn Phe Cys Gln Phe Gly Ser Trp Val Gly Ser Asp Arg Asp
        355                 360                 365
Gly Asn Pro Ser Val Thr Ser Ala Val Thr Trp Gln Thr Ala Cys Tyr
    370                 375                 380
Gln Arg Ser Leu Val Leu Asp Arg Tyr Ile Thr Ala Val Glu His Leu
385                 390                 395                 400
Arg Asn Val Leu Ser Leu Ser Met His Trp Ser Glu Val Leu Pro Glu
                405                 410                 415
Leu Leu Ser Ser Leu Glu Gln Glu Ser Met Leu Phe Pro Glu Thr Tyr
            420                 425                 430
Glu Gln Leu Ala Val Arg Tyr Arg Gln Glu Pro Tyr Arg Leu Lys Leu
        435                 440                 445
Ser Tyr Ile Leu Glu Arg Leu His Asn Thr Arg Asp Arg Asn Thr Arg
    450                 455                 460
Leu Gln Gln Gln Gln Glu Lys Asp Pro Thr Thr Pro Leu Pro Glu Tyr
465                 470                 475                 480
Arg Asp Gly Thr Leu Tyr Gln Ala Gly Thr Ala Phe Leu Glu Asp Leu
                485                 490                 495
Lys Leu Ile Gln His Asn Leu Lys Gln Thr Gly Leu Ser Cys Tyr Glu
            500                 505                 510
Leu Glu Lys Leu Ile Cys Gln Val Glu Ile Phe Gly Phe Asn Leu Val
        515                 520                 525
His Leu Asp Ile Arg Gln Glu Ser Ser Arg His Ser Asp Ala Ile Asn
    530                 535                 540
Glu Ile Cys Glu Tyr Leu Gln Ile Leu Pro Gln Pro Tyr Asn Glu Leu
545                 550                 555                 560
Ser Glu Ala Glu Arg Thr Ala Trp Leu Val Gln Glu Leu Lys Thr Arg
                565                 570                 575
Arg Pro Leu Val Pro Ala Arg Met Pro Phe Ser Glu Ser Thr Arg Glu
            580                 585                 590
Ile Ile Glu Thr Leu Arg Met Val Lys Gln Leu Gln Glu Glu Phe Gly
        595                 600                 605
Glu Ala Ala Cys Gln Thr Tyr Ile Ile Ser Met Ser Arg Glu Leu Ser
    610                 615                 620
Asp Leu Leu Glu Val Leu Leu Leu Ala Lys Glu Val Gly Leu Tyr Asp
625                 630                 635                 640
Pro Val Thr Gly Lys Ser Ser Leu Gln Val Ile Pro Leu Phe Glu Thr
                645                 650                 655
Val Glu Asp Leu Gln Asn Ala Pro Arg Val Met Thr Ala Leu Phe Glu
            660                 665                 670
Leu Pro Phe Tyr Thr Gln Leu Asn Pro Thr Gln Ser Glu Pro Leu Gln
        675                 680                 685
Glu Val Met Leu Gly Tyr Ser Asp Ser Asn Lys Asp Ser Gly Phe Leu
    690                 695                 700
Ser Ser Asn Trp Glu Ile His Lys Ala Gln Lys Ala Leu Gly Thr Val
705                 710                 715                 720
Ala Arg Asp His Arg Val Lys Leu Arg Ile Phe His Gly Arg Gly Gly
                725                 730                 735
Ser Val Gly Arg Gly Gly Gly Pro Ala Tyr Glu Ala Ile Leu Ala Gln
```

```
            740                 745                 750
Pro Gly Arg Thr Thr Asp Gly Arg Ile Lys Ile Thr Glu Gln Gly Glu
        755                 760                 765
Val Leu Ala Ser Lys Tyr Ala Leu Pro Glu Leu Ala Leu Tyr Asn Leu
    770                 775                 780
Glu Thr Ile Thr Thr Ala Val Ile Gln Ser Ser Leu Leu Gly Ser Gly
785                 790                 795                 800
Phe Asp Asp Ile Glu Pro Trp Asn Gln Ile Met Glu Glu Leu Ala Ala
                805                 810                 815
Arg Ser Arg Arg His Tyr Arg Ala Leu Val Tyr Glu Gln Pro Asp Leu
            820                 825                 830
Val Asp Phe Phe Asn Gln Val Thr Pro Ile Glu Glu Ile Ser Lys Leu
        835                 840                 845
Gln Ile Ser Ser Arg Pro Ala Arg Arg Lys Thr Gly Lys Arg Asp Leu
    850                 855                 860
Gly Ser Leu Arg Ala Ile Pro Trp Val Phe Ser Trp Thr Gln Ser Arg
865                 870                 875                 880
Phe Leu Leu Pro Ser Trp Tyr Gly Val Gly Thr Ala Leu Gln Glu Phe
                885                 890                 895
Leu Gln Glu Arg Pro Glu Gln Asn Leu Asn Leu Leu Arg Tyr Phe Tyr
            900                 905                 910
Glu Lys Trp Pro Phe Phe Arg Met Val Ile Ser Lys Val Glu Met Thr
        915                 920                 925
Leu Ala Lys Val Asp Leu Gln Ile Ala His His Tyr Val His Glu Leu
    930                 935                 940
Ala Asn Pro Glu Asp Gln Glu Arg Phe Glu Arg Val Phe Ser Gln Ile
945                 950                 955                 960
Ala Ala Glu Phe Gln Leu Thr Cys His Leu Val Leu Thr Ile Thr Asn
                965                 970                 975
His Gly Arg Leu Leu Asp Gly Asp Pro Glu Leu Gln Arg Ser Val Gln
            980                 985                 990
Leu Arg Asn Gly Thr Ile Val Pro  Leu Gly Phe Leu Gln  Val Ala Leu
        995                 1000                1005
Leu Lys  Arg Leu Arg Gln Tyr  Arg Gln Gln Thr Glu  Thr Thr Gly
    1010                1015                1020
Leu Met  Arg Ser Arg Tyr Ser  Lys Gly Glu Leu Leu  Arg Gly Ala
    1025                1030                1035
Leu Leu  Thr Ile Asn Gly Ile  Ala Ala Gly Met Arg  Asn Thr Gly
    1040                1045                1050
```

<210> 5
<211> 2988
<212> DNA
<213> Synechococcus sp.

<400> 5

```
atgaaccaag tcatgcatcc ccccagtgcc gaagctgaac ttttgtccac ttcccaatct      60
ctcctccggc aacgcctcac gctggttgaa gacatttggc aggcggtgtt gcagaaagaa     120
tgtggtcaaa aactcgtcga acggctcaat catctccggg caacccgaac cgccgatggc     180
caaagcctaa acttctctcc aagtagtatt tctgagctga tcgaaaccct agatctagaa     240
gatgcgatcc gggcagcacg ggcttttgcc ctctatttcc agctgatcaa tagcgtcgag     300
caacattacg aacagcgcga acaacaacaa tttcggcgga acctcgcctc tgccaatgcc     360
agcgaagcca acggtaacag cgtccataca gaaattgccc ccacccaagc cggcaccttt     420
gattggttgt ttccccatct caagcaccaa aatatgccgc cccagacgat ccagcgtctt     480
ctcaatcagt tggatattcg actggtattt acggcccacc ccacggagat tgtccgccac     540
acgattcgca ataagcagcg gcggattgcg gggatcctac ggcaactgga tcaaaccgaa     600
gaagggctga aaagcttggg gacaagcgat tcttgggaaa ttgaaaatat tcagcagcaa     660
cttaccgaag aaattcggct ctggtggcgc accgatgagc tgcaccaatt taaaccccag     720
gtgttggacg aagtggacta tgcgttgcat tattttgagg aagttctgtt tgacaccctg     780
cccgaattat cggtacggct ccaacaggcc ctaaaggcat ctttcccaac cctcaaggtg     840
cccaccacca atttttgtaa ttttggctct tgggtcgggg gcgatcgcga tggcaacccct    900
tctgtcactc ctgacgttac gtggaagaca gcttgctatc aacggggttt ggtgctggaa     960
cgctacatcg cctcggtgga atccctctcg gatgtgttga gcttatcgct gcactggagt    1020
aatgtgctgc cggatctgct gcattcatcg taccaagatc agaatatttt ccccgacgtc    1080
tatcaaagcc tggccagtcg tcaccgtcaa gaaccctatc gtctcaagtc agcctacatt    1140
aagcggcgct tggaaaatac cttggagcgc aaccgtcgtc tagcgaatat gcccgcctgg    1200
gaaaataaag tagaagcggc cgatgacaag gtttatattt gtggccagga gtttctcgca    1260
gacctaaaac tgatccgcga aagcctggta cagacggaaa tcaactgtgc ggccctggat    1320
aaactcattt gccaagtcga aatttttagc tttgtgctca ctcgccttga ttttcgccaa    1380
gaatcaacgc gccactccga tgcgatcgcc gaaattgttg actacctgag tattgcccaa    1440
atcctacaac gacctaagcg acgcagaaaa aaccaccttg gtctagtgca ggaattaaaa    1500
acccgccgtc ccttgattcc gaaggaaatg cacttttcgg aaagaaccgt cgagacgatc    1560
caaacgctcc aggtgctgcg ccgtctccag caggaatttg gcatcggcat ttgccagacc    1620
tacatcatca gcatgaccaa cgaagtcagt gatgtcttag aggtgctgct cttggcccag    1680
```

```
gaagcgggtt tgtatgaccc cctcacaggg atgaccacca tccgcattgc ccccctcttt   1740
gagacggtgg acgacctgcg taacgccccg gaaatcatgc aggcgttgtt tgaaattccc   1800
ctctatcgcg cttgtttagc tggtggctat gaaccccccg ccgatggccg ctgtgatgaa   1860
acctttggcg atcgcctggt gccgaatctc caggaaatta tgctcggcta ttccgacagc   1920
aacaaagatt ccggcttcct cagtagtaat tgggaaatcc acaaagccca gaaaaatctc   1980
caacaagtgg ccgatcccta cggcattgac ctccgcattt tccatggtcg cggtggttcc   2040
gttggtcggg gcggcggccc tgcctacgcc gctattttgg cccaaccgcc caacaccatc   2100
aatggccgga ttaaaatcac cgaacagggg gaagtgctgg cgtctaaata ttccctccca   2160
gacttggcgc tatatcacct cgaaagcgtt tctacggcgg tgattcaatc gagtttgtta   2220
gccagtggct ttgatgatat tcagccctgg aaccggatta tggaagacct ctcccagcga   2280
tcgcgggcgg cttaccgggc gttaatttac gaagaacccg acttcctcga ctttttcatg   2340
tccgtgacgc cgattccgga aattagccaa ctgcaaatca gttcccgtcc ggcccgtcgc   2400
aaaaaaggca ataaagattt gagtagtctc cgggcgattc cctgggtctt tagctggacc   2460
caaagccgct tcctcgtgcc cgcttggtat ggtgtgggta ccgcgctcca gggctttttt   2520
gaagaggatc ccgtcgagaa cctgaagtta atgcgctatt tctacagtaa atggcccttt   2580
ttccggatgg tgatctcaaa ggtagagatg accctttcta aggtggactt acaaatggcg   2640
agccactatg tccacgaact ggcggaaaaa gaagatattc cccggtttga aaagcttcta   2700
gagcagattt cccaggaata caacctcacg aagcggctaa tcctcgaaat taccgaaaat   2760
gaagccctcc tcgatggcga tcgcccccctc cagcgttctg tccaactgag aaatggcacc   2820
attgtccccc tcggtttcct gcaagtctca ttgctcaaac ggctacgtca atacaccagg   2880
gaaacccaag ccagtatcgt tcacttccgc tacagcaaag aagaactcct gcggggcgcc   2940
cttttaacca tcaatggcat cgccgccgga atgcggaaca cgggttga                 2988

<210>  6
<211>  995
<212>  PRT
<213>  Synechococcus sp.

<400>  6
Met Asn Gln Val Met His Pro Pro Ser Ala Glu Ala Glu Leu Leu Ser
1               5                   10                  15
Thr Ser Gln Ser Leu Leu Arg Gln Arg Leu Thr Leu Val Glu Asp Ile
            20                  25                  30
Trp Gln Ala Val Leu Gln Lys Glu Cys Gly Gln Lys Leu Val Glu Arg
        35                  40                  45
Leu Asn His Leu Arg Ala Thr Arg Thr Ala Asp Gly Gln Ser Leu Asn
    50                  55                  60
Phe Ser Pro Ser Ser Ile Ser Glu Leu Ile Glu Thr Leu Asp Leu Glu
65                  70                  75                  80
Asp Ala Ile Arg Ala Ala Arg Ala Phe Ala Leu Tyr Phe Gln Leu Ile
                85                  90                  95
Asn Ser Val Glu Gln His Tyr Glu Gln Arg Glu Gln Gln Gln Phe Arg
            100                 105                 110
Arg Asn Leu Ala Ser Ala Asn Ala Ser Glu Ala Asn Gly Asn Ser Val
        115                 120                 125
His Thr Glu Ile Ala Pro Thr Gln Ala Gly Thr Phe Asp Trp Leu Phe
    130                 135                 140
Pro His Leu Lys His Gln Asn Met Pro Pro Gln Thr Ile Gln Arg Leu
145                 150                 155                 160
Leu Asn Gln Leu Asp Ile Arg Leu Val Phe Thr Ala His Pro Thr Glu
                165                 170                 175
Ile Val Arg His Thr Ile Arg Asn Lys Gln Arg Arg Ile Ala Gly Ile
            180                 185                 190
Leu Arg Gln Leu Asp Gln Thr Glu Glu Gly Leu Lys Ser Leu Gly Thr
        195                 200                 205
Ser Asp Ser Trp Glu Ile Glu Asn Ile Gln Gln Gln Leu Thr Glu Glu
    210                 215                 220
Ile Arg Leu Trp Trp Arg Thr Asp Glu Leu His Gln Phe Lys Pro Gln
225                 230                 235                 240
Val Leu Asp Glu Val Asp Tyr Ala Leu His Tyr Phe Glu Glu Val Leu
                245                 250                 255
Phe Asp Thr Leu Pro Glu Leu Ser Val Arg Leu Gln Gln Ala Leu Lys
            260                 265                 270
Ala Ser Phe Pro Thr Leu Lys Val Pro Thr Thr Asn Phe Cys Asn Phe
        275                 280                 285
Gly Ser Trp Val Gly Gly Asp Arg Asp Gly Asn Pro Ser Val Thr Pro
    290                 295                 300
Asp Val Thr Trp Lys Thr Ala Cys Tyr Gln Arg Gly Leu Val Leu Glu
305                 310                 315                 320
Arg Tyr Ile Ala Ser Val Glu Ser Leu Ser Asp Val Leu Ser Leu Ser
                325                 330                 335
Leu His Trp Ser Asn Val Leu Pro Asp Leu Leu His Ser Ser Tyr Gln
            340                 345                 350
Asp Gln Asn Ile Phe Pro Asp Val Tyr Gln Ser Leu Ala Ser Arg His
```

```
        355                 360                 365
Arg Gln Glu Pro Tyr Arg Leu Lys Ser Ala Tyr Ile Lys Arg Arg Leu
    370                 375                 380
Glu Asn Thr Leu Glu Arg Asn Arg Arg Leu Ala Asn Met Pro Ala Trp
385                 390                 395                 400
Glu Asn Lys Val Glu Ala Ala Asp Asp Lys Val Tyr Ile Cys Gly Gln
                405                 410                 415
Glu Phe Leu Ala Asp Leu Lys Leu Ile Arg Glu Ser Leu Val Gln Thr
            420                 425                 430
Glu Ile Asn Cys Ala Ala Leu Asp Lys Leu Ile Cys Gln Val Glu Ile
        435                 440                 445
Phe Ser Phe Val Leu Thr Arg Leu Asp Phe Arg Gln Glu Ser Thr Arg
    450                 455                 460
His Ser Asp Ala Ile Ala Glu Ile Val Asp Tyr Leu Ser Ile Ala Gln
465                 470                 475                 480
Ile Leu Gln Arg Pro Lys Arg Arg Arg Lys Asn His Leu Gly Leu Val
                485                 490                 495
Gln Glu Leu Lys Thr Arg Arg Pro Leu Ile Pro Lys Glu Met His Phe
            500                 505                 510
Ser Glu Arg Thr Val Glu Thr Ile Gln Thr Leu Gln Val Leu Arg Arg
        515                 520                 525
Leu Gln Gln Glu Phe Gly Ile Gly Ile Cys Gln Thr Tyr Ile Ile Ser
    530                 535                 540
Met Thr Asn Glu Val Ser Asp Val Leu Glu Val Leu Leu Leu Ala Gln
545                 550                 555                 560
Glu Ala Gly Leu Tyr Asp Pro Leu Thr Gly Met Thr Thr Ile Arg Ile
                565                 570                 575
Ala Pro Leu Phe Glu Thr Val Asp Asp Leu Arg Asn Ala Pro Glu Ile
            580                 585                 590
Met Gln Ala Leu Phe Glu Ile Pro Leu Tyr Arg Ala Cys Leu Ala Gly
        595                 600                 605
Gly Tyr Glu Pro Pro Ala Asp Gly Arg Cys Asp Glu Thr Phe Gly Asp
    610                 615                 620
Arg Leu Val Pro Asn Leu Gln Glu Ile Met Leu Gly Tyr Ser Asp Ser
625                 630                 635                 640
Asn Lys Asp Ser Gly Phe Leu Ser Ser Asn Trp Glu Ile His Lys Ala
                645                 650                 655
Gln Lys Asn Leu Gln Gln Val Ala Asp Pro Tyr Gly Ile Asp Leu Arg
            660                 665                 670
Ile Phe His Gly Arg Gly Gly Ser Val Gly Arg Gly Gly Gly Pro Ala
        675                 680                 685
Tyr Ala Ala Ile Leu Ala Gln Pro Pro Asn Thr Ile Asn Gly Arg Ile
    690                 695                 700
Lys Ile Thr Glu Gln Gly Glu Val Leu Ala Ser Lys Tyr Ser Leu Pro
705                 710                 715                 720
Asp Leu Ala Leu Tyr His Leu Glu Ser Val Ser Thr Ala Val Ile Gln
                725                 730                 735
Ser Ser Leu Leu Ala Ser Gly Phe Asp Asp Ile Gln Pro Trp Asn Arg
            740                 745                 750
Ile Met Glu Asp Leu Ser Gln Arg Ser Arg Ala Ala Tyr Arg Ala Leu
        755                 760                 765
Ile Tyr Glu Glu Pro Asp Phe Leu Asp Phe Phe Met Ser Val Thr Pro
    770                 775                 780
Ile Pro Glu Ile Ser Gln Leu Gln Ile Ser Ser Arg Pro Ala Arg Arg
785                 790                 795                 800
Lys Lys Gly Asn Lys Asp Leu Ser Ser Leu Arg Ala Ile Pro Trp Val
                805                 810                 815
Phe Ser Trp Thr Gln Ser Arg Phe Leu Val Pro Ala Trp Tyr Gly Val
            820                 825                 830
Gly Thr Ala Leu Gln Gly Phe Phe Glu Glu Asp Pro Val Glu Asn Leu
        835                 840                 845
Lys Leu Met Arg Tyr Phe Tyr Ser Lys Trp Pro Phe Phe Arg Met Val
    850                 855                 860
Ile Ser Lys Val Glu Met Thr Leu Ser Lys Val Asp Leu Gln Met Ala
865                 870                 875                 880
Ser His Tyr Val His Glu Leu Ala Glu Lys Glu Asp Ile Pro Arg Phe
                885                 890                 895
Glu Lys Leu Leu Glu Gln Ile Ser Gln Glu Tyr Asn Leu Thr Lys Arg
            900                 905                 910
Leu Ile Leu Glu Ile Thr Glu Asn Glu Ala Leu Leu Asp Gly Asp Arg
        915                 920                 925
Pro Leu Gln Arg Ser Val Gln Leu Arg Asn Gly Thr Ile Val Pro Leu
    930                 935                 940
Gly Phe Leu Gln Val Ser Leu Leu Lys Arg Leu Arg Gln Tyr Thr Arg
```

945 950 955 960
Glu Thr Gln Ala Ser Ile Val His Phe Arg Tyr Ser Lys Glu Glu Leu
965 970 975
Leu Arg Gly Ala Leu Leu Thr Ile Asn Gly Ile Ala Ala Gly Met Arg
980 985 990
Asn Thr Gly
995

<210> 7
<211> 3333
<212> DNA
<213> Anabaena variabilis

<400> 7
cgcctgccta ttttaccctt ccctgatgac gatgaatcaa ccaattaaag tttaaaaata 60
tacttcaaaa atatgctcac ataagtagat aagagcataa gttaccaaaa atcaatctgt 120
taaacagtaa ttggctttac aagagaagca agtatgggtt ctgtttttata ctctttatct 180
gaatctgcta atttatatcc agcatcggaa ttattcttgc gtcatcgtct ccaaatagta 240
gaagaactgt gggagtcggt actgcggcaa gaatgcggcc aaaatatggt ggatttgtta 300
cggcagttgc gtgatttgtg ttcaccagaa gggcaagcca caaaagacca agcagtttct 360
gctgtcaagt taattgaaca gttgaatatt aatgaagcca ttcgggcagc tagggctttt 420
gctttgtatt ttcagctgat caacatcata gagcaggaat atgagcaaag gcagcaatta 480
actcgctatt ctgacttaga agcggaaaca gcacccctaa atggttccga aaacattacc 540
tcatcctcta accacaacga agatgatgtt attttcaacc ggggtttagg gactgatttc 600
ttaggaaaaa attggactaa tcgtggacaa gggaaacaaa aaggtacttt tgcagcgtta 660
tttcccttat tgtctaaatt gaatgtccca ccccaacaaa ttcaacgcct gatttcccaa 720
ctagatgtcc gtttggtatt cactgcccac ccaacggaaa ttgtccgcca caccattcgg 780
gacaaacaac ggcaagtagt agatttgttg caacaactcg atgaggtgga aatcgagct 840
aaggacggcg gcggctatcc ttgggaagcg ggggaaattc gggaaaaatt gctagaagaa 900
attcgcttgt ggtggcgtac agacgaactc caccagttta agcccactgt gctggatgaa 960
gtggattatg ctttgcatta cttccaagaa gttttatttg atgggattcc ccaactgtat 1020
aagcgtttca aatacgccct aaatcaaact ttctcctggt tagaaccacc aagcaaagat 1080
ttctgttcct ttggttcttg ggtaggttca gacagagatg gtaatccatc ggtaacaccg 1140
gaaattacct ggcagacagc ttgttatcag cgcaaaatgg tgctggaaag atatatcaag 1200
tctgtcaccc aattgattga attattaagt atctccatgc actggagtga cgttttacca 1260
gacttattgg aatccttgga gttagatcag tcccagttaa gtgaagtata cgatgctctg 1320
gcgctgagat atcgccaaga accctatcgt ctcaaactag cttacgtcct caagcgcctg 1380
gaaaataccc gcgatcgcaa tttagcttta tataaaggtg aaacccccac aaatgaagat 1440
agccccatgt atcgttcggg gtcggaattt ttggcggaac tgcgattaat tcagcataac 1500
ttgacggaaa caggtttaag ctgccgagaa ttggataatt tgatctgtca agtggaaatt 1560
tttgacttta acctgacaaa attagacatc cgtcaagaat ccaccagaca ttcggacgcg 1620
ttaaacgaga ttctcgacta tctccaatta ttaccccagc catacaacga cctctccgaa 1680
gaacagcgag ttgcttggct aacaaccgaa ctgcaaaccc gtcgcccatt aatttcgtca 1740
gaactaccat tctctgacaa aactaatgat gtcattaaaa ctttccgcgt agtgcgatcg 1800
ctccaacaag aatttggcat taatatctgc caaacttaca tcattagtat gtgtcgtcaa 1860
gtcagcgatg tcttagaagt tttgcttcta gccaaagaag ccagactttt tgacccagcg 1920
atcgccgtcg gtacaattca ggttgtacca ttatttgaga ctgtcgaaga tttacaacgt 1980
tctcgcagcg tcatgcgaca gttatttgaa ttacctctat accgcgcttt gctagctggt 2040
ggttacaaaa ataccgaagt aaaagtaccg aatactgagc taacacccca atccccagcc 2100
cccagtcccc agtccgtact cacccccgac ttacaagaag tcatgctggg atattccgac 2160
agcaacaaag actccggctt cttgagtagc aattgggaaa ttcataaagc ccaaaaatca 2220
ttacagcaaa tcgccgaaga atatggcgta aatttgcgga ttttccacgg gcgcggcggt 2280
tctgtaggac gaggcggcgg cccagcccac gaggcgattt tggctcaacc aggacacagc 2340
atcaacgggc ggattaaaat taccgaacaa ggggaagtat tagcctccaa gtactccttg 2400
ctagatttag ccctgtacaa cttggaaacc atcaccacgg cggtgattca agccagcctc 2460
ctgcgaactg ggtttgatga tatcgaacct tggaacgaaa ttatggaaga attagcagcg 2520
cgatcgcggc aacattatcg tggtttaatt tacgaacagc ctgatttcat tgactttttc 2580
caccaagtca cacccattga agaaatcagt caactacaaa ttagttctcg tcccgcccgt 2640
cgtccttctg gtaagaaaga tttaagcagt ctccgcgcca ttccttgggt atttagctgg 2700
acacaaacac gattcttact accttcttgg tacggtgtcg gtacagcttt acaagaattt 2760
ttcaacgaag aaccagaaga acatctcaaa ttgatgcgct acttttatgt caagtggcct 2820
ttcttcaaga tggtgatttc taaagtagaa atgaccttgg cgaaagtaga tatgcaaatg 2880
gctggtcact acgtacaaga attgtctgac cctgaagaca aaccccggtt tgagaaagta 2940
tttgagcaaa tcgccaacga atattatctc accagagatt tagtcttaaa aatcaccgac 3000
cacggtcggc ttttagatgg tgatcctgta ctccagcgtt ctgtacagtt acgcaatggt 3060
acaatcgtcc cactagggtt tatccaagtt tccctcctca agcgcctacg ccagtccaaa 3120
aataatactg caacctctgg tgtcattcac tctcgttaca gtaagggaga attattgcgc 3180
ggcgcactgt taaccattaa tggtattgcc gcagggatga gaaatacagg ttgatttgtg 3240
agttgtcagt agttagtggt cagtggtaaa acacatacaa ctgacaactg acaaccgaac 3300
aaatggtcaa aaataaaatc ttaatctgta ctg 3333

<210> 8
<211> 982
<212> PRT

<213> Anabaena variabilis

<400> 8

```
Met Val Asp Leu Leu Arg Gln Leu Arg Asp Leu Cys Ser Pro Glu Gly
1               5                   10                  15
Gln Ala Thr Lys Asp Gln Ala Val Ser Ala Val Lys Leu Ile Glu Gln
            20                  25                  30
Leu Asn Ile Asn Glu Ala Ile Arg Ala Ala Arg Ala Phe Ala Leu Tyr
        35                  40                  45
Phe Gln Leu Ile Asn Ile Ile Glu Gln Glu Tyr Glu Gln Arg Gln Gln
    50                  55                  60
Leu Thr Arg Tyr Ser Asp Leu Glu Ala Glu Thr Ala Pro Leu Asn Gly
65                  70                  75                  80
Ser Glu Asn Ile Thr Ser Ser Ser Asn His Asn Glu Asp Asp Val Ile
                85                  90                  95
Phe Asn Arg Gly Leu Gly Thr Asp Phe Leu Gly Lys Asn Trp Thr Asn
            100                 105                 110
Arg Gly Gln Gly Lys Gln Lys Gly Thr Phe Ala Ala Leu Phe Pro Leu
        115                 120                 125
Leu Ser Lys Leu Asn Val Pro Pro Gln Gln Ile Gln Arg Leu Ile Ser
    130                 135                 140
Gln Leu Asp Val Arg Leu Val Phe Thr Ala His Pro Thr Glu Ile Val
145                 150                 155                 160
Arg His Thr Ile Arg Asp Lys Gln Arg Gln Val Val Asp Leu Leu Gln
                165                 170                 175
Gln Leu Asp Glu Val Glu Asn Arg Ala Lys Asp Gly Gly Gly Tyr Pro
            180                 185                 190
Trp Glu Ala Gly Glu Ile Arg Glu Lys Leu Leu Glu Glu Ile Arg Leu
        195                 200                 205
Trp Trp Arg Thr Asp Glu Leu His Gln Phe Lys Pro Thr Val Leu Asp
    210                 215                 220
Glu Val Asp Tyr Ala Leu His Tyr Phe Gln Glu Val Leu Phe Asp Gly
225                 230                 235                 240
Ile Pro Gln Leu Tyr Lys Arg Phe Lys Tyr Ala Leu Asn Gln Thr Phe
                245                 250                 255
Ser Trp Leu Glu Pro Pro Ser Lys Asp Phe Cys Ser Phe Gly Ser Trp
            260                 265                 270
Val Gly Ser Asp Arg Asp Gly Asn Pro Ser Val Thr Pro Glu Ile Thr
        275                 280                 285
Trp Gln Thr Ala Cys Tyr Gln Arg Lys Met Val Leu Glu Arg Tyr Ile
    290                 295                 300
Lys Ser Val Thr Gln Leu Ile Glu Leu Leu Ser Ile Ser Met His Trp
305                 310                 315                 320
Ser Asp Val Leu Pro Asp Leu Leu Glu Ser Leu Glu Leu Asp Gln Ser
                325                 330                 335
Gln Leu Ser Glu Val Tyr Asp Ala Leu Ala Leu Arg Tyr Arg Gln Glu
            340                 345                 350
Pro Tyr Arg Leu Lys Leu Ala Tyr Val Leu Lys Arg Leu Glu Asn Thr
        355                 360                 365
Arg Asp Arg Asn Leu Ala Leu Tyr Lys Gly Glu Thr Pro Thr Asn Glu
    370                 375                 380
Asp Ser Pro Met Tyr Arg Ser Gly Ser Glu Phe Leu Ala Glu Leu Arg
385                 390                 395                 400
Leu Ile Gln His Asn Leu Thr Glu Thr Gly Leu Ser Cys Arg Glu Leu
                405                 410                 415
Asp Asn Leu Ile Cys Gln Val Glu Ile Phe Asp Phe Asn Leu Thr Lys
            420                 425                 430
Leu Asp Ile Arg Gln Glu Ser Thr Arg His Ser Asp Ala Leu Asn Glu
        435                 440                 445
Ile Leu Asp Tyr Leu Gln Leu Leu Pro Gln Pro Tyr Asn Asp Leu Ser
    450                 455                 460
Glu Glu Gln Arg Val Ala Trp Leu Thr Thr Glu Leu Gln Thr Arg Arg
465                 470                 475                 480
Pro Leu Ile Ser Ser Glu Leu Pro Phe Ser Asp Lys Thr Asn Asp Val
                485                 490                 495
Ile Lys Thr Phe Arg Val Val Arg Ser Leu Gln Gln Glu Phe Gly Ile
            500                 505                 510
Asn Ile Cys Gln Thr Tyr Ile Ile Ser Met Cys Arg Gln Val Ser Asp
        515                 520                 525
Val Leu Glu Val Leu Leu Leu Ala Lys Glu Ala Arg Leu Phe Asp Pro
    530                 535                 540
Ala Ile Ala Val Gly Thr Ile Gln Val Val Pro Leu Phe Glu Thr Val
545                 550                 555                 560
Glu Asp Leu Gln Arg Ser Arg Ser Val Met Arg Gln Leu Phe Glu Leu
```

```
                565                 570                 575
Pro Leu Tyr Arg Ala Leu Leu Ala Gly Gly Tyr Lys Asn Thr Glu Val
            580                 585                 590
Lys Val Pro Asn Thr Glu Leu Thr Pro Gln Ser Pro Ala Pro Ser Pro
        595                 600                 605
Gln Ser Val Leu Thr Pro Asp Leu Gln Glu Val Met Leu Gly Tyr Ser
    610                 615                 620
Asp Ser Asn Lys Asp Ser Gly Phe Leu Ser Ser Asn Trp Glu Ile His
625                 630                 635                 640
Lys Ala Gln Lys Ser Leu Gln Gln Ile Ala Glu Glu Tyr Gly Val Asn
                645                 650                 655
Leu Arg Ile Phe His Gly Arg Gly Gly Ser Val Gly Arg Gly Gly Gly
            660                 665                 670
Pro Ala His Glu Ala Ile Leu Ala Gln Pro Gly His Ser Ile Asn Gly
        675                 680                 685
Arg Ile Lys Ile Thr Glu Gln Gly Glu Val Leu Ala Ser Lys Tyr Ser
    690                 695                 700
Leu Leu Asp Leu Ala Leu Tyr Asn Leu Glu Thr Ile Thr Thr Ala Val
705                 710                 715                 720
Ile Gln Ala Ser Leu Leu Arg Thr Gly Phe Asp Asp Ile Glu Pro Trp
                725                 730                 735
Asn Glu Ile Met Glu Glu Leu Ala Ala Arg Ser Arg Gln His Tyr Arg
            740                 745                 750
Gly Leu Ile Tyr Glu Gln Pro Asp Phe Ile Asp Phe Phe His Gln Val
        755                 760                 765
Thr Pro Ile Glu Glu Ile Ser Gln Leu Gln Ile Ser Ser Arg Pro Ala
    770                 775                 780
Arg Arg Pro Ser Gly Lys Lys Asp Leu Ser Ser Leu Arg Ala Ile Pro
785                 790                 795                 800
Trp Val Phe Ser Trp Thr Gln Thr Arg Phe Leu Leu Pro Ser Trp Tyr
                805                 810                 815
Gly Val Gly Thr Ala Leu Gln Glu Phe Phe Asn Glu Glu Pro Glu Glu
            820                 825                 830
His Leu Lys Leu Met Arg Tyr Phe Tyr Val Lys Trp Pro Phe Phe Lys
        835                 840                 845
Met Val Ile Ser Lys Val Glu Met Thr Leu Ala Lys Val Asp Met Gln
    850                 855                 860
Met Ala Gly His Tyr Val Gln Glu Leu Ser Asp Pro Glu Asp Lys Pro
865                 870                 875                 880
Arg Phe Glu Lys Val Phe Glu Gln Ile Ala Asn Glu Tyr Tyr Leu Thr
                885                 890                 895
Arg Asp Leu Val Leu Lys Ile Thr Asp His Gly Arg Leu Leu Asp Gly
            900                 905                 910
Asp Pro Val Leu Gln Arg Ser Val Gln Leu Arg Asn Gly Thr Ile Val
        915                 920                 925
Pro Leu Gly Phe Ile Gln Val Ser Leu Leu Lys Arg Leu Arg Gln Ser
    930                 935                 940
Lys Asn Asn Thr Ala Thr Ser Gly Val Ile His Ser Arg Tyr Ser Lys
945                 950                 955                 960
Gly Glu Leu Leu Arg Gly Ala Leu Leu Thr Ile Asn Gly Ile Ala Ala
                965                 970                 975
Gly Met Arg Asn Thr Gly
            980
```

<210> 9
<211> 3241
<212> DNA
<213> Thermosynechococcus vulcanus

<400> 9

```
gtgaattatg gcgttcgcga tcgcctgcaa cacctggaac tgattctgag cagcaaaaca      60
gcttagaact gttaacaaat ccttaatacg cctgttatca attgttgtac cgccctggtt     120
caggattggg taaagtaagg agtattatct ttgcaggctg gggtaaatat gacatcagtc     180
ctcgatgtga ccaatcgcga tcgcttaatt gaaagtgaaa gtttggcagc ccgtacccta     240
caggaacggt tgcgactggt ggaagaggtc ttggtcgatg tcttggcggc agaatcgggt     300
caagaattgg ttgatctatt gcggcgcttg gggctctct cttcgccgga aggtcatgtg      360
ctccatgccc cagaagggga attgctgaag gttattgaat ccctcgaact caatgaggcc     420
attagagcgg cccgggcttt taacctctac tttcaaatta tcaacatcgt tgagcagcat     480
tacgaacaac aatacaaccg tgaacgcgct gcccaagagg gattgcgccg ccgcagtgtc     540
atgagtgaac caatttccgg tgtcagtggt gaaggctttc cgctgcctca tactgctgcc     600
aacgcaacgg atgtgcgcag tgggccgagt gaacgcctag agcatagtct ctacgaagcc     660
attcccgcta ctcagcagta tggttccttt gcttggctct ttcctcggct gcagatgctg     720
aatgtgccgc cgcgccatat tcaaaagctt ttggatcaac tggacattaa gttggttttc     780
actgctcacc cgacggagat tgtgcggcaa acgattcgtg ataagcagcg gcgggttgcc     840
```

```
cgattacttg agcaactgga tgtgctggag gggcttctc cacacctaac ggattggaac     900
gcccaaactt tacgggcaca actgatggag gaaattcgcc tctggtggcg caccgatgag    960
ttgcaccaat ttaagccgga ggtgctcgat gaggtggaat acaccctcca ctacttcaag   1020
gaggtcattt ttgctgtcat tcccaagctc tatcgccgtc tggagcagtc attacatgaa   1080
acctttcccg cgcttcagcc cccccgtcac cgtttctgcc gctttggctc ttgggtgggg   1140
ggcgatcgcg atggcaatcc ctatgtcaaa ccagaagtaa cgtggcaaac ggcctgctat   1200
cagcgcaact tagttcttga ggagtatatt aagtccgttg agcgcttaat caatttgctc   1260
agcctgtccc tgcactggtg cgatgtgctg ccagatttgc tagattccct tgagcaggat   1320
caacggcaac tcccgagtat ctatgagcag tatgcggtgc gctatcggca ggaaccctac   1380
cgcctgaaac tggcctatgt gctcaaacgg ctgcaaaata cccgcgatcg caaccgggcg   1440
ctgcaaacct attgcattcg ccgcaatgag gcggaagagt taaataatgg acagttttac   1500
cgccacggtg aagaattctt ggcagaactg ctgctcattc agcgtaacct caaggaaacg   1560
ggattggcct gccgcgaatt ggatgatttg atttgccagg tggaggtctt tggctttaat   1620
ctagcagcct tggatattcg ccaagaaagt acctgtcacg ctgaggccct caatgaaatt   1680
accgcctatt tgggtattct cccctgtccc tatacagaac tctcagaagc cgaacgcacc   1740
cgctggctcc tcagtgaact ctcgaccccgt cgccccttga ttccagggga actccccttt  1800
agcgatcgca ccaatgaaat cattgaaaca ttccgcatgg tgcggcaact ccagcaggaa   1860
tttggcaccg atttgtgcaa tacctacatc atcagcatga gccatgaggt cagcgatctg   1920
ttggaggtac tcctctttgc taaggaggca ggcctttttg atccagccac tggcgctagt   1980
accctgcaag ccattcccct gtttgaaacg gtggaggatc tcaagcacgc cccagcggtg   2040
ctgacccaac tattctctct ccccttttgc cggagctatc ttggaagcaa cagtaccccc   2100
tttctgcagg aggtcatgct gggctattcc gacagcaata aggattcggg cttcctcagt   2160
agcaactggg aaatttataa ggcacaacaa cagctgcaga aaattgctga gagttttggc   2220
ttccaactgc gcattttcca cggtcggggt ggttcagtgg gtcggggtgg tggacctgcc   2280
tatgcggcga ttttggcaca gccagcacaa acgattaagg gacgaatcaa gattactgag   2340
caggggggagg tactggcttc caaatactcg ttgccggaac tcgcgctctt taacctcgaa  2400
acagtggcca cagcggtcat ccaagctagt ttgctccgca gtagtattga tgagattgag   2460
ccttggcacg agattatgga ggagttggct acgcgatcgc gccagtgcta tcgccatctc   2520
atctatgagc agccagaatt cattgaattc tttaacgaag tcaccccaat ccaagagatt   2580
agccaactgc aaattagctc acggccaaca cggcgggggg ggaagaaaac ccttgagagc   2640
ctgcgggcaa ttccttgggt ctttagttgg acgcaaaccc gtttcctgct gccggcttgg   2700
tatggcgtgg gtactgccct gaaggaattc cttgaggaaa aacccgctga gcatctctcc   2760
ctcttgcgct acttctacta taagtggcct ttcttccgca tggtgatctc taaggttgag   2820
atgacccttg ccaaggttga tctagagatt gcccgctact atgtccaaga actcagccag   2880
ccccaaaacc gtgaagcctt ctgccgcctc tacgatcaga ttgctcagga atatcgcctg   2940
accacggaat tagtcctcac gattactggc catgagcggc tactcgatgg ggatccggcg   3000
cttcagcgat cggtgcaact gcgcaatcgc accattgttc ctttggcctt cctgcaagta   3060
tctcttttga aacggctgcg ccagcacaat agccaaacca cctctggggc aattttgcgc   3120
tcccgctatg gtcggggtga attgctacgg ggggcactct tgaccatcaa tggcatagca   3180
gcggggatgc gcaatacagg ctaagcaacg gcgagggtga atcatggacc cgacgacccg   3240
c                                                                   3241
```

<210> 10
<211> 1011
<212> PRT
<213> Thermosynechococcus vulcanus

<400> 10

```
Met Thr Ser Val Leu Asp Val Thr Asn Arg Asp Arg Leu Ile Glu Ser
1               5                   10                  15
Glu Ser Leu Ala Ala Arg Thr Leu Gln Glu Arg Leu Arg Leu Val Glu
            20                  25                  30
Glu Val Leu Val Asp Val Leu Ala Ala Glu Ser Gly Gln Glu Leu Val
        35                  40                  45
Asp Leu Leu Arg Arg Leu Gly Ala Leu Ser Ser Pro Glu Gly His Val
    50                  55                  60
Leu His Ala Pro Glu Gly Glu Leu Leu Lys Val Ile Glu Ser Leu Glu
65                  70                  75                  80
Leu Asn Glu Ala Ile Arg Ala Ala Arg Ala Phe Asn Leu Tyr Phe Gln
                85                  90                  95
Ile Ile Asn Ile Val Glu Gln His Tyr Glu Gln Gln Tyr Asn Arg Glu
            100                 105                 110
Arg Ala Ala Gln Glu Gly Leu Arg Arg Arg Ser Val Met Ser Glu Pro
        115                 120                 125
Ile Ser Gly Val Ser Gly Glu Gly Phe Pro Leu Pro His Thr Ala Ala
    130                 135                 140
Asn Ala Thr Asp Val Arg Ser Gly Pro Ser Glu Arg Leu Glu His Ser
145                 150                 155                 160
Leu Tyr Glu Ala Ile Pro Ala Thr Gln Gln Tyr Gly Ser Phe Ala Trp
                165                 170                 175
Leu Phe Pro Arg Leu Gln Met Leu Asn Val Pro Pro Arg His Ile Gln
            180                 185                 190
Lys Leu Leu Asp Gln Leu Asp Ile Lys Leu Val Phe Thr Ala His Pro
        195                 200                 205
```

```
Thr Glu Ile Val Arg Gln Thr Ile Arg Asp Lys Gln Arg Arg Val Ala
    210                 215                 220
Arg Leu Leu Glu Gln Leu Asp Val Leu Glu Gly Ala Ser Pro His Leu
225                 230                 235                 240
Thr Asp Trp Asn Ala Gln Thr Leu Arg Ala Gln Leu Met Glu Glu Ile
                245                 250                 255
Arg Leu Trp Trp Arg Thr Asp Glu Leu His Gln Phe Lys Pro Glu Val
            260                 265                 270
Leu Asp Glu Val Glu Tyr Thr Leu His Tyr Phe Lys Glu Val Ile Phe
        275                 280                 285
Ala Val Ile Pro Lys Leu Tyr Arg Arg Leu Glu Gln Ser Leu His Glu
    290                 295                 300
Thr Phe Pro Ala Leu Gln Pro Pro Arg His Arg Phe Cys Arg Phe Gly
305                 310                 315                 320
Ser Trp Val Gly Gly Asp Arg Asp Gly Asn Pro Tyr Val Lys Pro Glu
                325                 330                 335
Val Thr Trp Gln Thr Ala Cys Tyr Gln Arg Asn Leu Val Leu Glu Glu
            340                 345                 350
Tyr Ile Lys Ser Val Glu Arg Leu Ile Asn Leu Leu Ser Leu Ser Leu
        355                 360                 365
His Trp Cys Asp Val Leu Pro Asp Leu Leu Asp Ser Leu Glu Gln Asp
    370                 375                 380
Gln Arg Gln Leu Pro Ser Ile Tyr Glu Gln Tyr Ala Val Arg Tyr Arg
385                 390                 395                 400
Gln Glu Pro Tyr Arg Leu Lys Leu Ala Tyr Val Leu Lys Arg Leu Gln
                405                 410                 415
Asn Thr Arg Asp Arg Asn Arg Ala Leu Gln Thr Tyr Cys Ile Arg Arg
            420                 425                 430
Asn Glu Ala Glu Glu Leu Asn Asn Gly Gln Phe Tyr Arg His Gly Glu
        435                 440                 445
Glu Phe Leu Ala Glu Leu Leu Leu Ile Gln Arg Asn Leu Lys Glu Thr
    450                 455                 460
Gly Leu Ala Cys Arg Glu Leu Asp Asp Leu Ile Cys Gln Val Glu Val
465                 470                 475                 480
Phe Gly Phe Asn Leu Ala Ala Leu Asp Ile Arg Gln Glu Ser Thr Cys
                485                 490                 495
His Ala Glu Ala Leu Asn Glu Ile Thr Ala Tyr Leu Gly Ile Leu Pro
            500                 505                 510
Cys Pro Tyr Thr Glu Leu Ser Glu Ala Glu Arg Thr Arg Trp Leu Leu
        515                 520                 525
Ser Glu Leu Ser Thr Arg Arg Pro Leu Ile Pro Gly Glu Leu Pro Phe
    530                 535                 540
Ser Asp Arg Thr Asn Glu Ile Ile Glu Thr Phe Arg Met Val Arg Gln
545                 550                 555                 560
Leu Gln Gln Glu Phe Gly Thr Asp Leu Cys Asn Thr Tyr Ile Ile Ser
                565                 570                 575
Met Ser His Glu Val Ser Asp Leu Leu Glu Val Leu Leu Phe Ala Lys
            580                 585                 590
Glu Ala Gly Leu Phe Asp Pro Ala Thr Gly Ala Ser Thr Leu Gln Ala
        595                 600                 605
Ile Pro Leu Phe Glu Thr Val Glu Asp Leu Lys His Ala Pro Ala Val
    610                 615                 620
Leu Thr Gln Leu Phe Ser Leu Pro Phe Cys Arg Ser Tyr Leu Gly Ser
625                 630                 635                 640
Asn Ser Thr Pro Phe Leu Gln Glu Val Met Leu Gly Tyr Ser Asp Ser
                645                 650                 655
Asn Lys Asp Ser Gly Phe Leu Ser Ser Asn Trp Glu Ile Tyr Lys Ala
            660                 665                 670
Gln Gln Gln Leu Gln Lys Ile Ala Glu Ser Phe Gly Phe Gln Leu Arg
        675                 680                 685
Ile Phe His Gly Arg Gly Gly Ser Val Gly Arg Gly Gly Gly Pro Ala
    690                 695                 700
Tyr Ala Ala Ile Leu Ala Gln Pro Ala Gln Thr Ile Lys Gly Arg Ile
705                 710                 715                 720
Lys Ile Thr Glu Gln Gly Glu Val Leu Ala Ser Lys Tyr Ser Leu Pro
                725                 730                 735
Glu Leu Ala Leu Phe Asn Leu Glu Thr Val Ala Thr Ala Val Ile Gln
            740                 745                 750
Ala Ser Leu Leu Arg Ser Ser Ile Asp Glu Ile Glu Pro Trp His Glu
        755                 760                 765
Ile Met Glu Glu Leu Ala Thr Arg Ser Arg Gln Cys Tyr Arg His Leu
    770                 775                 780
Ile Tyr Glu Gln Pro Glu Phe Ile Glu Phe Phe Asn Glu Val Thr Pro
785                 790                 795                 800
```

```
Ile Gln Glu Ile Ser Gln Leu Gln Ile Ser Ser Arg Pro Thr Arg Arg
                805                 810                 815
Gly Gly Lys Lys Thr Leu Glu Ser Leu Arg Ala Ile Pro Trp Val Phe
            820                 825                 830
Ser Trp Thr Gln Thr Arg Phe Leu Leu Pro Ala Trp Tyr Gly Val Gly
        835                 840                 845
Thr Ala Leu Lys Glu Phe Leu Glu Glu Lys Pro Ala Glu His Leu Ser
    850                 855                 860
Leu Leu Arg Tyr Phe Tyr Tyr Lys Trp Pro Phe Phe Arg Met Val Ile
865                 870                 875                 880
Ser Lys Val Glu Met Thr Leu Ala Lys Val Asp Leu Glu Ile Ala Arg
                885                 890                 895
Tyr Tyr Val Gln Glu Leu Ser Gln Pro Gln Asn Arg Glu Ala Phe Cys
            900                 905                 910
Arg Leu Tyr Asp Gln Ile Ala Gln Glu Tyr Arg Leu Thr Thr Glu Leu
        915                 920                 925
Val Leu Thr Ile Thr Gly His Glu Arg Leu Leu Asp Gly Asp Pro Ala
    930                 935                 940
Leu Gln Arg Ser Val Gln Leu Arg Asn Arg Thr Ile Val Pro Leu Gly
945                 950                 955                 960
Phe Leu Gln Val Ser Leu Leu Lys Arg Leu Arg Gln His Asn Ser Gln
                965                 970                 975
Thr Thr Ser Gly Ala Ile Leu Arg Ser Arg Tyr Gly Arg Gly Glu Leu
            980                 985                 990
Leu Arg Gly Ala Leu Leu Thr Ile  Asn Gly Ile Ala Ala  Gly Met Arg
        995                 1000                1005
Asn Thr  Gly
    1010
```

<210> 11
<211> 2736
<212> DNA
<213> Streptomyces coelicolor

<400> 11

```
gtgagcagtg ccgacgacca gaccaccacg acgaccagca gtgaactgcg cgccgacatc      60
cgccggctgg gtgatctcct cggggagacc ctggtccggc aggagggccc cgaactgctg     120
gaactcgtcg agaaggtacg ccgactcacc cgagaggacg gcgaggccgc cgccgaactg     180
ctgcgcggca ccgaactgga gaccgccgcc aagctcgtcc gcgccttctc cacctacttc     240
cacctggcca acgtcaccga gcaggtccac cgcggccgcg agctgggcgc caagcgcgcc     300
gccgagggcg gactgctcgc ccgtacggcc gaccggctga aggacgccga ccccgagcac     360
ctgcgcgaga cggtccgcaa cctcaacgtg cgccccgtgt tcaccgcgca ccccaccgag     420
gccgcccgcc gctccgtcct caacaagctg cgccgcatcg ccgccctcct ggacaccccg     480
gtcaacgagt cggaccggcg ccgcctggac acccgcctcg ccgagaacat cgacctcgtc     540
tggcagaccg acgagctgcg cgtcgtgcgc cccgagcccg ccgacgaggc ccgcaacgcc     600
atctactacc tcgacgagct gcacctgggc gccgtcggcg acgtcctcga agacctcacc     660
gccgagctgg agcgggccgg cgtcaagctc cccgacgaca cccgcccccct caccttcggc     720
acctggatcg gcggcgaccg cgacggcaac cccaacgtca ccccccaggt gacctgggac     780
gtcctcatcc tccagcacga gcacggcatc aacgacgccc tggagatgat cgacgagctg     840
cgcggcttcc tctctaactc catccggtac gccggtgcga ccgaggaact gctcgcctcg     900
ctccaggccg acctggaacg cctccccgag atcagccccc gctacaagcg cctcaacgcc     960
gaggagccct accggctcaa ggccacctgc atccgccaga agctggagaa caccaagcag    1020
cgcctcgcca agggcacccc ccacgaggac ggccgcgact acctcggcac cgcccagctc    1080
atcgacgacc tgcgcatcgt ccagacctcg ctgcgcgaac accgcggcgg cctgttcgcc    1140
gacgggcgcc tcgcccgcac catccgcacc ctggccgcct tcggcctcca gctcgccacc    1200
atggacgtcc gcgagcacgc cgacgcccac caccacgccc tcggccagct cttcgaccgg    1260
ctcggcgagg agtcctggcg ctacgccgac atgccgcgcg agtaccgcac caagctcctc    1320
gccaaggaac tgcgctcccg caggccgctg gcccccagcc ccgcccccgt cgacgcgccc    1380
ggcgagaaga ccctcggcgt cttccagacc gtccgccgcg ccctggaggt cttcggcccc    1440
gaggtcatcg agtcctacat catctccatg tgccagggcg ccgacgacgt cttcgccgcg    1500
gcggtactgg cccgcgaggc cgggctgatc gacctgcacg ccggctgggc gaagatcggc    1560
atcgtgccgc tgctggagac caccgacgag ctgaaggccg ccgacaccat cctggaggac    1620
ctgctcgccg acccctccta ccggcgcctg gtcgcgctgc gcggcgacgt ccaggaggtc    1680
atgctcggct actccgactc ctccaagttc ggcggcatca ccaccagcca gtgggagatc    1740
caccgcgccc agcgccggct gcgcgacgtc gcccaccgct acggcgtacg gctgcgcctc    1800
ttccacggcc gcggcggcac cgtcggccgc ggcggcggcc ccacccacga cgccatcctc    1860
gcccagccct ggggcaccct ggagggcgag atcaaggtca ccgagcaggg cgaggtcatc    1920
tccgacaagt acctcatccc cgccctcgcc cgggagaacc tggagctgac cgtcgcggcc    1980
accctccagg cctccgccct gcacaccgcg ccccgccagt ccgacgaggc cctggcccgc    2040
tgggacgccg cgatggacgt cgtctccgac gccgcccaca ccgcctaccg gcacctggtc    2100
gaggaccccg acctgccgac ctacttcctg gcctccaccc cggtcgacca gctcgccgac    2160
ctgcacctgg gctcgcggcc ctcccgccgc cccggctcgg gcgtctcgct cgacggactg    2220
cgcgccatcc cgtgggtgtt cggctggacc cagtcccggc agatcgtccc cggctggtac    2280
ggcgtcggct ccggcctcaa ggccctgcgc gaggcgggcc tggacaccgt gctcgacgag    2340
```

```
atgcaccagc agtggcactt cttccgcaac ttcatctcca acgtcgagat gaccctcgcc    2400
aagaccgacc tgcgcatcgc ccagcactac gtcgacaccc tcgtcccgga cgagctcaag    2460
cacgtcttcg acaccatcaa ggccgagcac gagctcaccg tcgccgaggt cctgcgcgtc    2520
accggcgaga gtgaactgct ggacgccgac ccggtcctca agcagacctt caccatccgc    2580
gacgcctacc tcgaccccat ctcctacctc caggtcgccc tcctcggccg tcagcgcgag    2640
gccgccgccg cgaacgagga cccggacccc ctcctcgccc gagccctcct cctcaccgtc    2700
aacggcgtgg cagcgggcct gcgcaacacc ggctga                              2736

<210>  12
<211>  911
<212>  PRT
<213>  Streptomyces coelicolor

<400>  12
Met Ser Ser Ala Asp Asp Gln Thr Thr Thr Thr Thr Ser Ser Glu Leu
1               5                   10                  15
Arg Ala Asp Ile Arg Arg Leu Gly Asp Leu Leu Gly Glu Thr Leu Val
            20                  25                  30
Arg Gln Glu Gly Pro Glu Leu Leu Glu Leu Val Glu Lys Val Arg Arg
        35                  40                  45
Leu Thr Arg Glu Asp Gly Glu Ala Ala Ala Glu Leu Leu Arg Gly Thr
    50                  55                  60
Glu Leu Glu Thr Ala Ala Lys Leu Val Arg Ala Phe Ser Thr Tyr Phe
65                  70                  75                  80
His Leu Ala Asn Val Thr Glu Gln Val His Arg Gly Arg Glu Leu Gly
                85                  90                  95
Ala Lys Arg Ala Ala Glu Gly Gly Leu Leu Ala Arg Thr Ala Asp Arg
            100                 105                 110
Leu Lys Asp Ala Asp Pro Glu His Leu Arg Glu Thr Val Arg Asn Leu
        115                 120                 125
Asn Val Arg Pro Val Phe Thr Ala His Pro Thr Glu Ala Ala Arg Arg
    130                 135                 140
Ser Val Leu Asn Lys Leu Arg Arg Ile Ala Ala Leu Leu Asp Thr Pro
145                 150                 155                 160
Val Asn Glu Ser Asp Arg Arg Arg Leu Asp Thr Arg Leu Ala Glu Asn
                165                 170                 175
Ile Asp Leu Val Trp Gln Thr Asp Glu Leu Arg Val Val Arg Pro Glu
            180                 185                 190
Pro Ala Asp Glu Ala Arg Asn Ala Ile Tyr Tyr Leu Asp Glu Leu His
        195                 200                 205
Leu Gly Ala Val Gly Asp Val Leu Glu Asp Leu Thr Ala Glu Leu Glu
    210                 215                 220
Arg Ala Gly Val Lys Leu Pro Asp Asp Thr Arg Pro Leu Thr Phe Gly
225                 230                 235                 240
Thr Trp Ile Gly Gly Asp Arg Asp Gly Asn Pro Asn Val Thr Pro Gln
                245                 250                 255
Val Thr Trp Asp Val Leu Ile Leu Gln His Glu His Gly Ile Asn Asp
            260                 265                 270
Ala Leu Glu Met Ile Asp Glu Leu Arg Gly Phe Leu Ser Asn Ser Ile
        275                 280                 285
Arg Tyr Ala Gly Ala Thr Glu Glu Leu Leu Ala Ser Leu Gln Ala Asp
    290                 295                 300
Leu Glu Arg Leu Pro Glu Ile Ser Pro Arg Tyr Lys Arg Leu Asn Ala
305                 310                 315                 320
Glu Glu Pro Tyr Arg Leu Lys Ala Thr Cys Ile Arg Gln Lys Leu Glu
                325                 330                 335
Asn Thr Lys Gln Arg Leu Ala Lys Gly Thr Pro His Glu Asp Gly Arg
            340                 345                 350
Asp Tyr Leu Gly Thr Ala Gln Leu Ile Asp Asp Leu Arg Ile Val Gln
        355                 360                 365
Thr Ser Leu Arg Glu His Arg Gly Gly Leu Phe Ala Asp Gly Arg Leu
    370                 375                 380
Ala Arg Thr Ile Arg Thr Leu Ala Ala Phe Gly Leu Gln Leu Ala Thr
385                 390                 395                 400
Met Asp Val Arg Glu His Ala Asp Ala His His His Ala Leu Gly Gln
                405                 410                 415
Leu Phe Asp Arg Leu Gly Glu Glu Ser Trp Arg Tyr Ala Asp Met Pro
            420                 425                 430
Arg Glu Tyr Arg Thr Lys Leu Leu Ala Lys Glu Leu Arg Ser Arg Arg
        435                 440                 445
Pro Leu Ala Pro Ser Pro Ala Pro Val Asp Ala Pro Gly Glu Lys Thr
    450                 455                 460
Leu Gly Val Phe Gln Thr Val Arg Arg Ala Leu Glu Val Phe Gly Pro
465                 470                 475                 480
```

```
Glu Val Ile Glu Ser Tyr Ile Ile Ser Met Cys Gln Gly Ala Asp Asp
                485                 490                 495
Val Phe Ala Ala Ala Val Leu Ala Arg Glu Ala Gly Leu Ile Asp Leu
            500                 505                 510
His Ala Gly Trp Ala Lys Ile Gly Ile Val Pro Leu Leu Glu Thr Thr
        515                 520                 525
Asp Glu Leu Lys Ala Ala Asp Thr Ile Leu Glu Asp Leu Leu Ala Asp
    530                 535                 540
Pro Ser Tyr Arg Arg Leu Val Ala Leu Arg Gly Asp Val Gln Glu Val
545                 550                 555                 560
Met Leu Gly Tyr Ser Asp Ser Ser Lys Phe Gly Gly Ile Thr Thr Ser
                565                 570                 575
Gln Trp Glu Ile His Arg Ala Gln Arg Arg Leu Arg Asp Val Ala His
            580                 585                 590
Arg Tyr Gly Val Arg Leu Arg Leu Phe His Gly Arg Gly Gly Thr Val
        595                 600                 605
Gly Arg Gly Gly Gly Pro Thr His Asp Ala Ile Leu Ala Gln Pro Trp
    610                 615                 620
Gly Thr Leu Glu Gly Glu Ile Lys Val Thr Glu Gln Gly Glu Val Ile
625                 630                 635                 640
Ser Asp Lys Tyr Leu Ile Pro Ala Leu Ala Arg Glu Asn Leu Glu Leu
                645                 650                 655
Thr Val Ala Ala Thr Leu Gln Ala Ser Ala Leu His Thr Ala Pro Arg
            660                 665                 670
Gln Ser Asp Glu Ala Leu Ala Arg Trp Asp Ala Ala Met Asp Val Val
        675                 680                 685
Ser Asp Ala Ala His Thr Ala Tyr Arg His Leu Val Glu Asp Pro Asp
    690                 695                 700
Leu Pro Thr Tyr Phe Leu Ala Ser Thr Pro Val Asp Gln Leu Ala Asp
705                 710                 715                 720
Leu His Leu Gly Ser Arg Pro Ser Arg Arg Pro Gly Ser Gly Val Ser
                725                 730                 735
Leu Asp Gly Leu Arg Ala Ile Pro Trp Val Phe Gly Trp Thr Gln Ser
            740                 745                 750
Arg Gln Ile Val Pro Gly Trp Tyr Gly Val Gly Ser Gly Leu Lys Ala
        755                 760                 765
Leu Arg Glu Ala Gly Leu Asp Thr Val Leu Asp Glu Met His Gln Gln
    770                 775                 780
Trp His Phe Phe Arg Asn Phe Ile Ser Asn Val Glu Met Thr Leu Ala
785                 790                 795                 800
Lys Thr Asp Leu Arg Ile Ala Gln His Tyr Val Asp Thr Leu Val Pro
                805                 810                 815
Asp Glu Leu Lys His Val Phe Asp Thr Ile Lys Ala Glu His Glu Leu
            820                 825                 830
Thr Val Ala Glu Val Leu Arg Val Thr Gly Glu Ser Glu Leu Leu Asp
        835                 840                 845
Ala Asp Pro Val Leu Lys Gln Thr Phe Thr Ile Arg Asp Ala Tyr Leu
    850                 855                 860
Asp Pro Ile Ser Tyr Leu Gln Val Ala Leu Leu Gly Arg Gln Arg Glu
865                 870                 875                 880
Ala Ala Ala Ala Asn Glu Asp Pro Asp Pro Leu Leu Ala Arg Ala Leu
                885                 890                 895
Leu Leu Thr Val Asn Gly Val Ala Ala Gly Leu Arg Asn Thr Gly
            900                 905                 910
```

<210> 13
<211> 3481
<212> DNA
<213> Rhodopseudomonas palustris

<400> 13

```
ctgcaggtga cgcgccagct cagcggcctt gccgccgcgc tcggcgtgct gaccctgctg      60
gcgctcggca ccttcacggc cggtttgcac atccgcgcct ggaagatcgc ggtggtcggc     120
gtcatccttg gcgtcagcgt cccggcgatc gccttcctgc agcaatcggc gctgctgatc     180
ctgctggtga tcggcctgat catcgccatc gtggtgccgt tcgtttgggc caagcgccgg     240
cccgcgccgg cttcggagcc gttattccag ccgccgttca cccgcaccac cacgccgccg     300
acctcggcca gctcgcggcc ggtgccgcag ccgccgctgt acgaaacgcc ggtcgcggcc     360
ccgatgccgg tcgcgccgcc gccggccccg acgcccagcg ctgccgagcc ggcctcgccg     420
ccgccgccgg cggacagcaa cacgccgccg gacaacgtcc gcaacatctc gggcgcgcgc     480
taactacgct tcgaccggcc cgcgcgacgt gcaccgccga ttcttgatct tgcgtgcggt     540
gccaaaatcc ttcaggatac cgatctgatc ctggagtgct gcgatgtcgt cgttgaacct     600
gtccgccggt cctgagccgg tttccgaacg tcctgacgac gcggccgcga tcgaggccga     660
gacccggctg cgcaacgaca tccggctgct cggccggatc ctcggcgaca ctgtgcgcga     720
gcaggaagga cagaccgtgt tcgatctggt cgagaacatc cgccagacct cgatccgatt     780
```

```
ccaccgcgac gacgacaaga ccgcacgcgc cgagctcgaa gccatcctgg acgggatgtc    840
gatccccgac acgatgcgga tcgttcgcgc cttcagctat ttctcacacc tcgccaacat    900
cgccgaagac cagaacaaca tccgccagat gcgcgccggc tcgaccgcgg gctcggcgcc    960
gcgtgccgga ctgctcgcca agacgctggc gcacgcgcgg caggaaggca tcagcgccgc   1020
ggagctgcgc aagttctttg cgaccgcgct ggtgagcccg gtgctgaccg cgcatccgac   1080
cgaagtgcgc cgcaagagca cgatggaccg cgaaatgcag atcgcttcgc tgctcgatca   1140
gcgcgaccgc gttcagctca ccgccgacga atgggccgac aacgaggagc ggctgcgccg   1200
cgccgtcgag acgctgtgga agaccaacct gctgcgccgg acaaagctga cggtgctgga   1260
tgaagtcacc aacggcctgt cgttctacga ctacaccttc ctgcgcgagg tgccgcggct   1320
gcacagtgcg ctggaagacc ggcttgccga tgcggccaag gccgaaggcg tcaacagcga   1380
cggcgagctg gcgagcttcc tgcggatggg aagctggatc ggcggcgatc gcgatggcaa   1440
tccgttcgtc actgccgagg tgctgcacgg caccttgaag ctgcagagca cgcgcgtgct   1500
gcgctattat ctcgacgagc tgcacgagct tggctcggag ttgtcgctgg cgtcgcatct   1560
cgccggcacc accgacaccg tcaaggcgct ggccgaaacc tcgcccgaca cctcaccgca   1620
tcgcaaatac gagccgtatc gcctcgcggt gtccggcatc tacgcgcggc tggcagcgac   1680
cgcgctcaag ctcgaggtcg agaacctccg cgcgccggtc ggcgaggccg agccttacgc   1740
cagcgcgcag gacttcaaag ccgatctcga cgcgatccat ctgtcgctca cccagcacaa   1800
ttccggtgtg atcgcgcgcg gccggctgcg ccagctccgc cgcgcgatcg actgctttgg   1860
cttccatctc gccagcctcg acatgcggca gaactcggcg gtgcacgagc gcaccattac   1920
cgagctgatg gacgcggcgc ggcccggcac ctcctatgcg atgctcgacg aggaagcgcg   1980
gatcgcgctc ttgatcagcg agctgcgcag cacccggccg ctgacctcga tgttcgtcaa   2040
atacagcgac gagacggtcg gcgagcttgc agtgttccgc gaagccgcga aggcgcacgc   2100
gacctacggg gcggcggcga tcccccaatg catcatctcg atgaccaagg gcgtttccga   2160
cctcttggag gtcgcggtgc tgctcaagga agtcgggctg atcgatccgt cggggcgcag   2220
cgccatcaac gtggtgccgc tgttcgagac catcgaggac ctgcaggcct gcgccaagat   2280
catggaccgg ctgctgtcga tcccggaata tcgccggctg gtcgacagcc gcggctcggt   2340
gcaggaggtg atgctcggct actccgacag caataaggac ggcggcttcg tcacctcggg   2400
ctgggagctg tacaaggccg agatcggact gatcgagatc ttcgagcacc atggcgtgcg   2460
gttgcggctg ttccacggcc gcggcggctc ggtcggccgc ggcggcggcc cgagctacga   2520
cgccatcgtg gcgcagccgg gtggggcggt gaacggccag atccgcatca ccgagcaggg   2580
cgagatcatc accaggaaat attccaacgt cgaagtcggc cgcaacaacc tcgagatcct   2640
cgccgccgcg acgttggaag caagcctgct gcagccgaag cgggtggcgc cgcatcgcga   2700
ttatctcgag gcgatggagc agctctccgc gctcgccttc aaggcgtatc gcggcctggt   2760
gtacgagacc gacggattcg tcgactactt ctgggcctcg acggtgatca acgagatctc   2820
gacgctgaac atcggcagcc gtccggcctc gcgcaagaaa acgcgcgcga tcgaggacct   2880
gcgcgcgatc ccctgggtat tctcgtgggc gcagtgccgg ctgatgctgc cgggctggta   2940
cggtttcggc agcgcggtgt cggcctgggt caccgcgcat cccgagacgg gcatcgcctt   3000
cctgcaaaag atgtatcagg agtggccgtt ctttcgcacg ctgctgtcga acatggacat   3060
ggtgctgtcg aagagctcga tcggcatcgc ctcgcgatat gccgagctgg tggaggacgt   3120
cgatgttcgc gagcgcatct tcggccgcat ccgcgccgaa tggcattcct cgatcgagta   3180
tctgttcgcg atcatgcagc aggaccgcct gctgcagagc aacccgctgc tcgaacgctc   3240
gatccgccac cgcttcccgt atctcgaccc cctgaaccac gtccaggtcc agctgctgcg   3300
cgaacaccgc acccacgacc cagacgaaca ggtgctccgc ggcgtgcaac tgacgatcaa   3360
cgggatttcg gcggggctgc ggaatagcgg gtgagggcgg gccgccgctg ggattggatt   3420
gggctgattt agccggtgga aaggcgcgcc tgttagattg gcggcgatgc tgaagcgggc   3480
g                                                                   3481
```

```
<210>  14
<211>  936
<212>  PRT
<213>  Rhodopseudomonas palustris

<400>  14
Met Ser Ser Leu Asn Leu Ser Ala Gly Pro Glu Pro Val Ser Glu Arg
1               5                   10                  15
Pro Asp Asp Ala Ala Ala Ile Glu Ala Glu Thr Arg Leu Arg Asn Asp
            20                  25                  30
Ile Arg Leu Leu Gly Arg Ile Leu Gly Asp Thr Val Arg Glu Gln Glu
        35                  40                  45
Gly Gln Thr Val Phe Asp Leu Val Glu Asn Ile Arg Gln Thr Ser Ile
    50                  55                  60
Arg Phe His Arg Asp Asp Asp Lys Thr Ala Arg Ala Glu Leu Glu Ala
65                  70                  75                  80
Ile Leu Asp Gly Met Ser Ile Pro Asp Thr Met Arg Ile Val Arg Ala
                85                  90                  95
Phe Ser Tyr Phe Ser His Leu Ala Asn Ile Ala Glu Asp Gln Asn Asn
            100                 105                 110
Ile Arg Gln Met Arg Ala Gly Ser Thr Ala Gly Ser Ala Pro Arg Ala
        115                 120                 125
Gly Leu Leu Ala Lys Thr Leu Ala His Ala Arg Gln Glu Gly Ile Ser
    130                 135                 140
Ala Ala Glu Leu Arg Lys Phe Phe Ala Thr Ala Leu Val Ser Pro Val
145                 150                 155                 160
Leu Thr Ala His Pro Thr Glu Val Arg Arg Lys Ser Thr Met Asp Arg
```

```
                165                 170                 175
Glu Met Gln Ile Ala Ser Leu Leu Asp Gln Arg Asp Arg Val Gln Leu
            180                 185                 190
Thr Ala Asp Glu Trp Ala Asp Asn Glu Glu Arg Leu Arg Arg Ala Val
        195                 200                 205
Glu Thr Leu Trp Lys Thr Asn Leu Leu Arg Arg Thr Lys Leu Thr Val
    210                 215                 220
Leu Asp Glu Val Thr Asn Gly Leu Ser Phe Tyr Asp Tyr Thr Phe Leu
225                 230                 235                 240
Arg Glu Val Pro Arg Leu His Ser Ala Leu Glu Asp Arg Leu Ala Asp
                245                 250                 255
Ala Ala Lys Ala Glu Gly Val Asn Ser Asp Gly Glu Leu Ala Ser Phe
            260                 265                 270
Leu Arg Met Gly Ser Trp Ile Gly Gly Asp Arg Asp Gly Asn Pro Phe
        275                 280                 285
Val Thr Ala Glu Val Leu His Gly Thr Leu Lys Leu Gln Ser Thr Arg
    290                 295                 300
Val Leu Arg Tyr Tyr Leu Asp Glu Leu His Glu Leu Gly Ser Glu Leu
305                 310                 315                 320
Ser Leu Ala Ser His Leu Ala Gly Thr Thr Asp Thr Val Lys Ala Leu
                325                 330                 335
Ala Glu Thr Ser Pro Asp Thr Ser Pro His Arg Lys Tyr Glu Pro Tyr
            340                 345                 350
Arg Leu Ala Val Ser Gly Ile Tyr Ala Arg Leu Ala Ala Thr Ala Leu
        355                 360                 365
Lys Leu Glu Val Glu Asn Leu Arg Ala Pro Val Gly Glu Ala Glu Pro
    370                 375                 380
Tyr Ala Ser Ala Gln Asp Phe Lys Ala Asp Leu Asp Ala Ile His Leu
385                 390                 395                 400
Ser Leu Thr Gln His Asn Ser Gly Val Ile Ala Arg Gly Arg Leu Arg
                405                 410                 415
Gln Leu Arg Arg Ala Ile Asp Cys Phe Gly Phe His Leu Ala Ser Leu
            420                 425                 430
Asp Met Arg Gln Asn Ser Ala Val His Glu Arg Thr Ile Thr Glu Leu
        435                 440                 445
Met Asp Ala Ala Arg Pro Gly Thr Ser Tyr Ala Met Leu Asp Glu Glu
    450                 455                 460
Ala Arg Ile Ala Leu Leu Ile Ser Glu Leu Arg Ser Thr Arg Pro Leu
465                 470                 475                 480
Thr Ser Met Phe Val Lys Tyr Ser Asp Glu Thr Val Gly Glu Leu Ala
                485                 490                 495
Val Phe Arg Glu Ala Ala Lys Ala His Ala Thr Tyr Gly Ala Ala Ala
            500                 505                 510
Ile Pro Gln Cys Ile Ile Ser Met Thr Lys Gly Val Ser Asp Leu Leu
        515                 520                 525
Glu Val Ala Val Leu Leu Lys Glu Val Gly Leu Ile Asp Pro Ser Gly
    530                 535                 540
Arg Ser Ala Ile Asn Val Val Pro Leu Phe Glu Thr Ile Glu Asp Leu
545                 550                 555                 560
Gln Ala Cys Ala Lys Ile Met Asp Arg Leu Leu Ser Ile Pro Glu Tyr
                565                 570                 575
Arg Arg Leu Val Asp Ser Arg Gly Ser Val Gln Glu Val Met Leu Gly
            580                 585                 590
Tyr Ser Asp Ser Asn Lys Asp Gly Gly Phe Val Thr Ser Gly Trp Glu
        595                 600                 605
Leu Tyr Lys Ala Glu Ile Gly Leu Ile Glu Ile Phe Glu His His Gly
    610                 615                 620
Val Arg Leu Arg Leu Phe His Gly Arg Gly Gly Ser Val Gly Arg Gly
625                 630                 635                 640
Gly Gly Pro Ser Tyr Asp Ala Ile Val Ala Gln Pro Gly Gly Ala Val
                645                 650                 655
Asn Gly Gln Ile Arg Ile Thr Glu Gln Gly Glu Ile Ile Thr Arg Lys
            660                 665                 670
Tyr Ser Asn Val Glu Val Gly Arg Asn Asn Leu Glu Ile Leu Ala Ala
        675                 680                 685
Ala Thr Leu Glu Ala Ser Leu Leu Gln Pro Lys Arg Val Ala Pro His
    690                 695                 700
Arg Asp Tyr Leu Glu Ala Met Glu Gln Leu Ser Ala Leu Ala Phe Lys
705                 710                 715                 720
Ala Tyr Arg Gly Leu Val Tyr Glu Thr Asp Gly Phe Val Asp Tyr Phe
                725                 730                 735
Trp Ala Ser Thr Val Ile Asn Glu Ile Ser Thr Leu Asn Ile Gly Ser
            740                 745                 750
Arg Pro Ala Ser Arg Lys Lys Thr Arg Ala Ile Glu Asp Leu Arg Ala
```

```
        755                 760                 765
Ile Pro Trp Val Phe Ser Trp Ala Gln Cys Arg Leu Met Leu Pro Gly
    770                 775                 780
Trp Tyr Gly Phe Gly Ser Ala Val Ser Ala Trp Val Thr Ala His Pro
785                 790                 795                 800
Glu Thr Gly Ile Ala Phe Leu Gln Lys Met Tyr Gln Glu Trp Pro Phe
                805                 810                 815
Phe Arg Thr Leu Leu Ser Asn Met Asp Met Val Leu Ser Lys Ser Ser
            820                 825                 830
Ile Gly Ile Ala Ser Arg Tyr Ala Glu Leu Val Glu Asp Val Asp Val
        835                 840                 845
Arg Glu Arg Ile Phe Gly Arg Ile Arg Ala Glu Trp His Ser Ser Ile
    850                 855                 860
Glu Tyr Leu Phe Ala Ile Met Gln Gln Asp Arg Leu Leu Gln Ser Asn
865                 870                 875                 880
Pro Leu Leu Glu Arg Ser Ile Arg His Arg Phe Pro Tyr Leu Asp Pro
                885                 890                 895
Leu Asn His Val Gln Val Gln Leu Leu Arg Glu His Arg Thr His Asp
            900                 905                 910
Pro Asp Glu Gln Val Leu Arg Gly Val Gln Leu Thr Ile Asn Gly Ile
        915                 920                 925
Ser Ala Gly Leu Arg Asn Ser Gly
    930                 935
```

```
<210>  15
<211>  2832
<212>  DNA
<213>  Welwitschia mirabilis

<220>
<221>  misc_feature
<222>  (1741)..(1743)
<223>  n is a, c, g, or t

<400>  15
agcatcgacg cgcaactgcg gcttttggtc ccaacgaagg tttccgaaga tgataagctt      60
attgagtatg acgctcttct gatggaccgc tttctggata tcttgcaaga cctacatgga     120
gaagaaatta gagaaacggt tcaagagtgc tatgagcttt caggagaata cgaaggaaag     180
tttgacacgg caaagttgga ggagcttgga ggagtgttaa caagtctaga tgctggagac     240
tccattgttg ttgcagctct ttctcacatg cttaacctag cgaacttggc tgaggaggtt     300
cagatagcat accgaagacg aattaaacac aaaaagaaag gggatttcgc ggatgagaat     360
tccgcaacga cggaatcaga cattgaagaa acctttaaaa ggcttgtaaa tcagcttgga     420
aggtctcctc aagaagtttt tgacgctctc aaaaatcaaa caattgattt ggtcttgact     480
gcacatccaa cgcatcagtc ggtatccctg ctacaaaagc atggcaggat ccgaaactgc     540
ttgtctcagt tgtatgcgaa agacattacg cccgatgaaa aacaggagct tgatgaagct     600
ttacagaggg agattcaagc tgccttcaga actgatgaaa ttcggcgcac tcctcccacc     660
cctcaagatg agatgcgagc aggaatgagt tatttccatg aaaccatatg gaaaggtgtc     720
cctaagttct tacgtcgtgt tgacactgct ctaaagtcga ttggtataaa tgagagactt     780
ccttacaatg cccccctaat acaattctcg tcgtggatgg gcggcgaccg cgacgggaat     840
cctagagtca ctccagaagt cacgcgagac gtttgtctcc ttgcgaggat gatggctaca     900
aatttatact actctcagat agaggacctt atgtttgagt tgtcaatgtg gcgatgtagc     960
gatgagcttc ggcaacgtgc tctgcagctt cataattctg caaagagaga tgcaaaacat    1020
tacatagagt tttggaagca agtacctccc aatgagccat ttagggtaat tttgggagat    1080
gttagggaca agttatataa cactcgggaa cgcactcgcc agttgcttgc caatggtttc    1140
tcagatgtgc cagaagaagc acttacgagt gtagatcagt tgttggaacc gttagagttg    1200
tgctatcgat ctctgtgctc taccggcgat caacccattg cagatggcag tcttcttgat    1260
ttcatgcgtc aagtctcaac ctttggctta actcttgtga agttggatat tagacaagaa    1320
tcagatcgac acactgatgt tatcgacgca ataacaagac atctaggcct tgggtcctat    1380
aaagagtggc cagaagacaa acgacaggag tggctgttgg ctgaactacg tggaaaacgt    1440
ccactatttg gacccgacct tccaactaca gatgaaataa gagaagttct tgacacattc    1500
cacgtggtag ctgaacttcc tccagacaac tttggagctt acatcatatc aatggctact    1560
gctccttcag atgttttagt cgttgagtta ctgcagaggg aatgccgtgt gaaaaagcct    1620
ctgcgagttg tgcctttgtt tgagaagctt gctgatttag agaatcgtcc cgcggctttg    1680
gcaaggctgt tctcaattga ttggtataga aacagaattg atggaaagca agaagtaatg    1740
nnnggttact cagactccgg caaggatgcc ggaagactat ctgcagcatg gcaattatac    1800
aaggctcaag aggaattgat caaggttgca aaacagtttg gaattaagct gacaatgttt    1860
catggtcgtg gagggaccgt tggaagagga ggtggtccaa cccatctggc tatattatct    1920
caacctccag acacaattca cgggtcattg cgagttaccg tccaagggga ggttattgag    1980
cagtgttttg gggaggaaca tctgtgtttc cgaactcttc agcgctttac tgctgccaca    2040
ttggaacatg gaatgcatcc tccaattgca cctaagcccg aatggcgtca actgatggat    2100
gaaatggctg ttgttgctac tcaagagtat aggtcttatg ttttccacaa caagagattt    2160
gtggagtatt tccgatccgc aactcccgag ttggagtatg gacggatgaa tataggcagt    2220
cgtccatcaa aaagaaagcc cagtggaggc attgaatcac ttcgtgcaat tccatggata    2280
tttgcttgga cacagaccag attccatctt cctgtttggc ttggatttgg tgctgctttc    2340
```

```
aagagtgtta tcgagaagga cattcgaaat cttcacacgc tgcaacaaat gtataacgaa    2400
tggccatttt tccgtgtcac cattgaccta gttgaaatgg tgtttgccaa gcaccctgaa    2460
attgctgcat tatacgataa actgcttgta tcatcagtct tgtgggcttt cggggaacaa    2520
ctaagaaaaa actatgtaga gactaagacc cttctgctgc aggttgctgg acacaaagaa    2580
gtcttagaag gcgacccata tctgaaacaa cgattgagac ttcgtgactc atatatcaca    2640
accttgaatg cacttcaggc atatacttta aaacggatac gagatccaag ctaccatgtc    2700
accctcaggc ctcatttatc taaagaaagc tcaaccaagc cagcagcaga attggtaaaa    2760
ttaaacccaa ccagtgagta tgcaccggga ttagaagata cattgatctt aaccatgaag    2820
ggcattgctg cc                                                        2832

<210>  16
<211>  944
<212>  PRT
<213>  Welwitschia mirabilis

<220>
<221>  UNSURE
<222>  (581)..(581)
<223>  Xaa can be any naturally occurring amino acid

<400>  16
Ser Ile Asp Ala Gln Leu Arg Leu Leu Val Pro Thr Lys Val Ser Glu
1               5                   10                  15
Asp Asp Lys Leu Ile Glu Tyr Asp Ala Leu Leu Met Asp Arg Phe Leu
            20                  25                  30
Asp Ile Leu Gln Asp Leu His Gly Glu Glu Ile Arg Glu Thr Val Gln
        35                  40                  45
Glu Cys Tyr Glu Leu Ser Gly Glu Tyr Glu Gly Lys Phe Asp Thr Ala
    50                  55                  60
Lys Leu Glu Glu Leu Gly Gly Val Leu Thr Ser Leu Asp Ala Gly Asp
65                  70                  75                  80
Ser Ile Val Val Ala Ala Leu Ser His Met Leu Asn Leu Ala Asn Leu
                85                  90                  95
Ala Glu Glu Val Gln Ile Ala Tyr Arg Arg Arg Ile Lys His Lys Lys
            100                 105                 110
Lys Gly Asp Phe Ala Asp Glu Asn Ser Ala Thr Thr Glu Ser Asp Ile
        115                 120                 125
Glu Glu Thr Phe Lys Arg Leu Val Asn Gln Leu Gly Arg Ser Pro Gln
    130                 135                 140
Glu Val Phe Asp Ala Leu Lys Asn Gln Thr Ile Asp Leu Val Leu Thr
145                 150                 155                 160
Ala His Pro Thr His Gln Ser Val Ser Leu Leu Gln Lys His Gly Arg
                165                 170                 175
Ile Arg Asn Cys Leu Ser Gln Leu Tyr Ala Lys Asp Ile Thr Pro Asp
            180                 185                 190
Glu Lys Gln Glu Leu Asp Glu Ala Leu Gln Arg Glu Ile Gln Ala Ala
        195                 200                 205
Phe Arg Thr Asp Glu Ile Arg Arg Thr Pro Pro Thr Pro Gln Asp Glu
    210                 215                 220
Met Arg Ala Gly Met Ser Tyr Phe His Glu Thr Ile Trp Lys Gly Val
225                 230                 235                 240
Pro Lys Phe Leu Arg Arg Val Asp Thr Ala Leu Lys Ser Ile Gly Ile
                245                 250                 255
Asn Glu Arg Leu Pro Tyr Asn Ala Pro Leu Ile Gln Phe Ser Ser Trp
            260                 265                 270
Met Gly Gly Asp Arg Asp Gly Asn Pro Arg Val Thr Pro Glu Val Thr
        275                 280                 285
Arg Asp Val Cys Leu Leu Ala Arg Met Met Ala Thr Asn Leu Tyr Tyr
    290                 295                 300
Ser Gln Ile Glu Asp Leu Met Phe Glu Leu Ser Met Trp Arg Cys Ser
305                 310                 315                 320
Asp Glu Leu Arg Gln Arg Ala Leu Gln Leu His Asn Ser Ala Lys Arg
                325                 330                 335
Asp Ala Lys His Tyr Ile Glu Phe Trp Lys Gln Val Pro Pro Asn Glu
            340                 345                 350
Pro Phe Arg Val Ile Leu Gly Asp Val Arg Asp Lys Leu Tyr Asn Thr
        355                 360                 365
Arg Glu Arg Thr Arg Gln Leu Leu Ala Asn Gly Phe Ser Asp Val Pro
    370                 375                 380
Glu Glu Ala Leu Thr Ser Val Asp Gln Leu Leu Glu Pro Leu Glu Leu
385                 390                 395                 400
Cys Tyr Arg Ser Leu Cys Ser Thr Gly Asp Gln Pro Ile Ala Asp Gly
                405                 410                 415
Ser Leu Leu Asp Phe Met Arg Gln Val Ser Thr Phe Gly Leu Thr Leu
```

```
                420                 425                 430
Val Lys Leu Asp Ile Arg Gln Glu Ser Asp Arg His Thr Asp Val Ile
        435                 440                 445
Asp Ala Ile Thr Arg His Leu Gly Leu Gly Ser Tyr Lys Glu Trp Pro
    450                 455                 460
Glu Asp Lys Arg Gln Glu Trp Leu Leu Ala Glu Leu Arg Gly Lys Arg
465                 470                 475                 480
Pro Leu Phe Gly Pro Asp Leu Pro Thr Thr Asp Glu Ile Arg Glu Val
                485                 490                 495
Leu Asp Thr Phe His Val Val Ala Glu Leu Pro Pro Asp Asn Phe Gly
            500                 505                 510
Ala Tyr Ile Ile Ser Met Ala Thr Ala Pro Ser Asp Val Leu Val Val
        515                 520                 525
Glu Leu Leu Gln Arg Glu Cys Arg Val Lys Lys Pro Leu Arg Val Val
    530                 535                 540
Pro Leu Phe Glu Lys Leu Ala Asp Leu Glu Asn Arg Pro Ala Ala Leu
545                 550                 555                 560
Ala Arg Leu Phe Ser Ile Asp Trp Tyr Arg Asn Arg Ile Asp Gly Lys
                565                 570                 575
Gln Glu Val Met Xaa Gly Tyr Ser Asp Ser Gly Lys Asp Ala Gly Arg
            580                 585                 590
Leu Ser Ala Ala Trp Gln Leu Tyr Lys Ala Gln Glu Glu Leu Ile Lys
        595                 600                 605
Val Ala Lys Gln Phe Gly Ile Lys Leu Thr Met Phe His Gly Arg Gly
    610                 615                 620
Gly Thr Val Gly Arg Gly Gly Gly Pro Thr His Leu Ala Ile Leu Ser
625                 630                 635                 640
Gln Pro Pro Asp Thr Ile His Gly Ser Leu Arg Val Thr Val Gln Gly
                645                 650                 655
Glu Val Ile Glu Gln Cys Phe Gly Glu Glu His Leu Cys Phe Arg Thr
            660                 665                 670
Leu Gln Arg Phe Thr Ala Ala Thr Leu Glu His Gly Met His Pro Pro
        675                 680                 685
Ile Ala Pro Lys Pro Glu Trp Arg Gln Leu Met Asp Glu Met Ala Val
    690                 695                 700
Val Ala Thr Gln Glu Tyr Arg Ser Tyr Val Phe His Asn Lys Arg Phe
705                 710                 715                 720
Val Glu Tyr Phe Arg Ser Ala Thr Pro Glu Leu Glu Tyr Gly Arg Met
                725                 730                 735
Asn Ile Gly Ser Arg Pro Ser Lys Arg Lys Pro Ser Gly Gly Ile Glu
            740                 745                 750
Ser Leu Arg Ala Ile Pro Trp Ile Phe Ala Trp Thr Gln Thr Arg Phe
        755                 760                 765
His Leu Pro Val Trp Leu Gly Phe Gly Ala Ala Phe Lys Ser Val Ile
    770                 775                 780
Glu Lys Asp Ile Arg Asn Leu His Thr Leu Gln Gln Met Tyr Asn Glu
785                 790                 795                 800
Trp Pro Phe Phe Arg Val Thr Ile Asp Leu Val Glu Met Val Phe Ala
                805                 810                 815
Lys His Pro Glu Ile Ala Ala Leu Tyr Asp Lys Leu Leu Val Ser Ser
            820                 825                 830
Val Leu Trp Ala Phe Gly Glu Gln Leu Arg Lys Asn Tyr Val Glu Thr
        835                 840                 845
Lys Thr Leu Leu Leu Gln Val Ala Gly His Lys Glu Val Leu Glu Gly
    850                 855                 860
Asp Pro Tyr Leu Lys Gln Arg Leu Arg Leu Arg Asp Ser Tyr Ile Thr
865                 870                 875                 880
Thr Leu Asn Ala Leu Gln Ala Tyr Thr Leu Lys Arg Ile Arg Asp Pro
                885                 890                 895
Ser Tyr His Val Thr Leu Arg Pro His Leu Ser Lys Glu Ser Ser Thr
            900                 905                 910
Lys Pro Ala Ala Glu Leu Val Lys Leu Asn Pro Thr Ser Glu Tyr Ala
        915                 920                 925
Pro Gly Leu Glu Asp Thr Leu Ile Leu Thr Met Lys Gly Ile Ala Ala
    930                 935                 940
```

<210> 17
<211> 4776
<212> DNA
<213> Chlamydomonas reinhardtii

<400> 17

```
atgacggact ccacatatga ttttggagcc gtgagggacg atctgacgcc gcttgaggat      60
gactgcaagc ttctcggtag cctgcttgac gactgtctcc gcgtcgaaat cggcgagacc     120
```

```
atgttcaaga agattgaacg catccgagca ctggcgcaat gtgcctcaaa tctgtcgatc    180
aagggagacg cgggcgcctc cgacatgctg tcgcaccggc tggcggagga gctgatgaac    240
ctggacatgg acgaggcggt gccgctcaca cgcgcctgcg gccactacct caacctgtcc    300
ggcatcgcag agctgcacca cggagtgcgc cgcgaccgcg ccactcgcga gcccaacccc    360
aacagctgcg acgcggtgtt tgcgcggctg atcacggagg gcgtggaccc cgaggagctg    420
taccggcggg tgtcggagca gaacgtggag gtggtgctca ccgcgcaccc cacacaggtg    480
aaccggcgca cgctgcagta caagcacact cgcattgcgg cgctgctgca gcagcacgac    540
cgctccgacc tgacggcgga ggagcggcgc aacatggtga gcgagctgca gcgggaggtg    600
gcggcgctgt ggcagacgga cgagctgagg aggcagaagc ccacgccgct ggacgaggcg    660
cgcggcggtc tgcacattgt ggagcagtcg ctgtgggccg cggtgccgca gtacatgcgc    720
cgcctcagcg ccgcgctcaa gaaacacacc gggcacgacc tgccgctgca ggccacgccc    780
ttccgcttcg gcagctggat gggcggcgac cgcgatggca accccaacgt gaccgccaag    840
gtgacggcgc acgtgacggc cctggcgcgc tggatggcgg cggatctgta cctgcgtgag    900
atcgacacgc tgaggtttga gctgtccatg aaccagtgca gtgcggcggt gtggaagatg    960
gcccgccgca tcatcgccga gggccacacc aagcgcgccg gcgtggtccg ggccaaggcc   1020
gccgccgccc tgcaccagac cgcaacagac gcagccagcc atggcggctc cgccgcctcg   1080
gcggccgccg ccgccgccgc aggcggcgac gtcgtcgctg acggcaccag cggcggcggc   1140
gctgccgccg ccgccggccc cgccgccgct gccgcggcgg acgacgcgtt caccttcagc   1200
cggttggggc ggccccggcc ggagcggccg agcacggacg tgcggagtgt gggcgtgctg   1260
gcgggcggcg agggcgcggc gttcccgggc ggcatgatcc tgggcacgca gccggtgtcg   1320
gcgcacacgg cggcggaggt gtcggtgccg cacgagctgc cgggacagga cgtggagggc   1380
ggctcggaga tggacttcaa cgagtcccgc cgcgcctcgg acgccggaga cctgggcgcc   1440
tcgcagcacc ccatgctggg cgggccctcc gccggcgcct ccgccgagcc caccgcccac   1500
ggctacacca ccaccgccac cgccgccgcc gccgccgccg acggcacgca gcccgagccc   1560
gaagtccccg gcacgcccag ctacgccgac cccggcaccc ccgaccgcct tggcgcgctg   1620
cccggcccct tcacgcccgg ccccacgccc ttccgcgagg cggccaacgc cgccatgagc   1680
accgccgcca gcggcggcgc gggcggcggc ggcggcggcg gcgcgaaccg cgcggcctcg   1740
ggcctgggcg gcgacccccac cttcacgcgt cgcagcctga tggcgcagcg gctgggaacc   1800
agctcggtgc agttcgcgcg cgcgcacgag caccccggct tccaccccta ccgcatcgtg   1860
ttgggccacg tgcgcgacag gctggcggcc acgcggcggc gcatggagga cctgctgagc   1920
ggccgcgagc cggcgggcga ggcgcacggc ggcgtggggg cgggcggcgg cggcggcggc   1980
ggcgcggcgc cgtggtatga gagcgaggac gagctggcgg agccgctgat ggcgtgctac   2040
tggtcgctgt gggagtgcgg cggcggcgtc attgcggacg ggcggctgct ggacctcatc   2100
cgccgcgtgt acaccttcgg catgtgcctc atgaagctag acctgcgcca ggagtccacc   2160
cgccacgccg aggccctgga cgccgtgacc agctacctgg gtctgggctc ctacctggag   2220
tggagcgagg accagaaaat cgagtggctc acaaaggagc tgcagggccg gcggccgctc   2280
atccccgccg acatgcccat gagcgccgag gtgcgcgagg tgctggacac cttcaaggtg   2340
gccgcccatc tgggccgtga caacctgggc gcctacgtca tctccatgac caagggcgcc   2400
tcggacgtga tggcggtgga gctgctgcag cgcgaggcgc gcatgcaggt gggcgccgag   2460
gcgggcggcc gcggcggcgg cgggcccgag gacggcggct cgctgcgcgt ggtgccgctg   2520
ttcgagacgc tggaggacct ggacgcggcg gaggacgtga tgacgcggct gctgaccaac   2580
ccctggtacc gcgagcacct gcgggcagtg cacggcgacg cgcaggaggt gatgctggga   2640
tacagcgaca gcggcaagga cgccggccgc ctggctgcca actgggcgct gtacaagtgt   2700
caggagcggc tggtggccat caccaaggcc aacaacgtca agctcacgct gttccacggc   2760
cgcggcggca ccgtgggccg cggcggcggg cccacccaca tcgccatcca gtcgcagccg   2820
cccggctctg tggaggggac cttccgcatc accgagcagg gcgagatggt ccaggccaag   2880
ttcggcatca gcggcgtggc tctgagccag ctggagacct acaccaccgc tgtgctgctg   2940
gccaccatgc gcccgcccag cccgccgcgc cgcgaggagt ggcgcgccgt gatggagatg   3000
ctcagccgcg tcagctgcga gagctaccgc aacatcgtgc accacagccc gctcttcctg   3060
cgctacttca agcacgccac gcccgaggcc gagctgggca acctctacat cggctctcgc   3120
ccggctcggc ggcgcaacaa ggacgcctcc atctccacgc tgcgcgccat cccctggatc   3180
ttcgcctgga cgcagaaccg cctcatcctg ccctcctggc tgggcattgg cgccgccctc   3240
accgcggcca tgacccaggg ccacctgccc acgctgcagg ccatgtaccg cgagtggccc   3300
ttcttcggat ccacagtgga tctgattgag atgatcctgg ccaagaccga cccgcgcatc   3360
gcggcgctgt acgaggaggt gctggtcaac gaccccgagg agaagaagct gggagccgag   3420
ctgcgcgagc ggctgcagcg ctgccagggc gccatcctca aggtgaccgg gcacgagaac   3480
ctgctgtcca acaaccccac attgagcaag ctcatctcca tgcgctcgcc cttcgtggac   3540
cccatcaaca tcctgcaggt ggaggtgctg cggcgcctgc gccaggaccc caacaacatg   3600
cgcctgcgcg acgcgctgct catctccatc aacggcatcg ccgccggcat gcgcaacacc   3660
ggctaagcag cggcggcgct gctgctggtg atggaggtgg aggtggagat ggacgtggtg   3720
cgcaccaggc acaaggcgga ggcaggtgcc agtggaggtg gggcgtcccg ggcggaggca   3780
tgagcggcgt cgacggctgg cgccgcggta tgatgtgctg cggcacgagc gacggcacgt   3840
gcggtctaaa gctgctggat gtgcttggca cggcgcgcaa gaggggtgtg ctgggggcgg   3900
aggtggtgct gaaaatgttt ggttagttgg ttggatgttt gggctagctg gtgcgctgga   3960
gcgggtaagg caagtggtgg gtggggtatg cgggtgcgct ggggtgtcca gggcggctgc   4020
gatcaatgcg ggttggtggg caaccccgtc ccggcgctga atcgctgcgt ggatctgacc   4080
tttgcggaat tcctgcctgc cttccgcgcc tggcaattta ggaactcgtt tgagcgtttg   4140
acgttgtcct agggcggcat gaccgagcat cgcgtgcatg gcagcggggc tgcgatcgcg   4200
cgtggtgagc cacagacgtt gatagtgata tttgaggcag cccagaacgc atggcggaat   4260
ggcgtggtct ttgagagctg agtgtgtcag actggcatag ggcgctgtgg ctgactaacg   4320
tcccgcacgt gtgcgactgc agaccatgtt tgcaggggca ggtactttac ttgatgtgca   4380
tggcattctc acataaagct ccgaaggatc atttgtggcg caccaaccag agacctagcc   4440
cagagcagag tgtcagcagc gatatcaaag tcattttgtg ggtggttgtc tgggtactcc   4500
gaggcgcgta cgttgagcgc gtgctcgctg cataccgcat acggaatcgg ataagtgaaa   4560
```

```
ggaggtcaca tggccagggg ctgcagatgc tggtgagggc tgcctgccta agcggcgtgt   4620
ccgggacggc agggaagcac gcatgccatt cgacgtgtgc ggtgcaaatg agaccgtgga   4680
gtcctcgggc agggcttgac agcacaagcg tggagcgcgc acacagcgca cagactatgt   4740
tttccttgtg ctctttaaca cagcgcacag aaaaaa                             4776

<210>  18
<211>  1221
<212>  PRT
<213>  Chlamydomonas reinhardtii

<400>  18
Met Thr Asp Ser Thr Tyr Asp Phe Gly Ala Val Arg Asp Asp Leu Thr
1               5                   10                  15
Pro Leu Glu Asp Asp Cys Lys Leu Leu Gly Ser Leu Leu Asp Asp Cys
            20                  25                  30
Leu Arg Val Glu Ile Gly Glu Thr Met Phe Lys Lys Ile Glu Arg Ile
        35                  40                  45
Arg Ala Leu Ala Gln Cys Ala Ser Asn Leu Ser Ile Lys Gly Asp Ala
    50                  55                  60
Gly Ala Ser Asp Met Leu Ser His Arg Leu Ala Glu Glu Leu Met Asn
65                  70                  75                  80
Leu Asp Met Asp Glu Ala Val Pro Leu Thr Arg Ala Cys Gly His Tyr
                85                  90                  95
Leu Asn Leu Ser Gly Ile Ala Glu Leu His His Gly Val Arg Arg Asp
            100                 105                 110
Arg Ala Thr Arg Glu Pro Asn Pro Asn Ser Cys Asp Ala Val Phe Ala
        115                 120                 125
Arg Leu Ile Thr Glu Gly Val Asp Pro Glu Glu Leu Tyr Arg Ala Val
    130                 135                 140
Ser Glu Gln Asn Val Glu Val Val Leu Thr Ala His Pro Thr Gln Val
145                 150                 155                 160
Asn Arg Arg Thr Leu Gln Tyr Lys His Thr Arg Ile Ala Ala Leu Leu
                165                 170                 175
Gln Gln His Asp Arg Ser Asp Leu Thr Ala Glu Glu Arg Arg Asn Met
            180                 185                 190
Val Ser Glu Leu Gln Arg Glu Val Ala Ala Leu Trp Gln Thr Asp Glu
        195                 200                 205
Leu Arg Arg Gln Lys Pro Thr Pro Leu Asp Glu Ala Arg Gly Gly Leu
    210                 215                 220
His Ile Val Glu Gln Ser Leu Trp Ala Ala Val Pro Gln Tyr Met Arg
225                 230                 235                 240
Arg Leu Ser Ala Ala Leu Lys Lys His Thr Gly His Asp Leu Pro Leu
                245                 250                 255
Gln Ala Thr Pro Phe Arg Phe Gly Ser Trp Met Gly Gly Asp Arg Asp
            260                 265                 270
Gly Asn Pro Asn Val Thr Ala Lys Val Thr Ala His Val Thr Ala Leu
        275                 280                 285
Ala Arg Trp Met Ala Ala Asp Leu Tyr Leu Arg Glu Ile Asp Thr Leu
    290                 295                 300
Arg Phe Glu Leu Ser Met Asn Gln Cys Ser Ala Ala Val Trp Lys Met
305                 310                 315                 320
Ala Arg Arg Ile Ile Ala Glu Gly His Thr Lys Arg Ala Gly Val Val
                325                 330                 335
Arg Ala Lys Ala Ala Ala Ala Leu His Gln Thr Ala Thr Asp Ala Ala
            340                 345                 350
Ser His Gly Gly Ser Ala Ala Ser Ala Ala Ala Ala Ala Ala Ala Gly
        355                 360                 365
Gly Asp Val Val Ala Asp Gly Thr Ser Gly Gly Gly Ala Ala Ala Ala
    370                 375                 380
Ala Gly Pro Ala Ala Ala Ala Ala Ala Asp Asp Ala Phe Thr Phe Ser
385                 390                 395                 400
Arg Leu Gly Arg Pro Arg Pro Glu Arg Pro Ser Thr Asp Val Arg Ser
                405                 410                 415
Val Gly Val Leu Ala Gly Gly Glu Gly Ala Ala Phe Pro Gly Gly Met
            420                 425                 430
Ile Leu Gly Thr Gln Pro Val Ser Ala His Thr Ala Ala Glu Val Ser
        435                 440                 445
Val Pro His Glu Leu Pro Gly Gln Asp Val Glu Gly Gly Ser Glu Met
    450                 455                 460
Asp Phe Asn Glu Ser Arg Arg Ala Ser Asp Ala Gly Asp Leu Gly Ala
465                 470                 475                 480
Ser Gln His Pro Met Leu Gly Gly Pro Ser Ala Gly Ala Ser Ala Glu
                485                 490                 495
Pro Thr Ala His Gly Tyr Thr Thr Thr Ala Thr Ala Ala Ala Ala Ala
```

500 505 510
Ala Asp Gly Thr Gln Pro Glu Pro Glu Val Pro Gly Thr Pro Ser Tyr
515 520 525
Ala Asp Pro Gly Thr Pro Asp Arg Leu Gly Ala Leu Pro Gly Pro Phe
530 535 540
Thr Pro Gly Pro Thr Pro Phe Arg Glu Ala Ala Asn Ala Ala Met Ser
545 550 555 560
Thr Ala Ala Ser Gly Gly Ala Gly Gly Gly Gly Gly Gly Gly Ala Asn
565 570 575
Arg Ala Ala Ser Gly Leu Gly Gly Asp Pro Thr Phe Thr Arg Arg Ser
580 585 590
Leu Met Ala Gln Arg Leu Gly Thr Ser Ser Val Gln Phe Ala Arg Ala
595 600 605
His Glu His Pro Gly Phe His Pro Tyr Arg Ile Val Leu Gly His Val
610 615 620
Arg Asp Arg Leu Ala Ala Thr Arg Arg Arg Met Glu Asp Leu Leu Ser
625 630 635 640
Gly Arg Glu Pro Ala Gly Glu Ala His Gly Gly Val Gly Ala Gly Gly
645 650 655
Gly Gly Gly Gly Gly Ala Ala Pro Trp Tyr Glu Ser Glu Asp Glu Leu
660 665 670
Ala Glu Pro Leu Met Ala Cys Tyr Trp Ser Leu Trp Glu Cys Gly Gly
675 680 685
Gly Val Ile Ala Asp Gly Arg Leu Leu Asp Leu Ile Arg Arg Val Tyr
690 695 700
Thr Phe Gly Met Cys Leu Met Lys Leu Asp Leu Arg Gln Glu Ser Thr
705 710 715 720
Arg His Ala Glu Ala Leu Asp Ala Val Thr Ser Tyr Leu Gly Leu Gly
725 730 735
Ser Tyr Leu Glu Trp Ser Glu Asp Gln Lys Ile Glu Trp Leu Thr Lys
740 745 750
Glu Leu Gln Gly Arg Arg Pro Leu Ile Pro Ala Asp Met Pro Met Ser
755 760 765
Ala Glu Val Arg Glu Val Leu Asp Thr Phe Lys Val Ala Ala His Leu
770 775 780
Gly Arg Asp Asn Leu Gly Ala Tyr Val Ile Ser Met Thr Lys Gly Ala
785 790 795 800
Ser Asp Val Met Ala Val Glu Leu Leu Gln Arg Glu Ala Arg Met Gln
805 810 815
Val Gly Ala Glu Ala Gly Gly Arg Gly Gly Gly Gly Pro Glu Asp Gly
820 825 830
Gly Ser Leu Arg Val Val Pro Leu Phe Glu Thr Leu Glu Asp Leu Asp
835 840 845
Ala Ala Glu Asp Val Met Thr Arg Leu Leu Thr Asn Pro Trp Tyr Arg
850 855 860
Glu His Leu Arg Ala Val His Gly Asp Ala Gln Glu Val Met Leu Gly
865 870 875 880
Tyr Ser Asp Ser Gly Lys Asp Ala Gly Arg Leu Ala Ala Asn Trp Ala
885 890 895
Leu Tyr Lys Cys Gln Glu Arg Leu Val Ala Ile Thr Lys Ala Asn Asn
900 905 910
Val Lys Leu Thr Leu Phe His Gly Arg Gly Gly Thr Val Gly Arg Gly
915 920 925
Gly Gly Pro Thr His Ile Ala Ile Gln Ser Gln Pro Pro Gly Ser Val
930 935 940
Glu Gly Thr Phe Arg Ile Thr Glu Gln Gly Glu Met Val Gln Ala Lys
945 950 955 960
Phe Gly Ile Ser Gly Val Ala Leu Ser Gln Leu Glu Thr Tyr Thr Thr
965 970 975
Ala Val Leu Leu Ala Thr Met Arg Pro Pro Ser Pro Pro Arg Arg Glu
980 985 990
Glu Trp Arg Ala Val Met Glu Met Leu Ser Arg Val Ser Cys Glu Ser
995 1000 1005
Tyr Arg Asn Ile Val His His Ser Pro Leu Phe Leu Arg Tyr Phe
1010 1015 1020
Lys His Ala Thr Pro Glu Ala Glu Leu Gly Asn Leu Tyr Ile Gly
1025 1030 1035
Ser Arg Pro Ala Arg Arg Arg Asn Lys Asp Ala Ser Ile Ser Thr
1040 1045 1050
Leu Arg Ala Ile Pro Trp Ile Phe Ala Trp Thr Gln Asn Arg Leu
1055 1060 1065
Ile Leu Pro Ser Trp Leu Gly Ile Gly Ala Ala Leu Thr Ala Ala
1070 1075 1080
Met Thr Gln Gly His Leu Pro Thr Leu Gln Ala Met Tyr Arg Glu

```
    1085                    1090                    1095
Trp Pro  Phe Phe Gly Ser Thr  Val Asp Leu Ile Glu  Met Ile Leu
    1100                    1105                    1110
Ala Lys  Thr Asp Pro Arg Ile  Ala Ala Leu Tyr Glu  Glu Val Leu
    1115                    1120                    1125
Val Asn  Asp Pro Glu Glu Lys  Lys Leu Gly Ala Glu  Leu Arg Glu
    1130                    1135                    1140
Arg Leu  Gln Arg Cys Gln Gly  Ala Ile Leu Lys Val  Thr Gly His
    1145                    1150                    1155
Glu Asn  Leu Leu Ser Asn Asn  Pro Thr Leu Ser Lys  Leu Ile Ser
    1160                    1165                    1170
Met Arg  Ser Pro Phe Val Asp  Pro Ile Asn Ile Leu  Gln Val Glu
    1175                    1180                    1185
Val Leu  Arg Arg Leu Arg Gln  Asp Pro Asn Asn Met  Arg Leu Arg
    1190                    1195                    1200
Asp Ala  Leu Leu Ile Ser Ile  Asn Gly Ile Ala Ala  Gly Met Arg
    1205                    1210                    1215
Asn Thr  Gly
    1220

<210>  19
<211>  3099
<212>  DNA
<213>  Glycine max

<400>  19
atgactgaca tcactggtga tattgctgag gaaatctcct tccagagctt cgatgatgac     60
tgcaggttgc ttggtaatct cctcaatgac attctccagc gtgaagttgg caccaacttg    120
cttgacaaga tcgaaaggac tcgagtcctt gctcagagtg gttgtaatat gaggcaggcg    180
ggtattgtaa acatggcaga gatgcttgag aagcagttgg cttcggagtt atcaaagatg    240
acactagaag aagctttcac ccttgctcgt gccttcagcc attatcttac tttgatgggt    300
atagctgaga cccaccatag ggttcgtaaa ggagggaata tggcacaaat tgcaaaatct    360
tgcgatgata tatttaacca gctggtgcag ggtggagtcc ccccagaaga actttatgac    420
acagtctgca agcgggaggt tgaaattgtt ctcactgctc atcccacaca gattaaccgt    480
cgaaccttac agtttaaaca cattagaatt gctcatcttt tggattacaa tgatcgacct    540
gatcttagca ctgaagatcg agaaatggtg attgaagatc tggtgagaga gataacttca    600
atatggcaga cagatgagct taggcgccag aaacccactc cagttgatga agctagagct    660
ggtttcaata ttgtggagca gtcactctgg aaagctgtcc ctcattattt acgtcgtgtc    720
agcaatgcat taaagaagca tacaggaaag ccacttcctt tgacttgcac tcccataaag    780
tttggatctt ggatgggagg tgatagagat ggaaacccaa atgtgacagc aaaggtcaca    840
aaagatgttt cacttctatc tagatggatg gcgattgacc tctatattcg ggaagtggat    900
agcctcagat ttgagctatc catgaaccag tgcagtgata ggttgtcaag attggcacat    960
gaaattctag aagccaagca tgagaatcgc cgtgagaatt ggaatcagtc tgcgaataga   1020
agtctcacac ttccaacaca acttccagct agagctcatt taccttctat tgctgaaaat   1080
ggtgaatctc ggcatcccag actagacatt ccagcacctg attacatgca atccaatcac   1140
aaggatggtg ggtttctgt aagttcaact acatcaaaac ttgccaatcc caatactcga    1200
ttaccaggaa caagttcagc aaattccagt gcttcttcag ctgcacttgg tcaaaagaaa   1260
ttgtatgcag aatcccagac aggaaagtcc acttttcaaa agcttttgga gccaatgctt   1320
cctcaacttc ctggaattgc tccttataga attgtcctgg ggaatgttaa ggataagctt   1380
gagaaaagtc gtagacggtt agaaattctt cttgaggatg ttgcatgtga ctatgatcct   1440
ttggattact atgaaacatc tgatcagctt ttggaacctc tgctcctctg ttatgaatct   1500
ctgcaatcgt gtggatctgg ggtgctagct gatggtcgac ttgctgatct gattcgtaga   1560
gttgctacct ttggaatggt gttaatgaag cttgacttgc gtcaggaatc tgggagacat   1620
gcagaagcac ttgatgcaat aacacagtac ttagatatgg gtacttacag tgaatgggat   1680
gaagaaaaga agttggactt cttaacaaga gaacttaaag ggaagaggcc tcttgttcct   1740
gttagtatag aggttcatcc tgatgttaaa gaagtcttgg atacattccg aattgccgct   1800
gaactgggga gtgattcact tggagcttat gtgatctcta tggcctcaaa tgcaagtgat   1860
gtccttgcag tagagctttt acagaaggat gcacggcttg ctgctattgg ggagttggga   1920
aaagcatgtc ctggtggaac gttgcgggtt gtccctctgt ttgaaactgt gaaagacctg   1980
agaggagctg gttcagttat ccggaaactt ttatcaatag actggtacca tgaacacatc   2040
gttaagaacc ataatggaca tcaagaggtt atggttggat attctgattc tggtaaagat   2100
gctggtcgct ttactgctgc ttgggaactt tacaaagctc aggaggatgt tgtagctgct   2160
tgcaatgatt atggaataaa ggttactcta ttccatggtc gtggaggcag tattggtcgt   2220
ggtggtggcc caacatatct ggctattcag tcccaacccc ctggctctgt gatgggaacg   2280
cttcggtcta ctgagcaggg agagatggta gaggctaagt ttgggttgcc acagatagct   2340
gttagacaac ttgagatata cacaacagct gtactacttg caaccccttcg tccacctatc   2400
ccaccccgag aagaaaaatg gcgtaatgtc atggaagaga tctcaaacat cagttgtcag   2460
tgtgaccgca atgtagtgta tgaaaatcca gaattcctgg cctacttcca tgaagccaca   2520
ccagaggcag aacttggctt ccttaacata ggtagccgcc ctacaagaag gaagagctca   2580
gtaggaatcg gacaccttcg tgcaattccc tggttatttg catggacaca aacaagattc   2640
gttcttccag cttggcttgg agtcggagca ggtttaaaag gagcttgcga gaaaggttac   2700
accgaagagc taaaagccat gtacaaagaa tggcccttct ttcaaagtac catagatctt   2760
attgagatgg ttttggggaa agctgacatt cctatagcca agcactatga tgaagtcctt   2820
gtgacaaagg agaggcaaga gcttggccat gaactaagaa gtgagctcat gacagctgaa   2880
```

```
aagtttgtca tggttattag tgggcacgag aaacttcagc agaataatag gagcttgagg   2940
aggctaattg agaatagact tcccttcctt aatcccttga acatgttgca ggtggagata   3000
ctcaagaggt taagacgtga tgatgacaac cgtaagatca gagatgcttt gcttatcacc   3060
ataaatggga ttgctgcagg gatgaagaat acaggttga                          3099

<210>  20
<211>  1032
<212>  PRT
<213>  Glycine max

<400>  20
Met Thr Asp Ile Thr Gly Asp Ile Ala Glu Glu Ile Ser Phe Gln Ser
1               5                   10                  15
Phe Asp Asp Asp Cys Arg Leu Leu Gly Asn Leu Leu Asn Asp Ile Leu
            20                  25                  30
Gln Arg Glu Val Gly Thr Asn Leu Leu Asp Lys Ile Glu Arg Thr Arg
        35                  40                  45
Val Leu Ala Gln Ser Gly Cys Asn Met Arg Gln Ala Gly Ile Val Asn
    50                  55                  60
Met Ala Glu Met Leu Glu Lys Gln Leu Ala Ser Glu Leu Ser Lys Met
65                  70                  75                  80
Thr Leu Glu Glu Ala Phe Thr Leu Ala Arg Ala Phe Ser His Tyr Leu
                85                  90                  95
Thr Leu Met Gly Ile Ala Glu Thr His His Arg Val Arg Lys Gly Gly
            100                 105                 110
Asn Met Ala Gln Ile Ala Lys Ser Cys Asp Asp Ile Phe Asn Gln Leu
        115                 120                 125
Val Gln Gly Gly Val Pro Pro Glu Glu Leu Tyr Asp Thr Val Cys Lys
    130                 135                 140
Arg Glu Val Glu Ile Val Leu Thr Ala His Pro Thr Gln Ile Asn Arg
145                 150                 155                 160
Arg Thr Leu Gln Phe Lys His Ile Arg Ile Ala His Leu Leu Asp Tyr
                165                 170                 175
Asn Asp Arg Pro Asp Leu Ser Thr Glu Asp Arg Glu Met Val Ile Glu
            180                 185                 190
Asp Leu Val Arg Glu Ile Thr Ser Ile Trp Gln Thr Asp Glu Leu Arg
        195                 200                 205
Arg Gln Lys Pro Thr Pro Val Asp Glu Ala Arg Ala Gly Phe Asn Ile
    210                 215                 220
Val Glu Gln Ser Leu Trp Lys Ala Val Pro His Tyr Leu Arg Arg Val
225                 230                 235                 240
Ser Asn Ala Leu Lys Lys His Thr Gly Lys Pro Leu Pro Leu Thr Cys
                245                 250                 255
Thr Pro Ile Lys Phe Gly Ser Trp Met Gly Gly Asp Arg Asp Gly Asn
            260                 265                 270
Pro Asn Val Thr Ala Lys Val Thr Lys Asp Val Ser Leu Leu Ser Arg
        275                 280                 285
Trp Met Ala Ile Asp Leu Tyr Ile Arg Glu Val Asp Ser Leu Arg Phe
    290                 295                 300
Glu Leu Ser Met Asn Gln Cys Ser Asp Arg Leu Ser Arg Leu Ala His
305                 310                 315                 320
Glu Ile Leu Glu Ala Lys His Glu Asn Arg Arg Glu Asn Trp Asn Gln
                325                 330                 335
Ser Ala Asn Arg Ser Leu Thr Leu Pro Thr Gln Leu Pro Ala Arg Ala
            340                 345                 350
His Leu Pro Ser Ile Ala Glu Asn Gly Glu Ser Arg His Pro Arg Leu
        355                 360                 365
Asp Ile Pro Ala Pro Asp Tyr Met Gln Ser Asn His Lys Asp Gly Gly
    370                 375                 380
Val Ser Val Ser Ser Thr Thr Ser Lys Leu Ala Asn Pro Asn Thr Arg
385                 390                 395                 400
Leu Pro Gly Thr Ser Ser Ala Asn Ser Ser Ala Ser Ser Ala Ala Leu
                405                 410                 415
Gly Gln Lys Lys Leu Tyr Ala Glu Ser Gln Thr Gly Lys Ser Thr Phe
            420                 425                 430
Gln Lys Leu Leu Glu Pro Met Leu Pro Gln Leu Pro Gly Ile Ala Pro
        435                 440                 445
Tyr Arg Ile Val Leu Gly Asn Val Lys Asp Lys Leu Glu Lys Ser Arg
    450                 455                 460
Arg Arg Leu Glu Ile Leu Leu Glu Asp Val Ala Cys Asp Tyr Asp Pro
465                 470                 475                 480
Leu Asp Tyr Tyr Glu Thr Ser Asp Gln Leu Leu Glu Pro Leu Leu Leu
                485                 490                 495
Cys Tyr Glu Ser Leu Gln Ser Cys Gly Ser Gly Val Leu Ala Asp Gly
```

```
            500                 505                 510
Arg Leu Ala Asp Leu Ile Arg Arg Val Ala Thr Phe Gly Met Val Leu
        515                 520                 525
Met Lys Leu Asp Leu Arg Gln Glu Ser Gly Arg His Ala Glu Ala Leu
    530                 535                 540
Asp Ala Ile Thr Gln Tyr Leu Asp Met Gly Thr Tyr Ser Glu Trp Asp
545                 550                 555                 560
Glu Glu Lys Lys Leu Asp Phe Leu Thr Arg Glu Leu Lys Gly Lys Arg
                565                 570                 575
Pro Leu Val Pro Val Ser Ile Glu Val His Pro Asp Val Lys Glu Val
            580                 585                 590
Leu Asp Thr Phe Arg Ile Ala Ala Glu Leu Gly Ser Asp Ser Leu Gly
        595                 600                 605
Ala Tyr Val Ile Ser Met Ala Ser Asn Ala Ser Asp Val Leu Ala Val
    610                 615                 620
Glu Leu Leu Gln Lys Asp Ala Arg Leu Ala Ala Ile Gly Glu Leu Gly
625                 630                 635                 640
Lys Ala Cys Pro Gly Gly Thr Leu Arg Val Val Pro Leu Phe Glu Thr
                645                 650                 655
Val Lys Asp Leu Arg Gly Ala Gly Ser Val Ile Arg Lys Leu Leu Ser
            660                 665                 670
Ile Asp Trp Tyr His Glu His Ile Val Lys Asn His Asn Gly His Gln
        675                 680                 685
Glu Val Met Val Gly Tyr Ser Asp Ser Gly Lys Asp Ala Gly Arg Phe
    690                 695                 700
Thr Ala Ala Trp Glu Leu Tyr Lys Ala Gln Glu Asp Val Val Ala Ala
705                 710                 715                 720
Cys Asn Asp Tyr Gly Ile Lys Val Thr Leu Phe His Gly Arg Gly Gly
                725                 730                 735
Ser Ile Gly Arg Gly Gly Gly Pro Thr Tyr Leu Ala Ile Gln Ser Gln
            740                 745                 750
Pro Pro Gly Ser Val Met Gly Thr Leu Arg Ser Thr Glu Gln Gly Glu
        755                 760                 765
Met Val Glu Ala Lys Phe Gly Leu Pro Gln Ile Ala Val Arg Gln Leu
    770                 775                 780
Glu Ile Tyr Thr Thr Ala Val Leu Leu Ala Thr Leu Arg Pro Pro Ile
785                 790                 795                 800
Pro Pro Arg Glu Glu Lys Trp Arg Asn Val Met Glu Glu Ile Ser Asn
                805                 810                 815
Ile Ser Cys Gln Cys Asp Arg Asn Val Val Tyr Glu Asn Pro Glu Phe
            820                 825                 830
Leu Ala Tyr Phe His Glu Ala Thr Pro Glu Ala Glu Leu Gly Phe Leu
        835                 840                 845
Asn Ile Gly Ser Arg Pro Thr Arg Arg Lys Ser Ser Val Gly Ile Gly
    850                 855                 860
His Leu Arg Ala Ile Pro Trp Leu Phe Ala Trp Thr Gln Thr Arg Phe
865                 870                 875                 880
Val Leu Pro Ala Trp Leu Gly Val Gly Ala Gly Leu Lys Gly Ala Cys
                885                 890                 895
Glu Lys Gly Tyr Thr Glu Glu Leu Lys Ala Met Tyr Lys Glu Trp Pro
            900                 905                 910
Phe Phe Gln Ser Thr Ile Asp Leu Ile Glu Met Val Leu Gly Lys Ala
        915                 920                 925
Asp Ile Pro Ile Ala Lys His Tyr Asp Glu Val Leu Val Thr Lys Glu
    930                 935                 940
Arg Gln Glu Leu Gly His Glu Leu Arg Ser Glu Leu Met Thr Ala Glu
945                 950                 955                 960
Lys Phe Val Met Val Ile Ser Gly His Glu Lys Leu Gln Gln Asn Asn
                965                 970                 975
Arg Ser Leu Arg Arg Leu Ile Glu Asn Arg Leu Pro Phe Leu Asn Pro
            980                 985                 990
Leu Asn Met Leu Gln Val Glu Ile  Leu Lys Arg Leu Arg  Arg Asp Asp
        995                 1000                1005
Asp Asn  Arg Lys Ile Arg Asp  Ala Leu Leu Ile Thr  Ile Asn Gly
    1010                1015                1020
Ile Ala  Ala Gly Met Lys Asn  Thr Gly
    1025                1030
```

<210>  21
<211>  3159
<212>  DNA
<213>  Ricinus communis

<400>  21

```
atgacggaca ccacagatga tattgcagag gaaatctcat ttcagagttt tgatgatgat     60
tgtaagcttc ttggcaattt gttaaacgat gttttgcaga gagaagttgg ttctaaattc    120
atggagaagc ttgaacgcaa tcgcatccta gctcagagtg cttgtaatat gagattggcg    180
gggatagaag ataccgctga attgctggag aagcagctgg cattggagat atcaaggatg    240
acattagagg aagcattgac acttgctcgt gcctttagtc attaccttaa tttgatgggc    300
attgctgaga cccatcacag ggttcgtaag gcacggagta tgacacatct atcaaaatct    360
tgtgatgaca tatttaatca gctgctgcag agtggcatat ctgcagagga gctttatgac    420
acagtttgca agcaggaggt tgaaattgtg ctcactgcac atcctactca aattaatcgt    480
cgtaccttgc aatataagca catcagaatt gctcatcttt tagattataa tgaccggcct    540
gatcttactc atgaagatcg agaaatgctg attgaagatc tggtgagaga aattacttca    600
atatggcaga cagatgagct taggcgccac aagcctacac cagtagatga agctagggct    660
ggcttgaaca ttgtggagca gtccctgtgg aaagctcttc cccattattt acgtcgtgtc    720
agtactgccc taaagaagca tacaggaaag ccacttcctt tgacttgcac gccaataagg    780
tttgggtctt ggatgggggg tgatagagat ggtaatccga atgtaacagc aaaggtcaca    840
agagatgttt ctcttttatc taggtggatg gctgttgacc tttacattcg agaagttgat    900
agcctgagat ttgaactatc catggttcaa tgcagtgata gattgttgaa agtggcaaat    960
gacattttaa tagaagaaac ttcatctgaa gatcaccatg agagttggaa tcaacctgca   1020
agcagaagtc aaacaaagtt tcctagaaaa tctcttccta cacaacttcc acctagagct   1080
gatcttcctg cttgcactga atgtaatgat ggtgaatctc agtatcccaa actagaactt   1140
ccaggaactg attacatgcc ctttaatcgc caggaagctc taggttcttc atattcagaa   1200
tcttcatccc aggatatcaa tcatggttta cctaaaacaa ctggaaatgg aagtgttgct   1260
aattctagtg gatctccgcg ggcatctttc agctctgctc aactggttgc acagaggaaa   1320
ctatttgcag aatccaagat aggaagatct agcttccaga agcttctaga accaagtctg   1380
cctcaacgtc ctggaattgc tccttataga attgttcttg gaaatgtaaa agataagctt   1440
atgaggacaa gaagacgtct ggaacttctt ctagaggatc ttccttgtga atatgatcaa   1500
tgggattact atgagacaac agatcaatta ttggatccac tgcttctgtg ctacgagtct   1560
cttcaatcat gtggagctgg ggttctagct gatggtcggc tagctgacct gataagaaga   1620
gttgctacat ttgggatggt gttaatgaag ctagacttgc gccaggaatc tggtagacat   1680
gctgatactc ttgatgcaat caccaaatat ttggaaatgg gcacgtatag tgagtgggat   1740
gaagaaaaga agctggaatt tctgacaaga gaactgaaag gaaaacggcc actggttcct   1800
cctactattg aggttgctcc tgatgttaaa gaagtcttgg atgctttccg tgttgctgct   1860
gagctgggga gtgattcact tggagcttat gtgatttcta tggcatccaa tgcaagtgat   1920
gtccttgctg tggagcttct gcagaaggat gcccggcttg ctgttagtgg ggagctagga   1980
agaccatgcc ctggtggaac gttgcgagta gttccgttgt ttgaaactgt gaaagacctg   2040
agaggtgctg gttcggtgat cagaaaactg ttatcaattg actggtatag agaacacata   2100
attaagaacc ataacgggca tcaggaggtg atggttggct attctgattc tggtaaagat   2160
gctggacgct ttactgctgc atgggaactt tacaaagccc aagaggatgt tgtggcagcc   2220
tgtaatgatt ttggtatcaa agtaacacta ttccatgggc gtggagggag tattggtcgt   2280
ggtggtggcc ccacatatct tgccattcaa tcccaaccac ctggttcagt tatgggtact   2340
cttcggtcaa ctgagcaagg agaaatggtg caggccaagt ttgggctgcc acatactgcc   2400
atcagacaac tagagatata cacaactgct gtgctgcttg caacccttcg tcctccacat   2460
ccacctcgag aagaacaatg gcgcaatgtc atggaggaga tttcaaaaat cagttgccag   2520
aattaccgga gcacagtcta tgaaaaccca gaattcctcg cctacttcca tgaggctact   2580
ccccaggctg agcttggctt tcttaacata ggaagccgcc ccacaagaag aaagagctca   2640
actggtattg gacatcttcg tgccattcca tgggtattcg catggaccca gaccagattt   2700
gttcttcctg cttggcttgg agttggagca ggtttaaagg gtgcttgtga gaagggattt   2760
actgaagact tgaaagcaat gtacaaagaa tggcctttct tccaatctac tattgatctt   2820
atagagatgg tactagggaa ggcagacatt cctatagcca agcactatga tgaagttctt   2880
gtatctgaga gcaggcgaga gcttggagct gagcttagga gtgagctctt aacaacagaa   2940
aagtatgtgt tggtggttag tgggcatgag aaactatctc agaacaaccg cagcctgcgg   3000
aggctaatag agagcaggct gccttatctc aatcctatga atatgttgca ggtggaggtt   3060
ctaaagaggt tgaggcggga cgacgacaac aataagctca gagatgccct gcttattacc   3120
ataaatggga ttgctgctgg aatgaggaac acaggctga                          3159
```

<210> 22
<211> 1052
<212> PRT
<213> Ricinus communis

<400> 22

```
Met Thr Asp Thr Thr Asp Asp Ile Ala Glu Glu Ile Ser Phe Gln Ser
1               5                   10                  15
Phe Asp Asp Asp Cys Lys Leu Leu Gly Asn Leu Leu Asn Asp Val Leu
            20                  25                  30
Gln Arg Glu Val Gly Ser Lys Phe Met Glu Lys Leu Glu Arg Asn Arg
        35                  40                  45
Ile Leu Ala Gln Ser Ala Cys Asn Met Arg Leu Ala Gly Ile Glu Asp
    50                  55                  60
Thr Ala Glu Leu Leu Glu Lys Gln Leu Ala Leu Glu Ile Ser Arg Met
65                  70                  75                  80
Thr Leu Glu Glu Ala Leu Thr Leu Ala Arg Ala Phe Ser His Tyr Leu
                85                  90                  95
Asn Leu Met Gly Ile Ala Glu Thr His His Arg Val Arg Lys Ala Arg
            100                 105                 110
```

```
Ser Met Thr His Leu Ser Lys Ser Cys Asp Asp Ile Phe Asn Gln Leu
        115                 120                 125
Leu Gln Ser Gly Ile Ser Ala Glu Glu Leu Tyr Asp Thr Val Cys Lys
    130                 135                 140
Gln Glu Val Glu Ile Val Leu Thr Ala His Pro Thr Gln Ile Asn Arg
145                 150                 155                 160
Arg Thr Leu Gln Tyr Lys His Ile Arg Ile Ala His Leu Leu Asp Tyr
                165                 170                 175
Asn Asp Arg Pro Asp Leu Thr His Glu Asp Arg Glu Met Leu Ile Glu
            180                 185                 190
Asp Leu Val Arg Glu Ile Thr Ser Ile Trp Gln Thr Asp Glu Leu Arg
        195                 200                 205
Arg His Lys Pro Thr Pro Val Asp Glu Ala Arg Ala Gly Leu Asn Ile
    210                 215                 220
Val Glu Gln Ser Leu Trp Lys Ala Leu Pro His Tyr Leu Arg Arg Val
225                 230                 235                 240
Ser Thr Ala Leu Lys Lys His Thr Gly Lys Pro Leu Pro Leu Thr Cys
                245                 250                 255
Thr Pro Ile Arg Phe Gly Ser Trp Met Gly Gly Asp Arg Asp Gly Asn
            260                 265                 270
Pro Asn Val Thr Ala Lys Val Thr Arg Asp Val Ser Leu Leu Ser Arg
        275                 280                 285
Trp Met Ala Val Asp Leu Tyr Ile Arg Glu Val Asp Ser Leu Arg Phe
    290                 295                 300
Glu Leu Ser Met Val Gln Cys Ser Asp Arg Leu Leu Lys Val Ala Asn
305                 310                 315                 320
Asp Ile Leu Ile Glu Glu Thr Ser Ser Glu Asp His His Glu Ser Trp
                325                 330                 335
Asn Gln Pro Ala Ser Arg Ser Gln Thr Lys Phe Pro Arg Lys Ser Leu
            340                 345                 350
Pro Thr Gln Leu Pro Pro Arg Ala Asp Leu Pro Ala Cys Thr Glu Cys
        355                 360                 365
Asn Asp Gly Glu Ser Gln Tyr Pro Lys Leu Glu Leu Pro Gly Thr Asp
    370                 375                 380
Tyr Met Pro Phe Asn Arg Gln Glu Ala Leu Gly Ser Ser Tyr Ser Glu
385                 390                 395                 400
Ser Ser Ser Gln Asp Ile Asn His Gly Leu Pro Lys Thr Thr Gly Asn
                405                 410                 415
Gly Ser Val Ala Asn Ser Ser Gly Ser Pro Arg Ala Ser Phe Ser Ser
            420                 425                 430
Ala Gln Leu Val Ala Gln Arg Lys Leu Phe Ala Glu Ser Lys Ile Gly
        435                 440                 445
Arg Ser Ser Phe Gln Lys Leu Leu Glu Pro Ser Leu Pro Gln Arg Pro
    450                 455                 460
Gly Ile Ala Pro Tyr Arg Ile Val Leu Gly Asn Val Lys Asp Lys Leu
465                 470                 475                 480
Met Arg Thr Arg Arg Arg Leu Glu Leu Leu Leu Glu Asp Leu Pro Cys
                485                 490                 495
Glu Tyr Asp Gln Trp Asp Tyr Tyr Glu Thr Thr Asp Gln Leu Leu Asp
            500                 505                 510
Pro Leu Leu Leu Cys Tyr Glu Ser Leu Gln Ser Cys Gly Ala Gly Val
        515                 520                 525
Leu Ala Asp Gly Arg Leu Ala Asp Leu Ile Arg Arg Val Ala Thr Phe
    530                 535                 540
Gly Met Val Leu Met Lys Leu Asp Leu Arg Gln Glu Ser Gly Arg His
545                 550                 555                 560
Ala Asp Thr Leu Asp Ala Ile Thr Lys Tyr Leu Glu Met Gly Thr Tyr
                565                 570                 575
Ser Glu Trp Asp Glu Glu Lys Lys Leu Glu Phe Leu Thr Arg Glu Leu
            580                 585                 590
Lys Gly Lys Arg Pro Leu Val Pro Pro Thr Ile Glu Val Ala Pro Asp
        595                 600                 605
Val Lys Glu Val Leu Asp Ala Phe Arg Val Ala Ala Glu Leu Gly Ser
    610                 615                 620
Asp Ser Leu Gly Ala Tyr Val Ile Ser Met Ala Ser Asn Ala Ser Asp
625                 630                 635                 640
Val Leu Ala Val Glu Leu Leu Gln Lys Asp Ala Arg Leu Ala Val Ser
                645                 650                 655
Gly Glu Leu Gly Arg Pro Cys Pro Gly Gly Thr Leu Arg Val Val Pro
            660                 665                 670
Leu Phe Glu Thr Val Lys Asp Leu Arg Gly Ala Gly Ser Val Ile Arg
        675                 680                 685
Lys Leu Leu Ser Ile Asp Trp Tyr Arg Glu His Ile Ile Lys Asn His
    690                 695                 700
```

Asn Gly His Gln Glu Val Met Val Gly Tyr Ser Asp Ser Gly Lys Asp
705 710 715 720
Ala Gly Arg Phe Thr Ala Ala Trp Glu Leu Tyr Lys Ala Gln Glu Asp
725 730 735
Val Val Ala Ala Cys Asn Asp Phe Gly Ile Lys Val Thr Leu Phe His
740 745 750
Gly Arg Gly Gly Ser Ile Gly Arg Gly Gly Gly Pro Thr Tyr Leu Ala
755 760 765
Ile Gln Ser Gln Pro Pro Gly Ser Val Met Gly Thr Leu Arg Ser Thr
770 775 780
Glu Gln Gly Glu Met Val Gln Ala Lys Phe Gly Leu Pro His Thr Ala
785 790 795 800
Ile Arg Gln Leu Glu Ile Tyr Thr Thr Ala Val Leu Leu Ala Thr Leu
805 810 815
Arg Pro Pro His Pro Pro Arg Glu Glu Gln Trp Arg Asn Val Met Glu
820 825 830
Glu Ile Ser Lys Ile Ser Cys Gln Asn Tyr Arg Ser Thr Val Tyr Glu
835 840 845
Asn Pro Glu Phe Leu Ala Tyr Phe His Glu Ala Thr Pro Gln Ala Glu
850 855 860
Leu Gly Phe Leu Asn Ile Gly Ser Arg Pro Thr Arg Arg Lys Ser Ser
865 870 875 880
Thr Gly Ile Gly His Leu Arg Ala Ile Pro Trp Val Phe Ala Trp Thr
885 890 895
Gln Thr Arg Phe Val Leu Pro Ala Trp Leu Gly Val Gly Ala Gly Leu
900 905 910
Lys Gly Ala Cys Glu Lys Gly Phe Thr Glu Asp Leu Lys Ala Met Tyr
915 920 925
Lys Glu Trp Pro Phe Phe Gln Ser Thr Ile Asp Leu Ile Glu Met Val
930 935 940
Leu Gly Lys Ala Asp Ile Pro Ile Ala Lys His Tyr Asp Glu Val Leu
945 950 955 960
Val Ser Glu Ser Arg Arg Glu Leu Gly Ala Glu Leu Arg Ser Glu Leu
965 970 975
Leu Thr Thr Glu Lys Tyr Val Leu Val Val Ser Gly His Glu Lys Leu
980 985 990
Ser Gln Asn Asn Arg Ser Leu Arg Arg Leu Ile Glu Ser Arg Leu Pro
995 1000 1005
Tyr Leu Asn Pro Met Asn Met Leu Gln Val Glu Val Leu Lys Arg
1010 1015 1020
Leu Arg Arg Asp Asp Asp Asn Asn Lys Leu Arg Asp Ala Leu Leu
1025 1030 1035
Ile Thr Ile Asn Gly Ile Ala Ala Gly Met Arg Asn Thr Gly
1040 1045 1050

<210> 23
<211> 3350
<212> DNA
<213> Arabidopsis thaliana

<400> 23
acacttgtta gatttacttt tcctccgcac tatcagcaga tttttctcat ctgggtctgt 60
ttgtttctgt tgactcaaag tttccgactt tcttattttc tgtctccgat ttcatcgccg 120
aaatctcagt caattttgtc tttagatctt taatttttgg atccctttaa ttattttgtt 180
tggtcttact tcagagcgaa gcaggtgaaa aaatggcgaa tcggaagtta gagaagatgg 240
catcgattga tgttcatctt cgtcaactgg ttcctggcaa agttagtgaa gacgacaagc 300
ttgttgagta tgatgctttg cttctagatc ggtttctcga tatcctccag gatttgcacg 360
gtgaagatct ccgtgaaact gttcaagagc tttatgagca ttctgcagaa tacgaaggga 420
agcatgaacc taagaagcta gaggagctag ggagtgtgct aacgagttta gatccaggag 480
attccattgt tatcgctaaa gctttctctc atatgcttaa cttagccaat ttggctgagg 540
aagtgcagat tgcttatcgc cgtaggatca agaagctgaa gaaaggtgat tttgttgatg 600
agagctctgc tactactgaa tctgatcttg aagaaacttt caagaagctt gttggagatc 660
tgaacaagtc tcctgaagag atctttgatg ctctcaagaa tcagactgtg gatttggttt 720
tgactgctca tcctactcag tctgtgagaa gatcattgct tcagaaacat gggaggataa 780
gagactgtct ggctcaacta tatgctaagg atattactcc tgatgacaag caagagctcg 840
atgaggctct tcagagagag attcaagctg cattccgaac agatgaaatc aaaagaacac 900
cacctactcc tcaagatgag atgagagcgg gaatgagtta tttccatgaa actatctgga 960
aaggtgttcc taagtttctg cgccgtgttg acacggcttt gaaaaacata gggatcgaag 1020
aacgtgttcc atataatgct ccattgattc agttctcttc ttggatgggt ggtgatcgtg 1080
acggtaaccc aagggttaca cctgaagtca ccagagatgt ttgcttgtta gctagaatga 1140
tggctgctac tatgtacttt aaccaaatcg aagatcttat gtttgagatg tctatgtggc 1200
gttgcaatga cgagctgcgt gcgcgagctg atgaagttca tgcaaattcg aggaaagatg 1260
ctgcaaaaca ttacatagaa ttctggaagt caattcctac aactgagcca taccgtgtga 1320
ttcttggcga tgtaagggac aagctttatc acacacgtga acgcgctcat caactgctca 1380

```
gcaatggaca ctctgatgtc cctgtagagg ctactttcat taacttggaa cagttcttgg   1440
aacctcttga gctctgttac cgatctctgt gttcatgtgg tgatcgtcca atagcagatg   1500
gaagccttct tgatttcttg aggcaagtct caacctttgg gctctctctt gtgagacttg   1560
acataaggca agaatctgac cgccacactg atgtattgga tgctatcacc acgcatttag   1620
atatcggatc ctacagagag tggtctgaag aacgccgcca agaatggctt ttatctgagc   1680
taagtggcaa acgtccgctt ttcggttctg atcttcctaa aaccgaagaa atagctgatg   1740
ttctggacac gtttcatgtc atagccgagc taccagcaga tagctttggt gcttacatta   1800
tctctatggc aactgcacct tctgatgtat tagctgttga gcttttacag cgtgaatgcc   1860
gagtgaaaca gcctttgaga gttgttccgc tctttgagaa gctagcagat ctggaagcag   1920
ctcctgctgc agttgctagg ctcttttctg ttgattggta caaaaaccga attaacggta   1980
agcaagaggt tatgattggt tattcggatt caggaaaaga tgctggacgg ttatctgctg   2040
cttggcagtt atacaaagct caagaagagc ttgtgaaggt tgctaaagag tacggtgtga   2100
agctaacaat gtttcacggt cgtggtggca cggtcggaag aggaggtgga ccaacccatc   2160
ttgctatatt gtctcagcct ccggatacta ttaacggttc cctccgtgtc acagttcaag   2220
gtgaagtcat cgagcaatcg tttggtgaag agcacttatg ctttagaaca cttcagcgtt   2280
tcacagctgc tacactcgag cacggtatgc gtcctccaat ttcgcctaaa ccagaatggc   2340
gcgctttgct ggatgaaatg gcggttgttg caaccgagga gtatcgctca gttgtgttcc   2400
aagaacctcg gtttgtcgag tacttccgcc tcgctacacc ggaactggag tatggacgta   2460
tgaatatcgg aagcagacct tcgaagcgta aaccaagcgg tggcattgaa tctctccgtg   2520
caattccatg gatcttcgct tggactcaaa caagattcca tcttcctgta tggcttggat   2580
tcggatcagc aattagacat gtgatcgaaa aagacgtcag gaacctccat atgctccaag   2640
atatgtacca acactggcct ttctttagag tcaccattga tctaatcgaa atggtgttcg   2700
ctaaaggaga tcctggtatt gctgctttgt acgataagct tcttgtttca gaggaactct   2760
ggcctttggg tgagaaactc agagctaact tcgaagaaac caagaaactc atcctccaga   2820
ccgctggaca caaagatctt cttgaaggtg atccttactt gaaacagaga ctgagacttc   2880
gtgattctta cattacaact ctcaatgtct gtcaagctta cacattgaag agaatccgtg   2940
atccgagtta ccatgtgact ctgcgaccac acatttctaa ggagatagcg gaatcgagca   3000
aaccagcaaa agaactcatc gagcttaacc cgactagcga atacgcgcca ggacttgaag   3060
atacactcat cttgacgatg aagggtattg ctgctggtct acaaaacacc ggttaagcta   3120
caaagagatg gttaaacaaa ctttgaatct ctctttctct ctcaagtctc tcctttttttt   3180
aactacagat ttggaaaata aggttggatt ctggtttatt ttatgtatcc accgtcaaaa   3240
tgttgatttt cgtgtacgag tacttcgaga tcattgaaca catgctctgt tttttctca    3300
agtttaataa aacagaacaa gagaatcttt tcttgtttat tttcttatct              3350
```

```
<210>  24
<211>  967
<212>  PRT
<213>  Arabidopsis thaliana

<400>  24
Met Ala Asn Arg Lys Leu Glu Lys Met Ala Ser Ile Asp Val His Leu
1               5                   10                  15
Arg Gln Leu Val Pro Gly Lys Val Ser Glu Asp Asp Lys Leu Val Glu
            20                  25                  30
Tyr Asp Ala Leu Leu Leu Asp Arg Phe Leu Asp Ile Leu Gln Asp Leu
        35                  40                  45
His Gly Glu Asp Leu Arg Glu Thr Val Gln Glu Leu Tyr Glu His Ser
    50                  55                  60
Ala Glu Tyr Glu Gly Lys His Glu Pro Lys Lys Leu Glu Glu Leu Gly
65                  70                  75                  80
Ser Val Leu Thr Ser Leu Asp Pro Gly Asp Ser Ile Val Ile Ala Lys
                85                  90                  95
Ala Phe Ser His Met Leu Asn Leu Ala Asn Leu Ala Glu Glu Val Gln
            100                 105                 110
Ile Ala Tyr Arg Arg Arg Ile Lys Lys Leu Lys Lys Gly Asp Phe Val
        115                 120                 125
Asp Glu Ser Ser Ala Thr Thr Glu Ser Asp Leu Glu Glu Thr Phe Lys
    130                 135                 140
Lys Leu Val Gly Asp Leu Asn Lys Ser Pro Glu Glu Ile Phe Asp Ala
145                 150                 155                 160
Leu Lys Asn Gln Thr Val Asp Leu Val Leu Thr Ala His Pro Thr Gln
                165                 170                 175
Ser Val Arg Arg Ser Leu Leu Gln Lys His Gly Arg Ile Arg Asp Cys
            180                 185                 190
Leu Ala Gln Leu Tyr Ala Lys Asp Ile Thr Pro Asp Asp Lys Gln Glu
        195                 200                 205
Leu Asp Glu Ala Leu Gln Arg Glu Ile Gln Ala Ala Phe Arg Thr Asp
    210                 215                 220
Glu Ile Lys Arg Thr Pro Pro Thr Pro Gln Asp Glu Met Arg Ala Gly
225                 230                 235                 240
Met Ser Tyr Phe His Glu Thr Ile Trp Lys Gly Val Pro Lys Phe Leu
                245                 250                 255
Arg Arg Val Asp Thr Ala Leu Lys Asn Ile Gly Ile Glu Glu Arg Val
            260                 265                 270
```

```
Pro Tyr Asn Ala Pro Leu Ile Gln Phe Ser Ser Trp Met Gly Gly Asp
        275                 280                 285
Arg Asp Gly Asn Pro Arg Val Thr Pro Glu Val Thr Arg Asp Val Cys
    290                 295                 300
Leu Leu Ala Arg Met Met Ala Ala Thr Met Tyr Phe Asn Gln Ile Glu
305                 310                 315                 320
Asp Leu Met Phe Glu Met Ser Met Trp Arg Cys Asn Asp Glu Leu Arg
                325                 330                 335
Ala Arg Ala Asp Glu Val His Ala Asn Ser Arg Lys Asp Ala Ala Lys
            340                 345                 350
His Tyr Ile Glu Phe Trp Lys Ser Ile Pro Thr Thr Glu Pro Tyr Arg
        355                 360                 365
Val Ile Leu Gly Asp Val Arg Asp Lys Leu Tyr His Thr Arg Glu Arg
    370                 375                 380
Ala His Gln Leu Leu Ser Asn Gly His Ser Asp Val Pro Val Glu Ala
385                 390                 395                 400
Thr Phe Ile Asn Leu Glu Gln Phe Leu Glu Pro Leu Glu Leu Cys Tyr
                405                 410                 415
Arg Ser Leu Cys Ser Cys Gly Asp Arg Pro Ile Ala Asp Gly Ser Leu
            420                 425                 430
Leu Asp Phe Leu Arg Gln Val Ser Thr Phe Gly Leu Ser Leu Val Arg
        435                 440                 445
Leu Asp Ile Arg Gln Glu Ser Asp Arg His Thr Asp Val Leu Asp Ala
    450                 455                 460
Ile Thr Thr His Leu Asp Ile Gly Ser Tyr Arg Glu Trp Ser Glu Glu
465                 470                 475                 480
Arg Arg Gln Glu Trp Leu Leu Ser Glu Leu Ser Gly Lys Arg Pro Leu
                485                 490                 495
Phe Gly Ser Asp Leu Pro Lys Thr Glu Glu Ile Ala Asp Val Leu Asp
            500                 505                 510
Thr Phe His Val Ile Ala Glu Leu Pro Ala Asp Ser Phe Gly Ala Tyr
        515                 520                 525
Ile Ile Ser Met Ala Thr Ala Pro Ser Asp Val Leu Ala Val Glu Leu
    530                 535                 540
Leu Gln Arg Glu Cys Arg Val Lys Gln Pro Leu Arg Val Val Pro Leu
545                 550                 555                 560
Phe Glu Lys Leu Ala Asp Leu Glu Ala Ala Pro Ala Ala Val Ala Arg
                565                 570                 575
Leu Phe Ser Val Asp Trp Tyr Lys Asn Arg Ile Asn Gly Lys Gln Glu
            580                 585                 590
Val Met Ile Gly Tyr Ser Asp Ser Gly Lys Asp Ala Gly Arg Leu Ser
        595                 600                 605
Ala Ala Trp Gln Leu Tyr Lys Ala Gln Glu Glu Leu Val Lys Val Ala
    610                 615                 620
Lys Glu Tyr Gly Val Lys Leu Thr Met Phe His Gly Arg Gly Gly Thr
625                 630                 635                 640
Val Gly Arg Gly Gly Gly Pro Thr His Leu Ala Ile Leu Ser Gln Pro
                645                 650                 655
Pro Asp Thr Ile Asn Gly Ser Leu Arg Val Thr Val Gln Gly Glu Val
            660                 665                 670
Ile Glu Gln Ser Phe Gly Glu Glu His Leu Cys Phe Arg Thr Leu Gln
        675                 680                 685
Arg Phe Thr Ala Ala Thr Leu Glu His Gly Met Arg Pro Pro Ile Ser
    690                 695                 700
Pro Lys Pro Glu Trp Arg Ala Leu Leu Asp Glu Met Ala Val Val Ala
705                 710                 715                 720
Thr Glu Glu Tyr Arg Ser Val Val Phe Gln Glu Pro Arg Phe Val Glu
                725                 730                 735
Tyr Phe Arg Leu Ala Thr Pro Glu Leu Glu Tyr Gly Arg Met Asn Ile
            740                 745                 750
Gly Ser Arg Pro Ser Lys Arg Lys Pro Ser Gly Gly Ile Glu Ser Leu
        755                 760                 765
Arg Ala Ile Pro Trp Ile Phe Ala Trp Thr Gln Thr Arg Phe His Leu
    770                 775                 780
Pro Val Trp Leu Gly Phe Gly Ser Ala Ile Arg His Val Ile Glu Lys
785                 790                 795                 800
Asp Val Arg Asn Leu His Met Leu Gln Asp Met Tyr Gln His Trp Pro
                805                 810                 815
Phe Phe Arg Val Thr Ile Asp Leu Ile Glu Met Val Phe Ala Lys Gly
            820                 825                 830
Asp Pro Gly Ile Ala Ala Leu Tyr Asp Lys Leu Leu Val Ser Glu Glu
        835                 840                 845
Leu Trp Pro Phe Gly Glu Lys Leu Arg Ala Asn Phe Glu Glu Thr Lys
    850                 855                 860
```

```
Lys Leu Ile Leu Gln Thr Ala Gly His Lys Asp Leu Leu Glu Gly Asp
865                 870                 875                 880
Pro Tyr Leu Lys Gln Arg Leu Arg Leu Arg Asp Ser Tyr Ile Thr Thr
                885                 890                 895
Leu Asn Val Cys Gln Ala Tyr Thr Leu Lys Arg Ile Arg Asp Pro Ser
            900                 905                 910
Tyr His Val Thr Leu Arg Pro His Ile Ser Lys Glu Ile Ala Glu Ser
        915                 920                 925
Ser Lys Pro Ala Lys Glu Leu Ile Glu Leu Asn Pro Thr Ser Glu Tyr
    930                 935                 940
Ala Pro Gly Leu Glu Asp Thr Leu Ile Leu Thr Met Lys Gly Ile Ala
945                 950                 955                 960
Ala Gly Leu Gln Asn Thr Gly
                965

<210>  25
<211>  3309
<212>  DNA
<213>  Arabidopsis thaliana

<400>  25
gtctcgttta aatttttata aactccataa ttttatctta aagtgaatct tttttgtttt      60
ttttgttcca gctttatcgg atatatttcc ttgattttct ccgattgtgg tcaatctgga     120
aaattattga gaatctctcc ctcacttaac caaaagcgtt tttaatcaga tagagagaga     180
ggaaaaagca tcaaccaaac catggctgcg agaaatttgg agaagatggc ttctattgat     240
gctcagctca ggcttcttgc tcctggcaag gtttctgaag acgacaagct tatcgagtac     300
gatgctctgt tactggatcg atttctcgat attcttcagg atttgcatgg cgaggatgtc     360
agagaattcg ttcaagaatg ctacgaagtt gcagctgatt acgatggaaa ccgcaacact     420
gagaagcttg aggagcttgg aaatatgctg acgagtttgg atccagggga ttcaattgtt     480
gtcactaaat cattctccaa catgcttagc ttggctaatc tggctgagga agtccagatt     540
gcttaccggc gtaggattaa gaaactcaag aaaggtgatt tcgctgatga ggcctctgca     600
acaacggaat ctgacattga agagactctc aagaggcttt tgcagcttaa caagactcct     660
gaagaggtct ttgatgctct taagaatcag actgttgact tggttttaac tgctcatccc     720
actcaatctg ttcgtcggtc tttgctccaa aagtttggaa ggattcgtga ttgtttgacg     780
cagttatatg caaaggacat tactcctgat gacaaacaag aactcgatga agctctgcaa     840
cgagagattc aagctgcttt tcgcacagat gaaatccgaa gaactcctcc tacaccgcaa     900
gatgaaatga gagcagggat gagctacttc catgagacaa tctggaaagg agttccaaag     960
ttcttaagac gtgttgacac agctttaaag aacattggaa tcaacgagcg tgttccttac    1020
aatgcgcctc tcattcagtt ctcttcttgg atgggcggag accgtgatgg aaacccgcga    1080
gtaactcctg aagttacaag agatgtatgc ttattagcta gaatgatggc tgctaatctc    1140
tacttctccc agatagaaga tcttatgttt gagatgtcta tgtggcgttg caatgaggaa    1200
cttcgggttc gtgcagaacg tcaaagatgt gcgaagaggg atgcaaaaca ctatatagaa    1260
ttctggaaac aaatccctgc gaatgagcca taccgagcta ttcttggaga tgtgagggac    1320
aagctgtaca acacacgtga gcgtgcacgt cagttattgt caagcggagt ttcggacgtt    1380
cccgaagacg cggttttcac aagtgtggat cagtttttgg agccacttga gctttgttac    1440
aggtcgctct gtgattgcgg tgacagacct attgctgatg gaagcctgct tgatttctta    1500
cgccaagtgt caacatttgg ccttgctctt gtgaaacttg atatccgtca agaatctgaa    1560
agacactctg atgtcttgga tgccatcacg acgcacttag gtattggttc ttacaaagaa    1620
tggtcggagg ataaaagaca ggaatggctg ttatctgagc taagcgggaa acgccctctc    1680
tttggaccgg atcttcccaa aaccgaagag gttgcagatg tgttggacac tttcaaagtc    1740
atttctgagc ttccttcgga tagttttggt gcttatatta tctcaatggc cactgctcca    1800
tcagacgtgc tcgctgttga gcttttgcaa cgcgaatgcg ggatcactga tcctctgaga    1860
gttgtcccgt tgttcgagaa gctagcggat ttagaatccg cacctgctgc agttgcccgt    1920
ctcttctcca tagaatggta cagaaacagg atcaatggaa agcaagaagt catgatcggg    1980
tactctgact cgggcaaaga tgctggtcgt ttatcagcgg cttggcagtt atacaagact    2040
caagaagagc tcgtgaaggt ggcaaaagaa tacggagtca agctgacaat gttccacgga    2100
agaggtggga ccgttggacg aggaggtgga cctacccatc ttgctatttt gtctcagcct    2160
ccggatacca ttcatgggca attgagggta acggttcaag gtgaagttat tgaacagtct    2220
ttcggagaag agcacttatg ctttaggact cttcagcgtt tcacagctgc aacacttgag    2280
catggaatgc atccaccggt ttcccctaag cctgagtggc gtgtcctcat ggatgaaatg    2340
gctataattg ccactgaaga ataccgttct gttgtcttca aggagccccg ttttgttgag    2400
tacttccgtc tggcaacacc agagctcgag tatggaagga tgaacatagg aagccgacca    2460
tcaaaacgta aaccaagcgg aggaatcgag tcgctgcgtg caatcccgtg gatctttgcg    2520
tggactcaga cgaggttcac ttacccgtgt ggcttggctt tggaggagca ttcaaacgcg    2580
tgatacagaa ggacagtaag aatctcaaca tgctcaaaga gatgtacaac caatggccat    2640
tcttccgtgt cacaattgat ctagtcgaaa tggttttcgc caaaggagat cccggaattg    2700
cggctctgta tgaccgcctc ctcgtctctg aagaacttca accattcggt gaacaacttc    2760
gagttaacta ccaagagacc agacgcctcc tcctccaggt tgcaggtcac aaagacattt    2820
tagaaggtga cccttacttg aggcaaaggc tgcagcttcg tgacccatac atcacgacat    2880
tgaacgtgtg tcaagcctat acactcaagc agatccgtga cccaagcttc cacgtcaaag    2940
tccggccaca tctctctaag gactacatgg agtctagtcc agcggctgag ctcgtgaaac    3000
tgaatccaaa gagtgaatac gcaccgggac ttgaagatac ggttatcctc accatgaagg    3060
gtatcgctgc tggtatgcaa aacaccggtt aaggcagttt aaaacgttct tgtaccattc    3120
cctaaatcta cgctatgtaa tgtattatgt tctatgatgt gatgaaatct ctccaactcc    3180
```

```
tatcccgtac gctttttaat gagtatgata atttcttgtg ttattctatt gttgttatgt   3240
taccatatct aggaaatata tttctgaaag aaacaagaaa agaaatcttt cttttcgttc   3300
taagatgtt                                                           3309

<210>  26
<211>  793
<212>  PRT
<213>  Arabidopsis thaliana

<400>  26
Met Ala Ala Arg Asn Leu Glu Lys Met Ala Ser Ile Asp Ala Gln Leu
1               5                   10                  15
Arg Leu Leu Ala Pro Gly Lys Val Ser Glu Asp Asp Lys Leu Ile Glu
            20                  25                  30
Tyr Asp Ala Leu Leu Leu Asp Arg Phe Leu Asp Ile Leu Gln Asp Leu
        35                  40                  45
His Gly Glu Asp Val Arg Glu Phe Val Gln Glu Cys Tyr Glu Val Ala
    50                  55                  60
Ala Asp Tyr Asp Gly Asn Arg Asn Thr Glu Lys Leu Glu Glu Leu Gly
65                  70                  75                  80
Asn Met Leu Thr Ser Leu Asp Pro Gly Asp Ser Ile Val Val Thr Lys
                85                  90                  95
Ser Phe Ser Asn Met Leu Ser Leu Ala Asn Leu Ala Glu Glu Val Gln
            100                 105                 110
Ile Ala Tyr Arg Arg Arg Ile Lys Lys Leu Lys Lys Gly Asp Phe Ala
        115                 120                 125
Asp Glu Ala Ser Ala Thr Thr Glu Ser Asp Ile Glu Glu Thr Leu Lys
    130                 135                 140
Arg Leu Leu Gln Leu Asn Lys Thr Pro Glu Glu Val Phe Asp Ala Leu
145                 150                 155                 160
Lys Asn Gln Thr Val Asp Leu Val Leu Thr Ala His Pro Thr Gln Ser
                165                 170                 175
Val Arg Arg Ser Leu Leu Gln Lys Phe Gly Arg Ile Arg Asp Cys Leu
            180                 185                 190
Thr Gln Leu Tyr Ala Lys Asp Ile Thr Pro Asp Asp Lys Gln Glu Leu
        195                 200                 205
Asp Glu Ala Leu Gln Arg Glu Ile Gln Ala Ala Phe Arg Thr Asp Glu
    210                 215                 220
Ile Arg Arg Thr Pro Pro Thr Pro Gln Asp Glu Met Arg Ala Gly Met
225                 230                 235                 240
Ser Tyr Phe His Glu Thr Ile Trp Lys Gly Val Pro Lys Phe Leu Arg
                245                 250                 255
Arg Val Asp Thr Ala Leu Lys Asn Ile Gly Ile Asn Glu Arg Val Pro
            260                 265                 270
Tyr Asn Ala Pro Leu Ile Gln Phe Ser Ser Trp Met Gly Gly Asp Arg
        275                 280                 285
Asp Gly Asn Pro Arg Val Thr Pro Glu Val Thr Arg Asp Val Cys Leu
    290                 295                 300
Leu Ala Arg Met Met Ala Ala Asn Leu Tyr Phe Ser Gln Ile Glu Asp
305                 310                 315                 320
Leu Met Phe Glu Met Ser Met Trp Arg Cys Asn Glu Glu Leu Arg Val
                325                 330                 335
Arg Ala Glu Arg Gln Arg Cys Ala Lys Arg Asp Ala Lys His Tyr Ile
            340                 345                 350
Glu Phe Trp Lys Gln Ile Pro Ala Asn Glu Pro Tyr Arg Ala Ile Leu
        355                 360                 365
Gly Asp Val Arg Asp Lys Leu Tyr Asn Thr Arg Glu Arg Ala Arg Gln
    370                 375                 380
Leu Leu Ser Ser Gly Val Ser Asp Val Pro Glu Asp Ala Val Phe Thr
385                 390                 395                 400
Ser Val Asp Gln Phe Leu Glu Pro Leu Glu Leu Cys Tyr Arg Ser Leu
                405                 410                 415
Cys Asp Cys Gly Asp Arg Pro Ile Ala Asp Gly Ser Leu Leu Asp Phe
            420                 425                 430
Leu Arg Gln Val Ser Thr Phe Gly Leu Ala Leu Val Lys Leu Asp Ile
        435                 440                 445
Arg Gln Glu Ser Glu Arg His Ser Asp Val Leu Asp Ala Ile Thr Thr
    450                 455                 460
His Leu Gly Ile Gly Ser Tyr Lys Glu Trp Ser Glu Asp Lys Arg Gln
465                 470                 475                 480
Glu Trp Leu Leu Ser Glu Leu Ser Gly Lys Arg Pro Leu Phe Gly Pro
                485                 490                 495
Asp Leu Pro Lys Thr Glu Glu Val Ala Asp Val Leu Asp Thr Phe Lys
            500                 505                 510
```

```
Val Ile Ser Glu Leu Pro Ser Asp Ser Phe Gly Ala Tyr Ile Ile Ser
        515                 520                 525
Met Ala Thr Ala Pro Ser Asp Val Leu Ala Val Glu Leu Leu Gln Arg
    530                 535                 540
Glu Cys Gly Ile Thr Asp Pro Leu Arg Val Val Pro Leu Phe Glu Lys
545                 550                 555                 560
Leu Ala Asp Leu Glu Ser Ala Pro Ala Ala Val Ala Arg Leu Phe Ser
                565                 570                 575
Ile Glu Trp Tyr Arg Asn Arg Ile Asn Gly Lys Gln Glu Val Met Ile
            580                 585                 590
Gly Tyr Ser Asp Ser Gly Lys Asp Ala Gly Arg Leu Ser Ala Ala Trp
        595                 600                 605
Gln Leu Tyr Lys Thr Gln Glu Glu Leu Val Lys Val Ala Lys Glu Tyr
    610                 615                 620
Gly Val Lys Leu Thr Met Phe His Gly Arg Gly Gly Thr Val Gly Arg
625                 630                 635                 640
Gly Gly Gly Pro Thr His Leu Ala Ile Leu Ser Gln Pro Pro Asp Thr
                645                 650                 655
Ile His Gly Gln Leu Arg Val Thr Val Gln Gly Glu Val Ile Glu Gln
            660                 665                 670
Ser Phe Gly Glu Glu His Leu Cys Phe Arg Thr Leu Gln Arg Phe Thr
        675                 680                 685
Ala Ala Thr Leu Glu His Gly Met His Pro Pro Val Ser Pro Lys Pro
    690                 695                 700
Glu Trp Arg Val Leu Met Asp Glu Met Ala Ile Ile Ala Thr Glu Glu
705                 710                 715                 720
Tyr Arg Ser Val Val Phe Lys Glu Pro Arg Phe Val Glu Tyr Phe Arg
                725                 730                 735
Leu Ala Thr Pro Glu Leu Glu Tyr Gly Arg Met Asn Ile Gly Ser Arg
            740                 745                 750
Pro Ser Lys Arg Lys Pro Ser Gly Gly Ile Glu Ser Leu Arg Ala Ile
        755                 760                 765
Pro Trp Ile Phe Ala Trp Thr Gln Thr Arg Phe Thr Tyr Pro Cys Gly
    770                 775                 780
Leu Ala Leu Glu Glu His Ser Asn Ala
785                 790
```

<210> 27
<211> 3231
<212> DNA
<213> Arabidopsis thaliana

<400> 27

```
ccacgcgtcc gaatccgcag agatttcttc ttttcagaag aagtaagagg gtggcgaaga     60
agatttgatt gatcggcgat aatggcgggt cggaacatag agaagatggc atctattgat    120
gctcagcttc ggcaactcgt tcctgctaaa gtcagtgaag acgataagct tgttgagtac    180
gatgctcttc tccttgatcg ctttctcgac attctccagg atttacacgg cgaggatctc    240
cgtgaaacgg ttcaagagtt atacgagctt tctgctgagt atgaagggaa gcgtgagcct    300
agcaagcttg aggagctagg gagtgtccta acgagtttgg atcctggtga ctcaattgtt    360
atctccaagg ctttctctca catgcttaac ttagccaatt tggctgagga ggtgcagatt    420
gctcaccgtc gcaggatcaa gaagctgaag aaaggtgatt tcgttgatga gagttctgca    480
actactgaat ccgatattga agagactttt aagaggctcg tttcggatct tggtaagtct    540
cctgaagaga tctttgatgc cttgaagaat cagactgtgg atctggtttt gactgctcat    600
cctactcagt ctgtgcgtag atcattgctt cagaagcatg gaggataag ggactgtctt    660
gctcaactct atgcaaagga cattactcct gatgacaagc aggagctaga tgagtctctg    720
caaagagaga ttcaagctgc attccgaaca gatgagatta gaagaacacc tccaacccca    780
caagatgaaa tgagagctgg aatgagttat ttccacgaga caatctggaa aggtgtcccc    840
aagttcttgc gccgtgtgga cacagctctg aaaaacattg ggattgatga acgtgttcct    900
tacaatgccc cattgattca attctcttcg tggatgggcg gtgatcgtga tggtaatccg    960
agggtcacac ctgaggtcac tagagatgtg tgcttgttgg ctagaatgat ggctgccaat   1020
ctctactata accaaatcga gaatctgatg tttgagttat ctatgtggcg ttgcactgat   1080
gaattccgtg tgcgggcgga tgaactgcac aggaactcaa ggaaagatgc tgcaaaacat   1140
tacatagaat tctggaagac aattcctcca actgagccat accgtgtgat tcttggtgat   1200
gtgagggata agctgtatca cacacgtgag cgttcccgcc aattgctgag taatggaatc   1260
tcggatattc ctgaagaagc taccttcact aatgtggaac agttcttgga gcctcttgag   1320
ctctgttacc gatcactatg ttcatgtggt gacagcccga tagctgatgg aagccttctt   1380
gatttcttga ggcaagtctc tacctttgga ctctcccttg tgagacttga catcaggcaa   1440
gagtctgaac gccacacaga tgtcttggat gctatcacca agcacttgga catcggttcc   1500
tcctatagag actggtctga agaaggccga caggaatggc ttcttgctga actaagcggc   1560
aaacgtccac ttttcggacc tgatcttccc aaaaccgaag aaatttctga tgtcctggac   1620
acattcaaag tcatatctga gctgccttca gattgttttg gagcttatat tatctctatg   1680
gcaacttcac ctagtgatgt gcttgcggtt gagcttttac agcgcgaatg ccatgtgaaa   1740
aatccactta gagttgttcc actctttgag aagctagctg atcttgaagc agctcctgcc   1800
gctgttgcaa gactcttttc tatagactgg tacaaaaacc gtattaacgg taaacaagag   1860
```

```
gttatgattg gttactcaga ttcagggaaa gatgcagggc gtctctcagc tgcttgggag   1920
ctatacaaag ctcaagaaga gcttgtgaag gttgctaaga aatatggagt gaagctaact   1980
atgttccatg gccgtggtgg cacagtcgga agaggaggtg gtcctactca tcttgctata   2040
ttgtctcagc caccagatac agttaatggc tctcttcgag tcacggttca gggtgaagtc   2100
attgagcaat catttgggga ggcacactta tgctttagaa cacttcaacg tttcacagca   2160
gctactctag agcacggaat gaaccctccg atttcaccaa aacccgagtg gcgtgctttg   2220
cttgatgaaa tggcggttgt tgcaactgag gaataccgat ctgtcgtttt ccaagaacct   2280
cgattcgtcg agtatttccg cctcgctact ccggagctgg agtatggacg tatgaatatt   2340
ggaagtagac cttcaaagcg aaaaccaagc ggtgggatcg aatctctccg tgcaatccca   2400
tggatctttg cttggacgca aacaagattc catcttcctg tatggttagg tttcggagca   2460
gcatttaggt atgcgatcaa gaaggatgtg agaaaccttc acatgctgca agatatgtat   2520
aaacaatggc ccttttttccg agtcaccatc gatctaattg aaatggtgtt cgccaaggga   2580
gaccccggga tcgctgcttt gtacgacaaa cttcttgtct cagaagattt atgggctttt   2640
ggagagaaac tcagagccaa ctttgatgaa accaagaacc tcgtcctcca gactgctgga   2700
cataaagacc ttcttgaagg agatccttac ttgaaacaga gactaaggct acgtgactct   2760
tacattacga ccctcaacgt ttgccaagcc tacacattga agaggatccg tgatgcaaac   2820
tacaatgtga ctctgcgacc acacatttct aaagagatca tgcaatcaag caaatcagca   2880
caagagctcg tcaagcttaa ccccacgagt gaatacgcgc ctggacttga ggacacactt   2940
atcttaacca tgaagggtat tgctgcagga ttgcaaaaca ccggttaagt gagtcagtga   3000
aagaaaacaa aacttcgaat ctctcttttt tatctaccct ttttaataat ctcttttttt   3060
ctagaatcca aaataattac ggttggatta cagtttactt tatgtatcca ccgttgaaat   3120
cttaatcttc cattgtatca aacgtcactg actctgtttc tggaagtgta aacaagaaca   3180
gagacagtga atcttaatgt tatcttcttt gtctaaaaaa aaaaaaaaaa a            3231
```

<210> 28
<211> 968
<212> PRT
<213> Arabidopsis thaliana

<400> 28

```
Met Ala Gly Arg Asn Ile Glu Lys Met Ala Ser Ile Asp Ala Gln Leu
1               5                   10                  15
Arg Gln Leu Val Pro Ala Lys Val Ser Glu Asp Asp Lys Leu Val Glu
            20                  25                  30
Tyr Asp Ala Leu Leu Leu Asp Arg Phe Leu Asp Ile Leu Gln Asp Leu
        35                  40                  45
His Gly Glu Asp Leu Arg Glu Thr Val Gln Glu Leu Tyr Glu Leu Ser
    50                  55                  60
Ala Glu Tyr Glu Gly Lys Arg Glu Pro Ser Lys Leu Glu Glu Leu Gly
65                  70                  75                  80
Ser Val Leu Thr Ser Leu Asp Pro Gly Asp Ser Ile Val Ile Ser Lys
                85                  90                  95
Ala Phe Ser His Met Leu Asn Leu Ala Asn Leu Ala Glu Glu Val Gln
            100                 105                 110
Ile Ala His Arg Arg Arg Ile Lys Lys Leu Lys Lys Gly Asp Phe Val
        115                 120                 125
Asp Glu Ser Ser Ala Thr Thr Glu Ser Asp Ile Glu Glu Thr Phe Lys
    130                 135                 140
Arg Leu Val Ser Asp Leu Gly Lys Ser Pro Glu Glu Ile Phe Asp Ala
145                 150                 155                 160
Leu Lys Asn Gln Thr Val Asp Leu Val Leu Thr Ala His Pro Thr Gln
                165                 170                 175
Ser Val Arg Arg Ser Leu Leu Gln Lys His Gly Arg Ile Arg Asp Cys
            180                 185                 190
Leu Ala Gln Leu Tyr Ala Lys Asp Ile Thr Pro Asp Asp Lys Gln Glu
        195                 200                 205
Leu Asp Glu Ser Leu Gln Arg Glu Ile Gln Ala Ala Phe Arg Thr Asp
    210                 215                 220
Glu Ile Arg Arg Thr Pro Pro Thr Pro Gln Asp Glu Met Arg Ala Gly
225                 230                 235                 240
Met Ser Tyr Phe His Glu Thr Ile Trp Lys Gly Val Pro Lys Phe Leu
                245                 250                 255
Arg Arg Val Asp Thr Ala Leu Lys Asn Ile Gly Ile Asp Glu Arg Val
            260                 265                 270
Pro Tyr Asn Ala Pro Leu Ile Gln Phe Ser Ser Trp Met Gly Gly Asp
        275                 280                 285
Arg Asp Gly Asn Pro Arg Val Thr Pro Glu Val Thr Arg Asp Val Cys
    290                 295                 300
Leu Leu Ala Arg Met Met Ala Ala Asn Leu Tyr Tyr Asn Gln Ile Glu
305                 310                 315                 320
Asn Leu Met Phe Glu Leu Ser Met Trp Arg Cys Thr Asp Glu Phe Arg
                325                 330                 335
Val Arg Ala Asp Glu Leu His Arg Asn Ser Arg Lys Asp Ala Ala Lys
            340                 345                 350
```

His Tyr Ile Glu Phe Trp Lys Thr Ile Pro Pro Thr Glu Pro Tyr Arg
        355                 360                 365
Val Ile Leu Gly Asp Val Arg Asp Lys Leu Tyr His Thr Arg Glu Arg
    370                 375                 380
Ser Arg Gln Leu Leu Ser Asn Gly Ile Ser Asp Ile Pro Glu Glu Ala
385                 390                 395                 400
Thr Phe Thr Asn Val Glu Gln Phe Leu Glu Pro Leu Glu Leu Cys Tyr
                405                 410                 415
Arg Ser Leu Cys Ser Cys Gly Asp Ser Pro Ile Ala Asp Gly Ser Leu
            420                 425                 430
Leu Asp Phe Leu Arg Gln Val Ser Thr Phe Gly Leu Ser Leu Val Arg
        435                 440                 445
Leu Asp Ile Arg Gln Glu Ser Glu Arg His Thr Asp Val Leu Asp Ala
    450                 455                 460
Ile Thr Lys His Leu Asp Ile Gly Ser Ser Tyr Arg Asp Trp Ser Glu
465                 470                 475                 480
Glu Gly Arg Gln Glu Trp Leu Leu Ala Glu Leu Ser Gly Lys Arg Pro
                485                 490                 495
Leu Phe Gly Pro Asp Leu Pro Lys Thr Glu Glu Ile Ser Asp Val Leu
            500                 505                 510
Asp Thr Phe Lys Val Ile Ser Glu Leu Pro Ser Asp Cys Phe Gly Ala
        515                 520                 525
Tyr Ile Ile Ser Met Ala Thr Ser Pro Ser Asp Val Leu Ala Val Glu
    530                 535                 540
Leu Leu Gln Arg Glu Cys His Val Lys Asn Pro Leu Arg Val Val Pro
545                 550                 555                 560
Leu Phe Glu Lys Leu Ala Asp Leu Glu Ala Ala Pro Ala Ala Val Ala
                565                 570                 575
Arg Leu Phe Ser Ile Asp Trp Tyr Lys Asn Arg Ile Asn Gly Lys Gln
            580                 585                 590
Glu Val Met Ile Gly Tyr Ser Asp Ser Gly Lys Asp Ala Gly Arg Leu
        595                 600                 605
Ser Ala Ala Trp Glu Leu Tyr Lys Ala Gln Glu Glu Leu Val Lys Val
    610                 615                 620
Ala Lys Lys Tyr Gly Val Lys Leu Thr Met Phe His Gly Arg Gly Gly
625                 630                 635                 640
Thr Val Gly Arg Gly Gly Gly Pro Thr His Leu Ala Ile Leu Ser Gln
                645                 650                 655
Pro Pro Asp Thr Val Asn Gly Ser Leu Arg Val Thr Val Gln Gly Glu
            660                 665                 670
Val Ile Glu Gln Ser Phe Gly Glu Ala His Leu Cys Phe Arg Thr Leu
        675                 680                 685
Gln Arg Phe Thr Ala Ala Thr Leu Glu His Gly Met Asn Pro Pro Ile
    690                 695                 700
Ser Pro Lys Pro Glu Trp Arg Ala Leu Leu Asp Glu Met Ala Val Val
705                 710                 715                 720
Ala Thr Glu Glu Tyr Arg Ser Val Val Phe Gln Glu Pro Arg Phe Val
                725                 730                 735
Glu Tyr Phe Arg Leu Ala Thr Pro Glu Leu Glu Tyr Gly Arg Met Asn
            740                 745                 750
Ile Gly Ser Arg Pro Ser Lys Arg Lys Pro Ser Gly Gly Ile Glu Ser
        755                 760                 765
Leu Arg Ala Ile Pro Trp Ile Phe Ala Trp Thr Gln Thr Arg Phe His
    770                 775                 780
Leu Pro Val Trp Leu Gly Phe Gly Ala Ala Phe Arg Tyr Ala Ile Lys
785                 790                 795                 800
Lys Asp Val Arg Asn Leu His Met Leu Gln Asp Met Tyr Lys Gln Trp
                805                 810                 815
Pro Phe Phe Arg Val Thr Ile Asp Leu Ile Glu Met Val Phe Ala Lys
            820                 825                 830
Gly Asp Pro Gly Ile Ala Ala Leu Tyr Asp Lys Leu Leu Val Ser Glu
        835                 840                 845
Asp Leu Trp Ala Phe Gly Glu Lys Leu Arg Ala Asn Phe Asp Glu Thr
    850                 855                 860
Lys Asn Leu Val Leu Gln Thr Ala Gly His Lys Asp Leu Leu Glu Gly
865                 870                 875                 880
Asp Pro Tyr Leu Lys Gln Arg Leu Arg Leu Arg Asp Ser Tyr Ile Thr
                885                 890                 895
Thr Leu Asn Val Cys Gln Ala Tyr Thr Leu Lys Arg Ile Arg Asp Ala
            900                 905                 910
Asn Tyr Asn Val Thr Leu Arg Pro His Ile Ser Lys Glu Ile Met Gln
        915                 920                 925
Ser Ser Lys Ser Ala Gln Glu Leu Val Lys Leu Asn Pro Thr Ser Glu
    930                 935                 940

Tyr Ala Pro Gly Leu Glu Asp Thr Leu Ile Leu Thr Met Lys Gly Ile
945                 950                 955                 960
Ala Ala Gly Leu Gln Asn Thr Gly
                965

<210> 29
<211> 3102
<212> DNA
<213> Arabidopsis thaliana

<400> 29
acaatgacgg acacaacaga cgatatcgca gaggaaatct cattccaaag cttcgaagat 60
gactgcaaat tgctcggtag tctcttccat gatgtgttac aaagggaagt tggcaaccca 120
ttcatggaaa aagtcgaacg cattcggatt cttgctcaga gtgcgttaaa tttgcgtatg 180
gctggtattg aggataccgc aaaccttttg gagaagcaat tgactagtga aatatccaaa 240
atgccactag aagaagcctt aacgttggct cgtacattca ctcattctct taacttaatg 300
ggcattgcag acactcatca cagaatgcac aaagtccata acgttacaca acttgcaaga 360
tcttgtgatg atatattcag ccagctattg caaagtggaa tctctccaga cgaactttat 420
aaaactgttt gcaaacagga ggtcgaaatt gttcttactg ctcatcctac ccaaataaat 480
cgaagaacct tgcagtacaa gcatattaga attgctcatc ttctagaata taacactaga 540
tcagatctaa gcgttgaaga tcgcgaaacg ctcattgaag atttggttag agagattact 600
tcactgtggc aaactgatga gcttagacgt cagaaaccta ctccagttga tgaagctaga 660
gctggtctaa acatagtgga gcaatccctt tggaaagcag taccacaata cctgcgtcgt 720
gtcagcaatt ccttgaagaa gtttacaggg aagccacttc cactaacatg cactcctatg 780
aaatttggtt cttggatggg aggtgataga gatggaaatc caaatgtcac ggcaaaggtc 840
acgaaagaag tatctctctt gtctagatgg atggctattg atttgtacat aagagaggtt 900
gatagcttaa gatttgaatt atctacggat cgatgcagtg ataggttttc aagattagct 960
gataaaattc ttgaaaagga ttatgataga ggaaaatcaa atttccaaaa gcaacaaagt 1020
tcatcatgct tgccaacaca acttccagct agagctcacc ttcctgcttg cattgacttt 1080
ggtgaatcac gacataccaa atttgaaatt gcgacgacag attatatgcc acccaatctc 1140
cagaagcaga atgaacaaga cttttcggaa agcgactggg agaaaattga caatggttcg 1200
cggtccggtc ttacttctcg aggttctttc tcatctactt ctcaacttct tctccagaga 1260
aaactatttg aggaatctca ggttgggaag actagtttcc aaaagctact agaaccacct 1320
ccacttaaac gagctggaag tgctccttat cgtattgttc ttggagaagt aaaagaaaag 1380
cttgtgaaga caagaagact tcttgaactt cttattgagg gtcttccttg tgagtatgac 1440
cctaaaaact cctatgaaac atcagatcag cttcttgaac cattgctcct ctgttacgaa 1500
tctctgcaat catcgggtgc tagggtacta gctgatggac gacttgctga tctgattcgt 1560
agagtttcta cctttggaat ggttttggtg aaactcgact tacgccagga agctgcaaga 1620
cattctgaag ctttggatgc aattacaaca tacttggata tgggtactta tagtgaatgg 1680
gatgaagaga agaaattaga atttttgaca agagaactaa aagggaaacg acctcttgtt 1740
cctcaatgta ttaaggttgg tcctgacgtc aaagaagtat tggacacatt ccgagtcgct 1800
gctgaacttg gaagtgaatc acttggcgct tacgttattt ctatggcttc aaatgcaagt 1860
gatgtcctcg ctgtggaact tcttcaaaaa gatgctcgac ttgctttaac tagcgaacat 1920
ggaaaaccat gtcctggtgg aacgctacga gtggtacctc tttttgaaac ggtgaatgat 1980
ttaagagccg ctggtccttc gataaggaaa ttgctctcaa tcgattggta tagggaacac 2040
atccaaaaga accacaacgg tcaccaagag gtgatggttg gatactctga ttctggaaaa 2100
gatgctggac gttttactgc agcatgggaa ctctacaaag ctcaagaaaa tgttgttgct 2160
gcttgtaatg aatttggaat caaaataaca ttatttcatg gacgaggagg aagcattggt 2220
cgtggtggtg gtccaaccta tctcgctatt cagtcccaac caccaggctc tgtaatgggc 2280
tctttgcgtt caactgagca aggtgagatg gttcaagcta agtttgggat accacaaacg 2340
gctgttaggc aactagaggt atacacaacc gcggttctac tcgctacctt aaagcctcct 2400
cagccacctc gagaggaaaa atggcgaaac ctaatggaag aaatctctgg aatcagttgc 2460
caacactata gaagcacagt gtatgaaaac ccagagtttc tatcttattt tcatgaggca 2520
acaccgcaag cagaacttgg tttcctcaat ataggaagcc gaccaacacg aagaaagagc 2580
tctagtggaa taggacatct ccgagctatc ccttgggtct ttgcttggac tcaaacaagg 2640
tttgttcttc cagcttggct tggtgtaggg gctggtttaa agggagtttc tgagaagggt 2700
catgcggatg atcttaaaga gatgtacaaa gaatggccat tttttcagtc caccccttgaa 2760
cttatagaga tggtgttagc taaagcagac attccaatga ccaaacacta cgacgaacaa 2820
cttgtgtctg agaaaagaag aggacttggc actgagctaa gaaaagaact aatgactact 2880
gagaagtacg ttcttgtgat aagtggtcac gagaaactct tgcaggacaa taagagcttg 2940
aagaaactca ttgatagtag acttccgtat ctcaacgcaa tgaacatgtt acaagttgaa 3000
attcttaaga ggctaagacg tgatgaagat aacaataagc taagagatgc tttgcttatc 3060
acaatcaatg gtattgctgc aggaatgaga aataccggtt aa 3102

<210> 30
<211> 1032
<212> PRT
<213> Arabidopsis thaliana

<400> 30
Met Thr Asp Thr Thr Asp Asp Ile Ala Glu Glu Ile Ser Phe Gln Ser
1               5                   10                  15
Phe Glu Asp Asp Cys Lys Leu Leu Gly Ser Leu Phe His Asp Val Leu
            20                  25                  30

```
Gln Arg Glu Val Gly Asn Pro Phe Met Glu Lys Val Glu Arg Ile Arg
        35                  40                  45
Ile Leu Ala Gln Ser Ala Leu Asn Leu Arg Met Ala Gly Ile Glu Asp
    50                  55                  60
Thr Ala Asn Leu Leu Glu Lys Gln Leu Thr Ser Glu Ile Ser Lys Met
65                  70                  75                  80
Pro Leu Glu Glu Ala Leu Thr Leu Ala Arg Thr Phe Thr His Ser Leu
                85                  90                  95
Asn Leu Met Gly Ile Ala Asp Thr His His Arg Met His Lys Val His
            100                 105                 110
Asn Val Thr Gln Leu Ala Arg Ser Cys Asp Asp Ile Phe Ser Gln Leu
        115                 120                 125
Leu Gln Ser Gly Ile Ser Pro Asp Glu Leu Tyr Lys Thr Val Cys Lys
    130                 135                 140
Gln Glu Val Glu Ile Val Leu Thr Ala His Pro Thr Gln Ile Asn Arg
145                 150                 155                 160
Arg Thr Leu Gln Tyr Lys His Ile Arg Ile Ala His Leu Leu Glu Tyr
                165                 170                 175
Asn Thr Arg Ser Asp Leu Ser Val Glu Asp Arg Glu Thr Leu Ile Glu
            180                 185                 190
Asp Leu Val Arg Glu Ile Thr Ser Leu Trp Gln Thr Asp Glu Leu Arg
        195                 200                 205
Arg Gln Lys Pro Thr Pro Val Asp Glu Ala Arg Ala Gly Leu Asn Ile
    210                 215                 220
Val Glu Gln Ser Leu Trp Lys Ala Val Pro Gln Tyr Leu Arg Arg Val
225                 230                 235                 240
Ser Asn Ser Leu Lys Lys Phe Thr Gly Lys Pro Leu Pro Leu Thr Cys
                245                 250                 255
Thr Pro Met Lys Phe Gly Ser Trp Met Gly Gly Asp Arg Asp Gly Asn
            260                 265                 270
Pro Asn Val Thr Ala Lys Val Thr Lys Glu Val Ser Leu Leu Ser Arg
        275                 280                 285
Trp Met Ala Ile Asp Leu Tyr Ile Arg Glu Val Asp Ser Leu Arg Phe
    290                 295                 300
Glu Leu Ser Thr Asp Arg Cys Ser Asp Arg Phe Ser Arg Leu Ala Asp
305                 310                 315                 320
Lys Ile Leu Glu Lys Asp Tyr Asp Arg Gly Lys Ser Asn Phe Gln Lys
                325                 330                 335
Gln Gln Ser Ser Ser Cys Leu Pro Thr Gln Leu Pro Ala Arg Ala His
            340                 345                 350
Leu Pro Ala Cys Ile Asp Phe Gly Glu Ser Arg His Thr Lys Phe Glu
        355                 360                 365
Ile Ala Thr Thr Asp Tyr Met Pro Pro Asn Leu Gln Lys Gln Asn Glu
    370                 375                 380
Gln Asp Phe Ser Glu Ser Asp Trp Glu Lys Ile Asp Asn Gly Ser Arg
385                 390                 395                 400
Ser Gly Leu Thr Ser Arg Gly Ser Phe Ser Ser Thr Ser Gln Leu Leu
                405                 410                 415
Leu Gln Arg Lys Leu Phe Glu Glu Ser Gln Val Gly Lys Thr Ser Phe
            420                 425                 430
Gln Lys Leu Leu Glu Pro Pro Pro Leu Lys Arg Ala Gly Ser Ala Pro
        435                 440                 445
Tyr Arg Ile Val Leu Gly Glu Val Lys Glu Lys Leu Val Lys Thr Arg
    450                 455                 460
Arg Leu Leu Glu Leu Leu Ile Glu Gly Leu Pro Cys Glu Tyr Asp Pro
465                 470                 475                 480
Lys Asn Ser Tyr Glu Thr Ser Asp Gln Leu Leu Glu Pro Leu Leu Leu
                485                 490                 495
Cys Tyr Glu Ser Leu Gln Ser Ser Gly Ala Arg Val Leu Ala Asp Gly
            500                 505                 510
Arg Leu Ala Asp Leu Ile Arg Arg Val Ser Thr Phe Gly Met Val Leu
        515                 520                 525
Val Lys Leu Asp Leu Arg Gln Glu Ala Ala Arg His Ser Glu Ala Leu
    530                 535                 540
Asp Ala Ile Thr Thr Tyr Leu Asp Met Gly Thr Tyr Ser Glu Trp Asp
545                 550                 555                 560
Glu Glu Lys Lys Leu Glu Phe Leu Thr Arg Glu Leu Lys Gly Lys Arg
                565                 570                 575
Pro Leu Val Pro Gln Cys Ile Lys Val Gly Pro Asp Val Lys Glu Val
            580                 585                 590
Leu Asp Thr Phe Arg Val Ala Ala Glu Leu Gly Ser Glu Ser Leu Gly
        595                 600                 605
Ala Tyr Val Ile Ser Met Ala Ser Asn Ala Ser Asp Val Leu Ala Val
    610                 615                 620
```

```
Glu Leu Leu Gln Lys Asp Ala Arg Leu Ala Leu Thr Ser Glu His Gly
625                 630                 635                 640
Lys Pro Cys Pro Gly Gly Thr Leu Arg Val Val Pro Leu Phe Glu Thr
                645                 650                 655
Val Asn Asp Leu Arg Ala Ala Gly Pro Ser Ile Arg Lys Leu Leu Ser
            660                 665                 670
Ile Asp Trp Tyr Arg Glu His Ile Gln Lys Asn His Asn Gly His Gln
        675                 680                 685
Glu Val Met Val Gly Tyr Ser Asp Ser Gly Lys Asp Ala Gly Arg Phe
    690                 695                 700
Thr Ala Ala Trp Glu Leu Tyr Lys Ala Gln Glu Asn Val Val Ala Ala
705                 710                 715                 720
Cys Asn Glu Phe Gly Ile Lys Ile Thr Leu Phe His Gly Arg Gly Gly
                725                 730                 735
Ser Ile Gly Arg Gly Gly Gly Pro Thr Tyr Leu Ala Ile Gln Ser Gln
            740                 745                 750
Pro Pro Gly Ser Val Met Gly Ser Leu Arg Ser Thr Glu Gln Gly Glu
        755                 760                 765
Met Val Gln Ala Lys Phe Gly Ile Pro Gln Thr Ala Val Arg Gln Leu
    770                 775                 780
Glu Val Tyr Thr Thr Ala Val Leu Leu Ala Thr Leu Lys Pro Pro Gln
785                 790                 795                 800
Pro Pro Arg Glu Glu Lys Trp Arg Asn Leu Met Glu Glu Ile Ser Gly
                805                 810                 815
Ile Ser Cys Gln His Tyr Arg Ser Thr Val Tyr Glu Asn Pro Glu Phe
            820                 825                 830
Leu Ser Tyr Phe His Glu Ala Thr Pro Gln Ala Glu Leu Gly Phe Leu
        835                 840                 845
Asn Ile Gly Ser Arg Pro Thr Arg Arg Lys Ser Ser Ser Gly Ile Gly
    850                 855                 860
His Leu Arg Ala Ile Pro Trp Val Phe Ala Trp Thr Gln Thr Arg Phe
865                 870                 875                 880
Val Leu Pro Ala Trp Leu Gly Val Gly Ala Gly Leu Lys Gly Val Ser
                885                 890                 895
Glu Lys Gly His Ala Asp Asp Leu Lys Glu Met Tyr Lys Glu Trp Pro
            900                 905                 910
Phe Phe Gln Ser Thr Leu Glu Leu Ile Glu Met Val Leu Ala Lys Ala
        915                 920                 925
Asp Ile Pro Met Thr Lys His Tyr Asp Glu Gln Leu Val Ser Glu Lys
    930                 935                 940
Arg Arg Gly Leu Gly Thr Glu Leu Arg Lys Glu Leu Met Thr Thr Glu
945                 950                 955                 960
Lys Tyr Val Leu Val Ile Ser Gly His Glu Lys Leu Leu Gln Asp Asn
                965                 970                 975
Lys Ser Leu Lys Lys Leu Ile Asp Ser Arg Leu Pro Tyr Leu Asn Ala
            980                 985                 990
Met Asn Met Leu Gln Val Glu Ile  Leu Lys Arg Leu Arg  Arg Asp Glu
        995                 1000                1005
Asp Asn  Asn Lys Leu Arg Asp  Ala Leu Leu Ile Thr  Ile Asn Gly
    1010                1015                1020
Ile Ala  Ala Gly Met Arg Asn  Thr Gly
    1025                1030
```

<210> 31
<211> 6018
<212> DNA
<213> Sorghum bicolor

<400> 31

```
aggcctagta ttgcttcaaa ttctgcggca attcaactca acaaatgctt atcctgagtt      60
tgtttatatg tactccatta gttttattaa agataagttc tcttgattat tctacgtaga     120
tagctttgtt gtacactcat tatattattt tatatataca taatacaaaa taatatttat     180
ctaaaaaaat cagaacaatg tgcacatact aataaattcc catttcaatg ttggaccatg     240
agcagatctg acggtacaca tgttcaagtg tctccgttct cctgtggaaa taataaatcg     300
cagccgtcca cgtgacacac aacacagatg tctctcgatc agtataggct gttcctgttc     360
ttcgctttgg taacaaatcc cctgcaaatt gatcgataat ataagaataa aataacgtgc     420
atctcgtaca ttcgcttgga cctgcctgct aaatgctaat gctaatgcta ccatcttcct     480
cctccgtcta taaagtggcc gccagaatcc gctcctccta caattgcagc agcctcctct     540
cctctcctcc ctgcctggtt cttgcttgca gttcttgaac caaatccaca ccgccggcgc     600
gcttagcatt ggggctagct aggggggccga tcgatatggc ggccttaggg gcgaagatgg     660
agcggctgtc gtcgatcgac gcgcagctgc ggatgctggt gccggggaag gtctcggagg     720
acgacaagct catcgagtac gacgcgctcc tcctcgaccg cttcctcgac atcctccagg     780
acctccacgg cgacgacctc aaggagatgg taactctcct ccgactggtt cttagactct     840
tgttgttctt ttgttttact ggttcgggct tgcgatgcag ctttaatctt gggttggatc     900
```

```
caagtaatct agttcagtcc tgtggtcttt acctgttatt tttaccttta tttaatcttc     960
catgtttttt ttacttattt gtcttctttt tcctgagaaa aaagtatcaa tgtttagtaa    1020
aaaaaaaggc atgaaattat tgccttcttt gttctttgtt ttcttttcgt gcgattcctt    1080
tttagagtgc cagcttgagt agtattgaag agtgtgtggt tgacttcaga gtccactctt    1140
cagattaagt gattaacact tcaggtttaa gtgtaactac aaggtggttg atttgctgct    1200
taatttggcg ttgtcaagta tagtaattat actaaaatat aaattatagt ggatggccag    1260
gtgctggatt aacgttccta gtgttcaatt tcttcttctt cttcttcttt ttgttttgtt    1320
attgctcaaa gattggtttt tcagtggcaa agcgatcatc tcgatttttg attgattggg    1380
tcggatatgg ttcgaatttg atctcaatca ttcggtagaa cacttcctat gtggaaatcc    1440
atctgtaaca agtgtggatc ttagttgtat atatgttcct ggtccattaa ctgactttgg    1500
accgattgat tgactgaaac aacgaaacgc tgcagattcc acgtcatctt ttgagttctt    1560
gacttttcgt ttggatggca acatatagtt gacaaaacaa actttatctc cagcaacgca    1620
actttacacc tgattgcagt tcatagcacg tgaacccctg tcatctttct agcaccttcc    1680
ctgacagaac atggacaccc agaacatgtg acaactaata gacaaactat gaaaattttt    1740
ggatcaaaat aaagccccog ttgaagtggg tatgtttgta ttgctcagaa agtgtgactt    1800
accatattaa atttatttga catggccagc tggcagcaat actacatatg taaatttatt    1860
tggaactgat atagtgatat gtggattgag tatggtagta acaaaatcca gaaaccgaca    1920
tatctttctc tctttttttt aatcatgtat ggtcattaat tgagttctct tgctgattag    1980
gttcaagaat gctatgaggt agctgctgag tatgaaacaa aacatgactt gcaaaagctt    2040
gatgaactcg ggaagatgat aacaagcttg gatcctgggg actctattgt gatcgccaag    2100
tctttctcgc acatgcttaa cttggccaac ttggctgagg aagtccagat agcatacagg    2160
aggagaatca agctcaagaa gggagacttt gctgatgaga actcggcaat cacagaatct    2220
gacattgagg aaacacttaa gaggcttgtt gttgacctga agaagtcacc tgctgaggta    2280
tttgatgccc tcaagagcca gactgttgat ctggttttga ctgcacatcc aacacagtct    2340
gtgaggaggt cactgctcca gaaacactcg aggttggttt gccaaacgtt gcattgtagt    2400
tgacatgcac aactccgttt tagcctttaa tcttgatccc aacattctta ccaggatacg    2460
aaactgtttg gttcaactct actcaaaaga tatcactcca gatgataagc aggaacttga    2520
tgaggctctg cagagagagg tatttatttc aatctaatgt ttattacctg gaattctttg    2580
aagcttgaga ccaaagatat caattcttaa ttccttacat ggactcctct ctcctttttc    2640
aagatccaag ctgcctttag aactgatgag atccgaagaa cgcagcccac tccccaagat    2700
gaaatgcgtg ctggtatgag ctacttccat gaaacaattt ggaagggtgt tccaaagttc    2760
ttgcgccgag ttgatactgc attgaagaac attgggatta acgagcgtgt tccttacaat    2820
gcacctctta ttcagttctc ttcttggatg ggaggagatc gtgatggtgc acgtgacatc    2880
tctacatatt tgtcttttct tctcatggca ttataaatat gagtatttgc agcacctgtt    2940
attatctttg ttgctagtgg ttgggaagat gactattcat tgtccatgtg tctgcagtct    3000
gcacctctgt cctttgtatt ctaaagcaat gtttctacaa atgcaggaaa tccaagagtg    3060
acaccagagg ttaccaggga tgtctgcttg cttgccagga tgatggcatc gaacttgtac    3120
tgctctcaga ttgaggatct catgtttgag gttgctatta ctttcagaat cagagtatag    3180
ttcataacat catttctatc agatgattga acttgataac ttgaaatgct tgcagttgtc    3240
tatgtggcga tgcagtgatg agctgcgcat gcgagctgat gagctgcatc gctccactaa    3300
gaaggatgcc aagcattaca taggtagtta ccagtgacat cattgggtct tcttaacaat    3360
ggcctgttgt tattaggcca tatttttggc atttaataa tttttccttc ttcatttctg    3420
ctgtagagtt ttggaagaag gttcctccaa atgagccata tcgggtgata ctgagtgatg    3480
tcagggataa actctacaac actcgtgagc ggtcacgaga gcttttatcc agtggacatt    3540
ctgatattcc tgaggaagct acattgacaa ctgttgagca ggttgagaat taatcagtaa    3600
tcttcagaaa ataatattga tttcttggtt ttccctcagt gggttctgat atgtagatgc    3660
caacttttgc agctgttgga gcccttggaa ctatgttaca gatcactctg tgcttgtggt    3720
gaccgtgtaa ttgctgatgg aagtcttctg gatttcttgc gtcaagtctc cacctttgga    3780
ctctcccttg tgaggcttga cattaggcaa gaatcagatc ggcatactga tgtccttgat    3840
gccatcacta cgtacctggg gataggatct taccgcgagt ggcctgaaga acgccgccaa    3900
gaatggctac tgtctgaact taatggcaag cgccctctgt ttggcccgga ccttcccaag    3960
actgaggaga ttgctgatgt tctagacaca ttccacgtta tcgctgagct tcctgctgac    4020
aattttggtg cttatatcat ttccatggca acagctcctt cggatgtact tgctgttgag    4080
ctcctccaac gtgagtgtca tgtaaagaca ccacttagag tagtcccact gtttgagaag    4140
ttggctgatc ttgaggctgc cccagctgca ttggccagac tgttctcaat cgattggtat    4200
agacagagga tcaatggcaa gcaggaggtc atgattgggt attcagattc aggcaaagat    4260
gcaggtcgac tctcagcagc ttggcagcta tacaaagctc aggaggagct catcaaggtt    4320
gctaaggact tcggtgtgaa gttgacgatg ttccatggac gtggtgggac tgttggaagg    4380
ggtggtggtc ccactcacct tgccatcttg tcccagccac cagacacgat ccatggatca    4440
ctccgggtca ctgttcaagg tgaagtcatt gagcagtctt ttggtgagga gcacttgtcg    4500
tttaggacac tgcaacgttt cacggctgct accctggagc atggaatgca cccaccaaat    4560
gcaccaaagc cagaatggcg aacccttctt gatgagatgg cagttgtggc aactgaggaa    4620
tatcggtcca ttgtcttta agagccacgc ttcgttgagt attttcgcct cgtgcagcat    4680
cgttgagtat ttccgcctcg tagtccttgt tgtccattgt catactacag taacataagc    4740
ctgcaaaacg taacttttga aataggtcgc ctgacttgag acttaatatg aatgtagcac    4800
aatgatttac tgcactatat ctttttgcaa acctaactaa aatggttaat gaaatatttg    4860
atttgaccct acatttagcg aatatttact ttgtgatatt tgtgttgaca ggcaacacct    4920
gaaacagagt atggtaggat gaacatagga agcaggccat ccaagagaaa gccgagcgga    4980
ggtatcgagt cactccgagc aatcccatgg atctttgctt ggacacaaac acggttccac    5040
ctcccggtat ggctaggatt tggaggcgca ttcaagcatg tcctccagaa ggacatcagg    5100
aacctccaca tgctccagga aatgtacaat gagtggccat ttttcagggt cactattgat    5160
ctggtggaga tggtgttcgc caagggtaac cctggcattg ctgcacttta tgacaaactc    5220
ctagtttcag aggaactgcg tccattgggt gagaagctga gggccaacta tgaagaaacc    5280
caaaagcttc tacttcaggt cgctgtcctg tccaaaagac acatcgatac attcttgcac    5340
```

tttgttgagc taccattcta gtagcgtttt atcattgaac tctgttttgg acttctatca 5400
ggttgccggg cacagggatc tcctggaagg tgacccttac ctgaagcagc ggctccgcct 5460
ccgtgacgcg tacatcacca ccctgaacgt ctgccaggcc tacaccctga agcggatccg 5520
tgacccggac taccatgtcg cgctgcgccc ccacctctcc aaggagatca tggaccccac 5580
caaggcggct tcagagctgg tgaagctgaa ccctggcagc gagtatgcac cggggctgga 5640
ggacaccctc attctgacca tgaagggcat agcagccggt ctccagaaca ccggttaaaa 5700
cacaaagagg ggatcttttt ttttaatcct gatcattgtc tctaggtcct ttgtatttac 5760
gcagatttta gctgcatctt cggccgtctc tctacaccgt gaggaaataa tgcgattttg 5820
ccaaagtggc ggtaaataaa agggagatga ggatcataca tgtttttctt attagtagac 5880
cgttgtgttg caactttact atgcgataat ggaacatgct gtagcctaat tgaagtgccg 5940
tatctcaagc atttttgcag gcaggctctg aattgttgga attgttccaa tgttcccttt 6000
gcaatctgtt gcctgcag 6018

<210> 32
<211> 960
<212> PRT
<213> Sorghum bicolor

<400> 32
Met Glu Arg Leu Ser Ser Ile Asp Ala Gln Leu Arg Met Leu Val Pro
1 5 10 15
Gly Lys Val Ser Glu Asp Asp Lys Leu Ile Glu Tyr Asp Ala Leu Leu
20 25 30
Leu Asp Arg Phe Leu Asp Ile Leu Gln Asp Leu His Gly Asp Asp Leu
35 40 45
Lys Glu Met Val Gln Glu Cys Tyr Glu Val Ala Ala Glu Tyr Glu Thr
50 55 60
Lys His Asp Leu Gln Lys Leu Asp Glu Leu Gly Lys Met Ile Thr Ser
65 70 75 80
Leu Asp Pro Gly Asp Ser Ile Val Ile Ala Lys Ser Phe Ser His Met
85 90 95
Leu Asn Leu Ala Asn Leu Ala Glu Glu Val Gln Ile Ala Tyr Arg Arg
100 105 110
Arg Ile Lys Leu Lys Lys Gly Asp Phe Ala Asp Glu Asn Ser Ala Ile
115 120 125
Thr Glu Ser Asp Ile Glu Glu Thr Leu Lys Arg Leu Val Val Asp Leu
130 135 140
Lys Lys Ser Pro Ala Glu Val Phe Asp Ala Leu Lys Ser Gln Thr Val
145 150 155 160
Asp Leu Val Leu Thr Ala His Pro Thr Gln Ser Val Arg Arg Ser Leu
165 170 175
Leu Gln Lys His Ser Arg Ile Arg Asn Cys Leu Val Gln Leu Tyr Ser
180 185 190
Lys Asp Ile Thr Pro Asp Asp Lys Gln Glu Leu Asp Glu Ala Leu Gln
195 200 205
Arg Glu Ile Gln Ala Ala Phe Arg Thr Asp Glu Ile Arg Arg Thr Gln
210 215 220
Pro Thr Pro Gln Asp Glu Met Arg Ala Gly Met Ser Tyr Phe His Glu
225 230 235 240
Thr Ile Trp Lys Gly Val Pro Lys Phe Leu Arg Arg Val Asp Thr Ala
245 250 255
Leu Lys Asn Ile Gly Ile Asn Glu Arg Val Pro Tyr Asn Ala Pro Leu
260 265 270
Ile Gln Phe Ser Ser Trp Met Gly Gly Asp Arg Asp Gly Asn Pro Arg
275 280 285
Val Thr Pro Glu Val Thr Arg Asp Val Cys Leu Leu Ala Arg Met Met
290 295 300
Ala Ser Asn Leu Tyr Cys Ser Gln Ile Glu Asp Leu Met Phe Glu Leu
305 310 315 320
Ser Met Trp Arg Cys Ser Asp Glu Leu Arg Met Arg Ala Asp Glu Leu
325 330 335
His Arg Ser Thr Lys Lys Asp Ala Lys His Tyr Ile Glu Phe Trp Lys
340 345 350
Lys Val Pro Pro Asn Glu Pro Tyr Arg Val Ile Leu Ser Asp Val Arg
355 360 365
Asp Lys Leu Tyr Asn Thr Arg Glu Arg Ser Arg Glu Leu Leu Ser Ser
370 375 380
Gly His Ser Asp Ile Pro Glu Glu Ala Thr Leu Thr Thr Val Glu Gln
385 390 395 400
Leu Leu Glu Pro Leu Glu Leu Cys Tyr Arg Ser Leu Cys Ala Cys Gly
405 410 415
Asp Arg Val Ile Ala Asp Gly Ser Leu Leu Asp Phe Leu Arg Gln Val
420 425 430
Ser Thr Phe Gly Leu Ser Leu Val Arg Leu Asp Ile Arg Gln Glu Ser

```
        435                 440                 445
Asp Arg His Thr Asp Val Leu Asp Ala Ile Thr Thr Tyr Leu Gly Ile
    450                 455                 460
Gly Ser Tyr Arg Glu Trp Pro Glu Glu Arg Arg Gln Glu Trp Leu Leu
465                 470                 475                 480
Ser Glu Leu Asn Gly Lys Arg Pro Leu Phe Gly Pro Asp Leu Pro Lys
                485                 490                 495
Thr Glu Glu Ile Ala Asp Val Leu Asp Thr Phe His Val Ile Ala Glu
            500                 505                 510
Leu Pro Ala Asp Asn Phe Gly Ala Tyr Ile Ile Ser Met Ala Thr Ala
        515                 520                 525
Pro Ser Asp Val Leu Ala Val Glu Leu Leu Gln Arg Glu Cys His Val
    530                 535                 540
Lys Thr Pro Leu Arg Val Val Pro Leu Phe Glu Lys Leu Ala Asp Leu
545                 550                 555                 560
Glu Ala Ala Pro Ala Ala Leu Ala Arg Leu Phe Ser Ile Asp Trp Tyr
                565                 570                 575
Arg Gln Arg Ile Asn Gly Lys Gln Glu Val Met Ile Gly Tyr Ser Asp
            580                 585                 590
Ser Gly Lys Asp Ala Gly Arg Leu Ser Ala Ala Trp Gln Leu Tyr Lys
        595                 600                 605
Ala Gln Glu Glu Leu Ile Lys Val Ala Lys Asp Phe Gly Val Lys Leu
    610                 615                 620
Thr Met Phe His Gly Arg Gly Gly Thr Val Gly Arg Gly Gly Gly Pro
625                 630                 635                 640
Thr His Leu Ala Ile Leu Ser Gln Pro Pro Asp Thr Ile His Gly Ser
                645                 650                 655
Leu Arg Val Thr Val Gln Gly Glu Val Ile Glu Gln Ser Phe Gly Glu
            660                 665                 670
Glu His Leu Ser Phe Arg Thr Leu Gln Arg Phe Thr Ala Ala Thr Leu
        675                 680                 685
Glu His Gly Met His Pro Pro Asn Ala Pro Lys Pro Glu Trp Arg Thr
    690                 695                 700
Leu Leu Asp Glu Met Ala Val Val Ala Thr Glu Glu Tyr Arg Ser Ile
705                 710                 715                 720
Val Phe Gln Glu Pro Arg Phe Val Glu Tyr Phe Arg Leu Ala Thr Pro
                725                 730                 735
Glu Thr Glu Tyr Gly Arg Met Asn Ile Gly Ser Arg Pro Ser Lys Arg
            740                 745                 750
Lys Pro Ser Gly Gly Ile Glu Ser Leu Arg Ala Ile Pro Trp Ile Phe
        755                 760                 765
Ala Trp Thr Gln Thr Arg Phe His Leu Pro Val Trp Leu Gly Phe Gly
    770                 775                 780
Gly Ala Phe Lys His Val Leu Gln Lys Asp Ile Arg Asn Leu His Met
785                 790                 795                 800
Leu Gln Glu Met Tyr Asn Glu Trp Pro Phe Phe Arg Val Thr Ile Asp
                805                 810                 815
Leu Val Glu Met Val Phe Ala Lys Gly Asn Pro Gly Ile Ala Ala Leu
            820                 825                 830
Tyr Asp Lys Leu Leu Val Ser Glu Glu Leu Arg Pro Leu Gly Glu Lys
        835                 840                 845
Leu Arg Ala Asn Tyr Glu Glu Thr Gln Lys Leu Leu Leu Gln Val Ala
    850                 855                 860
Gly His Arg Asp Leu Leu Glu Gly Asp Pro Tyr Leu Lys Gln Arg Leu
865                 870                 875                 880
Arg Leu Arg Asp Ala Tyr Ile Thr Thr Leu Asn Val Cys Gln Ala Tyr
                885                 890                 895
Thr Leu Lys Arg Ile Arg Asp Pro Asp Tyr His Val Ala Leu Arg Pro
            900                 905                 910
His Leu Ser Lys Glu Ile Met Asp Pro Thr Lys Ala Ala Ser Glu Leu
        915                 920                 925
Val Lys Leu Asn Pro Gly Ser Glu Tyr Ala Pro Gly Leu Glu Asp Thr
    930                 935                 940
Leu Ile Leu Thr Met Lys Gly Ile Ala Ala Gly Leu Gln Asn Thr Gly
945                 950                 955                 960
```

<210> 33
<211> 2901
<212> DNA
<213> Glycine max

<400> 33

```
atggctgctc ggaacatcga gaagatggcc tcaattgatg ctcaactgag gttgctggcg      60
ccacgcaagg tgtctgatga tgacaaactc gttgagtatg atgccttgtt gttggatcgt     120
```

```
ttccttgaca ttcttcagga tttgcatggt gaagatatca gacaaacggt ccaagattgt     180
tacgagctgt cagctgagta cgaaggagag cataagcctg aaaagttgga ggaacttggg     240
aatatgctga cgggtcttga tgctggggat tcaattgtta ttgccaagtc attttctcac     300
atgctcaatt tggccaactt ggcagaagaa gttcaaattg cgtatcgaag aaggatcaag     360
ttactgaaga agggtgattt tgctgatgag aactctgcca ttactgagtc agacattgaa     420
gagaccttca agaagcttgt ggctcaactg aagaagacac cccaggaaat ctttgatgct     480
ttgaagaacc aaactgtgga tttggttcta actgctcatc ctactcagtc tgttcgcaga     540
tctttgctgc aaaagcatgg aaggataagg aattgtttga cacaattata tgctaaggac     600
ataacaccag atgataagca ggaacttgat gaggctttac aaagagagat tcaagctgca     660
tttcgcacag atgaaattcg aaggactcct cctaccccac aagatgagat gagggcagga     720
atgagctact ttcatgagac aatttggaaa ggtgtaccac agtttctgcg tcgggtagat     780
acagctctga agaacattgg aattaatgaa cgtgtcccat ataatgctcc tgttattcag     840
ttctcttctt ggatgggagg agaccgtgat ggtaatccta gagtaacccc tgaagttaca     900
agggatgtgt gtttgctggc tagaatgatg gctgctaata tgtacttctc tcagatagag     960
gatctcatgt ttgagttgtc tatgtggcgt tgcactgacg agctacgtgt tcgtgctcat    1020
gaactccata ggtcctcaaa gagagatgca aaacattata ttgagttttg gaaacagatt    1080
cctccaaatg agccatatcg tgttattctt ggtgatgtca gagacaaact atataacatt    1140
cgggaacgtg ctcgccattt attagccaat gggacatctg atatccctga ggagacaacc    1200
ttcactaacg ttgagcagtt tctggagccc cttgaactat gctacaggtc actctgtgca    1260
tgtggtgaca gaccaatagc agatggaagc ctccttgatt tcctgcggca agtttccaca    1320
tttggactct cactagtgag acttgacatc cgtcaagagt cagataggca cactgatgtt    1380
atggatgcaa tcacaaaaca cttagacatt ggatcatacc gagagtggcc cgaggaaaag    1440
aggcaggagt ggctcttgtc tgaactcagt ggaaagcgcc ctctctttgg ccatgacctt    1500
cccaaaacag aagaaatcac cgatgttttg gaaacattcc gtgtcatttc agagcttccc    1560
tcagacaact tcggtgccta catcatatca atggcaacat ccccatctga tgtgcttgct    1620
gttgagcttt tacaacgtga atgccatgtg aagcagccac taagggtggt gccactgttt    1680
gaaaagcttg ctgatcttga ggccgctccg gctgcggtgg cacgtctttt ctctatagat    1740
tggtacagaa accgcattga tgggaagcaa gaagttatga tagggtactc agactcagga    1800
aaagatgcag gccgtctttc tgcggcttgg gcgctttaca aggctcaaga agaacttgtg    1860
aaggttgcta aggagtatgg tgttaaactt acaatgtttc atgggagagg agggactgtt    1920
ggaagaggag gaggtccaac tcatcttgct atattgtctc agccaccaga caccattcat    1980
ggctcacttc gggtaacggt tcagggtgaa gtcattgagc agtcttttgg agaggaacac    2040
ttgtgcttta gaacacttca gcgcttcact gcagctacac ttgagcatgg catgcatcct    2100
cctgtgtcac cgaaacccga atggcgtgcc ctcatggatg aaatggctgt cattgcaaca    2160
aaggagtatc gctctgttgt tttcaaagaa cctcgttttg ttgaatattt cagatgtgca    2220
actcctgagt tggagtatgg aagaatgaac attggcagtc gtccatcaaa gcgaaagcca    2280
agtggaggaa ttgaatcact acgtgctatt ccttggattt ttgcatggac acaaacgagg    2340
tttcatttgc cagtgtggct tggttttggg tcagcattta agcatgtagt tgagaaagat    2400
ccgaagaatc tccaaatgct tcaggacatg tacaatcaat ggcctttctt cagggtcacc    2460
ctggacttgg ttgagatggt gtttgctaag ggagacccgg ggattgcagc cctatttgac    2520
aaactcctag tatcagaaga gctgcgtcca tttggagaaa atttaagagc taaatacgaa    2580
gaaaccaaga gctttctcct ccaggttgct gggcacaagg atattcttga aggagacccc    2640
tacttgaagc aaagacttcg tcttcgtgac tcatacatca caaccctcaa tgtgttgcaa    2700
gcttacacat tgaagcgaat tcgtgatcct gactaccatg tgaagttgag gccacacttg    2760
tcaaaggact acatggaatc aagcaagcca gcagcagagc ttgttaaact taaccccaaa    2820
agcgagtatg ctcctggtct tgaggacacc cttattttga caatgaaggg tattgctgct    2880
ggaatgcaaa ataccggtta a                                              2901
```

```
<210>  34
<211>  966
<212>  PRT
<213>  Glycine max

<400>  34
Met Ala Ala Arg Asn Ile Glu Lys Met Ala Ser Ile Asp Ala Gln Leu
1               5                   10                  15
Arg Leu Leu Ala Pro Arg Lys Val Ser Asp Asp Asp Lys Leu Val Glu
            20                  25                  30
Tyr Asp Ala Leu Leu Leu Asp Arg Phe Leu Asp Ile Leu Gln Asp Leu
        35                  40                  45
His Gly Glu Asp Ile Arg Gln Thr Val Gln Asp Cys Tyr Glu Leu Ser
    50                  55                  60
Ala Glu Tyr Glu Gly Glu His Lys Pro Glu Lys Leu Glu Glu Leu Gly
65                  70                  75                  80
Asn Met Leu Thr Gly Leu Asp Ala Gly Asp Ser Ile Val Ile Ala Lys
                85                  90                  95
Ser Phe Ser His Met Leu Asn Leu Ala Asn Leu Ala Glu Glu Val Gln
            100                 105                 110
Ile Ala Tyr Arg Arg Arg Ile Lys Leu Leu Lys Lys Gly Asp Phe Ala
        115                 120                 125
Asp Glu Asn Ser Ala Ile Thr Glu Ser Asp Ile Glu Glu Thr Phe Lys
    130                 135                 140
Lys Leu Val Ala Gln Leu Lys Lys Thr Pro Gln Glu Ile Phe Asp Ala
145                 150                 155                 160
```

```
Leu Lys Asn Gln Thr Val Asp Leu Val Leu Thr Ala His Pro Thr Gln
                165                 170                 175
Ser Val Arg Arg Ser Leu Leu Gln Lys His Gly Arg Ile Arg Asn Cys
            180                 185                 190
Leu Thr Gln Leu Tyr Ala Lys Asp Ile Thr Pro Asp Asp Lys Gln Glu
        195                 200                 205
Leu Asp Glu Ala Leu Gln Arg Glu Ile Gln Ala Ala Phe Arg Thr Asp
    210                 215                 220
Glu Ile Arg Arg Thr Pro Pro Thr Pro Gln Asp Glu Met Arg Ala Gly
225                 230                 235                 240
Met Ser Tyr Phe His Glu Thr Ile Trp Lys Gly Val Pro Gln Phe Leu
                245                 250                 255
Arg Arg Val Asp Thr Ala Leu Lys Asn Ile Gly Ile Asn Glu Arg Val
            260                 265                 270
Pro Tyr Asn Ala Pro Val Ile Gln Phe Ser Ser Trp Met Gly Gly Asp
        275                 280                 285
Arg Asp Gly Asn Pro Arg Val Thr Pro Glu Val Thr Arg Asp Val Cys
    290                 295                 300
Leu Leu Ala Arg Met Met Ala Ala Asn Met Tyr Phe Ser Gln Ile Glu
305                 310                 315                 320
Asp Leu Met Phe Glu Leu Ser Met Trp Arg Cys Thr Asp Glu Leu Arg
                325                 330                 335
Val Arg Ala His Glu Leu His Arg Ser Ser Lys Arg Asp Ala Lys His
            340                 345                 350
Tyr Ile Glu Phe Trp Lys Gln Ile Pro Pro Asn Glu Pro Tyr Arg Val
        355                 360                 365
Ile Leu Gly Asp Val Arg Asp Lys Leu Tyr Asn Ile Arg Glu Arg Ala
    370                 375                 380
Arg His Leu Leu Ala Asn Gly Thr Ser Asp Ile Pro Glu Glu Thr Thr
385                 390                 395                 400
Phe Thr Asn Val Glu Gln Phe Leu Glu Pro Leu Glu Leu Cys Tyr Arg
                405                 410                 415
Ser Leu Cys Ala Cys Gly Asp Arg Pro Ile Ala Asp Gly Ser Leu Leu
            420                 425                 430
Asp Phe Leu Arg Gln Val Ser Thr Phe Gly Leu Ser Leu Val Arg Leu
        435                 440                 445
Asp Ile Arg Gln Glu Ser Asp Arg His Thr Asp Val Met Asp Ala Ile
    450                 455                 460
Thr Lys His Leu Asp Ile Gly Ser Tyr Arg Glu Trp Pro Glu Glu Lys
465                 470                 475                 480
Arg Gln Glu Trp Leu Leu Ser Glu Leu Ser Gly Lys Arg Pro Leu Phe
                485                 490                 495
Gly His Asp Leu Pro Lys Thr Glu Glu Ile Thr Asp Val Leu Glu Thr
            500                 505                 510
Phe Arg Val Ile Ser Glu Leu Pro Ser Asp Asn Phe Gly Ala Tyr Ile
        515                 520                 525
Ile Ser Met Ala Thr Ser Pro Ser Asp Val Leu Ala Val Glu Leu Leu
    530                 535                 540
Gln Arg Glu Cys His Val Lys Gln Pro Leu Arg Val Val Pro Leu Phe
545                 550                 555                 560
Glu Lys Leu Ala Asp Leu Glu Ala Ala Pro Ala Ala Val Ala Arg Leu
                565                 570                 575
Phe Ser Ile Asp Trp Tyr Arg Asn Arg Ile Asp Gly Lys Gln Glu Val
            580                 585                 590
Met Ile Gly Tyr Ser Asp Ser Gly Lys Asp Ala Gly Arg Leu Ser Ala
        595                 600                 605
Ala Trp Ala Leu Tyr Lys Ala Gln Glu Glu Leu Val Lys Val Ala Lys
    610                 615                 620
Glu Tyr Gly Val Lys Leu Thr Met Phe His Gly Arg Gly Gly Thr Val
625                 630                 635                 640
Gly Arg Gly Gly Gly Pro Thr His Leu Ala Ile Leu Ser Gln Pro Pro
                645                 650                 655
Asp Thr Ile His Gly Ser Leu Arg Val Thr Val Gln Gly Glu Val Ile
            660                 665                 670
Glu Gln Ser Phe Gly Glu Glu His Leu Cys Phe Arg Thr Leu Gln Arg
        675                 680                 685
Phe Thr Ala Ala Thr Leu Glu His Gly Met His Pro Pro Val Ser Pro
    690                 695                 700
Lys Pro Glu Trp Arg Ala Leu Met Asp Glu Met Ala Val Ile Ala Thr
705                 710                 715                 720
Lys Glu Tyr Arg Ser Val Val Phe Lys Glu Pro Arg Phe Val Glu Tyr
                725                 730                 735
Phe Arg Cys Ala Thr Pro Glu Leu Glu Tyr Gly Arg Met Asn Ile Gly
            740                 745                 750
```

```
Ser Arg Pro Ser Lys Arg Lys Pro Ser Gly Gly Ile Glu Ser Leu Arg
        755                 760                 765
Ala Ile Pro Trp Ile Phe Ala Trp Thr Gln Thr Arg Phe His Leu Pro
    770                 775                 780
Val Trp Leu Gly Phe Gly Ser Ala Phe Lys His Val Val Glu Lys Asp
785                 790                 795                 800
Pro Lys Asn Leu Gln Met Leu Gln Asp Met Tyr Asn Gln Trp Pro Phe
                805                 810                 815
Phe Arg Val Thr Leu Asp Leu Val Glu Met Val Phe Ala Lys Gly Asp
            820                 825                 830
Pro Gly Ile Ala Ala Leu Phe Asp Lys Leu Leu Val Ser Glu Glu Leu
        835                 840                 845
Arg Pro Phe Gly Glu Asn Leu Arg Ala Lys Tyr Glu Glu Thr Lys Ser
    850                 855                 860
Phe Leu Leu Gln Val Ala Gly His Lys Asp Ile Leu Glu Gly Asp Pro
865                 870                 875                 880
Tyr Leu Lys Gln Arg Leu Arg Leu Arg Asp Ser Tyr Ile Thr Thr Leu
                885                 890                 895
Asn Val Leu Gln Ala Tyr Thr Leu Lys Arg Ile Arg Asp Pro Asp Tyr
            900                 905                 910
His Val Lys Leu Arg Pro His Leu Ser Lys Asp Tyr Met Glu Ser Ser
        915                 920                 925
Lys Pro Ala Ala Glu Leu Val Lys Leu Asn Pro Lys Ser Glu Tyr Ala
    930                 935                 940
Pro Gly Leu Glu Asp Thr Leu Ile Leu Thr Met Lys Gly Ile Ala Ala
945                 950                 955                 960
Gly Met Gln Asn Thr Gly
                965
```

<210> 35
<211> 8638
<212> DNA
<213> Saccharum officinarum

<220>
<221> misc_feature
<222> (1324)..(1324)
<223> n is a, c, g, or t

<400> 35

```
gatccgtcac tgtcctcatg taatactcac acccttttgga tacaaaaata cgagcatacg      60
gctatgtttt caagagtaca acaacttctt tgccatgtct accatagctt gcccaatata     120
aaaatcgaac atatctctct agagccaacc tccttcattc attattcatg tccatggaca     180
aaccacaaaa ttcactcgat tttcactcta gatggtgtga taaatcttta tctatctatc     240
tgtctgtcta ggtagccagc tggttttttt ttatctgtct gtctatctat ctatctatta     300
ttaaagaata aaactttact tccaactcat tttgatttat tcccaaaaaa tacatgtcct     360
ttctttcgtc ccattctttt tttaaaatgt gttgaattta atgcaacata cggacacact     420
tgctgacgaa caaaattccg ataaaacaaa ggtcttgctc tcccgacgcg acgggccgcc     480
accatgaaac ctgtaggcga gagccgagag gagtaagact atccgcactc gtggtactct     540
aaattcatca tctaaaagca atatttcatt atagtcactc ctctatacca ctttctttcg     600
cactcatcca tcctctgtaa tcatcctcta taccaactac cagctgatgg ggtccacacg     660
tcatactctt attttctctc tcccttcccc tctcctccct cttctctctc ctctatcttc     720
ttttccggct gcttccggtt gggcgcagca cacgaggtgg ggccgccctg ggcacgtagc     780
ggactgctgc gacatgccag gaatggcgga cgatgagtgg gtagcggtcg gtttgcgtcc     840
ccggtgcagc cgatttgagt cgctatgtcc acgctgtccc tttagcgctc gtgtatacgt     900
cccggtgcgg acggtcttaa cgctccacta ggatgggctt tcaccactgg agatgaaaac     960
gaaacagaaa tattccgaat tgactgtttt cgttttctat attatgaacc ggttttttaa    1020
catgaaaaat tgtcagtttc aattggtttc acaatcattt tctaccattt ttttgtattc    1080
tttaattgct aaatgtagaa atattccgaa ccgaccgttt ttatttttct atactgtaag    1140
ctcgttttcg accgttttgt tcctggttca ccgccacagt ccgagttcga agggatatac    1200
gacgaatcca tccagcacaa gcgacagtcc cgtcctgtcc tataaatagc agaccacagc    1260
agagagagag agagggtgtc cctctccgct ccgtcacagg aaacccggta tagcatcaag    1320
gaangttttt ttgtcgtgtt gattgatctc cctcccettt cgccccccgtc ctgcctcctg    1380
gcctcacccc agaaaaaaaa aggtgagatg ctttcctctc ctctcccttc gatccagtag    1440
gtttgcagtc tgtttcctag atcttgttta ggcatttgtc catcttggag tggcaaagtc    1500
cctagctccc acaacggctg attgctgtag gttttgatca tacttttttt tttgcattcg    1560
agaaagcccc gtgctttgtt ggaatctgcg tgcttagggc tgggtggatc gtcttctcgc    1620
ttcaagaaca tgtcgaaatc cgtggattgg agggatttgt ggggttttct cttctctttt    1680
ggttggaatt gtgggttttg atctgtgtgg attctgttgc agggggcgag aagggaaggg    1740
gagtgcttgg gggggagaga agtagatggc gcgcaatgcg gtggacaagg cgacgtccat    1800
cgacgcgcag ctgcggctgc tggccccgca gaagctctcc gacgatgaca agcttgtcga    1860
gtacgacgcc ctgctcctcg accggttcct cgacatccta caggacctgc acggcgagga    1920
catcagggag acggtatgta aatgctccaa ttgtttcctg cccgtggtgc tatttgttcc    1980
tttcttttcg gtgctggcaa ttgatttagg tggtagatag ctcgatcatg cttgaaaatc    2040
```

tgtttatact gtcatgcatg catgggtcgc gatagcagga gaatcagaca cagcatctgg 2100
gccttgtccg tgggtgtttc tatatggtga tattcttctc atctgttttg gtattgcact 2160
ctggtgatgg aaactagtgc tgcatttaac aaatcatagt agcactgggg gtccacccat 2220
ttgttctaca gttgctgatc aatactgtgc tagttcccac tttgtgtttt ctagcttttg 2280
aaattggtac atcaccatga tgttcaaaac tggtggggtt atcatgatgt gtggagcact 2340
tgcactataa tcatggcctt ttgatactct cgagttatta tgccctttga actattgttg 2400
ttgctgatac gaggtagttt gcagatcaga tttattgtct tttctatgca taaatagtat 2460
gataaaatgg taaaaggcca cctttttttac aatgcataga cctatcacgc tattttacat 2520
tatcatttta ccactcaagt aagtcccatc tccttgccca ttttttttgt atatctgcaa 2580
gccaatcttt gcttgacatt ctgataggtg gcttgatttc tgtggtcatc cgttacacag 2640
caattccagt gtgttcacca actcactcta cattcgataa aatttatact tttttgcaat 2700
gccgtaaagt ttccctttct catgaatagg tgcaagaatg ctatgagtta gctgctgagt 2760
atgaaaataa gcttgacccc aaaatgctgg atgagattgg gaatgtgctg accagcttgg 2820
atcctggaga ctccattgtc ataaccaagt cattctcaca catgcttatc ctggcaaact 2880
tggctgagga ggtacaaatt gcataccgca ggagaataaa actgaagaag ggtgactttg 2940
ttgatgagaa ctcagcaaca actgaatcag acatcgagga gacattaaag aggcttatgc 3000
atcagcttaa gaagtcccct ctggaggtat ttgatgctct caagaatcaa actgttgacc 3060
tcgtcctgac tgcacatcca actcagtcag tccggagatc actgctccaa aagcatggca 3120
ggttagtctt gaagctttct gaattgttag atctcaggat gagatttact cgatggaatt 3180
gaatatttgc tatgcagaaa tgttggttct gtttgttctt tatgcacatc agagcaatgc 3240
aatttgctca cactgaattt cacatactgc tttcatagaa aacctgatgt ttttttgtcc 3300
acttttcttt tatgtgtaaa tattccttgc atggttttgc tgtagtctgc cttttaagaa 3360
acaacagcaa tagaaaaaaa taatatattt ctcgaggcat gttaaatcat acgttatatg 3420
tgccttttga tgccttagat tcctcacctt tcaatgtgca ggataagaaa ctgtttaact 3480
caactttatg caaaggacat cactccagat gagaaacagg aacttgatga agcacttcaa 3540
agagaggtaa tttgcttaga tattttggta tattagtgtt gtataatact atctgtgtag 3600
tagttaatca ggaaaacttt gtcaccttca ctaaatatat atttatatga ttctaatatc 3660
ctgttccatt tcttatctac agattcaagc tgcctttaga acagatgaaa tcaggcgggc 3720
gcctcctact ccgcaggatg aaatgcgtgc tggaatgagt tattttcatg agactatatg 3780
gaagggtgta cccaagttct tacgtagggt tgacactgct cttaagaaca ttggcataaa 3840
tgagcggttg ccatataatg cccctatcat tcagttttct tcttggatgg gtggtgatcg 3900
tgatggtgca tggcattctc tctccttttt tttggatagt atatttgatg atgtgccgaa 3960
tcatgcaact gtagttcaca agaaagaagt gcatatataa ctctaaactg atatccacaa 4020
cagggaatcc aagagtcacg ccagagatta caagggatgt atgcttgttg gctagaatga 4080
tggctgctaa tttgtataat gcccagatag aagacctaat gtttgaggta aaaataagga 4140
cacagttttt atttgatatt gtagtgatat tagtctcaca agaattcttt ttatcgcttg 4200
cagttatcta tgtggcgctg cagtgacgag ttacgtgtaa aggtcgatga gttacaccga 4260
tcctccaaga aagatacaac aaaacactat ataggtgcag attatattat gatatttttg 4320
ttaaacttat atattatatc ttcaacatcc ttgcctacca tctgctgcaa aatatttaac 4380
caaactagtc gccaatctgt tctttccaat ggatgaaata gctacacatt ttataaagta 4440
cctccatcca gaaaagagtg cagttctagt tctactctta gtcaaacaat ctaaagtttg 4500
accaagttca tagaaaagaa tatcaataat tatgacacca aataggtata ctatgaaaat 4560
atatttcatg acaaatctaa tgatacttat ttggtatcat aaatattgat attttttgg 4620
cacaaagtta gtcaaacttg agatggtttg acttagtcat aaatatattt ggtatccaga 4680
attgcattct tttccggatg aagggagtat cttacttatg attaatatat ggatcctcat 4740
cataaccata aatgtggttc cactatgctg ctgaagatta atctgagaca tgcagatcag 4800
gattctaatc taaatggttc taacccaagt cagcaattgg ggaaaaaata ggcatggcaa 4860
atccatattc caaaagggtt ggactgtaag aatcagaaac agttaaaagg gcagacccag 4920
tgccagaggc tcccacacga gtggggtcta gggaaaggaa taaccgaagt aaagtcttac 4980
ctccgcagaa attctacaga gagaaacagt taccttgcaa aaaaaaaggc attgaaggat 5040
ttgaaacatg caataaaact aatgtattta gctgttagct taataaatat gatcatgaag 5100
tattaagaaa tagctgtcat caggactcag gagttgcatt gatatatgtg ctttcttgcc 5160
aacataggct ggmatttgaa tcatgtttcg ttctcttgat ttgtttctga gacacgtgga 5220
tgtctatttc tgtcgttgta ctattttaat attggaaaga accatgtcac tatggagtaa 5280
ttttttttaaa gacaaattct tgctattttc atgcagactt tgatatttag gatgacattt 5340
ttggcagcaa cagacatgat attgatgcat gctatttttg tctattcttt tagattaatg 5400
cagccaatta gacaaaacta ggtgaagtta atacatgctg acatgctttt ccatgcagag 5460
ttctggaaac aagttccccc cagtgaacct taccgtgtga ttctgagtga tgtcagagat 5520
aaactctaca acacgcgtga acgagcacgc catttattag ctagtgggtt ttctgaaatt 5580
ccagaggaag caaccttcac cgatgttgaa caggtatgta aagtgaaaac agtagtgttc 5640
atttgatgtt cctggctagg ataaaaaatt aatctgtacc atacactttt agtcctatat 5700
taagattgga ttctttcatt ctatggtgta ctgtaaaagg ttgaaacatt cccaagataa 5760
aggatctttg tagctgtttc aggatatatt atgttttatg aattcttgtc tcgattgaat 5820
ctttttctc ttgcagttct tggagcctct tgagctctgt tacagatccc tctgtgcctg 5880
tggtgatcgc tctgttgcag atggaagcct tcttgacttc ttacggcaag tgtctacgtt 5940
tggactatcc cttgttagac ttgatatcag gcaagaatct gaccgacata ctgatgttat 6000
ggatgctata actgaatacc tcggcattgg atcataccgt aaatggacag aggagaaacg 6060
ccaagaatgg ctactctcgg aacttaatgg aaagaggccg ttatttggtc ctgaccttcc 6120
caagtcagat gagattgctg atgttctaga tactttccac gtattagctg aacttccttc 6180
agacagcttt ggtgcatatg taatatctat ggcgacagct ccctcagatg ttcttgctgt 6240
cgagctcttg cagcgtgaat gtcatgtgaa aaaaccactg agagttgtgc cattgttcga 6300
aaaactggca gatcttgagg cagcacccgc agctttagct cggctgttct ctgttgaatg 6360
gtacagaaac aggatcaatg gcaagcagga agtgatgatc gggtattcag attctggcaa 6420
ggatgctggt cgtttctctg ctgcttggca actgtacaag gctcaagagg agctaattaa 6480

```
tgttgctaag ctgtatgggg ttaagttaac tatgttccac ggacgaggtg ggactgtggg   6540
aagaggtggg ggccctaccc atcttgctat actgtcacaa cctcctgaaa ctattcatgg   6600
atcacttagg gtaactgttc aaggtgaagt cattgagcaa tcttttggag aggagcattt   6660
gtgctttaga accctgcaac gtttcacagc tgctactctt gaacatggta tgcatcctcc   6720
aatttcgcca aaaccggaat ggcgtgcttt aatggatgaa atggctattg ttgccacaaa   6780
ggaataccga tcaattgtat ttgaagaacc acgctttgtt gaatatttcc gccttgtaag   6840
tcttcggttt tctttgcttg tgttttcctt ttgaacacac ctttacttgt ctttatattg   6900
ttattatgca attaccacta tgagatttta attgtagctt ttatttaggc cttggtattt   6960
cactcttgtt tttgttagtt gggaattcat ctagctagtc ctgaagatta atatactgtt   7020
tcatttcgtt tgttttactg tcatgtcatt actccttgta gtaccctata aatttattat   7080
tttttggtaa tgggtccatt tttctgcata actaatcctg gcatttgtgt ctttctttca   7140
aataggcaac accagagatg gaatatggta ggatgaatat tggaagcaga ccatcgaaaa   7200
gaaagccaag tgcaggcatt gaatcactgc gtgcaatccc ttggatcttt gcttggacgc   7260
agactcggtt ccacctacca gtgtggcttg gttttggagc ggcattcaag catgtcttgg   7320
ataaggacat acggaatctt caaacccttc aagagatgta caatcagtgg ccatttttca   7380
gggttacaat agacttggtt gagatggtgt ttgccaaagg cgatcctggt atagcggctc   7440
tgtatgacaa gcttctagtt tcagaggatt tgtggtcatt cggcaagcgt cttagagcca   7500
actatgaaga aacaaagcaa cttcttctac aggtgatttg tctaagatct tcttgcattc   7560
gttatggact gctagagttg catgtagact atgtgatgcc tctaccaggc ttgaacattg   7620
gtgcgtctga atgagttgat aattaagtta atccgtaatc tatagccaca tcatgcccaa   7680
ctatttccag agaatgttta gatacaagct caaactggta gataaaccaa ggaggaaatg   7740
gttctggcaa taggatgtcc attcaccccg tcttgttctt gtctcaccca ctgcattgtg   7800
gcctgctgaa tttcaggttg ctgggcacaa agaccttctg gagggcgacc cttacctgaa   7860
gcagaggctg cgcatccgtg actcctacat cactgccctg aacgtgtgcc aagcttacat   7920
gctgaagcgc atcagggacc ctggcttcca ggtgaacccg gggcctcacc tgtcgaagga   7980
catcatggac atgggcaagc cggcctcgga gcttgtgaag ctcaacacca cgagcgagta   8040
cgccccggga ctggaggaca ccctcatcct gaccatgaag ggcatcgctg ccggaatgca   8100
gaacaccggc tgaaagaaca ggatccggag tgccttcttt attctgagct gatgtatcta   8160
tcgtagcgta ggggatcatg gcgtgaaccg gttaacaaga cggggtgca cacctggata    8220
tggggatta cctgttttag cagtagtagt atttactttt atgctatgct ctatcgaaac    8280
tcctatgtat gcatatccat gttgcttgag cctgcactag cttttggatc atagcttgtt   8340
aagtaatgcg cagggcatct tgttacggtg aaataactct gtgcttgtgc ttggtgctgg   8400
gctgctggca tatttgtgcc gaggagtcta ctgaattgca tggatcattg taccttttct   8460
gtcgtgcggt atcttgtcga atactactgt cctgggctaa cggacaggct gtagagacta   8520
gatgcgcacg tcgtgtatca cgcacgcaca acggcccct ggaaactgcg gaggcccgga    8580
gccctcggac gtgcacgcgc aaacaggggc tttgtcaaaa ttcaattttt cttctaat     8638
```

```
<210>  36
<211>  966
<212>  PRT
<213>  Saccharum officinarum

<400>  36
Met Ala Arg Asn Ala Val Asp Lys Ala Thr Ser Ile Asp Ala Gln Leu
1               5                   10                  15
Arg Leu Leu Ala Pro Gln Lys Leu Ser Asp Asp Asp Lys Leu Val Glu
            20                  25                  30
Tyr Asp Ala Leu Leu Leu Asp Arg Phe Leu Asp Ile Leu Gln Asp Leu
        35                  40                  45
His Gly Glu Asp Ile Arg Glu Thr Val Gln Glu Cys Tyr Glu Leu Ala
    50                  55                  60
Ala Glu Tyr Glu Asn Lys Leu Asp Pro Lys Met Leu Asp Glu Ile Gly
65                  70                  75                  80
Asn Val Leu Thr Ser Leu Asp Pro Gly Asp Ser Ile Val Ile Thr Lys
                85                  90                  95
Ser Phe Ser His Met Leu Ile Leu Ala Asn Leu Ala Glu Glu Val Gln
            100                 105                 110
Ile Ala Tyr Arg Arg Arg Ile Lys Leu Lys Lys Gly Asp Phe Val Asp
        115                 120                 125
Glu Asn Ser Ala Thr Thr Glu Ser Asp Ile Glu Glu Thr Leu Lys Arg
    130                 135                 140
Leu Met His Gln Leu Lys Lys Ser Pro Leu Glu Val Phe Asp Ala Leu
145                 150                 155                 160
Lys Asn Gln Thr Val Asp Leu Val Leu Thr Ala His Pro Thr Gln Ser
                165                 170                 175
Val Arg Arg Ser Leu Leu Gln Lys His Gly Arg Ile Arg Asn Cys Leu
            180                 185                 190
Thr Gln Leu Tyr Ala Lys Asp Ile Thr Pro Asp Glu Lys Gln Glu Leu
        195                 200                 205
Asp Glu Ala Leu Gln Arg Glu Ile Gln Ala Ala Phe Arg Thr Asp Glu
    210                 215                 220
Ile Arg Arg Ala Pro Pro Thr Pro Gln Asp Glu Met Arg Ala Gly Met
225                 230                 235                 240
Ser Tyr Phe His Glu Thr Ile Trp Lys Gly Val Pro Lys Phe Leu Arg
```

```
                245                 250                 255
Arg Val Asp Thr Ala Leu Lys Asn Ile Gly Ile Asn Glu Arg Leu Pro
            260                 265                 270
Tyr Asn Ala Pro Ile Ile Gln Phe Ser Ser Trp Met Gly Gly Asp Arg
        275                 280                 285
Asp Gly Asn Pro Arg Val Thr Pro Glu Ile Thr Arg Asp Val Cys Leu
    290                 295                 300
Leu Ala Arg Met Met Ala Ala Asn Leu Tyr Asn Ala Gln Ile Glu Asp
305                 310                 315                 320
Leu Met Phe Glu Leu Ser Met Trp Arg Cys Ser Asp Glu Leu Arg Val
                325                 330                 335
Lys Val Asp Glu Leu His Arg Ser Ser Lys Lys Asp Thr Thr Lys His
            340                 345                 350
Tyr Ile Glu Phe Trp Lys Gln Val Pro Pro Ser Glu Pro Tyr Arg Val
        355                 360                 365
Ile Leu Ser Asp Val Arg Asp Lys Leu Tyr Asn Thr Arg Glu Arg Ala
    370                 375                 380
Arg His Leu Leu Ala Ser Gly Phe Ser Glu Ile Pro Glu Glu Ala Thr
385                 390                 395                 400
Phe Thr Asp Val Glu Gln Phe Leu Glu Pro Leu Glu Leu Cys Tyr Arg
                405                 410                 415
Ser Leu Cys Ala Cys Gly Asp Arg Ser Val Ala Asp Gly Ser Leu Leu
            420                 425                 430
Asp Phe Leu Arg Gln Val Ser Thr Phe Gly Leu Ser Leu Val Arg Leu
        435                 440                 445
Asp Ile Arg Gln Glu Ser Asp Arg His Thr Asp Val Met Asp Ala Ile
    450                 455                 460
Thr Glu Tyr Leu Gly Ile Gly Ser Tyr Arg Lys Trp Thr Glu Glu Lys
465                 470                 475                 480
Arg Gln Glu Trp Leu Leu Ser Glu Leu Asn Gly Lys Arg Pro Leu Phe
                485                 490                 495
Gly Pro Asp Leu Pro Lys Ser Asp Glu Ile Ala Asp Val Leu Asp Thr
            500                 505                 510
Phe His Val Leu Ala Glu Leu Pro Ser Asp Ser Phe Gly Ala Tyr Val
        515                 520                 525
Ile Ser Met Ala Thr Ala Pro Ser Asp Val Leu Ala Val Glu Leu Leu
    530                 535                 540
Gln Arg Glu Cys His Val Lys Lys Pro Leu Arg Val Val Pro Leu Phe
545                 550                 555                 560
Glu Lys Leu Ala Asp Leu Glu Ala Ala Pro Ala Ala Leu Ala Arg Leu
                565                 570                 575
Phe Ser Val Glu Trp Tyr Arg Asn Arg Ile Asn Gly Lys Gln Glu Val
            580                 585                 590
Met Ile Gly Tyr Ser Asp Ser Gly Lys Asp Ala Gly Arg Phe Ser Ala
        595                 600                 605
Ala Trp Gln Leu Tyr Lys Ala Gln Glu Glu Leu Ile Asn Val Ala Lys
    610                 615                 620
Leu Tyr Gly Val Lys Leu Thr Met Phe His Gly Arg Gly Gly Thr Val
625                 630                 635                 640
Gly Arg Gly Gly Gly Pro Thr His Leu Ala Ile Leu Ser Gln Pro Pro
                645                 650                 655
Glu Thr Ile His Gly Ser Leu Arg Val Thr Val Gln Gly Glu Val Ile
            660                 665                 670
Glu Gln Ser Phe Gly Glu Glu His Leu Cys Phe Arg Thr Leu Gln Arg
        675                 680                 685
Phe Thr Ala Ala Thr Leu Glu His Gly Met His Pro Pro Ile Ser Pro
    690                 695                 700
Lys Pro Glu Trp Arg Ala Leu Met Asp Glu Met Ala Ile Val Ala Thr
705                 710                 715                 720
Lys Glu Tyr Arg Ser Ile Val Phe Glu Glu Pro Arg Phe Val Glu Tyr
                725                 730                 735
Phe Arg Leu Ala Thr Pro Glu Met Glu Tyr Gly Arg Met Asn Ile Gly
            740                 745                 750
Ser Arg Pro Ser Lys Arg Lys Pro Ser Ala Gly Ile Glu Ser Leu Arg
        755                 760                 765
Ala Ile Pro Trp Ile Phe Ala Trp Thr Gln Thr Arg Phe His Leu Pro
    770                 775                 780
Val Trp Leu Gly Phe Gly Ala Ala Phe Lys His Val Leu Asp Lys Asp
785                 790                 795                 800
Ile Arg Asn Leu Gln Thr Leu Gln Glu Met Tyr Asn Gln Trp Pro Phe
                805                 810                 815
Phe Arg Val Thr Ile Asp Leu Val Glu Met Val Phe Ala Lys Gly Asp
            820                 825                 830
Pro Gly Ile Ala Ala Leu Tyr Asp Lys Leu Leu Val Ser Glu Asp Leu
```

```
        835                 840                 845
Trp Ser Phe Gly Lys Arg Leu Arg Ala Asn Tyr Glu Glu Thr Lys Gln
    850                 855                 860
Leu Leu Leu Gln Val Ala Gly His Lys Asp Leu Leu Glu Gly Asp Pro
865                 870                 875                 880
Tyr Leu Lys Gln Arg Leu Arg Ile Arg Asp Ser Tyr Ile Thr Ala Leu
                885                 890                 895
Asn Val Cys Gln Ala Tyr Met Leu Lys Arg Ile Arg Asp Pro Gly Phe
            900                 905                 910
Gln Val Asn Pro Gly Pro His Leu Ser Lys Asp Ile Met Asp Met Gly
        915                 920                 925
Lys Pro Ala Ser Glu Leu Val Lys Leu Asn Thr Thr Ser Glu Tyr Ala
    930                 935                 940
Pro Gly Leu Glu Asp Thr Leu Ile Leu Thr Met Lys Gly Ile Ala Ala
945                 950                 955                 960
Gly Met Gln Asn Thr Gly
                965

<210>  37
<211>  3522
<212>  DNA
<213>  Pinus abies

<400>  37
gcttgtccta acaatagaaa catggctttg agcatctcaa cctggatctt gaaataactt      60
ccgagcccga gatcattgaa actttgaaga aatcgtgttg ttgaattttt attgttgcag     120
aggaaagatc agaattttga gattttggcg aagcggaact tggatttagg agaaatctgt     180
tgcagagatg gcgcgcaaca acttggagaa gatggcatcc attgatgccc agatgaggct     240
cttggttcct ggaaaagttt ctgaggatga caagttgatt gagtacgatg cccttctact     300
ggatcgcttc cttgacatcc tgcaggactt acatggagaa gatatcaggg cgatggttca     360
agaatgctat gagcgttctg gtgaatatga gggaaagaat gatcctcaca agctggaaga     420
gttgggaaat gtattaacaa gtctggatcc tggggattca attgttgtgg ccagctcatt     480
ttcccacatg cttaatttag ctaatttagc tgaagaagtt cagattgctt accggcgccg     540
aaataaaata aagaggggcg gctttgcaga tgaaagcaat gcaactactg aatcagacat     600
tgaagaaact tttaaaaggc ttgtaaacca gttaggaaaa tcaccggcag aggtgtttga     660
tgctcttaag aatcagactg ttgatttggt gttgacagca catccaacac agtcagtccg     720
cagatcattg ctgcagaagc atgctaggat tcggaattgc ttgtctcagt tatatgggaa     780
agatattacc cctgatgaga agcaggagct tgatgaagct ttgctaagag agattcaagc     840
tgccttccgc acagatgaaa ttcggcgtac tcctcctact ccacaagatg agatgcgagc     900
aggaatgagc tattttcatg aaacaatttg gaagggtgtc cctaagtttt tacgccgtat     960
tgatactgcc ctgaagtcaa ttgggatcaa tgaacgagtg ccgtataatg cacctcttat    1020
acagttttct tcatggatgg gaggtgatcg agatggaaat cctagggtaa ctccagaagt    1080
aacaagagat gtatgcttac ttgcaagaat gatggcagca aatttatatt attcccagat    1140
agaggatctt atgtttgagt tgtccatgtg gcgttgtagt gatgagctga gagcacgagc    1200
cctacaactc catagtgcat caaagaaaga tgcaaagcat tacatagaat tttggaaaca    1260
gattcctcca aatgaacctt ttagggtgat attgggagat gtacgagata aattgtacaa    1320
tactcgagaa cgtactcgcc aattactttc taatggaatt tctgacatac cagaggaagt    1380
aacctttaca aatattgacg agttttggga gccacttgaa ctttgttacc gatcactgtg    1440
ttctactggg gaccagccaa ttgcagatgg cagtcttctt gattttatgc gtcaagtttc    1500
aacatttggt ttgtcatttg ttaagctgga tattagacag gaatctgaca gacacagtga    1560
tgttgctgat gcaattacaa ggcatttagg cattggatcc tacaaagagt ggtcagagga    1620
acaacgacaa gcatggctct tgtcagaact gcaagggaaa cgtccccttgt ttgggccgga   1680
cctcccaaag acagatgagg ttcgagatgt tctagacaca tttcatgtaa tatctgagct    1740
tcctgctgac aattttggag cttatattat ttcaatggca actgcagcat cagatgtttt    1800
agtggttgag ttattacaac gggaatgcca tgtgaaaaaa ccattacgtg ttgtaccatt    1860
atttgagaag cttgcagatc tagaggctgc tcctgctgct ttggctagat tgttttcaat    1920
aaactggtac agaaacagaa ttgatggaaa gcaagaagtc atgattggtt actctgattc    1980
tggaaaggat gctggtaggt tgagtgcagg ttgggctttg tacaaagcac aggaagacct    2040
tataaaggtt gctaaagaat ttggtattaa gttgacaatg ttccatggtc gtggaggaac    2100
tgtaggcagg gggggaggcc caacccatct ggccatattg tcacaacctc cagacacaat    2160
tcatggctct ttcgggtca ctgttcaagg ggaagtcata gaacagtcat ttggtgagga    2220
acatttgtgt ttcagaactc tccaacgatt tactgctgcc actcttgagc atggaatgcg    2280
gcctccagtt gcaccaaagc ctgagtggcg tgaactgatg gatgaaatgg ctgttgttgc    2340
tacgaaggag tacaggtcaa ttgttttcca ggacccaaga ttcgttgaat atttccgttc    2400
tgcaacacca gaattggagt atggtcgaat gaacatcggg agtcgcccct caaaaaggaa    2460
gccaagtggg ggcattgagt cactccgtgc catcccatgg atatttgctt ggacacaaac    2520
tcgatttcat cttcctgttt ggcttggatt tggtgcagca ttcaagcatg ttatggagaa    2580
ggatataaga aatctccata tgctgcagca gatgtacaat gaatggccat tctttcgggt    2640
tacaattgat ctaattgaaa tggtttttgc aaaaggtgat ccagggatag ctgctttgta    2700
tgacaaacta ctggtatctg atgatttgtg ggccattggt gaaaaattga gagctaacta    2760
tggtgaaacc aaagatttgc tactgcaggt tgctggacat aaagatctac ttgaaggtga    2820
tccatacttg aaacagcgac tcagacttcg tgactcatac attacaactc tgaatgtttg    2880
tcaggcttat actttgaaaa gaattagaga ccccaactat catgttaatc ttaggcctca    2940
tttgtcgaag gaaagttcaa ccaaaccggc agctgaattg gtcaaactga atccaacgag    3000
```

```
tgagtatgca ccaggtctgg aggatacatt gattctaacc atgaagggca tagcagctgg   3060
tatgcagaac actggttaga aggattttgg aaacggatca ggcattttct catgttagga   3120
gtcaggacta taacggaagt ttcttgagac ctacttctga gggaccttct tcagattcca   3180
gtatttaagg cagcttctaa atatgtcaaa gcaccagcaa gaatgatctg ccagtgtaga   3240
tatggtcatt taataagctc gagaagagag caaatgttgg ttgtcagttg acaactttca   3300
aggattttat tataacgagt ctaggatcat atatgatctt aggatcccta atatatatga   3360
aaagccctaa aacatgtagt tttgtgaact ggacttgagg accaggactc ctttatatgc   3420
aaggcttggg aagtacaatg atttaacttg atgaatgcat taataatgat ctttagcatg   3480
ctgttgtttg tggcaaaaaa aaaaaaaaaa aaaaaaaaaa aa                       3522

<210>  38
<211>  963
<212>  PRT
<213>  Pinus abies

<400>  38
Met Ala Arg Asn Asn Leu Glu Lys Met Ala Ser Ile Asp Ala Gln Met
1               5                   10                  15
Arg Leu Leu Val Pro Gly Lys Val Ser Glu Asp Asp Lys Leu Ile Glu
            20                  25                  30
Tyr Asp Ala Leu Leu Leu Asp Arg Phe Leu Asp Ile Leu Gln Asp Leu
        35                  40                  45
His Gly Glu Asp Ile Arg Ala Met Val Gln Glu Cys Tyr Glu Arg Ser
    50                  55                  60
Gly Glu Tyr Glu Gly Lys Asn Asp Pro His Lys Leu Glu Glu Leu Gly
65                  70                  75                  80
Asn Val Leu Thr Ser Leu Asp Pro Gly Asp Ser Ile Val Val Ala Ser
                85                  90                  95
Ser Phe Ser His Met Leu Asn Leu Ala Asn Leu Ala Glu Glu Val Gln
            100                 105                 110
Ile Ala Tyr Arg Arg Arg Asn Lys Ile Lys Arg Gly Gly Phe Ala Asp
        115                 120                 125
Glu Ser Asn Ala Thr Thr Glu Ser Asp Ile Glu Glu Thr Phe Lys Arg
    130                 135                 140
Leu Val Asn Gln Leu Gly Lys Ser Pro Ala Glu Val Phe Asp Ala Leu
145                 150                 155                 160
Lys Asn Gln Thr Val Asp Leu Val Leu Thr Ala His Pro Thr Gln Ser
                165                 170                 175
Val Arg Arg Ser Leu Leu Gln Lys His Ala Arg Ile Arg Asn Cys Leu
            180                 185                 190
Ser Gln Leu Tyr Gly Lys Asp Ile Thr Pro Asp Glu Lys Gln Glu Leu
        195                 200                 205
Asp Glu Ala Leu Leu Arg Glu Ile Gln Ala Ala Phe Arg Thr Asp Glu
    210                 215                 220
Ile Arg Arg Thr Pro Pro Thr Pro Gln Asp Glu Met Arg Ala Gly Met
225                 230                 235                 240
Ser Tyr Phe His Glu Thr Ile Trp Lys Gly Val Pro Lys Phe Leu Arg
                245                 250                 255
Arg Ile Asp Thr Ala Leu Lys Ser Ile Gly Ile Asn Glu Arg Val Pro
            260                 265                 270
Tyr Asn Ala Pro Leu Ile Gln Phe Ser Ser Trp Met Gly Gly Asp Arg
        275                 280                 285
Asp Gly Asn Pro Arg Val Thr Pro Glu Val Thr Arg Asp Val Cys Leu
    290                 295                 300
Leu Ala Arg Met Met Ala Ala Asn Leu Tyr Tyr Ser Gln Ile Glu Asp
305                 310                 315                 320
Leu Met Phe Glu Leu Ser Met Trp Arg Cys Ser Asp Glu Leu Arg Ala
                325                 330                 335
Arg Ala Leu Gln Leu His Ser Ala Ser Lys Lys Asp Ala Lys His Tyr
            340                 345                 350
Ile Glu Phe Trp Lys Gln Ile Pro Pro Asn Glu Pro Phe Arg Val Ile
        355                 360                 365
Leu Gly Asp Val Arg Asp Lys Leu Tyr Asn Thr Arg Glu Arg Thr Arg
    370                 375                 380
Gln Leu Leu Ser Asn Gly Ile Ser Asp Ile Pro Glu Glu Val Thr Phe
385                 390                 395                 400
Thr Asn Ile Asp Glu Phe Leu Glu Pro Leu Glu Leu Cys Tyr Arg Ser
                405                 410                 415
Leu Cys Ser Thr Gly Asp Gln Pro Ile Ala Asp Gly Ser Leu Leu Asp
            420                 425                 430
Phe Met Arg Gln Val Ser Thr Phe Gly Leu Ser Phe Val Lys Leu Asp
        435                 440                 445
Ile Arg Gln Glu Ser Asp Arg His Ser Asp Val Ala Asp Ala Ile Thr
    450                 455                 460
```

```
Arg His Leu Gly Ile Gly Ser Tyr Lys Glu Trp Ser Glu Glu Gln Arg
465                 470                 475                 480
Gln Ala Trp Leu Leu Ser Glu Leu Gln Gly Lys Arg Pro Leu Phe Gly
                485                 490                 495
Pro Asp Leu Pro Lys Thr Asp Glu Val Arg Asp Val Leu Asp Thr Phe
            500                 505                 510
His Val Ile Ser Glu Leu Pro Ala Asp Asn Phe Gly Ala Tyr Ile Ile
        515                 520                 525
Ser Met Ala Thr Ala Ala Ser Asp Val Leu Val Val Glu Leu Leu Gln
    530                 535                 540
Arg Glu Cys His Val Lys Lys Pro Leu Arg Val Val Pro Leu Phe Glu
545                 550                 555                 560
Lys Leu Ala Asp Leu Glu Ala Ala Pro Ala Ala Leu Ala Arg Leu Phe
                565                 570                 575
Ser Ile Asn Trp Tyr Arg Asn Arg Ile Asp Gly Lys Gln Glu Val Met
            580                 585                 590
Ile Gly Tyr Ser Asp Ser Gly Lys Asp Ala Gly Arg Leu Ser Ala Gly
        595                 600                 605
Trp Ala Leu Tyr Lys Ala Gln Glu Asp Leu Ile Lys Val Ala Lys Glu
    610                 615                 620
Phe Gly Ile Lys Leu Thr Met Phe His Gly Arg Gly Gly Thr Val Gly
625                 630                 635                 640
Arg Gly Gly Gly Pro Thr His Leu Ala Ile Leu Ser Gln Pro Pro Asp
                645                 650                 655
Thr Ile His Gly Ser Phe Arg Val Thr Val Gln Gly Glu Val Ile Glu
            660                 665                 670
Gln Ser Phe Gly Glu Glu His Leu Cys Phe Arg Thr Leu Gln Arg Phe
        675                 680                 685
Thr Ala Ala Thr Leu Glu His Gly Met Arg Pro Pro Val Ala Pro Lys
    690                 695                 700
Pro Glu Trp Arg Glu Leu Met Asp Glu Met Ala Val Val Ala Thr Lys
705                 710                 715                 720
Glu Tyr Arg Ser Ile Val Phe Gln Asp Pro Arg Phe Val Glu Tyr Phe
                725                 730                 735
Arg Ser Ala Thr Pro Glu Leu Glu Tyr Gly Arg Met Asn Ile Gly Ser
            740                 745                 750
Arg Pro Ser Lys Arg Lys Pro Ser Gly Gly Ile Glu Ser Leu Arg Ala
        755                 760                 765
Ile Pro Trp Ile Phe Ala Trp Thr Gln Thr Arg Phe His Leu Pro Val
    770                 775                 780
Trp Leu Gly Phe Gly Ala Ala Phe Lys His Val Met Glu Lys Asp Ile
785                 790                 795                 800
Arg Asn Leu His Met Leu Gln Gln Met Tyr Asn Glu Trp Pro Phe Phe
                805                 810                 815
Arg Val Thr Ile Asp Leu Ile Glu Met Val Phe Ala Lys Gly Asp Pro
            820                 825                 830
Gly Ile Ala Ala Leu Tyr Asp Lys Leu Leu Val Ser Asp Asp Leu Trp
        835                 840                 845
Ala Ile Gly Glu Lys Leu Arg Ala Asn Tyr Gly Glu Thr Lys Asp Leu
    850                 855                 860
Leu Leu Gln Val Ala Gly His Lys Asp Leu Leu Glu Gly Asp Pro Tyr
865                 870                 875                 880
Leu Lys Gln Arg Leu Arg Leu Arg Asp Ser Tyr Ile Thr Thr Leu Asn
                885                 890                 895
Val Cys Gln Ala Tyr Thr Leu Lys Arg Ile Arg Asp Pro Asn Tyr His
            900                 905                 910
Val Asn Leu Arg Pro His Leu Ser Lys Glu Ser Ser Thr Lys Pro Ala
        915                 920                 925
Ala Glu Leu Val Lys Leu Asn Pro Thr Ser Glu Tyr Ala Pro Gly Leu
    930                 935                 940
Glu Asp Thr Leu Ile Leu Thr Met Lys Gly Ile Ala Ala Gly Met Gln
945                 950                 955                 960
Asn Thr Gly
```

```
<210>  39
<211>  3158
<212>  DNA
<213>  Lotus japonicus

<220>
<221>  misc_feature
<222>  (3088)..(3088)
<223>  n is a, c, g, or t
```

<400> 39
gggtttgtga agttacaatg gcgaacagga acttggagaa gatggcatcc attgatgctc 60
agcttcggca attggttcct gctaaagtca gcgaggatga taagctggtt gagtatgatg 120
ctctgctact ggatcggttc cttgatattc tccaggattt acatggggag gatctgagag 180
aaactgttca agaagtatat gagctttctg ctgagtatga aagaaagcat gaacctcaga 240
gactggaagt acttggaaat ctgataacta gtttggacgc tggagattcc attgttgttg 300
ccaagtcctt tgcccacatg cttaatttgg caaacttagc tgaagaggtc cagattgctc 360
accgccgtag gatcaagttg aaaaaggggg atttcgctga tgagggcaat gcaaccactg 420
aatcagatat tgaagaaact ttcaagaaac ttgtaggtga aatgaagaag tctcctcagg 480
aagtttttga tgaactgaaa aatcagactg ttgatctggt tcttactgct catcctactc 540
aatcagttcg tagatctttg ctgcagaagc atggaaggat acgaaattgc ttaactcaat 600
tgtatgccaa agacatcact cctgatgata agcaggagct tgatgaggct ctacagagag 660
agatccaagc tgctttccgt actgatgaaa tcaggagaac cgctccaaca ccacaagatg 720
aaatgagagc agggatgagc tactttcatg aaacaatttg gaagggtgtg cccaaatttc 780
tgcgtcgtgt tgatacagct ttgaagaata tagggattaa tgagcgtgtc ccgtataatg 840
ctccacttat tcaattttct tcttggatgg gtggtgatcg tgatggcaat ccaagagtaa 900
ctcctgaagt gacaagggat gtttgcttat tggctagaat gatggctgct aatttgtatt 960
attcacagat tgaggatctt atgtttgaac tgtccatgtg gcgctgcaat gatgagctgc 1020
gtgtccgggc tgaggaactt accagttctt ccaataaaga tgcagttgca aaacactaca 1080
tagagttttg gaaaaacatt cctccaagtg aaccgtatcg tgtggtactg ggtgaagtaa 1140
ggaacagact ctaccagact cgtgagcgtt ctcgccattt gcttgctaat gagtactctg 1200
acattctaga ggaacacact ttcactaatg tggaggagtt cctggaaccc ctcgagctct 1260
gttatagatc actttgtgcc tgtggtgatc gggcaattgc tgatggaagc cttctagatt 1320
ttttgaggca agtctctact tttggactgt ccctagttag gcttgatatt aggcaagagt 1380
ccgaccgtct tactgatgtg ctggatgcca tcaccaaaca tttgggaatc ggctcctacc 1440
tggaatggtc tgaagaaaag cggcagcaat ggcttttgtc cgagttgagt ggcaaacgcc 1500
ctctctttgg acctgatctt ccccaaactg aagagatcaa agatgttctc gacacatttc 1560
atgtcatagc agaacttcca tctgataact ttggagcata tatcatatca atggcaactg 1620
caccatctga tgtgcttgca gttgaactcc tgcaacgtga atgccatgtc aagaatccgt 1680
taagagtcgt cccattgttt gagaagcttg ctgatctgga aacagctccc gctgctttgg 1740
ctcggttgtt ctccgttgag tggtaccgaa accggatcaa cgggaaacaa gaagtcatga 1800
ttggctattc tgattcaggt aaagatgctg gaaggttctc tgcagcgtgg cagctatata 1860
aggctcaaga agagcttatt aaggtagcta aggaatatgg tgttaagcta acaatgttcc 1920
atggtcgtgg agggactgtc ggcagaggag gcggtcctac tcaccttgct atcctgtctc 1980
agcctccaga cacaatccat ggatcactcc gtgttactgt ccaaggagaa gttatcgagc 2040
agtcatttgg agaggagcac ttgtgtttca gaacccttca acgttacact gcagctactc 2100
tagaacatgg aatgcatccc ccaatttccc ccaaaccaga atggcgtgct ttgatggacg 2160
agatggctgt tattgccact gaggaatacc gttctattgt attcaaagaa ccccgttttg 2220
ttgagtattt ccgcctggct acaccggagt tggagtatgg ccgaatgaat attggaagtc 2280
ggccggcaaa gagaaagcct agtggaggca ttgagacgct ccgcgctata ccttggatct 2340
ttgcctggac acagacaagg tttcaccttc cagtgtggct agggtttggg gctgcattca 2400
aacatgtgat tgcaaaggat atcagaaatc ttaacatgct gcaagagatg tacaatcaat 2460
ggcctttctt tagggtcact attgatttag tggaaatggt gttcgccaag ggagaccccg 2520
gtatagctgc tttgtatgac aggctccttg tttcagaaga tttgtggtca tttggggaac 2580
agttgaggac catgtttgaa gaaactaagc aactgctact tcaggtggct gcacacaagg 2640
atcttcttga aggtgatcct tacttgaaac aaagactccg gttgcgtgat tcttacatca 2700
ctaccctcaa tgtgtgccaa gcttacacat tgaagcgaat ccgcgatccg aactataatg 2760
tgaagctacg cccccacatc tctaaagagg ctatagacgt gagtaaacct gcggatgaac 2820
ttgttactct gaacccaaca agtgaatatg cacctggttt ggaagacacc ctcattctca 2880
ccatgaaggg tattgctgcc ggcatgcaaa acactggtta aatctgagag attctacttc 2940
tgttttctgt tctcctgttt tatcaaaata atgaatttag atacgactca cccaaagagg 3000
gtgtttgttt tccttttccc agttgaaata tcatttgata taaattatgt ttccatgctt 3060
tgctatataa agcatcgttt ggctgctntg aaacaatgat taatgccagt atgcaactga 3120
ttatgtttta actgaaaaaa aaaaaaaaaa aaaaaaa 3158

<210> 40
<211> 967
<212> PRT
<213> Lotus japonicus

<400> 40
Met Ala Asn Arg Asn Leu Glu Lys Met Ala Ser Ile Asp Ala Gln Leu
1 5 10 15
Arg Gln Leu Val Pro Ala Lys Val Ser Glu Asp Asp Lys Leu Val Glu
20 25 30
Tyr Asp Ala Leu Leu Leu Asp Arg Phe Leu Asp Ile Leu Gln Asp Leu
35 40 45
His Gly Glu Asp Leu Arg Glu Thr Val Gln Glu Val Tyr Glu Leu Ser
50 55 60
Ala Glu Tyr Glu Arg Lys His Glu Pro Gln Arg Leu Glu Val Leu Gly
65 70 75 80
Asn Leu Ile Thr Ser Leu Asp Ala Gly Asp Ser Ile Val Val Ala Lys
85 90 95

```
Ser Phe Ala His Met Leu Asn Leu Ala Asn Leu Ala Glu Glu Val Gln
            100                 105                 110
Ile Ala His Arg Arg Arg Ile Lys Leu Lys Lys Gly Asp Phe Ala Asp
        115                 120                 125
Glu Gly Asn Ala Thr Thr Glu Ser Asp Ile Glu Glu Thr Phe Lys Lys
    130                 135                 140
Leu Val Gly Glu Met Lys Lys Ser Pro Gln Glu Val Phe Asp Glu Leu
145                 150                 155                 160
Lys Asn Gln Thr Val Asp Leu Val Leu Thr Ala His Pro Thr Gln Ser
                165                 170                 175
Val Arg Arg Ser Leu Leu Gln Lys His Gly Arg Ile Arg Asn Cys Leu
            180                 185                 190
Thr Gln Leu Tyr Ala Lys Asp Ile Thr Pro Asp Asp Lys Gln Glu Leu
        195                 200                 205
Asp Glu Ala Leu Gln Arg Glu Ile Gln Ala Ala Phe Arg Thr Asp Glu
    210                 215                 220
Ile Arg Arg Thr Ala Pro Thr Pro Gln Asp Glu Met Arg Ala Gly Met
225                 230                 235                 240
Ser Tyr Phe His Glu Thr Ile Trp Lys Gly Val Pro Lys Phe Leu Arg
                245                 250                 255
Arg Val Asp Thr Ala Leu Lys Asn Ile Gly Ile Asn Glu Arg Val Pro
            260                 265                 270
Tyr Asn Ala Pro Leu Ile Gln Phe Ser Ser Trp Met Gly Gly Asp Arg
        275                 280                 285
Asp Gly Asn Pro Arg Val Thr Pro Glu Val Thr Arg Asp Val Cys Leu
    290                 295                 300
Leu Ala Arg Met Met Ala Ala Asn Leu Tyr Tyr Ser Gln Ile Glu Asp
305                 310                 315                 320
Leu Met Phe Glu Leu Ser Met Trp Arg Cys Asn Asp Glu Leu Arg Val
                325                 330                 335
Arg Ala Glu Glu Leu Thr Ser Ser Ser Asn Lys Asp Ala Val Ala Lys
            340                 345                 350
His Tyr Ile Glu Phe Trp Lys Asn Ile Pro Pro Ser Glu Pro Tyr Arg
        355                 360                 365
Val Val Leu Gly Glu Val Arg Asn Arg Leu Tyr Gln Thr Arg Glu Arg
    370                 375                 380
Ser Arg His Leu Leu Ala Asn Glu Tyr Ser Asp Ile Leu Glu Glu His
385                 390                 395                 400
Thr Phe Thr Asn Val Glu Glu Phe Leu Glu Pro Leu Glu Leu Cys Tyr
                405                 410                 415
Arg Ser Leu Cys Ala Cys Gly Asp Arg Ala Ile Ala Asp Gly Ser Leu
            420                 425                 430
Leu Asp Phe Leu Arg Gln Val Ser Thr Phe Gly Leu Ser Leu Val Arg
        435                 440                 445
Leu Asp Ile Arg Gln Glu Ser Asp Arg Leu Thr Asp Val Leu Asp Ala
    450                 455                 460
Ile Thr Lys His Leu Gly Ile Gly Ser Tyr Leu Glu Trp Ser Glu Glu
465                 470                 475                 480
Lys Arg Gln Gln Trp Leu Leu Ser Glu Leu Ser Gly Lys Arg Pro Leu
                485                 490                 495
Phe Gly Pro Asp Leu Pro Gln Thr Glu Glu Ile Lys Asp Val Leu Asp
            500                 505                 510
Thr Phe His Val Ile Ala Glu Leu Pro Ser Asp Asn Phe Gly Ala Tyr
        515                 520                 525
Ile Ile Ser Met Ala Thr Ala Pro Ser Asp Val Leu Ala Val Glu Leu
    530                 535                 540
Leu Gln Arg Glu Cys His Val Lys Asn Pro Leu Arg Val Val Pro Leu
545                 550                 555                 560
Phe Glu Lys Leu Ala Asp Leu Glu Thr Ala Pro Ala Ala Leu Ala Arg
                565                 570                 575
Leu Phe Ser Val Glu Trp Tyr Arg Asn Arg Ile Asn Gly Lys Gln Glu
            580                 585                 590
Val Met Ile Gly Tyr Ser Asp Ser Gly Lys Asp Ala Gly Arg Phe Ser
        595                 600                 605
Ala Ala Trp Gln Leu Tyr Lys Ala Gln Glu Glu Leu Ile Lys Val Ala
    610                 615                 620
Lys Glu Tyr Gly Val Lys Leu Thr Met Phe His Gly Arg Gly Gly Thr
625                 630                 635                 640
Val Gly Arg Gly Gly Gly Pro Thr His Leu Ala Ile Leu Ser Gln Pro
                645                 650                 655
Pro Asp Thr Ile His Gly Ser Leu Arg Val Thr Val Gln Gly Glu Val
            660                 665                 670
Ile Glu Gln Ser Phe Gly Glu Glu His Leu Cys Phe Arg Thr Leu Gln
        675                 680                 685
```

```
Arg Tyr Thr Ala Ala Thr Leu Glu His Gly Met His Pro Pro Ile Ser
    690                 695                 700
Pro Lys Pro Glu Trp Arg Ala Leu Met Asp Glu Met Ala Val Ile Ala
705                 710                 715                 720
Thr Glu Glu Tyr Arg Ser Ile Val Phe Lys Glu Pro Arg Phe Val Glu
                725                 730                 735
Tyr Phe Arg Leu Ala Thr Pro Glu Leu Glu Tyr Gly Arg Met Asn Ile
            740                 745                 750
Gly Ser Arg Pro Ala Lys Arg Lys Pro Ser Gly Gly Ile Glu Thr Leu
        755                 760                 765
Arg Ala Ile Pro Trp Ile Phe Ala Trp Thr Gln Thr Arg Phe His Leu
    770                 775                 780
Pro Val Trp Leu Gly Phe Gly Ala Ala Phe Lys His Val Ile Ala Lys
785                 790                 795                 800
Asp Ile Arg Asn Leu Asn Met Leu Gln Glu Met Tyr Asn Gln Trp Pro
                805                 810                 815
Phe Phe Arg Val Thr Ile Asp Leu Val Glu Met Val Phe Ala Lys Gly
            820                 825                 830
Asp Pro Gly Ile Ala Ala Leu Tyr Asp Arg Leu Leu Val Ser Glu Asp
        835                 840                 845
Leu Trp Ser Phe Gly Glu Gln Leu Arg Thr Met Phe Glu Glu Thr Lys
    850                 855                 860
Gln Leu Leu Leu Gln Val Ala Ala His Lys Asp Leu Leu Glu Gly Asp
865                 870                 875                 880
Pro Tyr Leu Lys Gln Arg Leu Arg Leu Arg Asp Ser Tyr Ile Thr Thr
                885                 890                 895
Leu Asn Val Cys Gln Ala Tyr Thr Leu Lys Arg Ile Arg Asp Pro Asn
            900                 905                 910
Tyr Asn Val Lys Leu Arg Pro His Ile Ser Lys Glu Ala Ile Asp Val
        915                 920                 925
Ser Lys Pro Ala Asp Glu Leu Val Thr Leu Asn Pro Thr Ser Glu Tyr
    930                 935                 940
Ala Pro Gly Leu Glu Asp Thr Leu Ile Leu Thr Met Lys Gly Ile Ala
945                 950                 955                 960
Ala Gly Met Gln Asn Thr Gly
                965

<210>  41
<211>  3189
<212>  DNA
<213>  Nicotiana tabacum

<400>  41
aaaaattgtc cacttgtagg aaaggagatt attggggttg ttgatagtgt gaaaaatggc      60
gacacggagt ttggagaaat tggcatcaat tgatgcacag ttaagggcat tggttcctgg     120
caaagtttct gaggatgata agttggttga gtatgatgct ttgcttttgg atcggtttct     180
tgatattctt caggatttac atggggaaga tcttaaggaa acggtccaag aatgttatga     240
actttctgcg gaatacgaag gcaagcacga tccaaagaag ctggaggagc ttggaaatgt     300
gttgacaagt ttggatccag gggattccat tgtcattgct aaagctttct ctcacatgct     360
taatttggcc aacttggccg aggaggtgca gatcgcctac cgtcgacgac aaaagttgaa     420
aagggggggac tttgctgatg agaacaatgc aacaactgaa tcagatattg aagaaacttt     480
caagaaactt gtaggggact tgaaaaagtc tcctcaagaa gttttcgatg ctctgaaaaa     540
tcagactgtg gatttagtct taactgctca tcctactcaa tctgtccgaa gatctttgct     600
tcaaaagcac ggaaggatcc gagattgctt ggctcagttg tatgctaaag acattacacc     660
cgatgataaa caagagctcg atgaggcttt acagagggag attcaagctg ctttccgcac     720
tgatgaaatt cggaggactg ctccaactcc acaagatgaa atgagagctg gaatgagcta     780
ctttcatgaa actatttgga agggtgttcc aaagttcctt cgtcgtgttg atacagctct     840
taaaaacata gggattaacg aacgacttcc ttacaatgct cctcttattc aattctcctc     900
ttggatgggt ggtgaccggg atggcaatcc aagagtgact cttgaggtca caagagatgt     960
ctgcttacta gcaagaatga tggcagcgaa tttgtactat tcgcaaatag aggaactcat    1020
gtttgagtta tctatgtggc gttgcaatga tgaccttcgt attcgagcag ctgaacttta    1080
caggtcctca aggagggaca ctaagcacta catagaattt tggaaaacaa ttccgccaag    1140
tgaaccatac cgtgtaattc ttggcgatgt gagagataag ctgtatcaga cacgtgagcg    1200
tactcgccaa atgttagccc atggaatctc tgatattcct gaggatgcaa cttataataa    1260
tgttgagcag ttcttggaac ctcttgaact ctgctacaga tctctttgtg aatgtggtga    1320
tcgccccatt gccgatggaa gccttctgga tttcttaga caagtttcta cctttggact     1380
ctcatttgtt agacttgaca taagacaaga gtcagaccgc cacactgacg tccttgatgc    1440
cattacccag cacttggaaa tcggttcata tcgagagtgg tctgaagaac gtagacagga    1500
gtggcttctt tctgaactca gcggcaagag acctttattt ggacctgatc ttccaagaac    1560
cgaagaaatt gctgacgtct tggatacgct ccatgtcata gcagaacttc catctgactg    1620
cttcggggca tatatcatct caatggccac tgcaccatct gacgtgcttg cagttgagct    1680
cctacagcgt gaatgccatg tgaagcaacc tttacgagtg gttccacttt tcgaaaagtt    1740
ggatgatctg gagtctgcgt ctgcggctgt tgcacgtctc ttttcgattg agtggtacag    1800
aaaccggatt aacggtaaac aagaggtcat ggtcgggtat tcagactctg gcaaggatgc    1860
```

```
tggtcgtttc tcagcagcgt ggcagctata taaggctcaa gaggagctta taaaagttgc    1920
caaagaacat ggtgtgaagc taactatgtt ccacggtaga ggtggtacag taggaagagg    1980
aggtggcccc acccatcttg ctatattgtc tcagccacca gatacaattc agggatctct    2040
ccgtgtcaca gttcagggtg aagttatcga gcaatcgttt ggggaggaac acttgtgttt    2100
taggactctc caacgtttca ctgctgctac ccttgaacat gggatgcatc cacccgtctc    2160
tcccaaacca gaatggcgtg cacttatgga tgaaatcgcg gttattgcta cagagaagta    2220
taggtcaata gttttcaaag aacctcgatt tgttgagtat tccgccttgg ccacacctga    2280
gttagagtat ggtcgaatga acattggaag ccgtccatca aagcgtaaac ctagtggagg    2340
aatagaatca ctcagagcta ttccatggat ctttgcctgg acgcaaacta ggtttcatct    2400
ccccgtctgg cttggctttg ggcggcatt taagtatgcc attgacaagg atataaaaaa    2460
ccttcgcatg tttcacgaaa tgtacaatga atggccattc tttagagtca ctattgactt    2520
ggttgagatg gtgttcgcca agggaaaccc cggtattgca gctttgtatg acaagcttct    2580
cgtttcagaa gatttgttgc ctttcggcga gcttttgagg tccaactacg aggagacaag    2640
gagcctccta ctccagattg ctggacacaa ggatcttctg gagggagacc cctacttgaa    2700
acaacgactc aggctgcgtg attcctacat cacaacctta aacttgttgc aagcctacac    2760
tctcaagcgt atacgtgatc ctaactatca tgtcacgcta aggcctcata tttcgaagga    2820
ttacatggaa tcgaagtcag ctgctgaact tgtgcagctg aacccgacaa gtgaatatgc    2880
ccccggcttg gaggacacac tcatcttgac aatgaagggt attgctgctg gactgcagaa    2940
taccggttaa actcattctg atgttcgtgt tttttctaat tttttatata ctcgaaaagc    3000
atcattctgg tttatgagag gtagatgttt ttcatttctg ttgtgttttg tggtggtgaa    3060
agttttttat tccaccctat atatcatgtt tgataaaact aataagactc catttgagtg    3120
aggtttactt caccaaaatg aactgtaatc tcattgttgg ctcaatggaa tatcaagttt    3180
cttagatca                                                            3189
```

```
<210>  42
<211>  964
<212>  PRT
<213>  Nicotiana tabacum

<400>  42
Met Ala Thr Arg Ser Leu Glu Lys Leu Ala Ser Ile Asp Ala Gln Leu
1               5                   10                  15
Arg Ala Leu Val Pro Gly Lys Val Ser Glu Asp Asp Lys Leu Val Glu
            20                  25                  30
Tyr Asp Ala Leu Leu Leu Asp Arg Phe Leu Asp Ile Leu Gln Asp Leu
        35                  40                  45
His Gly Glu Asp Leu Lys Glu Thr Val Gln Glu Cys Tyr Glu Leu Ser
    50                  55                  60
Ala Glu Tyr Glu Gly Lys His Asp Pro Lys Lys Leu Glu Glu Leu Gly
65                  70                  75                  80
Asn Val Leu Thr Ser Leu Asp Pro Gly Asp Ser Ile Val Ile Ala Lys
                85                  90                  95
Ala Phe Ser His Met Leu Asn Leu Ala Asn Leu Ala Glu Glu Val Gln
            100                 105                 110
Ile Ala Tyr Arg Arg Arg Gln Lys Leu Lys Arg Gly Asp Phe Ala Asp
        115                 120                 125
Glu Asn Asn Ala Thr Thr Glu Ser Asp Ile Glu Glu Thr Phe Lys Lys
    130                 135                 140
Leu Val Gly Asp Leu Lys Lys Ser Pro Gln Glu Val Phe Asp Ala Leu
145                 150                 155                 160
Lys Asn Gln Thr Val Asp Leu Val Leu Thr Ala His Pro Thr Gln Ser
                165                 170                 175
Val Arg Arg Ser Leu Leu Gln Lys His Gly Arg Ile Arg Asp Cys Leu
            180                 185                 190
Ala Gln Leu Tyr Ala Lys Asp Ile Thr Pro Asp Asp Lys Gln Glu Leu
        195                 200                 205
Asp Glu Ala Leu Gln Arg Glu Ile Gln Ala Ala Phe Arg Thr Asp Glu
    210                 215                 220
Ile Arg Arg Thr Ala Pro Thr Pro Gln Asp Glu Met Arg Ala Gly Met
225                 230                 235                 240
Ser Tyr Phe His Glu Thr Ile Trp Lys Gly Val Pro Lys Phe Leu Arg
                245                 250                 255
Arg Val Asp Thr Ala Leu Lys Asn Ile Gly Ile Asn Glu Arg Leu Pro
            260                 265                 270
Tyr Asn Ala Pro Leu Ile Gln Phe Ser Ser Trp Met Gly Gly Asp Arg
        275                 280                 285
Asp Gly Asn Pro Arg Val Thr Leu Glu Val Thr Arg Asp Val Cys Leu
    290                 295                 300
Leu Ala Arg Met Met Ala Ala Asn Leu Tyr Tyr Ser Gln Ile Glu Glu
305                 310                 315                 320
Leu Met Phe Glu Leu Ser Met Trp Arg Cys Asn Asp Asp Leu Arg Ile
                325                 330                 335
Arg Ala Ala Glu Leu Tyr Arg Ser Ser Arg Arg Asp Thr Lys His Tyr
            340                 345                 350
```

```
Ile Glu Phe Trp Lys Thr Ile Pro Pro Ser Glu Pro Tyr Arg Val Ile
            355                 360                 365
Leu Gly Asp Val Arg Asp Lys Leu Tyr Gln Thr Arg Glu Arg Thr Arg
    370                 375                 380
Gln Met Leu Ala His Gly Ile Ser Asp Ile Pro Glu Asp Ala Thr Tyr
385                 390                 395                 400
Asn Asn Val Glu Gln Phe Leu Glu Pro Leu Glu Leu Cys Tyr Arg Ser
                405                 410                 415
Leu Cys Glu Cys Gly Asp Arg Pro Ile Ala Asp Gly Ser Leu Leu Asp
            420                 425                 430
Phe Leu Arg Gln Val Ser Thr Phe Gly Leu Ser Phe Val Arg Leu Asp
        435                 440                 445
Ile Arg Gln Glu Ser Asp Arg His Thr Asp Val Leu Asp Ala Ile Thr
    450                 455                 460
Gln His Leu Glu Ile Gly Ser Tyr Arg Glu Trp Ser Glu Glu Arg Arg
465                 470                 475                 480
Gln Glu Trp Leu Leu Ser Glu Leu Ser Gly Lys Arg Pro Leu Phe Gly
                485                 490                 495
Pro Asp Leu Pro Arg Thr Glu Glu Ile Ala Asp Val Leu Asp Thr Leu
            500                 505                 510
His Val Ile Ala Glu Leu Pro Ser Asp Cys Phe Gly Ala Tyr Ile Ile
        515                 520                 525
Ser Met Ala Thr Ala Pro Ser Asp Val Leu Ala Val Glu Leu Leu Gln
    530                 535                 540
Arg Glu Cys His Val Lys Gln Pro Leu Arg Val Val Pro Leu Phe Glu
545                 550                 555                 560
Lys Leu Asp Asp Leu Glu Ser Ala Ser Ala Ala Val Ala Arg Leu Phe
                565                 570                 575
Ser Ile Glu Trp Tyr Arg Asn Arg Ile Asn Gly Lys Gln Glu Val Met
            580                 585                 590
Val Gly Tyr Ser Asp Ser Gly Lys Asp Ala Gly Arg Phe Ser Ala Ala
        595                 600                 605
Trp Gln Leu Tyr Lys Ala Gln Glu Glu Leu Ile Lys Val Ala Lys Glu
    610                 615                 620
His Gly Val Lys Leu Thr Met Phe His Gly Arg Gly Gly Thr Val Gly
625                 630                 635                 640
Arg Gly Gly Gly Pro Thr His Leu Ala Ile Leu Ser Gln Pro Pro Asp
                645                 650                 655
Thr Ile Gln Gly Ser Leu Arg Val Thr Val Gln Gly Glu Val Ile Glu
            660                 665                 670
Gln Ser Phe Gly Glu Glu His Leu Cys Phe Arg Thr Leu Gln Arg Phe
        675                 680                 685
Thr Ala Ala Thr Leu Glu His Gly Met His Pro Pro Val Ser Pro Lys
    690                 695                 700
Pro Glu Trp Arg Ala Leu Met Asp Glu Ile Ala Val Ile Ala Thr Glu
705                 710                 715                 720
Lys Tyr Arg Ser Ile Val Phe Lys Glu Pro Arg Phe Val Glu Tyr Ser
                725                 730                 735
Ala Leu Ala Thr Pro Glu Leu Glu Tyr Gly Arg Met Asn Ile Gly Ser
            740                 745                 750
Arg Pro Ser Lys Arg Lys Pro Ser Gly Gly Ile Glu Ser Leu Arg Ala
        755                 760                 765
Ile Pro Trp Ile Phe Ala Trp Thr Gln Thr Arg Phe His Leu Pro Val
    770                 775                 780
Trp Leu Gly Phe Gly Ala Ala Phe Lys Tyr Ala Ile Asp Lys Asp Ile
785                 790                 795                 800
Lys Asn Leu Arg Met Phe His Glu Met Tyr Asn Glu Trp Pro Phe Phe
                805                 810                 815
Arg Val Thr Ile Asp Leu Val Glu Met Val Phe Ala Lys Gly Asn Pro
            820                 825                 830
Gly Ile Ala Ala Leu Tyr Asp Lys Leu Leu Val Ser Glu Asp Leu Leu
        835                 840                 845
Pro Phe Gly Glu Leu Leu Arg Ser Asn Tyr Glu Glu Thr Arg Ser Leu
    850                 855                 860
Leu Leu Gln Ile Ala Gly His Lys Asp Leu Leu Glu Gly Asp Pro Tyr
865                 870                 875                 880
Leu Lys Gln Arg Leu Arg Leu Arg Asp Ser Tyr Ile Thr Thr Leu Asn
                885                 890                 895
Leu Leu Gln Ala Tyr Thr Leu Lys Arg Ile Arg Asp Pro Asn Tyr His
            900                 905                 910
Val Thr Leu Arg Pro His Ile Ser Lys Asp Tyr Met Glu Ser Lys Ser
        915                 920                 925
Ala Ala Glu Leu Val Gln Leu Asn Pro Thr Ser Glu Tyr Ala Pro Gly
    930                 935                 940
```

Leu Glu Asp Thr Leu Ile Leu Thr Met Lys Gly Ile Ala Ala Gly Leu
945 950 955 960
Gln Asn Thr Gly

<210> 43
<211> 3048
<212> DNA
<213> Glycine max

<400> 43
```
ttttaggtga gggactgaaa ggtagcaatg gcgaccagga acttggaaaa gatggcatcc      60
attgatgcac agcttaggca attggctcct gccaaagtga gtgaggatga caaactgatt     120
gagtatgatg ctcttctgct ggatcggttc cttgatatcc ttcaagattt acatggggag     180
gatctgaagg aaacagttca agaagtgtat gaactttcag ctgagtatga aggaaagcat     240
gaccctaaga aactggaaga acttggaaat ttgataacca gtttggatgc tggggactct     300
attcttgttg ccaagtcctt ttcccacatg cttaatttgg ccaacttggc tgaagaggtc     360
cagatttctc gccgccgaag aaacaagttg aagaaagggg attttgcaga tgagaacaat     420
gcaactacag aatcagacat tgaagaaact ctcaagaaac ttgtatttga cttgaagaag     480
tctcctcagg aagtttttga tgcactgaaa aaccagactg ttgatttggt tcttactgct     540
catcctactc aatcaattcg tagatctttg cttcaaaagc atggaaggat aaggaattgt     600
ttatctcaat tgtatgccaa agacattact cctgatgata agcaggagct tgatgaggct     660
ctacaaaggg agattcaagc tgccttccgt acagatgaaa tcaggaggac ccctccaaca     720
ccacaagatg agatgagagc agggatgagc tacttccatg aaacaatttg gaacggtgtt     780
cccagatttc tgcgccgtgt agacacagct ttgaacaata tcgggattaa agagcgtgtt     840
ccttataatg ctccccttat tcaattttct tcttggatgg ggggtgatcg cgatggtaat     900
ccaagagtaa ctcctgaagt gacaagggat gtttgcttat tggctagaat gatggctgct     960
aatttgtatt attcccagat agaagatctt atgtttgagc tctctatgtg gcgctgcaat    1020
gatgaactac gcgttcgtgc agaagaactt cacaggtctt ccaagaaaga tgaagttgca    1080
aaacactata tagaattttg gaaaaaggtt cccccaaatg aaccatatcg tgtggtactc    1140
ggtgaagtaa gggataggct ctatcagact cgtgagcgtt ctcgccattt gctttctaat    1200
gggtactctg acattccaga ggaagccact ttcaccaatg ttgaggagtt cctggaatct    1260
cttgaactat gttacagatc actatgtgct tgtggtgata gagcaattgc tgatggaagc    1320
cttcttgatt tcatgagaca agtctccact tttggactgt cactagtgag gcttgatatc    1380
aggcaagagt cagatcgtca cactgatgtg ctggatgcca ttaccaaaca cttggaaatt    1440
ggctcgtacc aggaatggtc tgaagagaaa agacaggaat ggttgttgtc tgagttaagt    1500
ggcaaaaggc ctctatttgg acctgacctt cctcaaactg aagaaattag agatgttttg    1560
gacacatttc atgtcatagc agaactacca ccagacaact ttggagccta tatcatatca    1620
atggcaactg caccatctga tgtgcttgca gttgagcttc tacaacgtga atgtcacatc    1680
aagcatccct taagagttgt gccattgttt gagaagctag ctgatctaga ggctgctcct    1740
gctgctctgg cacggttgtt ctcgatagac tggtacagaa ataggatcaa tgggaagcaa    1800
gaagtgatga ttggatactc agattcaggg aaagatgctg ggaggttctc tgcagcatgg    1860
cagctatata aggctcagga ggaacttata aatgttgcca agaaatttgg tgttaagcta    1920
accatgttcc atggtcgcgg tggaactgtt ggaagaggag gtggacctac tcatcttgct    1980
attctgtctc aacctccaga cacaatccat ggatcacttc gtgtgacagt ccaaggtgaa    2040
gtcattgagc aatcatttgg agaacaacac ttgtgcttta gaacactaca acgtttcact    2100
gccgccactc tagaacatgg catgcacccc ccaatttcgc caaaaccaga atggcgtgct    2160
ttgatggatc agatggctgt cattgctact gaggaatacc gttccattgt attcaaggaa    2220
ccacgctttg ttgagtattt ccgcctggct acaccagagt tggagtatgg aaggatgaat    2280
attggaagtc gaccagcaaa gagaagacct agtggaggca ttgaaacact gcgtgcaata    2340
ccttggattt ttgcatggac tcagacaagg tttcatcttc cagtgtggct aggctttgga    2400
gcagcattta aaaaagtcat tgaggaaaat gttaagaatc tcaatatgct gcaagagatg    2460
tacaatcaat ggcctttctt tagggtcaca cttgatttgg tggaaatggt gtttgccaaa    2520
ggagatccga aaattgccgc tctgaatgat agactccttg tttcaaagga tctgtggccg    2580
tttggggatc aattgaggaa caaatatgaa gaaactagga aactcctact tcaggtggct    2640
ggacacaagg aaattcttga aggggaccct tacttgaagc aaagactcag gcttcgtcat    2700
gctcccatta ccaccctcaa tattgtccaa gcttacacat tgaaacgtat ccgtgatcct    2760
aactacaatg tgaaggtgcg cccccgcata tcaaaggaat ctgcagaggc aagcaaatca    2820
gctgatgaac ttgtcaaatt gaacccaaca agtgaatatg cccctggttt ggaagacaca    2880
ctcattctca ctatgaaggg tattgctgct ggcatgcaga acactggtta agtttgtttg    2940
aatactgaaa gtgagtgctc tgagttttgc tcacagaata cacttttttt gttttgttat    3000
tatgttgccc cacctttctg tacaatttgg tatgctgctc tagaattc                  3048
```

<210> 44
<211> 967
<212> PRT
<213> Glycine max

<400> 44
Met Ala Thr Arg Asn Leu Glu Lys Met Ala Ser Ile Asp Ala Gln Leu
1 5 10 15
Arg Gln Leu Ala Pro Ala Lys Val Ser Glu Asp Asp Lys Leu Ile Glu
20 25 30
Tyr Asp Ala Leu Leu Leu Asp Arg Phe Leu Asp Ile Leu Gln Asp Leu

```
            35                  40                  45
His Gly Glu Asp Leu Lys Glu Thr Val Gln Glu Val Tyr Glu Leu Ser
    50                  55                  60
Ala Glu Tyr Glu Gly Lys His Asp Pro Lys Lys Leu Glu Glu Leu Gly
65                  70                  75                  80
Asn Leu Ile Thr Ser Leu Asp Ala Gly Asp Ser Ile Leu Val Ala Lys
                85                  90                  95
Ser Phe Ser His Met Leu Asn Leu Ala Asn Leu Ala Glu Glu Val Gln
            100                 105                 110
Ile Ser Arg Arg Arg Arg Asn Lys Leu Lys Lys Gly Asp Phe Ala Asp
        115                 120                 125
Glu Asn Asn Ala Thr Thr Glu Ser Asp Ile Glu Glu Thr Leu Lys Lys
    130                 135                 140
Leu Val Phe Asp Leu Lys Lys Ser Pro Gln Glu Val Phe Asp Ala Leu
145                 150                 155                 160
Lys Asn Gln Thr Val Asp Leu Val Leu Thr Ala His Pro Thr Gln Ser
                165                 170                 175
Ile Arg Arg Ser Leu Leu Gln Lys His Gly Arg Ile Arg Asn Cys Leu
            180                 185                 190
Ser Gln Leu Tyr Ala Lys Asp Ile Thr Pro Asp Asp Lys Gln Glu Leu
        195                 200                 205
Asp Glu Ala Leu Gln Arg Glu Ile Gln Ala Ala Phe Arg Thr Asp Glu
    210                 215                 220
Ile Arg Arg Thr Pro Pro Thr Pro Gln Asp Glu Met Arg Ala Gly Met
225                 230                 235                 240
Ser Tyr Phe His Glu Thr Ile Trp Asn Gly Val Pro Arg Phe Leu Arg
                245                 250                 255
Arg Val Asp Thr Ala Leu Asn Asn Ile Gly Ile Lys Glu Arg Val Pro
            260                 265                 270
Tyr Asn Ala Pro Leu Ile Gln Phe Ser Ser Trp Met Gly Gly Asp Arg
        275                 280                 285
Asp Gly Asn Pro Arg Val Thr Pro Glu Val Thr Arg Asp Val Cys Leu
    290                 295                 300
Leu Ala Arg Met Met Ala Ala Asn Leu Tyr Tyr Ser Gln Ile Glu Asp
305                 310                 315                 320
Leu Met Phe Glu Leu Ser Met Trp Arg Cys Asn Asp Glu Leu Arg Val
                325                 330                 335
Arg Ala Glu Glu Leu His Arg Ser Ser Lys Lys Asp Glu Val Ala Lys
            340                 345                 350
His Tyr Ile Glu Phe Trp Lys Lys Val Pro Pro Asn Glu Pro Tyr Arg
        355                 360                 365
Val Val Leu Gly Glu Val Arg Asp Arg Leu Tyr Gln Thr Arg Glu Arg
    370                 375                 380
Ser Arg His Leu Leu Ser Asn Gly Tyr Ser Asp Ile Pro Glu Glu Ala
385                 390                 395                 400
Thr Phe Thr Asn Val Glu Glu Phe Leu Glu Ser Leu Glu Leu Cys Tyr
                405                 410                 415
Arg Ser Leu Cys Ala Cys Gly Asp Arg Ala Ile Ala Asp Gly Ser Leu
            420                 425                 430
Leu Asp Phe Met Arg Gln Val Ser Thr Phe Gly Leu Ser Leu Val Arg
        435                 440                 445
Leu Asp Ile Arg Gln Glu Ser Asp Arg His Thr Asp Val Leu Asp Ala
    450                 455                 460
Ile Thr Lys His Leu Glu Ile Gly Ser Tyr Gln Glu Trp Ser Glu Glu
465                 470                 475                 480
Lys Arg Gln Glu Trp Leu Leu Ser Glu Leu Ser Gly Lys Arg Pro Leu
                485                 490                 495
Phe Gly Pro Asp Leu Pro Gln Thr Glu Glu Ile Arg Asp Val Leu Asp
            500                 505                 510
Thr Phe His Val Ile Ala Glu Leu Pro Pro Asp Asn Phe Gly Ala Tyr
        515                 520                 525
Ile Ile Ser Met Ala Thr Ala Pro Ser Asp Val Leu Ala Val Glu Leu
    530                 535                 540
Leu Gln Arg Glu Cys His Ile Lys His Pro Leu Arg Val Val Pro Leu
545                 550                 555                 560
Phe Glu Lys Leu Ala Asp Leu Glu Ala Ala Pro Ala Ala Leu Ala Arg
                565                 570                 575
Leu Phe Ser Ile Asp Trp Tyr Arg Asn Arg Ile Asn Gly Lys Gln Glu
            580                 585                 590
Val Met Ile Gly Tyr Ser Asp Ser Gly Lys Asp Ala Gly Arg Phe Ser
        595                 600                 605
Ala Ala Trp Gln Leu Tyr Lys Ala Gln Glu Glu Leu Ile Asn Val Ala
    610                 615                 620
Lys Lys Phe Gly Val Lys Leu Thr Met Phe His Gly Arg Gly Gly Thr
```

```
625                 630                 635                 640
Val Gly Arg Gly Gly Gly Pro Thr His Leu Ala Ile Leu Ser Gln Pro
                645                 650                 655
Pro Asp Thr Ile His Gly Ser Leu Arg Val Thr Val Gln Gly Glu Val
            660                 665                 670
Ile Glu Gln Ser Phe Gly Glu Gln His Leu Cys Phe Arg Thr Leu Gln
        675                 680                 685
Arg Phe Thr Ala Ala Thr Leu Glu His Gly Met His Pro Pro Ile Ser
    690                 695                 700
Pro Lys Pro Glu Trp Arg Ala Leu Met Asp Gln Met Ala Val Ile Ala
705                 710                 715                 720
Thr Glu Glu Tyr Arg Ser Ile Val Phe Lys Glu Pro Arg Phe Val Glu
                725                 730                 735
Tyr Phe Arg Leu Ala Thr Pro Glu Leu Glu Tyr Gly Arg Met Asn Ile
            740                 745                 750
Gly Ser Arg Pro Ala Lys Arg Arg Pro Ser Gly Gly Ile Glu Thr Leu
        755                 760                 765
Arg Ala Ile Pro Trp Ile Phe Ala Trp Thr Gln Thr Arg Phe His Leu
    770                 775                 780
Pro Val Trp Leu Gly Phe Gly Ala Ala Phe Lys Lys Val Ile Glu Glu
785                 790                 795                 800
Asn Val Lys Asn Leu Asn Met Leu Gln Glu Met Tyr Asn Gln Trp Pro
                805                 810                 815
Phe Phe Arg Val Thr Leu Asp Leu Val Glu Met Val Phe Ala Lys Gly
            820                 825                 830
Asp Pro Lys Ile Ala Ala Leu Asn Asp Arg Leu Leu Val Ser Lys Asp
        835                 840                 845
Leu Trp Pro Phe Gly Asp Gln Leu Arg Asn Lys Tyr Glu Glu Thr Arg
    850                 855                 860
Lys Leu Leu Leu Gln Val Ala Gly His Lys Glu Ile Leu Glu Gly Asp
865                 870                 875                 880
Pro Tyr Leu Lys Gln Arg Leu Arg Leu Arg His Ala Pro Ile Thr Thr
                885                 890                 895
Leu Asn Ile Val Gln Ala Tyr Thr Leu Lys Arg Ile Arg Asp Pro Asn
            900                 905                 910
Tyr Asn Val Lys Val Arg Pro Arg Ile Ser Lys Glu Ser Ala Glu Ala
        915                 920                 925
Ser Lys Ser Ala Asp Glu Leu Val Lys Leu Asn Pro Thr Ser Glu Tyr
    930                 935                 940
Ala Pro Gly Leu Glu Asp Thr Leu Ile Leu Thr Met Lys Gly Ile Ala
945                 950                 955                 960
Ala Gly Met Gln Asn Thr Gly
                965
```

<210> 45
<211> 5920
<212> DNA
<213> Solanum tuberosum

<400> 45

```
ctatacctta ttgagtgtcc aaagtaaaga cttttttttc tcatcttttg tgttctgaca     60
taccaaaatt caggtactga acattattta tgttctccaa tttgccatac tcaaaatttc    120
aatctcttgt tttttttgtt tttttttgct aacaaataaa tcaacaagaa tataaaactt    180
agtagagagt tgccaactgg ggtttttgtt tgttttgaat tgccagaaca agagaaaaaa    240
aagtggtccc ttttttgtac ttttcttggg gtcatgatca gatattgtgg tcactgtaat    300
ttgtaaaaaa aatttgatga tttttatgtt tatgtctatt gctgcagagt gttgactgtt    360
gagtgagaaa tgactacgag gaatttggag aaacttgcat cgattgatgc acagttgagg    420
caattggtgc ctgctaaagt ttctgaagat gataaacttg ttgagtatga tgctttgctt    480
ttggataggt ttcttgatat tcttcaagat ttacatgggg aggatctcaa agaaacagta    540
tgcatttttc ccaatttatg tttgctgtta atgtaaccta tgaatgattt agacaattgt    600
aaattatcta cctaagttgt agtcttttat gttgtgtacc ctttgatgaa gttctgtgat    660
ttttgtcttt tggttactaa ggggttgttg tttttgtctt ccacttttaa tatggtttat    720
ttcttgaagt taaagattgg atttttatgt cacttgtaaa aaaggggaat aaaaagattg    780
gatttttatg tctgttctga ctgagttgac ttcaaacact tgaattggtt actaaagggt    840
gttgtttttg tcttccactt ttaatctggt ctatttcttg aagtaaaaaa gattgaattt    900
ttatgtcact tacaaaaaaa ggaacaaaaa gattggattt ttatgtctgt tctgactgag    960
tgaactacaa acatttgatt ctgaggtgtg tgtaatactt agagatggta tcgatataat   1020
ggtattcata tgcttcagta tagtttgact gtgttagtgg atgaaatccc atgtgtatgt   1080
tgacttgaga tcctaaactt gtgttgacca agtagtcttg ttttttcgga atgttttgtc   1140
gtgttttctt tagtatttgc cagatttctt cacttctgtt atttcttttt cgatcttatt   1200
ttccttaagc tgagggtata tcagaaacag tctctctacc tctcaaggta ggggtaaggt   1260
cttggtacac tctaccctcc ccagacccta cttgtggaat tacactgagt atgttgttgt   1320
aattgcaaaa gactggtcaa catttaagtc tttgatattt tcaatctcac attaggtcca   1380
agagtgttat gagctttctg ccgagtatga agccaagcat gatccgaaga agctggagga   1440
```

```
acttggcaat gtgttgacaa gtttggaccc aggagattca attgtcattg ctaaagcttt   1500
ctctcacatg cttaacttgg ccaatttggc tgaggaggtt cagattgcct accgtaggcg   1560
ccaaaaattg aagaaaaagg gagattttgg ggatgagagc aatgcaacaa ctgagtcaga   1620
tattgaagaa actttcaaga aattggtggg ggacttgaag aagtcccctc aagaagtttt   1680
tgatgctatc aagaatcaga ctgtggatct ggtcctaaca gctcatccta cacaatctgt   1740
acgaagatca ttgcttcaaa agcatggaag gtttggtctt taatcaccat gtttattgtc   1800
ttcagcagtt agagttgtct ccttggcttg tcattttatg ctagtttgtg cttcgatttt   1860
tccctaataa atataattgg aaatgctctt ttcggaccaa attttcgtgc tttcgtcggt   1920
tgtttactta cttgttctaa ccaaattttc aggatccgtg actgcttggc tcagttgtat   1980
gctaaagaca ttacacccga tgataaacaa gaacttgatg aggctttaca gagggaggta   2040
actatcgacg tttagttact tgatacatgt gtttttcctt gaatgaaatt ctattattag   2100
taccttcaat tctggtgtct ttctgactct actaatggat attttggtgc ttcaatatca   2160
tatagatatg tcttgatctg tcaggcctta tattatcaat ggacttactt ttttcatccc   2220
tctcatgaag attggagaag caaaaattat ctcataagtt acccatttaa tagttgcccc   2280
ttcattcatt tgaatgattt taccctgtaa aaagtggagc tggttatatg attgctctaa   2340
aattggatat gaagcaataa tagcaatctt cgaactcaaa tatgtgtcgc attctgtgga   2400
tcatggttta ttggtatgat gcacccatta ttcttttttg acaaatatct gctacttact   2460
tcctctgtag atccaagctg cgttccgaac tgatgagatc cgaagaactc ctcctactcc   2520
acaagatgaa atgagagctg gaatgagcta tttccatgaa acaatttgga agggtgtacc   2580
aaagtttctg cgccgtgttg atacagctct taaaaacata gggattaatg aacgagttcc   2640
ttataatgct ccccttattc aattctcctc ttggatgggc ggtgatcgtg atggtatgat   2700
tcctttactg tggagaatgt ttgcagttac tttagctaaa agctgagaaa acaaaatagg   2760
aattcggatt acataatttt gaaggggtgg aggaaagcaa aatactcctt ctgcttgaac   2820
gtggattact tagttactac atgcagtaga taatccttat ttatctgtca actctctgct   2880
accaagtaca ggtaatccaa gagtgactcc tgaggtcaca agagatgttt gcttattggc   2940
cagaatgatg gcagctaact tgtactattc gcaaatagag gacctcatgt ttgaggtatc   3000
caacaattaa atgtttttga gacttttgtc ttggttcaat tagtgaaaaa atcgcactat   3060
ctacactgtg atctttttct aaattgtaca atctttcagt tatctatgtg gcgctgcaat   3120
gaagaacttc gtgtgcgagc agatgatctc caaaggtctt caaggagaga tgaaaaacac   3180
tacataggta actaattgaa tattgctatt aaggttgatc catgaaattg aatcaaagaa   3240
gagttttcta gttgcatata accttgatag gacttttaaa tgcgtgtttg aagtatgatt   3300
caaattctcc tttcaagtga gccaagggtg tctttcatca ttgccgatgg gaaaaaaaaa   3360
gggtgagcaa agggtgttat tatttatgat ttaaagattg agaaaaagag gtcattctag   3420
catctggtat gctgattaga aattgcacga gaagctctct gtattctctt ggaaagatgt   3480
tcctttcttc caaaaaaatc tttggacaga gaaatcgcat aatctgtgat ctttcaacat   3540
tttcttctca tgcagaattt tggaagcaag ttcctccaaa tgaaccctat cgtgtaattc   3600
ttggtgatgt gagagataag ttgtatcaga cacgtgagcg tgctcgccaa ctgttaggcc   3660
atggatactc tgaaattcca gaggaagcaa cttatactaa tattgagcag gttcataata   3720
ctatacttct gtcttttcct gtgactgctt tctatatgta gacccttcta aattaagcag   3780
tgaatattca cttgtactct tgaaagctta actagtttta ctaaacagct tgacatgtta   3840
ttcatcgaaa aaatgaacta gaaaaacatg aggaagtgtg tctctttctt gttgaataac   3900
agcataagca tttgcatggt tgttctacca aaccaaaaac atataaatat ctgtgttgca   3960
tgtcatataa cactaatatg gtcatcattt tatgtagttc ttggagcctc ttgagctctg   4020
ctacagatct ctttgtgctt gtggtgacct ctccatcgcg gatggtagcc ttctggattt   4080
cctgagacaa gtttctacgt ttggactttc acttgtgaga cttgacataa gacaagagtc   4140
ggaccgccat actgatgtgc ttgacgctat tacgcaacat ttggaaattg gttcatatcg   4200
agactggtct gaagaacgta gacaagagtg gcttctgtct gaactcagtg gcaagagacc   4260
tttatttgga cctgatcttc caaaaactga agaaattgct gatgttttgg atacgttcca   4320
tgtcatagcc gaactcccag cagactgctt cggggcatac atcatctcaa tggccactgc   4380
accatctgat gttcttgcag tagagcttct acagcgtgaa tgccgagtga ggcaaccttt   4440
acgagttgtt ccactttttg agaagttggc tgatctggat gctgctcctg cagccgttgc   4500
acgtcttttc tcaattgagt ggtacagaaa ccggattaat ggcaagcaag aagtgatgat   4560
tggatactcc gactctggca aggatgcagg tcggctatca gcagcctggc agttatataa   4620
ggctcaagag gagcttatac aagtcgccaa ggagttcgac gtgaagctaa ccatgttcca   4680
tggtagaggt ggtacagtgg gaagaggagg tggccccgcc catcttgcca tattgtctca   4740
accacccgaa acaattcacg gatctctccg tgttacagtt caggtgaggt ttattgagca   4800
gtcttttggg gaagaacact tgtgttttag gacactccag cgttttactg ctgctacact   4860
tgaacatggg atgcatccac cagtctctcc aaaaccagaa tggcgtgcac ttatggacga   4920
aattgcagtt gttgcaacag aaaagtatcg atcaatagtt tttaaggaac cccgatttgt   4980
cgagtatttc cgcctggtaa gtatgctagc aatgaactca gttattaatt gtagatgctg   5040
tcacaaatat tgccttgagt tgtaataagc tatttttcat tgatcatgtt tgcaggccac   5100
acccgagtta gagtatggtc gaatgaacat tggcagccgt ccatcaaagc gtaaacccag   5160
cggaggcata gaatcactta gagctattcc gtggatcttt gcttggactc aaactagatt   5220
ccatcttcca gtctggctcg gctttggagc agcatttaag tatgccattg aaaaggatat   5280
caaaaacctt cgcatgctgc aggaaatgta caatgcatgg cctttcttta gggtaactat   5340
cgatttggtt gagatggtgt tcgccaaagg agacccaggc attgctgcat tgttcgacaa   5400
gcttctggtg tccgaagatt tgtggtcttt cggtgaactt ttgaggtcaa agtatgagga   5460
gacaaagagc ctcctcctgc aggtaatgtg acatatactt ctaaaaagga tcggttgaac   5520
acttattgtg ctcgattgct tcacaaactg gttccaagtt agtacatcta aacataattt   5580
tccctttccc tttttaaaat agattgctgg acacaaggat cttctggagg gtgatccata   5640
cttaaaacaa cgactcaggt tgcgtgactc ctacatcaca acattgaatg tgtgtcaagc   5700
ctacacccta aagcgtattc gtgacccaga ctatagtgtc acaccaaggc ctcacatttc   5760
caaggaatac atggaagcaa aaccagctac agaacttgtg aacctgaacc cgactagcga   5820
atatgcacct ggcttggagg acacactcat cttaaccatg aaaggtattg ctgctggaat   5880
```

gcagaatact ggttagtcgt cacctggaat atggaagatg 5920

<210> 46
<211> 965
<212> PRT
<213> Solanum tuberosum

<400> 46
Met Thr Thr Arg Asn Leu Glu Lys Leu Ala Ser Ile Asp Ala Gln Leu
1 5 10 15
Arg Gln Leu Val Pro Ala Lys Val Ser Glu Asp Asp Lys Leu Val Glu
20 25 30
Tyr Asp Ala Leu Leu Leu Asp Arg Phe Leu Asp Ile Leu Gln Asp Leu
35 40 45
His Gly Glu Asp Leu Lys Glu Thr Val Gln Glu Cys Tyr Glu Leu Ser
50 55 60
Ala Glu Tyr Glu Ala Lys His Asp Pro Lys Lys Leu Glu Glu Leu Gly
65 70 75 80
Asn Val Leu Thr Ser Leu Asp Pro Gly Asp Ser Ile Val Ile Ala Lys
85 90 95
Ala Phe Ser His Met Leu Asn Leu Ala Asn Leu Ala Glu Glu Val Gln
100 105 110
Ile Ala Tyr Arg Arg Arg Gln Lys Leu Lys Lys Lys Gly Asp Phe Gly
115 120 125
Asp Glu Ser Asn Ala Thr Thr Glu Ser Asp Ile Glu Glu Thr Phe Lys
130 135 140
Lys Leu Val Gly Asp Leu Lys Lys Ser Pro Gln Glu Val Phe Asp Ala
145 150 155 160
Ile Lys Asn Gln Thr Val Asp Leu Val Leu Thr Ala His Pro Thr Gln
165 170 175
Ser Val Arg Arg Ser Leu Leu Gln Lys His Gly Arg Ile Arg Asp Cys
180 185 190
Leu Ala Gln Leu Tyr Ala Lys Asp Ile Thr Pro Asp Asp Lys Gln Glu
195 200 205
Leu Asp Glu Ala Leu Gln Arg Glu Ile Gln Ala Ala Phe Arg Thr Asp
210 215 220
Glu Ile Arg Arg Thr Pro Pro Thr Pro Gln Asp Glu Met Arg Ala Gly
225 230 235 240
Met Ser Tyr Phe His Glu Thr Ile Trp Lys Gly Val Pro Lys Phe Leu
245 250 255
Arg Arg Val Asp Thr Ala Leu Lys Asn Ile Gly Ile Asn Glu Arg Val
260 265 270
Pro Tyr Asn Ala Pro Leu Ile Gln Phe Ser Ser Trp Met Gly Gly Asp
275 280 285
Arg Asp Gly Asn Pro Arg Val Thr Pro Glu Val Thr Arg Asp Val Cys
290 295 300
Leu Leu Ala Arg Met Met Ala Ala Asn Leu Tyr Tyr Ser Gln Ile Glu
305 310 315 320
Asp Leu Met Phe Glu Leu Ser Met Trp Arg Cys Asn Glu Glu Leu Arg
325 330 335
Val Arg Ala Asp Asp Leu Gln Arg Ser Ser Arg Arg Asp Glu Lys His
340 345 350
Tyr Ile Glu Phe Trp Lys Gln Val Pro Pro Asn Glu Pro Tyr Arg Val
355 360 365
Ile Leu Gly Asp Val Arg Asp Lys Leu Tyr Gln Thr Arg Glu Arg Ala
370 375 380
Arg Gln Leu Leu Gly His Gly Tyr Ser Glu Ile Pro Glu Glu Ala Thr
385 390 395 400
Tyr Thr Asn Ile Glu Gln Phe Leu Glu Pro Leu Glu Leu Cys Tyr Arg
405 410 415
Ser Leu Cys Ala Cys Gly Asp Leu Ser Ile Ala Asp Gly Ser Leu Leu
420 425 430
Asp Phe Leu Arg Gln Val Ser Thr Phe Gly Leu Ser Leu Val Arg Leu
435 440 445
Asp Ile Arg Gln Glu Ser Asp Arg His Thr Asp Val Leu Asp Ala Ile
450 455 460
Thr Gln His Leu Glu Ile Gly Ser Tyr Arg Asp Trp Ser Glu Glu Arg
465 470 475 480
Arg Gln Glu Trp Leu Leu Ser Glu Leu Ser Gly Lys Arg Pro Leu Phe
485 490 495
Gly Pro Asp Leu Pro Lys Thr Glu Glu Ile Ala Asp Val Leu Asp Thr
500 505 510
Phe His Val Ile Ala Glu Leu Pro Ala Asp Cys Phe Gly Ala Tyr Ile
515 520 525

```
Ile Ser Met Ala Thr Ala Pro Ser Asp Val Leu Ala Val Glu Leu Leu
    530                 535                 540
Gln Arg Glu Cys Arg Val Arg Gln Pro Leu Arg Val Val Pro Leu Phe
545                 550                 555                 560
Glu Lys Leu Ala Asp Leu Asp Ala Ala Pro Ala Ala Val Ala Arg Leu
                565                 570                 575
Phe Ser Ile Glu Trp Tyr Arg Asn Arg Ile Asn Gly Lys Gln Glu Val
            580                 585                 590
Met Ile Gly Tyr Ser Asp Ser Gly Lys Asp Ala Gly Arg Leu Ser Ala
        595                 600                 605
Ala Trp Gln Leu Tyr Lys Ala Gln Glu Glu Leu Ile Gln Val Ala Lys
    610                 615                 620
Glu Phe Asp Val Lys Leu Thr Met Phe His Gly Arg Gly Gly Thr Val
625                 630                 635                 640
Gly Arg Gly Gly Gly Pro Ala His Leu Ala Ile Leu Ser Gln Pro Pro
                645                 650                 655
Glu Thr Ile His Gly Ser Leu Arg Val Thr Val Gln Gly Glu Val Ile
            660                 665                 670
Glu Gln Ser Phe Gly Glu Glu His Leu Cys Phe Arg Thr Leu Gln Arg
        675                 680                 685
Phe Thr Ala Ala Thr Leu Glu His Gly Met His Pro Pro Val Ser Pro
    690                 695                 700
Lys Pro Glu Trp Arg Ala Leu Met Asp Glu Ile Ala Val Val Ala Thr
705                 710                 715                 720
Glu Lys Tyr Arg Ser Ile Val Phe Lys Glu Pro Arg Phe Val Glu Tyr
                725                 730                 735
Phe Arg Leu Ala Thr Pro Glu Leu Glu Tyr Gly Arg Met Asn Ile Gly
            740                 745                 750
Ser Arg Pro Ser Lys Arg Lys Pro Ser Gly Gly Ile Glu Ser Leu Arg
        755                 760                 765
Ala Ile Pro Trp Ile Phe Ala Trp Thr Gln Thr Arg Phe His Leu Pro
    770                 775                 780
Val Trp Leu Gly Phe Gly Ala Ala Phe Lys Tyr Ala Ile Glu Lys Asp
785                 790                 795                 800
Ile Lys Asn Leu Arg Met Leu Gln Glu Met Tyr Asn Ala Trp Pro Phe
                805                 810                 815
Phe Arg Val Thr Ile Asp Leu Val Glu Met Val Phe Ala Lys Gly Asp
            820                 825                 830
Pro Gly Ile Ala Ala Leu Phe Asp Lys Leu Leu Val Ser Glu Asp Leu
        835                 840                 845
Trp Ser Phe Gly Glu Leu Leu Arg Ser Lys Tyr Glu Glu Thr Lys Ser
    850                 855                 860
Leu Leu Leu Gln Ile Ala Gly His Lys Asp Leu Leu Glu Gly Asp Pro
865                 870                 875                 880
Tyr Leu Lys Gln Arg Leu Arg Leu Arg Asp Ser Tyr Ile Thr Thr Leu
                885                 890                 895
Asn Val Cys Gln Ala Tyr Thr Leu Lys Arg Ile Arg Asp Pro Asp Tyr
            900                 905                 910
Ser Val Thr Pro Arg Pro His Ile Ser Lys Glu Tyr Met Glu Ala Lys
        915                 920                 925
Pro Ala Thr Glu Leu Val Asn Leu Asn Pro Thr Ser Glu Tyr Ala Pro
    930                 935                 940
Gly Leu Glu Asp Thr Leu Ile Leu Thr Met Lys Gly Ile Ala Ala Gly
945                 950                 955                 960
Met Gln Asn Thr Gly
                965
```

<210> 47
<211> 3317
<212> DNA
<213> Medicago sativa

<400> 47

```
aaacactgtc cttctgatcc atttttccat tccttgtcat catcttcttc ttcgtatcta      60
actaactgtc tggcacacaa cacggtgaga gagtgaattg ctacaatggc aaacaagatg     120
gaaaaaatgg catcaattga tgcacagctt agacaattgg ttcctgcaaa agtgagtgaa     180
gatgataaac ttattgagta tgatgctttg ttgttggatc ggtttcttga tattcttcaa     240
gatttacatg gagaggatct gaaggattct gttcaagaag tgtatgaact gtctgctgaa     300
tatgaaagaa agcatgatcc taagaaactt gaagagcttg gaaatttgat cacaagtttc     360
gatgcaggtg actcaattgt tgttgccaag tccttttcac acatgcttaa cttggccaac     420
ttagctgaag aggttcaaat tgcgcaccgc cgaaggaaca agttgaagaa aggtgatttt     480
agggatgaga gcaatgcaac cactgaatct gacattgagg aaactctcaa gaaacttgtg     540
tttgacatga agaaatctcc tcaagaggtt tttgatgcat tgaagaacca gactgttgat     600
cttgttctta ctgctcatcc tactcagtcg gttcgtcgat ctttgcttca aaagcacgga     660
```

```
agggtaagga actgtttatc tcaattgtat gctaaagaca tcactcctga tgataagcag     720
gagcttgatg aagctctcca gagggagatt caagctgcat tccgtactga cgaaatcaag     780
aggactccac caactcccca agatgaaatg agagctggga tgagttactt ccatgaaaca     840
atttggaagg gtgtccctaa atttcttcgc cgtgttgata cggcattgaa gaacataggg     900
attaacgaac gtgttcccta taatgctcct cttattcaat tttcttcttg gatgggtggt     960
gatcgtgacg gtaatccaag agtgactcct gaagtgacaa gggatgtttg cttactagct    1020
agaatgatgg ctgctaactt gtattattca cagatagaag atcttatgtt tgaactttct    1080
atgtggcgtt gcaatgacga gctacgtgtt cgcgcagaag aacttcacag gaattccaag    1140
aaagatgaag ttgcaaaaca ctatatagag ttttggaaaa aaattccttt gaatgaacca    1200
taccgtgttg tactcgggga ggtaagggac aagctctatc gcactcgtga gcgttctcgt    1260
tatctcctag ctcatggcta ctgtgaaatt cctgaagaag ccacattcac caatgtcgat    1320
gagtttctgg aacctcttga actctgctac agatcactct gtgcttgtgg tgatcgtgca    1380
attgctgatg gaagccttct tgatttcttg aggcaagttt ccacttttgg actgtcactt    1440
gtaaggcttg atatacggca agagtctgat cgtcacactg acgtgatgga tgccattacc    1500
aaacatttgg aaattggatc ctaccaagaa tggtctgaag aaaaaagaca ggaatggctt    1560
ttgtccgagt tgattggcaa aaggccactc tttggacctg acctacccca aaccgatgaa    1620
attagagatg ttttagacac gttccgtgtc atagcagaac ttccatctga caactttgga    1680
gcctacatca tttcgatggc aactgcaccg tctgatgtgc tggcagttga gcttcttcaa    1740
cgtgaatgca aagtcaggaa tccattaaga gtcgttccgt tgtttgaaaa gcttgatgat    1800
cttgagtctg ctcctgctgc attggctcgg ttgttctcca tagactggta cattaaccgg    1860
atcgatggga agcaagaagt tatgattgga tattctgatt caggaaaaga tgctggaagg    1920
ttttctgcag catggcagct atataaggct caggaggacc tcatcaaagt cgcacagaaa    1980
tttggtgtta agctaaccat gttccacggt cgtggtggaa ctgttggaag aggaggtgga    2040
cctacccatc ttgctatctt gtctcaacca ccagaaacaa ttcacggatc tcttcgtgtg    2100
acagttcaag gtgaagttat tgaacagtcg ttcggtgagg aacacttgtg ctttaggaca    2160
ctgcaacgtt tcactgctgc tactctagaa catggaatgc gtcccccaag ctctccaaaa    2220
ccagaatggc gcgccttgat ggatcagatg gctgtcattg caactgagga ataccgttca    2280
attgtgttca aggaaccacg ttttgttgag tatttccgtc tggctacacc agagatggag    2340
tatggtagga tgaacattgg aagtcgaccg gcaaagagaa ggcctagtgg aggcattgaa    2400
acactgcgtg cgataccatg gatctttgcc tggacacaga caaggtttca tcttccagta    2460
tggctgggct ttggagcagc atttagacaa gttgttcaga aggatgttaa gaatctccat    2520
atgctgcaag agatgtacaa tcaatggcct ttctttaggg ttacaattga tttagttgaa    2580
atggtgtttg ccaagggtga ccctggtatt gcagcactga atgataggct cctagtttca    2640
aaggatctgt ggccatttgg ggaacaattg agaagcaaat atgaagaaac taagaaactc    2700
ctacttcagg tggctgcaca caaggaagtt cttgaaggtg acccctactt gaagcaaaga    2760
ctcagactcc gtgattcgta cattacaacc cttaatgttt tccaagccta cacattgaaa    2820
cggatccgcg atccaaacta caaggtggag gtgcgccccc caatatcgaa agagtctgct    2880
gaaacaagta aaccagctga tgaacttgta acattgaatc caacaagtga atatgctcct    2940
ggtttggaag acacactcat tcttaccatg aagggtattg ctgctggcat gcagaacact    3000
ggttaaattt tggttacatt tttcacttgt atttgtttct ttatgttaag tgtgtactaa    3060
gatttcataa atactagatg aatctagttg caaacaagca cttcaagtga gtgcttttt     3120
ctttttcttt ttcttttcat aagaatttca catcaggttt tgttggtgtg cttccttact    3180
ttgctgccac acaaatgagt tatgcaattg atgttatgtt tcaaggcata aattttgttg    3240
agtgctgcta ctatacgctt tcttgttcaa tttaatatga gactgaaaaa catagaaaat    3300
aggaacaatt ataataa                                                   3317

<210>  48
<211>  966
<212>  PRT
<213>  Medicago sativa

<400>  48
Met Ala Asn Lys Met Glu Lys Met Ala Ser Ile Asp Ala Gln Leu Arg
1               5                   10                  15
Gln Leu Val Pro Ala Lys Val Ser Glu Asp Asp Lys Leu Ile Glu Tyr
            20                  25                  30
Asp Ala Leu Leu Leu Asp Arg Phe Leu Asp Ile Leu Gln Asp Leu His
        35                  40                  45
Gly Glu Asp Leu Lys Asp Ser Val Gln Glu Val Tyr Glu Leu Ser Ala
    50                  55                  60
Glu Tyr Glu Arg Lys His Asp Pro Lys Lys Leu Glu Glu Leu Gly Asn
65                  70                  75                  80
Leu Ile Thr Ser Phe Asp Ala Gly Asp Ser Ile Val Val Ala Lys Ser
                85                  90                  95
Phe Ser His Met Leu Asn Leu Ala Asn Leu Ala Glu Glu Val Gln Ile
            100                 105                 110
Ala His Arg Arg Arg Asn Lys Leu Lys Lys Gly Asp Phe Arg Asp Glu
        115                 120                 125
Ser Asn Ala Thr Thr Glu Ser Asp Ile Glu Glu Thr Leu Lys Lys Leu
    130                 135                 140
Val Phe Asp Met Lys Lys Ser Pro Gln Glu Val Phe Asp Ala Leu Lys
145                 150                 155                 160
Asn Gln Thr Val Asp Leu Val Leu Thr Ala His Pro Thr Gln Ser Val
                165                 170                 175
```

```
Arg Arg Ser Leu Leu Gln Lys His Gly Arg Val Arg Asn Cys Leu Ser
            180                 185                 190
Gln Leu Tyr Ala Lys Asp Ile Thr Pro Asp Asp Lys Gln Glu Leu Asp
        195                 200                 205
Glu Ala Leu Gln Arg Glu Ile Gln Ala Ala Phe Arg Thr Asp Glu Ile
    210                 215                 220
Lys Arg Thr Pro Pro Thr Pro Gln Asp Glu Met Arg Ala Gly Met Ser
225                 230                 235                 240
Tyr Phe His Glu Thr Ile Trp Lys Gly Val Pro Lys Phe Leu Arg Arg
                245                 250                 255
Val Asp Thr Ala Leu Lys Asn Ile Gly Ile Asn Glu Arg Val Pro Tyr
            260                 265                 270
Asn Ala Pro Leu Ile Gln Phe Ser Ser Trp Met Gly Gly Asp Arg Asp
        275                 280                 285
Gly Asn Pro Arg Val Thr Pro Glu Val Thr Arg Asp Val Cys Leu Leu
    290                 295                 300
Ala Arg Met Met Ala Ala Asn Leu Tyr Tyr Ser Gln Ile Glu Asp Leu
305                 310                 315                 320
Met Phe Glu Leu Ser Met Trp Arg Cys Asn Asp Glu Leu Arg Val Arg
                325                 330                 335
Ala Glu Glu Leu His Arg Asn Ser Lys Lys Asp Glu Val Ala Lys His
            340                 345                 350
Tyr Ile Glu Phe Trp Lys Lys Ile Pro Leu Asn Glu Pro Tyr Arg Val
        355                 360                 365
Val Leu Gly Glu Val Arg Asp Lys Leu Tyr Arg Thr Arg Glu Arg Ser
    370                 375                 380
Arg Tyr Leu Leu Ala His Gly Tyr Cys Glu Ile Pro Glu Glu Ala Thr
385                 390                 395                 400
Phe Thr Asn Val Asp Glu Phe Leu Glu Pro Leu Glu Leu Cys Tyr Arg
                405                 410                 415
Ser Leu Cys Ala Cys Gly Asp Arg Ala Ile Ala Asp Gly Ser Leu Leu
            420                 425                 430
Asp Phe Leu Arg Gln Val Ser Thr Phe Gly Leu Ser Leu Val Arg Leu
        435                 440                 445
Asp Ile Arg Gln Glu Ser Asp Arg His Thr Asp Val Met Asp Ala Ile
    450                 455                 460
Thr Lys His Leu Glu Ile Gly Ser Tyr Gln Glu Trp Ser Glu Glu Lys
465                 470                 475                 480
Arg Gln Glu Trp Leu Leu Ser Glu Leu Ile Gly Lys Arg Pro Leu Phe
                485                 490                 495
Gly Pro Asp Leu Pro Gln Thr Asp Glu Ile Arg Asp Val Leu Asp Thr
            500                 505                 510
Phe Arg Val Ile Ala Glu Leu Pro Ser Asp Asn Phe Gly Ala Tyr Ile
        515                 520                 525
Ile Ser Met Ala Thr Ala Pro Ser Asp Val Leu Ala Val Glu Leu Leu
    530                 535                 540
Gln Arg Glu Cys Lys Val Arg Asn Pro Leu Arg Val Val Pro Leu Phe
545                 550                 555                 560
Glu Lys Leu Asp Asp Leu Glu Ser Ala Pro Ala Ala Leu Ala Arg Leu
                565                 570                 575
Phe Ser Ile Asp Trp Tyr Ile Asn Arg Ile Asp Gly Lys Gln Glu Val
            580                 585                 590
Met Ile Gly Tyr Ser Asp Ser Gly Lys Asp Ala Gly Arg Phe Ser Ala
        595                 600                 605
Ala Trp Gln Leu Tyr Lys Ala Gln Glu Asp Leu Ile Lys Val Ala Gln
    610                 615                 620
Lys Phe Gly Val Lys Leu Thr Met Phe His Gly Arg Gly Gly Thr Val
625                 630                 635                 640
Gly Arg Gly Gly Gly Pro Thr His Leu Ala Ile Leu Ser Gln Pro Pro
                645                 650                 655
Glu Thr Ile His Gly Ser Leu Arg Val Thr Val Gln Gly Glu Val Ile
            660                 665                 670
Glu Gln Ser Phe Gly Glu Glu His Leu Cys Phe Arg Thr Leu Gln Arg
        675                 680                 685
Phe Thr Ala Ala Thr Leu Glu His Gly Met Arg Pro Pro Ser Ser Pro
    690                 695                 700
Lys Pro Glu Trp Arg Ala Leu Met Asp Gln Met Ala Val Ile Ala Thr
705                 710                 715                 720
Glu Glu Tyr Arg Ser Ile Val Phe Lys Glu Pro Arg Phe Val Glu Tyr
                725                 730                 735
Phe Arg Leu Ala Thr Pro Glu Met Glu Tyr Gly Arg Met Asn Ile Gly
            740                 745                 750
Ser Arg Pro Ala Lys Arg Arg Pro Ser Gly Gly Ile Glu Thr Leu Arg
        755                 760                 765
```

```
Ala Ile Pro Trp Ile Phe Ala Trp Thr Gln Thr Arg Phe His Leu Pro
    770                 775                 780
Val Trp Leu Gly Phe Gly Ala Ala Phe Arg Gln Val Val Gln Lys Asp
785                 790                 795                 800
Val Lys Asn Leu His Met Leu Gln Glu Met Tyr Asn Gln Trp Pro Phe
                805                 810                 815
Phe Arg Val Thr Ile Asp Leu Val Glu Met Val Phe Ala Lys Gly Asp
            820                 825                 830
Pro Gly Ile Ala Ala Leu Asn Asp Arg Leu Leu Val Ser Lys Asp Leu
        835                 840                 845
Trp Pro Phe Gly Glu Gln Leu Arg Ser Lys Tyr Glu Glu Thr Lys Lys
    850                 855                 860
Leu Leu Leu Gln Val Ala Ala His Lys Glu Val Leu Glu Gly Asp Pro
865                 870                 875                 880
Tyr Leu Lys Gln Arg Leu Arg Leu Arg Asp Ser Tyr Ile Thr Thr Leu
                885                 890                 895
Asn Val Phe Gln Ala Tyr Thr Leu Lys Arg Ile Arg Asp Pro Asn Tyr
            900                 905                 910
Lys Val Glu Val Arg Pro Pro Ile Ser Lys Glu Ser Ala Glu Thr Ser
        915                 920                 925
Lys Pro Ala Asp Glu Leu Val Thr Leu Asn Pro Thr Ser Glu Tyr Ala
    930                 935                 940
Pro Gly Leu Glu Asp Thr Leu Ile Leu Thr Met Lys Gly Ile Ala Ala
945                 950                 955                 960
Gly Met Gln Asn Thr Gly
                965

<210>  49
<211>  3213
<212>  DNA
<213>  Zea mays

<400>  49
gccctctcct ggctcctctc cttcctgctt ggtttttgct ttcagctctt gaaccgaatc      60
cactgccgcg gcgcttagca ttggggacta gggggccgac atggctgcct tagggccgaa     120
gatggagcgt ctgtcgtcga tcgacgcgca gctgcggatg ctggtgccgg ggaaggtttc     180
ggaggacgat aagctcatcg agtacgacgc tctcctcctc gatcggttcc tcgacatcct     240
tcaggacctc catggcgacg acctcaagga aatggttcaa gaatgctatg aggtagctgc     300
tgagtatgaa acaaaaacatg acttgcaaaa gcttgatgaa ctcgggaaga tgataacaag     360
cttggatcct ggggactcta tcgtgattgc taagtctctc tcgcacatgc ttaacttggc     420
caacttggct gaggaagtcc agatagccta caggaggaga atcaagctca agaagggaga     480
ctttgctgat gagaactcgg caatcacaga atctgacatt gaggaaacac ttaagaggct     540
tgttgttgac ctgaagaagt cacctgctga ggtatttgat gccctcaaga gccagactgt     600
tgatctggtt ttgactgcac atccaacaca gtctgtgagg aggtcactgc tccagaaaca     660
ctcgaggata cgcaactgtt tggttcaact ctactcaaaa gatatcactc cggatgataa     720
gcaggaactt gatgaggctc tgcaaagaga gatccaagct gcctttagaa ctgatgagat     780
ccgaagaaca cagcccactc cccaagatga aatgcgtgct ggtatgagct acttccacga     840
aacaatttgg aagggtgttc caaagttctt gcgccgggtt gatactgcat tgaagaacat     900
tgggattaac gagcgtgttc cttacaatgc acctcttatt caattctctt cttggatggg     960
gggagatcgt gatggaaatc caagagtgac accagaggtt accagggatg tctgcttgct    1020
tgccagaatg atggcatcaa acttgtactg ctctcagatt gaggatctca tgtttgagtt    1080
gtctatgtgg cgatgcagtg atgaactgcg catgcgagct gatgtgctgc atctctccac    1140
taagaaggat gccaaacatt acatagagtt ttggaagaag gttcctccaa atgagccata    1200
tcgggtgata ctgagtgatg tcagggataa actgtacaac actcgtgaac ggtcacgaga    1260
gcttttatcc agtgggcatt ctgatattcc tgaggaagct acattgacaa atgttgagca    1320
gctgttggag cccttggaac tatgttacag atcactctgt gcttgtggtg actctgtaat    1380
tgctgatgga acccttctgg atttcttgcg tcaagtgtcc acctttggac tctcccttgt    1440
gaggcttgac attaggcaag agtcagatag gcatactgat gtccttgatg ccatcactac    1500
atacctgggg ataggatctt accgtgaatg gactgaagaa cgccgccaag aatggttact    1560
gtctgaactt aatggcaagc gccctctgtt tggctcagac cttcccaaga ctgaggaaat    1620
ttctgatgtt ctagacacat tccatgttat tgctgagctt ccctctgaca attttggtgc    1680
gtatatcatt tccatggcga cagctccgtc ggacgtcctt gctgttgaac tcctccaacg    1740
tgagtgtcat gtgaaaacac cacttagagt agtcccgctg tttgagaagt tggccgatct    1800
tgaggctgcc ccggctgcat tggccagact gttctcaata gattggtaca gacagaggat    1860
caatggcaag caggaggtca tgattgggta ttcagactcg ggcaaagatg ctggtcgcct    1920
ctcagcagct tggcagctat acaaagctca ggaggagctc atcaaggttg ctaaggactt    1980
tggtgtgaag ttaacgatgt tccatggacg tggtgggact gttggaaggg gtggtggtcc    2040
cactcacctt gccatcttgt cccagccacc agacacgatc cacggatcac tcagggtcac    2100
tgtgcaaggt gaagtcattg aacagtcatt tggtgaggag cacttgtgct ttaggacact    2160
gcaacgtttc acggctgcta ccctggagca tggcatgcac ccaccaaatg caccaaagcc    2220
agaatggcga gcccttcttg atgagatggc agttgtggca actgaggaat atcggtccat    2280
tgtcttcaaa gagccacgct ttgtcgagta tttccgcctt gcaacccctg aaacagagta    2340
tggtaggatg aacataggaa gcaggccatc caagagaaag ccgagcggag gtatcgactc    2400
actccgagca atcccatgga tctttgcttg gacacaaacg cggttccacc tcccggtctg    2460
```

gctaggattt ggagccgcat tcaagaatgt cctccagaag gacatcagga acctccacat 2520
gctccaggaa atgtacaatg agtggccatt tttcagggtg actattgatc tggtggagat 2580
ggtgttcgcc aagggtaatc ctggcattgc cgcactgtat gacaaactcc tcgtttcaga 2640
ggaactgcat ccattgggtg agaagctgag ggccaactat gaggaaaccc agaagcttct 2700
acttcaggtt gctgggcaca gggatcttct ggaaggtgac ctctacctga agcagcggct 2760
ccgcctgcgt gatgcgtaca tcaccaccct gaacgtctgc caggcctaca ccctgaagcg 2820
gatccgtgac ccggactacc atgtcgcgct gcgcccccac ctctccaagg agatcatgga 2880
ctcgaccaag gctgcggcgg acgtggtgaa gctgaaccct ggcagcgaat acgcaccggg 2940
gctggaggac accctcatcc tgaccatgaa gggcatagca gccggcctcc agaacaccgg 3000
ttaaaccacg aagaggggat ctttttctc ctcctgatca ttggctctag gtccttcgta 3060
ttttcgcgga ttgtaacttc ggccacctct ctacaccgtg aggaaataat ggtggtttct 3120
gcaaaagtgg tggtaaataa aagggcgaca actatcttgt ttttcatagt aacttataac 3180
tttacgatgc gataatggaa catggcttgc gtt 3213

<210> 50
<211> 967
<212> PRT
<213> Zea mays

<400> 50
Met Ala Ala Leu Gly Pro Lys Met Glu Arg Leu Ser Ser Ile Asp Ala
1 5 10 15
Gln Leu Arg Met Leu Val Pro Gly Lys Val Ser Glu Asp Asp Lys Leu
20 25 30
Ile Glu Tyr Asp Ala Leu Leu Leu Asp Arg Phe Leu Asp Ile Leu Gln
35 40 45
Asp Leu His Gly Asp Asp Leu Lys Glu Met Val Gln Glu Cys Tyr Glu
50 55 60
Val Ala Ala Glu Tyr Glu Thr Lys His Asp Leu Gln Lys Leu Asp Glu
65 70 75 80
Leu Gly Lys Met Ile Thr Ser Leu Asp Pro Gly Asp Ser Ile Val Ile
85 90 95
Ala Lys Ser Leu Ser His Met Leu Asn Leu Ala Asn Leu Ala Glu Glu
100 105 110
Val Gln Ile Ala Tyr Arg Arg Arg Ile Lys Leu Lys Lys Gly Asp Phe
115 120 125
Ala Asp Glu Asn Ser Ala Ile Thr Glu Ser Asp Ile Glu Glu Thr Leu
130 135 140
Lys Arg Leu Val Val Asp Leu Lys Lys Ser Pro Ala Glu Val Phe Asp
145 150 155 160
Ala Leu Lys Ser Gln Thr Val Asp Leu Val Leu Thr Ala His Pro Thr
165 170 175
Gln Ser Val Arg Arg Ser Leu Leu Gln Lys His Ser Arg Ile Arg Asn
180 185 190
Cys Leu Val Gln Leu Tyr Ser Lys Asp Ile Thr Pro Asp Asp Lys Gln
195 200 205
Glu Leu Asp Glu Ala Leu Gln Arg Glu Ile Gln Ala Ala Phe Arg Thr
210 215 220
Asp Glu Ile Arg Arg Thr Gln Pro Thr Pro Gln Asp Glu Met Arg Ala
225 230 235 240
Gly Met Ser Tyr Phe His Glu Thr Ile Trp Lys Gly Val Pro Lys Phe
245 250 255
Leu Arg Arg Val Asp Thr Ala Leu Lys Asn Ile Gly Ile Asn Glu Arg
260 265 270
Val Pro Tyr Asn Ala Pro Leu Ile Gln Phe Ser Ser Trp Met Gly Gly
275 280 285
Asp Arg Asp Gly Asn Pro Arg Val Thr Pro Glu Val Thr Arg Asp Val
290 295 300
Cys Leu Leu Ala Arg Met Met Ala Ser Asn Leu Tyr Cys Ser Gln Ile
305 310 315 320
Glu Asp Leu Met Phe Glu Leu Ser Met Trp Arg Cys Ser Asp Glu Leu
325 330 335
Arg Met Arg Ala Asp Val Leu His Leu Ser Thr Lys Lys Asp Ala Lys
340 345 350
His Tyr Ile Glu Phe Trp Lys Lys Val Pro Pro Asn Glu Pro Tyr Arg
355 360 365
Val Ile Leu Ser Asp Val Arg Asp Lys Leu Tyr Asn Thr Arg Glu Arg
370 375 380
Ser Arg Glu Leu Leu Ser Ser Gly His Ser Asp Ile Pro Glu Glu Ala
385 390 395 400
Thr Leu Thr Asn Val Glu Gln Leu Leu Glu Pro Leu Glu Leu Cys Tyr
405 410 415
Arg Ser Leu Cys Ala Cys Gly Asp Ser Val Ile Ala Asp Gly Thr Leu
420 425 430

Leu Asp Phe Leu Arg Gln Val Ser Thr Phe Gly Leu Ser Leu Val Arg
435 440 445
Leu Asp Ile Arg Gln Glu Ser Asp Arg His Thr Asp Val Leu Asp Ala
450 455 460
Ile Thr Thr Tyr Leu Gly Ile Gly Ser Tyr Arg Glu Trp Thr Glu Glu
465 470 475 480
Arg Arg Gln Glu Trp Leu Leu Ser Glu Leu Asn Gly Lys Arg Pro Leu
485 490 495
Phe Gly Ser Asp Leu Pro Lys Thr Glu Glu Ile Ser Asp Val Leu Asp
500 505 510
Thr Phe His Val Ile Ala Glu Leu Pro Ser Asp Asn Phe Gly Ala Tyr
515 520 525
Ile Ile Ser Met Ala Thr Ala Pro Ser Asp Val Leu Ala Val Glu Leu
530 535 540
Leu Gln Arg Glu Cys His Val Lys Thr Pro Leu Arg Val Val Pro Leu
545 550 555 560
Phe Glu Lys Leu Ala Asp Leu Glu Ala Ala Pro Ala Ala Leu Ala Arg
565 570 575
Leu Phe Ser Ile Asp Trp Tyr Arg Gln Arg Ile Asn Gly Lys Gln Glu
580 585 590
Val Met Ile Gly Tyr Ser Asp Ser Gly Lys Asp Ala Gly Arg Leu Ser
595 600 605
Ala Ala Trp Gln Leu Tyr Lys Ala Gln Glu Glu Leu Ile Lys Val Ala
610 615 620
Lys Asp Phe Gly Val Lys Leu Thr Met Phe His Gly Arg Gly Gly Thr
625 630 635 640
Val Gly Arg Gly Gly Gly Pro Thr His Leu Ala Ile Leu Ser Gln Pro
645 650 655
Pro Asp Thr Ile His Gly Ser Leu Arg Val Thr Val Gln Gly Glu Val
660 665 670
Ile Glu Gln Ser Phe Gly Glu Glu His Leu Cys Phe Arg Thr Leu Gln
675 680 685
Arg Phe Thr Ala Ala Thr Leu Glu His Gly Met His Pro Pro Asn Ala
690 695 700
Pro Lys Pro Glu Trp Arg Ala Leu Leu Asp Glu Met Ala Val Val Ala
705 710 715 720
Thr Glu Glu Tyr Arg Ser Ile Val Phe Lys Glu Pro Arg Phe Val Glu
725 730 735
Tyr Phe Arg Leu Ala Thr Pro Glu Thr Glu Tyr Gly Arg Met Asn Ile
740 745 750
Gly Ser Arg Pro Ser Lys Arg Lys Pro Ser Gly Gly Ile Asp Ser Leu
755 760 765
Arg Ala Ile Pro Trp Ile Phe Ala Trp Thr Gln Thr Arg Phe His Leu
770 775 780
Pro Val Trp Leu Gly Phe Gly Ala Ala Phe Lys Asn Val Leu Gln Lys
785 790 795 800
Asp Ile Arg Asn Leu His Met Leu Gln Glu Met Tyr Asn Glu Trp Pro
805 810 815
Phe Phe Arg Val Thr Ile Asp Leu Val Glu Met Val Phe Ala Lys Gly
820 825 830
Asn Pro Gly Ile Ala Ala Leu Tyr Asp Lys Leu Leu Val Ser Glu Glu
835 840 845
Leu His Pro Leu Gly Glu Lys Leu Arg Ala Asn Tyr Glu Glu Thr Gln
850 855 860
Lys Leu Leu Leu Gln Val Ala Gly His Arg Asp Leu Leu Glu Gly Asp
865 870 875 880
Leu Tyr Leu Lys Gln Arg Leu Arg Leu Arg Asp Ala Tyr Ile Thr Thr
885 890 895
Leu Asn Val Cys Gln Ala Tyr Thr Leu Lys Arg Ile Arg Asp Pro Asp
900 905 910
Tyr His Val Ala Leu Arg Pro His Leu Ser Lys Glu Ile Met Asp Ser
915 920 925
Thr Lys Ala Ala Ala Asp Val Val Lys Leu Asn Pro Gly Ser Glu Tyr
930 935 940
Ala Pro Gly Leu Glu Asp Thr Leu Ile Leu Thr Met Lys Gly Ile Ala
945 950 955 960
Ala Gly Leu Gln Asn Thr Gly
965

<210> 51
<211> 5845
<212> DNA
<213> Triticum aestivum

<400> 51
gcggcgcgag cactaatcga ctcactatag ggcgtcgact cgatcttgag ggctgaggac 60
ggtggaatca ggaccgtcca cgtggacatg tggatgctcc caccataatc cgtaaactaa 120
tcccagtccc gatcgaaatc aaatcacgaa cactaccact tggacacaaa ttaatttgat 180
ttacagagat ctctcgagtc ccggtgaatc ccggcggcac tcctgccatc cgccttctat 240
aaataggcag caactggagt cacgacctcc tcagtcccgg ccccaaccac atcctccccc 300
caatctagct ccctccctcc ctaccacgct ggtccctgcg aggcggcgcg gcgagcgcga 360
gcagtcgggt cggctccggt caaagcgagc cggcgatggc gttgtcggcg ccgggcggcg 420
gcagcgggaa gatcgagcgg ctgtcgtcga tcgacgcgca gctgcggctg ctcgtccccg 480
ccaaggtgtc cgaggacgac aagctcattg agtacgacgc gctcctcctc gaccgcttcc 540
tcgacgtcct gcagggcctc cacggcgacg atctcaggga gatggtaact ctttttttctt 600
cttgttttct ctctctcgca ctgttcgatc tcgcaccgga tccgagctgt cccatcaccc 660
atgtgctact gctgcgctgc tcccattgat tcctctgaac tttacatgtg gcgcttcagg 720
gttcgagtgg agttttcagc tcagtgttta cggagtgggg ttgactttgg gttaagtctc 780
gatgtcggta gatttgtgtg taatctggtg ttgttaagtg gtagtttgga cgattgccca 840
gctgctcagt ttttctgcca caaacaaatt ttttttcgca gctaatctat atcccattgt 900
ggtcttgatt ttgggagcac tgctttaatt agtcgaactg ggttactgat tgaatctcaa 960
atcgtgtcgg tcaagcatga cttcatccgt ggaatttgcc tcgctctatc attgttgtgt 1020
caactgattt tggcatataa gtgctggatc ttatgttggt catatacggg atactaaata 1080
actggcttag tagttgtgtc aactgaagta atatgttaac tgacttggat ctactgtaat 1140
tatgaaaaat agctaacagt gttcatctct tagtctacga atatactagt tgggttaaac 1200
tgtcgataca acggatagac gccttcttag gctaaccggc atggaagtgc tgataatatt 1260
tacatgtatg gaagtctcga gacattgtcc attgcttgta gaagaccgtt acatcttgtt 1320
tataccttac ctaacaaagc ctgagatatg gcgtttccag agaaccaaac taggggaaaa 1380
aaggttcttg aaatcgactt gccttgactt ttgttgactt ggtggctcac aacctgtttg 1440
gtaccttgct tgtcaattga ttaagaagac gtaggcatat aaacaatcag gttgctggtc 1500
ggcttgatta tctttttggt accttgcttg tcagttgatt aagaagacat cctatatctt 1560
atgttgattg atggacagcc ataattggat tattattttc actaaacagg ttcaagaatg 1620
ctatgaggtg gctgctgaat atgaaactaa gcatgacctg gaaaagcttg atgaactcgg 1680
agagatgata acaagcttgg atcctggtga ctctattgtg attgccaaag ctttttcaca 1740
catgcttaac ttggccaact tggctgagga agtgcagatc gcatacagga ggagagtcaa 1800
gctcaagaag ggagactttg ctgatgaaaa ttcagcaatc acagaatctg acatcgagga 1860
aactcttaag aggcttgttt ttgacatgaa gaagtcccct gctgaggttt ttgatgccct 1920
caagaatcag actgttgatc tggttttgac tgcacatcca acacaatctg taaggaggtc 1980
actgctccag aagcattcga ggttagttaa ccaaacattg tgatgatagt agtcatgata 2040
caatttctat atattaatcc tgattacaat gactttatat caggatccgg aactgtttgg 2100
ttcagttata ctcgaaggat atcactccag atgacaagca ggaacttgac gaggctctcc 2160
agagagaggt gcttactgct atccttatgt tgattttcat gctgtttcta aggcacattg 2220
ttaacatgac aatgcagtca cagtagcctt acaacatcag acagctgaga ttctttctta 2280
cacttaacct agaatcctcc tttttagatc caagctgcct ttagaactga tgagatccga 2340
agactcagcc cgactcccca agatcacatg cgtgctggta tgagcgactt tcatgagaca 2400
atatggaagg gtgttccaaa gtttttgcgc cgtgttgata cggcattgaa gaacattggg 2460
atcaatgagc gtgttcctta taatgctcct cttatccaat tctcttcttg gatgggtgga 2520
gatcgtgatg gtgagtggaa tctctgtccc tcgtgatact gtgcagcgta catctatgat 2580
gtatctacag tcactgcaag cttttattac cttggtggtg tctgttagat gctacatttt 2640
ctgtatctgg tcgtatcaag aactatggat aaagccaact gttttatgta tctaaacttc 2700
tagtaccttt acaaatacag gaaatccaag agtgacacca gaagttacaa gggacgtctg 2760
tttgcttgcc agaatgatgg cagctaattt gtattgtgca cagattgagg atctcatgtt 2820
tgaggtagaa attgattgat tacttttaac taagttcaca tatatttgat cattagaagt 2880
tggataaaac ttcaaactat ttcagttgtc tatgtggcga tgcaatgatg agctacgctc 2940
gcgagctgat gagctgcacc gctcttctaa gaaggatgct aagcattaca taggtacata 3000
acagtaacag ttttttttaaa tgtaaaatag taggtcagac ccctgccaga gctttaaaaa 3060
ggcagtaaca gcttctaggc atgaaatttc acataacata tgcctttttt ttgttctact 3120
tattaatttg tgatgttcat ctccgatata tagagttctg gaagaaggtt cctccgaatg 3180
agccatatcg ggtaatactg ggtgatgtta gggataatct ttacaacacg cgtgagcgat 3240
cacgtgagtt gttatccagt ggacattccg acatacctga ggaagctact ctgacaaatc 3300
ttgagcaggt tgagtaatgt ctacgaaata taattggaac tatacatttt ttcttcaatg 3360
gtactatgtt tggtagagaa atggtcttta atatatgtcg gtcttctctt gcagctgttg 3420
gagcccttgg agctctgtta caggtcactg tgtgcttgtg gtgaccgtgt tattgctgat 3480
ggaacccttc ttgatttctt gcgccaagtc tctacctttg gactttccct cgtgaagctt 3540
gacatcaggc aagagtctga caggcacact gatgctctgg atgcaatcac ctcatacctg 3600
ggaataggat cttatcgtga gtggtctgag gaacaccgtc aagaatggct gttgtctgaa 3660
ctcaatggga agcgcccatt atttggcgca gaccttccca tgacagagga agttgctgat 3720
gttatgggcg cgttccaggt tattgctgag ctgcccggtg acaattttgg agcatatgtc 3780
atctcaatgg caacatctcc ttcagatgtt cttgctgtgg agctcctcca acgtgagtgc 3840
catatcaaga caccacttag agttgtcccc ctgtttgaga agttggccga tctcgaggct 3900
gccccagcag cactggccag actcttctca atagattggt acagagagag gatcaatggc 3960
aagcaggagg tcatgattgg gtattcagac tcaggcaagg atgcaggccg tctctcagca 4020
gcttggcaga tgtacaaggc tcaggaggac ctcgtcaagg tcgctaagca atttggagtg 4080
aaattgacga tgttccatgg acgaggtggg actgttggaa ggggtggtgg ccccactcat 4140
cttgctatct tgtctcagcc accggacacg atcaatggat cactccgggt cactgttcag 4200
ggtgaagtta tcgagcagag ctttggggaa gaacacttgt gcttcaggac gctccagcgt 4260
ttcacagctg ctactcttga gcatgggatg cgtccaccca tttcaccaaa gccagagtgg 4320
cgagctcttc tcgatgagat ggctgtggtg gcaactgaag aatatcggtc aattgtcttc 4380

```
caagaaccac gcttcgtcga gtatttccgc cttgtaagtc cttgctctca ttgaccatca   4440
ggctaggaaa catattaact gcttgaatag acttttcaat atgttgcaaa cctgaacctg   4500
ccagtgagat ggaattcatg caatgattta cggtggaaac ctatatcatg aaaataattt   4560
caaaaaataa tgaattgtga tagcacttag ggtaccttct tttgcaactg taatttctat   4620
gacagtacaa accctcgcca atgttatatt ggcagctcag gttattgcag taaggtttaa   4680
acaaaacaaa aacaaacctt ggtgccattc aggttgaacc ttcagtcaaa taatttcttt   4740
ctgatatttg tgtttgacag gcaacaccgg agacagagta tggcaggatg aatataggga   4800
gcaggccatc caagagaaag ccaagtggtg gcattgaatc actccgtgca atcccatgga   4860
tcttcgcatg gacgcagaca cggttccacc tcccggtctg gctgggcttt ggtggtgcct   4920
tcaagcatat cctcaagaag gacatcagga acttccacat gctccaggag atgtacaacg   4980
agtggccatt cttcagggtc accatcgatc ttgttgagat ggtgttcgcc aagggtaatc   5040
ctggcattgc tgccttgtat gacaggctcc tagtttcaga ggggctacag cccctgggtg   5100
agaagctgag ggccaactat gaggagaccc agaagctgct tcttcaggtt tttgttacta   5160
aaacgcagaa ctcctgtact tcgttcaaca acactgccag cattgatctt ctaggccagt   5220
tgttaaaata ttgacaataa aactggattt tcttgatttc aggttgctgg gcacaaggat   5280
cttcttgaag gtgatcccta cctgaagcag cggctccgcc tccgtgacgc gtacatcacc   5340
accatgaacg tctgccaggc atacacattg aagaggatcc gtgacccaga ctaccatgtt   5400
gcgctgcgcc cccatctgtc caaggaggtg atggacacga gcaagccggc tgctgagctc   5460
gtgacgctga acccggcgag tgagtacgcg ccggggctgg aggataccct catcttgacc   5520
atgaagggca ttgctgccgg tctccagaac accggttagg gcaaggggag attgccggat   5580
tatctttttt ttgtaccttg atgagatatt ttccttttat cgagtgctgt gggccttgta   5640
tctcagcgga tgttgctgca tctgccccta tatccagtga ggaataatgg catttcgcaa   5700
gtagtataat atttataaat aaaggcaaac aatgataatt tctaatctgt ttgtcagtcg   5760
tacttggaac acttaaggac atgtaccatg gtgctatctt aggagtgcca tattggataa   5820
atgatgtggt ggagaaaaga gaatt                                         5845

<210>  52
<211>  972
<212>  PRT
<213>  Triticum aestivum

<400>  52
Met Ala Leu Ser Ala Pro Gly Gly Gly Ser Gly Lys Ile Glu Arg Leu
1               5                   10                  15
Ser Ser Ile Asp Ala Gln Leu Arg Leu Leu Val Pro Ala Lys Val Ser
            20                  25                  30
Glu Asp Asp Lys Leu Ile Glu Tyr Asp Ala Leu Leu Leu Asp Arg Phe
        35                  40                  45
Leu Asp Val Leu Gln Gly Leu His Gly Asp Asp Leu Arg Glu Met Val
    50                  55                  60
Gln Glu Cys Tyr Glu Val Ala Ala Glu Tyr Glu Thr Lys His Asp Leu
65                  70                  75                  80
Glu Lys Leu Asp Glu Leu Gly Glu Met Ile Thr Ser Leu Asp Pro Gly
                85                  90                  95
Asp Ser Ile Val Ile Ala Lys Ala Phe Ser His Met Leu Asn Leu Ala
            100                 105                 110
Asn Leu Ala Glu Glu Val Gln Ile Ala Tyr Arg Arg Arg Val Lys Leu
        115                 120                 125
Lys Lys Gly Asp Phe Ala Asp Glu Asn Ser Ala Ile Thr Glu Ser Asp
    130                 135                 140
Ile Glu Glu Thr Leu Lys Arg Leu Val Phe Asp Met Lys Lys Ser Pro
145                 150                 155                 160
Ala Glu Val Phe Asp Ala Leu Lys Asn Gln Thr Val Asp Leu Val Leu
                165                 170                 175
Thr Ala His Pro Thr Gln Ser Val Arg Arg Ser Leu Leu Gln Lys His
            180                 185                 190
Ser Arg Ile Arg Asn Cys Leu Val Gln Leu Tyr Ser Lys Asp Ile Thr
        195                 200                 205
Pro Asp Asp Lys Gln Glu Leu Asp Glu Ala Leu Gln Arg Glu Ile Gln
    210                 215                 220
Ala Ala Phe Arg Thr Asp Glu Ile Arg Arg Leu Ser Pro Thr Pro Gln
225                 230                 235                 240
Asp His Met Arg Ala Gly Met Ser Asp Phe His Glu Thr Ile Trp Lys
                245                 250                 255
Gly Val Pro Lys Phe Leu Arg Arg Val Asp Thr Ala Leu Lys Asn Ile
            260                 265                 270
Gly Ile Asn Glu Arg Val Pro Tyr Asn Ala Pro Leu Ile Gln Phe Ser
        275                 280                 285
Ser Trp Met Gly Gly Asp Arg Asp Gly Asn Pro Arg Val Thr Pro Glu
    290                 295                 300
Val Thr Arg Asp Val Cys Leu Leu Ala Arg Met Met Ala Ala Asn Leu
305                 310                 315                 320
Tyr Cys Ala Gln Ile Glu Asp Leu Met Phe Glu Leu Ser Met Trp Arg
                325                 330                 335
```

```
Cys Asn Asp Glu Leu Arg Ser Arg Ala Asp Glu Leu His Arg Ser Ser
            340                 345                 350
Lys Lys Asp Ala Lys His Tyr Ile Glu Phe Trp Lys Lys Val Pro Pro
        355                 360                 365
Asn Glu Pro Tyr Arg Val Ile Leu Gly Asp Val Arg Asp Asn Leu Tyr
    370                 375                 380
Asn Thr Arg Glu Arg Ser Arg Glu Leu Leu Ser Ser Gly His Ser Asp
385                 390                 395                 400
Ile Pro Glu Glu Ala Thr Leu Thr Asn Leu Glu Gln Leu Leu Glu Pro
                405                 410                 415
Leu Glu Leu Cys Tyr Arg Ser Leu Cys Ala Cys Gly Asp Arg Val Ile
            420                 425                 430
Ala Asp Gly Thr Leu Leu Asp Phe Leu Arg Gln Val Ser Thr Phe Gly
        435                 440                 445
Leu Ser Leu Val Lys Leu Asp Ile Arg Gln Glu Ser Asp Arg His Thr
    450                 455                 460
Asp Ala Leu Asp Ala Ile Thr Ser Tyr Leu Gly Ile Gly Ser Tyr Arg
465                 470                 475                 480
Glu Trp Ser Glu Glu His Arg Gln Glu Trp Leu Leu Ser Glu Leu Asn
                485                 490                 495
Gly Lys Arg Pro Leu Phe Gly Ala Asp Leu Pro Met Thr Glu Glu Val
            500                 505                 510
Ala Asp Val Met Gly Ala Phe Gln Val Ile Ala Glu Leu Pro Gly Asp
        515                 520                 525
Asn Phe Gly Ala Tyr Val Ile Ser Met Ala Thr Ser Pro Ser Asp Val
    530                 535                 540
Leu Ala Val Glu Leu Leu Gln Arg Glu Cys His Ile Lys Thr Pro Leu
545                 550                 555                 560
Arg Val Val Pro Leu Phe Glu Lys Leu Ala Asp Leu Glu Ala Ala Pro
                565                 570                 575
Ala Ala Leu Ala Arg Leu Phe Ser Ile Asp Trp Tyr Arg Glu Arg Ile
            580                 585                 590
Asn Gly Lys Gln Glu Val Met Ile Gly Tyr Ser Asp Ser Gly Lys Asp
        595                 600                 605
Ala Gly Arg Leu Ser Ala Ala Trp Gln Met Tyr Lys Ala Gln Glu Asp
    610                 615                 620
Leu Val Lys Val Ala Lys Gln Phe Gly Val Lys Leu Thr Met Phe His
625                 630                 635                 640
Gly Arg Gly Gly Thr Val Gly Arg Gly Gly Gly Pro Thr His Leu Ala
                645                 650                 655
Ile Leu Ser Gln Pro Pro Asp Thr Ile Asn Gly Ser Leu Arg Val Thr
            660                 665                 670
Val Gln Gly Glu Val Ile Glu Gln Ser Phe Gly Glu Glu His Leu Cys
        675                 680                 685
Phe Arg Thr Leu Gln Arg Phe Thr Ala Ala Thr Leu Glu His Gly Met
    690                 695                 700
Arg Pro Pro Ile Ser Pro Lys Pro Glu Trp Arg Ala Leu Leu Asp Glu
705                 710                 715                 720
Met Ala Val Val Ala Thr Glu Glu Tyr Arg Ser Ile Val Phe Gln Glu
                725                 730                 735
Pro Arg Phe Val Glu Tyr Phe Arg Leu Ala Thr Pro Glu Thr Glu Tyr
            740                 745                 750
Gly Arg Met Asn Ile Gly Ser Arg Pro Ser Lys Arg Lys Pro Ser Gly
        755                 760                 765
Gly Ile Glu Ser Leu Arg Ala Ile Pro Trp Ile Phe Ala Trp Thr Gln
    770                 775                 780
Thr Arg Phe His Leu Pro Val Trp Leu Gly Phe Gly Gly Ala Phe Lys
785                 790                 795                 800
His Ile Leu Lys Lys Asp Ile Arg Asn Phe His Met Leu Gln Glu Met
                805                 810                 815
Tyr Asn Glu Trp Pro Phe Phe Arg Val Thr Ile Asp Leu Val Glu Met
            820                 825                 830
Val Phe Ala Lys Gly Asn Pro Gly Ile Ala Ala Leu Tyr Asp Arg Leu
        835                 840                 845
Leu Val Ser Glu Gly Leu Gln Pro Leu Gly Glu Lys Leu Arg Ala Asn
    850                 855                 860
Tyr Glu Glu Thr Gln Lys Leu Leu Leu Gln Val Ala Gly His Lys Asp
865                 870                 875                 880
Leu Leu Glu Gly Asp Pro Tyr Leu Lys Gln Arg Leu Arg Leu Arg Asp
                885                 890                 895
Ala Tyr Ile Thr Thr Met Asn Val Cys Gln Ala Tyr Thr Leu Lys Arg
            900                 905                 910
Ile Arg Asp Pro Asp Tyr His Val Ala Leu Arg Pro His Leu Ser Lys
        915                 920                 925
```

Glu Val Met Asp Thr Ser Lys Pro Ala Ala Glu Leu Val Thr Leu Asn
930 935 940
Pro Ala Ser Glu Tyr Ala Pro Gly Leu Glu Asp Thr Leu Ile Leu Thr
945 950 955 960
Met Lys Gly Ile Ala Ala Gly Leu Gln Asn Thr Gly
965 970

<210> 53
<211> 3535
<212> DNA
<213> Oryza sativa

<400> 53
aggtggaaga gacggacaga ggccactacc cgtgcaccac gccccctctc ccacaatctc 60
gcaaaaaagt tgattttctt tttcagcttc ctcctcccct cctccgcttc tcatcaatcc 120
actagcatat cagatcagat tcctgtacac ttccctttcc tcgcatccaa tcccgactat 180
caaatcatca ccatcctttt gcaaaaccaa aaaaaaaaaa gtagataaaa gaagaaggtt 240
cttgcatcca agagaggagg acactgtccg tcgtgcagcg tttcttgctt ggttgaatcg 300
ggtggttttg ggggggatgg cggggaaggt ggagaagatg gcgtcgatcg acgcgcagct 360
gcggatgctg gcgccggcga agctgtcgga ggacgacaag ctggtggagt acgacgccct 420
cctcctcgac cgcttcctcg acatcctcca ggacctccac ggcgacgacc tccgcgagct 480
ggtccaagaa tgctatgaga tcgctgctga gtatgaaggg aagcatgact cccaaaagct 540
cgatgaactt ggcaacatgt tgacaagctt ggatcctggt gactccattg tgatggccaa 600
agctttctcc cacatgctta atttggctaa cttggccgag gaggtccaga ttgcatacag 660
gaggagaatc aagctcaaga agggtgactt tgctgatgag aactcggcac tgacagaatc 720
tgacattgaa gaaactttta agaggcttgt tgttgacctg aagaaatcac cagcagaggt 780
ctttgatgcc cttaagagtc agactgttga cctggttttg actgcacatc caacacagtc 840
agtgaggagg tcactgcttc aaaaacactc aaggataagg aattgtttgg ttcaactata 900
ctcaaaggat atcacaccag atgataaaca ggagcttgat gaggctctcc agagggagat 960
tcaagcagcc tttagaactg atgagatccg aagaacccag ccaactcctc aagatgaaat 1020
gagggctggt atgagttact ttcatgagac aatctggaaa ggtgttccga agttcttacg 1080
ccgtctggac actgccttga agaacatcgg aattgatgag cgtgttcctt ataatgcacc 1140
tctcattcag ttctcttctt ggatgggagg agaccgtgat ggaaatccaa gagtgacacc 1200
agaagtcaca agggatgtat gcttgcttgc tagaatgatg gcttcaaatt tatactgctc 1260
gcaaattgag gatctcatgt ttgagctgtc tatgtggcga tgcaatgatg agcttcgggc 1320
acgagcagat gagctgcatc tctcctcaaa gaaagatgca aagcactata tagaattctg 1380
gaagaaggtt ccgcccagtg agccataccg agttgttcta ggtgatgtga gagacaagct 1440
gtacaacaca cgtgaacgag cacgccagtt attatccagc ggatattctg acattcctga 1500
agaaaccact ctcaccagtg ttgaacagtt cttagagcct ctggagctat gttacaggtc 1560
actctgcgat tgcggtgacc gtgtaattgc tgatgggacc cttctggact tcttgcgcca 1620
ggtgtctacc tttgggctgt gccttgtgag gcttgacatt aggcaagaat ctgacaggca 1680
caccgatgtc ctcgatgcca tcaccacata cctgggaata ggatcatacc gtgagtggtc 1740
cgaggaacgc cgtcaggact ggctcttgtc tgaactcaat gggaagcgcc cattgtttgg 1800
cccagacctt cccaagaccg atgagattgc tgacgttcta gacacgttcc gcgtgatagc 1860
tgagcttccc gctgacaatt ttggggccta tatcatttcc atggcgacag ctccgtcaga 1920
cgtgcttgct gttgagctcc tccaacgtga atgccatgtg aagacaccac ttagagttgt 1980
cccactgttt gagaagttgg ctgatcttga atctgcccca gcagcagtgg ccagactgtt 2040
ctccatagat tggtacagag aaaggataaa tggcaaacag gaggtcatga ttggctattc 2100
ggactcgggt aaggacgccg gtcgtctctc ggcagcttgg cagctgtaca agtctcagga 2160
ggagctcatc aacgttgcca aggagtttgg ggtgaagttg acaatgttcc atgggcgcgg 2220
tgggactgtc ggaagaggcg gaggtcccac tcatcttgcc atcttgtctc agccaccaga 2280
cacaatccat ggatctctcc gggtcactgt ccagggtgaa gtcattgagc agtcctttgg 2340
tgaggagcac ctttgcttca ggacactgca gcgtttcacg gctgccactc tggagcatgg 2400
catgcatcca ccaattgcgc cgaaaccaga atggcgtgct ctgcttgatg agatggctgt 2460
ggtggctaca aaggaatacc ggtccatcgt cttccaggaa ccacgctttg tcgagtattt 2520
ccgccttgca acaccagaga tggagtatgg aaggatgaat ataggaagca ggccatccaa 2580
gagaaagccg agtggtggca tcgagtcact ccgtgccatc ccatggatct tcgcgtggac 2640
gcaaactcgc ttccaccttc ctgtctggct tggcttcggt tctgccttca agcacatcct 2700
ggagaaggac atcaggaacc tccacatgct ccaggagatg tacaatgagt ggccgttctt 2760
cagagtcacg atcgatctgg ttgagatggt tttcgccaag ggtgaccctg gcatcgccgc 2820
attgtacgac aagctccttg tctccgagga gctatggcct ctgggagaga aactgagggc 2880
caactgtgaa gaaaccaaac agcttctcct tcaggttgct ggacacaagg atcttctcga 2940
aggtgatctc tacctgaagc agcgccttcg cctccgcaat gcctacatca ccaccctgaa 3000
tgtctgccag gcttacacga tgaagcgaat ccgcgacccg gactaccatg tcacgctgcg 3060
cccccacatg tccaaggaga tcatggactg gtctaagccg gctgcggagc tggtgaagct 3120
gaacccgacc agtgagtatg cgccggggct ggaggacacc ctcatcctga ccatgaaggg 3180
aattgctgct ggaatgcaga acaccggcta agcgaactat caactatgaa gtgcctgaat 3240
tcacaccgtt ttatatggac tcttatcttg atttcggtca tttgctctcg gccatttaat 3300
tttgccgatg cagaggctcg tgcagtttct aatttctttc ggaagatatt ttcaataatg 3360
gagttgtaca agaacatcaa ttaatcaaaa taaagtggat attatgttca ttatcgttta 3420
gagcaacctg gtaaagccat ttttttgtgt agaccacata gtgagtgtgt tgaaattcca 3480
tttgcttaat gaaactgcca ttatctaagt aaagtcaaag ggctccttta tattg 3535

<210> 54

<211> 964
<212> PRT
<213> Oryza sativa

<400> 54
Met Ala Gly Lys Val Glu Lys Met Ala Ser Ile Asp Ala Gln Leu Arg
1 5 10 15
Met Leu Ala Pro Ala Lys Leu Ser Glu Asp Asp Lys Leu Val Glu Tyr
20 25 30
Asp Ala Leu Leu Leu Asp Arg Phe Leu Asp Ile Leu Gln Asp Leu His
35 40 45
Gly Asp Asp Leu Arg Glu Leu Val Gln Glu Cys Tyr Glu Ile Ala Ala
50 55 60
Glu Tyr Glu Gly Lys His Asp Ser Gln Lys Leu Asp Glu Leu Gly Asn
65 70 75 80
Met Leu Thr Ser Leu Asp Pro Gly Asp Ser Ile Val Met Ala Lys Ala
85 90 95
Phe Ser His Met Leu Asn Leu Ala Asn Leu Ala Glu Glu Val Gln Ile
100 105 110
Ala Tyr Arg Arg Arg Ile Lys Leu Lys Lys Gly Asp Phe Ala Asp Glu
115 120 125
Asn Ser Ala Leu Thr Glu Ser Asp Ile Glu Glu Thr Phe Lys Arg Leu
130 135 140
Val Val Asp Leu Lys Lys Ser Pro Ala Glu Val Phe Asp Ala Leu Lys
145 150 155 160
Ser Gln Thr Val Asp Leu Val Leu Thr Ala His Pro Thr Gln Ser Val
165 170 175
Arg Arg Ser Leu Leu Gln Lys His Ser Arg Ile Arg Asn Cys Leu Val
180 185 190
Gln Leu Tyr Ser Lys Asp Ile Thr Pro Asp Asp Lys Gln Glu Leu Asp
195 200 205
Glu Ala Leu Gln Arg Glu Ile Gln Ala Ala Phe Arg Thr Asp Glu Ile
210 215 220
Arg Arg Thr Gln Pro Thr Pro Gln Asp Glu Met Arg Ala Gly Met Ser
225 230 235 240
Tyr Phe His Glu Thr Ile Trp Lys Gly Val Pro Lys Phe Leu Arg Arg
245 250 255
Leu Asp Thr Ala Leu Lys Asn Ile Gly Ile Asp Glu Arg Val Pro Tyr
260 265 270
Asn Ala Pro Leu Ile Gln Phe Ser Ser Trp Met Gly Gly Asp Arg Asp
275 280 285
Gly Asn Pro Arg Val Thr Pro Glu Val Thr Arg Asp Val Cys Leu Leu
290 295 300
Ala Arg Met Met Ala Ser Asn Leu Tyr Cys Ser Gln Ile Glu Asp Leu
305 310 315 320
Met Phe Glu Leu Ser Met Trp Arg Cys Asn Asp Glu Leu Arg Ala Arg
325 330 335
Ala Asp Glu Leu His Leu Ser Ser Lys Lys Asp Ala Lys His Tyr Ile
340 345 350
Glu Phe Trp Lys Lys Val Pro Pro Ser Glu Pro Tyr Arg Val Val Leu
355 360 365
Gly Asp Val Arg Asp Lys Leu Tyr Asn Thr Arg Glu Arg Ala Arg Gln
370 375 380
Leu Leu Ser Ser Gly Tyr Ser Asp Ile Pro Glu Glu Thr Thr Leu Thr
385 390 395 400
Ser Val Glu Gln Phe Leu Glu Pro Leu Glu Leu Cys Tyr Arg Ser Leu
405 410 415
Cys Asp Cys Gly Asp Arg Val Ile Ala Asp Gly Thr Leu Leu Asp Phe
420 425 430
Leu Arg Gln Val Ser Thr Phe Gly Leu Cys Leu Val Arg Leu Asp Ile
435 440 445
Arg Gln Glu Ser Asp Arg His Thr Asp Val Leu Asp Ala Ile Thr Thr
450 455 460
Tyr Leu Gly Ile Gly Ser Tyr Arg Glu Trp Ser Glu Glu Arg Arg Gln
465 470 475 480
Asp Trp Leu Leu Ser Glu Leu Asn Gly Lys Arg Pro Leu Phe Gly Pro
485 490 495
Asp Leu Pro Lys Thr Asp Glu Ile Ala Asp Val Leu Asp Thr Phe Arg
500 505 510
Val Ile Ala Glu Leu Pro Ala Asp Asn Phe Gly Ala Tyr Ile Ile Ser
515 520 525
Met Ala Thr Ala Pro Ser Asp Val Leu Ala Val Glu Leu Leu Gln Arg
530 535 540
Glu Cys His Val Lys Thr Pro Leu Arg Val Val Pro Leu Phe Glu Lys

```
545                 550                 555                 560
Leu Ala Asp Leu Glu Ser Ala Pro Ala Ala Val Ala Arg Leu Phe Ser
                565                 570                 575
Ile Asp Trp Tyr Arg Glu Arg Ile Asn Gly Lys Gln Glu Val Met Ile
            580                 585                 590
Gly Tyr Ser Asp Ser Gly Lys Asp Ala Gly Arg Leu Ser Ala Ala Trp
        595                 600                 605
Gln Leu Tyr Lys Ser Gln Glu Glu Leu Ile Asn Val Ala Lys Glu Phe
    610                 615                 620
Gly Val Lys Leu Thr Met Phe His Gly Arg Gly Gly Thr Val Gly Arg
625                 630                 635                 640
Gly Gly Gly Pro Thr His Leu Ala Ile Leu Ser Gln Pro Pro Asp Thr
                645                 650                 655
Ile His Gly Ser Leu Arg Val Thr Val Gln Gly Glu Val Ile Glu Gln
            660                 665                 670
Ser Phe Gly Glu Glu His Leu Cys Phe Arg Thr Leu Gln Arg Phe Thr
        675                 680                 685
Ala Ala Thr Leu Glu His Gly Met His Pro Pro Ile Ala Pro Lys Pro
    690                 695                 700
Glu Trp Arg Ala Leu Leu Asp Glu Met Ala Val Val Ala Thr Lys Glu
705                 710                 715                 720
Tyr Arg Ser Ile Val Phe Gln Glu Pro Arg Phe Val Glu Tyr Phe Arg
                725                 730                 735
Leu Ala Thr Pro Glu Met Glu Tyr Gly Arg Met Asn Ile Gly Ser Arg
            740                 745                 750
Pro Ser Lys Arg Lys Pro Ser Gly Gly Ile Glu Ser Leu Arg Ala Ile
        755                 760                 765
Pro Trp Ile Phe Ala Trp Thr Gln Thr Arg Phe His Leu Pro Val Trp
    770                 775                 780
Leu Gly Phe Gly Ser Ala Phe Lys His Ile Leu Glu Lys Asp Ile Arg
785                 790                 795                 800
Asn Leu His Met Leu Gln Glu Met Tyr Asn Glu Trp Pro Phe Phe Arg
                805                 810                 815
Val Thr Ile Asp Leu Val Glu Met Val Phe Ala Lys Gly Asp Pro Gly
            820                 825                 830
Ile Ala Ala Leu Tyr Asp Lys Leu Leu Val Ser Glu Glu Leu Trp Pro
        835                 840                 845
Leu Gly Glu Lys Leu Arg Ala Asn Cys Glu Glu Thr Lys Gln Leu Leu
    850                 855                 860
Leu Gln Val Ala Gly His Lys Asp Leu Leu Glu Gly Asp Leu Tyr Leu
865                 870                 875                 880
Lys Gln Arg Leu Arg Leu Arg Asn Ala Tyr Ile Thr Thr Leu Asn Val
                885                 890                 895
Cys Gln Ala Tyr Thr Met Lys Arg Ile Arg Asp Pro Asp Tyr His Val
            900                 905                 910
Thr Leu Arg Pro His Met Ser Lys Glu Ile Met Asp Trp Ser Lys Pro
        915                 920                 925
Ala Ala Glu Leu Val Lys Leu Asn Pro Thr Ser Glu Tyr Ala Pro Gly
    930                 935                 940
Leu Glu Asp Thr Leu Ile Leu Thr Met Lys Gly Ile Ala Ala Gly Met
945                 950                 955                 960
Gln Asn Thr Gly


<210>  55
<211>  2928
<212>  DNA
<213>  Oryza sativa

<400>  55
atgtcggcga tgtcggcggc gggcggcggc ggcggcgtgg ggaaggtgga gcggctgtcg      60
tcgatcgacg cgcagctgcg gcagctggtg ccggcgaagc tgtcggagga cgacaagctc     120
atcgagtacg acgcgctcct cctcgaccga ttcctcgacg tcctccatgg cctccacggc     180
gacgacctca aggatctcgt tcaagaatgc tatgaggtgg ccgcggagta tgaaacaaag     240
cacgacgtgc aaaagctcga tgaactcggc aacatgataa caagcttgga tccgggcgac     300
tcgattgtta ttgcgaaagc cttttcgcac atgcttaact tggccaactt ggctgaagaa     360
gtccagatcg catacaggag gaggatcaag ctcaagaagg gtgatttcgc tgacgaaaac     420
tcggcaatga ctgaatccga cattgaggaa acactcaaga ggcttgtttt tgacctgaag     480
aaatcccctg ctgaggtctt tgatgccctc aagagtcaga ctgttgacct tgttttgact     540
gcacatccaa cacagtctgt gaggaggtca ctgctccaga aacactccag gattcggaac     600
tgtctggttc agctttattc aaaggatatc actccagatg acaagcaaga gcttgatgag     660
gctctccaga gagagatcca agctgccttt agaaccgatg agatccgaag aacacagccc     720
actccccaag atgaaatgcg tgctggcatg agctacttcc atgaaacaat atggaagggt     780
gttccaaagt tcttgcgccg tgtggatact gcattgaaga acatcggtat cgacgagcgt     840
```

```
gttccttaca atgctcctct tattcagttc tcgtcttgga tgggaggaga tcgtgatgga    900
aatccaagag tcacaccaga ggttacaagg gatgtctgct tgcttgcaag aatgatggca    960
gcaaacttgt attgctcaca aattgaggat ctcatgtttg agttatctat gtggcgatgt   1020
aacgaggagc tacgctcacg agctgatgat ctgcaccgct cttctaagaa ggacgcaaag   1080
cattacatag agttttggaa gaaggttcct ctgaatgaac cataccgggt gatactgagt   1140
gatgtgagag ataagcttta caatacacgt gaacgatcac gggagttgtt gtccagcgga   1200
tattgcgaca tccctgagga agcaactctg acaaatgttg agcagctatt ggagcccttg   1260
gagctatgct acaggtcgct gtgcgcctgt ggtgaccgtg ccatcgctga tggaagcctt   1320
cttgatttct tgcgccaagt ttccaccttt ggactctccc ttgtgaggct tgacattagg   1380
caagaatccg acagacacac cgatgtcctt gatgccatca cgacatacct gggcatagga   1440
tcttaccgtg agtggtccga ggaacgccgt caagagtggc tgttgtccga actcaacggg   1500
aagcgaccat tgtttggccc agaccttccc aggacagatg aggttgctga tgttctagac   1560
acattccatg tgattgccga gctccctgct gacagttttg gagcctatgt tatttccatg   1620
gcaacagccc cctcggatgt ccttgctgtt gagctcctcc aacgcgagtg ccatgtcaag   1680
acaccgctta gagttgtccc cctgttcgag aagttggctg atcttgagtc tgcccctgcg   1740
gcattgacca ggctcttctc aataagttgg tacaggcaga ggatcaatgg taagcaggag   1800
gtcatgattg ggtattcaga ttcaggcaag gatgctggcc gtctctcggc agcttggcag   1860
atgtacaagg ctcaggagca gctcgtcaag gtagcgaagg atttcggggt caagttgacg   1920
atgttccatg gacgaggtgg aaccgttggt aggggcggtg ggccgactca ccttgccatc   1980
ttgtctcagc ctccagacac gatcaacgga tcgctccgtg tcactgttca gggtgaagtc   2040
attgagcaga gctttggtga ggagcacttg tgtttcagga cactgcagcg tttcaccgct   2100
gctacacttg agcacggcat gcatccaccc agtgcaccga agccagaatg gcgagctctt   2160
cttgatgaga tggctgtggt ggcaaccaag gagtatcgat ccgtagtgtt ccaagagcca   2220
cgctttgtgg agtatttccg ccttgcaaca cctgagacag agtatggaag gatgaacata   2280
ggaagcaggc catccaagag gaagccgagt ggaggcattg aatcactccg tgcgatccca   2340
tggatcttcg catggacgca gacccgcttc caccttcccg tctggcttgg attcggtgct   2400
gcattcaagc acgcccttca gaaggacatc aggaatctcc acatgctcca ggagatgtac   2460
aacgagtggc cgttcttcag ggtcactctc gacctgattg agatggtttt cgccaagggt   2520
aatcctggca ttgctgccct ttacgacaaa ctcctcgttt ctgaggatct acagccactg   2580
ggtgagaagc tgagagccaa ctatgtagag acacaaaagc tgcttcttca ggttgctggc   2640
catagggatc ttcttgaagg tgacccctac ctgaagcagc ggctccgact ccgcgacgcc   2700
tacataacca ccctgaatgt ctgccaagcc tacacgctga aacggatccg cgacccggat   2760
taccacgtca cgctgcgccc tcacctgtcc aaggaggtga tggatgggag caagccggcc   2820
gcggagcttg tgaagctgaa cccgggtagc gagtacgcgc cagggctgga ggacaccctc   2880
atcctgacca tgaagggcat tgctgcaggg ttgcagaaca ctggttaa                2928
```

```
<210>  56
<211>  975
<212>  PRT
<213>  Oryza sativa

<400>  56
Met Ser Ala Met Ser Ala Ala Gly Gly Gly Gly Gly Val Gly Lys Val
1               5                   10                  15
Glu Arg Leu Ser Ser Ile Asp Ala Gln Leu Arg Gln Leu Val Pro Ala
            20                  25                  30
Lys Leu Ser Glu Asp Asp Lys Leu Ile Glu Tyr Asp Ala Leu Leu Leu
        35                  40                  45
Asp Arg Phe Leu Asp Val Leu His Gly Leu His Gly Asp Asp Leu Lys
    50                  55                  60
Asp Leu Val Gln Glu Cys Tyr Glu Val Ala Ala Glu Tyr Glu Thr Lys
65                  70                  75                  80
His Asp Val Gln Lys Leu Asp Glu Leu Gly Asn Met Ile Thr Ser Leu
                85                  90                  95
Asp Pro Gly Asp Ser Ile Val Ile Ala Lys Ala Phe Ser His Met Leu
            100                 105                 110
Asn Leu Ala Asn Leu Ala Glu Glu Val Gln Ile Ala Tyr Arg Arg Arg
        115                 120                 125
Ile Lys Leu Lys Lys Gly Asp Phe Ala Asp Glu Asn Ser Ala Met Thr
    130                 135                 140
Glu Ser Asp Ile Glu Glu Thr Leu Lys Arg Leu Val Phe Asp Leu Lys
145                 150                 155                 160
Lys Ser Pro Ala Glu Val Phe Asp Ala Leu Lys Ser Gln Thr Val Asp
                165                 170                 175
Leu Val Leu Thr Ala His Pro Thr Gln Ser Val Arg Arg Ser Leu Leu
            180                 185                 190
Gln Lys His Ser Arg Ile Arg Asn Cys Leu Val Gln Leu Tyr Ser Lys
        195                 200                 205
Asp Ile Thr Pro Asp Asp Lys Gln Glu Leu Asp Glu Ala Leu Gln Arg
    210                 215                 220
Glu Ile Gln Ala Ala Phe Arg Thr Asp Glu Ile Arg Arg Thr Gln Pro
225                 230                 235                 240
Thr Pro Gln Asp Glu Met Arg Ala Gly Met Ser Tyr Phe His Glu Thr
                245                 250                 255
```

```
Ile Trp Lys Gly Val Pro Lys Phe Leu Arg Arg Val Asp Thr Ala Leu
            260                 265                 270
Lys Asn Ile Gly Ile Asp Glu Arg Val Pro Tyr Asn Ala Pro Leu Ile
        275                 280                 285
Gln Phe Ser Ser Trp Met Gly Gly Asp Arg Asp Gly Asn Pro Arg Val
    290                 295                 300
Thr Pro Glu Val Thr Arg Asp Val Cys Leu Leu Ala Arg Met Met Ala
305                 310                 315                 320
Ala Asn Leu Tyr Cys Ser Gln Ile Glu Asp Leu Met Phe Glu Leu Ser
                325                 330                 335
Met Trp Arg Cys Asn Glu Glu Leu Arg Ser Arg Ala Asp Asp Leu His
            340                 345                 350
Arg Ser Ser Lys Lys Asp Ala Lys His Tyr Ile Glu Phe Trp Lys Lys
        355                 360                 365
Val Pro Leu Asn Glu Pro Tyr Arg Val Ile Leu Ser Asp Val Arg Asp
    370                 375                 380
Lys Leu Tyr Asn Thr Arg Glu Arg Ser Arg Glu Leu Leu Ser Ser Gly
385                 390                 395                 400
Tyr Cys Asp Ile Pro Glu Glu Ala Thr Leu Thr Asn Val Glu Gln Leu
                405                 410                 415
Leu Glu Pro Leu Glu Leu Cys Tyr Arg Ser Leu Cys Ala Cys Gly Asp
            420                 425                 430
Arg Ala Ile Ala Asp Gly Ser Leu Leu Asp Phe Leu Arg Gln Val Ser
        435                 440                 445
Thr Phe Gly Leu Ser Leu Val Arg Leu Asp Ile Arg Gln Glu Ser Asp
    450                 455                 460
Arg His Thr Asp Val Leu Asp Ala Ile Thr Thr Tyr Leu Gly Ile Gly
465                 470                 475                 480
Ser Tyr Arg Glu Trp Ser Glu Glu Arg Arg Gln Glu Trp Leu Leu Ser
                485                 490                 495
Glu Leu Asn Gly Lys Arg Pro Leu Phe Gly Pro Asp Leu Pro Arg Thr
            500                 505                 510
Asp Glu Val Ala Asp Val Leu Asp Thr Phe His Val Ile Ala Glu Leu
        515                 520                 525
Pro Ala Asp Ser Phe Gly Ala Tyr Val Ile Ser Met Ala Thr Ala Pro
    530                 535                 540
Ser Asp Val Leu Ala Val Glu Leu Leu Gln Arg Glu Cys His Val Lys
545                 550                 555                 560
Thr Pro Leu Arg Val Val Pro Leu Phe Glu Lys Leu Ala Asp Leu Glu
                565                 570                 575
Ser Ala Pro Ala Ala Leu Thr Arg Leu Phe Ser Ile Ser Trp Tyr Arg
            580                 585                 590
Gln Arg Ile Asn Gly Lys Gln Glu Val Met Ile Gly Tyr Ser Asp Ser
        595                 600                 605
Gly Lys Asp Ala Gly Arg Leu Ser Ala Ala Trp Gln Met Tyr Lys Ala
    610                 615                 620
Gln Glu Gln Leu Val Lys Val Ala Lys Asp Phe Gly Val Lys Leu Thr
625                 630                 635                 640
Met Phe His Gly Arg Gly Gly Thr Val Gly Arg Gly Gly Gly Pro Thr
                645                 650                 655
His Leu Ala Ile Leu Ser Gln Pro Pro Asp Thr Ile Asn Gly Ser Leu
            660                 665                 670
Arg Val Thr Val Gln Gly Glu Val Ile Glu Gln Ser Phe Gly Glu Glu
        675                 680                 685
His Leu Cys Phe Arg Thr Leu Gln Arg Phe Thr Ala Ala Thr Leu Glu
    690                 695                 700
His Gly Met His Pro Pro Ser Ala Pro Lys Pro Glu Trp Arg Ala Leu
705                 710                 715                 720
Leu Asp Glu Met Ala Val Val Ala Thr Lys Glu Tyr Arg Ser Val Val
                725                 730                 735
Phe Gln Glu Pro Arg Phe Val Glu Tyr Phe Arg Leu Ala Thr Pro Glu
            740                 745                 750
Thr Glu Tyr Gly Arg Met Asn Ile Gly Ser Arg Pro Ser Lys Arg Lys
        755                 760                 765
Pro Ser Gly Gly Ile Glu Ser Leu Arg Ala Ile Pro Trp Ile Phe Ala
    770                 775                 780
Trp Thr Gln Thr Arg Phe His Leu Pro Val Trp Leu Gly Phe Gly Ala
785                 790                 795                 800
Ala Phe Lys His Ala Leu Gln Lys Asp Ile Arg Asn Leu His Met Leu
                805                 810                 815
Gln Glu Met Tyr Asn Glu Trp Pro Phe Phe Arg Val Thr Leu Asp Leu
            820                 825                 830
Ile Glu Met Val Phe Ala Lys Gly Asn Pro Gly Ile Ala Ala Leu Tyr
        835                 840                 845
```

```
Asp Lys Leu Leu Val Ser Glu Asp Leu Gln Pro Leu Gly Glu Lys Leu
    850                 855                 860
Arg Ala Asn Tyr Val Glu Thr Gln Lys Leu Leu Leu Gln Val Ala Gly
865                 870                 875                 880
His Arg Asp Leu Leu Glu Gly Asp Pro Tyr Leu Lys Gln Arg Leu Arg
                885                 890                 895
Leu Arg Asp Ala Tyr Ile Thr Thr Leu Asn Val Cys Gln Ala Tyr Thr
            900                 905                 910
Leu Lys Arg Ile Arg Asp Pro Asp Tyr His Val Thr Leu Arg Pro His
        915                 920                 925
Leu Ser Lys Glu Val Met Asp Gly Ser Lys Pro Ala Ala Glu Leu Val
    930                 935                 940
Lys Leu Asn Pro Gly Ser Glu Tyr Ala Pro Gly Leu Glu Asp Thr Leu
945                 950                 955                 960
Ile Leu Thr Met Lys Gly Ile Ala Ala Gly Leu Gln Asn Thr Gly
                965                 970                 975
```

<210> 57
<211> 3307
<212> DNA
<213> Oryza sativa

<400> 57

```
cccacactct ctacccccaa acttgccctc caatctatct ccgccgccgc cgccgcctcc      60
gccgcgagag ggcgtcgccg agctgcgcgg gaggctcgcc tcccgcagca tctggccgga     120
ttcgtcgtag tcgaggcagc tgtaagcgag agagagggag atggccgcgg ccgccgggaa     180
ggcggcgatg gagcggcacc agtcgatcga cgcgcagctg cggctgctcg cgccggggaa     240
ggtatccgag gacgacaagc tcgtcgagta cgacgccctc ctcgtcgacc gcttcctcga     300
catcctccag gacctgcacg gccctcacct ccgcgaattc gtgcaggagt gctacgagct     360
gtcggcggag tacgagaacg acagggacga ggcgcggctc gacgagctgg ggaggaagct     420
caccagcctg ccgccggggg actccatcgt cgtctccagc tccttctcgc acatgctcaa     480
cctcgccaac ctcgccgagg aggtgcagat cgcgcaccgc cgccggatca agctcaagcg     540
cggggacttc gccgacgagg cctccgcgcc caccgagtcc gacatcgagg agacgctcaa     600
gcgcctcgtc acccagctcg gcaagtcgag ggaggaggtg ttcgatgcgc tcaagaacca     660
gaccgttgac ctcgtgttca ccgcgcatcc aacgcagtcc gtcaggaggt ccctgctcca     720
gaagcacggg aggatccgga attgcctgag gcagttatat gcgaaagaca tcactgctga     780
tgacaagcag gagcttgatg aggccctgca gagggagatt caagcagctt tcagaactga     840
tgaaatccgc aggactcctc ccactcctca ggatgaaatg cgcgccggga tgagttattt     900
tcatgaaact atatggaagg gtgtaccaaa gttcttgcgc cgcattgata ctgctctgaa     960
aaatattggg attaatgagc gtcttcctta caatgctcct ctcatccagt tctcatcctg    1020
gatgggtggt gaccgtgatg gaaacccaag agttacacca gaggttacaa gagatgtatg    1080
cttgttggca agaatgatgg ctgctaacct gtacttctct cagatagaag atctgatgtt    1140
tgagctctct atgtggcgct gcagtgatga acttcgaatt cgtgcagatg atttgcattg    1200
ctcctccaga aaagctgcaa agcactatat agaattctgg aagcaaattc ctccaaatga    1260
gccttatcgt gtcatacttg gtggtgtcag ggataaattg tactatacgc gtgaacgtac    1320
tcgccatcta ttgacaactg gagtttctga aattccagag gaggcaacgt ttactaatgt    1380
tgaagagttt ctggagccgc ttgagctgtg ctacagatca ttatgtgctt gtggtgacaa    1440
acctatagct gatggaagcc ttcttgattt cttgcgtcaa gtatcaactt ttgggctggc    1500
tcttgtaaaa cttgacataa ggcaggagtc tgatcgacac actgatgtcc ttgatgccat    1560
aactacatat cttgggattg gatcttacgc tgaatggtct gaggagaaac gccaggattg    1620
gcttttgtcc gaactaaggg gcaaacgtcc tttgtttggt cctgatcttc ctcagactga    1680
agaaattgct gatgttttag gaacatttca cgtccttgct gagctcccgg cagattgttt    1740
tggtgcttac atcatctcaa tggcaactgc accatctgat gtgcttgctg tggagcttct    1800
acagcgggag tgccatataa aacagcctct gcgagttgtt cccctatttg agaaacttgc    1860
agatcttgaa gcagctccag cagcggtggc acgactattt tcaatagact ggtacatgaa    1920
taggattaat ggcaagcagg aggtcatgat tggatactca gactctggca aggatgctgg    1980
tcgtctttct gcagcatggc aaatgtacaa agcacaagag gagcttgtca aggtggcaaa    2040
gcattatggt gtaaagttga caatgttcca tggaagaggt ggaacagttg gcagaggagg    2100
tggtcccagt catcttgcca tattatctca accaccagac acgatacatg gatcacttcg    2160
tgtaacagta cagggcgagg ttattgaaca ctcgtttgga gaggaacact tgtgctttag    2220
aactctgcag cgctttactg cagctactct tgagcatgga atgcatcctc caatttcccc    2280
caaaccagaa tggcgtgctc taatggatga gatggctgtt gtggcaacaa aggaatatcg    2340
atcaatcgtc ttcaaggagc cacggtttgt tgaatacttc cgatcggcaa cacctgagac    2400
agaatatggc aggatgaaca ttggtagccg accatcaaag agaaagccta gtggtggcat    2460
tgaatcgctc cgtgcaattc cttggatttt tgcttggaca caaactaggt ttcatcttcc    2520
tgtatggctt ggatttggtg gagcgttcaa acatataatg cagaaggata tcaggaacat    2580
ccatacactg aaagaaatgt acaatgagtg gccattcttc agggtcacct tggacttgct    2640
tgagatggtt ttcgctaagg gagatccagg aattgctgct ttatatgaca aattgcttgt    2700
ggcaggtgat ctgcaatcct ttggagagca gctgagaaac aactttgaag agacaaaaca    2760
gctactcctt caggttgccg gtcacaagga tattctggaa ggggatcctt acctgaagca    2820
gcgtctgcgg ctgcgtgagt cgtacatcac taccttgaac gtttgccaag cctacaccct    2880
gaagcggata agggacccta gcttcgaggt gatgtcgcag ccggccttgt cgaaggagtt    2940
cgtcgacagc aaccagcctg ccgagctggt ccaactgaac gctgcgagcg agtacgcacc    3000
tggcctggag gacaccctca tcctgacgat gaagggcatt gctgccggca tgcagaacac    3060
```

```
aggctagatt gtccacaaga atgcttccag caggtgcaat caatcaatca tcaataactc   3120
cttccatgat gagatggtga atatgtttaa ctagtacgtt cgccgatgct agtgaattct   3180
ggagactttt ttttgctcgc acgttttctt ttggttgtgt acttgatgta aatgtcaagg   3240
ggggacttat ttggcttttg cagcccgatc caaaataaaa tatggaccaa tcattctacg   3300
aaaaaaa                                                             3307

<210>  58
<211>  959
<212>  PRT
<213>  Oryza sativa

<400>  58
Met Glu Arg His Gln Ser Ile Asp Ala Gln Leu Arg Leu Leu Ala Pro
1               5                   10                  15
Gly Lys Val Ser Glu Asp Asp Lys Leu Val Glu Tyr Asp Ala Leu Leu
            20                  25                  30
Val Asp Arg Phe Leu Asp Ile Leu Gln Asp Leu His Gly Pro His Leu
        35                  40                  45
Arg Glu Phe Val Gln Glu Cys Tyr Glu Leu Ser Ala Glu Tyr Glu Asn
    50                  55                  60
Asp Arg Asp Glu Ala Arg Leu Asp Glu Leu Gly Arg Lys Leu Thr Ser
65                  70                  75                  80
Leu Pro Pro Gly Asp Ser Ile Val Val Ser Ser Ser Phe Ser His Met
                85                  90                  95
Leu Asn Leu Ala Asn Leu Ala Glu Glu Val Gln Ile Ala His Arg Arg
            100                 105                 110
Arg Ile Lys Leu Lys Arg Gly Asp Phe Ala Asp Glu Ala Ser Ala Pro
        115                 120                 125
Thr Glu Ser Asp Ile Glu Glu Thr Leu Lys Arg Leu Val Thr Gln Leu
    130                 135                 140
Gly Lys Ser Arg Glu Glu Val Phe Asp Ala Leu Lys Asn Gln Thr Val
145                 150                 155                 160
Asp Leu Val Phe Thr Ala His Pro Thr Gln Ser Val Arg Arg Ser Leu
                165                 170                 175
Leu Gln Lys His Gly Arg Ile Arg Asn Cys Leu Arg Gln Leu Tyr Ala
            180                 185                 190
Lys Asp Ile Thr Ala Asp Asp Lys Gln Glu Leu Asp Glu Ala Leu Gln
        195                 200                 205
Arg Glu Ile Gln Ala Ala Phe Arg Thr Asp Glu Ile Arg Arg Thr Pro
    210                 215                 220
Pro Thr Pro Gln Asp Glu Met Arg Ala Gly Met Ser Tyr Phe His Glu
225                 230                 235                 240
Thr Ile Trp Lys Gly Val Pro Lys Phe Leu Arg Arg Ile Asp Thr Ala
                245                 250                 255
Leu Lys Asn Ile Gly Ile Asn Glu Arg Leu Pro Tyr Asn Ala Pro Leu
            260                 265                 270
Ile Gln Phe Ser Ser Trp Met Gly Gly Asp Arg Asp Gly Asn Pro Arg
        275                 280                 285
Val Thr Pro Glu Val Thr Arg Asp Val Cys Leu Leu Ala Arg Met Met
    290                 295                 300
Ala Ala Asn Leu Tyr Phe Ser Gln Ile Glu Asp Leu Met Phe Glu Leu
305                 310                 315                 320
Ser Met Trp Arg Cys Ser Asp Glu Leu Arg Ile Arg Ala Asp Asp Leu
                325                 330                 335
His Cys Ser Ser Arg Lys Ala Ala Lys His Tyr Ile Glu Phe Trp Lys
            340                 345                 350
Gln Ile Pro Pro Asn Glu Pro Tyr Arg Val Ile Leu Gly Gly Val Arg
        355                 360                 365
Asp Lys Leu Tyr Tyr Thr Arg Glu Arg Thr Arg His Leu Leu Thr Thr
    370                 375                 380
Gly Val Ser Glu Ile Pro Glu Glu Ala Thr Phe Thr Asn Val Glu Glu
385                 390                 395                 400
Phe Leu Glu Pro Leu Glu Leu Cys Tyr Arg Ser Leu Cys Ala Cys Gly
                405                 410                 415
Asp Lys Pro Ile Ala Asp Gly Ser Leu Leu Asp Phe Leu Arg Gln Val
            420                 425                 430
Ser Thr Phe Gly Leu Ala Leu Val Lys Leu Asp Ile Arg Gln Glu Ser
        435                 440                 445
Asp Arg His Thr Asp Val Leu Asp Ala Ile Thr Thr Tyr Leu Gly Ile
    450                 455                 460
Gly Ser Tyr Ala Glu Trp Ser Glu Glu Lys Arg Gln Asp Trp Leu Leu
465                 470                 475                 480
Ser Glu Leu Arg Gly Lys Arg Pro Leu Phe Gly Pro Asp Leu Pro Gln
                485                 490                 495
```

```
Thr Glu Glu Ile Ala Asp Val Leu Gly Thr Phe His Val Leu Ala Glu
            500                 505                 510
Leu Pro Ala Asp Cys Phe Gly Ala Tyr Ile Ile Ser Met Ala Thr Ala
        515                 520                 525
Pro Ser Asp Val Leu Ala Val Glu Leu Leu Gln Arg Glu Cys His Ile
    530                 535                 540
Lys Gln Pro Leu Arg Val Val Pro Leu Phe Glu Lys Leu Ala Asp Leu
545                 550                 555                 560
Glu Ala Ala Pro Ala Ala Val Ala Arg Leu Phe Ser Ile Asp Trp Tyr
                565                 570                 575
Met Asn Arg Ile Asn Gly Lys Gln Glu Val Met Ile Gly Tyr Ser Asp
            580                 585                 590
Ser Gly Lys Asp Ala Gly Arg Leu Ser Ala Ala Trp Gln Met Tyr Lys
        595                 600                 605
Ala Gln Glu Glu Leu Val Lys Val Ala Lys His Tyr Gly Val Lys Leu
    610                 615                 620
Thr Met Phe His Gly Arg Gly Gly Thr Val Gly Arg Gly Gly Gly Pro
625                 630                 635                 640
Ser His Leu Ala Ile Leu Ser Gln Pro Pro Asp Thr Ile His Gly Ser
                645                 650                 655
Leu Arg Val Thr Val Gln Gly Glu Val Ile Glu His Ser Phe Gly Glu
            660                 665                 670
Glu His Leu Cys Phe Arg Thr Leu Gln Arg Phe Thr Ala Ala Thr Leu
        675                 680                 685
Glu His Gly Met His Pro Pro Ile Ser Pro Lys Pro Glu Trp Arg Ala
    690                 695                 700
Leu Met Asp Glu Met Ala Val Val Ala Thr Lys Glu Tyr Arg Ser Ile
705                 710                 715                 720
Val Phe Lys Glu Pro Arg Phe Val Glu Tyr Phe Arg Ser Ala Thr Pro
                725                 730                 735
Glu Thr Glu Tyr Gly Arg Met Asn Ile Gly Ser Arg Pro Ser Lys Arg
            740                 745                 750
Lys Pro Ser Gly Gly Ile Glu Ser Leu Arg Ala Ile Pro Trp Ile Phe
        755                 760                 765
Ala Trp Thr Gln Thr Arg Phe His Leu Pro Val Trp Leu Gly Phe Gly
    770                 775                 780
Gly Ala Phe Lys His Ile Met Gln Lys Asp Ile Arg Asn Ile His Thr
785                 790                 795                 800
Leu Lys Glu Met Tyr Asn Glu Trp Pro Phe Phe Arg Val Thr Leu Asp
                805                 810                 815
Leu Leu Glu Met Val Phe Ala Lys Gly Asp Pro Gly Ile Ala Ala Leu
            820                 825                 830
Tyr Asp Lys Leu Leu Val Ala Gly Asp Leu Gln Ser Phe Gly Glu Gln
        835                 840                 845
Leu Arg Asn Asn Phe Glu Glu Thr Lys Gln Leu Leu Leu Gln Val Ala
    850                 855                 860
Gly His Lys Asp Ile Leu Glu Gly Asp Pro Tyr Leu Lys Gln Arg Leu
865                 870                 875                 880
Arg Leu Arg Glu Ser Tyr Ile Thr Thr Leu Asn Val Cys Gln Ala Tyr
                885                 890                 895
Thr Leu Lys Arg Ile Arg Asp Pro Ser Phe Glu Val Met Ser Gln Pro
            900                 905                 910
Ala Leu Ser Lys Glu Phe Val Asp Ser Asn Gln Pro Ala Glu Leu Val
        915                 920                 925
Gln Leu Asn Ala Ala Ser Glu Tyr Ala Pro Gly Leu Glu Asp Thr Leu
    930                 935                 940
Ile Leu Thr Met Lys Gly Ile Ala Ala Gly Met Gln Asn Thr Gly
945                 950                 955
```

```
<210>  59
<211>  3808
<212>  DNA
<213>  Oryza sativa

<400>  59
acccacacac ccacactctc tacccccaaa cttgccctcc aatctatctc cgccgccgcc      60
gccgcctccg ccgcgagagg gcgtcgccga gctgcgcggg aggctcgcct cccgcagcat     120
ctggccggat tcgtcgtagt cgaggcagct gtaagcgaga gagagggaga tggccgcggc     180
cgccgggaag gcggcgatgg agcggcacca gtcgatcgac gcgcagctgc ggctgctcgc     240
gccggggaag gtatccgagg acgacaagct cgtcgagtac gacgccctcc tcgtcgaccg     300
cttcctcgac atcctccagg acctgcacgg ccctcacctc cgcgaattcg tgcaggagtg     360
ctacgagctg tcggcggagt acgagaacga cagggacgag gcgcggctcg acgagctggg     420
gaggaagctc accagcctgc cgccggggga ctccatcgtc gtctccagct ccttctcgca     480
catgctcaac ctcgccaacc tcgccgagga ggtgcagatc gcgcaccgcc gccggatcaa     540
```

```
gctcaagcgc ggggacttcg ccgacgaggc ctccgcgccc accgagtccg acatcgagga    600
gacgctcaag cgcctcgtca cccagctcgg caagtcgagg gaggaggtgt tcgatgcgct    660
caagaaccag accgttgacc tcgtgttcac cgcgcatcca acgcagtccg tcaggaggtc    720
cctgctccag aagcacggga ggatccggaa ttgcctgagg cagttatatg cgaaagacat    780
cactgctgat gacaagcagg agcttgatga ggccctgcag agggagattc aagcagcttt    840
cagaactgat gaaatccgca ggactcctcc cactcctcag gatgaaatgc gcgccgggat    900
gagttatttt catgaaacta tatggaaggg tgtaccaaag ttcttgcgcc gcattgatac    960
tgctctgaaa aatattggga ttaatgagcg tcttccttac aatgctcctc tcatccagtt   1020
ctcatcctgg atgggtggtg accgtgatgg aaacccaaga gttacaccag aggttacaag   1080
agatgtatgc ttgttggcaa gaatgatggc tgctaacctg tacttctctc agatagaaga   1140
tctgatgttt gagctctcta tgtggcgctg cagtgatgaa cttcgaattc gtgcagatga   1200
tttgcattgc tcctccagaa aagctgcaaa gcactatata gaattctgga agcaaattcc   1260
tccaaatgag ccttatcgtg tcatacttgg tggtgtcagg gataaattgt actatacgcg   1320
tgaacgtact cgccatctat tgacaactgg agtttctgaa attccagagg aggcaacgtt   1380
tactaatgtt gaagagtttc tggagccgct tgagctgtgc tacagatcat tatgtgcttg   1440
tggtgacaaa cctatagctg atggaagcct tcttgatttc ttgcgtcaag tatcaacttt   1500
tgggctggct cttgtaaaac ttgacataag gcaggagtct gatcgacaca ctgatgtcct   1560
tgatgccata actacatatc ttgggattgg atcttacgct gaatggtctg aggagaaacg   1620
ccaggattgg cttttgtccg aactaagggg caaacgtcct ttgtttggtc ctgatcttcc   1680
tcagactgaa gaaattgctg atgttttagg aacatttcac gtccttgctg agctcccggc   1740
agattgtttt ggtgcttaca tcatctcaat ggcaactgca ccatctgatg tgcttgctgt   1800
ggagcttcta cagcgggagt gccatataaa acagcctctg cgagttgttc cctatttgga   1860
gaaacttgca gatcttgaag cagctccagc agcggtggca cgactatttt caatagactg   1920
gtacatgaat aggattaatg gcaagcagga ggtcatgatt ggatactcag actctggcaa   1980
ggatgctggt cgtctttctg cagcatggca aatgtacaaa gcacaagagg agcttgtcaa   2040
ggtggcaaag cattatggtg taaagttgac aatgttccat ggaagaggtg gaacagttgg   2100
cagaggaggt ggtcccagtc atcttgccat attatctcaa ccaccagaca cgatacatgg   2160
atcacttcgt gtaacagtac aggcgaggt tattgaacac tcgtttggag aggaacactt   2220
gtgctttaga actctgcagc gctttactgc agctactctt gagcatggaa tgcatcctcc   2280
aatttccccc aaaccagaat ggcgtgctct aatggatgag atggctgttg tggcaacaaa   2340
ggaatatcga tcaatcgtct tcaaggagcc acggtttgtt gaatacttcc gatcggcaac   2400
acctgagaca gaatatggca ggatgaacat tggtagccga ccatcaaaga gaaagcctag   2460
tggtggcatt gaatcgctcc gtgcaattcc ttggattttt gcttggacac aaactaggtt   2520
tcatcttcct gtatggcttg gatttggtgg agcgttcaaa catataatgc agaaggatat   2580
caggaacatc catacactga aagaaatgta caatgagtgg ccattcttca gggtcacctt   2640
ggacttgctt gagatggttt tcgccaaggg agatccagga attgctgctt tatatgacaa   2700
attgcttgtg gcaggtgatc tgcaatcctt tggagagcag ctgagaaaca actttgaaga   2760
gacaaaacag ctactccttc aggttgccgg tcacaaggat attctggaag gggatcctta   2820
cctgaagcag cgtctgcggc tgcgtgagtc gtacatcact accttgaacg tttgccaagc   2880
ctacaccctg aagcggataa gggaccctag cttcgaggtg atgtcgcagc cggccttgtc   2940
gaaggagttc gtcgacagca accagcctgc cgagctggtc caactgaacg ctgcgagcga   3000
gtacgcacct ggcctggagg acaccctcat cctgacgatg aagggcattg ctgccggcat   3060
gcagaacaca ggctagattg tccacaagaa tgcttccagc aggtgcaatc aatcaatcat   3120
caataactcc ttccatgatg agatggtgaa tatgtttaac tacccgatcc aaaataaaat   3180
atggaccaat cattctacga agcttgattc tgaacgcttc gaatttgcat cagtggtgac   3240
agcggctgtg tttgcatctt taaacgacag actaaaagca ttgtgcaccg caagatgcag   3300
acttgacatg tgcttatagc agaagtatta catggagcac atctcagggt caggtatatt   3360
gcaatctcaa aaatcaggag atacacaatc agatggatat gaaatgagaa gcaaaattgt   3420
gtctgaagaa atgagaagca agaagacagt taaagggtct gatgggttac agtctgaaaa   3480
atcaggggat atgcaatcgg agagatatga aagtgacaaa attttgtcca gagaaataag   3540
aagcaagaaa acattgaaag gtcagttatc atgctcacct agcattatgc accataaaat   3600
gatcttggca gacaatataa tctagcataa aattatatac aacaaattag agattgtagc   3660
gttgataaat tttcaaggac tatatatcta tagttagtta cagtgagaaa taacaaaagt   3720
tacatatgta actacaatac atatagactg taactgggct aaaactgcaa gcatgcttga   3780
atttcttcaa aaaataaaga atttcttc                                      3808
```

<210> 60
<211> 968
<212> PRT
<213> Oryza sativa

<400> 60

```
Met Ala Ala Ala Ala Gly Lys Ala Ala Met Glu Arg His Gln Ser Ile
1               5                   10                  15
Asp Ala Gln Leu Arg Leu Leu Ala Pro Gly Lys Val Ser Glu Asp Asp
            20                  25                  30
Lys Leu Val Glu Tyr Asp Ala Leu Leu Val Asp Arg Phe Leu Asp Ile
        35                  40                  45
Leu Gln Asp Leu His Gly Pro His Leu Arg Glu Phe Val Gln Glu Cys
    50                  55                  60
Tyr Glu Leu Ser Ala Glu Tyr Glu Asn Asp Arg Asp Glu Ala Arg Leu
65                  70                  75                  80
Asp Glu Leu Gly Arg Lys Leu Thr Ser Leu Pro Pro Gly Asp Ser Ile
                85                  90                  95
```

```
Val Val Ser Ser Ser Phe Ser His Met Leu Asn Leu Ala Asn Leu Ala
            100                 105                 110
Glu Glu Val Gln Ile Ala His Arg Arg Arg Ile Lys Leu Lys Arg Gly
        115                 120                 125
Asp Phe Ala Asp Glu Ala Ser Ala Pro Thr Glu Ser Asp Ile Glu Glu
    130                 135                 140
Thr Leu Lys Arg Leu Val Thr Gln Leu Gly Lys Ser Arg Glu Glu Val
145                 150                 155                 160
Phe Asp Ala Leu Lys Asn Gln Thr Val Asp Leu Val Phe Thr Ala His
                165                 170                 175
Pro Thr Gln Ser Val Arg Arg Ser Leu Leu Gln Lys His Gly Arg Ile
            180                 185                 190
Arg Asn Cys Leu Arg Gln Leu Tyr Ala Lys Asp Ile Thr Ala Asp Asp
        195                 200                 205
Lys Gln Glu Leu Asp Glu Ala Leu Gln Arg Glu Ile Gln Ala Ala Phe
    210                 215                 220
Arg Thr Asp Glu Ile Arg Arg Thr Pro Pro Thr Pro Gln Asp Glu Met
225                 230                 235                 240
Arg Ala Gly Met Ser Tyr Phe His Glu Thr Ile Trp Lys Gly Val Pro
                245                 250                 255
Lys Phe Leu Arg Arg Ile Asp Thr Ala Leu Lys Asn Ile Gly Ile Asn
            260                 265                 270
Glu Arg Leu Pro Tyr Asn Ala Pro Leu Ile Gln Phe Ser Ser Trp Met
        275                 280                 285
Gly Gly Asp Arg Asp Gly Asn Pro Arg Val Thr Pro Glu Val Thr Arg
    290                 295                 300
Asp Val Cys Leu Leu Ala Arg Met Met Ala Ala Asn Leu Tyr Phe Ser
305                 310                 315                 320
Gln Ile Glu Asp Leu Met Phe Glu Leu Ser Met Trp Arg Cys Ser Asp
                325                 330                 335
Glu Leu Arg Ile Arg Ala Asp Asp Leu His Cys Ser Ser Arg Lys Ala
            340                 345                 350
Ala Lys His Tyr Ile Glu Phe Trp Lys Gln Ile Pro Pro Asn Glu Pro
        355                 360                 365
Tyr Arg Val Ile Leu Gly Gly Val Arg Asp Lys Leu Tyr Tyr Thr Arg
    370                 375                 380
Glu Arg Thr Arg His Leu Leu Thr Thr Gly Val Ser Glu Ile Pro Glu
385                 390                 395                 400
Glu Ala Thr Phe Thr Asn Val Glu Glu Phe Leu Glu Pro Leu Glu Leu
                405                 410                 415
Cys Tyr Arg Ser Leu Cys Ala Cys Gly Asp Lys Pro Ile Ala Asp Gly
            420                 425                 430
Ser Leu Leu Asp Phe Leu Arg Gln Val Ser Thr Phe Gly Leu Ala Leu
        435                 440                 445
Val Lys Leu Asp Ile Arg Gln Glu Ser Asp Arg His Thr Asp Val Leu
    450                 455                 460
Asp Ala Ile Thr Thr Tyr Leu Gly Ile Gly Ser Tyr Ala Glu Trp Ser
465                 470                 475                 480
Glu Glu Lys Arg Gln Asp Trp Leu Leu Ser Glu Leu Arg Gly Lys Arg
                485                 490                 495
Pro Leu Phe Gly Pro Asp Leu Pro Gln Thr Glu Glu Ile Ala Asp Val
            500                 505                 510
Leu Gly Thr Phe His Val Leu Ala Glu Leu Pro Ala Asp Cys Phe Gly
        515                 520                 525
Ala Tyr Ile Ile Ser Met Ala Thr Ala Pro Ser Asp Val Leu Ala Val
    530                 535                 540
Glu Leu Leu Gln Arg Glu Cys His Ile Lys Gln Pro Leu Arg Val Val
545                 550                 555                 560
Pro Leu Phe Glu Lys Leu Ala Asp Leu Glu Ala Ala Pro Ala Ala Val
                565                 570                 575
Ala Arg Leu Phe Ser Ile Asp Trp Tyr Met Asn Arg Ile Asn Gly Lys
            580                 585                 590
Gln Glu Val Met Ile Gly Tyr Ser Asp Ser Gly Lys Asp Ala Gly Arg
        595                 600                 605
Leu Ser Ala Ala Trp Gln Met Tyr Lys Ala Gln Glu Glu Leu Val Lys
    610                 615                 620
Val Ala Lys His Tyr Gly Val Lys Leu Thr Met Phe His Gly Arg Gly
625                 630                 635                 640
Gly Thr Val Gly Arg Gly Gly Gly Pro Ser His Leu Ala Ile Leu Ser
                645                 650                 655
Gln Pro Pro Asp Thr Ile His Gly Ser Leu Arg Val Thr Val Gln Gly
            660                 665                 670
Glu Val Ile Glu His Ser Phe Gly Glu Glu His Leu Cys Phe Arg Thr
        675                 680                 685
```

```
Leu Gln Arg Phe Thr Ala Ala Thr Leu Glu His Gly Met His Pro Pro
    690                 695                 700
Ile Ser Pro Lys Pro Glu Trp Arg Ala Leu Met Asp Glu Met Ala Val
705                 710                 715                 720
Val Ala Thr Lys Glu Tyr Arg Ser Ile Val Phe Lys Glu Pro Arg Phe
                725                 730                 735
Val Glu Tyr Phe Arg Ser Ala Thr Pro Glu Thr Glu Tyr Gly Arg Met
            740                 745                 750
Asn Ile Gly Ser Arg Pro Ser Lys Arg Lys Pro Ser Gly Gly Ile Glu
        755                 760                 765
Ser Leu Arg Ala Ile Pro Trp Ile Phe Ala Trp Thr Gln Thr Arg Phe
    770                 775                 780
His Leu Pro Val Trp Leu Gly Phe Gly Gly Ala Phe Lys His Ile Met
785                 790                 795                 800
Gln Lys Asp Ile Arg Asn Ile His Thr Leu Lys Glu Met Tyr Asn Glu
                805                 810                 815
Trp Pro Phe Phe Arg Val Thr Leu Asp Leu Leu Glu Met Val Phe Ala
            820                 825                 830
Lys Gly Asp Pro Gly Ile Ala Ala Leu Tyr Asp Lys Leu Leu Val Ala
        835                 840                 845
Gly Asp Leu Gln Ser Phe Gly Glu Gln Leu Arg Asn Asn Phe Glu Glu
    850                 855                 860
Thr Lys Gln Leu Leu Leu Gln Val Ala Gly His Lys Asp Ile Leu Glu
865                 870                 875                 880
Gly Asp Pro Tyr Leu Lys Gln Arg Leu Arg Leu Arg Glu Ser Tyr Ile
                885                 890                 895
Thr Thr Leu Asn Val Cys Gln Ala Tyr Thr Leu Lys Arg Ile Arg Asp
            900                 905                 910
Pro Ser Phe Glu Val Met Ser Gln Pro Ala Leu Ser Lys Glu Phe Val
        915                 920                 925
Asp Ser Asn Gln Pro Ala Glu Leu Val Gln Leu Asn Ala Ala Ser Glu
    930                 935                 940
Tyr Ala Pro Gly Leu Glu Asp Thr Leu Ile Leu Thr Met Lys Gly Ile
945                 950                 955                 960
Ala Ala Gly Met Gln Asn Thr Gly
                965
```

<210> 61
<211> 3055
<212> DNA
<213> Oryza sativa

<400> 61

```
gcattggccg ttcggatcgc ggtggcgctt cgtgaaggtg ggggagaagg gggaaaagga      60
gtcggctttc ggggagatgg cgcgcaatgc ggcggacaag gggacgtcca tcgacgcgca     120
gctgcggatc ctggcgccta agaagctctc ggaggacgac aagcttgtgg agtacgacgc     180
gctgctcctc gatcgcttcc tcgacatcct ccaggatctg catggggagg acatcaggga     240
gacggttcaa gaatgctatg aattagctgc tgagtatgaa agtaagattg cgtatcgtag     300
gaggataaaa ctgaagaagg gtgattttgc tgatgaaaac tctgctacaa ctgaatcaaa     360
ctttgaggag accctaaaaa ggcttgttgg tgagcttaag aagtcccctc atgaggtatt     420
tgatgctctc aagaatcaaa caatcgacct ggtcctgaca gcacatccaa ctcagtcagt     480
caggaggtcg ctgctacaaa agcatggcag gataagaagt tgtttaacaa aactttatgc     540
aaaagacata actccagatg agaagcagga actcgatgaa gcacttaaga gagagatcca     600
agccgccttt agaacagatg aaattcggcg agcacctcct acaccacagg atgaaatgcg     660
tgctggaatg agttattttc atgagacaat atggaagggt gtacccaagt tttacgtag      720
ggttgatact gcccttaaga acattggcat aaacgagcga gtgccctata atgctcctct     780
tattcaattt tcttcttgga tgggtggaga ccgtgatgga aatccaagag tcacaccaga     840
ggttacaagg gacgtatgcc tgttagctag aatgatggct gctaacttat actatgcaca     900
gatagaggat ctgatgtttg agctatctat gtggcgctgt agtgatgaac tacgtgtaaa     960
agctgatcag ttacaccgtt gcgcaaagaa aaacacaaca aagcactata tagagttctg    1020
gaaacaagtt cctccgagtg aaccctatcg tgtaatactg agcaatgtca gagataagtt    1080
gtacaataca cgcgagcgag cacgccattt attagccagt ggattttctg aaattcctga    1140
cgaagcaaca ttcactgatg ttgagcagtt cttggagcct cttgagctct gttacaggtc    1200
tctctgtgcc tgtggtgata attctattgc agatggcagt cttcttgact ttctacggca    1260
agtgtccaca tttggactat cccttgttag acttgatatt aggcaagaat ctgaccgaca    1320
tactgatgtt atggatgcca taactcaata ccttggaatt ggatcctatc gtgaatggtc    1380
agaggagaaa cgccaagaat ggcttctgtc agaactcaat ggaaagaggc cattatttgg    1440
tcctgacctt ccccagacgg atgaaattgc tgatgcccta gatacttttc atgtgatagc    1500
agaactaccc tatgatagct ttggtgcata tgtaatatcc atggcaacag ctccttcaga    1560
tgttctagca gttgagcttc tgcaacgtga atgtcatgtg aagaagccac tgagagttgt    1620
gccattgttt gaaaaactag cagatctgga agcagcacca gcagctcttg cccgactttt    1680
ctctgttgac tggtacagaa ataggatcga cggaaagcag gaagtaatga ttgggtattc    1740
agattctgga aaagatgctg gccgtttctc tgcagcttgg gaactgtata aagctcaaga    1800
ggagcttatt aaagttgcta agcagtttgg ggttaagttg actatgtttc atgggcgagg    1860
```

```
tggaactgtt ggaagaggtg ggggtcctac acaccttgct atactgtcac aacctccaga    1920
taccattcat ggatcacttc gtgtgactgt tcaaggtgaa gtcattgagc aatcttttgg    1980
agaggagcat ttgtgtttta ggacacttca acgttttaca tctgctactc ttgagcatgg    2040
tatgcatcca ccaatttcac caaagccaga atggcgtgct ttgatggatg aaatggccgc    2100
tgttgctaca aaagaatacc ggtccattgt cttccaagag gcacgatttg ttgaatattt    2160
ccgccttgca acaccagagt tggaatatgg taggatgaat attggaagta ggccatcaaa    2220
acgaaagcct agtggaggca ttgaatcact tcgtgcaatt ccttggatct ttgcttggac    2280
acaaactcga ttccacctac cagtgtggct tggttttggc gcagcgttca agcatgtctt    2340
gcagaaggac atacgcaatc ttcagatcct tcaggagatg tacaacgaat ggccattttt    2400
cagggttaca atagacttgg ttgagatggt gtttgctaag ggtgatcctg gtatagcagc    2460
tctgtatgac aagttgctag tttctgagga tttgtggcca tttggtgctc gtcttagagc    2520
aaactatgaa gaaacaaagc aacttcttct acaggttgct ggacacaaag acctctgga     2580
gggagatcct tacttgaggc agagactgcg tatccgtgat tcctacatca cagcgctgaa    2640
tgtttgccaa gcttgcacgc taaagcgtat cagggaccct ggcttccatg taagcccccg    2700
agctcacctc tccaaggaca taatggactc agggaagcca gccgctgagc ttgtgaagct    2760
gaacacaaca agcgagtacg gcccaggcct cgaggacact ctcatcctga ccatgaaggg    2820
catcgctgct ggtatgcaga acactgggtg aagtagaact ggttcgttct gatggtgtgt    2880
tatggtgtca tgaattagtg tagtttgcac ctctatgcaa tcactatttg cagtttgcac    2940
ctctgaactc tatatgtaaa taaggggatg ctaagcaacg tataactaag actggttata    3000
tccatggata ttacctgttt agcatgttat atatcgaact cttatgctgt tcacc         3055
```

```
<210>  62
<211>  924
<212>  PRT
<213>  Oryza sativa

<400>  62
Met Ala Arg Asn Ala Ala Asp Lys Gly Thr Ser Ile Asp Ala Gln Leu
1               5                   10                  15
Arg Ile Leu Ala Pro Lys Lys Leu Ser Glu Asp Asp Lys Leu Val Glu
            20                  25                  30
Tyr Asp Ala Leu Leu Leu Asp Arg Phe Leu Asp Ile Leu Gln Asp Leu
        35                  40                  45
His Gly Glu Asp Ile Arg Glu Thr Val Gln Glu Cys Tyr Glu Leu Ala
    50                  55                  60
Ala Glu Tyr Glu Ser Lys Ile Ala Tyr Arg Arg Arg Ile Lys Leu Lys
65                  70                  75                  80
Lys Gly Asp Phe Ala Asp Glu Asn Ser Ala Thr Thr Glu Ser Asn Phe
                85                  90                  95
Glu Glu Thr Leu Lys Arg Leu Val Gly Glu Leu Lys Lys Ser Pro His
            100                 105                 110
Glu Val Phe Asp Ala Leu Lys Asn Gln Thr Ile Asp Leu Val Leu Thr
        115                 120                 125
Ala His Pro Thr Gln Ser Val Arg Arg Ser Leu Leu Gln Lys His Gly
    130                 135                 140
Arg Ile Arg Ser Cys Leu Thr Lys Leu Tyr Ala Lys Asp Ile Thr Pro
145                 150                 155                 160
Asp Glu Lys Gln Glu Leu Asp Glu Ala Leu Lys Arg Glu Ile Gln Ala
                165                 170                 175
Ala Phe Arg Thr Asp Glu Ile Arg Arg Ala Pro Pro Thr Pro Gln Asp
            180                 185                 190
Glu Met Arg Ala Gly Met Ser Tyr Phe His Glu Thr Ile Trp Lys Gly
        195                 200                 205
Val Pro Lys Phe Leu Arg Arg Val Asp Thr Ala Leu Lys Asn Ile Gly
    210                 215                 220
Ile Asn Glu Arg Val Pro Tyr Asn Ala Pro Leu Ile Gln Phe Ser Ser
225                 230                 235                 240
Trp Met Gly Gly Asp Arg Asp Gly Asn Pro Arg Val Thr Pro Glu Val
                245                 250                 255
Thr Arg Asp Val Cys Leu Leu Ala Arg Met Met Ala Ala Asn Leu Tyr
            260                 265                 270
Tyr Ala Gln Ile Glu Asp Leu Met Phe Glu Leu Ser Met Trp Arg Cys
        275                 280                 285
Ser Asp Glu Leu Arg Val Lys Ala Asp Gln Leu His Arg Cys Ala Lys
    290                 295                 300
Lys Asn Thr Thr Lys His Tyr Ile Glu Phe Trp Lys Gln Val Pro Pro
305                 310                 315                 320
Ser Glu Pro Tyr Arg Val Ile Leu Ser Asn Val Arg Asp Lys Leu Tyr
                325                 330                 335
Asn Thr Arg Glu Arg Ala Arg His Leu Leu Ala Ser Gly Phe Ser Glu
            340                 345                 350
Ile Pro Asp Glu Ala Thr Phe Thr Asp Val Glu Gln Phe Leu Glu Pro
        355                 360                 365
Leu Glu Leu Cys Tyr Arg Ser Leu Cys Ala Cys Gly Asp Asn Ser Ile
```

```
        370                 375                 380
Ala Asp Gly Ser Leu Leu Asp Phe Leu Arg Gln Val Ser Thr Phe Gly
385                 390                 395                 400
Leu Ser Leu Val Arg Leu Asp Ile Arg Gln Glu Ser Asp Arg His Thr
                405                 410                 415
Asp Val Met Asp Ala Ile Thr Gln Tyr Leu Gly Ile Gly Ser Tyr Arg
            420                 425                 430
Glu Trp Ser Glu Glu Lys Arg Gln Glu Trp Leu Leu Ser Glu Leu Asn
        435                 440                 445
Gly Lys Arg Pro Leu Phe Gly Pro Asp Leu Pro Gln Thr Asp Glu Ile
    450                 455                 460
Ala Asp Ala Leu Asp Thr Phe His Val Ile Ala Glu Leu Pro Tyr Asp
465                 470                 475                 480
Ser Phe Gly Ala Tyr Val Ile Ser Met Ala Thr Ala Pro Ser Asp Val
                485                 490                 495
Leu Ala Val Glu Leu Leu Gln Arg Glu Cys His Val Lys Lys Pro Leu
            500                 505                 510
Arg Val Val Pro Leu Phe Glu Lys Leu Ala Asp Leu Glu Ala Ala Pro
        515                 520                 525
Ala Ala Leu Ala Arg Leu Phe Ser Val Asp Trp Tyr Arg Asn Arg Ile
    530                 535                 540
Asp Gly Lys Gln Glu Val Met Ile Gly Tyr Ser Asp Ser Gly Lys Asp
545                 550                 555                 560
Ala Gly Arg Phe Ser Ala Ala Trp Glu Leu Tyr Lys Ala Gln Glu Glu
                565                 570                 575
Leu Ile Lys Val Ala Lys Gln Phe Gly Val Lys Leu Thr Met Phe His
            580                 585                 590
Gly Arg Gly Gly Thr Val Gly Arg Gly Gly Gly Pro Thr His Leu Ala
        595                 600                 605
Ile Leu Ser Gln Pro Pro Asp Thr Ile His Gly Ser Leu Arg Val Thr
    610                 615                 620
Val Gln Gly Glu Val Ile Glu Gln Ser Phe Gly Glu Glu His Leu Cys
625                 630                 635                 640
Phe Arg Thr Leu Gln Arg Phe Thr Ser Ala Thr Leu Glu His Gly Met
                645                 650                 655
His Pro Pro Ile Ser Pro Lys Pro Glu Trp Arg Ala Leu Met Asp Glu
            660                 665                 670
Met Ala Ala Val Ala Thr Lys Glu Tyr Arg Ser Ile Val Phe Gln Glu
        675                 680                 685
Ala Arg Phe Val Glu Tyr Phe Arg Leu Ala Thr Pro Glu Leu Glu Tyr
    690                 695                 700
Gly Arg Met Asn Ile Gly Ser Arg Pro Ser Lys Arg Lys Pro Ser Gly
705                 710                 715                 720
Gly Ile Glu Ser Leu Arg Ala Ile Pro Trp Ile Phe Ala Trp Thr Gln
                725                 730                 735
Thr Arg Phe His Leu Pro Val Trp Leu Gly Phe Gly Ala Ala Phe Lys
            740                 745                 750
His Val Leu Gln Lys Asp Ile Arg Asn Leu Gln Ile Leu Gln Glu Met
        755                 760                 765
Tyr Asn Glu Trp Pro Phe Phe Arg Val Thr Ile Asp Leu Val Glu Met
    770                 775                 780
Val Phe Ala Lys Gly Asp Pro Gly Ile Ala Ala Leu Tyr Asp Lys Leu
785                 790                 795                 800
Leu Val Ser Glu Asp Leu Trp Pro Phe Gly Ala Arg Leu Arg Ala Asn
                805                 810                 815
Tyr Glu Glu Thr Lys Gln Leu Leu Leu Gln Val Ala Gly His Lys Asp
            820                 825                 830
Leu Leu Glu Gly Asp Pro Tyr Leu Arg Gln Arg Leu Arg Ile Arg Asp
        835                 840                 845
Ser Tyr Ile Thr Ala Leu Asn Val Cys Gln Ala Cys Thr Leu Lys Arg
    850                 855                 860
Ile Arg Asp Pro Gly Phe His Val Ser Pro Arg Ala His Leu Ser Lys
865                 870                 875                 880
Asp Ile Met Asp Ser Gly Lys Pro Ala Ala Glu Leu Val Lys Leu Asn
                885                 890                 895
Thr Thr Ser Glu Tyr Gly Pro Gly Leu Glu Asp Thr Leu Ile Leu Thr
            900                 905                 910
Met Lys Gly Ile Ala Ala Gly Met Gln Asn Thr Gly
        915                 920
```

<210> 63
<211> 2832
<212> DNA
<213> Welwitschia mirabilis

<220>
<221> misc_feature
<222> (1741)..(1743)
<223> n is a, c, g, or t

<400> 63
agcatcgacg cgcaactgcg gcttttggtc ccaacgaagg tttccgaaga tgataagctt 60
attgagtatg acgctcttct gatggaccgc tttctggata tcttgcaaga cctacatgga 120
gaagaaatta gagaaacggt tcaagagtgc tatgagcttt caggagaata cgaaggaaag 180
tttgacacgg caaagttgga ggagcttgga ggagtgttaa caagtctaga tgctggagac 240
tccattgttg ttgcagctct ttctcacatg cttaacctag cgaacttggc tgaggaggtt 300
cagatagcat accgaagacg aattaaacac aaaaagaaag ggatttcgc ggatgagaat 360
tccgcaacga cggaatcaga cattgaagaa acctttaaaa ggcttgtaaa tcagcttgga 420
aggtctcctc aagaagtttt tgacgctctc aaaaatcaaa caattgattt ggtcttgact 480
gcacatccaa cgcatcagtc ggtatccctg ctacaaaagc atggcaggat ccgaaactgc 540
ttgtctcagt tgtatgcgaa agacattacg cccgatgaaa aacaggagct tgatgaagct 600
ttacagaggg agattcaagc tgccttcaga actgatgaaa ttcggcgcac tcctcccacc 660
cctcaagatg agatgcgagc aggaatgagt tatttccatg aaaccatatg gaaaggtgtc 720
cctaagttct tacgtcgtgt tgacactgcc ctaaagtcga ttggtataaa tgagagactt 780
ccttacaatg ccccccctaat acaattctcg tcgtggatgg gcggcgaccg cgacgggaat 840
cctagagtca ctccagaagt cacgcgagac gtttgtctcc ttgcgaggat gatggctaca 900
aatttatact actctcagat agaggacctt atgtttgagt tgtcaatgtg gcgatgtagc 960
gatgagcttc ggcaacgtgc tctgcagctt cataattctg caaagagaga tgcaaaacat 1020
tacatagagt tttggaagca agtacctccc aatgagccat ttagggtaat tttgggagat 1080
gttagggaca agttatataa cactcgggaa cgcactcgcc agttgcttgc caatggtttc 1140
tcagatgtgc cagaagaagc acttacgagt gtagatcagt tgttggaacc gttagagttg 1200
tgctatcgat ctctgtgctc taccggcgat caacccattg cagatggcag tcttcttgat 1260
ttcatgcgtc aagtctcaac ctttggctta actcttgtga agttggatat tagacaagaa 1320
tcagatcgac acactgatgt tatcgacgca ataacaagac atctaggcct tgggtcctat 1380
aaagagtggc cagaagacaa acgacaggag tggctgttgg ctgaactacg tggaaaacgt 1440
ccactatttg gacccgacct tccaactaca gatgaaataa gagaagttct tgacacattc 1500
cacgtggtag ctgaacttcc tccagacaac tttggagctt acatcatatc aatggctact 1560
gctccttcag atgttttagt cgttgagtta ctgcagaggg aatgccgtgt gaaaaagcct 1620
ctgcgagttg tgcctttgtt tgagaagctt gctgatttag agaatcgtcc cgcggctttg 1680
gcaaggctgt tctcaattga ttggtataga aacagaattg atggaaagca agaagtaatg 1740
nnnggttact cagactccgg caaggatgcc ggaagactat ctgcagcatg gcaattatac 1800
aaggctcaag aggaattgat caaggttgca aaacagtttg gaattaagct gacaatgttt 1860
catggtcgtg gagggaccgt tggaagagga ggtggtccaa cccatctggc tatattatct 1920
caacctccag acacaattca cgggtcattg cgagttaccg tccaagggga ggttattgag 1980
cagtgttttg ggaggaaca tctgtgtttc cgaactcttc agcgctttac tgctgccaca 2040
ttggaacatg gaatgcatcc tccaattgca cctaagcccg aatggcgtca actgatggat 2100
gaaatggctg ttgttgctac tcaagagtat aggtcttatg ttttccacaa caagagattt 2160
gtggagtatt tccgatccgc aactcccgag ttggagtatg gacggatgaa tataggcagt 2220
cgtccatcaa aaagaaagcc cagtggaggc attgaatcac ttcgtgcaat tccatggata 2280
tttgcttgga cacagaccag attccatctt cctgtttggc ttggatttgg tgctgctttc 2340
aagagtgtta tcgagaagga cattcgaaat cttcacacgc tgcaacaaat gtataacgaa 2400
tggccatttt tccgtgtcac cattgaccta gttgaaatgg tgtttgccaa gcaccctgaa 2460
attgctgcat tatacgataa actgcttgta tcatcagtct tgtgggcttt cggggaacaa 2520
ctaagaaaaa actatgtaga gactaagacc cttctgctgc aggttgctgg acacaaagaa 2580
gtcttagaag gcgacccata tctgaaacaa cgattgagac ttcgtgactc atatatcaca 2640
accttgaatg cacttcaggc atatacttta aaacggatac gagatccaag ctaccatgtc 2700
accctcaggc ctcatttatc taaagaaagc tcaaccaagc cagcagcaga attggtaaaa 2760
ttaaacccaa ccagtgagta tgcaccggga ttagaagata cattgatctt aaccatgaag 2820
ggcattgctg cc 2832

<210> 64
<211> 944
<212> PRT
<213> Welwitschia mirabilis

<220>
<221> UNSURE
<222> (581)..(581)
<223> Xaa can be any naturally occurring amino acid

<400> 64
Ser Ile Asp Ala Gln Leu Arg Leu Leu Val Pro Thr Lys Val Ser Glu
1 5 10 15
Asp Asp Lys Leu Ile Glu Tyr Asp Ala Leu Leu Met Asp Arg Phe Leu
20 25 30
Asp Ile Leu Gln Asp Leu His Gly Glu Glu Ile Arg Glu Thr Val Gln
35 40 45
Glu Cys Tyr Glu Leu Ser Gly Glu Tyr Glu Gly Lys Phe Asp Thr Ala

```
        50                  55                  60
Lys Leu Glu Glu Leu Gly Gly Val Leu Thr Ser Leu Asp Ala Gly Asp
65                  70                  75                  80
Ser Ile Val Val Ala Ala Leu Ser His Met Leu Asn Leu Ala Asn Leu
                85                  90                  95
Ala Glu Glu Val Gln Ile Ala Tyr Arg Arg Arg Ile Lys His Lys Lys
            100                 105                 110
Lys Gly Asp Phe Ala Asp Glu Asn Ser Ala Thr Thr Glu Ser Asp Ile
        115                 120                 125
Glu Glu Thr Phe Lys Arg Leu Val Asn Gln Leu Gly Arg Ser Pro Gln
    130                 135                 140
Glu Val Phe Asp Ala Leu Lys Asn Gln Thr Ile Asp Leu Val Leu Thr
145                 150                 155                 160
Ala His Pro Thr His Gln Ser Val Ser Leu Leu Gln Lys His Gly Arg
                165                 170                 175
Ile Arg Asn Cys Leu Ser Gln Leu Tyr Ala Lys Asp Ile Thr Pro Asp
            180                 185                 190
Glu Lys Gln Glu Leu Asp Glu Ala Leu Gln Arg Glu Ile Gln Ala Ala
        195                 200                 205
Phe Arg Thr Asp Glu Ile Arg Arg Thr Pro Pro Thr Pro Gln Asp Glu
    210                 215                 220
Met Arg Ala Gly Met Ser Tyr Phe His Glu Thr Ile Trp Lys Gly Val
225                 230                 235                 240
Pro Lys Phe Leu Arg Arg Val Asp Thr Ala Leu Lys Ser Ile Gly Ile
                245                 250                 255
Asn Glu Arg Leu Pro Tyr Asn Ala Pro Leu Ile Gln Phe Ser Ser Trp
            260                 265                 270
Met Gly Gly Asp Arg Asp Gly Asn Pro Arg Val Thr Pro Glu Val Thr
        275                 280                 285
Arg Asp Val Cys Leu Leu Ala Arg Met Met Ala Thr Asn Leu Tyr Tyr
    290                 295                 300
Ser Gln Ile Glu Asp Leu Met Phe Glu Leu Ser Met Trp Arg Cys Ser
305                 310                 315                 320
Asp Glu Leu Arg Gln Arg Ala Leu Gln Leu His Asn Ser Ala Lys Arg
                325                 330                 335
Asp Ala Lys His Tyr Ile Glu Phe Trp Lys Gln Val Pro Pro Asn Glu
            340                 345                 350
Pro Phe Arg Val Ile Leu Gly Asp Val Arg Asp Lys Leu Tyr Asn Thr
        355                 360                 365
Arg Glu Arg Thr Arg Gln Leu Leu Ala Asn Gly Phe Ser Asp Val Pro
    370                 375                 380
Glu Glu Ala Leu Thr Ser Val Asp Gln Leu Leu Glu Pro Leu Glu Leu
385                 390                 395                 400
Cys Tyr Arg Ser Leu Cys Ser Thr Gly Asp Gln Pro Ile Ala Asp Gly
                405                 410                 415
Ser Leu Leu Asp Phe Met Arg Gln Val Ser Thr Phe Gly Leu Thr Leu
            420                 425                 430
Val Lys Leu Asp Ile Arg Gln Glu Ser Asp Arg His Thr Asp Val Ile
        435                 440                 445
Asp Ala Ile Thr Arg His Leu Gly Leu Gly Ser Tyr Lys Glu Trp Pro
    450                 455                 460
Glu Asp Lys Arg Gln Glu Trp Leu Leu Ala Glu Leu Arg Gly Lys Arg
465                 470                 475                 480
Pro Leu Phe Gly Pro Asp Leu Pro Thr Thr Asp Glu Ile Arg Glu Val
                485                 490                 495
Leu Asp Thr Phe His Val Val Ala Glu Leu Pro Pro Asp Asn Phe Gly
            500                 505                 510
Ala Tyr Ile Ile Ser Met Ala Thr Ala Pro Ser Asp Val Leu Val Val
        515                 520                 525
Glu Leu Leu Gln Arg Glu Cys Arg Val Lys Lys Pro Leu Arg Val Val
    530                 535                 540
Pro Leu Phe Glu Lys Leu Ala Asp Leu Glu Asn Arg Pro Ala Ala Leu
545                 550                 555                 560
Ala Arg Leu Phe Ser Ile Asp Trp Tyr Arg Asn Arg Ile Asp Gly Lys
                565                 570                 575
Gln Glu Val Met Xaa Gly Tyr Ser Asp Ser Gly Lys Asp Ala Gly Arg
            580                 585                 590
Leu Ser Ala Ala Trp Gln Leu Tyr Lys Ala Gln Glu Glu Leu Ile Lys
        595                 600                 605
Val Ala Lys Gln Phe Gly Ile Lys Leu Thr Met Phe His Gly Arg Gly
    610                 615                 620
Gly Thr Val Gly Arg Gly Gly Gly Pro Thr His Leu Ala Ile Leu Ser
625                 630                 635                 640
Gln Pro Pro Asp Thr Ile His Gly Ser Leu Arg Val Thr Val Gln Gly
```

```
                645                 650                 655
Glu Val Ile Glu Gln Cys Phe Gly Glu Glu His Leu Cys Phe Arg Thr
            660                 665                 670
Leu Gln Arg Phe Thr Ala Ala Thr Leu Glu His Gly Met His Pro Pro
        675                 680                 685
Ile Ala Pro Lys Pro Glu Trp Arg Gln Leu Met Asp Glu Met Ala Val
    690                 695                 700
Val Ala Thr Gln Glu Tyr Arg Ser Tyr Val Phe His Asn Lys Arg Phe
705                 710                 715                 720
Val Glu Tyr Phe Arg Ser Ala Thr Pro Glu Leu Glu Tyr Gly Arg Met
                725                 730                 735
Asn Ile Gly Ser Arg Pro Ser Lys Arg Lys Pro Ser Gly Gly Ile Glu
            740                 745                 750
Ser Leu Arg Ala Ile Pro Trp Ile Phe Ala Trp Thr Gln Thr Arg Phe
        755                 760                 765
His Leu Pro Val Trp Leu Gly Phe Gly Ala Ala Phe Lys Ser Val Ile
    770                 775                 780
Glu Lys Asp Ile Arg Asn Leu His Thr Leu Gln Gln Met Tyr Asn Glu
785                 790                 795                 800
Trp Pro Phe Phe Arg Val Thr Ile Asp Leu Val Glu Met Val Phe Ala
                805                 810                 815
Lys His Pro Glu Ile Ala Ala Leu Tyr Asp Lys Leu Leu Val Ser Ser
            820                 825                 830
Val Leu Trp Ala Phe Gly Glu Gln Leu Arg Lys Asn Tyr Val Glu Thr
        835                 840                 845
Lys Thr Leu Leu Leu Gln Val Ala Gly His Lys Glu Val Leu Glu Gly
    850                 855                 860
Asp Pro Tyr Leu Lys Gln Arg Leu Arg Leu Arg Asp Ser Tyr Ile Thr
865                 870                 875                 880
Thr Leu Asn Ala Leu Gln Ala Tyr Thr Leu Lys Arg Ile Arg Asp Pro
                885                 890                 895
Ser Tyr His Val Thr Leu Arg Pro His Leu Ser Lys Glu Ser Ser Thr
            900                 905                 910
Lys Pro Ala Ala Glu Leu Val Lys Leu Asn Pro Thr Ser Glu Tyr Ala
        915                 920                 925
Pro Gly Leu Glu Asp Thr Leu Ile Leu Thr Met Lys Gly Ile Ala Ala
    930                 935                 940
```

<210> 65
<211> 4776
<212> DNA
<213> Chlamydomonas reinhardtii

<400> 65

```
atgacggact ccacatatga ttttggagcc gtgagggacg atctgacgcc gcttgaggat      60
gactgcaagc ttctcggtag cctgcttgac gactgtctcc gcgtcgaaat cggcgagacc     120
atgttcaaga agattgaacg catccgagca ctggcgcaat gtgcctcaaa tctgtcgatc     180
aagggagacg cgggcgcctc cgacatgctg tcgcaccggc tggcggagga gctgatgaac     240
ctggacatgg acgaggcggt gccgctcaca cgcgcctgcg gccactacct caacctgtcc     300
ggcatcgcag agctgcacca cggagtgcgc cgcgaccgcg ccactcgcga gcccaacccc     360
aacagctgcg acgcggtgtt tgcgcggctg atcacggagg gcgtggaccc cgaggagctg     420
taccgggcgg tgtcggagca gaacgtggag gtggtgctca ccgcgcaccc cacacaggtg     480
aaccggcgca cgctgcagta caagcacact cgcattgcgg cgctgctgca gcagcacgac     540
cgctccgacc tgacggcgga ggagcggcgc aacatggtga gcgagctgca gcgggaggtg     600
gcggcgctgt ggcagacgga cgagctgagg aggcagaagc ccacgccgct ggacgaggcg     660
cgcggcggtc tgcacattgt ggagcagtcg ctgtgggccg cggtgccgca gtacatgcgc     720
cgcctcagcg ccgcgctcaa gaaacacacc gggcacgacc tgccgctgca ggccacgccc     780
ttccgcttcg gcagctggat gggcggcgac cgcgatggca accccaacgt gaccgccaag     840
gtgacggcgc acgtgacggc cctggcgcgc tggatggcgg cggatctgta cctgcgtgag     900
atcgacacgc tgaggtttga gctgtccatg aaccagtgca gtgcggcggt gtggaagatg     960
gcccgccgca tcatcgccga gggccacacc aagcgcgccg gcgtggtccg ggccaaggcc    1020
gccgccgccc tgcaccagac cgcaacagac gcagccagcc atggcggctc cgccgcctcg    1080
gcggccgccg ccgccgccgc aggcggcgac gtcgtcgctg acggcaccag cggcggcggc    1140
gctgccgccg ccgccggccc cgccgccgct gccgcggcgg acgacgcgtt caccttcagc    1200
cggttggggc ggccccggcc ggagcggccg agcacggacg tgcggagtgt gggcgtgctg    1260
gcgggcggcg agggcgcggc gttcccgggc ggcatgatcc tgggcacgca gccggtgtcg    1320
gcgcacacgg cggcggaggt gtcggtgccg cacgagctgc cgggacagga cgtggagggc    1380
ggctcggaga tggacttcaa cgagtcccgc cgcgcctcgg acgccggaga cctgggcgcc    1440
tcgcagcacc ccatgctggg cgggccctcc gccggcgcct ccgccgagcc caccgcccac    1500
ggctacacca ccaccgccac cgccgccgcc gccgccgccg acggcacgca gcccgagccc    1560
gaagtccccg gcacgcccag ctacgccgac cccggcaccc ccgaccgcct tggcgcgctg    1620
cccggcccct tcacgcccgg ccccacgccc ttccgcgagg cggccaacgc cgccatgagc    1680
accgccgcca gcggcggcgc gggcggcggc ggcggcggcg gcgcgaaccg cgcggcctcg    1740
ggcctgggcg gcgaccccac cttcacgcgt cgcagcctga tggcgcagcg gctgggaacc    1800
```

agctcggtgc agttcgcgcg cgcgcacgag caccccggct tccacccccta ccgcatcgtg 1860
ttgggccacg tgcgcgacag gctggcggcc acgcggcggc gcatggagga cctgctgagc 1920
ggccgcgagc cggcgggcga ggcgcacggc ggcgtggggg cgggcggcgg cggcggcggc 1980
ggcgcggcgc cgtggtatga gagcgaggac gagctggcgg agccgctgat ggcgtgctac 2040
tggtcgctgt gggagtgcgg cggcggcgtc attgcggacg ggcggctgct ggacctcatc 2100
cgccgcgtgt acaccttcgg catgtgcctc atgaagctag acctgcgcca ggagtccacc 2160
cgccacgccg aggccctgga cgccgtgacc agctacctgg gtctgggctc ctacctggag 2220
tggagcgagg accagaaaat cgagtggctc acaaaggagc tgcagggccg gcggccgctc 2280
atccccgccg acatgcccat gagcgccgag gtgcgcgagg tgctggacac cttcaaggtg 2340
gccgcccatc tgggccgtga caacctgggc gcctacgtca tctccatgac caagggcgcc 2400
tcggacgtga tggcggtgga gctgctgcag cgcgaggcgc gcatgcaggt gggcgccgag 2460
gcgggcggcc gcggcggcgg cgggcccgag gacggcggct cgctgcgcgt ggtgccgctg 2520
ttcgagacgc tggaggacct ggacgcggcg gaggacgtga tgacgcggct gctgaccaac 2580
ccctggtacc gcgagcacct gcgggcagtg cacggcgacg cgcaggaggt gatgctggga 2640
tacagcgaca gcggcaagga cgccggccgc ctggctgcca actgggcgct gtacaagtgt 2700
caggagcggc tggtggccat caccaaggcc aacaacgtca agctcacgct gttccacggc 2760
cgcggcggca ccgtgggccg cggcggcggg cccacccaca tcgccatcca gtcgcagccg 2820
cccggctctg tggaggggac cttccgcatc accgagcagg gcgagatggt ccaggccaag 2880
ttcggcatca gcggcgtggc tctgagccag ctggagacct acaccaccgc tgtgctgctg 2940
gccaccatgc gcccgcccag cccgccgcgc cgcgaggagt ggcgcgccgt gatggagatg 3000
ctcagccgcg tcagctgcga gagctaccgc aacatcgtgc accacagccc gctcttcctg 3060
cgctacttca agcacgccac gcccgaggcc gagctgggca acctctacat cggctctcgc 3120
ccggctcggc ggcgcaacaa ggacgcctcc atctccacgc tgcgcgccat cccctggatc 3180
ttcgcctgga cgcagaaccg cctcatcctg ccctcctggc tgggcattgg cgccgccctc 3240
accgcggcca tgacccaggg ccacctgccc acgctgcagg ccatgtaccg cgagtggccc 3300
ttcttcggat ccacagtgga tctgattgag atgatcctgg ccaagaccga cccgcgcatc 3360
gcggcgctgt acgaggaggt gctggtcaac gaccccgagg agaagaagct gggagccgag 3420
ctgcgcgagc ggctgcagcg ctgccagggc gccatcctca aggtgaccgg gcacgagaac 3480
ctgctgtcca acaaccccac attgagcaag ctcatctcca tgcgctcgcc cttcgtggac 3540
cccatcaaca tcctgcaggt ggaggtgctg cggcgcctgc gccaggaccc caacaacatg 3600
cgcctgcgcg acgcgctgct catctccatc aacggcatcg ccgccggcat gcgcaacacc 3660
ggctaagcag cggcggcgct gctgctggtg atggaggtgg aggtggagat ggacgtggtg 3720
cgcaccaggc acaaggcgga ggcaggtgcc agtggaggtg gggcgtcccg ggcggaggca 3780
tgagcggcgt cgacggctgg cgccgcggta tgatgtgctg cggcacgagc gacggcacgt 3840
gcggtctaaa gctgctggat gtgcttggca cggcgcgcaa gaggggtgtg ctgggggcgg 3900
aggtggtgct gaaaatgttt ggttagttgg ttggatgttt gggctagctg gtgcgctgga 3960
gcgggtaagg caagtggtgg gtggggtatg cgggtgcgct ggggtgtcca gggcggctgc 4020
gatcaatgcg ggttggtggg caaccccgtc ccggcgctga atcgctgcgt ggatctgacc 4080
tttgcggaat tcctgcctgc cttccgcgcc tggcaattta ggaactcgtt tgagcgtttg 4140
acgttgtcct agggcggcat gaccgagcat cgcgtgcatg gcagcggggc tgcgatcgcg 4200
cgtggtgagc cacagacgtt gatagtgata tttgaggcag cccagaacgc atggcggaat 4260
ggcgtggtct ttgagagctg agtgtgtcag actggcatag ggcgctgtgg ctgactaacg 4320
tcccgcacgt gtgcgactgc agaccatgtt tgcaggggca ggtactttac ttgatgtgca 4380
tggcattctc acataaagct ccgaaggatc atttgtggcg caccaaccag agacctagcc 4440
cagagcagag tgtcagcagc gatatcaaag tcattttgtg ggtggttgtc tgggtactcc 4500
gaggcgcgta cgttgagcgc gtgctcgctg cataccgcat acggaatcgg ataagtgaaa 4560
ggaggtcaca tggccagggg ctgcagatgc tggtgagggc tgcctgccta agcggcgtgt 4620
ccgggacggc agggaagcac gcatgccatt cgacgtgtgc ggtgcaaatg agaccgtgga 4680
gtcctcgggc agggcttgac agcacaagcg tggagcgcgc acacagcgca cagactatgt 4740
tttccttgtg ctctttaaca cagcgcacag aaaaaa 4776

<210> 66
<211> 1221
<212> PRT
<213> Chlamydomonas reinhardtii

<400> 66
Met Thr Asp Ser Thr Tyr Asp Phe Gly Ala Val Arg Asp Asp Leu Thr
1 5 10 15
Pro Leu Glu Asp Asp Cys Lys Leu Leu Gly Ser Leu Leu Asp Asp Cys
20 25 30
Leu Arg Val Glu Ile Gly Glu Thr Met Phe Lys Lys Ile Glu Arg Ile
35 40 45
Arg Ala Leu Ala Gln Cys Ala Ser Asn Leu Ser Ile Lys Gly Asp Ala
50 55 60
Gly Ala Ser Asp Met Leu Ser His Arg Leu Ala Glu Glu Leu Met Asn
65 70 75 80
Leu Asp Met Asp Glu Ala Val Pro Leu Thr Arg Ala Cys Gly His Tyr
85 90 95
Leu Asn Leu Ser Gly Ile Ala Glu Leu His His Gly Val Arg Arg Asp
100 105 110
Arg Ala Thr Arg Glu Pro Asn Pro Asn Ser Cys Asp Ala Val Phe Ala
115 120 125
Arg Leu Ile Thr Glu Gly Val Asp Pro Glu Glu Leu Tyr Arg Ala Val

```
    130                 135                 140
Ser Glu Gln Asn Val Glu Val Val Leu Thr Ala His Pro Thr Gln Val
145                 150                 155                 160
Asn Arg Arg Thr Leu Gln Tyr Lys His Thr Arg Ile Ala Ala Leu Leu
                165                 170                 175
Gln Gln His Asp Arg Ser Asp Leu Thr Ala Glu Glu Arg Arg Asn Met
            180                 185                 190
Val Ser Glu Leu Gln Arg Glu Val Ala Ala Leu Trp Gln Thr Asp Glu
        195                 200                 205
Leu Arg Arg Gln Lys Pro Thr Pro Leu Asp Glu Ala Arg Gly Gly Leu
    210                 215                 220
His Ile Val Glu Gln Ser Leu Trp Ala Ala Val Pro Gln Tyr Met Arg
225                 230                 235                 240
Arg Leu Ser Ala Ala Leu Lys Lys His Thr Gly His Asp Leu Pro Leu
                245                 250                 255
Gln Ala Thr Pro Phe Arg Phe Gly Ser Trp Met Gly Gly Asp Arg Asp
            260                 265                 270
Gly Asn Pro Asn Val Thr Ala Lys Val Thr Ala His Val Thr Ala Leu
        275                 280                 285
Ala Arg Trp Met Ala Ala Asp Leu Tyr Leu Arg Glu Ile Asp Thr Leu
    290                 295                 300
Arg Phe Glu Leu Ser Met Asn Gln Cys Ser Ala Ala Val Trp Lys Met
305                 310                 315                 320
Ala Arg Arg Ile Ile Ala Glu Gly His Thr Lys Arg Ala Gly Val Val
                325                 330                 335
Arg Ala Lys Ala Ala Ala Ala Leu His Gln Thr Ala Thr Asp Ala Ala
            340                 345                 350
Ser His Gly Gly Ser Ala Ala Ser Ala Ala Ala Ala Ala Ala Ala Gly
        355                 360                 365
Gly Asp Val Val Ala Asp Gly Thr Ser Gly Gly Gly Ala Ala Ala Ala
    370                 375                 380
Ala Gly Pro Ala Ala Ala Ala Ala Ala Asp Asp Ala Phe Thr Phe Ser
385                 390                 395                 400
Arg Leu Gly Arg Pro Arg Pro Glu Arg Pro Ser Thr Asp Val Arg Ser
                405                 410                 415
Val Gly Val Leu Ala Gly Gly Glu Gly Ala Ala Phe Pro Gly Gly Met
            420                 425                 430
Ile Leu Gly Thr Gln Pro Val Ser Ala His Thr Ala Ala Glu Val Ser
        435                 440                 445
Val Pro His Glu Leu Pro Gly Gln Asp Val Glu Gly Gly Ser Glu Met
    450                 455                 460
Asp Phe Asn Glu Ser Arg Arg Ala Ser Asp Ala Gly Asp Leu Gly Ala
465                 470                 475                 480
Ser Gln His Pro Met Leu Gly Gly Pro Ser Ala Gly Ala Ser Ala Glu
                485                 490                 495
Pro Thr Ala His Gly Tyr Thr Thr Thr Ala Thr Ala Ala Ala Ala Ala
            500                 505                 510
Ala Asp Gly Thr Gln Pro Glu Pro Glu Val Pro Gly Thr Pro Ser Tyr
        515                 520                 525
Ala Asp Pro Gly Thr Pro Asp Arg Leu Gly Ala Leu Pro Gly Pro Phe
    530                 535                 540
Thr Pro Gly Pro Thr Pro Phe Arg Glu Ala Ala Asn Ala Ala Met Ser
545                 550                 555                 560
Thr Ala Ala Ser Gly Gly Ala Gly Gly Gly Gly Gly Gly Gly Ala Asn
                565                 570                 575
Arg Ala Ala Ser Gly Leu Gly Gly Asp Pro Thr Phe Thr Arg Arg Ser
            580                 585                 590
Leu Met Ala Gln Arg Leu Gly Thr Ser Ser Val Gln Phe Ala Arg Ala
        595                 600                 605
His Glu His Pro Gly Phe His Pro Tyr Arg Ile Val Leu Gly His Val
    610                 615                 620
Arg Asp Arg Leu Ala Ala Thr Arg Arg Arg Met Glu Asp Leu Leu Ser
625                 630                 635                 640
Gly Arg Glu Pro Ala Gly Glu Ala His Gly Gly Val Gly Ala Gly Gly
                645                 650                 655
Gly Gly Gly Gly Gly Ala Ala Pro Trp Tyr Glu Ser Glu Asp Glu Leu
            660                 665                 670
Ala Glu Pro Leu Met Ala Cys Tyr Trp Ser Leu Trp Glu Cys Gly Gly
        675                 680                 685
Gly Val Ile Ala Asp Gly Arg Leu Leu Asp Leu Ile Arg Arg Val Tyr
    690                 695                 700
Thr Phe Gly Met Cys Leu Met Lys Leu Asp Leu Arg Gln Glu Ser Thr
705                 710                 715                 720
Arg His Ala Glu Ala Leu Asp Ala Val Thr Ser Tyr Leu Gly Leu Gly
```

```
                725                 730                 735
Ser Tyr Leu Glu Trp Ser Glu Asp Gln Lys Ile Glu Trp Leu Thr Lys
            740                 745                 750
Glu Leu Gln Gly Arg Arg Pro Leu Ile Pro Ala Asp Met Pro Met Ser
        755                 760                 765
Ala Glu Val Arg Glu Val Leu Asp Thr Phe Lys Val Ala Ala His Leu
    770                 775                 780
Gly Arg Asp Asn Leu Gly Ala Tyr Val Ile Ser Met Thr Lys Gly Ala
785                 790                 795                 800
Ser Asp Val Met Ala Val Glu Leu Leu Gln Arg Glu Ala Arg Met Gln
                805                 810                 815
Val Gly Ala Glu Ala Gly Gly Arg Gly Gly Gly Gly Pro Glu Asp Gly
            820                 825                 830
Gly Ser Leu Arg Val Val Pro Leu Phe Glu Thr Leu Glu Asp Leu Asp
        835                 840                 845
Ala Ala Glu Asp Val Met Thr Arg Leu Leu Thr Asn Pro Trp Tyr Arg
    850                 855                 860
Glu His Leu Arg Ala Val His Gly Asp Ala Gln Glu Val Met Leu Gly
865                 870                 875                 880
Tyr Ser Asp Ser Gly Lys Asp Ala Gly Arg Leu Ala Ala Asn Trp Ala
                885                 890                 895
Leu Tyr Lys Cys Gln Glu Arg Leu Val Ala Ile Thr Lys Ala Asn Asn
            900                 905                 910
Val Lys Leu Thr Leu Phe His Gly Arg Gly Gly Thr Val Gly Arg Gly
        915                 920                 925
Gly Gly Pro Thr His Ile Ala Ile Gln Ser Gln Pro Pro Gly Ser Val
    930                 935                 940
Glu Gly Thr Phe Arg Ile Thr Glu Gln Gly Glu Met Val Gln Ala Lys
945                 950                 955                 960
Phe Gly Ile Ser Gly Val Ala Leu Ser Gln Leu Glu Thr Tyr Thr Thr
                965                 970                 975
Ala Val Leu Leu Ala Thr Met Arg Pro Pro Ser Pro Pro Arg Arg Glu
            980                 985                 990
Glu Trp Arg Ala Val Met Glu Met  Leu Ser Arg Val Ser  Cys Glu Ser
        995                 1000                1005
Tyr Arg  Asn Ile Val His His  Ser Pro Leu Phe Leu  Arg Tyr Phe
    1010                1015                1020
Lys His  Ala Thr Pro Glu Ala  Glu Leu Gly Asn Leu  Tyr Ile Gly
    1025                1030                1035
Ser Arg  Pro Ala Arg Arg Arg  Asn Lys Asp Ala Ser  Ile Ser Thr
    1040                1045                1050
Leu Arg  Ala Ile Pro Trp Ile  Phe Ala Trp Thr Gln  Asn Arg Leu
    1055                1060                1065
Ile Leu  Pro Ser Trp Leu Gly  Ile Gly Ala Ala Leu  Thr Ala Ala
    1070                1075                1080
Met Thr  Gln Gly His Leu Pro  Thr Leu Gln Ala Met  Tyr Arg Glu
    1085                1090                1095
Trp Pro  Phe Phe Gly Ser Thr  Val Asp Leu Ile Glu  Met Ile Leu
    1100                1105                1110
Ala Lys  Thr Asp Pro Arg Ile  Ala Ala Leu Tyr Glu  Glu Val Leu
    1115                1120                1125
Val Asn  Asp Pro Glu Glu Lys  Lys Leu Gly Ala Glu  Leu Arg Glu
    1130                1135                1140
Arg Leu  Gln Arg Cys Gln Gly  Ala Ile Leu Lys Val  Thr Gly His
    1145                1150                1155
Glu Asn  Leu Leu Ser Asn Asn  Pro Thr Leu Ser Lys  Leu Ile Ser
    1160                1165                1170
Met Arg  Ser Pro Phe Val Asp  Pro Ile Asn Ile Leu  Gln Val Glu
    1175                1180                1185
Val Leu  Arg Arg Leu Arg Gln  Asp Pro Asn Asn Met  Arg Leu Arg
    1190                1195                1200
Asp Ala  Leu Leu Ile Ser Ile  Asn Gly Ile Ala Ala  Gly Met Arg
    1205                1210                1215
Asn Thr  Gly
    1220
```

<210> 67
<211> 3099
<212> DNA
<213> Glycine max

<400> 67

```
atgactgaca tcactggtga tattgctgag gaaatctcct tccagagctt cgatgatgac      60
tgcaggttgc ttggtaatct cctcaatgac attctccagc gtgaagttgg caccaacttg     120
```

```
cttgacaaga tcgaaaggac tcgagtcctt gctcagagtg gttgtaatat gaggcaggcg    180
ggtattgtaa acatggcaga gatgcttgag aagcagttgg cttcggagtt atcaaagatg    240
acactagaag aagctttcac ccttgctcgt gccttcagcc attatcttac tttgatgggt    300
atagctgaga cccaccatag ggttcgtaaa ggagggaata tggcacaaat tgcaaaatct    360
tgcgatgata tatttaacca gctggtgcag ggtggagtcc ccccagaaga actttatgac    420
acagtctgca agcgggaggt tgaaattgtt ctcactgctc atcccacaca gattaaccgt    480
cgaaccttac agtttaaaca cattagaatt gctcatcttt tggattacaa tgatcgacct    540
gatcttagca ctgaagatcg agaaatggtg attgaagatc tggtgagaga gataacttca    600
atatggcaga cagatgagct taggcgccag aaacccactc cagttgatga agctagagct    660
ggtttcaata ttgtggagca gtcactctgg aaagctgtcc ctcattattt acgtcgtgtc    720
agcaatgcat taaagaagca tacaggaaag ccacttcctt tgacttgcac tcccataaag    780
tttggatctt ggatgggagg tgatagagat ggaaacccaa atgtgacagc aaaggtcaca    840
aaagatgttt cacttctatc tagatggatg gcgattgacc tctatattcg ggaagtggat    900
agcctcagat ttgagctatc catgaaccag tgcagtgata ggttgtcaag attggcacat    960
gaaattctag aagccaagca tgagaatcgc cgtgagaatt ggaatcagtc tgcgaataga   1020
agtctcacac ttccaacaca acttccagct agagctcatt taccttctat tgctgaaaat   1080
ggtgaatctc ggcatcccag actagacatt ccagcacctg attacatgca atccaatcac   1140
aaggatggtg gggtttctgt aagttcaact acatcaaaac ttgccaatcc caatactcga   1200
ttaccaggaa caagttcagc aaattccagt gcttcttcag ctgcacttgg tcaaaagaaa   1260
ttgtatgcag aatcccagac aggaaagtcc acttttcaaa agcttttgga gccaatgctt   1320
cctcaacttc ctggaattgc tccttataga attgtcctgg ggaatgttaa ggataagctt   1380
gagaaaagtc gtagacggtt agaaattctt cttgaggatg ttgcatgtga ctatgatcct   1440
ttggattact atgaaacatc tgatcagctt ttggaacctc tgctcctctg ttatgaatct   1500
ctgcaatcgt gtggatctgg ggtgctagct gatggtcgac ttgctgatct gattcgtaga   1560
gttgctacct ttggaatggt gttaatgaag cttgacttgc gtcaggaatc tgggagacat   1620
gcagaagcac ttgatgcaat aacacagtac ttagatatgg gtacttacag tgaatgggat   1680
gaagaaaaga agttggactt cttaacaaga gaacttaaag ggaagaggcc tcttgttcct   1740
gttagtatag aggttcatcc tgatgttaaa gaagtcttgg atacattccg aattgccgct   1800
gaactgggga gtgattcact tggagcttat gtgatctcta tggcctcaaa tgcaagtgat   1860
gtccttgcag tagagctttt acagaaggat gcacggcttg ctgctattgg ggagttggga   1920
aaagcatgtc ctggtggaac gttgcgggtt gtccctctgt ttgaaactgt gaaagacctg   1980
agaggagctg gttcagttat ccggaaactt ttatcaatag actggtacca tgaacacatc   2040
gttaagaacc ataatggaca tcaagaggtt atggttggat attctgattc tggtaaagat   2100
gctggtcgct ttactgctgc ttgggaactt tacaaagctc aggaggatgt tgtagctgct   2160
tgcaatgatt atggaataaa ggttactcta ttccatggtc gtggaggcag tattggtcgt   2220
ggtggtggcc caacatatct ggctattcag tcccaacccc ctggctctgt gatgggaacg   2280
cttcggtcta ctgagcaggg agagatggta gaggctaagt ttgggttgcc acagatagct   2340
gttagacaac ttgagatata cacaacagct gtactacttg caacccttcg tccacctatc   2400
ccaccccgag aagaaaaatg gcgtaatgtc atggaagaga tctcaaacat cagttgtcag   2460
tgtgaccgca atgtagtgta tgaaaatcca gaattcctgg cctacttcca tgaagccaca   2520
ccagaggcag aacttggctt ccttaacata ggtagccgcc ctacaagaag gaagagctca   2580
gtaggaatcg gacacccttcg tgcaattccc tggttatttg catggacaca aacaagattc   2640
gttcttccag cttggcttgg agtcggagca ggtttaaaag gagcttgcga gaaaggttac   2700
accgaagagc taaaagccat gtacaaagaa tggcccttct ttcaaagtac catagatctt   2760
attgagatgg ttttggggaa agctgacatt cctatagcca agcactatga tgaagtcctt   2820
gtgacaaagg agaggcaaga gcttggccat gaactaagaa gtgagctcat gacagctgaa   2880
aagtttgtca tggttattag tgggcacgag aaacttcagc agaataatag gagcttgagg   2940
aggctaattg agaatagact tcccttcctt aatcccttga acatgttgca ggtggagata   3000
ctcaagaggt taagacgtga tgatgacaac cgtaagatca gagatgcttt gcttatcacc   3060
ataaatggga ttgctgcagg gatgaagaat acaggttga                          3099
```

```
<210>  68
<211>  1032
<212>  PRT
<213>  Glycine max

<400>  68
Met Thr Asp Ile Thr Gly Asp Ile Ala Glu Glu Ile Ser Phe Gln Ser
1               5                   10                  15
Phe Asp Asp Asp Cys Arg Leu Leu Gly Asn Leu Leu Asn Asp Ile Leu
            20                  25                  30
Gln Arg Glu Val Gly Thr Asn Leu Leu Asp Lys Ile Glu Arg Thr Arg
        35                  40                  45
Val Leu Ala Gln Ser Gly Cys Asn Met Arg Gln Ala Gly Ile Val Asn
    50                  55                  60
Met Ala Glu Met Leu Glu Lys Gln Leu Ala Ser Glu Leu Ser Lys Met
65                  70                  75                  80
Thr Leu Glu Glu Ala Phe Thr Leu Ala Arg Ala Phe Ser His Tyr Leu
                85                  90                  95
Thr Leu Met Gly Ile Ala Glu Thr His His Arg Val Arg Lys Gly Gly
            100                 105                 110
Asn Met Ala Gln Ile Ala Lys Ser Cys Asp Asp Ile Phe Asn Gln Leu
        115                 120                 125
Val Gln Gly Gly Val Pro Pro Glu Glu Leu Tyr Asp Thr Val Cys Lys
```

```
        130                 135                 140
Arg Glu Val Glu Ile Val Leu Thr Ala His Pro Thr Gln Ile Asn Arg
145                 150                 155                 160
Arg Thr Leu Gln Phe Lys His Ile Arg Ile Ala His Leu Leu Asp Tyr
                165                 170                 175
Asn Asp Arg Pro Asp Leu Ser Thr Glu Asp Arg Glu Met Val Ile Glu
            180                 185                 190
Asp Leu Val Arg Glu Ile Thr Ser Ile Trp Gln Thr Asp Glu Leu Arg
        195                 200                 205
Arg Gln Lys Pro Thr Pro Val Asp Glu Ala Arg Ala Gly Phe Asn Ile
    210                 215                 220
Val Glu Gln Ser Leu Trp Lys Ala Val Pro His Tyr Leu Arg Arg Val
225                 230                 235                 240
Ser Asn Ala Leu Lys Lys His Thr Gly Lys Pro Leu Pro Leu Thr Cys
                245                 250                 255
Thr Pro Ile Lys Phe Gly Ser Trp Met Gly Gly Asp Arg Asp Gly Asn
            260                 265                 270
Pro Asn Val Thr Ala Lys Val Thr Lys Asp Val Ser Leu Leu Ser Arg
        275                 280                 285
Trp Met Ala Ile Asp Leu Tyr Ile Arg Glu Val Asp Ser Leu Arg Phe
    290                 295                 300
Glu Leu Ser Met Asn Gln Cys Ser Asp Arg Leu Ser Arg Leu Ala His
305                 310                 315                 320
Glu Ile Leu Glu Ala Lys His Glu Asn Arg Arg Glu Asn Trp Asn Gln
                325                 330                 335
Ser Ala Asn Arg Ser Leu Thr Leu Pro Thr Gln Leu Pro Ala Arg Ala
            340                 345                 350
His Leu Pro Ser Ile Ala Glu Asn Gly Glu Ser Arg His Pro Arg Leu
        355                 360                 365
Asp Ile Pro Ala Pro Asp Tyr Met Gln Ser Asn His Lys Asp Gly Gly
    370                 375                 380
Val Ser Val Ser Ser Thr Thr Ser Lys Leu Ala Asn Pro Asn Thr Arg
385                 390                 395                 400
Leu Pro Gly Thr Ser Ser Ala Asn Ser Ser Ala Ser Ser Ala Ala Leu
                405                 410                 415
Gly Gln Lys Lys Leu Tyr Ala Glu Ser Gln Thr Gly Lys Ser Thr Phe
            420                 425                 430
Gln Lys Leu Leu Glu Pro Met Leu Pro Gln Leu Pro Gly Ile Ala Pro
        435                 440                 445
Tyr Arg Ile Val Leu Gly Asn Val Lys Asp Lys Leu Glu Lys Ser Arg
    450                 455                 460
Arg Arg Leu Glu Ile Leu Leu Glu Asp Val Ala Cys Asp Tyr Asp Pro
465                 470                 475                 480
Leu Asp Tyr Tyr Glu Thr Ser Asp Gln Leu Leu Glu Pro Leu Leu Leu
                485                 490                 495
Cys Tyr Glu Ser Leu Gln Ser Cys Gly Ser Gly Val Leu Ala Asp Gly
            500                 505                 510
Arg Leu Ala Asp Leu Ile Arg Arg Val Ala Thr Phe Gly Met Val Leu
        515                 520                 525
Met Lys Leu Asp Leu Arg Gln Glu Ser Gly Arg His Ala Glu Ala Leu
    530                 535                 540
Asp Ala Ile Thr Gln Tyr Leu Asp Met Gly Thr Tyr Ser Glu Trp Asp
545                 550                 555                 560
Glu Glu Lys Lys Leu Asp Phe Leu Thr Arg Glu Leu Lys Gly Lys Arg
                565                 570                 575
Pro Leu Val Pro Val Ser Ile Glu Val His Pro Asp Val Lys Glu Val
            580                 585                 590
Leu Asp Thr Phe Arg Ile Ala Ala Glu Leu Gly Ser Asp Ser Leu Gly
        595                 600                 605
Ala Tyr Val Ile Ser Met Ala Ser Asn Ala Ser Asp Val Leu Ala Val
    610                 615                 620
Glu Leu Leu Gln Lys Asp Ala Arg Leu Ala Ala Ile Gly Glu Leu Gly
625                 630                 635                 640
Lys Ala Cys Pro Gly Gly Thr Leu Arg Val Val Pro Leu Phe Glu Thr
                645                 650                 655
Val Lys Asp Leu Arg Gly Ala Gly Ser Val Ile Arg Lys Leu Leu Ser
            660                 665                 670
Ile Asp Trp Tyr His Glu His Ile Val Lys Asn His Asn Gly His Gln
        675                 680                 685
Glu Val Met Val Gly Tyr Ser Asp Ser Gly Lys Asp Ala Gly Arg Phe
    690                 695                 700
Thr Ala Ala Trp Glu Leu Tyr Lys Ala Gln Glu Asp Val Val Ala Ala
705                 710                 715                 720
Cys Asn Asp Tyr Gly Ile Lys Val Thr Leu Phe His Gly Arg Gly Gly
```

```
                725                 730                 735
Ser Ile Gly Arg Gly Gly Gly Pro Thr Tyr Leu Ala Ile Gln Ser Gln
            740                 745                 750
Pro Pro Gly Ser Val Met Gly Thr Leu Arg Ser Thr Glu Gln Gly Glu
        755                 760                 765
Met Val Glu Ala Lys Phe Gly Leu Pro Gln Ile Ala Val Arg Gln Leu
    770                 775                 780
Glu Ile Tyr Thr Thr Ala Val Leu Leu Ala Thr Leu Arg Pro Pro Ile
785                 790                 795                 800
Pro Pro Arg Glu Glu Lys Trp Arg Asn Val Met Glu Glu Ile Ser Asn
                805                 810                 815
Ile Ser Cys Gln Cys Asp Arg Asn Val Val Tyr Glu Asn Pro Glu Phe
            820                 825                 830
Leu Ala Tyr Phe His Glu Ala Thr Pro Glu Ala Glu Leu Gly Phe Leu
        835                 840                 845
Asn Ile Gly Ser Arg Pro Thr Arg Arg Lys Ser Ser Val Gly Ile Gly
    850                 855                 860
His Leu Arg Ala Ile Pro Trp Leu Phe Ala Trp Thr Gln Thr Arg Phe
865                 870                 875                 880
Val Leu Pro Ala Trp Leu Gly Val Gly Ala Gly Leu Lys Gly Ala Cys
                885                 890                 895
Glu Lys Gly Tyr Thr Glu Glu Leu Lys Ala Met Tyr Lys Glu Trp Pro
            900                 905                 910
Phe Phe Gln Ser Thr Ile Asp Leu Ile Glu Met Val Leu Gly Lys Ala
        915                 920                 925
Asp Ile Pro Ile Ala Lys His Tyr Asp Glu Val Leu Val Thr Lys Glu
    930                 935                 940
Arg Gln Glu Leu Gly His Glu Leu Arg Ser Glu Leu Met Thr Ala Glu
945                 950                 955                 960
Lys Phe Val Met Val Ile Ser Gly His Glu Lys Leu Gln Gln Asn Asn
                965                 970                 975
Arg Ser Leu Arg Arg Leu Ile Glu Asn Arg Leu Pro Phe Leu Asn Pro
            980                 985                 990
Leu Asn Met Leu Gln Val Glu Ile  Leu Lys Arg Leu Arg  Arg Asp Asp
        995                 1000                1005
Asp Asn  Arg Lys Ile Arg Asp  Ala Leu Leu Ile Thr  Ile Asn Gly
    1010                1015                1020
Ile Ala  Ala Gly Met Lys Asn  Thr Gly
    1025                1030
```

<210> 69
<211> 3159
<212> DNA
<213> Ricinus communis

<400> 69

```
atgacggaca ccacagatga tattgcagag gaaatctcat ttcagagttt tgatgatgat     60
tgtaagcttc ttggcaattt gttaaacgat gttttgcaga gagaagttgg ttctaaattc    120
atggagaagc ttgaacgcaa tcgcatccta gctcagagtg cttgtaatat gagattggcg    180
gggatagaag ataccgctga attgctggag aagcagctgg cattggagat atcaaggatg    240
acattagagg aagcattgac acttgctcgt gcctttagtc attaccttaa tttgatgggc    300
attgctgaga cccatcacag ggttcgtaag gcacggagta tgacacatct atcaaaatct    360
tgtgatgaca tatttaatca gctgctgcag agtggcatat ctgcagagga gctttatgac    420
acagtttgca agcaggaggt tgaaattgtg ctcactgcac atcctactca aattaatcgt    480
cgtaccttgc aatataagca catcagaatt gctcatcttt tagattataa tgaccggcct    540
gatcttactc atgaagatcg agaaatgctg attgaagatc tggtgagaga aattacttca    600
atatggcaga cagatgagct taggcgccac aagcctacac cagtagatga agctagggct    660
ggcttgaaca ttgtggagca gtccctgtgg aaagctcttc ccattattt acgtcgtgtc    720
agtactgccc taaagaagca tacaggaaag ccacttcctt tgacttgcac gccaataagg    780
tttgggtctt ggatgggggg tgatagagat ggtaatccga atgtaacagc aaaggtcaca    840
agagatgttt ctcttttatc taggtggatg gctgttgacc tttacattcg agaagttgat    900
agcctgagat ttgaactatc catggttcaa tgcagtgata gattgttgaa agtggcaaat    960
gacattttaa tagaagaaac ttcatctgaa gatcaccatg agagttggaa tcaacctgca   1020
agcagaagtc aaacaaagtt tcctagaaaa tctcttccta cacaacttcc acctagagct   1080
gatcttcctg cttgcactga atgtaatgat ggtgaatctc agtatcccaa actagaactt   1140
ccaggaactg attacatgcc ctttaatcgc caggaagctc taggttcttc atattcagaa   1200
tcttcatccc aggatatcaa tcatggttta cctaaaacaa ctggaaatgg aagtgttgct   1260
aattctagtg gatctccgcg ggcatctttc agctctgctc aactggttgc acagaggaaa   1320
ctatttgcag aatccaagat aggaagatct agcttccaga agcttctaga accaagtctg   1380
cctcaacgtc ctggaattgc tccttataga attgttcttg gaaatgtaaa agataagctt   1440
atgaggacaa gaagacgtct ggaacttctt ctagaggatc ttccttgtga atatgatcaa   1500
tgggattact atgagacaac agatcaatta ttggatccac tgcttctgtg ctacgagtct   1560
cttcaatcat gtggagctgg ggttctagct gatggtcggc tagctgacct gataagaaga   1620
gttgctacat ttgggatggt gttaatgaag ctagacttgc gccaggaatc tggtagacat   1680
```

```
gctgatactc ttgatgcaat caccaaatat ttggaaatgg gcacgtatag tgagtgggat   1740
gaagaaaaga agctggaatt tctgacaaga gaactgaaag gaaaacggcc actggttcct   1800
cctactattg aggttgctcc tgatgttaaa gaagtcttgg atgctttccg tgttgctgct   1860
gagctgggga gtgattcact tggagcttat gtgatttcta tggcatccaa tgcaagtgat   1920
gtccttgctg tggagcttct gcagaaggat gcccggcttg ctgttagtgg ggagctagga   1980
agaccatgcc ctggtggaac gttgcgagta gttccgttgt ttgaaactgt gaaagacctg   2040
agaggtgctg gttcggtgat cagaaaactg ttatcaattg actggtatag agaacacata   2100
attaagaacc ataacgggca tcaggaggtg atggttggct attctgattc tggtaaagat   2160
gctggacgct ttactgctgc atgggaactt tacaaagccc aagaggatgt tgtggcagcc   2220
tgtaatgatt ttggtatcaa agtaacacta ttccatgggc gtggagggag tattggtcgt   2280
ggtggtggcc ccacatatct tgccattcaa tcccaaccac ctggttcagt tatgggtact   2340
cttcggtcaa ctgagcaagg agaaatggtg caggccaagt ttgggctgcc acatactgcc   2400
atcagacaac tagagatata cacaactgct gtgctgcttg caacccttcg tcctccacat   2460
ccacctcgag aagaacaatg gcgcaatgtc atggaggaga tttcaaaaat cagttgccag   2520
aattaccgga gcacagtcta tgaaaaccca gaattcctcg cctacttcca tgaggctact   2580
ccccaggctg agcttggctt tcttaacata ggaagccgcc ccacaagaag aaagagctca   2640
actggtattg gacatcttcg tgccattcca tgggtattcg catggaccca gaccagattt   2700
gttcttcctg cttggcttgg agttggagca ggtttaaagg gtgcttgtga gaagggattt   2760
actgaagact tgaaagcaat gtacaaagaa tggcctttct tccaatctac tattgatctt   2820
atagagatgg tactagggaa ggcagacatt cctatagcca agcactatga tgaagttctt   2880
gtatctgaga gcaggcgaga gcttggagct gagcttagga gtgagctctt aacaacagaa   2940
aagtatgtgt tggtggttag tgggcatgag aaactatctc agaacaaccg cagcctgcgg   3000
aggctaatag agagcaggct gccttatctc aatcctatga atatgttgca ggtggaggtt   3060
ctaaagaggt tgaggcggga cgacgacaac aataagctca gagatgccct gcttattacc   3120
ataaatggga ttgctgctgg aatgaggaac acaggctga                          3159
```

<210> 70
<211> 1052
<212> PRT
<213> Ricinus communis

```
<400> 70
Met Thr Asp Thr Thr Asp Asp Ile Ala Glu Glu Ile Ser Phe Gln Ser
1               5                   10                  15
Phe Asp Asp Asp Cys Lys Leu Leu Gly Asn Leu Leu Asn Asp Val Leu
            20                  25                  30
Gln Arg Glu Val Gly Ser Lys Phe Met Glu Lys Leu Glu Arg Asn Arg
        35                  40                  45
Ile Leu Ala Gln Ser Ala Cys Asn Met Arg Leu Ala Gly Ile Glu Asp
    50                  55                  60
Thr Ala Glu Leu Leu Glu Lys Gln Leu Ala Leu Glu Ile Ser Arg Met
65                  70                  75                  80
Thr Leu Glu Glu Ala Leu Thr Leu Ala Arg Ala Phe Ser His Tyr Leu
                85                  90                  95
Asn Leu Met Gly Ile Ala Glu Thr His His Arg Val Arg Lys Ala Arg
            100                 105                 110
Ser Met Thr His Leu Ser Lys Ser Cys Asp Asp Ile Phe Asn Gln Leu
        115                 120                 125
Leu Gln Ser Gly Ile Ser Ala Glu Glu Leu Tyr Asp Thr Val Cys Lys
    130                 135                 140
Gln Glu Val Glu Ile Val Leu Thr Ala His Pro Thr Gln Ile Asn Arg
145                 150                 155                 160
Arg Thr Leu Gln Tyr Lys His Ile Arg Ile Ala His Leu Leu Asp Tyr
                165                 170                 175
Asn Asp Arg Pro Asp Leu Thr His Glu Asp Arg Glu Met Leu Ile Glu
            180                 185                 190
Asp Leu Val Arg Glu Ile Thr Ser Ile Trp Gln Thr Asp Glu Leu Arg
        195                 200                 205
Arg His Lys Pro Thr Pro Val Asp Glu Ala Arg Ala Gly Leu Asn Ile
    210                 215                 220
Val Glu Gln Ser Leu Trp Lys Ala Leu Pro His Tyr Leu Arg Arg Val
225                 230                 235                 240
Ser Thr Ala Leu Lys Lys His Thr Gly Lys Pro Leu Pro Leu Thr Cys
                245                 250                 255
Thr Pro Ile Arg Phe Gly Ser Trp Met Gly Gly Asp Arg Asp Gly Asn
            260                 265                 270
Pro Asn Val Thr Ala Lys Val Thr Arg Asp Val Ser Leu Leu Ser Arg
        275                 280                 285
Trp Met Ala Val Asp Leu Tyr Ile Arg Glu Val Asp Ser Leu Arg Phe
    290                 295                 300
Glu Leu Ser Met Val Gln Cys Ser Asp Arg Leu Leu Lys Val Ala Asn
305                 310                 315                 320
Asp Ile Leu Ile Glu Glu Thr Ser Ser Glu Asp His His Glu Ser Trp
                325                 330                 335
```

```
Asn Gln Pro Ala Ser Arg Ser Gln Thr Lys Phe Pro Arg Lys Ser Leu
            340                 345                 350
Pro Thr Gln Leu Pro Pro Arg Ala Asp Leu Pro Ala Cys Thr Glu Cys
        355                 360                 365
Asn Asp Gly Glu Ser Gln Tyr Pro Lys Leu Glu Leu Pro Gly Thr Asp
    370                 375                 380
Tyr Met Pro Phe Asn Arg Gln Glu Ala Leu Gly Ser Ser Tyr Ser Glu
385                 390                 395                 400
Ser Ser Ser Gln Asp Ile Asn His Gly Leu Pro Lys Thr Thr Gly Asn
                405                 410                 415
Gly Ser Val Ala Asn Ser Ser Gly Ser Pro Arg Ala Ser Phe Ser Ser
            420                 425                 430
Ala Gln Leu Val Ala Gln Arg Lys Leu Phe Ala Glu Ser Lys Ile Gly
        435                 440                 445
Arg Ser Ser Phe Gln Lys Leu Leu Glu Pro Ser Leu Pro Gln Arg Pro
    450                 455                 460
Gly Ile Ala Pro Tyr Arg Ile Val Leu Gly Asn Val Lys Asp Lys Leu
465                 470                 475                 480
Met Arg Thr Arg Arg Arg Leu Glu Leu Leu Leu Glu Asp Leu Pro Cys
                485                 490                 495
Glu Tyr Asp Gln Trp Asp Tyr Tyr Glu Thr Thr Asp Gln Leu Leu Asp
            500                 505                 510
Pro Leu Leu Leu Cys Tyr Glu Ser Leu Gln Ser Cys Gly Ala Gly Val
        515                 520                 525
Leu Ala Asp Gly Arg Leu Ala Asp Leu Ile Arg Arg Val Ala Thr Phe
    530                 535                 540
Gly Met Val Leu Met Lys Leu Asp Leu Arg Gln Glu Ser Gly Arg His
545                 550                 555                 560
Ala Asp Thr Leu Asp Ala Ile Thr Lys Tyr Leu Glu Met Gly Thr Tyr
                565                 570                 575
Ser Glu Trp Asp Glu Glu Lys Lys Leu Glu Phe Leu Thr Arg Glu Leu
            580                 585                 590
Lys Gly Lys Arg Pro Leu Val Pro Pro Thr Ile Glu Val Ala Pro Asp
        595                 600                 605
Val Lys Glu Val Leu Asp Ala Phe Arg Val Ala Ala Glu Leu Gly Ser
    610                 615                 620
Asp Ser Leu Gly Ala Tyr Val Ile Ser Met Ala Ser Asn Ala Ser Asp
625                 630                 635                 640
Val Leu Ala Val Glu Leu Leu Gln Lys Asp Ala Arg Leu Ala Val Ser
                645                 650                 655
Gly Glu Leu Gly Arg Pro Cys Pro Gly Gly Thr Leu Arg Val Val Pro
            660                 665                 670
Leu Phe Glu Thr Val Lys Asp Leu Arg Gly Ala Gly Ser Val Ile Arg
        675                 680                 685
Lys Leu Leu Ser Ile Asp Trp Tyr Arg Glu His Ile Ile Lys Asn His
    690                 695                 700
Asn Gly His Gln Glu Val Met Val Gly Tyr Ser Asp Ser Gly Lys Asp
705                 710                 715                 720
Ala Gly Arg Phe Thr Ala Ala Trp Glu Leu Tyr Lys Ala Gln Glu Asp
                725                 730                 735
Val Val Ala Ala Cys Asn Asp Phe Gly Ile Lys Val Thr Leu Phe His
            740                 745                 750
Gly Arg Gly Gly Ser Ile Gly Arg Gly Gly Gly Pro Thr Tyr Leu Ala
        755                 760                 765
Ile Gln Ser Gln Pro Pro Gly Ser Val Met Gly Thr Leu Arg Ser Thr
    770                 775                 780
Glu Gln Gly Glu Met Val Gln Ala Lys Phe Gly Leu Pro His Thr Ala
785                 790                 795                 800
Ile Arg Gln Leu Glu Ile Tyr Thr Thr Ala Val Leu Leu Ala Thr Leu
                805                 810                 815
Arg Pro Pro His Pro Pro Arg Glu Glu Gln Trp Arg Asn Val Met Glu
            820                 825                 830
Glu Ile Ser Lys Ile Ser Cys Gln Asn Tyr Arg Ser Thr Val Tyr Glu
        835                 840                 845
Asn Pro Glu Phe Leu Ala Tyr Phe His Glu Ala Thr Pro Gln Ala Glu
    850                 855                 860
Leu Gly Phe Leu Asn Ile Gly Ser Arg Pro Thr Arg Arg Lys Ser Ser
865                 870                 875                 880
Thr Gly Ile Gly His Leu Arg Ala Ile Pro Trp Val Phe Ala Trp Thr
                885                 890                 895
Gln Thr Arg Phe Val Leu Pro Ala Trp Leu Gly Val Gly Ala Gly Leu
            900                 905                 910
Lys Gly Ala Cys Glu Lys Gly Phe Thr Glu Asp Leu Lys Ala Met Tyr
        915                 920                 925
```

```
Lys Glu Trp Pro Phe Phe Gln Ser Thr Ile Asp Leu Ile Glu Met Val
    930                 935                 940
Leu Gly Lys Ala Asp Ile Pro Ile Ala Lys His Tyr Asp Glu Val Leu
945                 950                 955                 960
Val Ser Glu Ser Arg Arg Glu Leu Gly Ala Glu Leu Arg Ser Glu Leu
                965                 970                 975
Leu Thr Thr Glu Lys Tyr Val Leu Val Val Ser Gly His Glu Lys Leu
            980                 985                 990
Ser Gln Asn Asn Arg Ser Leu Arg  Arg Leu Ile Glu Ser  Arg Leu Pro
        995                 1000                1005
Tyr Leu  Asn Pro Met Asn Met  Leu Gln Val Glu Val  Leu Lys Arg
    1010                 1015                1020
Leu Arg  Arg Asp Asp Asp Asn  Asn Lys Leu Arg Asp  Ala Leu Leu
    1025                 1030                1035
Ile Thr  Ile Asn Gly Ile Ala  Ala Gly Met Arg Asn  Thr Gly
    1040                 1045                1050
```

<210> 71
<211> 1568
<212> DNA
<213> Oryza sativa

<400> 71

```
cccacgcgtc cgcccacgcg tccgggacac cagaaacata gtacacttga gctcactcca     60
aactcaaaca ctcacaccaa tggctctcca agttcaggcc gcactcctgc cctctgctct    120
ctctgtcccc aagaagggta acttgagcgc ggtggtgaag gagccggggt tccttagcgt    180
gagcagaagg ccaagaagcc gtcgctggtg gtgagggcgg tggcgacgcg gcgggccggt    240
ggcgagcccc ggcgcgggca cgtcgaaggc ggacgggaag aagacgctgc ggcagggggt    300
ggtggtgatc accggcgcgt cgtcggggct cgggctcgcg gcggcgaagg cgcttggcgg    360
agacggggaa gtggcacgtg gtgatggcgt tccgcgactt tcctgaaggc ggcgacggcg    420
gcgaaggcgg cggggatggc ggcggggagc tacaccgtca tgcacctgga cctcgcctcc    480
ctcgacagcg tccgccagtt cgtggacaac ttccggcgct ccggcatgcc gctcgacgcg    540
ctggtgtgca acgccgcaca tctaccggcc gacggcgcgg caaccgacgt tcaacgccga    600
cgggtacgag atgagcgtcg ggtgaacca cctgggccac ttcctcctcg cccgcctcat    660
gctcgacgac ctcaagaaat ccgactaccc gtcgcggcgg ctcatcatcc tcggctccat    720
caccggcaac accaacacct tcgccggcaa cgtccctccc aaggccgggc taggcgacct    780
ccgggggctc gccggcgggc tccgcgggca gaacgggtcg gcgatgatcg acggcgcgga    840
gagcttcgac ggcgccaagg cgtacaagga cagcaagatc tgtaacatgc tgacgatgca    900
ggagttccac cggagattcc acgaggagac cgggatcacg ttcgcgtcgc tgtacccggg    960
gtgcatcgcg acgacgggct tgttccgcga gcacatcccg ctgttccggc tgctgttccc   1020
gccgttccag cggttcgtga cgaaggggtt cgtgtcggag gcggagtccg ggaagcggct   1080
ggcgcaggtg gtgggcgacc cgagcctgac caagtccggc gtgtactgga gctggaacaa   1140
ggactcggcg tcgttcgaga accagctctc gcaggaggcc agcgacccgg agaaggccag   1200
gaagctctgg gacctcagcg agaagctcgt cggcctcgtc tgagtttatt atttacccat   1260
tcgtttcaac tgttaatttc ttcggggttt agggggtttc agctttcagt gagagaggcc   1320
tgtcaagtga tgtacaatta gtaatttttt tttacccgac aaatcatgca ataaaaccac   1380
aggcttacat tatcgatttg tccacctaaa ttaagtttca actgttaatt tcttcggggt   1440
ttagggggtt tcagctttca gtgagagagg cctgtcaagt gatgtacaat tagtaatttt   1500
tttttacccg acaaatcatg caataaaacc acaggcttac attatcgatt tgtccaccta   1560
aattaagt                                                             1568
```

<210> 72
<211> 53
<212> DNA
<213> Artificial sequence

<220>
<223> primer: prm08763

<400> 72

```
ggggacaagt ttgtacaaaa aagcaggctt aaacaatgaa cttggcagtt cct            53
```

<210> 73
<211> 48
<212> DNA
<213> Artificial sequence

<220>
<223> primer: prm08764

<400> 73

```
ggggaccact ttgtacaaga aagctgggtt caaccagtat tacgcatt                  48
```

```
<210>  74
<211>  1295
<212>  DNA
<213>  Capsicum annuum

<400>  74
gccaactttg tacaaaaaag cagtcttaaa caatggaaga aattcaagta ccaccttatt      60
tcctctgtcc gatttcactg gagatgatga aagatccagt cacgatctcg actggaataa     120
catacgatcg agagaacatc gagagatgga tattctcagc taagaacaat acgtgtccgg     180
ttacgaagca atcccttacg agcatagagt tgacccctaa tgtcactcta cgacgattca     240
tccaatcgtg gtgtactctc aatgcgtccc atggcatcga gaggttccct acaccgaagc     300
ctccggtcag caaagctcaa atcttaaagc tcatgaaaga agcgaaatcg cgcgaaatgc     360
aaatgaaatc actcaacaca cttagatcca tagcttctgt gaacgatgct aacaagcgtt     420
gcatggagtc tgcgggcgca gcggagttct tagcctctgt tgtgattgat tcgagtgatg     480
tgttgggtga agagggtttc atgagtacta aagatgaagc attaagcatc ctctaccaac     540
tcaagttatc agaagatgga ttgaggtcac tcatcacgag tggaaatggt gaattcatcg     600
agtcactgac acgcgtcatg cagcgtggga gctatgagtc aagggcttac gcggtgatgc     660
tgatgaaaga catgttcgaa gcatccacac taccaactct attttcgagc ttgaagaaag     720
agttcttcgc tcaagtggtt cgagtcttga gggatgagat ctctcaaaag gccaccaaag     780
cgtcattgca agtgctagcg cacgcgtgtc catttgggag gaacagagtg aaagcagctg     840
aggcaggagc cgttagggtt ttggtggacc ttctgctcga ttcatccgag aaaaggacct     900
gcgaactgat gctaatcttg ctggatcaaa tttgtcagag cgcagaaggg agagccgagc     960
tgttgaatca tccgggaggg ttagccatcg tctcgaagaa aatacttagg gtttcgaaag    1020
tagggagcga aagggcaatc aagatattgc attcgatatc aaagttttca tcaacacaaa    1080
gtgttgttca agagatgttg agtttgggtg ttgtggcaaa gctttgtttg gtacttcaag    1140
ttgattgtgg aagtaaagct aaggatagag ctagagatat tctcaaaatt catgcaaagg    1200
catggaggaa ttctccttgt atacctatga atttattatc ttcatatcca ttttaggaaa    1260
ataggaccaa aagagcaccc agctttcttg tacaa                               1295

<210>  75
<211>  407
<212>  PRT
<213>  Capsicum annuum

<400>  75
Met Glu Glu Ile Gln Val Pro Pro Tyr Phe Leu Cys Pro Ile Ser Leu
1               5                   10                  15
Glu Met Met Lys Asp Pro Val Thr Ile Ser Thr Gly Ile Thr Tyr Asp
            20                  25                  30
Arg Glu Asn Ile Glu Arg Trp Ile Phe Ser Ala Lys Asn Asn Thr Cys
        35                  40                  45
Pro Val Thr Lys Gln Ser Leu Thr Ser Ile Glu Leu Thr Pro Asn Val
    50                  55                  60
Thr Leu Arg Arg Phe Ile Gln Ser Trp Cys Thr Leu Asn Ala Ser His
65                  70                  75                  80
Gly Ile Glu Arg Phe Pro Thr Pro Lys Pro Pro Val Ser Lys Ala Gln
                85                  90                  95
Ile Leu Lys Leu Met Lys Glu Ala Lys Ser Arg Glu Met Gln Met Lys
            100                 105                 110
Ser Leu Asn Thr Leu Arg Ser Ile Ala Ser Val Asn Asp Ala Asn Lys
        115                 120                 125
Arg Cys Met Glu Ser Ala Gly Ala Ala Glu Phe Leu Ala Ser Val Val
    130                 135                 140
Ile Asp Ser Ser Asp Val Leu Gly Glu Glu Gly Phe Met Ser Thr Lys
145                 150                 155                 160
Asp Glu Ala Leu Ser Ile Leu Tyr Gln Leu Lys Leu Ser Glu Asp Gly
                165                 170                 175
Leu Arg Ser Leu Ile Thr Ser Gly Asn Gly Glu Phe Ile Glu Ser Leu
            180                 185                 190
Thr Arg Val Met Gln Arg Gly Ser Tyr Glu Ser Arg Ala Tyr Ala Val
        195                 200                 205
Met Leu Met Lys Asp Met Phe Glu Ala Ser Thr Leu Pro Thr Leu Phe
    210                 215                 220
Ser Ser Leu Lys Lys Glu Phe Phe Ala Gln Val Val Arg Val Leu Arg
225                 230                 235                 240
Asp Glu Ile Ser Gln Lys Ala Thr Lys Ala Ser Leu Gln Val Leu Ala
                245                 250                 255
His Ala Cys Pro Phe Gly Arg Asn Arg Val Lys Ala Ala Glu Ala Gly
            260                 265                 270
Ala Val Arg Val Leu Val Asp Leu Leu Leu Asp Ser Ser Glu Lys Arg
        275                 280                 285
Thr Cys Glu Leu Met Leu Ile Leu Leu Asp Gln Ile Cys Gln Ser Ala
    290                 295                 300
Glu Gly Arg Ala Glu Leu Leu Asn His Pro Gly Gly Leu Ala Ile Val
```

```
305                 310                 315                 320
Ser Lys Lys Ile Leu Arg Val Ser Lys Val Gly Ser Glu Arg Ala Ile
                325                 330                 335
Lys Ile Leu His Ser Ile Ser Lys Phe Ser Ser Thr Gln Ser Val Val
            340                 345                 350
Gln Glu Met Leu Ser Leu Gly Val Val Ala Lys Leu Cys Leu Val Leu
        355                 360                 365
Gln Val Asp Cys Gly Ser Lys Ala Lys Asp Arg Ala Arg Asp Ile Leu
    370                 375                 380
Lys Ile His Ala Lys Ala Trp Arg Asn Ser Pro Cys Ile Pro Met Asn
385                 390                 395                 400
Leu Leu Ser Ser Tyr Pro Phe
                405
```

```
<210>  76
<211>  65
<212>  PRT
<213>  Artificial sequence

<220>
<223>  U-box domain

<400>  76
Phe Leu Cys Pro Ile Ser Leu Glu Met Met Lys Asp Pro Val Thr Ile
1               5                   10                  15
Ser Thr Gly Ile Thr Tyr Asp Arg Glu Asn Ile Glu Arg Trp Ile Phe
            20                  25                  30
Ser Ala Lys Asn Asn Thr Cys Pro Val Thr Lys Gln Ser Leu Thr Ser
        35                  40                  45
Ile Glu Leu Thr Pro Asn Val Thr Leu Arg Arg Phe Ile Gln Ser Trp
    50                  55                  60
Cys
65
```

```
<210>  77
<211>  14
<212>  PRT
<213>  Artificial sequence

<220>
<223>  motif 1

<400>  77
Gly Ile Thr Tyr Asp Arg Glu Asn Ile Glu Arg Trp Ile Phe
1               5                   10
```

```
<210>  78
<211>  8
<212>  PRT
<213>  Artificial sequence

<220>
<223>  motif 2

<400>  78
Gln Ser Trp Cys Thr Leu Asn Ala
1               5
```

```
<210>  79
<211>  8
<212>  PRT
<213>  Artificial sequence

<220>
<223>  motif 3

<400>  79
Gly Arg Ala Glu Leu Leu Asn His
1               5
```

```
<210>  80
<211>  13
<212>  PRT
<213>  Artificial sequence
```

<220>
<223> motif 4

<400> 80
Gly Leu Ala Ile Val Ser Lys Lys Ile Leu Arg Val Ser
1               5                   10

<210> 81
<211> 9
<212> PRT
<213> Artificial sequence

<220>
<223> motif 5

<400> 81
Lys Leu Cys Leu Val Leu Gln Val Asp
1               5

<210> 82
<211> 3
<212> PRT
<213> Artificial sequence

<220>
<223> motif 6

<400> 82
Ser Tyr Pro
1

<210> 83
<211> 1619
<212> DNA
<213> Arabidopsis thaliana

<400> 83
ctaacttatt caaaccaatt cacatcttcc caaacccaac tactacaact tgtattaaga 60
aaaagatata ttcccttagc ttctttgatc aatatattcg tcagggttct cgtcaaagtc 120
ctcagcatct tcatcatatg tccggaggaa taatggatga agagatcgag attcctccgt 180
tcttcctttg tcctatctct ttggagatca tgaaagatcc agtgatagtc tctaccggaa 240
taacctacga cagagacagc atcgagaaat ggctatttgc aggcaagaaa aactcgtgtc 300
cggtcaccaa acaagacata accgacgcgg atctcacccc gaatcacact ctacgccgtc 360
tcatccaatc ttggtgcacc ctaaacgcct cctacggcgt cgagaggatc cctaccccaa 420
gacctccgat ttgtaaatct gagatcgaaa agctcattag agattcagcc tcttcccatg 480
aaaaccaagt caagtgtctc aaacgacttc gtcagattgt gtcggagaac gcgaccaaca 540
agcggtgttt agaggcagcg ggagtaccgg agttcttggc caacatcgta agcaacgact 600
cagaaaatgg gagtttgacc gacgaagccc tcaacttact ttaccacctc gagacctcag 660
agacagtcct caagaatctt ttaaacaaca agaaagataa caatatcgta aagtcgttga 720
cgaagatcat gcagcgtggg atgtacgagt ccagagtcta tgccactttg cttctcaaga 780
acattctcga agtagcggat ccaatgcaga gtatgactct caagccagag gttttcactg 840
aggtcgtcca gatcttggac gaccggatct cgcagaaggc gaccaaagct gccatgcata 900
tattggtgaa catatgccca tggggaagga acagacacaa ggccgtggaa gctggagtaa 960
tctctgtgat catcgagctt ctcatggacg agagcttcac atcagagagg agaggtccag 1020
agatggcgat ggtggttctt gatctgttgt gtcagtgtgc ggagggacga gccgagttct 1080
tgaaccacgg agcagccata gcggtggtgt gcaagaagat acttagggtt tcacagacag 1140
caagcgatag agcggttagg gttttgttgt cggtgggaag gttctgcgca acgcctgctt 1200
tgttgcacga gatgttacag ttggggggttg tagcgaagct ttgtcttgtg cttcaagtaa 1260
gctgtggagg taagaccaaa gagaaggcaa aggagttgct taagttgcac gctagggtct 1320
ggaaggactc gccttgtctc ccaaaaaaca tgattcttgc atatccctgc tgatgaaaac 1380
gaagaaaaca atacaaacaa gtccaattct cagtgagagc aatcacatat atgagagatg 1440
ctgttgaaaa aactaaagga gtattcaatt attcatatca ttaatccaat tttccttagg 1500
tttcttattt cgtacttttg atgttctcaa tgtagataat agtggaaaat gttcatcaaa 1560
tattttttt tatgaaaaac tgcaaagata tcatatataa aatcacaagt taccaggct 1619

<210> 84
<211> 435
<212> PRT
<213> Arabidopsis thaliana

<400> 84
Met Asp Gln Glu Ile Glu Ile Pro Ser Phe Phe Leu Cys Pro Ile Ser
1               5                   10                  15

```
Leu Asp Ile Met Lys Asp Pro Val Ile Val Ser Thr Gly Ile Thr Tyr
            20                  25                  30
Asp Arg Glu Ser Ile Glu Lys Trp Leu Phe Ser Gly Lys Lys Asn Ser
        35                  40                  45
Cys Pro Val Thr Lys Gln Val Ile Thr Glu Thr Asp Leu Thr Pro Asn
    50                  55                  60
His Thr Leu Arg Arg Leu Ile Gln Ser Trp Cys Thr Leu Asn Ala Ser
65                  70                  75                  80
Tyr Gly Ile Glu Arg Ile Pro Thr Pro Lys Pro Pro Ile Cys Lys Ser
                85                  90                  95
Glu Ile Glu Lys Leu Ile Lys Glu Ser Ser Ser Ser His Leu Asn Gln
            100                 105                 110
Val Lys Cys Leu Lys Arg Leu Arg Gln Ile Val Ser Glu Asn Thr Thr
        115                 120                 125
Asn Lys Arg Cys Leu Glu Ala Ala Glu Val Pro Glu Phe Leu Ala Asn
    130                 135                 140
Ile Val Ser Asn Ser Val Asp Thr Tyr Asn Ser Pro Ser Ser Ser Leu
145                 150                 155                 160
Ser Ser Ser Asn Leu Asn Asp Met Cys Gln Ser Asn Met Leu Glu Asn
                165                 170                 175
Arg Phe Asp Ser Ser Arg Ser Leu Met Asp Glu Ala Leu Ser Val Leu
            180                 185                 190
Tyr His Leu Asp Thr Ser Glu Thr Ala Leu Lys Ser Leu Leu Asn Asn
        195                 200                 205
Lys Lys Gly Thr Asn Leu Val Lys Thr Leu Thr Lys Ile Met Gln Arg
    210                 215                 220
Gly Ile Tyr Glu Ser Arg Ala Tyr Ala Ala Leu Leu Leu Lys Lys Leu
225                 230                 235                 240
Leu Glu Val Ala Asp Pro Met Gln Ile Ile Leu Leu Glu Arg Glu Leu
                245                 250                 255
Phe Gly Glu Val Ile Gln Ile Leu His Asp Gln Ile Ser His Lys Ala
            260                 265                 270
Thr Arg Ser Ala Met Gln Ile Leu Val Ile Thr Cys Pro Trp Gly Arg
        275                 280                 285
Asn Arg His Lys Ala Val Glu Gly Gly Thr Ile Ser Met Ile Ile Glu
    290                 295                 300
Leu Leu Met Asp Asp Thr Phe Ser Ser Glu Arg Arg Asn Ser Glu Met
305                 310                 315                 320
Ala Met Val Val Leu Asp Met Leu Cys Gln Cys Ala Glu Gly Arg Ala
                325                 330                 335
Glu Phe Leu Asn His Gly Ala Ala Ile Ala Val Val Ser Lys Lys Ile
            340                 345                 350
Leu Arg Val Ser Gln Ile Thr Ser Glu Arg Ala Val Arg Val Leu Leu
        355                 360                 365
Ser Val Gly Arg Phe Cys Ala Thr Pro Ser Leu Leu Gln Glu Met Leu
    370                 375                 380
Gln Leu Gly Val Val Ala Lys Leu Cys Leu Val Leu Gln Val Ser Cys
385                 390                 395                 400
Gly Asn Lys Thr Lys Glu Lys Ala Lys Glu Leu Leu Lys Leu His Ala
                405                 410                 415
Arg Val Trp Arg Glu Ser Pro Cys Val Pro Arg Asn Leu Tyr Asp Ser
            420                 425                 430
Tyr Pro Ala
        435
```

```
<210>  85
<211>  1645
<212>  DNA
<213>  Arabidopsis thaliana

<400>  85
gctaacttat tcaaaccaat tcacatatct cctaaatctc aacgactgca ctttgaagct      60
ttttgtttag attcgttcgt cgtttccatt gattaagtct acttcttttt tttcatattt     120
tcgagcgggt caaatggatc aagagataga gattccttcc ttcttccttt gtccaatctc     180
tctagatatc atgaaagatc cggtgatagt ttccaccgga ataacctacg accgagaaag     240
catcgagaag tggctctttt ccggtaagaa aaactcatgt ccagtcacca aacaagtcat     300
aaccgaaact gatcttaccc caaaccacac tcttcgccgt ttgattcaat cttggtgtac     360
cctcaacgca tcatatggta tagagaggat cccaactcca aaacctccga tctgtaaatc     420
ggagatcgaa aagcttatca aagagtcgtc atcttcgcat ctaaaccaag tcaaatgcct     480
caaacgactt cgtcaaatag tatctgagaa tacaaccaac aaacggtgct tagaagccgc     540
agaagttccg gagtttttgg caaatatcgt aagcaactct gtagatacgt ataactctcc     600
ttcttcttca ctttcttctt cgaatctcaa cgacatgtgt cagtcaaaca tgttggagaa     660
tcgatttgat tcttcaagaa gcttgatgga cgaagcccta agcgtactct accatcttga     720
cacatcggag acagccctca agagtctttt aaacaacaag aaaggaacaa atcttgtgaa     780
```

```
aacgttgacg aagattatgc aacgtgggat ctacgagtca agagcctatg cggctttgct    840
tctcaagaag ctcctggaag ttgcggatcc aatgcagatc atattgttgg aacgagaact    900
tttcggtgag gtgattcaaa tcttgcacga ccagatctct cacaaggcga caagatcagc    960
aatgcaaatc ttggtgatca catgtccatg ggaaggaat agacacaagg cagtggaagg    1020
tggaacaatc tcgatgataa tcgagctttt aatggacgat actttctcat cagagagaag    1080
gaattcagag atggcgatgg tggttcttga tatgttatgt cagtgcgcag aaggaagagc    1140
tgagttcttg aatcacggtg ccgctattgc ggttgtgtct aagaagatat tgagggtctc    1200
acaaataact agtgaaaggg cagttagggt tttgctttcg gttgggaggt tctgcgcgac    1260
accctctttg ttgcaggaga tgttgcaatt gggagttgta gcaaagctgt gtctggtact    1320
tcaagttagc tgtggtaaca agactaaaga aaaggctaaa gaattgctta agcttcacgc    1380
tagggtttgg agggaatcac cttgtgtccc aagaaatttg tatgattcgt atcctgcttg    1440
aacaacgtac atgtttgaat aattctcatc agatacaaac aattctttga gaattattct    1500
tcgtataagt tcattttaaa acaaaaaaga gtggtttaaa ttttgctcaa actctgattt    1560
tctttcattt gtttttgttt tattatcttt caatgtttat cattatgtgt agataatagt    1620
ggacatattt tcatgaaatt atgaa                                          1645

<210>  86
<211>  411
<212>  PRT
<213>  Arabidopsis thaliana

<400>  86
Met Ser Gly Gly Ile Met Asp Glu Glu Ile Glu Ile Pro Pro Phe Phe
1               5                   10                  15
Leu Cys Pro Ile Ser Leu Glu Ile Met Lys Asp Pro Val Ile Val Ser
            20                  25                  30
Thr Gly Ile Thr Tyr Asp Arg Asp Ser Ile Glu Lys Trp Leu Phe Ala
        35                  40                  45
Gly Lys Lys Asn Ser Cys Pro Val Thr Lys Gln Asp Ile Thr Asp Ala
    50                  55                  60
Asp Leu Thr Pro Asn His Thr Leu Arg Arg Leu Ile Gln Ser Trp Cys
65                  70                  75                  80
Thr Leu Asn Ala Ser Tyr Gly Val Glu Arg Ile Pro Thr Pro Arg Pro
                85                  90                  95
Pro Ile Cys Lys Ser Glu Ile Glu Lys Leu Ile Arg Asp Ser Ala Ser
            100                 105                 110
Ser His Glu Asn Gln Val Lys Cys Leu Lys Arg Leu Arg Gln Ile Val
        115                 120                 125
Ser Glu Asn Ala Thr Asn Lys Arg Cys Leu Glu Ala Ala Gly Val Pro
    130                 135                 140
Glu Phe Leu Ala Asn Ile Val Ser Asn Asp Ser Glu Asn Gly Ser Leu
145                 150                 155                 160
Thr Asp Glu Ala Leu Asn Leu Leu Tyr His Leu Glu Thr Ser Glu Thr
                165                 170                 175
Val Leu Lys Asn Leu Leu Asn Asn Lys Lys Asp Asn Asn Ile Val Lys
            180                 185                 190
Ser Leu Thr Lys Ile Met Gln Arg Gly Met Tyr Glu Ser Arg Val Tyr
        195                 200                 205
Ala Thr Leu Leu Leu Lys Asn Ile Leu Glu Val Ala Asp Pro Met Gln
    210                 215                 220
Ser Met Thr Leu Lys Pro Glu Val Phe Thr Glu Val Val Gln Ile Leu
225                 230                 235                 240
Asp Asp Arg Ile Ser Gln Lys Ala Thr Lys Ala Ala Met His Ile Leu
                245                 250                 255
Val Asn Ile Cys Pro Trp Gly Arg Asn Arg His Lys Ala Val Glu Ala
            260                 265                 270
Gly Val Ile Ser Val Ile Ile Glu Leu Leu Met Asp Glu Ser Phe Thr
        275                 280                 285
Ser Glu Arg Arg Gly Pro Glu Met Ala Met Val Val Leu Asp Leu Leu
    290                 295                 300
Cys Gln Cys Ala Glu Gly Arg Ala Glu Phe Leu Asn His Gly Ala Ala
305                 310                 315                 320
Ile Ala Val Val Cys Lys Lys Ile Leu Arg Val Ser Gln Thr Ala Ser
                325                 330                 335
Asp Arg Ala Val Arg Val Leu Leu Ser Val Gly Arg Phe Cys Ala Thr
            340                 345                 350
Pro Ala Leu Leu His Glu Met Leu Gln Leu Gly Val Val Ala Lys Leu
        355                 360                 365
Cys Leu Val Leu Gln Val Ser Cys Gly Gly Lys Thr Lys Glu Lys Ala
    370                 375                 380
Lys Glu Leu Leu Lys Leu His Ala Arg Val Trp Lys Asp Ser Pro Cys
385                 390                 395                 400
Leu Pro Lys Asn Met Ile Leu Ala Tyr Pro Cys
                405                 410
```

```
<210>  87
<211>  1212
<212>  DNA
<213>  Medicago truncatula

<400>  87
atggatgaaa ttgatgttcc ttcacatttt ctttgcccaa tttccctaca acttatgaaa     60
gatcctgtca cactttccac aggaattacg tacgacagag aaaacataga aaaatggtta    120
ttttcattcc aaaacaatac ttgtcctgtt acaaaacaaa ccttattaga aaccgatctt    180
aataatctta taccaaacca tactttacgt cgtttgattc aatcttggtg taccccttaat   240
gcttcatttg gtgttgaacg tattccaaca ccaaaatcac caatagatag aacacagatt    300
cttaaactcc tcaatgaagc aaagaaattc ccagaaaaac aactcaattg tcttgtaaag    360
cttcgttcta tcgtcttcga aagcgaaagg aacaaaaaat gtttggaatc tgcaggtgca    420
atagagtttc tagctttaac catgaagaac aatttaaatt caagttctct gagtgaagca    480
gctattgaaa ttttgtttca tcttaaccct tctgaagaac atgttaagaa tctgataaac    540
aatgaaagca ttcaatttat tgagtcattg tttcatgttt tgaagcttgg aagctaccaa    600
tctagaggtt atgcaacaat gctgttgaaa tcggcgtttg aagtatccga tccgattcaa    660
ttaataagcg taaaaaaggc gatttttgaa gagataatga gagtgctagt tgataagatc    720
tcacaacaag cttcaaaagc tgcattaaaa ctacttctgg agctttacc atggggaaga     780
aaccgaatta aagcggttga aggaggcgtg gttttagtcc taatcgaact tcttttcgat    840
gtttcggaaa gaagggtttg tgaattgatg ttaataggtt tggatcaaat ttgtggttgt    900
gcagaaggga gagcagaatt gttgaatcat ggagcaggat tggctattgt gtcaaagaaa    960
atttgaggg tttctcatgt tgcaagtgat agaggtgtta gaattatgag ttctatttgt    1020
aggtattctg caaattctag agttttacat gaaatgttgc atgttggagc agtttcaaag   1080
ttgtgtttgg tgcttcaagt tgatagtaat ttcaagacaa aagagagggc taaggagata   1140
ctcaaattgc attctactgt ttggaagaat tctacatgta ttcctgttcc attgttatct   1200
tcttatccat ga                                                        1212

<210>  88
<211>  403
<212>  PRT
<213>  Medicago truncatula

<400>  88
Met Asp Glu Ile Asp Val Pro Ser His Phe Leu Cys Pro Ile Ser Leu
1               5                   10                  15
Gln Leu Met Lys Asp Pro Val Thr Leu Ser Thr Gly Ile Thr Tyr Asp
            20                  25                  30
Arg Glu Asn Ile Glu Lys Trp Leu Phe Ser Phe Gln Asn Asn Thr Cys
        35                  40                  45
Pro Val Thr Lys Gln Thr Leu Leu Glu Thr Asp Leu Asn Asn Leu Ile
    50                  55                  60
Pro Asn His Thr Leu Arg Arg Leu Ile Gln Ser Trp Cys Thr Leu Asn
65                  70                  75                  80
Ala Ser Phe Gly Val Glu Arg Ile Pro Thr Pro Lys Ser Pro Ile Asp
                85                  90                  95
Arg Thr Gln Ile Leu Lys Leu Leu Asn Glu Ala Lys Lys Phe Pro Glu
            100                 105                 110
Lys Gln Leu Asn Cys Leu Val Lys Leu Arg Ser Ile Val Phe Glu Ser
        115                 120                 125
Glu Arg Asn Lys Lys Cys Leu Glu Ser Ala Gly Ala Ile Glu Phe Leu
    130                 135                 140
Ala Leu Thr Met Lys Asn Asn Leu Asn Ser Ser Ser Leu Ser Glu Ala
145                 150                 155                 160
Ala Ile Glu Ile Leu Phe His Leu Asn Pro Ser Glu Glu His Val Lys
                165                 170                 175
Asn Leu Ile Asn Asn Glu Ser Ile Gln Phe Ile Glu Ser Leu Phe His
            180                 185                 190
Val Leu Lys Leu Gly Ser Tyr Gln Ser Arg Gly Tyr Ala Thr Met Leu
        195                 200                 205
Leu Lys Ser Ala Phe Glu Val Ser Asp Pro Ile Gln Leu Ile Ser Val
    210                 215                 220
Lys Lys Ala Ile Phe Glu Glu Ile Met Arg Val Leu Val Asp Lys Ile
225                 230                 235                 240
Ser Gln Gln Ala Ser Lys Ala Ala Leu Lys Leu Leu Leu Glu Leu Leu
                245                 250                 255
Pro Trp Gly Arg Asn Arg Ile Lys Ala Val Glu Gly Gly Val Val Leu
            260                 265                 270
Val Leu Ile Glu Leu Leu Phe Asp Val Ser Glu Arg Arg Val Cys Glu
        275                 280                 285
Leu Met Leu Ile Gly Leu Asp Gln Ile Cys Gly Cys Ala Glu Gly Arg
    290                 295                 300
Ala Glu Leu Leu Asn His Gly Ala Gly Leu Ala Ile Val Ser Lys Lys
```

```
305                 310                 315                 320
Ile Leu Arg Val Ser His Val Ala Ser Asp Arg Gly Val Arg Ile Met
                325                 330                 335
Ser Ser Ile Cys Arg Tyr Ser Ala Asn Ser Arg Val Leu His Glu Met
            340                 345                 350
Leu His Val Gly Ala Val Ser Lys Leu Cys Leu Val Leu Gln Val Asp
        355                 360                 365
Ser Asn Phe Lys Thr Lys Glu Arg Ala Lys Glu Ile Leu Lys Leu His
    370                 375                 380
Ser Thr Val Trp Lys Asn Ser Thr Cys Ile Pro Val Pro Leu Leu Ser
385                 390                 395                 400
Ser Tyr Pro


<210>  89
<211>  1480
<212>  DNA
<213>  Nicotiana benthamiana

<220>
<221>  misc_feature
<222>  (661)..(661)
<223>  n is a, c, g, or t

<220>
<221>  misc_feature
<222>  (770)..(770)
<223>  n is a, c, g, or t

<400>  89
gaaagaacgc atctttactc ttctctctct cgttatatat aatttgagtc ttgtttttat      60
ttgtaaagaa gaaaaaaaat agatatggaa gaagtagaga tacctcaata ttttttgtgt     120
cctatatcac ttcaaataat gaaagatccg gtgacgacgg tgaccggaat aacgtacgac     180
cgggaaagca tcgagatgtg gttgttaacg gcggaggaag agacggagac ggcggcgtgt     240
ccggtaacaa agcaaccttt gccaaaagac accgagttgt tgacaccaaa tcatatgctc     300
cggcggctaa ttcaagcttg gtgcatagtc aacgcagaga aaggcgttga ccgaattcca     360
acaccaaagt atcctatgaa caaatcgaat attctccgat tacttcgaca agttaataat     420
aatgatcatc agctttgtgc tgaagctttg aggaaaatgg ctgatttggt tagtgagaat     480
gaaaagaaca ggaaatgcat ggaacaagct ggtgcaatta aggctatggt ttgtttcata     540
gtaaaaagtt tcaaatatga gaaattattt cctggtattg aagaagcttt aaggattttc     600
catttaattt ggaatccaaa atccgaaaac aagcagtatg taaaggataa ccctgattta     660
nttcaagcaa ttttatggat tttaaagagc aatttgaata ccagtcacgt cctagtcaag     720
actcatgcaa tgatggtttt gaagaacgtg acagaagttt cgagttcgan tcttttaaca     780
ggattagacc ccgagttttt ccaacaaatg gcgaatacat tgcgaaaaaa tggaaagtac     840
tacatctctc aacaagcaac aaaagcagct ttacaagtgc taatagatgc atgtctatgg     900
ggaagaaata ggctaaaaat cgtcgaatcg ggagccattt tcgagctgat tgagcttgaa     960
ttatctaacc ctgagaaaag ggttagtgaa ttagtattct gtctattggc taatctatgc    1020
gtattagctg atgggagagc agaattgttg aaacatgcag ctggaattgc agtggtcaca    1080
aaaaggacac taaggatatc ttcagctaca gatgatagtg cacttcaaat tcttggttta    1140
atttcaaagt ttctgccac aaacgaagtg ttattggaaa tgttaagggt aggaggggta    1200
tcaaaacttt gcatggcaat tcaagcagat tgtgagcctc atttgaagaa gaaagcaaga    1260
gagatattga gagcacattc tcatgtttgg agtaattctc cttgtatctc cttgtataca    1320
gtttatcttt tgacagattt cctggacaat agcaagattt gagccctata accttgaata    1380
gttttttttt tccttgtata aatacagcta gccaacatac tgcctttttt gttatactgt    1440
aaaacagcat ctaaattacc cataaaaaaa aaaaaaaaaa                           1480

<210>  90
<211>  425
<212>  PRT
<213>  Nicotiana benthamiana

<220>
<221>  UNSURE
<222>  (193)..(193)
<223>  Xaa can be any naturally occurring amino acid

<220>
<221>  UNSURE
<222>  (229)..(229)
<223>  Xaa can be any naturally occurring amino acid

<400>  90
Met Glu Glu Val Glu Ile Pro Gln Tyr Phe Leu Cys Pro Ile Ser Leu
1               5                   10                  15
```

```
Gln Ile Met Lys Asp Pro Val Thr Thr Val Thr Gly Ile Thr Tyr Asp
            20                  25                  30
Arg Glu Ser Ile Glu Met Trp Leu Leu Thr Ala Glu Glu Glu Thr Glu
        35                  40                  45
Thr Ala Ala Cys Pro Val Thr Lys Gln Pro Leu Pro Lys Asp Thr Glu
    50                  55                  60
Leu Leu Thr Pro Asn His Met Leu Arg Arg Leu Ile Gln Ala Trp Cys
65                  70                  75                  80
Ile Val Asn Ala Glu Lys Gly Val Asp Arg Ile Pro Thr Pro Lys Tyr
                85                  90                  95
Pro Met Asn Lys Ser Asn Ile Leu Arg Leu Leu Arg Gln Val Asn Asn
            100                 105                 110
Asn Asp His Gln Leu Cys Ala Glu Ala Leu Arg Lys Met Ala Asp Leu
        115                 120                 125
Val Ser Glu Asn Glu Lys Asn Arg Lys Cys Met Glu Gln Ala Gly Ala
    130                 135                 140
Ile Lys Ala Met Val Cys Phe Ile Val Lys Ser Phe Lys Tyr Glu Lys
145                 150                 155                 160
Leu Phe Pro Gly Ile Glu Glu Ala Leu Arg Ile Phe His Leu Ile Trp
                165                 170                 175
Asn Pro Lys Ser Glu Asn Lys Gln Tyr Val Lys Asp Asn Pro Asp Leu
            180                 185                 190
Xaa Gln Ala Ile Leu Trp Ile Leu Lys Ser Asn Leu Asn Thr Ser His
        195                 200                 205
Val Leu Val Lys Thr His Ala Met Met Val Leu Lys Asn Val Thr Glu
    210                 215                 220
Val Ser Ser Ser Xaa Leu Leu Thr Gly Leu Asp Pro Glu Phe Phe Gln
225                 230                 235                 240
Gln Met Ala Asn Thr Leu Arg Lys Asn Gly Lys Tyr Tyr Ile Ser Gln
                245                 250                 255
Gln Ala Thr Lys Ala Ala Leu Gln Val Leu Ile Asp Ala Cys Leu Trp
            260                 265                 270
Gly Arg Asn Arg Leu Lys Ile Val Glu Ser Gly Ala Ile Phe Glu Leu
        275                 280                 285
Ile Glu Leu Glu Leu Ser Asn Pro Glu Lys Arg Val Ser Glu Leu Val
    290                 295                 300
Phe Cys Leu Leu Ala Asn Leu Cys Val Leu Ala Asp Gly Arg Ala Glu
305                 310                 315                 320
Leu Leu Lys His Ala Ala Gly Ile Ala Val Val Thr Lys Arg Thr Leu
                325                 330                 335
Arg Ile Ser Ser Ala Thr Asp Asp Ser Ala Leu Gln Ile Leu Gly Leu
            340                 345                 350
Ile Ser Lys Phe Ser Ala Thr Asn Glu Val Leu Leu Glu Met Leu Arg
        355                 360                 365
Val Gly Gly Val Ser Lys Leu Cys Met Ala Ile Gln Ala Asp Cys Glu
    370                 375                 380
Pro His Leu Lys Lys Lys Ala Arg Glu Ile Leu Arg Ala His Ser His
385                 390                 395                 400
Val Trp Ser Asn Ser Pro Cys Ile Ser Leu Tyr Thr Val Tyr Leu Leu
                405                 410                 415
Thr Asp Phe Leu Asp Asn Ser Lys Ile
            420                 425
```

<210> 91
<211> 1643
<212> DNA
<213> Oryza sativa

<400> 91

```
cattcccttc aactcaaact cctcttcttc ttcttcctcc catcgatctc tctccttcgc      60
gatcaactcg agctcgaact cgtctcgtgg tagtagcgag catggaggag gcggcggcgg     120
aggtgccgtc ctacttcctg tgcccgatct cgctggagat catgagggac ccggtgacgc     180
tggcgacggg gatcacctac gaccggagca gcatcgagcg gtggatgttc ggcggcggcg     240
gcggcgacgg ggggaagggg acgtgcccgg tgacgcggcg gcagctggcg ccggcggagc     300
gggaggcgac gcccaaccac acgctgcggc ggctcatcca ggcgtggtgc gccgcgcacg     360
ccgtcgagag gttccccacc ccgcgcccgc ccgtcgactc ctgccgcgtc gccgcgctcg     420
tcgacgaggg gacgacgacg atgctcggcg gcggcggcag gcagcggcag ctcgccgcgc     480
tccgggagat caaggccatc gccgccgaga gcgaccgcaa caagcgctgc gtcgaggcca     540
cgcccggcgc cgtcgagttc ctcgtctccg tcgtcgtcca gagccatgcc gccgcctcca     600
cctcagccag ctcggacgac gacgacctgt tcgactcggt gatcgattcc cccatgtcga     660
cgagctcgcc ggaggaggag gccctgggcg tgctctactc cctcaagccg tccgagccca     720
ccctgcgccg cgtcctcggc aaggacaatg gcgtcggctt cctcgacacc ctcgcctccg     780
tgctccgccg cccgagctac cgttcgcgcg cgtatgccat cctcctcctg aaggcggtga     840
cgtcggcgat gccgccggag cggctgatgg cggtgagccc cgagctggtg gaggaggtgg     900
```

```
tccgggtggt gtccgacggc gtgtcgtcga aggcggtgaa ggcggcgctg cacgtgctgt    960
gccggctgtg cccgtggggg cggaaccgcg tgaaggcggt ggaggccggc gcggtggcgg   1020
cgctggtgga gctcctcctc gacgaggagg gcggcggcgg gcggcggcgc gcggcggagc   1080
tggcggtggt ggcgatcgac cacctgtgcg ggtgcgcgga ggggaggtcg gagctggtgg   1140
cgcacccggc ggggctcgcc gtggtgtcca agagggcgat gcgggtgtcg ccggcggcca   1200
ccgagagcgc cgtgcgcgcg ctccacgccg tcgcgaggaa cgcggcgacg ccggccgtgc   1260
tgcaggagat gctcgccgtc ggcgtggtgg cgaagctgct gctggtgctg caggcggacg   1320
gcggcgagcg cgccagggcg agggcgaggg agatgctcag ggcgaacgct agggtttgga   1380
aggactcgcc atgcttgcaa gctcacctta aggcttctta cccctcctga tcatttcatc   1440
accatcacat ccatccaccg atctattaat tattattaat tgctaagagg agcacatgct   1500
ttaatttgca ctcctcttag tagttaatta gattttgtga ttacttttag ttctttaatt   1560
tgtttatgat ctgaatgtac aaaatacact gtaaatcttt cattaattct gtacaaaaat   1620
acactataaa tctttcattg cct                                           1643
```

<210> 92
<211> 442
<212> PRT
<213> Oryza sativa

<400> 92

```
Met Glu Glu Ala Ala Ala Glu Val Pro Ser Tyr Phe Leu Cys Pro Ile
1               5                   10                  15
Ser Leu Glu Ile Met Arg Asp Pro Val Thr Leu Ala Thr Gly Ile Thr
            20                  25                  30
Tyr Asp Arg Ser Ser Ile Glu Arg Trp Met Phe Gly Gly Gly Gly Gly
        35                  40                  45
Asp Gly Gly Lys Gly Thr Cys Pro Val Thr Arg Arg Gln Leu Ala Pro
    50                  55                  60
Ala Glu Arg Glu Ala Thr Pro Asn His Thr Leu Arg Arg Leu Ile Gln
65                  70                  75                  80
Ala Trp Cys Ala Ala His Ala Val Glu Arg Phe Pro Thr Pro Arg Pro
                85                  90                  95
Pro Val Asp Ser Cys Arg Val Ala Ala Leu Val Asp Glu Gly Thr Thr
            100                 105                 110
Thr Met Leu Gly Gly Gly Gly Arg Gln Arg Gln Leu Ala Ala Leu Arg
        115                 120                 125
Glu Ile Lys Ala Ile Ala Ala Glu Ser Asp Arg Asn Lys Arg Cys Val
    130                 135                 140
Glu Ala Thr Pro Gly Ala Val Glu Phe Leu Val Ser Val Val Val Gln
145                 150                 155                 160
Ser His Ala Ala Ala Ser Thr Ser Ala Ser Ser Asp Asp Asp Asp Leu
                165                 170                 175
Phe Asp Ser Val Ile Asp Ser Pro Met Ser Thr Ser Ser Pro Glu Glu
            180                 185                 190
Glu Ala Leu Gly Val Leu Tyr Ser Leu Lys Pro Ser Glu Pro Thr Leu
        195                 200                 205
Arg Arg Val Leu Gly Lys Asp Asn Gly Val Gly Phe Leu Asp Thr Leu
    210                 215                 220
Ala Ser Val Leu Arg Arg Pro Ser Tyr Arg Ser Arg Ala Tyr Ala Ile
225                 230                 235                 240
Leu Leu Leu Lys Ala Val Thr Ser Ala Met Pro Pro Glu Arg Leu Met
                245                 250                 255
Ala Val Ser Pro Glu Leu Val Glu Glu Val Val Arg Val Val Ser Asp
            260                 265                 270
Gly Val Ser Ser Lys Ala Val Lys Ala Ala Leu His Val Leu Cys Arg
        275                 280                 285
Leu Cys Pro Trp Gly Arg Asn Arg Val Lys Ala Val Glu Ala Gly Ala
    290                 295                 300
Val Ala Ala Leu Val Glu Leu Leu Leu Asp Glu Glu Gly Gly Gly Gly
305                 310                 315                 320
Arg Arg Arg Ala Ala Glu Leu Ala Val Val Ala Ile Asp His Leu Cys
                325                 330                 335
Gly Cys Ala Glu Gly Arg Ser Glu Leu Val Ala His Pro Ala Gly Leu
            340                 345                 350
Ala Val Val Ser Lys Arg Ala Met Arg Val Ser Pro Ala Ala Thr Glu
        355                 360                 365
Ser Ala Val Arg Ala Leu His Ala Val Ala Arg Asn Ala Ala Thr Pro
    370                 375                 380
Ala Val Leu Gln Glu Met Leu Ala Val Gly Val Val Ala Lys Leu Leu
385                 390                 395                 400
Leu Val Leu Gln Ala Asp Gly Gly Glu Arg Ala Arg Ala Arg Ala Arg
                405                 410                 415
Glu Met Leu Arg Ala Asn Ala Arg Val Trp Lys Asp Ser Pro Cys Leu
            420                 425                 430
```

Gln Ala His Leu Lys Ala Ser Tyr Pro Ser
435 440

<210> 93
<211> 1591
<212> DNA
<213> Oryza sativa

<400> 93
agtactcccc atctcccatc caccattgct gccatctcca ccacatacaa tacaacaact 60
actactcagc tcaccatcac cggagcaaga gccaagaaga cgacgacgac gacgagctag 120
cagcagcatt gcaaccgacg gcaatggagg agcagcagca ggtggaggtg gaggtggagg 180
tgccgtccta cttcgtgtgc cccatctcgc tgcagatcat gcgcgacccg gtgacgctcc 240
ccaccggcat cacctacgac cgcgacggca tcgagcgatg gctcctcacc gccggcacct 300
gcccgctcac caagcagccc gtgccgcccg actgcgaccc cacgcccaac cacaccctgc 360
gccgcctcat ccagtcgtgg tgcgccctcc acgccgacca tggcgtcgac ctcgtcccca 420
cccccaagcc ccccgccgac cgcgcccgcg tcgccgacct cgtctcccgc ctccgcgccg 480
ccacgagctc cgctgccctc ctcgacgcgc tgcgcgagct cagggacgtt gccgccgaga 540
gcgagcgtaa caggaagctc ctcgccgccg tccccggcgc ggtggatgtt ctggccgccg 600
tcgtcgtcgc gtcgtgtcga gacgccaagg ccgcgtgcga cgaggccctc gagatcgtct 660
gctcgctgga gctctcggag cggtgcctgg cgcggctcgt cgagaggaac gaggagctcg 720
tcgacgcgct cgtcgccacg ctgcagcgga cgaacaccac gtcccgggcg cacgcggcgc 780
tgctcctgga ggccgtgacg gcggtcatgc cgtcgaacag gctggtgtcc ctcccggagg 840
aggtgttcgg tgaggcggtg cagctgctcc gcgacagggt atcgagcccg gcgacgaggg 900
cggcgctgca cgtgctggtc ggcacgacgt cgtggggccg caaccgcgtg aaggcggtgg 960
acgccggcgc ggtggcggtg ctggtcgaca tgctcctgga cggccagtc gagcggcgcg 1020
ggtgcgagct ggccctggcg gcgctggacc ggatgtgcgg gtgcgcggag gggcggcgg 1080
cgctggtgtc gcacggcgcg ggcgtggcgg tggtggggag gaaggtgctg agggtgtcgg 1140
aggtggcgag cgagaaggcg gtgcgggtgc tgcggtcggt ggcgaggcac gcggcgacgg 1200
cggcggtggt gcaggagatg ggcagacgg gggcggtgga gaagctgtgc gtggtggcgc 1260
agtcggagca gtgcggggag aggacgcggg agcgggcgcg ggagacgctg cggctgcacg 1320
ccagagcgtg gaggaactcg ccctgcttgc agcctcacct ccaggccctc taccttctt 1380
gctgatcgaa ttctgattca tgatcaccgg cctcatcact agctagctac caacatacag 1440
cacagagaga attgaacact tgttcgattc ttcctttcgc tcgattaatt ttattgagtt 1500
gagatcgttc gatgtagtcc tatgaatcaa agtgtacaga tcgacgcaat agatgaacta 1560
aatcatgcgc gagctttttt tgtttgggtc t 1591

<210> 94
<211> 413
<212> PRT
<213> Oryza sativa

<400> 94
Met Glu Glu Gln Gln Gln Val Glu Val Glu Val Glu Val Pro Ser Tyr
1 5 10 15
Phe Val Cys Pro Ile Ser Leu Gln Ile Met Arg Asp Pro Val Thr Leu
20 25 30
Pro Thr Gly Ile Thr Tyr Asp Arg Asp Gly Ile Glu Arg Trp Leu Leu
35 40 45
Thr Ala Gly Thr Cys Pro Leu Thr Lys Gln Pro Val Pro Pro Asp Cys
50 55 60
Asp Pro Thr Pro Asn His Thr Leu Arg Arg Leu Ile Gln Ser Trp Cys
65 70 75 80
Ala Leu His Ala Asp His Gly Val Asp Leu Val Pro Thr Pro Lys Pro
85 90 95
Pro Ala Asp Arg Ala Arg Val Ala Asp Leu Val Ser Arg Leu Arg Ala
100 105 110
Ala Thr Ser Ser Ala Ala Leu Leu Asp Ala Leu Arg Glu Leu Arg Asp
115 120 125
Val Ala Ala Glu Ser Glu Arg Asn Arg Lys Leu Leu Ala Ala Val Pro
130 135 140
Gly Ala Val Asp Val Leu Ala Ala Val Val Val Ala Ser Cys Arg Asp
145 150 155 160
Ala Lys Ala Ala Cys Asp Glu Ala Leu Glu Ile Val Cys Ser Leu Glu
165 170 175
Leu Ser Glu Arg Cys Leu Ala Arg Leu Val Glu Arg Asn Glu Glu Leu
180 185 190
Val Asp Ala Leu Val Ala Thr Leu Gln Arg Thr Asn Thr Thr Ser Arg
195 200 205
Ala His Ala Ala Leu Leu Leu Glu Ala Val Thr Ala Val Met Pro Ser
210 215 220
Asn Arg Leu Val Ser Leu Pro Glu Glu Val Phe Gly Glu Ala Val Gln
225 230 235 240
Leu Leu Arg Asp Arg Val Ser Ser Pro Ala Thr Arg Ala Ala Leu His

```
                245                 250                 255
Val Leu Val Gly Thr Thr Ser Trp Gly Arg Asn Arg Val Lys Ala Val
            260                 265                 270
Asp Ala Gly Ala Val Ala Val Leu Val Asp Met Leu Leu Asp Gly Pro
        275                 280                 285
Val Glu Arg Arg Gly Cys Glu Leu Ala Leu Ala Ala Leu Asp Arg Met
    290                 295                 300
Cys Gly Cys Ala Glu Gly Arg Ala Ala Leu Val Ser His Gly Ala Gly
305                 310                 315                 320
Val Ala Val Val Gly Arg Lys Val Leu Arg Val Ser Glu Val Ala Ser
                325                 330                 335
Glu Lys Ala Val Arg Val Leu Arg Ser Val Ala Arg His Ala Ala Thr
            340                 345                 350
Ala Ala Val Val Gln Glu Met Gly Gln Thr Gly Ala Val Glu Lys Leu
        355                 360                 365
Cys Val Val Ala Gln Ser Glu Gln Cys Gly Glu Arg Thr Arg Glu Arg
    370                 375                 380
Ala Arg Glu Thr Leu Arg Leu His Ala Arg Ala Trp Arg Asn Ser Pro
385                 390                 395                 400
Cys Leu Gln Pro His Leu Gln Ala Leu Tyr Pro Ser Cys
                405                 410
```

<210> 95
<211> 1353
<212> DNA
<213> Oryza sativa

<400> 95

```
tcagcgaggg taggaagcat tcaagtgcga ggccaagcat ggcgagtccc tccacaccct      60
ggcgtgcatc ttgagcatct ccctcgccct cgccctcgtc cgctcgccgg aggcgccgac     120
ctgcaccagg aagagcagcc tcgccaccac gccgacggcc agcatctcct gcagcaccgc     180
cgacgtcgcc gagtgcctcg ccacggcgtg cagcgcgcgc acggcgctct cggcgcccgc     240
cgcggcgggc agccgcgtcg ccgcgcacgc caccgccgcg agccccgccg ggtgcgccac     300
cagcgccagg cgcccctccg cgcagccgca gatgtggtcg atcgccacgg cggcgagctc     360
gcacgcgcgc cggtcgccgc cgccgccgcc gcagccctcg tcgaggagga ggcgcacgag     420
cgccgacacg gcgccggcgt cgaccgcctt gacgcggttc cggccccacg ggcagagccg     480
gcagagcacg tggagcgcca ccttgaccgc cttcgccgac ggcctgtccg ccaccacccc     540
caccacgccg tcgaccagcg cggcgctcgc cgacgtcagc cgcgcggggg ccatcgccgg     600
gagcgccgat ttcagcaggt ggattccctg catccgcgag aggtagctcg gccgccgcag     660
cacgcacgcc atggtttcca agaaatcctc accgccgctg tcctccaaga ccctcttgaa     720
gctctcctcg gagagcttga gcgaatgcag gatgctcagg gccgcctcct ccggcgagct     780
cgccttcgcc ggatcatgca cgccgccgca cgtcgtctct tccggcttgg ccgacgtcgc     840
gtcgtccacg cccgccacgg aggtgcgctc cttgacgacg gacaggagga actccacggc     900
gccggaggcg ccctggacgc accgccggtt gcggtcgctc tcggcgacga tgtcggcgag     960
ctccctcagc gaagccatca gctcctcccg ctgacgacgc cggcgcagca gcggccgcgc    1020
ggcgtccacg atggcggcga cgcgggcggc gtcgacgggg gggcgcgggg tggggaagcg    1080
ctcgacggcg tggacggcgc accacccctg tatgaggcgg cggagcgtgt ggttgggcgt    1140
gggctcccgg tccgccggcg ccagccgctg cttggtcacc gggcactcgc cgtggccgtc    1200
ggtgaacacc caccgctcga tgctctcccg gtcgtaggtg atccccgtcg acagcgtcac    1260
cgggtcccgc atgatctcca gcgagatcgg gcacatgaag taggacggca cctccaccgc    1320
cggcgccgcc gccggcgacg actcctcacc cat                                 1353
```

<210> 96
<211> 450
<212> PRT
<213> Oryza sativa

<400> 96

```
Met Gly Glu Glu Ser Ser Pro Ala Ala Ala Pro Ala Val Glu Val Pro
1               5                   10                  15
Ser Tyr Phe Met Cys Pro Ile Ser Leu Glu Ile Met Arg Asp Pro Val
            20                  25                  30
Thr Leu Ser Thr Gly Ile Thr Tyr Asp Arg Glu Ser Ile Glu Arg Trp
        35                  40                  45
Val Phe Thr Asp Gly His Gly Glu Cys Pro Val Thr Lys Gln Arg Leu
    50                  55                  60
Ala Pro Ala Asp Arg Glu Pro Thr Pro Asn His Thr Leu Arg Arg Leu
65                  70                  75                  80
Ile Gln Gly Trp Cys Ala Val His Ala Val Glu Arg Phe Pro Thr Pro
                85                  90                  95
Arg Pro Pro Val Asp Ala Ala Arg Val Ala Ala Ile Val Asp Ala Ala
            100                 105                 110
Arg Pro Leu Leu Arg Arg Arg Arg Gln Arg Glu Glu Leu Met Ala Ser
        115                 120                 125
```

```
Leu Arg Glu Leu Ala Asp Ile Val Ala Glu Ser Asp Arg Asn Arg Arg
    130                 135                 140
Cys Val Gln Gly Ala Ser Gly Ala Val Glu Phe Leu Leu Ser Val Val
145                 150                 155                 160
Lys Glu Arg Thr Ser Val Ala Gly Val Asp Asp Ala Thr Ser Ala Lys
                165                 170                 175
Pro Glu Glu Thr Thr Cys Gly Gly Val His Asp Pro Ala Lys Ala Ser
            180                 185                 190
Ser Pro Glu Glu Ala Ala Leu Ser Ile Leu His Ser Leu Lys Leu Ser
        195                 200                 205
Glu Glu Ser Phe Lys Arg Val Leu Glu Asp Ser Gly Gly Glu Asp Phe
    210                 215                 220
Leu Glu Thr Met Ala Cys Val Leu Arg Arg Pro Ser Tyr Leu Ser Arg
225                 230                 235                 240
Met Gln Gly Ile His Leu Leu Lys Ser Ala Leu Pro Ala Met Ala Pro
                245                 250                 255
Ala Arg Leu Thr Ser Ala Ser Ala Ala Leu Val Asp Gly Val Val Gly
            260                 265                 270
Val Val Ala Asp Arg Pro Ser Ala Lys Ala Val Lys Val Ala Leu His
        275                 280                 285
Val Leu Cys Arg Leu Cys Pro Trp Gly Arg Asn Arg Val Lys Ala Val
    290                 295                 300
Asp Ala Gly Ala Val Ser Ala Leu Val Arg Leu Leu Leu Asp Glu Gly
305                 310                 315                 320
Cys Gly Gly Gly Gly Gly Asp Arg Arg Ala Cys Glu Leu Ala Ala Val
                325                 330                 335
Ala Ile Asp His Ile Cys Gly Cys Ala Glu Gly Arg Leu Ala Leu Val
            340                 345                 350
Ala His Pro Ala Gly Leu Ala Ala Val Ala Cys Ala Ala Thr Arg Leu
        355                 360                 365
Pro Ala Ala Ala Gly Ala Glu Ser Ala Val Arg Ala Leu His Ala Val
    370                 375                 380
Ala Arg His Ser Ala Thr Ser Ala Val Leu Gln Glu Met Leu Ala Val
385                 390                 395                 400
Gly Val Val Ala Arg Leu Leu Phe Leu Val Gln Val Gly Ala Ser Gly
                405                 410                 415
Glu Arg Thr Arg Ala Arg Ala Arg Glu Met Leu Lys Met His Ala Arg
            420                 425                 430
Val Trp Arg Asp Ser Pro Cys Leu Ala Ser His Leu Asn Ala Ser Tyr
        435                 440                 445
Pro Arg
    450

<210>  97
<211>  1248
<212>  DNA
<213>  Oryza sativa

<400>  97
atggaggagg cggcggcgga ggtgccgtcc tacttcctgt gcccgatctc gctggagatc      60
atgagggacc cggtgacgct ggcgacgggg atcacctacg accggagcag catcgagcgg     120
tggatgttcg gcggcggcgg cggcggcggc gggaagggga cgtgcccggt gacgcggcgg     180
cagctggcgc cggcggagcg ggaggcgacg cccaaccaca cgctgcggcg gctcatccag     240
gcgtggggac gacgacgatg ctcggcggcg gcggcaggca gcggcagctc gccgcgctcc     300
ggggagatca aggccatcgc cgccgagagc gaccgcaaca agcgctgcgt cgaggccacg     360
cccggcgccg tcgagttcct cgtctccgtc gtcgtccaga gccatgccgc cgcctccacc     420
tcagccagct cggacgacga cgacctgttc gactcggtga tcgattcccc catgtcgacg     480
agctcgccgg aggaggaggc cctgggcgtg ctctactccc tcaagccgtc cgagcccacc     540
ctgcgccgcg tcctcggcaa ggacaatggc gtcggcttcc tcgacaccct cgcctccgtg     600
ctccgccgcc cgagctaccg ttcgcgcgcg tatgccatcc tcctcctgaa ggcggtgacg     660
tcggcgatgc cgccggagcg gctgatggcg gtgagccccg agctggtgga ggaggtggtc     720
cgggtggtgt ccgacggcgt gtcgtcgaag gcggtgaagg cggcgctgca cgtgctgtgc     780
cggctgtgcc cgtgggggcg gaaccgcgtg aaggcggtgg aggccggcgc ggtggcggcg     840
ctggtggagc tcctcctcga cgaggagggc ggcggcgggc ggcggcgcgc ggcggagctg     900
gcggtggtgg cgatcgacca cctgtgcggg tgcgcggagg ggaggtcgga gctggtggcg     960
cacccggcgg ggctcgccgt ggtgtccaag agggcgatgc gggtgtcgcc ggcggccacc    1020
gagagcgccg tgcgcgcgct ccacgccgtc gcgaggaacg cggcgacgcc ggccgtgctg    1080
caggagatgc tcgccgtcgg cgtggtggcg aagctgctgc tggtgctgca ggcggacggc    1140
ggcgagcgcg ccagggcgag ggcgagggag atgctcaggg cgaacgctag ggtttggaag    1200
gactcgccat gcttgcaagc tcaccttaag gcttcttacc cctcctga                 1248

<210>  98
<211>  415
<212>  PRT
```

<213> Oryza sativa

<400> 98

```
Met Glu Glu Ala Ala Ala Glu Val Pro Ser Tyr Phe Leu Cys Pro Ile
1               5                   10                  15
Ser Leu Glu Ile Met Arg Asp Pro Val Thr Leu Ala Thr Gly Ile Thr
            20                  25                  30
Tyr Asp Arg Ser Ser Ile Glu Arg Trp Met Phe Gly Gly Gly Gly Gly
        35                  40                  45
Gly Gly Gly Lys Gly Thr Cys Pro Val Thr Arg Arg Gln Leu Ala Pro
    50                  55                  60
Ala Glu Arg Glu Ala Thr Pro Asn His Thr Leu Arg Arg Leu Ile Gln
65                  70                  75                  80
Ala Trp Gly Arg Arg Arg Cys Ser Ala Ala Ala Ala Gly Ser Gly Ser
                85                  90                  95
Ser Pro Arg Ser Gly Glu Ile Lys Ala Ile Ala Ala Glu Ser Asp Arg
            100                 105                 110
Asn Lys Arg Cys Val Glu Ala Thr Pro Gly Ala Val Glu Phe Leu Val
        115                 120                 125
Ser Val Val Val Gln Ser His Ala Ala Ala Ser Thr Ser Ala Ser Ser
    130                 135                 140
Asp Asp Asp Asp Leu Phe Asp Ser Val Ile Asp Ser Pro Met Ser Thr
145                 150                 155                 160
Ser Ser Pro Glu Glu Glu Ala Leu Gly Val Leu Tyr Ser Leu Lys Pro
                165                 170                 175
Ser Glu Pro Thr Leu Arg Arg Val Leu Gly Lys Asp Asn Gly Val Gly
            180                 185                 190
Phe Leu Asp Thr Leu Ala Ser Val Leu Arg Arg Pro Ser Tyr Arg Ser
        195                 200                 205
Arg Ala Tyr Ala Ile Leu Leu Leu Lys Ala Val Thr Ser Ala Met Pro
    210                 215                 220
Pro Glu Arg Leu Met Ala Val Ser Pro Glu Leu Val Glu Glu Val Val
225                 230                 235                 240
Arg Val Val Ser Asp Gly Val Ser Ser Lys Ala Val Lys Ala Ala Leu
                245                 250                 255
His Val Leu Cys Arg Leu Cys Pro Trp Gly Arg Asn Arg Val Lys Ala
            260                 265                 270
Val Glu Ala Gly Ala Val Ala Ala Leu Val Glu Leu Leu Leu Asp Glu
        275                 280                 285
Glu Gly Gly Gly Gly Arg Arg Arg Ala Ala Glu Leu Ala Val Val Ala
    290                 295                 300
Ile Asp His Leu Cys Gly Cys Ala Glu Gly Arg Ser Glu Leu Val Ala
305                 310                 315                 320
His Pro Ala Gly Leu Ala Val Val Ser Lys Arg Ala Met Arg Val Ser
                325                 330                 335
Pro Ala Ala Thr Glu Ser Ala Val Arg Ala Leu His Ala Val Ala Arg
            340                 345                 350
Asn Ala Ala Thr Pro Ala Val Leu Gln Glu Met Leu Ala Val Gly Val
        355                 360                 365
Val Ala Lys Leu Leu Leu Val Leu Gln Ala Asp Gly Gly Glu Arg Ala
    370                 375                 380
Arg Ala Arg Ala Arg Glu Met Leu Arg Ala Asn Ala Arg Val Trp Lys
385                 390                 395                 400
Asp Ser Pro Cys Leu Gln Ala His Leu Lys Ala Ser Tyr Pro Ser
                405                 410                 415
```

<210> 99
<211> 1215
<212> DNA
<213> Oryza sativa

<400> 99

```
atggcgagcg cggcggccga ggtgccgtcc tacttcgtct gcccgatctc gctgcagctc      60
atgcgcgacc ccgtcacgct ccctacgggg atctcgtacg accgcgccgc gatcgcgcgg     120
tggctcgccg cgccggggc ccgccggacg tgtcccgtca cgcggcagcc gctcgagcac      180
gggctggagc tcacgcccaa ccacaccctg cgccgcctga tccagtcgtg ggcggcctcc     240
gtctcccccg ggtcggccgt ggacgaggag gtggccgcgc tacgaccggt ttcgagcgac     300
gaggttgcgt cgctcctgtc cgacgccgcc gcggcgcagg tgggcgcgct caggaggctg     360
cgggagctgg cggcggagtg cgaggatagc agggcaatgc tggagtccca gggcggggtg     420
ttcgacgtgc tatctcgcgt cgtgaccagc ggcagcgcct gctcgacggc gcgggaggag     480
gcggttggtg tcctcgcgtc gctccggatc ccggagcagg agctgatcgg cgtctcgacc     540
aggcacggca acctggcgga gtccctcacg gccgtgctcc gctcgtcgaa tctccagtcg     600
cgggcgcacg cggtgcagct cgtgaggacg ctcgccgacg cggtggtccc ggcgtgggtg     660
atcggcctca acgcggagct cctcgccgag gtggtcggcg tggtccgcga ccgcgtctcg     720
```

```
gcgcgggcca ccaaggcgtc cctccacgcg ctcgccgcgc tctgcccgta cggccggcac     780
cgcgtgaaga tcgtgggcgc cggcgcggtg gcggcgctgg tggagctgct gctggacgag     840
cccgagcggc gcgtgtgcga gctggcgctg gccgtgctgg accggctgtg cacgtgcgcg     900
gaggggcgcg cggagctggt ggcgcacgcg gcgggcgtgg ccgtggtggg gaagaaggtg     960
ctgcgggtgt cggaggcggc gagcgagcgg gcggtgcgcg tgctgcggtc ggtggcgcgg    1020
cacgcggcga cgccggcggt gctgcaggag atggcgcagt gcggcgtggt ggggaagctg    1080
tgcctggcgc tgcggtcgga gcagtgcggg gtgaagacca aggagaaggc gcacgaggtg    1140
ctcaagctgc actccagggt ctggagggcc tcgccatgct tgtctcccag cttcttggcg    1200
ctttacccat catga                                                     1215
```

```
<210>  100
<211>  404
<212>  PRT
<213>  Oryza sativa

<400>  100
Met Ala Ser Ala Ala Ala Glu Val Pro Ser Tyr Phe Val Cys Pro Ile
1               5                   10                  15
Ser Leu Gln Leu Met Arg Asp Pro Val Thr Leu Pro Thr Gly Ile Ser
            20                  25                  30
Tyr Asp Arg Ala Ala Ile Ala Arg Trp Leu Ala Ala Pro Gly Ala Arg
        35                  40                  45
Arg Thr Cys Pro Val Thr Arg Gln Pro Leu Glu His Gly Leu Glu Leu
    50                  55                  60
Thr Pro Asn His Thr Leu Arg Arg Leu Ile Gln Ser Trp Ala Ala Ser
65                  70                  75                  80
Val Ser Pro Gly Ser Ala Val Asp Glu Glu Val Ala Ala Leu Arg Pro
                85                  90                  95
Val Ser Ser Asp Glu Val Ala Ser Leu Leu Ser Asp Ala Ala Ala Ala
            100                 105                 110
Gln Val Gly Ala Leu Arg Arg Leu Arg Glu Leu Ala Ala Glu Cys Glu
        115                 120                 125
Asp Ser Arg Ala Met Leu Glu Ser Gln Gly Gly Val Phe Asp Val Leu
    130                 135                 140
Ser Arg Val Val Thr Ser Gly Ser Ala Cys Ser Thr Ala Arg Glu Glu
145                 150                 155                 160
Ala Val Gly Val Leu Ala Ser Leu Arg Ile Pro Glu Gln Glu Leu Ile
                165                 170                 175
Gly Val Ser Thr Arg His Gly Asn Leu Ala Glu Ser Leu Thr Ala Val
            180                 185                 190
Leu Arg Ser Ser Asn Leu Gln Ser Arg Ala His Ala Val Gln Leu Val
        195                 200                 205
Arg Thr Leu Ala Asp Ala Val Val Pro Ala Trp Val Ile Gly Leu Asn
    210                 215                 220
Ala Glu Leu Leu Ala Glu Val Val Gly Val Val Arg Asp Arg Val Ser
225                 230                 235                 240
Ala Arg Ala Thr Lys Ala Ser Leu His Ala Leu Ala Ala Leu Cys Pro
                245                 250                 255
Tyr Gly Arg His Arg Val Lys Ile Val Gly Ala Gly Ala Val Ala Ala
            260                 265                 270
Leu Val Glu Leu Leu Leu Asp Glu Pro Glu Arg Arg Val Cys Glu Leu
        275                 280                 285
Ala Leu Ala Val Leu Asp Arg Leu Cys Thr Cys Ala Glu Gly Arg Ala
    290                 295                 300
Glu Leu Val Ala His Ala Ala Gly Val Ala Val Val Gly Lys Lys Val
305                 310                 315                 320
Leu Arg Val Ser Glu Ala Ala Ser Glu Arg Ala Val Arg Val Leu Arg
                325                 330                 335
Ser Val Ala Arg His Ala Ala Thr Pro Ala Val Leu Gln Glu Met Ala
            340                 345                 350
Gln Cys Gly Val Val Gly Lys Leu Cys Leu Ala Leu Arg Ser Glu Gln
        355                 360                 365
Cys Gly Val Lys Thr Lys Glu Lys Ala His Glu Val Leu Lys Leu His
    370                 375                 380
Ser Arg Val Trp Arg Ala Ser Pro Cys Leu Ser Pro Ser Phe Leu Ala
385                 390                 395                 400
Leu Tyr Pro Ser
```

```
<210>  101
<211>  1416
<212>  DNA
<213>  Oryza sativa
```

<400> 101
cccacgcgtc cgcccacgcg tccgggacac cagaaacata gtacacttga gctcactcca 60
aactcaaaca ctcacaccaa tggctctcca agttcaggcc gcactcctgc cctctgctct 120
ctctgtcccc aagaagggta acttgagcgc ggtggtgaag gagccggggt tccttagcgt 180
gagcagaagg ccaagaagcc gtcgctggtg gtgagggcgg tggcgacgcg gcgggccggt 240
ggcgagcccc ggcgcgggca cgtcgaaggc ggacgggaag aagacgctgc ggcagggggt 300
ggtggtgatc accggcgcgt cgtcggggct cgggctcgcg gcggcgaagg cgcttggcgg 360
agacggggaa gtggcacgtg gtgatggcgt tccgcgactt tcctgaaggc ggcgacggcg 420
gcgaaggcgg cggggatggc ggcggggagc tacaccgtca tgcacctgga cctcgcctcc 480
ctcgacagcg tccgccagtt cgtggacaac ttccggcgct ccggcatgcc gctcgacgcg 540
ctggtgtgca acgccgcaca tctaccggcc gacggcgcgg caaccgacgt tcaacgccga 600
cgggtacgag atgagcgtcg ggtgaacca cctgggccac ttcctcctcg cccgcctcat 660
gctcgacgac ctcaagaaat ccgactaccc gtcgcggcgg ctcatcatcc tcggctccat 720
caccggcaac accaacacct tcgccggcaa cgtccctccc aaggccgggc taggcgacct 780
ccgggggctc gccggcgggc tccgcgggca gaacgggtcg gcgatgatcg acggcgcgga 840
gagcttcgac ggcgccaagg cgtacaagga cagcaagatc tgtaacatgc tgacgatgca 900
ggagttccac cggagattcc acgaggagac cgggatcacg ttcgcgtcgc tgtacccggg 960
gtgcatcgcg acgacgggct tgttccgcga gcacatcccg ctgttccggc tgctgttccc 1020
gccgttccag cggttcgtga cgaagggggtt cgtgtcggag gcggagtccg ggaagcggct 1080
ggcgcaggtg gtgggcgacc cgagcctgac caagtccggc gtgtactgga gctggaacaa 1140
ggactcggcg tcgttcgaga accagctctc gcaggaggcc agcgacccgg agaaggccag 1200
gaagctctgg gacctcagcg agaagctcgt cggcctcgtc tgagtttatt atttacccat 1260
tcgtttcaac tgttaatttc ttcggggttt agggggtttc agctttcagt gagagaggcc 1320
tgtcaagtga tgtacaatta gtaatttttt tttacccgac aaatcatgca ataaaaccac 1380
aggcttacat tatcgatttg tccacctaaa ttaagt 1416

<210> 102
<211> 55
<212> DNA
<213> Artificial sequence

<220>
<223> primer: prm9075

<400> 102
ggggacaagt ttgtacaaaa aagcaggctt aaacaatgga agaaattcaa gtacc 55

<210> 103
<211> 51
<212> DNA
<213> Artificial sequence

<220>
<223> primer: prm8785

<400> 103
ggggaccact ttgtacaaga aagctgggtg ctcttttggt cctattttcc t 51

<210> 104
<211> 801
<212> DNA
<213> Pseudomonas fluorescens

<400> 104
tttgtacaaa aaagcaggct taaacaatga ctgacacccc aatgtccccc gccgaattcg 60
aagcggccct gcgcgccaag ggcgcctatt accacatcta tcacccgtat cacgtggcga 120
tgtatgaagg ccgggcgact cgcgagcaga tccagggctg ggtcgccaac cgcttttact 180
atcaggtgaa catcccgctc aaggacgccg cgatcctggc caactgcccg gaccgcgaaa 240
tccgtcgcga gtggattcaa cgcctgttgg accatgacgg cgcccccggt gaagacggcg 300
gtatcgaagc ctggttgcgt cttgggcaag cggtcggcct cgacccggat cagttgcgct 360
cccaggaact ggtgctgccc ggcgtgcgtt tcgccgtgga tgcctacgtc aacttcgccc 420
gccgggcccg ttggcaggaa gccgccagca gctcattgac cgagctgttt gcgccgcaga 480
tccaccaatc gcgcctcgac agttggccgc agcattaccc gtggatcgac ccggccggtt 540
acgaatattt ccgtacgcgc ctgggccagg ctcgccggga tgtggagcac ggtctggcaa 600
tcacgttgca gcactacacc acgcgggaag gccaggagcg catgctggaa attctccagt 660
tcaaactgga cattctttgg agcatgctcg acgccatgag catggcctac gaactgaacc 720
gcccgccgta ccacagcgtg accgaacagc gggtctggca taaaggaatc accttatgaa 780
cccagctttc ttgtacaaag t 801

<210> 105
<211> 250
<212> PRT
<213> Pseudomonas fluorescens

<400> 105
Met Thr Asp Thr Pro Met Ser Pro Ala Glu Phe Glu Ala Ala Leu Arg
1               5                   10                  15
Ala Lys Gly Ala Tyr Tyr His Ile Tyr His Pro Tyr His Val Ala Met
            20                  25                  30
Tyr Glu Gly Arg Ala Thr Arg Glu Gln Ile Gln Gly Trp Val Ala Asn
        35                  40                  45
Arg Phe Tyr Tyr Gln Val Asn Ile Pro Leu Lys Asp Ala Ala Ile Leu
    50                  55                  60
Ala Asn Cys Pro Asp Arg Glu Ile Arg Arg Glu Trp Ile Gln Arg Leu
65                  70                  75                  80
Leu Asp His Asp Gly Ala Pro Gly Glu Asp Gly Gly Ile Glu Ala Trp
                85                  90                  95
Leu Arg Leu Gly Gln Ala Val Gly Leu Asp Pro Asp Gln Leu Arg Ser
            100                 105                 110
Gln Glu Leu Val Leu Pro Gly Val Arg Phe Ala Val Asp Ala Tyr Val
        115                 120                 125
Asn Phe Ala Arg Arg Ala Arg Trp Gln Glu Ala Ala Ser Ser Ser Leu
    130                 135                 140
Thr Glu Leu Phe Ala Pro Gln Ile His Gln Ser Arg Leu Asp Ser Trp
145                 150                 155                 160
Pro Gln His Tyr Pro Trp Ile Asp Pro Ala Gly Tyr Glu Tyr Phe Arg
                165                 170                 175
Thr Arg Leu Gly Gln Ala Arg Arg Asp Val Glu His Gly Leu Ala Ile
            180                 185                 190
Thr Leu Gln His Tyr Thr Thr Arg Glu Gly Gln Glu Arg Met Leu Glu
        195                 200                 205
Ile Leu Gln Phe Lys Leu Asp Ile Leu Trp Ser Met Leu Asp Ala Met
    210                 215                 220
Ser Met Ala Tyr Glu Leu Asn Arg Pro Pro Tyr His Ser Val Thr Glu
225                 230                 235                 240
Gln Arg Val Trp His Lys Gly Ile Thr Leu
                245                 250

<210> 106
<211> 753
<212> DNA
<213> Pseudomonas fluorescens

<400> 106
atgaccgaca ccccgctgtc cccccgccgaa ttcgaagcgg ccctgcgcgc caagggcgcc 60
tattaccaca ttcatcaccc gtatcacgtg gcgatgtatg aaggccgggc cacccgtgag 120
cagatacagg gctgggtcgc caaccgcttc tactatcagg tgaacatccc cctgaaagac 180
gcggcgatcc tcgccaactg cccggatcgc gagatccgtc gcgagtggat tcagcgcctg 240
ctcgaccacg acggcgcgcc cggtgaagat ggcggcatcg aagcctggct gcgcctcggc 300
caggccgtcg gtctcgatcc ggatcaattg cgctcccagg aactggtgct gcccggcgtg 360
cgtttcgccg tcgacgccta cgtcaacttc gcccgccgcg ccagttggca ggaagccgcc 420
agcagctcgc tgaccgagct gttcgcgccg cagatccacc agtcgcgcct cgacagctgg 480
ccgcagcatt acccgtggat cgacccggcc ggctacgaat atttccgcac ccgtctcggc 540
caggcccggc gcgatgtcga gcatggtctg gcgatcacgc tggagcacta caagacccgc 600
gaaggccagg aacgcatgct ggaaattctc cagttcaaac tggacatcct ttggagcatg 660
ctcgatgcca tgagcatggc ctacgaactg aaccgcccgc cgtatcacag cgtcaccgat 720
caacgggtct ggcataaagg aatcgcctta tga 753

<210> 107
<211> 250
<212> PRT
<213> Pseudomonas fluorescens

<400> 107
Met Thr Asp Thr Pro Leu Ser Pro Ala Glu Phe Glu Ala Ala Leu Arg
1               5                   10                  15
Ala Lys Gly Ala Tyr Tyr His Ile His His Pro Tyr His Val Ala Met
            20                  25                  30
Tyr Glu Gly Arg Ala Thr Arg Glu Gln Ile Gln Gly Trp Val Ala Asn
        35                  40                  45
Arg Phe Tyr Tyr Gln Val Asn Ile Pro Leu Lys Asp Ala Ala Ile Leu
    50                  55                  60
Ala Asn Cys Pro Asp Arg Glu Ile Arg Arg Glu Trp Ile Gln Arg Leu
65                  70                  75                  80
Leu Asp His Asp Gly Ala Pro Gly Glu Asp Gly Gly Ile Glu Ala Trp
                85                  90                  95
Leu Arg Leu Gly Gln Ala Val Gly Leu Asp Pro Asp Gln Leu Arg Ser
            100                 105                 110

```
Gln Glu Leu Val Leu Pro Gly Val Arg Phe Ala Val Asp Ala Tyr Val
        115                 120                 125
Asn Phe Ala Arg Arg Ala Ser Trp Gln Glu Ala Ala Ser Ser Ser Leu
    130                 135                 140
Thr Glu Leu Phe Ala Pro Gln Ile His Gln Ser Arg Leu Asp Ser Trp
145                 150                 155                 160
Pro Gln His Tyr Pro Trp Ile Asp Pro Ala Gly Tyr Glu Tyr Phe Arg
                165                 170                 175
Thr Arg Leu Gly Gln Ala Arg Arg Asp Val Glu His Gly Leu Ala Ile
            180                 185                 190
Thr Leu Glu His Tyr Lys Thr Arg Glu Gly Gln Glu Arg Met Leu Glu
        195                 200                 205
Ile Leu Gln Phe Lys Leu Asp Ile Leu Trp Ser Met Leu Asp Ala Met
    210                 215                 220
Ser Met Ala Tyr Glu Leu Asn Arg Pro Pro Tyr His Ser Val Thr Asp
225                 230                 235                 240
Gln Arg Val Trp His Lys Gly Ile Ala Leu
                245                 250
```

```
<210>  108
<211>  753
<212>  DNA
<213>  Pseudomonas fluorescens

<400>  108
atgaccgaca ccccgctgtc cgccgccgaa ttcgagcagg cactgcgcgc caaaggcgcc      60
tactaccaca tccatcaccc ttatcacgtg gcgatgtatg aaggccgggc gacccgcgag     120
cagatccagg gctgggtcgc caaccggttc tattaccagg tgaacatccc catgaaggac     180
gcggcgatcc tcgccaactg tccggaccgg gagattcgcc gcgaatggat ccagcgcctg     240
ctggaccatg acggcgcccc gggcgaggac ggcggcatcg aggcctggct gcgcctgggc     300
caggccgtgg gcctggaccc ggaccagttg cgctcccagg aactggtgct gcccggtgtg     360
cgctttgccg tggacgccta tgtcaatttt gctcgccggg ccagttggca ggaggcggcc     420
agcagctcgc tgaccgaact gttcgcgccg caaatccacc agtcgcgcct ggacagctgg     480
ccccagcatt acccatggat cgacccccact ggctacgagt acttccgcac ccgcctgggc     540
caggcccgcc gtgatgtcga gcacggcctg gcgatcaccc tgcagcacta caccacccgt     600
gccggccagg aacgcatgct ggaaattctc cagttcaaac tggacatcct ttggagcatg     660
ctcgacgcca tgagcatggc ctatgaactg aaccgcccgc cctatcacag cgtcaccgat     720
cagcgggtct ggcataaggg gatcacccta tga                                  753
```

```
<210>  109
<211>  250
<212>  PRT
<213>  Pseudomonas fluorescens

<400>  109
Met Thr Asp Thr Pro Leu Ser Ala Ala Glu Phe Glu Gln Ala Leu Arg
1               5                   10                  15
Ala Lys Gly Ala Tyr Tyr His Ile His His Pro Tyr His Val Ala Met
            20                  25                  30
Tyr Glu Gly Arg Ala Thr Arg Glu Gln Ile Gln Gly Trp Val Ala Asn
        35                  40                  45
Arg Phe Tyr Tyr Gln Val Asn Ile Pro Met Lys Asp Ala Ala Ile Leu
    50                  55                  60
Ala Asn Cys Pro Asp Arg Glu Ile Arg Arg Glu Trp Ile Gln Arg Leu
65                  70                  75                  80
Leu Asp His Asp Gly Ala Pro Gly Glu Asp Gly Gly Ile Glu Ala Trp
                85                  90                  95
Leu Arg Leu Gly Gln Ala Val Gly Leu Asp Pro Asp Gln Leu Arg Ser
            100                 105                 110
Gln Glu Leu Val Leu Pro Gly Val Arg Phe Ala Val Asp Ala Tyr Val
        115                 120                 125
Asn Phe Ala Arg Arg Ala Ser Trp Gln Glu Ala Ala Ser Ser Ser Leu
    130                 135                 140
Thr Glu Leu Phe Ala Pro Gln Ile His Gln Ser Arg Leu Asp Ser Trp
145                 150                 155                 160
Pro Gln His Tyr Pro Trp Ile Asp Pro Thr Gly Tyr Glu Tyr Phe Arg
                165                 170                 175
Thr Arg Leu Gly Gln Ala Arg Arg Asp Val Glu His Gly Leu Ala Ile
            180                 185                 190
Thr Leu Gln His Tyr Thr Thr Arg Ala Gly Gln Glu Arg Met Leu Glu
        195                 200                 205
Ile Leu Gln Phe Lys Leu Asp Ile Leu Trp Ser Met Leu Asp Ala Met
    210                 215                 220
Ser Met Ala Tyr Glu Leu Asn Arg Pro Pro Tyr His Ser Val Thr Asp
```

```
225                 230                 235                 240
Gln Arg Val Trp His Lys Gly Ile Thr Leu
                245                 250
```

```
<210>  110
<211>  756
<212>  DNA
<213>  Pseudomonas syringae

<400>  110
atgagcgaag cgactgcgct gtcccccgcc gaattcgaac aggccttgcg cgccaagggt      60
gcctattacc acatctatca cccgttccat gtggcgatgt acgagggccg agcgacccgc     120
gagcagattc agggctgggt cgccaaccgt ttctactatc aggtgaacat tccgctcaag     180
gacgcggcga ttctggccaa ctgcccggat cgcgagatcc gtcgcgagtg gattcagcgc     240
ctgctcgatc atgacggtgc gcccggcgaa gacggcggca tcgaagcctg gctgcgcctc     300
ggccaggccg tgggactgga cccggaccag ctgcgctcgc aggaactggt gctgccaggc     360
gtgcgctttg ccgtcgacgc ctacgtgaac ttcgcccgcc gcgccaactg gcaggaagca     420
gccagcagct cgctgaccga actgttcgcc ccgcagatcc accagtcgcg tctggacagc     480
tggccgcagc actacccatg gatcgacccg gcaggctatg agtatttccg cactcgcctg     540
ggtcaggccc gacgcgatgt agagcacggc ctcgccatca cgttacagca ctacactacg     600
tacgaaggtc agcagcgtat gctggaaatc ctgcagttca aactcgatat cctgtggagc     660
atgctcgatg cgatgtccat ggcctacgag ctgaaccgcc cgccgtatca cagcgtcaca     720
gaccaaaggg tctggcataa ggggattacc ctatga                               756
```

```
<210>  111
<211>  251
<212>  PRT
<213>  Pseudomonas syringae

<400>  111
Met Ser Glu Ala Thr Ala Leu Ser Pro Ala Glu Phe Glu Gln Ala Leu
1               5                   10                  15
Arg Ala Lys Gly Ala Tyr Tyr His Ile Tyr His Pro Phe His Val Ala
            20                  25                  30
Met Tyr Glu Gly Arg Ala Thr Arg Glu Gln Ile Gln Gly Trp Val Ala
        35                  40                  45
Asn Arg Phe Tyr Tyr Gln Val Asn Ile Pro Leu Lys Asp Ala Ala Ile
    50                  55                  60
Leu Ala Asn Cys Pro Asp Arg Glu Ile Arg Arg Glu Trp Ile Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
        115                 120                 125
Val Asn Phe Ala Arg Arg Ala Asn Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Ala
            180                 185                 190
Ile Thr Leu Gln His Tyr Thr Thr Tyr Glu Gly Gln Gln Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu Asn Arg Pro Pro Tyr His Ser Val Thr
225                 230                 235                 240
Asp Gln Arg Val Trp His Lys Gly Ile Thr Leu
                245                 250
```

```
<210>  112
<211>  756
<212>  DNA
<213>  Pseudomonas syringae

<400>  112
atgagcgaag cgactgcgct gtcccccgcc gaattcgaac aggccttgcg cgccaagggt      60
gcctattacc acatctatca cccgtaccat gtggcgatgt acgagggtcg tgcaacccgc     120
gagcagattc agggctgggt cgccaaccgt ttctactatc aggtgaacat tccgctcaag     180
gacgcggcga ttctggccaa ctgcccggat cgcgagatcc gtcgcgagtg gattcagcgc     240
ctgctcgatc atgacggtgc gcccggcgaa gacggcggca ttgaagcctg gctgcgcctc     300
```

```
ggccaggccg tggggctcga cccggaccaa ctgcgctctc aggaactggt gctgccaggc    360
gtgcgttttg ccgtcgacgc ctacgtgaac ttcgcccgcc gcgccaactg gcaggaagcg    420
gccagcagct cgctgaccga actgttcgcg ccgcagatcc accaatcgcg cctggacagc    480
tggccgcagc attacccgtg gatcgacccg gcaggctacg agtatttccg cactcgcctg    540
ggccaggccc gacgcgacgt ggaacacggc ctcgccatca cgttacagca ctacactaca    600
tatgaaggcc agcagcgcat gctggaaatc ctgcagttca agctcgatat cctgtggagc    660
atgctcgatg cgatgtccat ggcctacgag ctgaaccgcc cgccgtatca cagcgttaca    720
gaccaaaagg tctggcataa ggggattacg ctatga                              756
```

<210> 113
<211> 251
<212> PRT
<213> Pseudomonas syringae

<400> 113

```
Met Ser Glu Ala Thr Ala Leu Ser Pro Ala Glu Phe Glu Gln Ala Leu
1               5                   10                  15
Arg Ala Lys Gly Ala Tyr Tyr His Ile Tyr His Pro Tyr His Val Ala
            20                  25                  30
Met Tyr Glu Gly Arg Ala Thr Arg Glu Gln Ile Gln Gly Trp Val Ala
        35                  40                  45
Asn Arg Phe Tyr Tyr Gln Val Asn Ile Pro Leu Lys Asp Ala Ala Ile
    50                  55                  60
Leu Ala Asn Cys Pro Asp Arg Glu Ile Arg Arg Glu Trp Ile Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
        115                 120                 125
Val Asn Phe Ala Arg Arg Ala Asn Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Ala
            180                 185                 190
Ile Thr Leu Gln His Tyr Thr Thr Tyr Glu Gly Gln Gln Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu Asn Arg Pro Pro Tyr His Ser Val Thr
225                 230                 235                 240
Asp Gln Lys Val Trp His Lys Gly Ile Thr Leu
                245                 250
```

<210> 114
<211> 756
<212> DNA
<213> Pseudomonas putida

<400> 114

```
tcatagggtg attcccttgt gccacacccg ttcctgggtg acggagtgat agggcgggcg     60
gttcagttcg taggccatgc tcatggcatc gagcatactc cagaggatat ccagcttgaa    120
ctgcagtatc tccagcatac gctcctggcc cgcacgggtg gtgtagtgct gcagggtaat    180
cgccaggccg tgttccacat cgcgtcgcgc ctggcccagg cgggtacgga aatactcata    240
gccggcgggg tcgatccacg ggtagtgctg cggccagctg tccaggcgtg actggtggat    300
ttgcggggcg aacagctcgg tcagcgaact gctggcggct tcctgccagc tggcgcggcg    360
ggcaaagttg acgtaggcgt ccacggcaaa gcgcacgcca ggcaatacca gttcttgcga    420
gcgcaattgg tccgggtcca gcccgacagc ctggcccagg cgcagccagg cttcgatgcc    480
gccatcctcg ccgggcgcac cgtcgtggtc gagcaggcgc tggatccact cgcggcgcac    540
ttcgcggtcc gggcagttgg ccaggatcgc ggcatccttc atcgggatgt tgacctggta    600
gtagaagcgg ttggccaccc agccctggat ctgctcgcgg gtggcgcgcc cctggtacat    660
cgccacgtgg tacgggtgat ggatgtggta ataggcgccc ttggcgcgca gggcctgctc    720
gaactcggca ggggacattg gcagtgcgtc gctcat                              756
```

<210> 115
<211> 251
<212> PRT
<213> Pseudomonas putida

<400> 115

```
Met Ser Asp Ala Leu Pro Met Ser Pro Ala Glu Phe Glu Gln Ala Leu
1               5                   10                  15
Arg Ala Lys Gly Ala Tyr Tyr His Ile His His Pro Tyr His Val Ala
            20                  25                  30
Met Tyr Gln Gly Arg Ala Thr Arg Glu Gln Ile Gln Gly Trp Val Ala
        35                  40                  45
Asn Arg Phe Tyr Tyr Gln Val Asn Ile Pro Met Lys Asp Ala Ala Ile
    50                  55                  60
Leu Ala Asn Cys Pro Asp Arg Glu Val Arg Arg Glu Trp Ile Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
        115                 120                 125
Val Asn Phe Ala Arg Arg Ala Ser Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Ala
            180                 185                 190
Ile Thr Leu Gln His Tyr Thr Thr Arg Ala Gly Gln Glu Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu Asn Arg Pro Pro Tyr His Ser Val Thr
225                 230                 235                 240
Gln Glu Arg Val Trp His Lys Gly Ile Thr Leu
                245                 250
```

<210> 116
<211> 756
<212> DNA
<213> Pseudomonas putida

<400> 116

```
atgagcgacg cactgcccat gtcccctgcc gaattcgagc aggctctgcg cgccaaaggc     60
gcctattacc acatccatca cccgtaccat gtggcgatgt accaaggccg cgccacccgc    120
gagcagatcc agggctgggt ggccaaccgt ttctactacc aggtcaacat cccgatgaag    180
gatgccgcga tcctggccaa ctgcccggac cgcgaagtgc gccgagagtg gatccagcgc    240
ctgctcgacc acgacggcgc acccggcgag gacggtggca tcgaagcctg gctgcgcctg    300
ggccaggccg tcggcctcga ccccgaccag ctgcgctccc aggagctggt actgcccggc    360
gtacgctttg ccgtggacgc ctacgtcaac ttcgcccgcc gcgccagctg gcaggaagcc    420
gccagcagct cgctgaccga actgtttgcc ccacaaatcc accagtcgcg gctggacagt    480
tggccacagc actacccgtg gatcgacccg gccggctacg agtacttccg cacccgcctt    540
ggccaggccc ggcgcgacgt ggagcacggc ctggcgatta ccctgcagca ctacaccacc    600
cgcgcgggcc aggagcgtat gctggagatc ctgcagttca agctggatat cctctggagc    660
atgctcgacg ccatgagcat ggcctacgag ctgaaccgcc cgccttatca ctccgtcacc    720
caggaccggg tgtggcacaa ggggatcacc ctatga                                756
```

<210> 117
<211> 251
<212> PRT
<213> Pseudomonas putida

<400> 117

```
Met Ser Asp Ala Leu Pro Met Ser Pro Ala Glu Phe Glu Gln Ala Leu
1               5                   10                  15
Arg Ala Lys Gly Ala Tyr Tyr His Ile His His Pro Tyr His Val Ala
            20                  25                  30
Met Tyr Gln Gly Arg Ala Thr Arg Glu Gln Ile Gln Gly Trp Val Ala
        35                  40                  45
Asn Arg Phe Tyr Tyr Gln Val Asn Ile Pro Met Lys Asp Ala Ala Ile
    50                  55                  60
Leu Ala Asn Cys Pro Asp Arg Glu Val Arg Arg Glu Trp Ile Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
```

```
            115                 120                 125
Val Asn Phe Ala Arg Arg Ala Ser Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Ala
            180                 185                 190
Ile Thr Leu Gln His Tyr Thr Thr Arg Ala Gly Gln Glu Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu Asn Arg Pro Pro Tyr His Ser Val Thr
225                 230                 235                 240
Gln Asp Arg Val Trp His Lys Gly Ile Thr Leu
                245                 250
```

<210> 118
<211> 756
<212> DNA
<213> Pseudomonas putida

<400> 118

```
atgagcgacg cactgcccat gtcccctgcc gaattcgagc aggctctgcg cgccaaaggc      60
gcctattacc acatccatca cccgtaccat gtggcgatgt accaaggccg cgccacccgc     120
gagcagatcc agggctgggt ggccaaccgt ttctactacc aggtcaacat cccgatgaag     180
gatgccgcga tcctggccaa ctgcccggac cgcgaagtgc gccgagagtg gatccagcgc     240
ctgctcgacc acgacggcgc acccggcgag gacggtggca tcgaagcctg gctgcgcctg     300
ggccaggccg tcggcctcga ccccgaccag ctgcgctccc aggagctggt actgcccggc     360
gtacgctttg ccgtggacgc ctacgtcaac ttcgcccgcc gcgccagctg gcaggaagcc     420
gccagcagct cgctgaccga actgtttgcc ccacaaatcc accagtcgcg gctggacagt     480
tggccacagc actacccgtg gatcgacccg gccggctacg agtacttccg cacccgcctt     540
ggccaggccc ggcgcgacgt ggagcacggc ctggcgatta ccctgcagca ctacaccacc     600
cgcgcgggcc aggagcgtat gctggagatc ctgcagttca agctggatat cctctggagc     660
atgctcgacg ccatgagcat ggcctacgag ctgaaccgcc cgccttatca ctccgtcacc     720
caggaccggg tgtggcacaa ggggatcacc ctatga                               756
```

<210> 119
<211> 251
<212> PRT
<213> Pseudomonas putida

<400> 119

```
Met Ser Asp Ala Leu Pro Met Ser Pro Ala Glu Phe Glu Gln Ala Leu
1               5                   10                  15
Arg Ala Lys Gly Ala Tyr Tyr His Ile His His Pro Tyr His Val Ala
            20                  25                  30
Met Tyr Gln Gly Arg Ala Thr Arg Glu Gln Ile Gln Gly Trp Val Ala
        35                  40                  45
Asn Arg Phe Tyr Tyr Gln Val Asn Ile Pro Met Lys Asp Ala Ala Ile
    50                  55                  60
Leu Ala Asn Cys Pro Asp Arg Glu Val Arg Arg Glu Trp Ile Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
        115                 120                 125
Val Asn Phe Ala Arg Arg Ala Ser Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Ala
            180                 185                 190
Ile Thr Leu Gln His Tyr Thr Thr Arg Ala Gly Gln Glu Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu Asn Arg Pro Pro Tyr His Ser Val Thr
225                 230                 235                 240
```

Gln Glu Arg Met Trp His Lys Gly Ile Thr Leu
245 250

<210> 120
<211> 756
<212> DNA
<213> Pseudomonas syringae

<400> 120
tcatggcgta atccccttat gccagacctt ctggtctgtg acgctgtggt acggggggcg 60
gttcaattca tacgccatgg acatggcatc gagcatgctc cacagaatgt cgagtttgaa 120
ctgcaggatt tccagcatgc gctgctggcc ttcatacgta gtgtagtgct gtaacgtgat 180
tgcgaggccg tgttccacat cgcgacgtgc ctgacccagg cgagtacgga aatactcgta 240
acctgtgggg tcaatccacg ggtaatgctg tggccagctg tccaggcgcg attgatgaat 300
ctgcggtgcg aacaactcgg tcagcgagct gctggcggcc tcttgccagt tggcgcggcg 360
ggcgaagttc acgtaggcat cgaccgcaaa gcgcacaccg ggcagtacca gttcctgcga 420
gcgcaactga tccgggtcca gccccacggc ctcgccgagg cgcagccagg cttcgatgcc 480
gccgtcttca ccgggcgcgc cgtcgtggtc cagcaggcgc tgaatccact cgcgacgaat 540
ctcgcgatcc gggcaattgg ccagaatggc ggcgtccttg agcgggatgt tcacctgata 600
gtaaaagcgg ttggcgaccc agccctgaat ctgctcgcgg gtcgcgcggc cttcatacat 660
cgccacatga aacggatgat agatgtggta ataggcgccc ttggcacgta aggcctgctc 720
gaattcagcc ggggacagcg cagtcgcttc actcat 756

<210> 121
<211> 251
<212> PRT
<213> Pseudomonas syringae

<400> 121
Met Ser Glu Ala Thr Ala Leu Ser Pro Ala Glu Phe Glu Gln Ala Leu
1 5 10 15
Arg Ala Lys Gly Ala Tyr Tyr His Ile Tyr His Pro Phe His Val Ala
20 25 30
Met Tyr Glu Gly Arg Ala Thr Arg Glu Gln Ile Gln Gly Trp Val Ala
35 40 45
Asn Arg Phe Tyr Tyr Gln Val Asn Ile Pro Leu Lys Asp Ala Ala Ile
50 55 60
Leu Ala Asn Cys Pro Asp Arg Glu Ile Arg Arg Glu Trp Ile Gln Arg
65 70 75 80
Leu Leu Asp His Asp Gly Ala Pro Gly Glu Asp Gly Gly Ile Glu Ala
85 90 95
Trp Leu Arg Leu Gly Glu Ala Val Gly Leu Asp Pro Asp Gln Leu Arg
100 105 110
Ser Gln Glu Leu Val Leu Pro Gly Val Arg Phe Ala Val Asp Ala Tyr
115 120 125
Val Asn Phe Ala Arg Arg Ala Asn Trp Gln Glu Ala Ala Ser Ser Ser
130 135 140
Leu Thr Glu Leu Phe Ala Pro Gln Ile His Gln Ser Arg Leu Asp Ser
145 150 155 160
Trp Pro Gln His Tyr Pro Trp Ile Asp Pro Thr Gly Tyr Glu Tyr Phe
165 170 175
Arg Thr Arg Leu Gly Gln Ala Arg Arg Asp Val Glu His Gly Leu Ala
180 185 190
Ile Thr Leu Gln His Tyr Thr Thr Tyr Glu Gly Gln Gln Arg Met Leu
195 200 205
Glu Ile Leu Gln Phe Lys Leu Asp Ile Leu Trp Ser Met Leu Asp Ala
210 215 220
Met Ser Met Ala Tyr Glu Leu Asn Arg Pro Pro Tyr His Ser Val Thr
225 230 235 240
Asp Gln Lys Val Trp His Lys Gly Ile Thr Pro
245 250

<210> 122
<211> 756
<212> DNA
<213> Pseudomonas entomophila

<400> 122
tcataaggcg attcccttgt gccagacccg ctcgcttgtg acgctgtgat agggcgggcg 60
gttcaattcg taggccatgc tcatggcatc gagcatgctc cagaggatat ccagcttgaa 120
ctgcaggatc tccagcatgc gcgcctggcc ctcgcgggtg gtgtagtgcg ccagggtgat 180
cgccaggcca tgctcgacat cacggcgggc ctggcccagg cgggtgcgga agtattcgta 240
gccggccggg tcgatccacg ggtagtgctg cggccagctg tccaggcgcg actggtggat 300
ctgcggggcg aacagctcgg tcagcgagct gctggcggcc tcctgccagc tggcgcggcg 360

```
ggcgaagttg acgtaggcgt cgacggcgaa gcgcacgccg ggcagcacca gctcctggct    420
gcgcagttgg tccgggtcca ggcccacggc ctggccgaga cgcagccagg cttcgatgcc    480
gccgtcctcg cccggcgcgc cgtcatggtc gagcaggcgc tggacccact cgcggcgcac    540
ctcgcggtcc gggcagttgg ccaggatcgc ggcatccttc atggggatgt tcacctggta    600
gtagaagcgg ttggccaccc agccctggat ctgctcgcgg gtggcgcggc cttcatacat    660
cgccacgtgg taggggtggt ggatgtggta gaaggcgccc ttggcgcgca gggcctgctc    720
gaactcggca ggtgacatcg gtgcggcgtc attcat                              756
```

<210> 123
<211> 251
<212> PRT
<213> Pseudomonas entomophila

<400> 123

```
Met Asn Asp Ala Ala Pro Met Ser Pro Ala Glu Phe Glu Gln Ala Leu
1               5                   10                  15
Arg Ala Lys Gly Ala Phe Tyr His Ile His His Pro Tyr His Val Ala
            20                  25                  30
Met Tyr Glu Gly Arg Ala Thr Arg Glu Gln Ile Gln Gly Trp Val Ala
        35                  40                  45
Asn Arg Phe Tyr Tyr Gln Val Asn Ile Pro Met Lys Asp Ala Ala Ile
    50                  55                  60
Leu Ala Asn Cys Pro Asp Arg Glu Val Arg Arg Glu Trp Val Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
        115                 120                 125
Val Asn Phe Ala Arg Arg Ala Ser Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Ala
            180                 185                 190
Ile Thr Leu Ala His Tyr Thr Thr Arg Glu Gly Gln Ala Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu Asn Arg Pro Pro Tyr His Ser Val Thr
225                 230                 235                 240
Ser Glu Arg Val Trp His Lys Gly Ile Ala Leu
                245                 250
```

<210> 124
<211> 756
<212> DNA
<213> Pseudomonas putida

<400> 124

```
atgagcgagg cactgccact gtcgcctgcc gaattcgagc aggccctgcg cgccaagggc     60
gcctactacc acattcatca cccctaccac gtggcgatgt acgaagggcg cgccacccgc    120
gaacagattc agggctgggt ggccaaccgc ttctattacc aggtgaacat cccgatgaag    180
gatgccgcga tcctggccaa ctgcccggac cgcgaagtgc gccgtgagtg gatccagcgc    240
ctgctcgacc acgacggagc cccgggcgaa gacggtggta tcgaagcctg gctgcgcctg    300
ggccaggccg tggggctcga cccggaccag ctgcgttccc aggagctggt gctgccaggt    360
gtacgcttcg ccgtcgatgc ctacgtgaac ttcgcccgcc gtgccagctg gcaggaagcg    420
gccagcagct cgctgaccga actgttcgcc ccgcagatcc accagtcgcg cctggacagc    480
tggccgcaac actatccgtg gatcgatcct gccggctacg agtacttccg cacccgcctg    540
ggccaggccc ggcgcgatgt cgaacacggt ctgacgatca cattgcagca ctacaccacc    600
cgggcggccc aggagcgtat gctggaaatc ctgcagttca agctggatat cctctggagc    660
atgctcgatg ccatgagcat ggcctacgag ctgcaccgcc cgccgtatca caccgtcacc    720
cagcaacggg tatggcacaa ggggattgcc ctatga                              756
```

<210> 125
<211> 251
<212> PRT
<213> Pseudomonas putida

<400> 125

```
Met Ser Glu Ala Leu Pro Leu Ser Pro Ala Glu Phe Glu Gln Ala Leu
```

```
1               5                   10                  15
Arg Ala Lys Gly Ala Tyr Tyr His Ile His His Pro Tyr His Val Ala
            20                  25                  30
Met Tyr Glu Gly Arg Ala Thr Arg Glu Gln Ile Gln Gly Trp Val Ala
        35                  40                  45
Asn Arg Phe Tyr Tyr Gln Val Asn Ile Pro Met Lys Asp Ala Ala Ile
    50                  55                  60
Leu Ala Asn Cys Pro Asp Arg Glu Val Arg Arg Glu Trp Ile Gln Arg
65                  70                  75                  80
Leu Leu Asp His Asp Gly Ala Pro Gly Glu Asp Gly Gly Ile Glu Ala
                85                  90                  95
Trp Leu Arg Leu Gly Gln Ala Val Gly Leu Asp Pro Asp Gln Leu Arg
            100                 105                 110
Ser Gln Glu Leu Val Leu Pro Gly Val Arg Phe Ala Val Asp Ala Tyr
        115                 120                 125
Val Asn Phe Ala Arg Arg Ala Ser Trp Gln Glu Ala Ala Ser Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Asp Pro Ala Gly Tyr Glu Tyr Phe
                165                 170                 175
Arg Thr Arg Leu Gly Gln Ala Arg Arg Asp Val Glu His Gly Leu Thr
            180                 185                 190
Ile Thr Leu Gln His Tyr Thr Thr Arg Ala Ala Gln Glu Arg Met Leu
        195                 200                 205
Glu Ile Leu Gln Phe Lys Leu Asp Ile Leu Trp Ser Met Leu Asp Ala
    210                 215                 220
Met Ser Met Ala Tyr Glu Leu His Arg Pro Pro Tyr His Thr Val Thr
225                 230                 235                 240
Gln Gln Arg Val Trp His Lys Gly Ile Ala Leu
                245                 250
```

<210> 126
<211> 753
<212> DNA
<213> Pseudomonas mendocina

<400> 126

```
atgagccaag ctgccatgac acctgccgag ttcgagcagg cgctgcgcgc caagggcgcg     60
ctgtaccaca tccatcatcc gtttcatcgc gccatgtacg aagggcgcgc cacccgcgag    120
cagattcagg gctgggtggc caaccgcttc tactatcagg tcaatattcc gctcaaggat    180
gcggcgatcc tcgccaactg tccggatcgc gaaactcggc gcgagtggat tcagcgcatc    240
ctcgaccatg acggtgcacc gggtgaggag ggcggtatcg aagcctggct gcgcctggcc    300
gagtcggtcg gcctcgaccg cgagcaggtg ctgtcgcagg agctggtgct gcccggcgtg    360
cgcttcgccg tcgacgccta cgtcaacttc gcccgccgcg ccagctggca ggaagcggcc    420
agcagctcgc tgaccgagct gttcgcgccg accatccacc agtcgcgcct ggacgcctgg    480
ccgcagcact acccgtggat cgatgcggcc ggttacgact acttccgcaa tcgtctgagc    540
caggcgcggc gcgacgtcga gcatggcctg cgcatcaccc tggagcacta ccgcacccgt    600
gatgcgcagg agcacatgct gaacatcctg cagttcaagc tggacgtgct gtggagcatg    660
ctcgacgcca tgagcatggc ctacgagctg gagcgcccgc cgtatcacac ggtgacggcc    720
gagcgtgtct ggcataaggg aatcgacctg tga                                 753
```

<210> 127
<211> 250
<212> PRT
<213> Pseudomonas mendocina

<400> 127

```
Met Ser Gln Ala Ala Met Thr Pro Ala Glu Phe Glu Gln Ala Leu Arg
1               5                   10                  15
Ala Lys Gly Ala Leu Tyr His Ile His His Pro Phe His Arg Ala Met
            20                  25                  30
Tyr Glu Gly Arg Ala Thr Arg Glu Gln Ile Gln Gly Trp Val Ala Asn
        35                  40                  45
Arg Phe Tyr Tyr Gln Val Asn Ile Pro Leu Lys Asp Ala Ala Ile Leu
    50                  55                  60
Ala Asn Cys Pro Asp Arg Glu Thr Arg Arg Glu Trp Ile Gln Arg Ile
65                  70                  75                  80
Leu Asp His Asp Gly Ala Pro Gly Glu Glu Gly Gly Ile Glu Ala Trp
                85                  90                  95
Leu Arg Leu Ala Glu Ser Val Gly Leu Asp Arg Glu Gln Val Leu Ser
            100                 105                 110
Gln Glu Leu Val Leu Pro Gly Val Arg Phe Ala Val Asp Ala Tyr Val
        115                 120                 125
```

```
Asn Phe Ala Arg Arg Ala Ser Trp Gln Glu Ala Ala Ser Ser Ser Leu
    130                 135                 140
Thr Glu Leu Phe Ala Pro Thr Ile His Gln Ser Arg Leu Asp Ala Trp
145                 150                 155                 160
Pro Gln His Tyr Pro Trp Ile Asp Ala Ala Gly Tyr Asp Tyr Phe Arg
                165                 170                 175
Asn Arg Leu Ser Gln Ala Arg Arg Asp Val Glu His Gly Leu Arg Ile
            180                 185                 190
Thr Leu Glu His Tyr Arg Thr Arg Asp Ala Gln Glu His Met Leu Asn
        195                 200                 205
Ile Leu Gln Phe Lys Leu Asp Val Leu Trp Ser Met Leu Asp Ala Met
    210                 215                 220
Ser Met Ala Tyr Glu Leu Glu Arg Pro Pro Tyr His Thr Val Thr Ala
225                 230                 235                 240
Glu Arg Val Trp His Lys Gly Ile Asp Leu
                245                 250
```

```
<210>  128
<211>  813
<212>  DNA
<213>  Pseudomonas aeruginosa

<400>  128
tcatggcgcc agccccccggt gccagacccg ctcacgggtc accgtatggt acggcggacg      60
ttccagctcg taggccatgc tcatcgcgtc gagcatgctc cacagcacgt ccagcttgaa     120
ttgcaggatc tccagcatgc gttcctgtgc ctcgcgggtc cgatagtgct ccagggtgat     180
ccgcaggccg tgctcgacgt cacgccgggc ctgggccagg cggctgcgga aatactcgta     240
gccggccgcc tcgatccacg gatagtggcg cggccagctg tccagccgcg actggtggat     300
ctgcggggcg aagagttcgg tcagcgaact gctcgccgcc tcctgccagc tggcgcgacg     360
ggcgaagttg acataggcgt cgacggcgaa gcgcacgccg ggcagcaccc gttcttcgga     420
cagcacctgc tcgcgctcca ggcccaccgc ctccgccagg cgcagccagg cctcgatgcc     480
tcccgcctcg ccgggggcgc cgtcgtggtc gaggatgcgc tggacccact cgcggcggac     540
ctcgcggtcg ggacagttgg ccaggatcgc cgcgtccttc agcgggatgt tgagctggta     600
gtagaagcgg ttcgccaccc agccctggat ctgttcgcga ctggcacggc cctcgtacat     660
cgcgacatgg aacggatggt ggatatggta gtagcgcccc ttgtcgcgca gcgcccgttc     720
gaactcggcg cggtccatgg cggcacggct catgtcggtc cccctacagc tggatgtgca     780
tgccatccca ggccacttcg atgccactgg cat                                  813
```

```
<210>  129
<211>  270
<212>  PRT
<213>  Pseudomonas aeruginosa

<400>  129
Met Pro Val Ala Ser Lys Trp Pro Gly Met Ala Cys Thr Ser Ser Cys
1               5                   10                  15
Arg Gly Thr Asp Met Ser Arg Ala Ala Met Asp Arg Ala Glu Phe Glu
            20                  25                  30
Arg Ala Leu Arg Asp Lys Gly Arg Tyr Tyr His Ile His His Pro Phe
        35                  40                  45
His Val Ala Met Tyr Glu Gly Arg Ala Ser Arg Glu Gln Ile Gln Gly
    50                  55                  60
Trp Val Ala Asn Arg Phe Tyr Tyr Gln Leu Asn Ile Pro Leu Lys Asp
65                  70                  75                  80
Ala Ala Ile Leu Ala Asn Cys Pro Asp Arg Glu Val Arg Arg Glu Trp
                85                  90                  95
Val Gln Arg Ile Leu Asp His Asp Gly Ala Pro Gly Glu Ala Gly Gly
            100                 105                 110
Ile Glu Ala Trp Leu Arg Leu Ala Glu Ala Val Gly Leu Glu Arg Glu
        115                 120                 125
Gln Val Leu Ser Glu Glu Arg Val Leu Pro Gly Val Arg Phe Ala Val
    130                 135                 140
Asp Ala Tyr Val Asn Phe Ala Arg Arg Ala Ser Trp Gln Glu Ala Ala
145                 150                 155                 160
Ser Ser Ser Leu Thr Glu Leu Phe Ala Pro Gln Ile His Gln Ser Arg
                165                 170                 175
Leu Asp Ser Trp Pro Arg His Tyr Pro Trp Ile Glu Ala Ala Gly Tyr
            180                 185                 190
Glu Tyr Phe Arg Ser Arg Leu Ala Gln Ala Arg Arg Asp Val Glu His
        195                 200                 205
Gly Leu Arg Ile Thr Leu Glu His Tyr Arg Thr Arg Glu Ala Gln Glu
    210                 215                 220
Arg Met Leu Glu Ile Leu Gln Phe Lys Leu Asp Val Leu Trp Ser Met
225                 230                 235                 240
```

```
Leu Asp Ala Met Ser Met Ala Tyr Glu Leu Glu Arg Pro Pro Tyr His
                245                 250                 255
Thr Val Thr Arg Glu Arg Val Trp His Arg Gly Leu Ala Pro
            260                 265                 270

<210>  130
<211>  753
<212>  DNA
<213>  Pseudomonas aeruginosa

<400>  130
atgagccgtg ccgccatgga tcgcgccgaa ttcgaacggg cgctgcgcga caagggacgc      60
tactaccata tccaccatcc gttccatgtc gcgatgtacg agggccgtgc cagtcgcgaa     120
cagatccagg gctgggtggc gaaccgtttc tactaccagg tcaacatccc gctgaaggac     180
gcggcgatcc tggccaactg tcccgaccgc gaggtccgcc gcgagtggat ccagcgtatc     240
ctcgaccacg acggcgcccc cggcgaggcg ggcggcatcg aggcctggct gcgtctggcg     300
gaagcggtgg gcctggagcg cgagcaggtg ctgtccgagg agcgggtgct gcccggcgtg     360
cgcttcgccg tcgacgccta cgtcaacttc gcccgtcgcg ccagttggca ggaggcggcg     420
agcagttcgc tgaccgaact cttcgccccg cagatccacc agtcgcggct ggacagctgg     480
ccgcgccact atccgtggat cgaggcggcc ggctacgagt atttccgcag tcgcctggcc     540
caggcccggc gcgacgtcga gcacggcctg cggatcaccc tggagcacta tcggacccgc     600
gaggcacagg aacgcatgct ggatatcctg caattcaagc tggacgtgtt gtggagcatg     660
ctcgacgcga tgagcatggc ctacgaactc gaacgcccgc cgtaccacac ggtgacccgc     720
gagcgggtct ggcatcgggg gctggcatca tga                                 753

<210>  131
<211>  250
<212>  PRT
<213>  Pseudomonas aeruginosa

<400>  131
Met Ser Arg Ala Ala Met Asp Arg Ala Glu Phe Glu Arg Ala Leu Arg
1               5                   10                  15
Asp Lys Gly Arg Tyr Tyr His Ile His His Pro Phe His Val Ala Met
            20                  25                  30
Tyr Glu Gly Arg Ala Ser Arg Glu Gln Ile Gln Gly Trp Val Ala Asn
        35                  40                  45
Arg Phe Tyr Tyr Gln Val Asn Ile Pro Leu Lys Asp Ala Ala Ile Leu
    50                  55                  60
Ala Asn Cys Pro Asp Arg Glu Val Arg Arg Glu Trp Ile Gln Arg Ile
65                  70                  75                  80
Leu Asp His Asp Gly Ala Pro Gly Glu Ala Gly Gly Ile Glu Ala Trp
                85                  90                  95
Leu Arg Leu Ala Glu Ala Val Gly Leu Glu Arg Glu Gln Val Leu Ser
            100                 105                 110
Glu Glu Arg Val Leu Pro Gly Val Arg Phe Ala Val Asp Ala Tyr Val
        115                 120                 125
Asn Phe Ala Arg Arg Ala Ser Trp Gln Glu Ala Ala Ser Ser Ser Leu
    130                 135                 140
Thr Glu Leu Phe Ala Pro Gln Ile His Gln Ser Arg Leu Asp Ser Trp
145                 150                 155                 160
Pro Arg His Tyr Pro Trp Ile Glu Ala Ala Gly Tyr Glu Tyr Phe Arg
                165                 170                 175
Ser Arg Leu Ala Gln Ala Arg Arg Asp Val Glu His Gly Leu Arg Ile
            180                 185                 190
Thr Leu Glu His Tyr Arg Thr Arg Glu Ala Gln Glu Arg Met Leu Asp
        195                 200                 205
Ile Leu Gln Phe Lys Leu Asp Val Leu Trp Ser Met Leu Asp Ala Met
    210                 215                 220
Ser Met Ala Tyr Glu Leu Glu Arg Pro Pro Tyr His Thr Val Thr Arg
225                 230                 235                 240
Glu Arg Val Trp His Arg Gly Leu Ala Ser
                245                 250

<210>  132
<211>  753
<212>  DNA
<213>  Pseudomonas aeruginosa

<400>  132
tcatggcgcc agcccccggt gccagacccg ctcacgggtc accgtatggt acggcggacg      60
ttccagctcg taggccatgc tcatcgcgtc gagcatgctc cacagcacgt ccagcttgaa     120
ttgcaggatc tccagcatgc gttcctgtgc ctcgcgggtc cgatagtgct ccagggtgat     180
ccgcaggccg tgctcgacgt cacgccgggc ctgggccagg cggctgcgga aatactcgta     240
```

gccggccgcc tcgatccacg gatagtggcg cggccagctg tccagccgcg actggtggat 300
ctgcggggcg aagagttcgg tcagcgaact gctcgccgcc tcctgccagc tggcgcgacg 360
ggcgaagttg acataggcgt cgacggcgaa gcgcacgccg ggcagcaccc gttcttcgga 420
cagcacctgc tcgcgctcca ggcccaccgc ctccgccagg cgcagccagg cctcgatgcc 480
tcccgcctcg ccgggggcgc cgtcgtggtc gaggatgcgc tggacccact cgcggcggac 540
ctcgcggtcg ggacagttgg ccaggatcgc cgcgtccttc agcgggatgt tgagctggta 600
gtagaagcgg ttcgccaccc agccctggat ctgttcgcga ctggcacggc cctcgtacat 660
cgcgacatgg aacggatggt ggatatggta gtagcgcccc ttgtcgcgca gcgcccgttc 720
gaactcggcg cggtccatgg cggcacggct cat 753

<210> 133
<211> 250
<212> PRT
<213> Pseudomonas aeruginosa

<400> 133
Met Ser Arg Ala Ala Met Asp Arg Ala Glu Phe Glu Arg Ala Leu Arg
1 5 10 15
Asp Lys Gly Arg Tyr Tyr His Ile His His Pro Phe His Val Ala Met
20 25 30
Tyr Glu Gly Arg Ala Ser Arg Glu Gln Ile Gln Gly Trp Val Ala Asn
35 40 45
Arg Phe Tyr Tyr Gln Leu Asn Ile Pro Leu Lys Asp Ala Ala Ile Leu
50 55 60
Ala Asn Cys Pro Asp Arg Glu Val Arg Arg Glu Trp Val Gln Arg Ile
65 70 75 80
Leu Asp His Asp Gly Ala Pro Gly Glu Ala Gly Gly Ile Glu Ala Trp
85 90 95
Leu Arg Leu Ala Glu Ala Val Gly Leu Glu Arg Glu Gln Val Leu Ser
100 105 110
Glu Glu Arg Val Leu Pro Gly Val Arg Phe Ala Val Asp Ala Tyr Val
115 120 125
Asn Phe Ala Arg Arg Ala Ser Trp Gln Glu Ala Ala Ser Ser Ser Leu
130 135 140
Thr Glu Leu Phe Ala Pro Gln Ile His Gln Ser Arg Leu Asp Ser Trp
145 150 155 160
Pro Arg His Tyr Pro Trp Ile Glu Ala Ala Gly Tyr Glu Tyr Phe Arg
165 170 175
Ser Arg Leu Ala Gln Ala Arg Arg Asp Val Glu His Gly Leu Arg Ile
180 185 190
Thr Leu Glu His Tyr Arg Thr Arg Glu Ala Gln Glu Arg Met Leu Glu
195 200 205
Ile Leu Gln Phe Lys Leu Asp Val Leu Trp Ser Met Leu Asp Ala Met
210 215 220
Ser Met Ala Tyr Glu Leu Glu Arg Pro Pro Tyr His Thr Val Thr Arg
225 230 235 240
Glu Arg Val Trp His Arg Gly Leu Ala Pro
245 250

<210> 134
<211> 753
<212> DNA
<213> Azotobacter vinelandii

<400> 134
tcacagtatc gttccccggt gccagacccg ctcatgggtc accgtgtgat acggcggacg 60
gtccagttcg taggccatgc tcatggcgtc gagcatgctc cagaggatgt ccagcttgaa 120
ctgcaggatc tccagcatgc gggcctgggc ctcgcgggtg gcgaagtgcg cgagggtgat 180
gcgcaggccg tgctcgacgt cgcggcgcgc ttccttcagg cgcctgcgga agtagtcgta 240
gccgccggcc tcgatccagg ggtagtgccg cggccaggtg tccaggcgcg actggtggat 300
ggtcggggcg aacagttcgg tcagcgagct gccggccgcc tcctgccaac tggcgcggcg 360
ggcgaagttg acgtaggcgt ccaccgcgaa acgcaccccc ggcagcacca gttcctggga 420
cagcaattgc tcgcgatcga ggccgaccgc ctcgccgagg cgcagccagg cctcgatgcc 480
gccctcctcg ccggggccgc catcgtggtc gaggatgcgc tggacccact cgcggcggac 540
ttcgcggtcc gggcagttgg cgaggatcgc cgcgtccttc aggggaatgt tcacctggta 600
gtagaagcga ttggccaccc agccccggat ctgctcgcgg gtcgagcgac cctcgtacat 660
ggcgacctgg aatggatggt ggatgtggta gtaggcgccc ttggcgcgca acgcccgctc 720
gaattcggcg ggactcatgg ctgtctgggt cat 753

<210> 135
<211> 250
<212> PRT
<213> Azotobacter vinelandii

<400> 135
Met Thr Gln Thr Ala Met Ser Pro Ala Glu Phe Glu Arg Ala Leu Arg
1 5 10 15
Ala Lys Gly Ala Tyr Tyr His Ile His His Pro Phe Gln Val Ala Met
20 25 30
Tyr Glu Gly Arg Ser Thr Arg Glu Gln Ile Arg Gly Trp Val Ala Asn
35 40 45
Arg Phe Tyr Tyr Gln Val Asn Ile Pro Leu Lys Asp Ala Ala Ile Leu
50 55 60
Ala Asn Cys Pro Asp Arg Glu Val Arg Arg Glu Trp Val Gln Arg Ile
65 70 75 80
Leu Asp His Asp Gly Gly Pro Gly Glu Glu Gly Gly Ile Glu Ala Trp
85 90 95
Leu Arg Leu Gly Glu Ala Val Gly Leu Asp Arg Glu Gln Leu Leu Ser
100 105 110
Gln Glu Leu Val Leu Pro Gly Val Arg Phe Ala Val Asp Ala Tyr Val
115 120 125
Asn Phe Ala Arg Arg Ala Ser Trp Gln Glu Ala Ala Gly Ser Ser Leu
130 135 140
Thr Glu Leu Phe Ala Pro Thr Ile His Gln Ser Arg Leu Asp Thr Trp
145 150 155 160
Pro Arg His Tyr Pro Trp Ile Glu Ala Gly Gly Tyr Asp Tyr Phe Arg
165 170 175
Arg Arg Leu Lys Glu Ala Arg Arg Asp Val Glu His Gly Leu Arg Ile
180 185 190
Thr Leu Ala His Phe Ala Thr Arg Glu Ala Gln Ala Arg Met Leu Glu
195 200 205
Ile Leu Gln Phe Lys Leu Asp Ile Leu Trp Ser Met Leu Asp Ala Met
210 215 220
Ser Met Ala Tyr Glu Leu Asp Arg Pro Pro Tyr His Thr Val Thr His
225 230 235 240
Glu Arg Val Trp His Arg Gly Thr Ile Leu
245 250

<210> 136
<211> 756
<212> DNA
<213> Kluyvera intermedia

<400> 136
atgatcatca ctgaagcatt atcgcccgcc gcgtttgagc aggcgctgcg cgataaaggc 60
gcgttctacc acatacatca cccgtatcac attgccatgc acaatggcga ggcgacgcgc 120
gagcagattc agggctgggt ggcgaaccgc ttctattatc agaccaacat tccgctgaaa 180
gatgcggcga tcatggcgaa ttgcccggat gcggcgacgc ggcgtaaatg ggtgcagcgc 240
atcctcgatc acgacggcag caacggtcac gaaggcggga ttgaagcctg gctgcagttg 300
ggtgaggcgg tggggctgga acgtgacgtg ttgctgtcag agaagctggt gctgccgggc 360
gtgcgttttg cggtggacgc ctacgtgaac ttcgcgcgcc gtgctaactg gcaggaagcg 420
gcctgtagct cgctcaccga gctgtttgcg ccgcagattc atcagtcgcg tctcgacagc 480
tggccgcagc actatccgtg gatcaaagag gaaggctatt tctacttccg cagccgtttg 540
agccaggcga accgcgatgt ggagcatggg ctggcgctgg cgcttgaggt gtttacccgc 600
gccgaacagc agaaccgcat gctggagatc ctgcagttta aactcgatat tctctggacc 660
atgctcgatg ccatgaccat ggcgtatgcg ctgcaacgtc cgccttatca tacggtcacc 720
gatcaggtgt cctggcacag cacaagactg gtataa 756

<210> 137
<211> 251
<212> PRT
<213> Kluyvera intermedia

<400> 137
Met Ile Ile Thr Glu Ala Leu Ser Pro Ala Ala Phe Glu Gln Ala Leu
1 5 10 15
Arg Asp Lys Gly Ala Phe Tyr His Ile His His Pro Tyr His Ile Ala
20 25 30
Met His Asn Gly Glu Ala Thr Arg Glu Gln Ile Gln Gly Trp Val Ala
35 40 45
Asn Arg Phe Tyr Tyr Gln Thr Asn Ile Pro Leu Lys Asp Ala Ala Ile
50 55 60
Met Ala Asn Cys Pro Asp Ala Ala Thr Arg Arg Lys Trp Val Gln Arg
65 70 75 80
Ile Leu Asp His Asp Gly Ser Asn Gly His Glu Gly Gly Ile Glu Ala
85 90 95
Trp Leu Gln Leu Gly Glu Ala Val Gly Leu Glu Arg Asp Val Leu Leu
100 105 110

```
Ser Glu Lys Leu Val Leu Pro Gly Val Arg Phe Ala Val Asp Ala Tyr
        115                 120                 125
Val Asn Phe Ala Arg Arg Ala Asn Trp Gln Glu Ala Ala Cys Ser Ser
    130                 135                 140
Leu Thr Glu Leu Phe Ala Pro Gln Ile His Gln Ser Arg Leu Asp Ser
145                 150                 155                 160
Trp Pro Gln His Tyr Pro Trp Ile Lys Glu Glu Gly Tyr Phe Tyr Phe
                165                 170                 175
Arg Ser Arg Leu Ser Gln Ala Asn Arg Asp Val Glu His Gly Leu Ala
            180                 185                 190
Leu Ala Leu Glu Val Phe Thr Arg Ala Glu Gln Gln Asn Arg Met Leu
        195                 200                 205
Glu Ile Leu Gln Phe Lys Leu Asp Ile Leu Trp Thr Met Leu Asp Ala
    210                 215                 220
Met Thr Met Ala Tyr Ala Leu Gln Arg Pro Pro Tyr His Thr Val Thr
225                 230                 235                 240
Asp Gln Val Ser Trp His Ser Thr Arg Leu Val
                245                 250
```

<210> 138
<211> 771
<212> DNA
<213> Oceanospirillum sp.

<400> 138

```
tcactcatta aacagcccct tgtgaaatac cttttcaccc gtcactgtat ggtatggtgg      60
gcgctccagt tcataggcca ttgtcattgc atcaagcatg gtccaaagca catccaactt     120
aaactgaagt atttccagca ttctgtcttg ctgttcccgg gtagtgtaat gatccagcgt     180
aatttcaaga ccatgaacca catcacgacg cgcttcggta agccggttgc ggaagtactg     240
ataaccctct tctttgatcc atgggtaatg ctgaggccat gtatcaaggc gattctgatg     300
aatggttggc gcaaacagct cagtcaatga agagctggca gcttcttgcc aggtcgctct     360
gcgcgcaaaa ttgatataag catctacagc gaagcgaaca ccaggaagaa catgctcacc     420
tgataggatc tcttcgcggg ttagaccgac agcctcacca agacgaagcc atgcttcaat     480
accgccctcg gcaccatcat aaccatcatg atctaataca cgctgcaccc aatgacgacg     540
tacatcccgg tcagggcaat ttgccataat tgcagcatct ttcacgggga tgcttatctg     600
ataatagaaa cggtttgcga cccagcctcg aatctgttca ggagtagact ggccgttgta     660
cattgcaata tggtaagggt gttgagtgtg ataaagatcg cctttcgctc taagcgcttt     720
ttcaaacgct tcacgactca tgggtaattt atcgttggcc tgtgtattca t              771
```

<210> 139
<211> 256
<212> PRT
<213> Oceanospirillum sp.

<400> 139

```
Met Asn Thr Gln Ala Asn Asp Lys Leu Pro Met Ser Arg Glu Ala Phe
1               5                   10                  15
Glu Lys Ala Leu Arg Ala Lys Gly Asp Leu Tyr His Thr Gln His Pro
            20                  25                  30
Tyr His Ile Ala Met Tyr Asn Gly Gln Ser Thr Pro Glu Gln Ile Arg
        35                  40                  45
Gly Trp Val Ala Asn Arg Phe Tyr Tyr Gln Ile Ser Ile Pro Val Lys
    50                  55                  60
Asp Ala Ala Ile Met Ala Asn Cys Pro Asp Arg Asp Val Arg Arg His
65                  70                  75                  80
Trp Val Gln Arg Val Leu Asp His Asp Gly Tyr Asp Gly Ala Glu Gly
                85                  90                  95
Gly Ile Glu Ala Trp Leu Arg Leu Gly Glu Ala Val Gly Leu Thr Arg
            100                 105                 110
Glu Glu Ile Leu Ser Gly Glu His Val Leu Pro Gly Val Arg Phe Ala
        115                 120                 125
Val Asp Ala Tyr Ile Asn Phe Ala Arg Arg Ala Thr Trp Gln Glu Ala
    130                 135                 140
Ala Ser Ser Ser Leu Thr Glu Leu Phe Ala Pro Thr Ile His Gln Asn
145                 150                 155                 160
Arg Leu Asp Thr Trp Pro Gln His Tyr Pro Trp Ile Lys Glu Glu Gly
                165                 170                 175
Tyr Gln Tyr Phe Arg Asn Arg Leu Thr Glu Ala Arg Arg Asp Val Val
            180                 185                 190
His Gly Leu Glu Ile Thr Leu Asp His Tyr Thr Thr Arg Glu Gln Gln
        195                 200                 205
Asp Arg Met Leu Glu Ile Leu Gln Phe Lys Leu Asp Val Leu Trp Thr
    210                 215                 220
Met Leu Asp Ala Met Thr Met Ala Tyr Glu Leu Glu Arg Pro Pro Tyr
```

225 230 235 240
His Thr Val Thr Gly Glu Lys Val Phe His Lys Gly Leu Phe Asn Glu
245 250 255

<210> 140
<211> 756
<212> DNA
<213> Klebsiella pneumoniae

<400> 140
atgctgatca ctgacacgct gtcgccgcag gcctttgaag aggctctgcg ggctaaaggc 60
gacttctacc atattcacca cccttaccac atcgccatgc ataacggcaa cgcgacccgc 120
gagcaaattc agggttgggt ggcgaaccgg ttttattacc agaccactat tccgctgaaa 180
gacgcggcga ttatggccaa ctgcccggat gcgcagaccc ggcgcaaatg ggtgcagcgg 240
atcctcgacc acgacggcag ccacggcgag gacggcggga ttgaagcctg gctgcggctg 300
ggggaagcgg tcggtttgag ccgcgacgat ctgctcagcg agcgtcacgt gctgcccggt 360
gtgcgcttcg cggtggatgc ctatcttaat ttcgcccgtc gcgcctgctg gcaggaggcg 420
gcctgcagct ccctgaccga gctgttcgcc ccgcagatcc atcagtcgcg cctcgacagc 480
tggccgcagc actatccatg gatcaaagag gaaggctatt tttacttccg cagtcgtctg 540
agccaggcta accgcgacgt tgagcatggc ctggcgctgg cgaaggccta ctgcgacagc 600
gctgaaaaac agaaccggat gctggagatc ctgcagttta agctcgacat tctgtggtcg 660
atgctcgatg ccatgaccat ggcctatgcc ctgcagcgcc cgccctatca cacggtcacc 720
gacaaggcgg cctggcacac gacccgactg gtgtaa 756

<210> 141
<211> 251
<212> PRT
<213> Klebsiella pneumoniae

<400> 141
Met Leu Ile Thr Asp Thr Leu Ser Pro Gln Ala Phe Glu Glu Ala Leu
1 5 10 15
Arg Ala Lys Gly Asp Phe Tyr His Ile His His Pro Tyr His Ile Ala
20 25 30
Met His Asn Gly Asn Ala Thr Arg Glu Gln Ile Gln Gly Trp Val Ala
35 40 45
Asn Arg Phe Tyr Tyr Gln Thr Thr Ile Pro Leu Lys Asp Ala Ala Ile
50 55 60
Met Ala Asn Cys Pro Asp Ala Gln Thr Arg Arg Lys Trp Val Gln Arg
65 70 75 80
Ile Leu Asp His Asp Gly Ser His Gly Glu Asp Gly Gly Ile Glu Ala
85 90 95
Trp Leu Arg Leu Gly Glu Ala Val Gly Leu Ser Arg Asp Asp Leu Leu
100 105 110
Ser Glu Arg His Val Leu Pro Gly Val Arg Phe Ala Val Asp Ala Tyr
115 120 125
Leu Asn Phe Ala Arg Arg Ala Cys Trp Gln Glu Ala Ala Cys Ser Ser
130 135 140
Leu Thr Glu Leu Phe Ala Pro Gln Ile His Gln Ser Arg Leu Asp Ser
145 150 155 160
Trp Pro Gln His Tyr Pro Trp Ile Lys Glu Glu Gly Tyr Phe Tyr Phe
165 170 175
Arg Ser Arg Leu Ser Gln Ala Asn Arg Asp Val Glu His Gly Leu Ala
180 185 190
Leu Ala Lys Ala Tyr Cys Asp Ser Ala Glu Lys Gln Asn Arg Met Leu
195 200 205
Glu Ile Leu Gln Phe Lys Leu Asp Ile Leu Trp Ser Met Leu Asp Ala
210 215 220
Met Thr Met Ala Tyr Ala Leu Gln Arg Pro Pro Tyr His Thr Val Thr
225 230 235 240
Asp Lys Ala Ala Trp His Thr Thr Arg Leu Val
245 250

<210> 142
<211> 660
<212> DNA
<213> Pseudomonas stutzeri

<400> 142
atgtatgccg ggcaggcgac gcgcgagcag attcagggtt gggttgccaa ccgcttctac 60
tatcaggtct gcatcccggt gaaggatgca gcgatcatgg ccaactgtcc ggaccgcgac 120
acccgccgcg agtggattca gcgcatcatc gaccacgatg gcgcgccggg tgaggagggt 180
ggcatcgaag cctggctgcg actggccgag gccgtgggtc tggaccgcga gcaggtgctg 240
tcgcaggagc tggtgcttcc gggcgtgcgc ttcgcagtgg atgcctacgt gaactttgcc 300

cgtcgggcct gctggcagga agcagccagc agctcgctga ccgaactgtt cgcaccgacc 360
atccaccagt cgcggctgga tagctggccg cagcactatc cctggatcga cgcgtcgggc 420
tatgactact tccgcaagcg cctgaaggaa gcccgccgcg atgtcgagca cggcctgcgc 480
atcaccctgg accactatcg cacccgcgag gcgcaggagc gcatgctgga gatccttcag 540
ttcaagctcg acgtgttgtg gagcatgctg gacgccatga gcatggccta tgagctggac 600
cgaccgccgt accataccgt cacccgcgac cgggtctggc acaagggcat cagcttctga 660

<210> 143
<211> 219
<212> PRT
<213> Pseudomonas stutzeri

<400> 143
Met Tyr Ala Gly Gln Ala Thr Arg Glu Gln Ile Gln Gly Trp Val Ala
1 5 10 15
Asn Arg Phe Tyr Tyr Gln Val Cys Ile Pro Val Lys Asp Ala Ala Ile
20 25 30
Met Ala Asn Cys Pro Asp Arg Asp Thr Arg Arg Glu Trp Ile Gln Arg
35 40 45
Ile Ile Asp His Asp Gly Ala Pro Gly Glu Glu Gly Gly Ile Glu Ala
50 55 60
Trp Leu Arg Leu Ala Glu Ala Val Gly Leu Asp Arg Glu Gln Val Leu
65 70 75 80
Ser Gln Glu Leu Val Leu Pro Gly Val Arg Phe Ala Val Asp Ala Tyr
85 90 95
Val Asn Phe Ala Arg Arg Ala Cys Trp Gln Glu Ala Ala Ser Ser Ser
100 105 110
Leu Thr Glu Leu Phe Ala Pro Thr Ile His Gln Ser Arg Leu Asp Ser
115 120 125
Trp Pro Gln His Tyr Pro Trp Ile Asp Ala Ser Gly Tyr Asp Tyr Phe
130 135 140
Arg Lys Arg Leu Lys Glu Ala Arg Arg Asp Val Glu His Gly Leu Arg
145 150 155 160
Ile Thr Leu Asp His Tyr Arg Thr Arg Glu Ala Gln Glu Arg Met Leu
165 170 175
Glu Ile Leu Gln Phe Lys Leu Asp Val Leu Trp Ser Met Leu Asp Ala
180 185 190
Met Ser Met Ala Tyr Glu Leu Asp Arg Pro Pro Tyr His Thr Val Thr
195 200 205
Arg Asp Arg Val Trp His Lys Gly Ile Ser Phe
210 215

<210> 144
<211> 768
<212> DNA
<213> Acinetobacter sp.

<400> 144
ttgaggttta atatgaccac aacattattt tctcctgcgg aatttgaaca agccctgcgt 60
gacaaaggtc gctactatca tattcatcat ccctatcaca tcatgatgaa tgatgggcat 120
gccactaagc aacaaattca gggctgggtc gccaaccgtt tttattatca ggtcaatatc 180
cctttaaaag atgcggccat catggccaat tgcccagatc ctgcgacacg ccgtaaatgg 240
gtgcaacgta ttttggatca cgatggtcaa catgacgacc atggcggaat tgaagcatgg 300
ttgcgtctgg gtgaagccgt tggcctagac cgtgagacta ttttgtcaca aaagatggtt 360
ttgcccagtg tacgttttgc ggtagatgcc tatgtcaatt ttgcccgtcg tgcctgctgg 420
caagaagctg catgtagttc gcttaccgaa ctttttgcac ctgccatcca tcaatcacgt 480
ctggatactt ggcctacaca ttacccatgg attgatgcag aaggttacgc ctattttaga 540
ggacgcttga gtcaggccaa tcgtgacgtt gagcatggtt tggaactggc acttgagtac 600
tgcaataccg tagatcgcca gcagcgtatg ttgaatattt tacagttcaa actggatatt 660
ttgtggacga tccttgatgg tatgagcatg gcctatgtgc ttgagcgtgc accatatcac 720
acggttactc aagaagcggt ttggcatcaa aaaggattat taggatga 768

<210> 145
<211> 255
<212> PRT
<213> Acinetobacter sp.

<400> 145
Met Arg Phe Asn Met Thr Thr Thr Leu Phe Ser Pro Ala Glu Phe Glu
1 5 10 15
Gln Ala Leu Arg Asp Lys Gly Arg Tyr Tyr His Ile His His Pro Tyr
20 25 30
His Ile Met Met Asn Asp Gly His Ala Thr Lys Gln Gln Ile Gln Gly
35 40 45

```
Trp Val Ala Asn Arg Phe Tyr Tyr Gln Val Asn Ile Pro Leu Lys Asp
    50                  55                  60
Ala Ala Ile Met Ala Asn Cys Pro Asp Pro Ala Thr Arg Arg Lys Trp
65                  70                  75                  80
Val Gln Arg Ile Leu Asp His Asp Gly Gln His Asp Asp His Gly Gly
                85                  90                  95
Ile Glu Ala Trp Leu Arg Leu Gly Glu Ala Val Gly Leu Asp Arg Glu
            100                 105                 110
Thr Ile Leu Ser Gln Lys Met Val Leu Pro Ser Val Arg Phe Ala Val
        115                 120                 125
Asp Ala Tyr Val Asn Phe Ala Arg Arg Ala Cys Trp Gln Glu Ala Ala
    130                 135                 140
Cys Ser Ser Leu Thr Glu Leu Phe Ala Pro Ala Ile His Gln Ser Arg
145                 150                 155                 160
Leu Asp Thr Trp Pro Thr His Tyr Pro Trp Ile Asp Ala Glu Gly Tyr
                165                 170                 175
Ala Tyr Phe Arg Gly Arg Leu Ser Gln Ala Asn Arg Asp Val Glu His
            180                 185                 190
Gly Leu Glu Leu Ala Leu Glu Tyr Cys Asn Thr Val Asp Arg Gln Gln
        195                 200                 205
Arg Met Leu Asn Ile Leu Gln Phe Lys Leu Asp Ile Leu Trp Thr Ile
    210                 215                 220
Leu Asp Gly Met Ser Met Ala Tyr Val Leu Glu Arg Ala Pro Tyr His
225                 230                 235                 240
Thr Val Thr Gln Glu Ala Val Trp His Gln Lys Gly Leu Leu Gly
                245                 250                 255

<210>  146
<211>  750
<212>  PRT
<213>  Serratia marcescens

<400>  146
Ala Thr Gly Ala Cys Gly Cys Ala Ala Cys Cys Ala Cys Thr Cys Cys
1               5                   10                  15
Cys Cys Cys Thr Thr Gly Ala Cys Cys Cys Ala Cys Ala Gly Gly Cys
            20                  25                  30
Gly Thr Thr Thr Gly Ala Ala Gly Cys Gly Cys Gly Cys Cys Gly Thr
        35                  40                  45
Cys Cys Ala Cys Ala Ala Gly Gly Cys Gly Cys Thr Thr Ala Thr Thr
    50                  55                  60
Ala Cys Cys Ala Thr Ala Thr Cys Cys Ala Cys Cys Ala Cys Cys Cys
65                  70                  75                  80
Gly Thr Ala Cys Cys Ala Cys Ala Thr Cys Gly Cys Gly Ala Thr Gly
                85                  90                  95
Cys Ala Cys Ala Ala Cys Gly Gly Cys Gly Ala Ala Gly Cys Gly Ala
            100                 105                 110
Cys Cys Cys Gly Cys Gly Ala Gly Cys Ala Gly Ala Thr Cys Cys Ala
        115                 120                 125
Gly Gly Gly Cys Thr Gly Gly Gly Thr Gly Gly Cys Cys Ala Ala Cys
    130                 135                 140
Cys Gly Gly Thr Thr Cys Thr Ala Cys Thr Ala Cys Cys Ala Gly Ala
145                 150                 155                 160
Cys Cys Ala Gly Cys Ala Thr Thr Cys Cys Gly Ala Thr Thr Ala Ala
                165                 170                 175
Gly Gly Ala Thr Gly Cys Ala Gly Cys Gly Ala Thr Cys Ala Thr Gly
            180                 185                 190
Gly Cys Cys Ala Ala Cys Thr Gly Cys Cys Cys Ala Cys Ala Gly Cys
        195                 200                 205
Cys Gly Gly Ala Ala Ala Cys Ala Cys Gly Cys Cys Gly Cys Ala Ala
    210                 215                 220
Ala Thr Gly Gly Gly Thr Ala Cys Ala Gly Cys Gly Cys Ala Thr Cys
225                 230                 235                 240
Cys Thr Gly Gly Ala Thr Cys Ala Cys Cys Ala Cys Gly Gly Cys Ala
                245                 250                 255
Cys Gly Gly Ala Cys Gly Gly Cys Ala Gly Cys Gly Ala Ala Gly Gly
            260                 265                 270
Cys Gly Gly Cys Ala Thr Cys Gly Ala Gly Gly Cys Cys Thr Cys Thr
        275                 280                 285
Gly Ala Cys Cys Thr Gly Gly Gly Cys Gly Ala Ala Gly Cys Cys Gly
    290                 295                 300
Thr Thr Gly Gly Gly Cys Thr Gly Cys Ala Gly Cys Gly Cys Gly Ala
305                 310                 315                 320
Cys Ala Cys Thr Thr Thr Gly Cys Thr Cys Thr Cys Thr Gly Ala Ala
```

```
                325                 330                 335
Gly Ala Gly Cys Gly Gly Gly Thr Ala Cys Thr Gly Cys Cys Gly Gly
            340                 345                 350
Gly Cys Gly Thr Gly Cys Gly Cys Thr Thr Cys Gly Cys Gly Gly Thr
        355                 360                 365
Gly Gly Ala Cys Gly Cys Cys Thr Ala Cys Gly Thr Cys Ala Ala Cys
    370                 375                 380
Thr Thr Cys Gly Cys Gly Cys Gly Cys Cys Gly Cys Gly Cys Cys Thr
385                 390                 395                 400
Gly Cys Thr Gly Gly Cys Ala Gly Gly Ala Ala Gly Cys Gly Gly Cys
                405                 410                 415
Cys Thr Gly Cys Ala Gly Cys Thr Cys Ala Cys Thr Gly Ala Cys Cys
            420                 425                 430
Gly Ala Ala Cys Thr Gly Thr Thr Cys Gly Cys Gly Cys Cys Gly Cys
        435                 440                 445
Ala Ala Ala Thr Thr Cys Ala Cys Cys Ala Ala Thr Cys Gly Cys Gly
    450                 455                 460
Cys Cys Thr Cys Gly Ala Cys Ala Gly Cys Thr Gly Gly Cys Cys Gly
465                 470                 475                 480
Cys Ala Gly Cys Ala Cys Thr Ala Cys Cys Cys Gly Thr Gly Gly Ala
                485                 490                 495
Thr Cys Gly Cys Ala Gly Cys Cys Gly Thr Cys Gly Gly Cys Thr Ala
            500                 505                 510
Cys Gly Ala Cys Thr Ala Thr Thr Thr Cys Cys Gly Cys Ala Gly Cys
        515                 520                 525
Cys Gly Cys Cys Thr Cys Cys Gly Cys Cys Ala Gly Gly Cys Cys Ala
    530                 535                 540
Ala Cys Cys Gly Cys Gly Ala Thr Gly Thr Gly Gly Ala Ala Cys Ala
545                 550                 555                 560
Cys Gly Gly Cys Cys Thr Gly Gly Cys Gly Cys Thr Gly Gly Cys Gly
                565                 570                 575
Cys Thr Gly Gly Ala Cys Thr Ala Thr Thr Gly Cys Gly Ala Cys Ala
            580                 585                 590
Cys Cys Gly Thr Thr Gly Ala Ala Ala Ala Gly Cys Ala Gly Cys Ala
        595                 600                 605
Gly Cys Gly Cys Ala Thr Gly Cys Thr Gly Gly Ala Gly Ala Thr Cys
    610                 615                 620
Cys Thr Gly Cys Ala Gly Thr Thr Cys Ala Ala Gly Cys Thr Gly Gly
625                 630                 635                 640
Ala Cys Ala Thr Thr Cys Thr Gly Thr Gly Gly Ala Gly Cys Ala Thr
                645                 650                 655
Gly Cys Thr Gly Gly Ala Cys Gly Cys Cys Ala Thr Gly Ala Gly Cys
            660                 665                 670
Ala Thr Gly Gly Cys Cys Thr Ala Cys Ala Cys Cys Cys Thr Gly Ala
        675                 680                 685
Ala Cys Cys Gly Cys Cys Cys Ala Cys Cys Thr Thr Ala Cys Cys Ala
    690                 695                 700
Cys Ala Gly Cys Gly Thr Gly Ala Cys Cys Gly Cys Cys Gly Cys Gly
705                 710                 715                 720
Cys Gly Gly Gly Thr Ala Thr Gly Gly Cys Ala Cys Ala Ala Cys Cys
                725                 730                 735
Ala Gly Ala Gly Gly Cys Thr Gly Gly Thr Gly Thr Ala Ala
            740                 745                 750
```

<210> 147
<211> 249
<212> PRT
<213> Serratia marcescens

<400> 147

```
Met Thr Gln Pro Leu Pro Leu Asp Pro Gln Ala Phe Glu Ala Arg Arg
1               5                   10                  15
Pro Gln Gly Ala Tyr Tyr His Ile His His Pro Tyr His Ile Ala Met
            20                  25                  30
His Asn Gly Glu Ala Thr Arg Glu Gln Ile Gln Gly Trp Val Ala Asn
        35                  40                  45
Arg Phe Tyr Tyr Gln Thr Ser Ile Pro Ile Lys Asp Ala Ala Ile Met
    50                  55                  60
Ala Asn Cys Pro Gln Pro Glu Thr Arg Arg Lys Trp Val Gln Arg Ile
65                  70                  75                  80
Leu Asp His His Gly Thr Asp Gly Ser Glu Gly Gly Ile Glu Ala Ser
                85                  90                  95
Asp Leu Gly Glu Ala Val Gly Leu Gln Arg Asp Thr Leu Leu Ser Glu
            100                 105                 110
```

Glu Arg Val Leu Pro Gly Val Arg Phe Ala Val Asp Ala Tyr Val Asn
115 120 125
Phe Ala Arg Arg Ala Cys Trp Gln Glu Ala Ala Cys Ser Ser Leu Thr
130 135 140
Glu Leu Phe Ala Pro Gln Ile His Gln Ser Arg Leu Asp Ser Trp Pro
145 150 155 160
Gln His Tyr Pro Trp Ile Ala Ala Val Gly Tyr Asp Tyr Phe Arg Ser
165 170 175
Arg Leu Arg Gln Ala Asn Arg Asp Val Glu His Gly Leu Ala Leu Ala
180 185 190
Leu Asp Tyr Cys Asp Thr Val Glu Lys Gln Gln Arg Met Leu Glu Ile
195 200 205
Leu Gln Phe Lys Leu Asp Ile Leu Trp Ser Met Leu Asp Ala Met Ser
210 215 220
Met Ala Tyr Thr Leu Asn Arg Pro Pro Tyr His Ser Val Thr Ala Ala
225 230 235 240
Arg Val Trp His Asn Gln Arg Leu Val
245

<210> 148
<211> 732
<212> PRT
<213> Marinobacter algicola

<400> 148
Cys Thr Ala Cys Ala Gly Cys Cys Cys Cys Cys Gly Gly Thr Gly Cys
1 5 10 15
Cys Ala Gly Ala Cys Cys Gly Gly Cys Thr Gly Ala Cys Cys Gly Gly
20 25 30
Thr Thr Ala Cys Gly Gly Thr Gly Thr Gly Gly Thr Ala Cys Gly Gly
35 40 45
Cys Gly Gly Cys Gly Thr Gly Cys Gly Gly Thr Gly Cys Thr Gly Ala
50 55 60
Thr Ala Ala Gly Cys Cys Ala Thr Ala Gly Thr Cys Ala Gly Gly Gly
65 70 75 80
Cys Ala Thr Cys Cys Ala Ala Cys Ala Thr Ala Gly Ala Cys Cys Ala
85 90 95
Thr Ala Ala Gly Ala Thr Ala Thr Cys Cys Ala Gly Thr Thr Thr Gly
100 105 110
Ala Ala Thr Thr Gly Ala Ala Gly Gly Ala Thr Thr Thr Cys Cys Ala
115 120 125
Gly Gly Gly Cys Ala Cys Gly Gly Gly Cys Cys Thr Gly Cys Thr Gly
130 135 140
Thr Gly Cys Cys Gly Cys Gly Gly Thr Gly Gly Thr Ala Ala Ala Gly
145 150 155 160
Thr Gly Ala Thr Cys Cys Ala Gly Gly Gly Thr Ala Ala Thr Gly Gly
165 170 175
Thr Cys Ala Gly Gly Cys Cys Ala Thr Gly Thr Thr Cys Cys Ala Cys
180 185 190
Ala Thr Cys Gly Cys Gly Gly Cys Gly Cys Gly Cys Thr Thr Cys Cys
195 200 205
Gly Ala Cys Ala Ala Cys Cys Gly Cys Thr Thr Gly Cys Gly Gly Ala
210 215 220
Ala Ala Thr Ala Gly Gly Cys Gly Thr Ala Thr Cys Cys Ala Thr Cys
225 230 235 240
Gly Gly Thr Cys Thr Gly Gly Ala Thr Cys Cys Ala Gly Gly Gly Ala
245 250 255
Thr Ala Ala Thr Gly Cys Thr Gly Gly Gly Gly Cys Cys Ala Gly Cys
260 265 270
Thr Ala Thr Cys Gly Ala Gly Cys Cys Gly Gly Gly Ala Cys Thr Gly
275 280 285
Gly Thr Gly Ala Ala Thr Thr Thr Cys Cys Gly Gly Cys Gly Cys Ala
290 295 300
Ala Ala Cys Ala Gly Thr Thr Cys Thr Gly Thr Cys Ala Gly Gly Gly
305 310 315 320
Ala Gly Gly Ala Ala Cys Ala Gly Gly Cys Cys Gly Cys Thr Thr Cys
325 330 335
Cys Thr Gly Cys Cys Ala Ala Gly Thr Cys Gly Cys Cys Cys Gly Gly
340 345 350
Cys Gly Gly Gly Cys Ala Ala Ala Gly Thr Thr Cys Ala Gly Gly Thr
355 360 365
Ala Gly Gly Cys Gly Thr Cys Thr Ala Cys Gly Gly Cys Ala Ala Ala
370 375 380
Cys Cys Gly Thr Ala Cys Ala Cys Cys Cys Gly Gly Cys Ala Gly Cys

```
385                 390                 395                 400
Ala Cys Ala Thr Gly Gly Cys Gly Cys Thr Gly Gly Thr Cys Gly Gly
                405                 410                 415
Thr Gly Ala Cys Cys Thr Cys Gly Thr Cys Cys Cys Gly Gly Gly Thr
            420                 425                 430
Thr Ala Gly Cys Cys Cys Gly Ala Cys Cys Gly Cys Thr Thr Cys Cys
        435                 440                 445
Gly Cys Cys Ala Ala Gly Cys Thr Cys Ala Ala Cys Cys Ala Gly Gly
    450                 455                 460
Cys Cys Thr Cys Gly Ala Thr Ala Cys Cys Gly Cys Cys Thr Thr Cys
465                 470                 475                 480
Gly Thr Cys Gly Thr Cys Ala Ala Thr Gly Thr Gly Gly Cys Cys Ala
                485                 490                 495
Thr Cys Gly Thr Gly Ala Thr Cys Cys Ala Ala Cys Ala Cys Thr Cys
            500                 505                 510
Gly Thr Thr Gly Gly Ala Gly Cys Cys Ala Cys Ala Ala Cys Cys Gly
        515                 520                 525
Gly Cys Gly Gly Ala Cys Cys Gly Ala Gly Gly Cys Ala Thr Cys Cys
    530                 535                 540
Gly Gly Gly Cys Ala Gly Thr Thr Ala Gly Cys Cys Ala Thr Gly Ala
545                 550                 555                 560
Thr Cys Gly Cys Cys Gly Cys Ala Thr Cys Cys Thr Thr Gly Cys Gly
                565                 570                 575
Gly Gly Gly Thr Ala Thr Gly Thr Thr Gly Ala Thr Cys Thr Gly Gly
            580                 585                 590
Thr Ala Gly Thr Ala Gly Thr Ala Cys Cys Gly Gly Thr Thr Ala Gly
        595                 600                 605
Cys Gly Ala Cys Cys Cys Ala Gly Cys Cys Ala Cys Gly Ala Ala Thr
    610                 615                 620
Cys Thr Gly Cys Thr Cys Ala Gly Gly Ala Gly Thr Gly Cys Ala Gly
625                 630                 635                 640
Thr Cys Ala Cys Cys Gly Cys Cys Ala Thr Ala Cys Ala Thr Gly Gly
                645                 650                 655
Cys Thr Thr Thr Ala Thr Gly Gly Thr Ala Gly Gly Gly Ala Thr Gly
            660                 665                 670
Gly Thr Gly Ala Ala Thr Gly Thr Gly Gly Thr Ala Gly Thr Ala Cys
        675                 680                 685
Thr Gly Gly Cys Cys Thr Thr Thr Thr Gly Cys Ala Cys Gly Cys Ala
    690                 695                 700
Gly Gly Gly Cys Cys Thr Gly Cys Thr Cys Gly Ala Ala Ala Thr Cys
705                 710                 715                 720
Cys Cys Cys Ala Cys Gly Ala Thr Thr Cys Ala Thr
                725                 730
```

```
<210>  149
<211>  243
<212>  PRT
<213>  Marinobacter algicola

<400>  149
Met Asn Arg Gly Asp Phe Glu Gln Ala Leu Arg Ala Lys Gly Gln Tyr
1               5                   10                  15
Tyr His Ile His His Pro Tyr His Lys Ala Met Tyr Gly Gly Asp Cys
            20                  25                  30
Thr Pro Glu Gln Ile Arg Gly Trp Val Ala Asn Arg Tyr Tyr Tyr Gln
        35                  40                  45
Ile Asn Ile Pro Arg Lys Asp Ala Ala Ile Met Ala Asn Cys Pro Asp
    50                  55                  60
Ala Ser Val Arg Arg Leu Trp Leu Gln Arg Val Leu Asp His Asp Gly
65                  70                  75                  80
His Ile Asp Asp Glu Gly Gly Ile Glu Ala Trp Leu Ser Leu Ala Glu
                85                  90                  95
Ala Val Gly Leu Thr Arg Asp Glu Val Thr Asp Gln Arg His Val Leu
            100                 105                 110
Pro Gly Val Arg Phe Ala Val Asp Ala Tyr Leu Asn Phe Ala Arg Arg
        115                 120                 125
Ala Thr Trp Gln Glu Ala Ala Cys Ser Ser Leu Thr Glu Leu Phe Ala
    130                 135                 140
Pro Glu Ile His Gln Ser Arg Leu Asp Ser Trp Pro Gln His Tyr Pro
145                 150                 155                 160
Trp Ile Gln Thr Asp Gly Tyr Ala Tyr Phe Arg Lys Arg Leu Ser Glu
                165                 170                 175
Ala Arg Arg Asp Val Glu His Gly Leu Thr Ile Thr Leu Asp His Phe
            180                 185                 190
```

```
Thr Thr Ala Ala Gln Gln Ala Arg Ala Leu Glu Ile Leu Gln Phe Lys
        195                 200                 205
Leu Asp Ile Leu Trp Ser Met Leu Asp Ala Leu Thr Met Ala Tyr Gln
    210                 215                 220
His Arg Thr Pro Pro Tyr His Thr Val Thr Gly Gln Pro Val Trp His
225                 230                 235                 240
Arg Gly Leu


<210>  150
<211>  756
<212>  DNA
<213>  Marinobacter sp.

<400>  150
tcatagcccc ctgtgccaga cttgctgatc ggtaaccgta tggaacggcg gcgttttgtg      60
gctgtaggcc atggtcaggg cgtccagcag gctccacaga atatccagtt tgaactgtaa     120
aatttcgagg gcgcgggcct gctcggtagc tctggtgaaa tactccagcg taatcgccag     180
gccatgttcc acatcgcggc gggcttctga taggcgctta cggaagtagc tatagccggc     240
ctcgtcaatc cacggataat gttctggcca gctgtccaga cgagactggt ggatttctgg     300
cgcaaacagc tcggtcagcg aggaacaagc cgcttcttgc caagtcgcgc ggcgcgcaaa     360
gttcaagtag gcgtccaccg caaaacgcac gccgggcagc acgtggcgct ggtcgatcac     420
ctcttcccga gtcagcccca cggcttccgc caggcacaac caggcttcaa tgccgcccgc     480
atcaccgtcg tgaccatcgt ggtcaagaat gcgctgcaac cattgcttgc gcacgctggc     540
gtcggggcaa ttggccataa tggccgcatc tttacgcggg atcattacct gataataata     600
gcggttggcc acccagcccc ggatttgctc agggctgcac tggccaccat acatggcctg     660
atgatacggg tgatgaatat gatagagccg gcccttattg cgtaacgcct gttcaaaatc     720
cgcgcgattc agggtggtgt tcacaatggc gttcat                               756

<210>  151
<211>  251
<212>  PRT
<213>  Marinobacter sp.

<400>  151
Met Asn Ala Ile Val Asn Thr Thr Leu Asn Arg Ala Asp Phe Glu Gln
1               5                   10                  15
Ala Leu Arg Asn Lys Gly Arg Leu Tyr His Ile His His Pro Tyr His
            20                  25                  30
Gln Ala Met Tyr Gly Gly Gln Cys Ser Pro Glu Gln Ile Arg Gly Trp
        35                  40                  45
Val Ala Asn Arg Tyr Tyr Tyr Gln Val Met Ile Pro Arg Lys Asp Ala
    50                  55                  60
Ala Ile Met Ala Asn Cys Pro Asp Ala Ser Val Arg Lys Gln Trp Leu
65                  70                  75                  80
Gln Arg Ile Leu Asp His Asp Gly His Asp Gly Asp Ala Gly Gly Ile
                85                  90                  95
Glu Ala Trp Leu Cys Leu Ala Glu Ala Val Gly Leu Thr Arg Glu Glu
            100                 105                 110
Val Ile Asp Gln Arg His Val Leu Pro Gly Val Arg Phe Ala Val Asp
        115                 120                 125
Ala Tyr Leu Asn Phe Ala Arg Arg Ala Thr Trp Gln Glu Ala Ala Cys
    130                 135                 140
Ser Ser Leu Thr Glu Leu Phe Ala Pro Glu Ile His Gln Ser Arg Leu
145                 150                 155                 160
Asp Ser Trp Pro Glu His Tyr Pro Trp Ile Asp Glu Ala Gly Tyr Ser
                165                 170                 175
Tyr Phe Arg Lys Arg Leu Ser Glu Ala Arg Arg Asp Val Glu His Gly
            180                 185                 190
Leu Ala Ile Thr Leu Glu Tyr Phe Thr Arg Ala Thr Glu Gln Ala Arg
        195                 200                 205
Ala Leu Glu Ile Leu Gln Phe Lys Leu Asp Ile Leu Trp Ser Leu Leu
    210                 215                 220
Asp Ala Leu Thr Met Ala Tyr Ser His Lys Thr Pro Pro Phe His Thr
225                 230                 235                 240
Val Thr Asp Gln Gln Val Trp His Arg Gly Leu
                245                 250

<210>  152
<211>  759
<212>  DNA
<213>  Alpha proteobacterium

<400>  152
```

atgacaaagc aagcaccttg gtctcgcgat gaatttgaag caaagctccg tgctaaaggc 60
gagtactacc acatctatca tccgtttcat gtagcgatga ataaaggtga atgtagccaa 120
gaacaaattc aaggatgggt cgccaatcgc ttattttatc agttagcgat cccagtaaaa 180
gatgcaggga tcatggcaaa ttgctgggat ctgcaaaccc gtcgcaaatg gatccagcgt 240
atattggatc atgatggaga ccctgaagat actactctag gcggtattga agcttggtta 300
cgtctaggtg aagcggtggg tttacatcgt gaagatatgc tcaatgaacg ctttttatta 360
cctggtgtga agtttgccgt cggggcatat attaacttta cgcgtaaagc tagctgggaa 420
gaagcagcct gttcgtcact cactgagatg ttcgcacctg aaatccatca aagtcgcctc 480
gatacttggc caacccatta taaatggatt gatccagaag gattttccta ttttcaaatg 540
cgtttaaatc aggcacgtcg tgatgtacag cacggtttag agatcacttt agatcatttt 600
gaaacgcgta aacaacaaga gcgtgcattg aatattctgc aattcaaact tgatgtctta 660
tggagtatgg ctgatagtat cagcttagca tatgagttta accgcaagcc ttttggtgcg 720
gtaactgacg aaaatatttc tcacaaaggc ttcatgtaa 759

<210> 153
<211> 252
<212> PRT
<213> Alpha proteobacterium

<400> 153
Met Thr Lys Gln Ala Pro Trp Ser Arg Asp Glu Phe Glu Ala Lys Leu
1 5 10 15
Arg Ala Lys Gly Glu Tyr Tyr His Ile Tyr His Pro Phe His Val Ala
20 25 30
Met Asn Lys Gly Glu Cys Ser Gln Glu Gln Ile Gln Gly Trp Val Ala
35 40 45
Asn Arg Leu Phe Tyr Gln Leu Ala Ile Pro Val Lys Asp Ala Gly Ile
50 55 60
Met Ala Asn Cys Trp Asp Leu Gln Thr Arg Arg Lys Trp Ile Gln Arg
65 70 75 80
Ile Leu Asp His Asp Gly Asp Pro Glu Asp Thr Thr Leu Gly Gly Ile
85 90 95
Glu Ala Trp Leu Arg Leu Gly Glu Ala Val Gly Leu His Arg Glu Asp
100 105 110
Met Leu Asn Glu Arg Phe Leu Leu Pro Gly Val Lys Phe Ala Val Gly
115 120 125
Ala Tyr Ile Asn Phe Thr Arg Lys Ala Ser Trp Glu Glu Ala Ala Cys
130 135 140
Ser Ser Leu Thr Glu Met Phe Ala Pro Glu Ile His Gln Ser Arg Leu
145 150 155 160
Asp Thr Trp Pro Thr His Tyr Lys Trp Ile Asp Pro Glu Gly Phe Ser
165 170 175
Tyr Phe Gln Met Arg Leu Asn Gln Ala Arg Arg Asp Val Gln His Gly
180 185 190
Leu Glu Ile Thr Leu Asp His Phe Glu Thr Arg Lys Gln Gln Glu Arg
195 200 205
Ala Leu Asn Ile Leu Gln Phe Lys Leu Asp Val Leu Trp Ser Met Ala
210 215 220
Asp Ser Ile Ser Leu Ala Tyr Glu Phe Asn Arg Lys Pro Phe Gly Ala
225 230 235 240
Val Thr Asp Glu Asn Ile Ser His Lys Gly Phe Met
245 250

<210> 154
<211> 756
<212> DNA
<213> Colwellia psychrerythraea

<400> 154
tcatttaaaa attctcttat ggtaatttgg ctcagctatt aaaccttggt aaggcgctcg 60
gtcctgttga tatgctaagg tcatagcatc aagcatactc cacaaaatat ctaatttaaa 120
ttggagaata tttaatgcct cgtcttgtgc ttgacgagta gtaaagtgat ctaaggtgat 180
ttgtaggcca tgattaacat cgcgtcttgc ttgagataag cgcatttgaa aatactgtaa 240
gccttgtgtt tcaatccaag gataatttat aggccaactg tctagcctgc tttgatgtat 300
ttgaggggca aacatttccg tcagtgacga acatgcagct tgctgccagt tagcacggcg 360
agcaaagttt acataggcat ctacggcgaa tcgcacccca ggtaaaacaa atttctcatc 420
cagtaagtct tgtctatcta atcctaccgc ttggcccaat tgtaaccagg cttcaatacc 480
acctaaggtt ttgtcttcta cactgccatc gtgatctaat atgcgttgga tccatagacg 540
acgagtttcc atatcatgac aattagctaa tatggcggca tctttaatag gaatatttac 600
ttggtaataa aaacggtttg ccacccaacc ctgaatttgc tcaacactac attcaccttg 660
gtgcatggca atatgaaatg ggtgatgaat atggtaaagc tgccctttag cgcggagcat 720
ttcttcaaac tgctctcttg tccatggttg ttgcat 756

<210> 155

<211> 251
<212> PRT
<213> Colwellia psychrerythraea

<400> 155
Met Gln Gln Pro Trp Thr Arg Glu Gln Phe Glu Glu Met Leu Arg Ala
1 5 10 15
Lys Gly Gln Leu Tyr His Ile His His Pro Phe His Ile Ala Met His
20 25 30
Gln Gly Glu Cys Ser Val Glu Gln Ile Gln Gly Trp Val Ala Asn Arg
35 40 45
Phe Tyr Tyr Gln Val Asn Ile Pro Ile Lys Asp Ala Ala Ile Leu Ala
50 55 60
Asn Cys His Asp Met Glu Thr Arg Arg Leu Trp Ile Gln Arg Ile Leu
65 70 75 80
Asp His Asp Gly Ser Val Glu Asp Lys Thr Leu Gly Gly Ile Glu Ala
85 90 95
Trp Leu Gln Leu Gly Gln Ala Val Gly Leu Asp Arg Gln Asp Leu Leu
100 105 110
Asp Glu Lys Phe Val Leu Pro Gly Val Arg Phe Ala Val Asp Ala Tyr
115 120 125
Val Asn Phe Ala Arg Arg Ala Asn Trp Gln Gln Ala Ala Cys Ser Ser
130 135 140
Leu Thr Glu Met Phe Ala Pro Gln Ile His Gln Ser Arg Leu Asp Ser
145 150 155 160
Trp Pro Ile Asn Tyr Pro Trp Ile Glu Thr Gln Gly Leu Gln Tyr Phe
165 170 175
Gln Met Arg Leu Ser Gln Ala Arg Arg Asp Val Asn His Gly Leu Gln
180 185 190
Ile Thr Leu Asp His Phe Thr Thr Arg Gln Ala Gln Asp Glu Ala Leu
195 200 205
Asn Ile Leu Gln Phe Lys Leu Asp Ile Leu Trp Ser Met Leu Asp Ala
210 215 220
Met Thr Leu Ala Tyr Gln Gln Asp Arg Ala Pro Tyr Gln Gly Leu Ile
225 230 235 240
Ala Glu Pro Asn Tyr His Lys Arg Ile Phe Lys
245 250

<210> 156
<211> 723
<212> DNA
<213> Gamma proteobacterium

<400> 156
ttaccggcta cagctatgaa atggcggctc ttcatagaca taagccaact gcattgcatc 60
cagcatactc cataaaatat ccagctttaa ctgcagtata cccatggcct gctgctgctg 120
ttcgaaggtt ttaaagtagt ccagagtaat cgccaaacca tgctctacat ctcttcggc 180
ttcgctaagt cgcgtgcgga aatattgata gccgttggga tcgatccagg gatagtgttc 240
cggccaggtc gctaagcgtt tttgatgaat ggtcggggca aagagttcgg tgagtgatga 300
gcaggccgct tcctgccagt tagcgcggcg ggcaaagtta acgtaggcgt ctactgcaaa 360
gcgcacgccg ggtaatatat gttgctgact tataatttcg tctcgggtta aacccacagc 420
ctctcccaag cggagccagg cctcaatacc accactatcc tcatcgctgg tgccatcgtg 480
atcaagaata cgctgcaccc ataaacgacg aacagaggcg tcgtcacagt tggccataat 540
ggcggcgtct ttgatcggta tactgatttg ataataatag cggttagcca cccagccctg 600
tatctgcttt tgagtacagt tgccggtatt catcgcaata tggaaggggt gatgaatatg 660
gtagagcgcg cccttgtctc gtagctgctg ctcaaattct tctgcagtcc aggctttagt 720
cat 723

<210> 157
<211> 240
<212> PRT
<213> Gamma proteobacterium

<400> 157
Met Thr Lys Ala Trp Thr Ala Glu Glu Phe Glu Gln Gln Leu Arg Asp
1 5 10 15
Lys Gly Ala Leu Tyr His Ile His His Pro Phe His Ile Ala Met Asn
20 25 30
Thr Gly Asn Cys Thr Gln Lys Gln Ile Gln Gly Trp Val Ala Asn Arg
35 40 45
Tyr Tyr Tyr Gln Ile Ser Ile Pro Ile Lys Asp Ala Ala Ile Met Ala
50 55 60
Asn Cys Asp Asp Ala Ser Val Arg Arg Leu Trp Val Gln Arg Ile Leu
65 70 75 80

Asp His Asp Gly Thr Ser Asp Glu Asp Ser Gly Gly Ile Glu Ala Trp
85 90 95
Leu Arg Leu Gly Glu Ala Val Gly Leu Thr Arg Asp Glu Ile Ile Ser
100 105 110
Gln Gln His Ile Leu Pro Gly Val Arg Phe Ala Val Asp Ala Tyr Val
115 120 125
Asn Phe Ala Arg Arg Ala Asn Trp Gln Glu Ala Ala Cys Ser Ser Leu
130 135 140
Thr Glu Leu Phe Ala Pro Thr Ile His Gln Lys Arg Leu Ala Thr Trp
145 150 155 160
Pro Glu His Tyr Pro Trp Ile Asp Pro Asn Gly Tyr Gln Tyr Phe Arg
165 170 175
Thr Arg Leu Ser Glu Ala Arg Arg Asp Val Glu His Gly Leu Ala Ile
180 185 190
Thr Leu Asp Tyr Phe Lys Thr Phe Glu Gln Gln Gln Gln Ala Met Gly
195 200 205
Ile Leu Gln Leu Lys Leu Asp Ile Leu Trp Ser Met Leu Asp Ala Met
210 215 220
Gln Leu Ala Tyr Val Tyr Glu Glu Pro Pro Phe His Ser Cys Ser Arg
225 230 235 240

<210> 158
<211> 780
<212> DNA
<213> Gamma proteobacterium

<400> 158
atgtcgctcc ttgataccgc ttcatcgcga gacgcatcta atcaggcccc tctgacccga 60
gcagattttg aggcgcgtct gcgcgccaag ggctcgcgct atcacatcca ccatcctttt 120
catcaagcga tgtacgcggg tgagctaacg cctcgacaaa ttcggggctg ggtagccaac 180
aggtattact accaaataat gattcctcaa aaggatgcag cgattctggc caattgctct 240
gaccgggaaa tccgcgtgaa atggatccag cgtatccttg atcatgatgg tgccgacggt 300
gatgaaggcg gtattgaagc ctggttggaa ttggctgaag cggtgggact taagcgtgat 360
gaagtgaccg atcttcgcta cgtgctaccc ggtgtgcgct ttgcggtcga tgcttatgtc 420
aacttcgcac gtaacgccac ttggcaggaa gccgcttgct catcgctaac agagatgttc 480
gcgcctgaaa ttcatcggtc ccggctcaat acttggccgc aacattacag ctggattgac 540
caaagcggat tgaagtactt tcaaaagcgt ctaggagagg cccggcgtga tgttgagcat 600
ggcctggacg taactctcga ttatttcaaa tatcgcgaac agcaggaaaa ggcgctgggt 660
attttacagt ttaaactcga tattctgtgg acaatgctgg acgcaatgac catggcctac 720
gtgcacacca cgccccccata tcacagctgt gatgacggta ttcattcggt acctgggtaa 780

<210> 159
<211> 259
<212> PRT
<213> Gamma proteobacterium

<400> 159
Met Ser Leu Leu Asp Thr Ala Ser Ser Arg Asp Ala Ser Asn Gln Ala
1 5 10 15
Pro Leu Thr Arg Ala Asp Phe Glu Ala Arg Leu Arg Ala Lys Gly Ser
20 25 30
Arg Tyr His Ile His His Pro Phe His Gln Ala Met Tyr Ala Gly Glu
35 40 45
Leu Thr Pro Arg Gln Ile Arg Gly Trp Val Ala Asn Arg Tyr Tyr Tyr
50 55 60
Gln Ile Met Ile Pro Gln Lys Asp Ala Ala Ile Leu Ala Asn Cys Ser
65 70 75 80
Asp Arg Glu Ile Arg Val Lys Trp Ile Gln Arg Ile Leu Asp His Asp
85 90 95
Gly Ala Asp Gly Asp Glu Gly Gly Ile Glu Ala Trp Leu Glu Leu Ala
100 105 110
Glu Ala Val Gly Leu Lys Arg Asp Glu Val Thr Asp Leu Arg Tyr Val
115 120 125
Leu Pro Gly Val Arg Phe Ala Val Asp Ala Tyr Val Asn Phe Ala Arg
130 135 140
Asn Ala Thr Trp Gln Glu Ala Ala Cys Ser Ser Leu Thr Glu Met Phe
145 150 155 160
Ala Pro Glu Ile His Arg Ser Arg Leu Asn Thr Trp Pro Gln His Tyr
165 170 175
Ser Trp Ile Asp Gln Ser Gly Leu Lys Tyr Phe Gln Lys Arg Leu Gly
180 185 190
Glu Ala Arg Arg Asp Val Glu His Gly Leu Asp Val Thr Leu Asp Tyr
195 200 205
Phe Lys Tyr Arg Glu Gln Gln Glu Lys Ala Leu Gly Ile Leu Gln Phe

```
    210                 215                 220
Lys Leu Asp Ile Leu Trp Thr Met Leu Asp Ala Met Thr Met Ala Tyr
225                 230                 235                 240
Val His Thr Thr Pro Pro Tyr His Ser Cys Asp Asp Gly Ile His Ser
                245                 250                 255
Val Pro Gly


<210>  160
<211>  750
<212>  DNA
<213>  Shewanella woodyi

<400>  160
atgcaagcct ggactaaaga agaattcgaa caaaaactca aagataaagg gtcgctttac      60
catatacacc atccctacca taagatgatg cataaaggtg agtgcagcgt tgaacagatc     120
agaggttggg ttgccaaccg cttctattat cagatcaata ttcccgttaa agatgcagcc     180
atactggcaa actgtcgcga tgtaaaaact cgccgcatgt ggatccaacg tatagtggat     240
catgatggtt caattgaaga taacacctta ggtggtatcg aggcttggct aaaactcggt     300
gaagcggtgg gactgaatag acaagatatg ctagatgaaa agtatatcct gccaggggtt     360
cggttcgcgg tcgatgccta tgtgaatttt gccagacgag cctcatggca agaggcggcc     420
tgctcatcac ttaccgagat gtttgcgcca gagatacacc aaagtcgcct taatacctgg     480
ccagaaaact acccttggat aaaatccgaa ggattaagct actttcagat gcgactctct     540
caagcaaggc gagatgttaa tcatggctta gcgatcactt tggcacattt tacgacccga     600
gagcagcagg agcatgcact gaatattttg cagtttaaac tcgatattct ttggtccatg     660
ttagatgcaa tgacattagc ttatcagtat gagcgtgcgc cctattcaca gcttattgat     720
gcgcctgtct atcataaagg gatcttctga                                     750

<210>  161
<211>  249
<212>  PRT
<213>  Shewanella woodyi

<400>  161
Met Gln Ala Trp Thr Lys Glu Glu Phe Glu Gln Lys Leu Lys Asp Lys
1               5                   10                  15
Gly Ser Leu Tyr His Ile His His Pro Tyr His Lys Met Met His Lys
            20                  25                  30
Gly Glu Cys Ser Val Glu Gln Ile Arg Gly Trp Val Ala Asn Arg Phe
        35                  40                  45
Tyr Tyr Gln Ile Asn Ile Pro Val Lys Asp Ala Ala Ile Leu Ala Asn
    50                  55                  60
Cys Arg Asp Val Lys Thr Arg Arg Met Trp Ile Gln Arg Ile Val Asp
65                  70                  75                  80
His Asp Gly Ser Ile Glu Asp Asn Thr Leu Gly Gly Ile Glu Ala Trp
                85                  90                  95
Leu Lys Leu Gly Glu Ala Val Gly Leu Asn Arg Gln Asp Met Leu Asp
            100                 105                 110
Glu Lys Tyr Ile Leu Pro Gly Val Arg Phe Ala Val Asp Ala Tyr Val
        115                 120                 125
Asn Phe Ala Arg Arg Ala Ser Trp Gln Glu Ala Ala Cys Ser Ser Leu
    130                 135                 140
Thr Glu Met Phe Ala Pro Glu Ile His Gln Ser Arg Leu Asn Thr Trp
145                 150                 155                 160
Pro Glu Asn Tyr Pro Trp Ile Lys Ser Glu Gly Leu Ser Tyr Phe Gln
                165                 170                 175
Met Arg Leu Ser Gln Ala Arg Arg Asp Val Asn His Gly Leu Ala Ile
            180                 185                 190
Thr Leu Ala His Phe Thr Thr Arg Glu Gln Gln Glu His Ala Leu Asn
        195                 200                 205
Ile Leu Gln Phe Lys Leu Asp Ile Leu Trp Ser Met Leu Asp Ala Met
    210                 215                 220
Thr Leu Ala Tyr Gln Tyr Glu Arg Ala Pro Tyr Ser Gln Leu Ile Asp
225                 230                 235                 240
Ala Pro Val Tyr His Lys Gly Ile Phe
                245

<210>  162
<211>  726
<212>  DNA
<213>  Gamma proteobacterium

<400>  162
ctagtcgata ttgcagctat ggaacggcgc ctcattgtag acgtatgcca gttgcatagc      60
```

```
atccagcatg ctccacaata tatcgagctt catttgcaga atacccaatg catgctcttg     120
tgcttcgcgg gtttggaagt gatccagcgt gatttgcaat ccgtgctcaa catcacggcg     180
cgcttcactg aggcgagtac gaaaataacg gtagccctca gcttcaatcc aggggtaatg     240
ctcaggccag ctgtcgagtc ttttttgatg aataattggt gcaaataatt cggtcaaaga     300
cgaacaggcg gcttcctgcc agctcgcttt gcgggcaaag ttaacataag catctaccgc     360
gaaacggacg ccgggtaata cgtgttgaaa actagtgatc tcactgcgct gcagcccaac     420
ggcttcgccg aggcgcagcc aggcctcaat cccgccactg tcttgctcgc tagtgccgtc     480
gtggtccaat atacgctgta cccatagcct gcgcacggta ggctcggtac agttcgcaag     540
gatgttagcg tccttgatcg ggatattaac ctggtagtag taccgattgg cgacccagcc     600
ttgtatttgc tgctggttac attggccgct gttcattgct acatgcagag gatgatgaat     660
atgatacaag gcttctttgg cccgcaagcc tcgttcgaat tcttctcttg accatggttt     720
tgacat                                                                726
```

<210> 163
<211> 241
<212> PRT
<213> Gamma proteobacterium

<400> 163

```
Met Ser Lys Pro Trp Ser Arg Glu Glu Phe Glu Arg Gly Leu Arg Ala
1               5                   10                  15
Lys Glu Ala Leu Tyr His Ile His His Pro Leu His Val Ala Met Asn
            20                  25                  30
Ser Gly Gln Cys Asn Gln Gln Gln Ile Gln Gly Trp Val Ala Asn Arg
        35                  40                  45
Tyr Tyr Tyr Gln Val Asn Ile Pro Ile Lys Asp Ala Asn Ile Leu Ala
    50                  55                  60
Asn Cys Thr Glu Pro Thr Val Arg Arg Leu Trp Val Gln Arg Ile Leu
65                  70                  75                  80
Asp His Asp Gly Thr Ser Glu Gln Asp Ser Gly Gly Ile Glu Ala Trp
                85                  90                  95
Leu Arg Leu Gly Glu Ala Val Gly Leu Gln Arg Ser Glu Ile Thr Ser
            100                 105                 110
Phe Gln His Val Leu Pro Gly Val Arg Phe Ala Val Asp Ala Tyr Val
        115                 120                 125
Asn Phe Ala Arg Lys Ala Ser Trp Gln Glu Ala Ala Cys Ser Ser Leu
    130                 135                 140
Thr Glu Leu Phe Ala Pro Ile Ile His Gln Lys Arg Leu Asp Ser Trp
145                 150                 155                 160
Pro Glu His Tyr Pro Trp Ile Glu Ala Glu Gly Tyr Arg Tyr Phe Arg
                165                 170                 175
Thr Arg Leu Ser Glu Ala Arg Arg Asp Val Glu His Gly Leu Gln Ile
            180                 185                 190
Thr Leu Asp His Phe Gln Thr Arg Glu Ala Gln Glu His Ala Leu Gly
        195                 200                 205
Ile Leu Gln Met Lys Leu Asp Ile Leu Trp Ser Met Leu Asp Ala Met
    210                 215                 220
Gln Leu Ala Tyr Val Tyr Asn Glu Ala Pro Phe His Ser Cys Asn Ile
225                 230                 235                 240
Asp
```

<210> 164
<211> 753
<212> DNA
<213> Burkholderia xenovorans

<400> 164

```
tcattgcggg aaggcctttt cgatcgaatc cagcatggtc cacagaatgt cgagcttgaa      60
ttgcaggatc tcgagcgcgc gttcctgctg ttcgcgccgc gtgaaatgat cgagcgtgac     120
ggcgagcccg tgctccacgt cgcgttgtgc aagcgaaatg cgcgagcgga aatacgcgag     180
acccgaagac tcgatccacg gataatgttc cggccagctc gcgagccggt cgcgatgaac     240
ctgcggcgcg aacatttccg tgagcgacga gcagaccgat tcctgccacg gcgcgcggcg     300
cgcaaaattc acataggcgt cgacggcgaa gcgcacgccg ggcgccacct gtttcagcga     360
ccacagatcg tcacgcgaca gcccgaccgc gtcgcccagc cgcgcccatg tttcgatgcc     420
gccttcctcg tcgccgcaac cgtcgtggtc gagaatgcgc agcacccagc ggcggcgtgt     480
ttcgcgatcc gggcaattcg acagcacggc cgcatccttc agcggaatat tgatctgata     540
atagaagcga ttggcgaccc agccgcgaat ctgttcgcgg gagcaaccgc cgctattcat     600
cttcacattg aacggatgat gaatgtgata cgcggcgcct ttcgcgcgca actgtgcttc     660
gaattcttca cgggtccagg ccgggcgctc gtcgcctccg ttcgtttgcg cgttctggcg     720
cggcgccgtg cccaaggtat ctttcgcgtt cat                                  753
```

<210> 165
<211> 250

<212> PRT
<213> Burkholderia xenovorans

<400> 165
Met Asn Ala Lys Asp Thr Leu Gly Thr Ala Pro Arg Gln Asn Ala Gln
1 5 10 15
Thr Asn Gly Gly Asp Glu Arg Pro Ala Trp Thr Arg Glu Glu Phe Glu
20 25 30
Ala Gln Leu Arg Ala Lys Gly Ala Ala Tyr His Ile His His Pro Phe
35 40 45
Asn Val Lys Met Asn Ser Gly Gly Cys Ser Arg Glu Gln Ile Arg Gly
50 55 60
Trp Val Ala Asn Arg Phe Tyr Tyr Gln Ile Asn Ile Pro Leu Lys Asp
65 70 75 80
Ala Ala Val Leu Ser Asn Cys Pro Asp Arg Glu Thr Arg Arg Arg Trp
85 90 95
Val Leu Arg Ile Leu Asp His Asp Gly Cys Gly Asp Glu Glu Gly Gly
100 105 110
Ile Glu Thr Trp Ala Arg Leu Gly Asp Ala Val Gly Leu Ser Arg Asp
115 120 125
Asp Leu Trp Ser Leu Lys Gln Val Ala Pro Gly Val Arg Phe Ala Val
130 135 140
Asp Ala Tyr Val Asn Phe Ala Arg Arg Ala Pro Trp Gln Glu Ser Val
145 150 155 160
Cys Ser Ser Leu Thr Glu Met Phe Ala Pro Gln Val His Arg Asp Arg
165 170 175
Leu Ala Ser Trp Pro Glu His Tyr Pro Trp Ile Glu Ser Ser Gly Leu
180 185 190
Ala Tyr Phe Arg Ser Arg Ile Ser Leu Ala Gln Arg Asp Val Glu His
195 200 205
Gly Leu Ala Val Thr Leu Asp His Phe Thr Arg Arg Glu Gln Gln Glu
210 215 220
Arg Ala Leu Glu Ile Leu Gln Phe Lys Leu Asp Ile Leu Trp Thr Met
225 230 235 240
Leu Asp Ser Ile Glu Lys Ala Phe Pro Gln
245 250

<210> 166
<211> 726
<212> DNA
<213> Methylococcus capsulatus

<400> 166
atgagccaag acacgatccc ctggagccgc gaagaattcg aggctcggct gcgtgccaag 60
gaaaagtact accacatcca tcacccttac cacgtgttga tggcgagcgg tgagctgaac 120
cgcgagcaga tccagggctg ggtggcgaac cgcttctatt atcagatcac cattccgcgc 180
aaggattcgg ccatcatggc caactgcccg gaccgcgaaa cccgccgcaa atggatgcag 240
cgggtgatgg atcacgacgg ttacggcgat gatcccggcg ggatcgaggc atggatccag 300
ctaggcatcg cctgtggcct gagtcgcgag gacatcacct cgctcaggca tgtactgccc 360
ggcgtgcgtt tcgccgtcga cgcctacctc aacttcgctc gtaccgcgac ctggcaggaa 420
gccgcctgct cttcgctcac cgagctgttt gcgccgacca tccacaaaca acgcctggct 480
ggctggccgg atctgtatcc gtggatcgca gccgacggct tggcctattt ccgcaagcgg 540
gtgactcagg ccagccgcga tgtcgagcac ggcatcgaaa tcacgctgga tcatttcaag 600
acccgcgaac aacaggaacg ggccctggaa atcctccaat tcaagctcga catcctgtgg 660
tcgatgttgg acgccatgta cctcgcctat gtcgacaaaa aaccgcccta tttcaacatc 720
gcctga 726

<210> 167
<211> 241
<212> PRT
<213> Methylococcus capsulatus

<400> 167
Met Ser Gln Asp Thr Ile Pro Trp Ser Arg Glu Glu Phe Glu Ala Arg
1 5 10 15
Leu Arg Ala Lys Glu Lys Tyr Tyr His Ile His His Pro Tyr His Val
20 25 30
Leu Met Ala Ser Gly Glu Leu Asn Arg Glu Gln Ile Gln Gly Trp Val
35 40 45
Ala Asn Arg Phe Tyr Tyr Gln Ile Thr Ile Pro Arg Lys Asp Ser Ala
50 55 60
Ile Met Ala Asn Cys Pro Asp Arg Glu Thr Arg Arg Lys Trp Met Gln
65 70 75 80
Arg Val Met Asp His Asp Gly Tyr Gly Asp Asp Pro Gly Gly Ile Glu

```
                85                  90                  95
Ala Trp Ile Gln Leu Gly Ile Ala Cys Gly Leu Ser Arg Glu Asp Ile
            100                 105                 110
Thr Ser Leu Arg His Val Leu Pro Gly Val Arg Phe Ala Val Asp Ala
        115                 120                 125
Tyr Leu Asn Phe Ala Arg Thr Ala Thr Trp Gln Glu Ala Ala Cys Ser
    130                 135                 140
Ser Leu Thr Glu Leu Phe Ala Pro Thr Ile His Lys Gln Arg Leu Ala
145                 150                 155                 160
Gly Trp Pro Asp Leu Tyr Pro Trp Ile Ala Ala Asp Gly Leu Ala Tyr
                165                 170                 175
Phe Arg Lys Arg Val Thr Gln Ala Ser Arg Asp Val Glu His Gly Ile
            180                 185                 190
Glu Ile Thr Leu Asp His Phe Lys Thr Arg Glu Gln Gln Glu Arg Ala
        195                 200                 205
Leu Glu Ile Leu Gln Phe Lys Leu Asp Ile Leu Trp Ser Met Leu Asp
    210                 215                 220
Ala Met Tyr Leu Ala Tyr Val Asp Lys Lys Pro Pro Tyr Phe Asn Ile
225                 230                 235                 240
Ala


<210>  168
<211>  708
<212>  DNA
<213>  Limnobacter sp.

<400>  168
tcattttttt ctcttgtcgg gatacgccag tgcaatcgca tcggacatac tccaaagcac      60
gtccagtttg aattgcagaa tttcaagtgc acgttcctgt tgggcacggg ttttgaaata     120
attcaaggtc acttccaggc catgttcaac gtcgcgttgc gccaggctga ttctggagcg     180
aaaataattc aaaccttcag gtgcaaccca tttgtagtct ttgggccagt tgctcaggcg     240
ttgcttgtga atttctggtg caaacatttc agtcaacgat gagcacaccg cttcttgcca     300
gggcgcgcgg cgggcaaaat tcacgtaggc gtccactgca aaacgcacgc ctggaagcac     360
cagttgctgg ctccacagca cgtctttgtc aatgcctact gcttcaccca ggcgactcca     420
ggcttcaatg ccgccagcat tgccttccca gccatcgtga tccagaatgc gctgcaccca     480
acggcgacgg gtttcgcgat cgtcacaatt ggccatcacc gcagcgtctt tttgcggaat     540
gcacacttga taataaaagc ggtttgccac ccagccgcgc agttgttccc ggctcagttt     600
gccctcacgc atggccacgt tgaatgggtg gtgaatgtga tagcggtcgc cctttgcgcg     660
caagcgttct tcgaactcct ttttgctcca ggctttgtta ctgctcat                  708

<210>  169
<211>  235
<212>  PRT
<213>  Limnobacter sp.

<400>  169
Met Ser Ser Asn Lys Ala Trp Ser Lys Lys Glu Phe Glu Glu Arg Leu
1               5                   10                  15
Arg Ala Lys Gly Asp Arg Tyr His Ile His His Pro Phe Asn Val Ala
            20                  25                  30
Met Arg Glu Gly Lys Leu Ser Arg Glu Gln Leu Arg Gly Trp Val Ala
        35                  40                  45
Asn Arg Phe Tyr Tyr Gln Val Cys Ile Pro Gln Lys Asp Ala Ala Val
    50                  55                  60
Met Ala Asn Cys Asp Asp Arg Glu Thr Arg Arg Arg Trp Val Gln Arg
65                  70                  75                  80
Ile Leu Asp His Asp Gly Trp Glu Gly Asn Ala Gly Gly Ile Glu Ala
                85                  90                  95
Trp Ser Arg Leu Gly Glu Ala Val Gly Ile Asp Lys Asp Val Leu Trp
            100                 105                 110
Ser Gln Gln Leu Val Leu Pro Gly Val Arg Phe Ala Val Asp Ala Tyr
        115                 120                 125
Val Asn Phe Ala Arg Arg Ala Pro Trp Gln Glu Ala Val Cys Ser Ser
    130                 135                 140
Leu Thr Glu Met Phe Ala Pro Glu Ile His Lys Gln Arg Leu Ser Asn
145                 150                 155                 160
Trp Pro Lys Asp Tyr Lys Trp Val Ala Pro Glu Gly Leu Asn Tyr Phe
                165                 170                 175
Arg Ser Arg Ile Ser Leu Ala Gln Arg Asp Val Glu His Gly Leu Glu
            180                 185                 190
Val Thr Leu Asn Tyr Phe Lys Thr Arg Ala Gln Gln Glu Arg Ala Leu
        195                 200                 205
Glu Ile Leu Gln Phe Lys Leu Asp Val Leu Trp Ser Met Ser Asp Ala
```

```
    210                 215                 220
Ile Ala Leu Ala Tyr Pro Asp Lys Arg Lys Lys
225                 230                 235

<210>  170
<211>  708
<212>  DNA
<213>  Burkholderia phymatum

<400>  170
atgagtgtca aaggcagcag cggaccggca tggagccgcg atgaattcga agcgcaattg      60
cgtgcgaagg gacaggcgta tcacatccat catccgttca acgtgaaaat gaacagcggc     120
ggatgttcgc acgagcagat tcgcggctgg gtcgcgaacc gcttctacta tcagatcaac     180
attccgctga aggatgccgc ggtgctgtcg aactgtcccg accgcgacac gcgccgccgc     240
tgggtgctgc gcatactcga tcacgacggt tacggcgaag acgaaggcgg catcgaaaca     300
tgggcgcggc tcggcgatgc ggtcggcctg tcgcgcgatg aactgtggtc gctcgaacac     360
gtggtgccgg gcgtgcgctt cgcggtcgac gcatacgtga actttgcgcg gcgcgcgccg     420
tggcaggaag ccgtctgctc gtcgctcacc gaaatcttcg cgccgcagat ccacaaagac     480
cggctcgcca catggcccga acactacccg tggatcaagc ccgaagggct cgcatatttc     540
cgttcgcgca tttcgcttgc acaacgcgac gtcgaacacg ggctgtccgt cacgctcgat     600
catttccgca cgcgcgacca gcaacagcgc gcgctcgaca ttctgcaatt caagctcgac     660
attctgtgga cgatgctcga cgccatcgag aaggccttcc cagcatga                 708

<210>  171
<211>  235
<212>  PRT
<213>  Burkholderia phymatum

<400>  171
Met Ser Val Lys Gly Ser Ser Gly Pro Ala Trp Ser Arg Asp Glu Phe
1               5                   10                  15
Glu Ala Gln Leu Arg Ala Lys Gly Gln Ala Tyr His Ile His His Pro
            20                  25                  30
Phe Asn Val Lys Met Asn Ser Gly Gly Cys Ser His Glu Gln Ile Arg
        35                  40                  45
Gly Trp Val Ala Asn Arg Phe Tyr Tyr Gln Ile Asn Ile Pro Leu Lys
    50                  55                  60
Asp Ala Ala Val Leu Ser Asn Cys Pro Asp Arg Asp Thr Arg Arg Arg
65                  70                  75                  80
Trp Val Leu Arg Ile Leu Asp His Asp Gly Tyr Gly Glu Asp Glu Gly
                85                  90                  95
Gly Ile Glu Thr Trp Ala Arg Leu Gly Asp Ala Val Gly Leu Ser Arg
            100                 105                 110
Asp Glu Leu Trp Ser Leu Glu His Val Val Pro Gly Val Arg Phe Ala
        115                 120                 125
Val Asp Ala Tyr Val Asn Phe Ala Arg Arg Ala Pro Trp Gln Glu Ala
    130                 135                 140
Val Cys Ser Ser Leu Thr Glu Ile Phe Ala Pro Gln Ile His Lys Asp
145                 150                 155                 160
Arg Leu Ala Thr Trp Pro Glu His Tyr Pro Trp Ile Lys Pro Glu Gly
                165                 170                 175
Leu Ala Tyr Phe Arg Ser Arg Ile Ser Leu Ala Gln Arg Asp Val Glu
            180                 185                 190
His Gly Leu Ser Val Thr Leu Asp His Phe Arg Thr Arg Asp Gln Gln
        195                 200                 205
Gln Arg Ala Leu Asp Ile Leu Gln Phe Lys Leu Asp Ile Leu Trp Thr
    210                 215                 220
Met Leu Asp Ala Ile Glu Lys Ala Phe Pro Ala
225                 230                 235

<210>  172
<211>  720
<212>  DNA
<213>  Burkholderia cenocepacia

<400>  172
atgaacgcac cgatccccgc tacggcgttg cacgcgacgc cctggaccgc gaccgaattc      60
gaggcgcgcc tgcgtgcgct cgaatcgcgc tatcacattc atcatccgtt caaccggcgg     120
ctcaacagcg gcgcctgttc gcccacgcag atccgcggct gggtcgcgaa ccgcttctac     180
taccagatct gcatcccgct caaggatgcg gcgatcctgt cgaactgccc cgaccgcgag     240
acgcgacgcc gctggattca gcggctgatc gaccacgacg ggcagggcga caacgcaggc     300
ggcatcgagg cgtgggtgcg gctcggcgaa gcgtgcggcc tcacgcgcga cgagctgtgg     360
tcgctcgaac acgtgctgcc cggcgtgcgt ttcgcggtgg atgcgtacgt gaacttcgcg     420
cggcgcgcgc cgtggcagga agccgtgtgc tcgtcgctga ccgagatgtt cgcgccgcag     480
```

atccatctcg accggctcgc gggctggccg tcgcattacc cgtggatcga accggccggg 540
ctgcattact tccgcagccg cgtgccgctc gcgcagcgcg acgtcgagca cgggctcgcg 600
gtgacgctcg cgcatttcac gacaccggag gcgcagcagc gcgcgctcgg cattctgcaa 660
ttcaagctcg acatcctgtg gtcgatgctc gacgccgtcg aaaaggccta cccgttatga 720

<210> 173
<211> 239
<212> PRT
<213> Burkholderia cenocepacia

<400> 173
Met Asn Ala Pro Ile Pro Ala Thr Ala Leu His Ala Thr Pro Trp Thr
1 5 10 15
Ala Thr Glu Phe Glu Ala Arg Leu Arg Ala Leu Glu Ser Arg Tyr His
20 25 30
Ile His His Pro Phe Asn Arg Arg Leu Asn Ser Gly Ala Cys Ser Pro
35 40 45
Thr Gln Ile Arg Gly Trp Val Ala Asn Arg Phe Tyr Tyr Gln Ile Cys
50 55 60
Ile Pro Leu Lys Asp Ala Ala Ile Leu Ser Asn Cys Pro Asp Arg Glu
65 70 75 80
Thr Arg Arg Arg Trp Ile Gln Arg Leu Ile Asp His Asp Gly Gln Gly
85 90 95
Asp Asn Ala Gly Gly Ile Glu Ala Trp Val Arg Leu Gly Glu Ala Cys
100 105 110
Gly Leu Thr Arg Asp Glu Leu Trp Ser Leu Glu His Val Leu Pro Gly
115 120 125
Val Arg Phe Ala Val Asp Ala Tyr Val Asn Phe Ala Arg Arg Ala Pro
130 135 140
Trp Gln Glu Ala Val Cys Ser Ser Leu Thr Glu Met Phe Ala Pro Gln
145 150 155 160
Ile His Leu Asp Arg Leu Ala Gly Trp Pro Ser His Tyr Pro Trp Ile
165 170 175
Glu Pro Ala Gly Leu His Tyr Phe Arg Ser Arg Val Pro Leu Ala Gln
180 185 190
Arg Asp Val Glu His Gly Leu Ala Val Thr Leu Ala His Phe Thr Thr
195 200 205
Pro Glu Ala Gln Gln Arg Ala Leu Gly Ile Leu Gln Phe Lys Leu Asp
210 215 220
Ile Leu Trp Ser Met Leu Asp Ala Val Glu Lys Ala Tyr Pro Leu
225 230 235

<210> 174
<211> 720
<212> DNA
<213> Burkholderia cenocepacia

<400> 174
atgaacgcac cgatccccgc tacggcgttg cacgcgacgc cctggaccgc gaccgaattc 60
gaggcgcgcc tgcgtgcgct cgaatcgcgc tatcacattc atcatccgtt caaccggcgg 120
ctcaacagcg gcgcctgttc gcccacgcag atccgcggct gggtcgcgaa ccgcttctac 180
taccagatct gcatcccgct caaggatgcg gcgatcctgt cgaactgccc cgaccgcgag 240
acgcggcgcc gctggattca gcggctgatc gaccacgacg ggcagggcga caacgcaggc 300
ggcatcgagg cgtgggtgcg gctcggcgaa gcgtgcggcc tgacgcgcga cgagctgtgg 360
tcgctcgaac acgtgctgcc gggcgtgcgt ttcgcggtgg atgcgtatgt gaacttcgcg 420
cggcgcgcgc cctggcagga agccgtgtgc tcgtcgctga ccgagatgtt cgcgccgcag 480
atccatctcg accggctcgc gggctggccg tcgcattacc cgtggatcga accggccggg 540
ctgcattact tccgcagccg cgtgccgctc gcgcagcgcg acgtcgagca tgggctcgcg 600
gtgacgctcg cgcatttcac gacaccggac gcgcagcagc gcgcgctcgg cattctgcaa 660
ttcaagctcg acatcctgtg gtcgatgctc gacgccgtcg aaaaggccta cccgttatga 720

<210> 175
<211> 239
<212> PRT
<213> Burkholderia cenocepacia

<400> 175
Met Asn Ala Pro Ile Pro Ala Thr Ala Leu His Ala Thr Pro Trp Thr
1 5 10 15
Ala Thr Glu Phe Glu Ala Arg Leu Arg Ala Leu Glu Ser Arg Tyr His
20 25 30
Ile His His Pro Phe Asn Arg Arg Leu Asn Ser Gly Ala Cys Ser Pro
35 40 45
Thr Gln Ile Arg Gly Trp Val Ala Asn Arg Phe Tyr Tyr Gln Ile Cys

```
    50                  55                  60
Ile Pro Leu Lys Asp Ala Ala Ile Leu Ser Asn Cys Pro Asp Arg Glu
65                  70                  75                  80
Thr Arg Arg Arg Trp Ile Gln Arg Leu Ile Asp His Asp Gly Gln Gly
                85                  90                  95
Asp Asn Ala Gly Gly Ile Glu Ala Trp Val Arg Leu Gly Glu Ala Cys
            100                 105                 110
Gly Leu Thr Arg Asp Glu Leu Trp Ser Leu Glu His Val Leu Pro Gly
        115                 120                 125
Val Arg Phe Ala Val Asp Ala Tyr Val Asn Phe Ala Arg Arg Ala Pro
    130                 135                 140
Trp Gln Glu Ala Val Cys Ser Ser Leu Thr Glu Met Phe Ala Pro Gln
145                 150                 155                 160
Ile His Leu Asp Arg Leu Ala Gly Trp Pro Ser His Tyr Pro Trp Ile
                165                 170                 175
Glu Pro Ala Gly Leu His Tyr Phe Arg Ser Arg Val Pro Leu Ala Gln
            180                 185                 190
Arg Asp Val Glu His Gly Leu Ala Val Thr Leu Ala His Phe Thr Thr
        195                 200                 205
Pro Asp Ala Gln Gln Arg Ala Leu Gly Ile Leu Gln Phe Lys Leu Asp
    210                 215                 220
Ile Leu Trp Ser Met Leu Asp Ala Val Glu Lys Ala Tyr Pro Leu
225                 230                 235
```

<210> 176
<211> 720
<212> DNA
<213> Burkholderia cenocepacia

<400> 176

```
atgaacgcac cgatccccgc tacggcgttg cacgcgacgc cctggaccgc gaccgaattc      60
gaggcgcgcc tgcgtgcgct cgaatcgcgc tatcacatcc accatccgtt caaccggcgg     120
ctcaacagcg gcgcttgttc gcccacgcag attcgcggct gggtcgcgaa ccgtttctat     180
taccagatct gcatcccgct caaggatgcg gcgatcctgt cgaactgccc cgaccgcgac     240
acgcggcgcc gctggatcca gcggctgatc gaccacgacg ggcagggcga caacgccggc     300
ggcatcgagg cgtgggtgcg gctcggcgaa gcgtgcggcc tggcgcgcga cgagctgtgg     360
tcgctcgaac acgtgctgcc cggcgtgcgt ttcgcggtgg atgcgtatgt gaacttcgcg     420
cggcgcgcgc cctggcagga agccgtgtgc tcgtcgctga ccgagatgtt cgcgccgcag     480
atccatctcg accggctcgc gggctggccg tcgcattacc cgtggatcga gccggccggg     540
ctgcattact tccgcagccg cgtgccgctc gcgcagcgcg acgtcgagca tgggctcgcg     600
gtgacgctcg cgcatttcac gacaccggac gcgcagcagc gcgcgctcgg cattctgcaa     660
ttcaagctcg acatcctgtg gtcgatgctc gacgccgtcg aaaaggccta cccgttatga     720
```

<210> 177
<211> 239
<212> PRT
<213> Burkholderia cenocepacia

<400> 177

```
Met Asn Ala Pro Ile Pro Ala Thr Ala Leu His Ala Thr Pro Trp Thr
1               5                   10                  15
Ala Thr Glu Phe Glu Ala Arg Leu Arg Ala Leu Glu Ser Arg Tyr His
            20                  25                  30
Ile His His Pro Phe Asn Arg Arg Leu Asn Ser Gly Ala Cys Ser Pro
        35                  40                  45
Thr Gln Ile Arg Gly Trp Val Ala Asn Arg Phe Tyr Tyr Gln Ile Cys
    50                  55                  60
Ile Pro Leu Lys Asp Ala Ala Ile Leu Ser Asn Cys Pro Asp Arg Asp
65                  70                  75                  80
Thr Arg Arg Arg Trp Ile Gln Arg Leu Ile Asp His Asp Gly Gln Gly
                85                  90                  95
Asp Asn Ala Gly Gly Ile Glu Ala Trp Val Arg Leu Gly Glu Ala Cys
            100                 105                 110
Gly Leu Ala Arg Asp Glu Leu Trp Ser Leu Glu His Val Leu Pro Gly
        115                 120                 125
Val Arg Phe Ala Val Asp Ala Tyr Val Asn Phe Ala Arg Arg Ala Pro
    130                 135                 140
Trp Gln Glu Ala Val Cys Ser Ser Leu Thr Glu Met Phe Ala Pro Gln
145                 150                 155                 160
Ile His Leu Asp Arg Leu Ala Gly Trp Pro Ser His Tyr Pro Trp Ile
                165                 170                 175
Glu Pro Ala Gly Leu His Tyr Phe Arg Ser Arg Val Pro Leu Ala Gln
            180                 185                 190
Arg Asp Val Glu His Gly Leu Ala Val Thr Leu Ala His Phe Thr Thr
```

```
        195                 200                 205
Pro Asp Ala Gln Gln Arg Ala Leu Gly Ile Leu Gln Phe Lys Leu Asp
    210                 215                 220
Ile Leu Trp Ser Met Leu Asp Ala Val Glu Lys Ala Tyr Pro Leu
225                 230                 235
```

<210> 178
<211> 696
<212> DNA
<213> Dechloromonas aromatica

<400> 178

```
atgatgaccc cgtggaatca cgccgaattc gaggcgaaac tgcgcgacaa gggggccgct      60
taccacatct accatccgtt caacgtcttg ctgaacaccg ggcaggcgaa gcgcgaacag     120
atccagggct gggtggccaa ccgctactat taccagatca tcatcccggt caaggatgcg     180
gcgatcatgt ccaactgccc tgatcgcgaa atacggcgcg actggattca gcgcatcatc     240
gaccacgatg gccgtggcga cgacgccggt ggtatcgaag cgtggattcg cctaggtgaa     300
gcggtcgggc tggcgcgaga agaaatcacc gatctgcgcc acgttattcc agccgtgcgc     360
tttgcctgtg atgcctatct caacttctgt cgccggcagc cgtggcagga agcggtgtgt     420
tcgtcgctga ccgagttgtt cgcgccgaag attcacaagg agcgcctggc caactggccc     480
gagttctacc catggatcga atcgaccggc ctgcagtatt ttcgtaaccg ggttacccag     540
gccaggcgcg acgtcgagca ggggctggcg gttaccctag actacttcga taccccggcc     600
ctgcaggctc gtgcgctgga catcctgcag ttcaagctcg acatcctgtg gtcaatgaac     660
gatgccatgg cgctgcgcta tggcgtcaac aaatga                               696
```

<210> 179
<211> 231
<212> PRT
<213> Dechloromonas aromatica

<400> 179

```
Met Met Thr Pro Trp Asn His Ala Glu Phe Glu Ala Lys Leu Arg Asp
1               5                   10                  15
Lys Gly Ala Ala Tyr His Ile Tyr His Pro Phe Asn Val Leu Leu Asn
            20                  25                  30
Thr Gly Gln Ala Lys Arg Glu Gln Ile Gln Gly Trp Val Ala Asn Arg
        35                  40                  45
Tyr Tyr Tyr Gln Ile Ile Ile Pro Val Lys Asp Ala Ala Ile Met Ser
    50                  55                  60
Asn Cys Pro Asp Arg Glu Ile Arg Arg Asp Trp Ile Gln Arg Ile Ile
65                  70                  75                  80
Asp His Asp Gly Arg Gly Asp Asp Ala Gly Gly Ile Glu Ala Trp Ile
                85                  90                  95
Arg Leu Gly Glu Ala Val Gly Leu Ala Arg Glu Glu Ile Thr Asp Leu
            100                 105                 110
Arg His Val Ile Pro Ala Val Arg Phe Ala Cys Asp Ala Tyr Leu Asn
        115                 120                 125
Phe Cys Arg Arg Gln Pro Trp Gln Glu Ala Val Cys Ser Ser Leu Thr
    130                 135                 140
Glu Leu Phe Ala Pro Lys Ile His Lys Glu Arg Leu Ala Asn Trp Pro
145                 150                 155                 160
Glu Phe Tyr Pro Trp Ile Glu Ser Thr Gly Leu Gln Tyr Phe Arg Asn
                165                 170                 175
Arg Val Thr Gln Ala Arg Arg Asp Val Glu Gln Gly Leu Ala Val Thr
            180                 185                 190
Leu Asp Tyr Phe Asp Thr Pro Ala Leu Gln Ala Arg Ala Leu Asp Ile
        195                 200                 205
Leu Gln Phe Lys Leu Asp Ile Leu Trp Ser Met Asn Asp Ala Met Ala
    210                 215                 220
Leu Arg Tyr Gly Val Asn Lys
225                 230
```

<210> 180
<211> 723
<212> DNA
<213> Nitrosococcus oceani

<400> 180

```
gtggcgggaa aaaaaccctg gagccgtgaa gaattcgagc agaagctgca ggaaaaagaa      60
tgcttctacc acattcacca ccccttccag gtattaatga acaacggcaa gctgaaccag     120
taccaggtcc aaggatgggt agccaaccgc ttttattacc atacctgtat ccctctcaag     180
gatgccgcta tcctagccaa ctgctcccag cgatcagtgc ggcgccaatg ggtacagcga     240
atcctggatc atgacggtta cggcgaagac gtaggtggca tcgaagcctg gcttcagcta     300
ggcgaagccg tgggcttaag ccgggaaacg ctggaatccc agcaacaggt tctgcccgga     360
```

```
gtacgctttg ccgttgacgc ttatgtcaac tttgcccgcc atgctacctg gcaggaagct    420
gtctgctctt ctcttacgga gctctttgcc ccttcgctcc atcaacaacg attagacaac    480
tggcctcgct attacccttg gattaaagcg gaaggctacc attattttcg caagcgcctc    540
aacgaagccc gacgggatgt tcgacacggc ctagaagcga ccctggatta ctttcaaagc    600
cgcacccagc aggagcaggc gctagctatc ttacagttta aactggatgt actatggacc    660
ctactggatg ccctctggat ggcgtacatt gaaaatcgtc ccccctatca cacagagctt    720
taa                                                                  723

<210>  181
<211>  240
<212>  PRT
<213>  Nitrosococcus oceani

<400>  181
Met Ala Gly Lys Lys Pro Trp Ser Arg Glu Glu Phe Glu Gln Lys Leu
1               5                   10                  15
Gln Glu Lys Glu Cys Phe Tyr His Ile His His Pro Phe Gln Val Leu
            20                  25                  30
Met Asn Asn Gly Lys Leu Asn Gln Tyr Gln Val Gln Gly Trp Val Ala
        35                  40                  45
Asn Arg Phe Tyr Tyr His Thr Cys Ile Pro Leu Lys Asp Ala Ala Ile
    50                  55                  60
Leu Ala Asn Cys Ser Gln Arg Ser Val Arg Arg Gln Trp Val Gln Arg
65                  70                  75                  80
Ile Leu Asp His Asp Gly Tyr Gly Glu Asp Val Gly Gly Ile Glu Ala
                85                  90                  95
Trp Leu Gln Leu Gly Glu Ala Val Gly Leu Ser Arg Glu Thr Leu Glu
            100                 105                 110
Ser Gln Gln Gln Val Leu Pro Gly Val Arg Phe Ala Val Asp Ala Tyr
        115                 120                 125
Val Asn Phe Ala Arg His Ala Thr Trp Gln Glu Ala Val Cys Ser Ser
    130                 135                 140
Leu Thr Glu Leu Phe Ala Pro Ser Leu His Gln Gln Arg Leu Asp Asn
145                 150                 155                 160
Trp Pro Arg Tyr Tyr Pro Trp Ile Lys Ala Glu Gly Tyr His Tyr Phe
                165                 170                 175
Arg Lys Arg Leu Asn Glu Ala Arg Arg Asp Val Arg His Gly Leu Glu
            180                 185                 190
Ala Thr Leu Asp Tyr Phe Gln Ser Arg Thr Gln Gln Glu Gln Ala Leu
        195                 200                 205
Ala Ile Leu Gln Phe Lys Leu Asp Val Leu Trp Thr Leu Leu Asp Ala
    210                 215                 220
Leu Trp Met Ala Tyr Ile Glu Asn Arg Pro Pro Tyr His Thr Glu Leu
225                 230                 235                 240

<210>  182
<211>  714
<212>  DNA
<213>  Ralstonia pickettii

<400>  182
tcagcgagtg ggatacgcgc gctcaatcgc gtcaagcatc gaccagagga catcaagttt     60
gaattgcagg atgtcgaggg cgcgctcttg ctgcgcacgt gtcgtgaagt gtgccagtgt    120
caccttcagg ccatgttcga catcgcgctg tgcgagcgtg atccggctgc ggaagtagtg    180
cagaccaccg gcttcgatcc acggataatg ttgcggccag ttggctagcc ggtcgcggtg    240
aatctttggc gcaaacattt cggtcagcga tgagcacacc gcctcttgcc acggcacgct    300
ccgtgcaaaa ttcacgtaag cgtcgacggc aaagcgtaca cccggcacta catgcttgag    360
cgaccagagg tcttgctcag gtatgcctgc agcctcaccc agccgcaccc aggcctcgat    420
accgccaggg ctatcagcat tcccatcatg atcgagaatt ctctgcaccc aaagccggcg    480
ggtttcgcga tccgggcaat tggcgatgat ggcgccatcc ttgcgaggga tgttgacctg    540
gtaataaaaa cggttggcga tccagccccg aacctggtcg ggcgtcagtt caccattgtt    600
catgcgcacg ttgaacggat gatggatgtg gtagccagcg ccctttgcgc gcaactgcac    660
ctcgaactcc tcaggcggcc atgcagcgtc ggggcttgcg ttggttcggc tcaa          714

<210>  183
<211>  237
<212>  PRT
<213>  Ralstonia pickettii

<400>  183
Met Ser Arg Thr Asn Ala Ser Pro Asp Ala Ala Trp Pro Pro Glu Glu
1               5                   10                  15
Phe Glu Val Gln Leu Arg Ala Lys Gly Ala Gly Tyr His Ile His His
            20                  25                  30
```

```
Pro Phe Asn Val Arg Met Asn Asn Gly Glu Leu Thr Pro Asp Gln Val
            35                  40                  45
Arg Gly Trp Ile Ala Asn Arg Phe Tyr Tyr Gln Val Asn Ile Pro Arg
    50                  55                  60
Lys Asp Gly Ala Ile Ile Ala Asn Cys Pro Asp Arg Glu Thr Arg Arg
65                  70                  75                  80
Leu Trp Val Gln Arg Ile Leu Asp His Asp Gly Asn Ala Asp Ser Pro
                85                  90                  95
Gly Gly Ile Glu Ala Trp Val Arg Leu Gly Glu Ala Ala Gly Ile Pro
            100                 105                 110
Glu Gln Asp Leu Trp Ser Leu Lys His Val Val Pro Gly Val Arg Phe
        115                 120                 125
Ala Val Asp Ala Tyr Val Asn Phe Ala Arg Ser Val Pro Trp Gln Glu
    130                 135                 140
Ala Val Cys Ser Ser Leu Thr Glu Met Phe Ala Pro Lys Ile His Arg
145                 150                 155                 160
Asp Arg Leu Ala Asn Trp Pro Gln His Tyr Pro Trp Ile Glu Ala Gly
                165                 170                 175
Gly Leu His Tyr Phe Arg Ser Arg Ile Thr Leu Ala Gln Arg Asp Val
            180                 185                 190
Glu His Gly Leu Lys Val Thr Leu Ala His Phe Thr Thr Arg Ala Gln
        195                 200                 205
Gln Glu Arg Ala Leu Asp Ile Leu Gln Phe Lys Leu Asp Val Leu Trp
    210                 215                 220
Ser Met Leu Asp Ala Ile Glu Arg Ala Tyr Pro Thr Arg
225                 230                 235

<210>  184
<211>  792
<212>  DNA
<213>  Azoarcus sp.

<400>  184
atggaagccg aactgctcgc cgcgcctgct gcacgtacgc ttgcccccaa cgccggcacc      60
cacgaccgcc cgccgatgtc gcgcgaggaa ttcgaagcgc agctgcgcgc caagggcacc     120
agctaccaca tctaccaccc cttccacgtg atgatggccg aagggcgcct gacgccggcc     180
cagctgcgcg gctgggtggc gaaccgcttc tactaccaga tcgcgatccc ggtgaaggac     240
gcggccatca tgtccaactg cccggaccgc gagatccgcc gcgagtggat acagcgcatc     300
ctcgaccacg acggctacga gatcggcggt gtgtccgacc ccggcggcat cgaggcctgg     360
atacggctgg gcgaggcggt ggggctgagc cgcgacgatg tcacctcgct gcgcttcgtc     420
gcgccggcgg cgcgctttgc ggtggatgcc tacatcaatt tcgcccgcca gcggccgtgg     480
gaagaggcgg tggcttccag ccttaccgag ttgttcgcac cccacatcca ccagcagcgc     540
atcgacacct ggccgcaggt ctatccctgg gtgaagaccg agggtttgca gtatttccgc     600
aaccgcctga cccaggcgcg ccgcgacgtc gcccacggcc tgcgcttcac gctggagcac     660
ttcagccaga cgcgcgcgct gcaggaacgc gcgctggaga tcctgcagtt caagctcgac     720
gtgctgtggg cgctggccga cgcgatcatg ctcagccagt gcgaaatcga gatcaagggg     780
ccgaaggcat ga                                                         792

<210>  185
<211>  263
<212>  PRT
<213>  Azoarcus sp.

<400>  185
Met Glu Ala Glu Leu Leu Ala Ala Pro Ala Ala Arg Thr Leu Ala Pro
1               5                   10                  15
Asn Ala Gly Thr His Asp Arg Pro Pro Met Ser Arg Glu Glu Phe Glu
            20                  25                  30
Ala Gln Leu Arg Ala Lys Gly Thr Ser Tyr His Ile Tyr His Pro Phe
        35                  40                  45
His Val Met Met Ala Glu Gly Arg Leu Thr Pro Ala Gln Leu Arg Gly
    50                  55                  60
Trp Val Ala Asn Arg Phe Tyr Tyr Gln Ile Ala Ile Pro Val Lys Asp
65                  70                  75                  80
Ala Ala Ile Met Ser Asn Cys Pro Asp Arg Glu Ile Arg Arg Glu Trp
                85                  90                  95
Ile Gln Arg Ile Leu Asp His Asp Gly Tyr Glu Ile Gly Gly Val Ser
            100                 105                 110
Asp Pro Gly Gly Ile Glu Ala Trp Ile Arg Leu Gly Glu Ala Val Gly
        115                 120                 125
Leu Ser Arg Asp Asp Val Thr Ser Leu Arg Phe Val Ala Pro Ala Ala
    130                 135                 140
Arg Phe Ala Val Asp Ala Tyr Ile Asn Phe Ala Arg Gln Arg Pro Trp
145                 150                 155                 160
```

```
Glu Glu Ala Val Ala Ser Ser Leu Thr Glu Leu Phe Ala Pro His Ile
                165                 170                 175
His Gln Gln Arg Ile Asp Thr Trp Pro Gln Val Tyr Pro Trp Val Lys
            180                 185                 190
Thr Glu Gly Leu Gln Tyr Phe Arg Asn Arg Leu Thr Gln Ala Arg Arg
        195                 200                 205
Asp Val Ala His Gly Leu Arg Phe Thr Leu Glu His Phe Ser Gln Thr
    210                 215                 220
Arg Ala Leu Gln Glu Arg Ala Leu Glu Ile Leu Gln Phe Lys Leu Asp
225                 230                 235                 240
Val Leu Trp Ala Leu Ala Asp Ala Ile Met Leu Ser Gln Cys Glu Ile
                245                 250                 255
Glu Ile Lys Gly Pro Lys Ala
            260
```

<210> 186
<211> 720
<212> DNA
<213> Burkholderia multivorans

<400> 186

```
tcatgcgcaa tacgcctttt caatggcatc gaggatcgac cacagcacgt cgagcttgaa      60
cgtcagaatc tcgagcgcgc gccgttgcgc gtccgcggtc gtgaaatgcg cgagcgtgac     120
cgcgaggccg tgttcgacgt cgcgctgcgc gagcggcagg cggctgcgga aatactgcag     180
cccgtccgcg tcgatccacg aatagtgcga cggccagccg gcgagacggt cgaggtggat     240
ctgcggcgcg aacatctcgg tcagcgacga gcagaccgct tcctgccacg tcgcgcgccg     300
cgcgaagttc acgtacgcgt cgacggcaaa gcgcacgccc ggcagcacgt gctcgagcga     360
ccacagcgcc gcgcgatcga gaccgaccgc ctcgccgagc cgcacccacg cttcgatgcc     420
gccggcatcg tcgccgtgcc cgtcgtgatc gaggatgcgc tgcacccaca gccggcgcgt     480
gtcgcggtcc ggacagttcg agaggatcgc cgcgtccttc agcgggatgc tgatctgata     540
gtagaagcgg ttcgcgaccc agccgcgaat ctgcgcgggc gtgcacgcgc ccgcattgag     600
ccgccggttg aacggatggt gaatgtgata gcgcgattcg agcgcgcgca gacgcgcctc     660
gaattcgtcg gcgctccacg ggctcgcgtg cagcgccgcc gcggaaatcg gtgcgttcat     720
```

<210> 187
<211> 239
<212> PRT
<213> Burkholderia multivorans

<400> 187

```
Met Asn Ala Pro Ile Ser Ala Ala Ala Leu His Ala Ser Pro Trp Ser
1               5                   10                  15
Ala Asp Glu Phe Glu Ala Arg Leu Arg Ala Leu Glu Ser Arg Tyr His
            20                  25                  30
Ile His His Pro Phe Asn Arg Arg Leu Asn Ala Gly Ala Cys Thr Pro
        35                  40                  45
Ala Gln Ile Arg Gly Trp Val Ala Asn Arg Phe Tyr Tyr Gln Ile Ser
    50                  55                  60
Ile Pro Leu Lys Asp Ala Ala Ile Leu Ser Asn Cys Pro Asp Arg Asp
65                  70                  75                  80
Thr Arg Arg Leu Trp Val Gln Arg Ile Leu Asp His Asp Gly His Gly
                85                  90                  95
Asp Asp Ala Gly Gly Ile Glu Ala Trp Val Arg Leu Gly Glu Ala Val
            100                 105                 110
Gly Leu Asp Arg Ala Ala Leu Trp Ser Leu Glu His Val Leu Pro Gly
        115                 120                 125
Val Arg Phe Ala Val Asp Ala Tyr Val Asn Phe Ala Arg Arg Ala Thr
    130                 135                 140
Trp Gln Glu Ala Val Cys Ser Ser Leu Thr Glu Met Phe Ala Pro Gln
145                 150                 155                 160
Ile His Leu Asp Arg Leu Ala Gly Trp Pro Ser His Tyr Ser Trp Ile
                165                 170                 175
Asp Ala Asp Gly Leu Gln Tyr Phe Arg Ser Arg Leu Pro Leu Ala Gln
            180                 185                 190
Arg Asp Val Glu His Gly Leu Ala Val Thr Leu Ala His Phe Thr Thr
        195                 200                 205
Ala Asp Ala Gln Arg Arg Ala Leu Glu Ile Leu Thr Phe Lys Leu Asp
    210                 215                 220
Val Leu Trp Ser Ile Leu Asp Ala Ile Glu Lys Ala Tyr Cys Ala
225                 230                 235
```

<210> 188
<211> 735
<212> DNA

```
<213>  Verminephrobacter eiseniae

<400>  188
atgcccgccc ccgttgcccc agccattgcg cccaccgtcc cgccagccat ccccgacgcc      60
gagcgcgccg ccttcgaggc ccggctgcgc gcactgcaag cgcgctacca catccaccac     120
ccgttcaacc aacgcatggc cagcgggcaa tgctcacgcg cccagataca gggctgggtg     180
gcgaaccgct tttactacca ggtcaacatt ccgctcaagg atgcggccat cctgtcgaac     240
tgccccgacc gcgcaacgcg gcgcgaatgg gtggtgcgca ttctcgacca cgatggcagc     300
gccgccgagc ccggcggcat cgaagcctgg agccggctcg gcgaggccgt ggggctgacg     360
cgcgcggccc tgtggtcaca gcagatggtg ctgcccgggg tgcgctttgc ggtcgatgcc     420
tacgtcaact ttgcgcggcg cgcaccttgg caggaagccg tgtgctcgtc gctgaccgag     480
atgttcgcgc ccgccatcca ccaggaacgg ctggccggct ggccgcagca ctacccctgg     540
atcgaagccg aaggtctggc ctatttccgt tcacgcatcc cgctggcgca gcgcgatgtc     600
gagcatggcc tgcgcgtcac gctcgagcac tttcgcacgc cggccgagca gcagcgcgcc     660
acgcaaatcc tgcagttcaa gctcgacatc ctgtggtcga tgctcgacgc gatcgaaaag     720
gcgtaccccg aatga                                                      735

<210>  189
<211>  244
<212>  PRT
<213>  Verminephrobacter eiseniae

<400>  189
Met Pro Ala Pro Val Ala Pro Ala Ile Ala Pro Thr Val Pro Pro Ala
1               5                   10                  15
Ile Pro Asp Ala Glu Arg Ala Ala Phe Glu Ala Arg Leu Arg Ala Leu
            20                  25                  30
Gln Ala Arg Tyr His Ile His His Pro Phe Asn Gln Arg Met Ala Ser
        35                  40                  45
Gly Gln Cys Ser Arg Ala Gln Ile Gln Gly Trp Val Ala Asn Arg Phe
    50                  55                  60
Tyr Tyr Gln Val Asn Ile Pro Leu Lys Asp Ala Ala Ile Leu Ser Asn
65                  70                  75                  80
Cys Pro Asp Arg Ala Thr Arg Arg Glu Trp Val Val Arg Ile Leu Asp
                85                  90                  95
His Asp Gly Ser Ala Ala Glu Pro Gly Gly Ile Glu Ala Trp Ser Arg
            100                 105                 110
Leu Gly Glu Ala Val Gly Leu Thr Arg Ala Ala Leu Trp Ser Gln Gln
        115                 120                 125
Met Val Leu Pro Gly Val Arg Phe Ala Val Asp Ala Tyr Val Asn Phe
    130                 135                 140
Ala Arg Arg Ala Pro Trp Gln Glu Ala Val Cys Ser Ser Leu Thr Glu
145                 150                 155                 160
Met Phe Ala Pro Ala Ile His Gln Glu Arg Leu Ala Gly Trp Pro Gln
                165                 170                 175
His Tyr Pro Trp Ile Glu Ala Glu Gly Leu Ala Tyr Phe Arg Ser Arg
            180                 185                 190
Ile Pro Leu Ala Gln Arg Asp Val Glu His Gly Leu Arg Val Thr Leu
        195                 200                 205
Glu His Phe Arg Thr Pro Ala Glu Gln Gln Arg Ala Thr Gln Ile Leu
    210                 215                 220
Gln Phe Lys Leu Asp Ile Leu Trp Ser Met Leu Asp Ala Ile Glu Lys
225                 230                 235                 240
Ala Tyr Pro Glu


<210>  190
<211>  720
<212>  DNA
<213>  Burkholderia vietnamiensis

<400>  190
tcatgcggga tatgcctttt cgatcgcatc gagaatctgc cacaggacat ccagcttgaa      60
cgacaggatg tcgagcgcgc gccgttgcgc gtcaggtgtc gtgaaatgac tcagcgtcac     120
cgcgagcccg tgctcgacgt cgcgctgcgc gagcggcacg cggctgcgga aatactgcag     180
gccgtcggcc tcgatccacg ggtagtgcga cggccagccg gccagccggt cgaggtggat     240
ctgcggcgcg aacatctcgg tcagcgacga gcacacggct tcctgccacg gcgcgcgacg     300
cgcgaagttg acgtacgcat cgaccgcgaa gcgcacgccc ggcagcacgt gctcgagcga     360
ccacagctgc gcgcgctcga tgccgaccgc ctcgccgagc cgcgcccacg cctcgatgcc     420
gccttcgctc tcgccgtgcc cgtcgtggtc gagaatccgc tcgacccaca ggcggcgcgt     480
ctcgcggtcg gggcagttcg acaggatcgc cgcgtccttc agcggaatgc tgatctgata     540
gtagaagcgg ttcgcgaccc agccgcgaat ctgctcgcgc gagcattcgc cggcattcat     600
ccgccggttg aacggatgat gaatgtgata gcgcgattcg agcgcgcgca ggcgcgcctc     660
gaactcctgc gcgctccagg gcgtcgcgtg cagcgccgcg gcgggaatcg gtgcgttcat     720
```

<210> 191
<211> 239
<212> PRT
<213> Burkholderia vietnamiensis

<400> 191
Met Asn Ala Pro Ile Pro Ala Ala Ala Leu His Ala Thr Pro Trp Ser
1 5 10 15
Ala Gln Glu Phe Glu Ala Arg Leu Arg Ala Leu Glu Ser Arg Tyr His
20 25 30
Ile His His Pro Phe Asn Arg Arg Met Asn Ala Gly Glu Cys Ser Arg
35 40 45
Glu Gln Ile Arg Gly Trp Val Ala Asn Arg Phe Tyr Tyr Gln Ile Ser
50 55 60
Ile Pro Leu Lys Asp Ala Ala Ile Leu Ser Asn Cys Pro Asp Arg Glu
65 70 75 80
Thr Arg Arg Leu Trp Val Glu Arg Ile Leu Asp His Asp Gly His Gly
85 90 95
Glu Ser Glu Gly Gly Ile Glu Ala Trp Ala Arg Leu Gly Glu Ala Val
100 105 110
Gly Ile Glu Arg Ala Gln Leu Trp Ser Leu Glu His Val Leu Pro Gly
115 120 125
Val Arg Phe Ala Val Asp Ala Tyr Val Asn Phe Ala Arg Arg Ala Pro
130 135 140
Trp Gln Glu Ala Val Cys Ser Ser Leu Thr Glu Met Phe Ala Pro Gln
145 150 155 160
Ile His Leu Asp Arg Leu Ala Gly Trp Pro Ser His Tyr Pro Trp Ile
165 170 175
Glu Ala Asp Gly Leu Gln Tyr Phe Arg Ser Arg Val Pro Leu Ala Gln
180 185 190
Arg Asp Val Glu His Gly Leu Ala Val Thr Leu Ser His Phe Thr Thr
195 200 205
Pro Asp Ala Gln Arg Arg Ala Leu Asp Ile Leu Ser Phe Lys Leu Asp
210 215 220
Val Leu Trp Gln Ile Leu Asp Ala Ile Glu Lys Ala Tyr Pro Ala
225 230 235

<210> 192
<211> 735
<212> DNA
<213> Methylophilales bacterium

<400> 192
atgacaaaac cttggacaag agacgagttt gaagatcgac taagaaagaa gggtaagggg 60
tatcatatat accatccttt tcacgtaatg atgtacgaag gaaaacttag caaagaacaa 120
ctacaaagct gggttttaaa ccgtttttac tatcagataa gcatcccatt aaaagatgcg 180
gcaatcttat caaattgccc cgtgaaagaa atcagaaaag aatggatagt aagaatatta 240
gaccatgatg gagagggaga tgctgacggt gggattgagg catggattaa tttaggtaga 300
gcagtcggac taacacgaga ggatgttact tctttaaagc atgtaagtcc tggagttaaa 360
tttgccgtag atgcctatat aaatttcgca aaacaaaaat cgtggcaaga gtctgtttgc 420
tcctcattaa ctgaattatt tgcaccacac attcatcaac aaagaattag ctcatggccg 480
tctatgtacc catggatcga tgaatctggc ctcacatact ttaaaaagcg acttacggaa 540
gcgagacggg atgtggagca tggtttaggt gtaacccttg attattttag taaaacacgt 600
gaaacacaag agcaagcttt agaaatactt caatttaaac ttaatgtttt atgggttatt 660
gcagattcaa tcatgctagc atcatctaat attaaggtag aagatcgaga ctacttaagg 720
caaccaataa actaa 735

<210> 193
<211> 244
<212> PRT
<213> Methylophilales bacterium

<400> 193
Met Thr Lys Pro Trp Thr Arg Asp Glu Phe Glu Asp Arg Leu Arg Lys
1 5 10 15
Lys Gly Lys Gly Tyr His Ile Tyr His Pro Phe His Val Met Met Tyr
20 25 30
Glu Gly Lys Leu Ser Lys Glu Gln Leu Gln Ser Trp Val Leu Asn Arg
35 40 45
Phe Tyr Tyr Gln Ile Ser Ile Pro Leu Lys Asp Ala Ala Ile Leu Ser
50 55 60
Asn Cys Pro Val Lys Glu Ile Arg Lys Glu Trp Ile Val Arg Ile Leu
65 70 75 80

```
Asp His Asp Gly Glu Gly Asp Ala Asp Gly Gly Ile Glu Ala Trp Ile
                85                  90                  95
Asn Leu Gly Arg Ala Val Gly Leu Thr Arg Glu Asp Val Thr Ser Leu
            100                 105                 110
Lys His Val Ser Pro Gly Val Lys Phe Ala Val Asp Ala Tyr Ile Asn
        115                 120                 125
Phe Ala Lys Gln Lys Ser Trp Gln Glu Ser Val Cys Ser Ser Leu Thr
    130                 135                 140
Glu Leu Phe Ala Pro His Ile His Gln Gln Arg Ile Ser Ser Trp Pro
145                 150                 155                 160
Ser Met Tyr Pro Trp Ile Asp Glu Ser Gly Leu Thr Tyr Phe Lys Lys
                165                 170                 175
Arg Leu Thr Glu Ala Arg Arg Asp Val Glu His Gly Leu Gly Val Thr
            180                 185                 190
Leu Asp Tyr Phe Ser Lys Thr Arg Glu Thr Gln Glu Gln Ala Leu Glu
        195                 200                 205
Ile Leu Gln Phe Lys Leu Asn Val Leu Trp Val Ile Ala Asp Ser Ile
    210                 215                 220
Met Leu Ala Ser Ser Asn Ile Lys Val Glu Asp Arg Asp Tyr Leu Arg
225                 230                 235                 240
Gln Pro Ile Asn
```

<210> 194
<211> 753
<212> DNA
<213> Methylobacillus flagellatus

<400> 194

```
ttatcgtggc tgtctgagat agtcgacatg ttgttctacc ttgatgttgg ttgaggcaag      60
cataatggca tcggcaatga cccacagcac atccagcttg aattgcagaa tatccagtgc     120
ctttcttgc aattcacggc tctggctgaa gtaatccagg gtcacggcga gcccttgctc      180
gacatcgcgc ctggcttcgg tcaggcgctt cttgaaatag gtcaggccct cttccttaac     240
ccagggatac atgctgggcc aggagctgat gcgctgttga tggatgtgcg gcgcaaacag     300
ttcggtcaga gaggaacaga cggcctcctg ccaagggcgc tgcttggcga aattgacata     360
ggcatccact gcgaacctga caccggcgc gacaaacttg agggacgtca cgtcctcgcg      420
actcaggccg actgcctggc cgagctgtat ccacgcctcg atgccaccga cgttatttgc     480
atccccgtca tggtcaatga tgcgttggat ccatccctta cggatttcct tgtccgggca     540
gttggacaga atggccgcat ccttttgcgg gatgctgatc tggtaataaa agcggttggc     600
gacccagcac tgcaattgct cgcgggtgag cttgccttca tacatcatca cgtggaacgg     660
gtggtagata tggtatcccc tacccttgtc gcgcagcttc tgctcgaact cttcgcgtgt     720
ccagggaata ttatctgtca gtggtgcatt cat                                  753
```

<210> 195
<211> 250
<212> PRT
<213> Methylobacillus flagellatus

<400> 195

```
Met Asn Ala Pro Leu Thr Asp Asn Ile Pro Trp Thr Arg Glu Glu Phe
1               5                   10                  15
Glu Gln Lys Leu Arg Asp Lys Gly Arg Gly Tyr His Ile Tyr His Pro
            20                  25                  30
Phe His Val Met Met Tyr Glu Gly Lys Leu Thr Arg Glu Gln Leu Gln
        35                  40                  45
Cys Trp Val Ala Asn Arg Phe Tyr Tyr Gln Ile Ser Ile Pro Gln Lys
    50                  55                  60
Asp Ala Ala Ile Leu Ser Asn Cys Pro Asp Lys Glu Ile Arg Lys Gly
65                  70                  75                  80
Trp Ile Gln Arg Ile Ile Asp His Asp Gly Asp Ala Asn Asn Val Gly
                85                  90                  95
Gly Ile Glu Ala Trp Ile Gln Leu Gly Gln Ala Val Gly Leu Ser Arg
            100                 105                 110
Glu Asp Val Thr Ser Leu Lys Phe Val Ala Pro Gly Val Arg Phe Ala
        115                 120                 125
Val Asp Ala Tyr Val Asn Phe Ala Lys Gln Arg Pro Trp Gln Glu Ala
    130                 135                 140
Val Cys Ser Ser Leu Thr Glu Leu Phe Ala Pro His Ile His Gln Gln
145                 150                 155                 160
Arg Ile Ser Ser Trp Pro Ser Met Tyr Pro Trp Val Lys Glu Glu Gly
                165                 170                 175
Leu Thr Tyr Phe Lys Lys Arg Leu Thr Glu Ala Arg Arg Asp Val Glu
            180                 185                 190
Gln Gly Leu Ala Val Thr Leu Asp Tyr Phe Ser Gln Ser Arg Glu Leu
```

```
        195                 200                 205
Gln Glu Lys Ala Leu Asp Ile Leu Gln Phe Lys Leu Asp Val Leu Trp
    210                 215                 220
Val Ile Ala Asp Ala Ile Met Leu Ala Ser Thr Asn Ile Lys Val Glu
225                 230                 235                 240
Gln His Val Asp Tyr Leu Arg Gln Pro Arg
                245                 250
```

<210> 196
<211> 693
<212> DNA
<213> Ralstonia eutropha

<400> 196

```
atgacatatt ccaccgcatc gggtcccgcg gagttcgaag cgcggctgcg cgccaaagaa     60
accggctacc acattcatca cccgttcaac cagcgcatgg cggccggatt gtgttcgccg    120
ggacagatcc gcgcctgggt cgccaaccgc ttctactacc aggccaatat cccgctgaag    180
gacgccgcca tccttgccaa ctgccccgag cgcgacacgc gccgcgagtg ggtggtacgc    240
atcctggacc acgacgggac tgacacccag cccggtggca ttgaagcctg gctgcggctg    300
ggccaagcca tagggctcga gcgcgcggca ttgcttgacc atcggcacgt gctcccaggg    360
gtgcgctttg ccgtcgacgc ctatgtcaac tttgcgcggc gagcgccgtg gcaggaagcc    420
gtgtgctcct cgctcaccga gatgtttgcc ccggagatcc acaaggagcg gctcgcgggc    480
tggcccacgc attacccttg gatcgaggcc gccgggctcg attactttcg ctcgcgcatc    540
ccgctcgcgc agcgcgacgt cgagcacggc ctgcgcgtga cgctgggcta cttccgtaca    600
ccggagcaac agcaacgcgc gctcgatatc ctgcaattca agctcgacgt gctgtggtcg    660
atgctcgatg ccatccagct agccaaccca tga                                 693
```

<210> 197
<211> 230
<212> PRT
<213> Ralstonia eutropha

<400> 197

```
Met Thr Tyr Ser Thr Ala Ser Gly Pro Ala Glu Phe Glu Ala Arg Leu
1               5                   10                  15
Arg Ala Lys Glu Thr Gly Tyr His Ile His His Pro Phe Asn Gln Arg
            20                  25                  30
Met Ala Ala Gly Leu Cys Ser Pro Gly Gln Ile Arg Ala Trp Val Ala
        35                  40                  45
Asn Arg Phe Tyr Tyr Gln Ala Asn Ile Pro Leu Lys Asp Ala Ala Ile
    50                  55                  60
Leu Ala Asn Cys Pro Glu Arg Asp Thr Arg Arg Glu Trp Val Val Arg
65                  70                  75                  80
Ile Leu Asp His Asp Gly Thr Asp Thr Gln Pro Gly Gly Ile Glu Ala
                85                  90                  95
Trp Leu Arg Leu Gly Gln Ala Ile Gly Leu Glu Arg Ala Ala Leu Leu
            100                 105                 110
Asp His Arg His Val Leu Pro Gly Val Arg Phe Ala Val Asp Ala Tyr
        115                 120                 125
Val Asn Phe Ala Arg Arg Ala Pro Trp Gln Glu Ala Val Cys Ser Ser
    130                 135                 140
Leu Thr Glu Met Phe Ala Pro Glu Ile His Lys Glu Arg Leu Ala Gly
145                 150                 155                 160
Trp Pro Thr His Tyr Pro Trp Ile Glu Ala Ala Gly Leu Asp Tyr Phe
                165                 170                 175
Arg Ser Arg Ile Pro Leu Ala Gln Arg Asp Val Glu His Gly Leu Arg
            180                 185                 190
Val Thr Leu Gly Tyr Phe Arg Thr Pro Glu Gln Gln Gln Arg Ala Leu
        195                 200                 205
Asp Ile Leu Gln Phe Lys Leu Asp Val Leu Trp Ser Met Leu Asp Ala
    210                 215                 220
Ile Gln Leu Ala Asn Pro
225                 230
```

<210> 198
<211> 720
<212> DNA
<213> Burkholderia ambifaria

<400> 198

```
atgaacgcac cgattcccgc cgcggcgctg cacgcgacgc cctggagtgc gcaggagttc     60
gaggcgcgtc tgcgcgcgct cgaatcgcgc taccacatcc atcacccgtt caaccggcgc    120
ctgaataccg gcgcgtgctc gcccgagcag atccgcggct gggtcgcgaa ccgcttctac    180
taccagatca gcattccgct gaaggacgcg gcgatcctgt ccaattgccc ggaccgcgat    240
```

acgcgccgac tgtgggtcga gcggatcctc gatcacgatg gtcacggcgg gcaggaaggc 300
gggatcgagg catgggcgcg cctcggcgaa gcggtcggca tcgagcgcgc ggaattgtgg 360
tcgctcgtgc acgtgctgcc cggcgtgcgg ttcgcggtcg acgcgtacgt gaatttcgcg 420
cggcatgcgc catggcagga agccgtgtgc tcgtcgctga ccgagatgtt cgcgccacag 480
atccatctcg atcggctcgc ggggtggccg tcccactatc cgtggatcga ggcggacggg 540
ctgcagtatt tccgcagccg cgtgccgctc gcgcagcgcg acgtcgagca cgggctcgcg 600
gtcacgctga accatttcac gacaccggac gcgcaacggc gcgcgctcga catcctgtcg 660
ttcaagctgg atgtgctgtg gtcgattctc gacgcgattg aaaaggccta ccccgcatga 720

<210> 199
<211> 239
<212> PRT
<213> Burkholderia ambifaria

<400> 199
Met Asn Ala Pro Ile Pro Ala Ala Ala Leu His Ala Thr Pro Trp Ser
1 5 10 15
Ala Gln Glu Phe Glu Ala Arg Leu Arg Ala Leu Glu Ser Arg Tyr His
20 25 30
Ile His His Pro Phe Asn Arg Arg Leu Asn Thr Gly Ala Cys Ser Pro
35 40 45
Glu Gln Ile Arg Gly Trp Val Ala Asn Arg Phe Tyr Tyr Gln Ile Ser
50 55 60
Ile Pro Leu Lys Asp Ala Ala Ile Leu Ser Asn Cys Pro Asp Arg Asp
65 70 75 80
Thr Arg Arg Leu Trp Val Glu Arg Ile Leu Asp His Asp Gly His Gly
85 90 95
Gly Gln Glu Gly Gly Ile Glu Ala Trp Ala Arg Leu Gly Glu Ala Val
100 105 110
Gly Ile Glu Arg Ala Glu Leu Trp Ser Leu Val His Val Leu Pro Gly
115 120 125
Val Arg Phe Ala Val Asp Ala Tyr Val Asn Phe Ala Arg His Ala Pro
130 135 140
Trp Gln Glu Ala Val Cys Ser Ser Leu Thr Glu Met Phe Ala Pro Gln
145 150 155 160
Ile His Leu Asp Arg Leu Ala Gly Trp Pro Ser His Tyr Pro Trp Ile
165 170 175
Glu Ala Asp Gly Leu Gln Tyr Phe Arg Ser Arg Val Pro Leu Ala Gln
180 185 190
Arg Asp Val Glu His Gly Leu Ala Val Thr Leu Asn His Phe Thr Thr
195 200 205
Pro Asp Ala Gln Arg Arg Ala Leu Asp Ile Leu Ser Phe Lys Leu Asp
210 215 220
Val Leu Trp Ser Ile Leu Asp Ala Ile Glu Lys Ala Tyr Pro Ala
225 230 235

<210> 200
<211> 711
<212> DNA
<213> Ralstonia eutropha

<400> 200
tcatgggttg gcttgctgga tggcgtcgag catcgaccag agcacgtcga gcttgaactg 60
caggatgtcg agggcgcgct gctgttgcgc cgcgctgcgg aaatggccca gcgtcacgcg 120
caggccatgg tccacgtcgc gctgcgcgag cggaatgcga gagcggaagt agtccagccc 180
ggcggcctcg atccacgggt aatgcgtggg ccagccggcc aggcgttcct tgtggattgc 240
gggcgcgaac atctccgtca gcgacgagca cacggcttcc tgccacggtg cgcggcgcgc 300
aaagttgacg taggcatcga cggcaaagcg caccccgggc agcacgtgcc ggtggctcag 360
cagtgcttcg cgctccaggc ccaccgcctg gcccaggcgc agccaggcct cgatgccgcc 420
gggctgggtg tcggtgccgt cgtggtcaag gatgcgcacc acccactcgc ggcgggtgtc 480
gcgctcgggg caattggaca ggatggcggc gtccttcagc gggatgttgt cctgatagta 540
gaagcggttg gccacccatg cgcggatctg cgcgggcgaa cactggcctg ccgccatgcg 600
ctggttgaag ggatggtgga tgtggtagcc gctttccatg gcgcgcaggc gggcctcgaa 660
ctcggcctcg gtccaggcga tggggggggat ggggaggatg gcagttgtca t 711

<210> 201
<211> 236
<212> PRT
<213> Ralstonia eutropha

<400> 201
Met Thr Thr Ala Ile Leu Pro Ile Pro Pro Ile Ala Trp Thr Glu Ala
1 5 10 15
Glu Phe Glu Ala Arg Leu Arg Ala Met Glu Ser Gly Tyr His Ile His

```
            20                  25                  30
His Pro Phe Asn Gln Arg Met Ala Ala Gly Gln Cys Ser Pro Ala Gln
        35                  40                  45
Ile Arg Ala Trp Val Ala Asn Arg Phe Tyr Tyr Gln Asp Asn Ile Pro
    50                  55                  60
Leu Lys Asp Ala Ala Ile Leu Ser Asn Cys Pro Glu Arg Asp Thr Arg
65                  70                  75                  80
Arg Glu Trp Val Val Arg Ile Leu Asp His Asp Gly Thr Asp Thr Gln
                85                  90                  95
Pro Gly Gly Ile Glu Ala Trp Leu Arg Leu Gly Gln Ala Val Gly Leu
            100                 105                 110
Glu Arg Glu Ala Leu Leu Ser His Arg His Val Leu Pro Gly Val Arg
        115                 120                 125
Phe Ala Val Asp Ala Tyr Val Asn Phe Ala Arg Arg Ala Pro Trp Gln
    130                 135                 140
Glu Ala Val Cys Ser Ser Leu Thr Glu Met Phe Ala Pro Ala Ile His
145                 150                 155                 160
Lys Glu Arg Leu Ala Gly Trp Pro Thr His Tyr Pro Trp Ile Glu Ala
                165                 170                 175
Ala Gly Leu Asp Tyr Phe Arg Ser Arg Ile Pro Leu Ala Gln Arg Asp
            180                 185                 190
Val Asp His Gly Leu Arg Val Thr Leu Gly His Phe Arg Ser Ala Ala
        195                 200                 205
Gln Gln Gln Arg Ala Leu Asp Ile Leu Gln Phe Lys Leu Asp Val Leu
    210                 215                 220
Trp Ser Met Leu Asp Ala Ile Gln Gln Ala Asn Pro
225                 230                 235

<210>  202
<211>  711
<212>  DNA
<213>  Ralstonia pickettii

<400>  202
atgacacatc ctgttgcctg gacggccgaa gagttcgaag cacggctgcg cgcgaagcag      60
gcgggctatc acattcatca tccgttcaac ctgcgcatgc gcgctggcga atgcacgccg     120
gaggaaatcc gcacgtgggt tgcgaaccgt ttctactacc aggtcaacat cccgctcaag     180
gatgcggcca tcctgtccaa ctgccccgac cgggcaacgc gccgcgagtg gatcgtccgc     240
atccacgacc acgacggcga cgatgcgcag ccgggcggca tcgagagctg ggtgcgctta     300
ggcgttgccg tgggcctgac gcgcgacgcg ctgtggtctc accggcatct gctgccgggc     360
gtgcgctttg cggtggatgc gtatgtgaac tttgcgcgcc gcgcgccgtg gcaggaagca     420
gtgtgctcct cgctcacgga gatgttcgcg ccggacattc acaaggaacg actggccggc     480
tggcccgagc actatccgtg ggtgcagccg gaggggctgg cgtacttccg ctcgcgcatt     540
ccgctggcct cgcgcgacgt cgagcacggc ctgcgcgtga cactcgacta cttccgcacg     600
ccggagcagc aactgcgtgc gctggacatc ctgcagttca agctcgacat cctctggtcg     660
atgctcgatg ccatccaaca ggcccacgca catgagccct cccttgtctg a              711

<210>  203
<211>  236
<212>  PRT
<213>  Ralstonia pickettii

<400>  203
Met Thr His Pro Val Ala Trp Thr Ala Glu Glu Phe Glu Ala Arg Leu
1               5                   10                  15
Arg Ala Lys Gln Ala Gly Tyr His Ile His His Pro Phe Asn Leu Arg
            20                  25                  30
Met Arg Ala Gly Glu Cys Thr Pro Glu Glu Ile Arg Thr Trp Val Ala
        35                  40                  45
Asn Arg Phe Tyr Tyr Gln Val Asn Ile Pro Leu Lys Asp Ala Ala Ile
    50                  55                  60
Leu Ser Asn Cys Pro Asp Arg Ala Thr Arg Arg Glu Trp Ile Val Arg
65                  70                  75                  80
Ile His Asp His Asp Gly Asp Asp Ala Gln Pro Gly Gly Ile Glu Ser
                85                  90                  95
Trp Val Arg Leu Gly Val Ala Val Gly Leu Thr Arg Asp Ala Leu Trp
            100                 105                 110
Ser His Arg His Leu Leu Pro Gly Val Arg Phe Ala Val Asp Ala Tyr
        115                 120                 125
Val Asn Phe Ala Arg Arg Ala Pro Trp Gln Glu Ala Val Cys Ser Ser
    130                 135                 140
Leu Thr Glu Met Phe Ala Pro Asp Ile His Lys Glu Arg Leu Ala Gly
145                 150                 155                 160
Trp Pro Glu His Tyr Pro Trp Val Gln Pro Glu Gly Leu Ala Tyr Phe
```

```
                165                 170                 175
Arg Ser Arg Ile Pro Leu Ala Ser Arg Asp Val Glu His Gly Leu Arg
            180                 185                 190
Val Thr Leu Asp Tyr Phe Arg Thr Pro Glu Gln Gln Leu Arg Ala Leu
        195                 200                 205
Asp Ile Leu Gln Phe Lys Leu Asp Ile Leu Trp Ser Met Leu Asp Ala
    210                 215                 220
Ile Gln Gln Ala His Ala His Glu Pro Ser Leu Val
225                 230                 235

<210>  204
<211>  768
<212>  DNA
<213>  Acidiphilium cryptum

<400>  204
atgatgagag ccgatctcct ggccgaggcg ccaccagccg atgatggccg cgcgccctgg      60
tctcacgccg aattcgaggc gaagctgcgc gaggccggca gcgcctacca catccaccat     120
ccgttcaacg tgatgctcaa caccggcaag gcgacgccgg agcagatccg catgtgggtc     180
gccaaccgct tctattacca gatcgcgatc ccggtgaagg acgcggcgat cctctccaac     240
tgccccgacc gcgagatccg ccgcggctgg atccgccgcc tcctcgacca tgacgggttc     300
gactacgaac tgccccgacgg ctcgcgcctg cgcgacgagg gcggcatcga ggcctggctc     360
cgcctcggca tcgccaccgg cctcgcgcgc gaggagatgc tcgacctgcg ccacctcttg     420
cccggcgtgc gcttcgcggt cgacgcctat gtcaatttcg cccgccgcgc gccctggcag     480
gaggccgtct gctcctcgct gaccgagctt ttcgcccccg acatccaccg ccagcgcctc     540
gccacctggc ccggccacta tccctggatc gagagcgagg ggctcgacta cttccgcaac     600
cgcaccagcc aggccccgcg cgacgtcgtc cacggcctgc ggatcacgct cgcccatttc     660
gcgacccggc cgatgcagga gcgcgccctg cagatcctca agttcaagct ggacattctg     720
tggacgatga acgacgagat gggacgacat tacggcgtcg ccgcctga                  768

<210>  205
<211>  255
<212>  PRT
<213>  Acidiphilium cryptum

<400>  205
Met Met Arg Ala Asp Leu Leu Ala Glu Ala Pro Pro Ala Asp Asp Gly
1               5                   10                  15
Arg Ala Pro Trp Ser His Ala Glu Phe Glu Ala Lys Leu Arg Glu Ala
            20                  25                  30
Gly Ser Ala Tyr His Ile His His Pro Phe Asn Val Met Leu Asn Thr
        35                  40                  45
Gly Lys Ala Thr Pro Glu Gln Ile Arg Met Trp Val Ala Asn Arg Phe
    50                  55                  60
Tyr Tyr Gln Ile Ala Ile Pro Val Lys Asp Ala Ala Ile Leu Ser Asn
65                  70                  75                  80
Cys Pro Asp Arg Glu Ile Arg Arg Gly Trp Ile Arg Arg Leu Leu Asp
                85                  90                  95
His Asp Gly Phe Asp Tyr Glu Leu Pro Asp Gly Ser Arg Leu Arg Asp
            100                 105                 110
Glu Gly Gly Ile Glu Ala Trp Leu Arg Leu Gly Ile Ala Thr Gly Leu
        115                 120                 125
Ala Arg Glu Glu Met Leu Asp Leu Arg His Leu Leu Pro Gly Val Arg
    130                 135                 140
Phe Ala Val Asp Ala Tyr Val Asn Phe Ala Arg Arg Ala Pro Trp Gln
145                 150                 155                 160
Glu Ala Val Cys Ser Ser Leu Thr Glu Leu Phe Ala Pro Asp Ile His
                165                 170                 175
Arg Gln Arg Leu Ala Thr Trp Pro Gly His Tyr Pro Trp Ile Glu Ser
            180                 185                 190
Glu Gly Leu Asp Tyr Phe Arg Asn Arg Thr Ser Gln Ala Pro Arg Asp
        195                 200                 205
Val Val His Gly Leu Arg Ile Thr Leu Ala His Phe Ala Thr Arg Pro
    210                 215                 220
Met Gln Glu Arg Ala Leu Gln Ile Leu Lys Phe Lys Leu Asp Ile Leu
225                 230                 235                 240
Trp Thr Met Asn Asp Glu Met Gly Arg His Tyr Gly Val Ala Ala
                245                 250                 255

<210>  206
<211>  729
<212>  DNA
<213>  Burkholderia dolosa
```

<400> 206
atgaccacga tgaacgcacc gcttcccgct accgcgctgc acgcgacgcc ctggagcgcg 60
caggagttcg aggcgcacct gcgcgcgctc gaatcgcgct atcacattca tcatccgttc 120
aaccggcggc tgaactcggg cgcgtgctcg cccgagcaga tccgcggctg ggtcgcgaac 180
cgcttctact accagatcag cattccgctg aaggacgcgg cgatcctgtc gaattgtccg 240
gaccgcgaca cgcgccgact gtgggtgcag cggattctcg atcacgacgg acacggcggc 300
gacgcaggcg gaatcgaagc gtgggcgcgg ctcggcgaag cggtcggcat cccgcgctcg 360
caactgtggt cgctcgaacg cgtgctgccc ggcgtgcgct tcgcggtcga tgcgtatgtg 420
aacttcgcgc gccgcgcgcc gtggcaggaa gccgtgtgct cgtcgctgac ggagatgttc 480
gcgccgcaga tccacctcga ccggctcgcg ggctggccgt cgcactaccc gtggatcgac 540
gcggacgggc tgcagtattt ccgcagccgc gtgccgctcg cgcagcgcga cgtcgagcac 600
gggctcgcgg tgacgctgcg ccatttcacg acaccggaag cgcaacgacg agcgctcgac 660
atcctgtcgt tcaagctgga tgtcctgtgg tcgatcctcg atgcaatcga aaaggcgtat 720
cccgcatga 729

<210> 207
<211> 242
<212> PRT
<213> Burkholderia dolosa

<400> 207
Met Thr Thr Met Asn Ala Pro Leu Pro Ala Thr Ala Leu His Ala Thr
1 5 10 15
Pro Trp Ser Ala Gln Glu Phe Glu Ala His Leu Arg Ala Leu Glu Ser
20 25 30
Arg Tyr His Ile His His Pro Phe Asn Arg Arg Leu Asn Ser Gly Ala
35 40 45
Cys Ser Pro Glu Gln Ile Arg Gly Trp Val Ala Asn Arg Phe Tyr Tyr
50 55 60
Gln Ile Ser Ile Pro Leu Lys Asp Ala Ala Ile Leu Ser Asn Cys Pro
65 70 75 80
Asp Arg Asp Thr Arg Arg Leu Trp Val Gln Arg Ile Leu Asp His Asp
85 90 95
Gly His Gly Gly Asp Ala Gly Gly Ile Glu Ala Trp Ala Arg Leu Gly
100 105 110
Glu Ala Val Gly Ile Pro Arg Ser Gln Leu Trp Ser Leu Glu Arg Val
115 120 125
Leu Pro Gly Val Arg Phe Ala Val Asp Ala Tyr Val Asn Phe Ala Arg
130 135 140
Arg Ala Pro Trp Gln Glu Ala Val Cys Ser Ser Leu Thr Glu Met Phe
145 150 155 160
Ala Pro Gln Ile His Leu Asp Arg Leu Ala Gly Trp Pro Ser His Tyr
165 170 175
Pro Trp Ile Asp Ala Asp Gly Leu Gln Tyr Phe Arg Ser Arg Val Pro
180 185 190
Leu Ala Gln Arg Asp Val Glu His Gly Leu Ala Val Thr Leu Arg His
195 200 205
Phe Thr Thr Pro Glu Ala Gln Arg Arg Ala Leu Asp Ile Leu Ser Phe
210 215 220
Lys Leu Asp Val Leu Trp Ser Ile Leu Asp Ala Ile Glu Lys Ala Tyr
225 230 235 240
Pro Ala

<210> 208
<211> 720
<212> DNA
<213> Burkholderia dolosa

<400> 208
atgaacgcac cgcttcccgc taccgcgctg cacgcgacgc cctggagcgc gcaggagttc 60
gaggcgcacc tgcgcgcgct cgaatcgcgc tatcacattc atcatccgtt caaccggcgg 120
ctgaactcgg gcgcgtgctc gcccgagcag atccgcggct gggtcgcgaa ccgcttctac 180
taccagatca gcattccgct gaaggacgcg gcgatcctgt cgaattgtcc ggaccgcgac 240
acgcgccgac tgtgggtgca gcggattctc gatcacgacg gacacggcgg cgacgcaggc 300
ggaatcgaag cgtgggcgcg gctcggcgaa gcggtcggca tcccgcgctc gcaactgtgg 360
tcgctcgaac gcgtgctgcc cggcgtgcgc ttcgcggtcg atgcgtatgt gaacttcgcg 420
cgccgcgcgc cgtggcagga agccgtgtgc tcgtcgctga cggagatgtt cgcgccgcag 480
atccacctcg accggctcgc gggctggccg tcgcactacc cgtggatcga cgcggacggg 540
ctgcagtatt tccgcagccg cgtgccgctc gcgcagcgcg acgtcgagca cgggctcgcg 600
gtgacgctgc gccatttcac gacaccggaa gcgcaacgac gagcgctcga catcctgtcg 660
ttcaagctgg atgtcctgtg gtcgatcctc gatgcaatcg aaaaggcgta tcccgcatga 720

<210> 209

<211> 239
<212> PRT
<213> Burkholderia dolosa

<400> 209
```
Met Asn Ala Pro Leu Pro Ala Thr Ala Leu His Ala Thr Pro Trp Ser
1               5                   10                  15
Ala Gln Glu Phe Glu Ala His Leu Arg Ala Leu Glu Ser Arg Tyr His
            20                  25                  30
Ile His His Pro Phe Asn Arg Arg Leu Asn Ser Gly Ala Cys Ser Pro
        35                  40                  45
Glu Gln Ile Arg Gly Trp Val Ala Asn Arg Phe Tyr Tyr Gln Ile Ser
    50                  55                  60
Ile Pro Leu Lys Asp Ala Ala Ile Leu Ser Asn Cys Pro Asp Arg Asp
65                  70                  75                  80
Thr Arg Arg Leu Trp Val Gln Arg Ile Leu Asp His Asp Gly His Gly
                85                  90                  95
Gly Asp Ala Gly Gly Ile Glu Ala Trp Ala Arg Leu Gly Glu Ala Val
            100                 105                 110
Gly Ile Pro Arg Ser Gln Leu Trp Ser Leu Glu Arg Val Leu Pro Gly
        115                 120                 125
Val Arg Phe Ala Val Asp Ala Tyr Val Asn Phe Ala Arg Arg Ala Pro
    130                 135                 140
Trp Gln Glu Ala Val Cys Ser Ser Leu Thr Glu Met Phe Ala Pro Gln
145                 150                 155                 160
Ile His Leu Asp Arg Leu Ala Gly Trp Pro Ser His Tyr Pro Trp Ile
                165                 170                 175
Asp Ala Asp Gly Leu Gln Tyr Phe Arg Ser Arg Val Pro Leu Ala Gln
            180                 185                 190
Arg Asp Val Glu His Gly Leu Ala Val Thr Leu Arg His Phe Thr Thr
        195                 200                 205
Pro Glu Ala Gln Arg Arg Ala Leu Asp Ile Leu Ser Phe Lys Leu Asp
    210                 215                 220
Val Leu Trp Ser Ile Leu Asp Ala Ile Glu Lys Ala Tyr Pro Ala
225                 230                 235
```

<210> 210
<211> 732
<212> DNA
<213> Polaromonas naphthalenivorans

<400> 210
```
tcatacgcag gctttctcga tgccgtcgag catgctccac aggatgtcga gcttgaactg      60
caggatgtcc agcgccctgt gctgcgcctg gcgggtggtg aaatgggtga tggtgaccgc     120
cagcccgtgt tccacatcgc ggcttgccag cggaatacgg ctgcggaaat aagtcaggcc     180
ttcgggctcg atccaggcgt aatgcaaagg ccagctggca agccggtcct tgtggatctg     240
gggtgcgaac atttcggtca gcgatgagca cacggcttcc tgccacggtg ctttcgcggc     300
gaaattcacg taggcatcgc aggcgaaccg caccgccggc gccacgccct gcaacgacca     360
caattcctca cgcgacaggc cgaccgccag gcccaactgg gtccaggctt ccatgccgcc     420
agcggcgcag ccctggtagt cgccgtagcc gtcatggtcc agaatgcgtt ccacccacag     480
tcggcggtgc gcccggtcag gcatgttggc caaaatcgcc gcgtccttgc gcggaatgca     540
caactggtaa taaaaacggt tggccaccca gcaacggatt tcgtctgcgg tgcagccgcc     600
ctggttcatc cgtacattga agggatggtg aatgtgatag gccgtgcctt tggcgcgcag     660
ctgcctttca aactcggcag ggctccaggc tgatctgctg gcgtcaaagc agggtgattg     720
ggctatttcc at                                                         732
```

<210> 211
<211> 243
<212> PRT
<213> Polaromonas naphthalenivorans

<400> 211
```
Met Glu Ile Ala Gln Ser Pro Cys Phe Asp Ala Ser Arg Ser Ala Trp
1               5                   10                  15
Ser Pro Ala Glu Phe Glu Arg Gln Leu Arg Ala Lys Gly Thr Ala Tyr
            20                  25                  30
His Ile His His Pro Phe Asn Val Arg Met Asn Gln Gly Gly Cys Thr
        35                  40                  45
Ala Asp Glu Ile Arg Cys Trp Val Ala Asn Arg Phe Tyr Tyr Gln Leu
    50                  55                  60
Cys Ile Pro Arg Lys Asp Ala Ala Ile Leu Ala Asn Met Pro Asp Arg
65                  70                  75                  80
Ala His Arg Arg Leu Trp Val Glu Arg Ile Leu Asp His Asp Gly Tyr
                85                  90                  95
```

```
Gly Asp Tyr Gln Gly Cys Ala Ala Gly Gly Met Glu Ala Trp Thr Gln
            100                 105                 110
Leu Gly Leu Ala Val Gly Leu Ser Arg Glu Glu Leu Trp Ser Leu Gln
        115                 120                 125
Gly Val Ala Pro Ala Val Arg Phe Ala Cys Asp Ala Tyr Val Asn Phe
    130                 135                 140
Ala Ala Lys Ala Pro Trp Gln Glu Ala Val Cys Ser Ser Leu Thr Glu
145                 150                 155                 160
Met Phe Ala Pro Gln Ile His Lys Asp Arg Leu Ala Ser Trp Pro Leu
                165                 170                 175
His Tyr Ala Trp Ile Glu Pro Glu Gly Leu Thr Tyr Phe Arg Ser Arg
            180                 185                 190
Ile Pro Leu Ala Ser Arg Asp Val Glu His Gly Leu Ala Val Thr Ile
        195                 200                 205
Thr His Phe Thr Thr Arg Gln Ala Gln His Arg Ala Leu Asp Ile Leu
    210                 215                 220
Gln Phe Lys Leu Asp Ile Leu Trp Ser Met Leu Asp Gly Ile Glu Lys
225                 230                 235                 240
Ala Cys Val


<210>  212
<211>  741
<212>  DNA
<213>  Rickettsiella grylli

<400>  212
atgccgacta aacgctgcga agtatcattg tctcagagtg tatttgaaaa aaaaatttta      60
aaccttgaaa aatattatca tatccatcat cctttcaatc aattaatgaa cgatggccaa     120
ttgactcggc aacaaattca acagtgggtg attaatcgct tctattatca agtcaatatc     180
cctgtaaaag atgctgctat tttatccaat tgtcctctac gggagatacg aagagaatgg     240
gtgaaacgga tcattgaaca cgatggcgat gtcattaatc ccggtggaat tgaagcctgg     300
ctgacattgg gagaagcgtg cggactgacg cgtaacttat tgtggtctat gaaatatgtc     360
ctgccgggcg tgcgctttgc agtagacgct tacattcagt ttacacgaac gcatccatgg     420
caagctgcgg tttgttcatc gttaacggag ctttttgcac ccgaaatcca tcgccaacgt     480
ttaacgaatt ggcccatcca ttatccatgg atggatttaa acgcattagc ttattttcgt     540
tgtcgattaa atcaaatcaa tcatcatgtc aaaaaaggct tacaatggac actcgaaaat     600
tttcaaacac acgaacagca aacacaagca ttggctattt taaaatttaa attagaaatt     660
ctttggtcaa tttcagacag cctttactta gcctatgtat taggtcaccc tcccatcaca     720
ttttcaaagg atgatacatg a                                               741

<210>  213
<211>  246
<212>  PRT
<213>  Rickettsiella grylli

<400>  213
Met Pro Thr Lys Arg Cys Glu Val Ser Leu Ser Gln Ser Val Phe Glu
1               5                   10                  15
Lys Lys Ile Leu Asn Leu Glu Lys Tyr Tyr His Ile His His Pro Phe
            20                  25                  30
Asn Gln Leu Met Asn Asp Gly Gln Leu Thr Arg Gln Gln Ile Gln Gln
        35                  40                  45
Trp Val Ile Asn Arg Phe Tyr Tyr Gln Val Asn Ile Pro Val Lys Asp
    50                  55                  60
Ala Ala Ile Leu Ser Asn Cys Pro Leu Arg Glu Ile Arg Arg Glu Trp
65                  70                  75                  80
Val Lys Arg Ile Ile Glu His Asp Gly Asp Val Ile Asn Pro Gly Gly
                85                  90                  95
Ile Glu Ala Trp Leu Thr Leu Gly Glu Ala Cys Gly Leu Thr Arg Asn
            100                 105                 110
Leu Leu Trp Ser Met Lys Tyr Val Leu Pro Gly Val Arg Phe Ala Val
        115                 120                 125
Asp Ala Tyr Ile Gln Phe Thr Arg Thr His Pro Trp Gln Ala Ala Val
    130                 135                 140
Cys Ser Ser Leu Thr Glu Leu Phe Ala Pro Glu Ile His Arg Gln Arg
145                 150                 155                 160
Leu Thr Asn Trp Pro Ile His Tyr Pro Trp Met Asp Leu Asn Ala Leu
                165                 170                 175
Ala Tyr Phe Arg Cys Arg Leu Asn Gln Ile Asn His His Val Lys Lys
            180                 185                 190
Gly Leu Gln Trp Thr Leu Glu Asn Phe Gln Thr His Glu Gln Gln Thr
        195                 200                 205
Gln Ala Leu Ala Ile Leu Lys Phe Lys Leu Glu Ile Leu Trp Ser Ile
```

```
    210                 215                 220
Ser Asp Ser Leu Tyr Leu Ala Tyr Val Leu Gly His Pro Pro Ile Thr
225                 230                 235                 240
Phe Ser Lys Asp Asp Thr
                245
```

<210> 214
<211> 732
<212> DNA
<213> Methylibium petroleiphilum

<400> 214

```
ttgagcgccg acctgccctg gtctcccgca gaattcgagg cacgcctgcg cgccaaggag      60
tccgggtacc acatccacca ccccttcaac aagcggctca acagcggcgc gctgcagccc     120
ttccaggtgc gcggctgggt ggccaaccgc ttctactacc agcaggcgat cccgatgaag     180
gacgccgcgg tcatggccaa ctgcgaggac cgcgccacgc ggcgccgctg gatcgagcgc     240
atgctcgacc acgacggcca tggcgatcac gaggggcaga acgccggcgg tatcgagacc     300
tggacgcgcc tgggcatggc cgtgggcctg agccgcgagg acctctggtc gcaccgccac     360
gttcagccgg gcgtgcgctt cgcggtcgat gcctacgtga acttcgcgcg ccgcgcgccc     420
tggcgcgagg gcgtgatctc ctcgctgacc gagatgttcg cgcccaagat ccacgccgac     480
cggctggccg gctggcccag catgtacccg tggatcgccg ccgagggcct ggcctatttc     540
cgcagccgca tcccgctggc gcagcgcgac gtggagcacg gcctggaggt cgccatcgcg     600
ttcggcgaca cccgcgcgaa gcaggagcgc gcgatcgaga tcctgcagtt caagctcgac     660
gtgctgtggt cgctgctcga cgccgtggag cgcgcctacc ccgacgacct gcccgaggag     720
cgccgaccgt ga                                                         732
```

<210> 215
<211> 243
<212> PRT
<213> Methylibium petroleiphilum

<400> 215

```
Met Ser Ala Asp Leu Pro Trp Ser Pro Ala Glu Phe Glu Ala Arg Leu
1               5                   10                  15
Arg Ala Lys Glu Ser Gly Tyr His Ile His His Pro Phe Asn Lys Arg
            20                  25                  30
Leu Asn Ser Gly Ala Leu Gln Pro Phe Gln Val Arg Gly Trp Val Ala
        35                  40                  45
Asn Arg Phe Tyr Tyr Gln Gln Ala Ile Pro Met Lys Asp Ala Ala Val
    50                  55                  60
Met Ala Asn Cys Glu Asp Arg Ala Thr Arg Arg Arg Trp Ile Glu Arg
65                  70                  75                  80
Met Leu Asp His Asp Gly His Gly Asp His Glu Gly Gln Asn Ala Gly
                85                  90                  95
Gly Ile Glu Thr Trp Thr Arg Leu Gly Met Ala Val Gly Leu Ser Arg
            100                 105                 110
Glu Asp Leu Trp Ser His Arg His Val Gln Pro Gly Val Arg Phe Ala
        115                 120                 125
Val Asp Ala Tyr Val Asn Phe Ala Arg Arg Ala Pro Trp Arg Glu Gly
    130                 135                 140
Val Ile Ser Ser Leu Thr Glu Met Phe Ala Pro Lys Ile His Ala Asp
145                 150                 155                 160
Arg Leu Ala Gly Trp Pro Ser Met Tyr Pro Trp Ile Ala Ala Glu Gly
                165                 170                 175
Leu Ala Tyr Phe Arg Ser Arg Ile Pro Leu Ala Gln Arg Asp Val Glu
            180                 185                 190
His Gly Leu Glu Val Ala Ile Ala Phe Gly Asp Thr Arg Ala Lys Gln
        195                 200                 205
Glu Arg Ala Ile Glu Ile Leu Gln Phe Lys Leu Asp Val Leu Trp Ser
    210                 215                 220
Leu Leu Asp Ala Val Glu Arg Ala Tyr Pro Asp Asp Leu Pro Glu Glu
225                 230                 235                 240
Arg Arg Pro
```

<210> 216
<211> 639
<212> DNA
<213> Nitrococcus mobilis

<400> 216

```
atgatgatga atcgcggcga actctcgcga caccaactcc agggttgggt tgccaaccgc      60
tattattacc aaatcagcat cccactgaaa gacgcggcac tgatctccca atgtccggat     120
cgggccgtgc ggcggcactg gctgcacagg ctcctcgatc aggacggccg cacgggcagc     180
```

gaagggggga tcgaagcatg gatcaacctc ggcgaggccg tcggcctgag ccggacagag 240
ctgatctccg agcgtcaggt tttgcctggt gttcgattcg cggtggacgc ctatgtaaat 300
ttggtgcgct gggggtcttg gcaggacggg gtgtgcgcct cgctcaccga gctgttcgcg 360
ccgggcatcc atcgcgaacg tctgaacagc tggccgactc actatccctg ggtcgatccc 420
gccggcttag cctattttcg cagccgcctc gaagtagccc ggcaagacgg gcggcacgga 480
ctcgctttgg tactcgatca ttgcgacacc ccggcaagcc aggatcgcgc ggtgcgcatc 540
gtcaagttca aactcgaagt tctctgggcc atgctcgatg cgatgtacct ggcctatgtg 600
gtaggcatgc cgccgttttt taacgtcgcg ggcgactga 639

<210> 217
<211> 212
<212> PRT
<213> Nitrococcus mobilis

<400> 217
Met Met Met Asn Arg Gly Glu Leu Ser Arg His Gln Leu Gln Gly Trp
1 5 10 15
Val Ala Asn Arg Tyr Tyr Tyr Gln Ile Ser Ile Pro Leu Lys Asp Ala
20 25 30
Ala Leu Ile Ser Gln Cys Pro Asp Arg Ala Val Arg Arg His Trp Leu
35 40 45
His Arg Leu Leu Asp Gln Asp Gly Arg Thr Gly Ser Glu Gly Gly Ile
50 55 60
Glu Ala Trp Ile Asn Leu Gly Glu Ala Val Gly Leu Ser Arg Thr Glu
65 70 75 80
Leu Ile Ser Glu Arg Gln Val Leu Pro Gly Val Arg Phe Ala Val Asp
85 90 95
Ala Tyr Val Asn Leu Val Arg Trp Gly Ser Trp Gln Asp Gly Val Cys
100 105 110
Ala Ser Leu Thr Glu Leu Phe Ala Pro Gly Ile His Arg Glu Arg Leu
115 120 125
Asn Ser Trp Pro Thr His Tyr Pro Trp Val Asp Pro Ala Gly Leu Ala
130 135 140
Tyr Phe Arg Ser Arg Leu Glu Val Ala Arg Gln Asp Gly Arg His Gly
145 150 155 160
Leu Ala Leu Val Leu Asp His Cys Asp Thr Pro Ala Ser Gln Asp Arg
165 170 175
Ala Val Arg Ile Val Lys Phe Lys Leu Glu Val Leu Trp Ala Met Leu
180 185 190
Asp Ala Met Tyr Leu Ala Tyr Val Val Gly Met Pro Pro Phe Phe Asn
195 200 205
Val Ala Gly Asp
210

<210> 218
<211> 717
<212> DNA
<213> Mycobacterium smegmatis

<400> 218
tcagcgattt ccataagcct ggtcgatcgc atcgagcatg ctccacagca cgtcgcactt 60
gaaggacaag gccgacagcg cggccgtctg ggtttcctgc gtgacgcaat gccgtcgcac 120
cacctcaaga ccgtgttcgg agtcgcgggg cgcctgggtg atgcgagatc tgaagtaaga 180
gagttctttc gggtcgatcc aggtgtagtg acgttcgaac gccgcgagcc gctcggccat 240
caggtcgggt gcgaacagct cggtcagcga cgaggccacc gcctccagcc atggccgggt 300
gcgggtgaag ttcacgtagg cgtcgacggc gaagcgcacg cccggcagca ggtgtcgtgc 360
gtcttcgacc tccgccctgg tcaaaccgac cgcttcggcc agtcgcagcc acgcctcgat 420
tcccccggtt ccttcggtcg tgccgtcgtg gtcgatgatt cgctggaccc aacggcgccg 480
tatctcacgg tccgggcagg tgctgaggat cgctgcgtct ttccgggga tgttcacctg 540
gtagtagaag cggttggcca cccacccgcg gatctcctcg ggctgcact gcccggtgtt 600
catccgacgg tggaagggat gcaggtggtg gtaatccttg acgtgctcgc gaagcgcttc 660
ctcgaactcg tcgggcgtca gtgcctgggt tactgctcct cgcgccagcg gtgccat 717

<210> 219
<211> 238
<212> PRT
<213> Mycobacterium smegmatis

<400> 219
Met Ala Pro Leu Ala Arg Gly Ala Val Thr Gln Ala Leu Thr Pro Asp
1 5 10 15
Glu Phe Glu Glu Ala Leu Arg Glu His Val Lys Asp Tyr His His Leu
20 25 30
His Pro Phe His Arg Arg Met Asn Thr Gly Gln Cys Ser Pro Glu Glu

```
        35                  40                  45
Ile Arg Gly Trp Val Ala Asn Arg Phe Tyr Tyr Gln Val Asn Ile Pro
    50                  55                  60
Arg Lys Asp Ala Ala Ile Leu Ser Thr Cys Pro Asp Arg Glu Ile Arg
65                  70                  75                  80
Arg Arg Trp Val Gln Arg Ile Ile Asp His Asp Gly Thr Thr Glu Gly
                85                  90                  95
Thr Gly Gly Ile Glu Ala Trp Leu Arg Leu Ala Glu Ala Val Gly Leu
            100                 105                 110
Thr Arg Ala Glu Val Glu Asp Ala Arg His Leu Leu Pro Gly Val Arg
        115                 120                 125
Phe Ala Val Asp Ala Tyr Val Asn Phe Thr Arg Thr Arg Pro Trp Leu
    130                 135                 140
Glu Ala Val Ala Ser Ser Leu Thr Glu Leu Phe Ala Pro Asp Leu Met
145                 150                 155                 160
Ala Glu Arg Leu Ala Ala Phe Glu Arg His Tyr Thr Trp Ile Asp Pro
                165                 170                 175
Lys Glu Leu Ser Tyr Phe Arg Ser Arg Ile Thr Gln Ala Pro Arg Asp
            180                 185                 190
Ser Glu His Gly Leu Glu Val Val Arg Arg His Cys Val Thr Gln Glu
        195                 200                 205
Thr Gln Thr Ala Ala Leu Ser Ala Leu Ser Phe Lys Cys Asp Val Leu
    210                 215                 220
Trp Ser Met Leu Asp Ala Ile Asp Gln Ala Tyr Gly Asn Arg
225                 230                 235
```

<210> 220
<211> 690
<212> DNA
<213> Saccharopolyspora erythraea

<400> 220

```
tcagtcggcg tacgcgtggt cgatggcatc gagaatgctc cacaggacat cgcatttgaa      60
cgacagggcc gccacggctc gtgcctgctc gtcggccgac cggcagtgtt cggtcaccac     120
ctgcagggcg tgttcggagt cgcggggagc ctgctccagg cgggcccgga agtaaccgag     180
accgccgggg tcgatccacg gatagcagcg ctcgaacgcg gcgaggcgct gcgccatcag     240
gtccggtgcg aacagctcgg tcaacgacga cgccaccgcc tcggtccacg gccgggtgcg     300
ggcgaaggtg acgtaggcgt cgacggcgaa ccgcacgccg gggacgacat gccgttcgtc     360
gagcacttcc tcgcgggtca ggccgaccgc ctcggccagg tgcaaccagg cgtcgatacc     420
gcctcgcccg cccggcgcgc cgtcgtggtc gaggatccgc cgcacccagc gcctgcgcac     480
ctcgcggtcg gggcagttgg acaggatcgc ggcgtccttg agcgggatgc tctcctggta     540
gtagaagcgg ttggccaccc accccctggat ctggcggcgg ctcagccgtc ccccgttcat     600
cctgacgtgg aacgggtgct ggtcgtggta gcgccgcgac tgcgcgcgca acgccgcgac     660
gaagccgtcg gtgccggtga gcgccgccat                                      690
```

<210> 221
<211> 229
<212> PRT
<213> Saccharopolyspora erythraea

<400> 221

```
Met Ala Ala Leu Thr Gly Thr Asp Gly Phe Val Ala Ala Leu Arg Ala
1               5                   10                  15
Gln Ser Arg Arg Tyr His Asp Gln His Pro Phe His Val Arg Met Asn
            20                  25                  30
Gly Gly Arg Leu Ser Arg Arg Gln Ile Gln Gly Trp Val Ala Asn Arg
        35                  40                  45
Phe Tyr Tyr Gln Glu Ser Ile Pro Leu Lys Asp Ala Ala Ile Leu Ser
    50                  55                  60
Asn Cys Pro Asp Arg Glu Val Arg Arg Arg Trp Val Arg Arg Ile Leu
65                  70                  75                  80
Asp His Asp Gly Ala Pro Gly Gly Arg Gly Gly Ile Asp Ala Trp Leu
                85                  90                  95
His Leu Ala Glu Ala Val Gly Leu Thr Arg Glu Glu Val Leu Asp Glu
            100                 105                 110
Arg His Val Val Pro Gly Val Arg Phe Ala Val Asp Ala Tyr Val Thr
        115                 120                 125
Phe Ala Arg Thr Arg Pro Trp Thr Glu Ala Val Ala Ser Ser Leu Thr
    130                 135                 140
Glu Leu Phe Ala Pro Asp Leu Met Ala Gln Arg Leu Ala Ala Phe Glu
145                 150                 155                 160
Arg Cys Tyr Pro Trp Ile Asp Pro Gly Gly Leu Gly Tyr Phe Arg Ala
                165                 170                 175
Arg Leu Glu Gln Ala Pro Arg Asp Ser Glu His Ala Leu Gln Val Val
```

```
            180                 185                 190
Thr Glu His Cys Arg Ser Ala Asp Glu Gln Ala Arg Ala Val Ala Ala
        195                 200                 205
Leu Ser Phe Lys Cys Asp Val Leu Trp Ser Ile Leu Asp Ala Ile Asp
    210                 215                 220
His Ala Tyr Ala Asp
225
```

<210> 222
<211> 759
<212> DNA
<213> Sagittula stellata

<400> 222

```
atgaccaagg caccgcaatc gcgcgacgcg ttcgaagccc ggctgcgcca gatcggagcg      60
gagcgatatc acgacctgca tccgttccac gaccgtctgc acggtgggca gtgtaccatg     120
gaggaggtgc aggcctgggt catcaaccgc tactactacc agcactcgat cccgatgaag     180
gacgcggcct tcatgtcccg ggtggaggac cctgatttgc gccgcgcgtg gaggtcgcgg     240
atggaggatc acgacggaac cgccgacaac gaaggcggca taaggcgctg gctgcggctg     300
gcggaggcgg tgggtctgga tcccgactac gtctcgacga cagagggcgt cctgcccgca     360
acgaagttcg cggtggacgc ctacgtccgt ttcgtccgcg aaaagaccct gctcgaagcg     420
gtggcggcat cactcacgga actcttcgca ccgaagatcc acgccaaccg catcgaggga     480
ctgctgaaga attacgcctt cgccgacgac tcctcgctgt cgtatttccg caaccggttg     540
aaagaagcgc cgaaggatgt ggcctttggg ctggcctggg tgctggacca tgccgacacc     600
gccgagaagc aggacgtggc ggccagggcg ctgatcttca agacggatgt tctgtggtcc     660
caactcgacg cgctgtgggg cgcctacgtc gagccgcggc gcattccgcc cggcgcgtgg     720
cagcccggaa cggggcagct cttgcggcag gcgtcatga                            759
```

<210> 223
<211> 252
<212> PRT
<213> Sagittula stellata

<400> 223

```
Met Thr Lys Ala Pro Gln Ser Arg Asp Ala Phe Glu Ala Arg Leu Arg
1               5                   10                  15
Gln Ile Gly Ala Glu Arg Tyr His Asp Leu His Pro Phe His Asp Arg
            20                  25                  30
Leu His Gly Gly Gln Cys Thr Met Glu Glu Val Gln Ala Trp Val Ile
        35                  40                  45
Asn Arg Tyr Tyr Tyr Gln His Ser Ile Pro Met Lys Asp Ala Ala Phe
    50                  55                  60
Met Ser Arg Val Glu Asp Pro Asp Leu Arg Arg Ala Trp Arg Ser Arg
65                  70                  75                  80
Met Glu Asp His Asp Gly Thr Ala Asp Asn Glu Gly Gly Ile Arg Arg
                85                  90                  95
Trp Leu Arg Leu Ala Glu Ala Val Gly Leu Asp Pro Asp Tyr Val Ser
            100                 105                 110
Thr Thr Glu Gly Val Leu Pro Ala Thr Lys Phe Ala Val Asp Ala Tyr
        115                 120                 125
Val Arg Phe Val Arg Glu Lys Thr Leu Leu Glu Ala Val Ala Ala Ser
    130                 135                 140
Leu Thr Glu Leu Phe Ala Pro Lys Ile His Ala Asn Arg Ile Glu Gly
145                 150                 155                 160
Leu Leu Lys Asn Tyr Ala Phe Ala Asp Asp Ser Ser Leu Ser Tyr Phe
                165                 170                 175
Arg Asn Arg Leu Lys Glu Ala Pro Lys Asp Val Ala Phe Gly Leu Ala
            180                 185                 190
Trp Val Leu Asp His Ala Asp Thr Ala Glu Lys Gln Asp Val Ala Ala
        195                 200                 205
Arg Ala Leu Ile Phe Lys Thr Asp Val Leu Trp Ser Gln Leu Asp Ala
    210                 215                 220
Leu Trp Gly Ala Tyr Val Glu Pro Arg Arg Ile Pro Pro Gly Ala Trp
225                 230                 235                 240
Gln Pro Gly Thr Gly Gln Leu Leu Arg Gln Ala Ser
                245                 250
```

<210> 224
<211> 771
<212> DNA
<213> Sinorhizobium meliloti

<400> 224

```
gtgacgacgg caactgacag acaagctttc catgcccgcc tgctggagat cggcaaggag      60
```

```
cgctaccatg acaagcatcc gtttcatgcg atgctccatg gcggcggttg cacgacgacg    120
caagtccgcg cctgggtgat caaccgctat tattaccaga gccgcatccc catgaaggat    180
gcggcctttc tgtcgcgttg cgacgacccg gatatgcgcc gggcctggcg ctcccgcatc    240
gaagatcacg acggcggcgt cgaggagggc ggcggcatcc ggcgctggct gcgtctcgcc    300
gaggccgtgg gactcgatcc agcctatgtc ggctccgcaa ggggcgtact gccgtcgacc    360
cggtttgccg tggacgccta tgtttccttt gtgcgtgaga agccgctgct cgaggcggtg    420
gcctcctcgc tgacggagct ttttgcgccg aagatccact cggagcgcat tgccgggctg    480
ctggagcact acgccttcgc cgatgacgcg gcgctcgcct acttccgcca gaggctggcg    540
gaggtgcccc gggatgtcga gttcggcctc gcctatgtcc ttgaccatgc cgacacgcgg    600
gaaaagcagg acgcggccgc tcaggcactc accttcaaga cggatgttct ctgggcccag    660
ctcgatgcac tttattcggc ctatgtcacg ccggggcgca ttccgccggg ggcctgggac    720
ggccgggagg gggtgatccg cgagccgaga gcgcgggagg ccgcggaatg a             771
```

```
<210>  225
<211>  256
<212>  PRT
<213>  Sinorhizobium meliloti

<400>  225
Met Thr Thr Ala Thr Asp Arg Gln Ala Phe His Ala Arg Leu Leu Glu
1               5                   10                  15
Ile Gly Lys Glu Arg Tyr His Asp Lys His Pro Phe His Ala Met Leu
            20                  25                  30
His Gly Gly Gly Cys Thr Thr Thr Gln Val Arg Ala Trp Val Ile Asn
        35                  40                  45
Arg Tyr Tyr Tyr Gln Ser Arg Ile Pro Met Lys Asp Ala Ala Phe Leu
    50                  55                  60
Ser Arg Cys Asp Asp Pro Asp Met Arg Arg Ala Trp Arg Ser Arg Ile
65                  70                  75                  80
Glu Asp His Asp Gly Gly Val Glu Glu Gly Gly Gly Ile Arg Arg Trp
                85                  90                  95
Leu Arg Leu Ala Glu Ala Val Gly Leu Asp Pro Ala Tyr Val Gly Ser
            100                 105                 110
Ala Arg Gly Val Leu Pro Ser Thr Arg Phe Ala Val Asp Ala Tyr Val
        115                 120                 125
Ser Phe Val Arg Glu Lys Pro Leu Leu Glu Ala Val Ala Ser Ser Leu
    130                 135                 140
Thr Glu Leu Phe Ala Pro Lys Ile His Ser Glu Arg Ile Ala Gly Leu
145                 150                 155                 160
Leu Glu His Tyr Ala Phe Ala Asp Asp Ala Ala Leu Ala Tyr Phe Arg
                165                 170                 175
Gln Arg Leu Ala Glu Val Pro Arg Asp Val Glu Phe Gly Leu Ala Tyr
            180                 185                 190
Val Leu Asp His Ala Asp Thr Arg Glu Lys Gln Asp Ala Ala Ala Gln
        195                 200                 205
Ala Leu Thr Phe Lys Thr Asp Val Leu Trp Ala Gln Leu Asp Ala Leu
    210                 215                 220
Tyr Ser Ala Tyr Val Thr Pro Gly Arg Ile Pro Pro Gly Ala Trp Asp
225                 230                 235                 240
Gly Arg Glu Gly Val Ile Arg Glu Pro Arg Ala Arg Glu Ala Ala Glu
                245                 250                 255
```

```
<210>  226
<211>  1416
<212>  DNA
<213>  Oryza sativa

<400>  226
cccacgcgtc cgcccacgcg tccgggacac cagaaacata gtacacttga gctcactcca     60
aactcaaaca ctcacaccaa tggctctcca agttcaggcc gcactcctgc cctctgctct    120
ctctgtcccc aagaagggta acttgagcgc ggtggtgaag gagccggggt tccttagcgt    180
gagcagaagg ccaagaagcc gtcgctggtg gtgagggcgg tggcgacgcg gcgggccggt    240
ggcgagcccc ggcgcgggca cgtcgaaggc ggacgggaag aagacgctgc ggcaggggt     300
ggtggtgatc accggcgcgt cgtcggggct cgggctcgcg gcggcgaagg cgcttggcgg    360
agacggggaa gtggcacgtg gtgatggcgt tccgcgactt tcctgaaggc ggcgacggcg    420
gcgaaggcgg cggggatggc ggcggggagc tacaccgtca tgcacctgga cctcgcctcc    480
ctcgacagcg tccgccagtt cgtggacaac ttccggcgct ccggcatgcc gctcgacgcg    540
ctggtgtgca acgccgcaca tctaccggcc gacggcgcgg caaccgacgt tcaacgccga    600
cgggtacgag atgagcgtcg ggtgaacca  cctgggccac ttcctcctcg cccgcctcat    660
gctcgacgac ctcaagaaat ccgactaccc gtcgcggcgg ctcatcatcc tcggctccat    720
caccggcaac accaacacct tcgccggcaa cgtccctccc aaggccgggc taggcgacct    780
ccgggggctc gccggcgggc tccgcgggca gaacgggtcg gcgatgatcg acggcgcgga    840
gagcttcgac ggcgccaagg cgtacaagga cagcaagatc tgtaacatgc tgacgatgca    900
ggagttccac cggagattcc acgaggagac cgggatcacg ttcgcgtcgc tgtacccggg    960
```

gtgcatcgcg acgacgggct tgttccgcga gcacatcccg ctgttccggc tgctgttccc 1020
gccgttccag cggttcgtga cgaaggggtt cgtgtcggag gcggagtccg ggaagcggct 1080
ggcgcaggtg gtgggcgacc cgagcctgac caagtccggc gtgtactgga gctggaacaa 1140
ggactcggcg tcgttcgaga accagctctc gcaggaggcc agcgacccgg agaaggccag 1200
gaagctctgg gacctcagcg agaagctcgt cggcctcgtc tgagtttatt atttacccat 1260
tcgtttcaac tgttaatttc ttcgggtttt agggggtttc agctttcagt gagagaggcc 1320
tgtcaagtga tgtacaatta gtaatttttt tttacccgac aaatcatgca ataaaaccac 1380
aggcttacat tatcgatttg tccacctaaa ttaagt 1416

<210> 227
<211> 2194
<212> DNA
<213> Oryza sativa

<400> 227
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc tttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 228
<211> 55
<212> DNA
<213> Artificial sequence

<220>
<223> primer: prm08777

<400> 228
ggggacaagt ttgtacaaaa aagcaggctt aaacaatgac tgacacccca atgtc 55

<210> 229
<211> 51
<212> DNA
<213> Artificial sequence

<220>
<223> primer: prm08782

<400> 229
ggggaccact ttgtacaaga aagctgggtt cataaggtga ttcctttatg c 51